# EUROPEAN PATENT APPLICATION

(11) **EP 1 953 235 A2**
(43) Date of publication of application: **06.08.2008**
(21) Application number: 07107662.4
(22) Date of filing: 08.05.2007
(51) Int. Cl.: C12P 13/12, C12N 15/82, C07K 14/415, C07K 16/16

(54) **New genes related to a process for the production of fine chemicals**

(30) Priority: 17.05.2006 US 801017 P
(71) Applicant: Metanomics GmbH, 10589 Berlin (DE)
(72) Inventor: Plesch, Gunnar, 14482 Potsdam (DE); Puzio, Piotr, 13505 Berlin (DE); Blau, Astrid, 14532 Stahnsdorf (DE); Looser, Ralf, 13158 Berlin (DE); Wendel, Birgit, 12163 Berlin (DE); Kamlage, Beate, 10711 Berlin (DE); Schmitz, Oliver, 14624 Dallgow-Döberitz (DE)
(74) Representative: Fitzner, Uwe

(57) **Abstract**

The present invention relates to a process for the production of the fine chemical in a microorganism, a plant cell, a plant, a plant tissue or in one or more parts thereof. The invention furthermore relates to nucleic acid molecules, polypeptides, nucleic acid constructs, vectors, antisense molecules, antibodies, host cells, plant tissue, propagation material, harvested material, plants, microorganisms as well as agricultural compositions and to their use.

SEQUENCE LISTING

<110> Metanomics GmbH

<120> New genes for a process for the production of fine chemicals

<130> PF 58001 EP

<140> Current patent application

<141> 2007 05

<160> 317

<170> Biomax PatentTool according to PatentIN 3.1 format

<210> 1
<211> 1188
<212> DNA
<213> Helianthus annuus

<220>
<221> CDS
<222> (104)..(1063)

<400> 1
attcttcctt cattattacg aatttcactt caatcatcat cttatgcagc ggaaaacgtg 60

ttcatctatc tcaaacaagt tcgtcaaact ttgatcaatt tca atg gcg gaa ccg 115
Met Ala Glu Pro
1
aag aag ata ata att gac acg gat cct ggc atc gat gat gct atg gca 163
Lys Lys Ile Ile Ile Asp Thr Asp Pro Gly Ile Asp Asp Ala Met Ala
5                   10                  15                  20
ata ttt ctg gct ttg agg tca cct gaa gtt aat gtg att ggg ctt aca 211
Ile Phe Leu Ala Leu Arg Ser Pro Glu Val Asn Val Ile Gly Leu Thr
                25                  30                  35
act att tat ggc aat gtt tac acc act ctt gcc act aga aat gca tta 259
Thr Ile Tyr Gly Asn Val Tyr Thr Thr Leu Ala Thr Arg Asn Ala Leu
            40                  45                  50
cat ttg ttg gag gtt gct ggt aga aca gat atc cct gtt gca gaa gga 307
His Leu Leu Glu Val Ala Gly Arg Thr Asp Ile Pro Val Ala Glu Gly
        55                  60                  65
tct cat gtt agt tat gtg aaa gca acg aag ctt cga gtt gca gac ttt 355
Ser His Val Ser Tyr Val Lys Ala Thr Lys Leu Arg Val Ala Asp Phe
    70                  75                  80
gtt cac ggg gta gac ggg ctc gga aac cag aac ttc ccg cag ccg aag 403
Val His Gly Val Asp Gly Leu Gly Asn Gln Asn Phe Pro Gln Pro Lys
85                  90                  95                  100
tca aaa cca ata gaa aag tca gca gca gaa tat ttg gtg gag caa gct 451
Ser Lys Pro Ile Glu Lys Ser Ala Ala Glu Tyr Leu Val Glu Gln Ala
                105                 110                 115
cgt ctt tac ccc gga gaa atc act ttg gtc gca ttg ggt ccc ctt acc 499
Arg Leu Tyr Pro Gly Glu Ile Thr Leu Val Ala Leu Gly Pro Leu Thr
            120                 125                 130
aac atc gcc ttg gcc att gaa ctc gac ccc gcc ttc acc aaa aac att 547
Asn Ile Ala Leu Ala Ile Glu Leu Asp Pro Ala Phe Thr Lys Asn Ile
        135                 140                 145
gga cag att gtt ctt ctt ggt ggt gcg ttt tta gta aac ggg aat gtg 595
Gly Gln Ile Val Leu Leu Gly Gly Ala Phe Leu Val Asn Gly Asn Val
    150                 155                 160
aat cca gct agc gag gct aat att ttt ggt gat ccg gaa gct gca gat 643
Asn Pro Ala Ser Glu Ala Asn Ile Phe Gly Asp Pro Glu Ala Ala Asp
165                 170                 175                 180
att gtg ttt act tgc ggt gct gat gtg ttg gct att ggg ctc aat gtt 691
Ile Val Phe Thr Cys Gly Ala Asp Val Leu Ala Ile Gly Leu Asn Val
                185                 190                 195

act cat caa gtt att ctt aca gat cat gat cgt gac atc ttg gca gaa 739
Thr His Gln Val Ile Leu Thr Asp His Asp Arg Asp Ile Leu Ala Glu
            200                 205                 210
tca gat gga ata ttt gcc aag tat ttg agc aag atc ctg gaa ttt tac 787
Ser Asp Gly Ile Phe Ala Lys Tyr Leu Ser Lys Ile Leu Glu Phe Tyr
        215                 220                 225
ttt tct tat cat cgc gat gca tac aac atg aaa ggt gta tac ctt cat 835
Phe Ser Tyr His Arg Asp Ala Tyr Asn Met Lys Gly Val Tyr Leu His
    230                 235                 240
gac ccg aca acg att ctt gca gct gtg aat cct tca tta atg aca tac 883
Asp Pro Thr Thr Ile Leu Ala Ala Val Asn Pro Ser Leu Met Thr Tyr
245                 250                 255                 260
acg gaa ggt gtt gtc aga gtc cag aca act ggt atc aca agg ggc tta 931
Thr Glu Gly Val Val Arg Val Gln Thr Thr Gly Ile Thr Arg Gly Leu
                265                 270                 275
tcg ttg ttt ttc aac aaa cag aaa agg ttt aac gaa gag act gaa tgg 979
Ser Leu Phe Phe Asn Lys Gln Lys Arg Phe Asn Glu Glu Thr Glu Trp
            280                 285                 290
tgc aac aaa ccg aca gtg aag gtg gca gtt act gtg gac gct ccc gag 1027
Cys Asn Lys Pro Thr Val Lys Val Ala Val Thr Val Asp Ala Pro Glu
        295                 300                 305
gta gtt aaa ttg gtt atg gaa cgt ctt atg tat tga taactaacat 1073
Val Val Lys Leu Val Met Glu Arg Leu Met Tyr
310 315 320
ccccaactct ctatttcaaa gcctctgttt ctcaagagga accctaaagg ttcaaaaatg 1133

atatattttc tgtatttgtt ttattaatca attagcagcg aaaaaaaaaa aaaaa 1188

<210> 2
<211> 319
<212> PRT
<213> Helianthus annuus

<400> 2
Met Ala Glu Pro Lys Lys Ile Ile Ile Asp Thr Asp Pro Gly Ile Asp
1               5                   10                  15
Asp Ala Met Ala Ile Phe Leu Ala Leu Arg Ser Pro Glu Val Asn Val
            20                  25                  30
Ile Gly Leu Thr Thr Ile Tyr Gly Asn Val Tyr Thr Thr Leu Ala Thr
        35                  40                  45
Arg Asn Ala Leu His Leu Leu Glu Val Ala Gly Arg Thr Asp Ile Pro
    50                  55                  60
Val Ala Glu Gly Ser His Val Ser Tyr Val Lys Ala Thr Lys Leu Arg
65                  70                  75                  80
Val Ala Asp Phe Val His Gly Val Asp Gly Leu Gly Asn Gln Asn Phe
                85                  90                  95
Pro Gln Pro Lys Ser Lys Pro Ile Glu Lys Ser Ala Ala Glu Tyr Leu
            100                 105                 110
Val Glu Gln Ala Arg Leu Tyr Pro Gly Glu Ile Thr Leu Val Ala Leu
        115                 120                 125
Gly Pro Leu Thr Asn Ile Ala Leu Ala Ile Glu Leu Asp Pro Ala Phe
    130                 135                 140
Thr Lys Asn Ile Gly Gln Ile Val Leu Leu Gly Gly Ala Phe Leu Val
145                 150                 155                 160
Asn Gly Asn Val Asn Pro Ala Ser Glu Ala Asn Ile Phe Gly Asp Pro
                165                 170                 175
Glu Ala Ala Asp Ile Val Phe Thr Cys Gly Ala Asp Val Leu Ala Ile
            180                 185                 190
Gly Leu Asn Val Thr His Gln Val Ile Leu Thr Asp His Asp Arg Asp
        195                 200                 205
Ile Leu Ala Glu Ser Asp Gly Ile Phe Ala Lys Tyr Leu Ser Lys Ile
    210                 215                 220
Leu Glu Phe Tyr Phe Ser Tyr His Arg Asp Ala Tyr Asn Met Lys Gly
225                 230                 235                 240
Val Tyr Leu His Asp Pro Thr Thr Ile Leu Ala Ala Val Asn Pro Ser
                245                 250                 255

Leu Met Thr Tyr Thr Glu Gly Val Val Arg Val Gln Thr Thr Gly Ile
            260                 265                 270
Thr Arg Gly Leu Ser Leu Phe Phe Asn Lys Gln Lys Arg Phe Asn Glu
        275                 280                 285
Glu Thr Glu Trp Cys Asn Lys Pro Thr Val Lys Val Ala Val Thr Val
    290                 295                 300
Asp Ala Pro Glu Val Val Lys Leu Val Met Glu Arg Leu Met Tyr
305                 310                 315

<210> 3
<211> 28
<212> DNA
<213> Artificial sequence

<400> 3
atggcggaac cgaagaagat aataattg                                          28

<210> 4
<211> 24
<212> DNA
<213> Artificial sequence

<400> 4
tcaatacata agacgttcca taac                                              24


<210> 5
<211> 567
<212> PRT
<213> Artificial sequence

<220>
<221> Variant
<222> (2)..(2)
<223> xaa in position 2 is any amino acid
<220>
<221> Variant
<222> (4)..(4)
<223> xaa in position 4 is any amino acid
<220>
<221> Variant
<222> (6)..(6)
<223> xaa in position 6 is any amino acid
<220>
<221> Variant
<222> (8)..(22)
<223> xaa in position 8 to 22 is any or no amino acid
<220>
<221> Variant
<222> (24)..(25)
<223> xaa in position 24 to 25 is any amino acid
<220>
<221> Variant
<222> (27)..(44)
<223> xaa in position 27 to 44 is any amino acid
<220>
<221> Variant
<222> (45)..(59)
<223> xaa in position 45 to 59 is any or no amino acid
<220>
<221> Variant
<222> (62)..(69)
<223> xaa in position 62 to 69 is any amino acid
<220>
<221> Variant
<222> (71)..(84)
<223> xaa in position 71 to 84 is any amino acid
<220>
<221> Variant

<222> (85)..(109)
<223> xaa in position 85 to 109 is any or no amino acid
<220>
<221> Variant
<222> (111)..(120)
<223> xaa in position 111 to 120 is any amino acid
<220>
<221> Variant
<222> (121)..(161)
<223> xaa in position 121 to 161 is any or no amino acid
<220>
<221> Variant
<222> (164)..(165)
<223> xaa in position 164 to 165 is any amino acid
<220>
<221> Variant
<222> (167)..(195)
<223> xaa in position 167 to 195 is any amino acid
<220>
<221> Variant
<222> (196)..(242)
<223> xaa in position 196 to 242 is any or no amino acid
<220>
<221> Variant
<222> (244)..(245)
<223> xaa in position 244 to 245 is any amino acid
<220>
<221> Variant
<222> (248)..(248)
<223> xaa in position 248 is any amino acid
<220>
<221> Variant
<222> (250)..(255)
<223> xaa in position 250 to 255 is any amino acid
<220>
<221> Variant
<222> (256)..(256)
<223> xaa in position 256 is any or no amino acid
<220>
<221> Variant
<222> (258)..(268)
<223> xaa in position 258 to 268 is any amino acid
<220>
<221> Variant
<222> (272)..(276)
<223> xaa in position 272 to 276 is any amino acid
<220>
<221> Variant
<222> (277)..(293)
<223> xaa in position 277 to 293 is any or no amino acid
<220>
<221> Variant
<222> (295)..(298)
<223> xaa in position 295 to 298 is any amino acid
<220>
<221> Variant
<222> (301)..(301)
<223> xaa in position 301 is any amino acid
<220>
<221> Variant
<222> (303)..(305)
<223> xaa in position 303 to 305 is any amino acid
<220>
<221> Variant
<222> (308)..(308)
<223> xaa in position 308 is any amino acid
<220>
<221> Variant
<222> (311)..(312)

<223> xaa in position 311 to 312 is any amino acid
<220>
<221> Variant
<222> (314)..(363)
<223> xaa in position 314 to 363 is any amino acid
<220>
<221> Variant
<222> (364)..(454)
<223> xaa in position 364 to 454 is any or no amino acid
<220>
<221> Variant
<222> (456)..(474)
<223> xaa in position 456 to 474 is any amino acid
<220>
<221> Variant
<222> (475)..(566)
<223> xaa in position 475 to 566 is any or no amino acid

<400> 5
Asp xaa Asp xaa Gly xaa Asp xaa xaa xaa xaa xaa xaa xaa xaa xaa
1               5                   10                  15
xaa xaa xaa xaa xaa xaa Asp xaa xaa Ala xaa xaa xaa xaa xaa xaa
            20                  25                  30
xaa xaa xaa xaa xaa xaa xaa xaa xaa xaa xaa xaa xaa xaa xaa xaa
        35                  40                  45
xaa xaa xaa xaa xaa xaa xaa xaa xaa xaa xaa Gly Asn xaa xaa xaa
    50                  55                  60
xaa xaa xaa xaa xaa Asn xaa xaa xaa xaa xaa xaa xaa xaa xaa xaa
65                  70                  75                  80
xaa xaa xaa xaa xaa xaa xaa xaa xaa xaa xaa xaa xaa xaa xaa xaa
                85                  90                  95
xaa xaa xaa xaa xaa xaa xaa xaa xaa xaa xaa xaa Gly xaa xaa
            100                 105                 110
xaa xaa xaa xaa xaa xaa xaa xaa xaa xaa xaa xaa xaa xaa xaa xaa
        115                 120                 125
xaa xaa xaa xaa xaa xaa xaa xaa xaa xaa xaa xaa xaa xaa xaa xaa
    130                 135                 140
xaa xaa xaa xaa xaa xaa xaa xaa xaa xaa xaa xaa xaa xaa xaa xaa
145                 150                 155                 160
xaa His Gly xaa xaa Gly xaa xaa xaa xaa xaa xaa xaa xaa xaa xaa
                165                 170                 175
xaa xaa xaa xaa xaa xaa xaa xaa xaa xaa xaa xaa xaa xaa xaa xaa
            180                 185                 190
xaa xaa xaa xaa xaa xaa xaa xaa xaa xaa xaa xaa xaa xaa xaa xaa
        195                 200                 205
xaa xaa xaa xaa xaa xaa xaa xaa xaa xaa xaa xaa xaa xaa xaa xaa
    210                 215                 220
xaa xaa xaa xaa xaa xaa xaa xaa xaa xaa xaa xaa xaa xaa xaa xaa
225                 230                 235                 240
xaa xaa Gly xaa xaa Thr Asn xaa Ala xaa xaa xaa xaa xaa xaa
                245                 250                 255
Pro xaa xaa xaa xaa xaa xaa xaa xaa xaa xaa xaa Met Gly Gly xaa
            260                 265                 270
xaa xaa xaa xaa xaa xaa xaa xaa xaa xaa xaa xaa xaa xaa xaa xaa
        275                 280                 285
xaa xaa xaa xaa xaa Asn xaa xaa xaa xaa Ala Glu xaa Asn xaa xaa
    290                 295                 300
xaa Asp Pro xaa Ala Ala xaa xaa Val xaa xaa xaa xaa xaa xaa xaa
305                 310                 315                 320
xaa xaa xaa xaa xaa xaa xaa xaa xaa xaa xaa xaa xaa xaa xaa xaa
                325                 330                 335
xaa xaa xaa xaa xaa xaa xaa xaa xaa xaa xaa xaa xaa xaa xaa xaa
            340                 345                 350
xaa xaa xaa xaa xaa xaa xaa xaa xaa xaa xaa xaa xaa xaa xaa xaa
        355                 360                 365
xaa xaa xaa xaa xaa xaa xaa xaa xaa xaa xaa xaa xaa xaa xaa xaa
    370                 375                 380
xaa xaa xaa xaa xaa xaa xaa xaa xaa xaa xaa xaa xaa xaa xaa xaa
385                 390                 395                 400

Xaa Xaa Xaa Xaa Xaa Xaa Xaa Xaa Xaa Xaa Xaa Xaa Xaa Xaa Xaa Xaa
                405                 410                 415
Xaa Xaa Xaa Xaa Xaa Xaa Xaa Xaa Xaa Xaa Xaa Xaa Xaa Xaa Xaa Xaa
            420                 425                 430
Xaa Xaa Xaa Xaa Xaa Xaa Xaa Xaa Xaa Xaa Xaa Xaa Xaa Xaa Xaa Xaa
        435                 440                 445
Xaa Xaa Xaa Xaa Xaa Xaa Asp Xaa Xaa Xaa Xaa Xaa Xaa Xaa Xaa Xaa
    450                 455                 460
Xaa Xaa Xaa Xaa Xaa Xaa Xaa Xaa Xaa Xaa Xaa Xaa Xaa Xaa Xaa Xaa
465                 470                 475                 480
Xaa Xaa Xaa Xaa Xaa Xaa Xaa Xaa Xaa Xaa Xaa Xaa Xaa Xaa Xaa Xaa
                485                 490                 495
Xaa Xaa Xaa Xaa Xaa Xaa Xaa Xaa Xaa Xaa Xaa Xaa Xaa Xaa Xaa Xaa
            500                 505                 510
Xaa Xaa Xaa Xaa Xaa Xaa Xaa Xaa Xaa Xaa Xaa Xaa Xaa Xaa Xaa Xaa
        515                 520                 525
Xaa Xaa Xaa Xaa Xaa Xaa Xaa Xaa Xaa Xaa Xaa Xaa Xaa Xaa Xaa Xaa
    530                 535                 540
Xaa Xaa Xaa Xaa Xaa Xaa Xaa Xaa Xaa Xaa Xaa Xaa Xaa Xaa Xaa Xaa
545                 550                 555                 560
Xaa Xaa Xaa Xaa Xaa Xaa Gly
                565

<210> 6
<211> 73
<212> PRT
<213> Artificial sequence

<220>
<221> Variant
<222> (1)..(1)
<223> Xaa in position 1 is Ile, Val, Leu, Met, Phe or Ala
<220>
<221> Variant
<222> (2)..(3)
<223> Xaa in position 2 to 3 is any amino acid
<220>
<221> Variant
<222> (4)..(4)
<223> Xaa in position 4 is Asp, Phe, Ser or Asn
<220>
<221> Variant
<222> (5)..(5)
<223> Xaa in position 5 is any amino acid
<220>
<221> Variant
<222> (6)..(6)
<223> Xaa in position 6 is Asp or Asn
<220>
<221> Variant
<222> (7)..(7)
<223> Xaa in position 7 is any amino acid
<220>
<221> Variant
<222> (8)..(8)
<223> Xaa in position 8 is Gly, Ala or Ser
<220>
<221> Variant
<222> (9)..(9)
<223> Xaa in position 9 is any amino acid
<220>
<221> Variant
<222> (10)..(10)
<223> Xaa in position 10 is Asp, Gly or Glu
<220>
<221> Variant
<222> (11)..(25)
<223> Xaa in position 11 to 25 is any or no amino acid

<220>
<221> Variant
<222> (26)..(26)
<223> Xaa in position 26 is Asp or Ser
<220>
<221> Variant
<222> (27)..(28)
<223> Xaa in position 27 to 28 is any amino acid
<220>
<221> Variant
<222> (29)..(29)
<223> Xaa in position 29 is Ala, Ser or Thr
<220>
<221> Variant
<222> (30)..(30)
<223> Xaa in position 30 is Ile, Leu, Met, Thr or Val
<220>
<221> Variant
<222> (31)..(35)
<223> Xaa in position 31 to 35 is any amino acid
<220>
<221> Variant
<222> (36)..(53)
<223> Xaa in position 36 to 53 is any or no amino acid
<220>
<221> Variant
<222> (54)..(54)
<223> Xaa in position 54 is Leu, Val, Ile, Glu or Pro
<220>
<221> Variant
<222> (55)..(56)
<223> Xaa in position 55 to 56 is any amino acid
<220>
<221> Variant
<222> (57)..(57)
<223> Xaa in position 57 is Ile, Val, Leu or Met
<220>
<221> Variant
<222> (58)..(61)
<223> Xaa in position 58 to 61 is any amino acid
<220>
<221> Variant
<222> (62)..(62)
<223> Xaa in position 62 is any or no amino acid
<220>
<221> Variant
<222> (63)..(63)
<223> Xaa in position 63 is Gly, Asp or Ala
<220>
<221> Variant
<222> (64)..(64)
<223> Xaa in position 64 is Asn, Asp or Thr
<220>
<221> Variant
<222> (65)..(72)
<223> Xaa in position 65 to 72 is any amino acid
<220>
<221> Variant
<222> (73)..(73)
<223> Xaa in position 73 is Asn, Val, Ala, Arg or His

<400> 6
Xaa Xaa Xaa Xaa Xaa Xaa Xaa Xaa Xaa Xaa Xaa Xaa Xaa Xaa Xaa Xaa
1               5                   10                  15
Xaa Xaa Xaa Xaa Xaa Xaa Xaa Xaa Xaa Xaa Xaa Xaa Xaa Xaa Xaa Xaa
            20                  25                  30
Xaa Xaa Xaa Xaa Xaa Xaa Xaa Xaa Xaa Xaa Xaa Xaa Xaa Xaa Xaa Xaa
        35                  40                  45
Xaa Xaa Xaa Xaa Xaa Xaa Xaa Xaa Xaa Xaa Xaa Xaa Xaa Xaa Xaa Xaa

50                  55                  60
Xaa Xaa Xaa Xaa Xaa Xaa Xaa Xaa Xaa
65                  70

<210> 7
<211> 71
<212> PRT
<213> Artificial sequence

<220>
<221> Variant
<222> (1)..(1)
<223> Xaa in position 1 is Gly, Ala or Ser
<220>
<221> Variant
<222> (2)..(3)
<223> Xaa in position 2 to 3 is any amino acid
<220>
<221> Variant
<222> (4)..(4)
<223> Xaa in position 4 is Thr or Ser
<220>
<221> Variant
<222> (5)..(5)
<223> Xaa in position 5 is any amino acid
<220>
<221> Variant
<222> (6)..(6)
<223> Xaa in position 6 is Leu, Ile, Val, Ala, Met or Phe
<220>
<221> Variant
<222> (7)..(7)
<223> Xaa in position 7 is Ala, Ser or Gly
<220>
<221> Variant
<222> (8)..(24)
<223> Xaa in position 8 to 24 is any amino acid
<220>
<221> Variant
<222> (25)..(26)
<223> Xaa in position 25 to 26 is any or no amino acid
<220>
<221> Variant
<222> (27)..(27)
<223> Xaa in position 27 is Met, Leu or Val
<220>
<221> Variant
<222> (28)..(28)
<223> Xaa in position 28 is Gly, Ala or Ser
<220>
<221> Variant
<222> (30)..(37)
<223> Xaa in position 30 to 37 is any amino acid
<220>
<221> Variant
<222> (38)..(57)
<223> Xaa in position 38 to 57 is any or no amino acid
<220>
<221> Variant
<222> (58)..(58)
<223> Xaa in position 58 is Glu or Asp
<220>
<221> Variant
<222> (59)..(59)
<223> Xaa in position 59 is any amino acid
<220>
<221> Variant
<222> (61)..(63)

<223> xaa in position 61 to 63 is any amino acid
<220>
<221> Variant
<222> (65)..(65)
<223> xaa in position 65 is Pro, Ala or Val
<220>
<221> Variant
<222> (66)..(66)
<223> xaa in position 66 is any amino acid
<220>
<221> Variant
<222> (67)..(67)
<223> xaa in position 67 is Ala, Ser or Gly
<220>
<221> Variant
<222> (68)..(68)
<223> xaa in position 68 is Ala, Val, Ser, Thr or Leu
<220>
<221> Variant
<222> (69)..(70)
<223> xaa in position 69 to 70 is any amino acid
<220>
<221> Variant
<222> (71)..(71)
<223> xaa in position 71 is Val, Leu, Ile, Met or Phe

<400> 7
xaa xaa xaa xaa xaa xaa xaa xaa xaa xaa xaa xaa xaa xaa xaa xaa
1               5                   10                  15
xaa xaa xaa xaa xaa xaa xaa xaa xaa xaa xaa xaa Gly xaa xaa xaa
            20                  25                  30
xaa xaa xaa xaa xaa xaa xaa xaa xaa xaa xaa xaa xaa xaa xaa xaa
        35                  40                  45
xaa xaa xaa xaa xaa xaa xaa xaa xaa xaa xaa Asn xaa xaa xaa Asp
    50                  55                  60
xaa xaa xaa xaa xaa xaa xaa
65                  70

<210> 8
<211> 1176
<212> DNA
<213> Glycine max

<220>
<221> CDS
<222> (74)..(883)

<400> 8
cacataagca attaattaag cactctaaaa catatcttta tttccttgtc tacatatctt      60

gaaaaactca atc atg gct agt gtt tct tcg gtt tta gct cca ttt aga      109
               Met Ala Ser Val Ser Ser Val Leu Ala Pro Phe Arg
               1               5                   10
agg ctt gat ggg aag gtg gcg att atc act ggt ggt gcg agt ggt cta      157
Arg Leu Asp Gly Lys Val Ala Ile Ile Thr Gly Gly Ala Ser Gly Leu
        15                  20                  25
ggt gca gcc act gca aga ctc ttc tct aag cat gga gca tat gta gtc      205
Gly Ala Ala Thr Ala Arg Leu Phe Ser Lys His Gly Ala Tyr Val Val
    30                  35                  40
ata gct gat att caa gac gac ttg ggt ctc tct gtt gcc aaa gag tta      253
Ile Ala Asp Ile Gln Asp Asp Leu Gly Leu Ser Val Ala Lys Glu Leu
45                  50                  55                  60
gaa tct gct tcc tat gtc cat tgc gat gtg aca aag gaa gag gac gtt      301
Glu Ser Ala Ser Tyr Val His Cys Asp Val Thr Lys Glu Glu Asp Val
                65                  70                  75
gaa aac tgc gtg aac aca acg gtt tcc aag tat ggc aaa tta gat atc      349
Glu Asn Cys Val Asn Thr Thr Val Ser Lys Tyr Gly Lys Leu Asp Ile
            80                  85                  90

atg ttt aac aat gca ggt gta tct gat gag atc aaa aca agc att ctt     397
Met Phe Asn Asn Ala Gly Val Ser Asp Glu Ile Lys Thr Ser Ile Leu
        95                  100                 105
gac aac aac aag tct gat ttt gag aga gtg ata agt gtt aac ttg gtt     445
Asp Asn Asn Lys Ser Asp Phe Glu Arg Val Ile Ser Val Asn Leu Val
    110                 115                 120
ggt cct ttt ctg gga aca aag cat gct gca agg gtc atg att cct gct     493
Gly Pro Phe Leu Gly Thr Lys His Ala Ala Arg Val Met Ile Pro Ala
125                 130                 135                 140
aaa aag gga tgc ata atc aac aca gct agt gtt gct gga tgc ata ggt     541
Lys Lys Gly Cys Ile Ile Asn Thr Ala Ser Val Ala Gly Cys Ile Gly
                145                 150                 155
gga ggt gct aca cat gcc tac aca agt tca aag cac gca cta att gga     589
Gly Gly Ala Thr His Ala Tyr Thr Ser Ser Lys His Ala Leu Ile Gly
            160                 165                 170
ctg aca aaa aac act gcg gtg gag ctt gga caa cat ggt att agg gta     637
Leu Thr Lys Asn Thr Ala Val Glu Leu Gly Gln His Gly Ile Arg Val
        175                 180                 185
aat tgt ttg tca cct tat ctt gtt gtc aca ccg tta agt aag aaa tat     685
Asn Cys Leu Ser Pro Tyr Leu Val Val Thr Pro Leu Ser Lys Lys Tyr
    190                 195                 200
ttc aat att gat gaa gac aaa att cgt gag ata tat tca aac cta aaa     733
Phe Asn Ile Asp Glu Asp Lys Ile Arg Glu Ile Tyr Ser Asn Leu Lys
205                 210                 215                 220
ggt gct cat ctt gtg cct aac gat gtg gcc gaa gct gct ctt tac ttg     781
Gly Ala His Leu Val Pro Asn Asp Val Ala Glu Ala Ala Leu Tyr Leu
                225                 230                 235
gca ggt gat gag tcc aag tat gtt agt ggt cac aat ctt gtg ata gat     829
Ala Gly Asp Glu Ser Lys Tyr Val Ser Gly His Asn Leu Val Ile Asp
            240                 245                 250
gga ggg tac act gat gta aat gca gga ttt acc gtg ttt ggg cag tct     877
Gly Gly Tyr Thr Asp Val Asn Ala Gly Phe Thr Val Phe Gly Gln Ser
        255                 260                 265
cag taa aaattgtagc tgtgtcttca aagttaaatc attattgttg gtgtttgaag     933
Gln
    270
tgtctgtgtt tcgatggccc tgtagttcag aaacttgagc tgttagcatg tgtgaagaaa     993

aagaaggcaa attaatacag ggtgatatga tacctgcctt actgattatc gtggatgtgt     1053

gtgaagtgtc tgtattcgtg ctgttttggc tttcgtgact ctgtttcgat tgctttgaag     1113

ttcaaaaact ttataggatt atattaatat atatgcctca ctcttagcta aaaaaaaaaa     1173

aaa                                                                   1176

<210> 9
<211> 269
<212> PRT
<213> Glycine max

<400> 9
Met Ala Ser Val Ser Ser Val Leu Ala Pro Phe Arg Arg Leu Asp Gly
1               5                   10                  15
Lys Val Ala Ile Ile Thr Gly Gly Ala Ser Gly Leu Gly Ala Ala Thr
            20                  25                  30
Ala Arg Leu Phe Ser Lys His Gly Ala Tyr Val Val Ile Ala Asp Ile
        35                  40                  45
Gln Asp Asp Leu Gly Leu Ser Val Ala Lys Glu Leu Glu Ser Ala Ser
    50                  55                  60
Tyr Val His Cys Asp Val Thr Lys Glu Glu Asp Val Glu Asn Cys Val
65                  70                  75                  80
Asn Thr Thr Val Ser Lys Tyr Gly Lys Leu Asp Ile Met Phe Asn Asn

Ala Gly Val Ser Asp Glu Ile Lys Thr Ser Ile Leu Asp Asn Asn Lys
                85                  90                  95
Ser Asp Phe Glu Arg Val Ile Ser Val Asn Leu Val Gly Pro Phe Leu
            100                 105                 110
Gly Thr Lys His Ala Ala Arg Val Met Ile Pro Ala Lys Lys Gly Cys
        115                 120                 125
Ile Ile Asn Thr Ala Ser Val Ala Gly Cys Ile Gly Gly Gly Ala Thr
    130                 135                 140
His Ala Tyr Thr Ser Ser Lys His Ala Leu Ile Gly Leu Thr Lys Asn
145                 150                 155                 160
Thr Ala Val Glu Leu Gly Gln His Gly Ile Arg Val Asn Cys Leu Ser
                165                 170                 175
Pro Tyr Leu Val Val Thr Pro Leu Ser Lys Lys Tyr Phe Asn Ile Asp
            180                 185                 190
Glu Asp Lys Ile Arg Glu Ile Tyr Ser Asn Leu Lys Gly Ala His Leu
        195                 200                 205
Val Pro Asn Asp Val Ala Glu Ala Ala Leu Tyr Leu Ala Gly Asp Glu
    210                 215                 220
Ser Lys Tyr Val Ser Gly His Asn Leu Val Ile Asp Gly Gly Tyr Thr
225                 230                 235                 240
Asp Val Asn Ala Gly Phe Thr Val Phe Gly Gln Ser Gln
                245                 250                 255
            260                 265

<210> 10
<211> 1143
<212> DNA
<213> Glycine max

<220>
<221> CDS
<222> (79)..(927)

<400> 10
ccattcctga gtttccattg ccgcgacatc tgaattcctc cttgtcactc ataatcacca      60

aatagaagcc ataaacaa atg aat gcc ata gca gca gaa caa gtg ctg gag       111
                    Met Asn Ala Ile Ala Ala Glu Gln Val Leu Glu
                    1               5                   10
cca tgg cac aag cta gac gac aaa gtc gtt ttg gtg acg ggt gct tcc       159
Pro Trp His Lys Leu Asp Asp Lys Val Val Leu Val Thr Gly Ala Ser
            15                  20                  25
tcg ggg ctc ggg cga gat ttc tgc atc gac ctc gcc aaa gct ggc tgc       207
Ser Gly Leu Gly Arg Asp Phe Cys Ile Asp Leu Ala Lys Ala Gly Cys
        30                  35                  40
tgt gtc gtc gcg gca gct cgt cgc ctc gat cgc ctc act tcc ctc tgc       255
Cys Val Val Ala Ala Ala Arg Arg Leu Asp Arg Leu Thr Ser Leu Cys
    45                  50                  55
cac gaa atc aac cac cga tgg ccc tcg aac gtc gga atc cac cgc gcg       303
His Glu Ile Asn His Arg Trp Pro Ser Asn Val Gly Ile His Arg Ala
60                  65                  70                  75
gtg gcg gtg gag ctt gat gtc gcc gcc gat ggc ccc gcc atc gac agg       351
Val Ala Val Glu Leu Asp Val Ala Ala Asp Gly Pro Ala Ile Asp Arg
                80                  85                  90
gct gtg cag aag gcc tgg gac gcc ttt ggc cgc gtt gat tcc ttg att       399
Ala Val Gln Lys Ala Trp Asp Ala Phe Gly Arg Val Asp Ser Leu Ile
            95                  100                 105
aac aac gct ggt gtc aga gga agt gtt aaa tca ccc ttg aaa ttg tct       447
Asn Asn Ala Gly Val Arg Gly Ser Val Lys Ser Pro Leu Lys Leu Ser
        110                 115                 120
gaa gag gaa tgg gat cat gtc ttc aag act aac cta act ggt tgt tgg       495
Glu Glu Glu Trp Asp His Val Phe Lys Thr Asn Leu Thr Gly Cys Trp
    125                 130                 135
ttg gtg tca aaa tat gta tgc aaa cgc atg tgt gat atc cag ctt aag       543
Leu Val Ser Lys Tyr Val Cys Lys Arg Met Cys Asp Ile Gln Leu Lys
140                 145                 150                 155
gga tca att att aat att tct tca gtt tct ggt tta aat cgg ggg caa       591
Gly Ser Ile Ile Asn Ile Ser Ser Val Ser Gly Leu Asn Arg Gly Gln

                  160                     165                     170
ttg cct gga gct gct gca tat gca tct tcg aag gca ggg gta aac atg         639
Leu Pro Gly Ala Ala Ala Tyr Ala Ser Ser Lys Ala Gly Val Asn Met
            175                     180                     185
ctg act aag gtc atg gct atg gaa ttg ggg atg cac aaa att aga gta         687
Leu Thr Lys Val Met Ala Met Glu Leu Gly Met His Lys Ile Arg Val
        190                     195                     200
aat tcc ata tcc cct gga att ttc aaa tct gaa ata act gaa aat tta         735
Asn Ser Ile Ser Pro Gly Ile Phe Lys Ser Glu Ile Thr Glu Asn Leu
    205                     210                     215
tta caa aaa gat tgg ctg aat gat gtg gtc agg aaa ata atg cct ttg         783
Leu Gln Lys Asp Trp Leu Asn Asp Val Val Arg Lys Ile Met Pro Leu
220                     225                     230                     235
aga aga tta ggt act tca gat cca gca tta aca tct cta gct cgt tat         831
Arg Arg Leu Gly Thr Ser Asp Pro Ala Leu Thr Ser Leu Ala Arg Tyr
                240                     245                     250
ctt att cat gat tct tct gag tac gtc acg ggc aac aat ttt att gtc         879
Leu Ile His Asp Ser Ser Glu Tyr Val Thr Gly Asn Asn Phe Ile Val
            255                     260                     265
gat tat gga ggc acc tta cca ggt gta cca att tat tct tct ctg taa         927
Asp Tyr Gly Gly Thr Leu Pro Gly Val Pro Ile Tyr Ser Ser Leu
        270                     275                     280
attttggtac attatgcact aacgaaaagt aatactatga attattccat tacatatctt       987

caaattagac aatatggagt tctgaagtct atgtatatgg agaattattg tgaagtggct      1047

accaccttga tcttaccata taatgctcca gatgtccatg tataaatcat gtggtcgcga      1107

ttggagtttt gatatgttca taaaaaaaaa aaaaaa                                1143

<210> 11
<211> 282
<212> PRT
<213> Glycine max

<400> 11
Met Asn Ala Ile Ala Ala Glu Gln Val Leu Glu Pro Trp His Lys Leu
1               5                   10                  15
Asp Asp Lys Val Val Leu Val Thr Gly Ala Ser Ser Gly Leu Gly Arg
            20                  25                  30
Asp Phe Cys Ile Asp Leu Ala Lys Ala Gly Cys Cys Val Val Ala Ala
        35                  40                  45
Ala Arg Arg Leu Asp Arg Leu Thr Ser Leu Cys His Glu Ile Asn His
    50                  55                  60
Arg Trp Pro Ser Asn Val Gly Ile His Arg Ala Val Ala Val Glu Leu
65                  70                  75                  80
Asp Val Ala Ala Asp Gly Pro Ala Ile Asp Arg Ala Val Gln Lys Ala
                85                  90                  95
Trp Asp Ala Phe Gly Arg Val Asp Ser Leu Ile Asn Asn Ala Gly Val
            100                 105                 110
Arg Gly Ser Val Lys Ser Pro Leu Lys Leu Ser Glu Glu Glu Trp Asp
        115                 120                 125
His Val Phe Lys Thr Asn Leu Thr Gly Cys Trp Leu Val Ser Lys Tyr
    130                 135                 140
Val Cys Lys Arg Met Cys Asp Ile Gln Leu Lys Gly Ser Ile Ile Asn
145                 150                 155                 160
Ile Ser Ser Val Ser Gly Leu Asn Arg Gly Gln Leu Pro Gly Ala Ala
                165                 170                 175
Ala Tyr Ala Ser Ser Lys Ala Gly Val Asn Met Leu Thr Lys Val Met
            180                 185                 190
Ala Met Glu Leu Gly Met His Lys Ile Arg Val Asn Ser Ile Ser Pro
        195                 200                 205
Gly Ile Phe Lys Ser Glu Ile Thr Glu Asn Leu Leu Gln Lys Asp Trp
    210                 215                 220

Leu Asn Asp Val Val Arg Lys Ile Met Pro Leu Arg Arg Leu Gly Thr
225                 230                 235                 240
Ser Asp Pro Ala Leu Thr Ser Leu Ala Arg Tyr Leu Ile His Asp Ser
                245                 250                 255
Ser Glu Tyr Val Thr Gly Asn Asn Phe Ile Val Asp Tyr Gly Gly Thr
            260                 265                 270
Leu Pro Gly Val Pro Ile Tyr Ser Ser Leu
        275                 280

<210> 12
<211> 849
<212> DNA
<213> Brassica napus

<220>
<221> CDS
<222> (1)..(849)

<400> 12
atg agc aat cat caa acc acg gtg ttg aaa caa cta gag cca tgg tgt      48
Met Ser Asn His Gln Thr Thr Val Leu Lys Gln Leu Glu Pro Trp Cys
1               5                   10                  15
gag cta aac ggc aaa gtg gtt ctt ctg acc gga gct tct tct ggt ata      96
Glu Leu Asn Gly Lys Val Val Leu Leu Thr Gly Ala Ser Ser Gly Ile
            20                  25                  30
gga agt gag gtc tgt ctc gat ctg ggc aaa gct ggc tgt aag att atc      144
Gly Ser Glu Val Cys Leu Asp Leu Gly Lys Ala Gly Cys Lys Ile Ile
        35                  40                  45
gca gca gct cgt cgc gtc cac cgt ctc gaa tct ctc tgc tcc gaa atc      192
Ala Ala Ala Arg Arg Val His Arg Leu Glu Ser Leu Cys Ser Glu Ile
    50                  55                  60
aac agc tta agc tca acc ggg atc caa tta gcc gca ccg ctc gag cta      240
Asn Ser Leu Ser Ser Thr Gly Ile Gln Leu Ala Ala Pro Leu Glu Leu
65                  70                  75                  80
gac gtt tca tca gac gca gcc acc att caa aaa gct gtc aaa caa gct      288
Asp Val Ser Ser Asp Ala Ala Thr Ile Gln Lys Ala Val Lys Gln Ala
                85                  90                  95
tgg gac atc tac gga aag ata gat gtg ttg atc aac aac gct gga atc      336
Trp Asp Ile Tyr Gly Lys Ile Asp Val Leu Ile Asn Asn Ala Gly Ile
            100                 105                 110
aga ggc aat gtc aag acg agt tta gat ctg act gaa gac gag tgg aac      384
Arg Gly Asn Val Lys Thr Ser Leu Asp Leu Thr Glu Asp Glu Trp Asn
        115                 120                 125
aca gtg ttc aga acc aac tta acc gga cct tgg tta gta tcc aaa tac      432
Thr Val Phe Arg Thr Asn Leu Thr Gly Pro Trp Leu Val Ser Lys Tyr
    130                 135                 140
gtc tgc agt cta atg cgt gac gca aaa cgc ggc ggc tca gta ata aac      480
Val Cys Ser Leu Met Arg Asp Ala Lys Arg Gly Gly Ser Val Ile Asn
145                 150                 155                 160
gtc tcc tcc atc gcc ggt ctc cac cga ggt ttg tta ccc ggt gga gtc      528
Val Ser Ser Ile Ala Gly Leu His Arg Gly Leu Leu Pro Gly Gly Val
                165                 170                 175
gcc tat gct tgt tcc aaa ggt ggt gtt gac acc atg acg agg atg atg      576
Ala Tyr Ala Cys Ser Lys Gly Gly Val Asp Thr Met Thr Arg Met Met
            180                 185                 190
gct att gag tta ggt gta tac aac atc aga gtg aac tcg atc gca ccg      624
Ala Ile Glu Leu Gly Val Tyr Asn Ile Arg Val Asn Ser Ile Ala Pro
        195                 200                 205
ggg ctt ctc aag tca gag atc acg caa ggt ctt atg caa aaa gag tgg      672
Gly Leu Leu Lys Ser Glu Ile Thr Gln Gly Leu Met Gln Lys Glu Trp
    210                 215                 220
ctc aag aac gtg acc gag agg act atc ccg tta aag gtg caa cag acc      720
Leu Lys Asn Val Thr Glu Arg Thr Ile Pro Leu Lys Val Gln Gln Thr
225                 230                 235                 240
gtg gat ccg ggg ctt acc tcg ctc gtt cgc tat ctc agt cat gac tct      768
Val Asp Pro Gly Leu Thr Ser Leu Val Arg Tyr Leu Ser His Asp Ser
                245                 250                 255
tct caa tat gtc tcc ggc aac aca tac ata ctt gac tcc gga gct aca      816
Ser Gln Tyr Val Ser Gly Asn Thr Tyr Ile Leu Asp Ser Gly Ala Thr

                260                 265                 270
ata cct ggc ctg cct att ttt tct tct ctt tga                         849
Ile Pro Gly Leu Pro Ile Phe Ser Ser Leu
        275                 280

<210> 13
<211> 282
<212> PRT
<213> Brassica napus

<400> 13
Met Ser Asn His Gln Thr Thr Val Leu Lys Gln Leu Glu Pro Trp Cys
1               5                   10                  15
Glu Leu Asn Gly Lys Val Val Leu Leu Thr Gly Ala Ser Ser Gly Ile
            20                  25                  30
Gly Ser Glu Val Cys Leu Asp Leu Gly Lys Ala Gly Cys Lys Ile Ile
        35                  40                  45
Ala Ala Ala Arg Arg Val His Arg Leu Glu Ser Leu Cys Ser Glu Ile
    50                  55                  60
Asn Ser Leu Ser Ser Thr Gly Ile Gln Leu Ala Ala Pro Leu Glu Leu
65                  70                  75                  80
Asp Val Ser Ser Asp Ala Ala Thr Ile Gln Lys Ala Val Lys Gln Ala
                85                  90                  95
Trp Asp Ile Tyr Gly Lys Ile Asp Val Leu Ile Asn Asn Ala Gly Ile
            100                 105                 110
Arg Gly Asn Val Lys Thr Ser Leu Asp Leu Thr Glu Asp Glu Trp Asn
        115                 120                 125
Thr Val Phe Arg Thr Asn Leu Thr Gly Pro Trp Leu Val Ser Lys Tyr
    130                 135                 140
Val Cys Ser Leu Met Arg Asp Ala Lys Arg Gly Gly Ser Val Ile Asn
145                 150                 155                 160
Val Ser Ser Ile Ala Gly Leu His Arg Gly Leu Leu Pro Gly Gly Val
                165                 170                 175
Ala Tyr Ala Cys Ser Lys Gly Gly Val Asp Thr Met Thr Arg Met Met
            180                 185                 190
Ala Ile Glu Leu Gly Val Tyr Asn Ile Arg Val Asn Ser Ile Ala Pro
        195                 200                 205
Gly Leu Leu Lys Ser Glu Ile Thr Gln Gly Leu Met Gln Lys Glu Trp
    210                 215                 220
Leu Lys Asn Val Thr Glu Arg Thr Ile Pro Leu Lys Val Gln Gln Thr
225                 230                 235                 240
Val Asp Pro Gly Leu Thr Ser Leu Val Arg Tyr Leu Ser His Asp Ser
                245                 250                 255
Ser Gln Tyr Val Ser Gly Asn Thr Tyr Ile Leu Asp Ser Gly Ala Thr
            260                 265                 270
Ile Pro Gly Leu Pro Ile Phe Ser Ser Leu
        275                 280

<210> 14
<211> 858
<212> DNA
<213> Brassica napus

<220>
<221> CDS
<222> (1)..(858)

<400> 14
atg agc agc aat cat cag act gtg atg aag caa cta gag cca tgg tgc      48
Met Ser Ser Asn His Gln Thr Val Met Lys Gln Leu Glu Pro Trp Cys
1               5                   10                  15
gag ctc aaa gac aaa gtg gtt ctc cta aca gga gct tca tcc ggc atc      96
Glu Leu Lys Asp Lys Val Val Leu Leu Thr Gly Ala Ser Ser Gly Ile
            20                  25                  30
gga aga gag atc tgc ctc gat cta gcc aaa tcc ggc tgc aag atc atc      144
Gly Arg Glu Ile Cys Leu Asp Leu Ala Lys Ser Gly Cys Lys Ile Ile
        35                  40                  45
gca gca gct cgt cgt ctc gac cgt ctc caa tcc ctc tgc tcc gag atc      192
Ala Ala Ala Arg Arg Leu Asp Arg Leu Gln Ser Leu Cys Ser Glu Ile

50                  55                  60
aac gcc tta ttc tcc cca aca aaa acc aaa caa gcc gca cct ctc gag          240
Asn Ala Leu Phe Ser Pro Thr Lys Thr Lys Gln Ala Ala Pro Leu Glu
65                  70                  75                  80
cta gac gtc tcc tca gac tca tcc acc atc cga aac gca gtc aaa caa          288
Leu Asp Val Ser Ser Asp Ser Ser Thr Ile Arg Asn Ala Val Lys Gln
                85                  90                  95
gct tgg gac atc ttc gga aac atc gac gtc ttg atc aac aac gca ggc          336
Ala Trp Asp Ile Phe Gly Asn Ile Asp Val Leu Ile Asn Asn Ala Gly
            100                 105                 110
atc aga ggc aac gtc aag tcg agt ctg gac cta tcc gaa gaa gaa tgg          384
Ile Arg Gly Asn Val Lys Ser Ser Leu Asp Leu Ser Glu Glu Glu Trp
        115                 120                 125
gaa aga gtc ttc aga aca aac cta acc gga cct tgg cta gta tca aaa          432
Glu Arg Val Phe Arg Thr Asn Leu Thr Gly Pro Trp Leu Val Ser Lys
    130                 135                 140
cac gtc tgc gtt ctg atg cgc gac gcc aaa cgc cgc ggc gga gga tcg          480
His Val Cys Val Leu Met Arg Asp Ala Lys Arg Arg Gly Gly Gly Ser
145                 150                 155                 160
gtg ata aac gtt tcc tcc atc gcg ggg ctt cag cgc ggg aag cta ccc          528
Val Ile Asn Val Ser Ser Ile Ala Gly Leu Gln Arg Gly Lys Leu Pro
                165                 170                 175
ggc gcg ttg gcg tac gcg tgt tcg aaa gga ggt ctt gat att atg acg          576
Gly Ala Leu Ala Tyr Ala Cys Ser Lys Gly Gly Leu Asp Ile Met Thr
            180                 185                 190
aag atg atg gcg gtt gag ctg ggt gag tat ggt ata aga gtg aac tcg          624
Lys Met Met Ala Val Glu Leu Gly Glu Tyr Gly Ile Arg Val Asn Ser
        195                 200                 205
ata gct ccg ggg ctg ttt aag tcg gag atc acg gaa ggt ctg gtg agg          672
Ile Ala Pro Gly Leu Phe Lys Ser Glu Ile Thr Glu Gly Leu Val Arg
    210                 215                 220
aaa gag tgg atg aag aat gtg agg aag agg att gtt ccg ttg aag gtg          720
Lys Glu Trp Met Lys Asn Val Arg Lys Arg Ile Val Pro Leu Lys Val
225                 230                 235                 240
cag cag act gtg gac ccg ggg ctt acc tcg ctg gtt agg tat ctg att          768
Gln Gln Thr Val Asp Pro Gly Leu Thr Ser Leu Val Arg Tyr Leu Ile
                245                 250                 255
ccc cac tct tcc agg tat gtc tct cgc aat gtt tac att gtt gac gcg          816
Pro His Ser Ser Arg Tyr Val Ser Arg Asn Val Tyr Ile Val Asp Ala
            260                 265                 270
ggt gct aca ttg tct ggt cta acg att ttt tct tca ctt tga                  858
Gly Ala Thr Leu Ser Gly Leu Thr Ile Phe Ser Ser Leu
        275                 280                 285

<210> 15
<211> 285
<212> PRT
<213> Brassica napus

<400> 15
Met Ser Ser Asn His Gln Thr Val Met Lys Gln Leu Glu Pro Trp Cys
1               5                   10                  15
Glu Leu Lys Asp Lys Val Val Leu Leu Thr Gly Ala Ser Ser Gly Ile
            20                  25                  30
Gly Arg Glu Ile Cys Leu Asp Leu Ala Lys Ser Gly Cys Lys Ile Ile
        35                  40                  45
Ala Ala Ala Arg Arg Leu Asp Arg Leu Gln Ser Leu Cys Ser Glu Ile
    50                  55                  60
Asn Ala Leu Phe Ser Pro Thr Lys Thr Lys Gln Ala Ala Pro Leu Glu
65                  70                  75                  80
Leu Asp Val Ser Ser Asp Ser Ser Thr Ile Arg Asn Ala Val Lys Gln
                85                  90                  95
Ala Trp Asp Ile Phe Gly Asn Ile Asp Val Leu Ile Asn Asn Ala Gly
            100                 105                 110
Ile Arg Gly Asn Val Lys Ser Ser Leu Asp Leu Ser Glu Glu Glu Trp
        115                 120                 125
Glu Arg Val Phe Arg Thr Asn Leu Thr Gly Pro Trp Leu Val Ser Lys
    130                 135                 140
His Val Cys Val Leu Met Arg Asp Ala Lys Arg Arg Gly Gly Gly Ser

145                 150                 155                 160
Val Ile Asn Val Ser Ser Ile Ala Gly Leu Gln Arg Gly Lys Leu Pro
                165                 170                 175
Gly Ala Leu Ala Tyr Ala Cys Ser Lys Gly Gly Leu Asp Ile Met Thr
            180                 185                 190
Lys Met Met Ala Val Glu Leu Gly Glu Tyr Gly Ile Arg Val Asn Ser
        195                 200                 205
Ile Ala Pro Gly Leu Phe Lys Ser Glu Ile Thr Glu Gly Leu Val Arg
    210                 215                 220
Lys Glu Trp Met Lys Asn Val Arg Lys Arg Ile Val Pro Leu Lys Val
225                 230                 235                 240
Gln Gln Thr Val Asp Pro Gly Leu Thr Ser Leu Val Arg Tyr Leu Ile
                245                 250                 255
Pro His Ser Ser Arg Tyr Val Ser Arg Asn Val Tyr Ile Val Asp Ala
            260                 265                 270
Gly Ala Thr Leu Ser Gly Leu Thr Ile Phe Ser Ser Leu
        275                 280                 285

<210> 16
<211> 843
<212> DNA
<213> Zea mays

<220>
<221> CDS
<222> (1)..(843)

<400> 16
atg tcg tcg gcg ggg cca ccg ccg ccg ctg ccg ccg tgg agc agg ctg      48
Met Ser Ser Ala Gly Pro Pro Pro Pro Leu Pro Pro Trp Ser Arg Leu
1               5                   10                  15
gag ggg cag gtg gtg ctg gtg acg ggc gct tcc tcc ggc atc gga cgc      96
Glu Gly Gln Val Val Leu Val Thr Gly Ala Ser Ser Gly Ile Gly Arg
            20                  25                  30
gac ttc tgc ctc gac ctg gcg cgc gcc ggc tgc cgc gtc gtc gcc gcc      144
Asp Phe Cys Leu Asp Leu Ala Arg Ala Gly Cys Arg Val Val Ala Ala
        35                  40                  45
gcc cgc cgc gcc gat cgt ctc cgc tcg ctc tgc gac gag atc aac ccc      192
Ala Arg Arg Ala Asp Arg Leu Arg Ser Leu Cys Asp Glu Ile Asn Pro
    50                  55                  60
tcc gct gcc tcc cga gcc ccc cgc gcg gtg gct gtg gag gtc gac gtc      240
Ser Ala Ala Ser Arg Ala Pro Arg Ala Val Ala Val Glu Val Asp Val
65                  70                  75                  80
gcc gcc ggt ggt tcg gcc ctg gag gcg gcg gtg cag aag gcc tgg gac      288
Ala Ala Gly Gly Ser Ala Leu Glu Ala Ala Val Gln Lys Ala Trp Asp
                85                  90                  95
gcc ttt ggc cgt atc gac gcc ttg gtc aac aac gcc ggc ata cga ggt      336
Ala Phe Gly Arg Ile Asp Ala Leu Val Asn Asn Ala Gly Ile Arg Gly
            100                 105                 110
gca gtg cat tct cca tta gat tgg ccc gag gat gag tgg gac aga atc      384
Ala Val His Ser Pro Leu Asp Trp Pro Glu Asp Glu Trp Asp Arg Ile
        115                 120                 125
atc aag acg aac ctt acc gga tca tgg ctc gtg gcc aaa cat gtc tgt      432
Ile Lys Thr Asn Leu Thr Gly Ser Trp Leu Val Ala Lys His Val Cys
    130                 135                 140
cga cgc atg cgt gat gcc aag ctg aag ggt tca gtg gtt aac atc acc      480
Arg Arg Met Arg Asp Ala Lys Leu Lys Gly Ser Val Val Asn Ile Thr
145                 150                 155                 160
tct att gct ggc ctt aac cgt ggg cat ctg cct ggc tcc acg gga tac      528
Ser Ile Ala Gly Leu Asn Arg Gly His Leu Pro Gly Ser Thr Gly Tyr
                165                 170                 175
gca tcc tca aag gct gct gtg cat tat gcc acc aag att atg gct ttg      576
Ala Ser Ser Lys Ala Ala Val His Tyr Ala Thr Lys Ile Met Ala Leu
            180                 185                 190
gaa ttg ggc gcg gat cgc atc aga gtg aac tcg att gca cct gga ctc      624
Glu Leu Gly Ala Asp Arg Ile Arg Val Asn Ser Ile Ala Pro Gly Leu
        195                 200                 205
ttc aaa tca gag ata act gct cct ctg ttt caa aag agg tgg ttg agc      672
Phe Lys Ser Glu Ile Thr Ala Pro Leu Phe Gln Lys Arg Trp Leu Ser

210                 215                 220
acc gtt gct tca aag ata gtg ccg ctt aag gag cat ggc gct act gat        720
Thr Val Ala Ser Lys Ile Val Pro Leu Lys Glu His Gly Ala Thr Asp
225                 230                 235                 240
cct gca ttg acg tcg ctg gtc cgt ttt ctg atc cat gaa gca tcg tcg        768
Pro Ala Leu Thr Ser Leu Val Arg Phe Leu Ile His Glu Ala Ser Ser
                245                 250                 255
tat gtg act ggc aac atc ttc att gta gac tca ggt gcc acc ata cct        816
Tyr Val Thr Gly Asn Ile Phe Ile Val Asp Ser Gly Ala Thr Ile Pro
            260                 265                 270
ggt gtt ccg ata ttc tca tcc ctg taa                                    843
Gly Val Pro Ile Phe Ser Ser Leu
        275                 280

<210> 17
<211> 280
<212> PRT
<213> Zea mays

<400> 17
Met Ser Ser Ala Gly Pro Pro Pro Pro Leu Pro Pro Trp Ser Arg Leu
1               5                   10                  15
Glu Gly Gln Val Val Leu Val Thr Gly Ala Ser Ser Gly Ile Gly Arg
            20                  25                  30
Asp Phe Cys Leu Asp Leu Ala Arg Ala Gly Cys Arg Val Val Ala Ala
        35                  40                  45
Ala Arg Arg Ala Asp Arg Leu Arg Ser Leu Cys Asp Glu Ile Asn Pro
    50                  55                  60
Ser Ala Ala Ser Arg Ala Pro Arg Ala Val Ala Val Glu Val Asp Val
65                  70                  75                  80
Ala Ala Gly Gly Ser Ala Leu Glu Ala Ala Val Gln Lys Ala Trp Asp
                85                  90                  95
Ala Phe Gly Arg Ile Asp Ala Leu Val Asn Asn Ala Gly Ile Arg Gly
            100                 105                 110
Ala Val His Ser Pro Leu Asp Trp Pro Glu Asp Glu Trp Asp Arg Ile
        115                 120                 125
Ile Lys Thr Asn Leu Thr Gly Ser Trp Leu Val Ala Lys His Val Cys
    130                 135                 140
Arg Arg Met Arg Asp Ala Lys Leu Lys Gly Ser Val Val Asn Ile Thr
145                 150                 155                 160
Ser Ile Ala Gly Leu Asn Arg Gly His Leu Pro Gly Ser Thr Gly Tyr
                165                 170                 175
Ala Ser Ser Lys Ala Ala Val His Tyr Ala Thr Lys Ile Met Ala Leu
            180                 185                 190
Glu Leu Gly Ala Asp Arg Ile Arg Val Asn Ser Ile Ala Pro Gly Leu
        195                 200                 205
Phe Lys Ser Glu Ile Thr Ala Pro Leu Phe Gln Lys Arg Trp Leu Ser
    210                 215                 220
Thr Val Ala Ser Lys Ile Val Pro Leu Lys Glu His Gly Ala Thr Asp
225                 230                 235                 240
Pro Ala Leu Thr Ser Leu Val Arg Phe Leu Ile His Glu Ala Ser Ser
                245                 250                 255
Tyr Val Thr Gly Asn Ile Phe Ile Val Asp Ser Gly Ala Thr Ile Pro
            260                 265                 270
Gly Val Pro Ile Phe Ser Ser Leu
        275                 280

<210> 18
<211> 25
<212> DNA
<213> Artificial sequence

<400> 18
atggctagtg tttcttcggt tttag                                            25

<210> 19
<211> 21
<212> DNA
<213> Artificial sequence

<400> 19
ttactgagac tgcccaaaca c                                                21


<210> 20
<211> 266
<212> PRT
<213> Artificial sequence

<220>
<221> Variant
<222> (4)..(8)
<223> xaa in position 4 to 8 is any amino acid
<220>
<221> Variant
<222> (12)..(17)
<223> xaa in position 12 to 17 is any amino acid
<220>
<221> Variant
<222> (19)..(21)
<223> xaa in position 19 to 21 is any amino acid
<220>
<221> Variant
<222> (23)..(87)
<223> xaa in position 23 to 87 is any amino acid
<220>
<221> Variant
<222> (89)..(94)
<223> xaa in position 89 to 94 is any amino acid
<220>
<221> Variant
<222> (103)..(103)
<223> xaa in position 103 is any amino acid
<220>
<221> Variant
<222> (105)..(109)
<223> xaa in position 105 to 109 is any amino acid
<220>
<221> Variant
<222> (111)..(114)
<223> xaa in position 111 to 114 is any amino acid
<220>
<221> Variant
<222> (116)..(117)
<223> xaa in position 116 to 117 is any amino acid
<220>
<221> Variant
<222> (119)..(121)
<223> xaa in position 119 to 121 is any amino acid
<220>
<221> Variant
<222> (124)..(126)
<223> xaa in position 124 to 126 is any amino acid
<220>
<221> Variant
<222> (128)..(136)
<223> xaa in position 128 to 136 is any amino acid
<220>
<221> Variant
<222> (138)..(144)
<223> xaa in position 138 to 144 is any amino acid
<220>
<221> Variant
<222> (146)..(146)
<223> xaa in position 146 is any amino acid
<220>
<221> Variant
<222> (148)..(148)

<223> xaa in position 148 is any amino acid
<220>
<221> Variant
<222> (150)..(151)
<223> xaa in position 150 to 151 is any amino acid
<220>
<221> Variant
<222> (153)..(154)
<223> xaa in position 153 to 154 is any amino acid
<220>
<221> Variant
<222> (156)..(159)
<223> xaa in position 156 to 159 is any amino acid
<220>
<221> Variant
<222> (161)..(162)
<223> xaa in position 161 to 162 is any amino acid
<220>
<221> Variant
<222> (165)..(165)
<223> xaa in position 165 is any amino acid
<220>
<221> Variant
<222> (168)..(168)
<223> xaa in position 168 is any amino acid
<220>
<221> Variant
<222> (170)..(170)
<223> xaa in position 170 is any amino acid
<220>
<221> Variant
<222> (174)..(175)
<223> xaa in position 174 to 175 is any amino acid
<220>
<221> Variant
<222> (177)..(181)
<223> xaa in position 177 to 181 is any amino acid
<220>
<221> Variant
<222> (183)..(183)
<223> xaa in position 183 is any amino acid
<220>
<221> Variant
<222> (185)..(193)
<223> xaa in position 185 to 193 is any amino acid
<220>
<221> Variant
<222> (196)..(198)
<223> xaa in position 196 to 198 is any amino acid
<220>
<221> Variant
<222> (201)..(201)
<223> xaa in position 201 is any amino acid
<220>
<221> Variant
<222> (203)..(203)
<223> xaa in position 203 is any amino acid
<220>
<221> Variant
<222> (205)..(205)
<223> xaa in position 205 is any amino acid
<220>
<221> Variant
<222> (207)..(229)
<223> xaa in position 207 to 229 is any amino acid
<220>
<221> Variant
<222> (231)..(232)
<223> xaa in position 231 to 232 is any amino acid

<220>
<221> Variant
<222> (234)..(234)
<223> xaa in position 234 is any amino acid
<220>
<221> Variant
<222> (236)..(240)
<223> xaa in position 236 to 240 is any amino acid
<220>
<221> Variant
<222> (242)..(246)
<223> xaa in position 242 to 246 is any amino acid
<220>
<221> Variant
<222> (248)..(254)
<223> xaa in position 248 to 254 is any amino acid
<220>
<221> Variant
<222> (256)..(256)
<223> xaa in position 256 is any amino acid
<220>
<221> Variant
<222> (259)..(264)
<223> xaa in position 259 to 264 is any amino acid

<400> 20
Val Thr Gly xaa xaa xaa xaa xaa Gly Ile Gly xaa xaa xaa xaa xaa
1               5                   10                  15
xaa Leu xaa xaa xaa Gly xaa xaa xaa xaa xaa xaa xaa xaa xaa xaa
            20                  25                  30
xaa xaa xaa xaa xaa xaa xaa xaa xaa xaa xaa xaa xaa xaa xaa xaa
        35                  40                  45
xaa xaa xaa xaa xaa xaa xaa xaa xaa xaa xaa xaa xaa xaa xaa xaa
    50                  55                  60
xaa xaa xaa xaa xaa xaa xaa xaa xaa xaa xaa xaa xaa xaa xaa xaa
65                  70                  75                  80
xaa xaa xaa xaa xaa xaa xaa Gly xaa xaa xaa xaa xaa xaa Leu Val
                85                  90                  95
Asn Asn Ala Gly Ile Thr xaa Asp xaa xaa xaa xaa xaa Met xaa xaa
            100                 105                 110
xaa xaa Trp xaa xaa Val xaa xaa xaa Asn Leu xaa xaa xaa Phe xaa
        115                 120                 125
xaa xaa xaa xaa xaa xaa xaa xaa Met xaa xaa xaa xaa xaa xaa xaa
    130                 135                 140
Gly xaa Ile xaa Asn xaa xaa Ser xaa xaa Gly xaa xaa xaa xaa Gly
145                 150                 155                 160
xaa xaa Gly Gln xaa Asn Tyr xaa Ala xaa Lys Ala Gly xaa xaa Gly
                165                 170                 175
xaa xaa xaa xaa Ala xaa Glu xaa xaa xaa xaa xaa xaa xaa
            180                 185                 190
xaa Val Asn xaa xaa xaa Pro Gly xaa Ile xaa Thr xaa Met xaa xaa
        195                 200                 205
xaa xaa xaa xaa xaa xaa xaa xaa xaa xaa xaa xaa xaa xaa xaa xaa
    210                 215                 220
xaa xaa xaa xaa xaa Pro xaa xaa Arg xaa Gly xaa xaa xaa xaa xaa
225                 230                 235                 240
Ala xaa xaa xaa xaa xaa Leu xaa xaa xaa xaa xaa xaa xaa Tyr xaa
                245                 250                 255
Thr Gly xaa xaa xaa xaa xaa xaa Gly Gly
            260                 265


<210> 21
<211> 11
<212> PRT
<213> Artificial sequence

<220>
<221> Variant

<222> (1)..(1)
<223> Xaa in position 1 is Val or Ile
<220>
<221> Variant
<222> (2)..(2)
<223> Xaa in position 2 is Thr or Ser
<220>
<221> Variant
<222> (4)..(4)
<223> Xaa in position 4 is Ala, Ser or Gly
<220>
<221> Variant
<222> (5)..(6)
<223> Xaa in position 5 to 6 is any amino acid
<220>
<221> Variant
<222> (7)..(8)
<223> Xaa in position 7 to 8 is any or no amino acid
<220>
<221> Variant
<222> (9)..(9)
<223> Xaa in position 9 is Gly, Ala or Ser
<220>
<221> Variant
<222> (10)..(10)
<223> Xaa in position 10 is Ile, Leu or Val

<400> 21
Xaa Xaa Gly Xaa Xaa Xaa Xaa Xaa Xaa Xaa Gly
1               5                   10


<210> 22
<211> 7
<212> PRT
<213> Artificial sequence

<220>
<221> Variant
<222> (1)..(1)
<223> Xaa in position 1 is Leu, Val or Ile
<220>
<221> Variant
<222> (2)..(2)
<223> Xaa in position 2 is Val, Ile or Leu
<220>
<221> Variant
<222> (3)..(4)
<223> Xaa in position 3 to 4 is any amino acid
<220>
<221> Variant
<222> (7)..(7)
<223> Xaa in position 7 is Ile, Val or Leu

<400> 22
Xaa Xaa Xaa Xaa Ala Gly Xaa
1               5


<210> 23
<211> 25
<212> PRT
<213> Artificial sequence

<220>
<221> Variant
<222> (2)..(3)
<223> Xaa in position 2 to 3 is any amino acid
<220>

<221> Variant
<222> (4)..(4)
<223> xaa in position 4 is Gly or Asn
<220>
<221> Variant
<222> (5)..(5)
<223> xaa in position 5 is Gln or Glu
<220>
<221> Variant
<222> (6)..(7)
<223> xaa in position 6 to 7 is any amino acid
<220>
<221> Variant
<222> (9)..(9)
<223> xaa in position 9 is any amino acid
<220>
<221> Variant
<222> (10)..(11)
<223> xaa in position 10 to 11 is Ala, Ser or Thr
<220>
<221> Variant
<222> (13)..(13)
<223> xaa in position 13 is any amino acid
<220>
<221> Variant
<222> (14)..(14)
<223> xaa in position 14 is Gly or Ala
<220>
<221> Variant
<222> (15)..(16)
<223> xaa in position 15 to 16 is any amino acid
<220>
<221> Variant
<222> (17)..(17)
<223> xaa in position 17 is Gly, Ala or Ser
<220>
<221> Variant
<222> (18)..(22)
<223> xaa in position 18 to 22 is any amino acid
<220>
<221> Variant
<222> (23)..(23)
<223> xaa in position 23 is Ala or Ser
<220>
<221> Variant
<222> (24)..(24)
<223> xaa in position 24 is any amino acid

<400> 23
Gly xaa xaa xaa xaa xaa xaa Tyr xaa xaa xaa Lys xaa xaa xaa xaa
1               5                   10                  15
xaa xaa xaa xaa xaa xaa xaa xaa Glu
            20                  25


<210> 24
<211> 13
<212> PRT
<213> Artificial sequence

<220>
<221> Variant
<222> (1)..(1)
<223> xaa in position 1 is Ile or Val
<220>
<221> Variant
<222> (2)..(2)
<223> xaa in position 2 is any amino acid
<220>

<221> Variant
<222> (3)..(3)
<223> xaa in position 3 is Val, Phe, Ala or Cys
<220>
<221> Variant
<222> (5)..(5)
<223> xaa in position 5 is any amino acid
<220>
<221> Variant
<222> (6)..(6)
<223> xaa in position 6 is Val or Ile
<220>
<221> Variant
<222> (7)..(7)
<223> xaa in position 7 is any amino acid
<220>
<221> Variant
<222> (10)..(12)
<223> xaa in position 10 to 12 is any amino acid
<220>
<221> Variant
<222> (13)..(13)
<223> xaa in position 13 is Thr or Ser

<400> 24
xaa xaa xaa Asn xaa xaa xaa Pro Gly xaa xaa xaa xaa
1               5                   10

<210> 25
<211> 1806
<212> DNA
<213> Linum usitatissimum

<220>
<221> CDS
<222> (156)..(1439)

<400> 25
ttaaccccga gttcctttat tgcattttga tcgacaaacc ctcttcccct ccaccgccgc      60

gcctccaaca cagtcgccgt actcattcat ccgcagatct caggtaacca cctctctcct     120

ccgccggcgt cggcccgact tgttcttcac tgacc atg gga gaa aca agg gac        173
                                       Met Gly Glu Thr Arg Asp
                                       1               5
aac gat tac gag gag gag ctt ctc gat tac gag gaa gag gag gag aaa       221
Asn Asp Tyr Glu Glu Glu Leu Leu Asp Tyr Glu Glu Glu Glu Glu Lys
            10                  15                  20
gcc ccc gac tct atc act gcc aaa gtc aac ggc gaa gcc cct aag aag       269
Ala Pro Asp Ser Ile Thr Ala Lys Val Asn Gly Glu Ala Pro Lys Lys
        25                  30                  35
gga tat gtt ggg ata cac agt tcg gga ttc aga gac ttc ctt ttg aag       317
Gly Tyr Val Gly Ile His Ser Ser Gly Phe Arg Asp Phe Leu Leu Lys
    40                  45                  50
cca gag cta ctt cgg gct att gta gat tct ggg ttt gag cat cct tct       365
Pro Glu Leu Leu Arg Ala Ile Val Asp Ser Gly Phe Glu His Pro Ser
55                  60                  65                  70
gaa gtt caa cac gag tgt att ccc caa gcc atc ttg gga atg gat gtt       413
Glu Val Gln His Glu Cys Ile Pro Gln Ala Ile Leu Gly Met Asp Val
                75                  80                  85
atc tgc caa gct aag tct ggg atg gga aag act gct gtc ttt gtt ctg       461
Ile Cys Gln Ala Lys Ser Gly Met Gly Lys Thr Ala Val Phe Val Leu
            90                  95                  100
tct act ctc cag caa att gag cca act cct ggg caa gtt att gcc ctt       509
Ser Thr Leu Gln Gln Ile Glu Pro Thr Pro Gly Gln Val Ile Ala Leu
        105                 110                 115
gtt cta tgt cac aca aga gaa ctt gct tac cag atc tgc cac gag ttc       557

Val Leu Cys His Thr Arg Glu Leu Ala Tyr Gln Ile Cys His Glu Phe
    120                 125                 130
gag aga ttc agt acc tac ttg ccg gat acc aag gtt gct gta ttt tat      605
Glu Arg Phe Ser Thr Tyr Leu Pro Asp Thr Lys Val Ala Val Phe Tyr
135                 140                 145                 150
ggt ggt gtc aac atc aaa act cac agg gat ctt ctg aaa aat gaa tgc      653
Gly Gly Val Asn Ile Lys Thr His Arg Asp Leu Leu Lys Asn Glu Cys
                155                 160                 165
cct cac gtt gtt gtt gga aca cct ggt aga ata ctg gca ttg gcg cga      701
Pro His Val Val Val Gly Thr Pro Gly Arg Ile Leu Ala Leu Ala Arg
            170                 175                 180
gac aag gaa ctt tct ctg aaa aat gtt aga cat ttc att ctt gat gaa      749
Asp Lys Glu Leu Ser Leu Lys Asn Val Arg His Phe Ile Leu Asp Glu
        185                 190                 195
tgt gac aag atg ctt gaa tcc ctt gac atg cgg aga gat gtg cag gag      797
Cys Asp Lys Met Leu Glu Ser Leu Asp Met Arg Arg Asp Val Gln Glu
    200                 205                 210
ata ttc aag atg acc cct cat gac aag caa gtc atg atg ttt tct gca      845
Ile Phe Lys Met Thr Pro His Asp Lys Gln Val Met Met Phe Ser Ala
215                 220                 225                 230
aca cta agt aaa gaa atc cgc cca gta tgc aag aag ttc atg caa gat      893
Thr Leu Ser Lys Glu Ile Arg Pro Val Cys Lys Lys Phe Met Gln Asp
                235                 240                 245
ccg atg gaa att tat gtt gac gat gag gcc aag ctg aca ctt cat gga      941
Pro Met Glu Ile Tyr Val Asp Asp Glu Ala Lys Leu Thr Leu His Gly
            250                 255                 260
ctt gtc cag cac tac atc aaa ttg acc gag ctg gag aaa aac cgg aag      989
Leu Val Gln His Tyr Ile Lys Leu Thr Glu Leu Glu Lys Asn Arg Lys
        265                 270                 275
ctg aac gac ctt ctt gat gcg ctg gac ttc aat caa gtt gtt atc ttt      1037
Leu Asn Asp Leu Leu Asp Ala Leu Asp Phe Asn Gln Val Val Ile Phe
    280                 285                 290
gtc aag agt gtg agc cgt gca gct gag ctg aac aag ttg cta atc gag      1085
Val Lys Ser Val Ser Arg Ala Ala Glu Leu Asn Lys Leu Leu Ile Glu
295                 300                 305                 310
tgt aat ttc cct tca ata tgc att cac tct gga atg tcc cag gag gaa      1133
Cys Asn Phe Pro Ser Ile Cys Ile His Ser Gly Met Ser Gln Glu Glu
                315                 320                 325
agg ctg aca cgc tac aag ggt ttc aaa gag ggt cat aaa agg att ctt      1181
Arg Leu Thr Arg Tyr Lys Gly Phe Lys Glu Gly His Lys Arg Ile Leu
            330                 335                 340
gtt gct act gat ctg gtt gga agg gga ata gac ata gag cgg gtc aac      1229
Val Ala Thr Asp Leu Val Gly Arg Gly Ile Asp Ile Glu Arg Val Asn
        345                 350                 355
att gtt att aac tat gac atg cct gat tca gct gac aca tat ctt cac      1277
Ile Val Ile Asn Tyr Asp Met Pro Asp Ser Ala Asp Thr Tyr Leu His
    360                 365                 370
agg gtt ggt cgt gct ggt agg ttt ggc aca aag ggg ctt gcg att aca      1325
Arg Val Gly Arg Ala Gly Arg Phe Gly Thr Lys Gly Leu Ala Ile Thr
375                 380                 385                 390
ttt gta tca tca gct tcc gac tca gat gtt ctt aat cag gtt cag gag      1373
Phe Val Ser Ser Ala Ser Asp Ser Asp Val Leu Asn Gln Val Gln Glu
                395                 400                 405
agg ttt gag gtg gac ata aaa gaa ctg ccg gag caa att gat aca tct      1421
Arg Phe Glu Val Asp Ile Lys Glu Leu Pro Glu Gln Ile Asp Thr Ser
            410                 415                 420
aca tac atg ccc tcc taa ctgggaagta cgaaaaggaa gattacagag      1469
Thr Tyr Met Pro Ser
        425
caatgctgct gaatcggctc tgggttctgg agtatgggag gttgtgattt actgttactt      1529

gcaggccagt gtttggcttc ttttgttcat ggtgttagtc agagaagagt agaactgggc      1589

agaaaggtta aacgatgaag taatgttttt gctttgcctt atgcaagcag cagtgtaaac      1649

gatgaagtaa tgttttgttg gtcgtacaga ttagccagtc ccaagttttc agcctacgag      1709

gaggatcagt atgtgagttt aagtttagat gacgtgtcta ctgttcttct gcatccatgc     1769

ctattaaaat tttacatgtg acaaaaaaaa aaaaaaa                              1806

<210> 26
<211> 427
<212> PRT
<213> Linum usitatissimum

<400> 26
Met Gly Glu Thr Arg Asp Asn Asp Tyr Glu Glu Glu Leu Leu Asp Tyr
1               5                   10                  15
Glu Glu Glu Glu Glu Lys Ala Pro Asp Ser Ile Thr Ala Lys Val Asn
            20                  25                  30
Gly Glu Ala Pro Lys Lys Gly Tyr Val Gly Ile His Ser Ser Gly Phe
        35                  40                  45
Arg Asp Phe Leu Leu Lys Pro Glu Leu Leu Arg Ala Ile Val Asp Ser
    50                  55                  60
Gly Phe Glu His Pro Ser Glu Val Gln His Glu Cys Ile Pro Gln Ala
65                  70                  75                  80
Ile Leu Gly Met Asp Val Ile Cys Gln Ala Lys Ser Gly Met Gly Lys
                85                  90                  95
Thr Ala Val Phe Val Leu Ser Thr Leu Gln Gln Ile Glu Pro Thr Pro
            100                 105                 110
Gly Gln Val Ile Ala Leu Val Leu Cys His Thr Arg Glu Leu Ala Tyr
        115                 120                 125
Gln Ile Cys His Glu Phe Glu Arg Phe Ser Thr Tyr Leu Pro Asp Thr
    130                 135                 140
Lys Val Ala Val Phe Tyr Gly Gly Val Asn Ile Lys Thr His Arg Asp
145                 150                 155                 160
Leu Leu Lys Asn Glu Cys Pro His Val Val Val Gly Thr Pro Gly Arg
                165                 170                 175
Ile Leu Ala Leu Ala Arg Asp Lys Glu Leu Ser Leu Lys Asn Val Arg
            180                 185                 190
His Phe Ile Leu Asp Glu Cys Asp Lys Met Leu Glu Ser Leu Asp Met
        195                 200                 205
Arg Arg Asp Val Gln Glu Ile Phe Lys Met Thr Pro His Asp Lys Gln
    210                 215                 220
Val Met Met Phe Ser Ala Thr Leu Ser Lys Glu Ile Arg Pro Val Cys
225                 230                 235                 240
Lys Lys Phe Met Gln Asp Pro Met Glu Ile Tyr Val Asp Asp Glu Ala
                245                 250                 255
Lys Leu Thr Leu His Gly Leu Val Gln His Tyr Ile Lys Leu Thr Glu
            260                 265                 270
Leu Glu Lys Asn Arg Lys Leu Asn Asp Leu Leu Asp Ala Leu Asp Phe
        275                 280                 285
Asn Gln Val Val Ile Phe Val Lys Ser Val Ser Arg Ala Ala Glu Leu
    290                 295                 300
Asn Lys Leu Leu Ile Glu Cys Asn Phe Pro Ser Ile Cys Ile His Ser
305                 310                 315                 320
Gly Met Ser Gln Glu Glu Arg Leu Thr Arg Tyr Lys Gly Phe Lys Glu
                325                 330                 335
Gly His Lys Arg Ile Leu Val Ala Thr Asp Leu Val Gly Arg Gly Ile
            340                 345                 350
Asp Ile Glu Arg Val Asn Ile Val Ile Asn Tyr Asp Met Pro Asp Ser
        355                 360                 365
Ala Asp Thr Tyr Leu His Arg Val Gly Arg Ala Gly Arg Phe Gly Thr
    370                 375                 380
Lys Gly Leu Ala Ile Thr Phe Val Ser Ser Ala Ser Asp Ser Asp Val
385                 390                 395                 400
Leu Asn Gln Val Gln Glu Arg Phe Glu Val Asp Ile Lys Glu Leu Pro
                405                 410                 415
Glu Gln Ile Asp Thr Ser Thr Tyr Met Pro Ser
            420                 425

<210> 27
<211> 1687
<212> DNA
<213> Hordeum vulgare

<220>
<221> CDS
<222> (165)..(1409)

<400> 27
   gaattcccgg gtcgacagcg aaggctcctc ccatcccctc cccccaccgc ctccaccgac      60
   gcatcgatcg aatccgggac acgatctcgg attcgctcgg ccaatcgacg cgcaccagcc     120
   ccccggatcg tcgttcgtac tcccccaccg cgagcgcttc agcc atg gca gga atg      176
                                                    Met Ala Gly Met
                                                    1
   gca cca gag ggt tcc cag ttt gat gct aag cac tat gac agt aag atg      224
   Ala Pro Glu Gly Ser Gln Phe Asp Ala Lys His Tyr Asp Ser Lys Met
   5                   10                  15                  20
   act gag tta ctg aac caa ggt gac act gag gag ttt ttc acc tca tat      272
   Thr Glu Leu Leu Asn Gln Gly Asp Thr Glu Glu Phe Phe Thr Ser Tyr
                   25                  30                  35
   gat gag gtg cat gaa agt ttt gat gac atg ggc ctc caa gag aac ctt      320
   Asp Glu Val His Glu Ser Phe Asp Asp Met Gly Leu Gln Glu Asn Leu
               40                  45                  50
   ctt aga ggc atc tat gct tat ggt ttt gag aag cca tca gcc att cag      368
   Leu Arg Gly Ile Tyr Ala Tyr Gly Phe Glu Lys Pro Ser Ala Ile Gln
           55                  60                  65
   caa aga gga att gtt ccc ttc tgc aag ggt ctt gat gtg att caa caa      416
   Gln Arg Gly Ile Val Pro Phe Cys Lys Gly Leu Asp Val Ile Gln Gln
       70                  75                  80
   gct cag tct ggt aca gga aaa act gcc acc ttc tgt tct gga atc ttg      464
   Ala Gln Ser Gly Thr Gly Lys Thr Ala Thr Phe Cys Ser Gly Ile Leu
   85                  90                  95                  100
   cag cag ctt gac tat gga ttg gtt gag tgc cag gca ttg gtc ctt gct      512
   Gln Gln Leu Asp Tyr Gly Leu Val Glu Cys Gln Ala Leu Val Leu Ala
                   105                 110                 115
   ccg acc cgt gag ctt gca cag cag att gag aag gtc atg cgt gct ctt      560
   Pro Thr Arg Glu Leu Ala Gln Gln Ile Glu Lys Val Met Arg Ala Leu
               120                 125                 130
   ggt gac tac tta ggc gtc aag gtg cat gca tgt gtt ggt gga act tct      608
   Gly Asp Tyr Leu Gly Val Lys Val His Ala Cys Val Gly Gly Thr Ser
           135                 140                 145
   gtc cgt gag gat cag agg att ctt gcc agt ggt gtg cat gtt gtt gtc      656
   Val Arg Glu Asp Gln Arg Ile Leu Ala Ser Gly Val His Val Val Val
       150                 155                 160
   ggc aca cct ggt cgt gtg ttc gat atg ttg cgc agg caa tcc ctc cgc      704
   Gly Thr Pro Gly Arg Val Phe Asp Met Leu Arg Arg Gln Ser Leu Arg
   165                 170                 175                 180
   cca gat aac atc aag atg ttt gtg ttg gat gaa gct gat gag atg cta      752
   Pro Asp Asn Ile Lys Met Phe Val Leu Asp Glu Ala Asp Glu Met Leu
                   185                 190                 195
   tca cgt ggt ttc aag gat cag atc tat gat atc ttc cag ctt ctt cca      800
   Ser Arg Gly Phe Lys Asp Gln Ile Tyr Asp Ile Phe Gln Leu Leu Pro
               200                 205                 210
   tca aag att cag gtt ggg gtg ttc tct gct acc atg cct cct gag gct      848
   Ser Lys Ile Gln Val Gly Val Phe Ser Ala Thr Met Pro Pro Glu Ala
           215                 220                 225
   ctt gag ata acc cgc aag ttc atg aac aag ccc gtg agg att ctt gtc      896
   Leu Glu Ile Thr Arg Lys Phe Met Asn Lys Pro Val Arg Ile Leu Val
       230                 235                 240
   aag aga gat gag ctt acc ctt gag ggt atc aag caa ttc tat gtc aat      944
   Lys Arg Asp Glu Leu Thr Leu Glu Gly Ile Lys Gln Phe Tyr Val Asn
   245                 250                 255                 260
   gtt gag aag gaa gag tgg aag ctg gac aca ctc tgt gac ctg tac gag      992
   Val Glu Lys Glu Glu Trp Lys Leu Asp Thr Leu Cys Asp Leu Tyr Glu
                   265                 270                 275
   acc ttg gca att acc cag agt gtc atc ttt gtg aac acc cgc cgc aag      1040
   Thr Leu Ala Ile Thr Gln Ser Val Ile Phe Val Asn Thr Arg Arg Lys
               280                 285                 290
   gtg gac tgg ctc acc gac aag atg agg gga agg gac cac act gtc tct      1088
   Val Asp Trp Leu Thr Asp Lys Met Arg Gly Arg Asp His Thr Val Ser
           295                 300                 305
   gca aca cac gga gac atg gac cag aac act agg gac atc atc atg agg      1136
   Ala Thr His Gly Asp Met Asp Gln Asn Thr Arg Asp Ile Ile Met Arg
       310                 315                 320
   gag ttc aga tct ggc tct tct cgt gtg ctc atc acc acc gac ctg ctg      1184
   Glu Phe Arg Ser Gly Ser Ser Arg Val Leu Ile Thr Thr Asp Leu Leu
   325                 330                 335                 340
   gcc cgt ggt att gat gtg cag caa gtc tcc ctt gtc atc aac tac gac      1232
   Ala Arg Gly Ile Asp Val Gln Gln Val Ser Leu Val Ile Asn Tyr Asp
                   345                 350                 355
   ctg ccg acc cag cct gag aac tac ctc cat cgc att ggt cgt agt ggt      1280
   Leu Pro Thr Gln Pro Glu Asn Tyr Leu His Arg Ile Gly Arg Ser Gly
               360                 365                 370
   cgg ttc ggg agg aag gga gtt gcc atc aac ttc gtc acc cgt gaa gat      1328
   Arg Phe Gly Arg Lys Gly Val Ala Ile Asn Phe Val Thr Arg Glu Asp
           375                 380                 385
   gag agg atg ctg ttc gac atc cag aag ttc tac aac gtg gtc att gag      1376
   Glu Arg Met Leu Phe Asp Ile Gln Lys Phe Tyr Asn Val Val Ile Glu
       390                 395                 400
   gag ctc ccg gcc aat gtc gcc gac ctt ctc tag attattatca taggaatgca    1429
   Glu Leu Pro Ala Asn Val Ala Asp Leu Leu
   405                 410                 415
   gtaaggtgct cgataaagtt cgtttttgtt gaatatctcc caactctcag ttttggagtg    1489
   gtaaggtctg gttctgtttg aatcgcggtt aaggatcgag tttttttctt tttcttgtta    1549
   tgcaatgtcc ggaaattttt gtagtgcgta ggctgtgcca actcccgggc tagctgggat    1609
   agagtgacac tgatggagtt tttacttctc gttgtgaagc tattttattg cccctctgtt    1669
   tttaaaaaaa aaaaaaaa                                                   1687

<210> 28
<211> 414
<212> PRT
<213> Hordeum vulgare

<400> 28
Met Ala Gly Met Ala Pro Glu Gly Ser Gln Phe Asp Ala Lys His Tyr
1               5                   10                  15
Asp Ser Lys Met Thr Glu Leu Leu Asn Gln Gly Asp Thr Glu Glu Phe
            20                  25                  30
Phe Thr Ser Tyr Asp Glu Val His Glu Ser Phe Asp Asp Met Gly Leu
        35                  40                  45
Gln Glu Asn Leu Leu Arg Gly Ile Tyr Ala Tyr Gly Phe Glu Lys Pro
    50                  55                  60
Ser Ala Ile Gln Gln Arg Gly Ile Val Pro Phe Cys Lys Gly Leu Asp
65                  70                  75                  80
Val Ile Gln Gln Ala Gln Ser Gly Thr Gly Lys Thr Ala Thr Phe Cys
                85                  90                  95
Ser Gly Ile Leu Gln Gln Leu Asp Tyr Gly Leu Val Glu Cys Gln Ala
            100                 105                 110
Leu Val Leu Ala Pro Thr Arg Glu Leu Ala Gln Gln Ile Glu Lys Val
        115                 120                 125
Met Arg Ala Leu Gly Asp Tyr Leu Gly Val Lys Val His Ala Cys Val
    130                 135                 140

Gly Gly Thr Ser Val Arg Glu Asp Gln Arg Ile Leu Ala Ser Gly Val
145                 150                 155                 160
His Val Val Val Gly Thr Pro Gly Arg Val Phe Asp Met Leu Arg Arg
                165                 170                 175
Gln Ser Leu Arg Pro Asp Asn Ile Lys Met Phe Val Leu Asp Glu Ala
            180                 185                 190
Asp Glu Met Leu Ser Arg Gly Phe Lys Asp Gln Ile Tyr Asp Ile Phe
        195                 200                 205
Gln Leu Leu Pro Ser Lys Ile Gln Val Gly Val Phe Ser Ala Thr Met
    210                 215                 220
Pro Pro Glu Ala Leu Glu Ile Thr Arg Lys Phe Met Asn Lys Pro Val
225                 230                 235                 240
Arg Ile Leu Val Lys Arg Asp Glu Leu Thr Leu Glu Gly Ile Lys Gln
                245                 250                 255
Phe Tyr Val Asn Val Glu Lys Glu Glu Trp Lys Leu Asp Thr Leu Cys
            260                 265                 270
Asp Leu Tyr Glu Thr Leu Ala Ile Thr Gln Ser Val Ile Phe Val Asn
        275                 280                 285
Thr Arg Arg Lys Val Asp Trp Leu Thr Asp Lys Met Arg Gly Arg Asp
    290                 295                 300
His Thr Val Ser Ala Thr His Gly Asp Met Asp Gln Asn Thr Arg Asp
305                 310                 315                 320
Ile Ile Met Arg Glu Phe Arg Ser Gly Ser Ser Arg Val Leu Ile Thr
                325                 330                 335
Thr Asp Leu Leu Ala Arg Gly Ile Asp Val Gln Gln Val Ser Leu Val
            340                 345                 350
Ile Asn Tyr Asp Leu Pro Thr Gln Pro Glu Asn Tyr Leu His Arg Ile
        355                 360                 365
Gly Arg Ser Gly Arg Phe Gly Arg Lys Gly Val Ala Ile Asn Phe Val
    370                 375                 380
Thr Arg Glu Asp Glu Arg Met Leu Phe Asp Ile Gln Lys Phe Tyr Asn
385                 390                 395                 400
Val Val Ile Glu Glu Leu Pro Ala Asn Val Ala Asp Leu Leu
                405                 410

<210> 29
<211> 1239
<212> DNA
<213> Brassica napus

<220>
<221> CDS
<222> (1)..(1239)

<400> 29
atg gca gga tcc gca cca gaa ggc act cag ttt gat gct cgt cag ttc      48
Met Ala Gly Ser Ala Pro Glu Gly Thr Gln Phe Asp Ala Arg Gln Phe
1               5                   10                  15
gac cag aag ttg aac gaa gtt ctt gag gga cag gat gag ttc ttc acc      96
Asp Gln Lys Leu Asn Glu Val Leu Glu Gly Gln Asp Glu Phe Phe Thr
            20                  25                  30
tct tac gat gaa tcc cac gac agc ttt gac gcc atg ggt ctc caa gag      144
Ser Tyr Asp Glu Ser His Asp Ser Phe Asp Ala Met Gly Leu Gln Glu
        35                  40                  45
aat ctc ctc agg ggt atc tac gct tac ggt ttc gag aag cct tct gct      192
Asn Leu Leu Arg Gly Ile Tyr Ala Tyr Gly Phe Glu Lys Pro Ser Ala
    50                  55                  60
atc cag caa aga gga atc gtt ccc ttt tgc aaa ggc ctc gac gtg atc      240
Ile Gln Gln Arg Gly Ile Val Pro Phe Cys Lys Gly Leu Asp Val Ile
65                  70                  75                  80
cag cag gcc cag tcc ggt acc ggc aaa acc gcc act ttc tgc tcc ggc      288
Gln Gln Ala Gln Ser Gly Thr Gly Lys Thr Ala Thr Phe Cys Ser Gly
                85                  90                  95
gtc ctc cag cag cta gac tac tcc ctc gtc cag tgc caa gcc ctc gtc      336
Val Leu Gln Gln Leu Asp Tyr Ser Leu Val Gln Cys Gln Ala Leu Val
            100                 105                 110
ctc gcc ccg acc aga gag ctc gcc cag cag atc gag aag gtc atg aga      384
Leu Ala Pro Thr Arg Glu Leu Ala Gln Gln Ile Glu Lys Val Met Arg
        115                 120                 125

gcc ctc ggc gac tac ctc ggc gtc aag gtc cac gcc tgc gtc ggc ggg 432
Ala Leu Gly Asp Tyr Leu Gly Val Lys Val His Ala Cys Val Gly Gly
    130                 135                 140
acc agc gtc cgc gag gac cag cgc atc ctc cag gcc ggc gtc cac gtc 480
Thr Ser Val Arg Glu Asp Gln Arg Ile Leu Gln Ala Gly Val His Val
145                 150                 155                 160
gtc gtc ggc acc ccg ggg cgc gtg ttc gac atg ctg aag cgc cag tcc 528
Val Val Gly Thr Pro Gly Arg Val Phe Asp Met Leu Lys Arg Gln Ser
                165                 170                 175
ctc cgc gcc gac aac atc aag atg ttc gtc ctg gac gag gcc gac gag 576
Leu Arg Ala Asp Asn Ile Lys Met Phe Val Leu Asp Glu Ala Asp Glu
            180                 185                 190
atg ctc tcc cgc ggg ttc aag gac cag atc tac gac atc ttc cag ctc 624
Met Leu Ser Arg Gly Phe Lys Asp Gln Ile Tyr Asp Ile Phe Gln Leu
        195                 200                 205
ctc ccg ccg aag atc cag gtg gga gtc ttc tcg gcg acc atg ccg ccg 672
Leu Pro Pro Lys Ile Gln Val Gly Val Phe Ser Ala Thr Met Pro Pro
    210                 215                 220
gag gct ctc gag atc acg agg aag ttc atg agc aag ccg gtg agg atc 720
Glu Ala Leu Glu Ile Thr Arg Lys Phe Met Ser Lys Pro Val Arg Ile
225                 230                 235                 240
ctg gtg aag cgc gac gag ctg acc ttg gaa ggt atc aag cag ttt tac 768
Leu Val Lys Arg Asp Glu Leu Thr Leu Glu Gly Ile Lys Gln Phe Tyr
                245                 250                 255
gtc aac gtt gag aag gaa gac tgg aag ctc gag acg ctc tgc gac ctc 816
Val Asn Val Glu Lys Glu Asp Trp Lys Leu Glu Thr Leu Cys Asp Leu
            260                 265                 270
tac gag acg ctg gcc atc act cag agc gtc atc ttc gtg aac act agg 864
Tyr Glu Thr Leu Ala Ile Thr Gln Ser Val Ile Phe Val Asn Thr Arg
        275                 280                 285
cgg aag gtt gac tgg ctc act gat aaa atg agg ggg cgt gac cac acg 912
Arg Lys Val Asp Trp Leu Thr Asp Lys Met Arg Gly Arg Asp His Thr
    290                 295                 300
gtc tcg gcg act cac gga gac atg gac cag aac act aga gac att atc 960
Val Ser Ala Thr His Gly Asp Met Asp Gln Asn Thr Arg Asp Ile Ile
305                 310                 315                 320
atg aga gag ttc agg tct ggc tct tcg cgt gtg ctg atc acc act gat 1008
Met Arg Glu Phe Arg Ser Gly Ser Ser Arg Val Leu Ile Thr Thr Asp
                325                 330                 335
ctc ttg gct cgt ggt atc gat gtt cag cag gtg tct ttg gtg atc aac 1056
Leu Leu Ala Arg Gly Ile Asp Val Gln Gln Val Ser Leu Val Ile Asn
            340                 345                 350
tac gat ctg ccg act cag cct gag aac tac ctt cac cgt att gga aga 1104
Tyr Asp Leu Pro Thr Gln Pro Glu Asn Tyr Leu His Arg Ile Gly Arg
        355                 360                 365
agt ggg agg ttt ggg agg aaa ggt gtg gcg atc aac ttt gtg acc aag 1152
Ser Gly Arg Phe Gly Arg Lys Gly Val Ala Ile Asn Phe Val Thr Lys
    370                 375                 380
gat gat gag agg atg ctg ttt gat att cag aag ttt tac aac gtg gtt 1200
Asp Asp Glu Arg Met Leu Phe Asp Ile Gln Lys Phe Tyr Asn Val Val
385                 390                 395                 400
gtt gag gag ctg cct tcc aac gtt gct gat ctg ctg tga 1239
Val Glu Glu Leu Pro Ser Asn Val Ala Asp Leu Leu
                405                 410

<210> 30
<211> 412
<212> PRT
<213> Brassica napus

<400> 30
Met Ala Gly Ser Ala Pro Glu Gly Thr Gln Phe Asp Ala Arg Gln Phe
1               5                   10                  15
Asp Gln Lys Leu Asn Glu Val Leu Glu Gly Gln Asp Glu Phe Phe Thr
            20                  25                  30
Ser Tyr Asp Glu Ser His Asp Ser Phe Asp Ala Met Gly Leu Gln Glu
        35                  40                  45
Asn Leu Leu Arg Gly Ile Tyr Ala Tyr Gly Phe Glu Lys Pro Ser Ala
    50                  55                  60

Ile Gln Gln Arg Gly Ile Val Pro Phe Cys Lys Gly Leu Asp Val Ile
65                  70                  75                  80
Gln Gln Ala Gln Ser Gly Thr Gly Lys Thr Ala Thr Phe Cys Ser Gly
                85                  90                  95
Val Leu Gln Gln Leu Asp Tyr Ser Leu Val Gln Cys Gln Ala Leu Val
            100                 105                 110
Leu Ala Pro Thr Arg Glu Leu Ala Gln Gln Ile Glu Lys Val Met Arg
        115                 120                 125
Ala Leu Gly Asp Tyr Leu Gly Val Lys Val His Ala Cys Val Gly Gly
    130                 135                 140
Thr Ser Val Arg Glu Asp Gln Arg Ile Leu Gln Ala Gly Val His Val
145                 150                 155                 160
Val Val Gly Thr Pro Gly Arg Val Phe Asp Met Leu Lys Arg Gln Ser
                165                 170                 175
Leu Arg Ala Asp Asn Ile Lys Met Phe Val Leu Asp Glu Ala Asp Glu
            180                 185                 190
Met Leu Ser Arg Gly Phe Lys Asp Gln Ile Tyr Asp Ile Phe Gln Leu
        195                 200                 205
Leu Pro Pro Lys Ile Gln Val Gly Val Phe Ser Ala Thr Met Pro Pro
    210                 215                 220
Glu Ala Leu Glu Ile Thr Arg Lys Phe Met Ser Lys Pro Val Arg Ile
225                 230                 235                 240
Leu Val Lys Arg Asp Glu Leu Thr Leu Glu Gly Ile Lys Gln Phe Tyr
                245                 250                 255
Val Asn Val Glu Lys Glu Asp Trp Lys Leu Glu Thr Leu Cys Asp Leu
            260                 265                 270
Tyr Glu Thr Leu Ala Ile Thr Gln Ser Val Ile Phe Val Asn Thr Arg
        275                 280                 285
Arg Lys Val Asp Trp Leu Thr Asp Lys Met Arg Gly Arg Asp His Thr
    290                 295                 300
Val Ser Ala Thr His Gly Asp Met Asp Gln Asn Thr Arg Asp Ile Ile
305                 310                 315                 320
Met Arg Glu Phe Arg Ser Gly Ser Ser Arg Val Leu Ile Thr Thr Asp
                325                 330                 335
Leu Leu Ala Arg Gly Ile Asp Val Gln Gln Val Ser Leu Val Ile Asn
            340                 345                 350
Tyr Asp Leu Pro Thr Gln Pro Glu Asn Tyr Leu His Arg Ile Gly Arg
        355                 360                 365
Ser Gly Arg Phe Gly Arg Lys Gly Val Ala Ile Asn Phe Val Thr Lys
    370                 375                 380
Asp Asp Glu Arg Met Leu Phe Asp Ile Gln Lys Phe Tyr Asn Val Val
385                 390                 395                 400
Val Glu Glu Leu Pro Ser Asn Val Ala Asp Leu Leu
                405                 410

<210> 31
<211> 1227
<212> DNA
<213> Brassica napus

<220>
<221> CDS
<222> (1)..(1227)

<400> 31
atg gcg gaa gcg aat cct ggc cgt gga ggt gga aga aga agc ggc gga      48
Met Ala Glu Ala Asn Pro Gly Arg Gly Gly Gly Arg Arg Ser Gly Gly
1               5                   10                  15
cca atg gac gac gac aaa ttg gtc ttc gag acg acg gaa ggg atc gag      96
Pro Met Asp Asp Asp Lys Leu Val Phe Glu Thr Thr Glu Gly Ile Glu
            20                  25                  30
ccc atc acg aat ttc aac gac atg ggg atc aag gag gac gtt ctc cgc      144
Pro Ile Thr Asn Phe Asn Asp Met Gly Ile Lys Glu Asp Val Leu Arg
        35                  40                  45
ggc gtc tac gag tac ggt ttc gag aag ccc tcc gcg att cag cag aga      192
Gly Val Tyr Glu Tyr Gly Phe Glu Lys Pro Ser Ala Ile Gln Gln Arg
    50                  55                  60
gcg gtc atg ccg att ctc caa ggc cga gat gtg atc gct cag gct cag      240
Ala Val Met Pro Ile Leu Gln Gly Arg Asp Val Ile Ala Gln Ala Gln

65                  70                  75                  80
tcc ggt acc gga aag acc tca atg att gct ctc tcc gtc tgc caa atc      288
Ser Gly Thr Gly Lys Thr Ser Met Ile Ala Leu Ser Val Cys Gln Ile
                85                  90                  95
gtt gac act tcg tct aga gaa gtt cag gcg ttg ata ttg tcg cca acg      336
Val Asp Thr Ser Ser Arg Glu Val Gln Ala Leu Ile Leu Ser Pro Thr
            100                 105                 110
agg gag ctg gct tca cag acg gag aag act att caa gct att ggg ttg      384
Arg Glu Leu Ala Ser Gln Thr Glu Lys Thr Ile Gln Ala Ile Gly Leu
        115                 120                 125
cac gct aat att cag gcg cat gcg tgt att ggt ggg aag agc gtt gga      432
His Ala Asn Ile Gln Ala His Ala Cys Ile Gly Gly Lys Ser Val Gly
    130                 135                 140
gag gat atc agg aag ctg gag aat ggt gtc cat gtt gtg tct ggg acg      480
Glu Asp Ile Arg Lys Leu Glu Asn Gly Val His Val Val Ser Gly Thr
145                 150                 155                 160
cct ggg cgt gtt tgt gat atg att aag agg agg agt ctg cgt acc aga      528
Pro Gly Arg Val Cys Asp Met Ile Lys Arg Arg Ser Leu Arg Thr Arg
                165                 170                 175
gct att aag ctt ttg att ctt gat gaa tct gat gag atg ctg agc aga      576
Ala Ile Lys Leu Leu Ile Leu Asp Glu Ser Asp Glu Met Leu Ser Arg
            180                 185                 190
ggg ttc aag gac caa ata tat gat gtt tac aga tat ctt cca cct gat      624
Gly Phe Lys Asp Gln Ile Tyr Asp Val Tyr Arg Tyr Leu Pro Pro Asp
        195                 200                 205
ctt cag gtt tgc ttg gtt tct gca act ctt cct cac gag att ttg gag      672
Leu Gln Val Cys Leu Val Ser Ala Thr Leu Pro His Glu Ile Leu Glu
    210                 215                 220
atg aca tca aag ttt atg acg gaa cca gtg aag ata ctt gtg aag cgt      720
Met Thr Ser Lys Phe Met Thr Glu Pro Val Lys Ile Leu Val Lys Arg
225                 230                 235                 240
gat gag ttg act ctt gaa ggc att aaa caa ttt ttc gtt gct gtt gag      768
Asp Glu Leu Thr Leu Glu Gly Ile Lys Gln Phe Phe Val Ala Val Glu
                245                 250                 255
aaa gag gaa tgg aaa ttt gat aca ctc tgt gat ctt tat gac acg ctt      816
Lys Glu Glu Trp Lys Phe Asp Thr Leu Cys Asp Leu Tyr Asp Thr Leu
            260                 265                 270
act atc act caa gct gtt atc ttc tgc aac act aaa cga aag gtg gat      864
Thr Ile Thr Gln Ala Val Ile Phe Cys Asn Thr Lys Arg Lys Val Asp
        275                 280                 285
tgg cta agt gag aaa atg agg act aac aac ttc aca gtc tca tca atg      912
Trp Leu Ser Glu Lys Met Arg Thr Asn Asn Phe Thr Val Ser Ser Met
    290                 295                 300
cac ggt gac atg cct cag aag gaa aga gac gaa atc atg aat cag ttc      960
His Gly Asp Met Pro Gln Lys Glu Arg Asp Glu Ile Met Asn Gln Phe
305                 310                 315                 320
cgg tca ggc gac agt cgt gtt ttg atc acg aca gat gta tgg gca cgt      1008
Arg Ser Gly Asp Ser Arg Val Leu Ile Thr Thr Asp Val Trp Ala Arg
                325                 330                 335
ggg att gac gtg caa caa gtt tct ctc gtc atc aat tat gat ctc ccc      1056
Gly Ile Asp Val Gln Gln Val Ser Leu Val Ile Asn Tyr Asp Leu Pro
            340                 345                 350
aac aac cgt gag ctc tac atc cat cgt att gga cga tct ggt cgt ttc      1104
Asn Asn Arg Glu Leu Tyr Ile His Arg Ile Gly Arg Ser Gly Arg Phe
        355                 360                 365
ggc cgt aag ggt gtt gcg atc aac ttt gtt aaa agc gac gac atc aag      1152
Gly Arg Lys Gly Val Ala Ile Asn Phe Val Lys Ser Asp Asp Ile Lys
    370                 375                 380
atc ctc cgg gac att gag cag tac tac agt act cag att gac gag atg      1200
Ile Leu Arg Asp Ile Glu Gln Tyr Tyr Ser Thr Gln Ile Asp Glu Met
385                 390                 395                 400
cca atg aat gta gct gat ctt atc taa                                  1227
Pro Met Asn Val Ala Asp Leu Ile
                405

<210> 32
<211> 408
<212> PRT
<213> Brassica napus

<400> 32
   Met Ala Glu Ala Asn Pro Gly Arg Gly Gly Gly Arg Arg Ser Gly Gly
   1               5                   10                  15
   Pro Met Asp Asp Asp Lys Leu Val Phe Glu Thr Thr Glu Gly Ile Glu
               20                  25                  30
   Pro Ile Thr Asn Phe Asn Asp Met Gly Ile Lys Glu Asp Val Leu Arg
           35                  40                  45
   Gly Val Tyr Glu Tyr Gly Phe Glu Lys Pro Ser Ala Ile Gln Gln Arg
       50                  55                  60
   Ala Val Met Pro Ile Leu Gln Gly Arg Asp Val Ile Ala Gln Ala Gln
   65                  70                  75                  80
   Ser Gly Thr Gly Lys Thr Ser Met Ile Ala Leu Ser Val Cys Gln Ile
                   85                  90                  95
   Val Asp Thr Ser Ser Arg Glu Val Gln Ala Leu Ile Leu Ser Pro Thr
               100                 105                 110
   Arg Glu Leu Ala Ser Gln Thr Glu Lys Thr Ile Gln Ala Ile Gly Leu
           115                 120                 125
   His Ala Asn Ile Gln Ala His Ala Cys Ile Gly Gly Lys Ser Val Gly
       130                 135                 140
   Glu Asp Ile Arg Lys Leu Glu Asn Gly Val His Val Val Ser Gly Thr
   145                 150                 155                 160
   Pro Gly Arg Val Cys Asp Met Ile Lys Arg Arg Ser Leu Arg Thr Arg
                   165                 170                 175
   Ala Ile Lys Leu Leu Ile Leu Asp Glu Ser Asp Glu Met Leu Ser Arg
               180                 185                 190
   Gly Phe Lys Asp Gln Ile Tyr Asp Val Tyr Arg Tyr Leu Pro Pro Asp
           195                 200                 205
   Leu Gln Val Cys Leu Val Ser Ala Thr Leu Pro His Glu Ile Leu Glu
       210                 215                 220
   Met Thr Ser Lys Phe Met Thr Glu Pro Val Lys Ile Leu Val Lys Arg
   225                 230                 235                 240
   Asp Glu Leu Thr Leu Glu Gly Ile Lys Gln Phe Phe Val Ala Val Glu
                   245                 250                 255
   Lys Glu Glu Trp Lys Phe Asp Thr Leu Cys Asp Leu Tyr Asp Thr Leu
               260                 265                 270
   Thr Ile Thr Gln Ala Val Ile Phe Cys Asn Thr Lys Arg Lys Val Asp
           275                 280                 285
   Trp Leu Ser Glu Lys Met Arg Thr Asn Asn Phe Thr Val Ser Ser Met
       290                 295                 300
   His Gly Asp Met Pro Gln Lys Glu Arg Asp Glu Ile Met Asn Gln Phe
   305                 310                 315                 320
   Arg Ser Gly Asp Ser Arg Val Leu Ile Thr Thr Asp Val Trp Ala Arg
                   325                 330                 335
   Gly Ile Asp Val Gln Gln Val Ser Leu Val Ile Asn Tyr Asp Leu Pro
               340                 345                 350
   Asn Asn Arg Glu Leu Tyr Ile His Arg Ile Gly Arg Ser Gly Arg Phe
           355                 360                 365
   Gly Arg Lys Gly Val Ala Ile Asn Phe Val Lys Ser Asp Asp Ile Lys
       370                 375                 380
   Ile Leu Arg Asp Ile Glu Gln Tyr Tyr Ser Thr Gln Ile Asp Glu Met
   385                 390                 395                 400
   Pro Met Asn Val Ala Asp Leu Ile
                   405

<210> 33
<211> 1233
<212> DNA
<213> Brassica napus

<220>
<221> CDS
<222> (1)..(1233)

<400> 33
atg gca gga tcg gca aca cag ttt gat gat gcg cgt cag ttt gat cag       48
Met Ala Gly Ser Ala Thr Gln Phe Asp Asp Ala Arg Gln Phe Asp Gln
1               5                   10                  15
gca ctc agt gaa atc ctg gag gga cac gat gag ttc ttc acc tcc tat       96

Ala Leu Ser Glu Ile Leu Glu Gly His Asp Glu Phe Phe Thr Ser Tyr
            20                  25                  30
gat gag gtt tat gag agt ttt gat ggc atg ggt ttg caa gag aac ctt      144
Asp Glu Val Tyr Glu Ser Phe Asp Gly Met Gly Leu Gln Glu Asn Leu
        35                  40                  45
ctt agg ggt atc tat gct tat ggt ttt gaa aag cct tct gct att cag      192
Leu Arg Gly Ile Tyr Ala Tyr Gly Phe Glu Lys Pro Ser Ala Ile Gln
    50                  55                  60
caa aga gga atc gta ccc ttt tgc aag ggt ctt gat gtg atc cag cag      240
Gln Arg Gly Ile Val Pro Phe Cys Lys Gly Leu Asp Val Ile Gln Gln
65                  70                  75                  80
gca cag tct ggt act gga aaa acc gca act ttc tgt tct ggt gtc ttg      288
Ala Gln Ser Gly Thr Gly Lys Thr Ala Thr Phe Cys Ser Gly Val Leu
                85                  90                  95
cag cag ctt gac gtc acc ctt gtc cag tgc caa gct ctg gtc ttg gct      336
Gln Gln Leu Asp Val Thr Leu Val Gln Cys Gln Ala Leu Val Leu Ala
            100                 105                 110
ccc acc agg gag ctt gct cag cag att gag aag gtc atg cgt gct ctc      384
Pro Thr Arg Glu Leu Ala Gln Gln Ile Glu Lys Val Met Arg Ala Leu
        115                 120                 125
gga gac tac att ggc gtc aag gtt cat gcc tgt gtt ggt ggt acc agt      432
Gly Asp Tyr Ile Gly Val Lys Val His Ala Cys Val Gly Gly Thr Ser
    130                 135                 140
gtt ctt gac gat aag cgt atc ctc caa gct ggt gtc cat gtt gtt gtt      480
Val Leu Asp Asp Lys Arg Ile Leu Gln Ala Gly Val His Val Val Val
145                 150                 155                 160
gga act cca ggt cgt gtc ttt gac atg ctc caa aga cag tct ctt cgc      528
Gly Thr Pro Gly Arg Val Phe Asp Met Leu Gln Arg Gln Ser Leu Arg
                165                 170                 175
tct gac tgc atc aag atg ttt gtc ctt gat gaa gct gat gag atg ctc      576
Ser Asp Cys Ile Lys Met Phe Val Leu Asp Glu Ala Asp Glu Met Leu
            180                 185                 190
tcc cgt ggt ttc aag gat cag atc tat gac ata ttc cag ctt ctc cca      624
Ser Arg Gly Phe Lys Asp Gln Ile Tyr Asp Ile Phe Gln Leu Leu Pro
        195                 200                 205
cca aag att cag gtc gga gtg ttc tca gct aca atg cca cca gaa gct      672
Pro Lys Ile Gln Val Gly Val Phe Ser Ala Thr Met Pro Pro Glu Ala
    210                 215                 220
ctc gag atc aca agg aag ttc atg agc aaa cca gtg aga atc ctg gtg      720
Leu Glu Ile Thr Arg Lys Phe Met Ser Lys Pro Val Arg Ile Leu Val
225                 230                 235                 240
aag cgt gac gag cta acg ctc gaa ggt atc aag cag ttc tac gtg aac      768
Lys Arg Asp Glu Leu Thr Leu Glu Gly Ile Lys Gln Phe Tyr Val Asn
                245                 250                 255
gtg gag aaa gaa gag tgg aag ctc gaa act ctc tgt gac ctc tac gag      816
Val Glu Lys Glu Glu Trp Lys Leu Glu Thr Leu Cys Asp Leu Tyr Glu
            260                 265                 270
acc tta gcc atc act cag agc gtc atc ttc gtc aac act cgc cgc aag      864
Thr Leu Ala Ile Thr Gln Ser Val Ile Phe Val Asn Thr Arg Arg Lys
        275                 280                 285
gtg gac tgg ctc aca gac aaa atg aga agc cgt gac cac acg gtc tca      912
Val Asp Trp Leu Thr Asp Lys Met Arg Ser Arg Asp His Thr Val Ser
    290                 295                 300
gcc act cac gga gac atg gac caa aac acg aga gac atc atc atg aga      960
Ala Thr His Gly Asp Met Asp Gln Asn Thr Arg Asp Ile Ile Met Arg
305                 310                 315                 320
gag ttc agg tct ggt tct tct cgt gtt ctc atc aca acc gat ctc ttg      1008
Glu Phe Arg Ser Gly Ser Ser Arg Val Leu Ile Thr Thr Asp Leu Leu
                325                 330                 335
gct cgt ggt atc gat gtg cag caa gtg tct ctg gtc atc aac ttt gac      1056
Ala Arg Gly Ile Asp Val Gln Gln Val Ser Leu Val Ile Asn Phe Asp
            340                 345                 350
ctc cca act cag ccg gag aat tac ctt cac cgt atc gga aga agt gga      1104
Leu Pro Thr Gln Pro Glu Asn Tyr Leu His Arg Ile Gly Arg Ser Gly
        355                 360                 365
agg ttt ggg agg aaa ggt gcg gcg att aac ttt gtg act cgt gat gat      1152
Arg Phe Gly Arg Lys Gly Ala Ala Ile Asn Phe Val Thr Arg Asp Asp
    370                 375                 380
gag aga atg ctt gct gat att cag aaa ttc tac aat gtt gtt gtt gag      1200

Glu Arg Met Leu Ala Asp Ile Gln Lys Phe Tyr Asn Val Val Val Glu
385                 390                 395                 400
gag ctt cca tca aac cta gcc gat ctg ctt tga                         1233
Glu Leu Pro Ser Asn Leu Ala Asp Leu Leu
                405                 410

<210> 34
<211> 410
<212> PRT
<213> Brassica napus

<400> 34
Met Ala Gly Ser Ala Thr Gln Phe Asp Asp Ala Arg Gln Phe Asp Gln
1               5                   10                  15
Ala Leu Ser Glu Ile Leu Glu Gly His Asp Glu Phe Phe Thr Ser Tyr
            20                  25                  30
Asp Glu Val Tyr Glu Ser Phe Asp Gly Met Gly Leu Gln Glu Asn Leu
        35                  40                  45
Leu Arg Gly Ile Tyr Ala Tyr Gly Phe Glu Lys Pro Ser Ala Ile Gln
    50                  55                  60
Gln Arg Gly Ile Val Pro Phe Cys Lys Gly Leu Asp Val Ile Gln Gln
65                  70                  75                  80
Ala Gln Ser Gly Thr Gly Lys Thr Ala Thr Phe Cys Ser Gly Val Leu
                85                  90                  95
Gln Gln Leu Asp Val Thr Leu Val Gln Cys Gln Ala Leu Val Leu Ala
            100                 105                 110
Pro Thr Arg Glu Leu Ala Gln Gln Ile Glu Lys Val Met Arg Ala Leu
        115                 120                 125
Gly Asp Tyr Ile Gly Val Lys Val His Ala Cys Val Gly Gly Thr Ser
    130                 135                 140
Val Leu Asp Asp Lys Arg Ile Leu Gln Ala Gly Val His Val Val Val
145                 150                 155                 160
Gly Thr Pro Gly Arg Val Phe Asp Met Leu Gln Arg Gln Ser Leu Arg
                165                 170                 175
Ser Asp Cys Ile Lys Met Phe Val Leu Asp Glu Ala Asp Glu Met Leu
            180                 185                 190
Ser Arg Gly Phe Lys Asp Gln Ile Tyr Asp Ile Phe Gln Leu Leu Pro
        195                 200                 205
Pro Lys Ile Gln Val Gly Val Phe Ser Ala Thr Met Pro Pro Glu Ala
    210                 215                 220
Leu Glu Ile Thr Arg Lys Phe Met Ser Lys Pro Val Arg Ile Leu Val
225                 230                 235                 240
Lys Arg Asp Glu Leu Thr Leu Glu Gly Ile Lys Gln Phe Tyr Val Asn
                245                 250                 255
Val Glu Lys Glu Glu Trp Lys Leu Glu Thr Leu Cys Asp Leu Tyr Glu
            260                 265                 270
Thr Leu Ala Ile Thr Gln Ser Val Ile Phe Val Asn Thr Arg Arg Lys
        275                 280                 285
Val Asp Trp Leu Thr Asp Lys Met Arg Ser Arg Asp His Thr Val Ser
    290                 295                 300
Ala Thr His Gly Asp Met Asp Gln Asn Thr Arg Asp Ile Ile Met Arg
305                 310                 315                 320
Glu Phe Arg Ser Gly Ser Ser Arg Val Leu Ile Thr Thr Asp Leu Leu
                325                 330                 335
Ala Arg Gly Ile Asp Val Gln Gln Val Ser Leu Val Ile Asn Phe Asp
            340                 345                 350
Leu Pro Thr Gln Pro Glu Asn Tyr Leu His Arg Ile Gly Arg Ser Gly
        355                 360                 365
Arg Phe Gly Arg Lys Gly Ala Ala Ile Asn Phe Val Thr Arg Asp Asp
    370                 375                 380
Glu Arg Met Leu Ala Asp Ile Gln Lys Phe Tyr Asn Val Val Val Glu
385                 390                 395                 400
Glu Leu Pro Ser Asn Leu Ala Asp Leu Leu
                405                 410

<210> 35
<211> 1227
<212> DNA
<213> Brassica napus

<220>
<221> CDS
<222> (1)..(1227)

<400> 35
atg gcg gaa gcg aat cct ggc cgt gga ggt gga aga aga ggc ggc gga 48
Met Ala Glu Ala Asn Pro Gly Arg Gly Gly Gly Arg Arg Gly Gly Gly
1               5                   10                  15
cca atg gac gac gac aaa ttg gtc ttc gag acg acg gaa ggg atc gag 96
Pro Met Asp Asp Asp Lys Leu Val Phe Glu Thr Thr Glu Gly Ile Glu
            20                  25                  30
ccc atc acg aat ttc aac gat atg ggg atc aag gag gac gtt ctc cgc 144
Pro Ile Thr Asn Phe Asn Asp Met Gly Ile Lys Glu Asp Val Leu Arg
        35                  40                  45
ggc gtc tac gag tac ggt ttc gag aag ccc tcc gcg att cag cag aga 192
Gly Val Tyr Glu Tyr Gly Phe Glu Lys Pro Ser Ala Ile Gln Gln Arg
    50                  55                  60
gcg gtc atg ccg att ctc caa ggt cga gat gtg atc gct cag gct cag 240
Ala Val Met Pro Ile Leu Gln Gly Arg Asp Val Ile Ala Gln Ala Gln
65                  70                  75                  80
tcc ggt acc gga aag acc tcc atg att gct ctc tcc gtc tgc caa atc 288
Ser Gly Thr Gly Lys Thr Ser Met Ile Ala Leu Ser Val Cys Gln Ile
                85                  90                  95
gtt gac act tcg tct aga gaa gtt cag gcg ttg ata ttg tcc ccg acg 336
Val Asp Thr Ser Ser Arg Glu Val Gln Ala Leu Ile Leu Ser Pro Thr
            100                 105                 110
aga gag ctg gct tcg cag acg gag aag act att caa gct att ggg ttg 384
Arg Glu Leu Ala Ser Gln Thr Glu Lys Thr Ile Gln Ala Ile Gly Leu
        115                 120                 125
cac gct aat att cag gcg cat gcg tgt att ggt ggg aag agc gtt gga 432
His Ala Asn Ile Gln Ala His Ala Cys Ile Gly Gly Lys Ser Val Gly
    130                 135                 140
gag gat atc agg aag ctg gag aat ggt gtc cat gtt gtg tct ggg acg 480
Glu Asp Ile Arg Lys Leu Glu Asn Gly Val His Val Val Ser Gly Thr
145                 150                 155                 160
cct ggg cgt gta tgt gat atg att aag agg agg agt ctg cgt acc aga 528
Pro Gly Arg Val Cys Asp Met Ile Lys Arg Arg Ser Leu Arg Thr Arg
                165                 170                 175
gct att aag ctt ttg att ctt gat gaa tct gat gag atg ctg agc agg 576
Ala Ile Lys Leu Leu Ile Leu Asp Glu Ser Asp Glu Met Leu Ser Arg
            180                 185                 190
ggg ttt aag gac cag ata tat gat gtt tac aga tat ctt ccg cct gat 624
Gly Phe Lys Asp Gln Ile Tyr Asp Val Tyr Arg Tyr Leu Pro Pro Asp
        195                 200                 205
ctt cag gtt tgt ttg gtt tct gca act ctt cct cac gag att ttg gag 672
Leu Gln Val Cys Leu Val Ser Ala Thr Leu Pro His Glu Ile Leu Glu
    210                 215                 220
atg aca tca aag ttt atg acg gag cca gtg aag ata ctt gtg aag cgt 720
Met Thr Ser Lys Phe Met Thr Glu Pro Val Lys Ile Leu Val Lys Arg
225                 230                 235                 240
gat gag ttg act ctt gaa ggc att aaa caa ttt ttc gtt gct gtt gag 768
Asp Glu Leu Thr Leu Glu Gly Ile Lys Gln Phe Phe Val Ala Val Glu
                245                 250                 255
aaa gag gag tgg aaa ttt gat acg ctc tgt gat ctt tat gac act ctt 816
Lys Glu Glu Trp Lys Phe Asp Thr Leu Cys Asp Leu Tyr Asp Thr Leu
            260                 265                 270
act atc act caa gct gtt atc ttc tgc aac act aaa cga aag gtg gat 864
Thr Ile Thr Gln Ala Val Ile Phe Cys Asn Thr Lys Arg Lys Val Asp
        275                 280                 285
tgg cta agt gag aaa atg agg act aac aac ttc aca gtc tca tca atg 912
Trp Leu Ser Glu Lys Met Arg Thr Asn Asn Phe Thr Val Ser Ser Met
    290                 295                 300
cat ggt gac atg cct cag aag gag aga gac gag atc atg aat cag ttt 960
His Gly Asp Met Pro Gln Lys Glu Arg Asp Glu Ile Met Asn Gln Phe
305                 310                 315                 320
cgg tca ggg gac agt cgt gtt ttg atc acg aca gat gta tgg gca cgt 1008
Arg Ser Gly Asp Ser Arg Val Leu Ile Thr Thr Asp Val Trp Ala Arg
                325                 330                 335

ggg att gat gtg cag caa gtt tct ctt gtg atc aat tat gat ctc ccc 1056
Gly Ile Asp Val Gln Gln Val Ser Leu Val Ile Asn Tyr Asp Leu Pro
            340                 345                 350
aac aac cgt gag ctc tac atc cat cgt att gga cga tct ggt cgt ttc 1104
Asn Asn Arg Glu Leu Tyr Ile His Arg Ile Gly Arg Ser Gly Arg Phe
        355                 360                 365
ggc cgt aag ggt gtt gcg atc aac ttt gtt aaa agc gac gac atc aag 1152
Gly Arg Lys Gly Val Ala Ile Asn Phe Val Lys Ser Asp Asp Ile Lys
    370                 375                 380
atc ctc cgg gac att gag cag tac tac agt act cag att gac gag atg 1200
Ile Leu Arg Asp Ile Glu Gln Tyr Tyr Ser Thr Gln Ile Asp Glu Met
385                 390                 395                 400
cca atg aat gta gct gat ctt atc taa 1227
Pro Met Asn Val Ala Asp Leu Ile
                405

<210> 36
<211> 408
<212> PRT
<213> Brassica napus

<400> 36
Met Ala Glu Ala Asn Pro Gly Arg Gly Gly Gly Arg Arg Gly Gly Gly
1               5                   10                  15
Pro Met Asp Asp Asp Lys Leu Val Phe Glu Thr Thr Glu Gly Ile Glu
            20                  25                  30
Pro Ile Thr Asn Phe Asn Asp Met Gly Ile Lys Glu Asp Val Leu Arg
        35                  40                  45
Gly Val Tyr Glu Tyr Gly Phe Glu Lys Pro Ser Ala Ile Gln Gln Arg
    50                  55                  60
Ala Val Met Pro Ile Leu Gln Gly Arg Asp Val Ile Ala Gln Ala Gln
65                  70                  75                  80
Ser Gly Thr Gly Lys Thr Ser Met Ile Ala Leu Ser Val Cys Gln Ile
                85                  90                  95
Val Asp Thr Ser Ser Arg Glu Val Gln Ala Leu Ile Leu Ser Pro Thr
            100                 105                 110
Arg Glu Leu Ala Ser Gln Thr Glu Lys Thr Ile Gln Ala Ile Gly Leu
        115                 120                 125
His Ala Asn Ile Gln Ala His Ala Cys Ile Gly Gly Lys Ser Val Gly
    130                 135                 140
Glu Asp Ile Arg Lys Leu Glu Asn Gly Val His Val Val Ser Gly Thr
145                 150                 155                 160
Pro Gly Arg Val Cys Asp Met Ile Lys Arg Arg Ser Leu Arg Thr Arg
                165                 170                 175
Ala Ile Lys Leu Leu Ile Leu Asp Glu Ser Asp Glu Met Leu Ser Arg
            180                 185                 190
Gly Phe Lys Asp Gln Ile Tyr Asp Val Tyr Arg Tyr Leu Pro Pro Asp
        195                 200                 205
Leu Gln Val Cys Leu Val Ser Ala Thr Leu Pro His Glu Ile Leu Glu
    210                 215                 220
Met Thr Ser Lys Phe Met Thr Glu Pro Val Lys Ile Leu Val Lys Arg
225                 230                 235                 240
Asp Glu Leu Thr Leu Glu Gly Ile Lys Gln Phe Phe Val Ala Val Glu
                245                 250                 255
Lys Glu Glu Trp Lys Phe Asp Thr Leu Cys Asp Leu Tyr Asp Thr Leu
            260                 265                 270
Thr Ile Thr Gln Ala Val Ile Phe Cys Asn Thr Lys Arg Lys Val Asp
        275                 280                 285
Trp Leu Ser Glu Lys Met Arg Thr Asn Asn Phe Thr Val Ser Ser Met
    290                 295                 300
His Gly Asp Met Pro Gln Lys Glu Arg Asp Glu Ile Met Asn Gln Phe
305                 310                 315                 320
Arg Ser Gly Asp Ser Arg Val Leu Ile Thr Thr Asp Val Trp Ala Arg
                325                 330                 335
Gly Ile Asp Val Gln Gln Val Ser Leu Val Ile Asn Tyr Asp Leu Pro
            340                 345                 350
Asn Asn Arg Glu Leu Tyr Ile His Arg Ile Gly Arg Ser Gly Arg Phe
        355                 360                 365
Gly Arg Lys Gly Val Ala Ile Asn Phe Val Lys Ser Asp Asp Ile Lys

370                 375                 380
Ile Leu Arg Asp Ile Glu Gln Tyr Tyr Ser Thr Gln Ile Asp Glu Met
385                 390                 395                 400
Pro Met Asn Val Ala Asp Leu Ile
                405

<210> 37
<211> 1245
<212> DNA
<213> Zea mays

<220>
<221> CDS
<222> (1)..(1245)

<400> 37
atg gca gga atg gca ccc gag ggt tct cag ttt gat gct aag cac tat      48
Met Ala Gly Met Ala Pro Glu Gly Ser Gln Phe Asp Ala Lys His Tyr
1               5                   10                  15
gat tct aaa atg cag gag ctg ctg agc act ggt gag act gag gag ttc      96
Asp Ser Lys Met Gln Glu Leu Leu Ser Thr Gly Glu Thr Glu Glu Phe
            20                  25                  30
ttc act tca tat gat gaa gtt ttt gaa agt ttt gat gac atg ggc ctt      144
Phe Thr Ser Tyr Asp Glu Val Phe Glu Ser Phe Asp Asp Met Gly Leu
        35                  40                  45
caa gag aat ctt ctg aga ggc att tat gca tat ggt ttt gag aaa cca      192
Gln Glu Asn Leu Leu Arg Gly Ile Tyr Ala Tyr Gly Phe Glu Lys Pro
    50                  55                  60
tct gca att cag cag aga gga att gtt ccc ttc tgc aag ggg ctt gat      240
Ser Ala Ile Gln Gln Arg Gly Ile Val Pro Phe Cys Lys Gly Leu Asp
65                  70                  75                  80
gtc att cag caa gca caa tct ggt aca gga aag aca gca acc ttc tgt      288
Val Ile Gln Gln Ala Gln Ser Gly Thr Gly Lys Thr Ala Thr Phe Cys
                85                  90                  95
tct ggg atc ttg cag cag ctt gac tat ggt ctg gtt gaa tgc cag gcc      336
Ser Gly Ile Leu Gln Gln Leu Asp Tyr Gly Leu Val Glu Cys Gln Ala
            100                 105                 110
ctg gtc ctt gct ccc acc cgt gag ctt gca cag caa atc gag aaa gtc      384
Leu Val Leu Ala Pro Thr Arg Glu Leu Ala Gln Gln Ile Glu Lys Val
        115                 120                 125
atg cgc gcg ctt ggt gac tac ctt ggt gtt aag gtc cat gca tgt gtg      432
Met Arg Ala Leu Gly Asp Tyr Leu Gly Val Lys Val His Ala Cys Val
    130                 135                 140
gga gga aca tcc gtc cgt gag gac caa agg att ctc gcc agt ggt gtg      480
Gly Gly Thr Ser Val Arg Glu Asp Gln Arg Ile Leu Ala Ser Gly Val
145                 150                 155                 160
cat gtt gtt gtt ggt aca cct ggt cgt gtg ttt gac atg ctg cgt aga      528
His Val Val Val Gly Thr Pro Gly Arg Val Phe Asp Met Leu Arg Arg
                165                 170                 175
cag tcc ctc cgc cct gat aac atc aag atg ttt gtt ctt gat gaa gct      576
Gln Ser Leu Arg Pro Asp Asn Ile Lys Met Phe Val Leu Asp Glu Ala
            180                 185                 190
gat gaa atg ctt tct cgt ggt ttc aag gat cag att tat gac atc ttc      624
Asp Glu Met Leu Ser Arg Gly Phe Lys Asp Gln Ile Tyr Asp Ile Phe
        195                 200                 205
cag ctt ctg cca tcc aag att cag gtt gga gtc ttc tct gct acc atg      672
Gln Leu Leu Pro Ser Lys Ile Gln Val Gly Val Phe Ser Ala Thr Met
    210                 215                 220
cca cct gag gcc ctt gag att acc cgc aag ttc atg aac aaa cca gtg      720
Pro Pro Glu Ala Leu Glu Ile Thr Arg Lys Phe Met Asn Lys Pro Val
225                 230                 235                 240
aga att ctt gtg aag aga gat gag ctg acc ctt gag ggt atc aag cag      768
Arg Ile Leu Val Lys Arg Asp Glu Leu Thr Leu Glu Gly Ile Lys Gln
                245                 250                 255
ttc tat gtg aat gtg gac aag gaa gac tgg aag ctg gac aca ctg tgt      816
Phe Tyr Val Asn Val Asp Lys Glu Asp Trp Lys Leu Asp Thr Leu Cys
            260                 265                 270
gac ctg tat gag acc ctg gcc att acc cag agt gtc atc ttt gtc aac      864
Asp Leu Tyr Glu Thr Leu Ala Ile Thr Gln Ser Val Ile Phe Val Asn

                275                 280                 285
acc cgc cgc aag gtg gac tgg ctc act gac aag atg agg agc agg gac        912
Thr Arg Arg Lys Val Asp Trp Leu Thr Asp Lys Met Arg Ser Arg Asp
    290                 295                 300
cac acc gtt tct gcc act cat ggt gac atg gac cag aac act aga gac        960
His Thr Val Ser Ala Thr His Gly Asp Met Asp Gln Asn Thr Arg Asp
305                 310                 315                 320
atc atc atg agg gag ttc cgg tct ggc tcc tcc cgt gtg ctc atc acc        1008
Ile Ile Met Arg Glu Phe Arg Ser Gly Ser Ser Arg Val Leu Ile Thr
                325                 330                 335
acc gac ctg ctt gct cgt ggt att gat gtt cag cag gtg tcc ctg gtc        1056
Thr Asp Leu Leu Ala Arg Gly Ile Asp Val Gln Gln Val Ser Leu Val
            340                 345                 350
atc aac tat gat ctc ccc acc cag cca gag aat tac ctg cat cgc att        1104
Ile Asn Tyr Asp Leu Pro Thr Gln Pro Glu Asn Tyr Leu His Arg Ile
        355                 360                 365
ggt cgt agt ggt cgt ttc ggt agg aag ggt gtt gcc atc aac ttt gtg        1152
Gly Arg Ser Gly Arg Phe Gly Arg Lys Gly Val Ala Ile Asn Phe Val
    370                 375                 380
acc cgc gac gac gag cgg atg ctg ttc gac atc cag aag ttc tac aac        1200
Thr Arg Asp Asp Glu Arg Met Leu Phe Asp Ile Gln Lys Phe Tyr Asn
385                 390                 395                 400
gtg ttg att gag gag ctg cct gcc aac gtc gct gac ctt ctg tga            1245
Val Leu Ile Glu Glu Leu Pro Ala Asn Val Ala Asp Leu Leu
                405                 410

<210> 38
<211> 414
<212> PRT
<213> Zea mays

<400> 38
Met Ala Gly Met Ala Pro Glu Gly Ser Gln Phe Asp Ala Lys His Tyr
1               5                   10                  15
Asp Ser Lys Met Gln Glu Leu Leu Ser Thr Gly Glu Thr Glu Glu Phe
            20                  25                  30
Phe Thr Ser Tyr Asp Glu Val Phe Glu Ser Phe Asp Asp Met Gly Leu
        35                  40                  45
Gln Glu Asn Leu Leu Arg Gly Ile Tyr Ala Tyr Gly Phe Glu Lys Pro
    50                  55                  60
Ser Ala Ile Gln Gln Arg Gly Ile Val Pro Phe Cys Lys Gly Leu Asp
65                  70                  75                  80
Val Ile Gln Gln Ala Gln Ser Gly Thr Gly Lys Thr Ala Thr Phe Cys
                85                  90                  95
Ser Gly Ile Leu Gln Gln Leu Asp Tyr Gly Leu Val Glu Cys Gln Ala
            100                 105                 110
Leu Val Leu Ala Pro Thr Arg Glu Leu Ala Gln Gln Ile Glu Lys Val
        115                 120                 125
Met Arg Ala Leu Gly Asp Tyr Leu Gly Val Lys Val His Ala Cys Val
    130                 135                 140
Gly Gly Thr Ser Val Arg Glu Asp Gln Arg Ile Leu Ala Ser Gly Val
145                 150                 155                 160
His Val Val Val Gly Thr Pro Gly Arg Val Phe Asp Met Leu Arg Arg
                165                 170                 175
Gln Ser Leu Arg Pro Asp Asn Ile Lys Met Phe Val Leu Asp Glu Ala
            180                 185                 190
Asp Glu Met Leu Ser Arg Gly Phe Lys Asp Gln Ile Tyr Asp Ile Phe
        195                 200                 205
Gln Leu Leu Pro Ser Lys Ile Gln Val Gly Val Phe Ser Ala Thr Met
    210                 215                 220
Pro Pro Glu Ala Leu Glu Ile Thr Arg Lys Phe Met Asn Lys Pro Val
225                 230                 235                 240
Arg Ile Leu Val Lys Arg Asp Glu Leu Thr Leu Glu Gly Ile Lys Gln
                245                 250                 255
Phe Tyr Val Asn Val Asp Lys Glu Asp Trp Lys Leu Asp Thr Leu Cys
            260                 265                 270
Asp Leu Tyr Glu Thr Leu Ala Ile Thr Gln Ser Val Ile Phe Val Asn
        275                 280                 285
Thr Arg Arg Lys Val Asp Trp Leu Thr Asp Lys Met Arg Ser Arg Asp

290                 295                 300
His Thr Val Ser Ala Thr His Gly Asp Met Asp Gln Asn Thr Arg Asp
305                 310                 315                 320
Ile Ile Met Arg Glu Phe Arg Ser Gly Ser Ser Arg Val Leu Ile Thr
                325                 330                 335
Thr Asp Leu Leu Ala Arg Gly Ile Asp Val Gln Gln Val Ser Leu Val
            340                 345                 350
Ile Asn Tyr Asp Leu Pro Thr Gln Pro Glu Asn Tyr Leu His Arg Ile
        355                 360                 365
Gly Arg Ser Gly Arg Phe Gly Arg Lys Gly Val Ala Ile Asn Phe Val
    370                 375                 380
Thr Arg Asp Asp Glu Arg Met Leu Phe Asp Ile Gln Lys Phe Tyr Asn
385                 390                 395                 400
Val Leu Ile Glu Glu Leu Pro Ala Asn Val Ala Asp Leu Leu
                405                 410

<210> 39
<211> 1224
<212> DNA
<213> Zea mays

<220>
<221> CDS
<222> (1)..(1224)

<400> 39
atg gcg gcg ccc acc acc tcc cgc cgc ggc ccc ggc ggc gcg cgc aac      48
Met Ala Ala Pro Thr Thr Ser Arg Arg Gly Pro Gly Gly Ala Arg Asn
1               5                   10                  15
atg gac gac gag aac ctc acc ttc gag acc tcc ccg ggg gtc gag gtc      96
Met Asp Asp Glu Asn Leu Thr Phe Glu Thr Ser Pro Gly Val Glu Val
            20                  25                  30
gtc agc agc ttc gac cag atg ggc atc aag gac gac ctc ctc cgc ggc      144
Val Ser Ser Phe Asp Gln Met Gly Ile Lys Asp Asp Leu Leu Arg Gly
        35                  40                  45
atc tac ggc tac ggt ttc gag aag ccc tcc gcc atc cag cag cgc gcc      192
Ile Tyr Gly Tyr Gly Phe Glu Lys Pro Ser Ala Ile Gln Gln Arg Ala
    50                  55                  60
gtg ctc ccc atc atc aac ggc cgc gac gtc atc gcg cag gcc cag tcc      240
Val Leu Pro Ile Ile Asn Gly Arg Asp Val Ile Ala Gln Ala Gln Ser
65                  70                  75                  80
ggc acc ggc aag acg tcc atg atc tcc ctc acc gtc tgc cag atc gtc      288
Gly Thr Gly Lys Thr Ser Met Ile Ser Leu Thr Val Cys Gln Ile Val
                85                  90                  95
gac acc gcc gtg cgc gag gtg cag gct ttg att ctc tcg cct act agg      336
Asp Thr Ala Val Arg Glu Val Gln Ala Leu Ile Leu Ser Pro Thr Arg
            100                 105                 110
gag ctt gct tcc caa aca gag aga gtt atg ctg gct att ggg gac cac      384
Glu Leu Ala Ser Gln Thr Glu Arg Val Met Leu Ala Ile Gly Asp His
        115                 120                 125
ctc aac gtc caa gta cat gct tgc att ggt gga aaa agt atc ggt gag      432
Leu Asn Val Gln Val His Ala Cys Ile Gly Gly Lys Ser Ile Gly Glu
    130                 135                 140
gat atc agg agg ctc gag aat ggg gtg cat gtt gtc tcg gga act cca      480
Asp Ile Arg Arg Leu Glu Asn Gly Val His Val Val Ser Gly Thr Pro
145                 150                 155                 160
ggc aga gtc tgt gat atg atc aag agg agg acc ttg cga aca aga gcc      528
Gly Arg Val Cys Asp Met Ile Lys Arg Arg Thr Leu Arg Thr Arg Ala
                165                 170                 175
atc aag ctt cta gtt ctg gat gaa gct gat gag atg ttg agt aga ggc      576
Ile Lys Leu Leu Val Leu Asp Glu Ala Asp Glu Met Leu Ser Arg Gly
            180                 185                 190
ttt aag gat cag att tat gat gtc tac aga tat ctc ccg cca gaa ctt      624
Phe Lys Asp Gln Ile Tyr Asp Val Tyr Arg Tyr Leu Pro Pro Glu Leu
        195                 200                 205
cag gtg gtt ttg atc tct gca act ctt cct cat gag atc ttg gag atg      672
Gln Val Val Leu Ile Ser Ala Thr Leu Pro His Glu Ile Leu Glu Met
    210                 215                 220
act agt aag ttc atg acc gaa ccg gtt agg atc ctt gtg aag cgt gat      720

Thr Ser Lys Phe Met Thr Glu Pro Val Arg Ile Leu Val Lys Arg Asp
225                 230                 235                 240
gag ctg acc ctg gag ggc atc aaa caa ttc ttt gtt gct gtt gag aaa      768
Glu Leu Thr Leu Glu Gly Ile Lys Gln Phe Phe Val Ala Val Glu Lys
                245                 250                 255
gag gag tgg aag ttt gat aca ctt tgt gat ctt tat gat aca ctc acc      816
Glu Glu Trp Lys Phe Asp Thr Leu Cys Asp Leu Tyr Asp Thr Leu Thr
            260                 265                 270
atc acc caa gct gtt att ttc tgc aat act aag aga aag gtg gat tgg      864
Ile Thr Gln Ala Val Ile Phe Cys Asn Thr Lys Arg Lys Val Asp Trp
        275                 280                 285
ctc act gaa aaa atg cgt aca aat aac ttc acc gta tca gct atg cac      912
Leu Thr Glu Lys Met Arg Thr Asn Asn Phe Thr Val Ser Ala Met His
    290                 295                 300
ggt gac atg ccc caa caa gaa agg gat gcc att atg ggt gag ttc agg      960
Gly Asp Met Pro Gln Gln Glu Arg Asp Ala Ile Met Gly Glu Phe Arg
305                 310                 315                 320
tct ggc gca act cgt gtg cta ata act aca gat gtt tgg gct cga gga      1008
Ser Gly Ala Thr Arg Val Leu Ile Thr Thr Asp Val Trp Ala Arg Gly
                325                 330                 335
ctg gac gtt cag cag gtt tca ctt gtc ata aat tat gat ctc cca aat      1056
Leu Asp Val Gln Gln Val Ser Leu Val Ile Asn Tyr Asp Leu Pro Asn
            340                 345                 350
aat cgt gag ctt tac atc cat cgc att ggt cgt tct ggt cgt ttt ggg      1104
Asn Arg Glu Leu Tyr Ile His Arg Ile Gly Arg Ser Gly Arg Phe Gly
        355                 360                 365
cgc aag ggt gtg gcg atc aat ttc gtg cgc aag gat gac atc cgt ata      1152
Arg Lys Gly Val Ala Ile Asn Phe Val Arg Lys Asp Asp Ile Arg Ile
    370                 375                 380
ctg aga gat ata gag cag tac tac agc aca caa att gac gag atg cca      1200
Leu Arg Asp Ile Glu Gln Tyr Tyr Ser Thr Gln Ile Asp Glu Met Pro
385                 390                 395                 400
atg aat gtt gct gat ctt att tga                                      1224
Met Asn Val Ala Asp Leu Ile
                405

<210> 40
<211> 407
<212> PRT
<213> Zea mays

<400> 40
Met Ala Ala Pro Thr Thr Ser Arg Arg Gly Pro Gly Gly Ala Arg Asn
1               5                   10                  15
Met Asp Asp Glu Asn Leu Thr Phe Glu Thr Ser Pro Gly Val Glu Val
            20                  25                  30
Val Ser Ser Phe Asp Gln Met Gly Ile Lys Asp Asp Leu Leu Arg Gly
        35                  40                  45
Ile Tyr Gly Tyr Gly Phe Glu Lys Pro Ser Ala Ile Gln Gln Arg Ala
    50                  55                  60
Val Leu Pro Ile Ile Asn Gly Arg Asp Val Ile Ala Gln Ala Gln Ser
65                  70                  75                  80
Gly Thr Gly Lys Thr Ser Met Ile Ser Leu Thr Val Cys Gln Ile Val
                85                  90                  95
Asp Thr Ala Val Arg Glu Val Gln Ala Leu Ile Leu Ser Pro Thr Arg
            100                 105                 110
Glu Leu Ala Ser Gln Thr Glu Arg Val Met Leu Ala Ile Gly Asp His
        115                 120                 125
Leu Asn Val Gln Val His Ala Cys Ile Gly Gly Lys Ser Ile Gly Glu
    130                 135                 140
Asp Ile Arg Arg Leu Glu Asn Gly Val His Val Val Ser Gly Thr Pro
145                 150                 155                 160
Gly Arg Val Cys Asp Met Ile Lys Arg Arg Thr Leu Arg Thr Arg Ala
                165                 170                 175
Ile Lys Leu Leu Val Leu Asp Glu Ala Asp Glu Met Leu Ser Arg Gly
            180                 185                 190
Phe Lys Asp Gln Ile Tyr Asp Val Tyr Arg Tyr Leu Pro Pro Glu Leu
        195                 200                 205
Gln Val Val Leu Ile Ser Ala Thr Leu Pro His Glu Ile Leu Glu Met

210                 215                 220
Thr Ser Lys Phe Met Thr Glu Pro Val Arg Ile Leu Val Lys Arg Asp
225                 230                 235                 240
Glu Leu Thr Leu Glu Gly Ile Lys Gln Phe Phe Val Ala Val Glu Lys
                245                 250                 255
Glu Glu Trp Lys Phe Asp Thr Leu Cys Asp Leu Tyr Asp Thr Leu Thr
            260                 265                 270
Ile Thr Gln Ala Val Ile Phe Cys Asn Thr Lys Arg Lys Val Asp Trp
        275                 280                 285
Leu Thr Glu Lys Met Arg Thr Asn Asn Phe Thr Val Ser Ala Met His
    290                 295                 300
Gly Asp Met Pro Gln Gln Glu Arg Asp Ala Ile Met Gly Glu Phe Arg
305                 310                 315                 320
Ser Gly Ala Thr Arg Val Leu Ile Thr Thr Asp Val Trp Ala Arg Gly
                325                 330                 335
Leu Asp Val Gln Gln Val Ser Leu Val Ile Asn Tyr Asp Leu Pro Asn
            340                 345                 350
Asn Arg Glu Leu Tyr Ile His Arg Ile Gly Arg Ser Gly Arg Phe Gly
        355                 360                 365
Arg Lys Gly Val Ala Ile Asn Phe Val Arg Lys Asp Asp Ile Arg Ile
    370                 375                 380
Leu Arg Asp Ile Glu Gln Tyr Tyr Ser Thr Gln Ile Asp Glu Met Pro
385                 390                 395                 400
Met Asn Val Ala Asp Leu Ile
                405

<210> 41
<211> 1500
<212> DNA
<213> Zea mays

<220>
<221> CDS
<222> (1)..(1500)

<400> 41
atg cac cag cct aga gct cgc tac cca ccc ggc tat ggc tcc ggc ggt      48
Met His Gln Pro Arg Ala Arg Tyr Pro Pro Gly Tyr Gly Ser Gly Gly
1               5                   10                  15
gga ggc gga gcc gga cgt ggc ggt gga ggc gga aat gga gga ggt ggc      96
Gly Gly Gly Ala Gly Arg Gly Gly Gly Gly Gly Asn Gly Gly Gly Gly
            20                  25                  30
gga aac cac aac tac tac ggc cga aac ccg cag caa cac cac tac cac      144
Gly Asn His Asn Tyr Tyr Gly Arg Asn Pro Gln Gln His His Tyr His
        35                  40                  45
cag cag cag cag tcg cag cac tct cat agg aac tct tcg tac cag caa      192
Gln Gln Gln Gln Ser Gln His Ser His Arg Asn Ser Ser Tyr Gln Gln
    50                  55                  60
cag tgg ctg cgg cga gac cag gcc ccc gcc gtt gcg ggg gca gcg tcc      240
Gln Trp Leu Arg Arg Asp Gln Ala Pro Ala Val Ala Gly Ala Ala Ser
65                  70                  75                  80
gga aac gct gtg gcc aag acg gcg tct cag ctt gac gcc gta ggc tcg      288
Gly Asn Ala Val Ala Lys Thr Ala Ser Gln Leu Asp Ala Val Gly Ser
                85                  90                  95
agc tcc caa gac tgg aag gct caa cta aat ata cca gct cca gat aca      336
Ser Ser Gln Asp Trp Lys Ala Gln Leu Asn Ile Pro Ala Pro Asp Thr
            100                 105                 110
cgc tac agg aca gag gat gtc act gca aca aaa ggc aat gag ttt gaa      384
Arg Tyr Arg Thr Glu Asp Val Thr Ala Thr Lys Gly Asn Glu Phe Glu
        115                 120                 125
gat tat ttc ttg aaa cgt gaa ctg ctt atg gga atc tac gaa aag gga      432
Asp Tyr Phe Leu Lys Arg Glu Leu Leu Met Gly Ile Tyr Glu Lys Gly
    130                 135                 140
ttt gaa aga cca tct cct att caa gaa gaa agc att cct att gcg ctg      480
Phe Glu Arg Pro Ser Pro Ile Gln Glu Glu Ser Ile Pro Ile Ala Leu
145                 150                 155                 160
act gga agt gat att ctt gca agg gca aaa aat ggc act gga aag act      528
Thr Gly Ser Asp Ile Leu Ala Arg Ala Lys Asn Gly Thr Gly Lys Thr
                165                 170                 175

gct gcc ttt tgc att ccg gca ctt gaa aaa att gat cct gaa aat aat          576
Ala Ala Phe Cys Ile Pro Ala Leu Glu Lys Ile Asp Pro Glu Asn Asn
            180                 185                 190
gca att caa gtt gtt ata cta gta cca aca agg gaa ctg gct ctg caa          624
Ala Ile Gln Val Val Ile Leu Val Pro Thr Arg Glu Leu Ala Leu Gln
        195                 200                 205
act tca caa gtg tgt aaa gag ctt ggg aaa tac cta aac att gaa gtt          672
Thr Ser Gln Val Cys Lys Glu Leu Gly Lys Tyr Leu Asn Ile Glu Val
    210                 215                 220
atg gtt agc act gga gga acc agc ttg aag gat gac atc atg cgt tta          720
Met Val Ser Thr Gly Gly Thr Ser Leu Lys Asp Asp Ile Met Arg Leu
225                 230                 235                 240
tac caa cct gtt cat cta ctc gtt ggg act cct ggc cga ata ctt gat          768
Tyr Gln Pro Val His Leu Leu Val Gly Thr Pro Gly Arg Ile Leu Asp
                245                 250                 255
ctt acc agg aag ggc att tgt gtg ctg aaa gat tgt tca atg ctt gtc          816
Leu Thr Arg Lys Gly Ile Cys Val Leu Lys Asp Cys Ser Met Leu Val
            260                 265                 270
atg gat gag gca gac aag cta tta gct cca gag ttc cag cct tct gtg          864
Met Asp Glu Ala Asp Lys Leu Leu Ala Pro Glu Phe Gln Pro Ser Val
        275                 280                 285
gag gca ctt att cat ttc ctt cca cct agt cgg cag ctg ttg atg ttt          912
Glu Ala Leu Ile His Phe Leu Pro Pro Ser Arg Gln Leu Leu Met Phe
    290                 295                 300
tca gca acc ttt cct gta act gtc aag gaa ttc aaa gag aaa tat ctg          960
Ser Ala Thr Phe Pro Val Thr Val Lys Glu Phe Lys Glu Lys Tyr Leu
305                 310                 315                 320
cct aga cct tat gtg att aat cta atg gac gaa ctg acc ctg aaa gga          1008
Pro Arg Pro Tyr Val Ile Asn Leu Met Asp Glu Leu Thr Leu Lys Gly
                325                 330                 335
atc aca caa tat tat gct ttt gtt gaa gaa agg cag aaa gtt cat tgc          1056
Ile Thr Gln Tyr Tyr Ala Phe Val Glu Glu Arg Gln Lys Val His Cys
            340                 345                 350
ctg aat aca ctt ttc tca aag ctt caa att aat caa tcc att ata ttc          1104
Leu Asn Thr Leu Phe Ser Lys Leu Gln Ile Asn Gln Ser Ile Ile Phe
        355                 360                 365
tgc aac tct gtt aat aga gtt gag ctg ctg gct aaa aaa ata act gaa          1152
Cys Asn Ser Val Asn Arg Val Glu Leu Leu Ala Lys Lys Ile Thr Glu
    370                 375                 380
ctc ggt tat tca tgc ttc tac att cat gct aag atg ttg caa gac cac          1200
Leu Gly Tyr Ser Cys Phe Tyr Ile His Ala Lys Met Leu Gln Asp His
385                 390                 395                 400
aga aat cga gta ttt cat gat ttt cgt aat ggt gct tgc aga aac ctt          1248
Arg Asn Arg Val Phe His Asp Phe Arg Asn Gly Ala Cys Arg Asn Leu
                405                 410                 415
gtg tgc aca gat ctg ttt acc aga gga att gat atc cag gct gtt aat          1296
Val Cys Thr Asp Leu Phe Thr Arg Gly Ile Asp Ile Gln Ala Val Asn
            420                 425                 430
gtg gtc att aat ttt gac ttc cct aag aca tct gag aca tac tta cac          1344
Val Val Ile Asn Phe Asp Phe Pro Lys Thr Ser Glu Thr Tyr Leu His
        435                 440                 445
agg gtt ggc cgt tct ggg aga tat gga cac ctt ggt ttg gca gtg aac          1392
Arg Val Gly Arg Ser Gly Arg Tyr Gly His Leu Gly Leu Ala Val Asn
    450                 455                 460
ttg atc act tat gaa gac cgt ttc aac tta tat agg att gag caa gaa          1440
Leu Ile Thr Tyr Glu Asp Arg Phe Asn Leu Tyr Arg Ile Glu Gln Glu
465                 470                 475                 480
ctt gga acg gaa att aag aca ata ccc cca cag att gac ctg tcc ata          1488
Leu Gly Thr Glu Ile Lys Thr Ile Pro Pro Gln Ile Asp Leu Ser Ile
                485                 490                 495
tac tgc caa tga                                                          1500
Tyr Cys Gln

<210> 42
<211> 499
<212> PRT
<213> Zea mays

<400> 42
Met His Gln Pro Arg Ala Arg Tyr Pro Pro Gly Tyr Gly Ser Gly Gly
1               5                   10                  15
Gly Gly Gly Ala Gly Arg Gly Gly Gly Gly Gly Asn Gly Gly Gly Gly
            20                  25                  30
Gly Asn His Asn Tyr Tyr Gly Arg Asn Pro Gln Gln His His Tyr His
        35                  40                  45
Gln Gln Gln Gln Ser Gln His Ser His Arg Asn Ser Ser Tyr Gln Gln
    50                  55                  60
Gln Trp Leu Arg Arg Asp Gln Ala Pro Ala Val Ala Gly Ala Ala Ser
65                  70                  75                  80
Gly Asn Ala Val Ala Lys Thr Ala Ser Gln Leu Asp Ala Val Gly Ser
                85                  90                  95
Ser Ser Gln Asp Trp Lys Ala Gln Leu Asn Ile Pro Ala Pro Asp Thr
            100                 105                 110
Arg Tyr Arg Thr Glu Asp Val Thr Ala Thr Lys Gly Asn Glu Phe Glu
        115                 120                 125
Asp Tyr Phe Leu Lys Arg Glu Leu Leu Met Gly Ile Tyr Glu Lys Gly
    130                 135                 140
Phe Glu Arg Pro Ser Pro Ile Gln Glu Glu Ser Ile Pro Ile Ala Leu
145                 150                 155                 160
Thr Gly Ser Asp Ile Leu Ala Arg Ala Lys Asn Gly Thr Gly Lys Thr
                165                 170                 175
Ala Ala Phe Cys Ile Pro Ala Leu Glu Lys Ile Asp Pro Glu Asn Asn
            180                 185                 190
Ala Ile Gln Val Val Ile Leu Val Pro Thr Arg Glu Leu Ala Leu Gln
        195                 200                 205
Thr Ser Gln Val Cys Lys Glu Leu Gly Lys Tyr Leu Asn Ile Glu Val
    210                 215                 220
Met Val Ser Thr Gly Gly Thr Ser Leu Lys Asp Asp Ile Met Arg Leu
225                 230                 235                 240
Tyr Gln Pro Val His Leu Leu Val Gly Thr Pro Gly Arg Ile Leu Asp
                245                 250                 255
Leu Thr Arg Lys Gly Ile Cys Val Leu Lys Asp Cys Ser Met Leu Val
            260                 265                 270
Met Asp Glu Ala Asp Lys Leu Leu Ala Pro Glu Phe Gln Pro Ser Val
        275                 280                 285
Glu Ala Leu Ile His Phe Leu Pro Pro Ser Arg Gln Leu Leu Met Phe
    290                 295                 300
Ser Ala Thr Phe Pro Val Thr Val Lys Glu Phe Lys Glu Lys Tyr Leu
305                 310                 315                 320
Pro Arg Pro Tyr Val Ile Asn Leu Met Asp Glu Leu Thr Leu Lys Gly
                325                 330                 335
Ile Thr Gln Tyr Tyr Ala Phe Val Glu Glu Arg Gln Lys Val His Cys
            340                 345                 350
Leu Asn Thr Leu Phe Ser Lys Leu Gln Ile Asn Gln Ser Ile Ile Phe
        355                 360                 365
Cys Asn Ser Val Asn Arg Val Glu Leu Leu Ala Lys Lys Ile Thr Glu
    370                 375                 380
Leu Gly Tyr Ser Cys Phe Tyr Ile His Ala Lys Met Leu Gln Asp His
385                 390                 395                 400
Arg Asn Arg Val Phe His Asp Phe Arg Asn Gly Ala Cys Arg Asn Leu
                405                 410                 415
Val Cys Thr Asp Leu Phe Thr Arg Gly Ile Asp Ile Gln Ala Val Asn
            420                 425                 430
Val Val Ile Asn Phe Asp Phe Pro Lys Thr Ser Glu Thr Tyr Leu His
        435                 440                 445
Arg Val Gly Arg Ser Gly Arg Tyr Gly His Leu Gly Leu Ala Val Asn
    450                 455                 460
Leu Ile Thr Tyr Glu Asp Arg Phe Asn Leu Tyr Arg Ile Glu Gln Glu
465                 470                 475                 480
Leu Gly Thr Glu Ile Lys Thr Ile Pro Pro Gln Ile Asp Leu Ser Ile
                485                 490                 495
Tyr Cys Gln

<210> 43
<211> 1578
<212> DNA

<213> Zea mays

<220>
<221> CDS
<222> (1)..(1578)

<400> 43
atg gat cca cgc gcc agg tat cct tcg ggc atg ggc aac ggc cgc ggt 48
Met Asp Pro Arg Ala Arg Tyr Pro Ser Gly Met Gly Asn Gly Arg Gly
1               5                   10                  15
ggg aac ctc aac tac tac ggc cgc ggc cca ccg ccg caa cag ccg cat 96
Gly Asn Leu Asn Tyr Tyr Gly Arg Gly Pro Pro Pro Gln Gln Pro His
            20                  25                  30
cag cac cac aac cac cag cag acg tcg ggg gcg cac cac cac cag cag 144
Gln His His Asn His Gln Gln Thr Ser Gly Ala His His His Gln Gln
        35                  40                  45
acg tcg ggg gcg cac cac cac cag cag tac gcg cag agg cag cac cag 192
Thr Ser Gly Ala His His His Gln Gln Tyr Ala Gln Arg Gln His Gln
    50                  55                  60
cac cag cac cac ggc cac aat cgc cat cag caa cag cag cag cac cac 240
His Gln His His Gly His Asn Arg His Gln Gln Gln Gln Gln His His
65                  70                  75                  80
att tac cag cag caa cag cag cag tgg ctg cgg cgg agc cag att gcc 288
Ile Tyr Gln Gln Gln Gln Gln Gln Trp Leu Arg Arg Ser Gln Ile Ala
                85                  90                  95
cgc gag acc tcg ggc gca gcc gtg acc agc gag ccg aag tcc ctg gcg 336
Arg Glu Thr Ser Gly Ala Ala Val Thr Ser Glu Pro Lys Ser Leu Ala
            100                 105                 110
cca tcc acg gcc gcc gat ggc gtc gat tcg agt tca caa gat tgg aag 384
Pro Ser Thr Ala Ala Asp Gly Val Asp Ser Ser Ser Gln Asp Trp Lys
        115                 120                 125
gcc caa ttg aag ctg ccg cct cca gat aca cgc tac agg aca gag gat 432
Ala Gln Leu Lys Leu Pro Pro Pro Asp Thr Arg Tyr Arg Thr Glu Asp
    130                 135                 140
gtt aca gct acc aag gga aat gag ttt gaa gac tat ttt ctc aag cgt 480
Val Thr Ala Thr Lys Gly Asn Glu Phe Glu Asp Tyr Phe Leu Lys Arg
145                 150                 155                 160
gaa ctc cta atg ggt ata tac gag aag gga ttt gaa aga ccg tct cct 528
Glu Leu Leu Met Gly Ile Tyr Glu Lys Gly Phe Glu Arg Pro Ser Pro
                165                 170                 175
atc caa gag gaa agc att cct att gct tta act ggt agt gac att ctt 576
Ile Gln Glu Glu Ser Ile Pro Ile Ala Leu Thr Gly Ser Asp Ile Leu
            180                 185                 190
gca aga gcg aag aac gga act ggc aag act gct gca ttt tgt att cct 624
Ala Arg Ala Lys Asn Gly Thr Gly Lys Thr Ala Ala Phe Cys Ile Pro
        195                 200                 205
gca ctg gaa aag att gat caa gaa aaa aat gct att caa gtt gtt ata 672
Ala Leu Glu Lys Ile Asp Gln Glu Lys Asn Ala Ile Gln Val Val Ile
    210                 215                 220
ttg gtt ccc aca agg gaa ttg gct ctg caa aca tca caa gtt tgc aag 720
Leu Val Pro Thr Arg Glu Leu Ala Leu Gln Thr Ser Gln Val Cys Lys
225                 230                 235                 240
gag ctt ggt aag cat ttg aag atc cag gtt atg gtc acc act ggt gga 768
Glu Leu Gly Lys His Leu Lys Ile Gln Val Met Val Thr Thr Gly Gly
                245                 250                 255
acc agt ttg aag gat gat att gtt cgt ctg tac caa cct gtg cac tta 816
Thr Ser Leu Lys Asp Asp Ile Val Arg Leu Tyr Gln Pro Val His Leu
            260                 265                 270
ctc gtt gga aca cct ggg cgt att ttg gat ctt aca aag aaa ggt gtt 864
Leu Val Gly Thr Pro Gly Arg Ile Leu Asp Leu Thr Lys Lys Gly Val
        275                 280                 285
tgc att ttg aac gac tgt tca atg ctt att atg gat gag gca gac aag 912
Cys Ile Leu Asn Asp Cys Ser Met Leu Ile Met Asp Glu Ala Asp Lys
    290                 295                 300
ctt ctc tct cct gag ttt caa cct tcc ata gaa cag ttg att cgc tac 960
Leu Leu Ser Pro Glu Phe Gln Pro Ser Ile Glu Gln Leu Ile Arg Tyr
305                 310                 315                 320
ctc cct gct agt cga cag att ttg atg ttt tca gcg aca ttc cct gtg 1008
Leu Pro Ala Ser Arg Gln Ile Leu Met Phe Ser Ala Thr Phe Pro Val

325                 330                 335
act gtt aag gag ttt aag gac aaa tat ctg cct aaa cct tat gtg att      1056
Thr Val Lys Glu Phe Lys Asp Lys Tyr Leu Pro Lys Pro Tyr Val Ile
            340                 345                 350
aac ctt atg gac gaa ctt aca ctg aag gga att aca cag ttc tat gct      1104
Asn Leu Met Asp Glu Leu Thr Leu Lys Gly Ile Thr Gln Phe Tyr Ala
            355                 360                 365
ttt gtg gaa gaa aga cag aaa gtt cac tgt ctc aac act ctc ttt tcc      1152
Phe Val Glu Glu Arg Gln Lys Val His Cys Leu Asn Thr Leu Phe Ser
    370                 375                 380
aag ctt caa atc aac cag tca ata ata ttt tgt aac tct gta aat cgt      1200
Lys Leu Gln Ile Asn Gln Ser Ile Ile Phe Cys Asn Ser Val Asn Arg
385                 390                 395                 400
gtt gaa cta ttg gcg aaa aag atc aca gag ctt ggc tat tct tgc ttc      1248
Val Glu Leu Leu Ala Lys Lys Ile Thr Glu Leu Gly Tyr Ser Cys Phe
                405                 410                 415
tat att cat gca aag atg cta cag gac cac cgt aat aga gtt ttc cat      1296
Tyr Ile His Ala Lys Met Leu Gln Asp His Arg Asn Arg Val Phe His
            420                 425                 430
gat ttc cgt aat gga gca tgc agg aac ctt gtt tgc act gat ctt ttt      1344
Asp Phe Arg Asn Gly Ala Cys Arg Asn Leu Val Cys Thr Asp Leu Phe
        435                 440                 445
aca agg gga ata gat att caa gct gtg aat gtt gtt atc aat ttt gat      1392
Thr Arg Gly Ile Asp Ile Gln Ala Val Asn Val Val Ile Asn Phe Asp
    450                 455                 460
ttc ccc aag aac tct gaa aca tat ctc cac agg gtt ggt aga tct ggg      1440
Phe Pro Lys Asn Ser Glu Thr Tyr Leu His Arg Val Gly Arg Ser Gly
465                 470                 475                 480
aga ttt ggt cac ctt ggt ctg gcc gtt aat ttg atc acc tat gag gat      1488
Arg Phe Gly His Leu Gly Leu Ala Val Asn Leu Ile Thr Tyr Glu Asp
                485                 490                 495
cgg ttt aac ttg tat agg att gaa caa gag ctt ggg act gaa ata aaa      1536
Arg Phe Asn Leu Tyr Arg Ile Glu Gln Glu Leu Gly Thr Glu Ile Lys
            500                 505                 510
tca att cct cct cag ata gac cag gct ata tac tgc caa taa              1578
Ser Ile Pro Pro Gln Ile Asp Gln Ala Ile Tyr Cys Gln
        515                 520                 525

<210> 44
<211> 525
<212> PRT
<213> Zea mays

<400> 44
   Met Asp Pro Arg Ala Arg Tyr Pro Ser Gly Met Gly Asn Gly Arg Gly
   1               5                   10                  15
   Gly Asn Leu Asn Tyr Tyr Gly Arg Gly Pro Pro Pro Gln Gln Pro His
               20                  25                  30
   Gln His His Asn His Gln Gln Thr Ser Gly Ala His His His Gln Gln
           35                  40                  45
   Thr Ser Gly Ala His His His Gln Gln Tyr Ala Gln Arg Gln His Gln
       50                  55                  60
   His Gln His His Gly His Asn Arg His Gln Gln Gln Gln Gln His His
   65                  70                  75                  80
   Ile Tyr Gln Gln Gln Gln Gln Gln Trp Leu Arg Arg Ser Gln Ile Ala
                   85                  90                  95
   Arg Glu Thr Ser Gly Ala Ala Val Thr Ser Glu Pro Lys Ser Leu Ala
               100                 105                 110
   Pro Ser Thr Ala Ala Asp Gly Val Asp Ser Ser Ser Gln Asp Trp Lys
           115                 120                 125
   Ala Gln Leu Lys Leu Pro Pro Pro Asp Thr Arg Tyr Arg Thr Glu Asp
       130                 135                 140
   Val Thr Ala Thr Lys Gly Asn Glu Phe Glu Asp Tyr Phe Leu Lys Arg
   145                 150                 155                 160
   Glu Leu Leu Met Gly Ile Tyr Glu Lys Gly Phe Glu Arg Pro Ser Pro
                   165                 170                 175
   Ile Gln Glu Glu Ser Ile Pro Ile Ala Leu Thr Gly Ser Asp Ile Leu
               180                 185                 190
   Ala Arg Ala Lys Asn Gly Thr Gly Lys Thr Ala Ala Phe Cys Ile Pro
   195                 200                 205
   Ala Leu Glu Lys Ile Asp Gln Glu Lys Asn Ala Ile Gln Val Val Ile
       210                 215                 220
   Leu Val Pro Thr Arg Glu Leu Ala Leu Gln Thr Ser Gln Val Cys Lys
   225                 230                 235                 240
   Glu Leu Gly Lys His Leu Lys Ile Gln Val Met Val Thr Thr Gly Gly
                   245                 250                 255
   Thr Ser Leu Lys Asp Asp Ile Val Arg Leu Tyr Gln Pro Val His Leu
               260                 265                 270
   Leu Val Gly Thr Pro Gly Arg Ile Leu Asp Leu Thr Lys Lys Gly Val
           275                 280                 285
   Cys Ile Leu Asn Asp Cys Ser Met Leu Ile Met Asp Glu Ala Asp Lys
       290                 295                 300
   Leu Leu Ser Pro Glu Phe Gln Pro Ser Ile Glu Gln Leu Ile Arg Tyr
   305                 310                 315                 320
   Leu Pro Ala Ser Arg Gln Ile Leu Met Phe Ser Ala Thr Phe Pro Val
                   325                 330                 335
   Thr Val Lys Glu Phe Lys Asp Lys Tyr Leu Pro Lys Pro Tyr Val Ile
               340                 345                 350
   Asn Leu Met Asp Glu Leu Thr Leu Lys Gly Ile Thr Gln Phe Tyr Ala
           355                 360                 365
   Phe Val Glu Glu Arg Gln Lys Val His Cys Leu Asn Thr Leu Phe Ser
       370                 375                 380
   Lys Leu Gln Ile Asn Gln Ser Ile Ile Phe Cys Asn Ser Val Asn Arg
   385                 390                 395                 400
   Val Glu Leu Leu Ala Lys Lys Ile Thr Glu Leu Gly Tyr Ser Cys Phe
                   405                 410                 415
   Tyr Ile His Ala Lys Met Leu Gln Asp His Arg Asn Arg Val Phe His
               420                 425                 430
   Asp Phe Arg Asn Gly Ala Cys Arg Asn Leu Val Cys Thr Asp Leu Phe
           435                 440                 445
   Thr Arg Gly Ile Asp Ile Gln Ala Val Asn Val Val Ile Asn Phe Asp
       450                 455                 460
   Phe Pro Lys Asn Ser Glu Thr Tyr Leu His Arg Val Gly Arg Ser Gly
   465                 470                 475                 480
   Arg Phe Gly His Leu Gly Leu Ala Val Asn Leu Ile Thr Tyr Glu Asp
                   485                 490                 495
   Arg Phe Asn Leu Tyr Arg Ile Glu Gln Glu Leu Gly Thr Glu Ile Lys
               500                 505                 510
   Ser Ile Pro Pro Gln Ile Asp Gln Ala Ile Tyr Cys Gln
           515                 520                 525

<210> 45
<211> 2301
<212> DNA
<213> Zea mays

<220>
<221> CDS
<222> (1)..(2301)

<400> 45
atg tcg aag cgg ccg aag ctc gag ggt ttc agc atc ccg cgg ccc acc      48
Met Ser Lys Arg Pro Lys Leu Glu Gly Phe Ser Ile Pro Arg Pro Thr
1               5                   10                  15
tcc tac aac ttc gag cgc tcg cag tcc gtg cag cgg ctc tac cgt ccg      96
Ser Tyr Asn Phe Glu Arg Ser Gln Ser Val Gln Arg Leu Tyr Arg Pro
            20                  25                  30
acc gat gac cca gac ctc gac gac atc gcc ttc tcc gac gac gcc ccc      144
Thr Asp Asp Pro Asp Leu Asp Asp Ile Ala Phe Ser Asp Asp Ala Pro
        35                  40                  45
tca gat gcc caa gcc agc acc gcg gta gaa gga aag gcc gag aat gag      192
Ser Asp Ala Gln Ala Ser Thr Ala Val Glu Gly Lys Ala Glu Asn Glu
    50                  55                  60
gag gag atg gac ccg ctc gac gcc ttc atg gcc gag atc cag gag gag      240
Glu Glu Met Asp Pro Leu Asp Ala Phe Met Ala Glu Ile Gln Glu Glu
65                  70                  75                  80
atc cgt gcg ccg ccc ccg ccg ccc aag ccc gag gcg ctt cgc cgc acg      288
Ile Arg Ala Pro Pro Pro Pro Pro Lys Pro Glu Ala Leu Arg Arg Thr

85                  90                  95
gat tcc gac gat gac gag gat gac ccc gtc gag agc ttc ctg cgg gcc          336
Asp Ser Asp Asp Asp Glu Asp Asp Pro Val Glu Ser Phe Leu Arg Ala
            100                 105                 110
aag aag gac gct ggc ctc acg ctg gcc gcc gac gtc atg cgc gct ggt          384
Lys Lys Asp Ala Gly Leu Thr Leu Ala Ala Asp Val Met Arg Ala Gly
        115                 120                 125
tac gac tcc gat gag gag gtc tac gcc gct gcc aag gcc gtc gac gcc          432
Tyr Asp Ser Asp Glu Glu Val Tyr Ala Ala Ala Lys Ala Val Asp Ala
    130                 135                 140
ggc atg atg gaa tac gac tct gat gac aac ccc ata gtc atc gac aag          480
Gly Met Met Glu Tyr Asp Ser Asp Asp Asn Pro Ile Val Ile Asp Lys
145                 150                 155                 160
aag aaa ata gag ccc ata cca gca ctc gat cac tcg acc att gag tat          528
Lys Lys Ile Glu Pro Ile Pro Ala Leu Asp His Ser Thr Ile Glu Tyr
                165                 170                 175
gac gcc ttc aca aaa gat ttc tac gag gag aag cca tcg att tca ggt          576
Asp Ala Phe Thr Lys Asp Phe Tyr Glu Glu Lys Pro Ser Ile Ser Gly
            180                 185                 190
atg agt gac caa gag gta aca gat tat atg aaa agt ttg tca att cgt          624
Met Ser Asp Gln Glu Val Thr Asp Tyr Met Lys Ser Leu Ser Ile Arg
        195                 200                 205
gtt tct ggt ttt gat gtg ccg agg cca ata aaa aac ttt gag gat tgt          672
Val Ser Gly Phe Asp Val Pro Arg Pro Ile Lys Asn Phe Glu Asp Cys
    210                 215                 220
ggg ttt cat gta cct cta atg aat gct att gcc aaa caa gct tat gaa          720
Gly Phe His Val Pro Leu Met Asn Ala Ile Ala Lys Gln Ala Tyr Glu
225                 230                 235                 240
aaa cca act aca att cag tgc cag gct tta cct att gta ctt tca ggg          768
Lys Pro Thr Thr Ile Gln Cys Gln Ala Leu Pro Ile Val Leu Ser Gly
                245                 250                 255
aga gat atc att ggt att gca aaa act ggc tct ggt aaa act gca gct          816
Arg Asp Ile Ile Gly Ile Ala Lys Thr Gly Ser Gly Lys Thr Ala Ala
            260                 265                 270
ttt gtg ctc cct atg att gtt cat att atg gat cag cct gag ctt gag          864
Phe Val Leu Pro Met Ile Val His Ile Met Asp Gln Pro Glu Leu Glu
        275                 280                 285
aaa gaa gaa ggt cca ata gga gtc att tgt gct cca aca aga gaa ttg          912
Lys Glu Glu Gly Pro Ile Gly Val Ile Cys Ala Pro Thr Arg Glu Leu
    290                 295                 300
gca cat cag ata tat ctc gaa gct aag aag ttt gca aag cct tac aac          960
Ala His Gln Ile Tyr Leu Glu Ala Lys Lys Phe Ala Lys Pro Tyr Asn
305                 310                 315                 320
ctt cga gtt gct gct ata tat ggt ggt gtt tcc aaa ttt gac cag ttt          1008
Leu Arg Val Ala Ala Ile Tyr Gly Gly Val Ser Lys Phe Asp Gln Phe
                325                 330                 335
aaa gaa ctg aaa gca ggt tgt gaa ata gtc att gca act cca ggg aga          1056
Lys Glu Leu Lys Ala Gly Cys Glu Ile Val Ile Ala Thr Pro Gly Arg
            340                 345                 350
tta ata gac ttg ctg aag atg aag gca ttg aag atg ttc agg gca act          1104
Leu Ile Asp Leu Leu Lys Met Lys Ala Leu Lys Met Phe Arg Ala Thr
        355                 360                 365
tat ctg gtt ctt gat gaa gct gat cga atg ttc gat ctt gga ttt gag          1152
Tyr Leu Val Leu Asp Glu Ala Asp Arg Met Phe Asp Leu Gly Phe Glu
    370                 375                 380
cca caa ata cga tcc att gtt ggt caa att aga cca gat cga caa act          1200
Pro Gln Ile Arg Ser Ile Val Gly Gln Ile Arg Pro Asp Arg Gln Thr
385                 390                 395                 400
tta ctt ttt tct gca aca atg cca tac aaa gta gag cgt ttg gca aga          1248
Leu Leu Phe Ser Ala Thr Met Pro Tyr Lys Val Glu Arg Leu Ala Arg
                405                 410                 415
gaa ata tta act gat cct att aga gtt aca gtt ggt caa gtt ggc ggt          1296
Glu Ile Leu Thr Asp Pro Ile Arg Val Thr Val Gly Gln Val Gly Gly
            420                 425                 430
gct aat gaa gac att aaa caa gtt gtt aat gta ctc cct tct gat gtt          1344
Ala Asn Glu Asp Ile Lys Gln Val Val Asn Val Leu Pro Ser Asp Val
        435                 440                 445
gag aaa atg cct tgg ctt ttg gag aaa ttg cct gga atg att gat gat          1392
Glu Lys Met Pro Trp Leu Leu Glu Lys Leu Pro Gly Met Ile Asp Asp

450                 455                 460
gga gat gtt ctt gta ttc gca tct aaa aag gct aga gtg gac gag ata        1440
Gly Asp Val Leu Val Phe Ala Ser Lys Lys Ala Arg Val Asp Glu Ile
465                 470                 475                 480
gag aaa gag ttg aat cag aga gga ttc aga att gct gcg ctt cat ggc        1488
Glu Lys Glu Leu Asn Gln Arg Gly Phe Arg Ile Ala Ala Leu His Gly
                485                 490                 495
gac aag gat caa gct tct cgt atg gag acc ctg cag aag ttc aaa tct        1536
Asp Lys Asp Gln Ala Ser Arg Met Glu Thr Leu Gln Lys Phe Lys Ser
            500                 505                 510
ggg aca ttt cat gtt ctg gtt gca act gat gtt gct gca cga ggt ctt        1584
Gly Thr Phe His Val Leu Val Ala Thr Asp Val Ala Ala Arg Gly Leu
        515                 520                 525
gac atc aaa tcg att aaa aca gtt gtc aac ttt gat att gca aaa gag        1632
Asp Ile Lys Ser Ile Lys Thr Val Val Asn Phe Asp Ile Ala Lys Glu
    530                 535                 540
atg gat atg cat att cac cga att gga aga act ggt cgt gct ggc gat        1680
Met Asp Met His Ile His Arg Ile Gly Arg Thr Gly Arg Ala Gly Asp
545                 550                 555                 560
aaa gat ggc act gca tat act ctg att aca cag aag gag gca cat ttt        1728
Lys Asp Gly Thr Ala Tyr Thr Leu Ile Thr Gln Lys Glu Ala His Phe
                565                 570                 575
gct ggt gaa ctg gtt cac agt ttg att gct gct ggc cag gat gta ccc        1776
Ala Gly Glu Leu Val His Ser Leu Ile Ala Ala Gly Gln Asp Val Pro
            580                 585                 590
aat gaa ctc atg gat ctg gct atg aag gat gga aga ttc aga gca aaa        1824
Asn Glu Leu Met Asp Leu Ala Met Lys Asp Gly Arg Phe Arg Ala Lys
        595                 600                 605
cga gac tcc agg aaa ggt ggg aag aaa ggt ggc aaa gga aaa ggt ggt        1872
Arg Asp Ser Arg Lys Gly Gly Lys Lys Gly Gly Lys Gly Lys Gly Gly
    610                 615                 620
gtt ggc ggt gct ggg cgc ggg cgc ggt gtg cgt gga gtt gat ttt ggc        1920
Val Gly Gly Ala Gly Arg Gly Arg Gly Val Arg Gly Val Asp Phe Gly
625                 630                 635                 640
ctc ggc ata ggt tac aat gcc gaa tct ggt tca caa gtg cct gct gca        1968
Leu Gly Ile Gly Tyr Asn Ala Glu Ser Gly Ser Gln Val Pro Ala Ala
                645                 650                 655
aga tct gct gct gta aat tct ctg aag aca ggg atg ttg caa cga ttt        2016
Arg Ser Ala Ala Val Asn Ser Leu Lys Thr Gly Met Leu Gln Arg Phe
            660                 665                 670
aag agc agc ttt gtc tct gga tct tca aat aca cca agc agc agt gca        2064
Lys Ser Ser Phe Val Ser Gly Ser Ser Asn Thr Pro Ser Ser Ser Ala
        675                 680                 685
cct tcc ttt gta aga cca gca ctc cgt ggc ttt gtg tct ggc ggc acc        2112
Pro Ser Phe Val Arg Pro Ala Leu Arg Gly Phe Val Ser Gly Gly Thr
    690                 695                 700
att gga gga gac aca cgg cct gca cag gca gcc ccc act ttc atc cct        2160
Ile Gly Gly Asp Thr Arg Pro Ala Gln Ala Ala Pro Thr Phe Ile Pro
705                 710                 715                 720
gca tcc cgg cct gca cag cct gcc ccc gcg gtc cct gca tcc cag cct        2208
Ala Ser Arg Pro Ala Gln Pro Ala Pro Ala Val Pro Ala Ser Gln Pro
                725                 730                 735
gca gga aat aac aac gaa aat ggg aac ccg aat cca gaa agt tca cgg        2256
Ala Gly Asn Asn Asn Glu Asn Gly Asn Pro Asn Pro Glu Ser Ser Arg
            740                 745                 750
gat cag ttc agg gag aga aaa cga cca tca ggt tgg gat cgc tag            2301
Asp Gln Phe Arg Glu Arg Lys Arg Pro Ser Gly Trp Asp Arg
        755                 760                 765

<210> 46
<211> 766
<212> PRT
<213> Zea mays

<400> 46
Met Ser Lys Arg Pro Lys Leu Glu Gly Phe Ser Ile Pro Arg Pro Thr
1               5                   10                  15
Ser Tyr Asn Phe Glu Arg Ser Gln Ser Val Gln Arg Leu Tyr Arg Pro
            20                  25                  30

Thr Asp Asp Pro Asp Leu Asp Asp Ile Ala Phe Ser Asp Asp Ala Pro
            35                  40                  45
Ser Asp Ala Gln Ala Ser Thr Ala Val Glu Gly Lys Ala Glu Asn Glu
    50                  55                  60
Glu Glu Met Asp Pro Leu Asp Ala Phe Met Ala Glu Ile Gln Glu Glu
65                  70                  75                  80
Ile Arg Ala Pro Pro Pro Pro Pro Lys Pro Glu Ala Leu Arg Arg Thr
                85                  90                  95
Asp Ser Asp Asp Asp Glu Asp Asp Pro Val Glu Ser Phe Leu Arg Ala
            100                 105                 110
Lys Lys Asp Ala Gly Leu Thr Leu Ala Ala Asp Val Met Arg Ala Gly
        115                 120                 125
Tyr Asp Ser Asp Glu Glu Val Tyr Ala Ala Ala Lys Ala Val Asp Ala
    130                 135                 140
Gly Met Met Glu Tyr Asp Ser Asp Asp Asn Pro Ile Val Ile Asp Lys
145                 150                 155                 160
Lys Lys Ile Glu Pro Ile Pro Ala Leu Asp His Ser Thr Ile Glu Tyr
                165                 170                 175
Asp Ala Phe Thr Lys Asp Phe Tyr Glu Glu Lys Pro Ser Ile Ser Gly
            180                 185                 190
Met Ser Asp Gln Glu Val Thr Asp Tyr Met Lys Ser Leu Ser Ile Arg
        195                 200                 205
Val Ser Gly Phe Asp Val Pro Arg Pro Ile Lys Asn Phe Glu Asp Cys
    210                 215                 220
Gly Phe His Val Pro Leu Met Asn Ala Ile Ala Lys Gln Ala Tyr Glu
225                 230                 235                 240
Lys Pro Thr Thr Ile Gln Cys Gln Ala Leu Pro Ile Val Leu Ser Gly
                245                 250                 255
Arg Asp Ile Ile Gly Ile Ala Lys Thr Gly Ser Gly Lys Thr Ala Ala
            260                 265                 270
Phe Val Leu Pro Met Ile Val His Ile Met Asp Gln Pro Glu Leu Glu
        275                 280                 285
Lys Glu Glu Gly Pro Ile Gly Val Ile Cys Ala Pro Thr Arg Glu Leu
    290                 295                 300
Ala His Gln Ile Tyr Leu Glu Ala Lys Lys Phe Ala Lys Pro Tyr Asn
305                 310                 315                 320
Leu Arg Val Ala Ala Ile Tyr Gly Gly Val Ser Lys Phe Asp Gln Phe
                325                 330                 335
Lys Glu Leu Lys Ala Gly Cys Glu Ile Val Ile Ala Thr Pro Gly Arg
            340                 345                 350
Leu Ile Asp Leu Leu Lys Met Lys Ala Leu Lys Met Phe Arg Ala Thr
        355                 360                 365
Tyr Leu Val Leu Asp Glu Ala Asp Arg Met Phe Asp Leu Gly Phe Glu
    370                 375                 380
Pro Gln Ile Arg Ser Ile Val Gly Gln Ile Arg Pro Asp Arg Gln Thr
385                 390                 395                 400
Leu Leu Phe Ser Ala Thr Met Pro Tyr Lys Val Glu Arg Leu Ala Arg
                405                 410                 415
Glu Ile Leu Thr Asp Pro Ile Arg Val Thr Val Gly Gln Val Gly Gly
            420                 425                 430
Ala Asn Glu Asp Ile Lys Gln Val Val Asn Val Leu Pro Ser Asp Val
        435                 440                 445
Glu Lys Met Pro Trp Leu Leu Glu Lys Leu Pro Gly Met Ile Asp Asp
    450                 455                 460
Gly Asp Val Leu Val Phe Ala Ser Lys Lys Ala Arg Val Asp Glu Ile
465                 470                 475                 480
Glu Lys Glu Leu Asn Gln Arg Gly Phe Arg Ile Ala Ala Leu His Gly
                485                 490                 495
Asp Lys Asp Gln Ala Ser Arg Met Glu Thr Leu Gln Lys Phe Lys Ser
            500                 505                 510
Gly Thr Phe His Val Leu Val Ala Thr Asp Val Ala Ala Arg Gly Leu
        515                 520                 525
Asp Ile Lys Ser Ile Lys Thr Val Val Asn Phe Asp Ile Ala Lys Glu
    530                 535                 540
Met Asp Met His Ile His Arg Ile Gly Arg Thr Gly Arg Ala Gly Asp
545                 550                 555                 560
Lys Asp Gly Thr Ala Tyr Thr Leu Ile Thr Gln Lys Glu Ala His Phe
                565                 570                 575
Ala Gly Glu Leu Val His Ser Leu Ile Ala Ala Gly Gln Asp Val Pro

                 580                     585                     590
Asn Glu Leu Met Asp Leu Ala Met Lys Asp Gly Arg Phe Arg Ala Lys
            595                     600                     605
Arg Asp Ser Arg Lys Gly Gly Lys Lys Gly Gly Lys Gly Lys Gly Gly
        610                     615                     620
Val Gly Gly Ala Gly Arg Gly Arg Gly Val Arg Gly Val Asp Phe Gly
625                     630                     635                     640
Leu Gly Ile Gly Tyr Asn Ala Glu Ser Gly Ser Gln Val Pro Ala Ala
                645                     650                     655
Arg Ser Ala Ala Val Asn Ser Leu Lys Thr Gly Met Leu Gln Arg Phe
            660                     665                     670
Lys Ser Ser Phe Val Ser Gly Ser Ser Asn Thr Pro Ser Ser Ser Ala
        675                     680                     685
Pro Ser Phe Val Arg Pro Ala Leu Arg Gly Phe Val Ser Gly Gly Thr
    690                     695                     700
Ile Gly Gly Asp Thr Arg Pro Ala Gln Ala Ala Pro Thr Phe Ile Pro
705                     710                     715                     720
Ala Ser Arg Pro Ala Gln Pro Ala Pro Ala Val Pro Ala Ser Gln Pro
                725                     730                     735
Ala Gly Asn Asn Asn Glu Asn Gly Asn Pro Asn Pro Glu Ser Ser Arg
            740                     745                     750
Asp Gln Phe Arg Glu Arg Lys Arg Pro Ser Gly Trp Asp Arg
        755                     760                     765

<210> 47
<211> 1422
<212> DNA
<213> Zea mays

<220>
<221> CDS
<222> (1)..(1422)

<400> 47
atg aac ccg tat gat ctc cgc ttc gct gac ccc tca tcc tac cac gac       48
Met Asn Pro Tyr Asp Leu Arg Phe Ala Asp Pro Ser Ser Tyr His Asp
1               5                   10                  15
agg cgc agc gac ttg acc gtg gct cct acg ttc gct ccg ccg gct ccc       96
Arg Arg Ser Asp Leu Thr Val Ala Pro Thr Phe Ala Pro Pro Ala Pro
            20                  25                  30
gtg gct gcg gcg aac ctg aac cct tac gcc gcc gcc tac ccg ctg cct      144
Val Ala Ala Ala Asn Leu Asn Pro Tyr Ala Ala Ala Tyr Pro Leu Pro
        35                  40                  45
gtg ccc gtt ccc gtg ccc gcg gcc ccc gcg ggc ggc gac tac cag cgc      192
Val Pro Val Pro Val Pro Ala Ala Pro Ala Gly Gly Asp Tyr Gln Arg
    50                  55                  60
tac ggg cag gga ggc aga ggc cgt ggt ggc ggt ggc aga ggc ggt gga      240
Tyr Gly Gln Gly Gly Arg Gly Arg Gly Gly Gly Gly Arg Gly Gly Gly
65                  70                  75                  80
gga ggg tac gga ggt ggc ggc aga gga gga ggt cgc ggg agg gac ggg      288
Gly Gly Tyr Gly Gly Gly Gly Arg Gly Gly Gly Arg Gly Arg Asp Gly
                85                  90                  95
ctt gac tcg ctc gcc ctc cct aag ccg gac ttt cgg agc ctg ata ccg      336
Leu Asp Ser Leu Ala Leu Pro Lys Pro Asp Phe Arg Ser Leu Ile Pro
            100                 105                 110
ttc gag aaa aac ttc tac gtg gag tgt ccc tcc gtc cag gca atg tcg      384
Phe Glu Lys Asn Phe Tyr Val Glu Cys Pro Ser Val Gln Ala Met Ser
        115                 120                 125
gag gcg gat gtg gcg cag tac cgc cgc ctc cgg gat atc acc atc gag      432
Glu Ala Asp Val Ala Gln Tyr Arg Arg Leu Arg Asp Ile Thr Ile Glu
    130                 135                 140
ggc cgg gat gtg ccc aag ccg gtt aga tac ttc cag gaa gcc aac ttc      480
Gly Arg Asp Val Pro Lys Pro Val Arg Tyr Phe Gln Glu Ala Asn Phe
145                 150                 155                 160
cca gat tac tgc atg caa gct att gcc aaa tct gga ttt gtg gag cca      528
Pro Asp Tyr Cys Met Gln Ala Ile Ala Lys Ser Gly Phe Val Glu Pro
                165                 170                 175
aca cct att caa tct caa ggt tgg cct atg gct ttg aag ggt agg gac      576
Thr Pro Ile Gln Ser Gln Gly Trp Pro Met Ala Leu Lys Gly Arg Asp

                180                 185                 190
ctg att ggt atc gct caa aca ggc tca ggg aaa aca tta tct tat ctg         624
Leu Ile Gly Ile Ala Gln Thr Gly Ser Gly Lys Thr Leu Ser Tyr Leu
        195                 200                 205
tta cct ggg ttg gtt cat gtt ggt gcc cag cct cgg cta gaa caa ggc         672
Leu Pro Gly Leu Val His Val Gly Ala Gln Pro Arg Leu Glu Gln Gly
    210                 215                 220
gat ggc cca att gta tta att ctc gct ccc acg aga gaa ctg gct gtt         720
Asp Gly Pro Ile Val Leu Ile Leu Ala Pro Thr Arg Glu Leu Ala Val
225                 230                 235                 240
caa ata cag gaa gaa tct aca aaa ttt gga tca tat tca aga act aga         768
Gln Ile Gln Glu Glu Ser Thr Lys Phe Gly Ser Tyr Ser Arg Thr Arg
                245                 250                 255
agc aca tgt gtc tat ggt ggt gca cct aag ggt cca caa ata cgg gac         816
Ser Thr Cys Val Tyr Gly Gly Ala Pro Lys Gly Pro Gln Ile Arg Asp
            260                 265                 270
ctt aga aga gga gtt gaa att gtc att gca act cct ggc cgg ttg att         864
Leu Arg Arg Gly Val Glu Ile Val Ile Ala Thr Pro Gly Arg Leu Ile
        275                 280                 285
gat atg ctg gaa gct ggt cac aca aac cta cgt aga gtg aca tac ctt         912
Asp Met Leu Glu Ala Gly His Thr Asn Leu Arg Arg Val Thr Tyr Leu
    290                 295                 300
gtg ttg gat gag gcg gac cgg atg cta gat atg ggg ttt gag ccc caa         960
Val Leu Asp Glu Ala Asp Arg Met Leu Asp Met Gly Phe Glu Pro Gln
305                 310                 315                 320
att cgg aaa ata gta gca cag atc cgc cca gac agg caa acc ttg tac         1008
Ile Arg Lys Ile Val Ala Gln Ile Arg Pro Asp Arg Gln Thr Leu Tyr
                325                 330                 335
tgg agt gcc aca tgg cct aga gaa gtt gaa gcc tta gca aga cag ttt         1056
Trp Ser Ala Thr Trp Pro Arg Glu Val Glu Ala Leu Ala Arg Gln Phe
            340                 345                 350
cta caa aat cct tac aag gta atc ata ggc tca cct gaa ctt aaa gct         1104
Leu Gln Asn Pro Tyr Lys Val Ile Ile Gly Ser Pro Glu Leu Lys Ala
        355                 360                 365
aat cat tcc ata caa caa att gtt gaa gtt att tca gat cat gag aag         1152
Asn His Ser Ile Gln Gln Ile Val Glu Val Ile Ser Asp His Glu Lys
    370                 375                 380
tat cca aga ctc agt aag ctc ttg tct gat cta atg gat ggg agc cgt         1200
Tyr Pro Arg Leu Ser Lys Leu Leu Ser Asp Leu Met Asp Gly Ser Arg
385                 390                 395                 400
atc ttg ata ttc ttc caa aca aaa aag gat tgc gat aag atc acc cgg         1248
Ile Leu Ile Phe Phe Gln Thr Lys Lys Asp Cys Asp Lys Ile Thr Arg
                405                 410                 415
cag ctt aga atg gat ggg tgg cca gca tta tct ata cat ggt gat aaa         1296
Gln Leu Arg Met Asp Gly Trp Pro Ala Leu Ser Ile His Gly Asp Lys
            420                 425                 430
gct caa gct gaa cgg gac tat gtt ctt gca gag ttt aag agc ggc aag         1344
Ala Gln Ala Glu Arg Asp Tyr Val Leu Ala Glu Phe Lys Ser Gly Lys
        435                 440                 445
agc ccc ata atg gct gcc aca gat gtg gca gca cgt ggt ctt ggt atg         1392
Ser Pro Ile Met Ala Ala Thr Asp Val Ala Ala Arg Gly Leu Gly Met
    450                 455                 460
atg att tat ttg aat att agg att agg tag                                 1422
Met Ile Tyr Leu Asn Ile Arg Ile Arg
465                 470

<210> 48
<211> 473
<212> PRT
<213> Zea mays

<400> 48
Met Asn Pro Tyr Asp Leu Arg Phe Ala Asp Pro Ser Ser Tyr His Asp
1               5                   10                  15
Arg Arg Ser Asp Leu Thr Val Ala Pro Thr Phe Ala Pro Pro Ala Pro
            20                  25                  30
Val Ala Ala Ala Asn Leu Asn Pro Tyr Ala Ala Ala Tyr Pro Leu Pro
        35                  40                  45
Val Pro Val Pro Val Pro Ala Ala Pro Ala Gly Gly Asp Tyr Gln Arg

    50                  55                  60
Tyr Gly Gln Gly Gly Arg Gly Arg Gly Gly Gly Gly Arg Gly Gly Gly
65                  70                  75                  80
Gly Gly Tyr Gly Gly Gly Gly Arg Gly Gly Gly Arg Gly Arg Asp Gly
                85                  90                  95
Leu Asp Ser Leu Ala Leu Pro Lys Pro Asp Phe Arg Ser Leu Ile Pro
            100                 105                 110
Phe Glu Lys Asn Phe Tyr Val Glu Cys Pro Ser Val Gln Ala Met Ser
        115                 120                 125
Glu Ala Asp Val Ala Gln Tyr Arg Arg Leu Arg Asp Ile Thr Ile Glu
    130                 135                 140
Gly Arg Asp Val Pro Lys Pro Val Arg Tyr Phe Gln Glu Ala Asn Phe
145                 150                 155                 160
Pro Asp Tyr Cys Met Gln Ala Ile Ala Lys Ser Gly Phe Val Glu Pro
                165                 170                 175
Thr Pro Ile Gln Ser Gln Gly Trp Pro Met Ala Leu Lys Gly Arg Asp
            180                 185                 190
Leu Ile Gly Ile Ala Gln Thr Gly Ser Gly Lys Thr Leu Ser Tyr Leu
        195                 200                 205
Leu Pro Gly Leu Val His Val Gly Ala Gln Pro Arg Leu Glu Gln Gly
    210                 215                 220
Asp Gly Pro Ile Val Leu Ile Leu Ala Pro Thr Arg Glu Leu Ala Val
225                 230                 235                 240
Gln Ile Gln Glu Glu Ser Thr Lys Phe Gly Ser Tyr Ser Arg Thr Arg
                245                 250                 255
Ser Thr Cys Val Tyr Gly Gly Ala Pro Lys Gly Pro Gln Ile Arg Asp
            260                 265                 270
Leu Arg Arg Gly Val Glu Ile Val Ile Ala Thr Pro Gly Arg Leu Ile
        275                 280                 285
Asp Met Leu Glu Ala Gly His Thr Asn Leu Arg Arg Val Thr Tyr Leu
    290                 295                 300
Val Leu Asp Glu Ala Asp Arg Met Leu Asp Met Gly Phe Glu Pro Gln
305                 310                 315                 320
Ile Arg Lys Ile Val Ala Gln Ile Arg Pro Asp Arg Gln Thr Leu Tyr
                325                 330                 335
Trp Ser Ala Thr Trp Pro Arg Glu Val Glu Ala Leu Ala Arg Gln Phe
            340                 345                 350
Leu Gln Asn Pro Tyr Lys Val Ile Ile Gly Ser Pro Glu Leu Lys Ala
        355                 360                 365
Asn His Ser Ile Gln Gln Ile Val Glu Val Ile Ser Asp His Glu Lys
    370                 375                 380
Tyr Pro Arg Leu Ser Lys Leu Leu Ser Asp Leu Met Asp Gly Ser Arg
385                 390                 395                 400
Ile Leu Ile Phe Phe Gln Thr Lys Lys Asp Cys Asp Lys Ile Thr Arg
                405                 410                 415
Gln Leu Arg Met Asp Gly Trp Pro Ala Leu Ser Ile His Gly Asp Lys
            420                 425                 430
Ala Gln Ala Glu Arg Asp Tyr Val Leu Ala Glu Phe Lys Ser Gly Lys
        435                 440                 445
Ser Pro Ile Met Ala Ala Thr Asp Val Ala Ala Arg Gly Leu Gly Met
    450                 455                 460
Met Ile Tyr Leu Asn Ile Arg Ile Arg
465                 470

<210> 49
<211> 1500
<212> DNA
<213> Zea mays

<220>
<221> CDS
<222> (1)..(1500)

<400> 49
atg cac cag cct aga gct cgc tac cca ccc ggc tat ggc tcc ggc ggt      48
Met His Gln Pro Arg Ala Arg Tyr Pro Pro Gly Tyr Gly Ser Gly Gly
1               5                   10                  15
gga ggc gga gcc gga cgt ggc ggt gga ggc gga aat gga gga ggt ggc      96
Gly Gly Gly Ala Gly Arg Gly Gly Gly Gly Gly Asn Gly Gly Gly Gly

                20                  25                  30
gga aac cac aac tac tac ggc cga aac ccg cag caa cac cac tac cac          144
Gly Asn His Asn Tyr Tyr Gly Arg Asn Pro Gln Gln His His Tyr His
            35                  40                  45
cag cag cag cag tcg cag cac tct cat agg aac tct tcg tac cag caa          192
Gln Gln Gln Gln Ser Gln His Ser His Arg Asn Ser Ser Tyr Gln Gln
        50                  55                  60
cag tgg ctg cgg cga gac cag gcc ccc gcc gtt gcg ggg gca gcg tcc          240
Gln Trp Leu Arg Arg Asp Gln Ala Pro Ala Val Ala Gly Ala Ala Ser
65                  70                  75                  80
gga aac gct gtg gcc aag acg gcg tct cag ctt gac gcc gta ggc tcg          288
Gly Asn Ala Val Ala Lys Thr Ala Ser Gln Leu Asp Ala Val Gly Ser
                85                  90                  95
agc tcc caa gac tgg aag gct caa cta aat ata cca gct cca gat aca          336
Ser Ser Gln Asp Trp Lys Ala Gln Leu Asn Ile Pro Ala Pro Asp Thr
            100                 105                 110
cgc tac agg aca gag gat gtc act gca aca aaa ggc aat gag ttt gaa          384
Arg Tyr Arg Thr Glu Asp Val Thr Ala Thr Lys Gly Asn Glu Phe Glu
        115                 120                 125
gat tat ttc ttg aaa cgt gaa ctg ctt atg gga atc tac gaa aag gga          432
Asp Tyr Phe Leu Lys Arg Glu Leu Leu Met Gly Ile Tyr Glu Lys Gly
    130                 135                 140
ttt gaa aga cca tct cct att caa gaa gaa agc att cct att gcg ctg          480
Phe Glu Arg Pro Ser Pro Ile Gln Glu Glu Ser Ile Pro Ile Ala Leu
145                 150                 155                 160
act gga agt gat att ctt gca agg gca aaa aat ggc act gga aag act          528
Thr Gly Ser Asp Ile Leu Ala Arg Ala Lys Asn Gly Thr Gly Lys Thr
                165                 170                 175
gct gcc ttt tgc att ccg gca ctt gaa aaa att gat cct gaa aaa aat          576
Ala Ala Phe Cys Ile Pro Ala Leu Glu Lys Ile Asp Pro Glu Lys Asn
            180                 185                 190
gct att caa gtt gtt ata cta gta cca aca agg gaa ctg gct ctg caa          624
Ala Ile Gln Val Val Ile Leu Val Pro Thr Arg Glu Leu Ala Leu Gln
        195                 200                 205
act tca caa gtc tgt aaa gag ctc ggg aaa tac cta aac att caa gtt          672
Thr Ser Gln Val Cys Lys Glu Leu Gly Lys Tyr Leu Asn Ile Gln Val
    210                 215                 220
atg gtt agc act gga gga act agc ttg aag gat gac atc atg cgt tta          720
Met Val Ser Thr Gly Gly Thr Ser Leu Lys Asp Asp Ile Met Arg Leu
225                 230                 235                 240
tac caa cct gtt cat tta ctt gtt ggg act cct ggc cga ata ctt gat          768
Tyr Gln Pro Val His Leu Leu Val Gly Thr Pro Gly Arg Ile Leu Asp
                245                 250                 255
ctt acc agg aag ggc att tgt gtg ttg aaa gac tgt tca atg ctt gtc          816
Leu Thr Arg Lys Gly Ile Cys Val Leu Lys Asp Cys Ser Met Leu Val
            260                 265                 270
atg gat gag gca gac aag cta tta gct cca gag ttc cag cct tct gtg          864
Met Asp Glu Ala Asp Lys Leu Leu Ala Pro Glu Phe Gln Pro Ser Val
        275                 280                 285
gag gca ctt att cat ttc ctt cca cct agt cgg cag ctg ttg atg ttt          912
Glu Ala Leu Ile His Phe Leu Pro Pro Ser Arg Gln Leu Leu Met Phe
    290                 295                 300
tca gca acc ttt cct gta act gtc aag gaa ttc aaa gat aaa tat ctg          960
Ser Ala Thr Phe Pro Val Thr Val Lys Glu Phe Lys Asp Lys Tyr Leu
305                 310                 315                 320
cct aga cct tat gtg att aat cta atg gat gaa ctg acc ctg aaa gga          1008
Pro Arg Pro Tyr Val Ile Asn Leu Met Asp Glu Leu Thr Leu Lys Gly
                325                 330                 335
atc aca caa tat tat gct ttt gtt gaa gaa agg caa aaa gtt cat tgc          1056
Ile Thr Gln Tyr Tyr Ala Phe Val Glu Glu Arg Gln Lys Val His Cys
            340                 345                 350
ctg aat aca ctt ttc tca aag ctt caa att aat caa tcc att ata ttc          1104
Leu Asn Thr Leu Phe Ser Lys Leu Gln Ile Asn Gln Ser Ile Ile Phe
        355                 360                 365
tgc aac tct gtt aat aga gtt gag ctg ctg gct aaa aaa ata act gaa          1152
Cys Asn Ser Val Asn Arg Val Glu Leu Leu Ala Lys Lys Ile Thr Glu
    370                 375                 380
ctc ggt tat tca tgc ttc tac att cat gct aag atg ttg caa gac cac          1200
Leu Gly Tyr Ser Cys Phe Tyr Ile His Ala Lys Met Leu Gln Asp His

385                 390                 395                 400
aga aac cga gtg ttt cat gat ttt cgt aat ggt gct tgc aga aac ctt      1248
Arg Asn Arg Val Phe His Asp Phe Arg Asn Gly Ala Cys Arg Asn Leu
                405                 410                 415
gtg tgc aca gat ctg ttt acc aga gga att gat atc cag gct gtt aat      1296
Val Cys Thr Asp Leu Phe Thr Arg Gly Ile Asp Ile Gln Ala Val Asn
            420                 425                 430
gtg gtc att aat ttt gac ttc cct aag aca tct gag aca tac tta cac      1344
Val Val Ile Asn Phe Asp Phe Pro Lys Thr Ser Glu Thr Tyr Leu His
        435                 440                 445
agg gtt ggc cgt tct ggg aga tat gga cac ctt ggt ttg gca gtg aac      1392
Arg Val Gly Arg Ser Gly Arg Tyr Gly His Leu Gly Leu Ala Val Asn
    450                 455                 460
ttg atc act tat gaa gac cgt ttc aac tta tat agg att gag caa gaa      1440
Leu Ile Thr Tyr Glu Asp Arg Phe Asn Leu Tyr Arg Ile Glu Gln Glu
465                 470                 475                 480
ctt gga acg gaa att aag aca ata ccc cca cag att gac ctg tcc ata      1488
Leu Gly Thr Glu Ile Lys Thr Ile Pro Pro Gln Ile Asp Leu Ser Ile
                485                 490                 495
tac tgc caa tga                                                      1500
Tyr Cys Gln


<210> 50
<211> 499
<212> PRT
<213> Zea mays

<400> 50
Met His Gln Pro Arg Ala Arg Tyr Pro Pro Gly Tyr Gly Ser Gly Gly
1               5                   10                  15
Gly Gly Gly Ala Gly Arg Gly Gly Gly Gly Gly Asn Gly Gly Gly Gly
            20                  25                  30
Gly Asn His Asn Tyr Tyr Gly Arg Asn Pro Gln Gln His His Tyr His
        35                  40                  45
Gln Gln Gln Gln Ser Gln His Ser His Arg Asn Ser Ser Tyr Gln Gln
    50                  55                  60
Gln Trp Leu Arg Arg Asp Gln Ala Pro Ala Val Ala Gly Ala Ala Ser
65                  70                  75                  80
Gly Asn Ala Val Ala Lys Thr Ala Ser Gln Leu Asp Ala Val Gly Ser
                85                  90                  95
Ser Ser Gln Asp Trp Lys Ala Gln Leu Asn Ile Pro Ala Pro Asp Thr
            100                 105                 110
Arg Tyr Arg Thr Glu Asp Val Thr Ala Thr Lys Gly Asn Glu Phe Glu
        115                 120                 125
Asp Tyr Phe Leu Lys Arg Glu Leu Leu Met Gly Ile Tyr Glu Lys Gly
    130                 135                 140
Phe Glu Arg Pro Ser Pro Ile Gln Glu Glu Ser Ile Pro Ile Ala Leu
145                 150                 155                 160
Thr Gly Ser Asp Ile Leu Ala Arg Ala Lys Asn Gly Thr Gly Lys Thr
                165                 170                 175
Ala Ala Phe Cys Ile Pro Ala Leu Glu Lys Ile Asp Pro Glu Lys Asn
            180                 185                 190
Ala Ile Gln Val Val Ile Leu Val Pro Thr Arg Glu Leu Ala Leu Gln
        195                 200                 205
Thr Ser Gln Val Cys Lys Glu Leu Gly Lys Tyr Leu Asn Ile Gln Val
    210                 215                 220
Met Val Ser Thr Gly Gly Thr Ser Leu Lys Asp Asp Ile Met Arg Leu
225                 230                 235                 240
Tyr Gln Pro Val His Leu Leu Val Gly Thr Pro Gly Arg Ile Leu Asp
                245                 250                 255
Leu Thr Arg Lys Gly Ile Cys Val Leu Lys Asp Cys Ser Met Leu Val
            260                 265                 270
Met Asp Glu Ala Asp Lys Leu Leu Ala Pro Glu Phe Gln Pro Ser Val
        275                 280                 285
Glu Ala Leu Ile His Phe Leu Pro Pro Ser Arg Gln Leu Leu Met Phe
    290                 295                 300
Ser Ala Thr Phe Pro Val Thr Val Lys Glu Phe Lys Asp Lys Tyr Leu
305                 310                 315                 320

Pro Arg Pro Tyr Val Ile Asn Leu Met Asp Glu Leu Thr Leu Lys Gly
                325                 330                 335
Ile Thr Gln Tyr Tyr Ala Phe Val Glu Glu Arg Gln Lys Val His Cys
            340                 345                 350
Leu Asn Thr Leu Phe Ser Lys Leu Gln Ile Asn Gln Ser Ile Ile Phe
        355                 360                 365
Cys Asn Ser Val Asn Arg Val Glu Leu Leu Ala Lys Lys Ile Thr Glu
    370                 375                 380
Leu Gly Tyr Ser Cys Phe Tyr Ile His Ala Lys Met Leu Gln Asp His
385                 390                 395                 400
Arg Asn Arg Val Phe His Asp Phe Arg Asn Gly Ala Cys Arg Asn Leu
                405                 410                 415
Val Cys Thr Asp Leu Phe Thr Arg Gly Ile Asp Ile Gln Ala Val Asn
            420                 425                 430
Val Val Ile Asn Phe Asp Phe Pro Lys Thr Ser Glu Thr Tyr Leu His
        435                 440                 445
Arg Val Gly Arg Ser Gly Arg Tyr Gly His Leu Gly Leu Ala Val Asn
    450                 455                 460
Leu Ile Thr Tyr Glu Asp Arg Phe Asn Leu Tyr Arg Ile Glu Gln Glu
465                 470                 475                 480
Leu Gly Thr Glu Ile Lys Thr Ile Pro Pro Gln Ile Asp Leu Ser Ile
                485                 490                 495
Tyr Cys Gln


<210> 51
<211> 1506
<212> DNA
<213> Zea mays

<220>
<221> CDS
<222> (1)..(1506)

<400> 51
atg cgc gtg gcg gag cgg aag cgc gcg agg cga cgc ggc aac agg agg      48
Met Arg Val Ala Glu Arg Lys Arg Ala Arg Arg Arg Gly Asn Arg Arg
1               5                   10                  15
agg agg cca agg cta agg cga ccg cgg cag ccg aag cgg tgg cgg cgg      96
Arg Arg Pro Arg Leu Arg Arg Pro Arg Gln Pro Lys Arg Trp Arg Arg
            20                  25                  30
ctc gac gcg gcg tcc tct tcc tcc tcc atc acc gag ccg gcg gtg gag      144
Leu Asp Ala Ala Ser Ser Ser Ser Ser Ile Thr Glu Pro Ala Val Glu
        35                  40                  45
gag cag gcc aag cag atc gag gcg ctc tcc ctc gcc ccg tcg gtg gac      192
Glu Gln Ala Lys Gln Ile Glu Ala Leu Ser Leu Ala Pro Ser Val Asp
    50                  55                  60
gac gcc gca ggc gaa gag ggc ccg ccc ctc ctc gat gac tcc gac gac      240
Asp Ala Ala Gly Glu Glu Gly Pro Pro Leu Leu Asp Asp Ser Asp Asp
65                  70                  75                  80
tcg caa atc caa gcc gtg att tcg ggt ggc acg gtg tac gag tcg gcc      288
Ser Gln Ile Gln Ala Val Ile Ser Gly Gly Thr Val Tyr Glu Ser Ala
                85                  90                  95
acc acg ttc gag gat ctg aag ctg tcg ccc gag ctg ctt aag ggg ctg      336
Thr Thr Phe Glu Asp Leu Lys Leu Ser Pro Glu Leu Leu Lys Gly Leu
            100                 105                 110
cac gac gag atg ggg ttc agc cgc ccg agc aag atc cag gcg atc acc      384
His Asp Glu Met Gly Phe Ser Arg Pro Ser Lys Ile Gln Ala Ile Thr
        115                 120                 125
ctg ccc atg atc ctc acg ccg ccc tac aag gac ctc gtc gcg cag gcg      432
Leu Pro Met Ile Leu Thr Pro Pro Tyr Lys Asp Leu Val Ala Gln Ala
    130                 135                 140
cac aac ggc tct ggc aag acc acc tgt ttc gtc ctc ggc atg ctc agc      480
His Asn Gly Ser Gly Lys Thr Thr Cys Phe Val Leu Gly Met Leu Ser
145                 150                 155                 160
cgc gtc gac ccg caa cgc aag ata ccc cag gct atc tgc att tgc ccc      528
Arg Val Asp Pro Gln Arg Lys Ile Pro Gln Ala Ile Cys Ile Cys Pro
                165                 170                 175
acc agg gag ctt gcg cag cag aat aag gca gtt ctc atg agg atg ggc      576

Thr Arg Glu Leu Ala Gln Gln Asn Lys Ala Val Leu Met Arg Met Gly
            180                 185                 190
aag ttt act ggc att aca tgc gcc tgt gca atc ccg cca gct caa aaa      624
Lys Phe Thr Gly Ile Thr Cys Ala Cys Ala Ile Pro Pro Ala Gln Lys
        195                 200                 205
gac tac atg ccg atc tcg aaa atg gct ccg att act gac cag atc gtt      672
Asp Tyr Met Pro Ile Ser Lys Met Ala Pro Ile Thr Asp Gln Ile Val
    210                 215                 220
atc ggc aca tct ggt acg ctc atc aaa tgg att acc cat aag aag ctg      720
Ile Gly Thr Ser Gly Thr Leu Ile Lys Trp Ile Thr His Lys Lys Leu
225                 230                 235                 240
gcc aca agg gac atc aag ata ctt gtg ttt gat gag gca gat cat atg      768
Ala Thr Arg Asp Ile Lys Ile Leu Val Phe Asp Glu Ala Asp His Met
                245                 250                 255
ctt gct gag gat ggc ttt agg agt gat tct gaa agg atg atg agg gat      816
Leu Ala Glu Asp Gly Phe Arg Ser Asp Ser Glu Arg Met Met Arg Asp
            260                 265                 270
ata caa aga agc gct ggt ggt tgt cag gtg ctt ctc ttt tct gcg acc      864
Ile Gln Arg Ser Ala Gly Gly Cys Gln Val Leu Leu Phe Ser Ala Thr
        275                 280                 285
ttc aat gat agg gtg aag gat ttt gtt aca aag gtc att agg gat gga      912
Phe Asn Asp Arg Val Lys Asp Phe Val Thr Lys Val Ile Arg Asp Gly
    290                 295                 300
aat cag ata ttt gtg aag aag gaa gag ctt act ttg gaa aaa gtt aag      960
Asn Gln Ile Phe Val Lys Lys Glu Glu Leu Thr Leu Glu Lys Val Lys
305                 310                 315                 320
cag tac aaa gtt cag gta cct gat gaa tcg gca aaa ata gag gtt ata      1008
Gln Tyr Lys Val Gln Val Pro Asp Glu Ser Ala Lys Ile Glu Val Ile
                325                 330                 335
agg gac aag ata ttt gaa ttt gga cag aaa gtt ggc cag gtt atc ata      1056
Arg Asp Lys Ile Phe Glu Phe Gly Gln Lys Val Gly Gln Val Ile Ile
            340                 345                 350
ttt gtt aga aca aag caa agt act aag aat gtt cac aat gct ttg acg      1104
Phe Val Arg Thr Lys Gln Ser Thr Lys Asn Val His Asn Ala Leu Thr
        355                 360                 365
agg gag gac tat gtg tgc tcc tca atc caa gga tcc ctt gac caa tca      1152
Arg Glu Asp Tyr Val Cys Ser Ser Ile Gln Gly Ser Leu Asp Gln Ser
    370                 375                 380
gag agg gaa aag ata ata cag gaa ttc aaa gat ggc tac acc aag gtt      1200
Glu Arg Glu Lys Ile Ile Gln Glu Phe Lys Asp Gly Tyr Thr Lys Val
385                 390                 395                 400
ctt ata tca act gat gtt ctt gct cga ggt ttt gac caa gca cag gtt      1248
Leu Ile Ser Thr Asp Val Leu Ala Arg Gly Phe Asp Gln Ala Gln Val
                405                 410                 415
aac ctc gtc atc aac tac gac atg cca atc aaa ttt ggt aca aga gat      1296
Asn Leu Val Ile Asn Tyr Asp Met Pro Ile Lys Phe Gly Thr Arg Asp
            420                 425                 430
gaa cct gat tat gag gtg tac ttg cac aga att ggc aga gct ggg cgc      1344
Glu Pro Asp Tyr Glu Val Tyr Leu His Arg Ile Gly Arg Ala Gly Arg
        435                 440                 445
ttt ggc cgg aaa ggt gct gtg ttc aac ttg ctg tgt ggt caa acc gat      1392
Phe Gly Arg Lys Gly Ala Val Phe Asn Leu Leu Cys Gly Gln Thr Asp
    450                 455                 460
aat gtt gtg atg aag aag atc gag gac tat ttc cag cac agt gtg cct      1440
Asn Val Val Met Lys Lys Ile Glu Asp Tyr Phe Gln His Ser Val Pro
465                 470                 475                 480
gag gtt cgg aat tgg caa cgt gaa gaa gat ttt gag gct gct ctc aag      1488
Glu Val Arg Asn Trp Gln Arg Glu Glu Asp Phe Glu Ala Ala Leu Lys
                485                 490                 495
gat gca ggt tta ctt taa                                              1506
Asp Ala Gly Leu Leu
            500

<210> 52
<211> 501
<212> PRT
<213> Zea mays

<400> 52

Met Arg Val Ala Glu Arg Lys Arg Ala Arg Arg Arg Gly Asn Arg Arg
1               5                   10                  15
Arg Arg Pro Arg Leu Arg Arg Pro Arg Gln Pro Lys Arg Trp Arg Arg
            20                  25                  30
Leu Asp Ala Ala Ser Ser Ser Ser Ser Ile Thr Glu Pro Ala Val Glu
        35                  40                  45
Glu Gln Ala Lys Gln Ile Glu Ala Leu Ser Leu Ala Pro Ser Val Asp
    50                  55                  60
Asp Ala Ala Gly Glu Glu Gly Pro Pro Leu Leu Asp Asp Ser Asp Asp
65                  70                  75                  80
Ser Gln Ile Gln Ala Val Ile Ser Gly Gly Thr Val Tyr Glu Ser Ala
                85                  90                  95
Thr Thr Phe Glu Asp Leu Lys Leu Ser Pro Glu Leu Leu Lys Gly Leu
            100                 105                 110
His Asp Glu Met Gly Phe Ser Arg Pro Ser Lys Ile Gln Ala Ile Thr
        115                 120                 125
Leu Pro Met Ile Leu Thr Pro Pro Tyr Lys Asp Leu Val Ala Gln Ala
    130                 135                 140
His Asn Gly Ser Gly Lys Thr Thr Cys Phe Val Leu Gly Met Leu Ser
145                 150                 155                 160
Arg Val Asp Pro Gln Arg Lys Ile Pro Gln Ala Ile Cys Ile Cys Pro
                165                 170                 175
Thr Arg Glu Leu Ala Gln Gln Asn Lys Ala Val Leu Met Arg Met Gly
            180                 185                 190
Lys Phe Thr Gly Ile Thr Cys Ala Cys Ala Ile Pro Pro Ala Gln Lys
        195                 200                 205
Asp Tyr Met Pro Ile Ser Lys Met Ala Pro Ile Thr Asp Gln Ile Val
    210                 215                 220
Ile Gly Thr Ser Gly Thr Leu Ile Lys Trp Ile Thr His Lys Lys Leu
225                 230                 235                 240
Ala Thr Arg Asp Ile Lys Ile Leu Val Phe Asp Glu Ala Asp His Met
                245                 250                 255
Leu Ala Glu Asp Gly Phe Arg Ser Asp Ser Glu Arg Met Met Arg Asp
            260                 265                 270
Ile Gln Arg Ser Ala Gly Gly Cys Gln Val Leu Leu Phe Ser Ala Thr
        275                 280                 285
Phe Asn Asp Arg Val Lys Asp Phe Val Thr Lys Val Ile Arg Asp Gly
    290                 295                 300
Asn Gln Ile Phe Val Lys Lys Glu Glu Leu Thr Leu Glu Lys Val Lys
305                 310                 315                 320
Gln Tyr Lys Val Gln Val Pro Asp Glu Ser Ala Lys Ile Glu Val Ile
                325                 330                 335
Arg Asp Lys Ile Phe Glu Phe Gly Gln Lys Val Gly Gln Val Ile Ile
            340                 345                 350
Phe Val Arg Thr Lys Gln Ser Thr Lys Asn Val His Asn Ala Leu Thr
        355                 360                 365
Arg Glu Asp Tyr Val Cys Ser Ser Ile Gln Gly Ser Leu Asp Gln Ser
    370                 375                 380
Glu Arg Glu Lys Ile Ile Gln Glu Phe Lys Asp Gly Tyr Thr Lys Val
385                 390                 395                 400
Leu Ile Ser Thr Asp Val Leu Ala Arg Gly Phe Asp Gln Ala Gln Val
                405                 410                 415
Asn Leu Val Ile Asn Tyr Asp Met Pro Ile Lys Phe Gly Thr Arg Asp
            420                 425                 430
Glu Pro Asp Tyr Glu Val Tyr Leu His Arg Ile Gly Arg Ala Gly Arg
        435                 440                 445
Phe Gly Arg Lys Gly Ala Val Phe Asn Leu Leu Cys Gly Gln Thr Asp
    450                 455                 460
Asn Val Val Met Lys Lys Ile Glu Asp Tyr Phe Gln His Ser Val Pro
465                 470                 475                 480
Glu Val Arg Asn Trp Gln Arg Glu Glu Asp Phe Glu Ala Ala Leu Lys
                485                 490                 495
Asp Ala Gly Leu Leu
            500

<210> 53
<211> 1857
<212> DNA
<213> Zea mays

<220>
<221> CDS
<222> (1)..(1857)

<400> 53
atg tcc gcc atc gcg gcc tcc tcg gcc gcc gcc gcc gcg gcg tcg aac 48
Met Ser Ala Ile Ala Ala Ser Ser Ala Ala Ala Ala Ala Ala Ser Asn
1               5                   10                  15
tcc gat gag gaa gaa aac tat gag gag tac atc cca gtc gcc aag cgc 96
Ser Asp Glu Glu Glu Asn Tyr Glu Glu Tyr Ile Pro Val Ala Lys Arg
            20                  25                  30
cgg gcc atg gag gcc gag cgc ctc cgc cag ctc cgc ctc tcc aag tcg 144
Arg Ala Met Glu Ala Glu Arg Leu Arg Gln Leu Arg Leu Ser Lys Ser
        35                  40                  45
gcg ccg gcc tct gcc gtc tca tca ctc ccg atg cca cct ccc ccg cct 192
Ala Pro Ala Ser Ala Val Ser Ser Leu Pro Met Pro Pro Pro Pro Pro
    50                  55                  60
ctt ttg cca gcg cag acc tcc gtt ccc gac gcc gcc gcg aag ccc agc 240
Leu Leu Pro Ala Gln Thr Ser Val Pro Asp Ala Ala Ala Lys Pro Ser
65                  70                  75                  80
ctt ctc gtc aag gcc acg cag ctt aag cgc gcg gcc ccg gag gtg acc 288
Leu Leu Val Lys Ala Thr Gln Leu Lys Arg Ala Ala Pro Glu Val Thr
                85                  90                  95
gcc acg gag cag cgc atc cag cag gag aag gaa gtg att gaa aat ctc 336
Ala Thr Glu Gln Arg Ile Gln Gln Glu Lys Glu Val Ile Glu Asn Leu
            100                 105                 110
tca gac aac aaa tcg ctc agg tca gtc cgc gag atc gcc aag ggc atc 384
Ser Asp Asn Lys Ser Leu Arg Ser Val Arg Glu Ile Ala Lys Gly Ile
        115                 120                 125
atc tac acg gaa ccg atc cag acc ggg tgg aag ccg ccc ctc cgc ctc 432
Ile Tyr Thr Glu Pro Ile Gln Thr Gly Trp Lys Pro Pro Leu Arg Leu
    130                 135                 140
cgc cgc atg ccg ctc gcc aaa gcc aac gag ttc cgg cgc aag tgg cac 480
Arg Arg Met Pro Leu Ala Lys Ala Asn Glu Phe Arg Arg Lys Trp His
145                 150                 155                 160
atc atc gtc gac ggt gac gat ata ccg ccg cct gcg cgt gat ttc cgg 528
Ile Ile Val Asp Gly Asp Asp Ile Pro Pro Pro Ala Arg Asp Phe Arg
                165                 170                 175
gat ctc cgc ctc ccg gag ccc atg ctc cgg aag ctg cgc gag agg ggt 576
Asp Leu Arg Leu Pro Glu Pro Met Leu Arg Lys Leu Arg Glu Arg Gly
            180                 185                 190
atc gtc cag ccc acg ccg att cag gtg cag ggg ctg ccc gtt gtg ctc 624
Ile Val Gln Pro Thr Pro Ile Gln Val Gln Gly Leu Pro Val Val Leu
        195                 200                 205
tct ggg cgt gac atg ata ggc atc gct ttc acg ggg tcg ggg aag act 672
Ser Gly Arg Asp Met Ile Gly Ile Ala Phe Thr Gly Ser Gly Lys Thr
    210                 215                 220
ctg gtg ttc gtg ctg ccg ctt atc atg gtg gcg ctg cag gag gaa tca 720
Leu Val Phe Val Leu Pro Leu Ile Met Val Ala Leu Gln Glu Glu Ser
225                 230                 235                 240
ttg atg ccg atc gtg cct ggc gag ggc ccg ttt ggg atg atc ata tgc 768
Leu Met Pro Ile Val Pro Gly Glu Gly Pro Phe Gly Met Ile Ile Cys
                245                 250                 255
ccg tcg cgg gaa ctg gcc aag cag aca tat gat gtg att gag cag ttt 816
Pro Ser Arg Glu Leu Ala Lys Gln Thr Tyr Asp Val Ile Glu Gln Phe
            260                 265                 270
ctc att cca ctt aag gag gcc ggt tat ccg gag ata agg cat ttg ctt 864
Leu Ile Pro Leu Lys Glu Ala Gly Tyr Pro Glu Ile Arg His Leu Leu
        275                 280                 285
tgt att ggg ggt gtg gat atg agg aca cag ctc gat gtg ttg aag aag 912
Cys Ile Gly Gly Val Asp Met Arg Thr Gln Leu Asp Val Leu Lys Lys
    290                 295                 300
ggc gtg cac att gtg gtg gca acg cct ggt cgg ctc aag gat ttg ctt 960
Gly Val His Ile Val Val Ala Thr Pro Gly Arg Leu Lys Asp Leu Leu
305                 310                 315                 320
gcc aag aag aag atg aac ctt gac aac tgc agg tac tta acc tta gat 1008
Ala Lys Lys Lys Met Asn Leu Asp Asn Cys Arg Tyr Leu Thr Leu Asp
                325                 330                 335

gaa gcg gat agg ctg gtt gac cta gga ttt gaa gat gac atc aag gag          1056
Glu Ala Asp Arg Leu Val Asp Leu Gly Phe Glu Asp Asp Ile Lys Glu
            340                 345                 350
gtt ttc gac cat ttc aag gat caa agg caa act ctt ctc ttt tct gct          1104
Val Phe Asp His Phe Lys Asp Gln Arg Gln Thr Leu Leu Phe Ser Ala
        355                 360                 365
act atg cct cag aag att caa aat ttt gcg aag aac gcc ctt gtg aaa          1152
Thr Met Pro Gln Lys Ile Gln Asn Phe Ala Lys Asn Ala Leu Val Lys
    370                 375                 380
cca gtt act gtc aat gtg gga aga gct gga gca gca aat cta gac gtc          1200
Pro Val Thr Val Asn Val Gly Arg Ala Gly Ala Ala Asn Leu Asp Val
385                 390                 395                 400
att caa gaa gtc gaa tat gta aag gaa gat gct aga att ata tac ctc          1248
Ile Gln Glu Val Glu Tyr Val Lys Glu Asp Ala Arg Ile Ile Tyr Leu
                405                 410                 415
ctt gaa tgc ctc caa aag act cca cct cct gtt ctc att ttc tgt gaa          1296
Leu Glu Cys Leu Gln Lys Thr Pro Pro Pro Val Leu Ile Phe Cys Glu
            420                 425                 430
aac aag gct gat gtc gac tac atc cat gag tac ctt ctt cta aag gga          1344
Asn Lys Ala Asp Val Asp Tyr Ile His Glu Tyr Leu Leu Leu Lys Gly
        435                 440                 445
gtg gag gct gtt gct atc cat gga gga aaa gat caa gag gag aga cag          1392
Val Glu Ala Val Ala Ile His Gly Gly Lys Asp Gln Glu Glu Arg Gln
    450                 455                 460
aac gca att gac tcc ttc aag act ggt agg aag gat gtt ctg gtg gct          1440
Asn Ala Ile Asp Ser Phe Lys Thr Gly Arg Lys Asp Val Leu Val Ala
465                 470                 475                 480
act gat gtt gca tcg aag ggt ctt gat ttt cct gac att cag cat gtc          1488
Thr Asp Val Ala Ser Lys Gly Leu Asp Phe Pro Asp Ile Gln His Val
                485                 490                 495
att aac tat gac atg ccc gcc gaa ata gag aac tat gtc cac cga atc          1536
Ile Asn Tyr Asp Met Pro Ala Glu Ile Glu Asn Tyr Val His Arg Ile
            500                 505                 510
ggt cga act ggt cga tgt ggg aag act ggc ata gca act acc ttc atc          1584
Gly Arg Thr Gly Arg Cys Gly Lys Thr Gly Ile Ala Thr Thr Phe Ile
        515                 520                 525
aac aag aac caa act gag acc acg ctt ctc gat ctc aag cac ctg ctg          1632
Asn Lys Asn Gln Thr Glu Thr Thr Leu Leu Asp Leu Lys His Leu Leu
    530                 535                 540
aag gag gca aag cag agg att cca ccg gtg ttg gcg gag ctc aac gat          1680
Lys Glu Ala Lys Gln Arg Ile Pro Pro Val Leu Ala Glu Leu Asn Asp
545                 550                 555                 560
cca ttg gat gat gag gaa ata att gcc aag gag agt ggt gtc aaa ggt          1728
Pro Leu Asp Asp Glu Glu Ile Ile Ala Lys Glu Ser Gly Val Lys Gly
                565                 570                 575
tgt gcg tat tgt ggt ggg ctt ggc cat cgt gtc agt gac tgt ccg aag          1776
Cys Ala Tyr Cys Gly Gly Leu Gly His Arg Val Ser Asp Cys Pro Lys
            580                 585                 590
ttg gag cac caa aag tcc atg gca att gct ggc tcg aga aaa gac tac          1824
Leu Glu His Gln Lys Ser Met Ala Ile Ala Gly Ser Arg Lys Asp Tyr
        595                 600                 605
ttt ggc ggc ggg ggt tac cga gga gaa ata tga                              1857
Phe Gly Gly Gly Gly Tyr Arg Gly Glu Ile
    610                 615

<210> 54
<211> 618
<212> PRT
<213> Zea mays

<400> 54
Met Ser Ala Ile Ala Ala Ser Ser Ala Ala Ala Ala Ala Ala Ser Asn
1               5                   10                  15
Ser Asp Glu Glu Glu Asn Tyr Glu Glu Tyr Ile Pro Val Ala Lys Arg
            20                  25                  30
Arg Ala Met Glu Ala Glu Arg Leu Arg Gln Leu Arg Leu Ser Lys Ser
        35                  40                  45
Ala Pro Ala Ser Ala Val Ser Ser Leu Pro Met Pro Pro Pro Pro Pro
    50                  55                  60

Leu Leu Pro Ala Gln Thr Ser Val Pro Asp Ala Ala Ala Lys Pro Ser
65                  70                  75                  80
Leu Leu Val Lys Ala Thr Gln Leu Lys Arg Ala Ala Pro Glu Val Thr
                85                  90                  95
Ala Thr Glu Gln Arg Ile Gln Gln Glu Lys Glu Val Ile Glu Asn Leu
            100                 105                 110
Ser Asp Asn Lys Ser Leu Arg Ser Val Arg Glu Ile Ala Lys Gly Ile
        115                 120                 125
Ile Tyr Thr Glu Pro Ile Gln Thr Gly Trp Lys Pro Pro Leu Arg Leu
    130                 135                 140
Arg Arg Met Pro Leu Ala Lys Ala Asn Glu Phe Arg Arg Lys Trp His
145                 150                 155                 160
Ile Ile Val Asp Gly Asp Asp Ile Pro Pro Pro Ala Arg Asp Phe Arg
                165                 170                 175
Asp Leu Arg Leu Pro Glu Pro Met Leu Arg Lys Leu Arg Glu Arg Gly
            180                 185                 190
Ile Val Gln Pro Thr Pro Ile Gln Val Gln Gly Leu Pro Val Val Leu
        195                 200                 205
Ser Gly Arg Asp Met Ile Gly Ile Ala Phe Thr Gly Ser Gly Lys Thr
    210                 215                 220
Leu Val Phe Val Leu Pro Leu Ile Met Val Ala Leu Gln Glu Glu Ser
225                 230                 235                 240
Leu Met Pro Ile Val Pro Gly Glu Gly Pro Phe Gly Met Ile Ile Cys
                245                 250                 255
Pro Ser Arg Glu Leu Ala Lys Gln Thr Tyr Asp Val Ile Glu Gln Phe
            260                 265                 270
Leu Ile Pro Leu Lys Glu Ala Gly Tyr Pro Glu Ile Arg His Leu Leu
        275                 280                 285
Cys Ile Gly Gly Val Asp Met Arg Thr Gln Leu Asp Val Leu Lys Lys
    290                 295                 300
Gly Val His Ile Val Val Ala Thr Pro Gly Arg Leu Lys Asp Leu Leu
305                 310                 315                 320
Ala Lys Lys Lys Met Asn Leu Asp Asn Cys Arg Tyr Leu Thr Leu Asp
                325                 330                 335
Glu Ala Asp Arg Leu Val Asp Leu Gly Phe Glu Asp Asp Ile Lys Glu
            340                 345                 350
Val Phe Asp His Phe Lys Asp Gln Arg Gln Thr Leu Leu Phe Ser Ala
        355                 360                 365
Thr Met Pro Gln Lys Ile Gln Asn Phe Ala Lys Asn Ala Leu Val Lys
    370                 375                 380
Pro Val Thr Val Asn Val Gly Arg Ala Gly Ala Ala Asn Leu Asp Val
385                 390                 395                 400
Ile Gln Glu Val Glu Tyr Val Lys Glu Asp Ala Arg Ile Ile Tyr Leu
                405                 410                 415
Leu Glu Cys Leu Gln Lys Thr Pro Pro Pro Val Leu Ile Phe Cys Glu
            420                 425                 430
Asn Lys Ala Asp Val Asp Tyr Ile His Glu Tyr Leu Leu Leu Lys Gly
        435                 440                 445
Val Glu Ala Val Ala Ile His Gly Gly Lys Asp Gln Glu Glu Arg Gln
    450                 455                 460
Asn Ala Ile Asp Ser Phe Lys Thr Gly Arg Lys Asp Val Leu Val Ala
465                 470                 475                 480
Thr Asp Val Ala Ser Lys Gly Leu Asp Phe Pro Asp Ile Gln His Val
                485                 490                 495
Ile Asn Tyr Asp Met Pro Ala Glu Ile Glu Asn Tyr Val His Arg Ile
            500                 505                 510
Gly Arg Thr Gly Arg Cys Gly Lys Thr Gly Ile Ala Thr Thr Phe Ile
        515                 520                 525
Asn Lys Asn Gln Thr Glu Thr Thr Leu Leu Asp Leu Lys His Leu Leu
    530                 535                 540
Lys Glu Ala Lys Gln Arg Ile Pro Pro Val Leu Ala Glu Leu Asn Asp
545                 550                 555                 560
Pro Leu Asp Asp Glu Glu Ile Ile Ala Lys Glu Ser Gly Val Lys Gly
                565                 570                 575
Cys Ala Tyr Cys Gly Gly Leu Gly His Arg Val Ser Asp Cys Pro Lys
            580                 585                 590
Leu Glu His Gln Lys Ser Met Ala Ile Ala Gly Ser Arg Lys Asp Tyr
        595                 600                 605
Phe Gly Gly Gly Gly Tyr Arg Gly Glu Ile

610                 615

<210> 55
<211> 1722
<212> DNA
<213> Zea mays

<220>
<221> CDS
<222> (1)..(1722)

<400> 55
atg agg cta agt att ggg caa gtc cat cga aat gtt ctt gcg ctg gca       48
Met Arg Leu Ser Ile Gly Gln Val His Arg Asn Val Leu Ala Leu Ala
1               5                   10                  15
tct tct cgg tca tgt ttt gtt ctg gga gat cgt gtt tcg ttc agg atg       96
Ser Ser Arg Ser Cys Phe Val Leu Gly Asp Arg Val Ser Phe Arg Met
            20                  25                  30
ctg tcc caa cca cgg gta gct gga ttt cat cag act gct tgg cgt ggt       144
Leu Ser Gln Pro Arg Val Ala Gly Phe His Gln Thr Ala Trp Arg Gly
        35                  40                  45
agt caa att ttg gaa gat aac agg gga gga cca ttg act ctt gct agc       192
Ser Gln Ile Leu Glu Asp Asn Arg Gly Gly Pro Leu Thr Leu Ala Ser
    50                  55                  60
cta gaa gtg caa agc agg gtt gaa tat gga aaa aag ggg aaa atg gaa       240
Leu Glu Val Gln Ser Arg Val Glu Tyr Gly Lys Lys Gly Lys Met Glu
65                  70                  75                  80
cga act gga ggt cca aag cca agt tca aga gct tct tcc ctc tat gtg       288
Arg Thr Gly Gly Pro Lys Pro Ser Ser Arg Ala Ser Ser Leu Tyr Val
                85                  90                  95
aaa cca aag gtt tct tca aat gtc aaa cca gca aag agt gcc ttg atg       336
Lys Pro Lys Val Ser Ser Asn Val Lys Pro Ala Lys Ser Ala Leu Met
            100                 105                 110
aag cca gct ggg ata aag aag aca ctg aag gta gat gag gct ctg ttt       384
Lys Pro Ala Gly Ile Lys Lys Thr Leu Lys Val Asp Glu Ala Leu Phe
        115                 120                 125
tcg gcc aag tca ttt gaa gaa ctt ggt ttg cca cct ttg ctt gtt gac       432
Ser Ala Lys Ser Phe Glu Glu Leu Gly Leu Pro Pro Leu Leu Val Asp
    130                 135                 140
cag ttg aac aag gaa ggt ctg act gcc cca act gag gtc caa tcc gca       480
Gln Leu Asn Lys Glu Gly Leu Thr Ala Pro Thr Glu Val Gln Ser Ala
145                 150                 155                 160
gca att cct ata ata gca caa aag cat gat gtt gtg atc cag tca tat       528
Ala Ile Pro Ile Ile Ala Gln Lys His Asp Val Val Ile Gln Ser Tyr
                165                 170                 175
act ggc tca ggg aaa act ctt gct tat ctt ctt cca att ctt tct gat       576
Thr Gly Ser Gly Lys Thr Leu Ala Tyr Leu Leu Pro Ile Leu Ser Asp
            180                 185                 190
ata ggc cct ctg aag agg act atg gaa caa gac cat tct gag aaa aga       624
Ile Gly Pro Leu Lys Arg Thr Met Glu Gln Asp His Ser Glu Lys Arg
        195                 200                 205
tca ggt ata gaa gca gtc atc gtt gct cct tcg aga gaa tta gga atg       672
Ser Gly Ile Glu Ala Val Ile Val Ala Pro Ser Arg Glu Leu Gly Met
    210                 215                 220
cag att gtg cga gaa gtg gag aag att ttg ggt cct aat gac aaa aga       720
Gln Ile Val Arg Glu Val Glu Lys Ile Leu Gly Pro Asn Asp Lys Arg
225                 230                 235                 240
ctg gtc cag caa ctt gtt ggc gga gct aat cgt tcc agg cag gaa gaa       768
Leu Val Gln Gln Leu Val Gly Gly Ala Asn Arg Ser Arg Gln Glu Glu
                245                 250                 255
gca ttg aag aag aac aaa cca atc att gtt gtc gga aca cct ggc cgt       816
Ala Leu Lys Lys Asn Lys Pro Ile Ile Val Val Gly Thr Pro Gly Arg
            260                 265                 270
att tct gaa att tct gca gca ggt aag cta cac act cat ggc tgc cgt       864
Ile Ser Glu Ile Ser Ala Ala Gly Lys Leu His Thr His Gly Cys Arg
        275                 280                 285
ttt ctt gta ctg gat gaa gtc gac cag ctt ctg tct ttt aat tac cgt       912
Phe Leu Val Leu Asp Glu Val Asp Gln Leu Leu Ser Phe Asn Tyr Arg
    290                 295                 300

gaa gat atg cat aga att ttg gag cat gtt gga agg aga cct gga ggc          960
Glu Asp Met His Arg Ile Leu Glu His Val Gly Arg Arg Pro Gly Gly
305                 310                 315                 320
aca tct agg gat att ctt ggc cca ctt gcg aga cga tct gag cgt cag          1008
Thr Ser Arg Asp Ile Leu Gly Pro Leu Ala Arg Arg Ser Glu Arg Gln
                325                 330                 335
act atc ctg gtt tct gca aca ata cca ttt tca gtt ata cga gca gca          1056
Thr Ile Leu Val Ser Ala Thr Ile Pro Phe Ser Val Ile Arg Ala Ala
            340                 345                 350
agg agt tgg ggt cat gat cca gtt ctc att aga gct aaa agt gta gtt          1104
Arg Ser Trp Gly His Asp Pro Val Leu Ile Arg Ala Lys Ser Val Val
        355                 360                 365
cca ctt gat tca atc act gtg cca aga cct gcg tta tcc caa agt gac          1152
Pro Leu Asp Ser Ile Thr Val Pro Arg Pro Ala Leu Ser Gln Ser Asp
    370                 375                 380
gct aac ccc agt tcg tca tcg cag tca gtg aac caa gct gct gtt ggc          1200
Ala Asn Pro Ser Ser Ser Ser Gln Ser Val Asn Gln Ala Ala Val Gly
385                 390                 395                 400
agc ttg ccg cca tct ttg gaa cac tac tat tgt acg gcc aag gcg cag          1248
Ser Leu Pro Pro Ser Leu Glu His Tyr Tyr Cys Thr Ala Lys Ala Gln
                405                 410                 415
cac aag gtt gac aca tta cgg agg tgc atc cat gct ctg gaa gca cag          1296
His Lys Val Asp Thr Leu Arg Arg Cys Ile His Ala Leu Glu Ala Gln
            420                 425                 430
aca gtg att gca ttt atg aac aac acc aag cca ctg aag gat gtt gtg          1344
Thr Val Ile Ala Phe Met Asn Asn Thr Lys Pro Leu Lys Asp Val Val
        435                 440                 445
ttt aag ttg gag gcc cgt ggt atc aaa gcc act gag cta cat gga gac          1392
Phe Lys Leu Glu Ala Arg Gly Ile Lys Ala Thr Glu Leu His Gly Asp
    450                 455                 460
ctt gga aag ctt gca agg tcg aca gtt ttg aaa aag ttc aag gat ggt          1440
Leu Gly Lys Leu Ala Arg Ser Thr Val Leu Lys Lys Phe Lys Asp Gly
465                 470                 475                 480
gaa ttc aga gtt ctc gtt acg aat gag ctg tct gcc aga gga ctg gac          1488
Glu Phe Arg Val Leu Val Thr Asn Glu Leu Ser Ala Arg Gly Leu Asp
                485                 490                 495
gtg cca gaa tgt gac ctt gtg gtt aat ctg gac ctt cca act gac tcc          1536
Val Pro Glu Cys Asp Leu Val Val Asn Leu Asp Leu Pro Thr Asp Ser
            500                 505                 510
aca cat tat gct cac aga gct gga aga act gga cgt ctt ggg cgg aaa          1584
Thr His Tyr Ala His Arg Ala Gly Arg Thr Gly Arg Leu Gly Arg Lys
        515                 520                 525
ggg atc gtg gtc aca ata tgc gaa gaa agc gag gga ttt gtt gta aga          1632
Gly Ile Val Val Thr Ile Cys Glu Glu Ser Glu Gly Phe Val Val Arg
    530                 535                 540
aaa atg cgc aag cag tta gat gtg gcc atc aag cca tgc gag ttc act          1680
Lys Met Arg Lys Gln Leu Asp Val Ala Ile Lys Pro Cys Glu Phe Thr
545                 550                 555                 560
gaa ggc gag ctt gtt gtc cac aag gag gaa gat gtg gaa taa                  1722
Glu Gly Glu Leu Val Val His Lys Glu Glu Asp Val Glu
                565                 570

<210> 56
<211> 573
<212> PRT
<213> Zea mays

<400> 56
Met Arg Leu Ser Ile Gly Gln Val His Arg Asn Val Leu Ala Leu Ala
1               5                   10                  15
Ser Ser Arg Ser Cys Phe Val Leu Gly Asp Arg Val Ser Phe Arg Met
            20                  25                  30
Leu Ser Gln Pro Arg Val Ala Gly Phe His Gln Thr Ala Trp Arg Gly
        35                  40                  45
Ser Gln Ile Leu Glu Asp Asn Arg Gly Gly Pro Leu Thr Leu Ala Ser
    50                  55                  60
Leu Glu Val Gln Ser Arg Val Glu Tyr Gly Lys Lys Gly Lys Met Glu
65                  70                  75                  80
Arg Thr Gly Gly Pro Lys Pro Ser Ser Arg Ala Ser Ser Leu Tyr Val

85                  90                  95
Lys Pro Lys Val Ser Ser Asn Val Lys Pro Ala Lys Ser Ala Leu Met
            100                 105                 110
Lys Pro Ala Gly Ile Lys Lys Thr Leu Lys Val Asp Glu Ala Leu Phe
        115                 120                 125
Ser Ala Lys Ser Phe Glu Glu Leu Gly Leu Pro Pro Leu Leu Val Asp
    130                 135                 140
Gln Leu Asn Lys Glu Gly Leu Thr Ala Pro Thr Glu Val Gln Ser Ala
145                 150                 155                 160
Ala Ile Pro Ile Ile Ala Gln Lys His Asp Val Val Ile Gln Ser Tyr
                165                 170                 175
Thr Gly Ser Gly Lys Thr Leu Ala Tyr Leu Leu Pro Ile Leu Ser Asp
            180                 185                 190
Ile Gly Pro Leu Lys Arg Thr Met Glu Gln Asp His Ser Glu Lys Arg
        195                 200                 205
Ser Gly Ile Glu Ala Val Ile Val Ala Pro Ser Arg Glu Leu Gly Met
    210                 215                 220
Gln Ile Val Arg Glu Val Glu Lys Ile Leu Gly Pro Asn Asp Lys Arg
225                 230                 235                 240
Leu Val Gln Gln Leu Val Gly Gly Ala Asn Arg Ser Arg Gln Glu Glu
                245                 250                 255
Ala Leu Lys Lys Asn Lys Pro Ile Ile Val Val Gly Thr Pro Gly Arg
            260                 265                 270
Ile Ser Glu Ile Ser Ala Ala Gly Lys Leu His Thr His Gly Cys Arg
        275                 280                 285
Phe Leu Val Leu Asp Glu Val Asp Gln Leu Leu Ser Phe Asn Tyr Arg
    290                 295                 300
Glu Asp Met His Arg Ile Leu Glu His Val Gly Arg Arg Pro Gly Gly
305                 310                 315                 320
Thr Ser Arg Asp Ile Leu Gly Pro Leu Ala Arg Arg Ser Glu Arg Gln
                325                 330                 335
Thr Ile Leu Val Ser Ala Thr Ile Pro Phe Ser Val Ile Arg Ala Ala
            340                 345                 350
Arg Ser Trp Gly His Asp Pro Val Leu Ile Arg Ala Lys Ser Val Val
        355                 360                 365
Pro Leu Asp Ser Ile Thr Val Pro Arg Pro Ala Leu Ser Gln Ser Asp
    370                 375                 380
Ala Asn Pro Ser Ser Ser Ser Gln Ser Val Asn Gln Ala Ala Val Gly
385                 390                 395                 400
Ser Leu Pro Pro Ser Leu Glu His Tyr Tyr Cys Thr Ala Lys Ala Gln
                405                 410                 415
His Lys Val Asp Thr Leu Arg Arg Cys Ile His Ala Leu Glu Ala Gln
            420                 425                 430
Thr Val Ile Ala Phe Met Asn Asn Thr Lys Pro Leu Lys Asp Val Val
        435                 440                 445
Phe Lys Leu Glu Ala Arg Gly Ile Lys Ala Thr Glu Leu His Gly Asp
    450                 455                 460
Leu Gly Lys Leu Ala Arg Ser Thr Val Leu Lys Lys Phe Lys Asp Gly
465                 470                 475                 480
Glu Phe Arg Val Leu Val Thr Asn Glu Leu Ser Ala Arg Gly Leu Asp
                485                 490                 495
Val Pro Glu Cys Asp Leu Val Val Asn Leu Asp Leu Pro Thr Asp Ser
            500                 505                 510
Thr His Tyr Ala His Arg Ala Gly Arg Thr Gly Arg Leu Gly Arg Lys
        515                 520                 525
Gly Ile Val Val Thr Ile Cys Glu Glu Ser Glu Gly Phe Val Val Arg
    530                 535                 540
Lys Met Arg Lys Gln Leu Asp Val Ala Ile Lys Pro Cys Glu Phe Thr
545                 550                 555                 560
Glu Gly Glu Leu Val Val His Lys Glu Glu Asp Val Glu
                565                 570

<210> 57
<211> 1224
<212> DNA
<213> Zea mays

<220>
<221> CDS

<222> (1)..(1224)

<400> 57
atg gcg gcg ccc acc acc tcc cgc cgc ggc ccc ggc ggc gcg cgc aac 48
Met Ala Ala Pro Thr Thr Ser Arg Arg Gly Pro Gly Gly Ala Arg Asn
1               5                   10                  15
atg gac gac gag aac ctc acc ttc gag acc tcc ccg ggg gtc gag gtc 96
Met Asp Asp Glu Asn Leu Thr Phe Glu Thr Ser Pro Gly Val Glu Val
            20                  25                  30
gtc agc agc ttc gac cag atg ggc atc aag gac gac ctc ctc cgc ggc 144
Val Ser Ser Phe Asp Gln Met Gly Ile Lys Asp Asp Leu Leu Arg Gly
        35                  40                  45
atc tac ggc tac ggt ttc gag aag ccc tcc gcc atc cag cag cgc gcc 192
Ile Tyr Gly Tyr Gly Phe Glu Lys Pro Ser Ala Ile Gln Gln Arg Ala
    50                  55                  60
gtg ctc ccc atc atc aac ggc cgc gac gtc atc gcg cag gcc cag tcc 240
Val Leu Pro Ile Ile Asn Gly Arg Asp Val Ile Ala Gln Ala Gln Ser
65                  70                  75                  80
ggt acc ggc aag acg tcc atg atc tcc ctc acc gtc tgc cag atc gtc 288
Gly Thr Gly Lys Thr Ser Met Ile Ser Leu Thr Val Cys Gln Ile Val
                85                  90                  95
gac acc gcc gtg cgc gag gtg cag gct ttg att ctc tcg cct act agg 336
Asp Thr Ala Val Arg Glu Val Gln Ala Leu Ile Leu Ser Pro Thr Arg
            100                 105                 110
gag ctt gct tcc caa aca gag aga gtt atg ctg gct att ggg gac cac 384
Glu Leu Ala Ser Gln Thr Glu Arg Val Met Leu Ala Ile Gly Asp His
        115                 120                 125
ctc aac gtc caa gtg cac gct tgc att ggt gga aag agt att ggt gag 432
Leu Asn Val Gln Val His Ala Cys Ile Gly Gly Lys Ser Ile Gly Glu
    130                 135                 140
gac atc agg agg ctc gag aat ggg gtg cat gtc gtc tct gga act cca 480
Asp Ile Arg Arg Leu Glu Asn Gly Val His Val Val Ser Gly Thr Pro
145                 150                 155                 160
ggc aga gtc tgt gat atg atc aag agg agg aca ctg cgg acc aga gcc 528
Gly Arg Val Cys Asp Met Ile Lys Arg Arg Thr Leu Arg Thr Arg Ala
                165                 170                 175
atc aag ctt cta gtt ctg gat gaa gct gat gag atg ttg agt aga ggc 576
Ile Lys Leu Leu Val Leu Asp Glu Ala Asp Glu Met Leu Ser Arg Gly
            180                 185                 190
ttt aag gat cag att tac gat gtc tac aga tat ctc cct cca gaa ctt 624
Phe Lys Asp Gln Ile Tyr Asp Val Tyr Arg Tyr Leu Pro Pro Glu Leu
        195                 200                 205
cag gtg gtt ttg atc tct gca act ctt cct cat gag atc ttg gag atg 672
Gln Val Val Leu Ile Ser Ala Thr Leu Pro His Glu Ile Leu Glu Met
    210                 215                 220
act agt aag ttc atg acc gaa ccg gtt agg atc ctt gtg aag cgt gat 720
Thr Ser Lys Phe Met Thr Glu Pro Val Arg Ile Leu Val Lys Arg Asp
225                 230                 235                 240
gag ctg acc ctg gag ggc atc aaa caa ttc ttt gtt gct gtt gag aaa 768
Glu Leu Thr Leu Glu Gly Ile Lys Gln Phe Phe Val Ala Val Glu Lys
                245                 250                 255
gag gag tgg aag ttt gat aca ctt tgt gat ctt tat gat aca ctc acc 816
Glu Glu Trp Lys Phe Asp Thr Leu Cys Asp Leu Tyr Asp Thr Leu Thr
            260                 265                 270
atc acc caa gct gtt att ttc tgc aat act aag aga aag gtg gat tgg 864
Ile Thr Gln Ala Val Ile Phe Cys Asn Thr Lys Arg Lys Val Asp Trp
        275                 280                 285
ctc act gaa aaa atg cgt aca aat aac ttc acc gta tca gct atg cac 912
Leu Thr Glu Lys Met Arg Thr Asn Asn Phe Thr Val Ser Ala Met His
    290                 295                 300
ggt gac atg ccc caa caa gaa agg gat gcc att atg ggt gag ttc agg 960
Gly Asp Met Pro Gln Gln Glu Arg Asp Ala Ile Met Gly Glu Phe Arg
305                 310                 315                 320
tct ggc gca act cgt gtg cta ata act aca gat gtt tgg gct cga gga 1008
Ser Gly Ala Thr Arg Val Leu Ile Thr Thr Asp Val Trp Ala Arg Gly
                325                 330                 335
ctg gac gtt cag cag gtt tca ctt gtc ata aat tat gat ctc cca aat 1056
Leu Asp Val Gln Gln Val Ser Leu Val Ile Asn Tyr Asp Leu Pro Asn
            340                 345                 350

aat cgt gag ctt tac atc cat cgc att ggt cgt tct ggt cgt ttt ggg 1104
Asn Arg Glu Leu Tyr Ile His Arg Ile Gly Arg Ser Gly Arg Phe Gly
        355                 360                 365
cgc aag ggt gtg gcg atc aat ttc gtg cgc aag gat gac atc cgt ata 1152
Arg Lys Gly Val Ala Ile Asn Phe Val Arg Lys Asp Asp Ile Arg Ile
    370                 375                 380
ctg aga gat ata gag cag tac tac agc aca caa att gac gag atg cca 1200
Leu Arg Asp Ile Glu Gln Tyr Tyr Ser Thr Gln Ile Asp Glu Met Pro
385                 390                 395                 400
atg aat gtt gct gat ctt att tga 1224
Met Asn Val Ala Asp Leu Ile
                405

<210> 58
<211> 407
<212> PRT
<213> Zea mays

<400> 58
Met Ala Ala Pro Thr Thr Ser Arg Arg Gly Pro Gly Gly Ala Arg Asn
1               5                   10                  15
Met Asp Asp Glu Asn Leu Thr Phe Glu Thr Ser Pro Gly Val Glu Val
            20                  25                  30
Val Ser Ser Phe Asp Gln Met Gly Ile Lys Asp Asp Leu Leu Arg Gly
        35                  40                  45
Ile Tyr Gly Tyr Gly Phe Glu Lys Pro Ser Ala Ile Gln Gln Arg Ala
    50                  55                  60
Val Leu Pro Ile Ile Asn Gly Arg Asp Val Ile Ala Gln Ala Gln Ser
65                  70                  75                  80
Gly Thr Gly Lys Thr Ser Met Ile Ser Leu Thr Val Cys Gln Ile Val
                85                  90                  95
Asp Thr Ala Val Arg Glu Val Gln Ala Leu Ile Leu Ser Pro Thr Arg
            100                 105                 110
Glu Leu Ala Ser Gln Thr Glu Arg Val Met Leu Ala Ile Gly Asp His
        115                 120                 125
Leu Asn Val Gln Val His Ala Cys Ile Gly Gly Lys Ser Ile Gly Glu
    130                 135                 140
Asp Ile Arg Arg Leu Glu Asn Gly Val His Val Val Ser Gly Thr Pro
145                 150                 155                 160
Gly Arg Val Cys Asp Met Ile Lys Arg Arg Thr Leu Arg Thr Arg Ala
                165                 170                 175
Ile Lys Leu Leu Val Leu Asp Glu Ala Asp Glu Met Leu Ser Arg Gly
            180                 185                 190
Phe Lys Asp Gln Ile Tyr Asp Val Tyr Arg Tyr Leu Pro Pro Glu Leu
        195                 200                 205
Gln Val Val Leu Ile Ser Ala Thr Leu Pro His Glu Ile Leu Glu Met
    210                 215                 220
Thr Ser Lys Phe Met Thr Glu Pro Val Arg Ile Leu Val Lys Arg Asp
225                 230                 235                 240
Glu Leu Thr Leu Glu Gly Ile Lys Gln Phe Phe Val Ala Val Glu Lys
                245                 250                 255
Glu Glu Trp Lys Phe Asp Thr Leu Cys Asp Leu Tyr Asp Thr Leu Thr
            260                 265                 270
Ile Thr Gln Ala Val Ile Phe Cys Asn Thr Lys Arg Lys Val Asp Trp
        275                 280                 285
Leu Thr Glu Lys Met Arg Thr Asn Asn Phe Thr Val Ser Ala Met His
    290                 295                 300
Gly Asp Met Pro Gln Gln Glu Arg Asp Ala Ile Met Gly Glu Phe Arg
305                 310                 315                 320
Ser Gly Ala Thr Arg Val Leu Ile Thr Thr Asp Val Trp Ala Arg Gly
                325                 330                 335
Leu Asp Val Gln Gln Val Ser Leu Val Ile Asn Tyr Asp Leu Pro Asn
            340                 345                 350
Asn Arg Glu Leu Tyr Ile His Arg Ile Gly Arg Ser Gly Arg Phe Gly
        355                 360                 365
Arg Lys Gly Val Ala Ile Asn Phe Val Arg Lys Asp Asp Ile Arg Ile
    370                 375                 380
Leu Arg Asp Ile Glu Gln Tyr Tyr Ser Thr Gln Ile Asp Glu Met Pro
385                 390                 395                 400

Met Asn Val Ala Asp Leu Ile
                405

<210> 59
<211> 2127
<212> DNA
<213> Zea mays

<220>
<221> CDS
<222> (1)..(2127)

<400> 59
atg tct ccc gcc ctt gcc gct ggc gtg gag ccc atg gcc gtc gac gat      48
Met Ser Pro Ala Leu Ala Ala Gly Val Glu Pro Met Ala Val Asp Asp
1               5                   10                  15
tcc gcc tcc aag aag gcc aag cgc aag cag ctc aag gcc gcc gcc gcc      96
Ser Ala Ser Lys Lys Ala Lys Arg Lys Gln Leu Lys Ala Ala Ala Ala
            20                  25                  30
gcc gcg gag gca gag gcg gag gcc gct tcg gtc aag aag aaa gag aag      144
Ala Ala Glu Ala Glu Ala Glu Ala Ala Ser Val Lys Lys Lys Glu Lys
        35                  40                  45
aaa gag aag aag cga aag gtg aag gag ccg tcc cct ccg ctc cca tcg      192
Lys Glu Lys Lys Arg Lys Val Lys Glu Pro Ser Pro Pro Leu Pro Ser
    50                  55                  60
gcc gcg tcc tct gat gag gaa gag aaa agc agc acc agc tcc gag gag      240
Ala Ala Ser Ser Asp Glu Glu Glu Lys Ser Ser Thr Ser Ser Glu Glu
65                  70                  75                  80
aca gca ccc gcg gcg aaa aaa gcg aag aag gag aag acg aag aag aat      288
Thr Ala Pro Ala Ala Lys Lys Ala Lys Lys Glu Lys Thr Lys Lys Asn
                85                  90                  95
gtt gat gtc tcg tct tca gcc tca gat gac gat ggc gag atc aca gcc      336
Val Asp Val Ser Ser Ser Ala Ser Asp Asp Asp Gly Glu Ile Thr Ala
            100                 105                 110
agc agc gat gag gac ccc gcg gac ccg aac gcg ctg acg aac ttc agg      384
Ser Ser Asp Glu Asp Pro Ala Asp Pro Asn Ala Leu Thr Asn Phe Arg
        115                 120                 125
ata tcg gag tcg ctg agg cag agc ctc agg tcc aag ggg atc aag tca      432
Ile Ser Glu Ser Leu Arg Gln Ser Leu Arg Ser Lys Gly Ile Lys Ser
    130                 135                 140
ctg ttc ccc att cag gcc acc act ttc gat ctc gta ctc gat ggc agc      480
Leu Phe Pro Ile Gln Ala Thr Thr Phe Asp Leu Val Leu Asp Gly Ser
145                 150                 155                 160
gac ttg gtt ggc cga gcg cgc acc ggt cag gga aaa act ttg gct ttt      528
Asp Leu Val Gly Arg Ala Arg Thr Gly Gln Gly Lys Thr Leu Ala Phe
                165                 170                 175
gtc ttg ccc ata ttg gag tct ttg gtt aat ggg gca aac aag gca tct      576
Val Leu Pro Ile Leu Glu Ser Leu Val Asn Gly Ala Asn Lys Ala Ser
            180                 185                 190
aga cgg act gaa cat ggc agg acc cca agt gtt ctc gtt cta cta cca      624
Arg Arg Thr Glu His Gly Arg Thr Pro Ser Val Leu Val Leu Leu Pro
        195                 200                 205
aca aga gag ctg gcc aat cag gtg cac gct gac ttt gag ttt tat ggt      672
Thr Arg Glu Leu Ala Asn Gln Val His Ala Asp Phe Glu Phe Tyr Gly
    210                 215                 220
gca aca ttt ggg ctc tct gca tgt tgt gtg tat ggg gga tca cct tat      720
Ala Thr Phe Gly Leu Ser Ala Cys Cys Val Tyr Gly Gly Ser Pro Tyr
225                 230                 235                 240
cgt cct caa gaa atg gca ttg aga aga ggt gtg gat att gtt gtt gga      768
Arg Pro Gln Glu Met Ala Leu Arg Arg Gly Val Asp Ile Val Val Gly
                245                 250                 255
act cct ggt cgt gtc aag gat ttc att gtt aaa gga act ctc aat ttg      816
Thr Pro Gly Arg Val Lys Asp Phe Ile Val Lys Gly Thr Leu Asn Leu
            260                 265                 270
aaa tgc ttg aaa ttc cgt gtc ctt gat gaa gct gat gag atg cta aac      864
Lys Cys Leu Lys Phe Arg Val Leu Asp Glu Ala Asp Glu Met Leu Asn
        275                 280                 285
atg ggt ttt gtt gat gat gtc gag ctc att ctt ggc aag gta gaa gat      912
Met Gly Phe Val Asp Asp Val Glu Leu Ile Leu Gly Lys Val Glu Asp

290                 295                 300
gct acc aaa gta cag aca ctt ctg ttc agt gcc act ctg cca gat tgg        960
Ala Thr Lys Val Gln Thr Leu Leu Phe Ser Ala Thr Leu Pro Asp Trp
305                 310                 315                 320
gtg aat aag ctc tct atg agg ttt ctg aaa gtt gac agg aaa aca gtt        1008
Val Asn Lys Leu Ser Met Arg Phe Leu Lys Val Asp Arg Lys Thr Val
                325                 330                 335
gat ctt gtc ggt aac gag aaa ctg aag gcc agt gca tct gtt aag cac        1056
Asp Leu Val Gly Asn Glu Lys Leu Lys Ala Ser Ala Ser Val Lys His
            340                 345                 350
ctt gct ctt cct tgt aac aag gca gca agg gca caa ctt att cca gat        1104
Leu Ala Leu Pro Cys Asn Lys Ala Ala Arg Ala Gln Leu Ile Pro Asp
        355                 360                 365
att atc cga tgt tac agc cat gga ggc cga acc att atc ttc act gag        1152
Ile Ile Arg Cys Tyr Ser His Gly Gly Arg Thr Ile Ile Phe Thr Glu
    370                 375                 380
acg aag gat tct gca tca gag ctt tct ggt ttg att cct gga tcc cgt        1200
Thr Lys Asp Ser Ala Ser Glu Leu Ser Gly Leu Ile Pro Gly Ser Arg
385                 390                 395                 400
gcc ttg cat gga gat gtt gtg caa gct cag cgt gaa gtc att ctt gct        1248
Ala Leu His Gly Asp Val Val Gln Ala Gln Arg Glu Val Ile Leu Ala
                405                 410                 415
gga ttt cgt agc ggg aag ttc cag gtt ttg gtt gct aca aat gtg gcg        1296
Gly Phe Arg Ser Gly Lys Phe Gln Val Leu Val Ala Thr Asn Val Ala
            420                 425                 430
gct cgt ggt ctg gat att aat gat gtg cag ctt atc att cag tgt gaa        1344
Ala Arg Gly Leu Asp Ile Asn Asp Val Gln Leu Ile Ile Gln Cys Glu
        435                 440                 445
cct ccc cgg gac gtt gaa gct tac ata cac cgg tca ggt cgg aca ggg        1392
Pro Pro Arg Asp Val Glu Ala Tyr Ile His Arg Ser Gly Arg Thr Gly
    450                 455                 460
aga gca ggt aat act ggt gtt gct gtc atg ctt tat gag ccc aga tat        1440
Arg Ala Gly Asn Thr Gly Val Ala Val Met Leu Tyr Glu Pro Arg Tyr
465                 470                 475                 480
aag cac agt gtc agc aga cta gaa agg gag tct ggg gtt aaa ttt gag        1488
Lys His Ser Val Ser Arg Leu Glu Arg Glu Ser Gly Val Lys Phe Glu
                485                 490                 495
cat atc tct gcg cca caa ccc act gat gta gca caa tct gct ggc agt        1536
His Ile Ser Ala Pro Gln Pro Thr Asp Val Ala Gln Ser Ala Gly Ser
            500                 505                 510
gaa gct gca gat gca att gcg agt gtg tca gac agt gtt att cct atc        1584
Glu Ala Ala Asp Ala Ile Ala Ser Val Ser Asp Ser Val Ile Pro Ile
        515                 520                 525
ttc agg cag caa gca gag cag ttg cta agc tct tcc agt ctg tct gca        1632
Phe Arg Gln Gln Ala Glu Gln Leu Leu Ser Ser Ser Ser Leu Ser Ala
    530                 535                 540
gct gac ttg ctt gcc aaa gca ctt gca aag gca gtt ggt tac acg gac        1680
Ala Asp Leu Leu Ala Lys Ala Leu Ala Lys Ala Val Gly Tyr Thr Asp
545                 550                 555                 560
ata aag aaa aga tca ttg tta tct tcc ttg gag gat tac agt aca cta        1728
Ile Lys Lys Arg Ser Leu Leu Ser Ser Leu Glu Asp Tyr Ser Thr Leu
                565                 570                 575
cat ctt caa act ggc aga ccg atg tgg tca cct ggg ttt gct ttt act        1776
His Leu Gln Thr Gly Arg Pro Met Trp Ser Pro Gly Phe Ala Phe Thr
            580                 585                 590
ata ttg aaa agg ttc atg cca gaa gat aaa ctt gca gat gta aag ggt        1824
Ile Leu Lys Arg Phe Met Pro Glu Asp Lys Leu Ala Asp Val Lys Gly
        595                 600                 605
gcc acc ctc acc gct gat gga aca gga gtt gta ttt gat gtt cct gca        1872
Ala Thr Leu Thr Ala Asp Gly Thr Gly Val Val Phe Asp Val Pro Ala
    610                 615                 620
gca gat gtt gaa gat tac att caa gct tcg gag agt gcc gca cag gtg        1920
Ala Asp Val Glu Asp Tyr Ile Gln Ala Ser Glu Ser Ala Ala Gln Val
625                 630                 635                 640
aca att gat gaa gtc aag caa ctg cca ccc ttg caa gag aag gac cag        1968
Thr Ile Asp Glu Val Lys Gln Leu Pro Pro Leu Gln Glu Lys Asp Gln
                645                 650                 655
tcg aga ggc aac tct ggg gga gga aga ttt ggc cat gga ggt ggc cgg        2016
Ser Arg Gly Asn Ser Gly Gly Gly Arg Phe Gly His Gly Gly Gly Arg

                660                 665                 670
aga ttt tct ggt ggt ggt ggc ggc ggc aga ggg ttt tct ggc cgg gga       2064
Arg Phe Ser Gly Gly Gly Gly Gly Gly Arg Gly Phe Ser Gly Arg Gly
        675                 680                 685
ggt ggc ggc aga ggg ttt tct ggc aga gga ggc ggc ggc agt aga ttt       2112
Gly Gly Gly Arg Gly Phe Ser Gly Arg Gly Gly Gly Gly Ser Arg Phe
    690                 695                 700
aac agg agg aat tag                                                   2127
Asn Arg Arg Asn
705

<210> 60
<211> 708
<212> PRT
<213> Zea mays

<400> 60
Met Ser Pro Ala Leu Ala Ala Gly Val Glu Pro Met Ala Val Asp Asp
1               5                   10                  15
Ser Ala Ser Lys Lys Ala Lys Arg Lys Gln Leu Lys Ala Ala Ala Ala
            20                  25                  30
Ala Ala Glu Ala Glu Ala Glu Ala Ala Ser Val Lys Lys Lys Glu Lys
        35                  40                  45
Lys Glu Lys Lys Arg Lys Val Lys Glu Pro Ser Pro Pro Leu Pro Ser
    50                  55                  60
Ala Ala Ser Ser Asp Glu Glu Glu Lys Ser Ser Thr Ser Ser Glu Glu
65                  70                  75                  80
Thr Ala Pro Ala Ala Lys Lys Ala Lys Lys Glu Lys Thr Lys Lys Asn
                85                  90                  95
Val Asp Val Ser Ser Ser Ala Ser Asp Asp Asp Gly Glu Ile Thr Ala
            100                 105                 110
Ser Ser Asp Glu Asp Pro Ala Asp Pro Asn Ala Leu Thr Asn Phe Arg
        115                 120                 125
Ile Ser Glu Ser Leu Arg Gln Ser Leu Arg Ser Lys Gly Ile Lys Ser
    130                 135                 140
Leu Phe Pro Ile Gln Ala Thr Thr Phe Asp Leu Val Leu Asp Gly Ser
145                 150                 155                 160
Asp Leu Val Gly Arg Ala Arg Thr Gly Gln Gly Lys Thr Leu Ala Phe
                165                 170                 175
Val Leu Pro Ile Leu Glu Ser Leu Val Asn Gly Ala Asn Lys Ala Ser
            180                 185                 190
Arg Arg Thr Glu His Gly Arg Thr Pro Ser Val Leu Val Leu Leu Pro
        195                 200                 205
Thr Arg Glu Leu Ala Asn Gln Val His Ala Asp Phe Glu Phe Tyr Gly
    210                 215                 220
Ala Thr Phe Gly Leu Ser Ala Cys Cys Val Tyr Gly Gly Ser Pro Tyr
225                 230                 235                 240
Arg Pro Gln Glu Met Ala Leu Arg Arg Gly Val Asp Ile Val Val Gly
                245                 250                 255
Thr Pro Gly Arg Val Lys Asp Phe Ile Val Lys Gly Thr Leu Asn Leu
            260                 265                 270
Lys Cys Leu Lys Phe Arg Val Leu Asp Glu Ala Asp Glu Met Leu Asn
        275                 280                 285
Met Gly Phe Val Asp Asp Val Glu Leu Ile Leu Gly Lys Val Glu Asp
    290                 295                 300
Ala Thr Lys Val Gln Thr Leu Leu Phe Ser Ala Thr Leu Pro Asp Trp
305                 310                 315                 320
Val Asn Lys Leu Ser Met Arg Phe Leu Lys Val Asp Arg Lys Thr Val
                325                 330                 335
Asp Leu Val Gly Asn Glu Lys Leu Lys Ala Ser Ala Ser Val Lys His
            340                 345                 350
Leu Ala Leu Pro Cys Asn Lys Ala Ala Arg Ala Gln Leu Ile Pro Asp
        355                 360                 365
Ile Ile Arg Cys Tyr Ser His Gly Gly Arg Thr Ile Ile Phe Thr Glu
    370                 375                 380
Thr Lys Asp Ser Ala Ser Glu Leu Ser Gly Leu Ile Pro Gly Ser Arg
385                 390                 395                 400
Ala Leu His Gly Asp Val Val Gln Ala Gln Arg Glu Val Ile Leu Ala
                405                 410                 415

Gly Phe Arg Ser Gly Lys Phe Gln Val Leu Val Ala Thr Asn Val Ala
            420                 425                 430
Ala Arg Gly Leu Asp Ile Asn Asp Val Gln Leu Ile Ile Gln Cys Glu
        435                 440                 445
Pro Pro Arg Asp Val Glu Ala Tyr Ile His Arg Ser Gly Arg Thr Gly
    450                 455                 460
Arg Ala Gly Asn Thr Gly Val Ala Val Met Leu Tyr Glu Pro Arg Tyr
465                 470                 475                 480
Lys His Ser Val Ser Arg Leu Glu Arg Glu Ser Gly Val Lys Phe Glu
                485                 490                 495
His Ile Ser Ala Pro Gln Pro Thr Asp Val Ala Gln Ser Ala Gly Ser
            500                 505                 510
Glu Ala Ala Asp Ala Ile Ala Ser Val Ser Asp Ser Val Ile Pro Ile
        515                 520                 525
Phe Arg Gln Gln Ala Glu Gln Leu Leu Ser Ser Ser Ser Leu Ser Ala
    530                 535                 540
Ala Asp Leu Leu Ala Lys Ala Leu Ala Lys Ala Val Gly Tyr Thr Asp
545                 550                 555                 560
Ile Lys Lys Arg Ser Leu Leu Ser Ser Leu Glu Asp Tyr Ser Thr Leu
                565                 570                 575
His Leu Gln Thr Gly Arg Pro Met Trp Ser Pro Gly Phe Ala Phe Thr
            580                 585                 590
Ile Leu Lys Arg Phe Met Pro Glu Asp Lys Leu Ala Asp Val Lys Gly
        595                 600                 605
Ala Thr Leu Thr Ala Asp Gly Thr Gly Val Val Phe Asp Val Pro Ala
    610                 615                 620
Ala Asp Val Glu Asp Tyr Ile Gln Ala Ser Glu Ser Ala Ala Gln Val
625                 630                 635                 640
Thr Ile Asp Glu Val Lys Gln Leu Pro Pro Leu Gln Glu Lys Asp Gln
                645                 650                 655
Ser Arg Gly Asn Ser Gly Gly Gly Arg Phe Gly His Gly Gly Gly Arg
            660                 665                 670
Arg Phe Ser Gly Gly Gly Gly Gly Gly Arg Gly Phe Ser Gly Arg Gly
        675                 680                 685
Gly Gly Gly Arg Gly Phe Ser Gly Arg Gly Gly Gly Gly Ser Arg Phe
    690                 695                 700
Asn Arg Arg Asn
705

<210> 61
<211> 1290
<212> DNA
<213> Zea mays

<220>
<221> CDS
<222> (1)..(1290)

<400> 61
atg atg gga gag gcc aag gag aac gac gtt tat gag gag gag ctc ctg      48
Met Met Gly Glu Ala Lys Glu Asn Asp Val Tyr Glu Glu Glu Leu Leu
1               5                   10                  15
gac tac gag gag gac gac gac aag acg gtc gat ggc tcc gct gct aag      96
Asp Tyr Glu Glu Asp Asp Asp Lys Thr Val Asp Gly Ser Ala Ala Lys
            20                  25                  30
ccc acc gga gag gtc gca aag aag ggc tac gtc ggg atc cac agt tcc      144
Pro Thr Gly Glu Val Ala Lys Lys Gly Tyr Val Gly Ile His Ser Ser
        35                  40                  45
ggt ttc aga gac ttc ctg ctc aag cca gag ctg ctc cgt gct atc cag      192
Gly Phe Arg Asp Phe Leu Leu Lys Pro Glu Leu Leu Arg Ala Ile Gln
    50                  55                  60
gat tgt ggt ttt gag cat cct tcc gaa gtg caa cac gag tgt atc cct      240
Asp Cys Gly Phe Glu His Pro Ser Glu Val Gln His Glu Cys Ile Pro
65                  70                  75                  80
caa gcc att ctt gga atg gat gtc atc tgt caa gct aaa tct ggg atg      288
Gln Ala Ile Leu Gly Met Asp Val Ile Cys Gln Ala Lys Ser Gly Met
                85                  90                  95
ggg aaa act gct gtt ttt gtc ctt tca tcc ctc caa caa att gac cct      336
Gly Lys Thr Ala Val Phe Val Leu Ser Ser Leu Gln Gln Ile Asp Pro

            100                 105                 110
gtt gcg ggt cag gta gca gca ctt gta ctg tgc cac aca agg gaa ctg 384
Val Ala Gly Gln Val Ala Ala Leu Val Leu Cys His Thr Arg Glu Leu
        115                 120                 125
gct tat cag ata tgc cac gaa ttt gag agg ttc agc aaa tat ctt act 432
Ala Tyr Gln Ile Cys His Glu Phe Glu Arg Phe Ser Lys Tyr Leu Thr
    130                 135                 140
gaa gtt aaa gtt gct gtc ttc tat gga ggt gtt cac atc agg aag cat 480
Glu Val Lys Val Ala Val Phe Tyr Gly Gly Val His Ile Arg Lys His
145                 150                 155                 160
aaa gat ttg ttg aag aat gaa tgc cct cat att gtg gtt ggc acg cct 528
Lys Asp Leu Leu Lys Asn Glu Cys Pro His Ile Val Val Gly Thr Pro
                165                 170                 175
ggt agg ata ctt gct ctt gcc agg gat aag gat ctc tcc ttg aag aat 576
Gly Arg Ile Leu Ala Leu Ala Arg Asp Lys Asp Leu Ser Leu Lys Asn
            180                 185                 190
gtg agg cac ttc att ctt gat gaa tgt gac aaa atg ctg gaa tca ctt 624
Val Arg His Phe Ile Leu Asp Glu Cys Asp Lys Met Leu Glu Ser Leu
        195                 200                 205
gac atg cgg aga gat gtt cag gag atc ttc aaa atg act cct cat gat 672
Asp Met Arg Arg Asp Val Gln Glu Ile Phe Lys Met Thr Pro His Asp
    210                 215                 220
aag caa gtc atg atg ttt tca gca acg ctc agc aag gag atc cgt cct 720
Lys Gln Val Met Met Phe Ser Ala Thr Leu Ser Lys Glu Ile Arg Pro
225                 230                 235                 240
gta tgc aag aaa ttt atg caa gat ccg atg gaa ata tat gtt gat gat 768
Val Cys Lys Lys Phe Met Gln Asp Pro Met Glu Ile Tyr Val Asp Asp
                245                 250                 255
gag gca aag ttg acc ctc cat ggt ctt gta cag cac tat ata aaa ctt 816
Glu Ala Lys Leu Thr Leu His Gly Leu Val Gln His Tyr Ile Lys Leu
            260                 265                 270
agc gag gca gag aag aac cgg aaa ctg aat gat ctc ttg gac gct ctt 864
Ser Glu Ala Glu Lys Asn Arg Lys Leu Asn Asp Leu Leu Asp Ala Leu
        275                 280                 285
gat ttc aac caa gtt gtg ata ttc gtg aag agt gtt agc aga gct gcc 912
Asp Phe Asn Gln Val Val Ile Phe Val Lys Ser Val Ser Arg Ala Ala
    290                 295                 300
gaa ctg aat aag tta ctt tgt gaa tgc aac ttc cca tca atc tgc att 960
Glu Leu Asn Lys Leu Leu Cys Glu Cys Asn Phe Pro Ser Ile Cys Ile
305                 310                 315                 320
cat tct gga atg act cag gaa gaa agg cta aca aga tat aag aac ttc 1008
His Ser Gly Met Thr Gln Glu Glu Arg Leu Thr Arg Tyr Lys Asn Phe
                325                 330                 335
aag gaa ggc cac aag aga att ctt gtg gct aca gat tta gtt ggc agg 1056
Lys Glu Gly His Lys Arg Ile Leu Val Ala Thr Asp Leu Val Gly Arg
            340                 345                 350
ggc att gac att gag cgt gtc aac att gtg ata aac tat gac atg cca 1104
Gly Ile Asp Ile Glu Arg Val Asn Ile Val Ile Asn Tyr Asp Met Pro
        355                 360                 365
gac tca gct gat act tat ttg cac agg gtt gga agg gct gga cgt ttt 1152
Asp Ser Ala Asp Thr Tyr Leu His Arg Val Gly Arg Ala Gly Arg Phe
    370                 375                 380
ggc acg aag gga ctt gca att acc ttt gtt tcc tct gct tcg gat tct 1200
Gly Thr Lys Gly Leu Ala Ile Thr Phe Val Ser Ser Ala Ser Asp Ser
385                 390                 395                 400
gat gtt ctt aac cag gtg caa gaa agg ttt gag gta gac ata aag gaa 1248
Asp Val Leu Asn Gln Val Gln Glu Arg Phe Glu Val Asp Ile Lys Glu
                405                 410                 415
ctg cct gag caa att gac acc tcg acc tac atg cca tca taa 1290
Leu Pro Glu Gln Ile Asp Thr Ser Thr Tyr Met Pro Ser
            420                 425

<210> 62
<211> 429
<212> PRT
<213> Zea mays

<400> 62
Met Met Gly Glu Ala Lys Glu Asn Asp Val Tyr Glu Glu Glu Leu Leu

1                   5                   10                  15
Asp Tyr Glu Glu Asp Asp Asp Lys Thr Val Asp Gly Ser Ala Ala Lys
                20                  25                  30
Pro Thr Gly Glu Val Ala Lys Lys Gly Tyr Val Gly Ile His Ser Ser
            35                  40                  45
Gly Phe Arg Asp Phe Leu Leu Lys Pro Glu Leu Leu Arg Ala Ile Gln
    50                  55                  60
Asp Cys Gly Phe Glu His Pro Ser Glu Val Gln His Glu Cys Ile Pro
65                  70                  75                  80
Gln Ala Ile Leu Gly Met Asp Val Ile Cys Gln Ala Lys Ser Gly Met
                85                  90                  95
Gly Lys Thr Ala Val Phe Val Leu Ser Ser Leu Gln Gln Ile Asp Pro
                100                 105                 110
Val Ala Gly Gln Val Ala Ala Leu Val Leu Cys His Thr Arg Glu Leu
        115                 120                 125
Ala Tyr Gln Ile Cys His Glu Phe Glu Arg Phe Ser Lys Tyr Leu Thr
    130                 135                 140
Glu Val Lys Val Ala Val Phe Tyr Gly Gly Val His Ile Arg Lys His
145                 150                 155                 160
Lys Asp Leu Leu Lys Asn Glu Cys Pro His Ile Val Val Gly Thr Pro
                165                 170                 175
Gly Arg Ile Leu Ala Leu Ala Arg Asp Lys Asp Leu Ser Leu Lys Asn
            180                 185                 190
Val Arg His Phe Ile Leu Asp Glu Cys Asp Lys Met Leu Glu Ser Leu
        195                 200                 205
Asp Met Arg Arg Asp Val Gln Glu Ile Phe Lys Met Thr Pro His Asp
    210                 215                 220
Lys Gln Val Met Met Phe Ser Ala Thr Leu Ser Lys Glu Ile Arg Pro
225                 230                 235                 240
Val Cys Lys Lys Phe Met Gln Asp Pro Met Glu Ile Tyr Val Asp Asp
                245                 250                 255
Glu Ala Lys Leu Thr Leu His Gly Leu Val Gln His Tyr Ile Lys Leu
            260                 265                 270
Ser Glu Ala Glu Lys Asn Arg Lys Leu Asn Asp Leu Leu Asp Ala Leu
        275                 280                 285
Asp Phe Asn Gln Val Val Ile Phe Val Lys Ser Val Ser Arg Ala Ala
    290                 295                 300
Glu Leu Asn Lys Leu Leu Cys Glu Cys Asn Phe Pro Ser Ile Cys Ile
305                 310                 315                 320
His Ser Gly Met Thr Gln Glu Glu Arg Leu Thr Arg Tyr Lys Asn Phe
                325                 330                 335
Lys Glu Gly His Lys Arg Ile Leu Val Ala Thr Asp Leu Val Gly Arg
            340                 345                 350
Gly Ile Asp Ile Glu Arg Val Asn Ile Val Ile Asn Tyr Asp Met Pro
        355                 360                 365
Asp Ser Ala Asp Thr Tyr Leu His Arg Val Gly Arg Ala Gly Arg Phe
    370                 375                 380
Gly Thr Lys Gly Leu Ala Ile Thr Phe Val Ser Ser Ala Ser Asp Ser
385                 390                 395                 400
Asp Val Leu Asn Gln Val Gln Glu Arg Phe Glu Val Asp Ile Lys Glu
                405                 410                 415
Leu Pro Glu Gln Ile Asp Thr Ser Thr Tyr Met Pro Ser
            420                 425

<210> 63
<211> 1554
<212> DNA
<213> Zea mays

<220>
<221> CDS
<222> (1)..(1554)

<400> 63
atg gat cca cgc gcc agg tat cct tcg ggc atg ggc aac ggc cgc ggt      48
Met Asp Pro Arg Ala Arg Tyr Pro Ser Gly Met Gly Asn Gly Arg Gly
1               5                   10                  15
ggg acc cct aac tac tac ggc cgc ggc cca ccg ccg caa cag ccg cat      96
Gly Thr Pro Asn Tyr Tyr Gly Arg Gly Pro Pro Pro Gln Gln Pro His

                20                  25                  30
cag cac cac cac cag cag cag cag cag aca tcg ggg gcg cat cac cac          144
Gln His His His Gln Gln Gln Gln Gln Thr Ser Gly Ala His His His
            35                  40                  45
cag cag tac gcg cag agg cag cag caa cag cac cgc cac agc cac aac          192
Gln Gln Tyr Ala Gln Arg Gln Gln Gln Gln His Arg His Ser His Asn
        50                  55                  60
cac cag cag caa cag cag cag cac cag agt cag cag cag cag cag cag          240
His Gln Gln Gln Gln Gln Gln His Gln Ser Gln Gln Gln Gln Gln Gln
65                  70                  75                  80
tgg ctg cgg cgg aat cag att gcc cgc gag gcc gcg ggc gca gcc gtg          288
Trp Leu Arg Arg Asn Gln Ile Ala Arg Glu Ala Ala Gly Ala Ala Val
                85                  90                  95
acc agc gag ccg aag gcc ctg gcg cca tcc acg gcc gcc gac ggc gtc          336
Thr Ser Glu Pro Lys Ala Leu Ala Pro Ser Thr Ala Ala Asp Gly Val
            100                 105                 110
aat tcg agt tcg caa gat tgg aag acc caa ttg aag ctt ccg cct cct          384
Asn Ser Ser Ser Gln Asp Trp Lys Thr Gln Leu Lys Leu Pro Pro Pro
        115                 120                 125
gat aca cgc tac agg aca gag gat gtt aca gcc acc aag gga aat gag          432
Asp Thr Arg Tyr Arg Thr Glu Asp Val Thr Ala Thr Lys Gly Asn Glu
    130                 135                 140
ttt gaa gac tat ttt ctc aag cgt gaa ctc cta atg ggt att tac gaa          480
Phe Glu Asp Tyr Phe Leu Lys Arg Glu Leu Leu Met Gly Ile Tyr Glu
145                 150                 155                 160
aag gga ttt gaa aga ccc tct cct att caa gag gaa agc att cct att          528
Lys Gly Phe Glu Arg Pro Ser Pro Ile Gln Glu Glu Ser Ile Pro Ile
                165                 170                 175
gct tta act ggt agt gac att ctt gca aga gcg aag aat gga act ggc          576
Ala Leu Thr Gly Ser Asp Ile Leu Ala Arg Ala Lys Asn Gly Thr Gly
            180                 185                 190
aag act gct gca ttt tgt att cct gca ctg gaa aag att gat caa gaa          624
Lys Thr Ala Ala Phe Cys Ile Pro Ala Leu Glu Lys Ile Asp Gln Glu
        195                 200                 205
aaa aat gct att caa gtt gtt ata ttg gtt ccc aca agg gaa ttg gct          672
Lys Asn Ala Ile Gln Val Val Ile Leu Val Pro Thr Arg Glu Leu Ala
    210                 215                 220
cta caa aca tca caa gtt tgt aag gag ctt ggt aaa cat ttg aag atc          720
Leu Gln Thr Ser Gln Val Cys Lys Glu Leu Gly Lys His Leu Lys Ile
225                 230                 235                 240
caa gtt atg gtc acc act ggt gga acc agc ttg aag gat gat att gtt          768
Gln Val Met Val Thr Thr Gly Gly Thr Ser Leu Lys Asp Asp Ile Val
                245                 250                 255
cgt ctg tac caa cct gtg cac tta att gtt gga aca cct ggc cgt att          816
Arg Leu Tyr Gln Pro Val His Leu Ile Val Gly Thr Pro Gly Arg Ile
            260                 265                 270
ttg gat ctt aca aag aaa ggt gtt tgc att ttg aag gac tgt tca atg          864
Leu Asp Leu Thr Lys Lys Gly Val Cys Ile Leu Lys Asp Cys Ser Met
        275                 280                 285
ctt att atg gat gag gca gac aag ctt ctc tct cct gag ttt caa cct          912
Leu Ile Met Asp Glu Ala Asp Lys Leu Leu Ser Pro Glu Phe Gln Pro
    290                 295                 300
tcc ata gaa cag ttg att cgc tac ctc cct gct agt cga cag att ttg          960
Ser Ile Glu Gln Leu Ile Arg Tyr Leu Pro Ala Ser Arg Gln Ile Leu
305                 310                 315                 320
atg ttc tca gcg aca ttc cct gtg act gtt aag gag ttc aag gac aaa          1008
Met Phe Ser Ala Thr Phe Pro Val Thr Val Lys Glu Phe Lys Asp Lys
                325                 330                 335
tat ctt cct aaa cct tat gtg att aac ctt atg gat gaa ctt aca ctg          1056
Tyr Leu Pro Lys Pro Tyr Val Ile Asn Leu Met Asp Glu Leu Thr Leu
            340                 345                 350
aag gga att acg cag ttc tat gct ttt gtt gaa gaa cga cag aaa gtt          1104
Lys Gly Ile Thr Gln Phe Tyr Ala Phe Val Glu Glu Arg Gln Lys Val
        355                 360                 365
cac tgt ctc aac acc ctc ttt tcc aag ctt caa atc aat cag tca ata          1152
His Cys Leu Asn Thr Leu Phe Ser Lys Leu Gln Ile Asn Gln Ser Ile
    370                 375                 380
ata ttc tgt aac tct gta aat cgt gtt gaa cta ttg gcg aag aag atc          1200
Ile Phe Cys Asn Ser Val Asn Arg Val Glu Leu Leu Ala Lys Lys Ile

385                 390                 395                 400
aca gag ctt ggt tat tct tgc ttc tat att cat gca aag atg cta cag      1248
Thr Glu Leu Gly Tyr Ser Cys Phe Tyr Ile His Ala Lys Met Leu Gln
                405                 410                 415
gac cac cgc aat aga gtt ttc cat gat ttc cgt aat gga gcg tgc agg      1296
Asp His Arg Asn Arg Val Phe His Asp Phe Arg Asn Gly Ala Cys Arg
            420                 425                 430
aat ctt gtt tgt act gat ctt ttt aca agg gga ata gat att caa gct      1344
Asn Leu Val Cys Thr Asp Leu Phe Thr Arg Gly Ile Asp Ile Gln Ala
        435                 440                 445
gtg aat gtt gtt atc aat ttt gat ttc ccc aag aac tct gaa aca tat      1392
Val Asn Val Val Ile Asn Phe Asp Phe Pro Lys Asn Ser Glu Thr Tyr
    450                 455                 460
ctc cac agg gtt ggt aga tct ggg aga ttt ggc cac ctt ggt ctg gcc      1440
Leu His Arg Val Gly Arg Ser Gly Arg Phe Gly His Leu Gly Leu Ala
465                 470                 475                 480
gtt aat ttg atc acc tat gag gat cga ttt aac ttg tat agg att gaa      1488
Val Asn Leu Ile Thr Tyr Glu Asp Arg Phe Asn Leu Tyr Arg Ile Glu
                485                 490                 495
caa gag ctt ggg act gaa ata aaa cca att cct cct cag ata gac cag      1536
Gln Glu Leu Gly Thr Glu Ile Lys Pro Ile Pro Pro Gln Ile Asp Gln
            500                 505                 510
gct ata tat tgc caa tga                                              1554
Ala Ile Tyr Cys Gln
        515

<210> 64
<211> 517
<212> PRT
<213> Zea mays

<400> 64
Met Asp Pro Arg Ala Arg Tyr Pro Ser Gly Met Gly Asn Gly Arg Gly
1               5                   10                  15
Gly Thr Pro Asn Tyr Tyr Gly Arg Gly Pro Pro Pro Gln Gln Pro His
            20                  25                  30
Gln His His His Gln Gln Gln Gln Gln Thr Ser Gly Ala His His His
        35                  40                  45
Gln Gln Tyr Ala Gln Arg Gln Gln Gln Gln His Arg His Ser His Asn
    50                  55                  60
His Gln Gln Gln Gln Gln Gln His Gln Ser Gln Gln Gln Gln Gln Gln
65                  70                  75                  80
Trp Leu Arg Arg Asn Gln Ile Ala Arg Glu Ala Ala Gly Ala Ala Val
                85                  90                  95
Thr Ser Glu Pro Lys Ala Leu Ala Pro Ser Thr Ala Ala Asp Gly Val
            100                 105                 110
Asn Ser Ser Ser Gln Asp Trp Lys Thr Gln Leu Lys Leu Pro Pro Pro
        115                 120                 125
Asp Thr Arg Tyr Arg Thr Glu Asp Val Thr Ala Thr Lys Gly Asn Glu
    130                 135                 140
Phe Glu Asp Tyr Phe Leu Lys Arg Glu Leu Leu Met Gly Ile Tyr Glu
145                 150                 155                 160
Lys Gly Phe Glu Arg Pro Ser Pro Ile Gln Glu Glu Ser Ile Pro Ile
                165                 170                 175
Ala Leu Thr Gly Ser Asp Ile Leu Ala Arg Ala Lys Asn Gly Thr Gly
            180                 185                 190
Lys Thr Ala Ala Phe Cys Ile Pro Ala Leu Glu Lys Ile Asp Gln Glu
        195                 200                 205
Lys Asn Ala Ile Gln Val Val Ile Leu Val Pro Thr Arg Glu Leu Ala
    210                 215                 220
Leu Gln Thr Ser Gln Val Cys Lys Glu Leu Gly Lys His Leu Lys Ile
225                 230                 235                 240
Gln Val Met Val Thr Thr Gly Gly Thr Ser Leu Lys Asp Asp Ile Val
                245                 250                 255
Arg Leu Tyr Gln Pro Val His Leu Ile Val Gly Thr Pro Gly Arg Ile
            260                 265                 270
Leu Asp Leu Thr Lys Lys Gly Val Cys Ile Leu Lys Asp Cys Ser Met
        275                 280                 285
Leu Ile Met Asp Glu Ala Asp Lys Leu Leu Ser Pro Glu Phe Gln Pro

290                 295                 300
Ser Ile Glu Gln Leu Ile Arg Tyr Leu Pro Ala Ser Arg Gln Ile Leu
305                 310                 315                 320
Met Phe Ser Ala Thr Phe Pro Val Thr Val Lys Glu Phe Lys Asp Lys
                325                 330                 335
Tyr Leu Pro Lys Pro Tyr Val Ile Asn Leu Met Asp Glu Leu Thr Leu
            340                 345                 350
Lys Gly Ile Thr Gln Phe Tyr Ala Phe Val Glu Glu Arg Gln Lys Val
        355                 360                 365
His Cys Leu Asn Thr Leu Phe Ser Lys Leu Gln Ile Asn Gln Ser Ile
    370                 375                 380
Ile Phe Cys Asn Ser Val Asn Arg Val Glu Leu Leu Ala Lys Lys Ile
385                 390                 395                 400
Thr Glu Leu Gly Tyr Ser Cys Phe Tyr Ile His Ala Lys Met Leu Gln
                405                 410                 415
Asp His Arg Asn Arg Val Phe His Asp Phe Arg Asn Gly Ala Cys Arg
            420                 425                 430
Asn Leu Val Cys Thr Asp Leu Phe Thr Arg Gly Ile Asp Ile Gln Ala
        435                 440                 445
Val Asn Val Val Ile Asn Phe Asp Phe Pro Lys Asn Ser Glu Thr Tyr
    450                 455                 460
Leu His Arg Val Gly Arg Ser Gly Arg Phe Gly His Leu Gly Leu Ala
465                 470                 475                 480
Val Asn Leu Ile Thr Tyr Glu Asp Arg Phe Asn Leu Tyr Arg Ile Glu
                485                 490                 495
Gln Glu Leu Gly Thr Glu Ile Lys Pro Ile Pro Pro Gln Ile Asp Gln
            500                 505                 510
Ala Ile Tyr Cys Gln
        515

<210> 65
<211> 1926
<212> DNA
<213> Zea mays

<220>
<221> CDS
<222> (1)..(1926)

<400> 65
atg gca gca tcc acg gcg gcg gcg gcc gcg cgg aag gga gcg ctg acg      48
Met Ala Ala Ser Thr Ala Ala Ala Ala Ala Arg Lys Gly Ala Leu Thr
1               5                   10                  15
gat cag agg ttc tcg gag ctt tcc ccc gcg cta tcc ccg gag gtg gtg      96
Asp Gln Arg Phe Ser Glu Leu Ser Pro Ala Leu Ser Pro Glu Val Val
            20                  25                  30
gag gca ctg gac cgc ggc ggc ttc cag cgg tgc acg ccc gtg cag gcg      144
Glu Ala Leu Asp Arg Gly Gly Phe Gln Arg Cys Thr Pro Val Gln Ala
        35                  40                  45
gcg gcc atc ccg cac ctg ctg tcg cac aag gac gtc gcc gtc gac gcc      192
Ala Ala Ile Pro His Leu Leu Ser His Lys Asp Val Ala Val Asp Ala
    50                  55                  60
gcc aca ggc tcc ggg aag acc ctt gcc ttc atc gtc ccc gtc gtt gag      240
Ala Thr Gly Ser Gly Lys Thr Leu Ala Phe Ile Val Pro Val Val Glu
65                  70                  75                  80
atc ctc cgc cgc cgc tcc tcc ccg ccc aag tca cac gag gtc ctt gct      288
Ile Leu Arg Arg Arg Ser Ser Pro Pro Lys Ser His Glu Val Leu Ala
                85                  90                  95
ctg att atc tct ccc acg aga gaa ttg tcc tcg caa ata ttc aat gtg      336
Leu Ile Ile Ser Pro Thr Arg Glu Leu Ser Ser Gln Ile Phe Asn Val
            100                 105                 110
gcc cag ccc ttc ttc gcg acc ctg aat ggc gtg tcg tcc atg ctc ctg      384
Ala Gln Pro Phe Phe Ala Thr Leu Asn Gly Val Ser Ser Met Leu Leu
        115                 120                 125
gtg ggt ggg ctg gat atc aaa gca gag ctc aag aag gta gag gaa gag      432
Val Gly Gly Leu Asp Ile Lys Ala Glu Leu Lys Lys Val Glu Glu Glu
    130                 135                 140
ggc gca aac ata ttg gtg ggc act ccc ggg aag ctg ttt gat ata atg      480
Gly Ala Asn Ile Leu Val Gly Thr Pro Gly Lys Leu Phe Asp Ile Met

145                 150                 155                 160
cac act gat gct cta gag tac aag aat ctt gag att ttg atc cta gat      528
His Thr Asp Ala Leu Glu Tyr Lys Asn Leu Glu Ile Leu Ile Leu Asp
                165                 170                 175
gag gct gat agg ctc ttg gat atg gga ttc cag aaa cat att aac ttc      576
Glu Ala Asp Arg Leu Leu Asp Met Gly Phe Gln Lys His Ile Asn Phe
            180                 185                 190
ata ctt tca atg ttg cca aag ctg agg agg act ggg ctt ttc tca gct      624
Ile Leu Ser Met Leu Pro Lys Leu Arg Arg Thr Gly Leu Phe Ser Ala
        195                 200                 205
acc cag acg aaa gct gtt gcg gac cta tcc aaa gca ggg ttg aga aat      672
Thr Gln Thr Lys Ala Val Ala Asp Leu Ser Lys Ala Gly Leu Arg Asn
    210                 215                 220
cct ata agg gtt gag gtt aag aca gag gca aag tcc aca agc aaa gat      720
Pro Ile Arg Val Glu Val Lys Thr Glu Ala Lys Ser Thr Ser Lys Asp
225                 230                 235                 240
gct gga cag caa gag ctt ggt cca tcc ata aca cca tta ggg ctt cgg      768
Ala Gly Gln Gln Glu Leu Gly Pro Ser Ile Thr Pro Leu Gly Leu Arg
                245                 250                 255
ttg gag tat atg ata tgt gaa gca tca aag aag tca tct cag ctt gtt      816
Leu Glu Tyr Met Ile Cys Glu Ala Ser Lys Lys Ser Ser Gln Leu Val
            260                 265                 270
gat ttc ctt gtg cag aat agt ggg aag aaa ata atg gta tat ttt gca      864
Asp Phe Leu Val Gln Asn Ser Gly Lys Lys Ile Met Val Tyr Phe Ala
        275                 280                 285
aca tgt gct tgt gta gat tat tgg gct gtt gtt ctt cca cta att aag      912
Thr Cys Ala Cys Val Asp Tyr Trp Ala Val Val Leu Pro Leu Ile Lys
    290                 295                 300
tcg ctt aaa ggt tct cca ata att gct tac cat ggg aag atg aaa cag      960
Ser Leu Lys Gly Ser Pro Ile Ile Ala Tyr His Gly Lys Met Lys Gln
305                 310                 315                 320
agc ctt cga gag aaa gct ttg gca tca ttt tct gct ctt tca agt ggc      1008
Ser Leu Arg Glu Lys Ala Leu Ala Ser Phe Ser Ala Leu Ser Ser Gly
                325                 330                 335
gtt cta gtc tgc act gat gtt gca gca agg ggt ctt gac ata cca agt      1056
Val Leu Val Cys Thr Asp Val Ala Ala Arg Gly Leu Asp Ile Pro Ser
            340                 345                 350
gtt gat ctg att gtg cag tat gat cca cct caa gat ccc aac gtt ttc      1104
Val Asp Leu Ile Val Gln Tyr Asp Pro Pro Gln Asp Pro Asn Val Phe
        355                 360                 365
ata cat aga gct ggc cgt aca gca cgc tat gat caa gaa ggg gat gct      1152
Ile His Arg Ala Gly Arg Thr Ala Arg Tyr Asp Gln Glu Gly Asp Ala
    370                 375                 380
att gtt ttt ctc tta cca aag gag gat act tat gtt gag ttc ttg aag      1200
Ile Val Phe Leu Leu Pro Lys Glu Asp Thr Tyr Val Glu Phe Leu Lys
385                 390                 395                 400
ctt cga ggt gtt cct ctc aca gaa agg gaa tgc ctc gca aac act gat      1248
Leu Arg Gly Val Pro Leu Thr Glu Arg Glu Cys Leu Ala Asn Thr Asp
                405                 410                 415
gat gtc ata ccg cag att aga agt gct gcg ctc gaa gat cgt aat gtc      1296
Asp Val Ile Pro Gln Ile Arg Ser Ala Ala Leu Glu Asp Arg Asn Val
            420                 425                 430
atg gag aaa gga ctt aga gcg ttt gtt tcg ttt gtc cga gca tac aag      1344
Met Glu Lys Gly Leu Arg Ala Phe Val Ser Phe Val Arg Ala Tyr Lys
        435                 440                 445
gag cac cac tgc tct tac att ttt agg tgg aaa gat ctc gag att gga      1392
Glu His His Cys Ser Tyr Ile Phe Arg Trp Lys Asp Leu Glu Ile Gly
    450                 455                 460
aaa cta gct atg gag tat ggt tta ctc cag atc cca tcc atg cca gaa      1440
Lys Leu Ala Met Glu Tyr Gly Leu Leu Gln Ile Pro Ser Met Pro Glu
465                 470                 475                 480
gtg aag cat cat tct ctt tca cta gag gga ttt att tct gtt gat gat      1488
Val Lys His His Ser Leu Ser Leu Glu Gly Phe Ile Ser Val Asp Asp
                485                 490                 495
gtt gat att aca caa atc aag tac aag gat aaa gct cga gaa aaa caa      1536
Val Asp Ile Thr Gln Ile Lys Tyr Lys Asp Lys Ala Arg Glu Lys Gln
            500                 505                 510
cgc aag aag gcg ctg aaa agg aaa gca gaa gag gag gct caa aat cca      1584
Arg Lys Lys Ala Leu Lys Arg Lys Ala Glu Glu Glu Ala Gln Asn Pro

515                 520                 525
aag ccg gag agg aag aga cct cca gag aag ccg gag aag cca aag cgg      1632
Lys Pro Glu Arg Lys Arg Pro Pro Glu Lys Pro Glu Lys Pro Lys Arg
    530                 535                 540
aag aag acg ggc aag cag cgt cag tca atc caa act aag gag gac ctg      1680
Lys Lys Thr Gly Lys Gln Arg Gln Ser Ile Gln Thr Lys Glu Asp Leu
545                 550                 555                 560
gat gag ctg gcg cac gag tac cgg ctt ctg aag aag ctc aaa cgt gga      1728
Asp Glu Leu Ala His Glu Tyr Arg Leu Leu Lys Lys Leu Lys Arg Gly
                565                 570                 575
gac atc gat gaa gag gag tac gag aag ctc act ggg ttc gga gat tct      1776
Asp Ile Asp Glu Glu Glu Tyr Glu Lys Leu Thr Gly Phe Gly Asp Ser
            580                 585                 590
gat ggc gaa gct tcc gat ggg gat ggg agc aac ttg gac gaa agg aag      1824
Asp Gly Glu Ala Ser Asp Gly Asp Gly Ser Asn Leu Asp Glu Arg Lys
        595                 600                 605
aag ggc aac aag gcg cag aaa aag ccc aag cag aga gga aaa tgc aaa      1872
Lys Gly Asn Lys Ala Gln Lys Lys Pro Lys Gln Arg Gly Lys Cys Lys
    610                 615                 620
agt ggg tct aag aag ttc gaa ggg agg agt aaa ttg agg agc aaa aga      1920
Ser Gly Ser Lys Lys Phe Glu Gly Arg Ser Lys Leu Arg Ser Lys Arg
625                 630                 635                 640
aga tag                                                              1926
Arg


<210> 66
<211> 641
<212> PRT
<213> Zea mays

<400> 66
Met Ala Ala Ser Thr Ala Ala Ala Ala Ala Arg Lys Gly Ala Leu Thr
1               5                   10                  15
Asp Gln Arg Phe Ser Glu Leu Ser Pro Ala Leu Ser Pro Glu Val Val
            20                  25                  30
Glu Ala Leu Asp Arg Gly Gly Phe Gln Arg Cys Thr Pro Val Gln Ala
        35                  40                  45
Ala Ala Ile Pro His Leu Leu Ser His Lys Asp Val Ala Val Asp Ala
    50                  55                  60
Ala Thr Gly Ser Gly Lys Thr Leu Ala Phe Ile Val Pro Val Val Glu
65                  70                  75                  80
Ile Leu Arg Arg Arg Ser Ser Pro Pro Lys Ser His Glu Val Leu Ala
                85                  90                  95
Leu Ile Ile Ser Pro Thr Arg Glu Leu Ser Ser Gln Ile Phe Asn Val
            100                 105                 110
Ala Gln Pro Phe Phe Ala Thr Leu Asn Gly Val Ser Ser Met Leu Leu
        115                 120                 125
Val Gly Gly Leu Asp Ile Lys Ala Glu Leu Lys Lys Val Glu Glu Glu
    130                 135                 140
Gly Ala Asn Ile Leu Val Gly Thr Pro Gly Lys Leu Phe Asp Ile Met
145                 150                 155                 160
His Thr Asp Ala Leu Glu Tyr Lys Asn Leu Glu Ile Leu Ile Leu Asp
                165                 170                 175
Glu Ala Asp Arg Leu Leu Asp Met Gly Phe Gln Lys His Ile Asn Phe
            180                 185                 190
Ile Leu Ser Met Leu Pro Lys Leu Arg Arg Thr Gly Leu Phe Ser Ala
        195                 200                 205
Thr Gln Thr Lys Ala Val Ala Asp Leu Ser Lys Ala Gly Leu Arg Asn
    210                 215                 220
Pro Ile Arg Val Glu Val Lys Thr Glu Ala Lys Ser Thr Ser Lys Asp
225                 230                 235                 240
Ala Gly Gln Gln Glu Leu Gly Pro Ser Ile Thr Pro Leu Gly Leu Arg
                245                 250                 255
Leu Glu Tyr Met Ile Cys Glu Ala Ser Lys Lys Ser Ser Gln Leu Val
            260                 265                 270
Asp Phe Leu Val Gln Asn Ser Gly Lys Lys Ile Met Val Tyr Phe Ala
        275                 280                 285
Thr Cys Ala Cys Val Asp Tyr Trp Ala Val Val Leu Pro Leu Ile Lys

290                 295                 300
Ser Leu Lys Gly Ser Pro Ile Ile Ala Tyr His Gly Lys Met Lys Gln
305                 310                 315                 320
Ser Leu Arg Glu Lys Ala Leu Ala Ser Phe Ser Ala Leu Ser Ser Gly
                325                 330                 335
Val Leu Val Cys Thr Asp Val Ala Ala Arg Gly Leu Asp Ile Pro Ser
            340                 345                 350
Val Asp Leu Ile Val Gln Tyr Asp Pro Pro Gln Asp Pro Asn Val Phe
        355                 360                 365
Ile His Arg Ala Gly Arg Thr Ala Arg Tyr Asp Gln Glu Gly Asp Ala
    370                 375                 380
Ile Val Phe Leu Leu Pro Lys Glu Asp Thr Tyr Val Glu Phe Leu Lys
385                 390                 395                 400
Leu Arg Gly Val Pro Leu Thr Glu Arg Glu Cys Leu Ala Asn Thr Asp
                405                 410                 415
Asp Val Ile Pro Gln Ile Arg Ser Ala Ala Leu Glu Asp Arg Asn Val
            420                 425                 430
Met Glu Lys Gly Leu Arg Ala Phe Val Ser Phe Val Arg Ala Tyr Lys
        435                 440                 445
Glu His His Cys Ser Tyr Ile Phe Arg Trp Lys Asp Leu Glu Ile Gly
    450                 455                 460
Lys Leu Ala Met Glu Tyr Gly Leu Leu Gln Ile Pro Ser Met Pro Glu
465                 470                 475                 480
Val Lys His His Ser Leu Ser Leu Glu Gly Phe Ile Ser Val Asp Asp
                485                 490                 495
Val Asp Ile Thr Gln Ile Lys Tyr Lys Asp Lys Ala Arg Glu Lys Gln
            500                 505                 510
Arg Lys Lys Ala Leu Lys Arg Lys Ala Glu Glu Glu Ala Gln Asn Pro
        515                 520                 525
Lys Pro Glu Arg Lys Arg Pro Pro Glu Lys Pro Glu Lys Pro Lys Arg
    530                 535                 540
Lys Lys Thr Gly Lys Gln Arg Gln Ser Ile Gln Thr Lys Glu Asp Leu
545                 550                 555                 560
Asp Glu Leu Ala His Glu Tyr Arg Leu Leu Lys Lys Leu Lys Arg Gly
                565                 570                 575
Asp Ile Asp Glu Glu Glu Tyr Glu Lys Leu Thr Gly Phe Gly Asp Ser
            580                 585                 590
Asp Gly Glu Ala Ser Asp Gly Asp Gly Ser Asn Leu Asp Glu Arg Lys
        595                 600                 605
Lys Gly Asn Lys Ala Gln Lys Lys Pro Lys Gln Arg Gly Lys Cys Lys
    610                 615                 620
Ser Gly Ser Lys Lys Phe Glu Gly Arg Ser Lys Leu Arg Ser Lys Arg
625                 630                 635                 640
Arg


<210> 67
<211> 1233
<212> DNA
<213> Zea mays

<220>
<221> CDS
<222> (1)..(1233)

<400> 67
atg gca gga ttg gca cca gaa ggg tcg caa ttt gat gac aag cag tat        48
Met Ala Gly Leu Ala Pro Glu Gly Ser Gln Phe Asp Asp Lys Gln Tyr
1               5                   10                  15
gac aag aaa atg cag gag ata tta act gaa gac ttc ttc act tca tat        96
Asp Lys Lys Met Gln Glu Ile Leu Thr Glu Asp Phe Phe Thr Ser Tyr
            20                  25                  30
gat gat gtt tgt gaa agt ttt gac agc atg gga ctg caa gag aac ctt        144
Asp Asp Val Cys Glu Ser Phe Asp Ser Met Gly Leu Gln Glu Asn Leu
        35                  40                  45
ctg aga ggc atc tat gcc tat ggt ttt gag aag cca tcg gct att cag        192
Leu Arg Gly Ile Tyr Ala Tyr Gly Phe Glu Lys Pro Ser Ala Ile Gln
    50                  55                  60
caa agg gga ata gtt ccc ttc tgc aag ggg ctt gac gtg att cag caa        240

Gln Arg Gly Ile Val Pro Phe Cys Lys Gly Leu Asp Val Ile Gln Gln
65                  70                  75                  80
gct cag tct gga act gga aaa aca gct acc ttc tgt tct gga atc ttg     288
Ala Gln Ser Gly Thr Gly Lys Thr Ala Thr Phe Cys Ser Gly Ile Leu
                85                  90                  95
cag caa ctt gat tat gga ttg gtt gaa tgc cag gca ttg gtt ctt gct     336
Gln Gln Leu Asp Tyr Gly Leu Val Glu Cys Gln Ala Leu Val Leu Ala
            100                 105                 110
cca acc cgt gag ctt gct cag caa att gag aag gtt atg cgt gct ctt     384
Pro Thr Arg Glu Leu Ala Gln Gln Ile Glu Lys Val Met Arg Ala Leu
        115                 120                 125
ggg gac tat ttg ggt gtc aaa gtg cat gcc tgt gtt ggt gga aca tct     432
Gly Asp Tyr Leu Gly Val Lys Val His Ala Cys Val Gly Gly Thr Ser
    130                 135                 140
gtt cgt gag gac caa agg att ctt gcc agt ggt gtg cat gtt gtt gtg     480
Val Arg Glu Asp Gln Arg Ile Leu Ala Ser Gly Val His Val Val Val
145                 150                 155                 160
ggc aca cca gga cgt gtg ttt gat atg ttg cgc agg cag tct ctc cgt     528
Gly Thr Pro Gly Arg Val Phe Asp Met Leu Arg Arg Gln Ser Leu Arg
                165                 170                 175
ccc gac aac atc aag atg ttt gtg ttg gat gag gct gat gag atg ctc     576
Pro Asp Asn Ile Lys Met Phe Val Leu Asp Glu Ala Asp Glu Met Leu
            180                 185                 190
tcc cgt gga ttc aag gat cag atc tat gat atc ttc cag ctt ctt cca     624
Ser Arg Gly Phe Lys Asp Gln Ile Tyr Asp Ile Phe Gln Leu Leu Pro
        195                 200                 205
tcc aag att cag gtc ggt gtc ttc tca gct acc atg ccc cct gag gcc     672
Ser Lys Ile Gln Val Gly Val Phe Ser Ala Thr Met Pro Pro Glu Ala
    210                 215                 220
ctt gag atc acg cgc aag ttc atg aac aaa cct gtg agg att ctc gtc     720
Leu Glu Ile Thr Arg Lys Phe Met Asn Lys Pro Val Arg Ile Leu Val
225                 230                 235                 240
aag aga gat gag ctc acc ctt gag ggt atc aag caa ttc tat gtc aat     768
Lys Arg Asp Glu Leu Thr Leu Glu Gly Ile Lys Gln Phe Tyr Val Asn
                245                 250                 255
atc gac aag gaa gat tgg aag ctc gac act ctg tgt gac ctg tat gag     816
Ile Asp Lys Glu Asp Trp Lys Leu Asp Thr Leu Cys Asp Leu Tyr Glu
            260                 265                 270
acc ctg gca atc acc cag agt gtc atc ttt gtg aac aca cgc cgc aag     864
Thr Leu Ala Ile Thr Gln Ser Val Ile Phe Val Asn Thr Arg Arg Lys
        275                 280                 285
gtg gac tgg ctc acg gac aag atg agg agc agg gac cac acc gtg tca     912
Val Asp Trp Leu Thr Asp Lys Met Arg Ser Arg Asp His Thr Val Ser
    290                 295                 300
gct aca cac ggt gat atg gac cag aac acc agg gac atc atc atg agg     960
Ala Thr His Gly Asp Met Asp Gln Asn Thr Arg Asp Ile Ile Met Arg
305                 310                 315                 320
gag ttc agg tcc ggg tcc tct cgt gtg ctc atc acc acc gac ctg ctc     1008
Glu Phe Arg Ser Gly Ser Ser Arg Val Leu Ile Thr Thr Asp Leu Leu
                325                 330                 335
gcc cgt ggt atc gac gtc cag cag gtc tca ctt gtc atc aac tac gac     1056
Ala Arg Gly Ile Asp Val Gln Gln Val Ser Leu Val Ile Asn Tyr Asp
            340                 345                 350
ctg ccg acc cag cca gag aac tac ctc cac cgc atc ggc cgt agt ggt     1104
Leu Pro Thr Gln Pro Glu Asn Tyr Leu His Arg Ile Gly Arg Ser Gly
        355                 360                 365
cgg ttt gga agg aag ggt gtt gcc atc aac ttc gtc acc cgc gac gac     1152
Arg Phe Gly Arg Lys Gly Val Ala Ile Asn Phe Val Thr Arg Asp Asp
    370                 375                 380
gag cga atg ctg ttt gac atc cag agg ttc tac aac gtg acc gtt gag     1200
Glu Arg Met Leu Phe Asp Ile Gln Arg Phe Tyr Asn Val Thr Val Glu
385                 390                 395                 400
gag ctg ccg gcc aat gtc gcc gac ctt ctc tag                         1233
Glu Leu Pro Ala Asn Val Ala Asp Leu Leu
                405                 410

<210> 68
<211> 410
<212> PRT

<213> Zea mays

<400> 68
Met Ala Gly Leu Ala Pro Glu Gly Ser Gln Phe Asp Asp Lys Gln Tyr
1               5                   10                  15
Asp Lys Lys Met Gln Glu Ile Leu Thr Glu Asp Phe Phe Thr Ser Tyr
            20                  25                  30
Asp Asp Val Cys Glu Ser Phe Asp Ser Met Gly Leu Gln Glu Asn Leu
        35                  40                  45
Leu Arg Gly Ile Tyr Ala Tyr Gly Phe Glu Lys Pro Ser Ala Ile Gln
    50                  55                  60
Gln Arg Gly Ile Val Pro Phe Cys Lys Gly Leu Asp Val Ile Gln Gln
65                  70                  75                  80
Ala Gln Ser Gly Thr Gly Lys Thr Ala Thr Phe Cys Ser Gly Ile Leu
                85                  90                  95
Gln Gln Leu Asp Tyr Gly Leu Val Glu Cys Gln Ala Leu Val Leu Ala
            100                 105                 110
Pro Thr Arg Glu Leu Ala Gln Gln Ile Glu Lys Val Met Arg Ala Leu
        115                 120                 125
Gly Asp Tyr Leu Gly Val Lys Val His Ala Cys Val Gly Gly Thr Ser
    130                 135                 140
Val Arg Glu Asp Gln Arg Ile Leu Ala Ser Gly Val His Val Val Val
145                 150                 155                 160
Gly Thr Pro Gly Arg Val Phe Asp Met Leu Arg Arg Gln Ser Leu Arg
                165                 170                 175
Pro Asp Asn Ile Lys Met Phe Val Leu Asp Glu Ala Asp Glu Met Leu
            180                 185                 190
Ser Arg Gly Phe Lys Asp Gln Ile Tyr Asp Ile Phe Gln Leu Leu Pro
        195                 200                 205
Ser Lys Ile Gln Val Gly Val Phe Ser Ala Thr Met Pro Pro Glu Ala
    210                 215                 220
Leu Glu Ile Thr Arg Lys Phe Met Asn Lys Pro Val Arg Ile Leu Val
225                 230                 235                 240
Lys Arg Asp Glu Leu Thr Leu Glu Gly Ile Lys Gln Phe Tyr Val Asn
                245                 250                 255
Ile Asp Lys Glu Asp Trp Lys Leu Asp Thr Leu Cys Asp Leu Tyr Glu
            260                 265                 270
Thr Leu Ala Ile Thr Gln Ser Val Ile Phe Val Asn Thr Arg Arg Lys
        275                 280                 285
Val Asp Trp Leu Thr Asp Lys Met Arg Ser Arg Asp His Thr Val Ser
    290                 295                 300
Ala Thr His Gly Asp Met Asp Gln Asn Thr Arg Asp Ile Ile Met Arg
305                 310                 315                 320
Glu Phe Arg Ser Gly Ser Ser Arg Val Leu Ile Thr Thr Asp Leu Leu
                325                 330                 335
Ala Arg Gly Ile Asp Val Gln Gln Val Ser Leu Val Ile Asn Tyr Asp
            340                 345                 350
Leu Pro Thr Gln Pro Glu Asn Tyr Leu His Arg Ile Gly Arg Ser Gly
        355                 360                 365
Arg Phe Gly Arg Lys Gly Val Ala Ile Asn Phe Val Thr Arg Asp Asp
    370                 375                 380
Glu Arg Met Leu Phe Asp Ile Gln Arg Phe Tyr Asn Val Thr Val Glu
385                 390                 395                 400
Glu Leu Pro Ala Asn Val Ala Asp Leu Leu
                405                 410

<210> 69
<211> 1224
<212> DNA
<213> Zea mays

<220>
<221> CDS
<222> (1)..(1224)

<400> 69
atg gcg gcg ccc acc acc tcc cgc cgc ggc ccc ggc ggc gcg cgc aac      48
Met Ala Ala Pro Thr Thr Ser Arg Arg Gly Pro Gly Gly Ala Arg Asn
1               5                   10                  15

atg gac gac gag aac cta acc ttc gag acc tcc ccg ggg gtc gag gtc 96
Met Asp Asp Glu Asn Leu Thr Phe Glu Thr Ser Pro Gly Val Glu Val
            20                  25                  30
gtc agc agc ttc gac cag atg ggc atc aag gac gac ctc ctc cgc ggc 144
Val Ser Ser Phe Asp Gln Met Gly Ile Lys Asp Asp Leu Leu Arg Gly
        35                  40                  45
atc tac ggc tac ggt ttc gag aag ccc tcc gcc atc cag cag cgc gcc 192
Ile Tyr Gly Tyr Gly Phe Glu Lys Pro Ser Ala Ile Gln Gln Arg Ala
    50                  55                  60
gtg ctc ccc atc atc aac ggc cgc gac gtc atc gcg cag gcc cag tcc 240
Val Leu Pro Ile Ile Asn Gly Arg Asp Val Ile Ala Gln Ala Gln Ser
65                  70                  75                  80
ggt acc ggc aag acg tcc atg atc tcc ctc acc gtc tgc cag atc gtc 288
Gly Thr Gly Lys Thr Ser Met Ile Ser Leu Thr Val Cys Gln Ile Val
                85                  90                  95
gac acc gcc gtg cgc gag gtg cag gct ttg att ctc tcg cct act agg 336
Asp Thr Ala Val Arg Glu Val Gln Ala Leu Ile Leu Ser Pro Thr Arg
            100                 105                 110
gag ctt gct tcc caa aca gag aga gtt atg ctg gct att ggg gac cac 384
Glu Leu Ala Ser Gln Thr Glu Arg Val Met Leu Ala Ile Gly Asp His
        115                 120                 125
ctc aac gtc caa gtg cac gct tgc att ggt gga aag agt att ggt gag 432
Leu Asn Val Gln Val His Ala Cys Ile Gly Gly Lys Ser Ile Gly Glu
    130                 135                 140
gac atc agg agg ctc gag aat ggg gtg cat gtc gtc tct gga act cca 480
Asp Ile Arg Arg Leu Glu Asn Gly Val His Val Val Ser Gly Thr Pro
145                 150                 155                 160
ggc aga gtc tgt gat atg atc aag agg agg aca ctg cgg acc aga gcc 528
Gly Arg Val Cys Asp Met Ile Lys Arg Arg Thr Leu Arg Thr Arg Ala
                165                 170                 175
atc aag ctt cta gtt ctg gat gaa gct gat gag atg ttg agt aga ggc 576
Ile Lys Leu Leu Val Leu Asp Glu Ala Asp Glu Met Leu Ser Arg Gly
            180                 185                 190
ttt aag gat cag att tac gat gtc tac aga tat ctc cct cca gaa ctt 624
Phe Lys Asp Gln Ile Tyr Asp Val Tyr Arg Tyr Leu Pro Pro Glu Leu
        195                 200                 205
cag gtg gtt ttg atc tct gca act ctt cct cat gag atc ttg gag atg 672
Gln Val Val Leu Ile Ser Ala Thr Leu Pro His Glu Ile Leu Glu Met
    210                 215                 220
act agt aag ttc atg acc gaa ccg gtt agg atc ctt gtg aag cgt gat 720
Thr Ser Lys Phe Met Thr Glu Pro Val Arg Ile Leu Val Lys Arg Asp
225                 230                 235                 240
gag ctg acc ctg gag ggc atc aaa caa ttc ttt gtt gct gtt gag aaa 768
Glu Leu Thr Leu Glu Gly Ile Lys Gln Phe Phe Val Ala Val Glu Lys
                245                 250                 255
gag gag tgg aag ttt gat aca ctt tgt gat ctt tat gat aca ctc acc 816
Glu Glu Trp Lys Phe Asp Thr Leu Cys Asp Leu Tyr Asp Thr Leu Thr
            260                 265                 270
atc acc caa gct gtt att ttc tgc aat act aag aga aag gtg gat tgg 864
Ile Thr Gln Ala Val Ile Phe Cys Asn Thr Lys Arg Lys Val Asp Trp
        275                 280                 285
ctc act gaa aaa atg cgt aca aat aac ttc acc gta tca gct atg cac 912
Leu Thr Glu Lys Met Arg Thr Asn Asn Phe Thr Val Ser Ala Met His
    290                 295                 300
ggt gac atg ccc caa caa gaa agg gat gcc att atg ggt gag ttc agg 960
Gly Asp Met Pro Gln Gln Glu Arg Asp Ala Ile Met Gly Glu Phe Arg
305                 310                 315                 320
tct ggc gca act cgt gtg cta ata act aca gat gtt tgg gct cga gga 1008
Ser Gly Ala Thr Arg Val Leu Ile Thr Thr Asp Val Trp Ala Arg Gly
                325                 330                 335
ctg gac gtt cag cag gtt tca ctt gtc ata aat tat gat ctc cca aat 1056
Leu Asp Val Gln Gln Val Ser Leu Val Ile Asn Tyr Asp Leu Pro Asn
            340                 345                 350
aat cgt gag ctt tac atc cat cgc att ggt cgt tct ggt cgt ttt ggg 1104
Asn Arg Glu Leu Tyr Ile His Arg Ile Gly Arg Ser Gly Arg Phe Gly
        355                 360                 365
cgc aag ggt gtg gcg atc aat ttc gtg cgc aag gat gac atc cgt ata 1152
Arg Lys Gly Val Ala Ile Asn Phe Val Arg Lys Asp Asp Ile Arg Ile
    370                 375                 380

ctg aga gat ata gag cag tac tac agc aca caa att gac gag atg cca        1200
Leu Arg Asp Ile Glu Gln Tyr Tyr Ser Thr Gln Ile Asp Glu Met Pro
385                 390                 395                 400
atg aat gtt gct gat ctt att tga                                        1224
Met Asn Val Ala Asp Leu Ile
                405

<210> 70
<211> 407
<212> PRT
<213> Zea mays

<400> 70
Met Ala Ala Pro Thr Thr Ser Arg Arg Gly Pro Gly Gly Ala Arg Asn
1               5                   10                  15
Met Asp Asp Glu Asn Leu Thr Phe Glu Thr Ser Pro Gly Val Glu Val
            20                  25                  30
Val Ser Ser Phe Asp Gln Met Gly Ile Lys Asp Asp Leu Leu Arg Gly
        35                  40                  45
Ile Tyr Gly Tyr Gly Phe Glu Lys Pro Ser Ala Ile Gln Gln Arg Ala
    50                  55                  60
Val Leu Pro Ile Ile Asn Gly Arg Asp Val Ile Ala Gln Ala Gln Ser
65                  70                  75                  80
Gly Thr Gly Lys Thr Ser Met Ile Ser Leu Thr Val Cys Gln Ile Val
                85                  90                  95
Asp Thr Ala Val Arg Glu Val Gln Ala Leu Ile Leu Ser Pro Thr Arg
            100                 105                 110
Glu Leu Ala Ser Gln Thr Glu Arg Val Met Leu Ala Ile Gly Asp His
        115                 120                 125
Leu Asn Val Gln Val His Ala Cys Ile Gly Gly Lys Ser Ile Gly Glu
    130                 135                 140
Asp Ile Arg Arg Leu Glu Asn Gly Val His Val Val Ser Gly Thr Pro
145                 150                 155                 160
Gly Arg Val Cys Asp Met Ile Lys Arg Arg Thr Leu Arg Thr Arg Ala
                165                 170                 175
Ile Lys Leu Leu Val Leu Asp Glu Ala Asp Glu Met Leu Ser Arg Gly
            180                 185                 190
Phe Lys Asp Gln Ile Tyr Asp Val Tyr Arg Tyr Leu Pro Pro Glu Leu
        195                 200                 205
Gln Val Val Leu Ile Ser Ala Thr Leu Pro His Glu Ile Leu Glu Met
    210                 215                 220
Thr Ser Lys Phe Met Thr Glu Pro Val Arg Ile Leu Val Lys Arg Asp
225                 230                 235                 240
Glu Leu Thr Leu Glu Gly Ile Lys Gln Phe Phe Val Ala Val Glu Lys
                245                 250                 255
Glu Glu Trp Lys Phe Asp Thr Leu Cys Asp Leu Tyr Asp Thr Leu Thr
            260                 265                 270
Ile Thr Gln Ala Val Ile Phe Cys Asn Thr Lys Arg Lys Val Asp Trp
        275                 280                 285
Leu Thr Glu Lys Met Arg Thr Asn Asn Phe Thr Val Ser Ala Met His
    290                 295                 300
Gly Asp Met Pro Gln Gln Glu Arg Asp Ala Ile Met Gly Glu Phe Arg
305                 310                 315                 320
Ser Gly Ala Thr Arg Val Leu Ile Thr Thr Asp Val Trp Ala Arg Gly
                325                 330                 335
Leu Asp Val Gln Gln Val Ser Leu Val Ile Asn Tyr Asp Leu Pro Asn
            340                 345                 350
Asn Arg Glu Leu Tyr Ile His Arg Ile Gly Arg Ser Gly Arg Phe Gly
        355                 360                 365
Arg Lys Gly Val Ala Ile Asn Phe Val Arg Lys Asp Asp Ile Arg Ile
    370                 375                 380
Leu Arg Asp Ile Glu Gln Tyr Tyr Ser Thr Gln Ile Asp Glu Met Pro
385                 390                 395                 400
Met Asn Val Ala Asp Leu Ile
                405

<210> 71
<211> 1944
<212> DNA

<213> Zea mays

<220>
<221> CDS
<222> (1)..(1944)

<400> 71
atg cga tct tca tgg gct gac tca gtt gcg aac gcc gag gaa tcg gcg 48
Met Arg Ser Ser Trp Ala Asp Ser Val Ala Asn Ala Glu Glu Ser Ala
1               5                   10                  15
ccc gcg act gct gct gct aat ggc tct gtt gca act cat agc acc tcg 96
Pro Ala Thr Ala Ala Ala Asn Gly Ser Val Ala Thr His Ser Thr Ser
            20                  25                  30
cgc cct acg cgc agc tcc tac gtg cct cca cat ctt cgt ggc cgc tca 144
Arg Pro Thr Arg Ser Ser Tyr Val Pro Pro His Leu Arg Gly Arg Ser
        35                  40                  45
gct ggt gct gct gtt gaa gct caa gca ggc tta gta gca cca gca caa 192
Ala Gly Ala Ala Val Glu Ala Gln Ala Gly Leu Val Ala Pro Ala Gln
    50                  55                  60
ggt gga cca ctg cca ttg gct gct gca caa cct tct ggt cag ggt gct 240
Gly Gly Pro Leu Pro Leu Ala Ala Ala Gln Pro Ser Gly Gln Gly Ala
65                  70                  75                  80
gct gtt ggt ggc cct cgc tgg gct ggc att gtg aat ggt ggt ggt ggt 288
Ala Val Gly Gly Pro Arg Trp Ala Gly Ile Val Asn Gly Gly Gly Gly
                85                  90                  95
ggt ggc agc att ggt gct cct cgc cag ggc cat ggt ggc gga ggc ggg 336
Gly Gly Ser Ile Gly Ala Pro Arg Gln Gly His Gly Gly Gly Gly Gly
            100                 105                 110
ggc cgt ggt gct tgg aac tcc cgt cct ggt ggt tgg gac cgc aga gac 384
Gly Arg Gly Ala Trp Asn Ser Arg Pro Gly Gly Trp Asp Arg Arg Asp
        115                 120                 125
cgg gag cca gat ccg ttt gcg aaa gcc gag gct gaa gaa att gat ttc 432
Arg Glu Pro Asp Pro Phe Ala Lys Ala Glu Ala Glu Glu Ile Asp Phe
    130                 135                 140
gac ggc cag gag aat act ggc atc aat ttt gat gcc tat gaa gac atc 480
Asp Gly Gln Glu Asn Thr Gly Ile Asn Phe Asp Ala Tyr Glu Asp Ile
145                 150                 155                 160
cct gtt gag acc agt ggc cat gat gtg cct gca cca gtc aac aca ttt 528
Pro Val Glu Thr Ser Gly His Asp Val Pro Ala Pro Val Asn Thr Phe
                165                 170                 175
gca gag att gat ttg ggt gat gca ttg aat gac aat atc cgg agg tgc 576
Ala Glu Ile Asp Leu Gly Asp Ala Leu Asn Asp Asn Ile Arg Arg Cys
            180                 185                 190
aag tat gtg aaa cca aca cca gtg cag cgt tac gcc atc cca atc tcc 624
Lys Tyr Val Lys Pro Thr Pro Val Gln Arg Tyr Ala Ile Pro Ile Ser
        195                 200                 205
att gct ggg cgg gat ctc atg gcc tgc gca cag act gga tcc gga aaa 672
Ile Ala Gly Arg Asp Leu Met Ala Cys Ala Gln Thr Gly Ser Gly Lys
    210                 215                 220
acc gcc gca ttc tgt ttt cca atc atc agt ggg atc ttg aag tca cca 720
Thr Ala Ala Phe Cys Phe Pro Ile Ile Ser Gly Ile Leu Lys Ser Pro
225                 230                 235                 240
aag cca cac cag agg tca cgg agt aca agg acc gct tgc cct cta gct 768
Lys Pro His Gln Arg Ser Arg Ser Thr Arg Thr Ala Cys Pro Leu Ala
                245                 250                 255
ctg atc tta tca ccc act cgt gag ctt tca gtc caa atc cat gaa gaa 816
Leu Ile Leu Ser Pro Thr Arg Glu Leu Ser Val Gln Ile His Glu Glu
            260                 265                 270
gca agg aag ttt gca tac cag act ggt gtc aga gtt gtg gtt gca tat 864
Ala Arg Lys Phe Ala Tyr Gln Thr Gly Val Arg Val Val Val Ala Tyr
        275                 280                 285
ggt ggc gca cca ata act aac cag ctg agg gag tta gag aga ggt gtg 912
Gly Gly Ala Pro Ile Thr Asn Gln Leu Arg Glu Leu Glu Arg Gly Val
    290                 295                 300
gaa atc ctg gtg gca act cct ggt cgc ttg atg gat ctg ttg gag agg 960
Glu Ile Leu Val Ala Thr Pro Gly Arg Leu Met Asp Leu Leu Glu Arg
305                 310                 315                 320
gct aga gtc tca ctt caa atg ata aag tat tta gct ctt gat gaa gct 1008
Ala Arg Val Ser Leu Gln Met Ile Lys Tyr Leu Ala Leu Asp Glu Ala

325                 330                 335
gat cgg atg ctt gat atg ggt ttt gag cca cag ata cgt aaa att gtt        1056
Asp Arg Met Leu Asp Met Gly Phe Glu Pro Gln Ile Arg Lys Ile Val
            340                 345                 350
gag ggc atg gac atg cct caa cgt ggt gag agg cag aca atg ttg ttc        1104
Glu Gly Met Asp Met Pro Gln Arg Gly Glu Arg Gln Thr Met Leu Phe
        355                 360                 365
agt gca aca ttc cca aaa gag ata cag agg atg gcc gca gat ttc ctt        1152
Ser Ala Thr Phe Pro Lys Glu Ile Gln Arg Met Ala Ala Asp Phe Leu
    370                 375                 380
gct gat tac atc ttt ctt gct gtt ggg aga gtt ggt tca agc acc gat        1200
Ala Asp Tyr Ile Phe Leu Ala Val Gly Arg Val Gly Ser Ser Thr Asp
385                 390                 395                 400
ttg att gtt cag agg gtg gag ttt gtc ctc gct tca gac aaa cga agc        1248
Leu Ile Val Gln Arg Val Glu Phe Val Leu Ala Ser Asp Lys Arg Ser
                405                 410                 415
tac ctc atg gat ctt ctg cat gca caa aag gct aat ggc aca cat gga        1296
Tyr Leu Met Asp Leu Leu His Ala Gln Lys Ala Asn Gly Thr His Gly
            420                 425                 430
aag cac gct ctt aca ttg gtc ttt gtg gag aca aag agg ggg gct gat        1344
Lys His Ala Leu Thr Leu Val Phe Val Glu Thr Lys Arg Gly Ala Asp
        435                 440                 445
gcc ttg gaa gac ggg ttt ttt aga aat gga cat cca tgg act agc att        1392
Ala Leu Glu Asp Gly Phe Phe Arg Asn Gly His Pro Trp Thr Ser Ile
    450                 455                 460
cat gga gac agg aca caa cag gaa agg gag cat gcc ctt agg tcc ttc        1440
His Gly Asp Arg Thr Gln Gln Glu Arg Glu His Ala Leu Arg Ser Phe
465                 470                 475                 480
aag agc gga gca act ccc atc ctt gtg gcg act gat gtt gct gct cgt        1488
Lys Ser Gly Ala Thr Pro Ile Leu Val Ala Thr Asp Val Ala Ala Arg
                485                 490                 495
ggt ctt gac ata cca cat gtt gcc cat gtg att aat ttt gac ctc cct        1536
Gly Leu Asp Ile Pro His Val Ala His Val Ile Asn Phe Asp Leu Pro
            500                 505                 510
aat gat ata gat gac tat gtt cat cgg att gga agg act gga cgt gct        1584
Asn Asp Ile Asp Asp Tyr Val His Arg Ile Gly Arg Thr Gly Arg Ala
        515                 520                 525
ggg aaa tct ggt ctt gcg act gca ttc ttc aac gag agc aac act acg        1632
Gly Lys Ser Gly Leu Ala Thr Ala Phe Phe Asn Glu Ser Asn Thr Thr
    530                 535                 540
ctt gca agg ccg ttg agt gat ctc atg aaa gag gcc aac cag gag gtt        1680
Leu Ala Arg Pro Leu Ser Asp Leu Met Lys Glu Ala Asn Gln Glu Val
545                 550                 555                 560
cct aag tgg ctt gag gga tac gct gcc cga tca gcc tac gga ggt gga        1728
Pro Lys Trp Leu Glu Gly Tyr Ala Ala Arg Ser Ala Tyr Gly Gly Gly
                565                 570                 575
ggt ggt aga aac cgc aga caa ggt agc agt gcc aga ttt ggt ggc cgt        1776
Gly Gly Arg Asn Arg Arg Gln Gly Ser Ser Ala Arg Phe Gly Gly Arg
            580                 585                 590
gat ttc cgg cga gat agg ggc agt ggt gga gga tat ggt ggt ggt tcc        1824
Asp Phe Arg Arg Asp Arg Gly Ser Gly Gly Gly Tyr Gly Gly Gly Ser
        595                 600                 605
tac gga gga ggt ggt ggt ggt gga tat ggg gga tca tca gga tac ggt        1872
Tyr Gly Gly Gly Gly Gly Gly Gly Tyr Gly Gly Ser Ser Gly Tyr Gly
    610                 615                 620
ggt gcc tat ggc ggc ggt ggc agc agc ggc ggc ggc tat ggt ggt ggt        1920
Gly Ala Tyr Gly Gly Gly Gly Ser Ser Gly Gly Gly Tyr Gly Gly Gly
625                 630                 635                 640
cag agt acg agt tct tgg gac tga                                        1944
Gln Ser Thr Ser Ser Trp Asp
                645

<210> 72
<211> 647
<212> PRT
<213> Zea mays

<400> 72
Met Arg Ser Ser Trp Ala Asp Ser Val Ala Asn Ala Glu Glu Ser Ala

1                   5                   10                  15
Pro Ala Thr Ala Ala Ala Asn Gly Ser Val Ala Thr His Ser Thr Ser
                20                  25                  30
Arg Pro Thr Arg Ser Ser Tyr Val Pro Pro His Leu Arg Gly Arg Ser
            35                  40                  45
Ala Gly Ala Ala Val Glu Ala Gln Ala Gly Leu Val Ala Pro Ala Gln
        50                  55                  60
Gly Gly Pro Leu Pro Leu Ala Ala Ala Gln Pro Ser Gly Gln Gly Ala
65                  70                  75                  80
Ala Val Gly Gly Pro Arg Trp Ala Gly Ile Val Asn Gly Gly Gly Gly
                85                  90                  95
Gly Gly Ser Ile Gly Ala Pro Arg Gln Gly His Gly Gly Gly Gly Gly
            100                 105                 110
Gly Arg Gly Ala Trp Asn Ser Arg Pro Gly Gly Trp Asp Arg Arg Asp
        115                 120                 125
Arg Glu Pro Asp Pro Phe Ala Lys Ala Glu Ala Glu Glu Ile Asp Phe
    130                 135                 140
Asp Gly Gln Glu Asn Thr Gly Ile Asn Phe Asp Ala Tyr Glu Asp Ile
145                 150                 155                 160
Pro Val Glu Thr Ser Gly His Asp Val Pro Ala Pro Val Asn Thr Phe
                165                 170                 175
Ala Glu Ile Asp Leu Gly Asp Ala Leu Asn Asp Asn Ile Arg Arg Cys
            180                 185                 190
Lys Tyr Val Lys Pro Thr Pro Val Gln Arg Tyr Ala Ile Pro Ile Ser
        195                 200                 205
Ile Ala Gly Arg Asp Leu Met Ala Cys Ala Gln Thr Gly Ser Gly Lys
    210                 215                 220
Thr Ala Ala Phe Cys Phe Pro Ile Ile Ser Gly Ile Leu Lys Ser Pro
225                 230                 235                 240
Lys Pro His Gln Arg Ser Arg Ser Thr Arg Thr Ala Cys Pro Leu Ala
                245                 250                 255
Leu Ile Leu Ser Pro Thr Arg Glu Leu Ser Val Gln Ile His Glu Glu
            260                 265                 270
Ala Arg Lys Phe Ala Tyr Gln Thr Gly Val Arg Val Val Val Ala Tyr
        275                 280                 285
Gly Gly Ala Pro Ile Thr Asn Gln Leu Arg Glu Leu Glu Arg Gly Val
    290                 295                 300
Glu Ile Leu Val Ala Thr Pro Gly Arg Leu Met Asp Leu Leu Glu Arg
305                 310                 315                 320
Ala Arg Val Ser Leu Gln Met Ile Lys Tyr Leu Ala Leu Asp Glu Ala
                325                 330                 335
Asp Arg Met Leu Asp Met Gly Phe Glu Pro Gln Ile Arg Lys Ile Val
            340                 345                 350
Glu Gly Met Asp Met Pro Gln Arg Gly Glu Arg Gln Thr Met Leu Phe
        355                 360                 365
Ser Ala Thr Phe Pro Lys Glu Ile Gln Arg Met Ala Ala Asp Phe Leu
    370                 375                 380
Ala Asp Tyr Ile Phe Leu Ala Val Gly Arg Val Gly Ser Ser Thr Asp
385                 390                 395                 400
Leu Ile Val Gln Arg Val Glu Phe Val Leu Ala Ser Asp Lys Arg Ser
                405                 410                 415
Tyr Leu Met Asp Leu Leu His Ala Gln Lys Ala Asn Gly Thr His Gly
            420                 425                 430
Lys His Ala Leu Thr Leu Val Phe Val Glu Thr Lys Arg Gly Ala Asp
        435                 440                 445
Ala Leu Glu Asp Gly Phe Phe Arg Asn Gly His Pro Trp Thr Ser Ile
    450                 455                 460
His Gly Asp Arg Thr Gln Gln Glu Arg Glu His Ala Leu Arg Ser Phe
465                 470                 475                 480
Lys Ser Gly Ala Thr Pro Ile Leu Val Ala Thr Asp Val Ala Ala Arg
                485                 490                 495
Gly Leu Asp Ile Pro His Val Ala His Val Ile Asn Phe Asp Leu Pro
            500                 505                 510
Asn Asp Ile Asp Asp Tyr Val His Arg Ile Gly Arg Thr Gly Arg Ala
        515                 520                 525
Gly Lys Ser Gly Leu Ala Thr Ala Phe Phe Asn Glu Ser Asn Thr Thr
    530                 535                 540
Leu Ala Arg Pro Leu Ser Asp Leu Met Lys Glu Ala Asn Gln Glu Val
545                 550                 555                 560

Pro Lys Trp Leu Glu Gly Tyr Ala Ala Arg Ser Ala Tyr Gly Gly Gly
                565                 570                 575
Gly Gly Arg Asn Arg Arg Gln Gly Ser Ser Ala Arg Phe Gly Gly Arg
            580                 585                 590
Asp Phe Arg Arg Asp Arg Gly Ser Gly Gly Gly Tyr Gly Gly Gly Ser
        595                 600                 605
Tyr Gly Gly Gly Gly Gly Gly Gly Tyr Gly Gly Ser Ser Gly Tyr Gly
    610                 615                 620
Gly Ala Tyr Gly Gly Gly Gly Ser Ser Gly Gly Gly Tyr Gly Gly Gly
625                 630                 635                 640
Gln Ser Thr Ser Ser Trp Asp
                645

<210> 73
<211> 1584
<212> DNA
<213> Zea mays

<220>
<221> CDS
<222> (1)..(1584)

<400> 73
atg gat cca cgc gcc agg tat cct tcg ggc atg ggc aac ggc cgc ggt      48
Met Asp Pro Arg Ala Arg Tyr Pro Ser Gly Met Gly Asn Gly Arg Gly
1               5                   10                  15
ggg aac ctc aac tac tac ggc cgc ggc cca ccg ccg caa cag ccg cat      96
Gly Asn Leu Asn Tyr Tyr Gly Arg Gly Pro Pro Pro Gln Gln Pro His
            20                  25                  30
cag cac cac aac cac cag cag acg tcg ggg gcg cac cac cac cag cag      144
Gln His His Asn His Gln Gln Thr Ser Gly Ala His His His Gln Gln
        35                  40                  45
acg tcg ggg gcg cac cac cac cag cag tac gcg cag agg cag cac cag      192
Thr Ser Gly Ala His His His Gln Gln Tyr Ala Gln Arg Gln His Gln
    50                  55                  60
cac cag cac cag cac cac ggc cac aat cgc cat cag caa cag cag cag      240
His Gln His Gln His His Gly His Asn Arg His Gln Gln Gln Gln Gln
65                  70                  75                  80
cac cac att tac cag cag caa cag cag cag tgg ctg cgg cgg agc cag      288
His His Ile Tyr Gln Gln Gln Gln Gln Gln Trp Leu Arg Arg Ser Gln
                85                  90                  95
att gcc cgc gag acc tcg ggc gca gcc gtg acc agc gag ccg aag tcc      336
Ile Ala Arg Glu Thr Ser Gly Ala Ala Val Thr Ser Glu Pro Lys Ser
            100                 105                 110
ctg gcg cca tcc acg gcc gcc gat ggc gtc gat tcg agt tca caa gat      384
Leu Ala Pro Ser Thr Ala Ala Asp Gly Val Asp Ser Ser Ser Gln Asp
        115                 120                 125
tgg aag gcc caa ttg aag ctg ccg cct cca gat aca cgc tac agg aca      432
Trp Lys Ala Gln Leu Lys Leu Pro Pro Pro Asp Thr Arg Tyr Arg Thr
    130                 135                 140
gag gat gtt aca gct acc aag gga aat gag ttt gaa gac tat ttt ctc      480
Glu Asp Val Thr Ala Thr Lys Gly Asn Glu Phe Glu Asp Tyr Phe Leu
145                 150                 155                 160
aag cgt gaa ctc cta atg ggt ata tac gag aag gga ttt gaa aga ccg      528
Lys Arg Glu Leu Leu Met Gly Ile Tyr Glu Lys Gly Phe Glu Arg Pro
                165                 170                 175
tct cct atc caa gag gaa agc att cct att gct tta act ggt agt gac      576
Ser Pro Ile Gln Glu Glu Ser Ile Pro Ile Ala Leu Thr Gly Ser Asp
            180                 185                 190
att ctt gca aga gcg aag aac gga act ggc aag act gct gca ttt tgt      624
Ile Leu Ala Arg Ala Lys Asn Gly Thr Gly Lys Thr Ala Ala Phe Cys
        195                 200                 205
att cct gca ctg gaa aag att gat caa gaa aaa aat gct att caa gtt      672
Ile Pro Ala Leu Glu Lys Ile Asp Gln Glu Lys Asn Ala Ile Gln Val
    210                 215                 220
gtt ata ttg gtt ccc aca agg gaa ttg gct ctg caa aca tca caa gtt      720
Val Ile Leu Val Pro Thr Arg Glu Leu Ala Leu Gln Thr Ser Gln Val
225                 230                 235                 240
tgc aag gag ctt ggt aag cat ttg aag atc cag gtt atg gtc acc act      768

Cys Lys Glu Leu Gly Lys His Leu Lys Ile Gln Val Met Val Thr Thr
                245                 250                 255
ggt gga acc agt ttg aag gat gat att gtt cgt ctg tac caa cct gtg         816
Gly Gly Thr Ser Leu Lys Asp Asp Ile Val Arg Leu Tyr Gln Pro Val
            260                 265                 270
cac tta ctc gtt gga aca cct ggg cgt att ttg gat ctt aca aag aaa         864
His Leu Leu Val Gly Thr Pro Gly Arg Ile Leu Asp Leu Thr Lys Lys
        275                 280                 285
ggt gtt tgc att ttg aac gac tgt tca atg ctt att atg gat gag gca         912
Gly Val Cys Ile Leu Asn Asp Cys Ser Met Leu Ile Met Asp Glu Ala
    290                 295                 300
gac aag ctt ctc tct cct gag ttt caa cct tcc ata gaa cag ttg att         960
Asp Lys Leu Leu Ser Pro Glu Phe Gln Pro Ser Ile Glu Gln Leu Ile
305                 310                 315                 320
cgc tac ctc cct gct agt cga cag att ttg atg ttt tca gcg aca ttc         1008
Arg Tyr Leu Pro Ala Ser Arg Gln Ile Leu Met Phe Ser Ala Thr Phe
                325                 330                 335
cct gtg act gtt aag gag ttt aag gac aaa tat ctg cct aaa cct tat         1056
Pro Val Thr Val Lys Glu Phe Lys Asp Lys Tyr Leu Pro Lys Pro Tyr
            340                 345                 350
gtg att aac ctt atg gac gaa ctt aca ctg aag gga att aca cag ttc         1104
Val Ile Asn Leu Met Asp Glu Leu Thr Leu Lys Gly Ile Thr Gln Phe
        355                 360                 365
tat gct ttt gtg gaa gaa aga cag aaa gtt cac tgt ctc aac act ctc         1152
Tyr Ala Phe Val Glu Glu Arg Gln Lys Val His Cys Leu Asn Thr Leu
    370                 375                 380
ttt tcc aag ctt caa atc aac cag tca ata ata ttt tgt aac tct gta         1200
Phe Ser Lys Leu Gln Ile Asn Gln Ser Ile Ile Phe Cys Asn Ser Val
385                 390                 395                 400
aat cgt gtt gaa cta ttg gcg aaa aag atc aca gag ctt ggc tat tct         1248
Asn Arg Val Glu Leu Leu Ala Lys Lys Ile Thr Glu Leu Gly Tyr Ser
                405                 410                 415
tgc ttc tat att cat gca aag atg cta cag gac cac cgt aat aga gtt         1296
Cys Phe Tyr Ile His Ala Lys Met Leu Gln Asp His Arg Asn Arg Val
            420                 425                 430
ttc cat gat ttc cgt aat gga gca tgc agg aac ctt gtt tgc act gat         1344
Phe His Asp Phe Arg Asn Gly Ala Cys Arg Asn Leu Val Cys Thr Asp
        435                 440                 445
ctt ttt aca agg gga ata gat att caa gct gtg aat gtt gtt atc aat         1392
Leu Phe Thr Arg Gly Ile Asp Ile Gln Ala Val Asn Val Val Ile Asn
    450                 455                 460
ttt gat ttc ccc aag aac tct gaa aca tat ctc cac agg gtt ggt aga         1440
Phe Asp Phe Pro Lys Asn Ser Glu Thr Tyr Leu His Arg Val Gly Arg
465                 470                 475                 480
tct ggg aga ttt ggt cac ctt ggt ctg gcc gtt aat ttg atc acc tat         1488
Ser Gly Arg Phe Gly His Leu Gly Leu Ala Val Asn Leu Ile Thr Tyr
                485                 490                 495
gag gat cgg ttt aac ttg tat agg att gaa caa gag ctt ggg act gaa         1536
Glu Asp Arg Phe Asn Leu Tyr Arg Ile Glu Gln Glu Leu Gly Thr Glu
            500                 505                 510
ata aaa tca att cct cct cag ata gac cag gct ata tac tgc caa             1581
Ile Lys Ser Ile Pro Pro Gln Ile Asp Gln Ala Ile Tyr Cys Gln
        515                 520                 525
taa                                                                     1584

<210> 74
<211> 527
<212> PRT
<213> Zea mays

<400> 74
   Met Asp Pro Arg Ala Arg Tyr Pro Ser Gly Met Gly Asn Gly Arg Gly
   1               5                   10                  15
   Gly Asn Leu Asn Tyr Tyr Gly Arg Gly Pro Pro Pro Gln Gln Pro His
               20                  25                  30
   Gln His His Asn His Gln Gln Thr Ser Gly Ala His His His Gln Gln
           35                  40                  45
   Thr Ser Gly Ala His His His Gln Gln Tyr Ala Gln Arg Gln His Gln
       50                  55                  60
   His Gln His Gln His His Gly His Asn Arg His Gln Gln Gln Gln Gln
   65                  70                  75                  80
   His His Ile Tyr Gln Gln Gln Gln Gln Gln Trp Leu Arg Arg Ser Gln
                   85                  90                  95
   Ile Ala Arg Glu Thr Ser Gly Ala Ala Val Thr Ser Glu Pro Lys Ser
               100                 105                 110
   Leu Ala Pro Ser Thr Ala Ala Asp Gly Val Asp Ser Ser Ser Gln Asp
           115                 120                 125
   Trp Lys Ala Gln Leu Lys Leu Pro Pro Pro Asp Thr Arg Tyr Arg Thr
       130                 135                 140
   Glu Asp Val Thr Ala Thr Lys Gly Asn Glu Phe Glu Asp Tyr Phe Leu
   145                 150                 155                 160
   Lys Arg Glu Leu Leu Met Gly Ile Tyr Glu Lys Gly Phe Glu Arg Pro
                   165                 170                 175
   Ser Pro Ile Gln Glu Glu Ser Ile Pro Ile Ala Leu Thr Gly Ser Asp
               180                 185                 190
   Ile Leu Ala Arg Ala Lys Asn Gly Thr Gly Lys Thr Ala Ala Phe Cys
           195                 200                 205
   Ile Pro Ala Leu Glu Lys Ile Asp Gln Glu Lys Asn Ala Ile Gln Val
       210                 215                 220
   Val Ile Leu Val Pro Thr Arg Glu Leu Ala Leu Gln Thr Ser Gln Val
   225                 230                 235                 240
   Cys Lys Glu Leu Gly Lys His Leu Lys Ile Gln Val Met Val Thr Thr
                   245                 250                 255
   Gly Gly Thr Ser Leu Lys Asp Asp Ile Val Arg Leu Tyr Gln Pro Val
               260                 265                 270
   His Leu Leu Val Gly Thr Pro Gly Arg Ile Leu Asp Leu Thr Lys Lys
           275                 280                 285
   Gly Val Cys Ile Leu Asn Asp Cys Ser Met Leu Ile Met Asp Glu Ala
       290                 295                 300
   Asp Lys Leu Leu Ser Pro Glu Phe Gln Pro Ser Ile Glu Gln Leu Ile
   305                 310                 315                 320
   Arg Tyr Leu Pro Ala Ser Arg Gln Ile Leu Met Phe Ser Ala Thr Phe
                   325                 330                 335
   Pro Val Thr Val Lys Glu Phe Lys Asp Lys Tyr Leu Pro Lys Pro Tyr
               340                 345                 350
   Val Ile Asn Leu Met Asp Glu Leu Thr Leu Lys Gly Ile Thr Gln Phe
           355                 360                 365
   Tyr Ala Phe Val Glu Glu Arg Gln Lys Val His Cys Leu Asn Thr Leu
       370                 375                 380
   Phe Ser Lys Leu Gln Ile Asn Gln Ser Ile Ile Phe Cys Asn Ser Val
   385                 390                 395                 400
   Asn Arg Val Glu Leu Leu Ala Lys Lys Ile Thr Glu Leu Gly Tyr Ser
                   405                 410                 415
   Cys Phe Tyr Ile His Ala Lys Met Leu Gln Asp His Arg Asn Arg Val
               420                 425                 430
   Phe His Asp Phe Arg Asn Gly Ala Cys Arg Asn Leu Val Cys Thr Asp
           435                 440                 445
   Leu Phe Thr Arg Gly Ile Asp Ile Gln Ala Val Asn Val Val Ile Asn
       450                 455                 460
   Phe Asp Phe Pro Lys Asn Ser Glu Thr Tyr Leu His Arg Val Gly Arg
   465                 470                 475                 480
   Ser Gly Arg Phe Gly His Leu Gly Leu Ala Val Asn Leu Ile Thr Tyr
                   485                 490                 495
   Glu Asp Arg Phe Asn Leu Tyr Arg Ile Glu Gln Glu Leu Gly Thr Glu
               500                 505                 510
   Ile Lys Ser Ile Pro Pro Gln Ile Asp Gln Ala Ile Tyr Cys Gln
           515                 520                 525

<210> 75
<211> 21
<212> DNA
<213> Artificial sequence

<400> 75
atgggagaaa caagggacaa c                                                21

<210> 76
<211> 23
<212> DNA
<213> Artificial sequence

<400> 76
ttaggagggc atgtatgtag atg                                              23


<210> 77
<211> 414
<212> PRT
<213> Artificial sequence

<220>
<221> Variant
<222> (2)..(7)
<223> xaa in position 2 to 7 is any amino acid
<220>
<221> Variant
<222> (10)..(13)
<223> xaa in position 10 to 13 is any amino acid
<220>
<221> Variant
<222> (15)..(18)
<223> xaa in position 15 to 18 is any amino acid
<220>
<221> Variant
<222> (20)..(20)
<223> xaa in position 20 is any amino acid
<220>
<221> Variant
<222> (21)..(21)
<223> xaa in position 21 is any or no amino acid
<220>
<221> Variant
<222> (23)..(26)
<223> xaa in position 23 to 26 is any amino acid
<220>
<221> Variant
<222> (28)..(28)
<223> xaa in position 28 is any amino acid
<220>
<221> Variant
<222> (31)..(31)
<223> xaa in position 31 is any amino acid
<220>
<221> Variant
<222> (36)..(36)
<223> xaa in position 36 is any amino acid
<220>
<221> Variant
<222> (38)..(39)
<223> xaa in position 38 to 39 is any amino acid
<220>
<221> Variant
<222> (41)..(53)
<223> xaa in position 41 to 53 is any amino acid
<220>
<221> Variant
<222> (54)..(77)
<223> xaa in position 54 to 77 is any or no amino acid
<220>
<221> Variant
<222> (79)..(81)
<223> xaa in position 79 to 81 is any amino acid
<220>
<221> Variant
<222> (88)..(88)

<223> xaa in position 88 is any amino acid
<220>
<221> Variant
<222> (90)..(107)
<223> xaa in position 90 to 107 is any amino acid
<220>
<221> Variant
<222> (108)..(116)
<223> xaa in position 108 to 116 is any or no amino acid
<220>
<221> Variant
<222> (119)..(123)
<223> xaa in position 119 to 123 is any amino acid
<220>
<221> Variant
<222> (125)..(127)
<223> xaa in position 125 to 127 is any amino acid
<220>
<221> Variant
<222> (129)..(136)
<223> xaa in position 129 to 136 is any amino acid
<220>
<221> Variant
<222> (137)..(141)
<223> xaa in position 137 to 141 is any or no amino acid
<220>
<221> Variant
<222> (146)..(147)
<223> xaa in position 146 to 147 is any amino acid
<220>
<221> Variant
<222> (149)..(163)
<223> xaa in position 149 to 163 is any amino acid
<220>
<221> Variant
<222> (164)..(168)
<223> xaa in position 164 to 168 is any or no amino acid
<220>
<221> Variant
<222> (175)..(175)
<223> xaa in position 175 is any amino acid
<220>
<221> Variant
<222> (178)..(179)
<223> xaa in position 178 to 179 is any amino acid
<220>
<221> Variant
<222> (180)..(182)
<223> xaa in position 180 to 182 is any or no amino acid
<220>
<221> Variant
<222> (185)..(195)
<223> xaa in position 185 to 195 is any amino acid
<220>
<221> Variant
<222> (197)..(199)
<223> xaa in position 197 to 199 is any amino acid
<220>
<221> Variant
<222> (200)..(224)
<223> xaa in position 200 to 224 is any or no amino acid
<220>
<221> Variant
<222> (226)..(228)
<223> xaa in position 226 to 228 is any amino acid
<220>
<221> Variant
<222> (233)..(247)
<223> xaa in position 233 to 247 is any amino acid

<220>
<221> Variant
<222> (249)..(261)
<223> xaa in position 249 to 261 is any amino acid
<220>
<221> Variant
<222> (262)..(274)
<223> xaa in position 262 to 274 is any or no amino acid
<220>
<221> Variant
<222> (276)..(301)
<223> xaa in position 276 to 301 is any amino acid
<220>
<221> Variant
<222> (302)..(316)
<223> xaa in position 302 to 316 is any or no amino acid
<220>
<221> Variant
<222> (318)..(319)
<223> xaa in position 318 to 319 is any amino acid
<220>
<221> Variant
<222> (321)..(330)
<223> xaa in position 321 to 330 is any amino acid
<220>
<221> Variant
<222> (332)..(338)
<223> xaa in position 332 to 338 is any amino acid
<220>
<221> Variant
<222> (339)..(342)
<223> xaa in position 339 to 342 is any or no amino acid
<220>
<221> Variant
<222> (345)..(347)
<223> xaa in position 345 to 347 is any amino acid
<220>
<221> Variant
<222> (349)..(350)
<223> xaa in position 349 to 350 is any amino acid
<220>
<221> Variant
<222> (352)..(357)
<223> xaa in position 352 to 357 is any amino acid
<220>
<221> Variant
<222> (359)..(364)
<223> xaa in position 359 to 364 is any amino acid
<220>
<221> Variant
<222> (365)..(366)
<223> xaa in position 365 to 366 is any or no amino acid
<220>
<221> Variant
<222> (368)..(368)
<223> xaa in position 368 is any amino acid
<220>
<221> Variant
<222> (372)..(372)
<223> xaa in position 372 is any amino acid
<220>
<221> Variant
<222> (377)..(377)
<223> xaa in position 377 is any amino acid
<220>
<221> Variant
<222> (379)..(384)
<223> xaa in position 379 to 384 is any amino acid
<220>

<221> Variant
<222> (386)..(386)
<223> Xaa in position 386 is any amino acid
<220>
<221> Variant
<222> (388)..(390)
<223> Xaa in position 388 to 390 is any amino acid
<220>
<221> Variant
<222> (392)..(394)
<223> Xaa in position 392 to 394 is any amino acid
<220>
<221> Variant
<222> (396)..(396)
<223> Xaa in position 396 is any amino acid
<220>
<221> Variant
<222> (398)..(398)
<223> Xaa in position 398 is any amino acid
<220>
<221> Variant
<222> (409)..(411)
<223> Xaa in position 409 to 411 is any amino acid
<220>
<221> Variant
<222> (413)..(413)
<223> Xaa in position 413 is any amino acid

<400> 77
Gly Xaa Xaa Xaa Xaa Xaa Xaa Ile Gln Xaa Xaa Xaa Xaa Pro Xaa Xaa
1               5                   10                  15
Xaa Xaa Gly Xaa Xaa Asp Xaa Xaa Xaa Xaa Ala Xaa Thr Gly Xaa Gly
            20                  25                  30
Lys Thr Ala Xaa Phe Xaa Xaa Pro Xaa Xaa Xaa Xaa Xaa Xaa Xaa Xaa
        35                  40                  45
Xaa Xaa Xaa Xaa Xaa Xaa Xaa Xaa Xaa Xaa Xaa Xaa Xaa Xaa Xaa Xaa
    50                  55                  60
Xaa Xaa Xaa Xaa Xaa Xaa Xaa Xaa Xaa Xaa Xaa Xaa Xaa Leu Xaa Xaa
65                  70                  75                  80
Xaa Pro Thr Arg Glu Leu Ala Xaa Gln Xaa Xaa Xaa Xaa Xaa Xaa Xaa
                85                  90                  95
Xaa Xaa Xaa Xaa Xaa Xaa Xaa Xaa Xaa Xaa Xaa Xaa Xaa Xaa Xaa Xaa
            100                 105                 110
Xaa Xaa Xaa Xaa Gly Gly Xaa Xaa Xaa Xaa Xaa Gln Xaa Xaa Xaa Leu
        115                 120                 125
Xaa Xaa Xaa Xaa Xaa Xaa Xaa Xaa Xaa Xaa Xaa Xaa Xaa Thr Pro Gly
    130                 135                 140
Arg Xaa Xaa Asp Xaa Xaa Xaa Xaa Xaa Xaa Xaa Xaa Xaa Xaa Xaa Xaa
145                 150                 155                 160
Xaa Xaa Xaa Xaa Xaa Xaa Xaa Xaa Val Leu Asp Glu Ala Asp Xaa Met
                165                 170                 175
Leu Xaa Xaa Xaa Xaa Xaa Gly Phe Xaa Xaa Xaa Xaa Xaa Xaa Xaa Xaa
            180                 185                 190
Xaa Xaa Xaa Pro Xaa Xaa Xaa Xaa Xaa Xaa Xaa Xaa Xaa Xaa Xaa Xaa
        195                 200                 205
Xaa Xaa Xaa Xaa Xaa Xaa Xaa Xaa Xaa Xaa Xaa Xaa Xaa Xaa Xaa Xaa
    210                 215                 220
Gln Xaa Xaa Xaa Phe Ser Ala Thr Xaa Xaa Xaa Xaa Xaa Xaa Xaa Xaa
225                 230                 235                 240
Xaa Xaa Xaa Xaa Xaa Xaa Xaa Pro Xaa Xaa Xaa Xaa Xaa Xaa Xaa Xaa
                245                 250                 255
Xaa Xaa Xaa Xaa Xaa Xaa Xaa Xaa Xaa Xaa Xaa Xaa Xaa Xaa Xaa Xaa
            260                 265                 270
Xaa Xaa Gln Xaa Xaa Xaa Xaa Xaa Xaa Xaa Xaa Xaa Xaa Xaa Xaa Xaa
        275                 280                 285
Xaa Xaa Xaa Xaa Xaa Xaa Xaa Xaa Xaa Xaa Xaa Xaa Xaa Xaa Xaa Xaa
    290                 295                 300
Xaa Xaa Xaa Xaa Xaa Xaa Xaa Xaa Xaa Xaa Xaa Xaa Phe Xaa Xaa Thr
305                 310                 315                 320

xaa xaa xaa xaa xaa xaa xaa xaa xaa xaa Leu xaa xaa xaa xaa xaa
                325                 330                 335
xaa xaa xaa xaa xaa xaa His Gly xaa xaa xaa Gln xaa xaa Arg xaa
            340                 345                 350
xaa xaa xaa xaa xaa Phe xaa xaa xaa xaa xaa xaa xaa xaa Leu xaa
        355                 360                 365
Ala Thr Asp xaa Ala Ala Arg Gly xaa Asp xaa xaa xaa xaa xaa xaa
    370                 375                 380
Val xaa Asn xaa xaa xaa Pro xaa xaa xaa Glu xaa Tyr xaa His Arg
385                 390                 395                 400
Ile Gly Arg Thr Gly Arg Ala Gly xaa xaa xaa Gly xaa Ala
                405                 410

<210> 78
<211> 94
<212> PRT
<213> Artificial sequence

<220>
<221> Variant
<222> (1)..(1)
<223> xaa in position 1 is Leu, Val, Ile, Cys, Ala or Met
<220>
<221> Variant
<222> (2)..(5)
<223> xaa in position 2 to 5 is any amino acid
<220>
<221> Variant
<222> (6)..(6)
<223> xaa in position 6 is Tyr, Phe, Trp, Ile or Leu
<220>
<221> Variant
<222> (7)..(8)
<223> xaa in position 7 to 8 is any amino acid
<220>
<221> Variant
<222> (9)..(9)
<223> xaa in position 9 is Pro, Met, Ala, Leu, Cys or Thr
<220>
<221> Variant
<222> (10)..(10)
<223> xaa in position 10 is Thr, Ser, Phe or Met
<220>
<221> Variant
<222> (11)..(11)
<223> xaa in position 11 is any amino acid
<220>
<221> Variant
<222> (12)..(12)
<223> xaa in position 12 is Ile or Val
<220>
<221> Variant
<222> (14)..(16)
<223> xaa in position 14 to 16 is any amino acid
<220>
<221> Variant
<222> (17)..(17)
<223> xaa in position 17 is Ile, Leu, Trp or Val
<220>
<221> Variant
<222> (18)..(20)
<223> xaa in position 18 to 20 is any amino acid
<220>
<221> Variant
<222> (21)..(21)
<223> xaa in position 21 is Leu, Ile, Met, Val, Cys or Phe
<220>
<221> Variant

<222> (22)..(24)
<223> xaa in position 22 to 24 is any amino acid
<220>
<221> Variant
<222> (25)..(25)
<223> xaa in position 25 is any or no amino acid
<220>
<221> Variant
<222> (26)..(26)
<223> xaa in position 26 is Asp or Asn
<220>
<221> Variant
<222> (27)..(27)
<223> xaa in position 27 is Val, Leu, Ile, Met, Ala, Cys, Thr or Phe
<220>
<221> Variant
<222> (28)..(28)
<223> xaa in position 28 is Ile, Val, Leu, Met, Ala or Arg
<220>
<221> Variant
<222> (29)..(29)
<223> xaa in position 29 is Gly, Ala, Val or Gln
<220>
<221> Variant
<222> (30)..(30)
<223> xaa in position 30 is any amino acid
<220>
<221> Variant
<222> (31)..(31)
<223> xaa in position 31 is Ala, Ser or Gly
<220>
<221> Variant
<222> (32)..(32)
<223> xaa in position 32 is any amino acid
<220>
<221> Variant
<222> (33)..(33)
<223> xaa in position 33 is Thr, Ser or Asn
<220>
<221> Variant
<222> (35)..(35)
<223> xaa in position 35 is Thr or Ser
<220>
<221> Variant
<222> (39)..(39)
<223> xaa in position 39 is any amino acid
<220>
<221> Variant
<222> (40)..(40)
<223> xaa in position 40 is Ala, Ser, Gly or Thr
<220>
<221> Variant
<222> (41)..(41)
<223> xaa in position 41 is Phe or Tyr
<220>
<221> Variant
<222> (42)..(42)
<223> xaa in position 42 is any amino acid
<220>
<221> Variant
<222> (43)..(43)
<223> xaa in position 43 is Leu, Ile, Ser, Val, Ala, Thr or Cys
<220>
<221> Variant
<222> (44)..(44)
<223> xaa in position 44 is Pro, Gly, Ser, Ala or Thr
<220>
<221> Variant
<222> (45)..(45)

<223> xaa in position 45 is any amino acid
<220>
<221> Variant
<222> (46)..(46)
<223> xaa in position 46 is Leu, Ile, Phe, Cys, Val, Ala or Met
<220>
<221> Variant
<222> (47)..(56)
<223> xaa in position 47 to 56 is any amino acid
<220>
<221> Variant
<222> (57)..(81)
<223> xaa in position 57 to 81 is any or no amino acid
<220>
<221> Variant
<222> (82)..(82)
<223> xaa in position 82 is Leu, Ile, Val, Met or Cys
<220>
<221> Variant
<222> (83)..(83)
<223> xaa in position 83 is Val or Ile
<220>
<221> Variant
<222> (84)..(84)
<223> xaa in position 84 is Leu, Ile, Val or Met
<220>
<221> Variant
<222> (85)..(85)
<223> xaa in position 85 is Ala, Thr, Cys, Ser, Val, Leu or Glu
<220>
<221> Variant
<222> (91)..(91)
<223> xaa in position 91 is Ala, Val, Cys, Ser or Thr
<220>
<221> Variant
<222> (92)..(92)
<223> xaa in position 92 is any amino acid
<220>
<221> Variant
<222> (94)..(94)
<223> xaa in position 94 is Val, Ile, Thr or Ser

<400> 78
xaa xaa xaa xaa xaa xaa xaa xaa xaa xaa xaa xaa Gln xaa xaa xaa
1               5                   10                  15
xaa xaa xaa xaa xaa xaa xaa xaa xaa xaa xaa xaa xaa xaa xaa xaa
            20                  25                  30
xaa Gly xaa Gly Lys Thr xaa xaa xaa xaa xaa xaa xaa xaa xaa xaa
        35                  40                  45
xaa xaa xaa xaa xaa xaa xaa xaa xaa xaa xaa xaa xaa xaa xaa xaa
    50                  55                  60
xaa xaa xaa xaa xaa xaa xaa xaa xaa xaa xaa xaa xaa xaa xaa xaa
65                  70                  75                  80
xaa xaa xaa xaa xaa Pro Thr Arg Glu Leu xaa xaa Gln xaa
                85                  90


<210> 79
<211> 116
<212> PRT
<213> Artificial sequence

<220>
<221> Variant
<222> (2)..(2)
<223> xaa in position 2 is Gly or Lys
<220>
<221> Variant
<222> (3)..(7)

<223> xaa in position 3 to 7 is any amino acid
<220>
<221> Variant
<222> (8)..(8)
<223> xaa in position 8 is Gln, Asp, Trp or Glu
<220>
<221> Variant
<222> (9)..(11)
<223> xaa in position 9 to 11 is any amino acid
<220>
<221> Variant
<222> (12)..(12)
<223> xaa in position 12 is Leu, Ile, Val, Phe, Met or Arg
<220>
<221> Variant
<222> (13)..(15)
<223> xaa in position 13 to 15 is any amino acid
<220>
<221> Variant
<222> (16)..(20)
<223> xaa in position 16 to 20 is any or no amino acid
<220>
<221> Variant
<222> (21)..(21)
<223> xaa in position 21 is His, Asp, Gln, Glu, Ala, Asn or Lys
<220>
<221> Variant
<222> (22)..(22)
<223> xaa in position 22 is Ile, Val, Leu, Ala or Phe
<220>
<221> Variant
<222> (23)..(23)
<223> xaa in position 23 is Val, Leu, Ile, Met or Ala
<220>
<221> Variant
<222> (24)..(24)
<223> xaa in position 24 is Val, Ile or Ser
<220>
<221> Variant
<222> (25)..(25)
<223> xaa in position 25 is Gly or Ala
<220>
<221> Variant
<222> (27)..(27)
<223> xaa in position 27 is Pro or Thr
<220>
<221> Variant
<222> (30)..(30)
<223> xaa in position 30 is Leu, Val, Ile, Ala, Phe, Met or Thr
<220>
<221> Variant
<222> (31)..(31)
<223> xaa in position 31 is any amino acid
<220>
<221> Variant
<222> (32)..(32)
<223> xaa in position 32 is Asp or Glu
<220>
<221> Variant
<222> (33)..(33)
<223> xaa in position 33 is His, Leu, Met, Phe, Ile, Tyr or Cys
<220>
<221> Variant
<222> (34)..(42)
<223> xaa in position 34 to 42 is any amino acid
<220>
<221> Variant
<222> (43)..(48)
<223> xaa in position 43 to 48 is any or no amino acid

<220>
<221> Variant
<222> (49)..(49)
<223> Xaa in position 49 is Val, Ile, Leu, Cys, Thr, Pro or Met
<220>
<221> Variant
<222> (50)..(52)
<223> Xaa in position 50 to 52 is any amino acid
<220>
<221> Variant
<222> (53)..(53)
<223> Xaa in position 53 is Val, Ile, Ala or Thr
<220>
<221> Variant
<222> (54)..(54)
<223> Xaa in position 54 is Leu, Ile, Met, Phe or Val
<220>
<221> Variant
<222> (57)..(57)
<223> Xaa in position 57 is Ala, Ser or Gly
<220>
<221> Variant
<222> (59)..(59)
<223> Xaa in position 59 is Arg, Glu, Gln, Lys or His
<220>
<221> Variant
<222> (60)..(60)
<223> Xaa in position 60 is Met or Leu
<220>
<221> Variant
<222> (61)..(61)
<223> Xaa in position 61 is Leu, Phe, Met or Val
<220>
<221> Variant
<222> (62)..(63)
<223> Xaa in position 62 to 63 is any amino acid
<220>
<221> Variant
<222> (64)..(66)
<223> Xaa in position 64 to 66 is any or no amino acid
<220>
<221> Variant
<222> (67)..(67)
<223> Xaa in position 67 is Gly, Asp or Glu
<220>
<221> Variant
<222> (69)..(71)
<223> Xaa in position 69 to 71 is any amino acid
<220>
<221> Variant
<222> (72)..(72)
<223> Xaa in position 72 is Ile, Val, Leu or Met
<220>
<221> Variant
<222> (73)..(74)
<223> Xaa in position 73 to 74 is any amino acid
<220>
<221> Variant
<222> (75)..(75)
<223> Xaa in position 75 is Ile, Val, Leu or Met
<220>
<221> Variant
<222> (76)..(86)
<223> Xaa in position 76 to 86 is any amino acid
<220>
<221> Variant
<222> (87)..(111)
<223> Xaa in position 87 to 111 is any or no amino acid
<220>

<221> Variant
<222> (112)..(112)
<223> xaa in position 112 is Leu, Met, Phe, Val, Cys or Tyr
<220>
<221> Variant
<222> (113)..(113)
<223> xaa in position 113 is Phe, Leu, Trp, Val, Tyr or Cys
<220>
<221> Variant
<222> (114)..(114)
<223> xaa in position 114 is Ser, Thr or Phe

<400> 79
   Gly Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Thr Xaa Gly Arg Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Asp Glu Xaa Asp Xaa Xaa Xaa Xaa Xaa Xaa
       50                  55                  60
   Xaa Xaa Xaa Phe Xaa Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Xaa Xaa
               100                 105                 110
   Xaa Xaa Ala Thr
           115


<210> 80
<211> 99
<212> PRT
<213> Artificial sequence

<220>
<221> Variant
<222> (1)..(1)
<223> xaa in position 1 is Ile, Leu, Val or Met
<220>
<221> Variant
<222> (2)..(2)
<223> xaa in position 2 is Ile, Val, Ala, Leu or Met
<220>
<221> Variant
<222> (3)..(3)
<223> xaa in position 3 is Phe or Val
<220>
<221> Variant
<222> (4)..(5)
<223> xaa in position 4 to 5 is any amino acid
<220>
<221> Variant
<222> (6)..(6)
<223> xaa in position 6 is Thr, Arg, Ser, Ala, Met or Tyr
<220>
<221> Variant
<222> (7)..(7)
<223> xaa in position 7 is Lys, Arg, Gln, Val or Cys
<220>
<221> Variant
<222> (8)..(9)
<223> xaa in position 8 to 9 is any amino acid
<220>
<221> Variant
<222> (10)..(10)
<223> xaa in position 10 is Val, Ala, Thr, Cys, Ser or Ile
<220>

<221> Variant
<222> (11)..(12)
<223> Xaa in position 11 to 12 is any amino acid
<220>
<221> Variant
<222> (13)..(13)
<223> Xaa in position 13 is Leu, Val, Ile, Met or Arg
<220>
<221> Variant
<222> (14)..(16)
<223> Xaa in position 14 to 16 is any amino acid
<220>
<221> Variant
<222> (17)..(17)
<223> Xaa in position 17 is Leu, Met, Ile, Cys or Phe
<220>
<221> Variant
<222> (18)..(20)
<223> Xaa in position 18 to 20 is any amino acid
<220>
<221> Variant
<222> (21)..(24)
<223> Xaa in position 21 to 24 is any or no amino acid
<220>
<221> Variant
<222> (25)..(25)
<223> Xaa in position 25 is Ala, Val, Ser, Cys or Thr
<220>
<221> Variant
<222> (26)..(27)
<223> Xaa in position 26 to 27 is any amino acid
<220>
<221> Variant
<222> (28)..(28)
<223> Xaa in position 28 is Leu, Ile, Met, Tyr, Phe, Thr or Val
<220>
<221> Variant
<222> (29)..(29)
<223> Xaa in position 29 is His, Asn, Ser or Thr
<220>
<221> Variant
<222> (30)..(30)
<223> Xaa in position 30 is Gly, Ser or Ala
<220>
<221> Variant
<222> (31)..(33)
<223> Xaa in position 31 to 33 is any amino acid
<220>
<221> Variant
<222> (35)..(36)
<223> Xaa in position 35 to 36 is any amino acid
<220>
<221> Variant
<222> (38)..(40)
<223> Xaa in position 38 to 40 is any amino acid
<220>
<221> Variant
<222> (41)..(41)
<223> Xaa in position 41 is Leu, Met, Ile, Val or Phe
<220>
<221> Variant
<222> (42)..(43)
<223> Xaa in position 42 to 43 is any amino acid
<220>
<221> Variant
<222> (44)..(44)
<223> Xaa in position 44 is Phe, Leu, Ile or Met
<220>
<221> Variant

<222> (45)..(50)
<223> xaa in position 45 to 50 is any amino acid
<220>
<221> Variant
<222> (51)..(52)
<223> xaa in position 51 to 52 is any or no amino acid
<220>
<221> Variant
<222> (53)..(53)
<223> xaa in position 53 is Leu, Val, Met, Cys or Ile
<220>
<221> Variant
<222> (54)..(54)
<223> xaa in position 54 is Val, Ile or Thr
<220>
<221> Variant
<222> (55)..(55)
<223> xaa in position 55 is Ala, Cys, Thr, Ser or Gly
<220>
<221> Variant
<222> (56)..(56)
<223> xaa in position 56 is Thr or Ser
<220>
<221> Variant
<222> (57)..(57)
<223> xaa in position 57 is Asp, Ser, Ala or Asn
<220>
<221> Variant
<222> (58)..(58)
<223> xaa in position 58 is Val, Ile or Leu
<220>
<221> Variant
<222> (59)..(59)
<223> xaa in position 59 is Ala, Trp, Leu, Val or Phe
<220>
<221> Variant
<222> (60)..(60)
<223> xaa in position 60 is Ala, Ser, Gly or Thr
<220>
<221> Variant
<222> (63)..(63)
<223> xaa in position 63 is Leu or Ile
<220>
<221> Variant
<222> (64)..(64)
<223> xaa in position 64 is Asp or His
<220>
<221> Variant
<222> (65)..(65)
<223> xaa in position 65 is Ile, Val or Leu
<220>
<221> Variant
<222> (66)..(67)
<223> xaa in position 66 to 67 is any amino acid
<220>
<221> Variant
<222> (68)..(68)
<223> xaa in position 68 is Val, Ile, Leu or Thr
<220>
<221> Variant
<222> (69)..(70)
<223> xaa in position 69 to 70 is any amino acid
<220>
<221> Variant
<222> (71)..(71)
<223> xaa in position 71 is Val or Ile
<220>
<221> Variant
<222> (72)..(72)

<223> Xaa in position 72 is Ile, Val, Phe, Tyr or Leu
<220>
<221> Variant
<222> (73)..(73)
<223> Xaa in position 73 is Asn, His or Ser
<220>
<221> Variant
<222> (74)..(74)
<223> Xaa in position 74 is Tyr, Phe, Val, His, Leu, Ala or Ile
<220>
<221> Variant
<222> (75)..(75)
<223> Xaa in position 75 is any amino acid
<220>
<221> Variant
<222> (76)..(76)
<223> Xaa in position 76 is Leu, Ile, Val, Met, Phe, Ala or Thr
<220>
<221> Variant
<222> (77)..(77)
<223> Xaa in position 77 is Pro, Ala, Thr or Ser
<220>
<221> Variant
<222> (78)..(82)
<223> Xaa in position 78 to 82 is any amino acid
<220>
<221> Variant
<222> (83)..(83)
<223> Xaa in position 83 is Tyr, His or Phe
<220>
<221> Variant
<222> (84)..(84)
<223> Xaa in position 84 is Val, Ile, Thr, Leu, Gln or Met
<220>
<221> Variant
<222> (87)..(87)
<223> Xaa in position 87 is Ile, Val, Ser, Thr or Ala
<220>
<221> Variant
<222> (90)..(90)
<223> Xaa in position 90 is Thr, Ser or Gly
<220>
<221> Variant
<222> (91)..(91)
<223> Xaa in position 91 is Gly or Ala
<220>
<221> Variant
<222> (93)..(93)
<223> Xaa in position 93 is any amino acid
<220>
<221> Variant
<222> (94)..(94)
<223> Xaa in position 94 is Gly, Asp, Asn, Ala or Glu
<220>
<221> Variant
<222> (95)..(96)
<223> Xaa in position 95 to 96 is any amino acid
<220>
<221> Variant
<222> (98)..(98)
<223> Xaa in position 98 is any amino acid
<220>
<221> Variant
<222> (99)..(99)
<223> Xaa in position 99 is Ala, Ser, Val or Thr

<400> 80
Xaa Xaa Xaa Xaa Xaa Xaa Xaa Xaa Xaa Xaa Xaa Xaa Xaa Xaa Xaa Xaa
1               5                   10                  15

xaa xaa xaa xaa xaa xaa xaa xaa xaa xaa xaa xaa xaa xaa xaa xaa
                20                  25                  30
xaa Gln xaa xaa Arg xaa xaa xaa xaa xaa xaa xaa xaa xaa xaa xaa
        35                  40                  45
xaa xaa xaa xaa xaa xaa xaa xaa xaa xaa xaa xaa Arg Gly xaa xaa
    50                  55                  60
xaa xaa xaa xaa xaa xaa xaa xaa xaa xaa xaa xaa xaa xaa xaa xaa
65                  70                  75                  80
xaa xaa xaa xaa His Arg xaa Gly Arg xaa xaa Arg xaa xaa xaa xaa
                85                  90                  95
Gly xaa xaa


<210> 81
<211> 858
<212> DNA
<213> Brassica napus

<220>
<221> CDS
<222> (1)..(858)

<400> 81
atg agc agc aat cat cag act gtg atg aag caa cta gag cca tgg tgc      48
Met Ser Ser Asn His Gln Thr Val Met Lys Gln Leu Glu Pro Trp Cys
1               5                   10                  15
gag ctc aaa gac aaa gtg gtt ctc cta aca gga gct tca tcc ggc atc      96
Glu Leu Lys Asp Lys Val Val Leu Leu Thr Gly Ala Ser Ser Gly Ile
            20                  25                  30
gga aga gag atc tgc ctc gat cta gcc aaa tcc ggc tgc aag atc atc      144
Gly Arg Glu Ile Cys Leu Asp Leu Ala Lys Ser Gly Cys Lys Ile Ile
        35                  40                  45
gca gca gct cgt cgt ctc gac cgt ctc caa tcc ctc tgc tcc gag atc      192
Ala Ala Ala Arg Arg Leu Asp Arg Leu Gln Ser Leu Cys Ser Glu Ile
    50                  55                  60
aac gcc tta ttc tcc cca aca aaa acc aaa caa gcc gca cct ctc gag      240
Asn Ala Leu Phe Ser Pro Thr Lys Thr Lys Gln Ala Ala Pro Leu Glu
65                  70                  75                  80
cta gac gtc tcc tca gac tca tcc acc atc cga aac gca gtc aaa caa      288
Leu Asp Val Ser Ser Asp Ser Ser Thr Ile Arg Asn Ala Val Lys Gln
                85                  90                  95
gct tgg gac atc ttc gga aac atc gac gtc ttg atc aac aac gca ggc      336
Ala Trp Asp Ile Phe Gly Asn Ile Asp Val Leu Ile Asn Asn Ala Gly
            100                 105                 110
atc aga ggc aac gtc aag tcg agt ctg gac cta tcc gaa gaa gaa tgg      384
Ile Arg Gly Asn Val Lys Ser Ser Leu Asp Leu Ser Glu Glu Glu Trp
        115                 120                 125
gaa aga gtc ttc aga aca aac cta acc gga cct tgg cta gta tca aaa      432
Glu Arg Val Phe Arg Thr Asn Leu Thr Gly Pro Trp Leu Val Ser Lys
    130                 135                 140
cac gtc tgc gtt ctg atg cgc gac gcc aaa cgc cgc ggc gga gga tcg      480
His Val Cys Val Leu Met Arg Asp Ala Lys Arg Arg Gly Gly Gly Ser
145                 150                 155                 160
gtg ata aac gtt tcc tcc atc gcg ggg ctt cag cgc ggg aag cta ccc      528
Val Ile Asn Val Ser Ser Ile Ala Gly Leu Gln Arg Gly Lys Leu Pro
                165                 170                 175
ggc gcg ttg gcg tac gcg tgt tcg aaa gga ggt ctt gat att atg acg      576
Gly Ala Leu Ala Tyr Ala Cys Ser Lys Gly Gly Leu Asp Ile Met Thr
            180                 185                 190
aag atg atg gcg gtt gag ctg ggt gag tat ggt ata aga gtg aac tcg      624
Lys Met Met Ala Val Glu Leu Gly Glu Tyr Gly Ile Arg Val Asn Ser
        195                 200                 205
ata gct ccg ggg ctg ttt aag tcg gag atc acg gaa ggt ctg gtg agg      672
Ile Ala Pro Gly Leu Phe Lys Ser Glu Ile Thr Glu Gly Leu Val Arg
    210                 215                 220
aaa gag tgg atg aag aat gtg agg aag agg att gtt ccg ttg aag gtg      720
Lys Glu Trp Met Lys Asn Val Arg Lys Arg Ile Val Pro Leu Lys Val
225                 230                 235                 240
cag cag act gtg gac ccg ggg ctt acc tcg ctg gtt agg tat ctg att      768

Gln Gln Thr Val Asp Pro Gly Leu Thr Ser Leu Val Arg Tyr Leu Ile
                245                 250                 255
ccc cac tct tcc agg tat gtc tct cgc aat gtt tac att gtt gac gcg       816
Pro His Ser Ser Arg Tyr Val Ser Arg Asn Val Tyr Ile Val Asp Ala
            260                 265                 270
ggt gct aca ttg tct ggt cta acg att ttt tct tca ctt tga               858
Gly Ala Thr Leu Ser Gly Leu Thr Ile Phe Ser Ser Leu
        275                 280                 285

<210> 82
<211> 285
<212> PRT
<213> Brassica napus

<400> 82
   Met Ser Ser Asn His Gln Thr Val Met Lys Gln Leu Glu Pro Trp Cys
   1               5                   10                  15
   Glu Leu Lys Asp Lys Val Val Leu Leu Thr Gly Ala Ser Ser Gly Ile
               20                  25                  30
   Gly Arg Glu Ile Cys Leu Asp Leu Ala Lys Ser Gly Cys Lys Ile Ile
           35                  40                  45
   Ala Ala Ala Arg Arg Leu Asp Arg Leu Gln Ser Leu Cys Ser Glu Ile
       50                  55                  60
   Asn Ala Leu Phe Ser Pro Thr Lys Thr Lys Gln Ala Ala Pro Leu Glu
   65                  70                  75                  80
   Leu Asp Val Ser Ser Asp Ser Ser Thr Ile Arg Asn Ala Val Lys Gln
                   85                  90                  95
   Ala Trp Asp Ile Phe Gly Asn Ile Asp Val Leu Ile Asn Asn Ala Gly
               100                 105                 110
   Ile Arg Gly Asn Val Lys Ser Ser Leu Asp Leu Ser Glu Glu Glu Trp
           115                 120                 125
   Glu Arg Val Phe Arg Thr Asn Leu Thr Gly Pro Trp Leu Val Ser Lys
       130                 135                 140
   His Val Cys Val Leu Met Arg Asp Ala Lys Arg Arg Gly Gly Gly Ser
   145                 150                 155                 160
   Val Ile Asn Val Ser Ser Ile Ala Gly Leu Gln Arg Gly Lys Leu Pro
                   165                 170                 175
   Gly Ala Leu Ala Tyr Ala Cys Ser Lys Gly Gly Leu Asp Ile Met Thr
               180                 185                 190
   Lys Met Met Ala Val Glu Leu Gly Glu Tyr Gly Ile Arg Val Asn Ser
           195                 200                 205
   Ile Ala Pro Gly Leu Phe Lys Ser Glu Ile Thr Glu Gly Leu Val Arg
       210                 215                 220
   Lys Glu Trp Met Lys Asn Val Arg Lys Arg Ile Val Pro Leu Lys Val
   225                 230                 235                 240
   Gln Gln Thr Val Asp Pro Gly Leu Thr Ser Leu Val Arg Tyr Leu Ile
                   245                 250                 255
   Pro His Ser Ser Arg Tyr Val Ser Arg Asn Val Tyr Ile Val Asp Ala
               260                 265                 270
   Gly Ala Thr Leu Ser Gly Leu Thr Ile Phe Ser Ser Leu
           275                 280                 285

<210> 83
<211> 22
<212> DNA
<213> Artificial sequence

<400> 83
atgagcagca atcatcagac tg                                              22

<210> 84
<211> 24
<212> DNA
<213> Artificial sequence

<400> 84
tcaaagtgaa gaaaaaatcg ttag                                            24

<210> 85
<211> 279
<212> PRT
<213> Artificial sequence

<220>
<221> Variant
<222> (4)..(5)
<223> xaa in position 4 to 5 is any amino acid
<220>
<221> Variant
<222> (7)..(7)
<223> xaa in position 7 is any amino acid
<220>
<221> Variant
<222> (9)..(11)
<223> xaa in position 9 to 11 is any amino acid
<220>
<221> Variant
<222> (13)..(26)
<223> xaa in position 13 to 26 is any amino acid
<220>
<221> Variant
<222> (27)..(30)
<223> xaa in position 27 to 30 is any or no amino acid
<220>
<221> Variant
<222> (32)..(35)
<223> xaa in position 32 to 35 is any amino acid
<220>
<221> Variant
<222> (37)..(49)
<223> xaa in position 37 to 49 is any amino acid
<220>
<221> Variant
<222> (50)..(63)
<223> xaa in position 50 to 63 is any or no amino acid
<220>
<221> Variant
<222> (65)..(81)
<223> xaa in position 65 to 81 is any amino acid
<220>
<221> Variant
<222> (82)..(86)
<223> xaa in position 82 to 86 is any or no amino acid
<220>
<221> Variant
<222> (88)..(88)
<223> xaa in position 88 is any amino acid
<220>
<221> Variant
<222> (90)..(90)
<223> xaa in position 90 is any amino acid
<220>
<221> Variant
<222> (95)..(102)
<223> xaa in position 95 to 102 is any amino acid
<220>
<221> Variant
<222> (103)..(117)
<223> xaa in position 103 to 117 is any or no amino acid
<220>
<221> Variant
<222> (119)..(120)
<223> xaa in position 119 to 120 is any amino acid
<220>
<221> Variant
<222> (122)..(143)
<223> xaa in position 122 to 143 is any amino acid

<220>
<221> Variant
<222> (144)..(150)
<223> xaa in position 144 to 150 is any or no amino acid
<220>
<221> Variant
<222> (152)..(153)
<223> xaa in position 152 to 153 is any amino acid
<220>
<221> Variant
<222> (155)..(163)
<223> xaa in position 155 to 163 is any amino acid
<220>
<221> Variant
<222> (164)..(170)
<223> xaa in position 164 to 170 is any or no amino acid
<220>
<221> Variant
<222> (172)..(174)
<223> xaa in position 172 to 174 is any amino acid
<220>
<221> Variant
<222> (176)..(184)
<223> xaa in position 176 to 184 is any amino acid
<220>
<221> Variant
<222> (186)..(198)
<223> xaa in position 186 to 198 is any amino acid
<220>
<221> Variant
<222> (199)..(202)
<223> xaa in position 199 to 202 is any or no amino acid
<220>
<221> Variant
<222> (205)..(224)
<223> xaa in position 205 to 224 is any amino acid
<220>
<221> Variant
<222> (225)..(278)
<223> xaa in position 225 to 278 is any or no amino acid

<400> 85
Thr Gly Ala xaa xaa Gly xaa Gly xaa xaa xaa Ala xaa xaa xaa xaa
1               5                   10                  15
xaa xaa xaa xaa xaa xaa xaa xaa xaa xaa xaa xaa xaa xaa Arg xaa
            20                  25                  30
xaa xaa xaa Leu xaa xaa xaa xaa xaa xaa xaa xaa xaa xaa xaa xaa
        35                  40                  45
xaa xaa xaa xaa xaa xaa xaa xaa xaa xaa xaa xaa xaa xaa xaa Asp
    50                  55                  60
xaa xaa xaa xaa xaa xaa xaa xaa xaa xaa xaa xaa xaa xaa xaa xaa
65                  70                  75                  80
xaa xaa xaa xaa xaa xaa Asp xaa Leu xaa Asn Asn Ala Gly xaa xaa
                85                  90                  95
xaa xaa xaa xaa xaa xaa xaa xaa xaa xaa xaa xaa xaa xaa xaa xaa
            100                 105                 110
xaa xaa xaa xaa xaa Asn xaa xaa Gly xaa xaa xaa xaa xaa xaa xaa
        115                 120                 125
xaa xaa xaa xaa xaa xaa xaa xaa xaa xaa xaa xaa xaa xaa xaa xaa
    130                 135                 140
xaa xaa xaa xaa xaa xaa Ser xaa xaa Gly xaa xaa xaa xaa xaa xaa
145                 150                 155                 160
xaa xaa xaa xaa xaa xaa xaa xaa xaa xaa Tyr xaa xaa xaa Lys xaa
                165                 170                 175
xaa xaa xaa xaa xaa xaa xaa xaa Leu xaa xaa xaa xaa xaa xaa xaa
            180                 185                 190
xaa xaa xaa xaa xaa xaa xaa xaa xaa xaa Pro Gly xaa xaa xaa xaa
        195                 200                 205
xaa xaa xaa xaa xaa xaa xaa xaa xaa xaa xaa xaa xaa xaa xaa xaa

210                 215                 220
Xaa Xaa Xaa Xaa Xaa Xaa Xaa Xaa Xaa Xaa Xaa Xaa Xaa Xaa Xaa Xaa
225                 230                 235                 240
Xaa Xaa Xaa Xaa Xaa Xaa Xaa Xaa Xaa Xaa Xaa Xaa Xaa Xaa Xaa Xaa
                245                 250                 255
Xaa Xaa Xaa Xaa Xaa Xaa Xaa Xaa Xaa Xaa Xaa Xaa Xaa Xaa Xaa Xaa
            260                 265                 270
Xaa Xaa Xaa Xaa Xaa Xaa Ala
        275

<210> 86
<211> 122
<212> PRT
<213> Artificial sequence

<220>
<221> Variant
<222> (1)..(1)
<223> Xaa in position 1 is Ile, Val or Leu
<220>
<221> Variant
<222> (2)..(2)
<223> Xaa in position 2 is Thr, Met, Ser or Ala
<220>
<221> Variant
<222> (4)..(4)
<223> Xaa in position 4 is Ala, Gly or Ser
<220>
<221> Variant
<222> (5)..(5)
<223> Xaa in position 5 is Ser, Thr, Gly or Ala
<220>
<221> Variant
<222> (6)..(6)
<223> Xaa in position 6 is any amino acid
<220>
<221> Variant
<222> (7)..(7)
<223> Xaa in position 7 is Gly or Asn
<220>
<221> Variant
<222> (8)..(8)
<223> Xaa in position 8 is any amino acid
<220>
<221> Variant
<222> (10)..(21)
<223> Xaa in position 10 to 21 is any amino acid
<220>
<221> Variant
<222> (22)..(26)
<223> Xaa in position 22 to 26 is any or no amino acid
<220>
<221> Variant
<222> (27)..(27)
<223> Xaa in position 27 is Val, Leu, Ile, Phe or Met
<220>
<221> Variant
<222> (28)..(30)
<223> Xaa in position 28 to 30 is any amino acid
<220>
<221> Variant
<222> (31)..(31)
<223> Xaa in position 31 is Ala, Gly, Asp or Ser
<220>
<221> Variant
<222> (32)..(32)
<223> Xaa in position 32 is Arg, Thr or Gln
<220>

<221> Variant
<222> (33)..(36)
<223> Xaa in position 33 to 36 is any amino acid
<220>
<221> Variant
<222> (37)..(37)
<223> Xaa in position 37 is Leu, Ile, Val, Ala or Met
<220>
<221> Variant
<222> (38)..(39)
<223> Xaa in position 38 to 39 is any amino acid
<220>
<221> Variant
<222> (40)..(40)
<223> Xaa in position 40 is Leu, Ile, Val, Ala, Thr or Phe
<220>
<221> Variant
<222> (41)..(49)
<223> Xaa in position 41 to 49 is any amino acid
<220>
<221> Variant
<222> (50)..(64)
<223> Xaa in position 50 to 64 is any or no amino acid
<220>
<221> Variant
<222> (65)..(65)
<223> Xaa in position 65 is Asp, Asn, Glu or Ser
<220>
<221> Variant
<222> (66)..(66)
<223> Xaa in position 66 is Val, Leu, Ile or Met
<220>
<221> Variant
<222> (67)..(80)
<223> Xaa in position 67 to 80 is any amino acid
<220>
<221> Variant
<222> (81)..(86)
<223> Xaa in position 81 to 86 is any or no amino acid
<220>
<221> Variant
<222> (87)..(87)
<223> Xaa in position 87 is Ile, Val, Leu, Ala or Phe
<220>
<221> Variant
<222> (88)..(89)
<223> Xaa in position 88 to 89 is any amino acid
<220>
<221> Variant
<222> (90)..(90)
<223> Xaa in position 90 is Leu, Ile, Val, Cys, Met or Phe
<220>
<221> Variant
<222> (91)..(91)
<223> Xaa in position 91 is any amino acid
<220>
<221> Variant
<222> (92)..(92)
<223> Xaa in position 92 is Asn, Leu, Ala, Ser or Gly
<220>
<221> Variant
<222> (93)..(93)
<223> Xaa in position 93 is Asn, Cys, Ser or Val
<220>
<221> Variant
<222> (94)..(94)
<223> Xaa in position 94 is Ala or Gly
<220>
<221> Variant

<222> (96)..(103)
<223> xaa in position 96 to 103 is any amino acid
<220>
<221> Variant
<222> (104)..(118)
<223> xaa in position 104 to 118 is any or no amino acid
<220>
<221> Variant
<222> (119)..(119)
<223> xaa in position 119 is Asn or Gly
<220>
<221> Variant
<222> (120)..(120)
<223> xaa in position 120 is Val, Ile, Leu, Cys, Asn, Ser, Ala or Phe
<220>
<221> Variant
<222> (121)..(121)
<223> xaa in position 121 is any amino acid
<220>
<221> Variant
<222> (122)..(122)
<223> xaa in position 122 is Gly, Ala or Ser

<400> 86
xaa xaa Gly xaa xaa xaa xaa xaa Gly xaa xaa xaa xaa xaa xaa xaa
1               5                   10                  15
xaa xaa xaa xaa xaa xaa xaa xaa xaa xaa xaa xaa xaa xaa xaa xaa
            20                  25                  30
xaa xaa xaa xaa xaa xaa xaa xaa xaa xaa xaa xaa xaa xaa xaa xaa
        35                  40                  45
xaa xaa xaa xaa xaa xaa xaa xaa xaa xaa xaa xaa xaa xaa xaa xaa
    50                  55                  60
xaa xaa xaa xaa xaa xaa xaa xaa xaa xaa xaa xaa xaa xaa xaa xaa
65                  70                  75                  80
xaa xaa xaa xaa xaa xaa xaa xaa xaa xaa xaa xaa xaa xaa Gly xaa
                85                  90                  95
xaa xaa xaa xaa xaa xaa xaa xaa xaa xaa xaa xaa xaa xaa xaa xaa
            100                 105                 110
xaa xaa xaa xaa xaa xaa xaa xaa xaa xaa
        115                 120


<210> 87
<211> 59
<212> PRT
<213> Artificial sequence

<220>
<221> Variant
<222> (1)..(1)
<223> xaa in position 1 is Ile, Val, Phe or Leu
<220>
<221> Variant
<222> (2)..(2)
<223> xaa in position 2 is Ile, Val, Leu, Phe or Met
<220>
<221> Variant
<222> (3)..(5)
<223> xaa in position 3 to 5 is any amino acid
<220>
<221> Variant
<222> (7)..(8)
<223> xaa in position 7 to 8 is any amino acid
<220>
<221> Variant
<222> (9)..(9)
<223> xaa in position 9 is Gly, Ala or Ser
<220>
<221> Variant

<222> (10)..(17)
<223> Xaa in position 10 to 17 is any amino acid
<220>
<221> Variant
<222> (18)..(25)
<223> Xaa in position 18 to 25 is any or no amino acid
<220>
<221> Variant
<222> (26)..(26)
<223> Xaa in position 26 is Tyr or Phe
<220>
<221> Variant
<222> (27)..(27)
<223> Xaa in position 27 is any amino acid
<220>
<221> Variant
<222> (28)..(28)
<223> Xaa in position 28 is Ala, Gly or Ser
<220>
<221> Variant
<222> (29)..(29)
<223> Xaa in position 29 is Thr, Ser, Ala or Val
<220>
<221> Variant
<222> (31)..(32)
<223> Xaa in position 31 to 32 is any amino acid
<220>
<221> Variant
<222> (33)..(33)
<223> Xaa in position 33 is Val, Ile, Leu, Thr, Glu, Gln or Met
<220>
<221> Variant
<222> (34)..(35)
<223> Xaa in position 34 to 35 is any amino acid
<220>
<221> Variant
<222> (36)..(36)
<223> Xaa in position 36 is Phe, Leu, Ile, Met or Val
<220>
<221> Variant
<222> (37)..(39)
<223> Xaa in position 37 to 39 is any amino acid
<220>
<221> Variant
<222> (40)..(40)
<223> Xaa in position 40 is Leu, Met, Val, Ile, Trp or Phe
<220>
<221> Variant
<222> (41)..(42)
<223> Xaa in position 41 to 42 is any amino acid
<220>
<221> Variant
<222> (43)..(43)
<223> Xaa in position 43 is Glu, Asp or Gln
<220>
<221> Variant
<222> (44)..(46)
<223> Xaa in position 44 to 46 is any amino acid
<220>
<221> Variant
<222> (47)..(50)
<223> Xaa in position 47 to 50 is any or no amino acid
<220>
<221> Variant
<222> (51)..(51)
<223> Xaa in position 51 is Ile, Val, Leu or Phe
<220>
<221> Variant
<222> (52)..(55)

<223> xaa in position 52 to 55 is any amino acid
<220>
<221> Variant
<222> (56)..(56)
<223> xaa in position 56 is Ile, Val or Leu
<220>
<221> Variant
<222> (57)..(57)
<223> xaa in position 57 is any amino acid
<220>
<221> Variant
<222> (59)..(59)
<223> xaa in position 59 is Gly, Ala or Ser

<400> 87
xaa xaa xaa xaa xaa Ser xaa xaa xaa xaa xaa xaa xaa xaa xaa xaa
1               5                   10                  15
xaa xaa xaa xaa xaa xaa xaa xaa xaa xaa xaa xaa xaa Lys xaa xaa
            20                  25                  30
xaa xaa xaa xaa xaa xaa xaa xaa xaa xaa xaa xaa xaa xaa xaa xaa
        35                  40                  45
xaa xaa xaa xaa xaa xaa xaa xaa xaa Pro xaa
    50                  55

<210> 88
<211> 849
<212> DNA
<213> Brassica napus

<220>
<221> CDS
<222> (1)..(849)

<400> 88
atg agc aat cat caa acc acg gtg ttg aaa caa cta gag cca tgg tgt      48
Met Ser Asn His Gln Thr Thr Val Leu Lys Gln Leu Glu Pro Trp Cys
1               5                   10                  15
gag cta aac ggc aaa gtg gtt ctt ctg acc gga gct tct tct ggt ata      96
Glu Leu Asn Gly Lys Val Val Leu Leu Thr Gly Ala Ser Ser Gly Ile
            20                  25                  30
gga agt gag gtc tgt ctc gat ctg ggc aaa gct ggc tgt aag att atc      144
Gly Ser Glu Val Cys Leu Asp Leu Gly Lys Ala Gly Cys Lys Ile Ile
        35                  40                  45
gca gca gct cgt cgc gtc cac cgt ctc gaa tct ctc tgc tcc gaa atc      192
Ala Ala Ala Arg Arg Val His Arg Leu Glu Ser Leu Cys Ser Glu Ile
    50                  55                  60
aac agc tta agc tca acc ggg atc caa tta gcc gca ccg ctc gag cta      240
Asn Ser Leu Ser Ser Thr Gly Ile Gln Leu Ala Ala Pro Leu Glu Leu
65                  70                  75                  80
gac gtt tca tca gac gca gcc acc att caa aaa gct gtc aaa caa gct      288
Asp Val Ser Ser Asp Ala Ala Thr Ile Gln Lys Ala Val Lys Gln Ala
                85                  90                  95
tgg gac atc tac gga aag ata gat gtg ttg atc aac aac gct gga atc      336
Trp Asp Ile Tyr Gly Lys Ile Asp Val Leu Ile Asn Asn Ala Gly Ile
            100                 105                 110
aga ggc aat gtc aag acg agt tta gat ctg act gaa gac gag tgg aac      384
Arg Gly Asn Val Lys Thr Ser Leu Asp Leu Thr Glu Asp Glu Trp Asn
        115                 120                 125
aca gtg ttc aga acc aac tta acc gga cct tgg tta gta tcc aaa tac      432
Thr Val Phe Arg Thr Asn Leu Thr Gly Pro Trp Leu Val Ser Lys Tyr
    130                 135                 140
gtc tgc agt cta atg cgt gac gca aaa cgc ggc ggc tca gta ata aac      480
Val Cys Ser Leu Met Arg Asp Ala Lys Arg Gly Gly Ser Val Ile Asn
145                 150                 155                 160
gtc tcc tcc atc gcc ggt ctc cac cga ggt ttg tta ccc ggt gga gtc      528
Val Ser Ser Ile Ala Gly Leu His Arg Gly Leu Leu Pro Gly Gly Val
                165                 170                 175
gcc tat gct tgt tcc aaa ggt ggt gtt gac acc atg acg agg atg atg      576
Ala Tyr Ala Cys Ser Lys Gly Gly Val Asp Thr Met Thr Arg Met Met

180                 185                 190
gct att gag tta ggt gta tac aac atc aga gtg aac tcg atc gca ccg       624
Ala Ile Glu Leu Gly Val Tyr Asn Ile Arg Val Asn Ser Ile Ala Pro
        195                 200                 205
ggg ctt ctc aag tca gag atc acg caa ggt ctt atg caa aaa gag tgg       672
Gly Leu Leu Lys Ser Glu Ile Thr Gln Gly Leu Met Gln Lys Glu Trp
    210                 215                 220
ctc aag aac gtg acc gag agg act atc ccg tta aag gtg caa cag acc       720
Leu Lys Asn Val Thr Glu Arg Thr Ile Pro Leu Lys Val Gln Gln Thr
225                 230                 235                 240
gtg gat ccg ggg ctt acc tcg ctc gtt cgc tat ctc agt cat gac tct       768
Val Asp Pro Gly Leu Thr Ser Leu Val Arg Tyr Leu Ser His Asp Ser
                245                 250                 255
tct caa tat gtc tcc ggc aac aca tac ata ctt gac tcc gga gct aca       816
Ser Gln Tyr Val Ser Gly Asn Thr Tyr Ile Leu Asp Ser Gly Ala Thr
            260                 265                 270
ata cct ggc ctg cct att ttt tct tct ctt tga                           849
Ile Pro Gly Leu Pro Ile Phe Ser Ser Leu
        275                 280

<210> 89
<211> 282
<212> PRT
<213> Brassica napus

<400> 89
Met Ser Asn His Gln Thr Thr Val Leu Lys Gln Leu Glu Pro Trp Cys
1               5                   10                  15
Glu Leu Asn Gly Lys Val Val Leu Leu Thr Gly Ala Ser Ser Gly Ile
            20                  25                  30
Gly Ser Glu Val Cys Leu Asp Leu Gly Lys Ala Gly Cys Lys Ile Ile
        35                  40                  45
Ala Ala Ala Arg Arg Val His Arg Leu Glu Ser Leu Cys Ser Glu Ile
    50                  55                  60
Asn Ser Leu Ser Ser Thr Gly Ile Gln Leu Ala Ala Pro Leu Glu Leu
65                  70                  75                  80
Asp Val Ser Ser Asp Ala Ala Thr Ile Gln Lys Ala Val Lys Gln Ala
                85                  90                  95
Trp Asp Ile Tyr Gly Lys Ile Asp Val Leu Ile Asn Asn Ala Gly Ile
            100                 105                 110
Arg Gly Asn Val Lys Thr Ser Leu Asp Leu Thr Glu Asp Glu Trp Asn
        115                 120                 125
Thr Val Phe Arg Thr Asn Leu Thr Gly Pro Trp Leu Val Ser Lys Tyr
    130                 135                 140
Val Cys Ser Leu Met Arg Asp Ala Lys Arg Gly Gly Ser Val Ile Asn
145                 150                 155                 160
Val Ser Ser Ile Ala Gly Leu His Arg Gly Leu Leu Pro Gly Gly Val
                165                 170                 175
Ala Tyr Ala Cys Ser Lys Gly Gly Val Asp Thr Met Thr Arg Met Met
            180                 185                 190
Ala Ile Glu Leu Gly Val Tyr Asn Ile Arg Val Asn Ser Ile Ala Pro
        195                 200                 205
Gly Leu Leu Lys Ser Glu Ile Thr Gln Gly Leu Met Gln Lys Glu Trp
    210                 215                 220
Leu Lys Asn Val Thr Glu Arg Thr Ile Pro Leu Lys Val Gln Gln Thr
225                 230                 235                 240
Val Asp Pro Gly Leu Thr Ser Leu Val Arg Tyr Leu Ser His Asp Ser
                245                 250                 255
Ser Gln Tyr Val Ser Gly Asn Thr Tyr Ile Leu Asp Ser Gly Ala Thr
            260                 265                 270
Ile Pro Gly Leu Pro Ile Phe Ser Ser Leu
        275                 280

<210> 90
<211> 1143
<212> DNA
<213> Glycine max

<220>

<221> CDS
<222> (79)..(927)

<400> 90
ccattcctga gtttccattg ccgcgacatc tgaattcctc cttgtcactc ataatcacca     60

aatagaagcc ataaacaa atg aat gcc ata gca gca gaa caa gtg ctg gag     111
                    Met Asn Ala Ile Ala Ala Glu Gln Val Leu Glu
                    1               5                   10
cca tgg cac aag cta gac gac aaa gtc gtt ttg gtg acg ggt gct tcc     159
Pro Trp His Lys Leu Asp Asp Lys Val Val Leu Val Thr Gly Ala Ser
            15                  20                  25
tcg ggg ctc ggg cga gat ttc tgc atc gac ctc gcc aaa gct ggc tgc     207
Ser Gly Leu Gly Arg Asp Phe Cys Ile Asp Leu Ala Lys Ala Gly Cys
        30                  35                  40
tgt gtc gtc gcg gca gct cgt cgc ctc gat cgc ctc act tcc ctc tgc     255
Cys Val Val Ala Ala Ala Arg Arg Leu Asp Arg Leu Thr Ser Leu Cys
    45                  50                  55
cac gaa atc aac cac cga tgg ccc tcg aac gtc gga atc cac cgc gcg     303
His Glu Ile Asn His Arg Trp Pro Ser Asn Val Gly Ile His Arg Ala
60                  65                  70                  75
gtg gcg gtg gag ctt gat gtc gcc gcc gat ggc ccc gcc atc gac agg     351
Val Ala Val Glu Leu Asp Val Ala Ala Asp Gly Pro Ala Ile Asp Arg
                80                  85                  90
gct gtg cag aag gcc tgg gac gcc ttt ggc cgc gtt gat tcc ttg att     399
Ala Val Gln Lys Ala Trp Asp Ala Phe Gly Arg Val Asp Ser Leu Ile
            95                  100                 105
aac aac gct ggt gtc aga gga agt gtt aaa tca ccc ttg aaa ttg tct     447
Asn Asn Ala Gly Val Arg Gly Ser Val Lys Ser Pro Leu Lys Leu Ser
        110                 115                 120
gaa gag gaa tgg gat cat gtc ttc aag act aac cta act ggt tgt tgg     495
Glu Glu Glu Trp Asp His Val Phe Lys Thr Asn Leu Thr Gly Cys Trp
    125                 130                 135
ttg gtg tca aaa tat gta tgc aaa cgc atg tgt gat atc cag ctt aag     543
Leu Val Ser Lys Tyr Val Cys Lys Arg Met Cys Asp Ile Gln Leu Lys
140                 145                 150                 155
gga tca att att aat att tct tca gtt tct ggt tta aat cgg ggg caa     591
Gly Ser Ile Ile Asn Ile Ser Ser Val Ser Gly Leu Asn Arg Gly Gln
                160                 165                 170
ttg cct gga gct gct gca tat gca tct tcg aag gca ggg gta aac atg     639
Leu Pro Gly Ala Ala Ala Tyr Ala Ser Ser Lys Ala Gly Val Asn Met
            175                 180                 185
ctg act aag gtc atg gct atg gaa ttg ggg atg cac aaa att aga gta     687
Leu Thr Lys Val Met Ala Met Glu Leu Gly Met His Lys Ile Arg Val
        190                 195                 200
aat tcc ata tcc cct gga att ttc aaa tct gaa ata act gaa aat tta     735
Asn Ser Ile Ser Pro Gly Ile Phe Lys Ser Glu Ile Thr Glu Asn Leu
    205                 210                 215
tta caa aaa gat tgg ctg aat gat gtg gtc agg aaa ata atg cct ttg     783
Leu Gln Lys Asp Trp Leu Asn Asp Val Val Arg Lys Ile Met Pro Leu
220                 225                 230                 235
aga aga tta ggt act tca gat cca gca tta aca tct cta gct cgt tat     831
Arg Arg Leu Gly Thr Ser Asp Pro Ala Leu Thr Ser Leu Ala Arg Tyr
                240                 245                 250
ctt att cat gat tct tct gag tac gtc acg ggc aac aat ttt att gtc     879
Leu Ile His Asp Ser Ser Glu Tyr Val Thr Gly Asn Asn Phe Ile Val
            255                 260                 265
gat tat gga ggc acc tta cca ggt gta cca att tat tct tct ctg taa     927
Asp Tyr Gly Gly Thr Leu Pro Gly Val Pro Ile Tyr Ser Ser Leu
        270                 275                 280
attttggtac attatgcact aacgaaaagt aatactatga attattccat tacatatctt     987

caaattagac aatatggagt tctgaagtct atgtatatgg agaattattg tgaagtggct     1047

accaccttga tcttaccata taatgctcca gatgtccatg tataaatcat gtggtcgcga     1107

ttggagtttt gatatgttca taaaaaaaaa aaaaaa                                1143

<210> 91
<211> 282
<212> PRT
<213> Glycine max

<400> 91
Met Asn Ala Ile Ala Ala Glu Gln Val Leu Glu Pro Trp His Lys Leu
1               5                   10                  15
Asp Asp Lys Val Val Leu Val Thr Gly Ala Ser Ser Gly Leu Gly Arg
            20                  25                  30
Asp Phe Cys Ile Asp Leu Ala Lys Ala Gly Cys Cys Val Val Ala Ala
        35                  40                  45
Ala Arg Arg Leu Asp Arg Leu Thr Ser Leu Cys His Glu Ile Asn His
    50                  55                  60
Arg Trp Pro Ser Asn Val Gly Ile His Arg Ala Val Ala Val Glu Leu
65                  70                  75                  80
Asp Val Ala Ala Asp Gly Pro Ala Ile Asp Arg Ala Val Gln Lys Ala
                85                  90                  95
Trp Asp Ala Phe Gly Arg Val Asp Ser Leu Ile Asn Asn Ala Gly Val
            100                 105                 110
Arg Gly Ser Val Lys Ser Pro Leu Lys Leu Ser Glu Glu Glu Trp Asp
        115                 120                 125
His Val Phe Lys Thr Asn Leu Thr Gly Cys Trp Leu Val Ser Lys Tyr
    130                 135                 140
Val Cys Lys Arg Met Cys Asp Ile Gln Leu Lys Gly Ser Ile Ile Asn
145                 150                 155                 160
Ile Ser Ser Val Ser Gly Leu Asn Arg Gly Gln Leu Pro Gly Ala Ala
                165                 170                 175
Ala Tyr Ala Ser Ser Lys Ala Gly Val Asn Met Leu Thr Lys Val Met
            180                 185                 190
Ala Met Glu Leu Gly Met His Lys Ile Arg Val Asn Ser Ile Ser Pro
        195                 200                 205
Gly Ile Phe Lys Ser Glu Ile Thr Glu Asn Leu Leu Gln Lys Asp Trp
    210                 215                 220
Leu Asn Asp Val Val Arg Lys Ile Met Pro Leu Arg Arg Leu Gly Thr
225                 230                 235                 240
Ser Asp Pro Ala Leu Thr Ser Leu Ala Arg Tyr Leu Ile His Asp Ser
                245                 250                 255
Ser Glu Tyr Val Thr Gly Asn Asn Phe Ile Val Asp Tyr Gly Gly Thr
            260                 265                 270
Leu Pro Gly Val Pro Ile Tyr Ser Ser Leu
        275                 280

<210> 92
<211> 1176
<212> DNA
<213> Glycine max

<220>
<221> CDS
<222> (74)..(883)

<400> 92
cacataagca attaattaag cactctaaaa catatcttta tttccttgtc tacatatctt      60

gaaaaactca atc atg gct agt gtt tct tcg gtt tta gct cca ttt aga        109
               Met Ala Ser Val Ser Ser Val Leu Ala Pro Phe Arg
               1               5                   10
agg ctt gat ggg aag gtg gcg att atc act ggt ggt gcg agt ggt cta       157
Arg Leu Asp Gly Lys Val Ala Ile Ile Thr Gly Gly Ala Ser Gly Leu
        15                  20                  25
ggt gca gcc act gca aga ctc ttc tct aag cat gga gca tat gta gtc       205
Gly Ala Ala Thr Ala Arg Leu Phe Ser Lys His Gly Ala Tyr Val Val

30                  35                  40
ata gct gat att caa gac gac ttg ggt ctc tct gtt gcc aaa gag tta          253
Ile Ala Asp Ile Gln Asp Asp Leu Gly Leu Ser Val Ala Lys Glu Leu
45                  50                  55                  60
gaa tct gct tcc tat gtc cat tgc gat gtg aca aag gaa gag gac gtt          301
Glu Ser Ala Ser Tyr Val His Cys Asp Val Thr Lys Glu Glu Asp Val
                65                  70                  75
gaa aac tgc gtg aac aca acg gtt tcc aag tat ggc aaa tta gat atc          349
Glu Asn Cys Val Asn Thr Thr Val Ser Lys Tyr Gly Lys Leu Asp Ile
            80                  85                  90
atg ttt aac aat gca ggt gta tct gat gag atc aaa aca agc att ctt          397
Met Phe Asn Asn Ala Gly Val Ser Asp Glu Ile Lys Thr Ser Ile Leu
        95                  100                 105
gac aac aac aag tct gat ttt gag aga gtg ata agt gtt aac ttg gtt          445
Asp Asn Asn Lys Ser Asp Phe Glu Arg Val Ile Ser Val Asn Leu Val
    110                 115                 120
ggt cct ttt ctg gga aca aag cat gct gca agg gtc atg att cct gct          493
Gly Pro Phe Leu Gly Thr Lys His Ala Ala Arg Val Met Ile Pro Ala
125                 130                 135                 140
aaa aag gga tgc ata atc aac aca gct agt gtt gct gga tgc ata ggt          541
Lys Lys Gly Cys Ile Ile Asn Thr Ala Ser Val Ala Gly Cys Ile Gly
                145                 150                 155
gga ggt gct aca cat gcc tac aca agt tca aag cac gca cta att gga          589
Gly Gly Ala Thr His Ala Tyr Thr Ser Ser Lys His Ala Leu Ile Gly
            160                 165                 170
ctg aca aaa aac act gcg gtg gag ctt gga caa cat ggt att agg gta          637
Leu Thr Lys Asn Thr Ala Val Glu Leu Gly Gln His Gly Ile Arg Val
        175                 180                 185
aat tgt ttg tca cct tat ctt gtt gtc aca ccg tta agt aag aaa tat          685
Asn Cys Leu Ser Pro Tyr Leu Val Val Thr Pro Leu Ser Lys Lys Tyr
    190                 195                 200
ttc aat att gat gaa gac aaa att cgt gag ata tat tca aac cta aaa          733
Phe Asn Ile Asp Glu Asp Lys Ile Arg Glu Ile Tyr Ser Asn Leu Lys
205                 210                 215                 220
ggt gct cat ctt gtg cct aac gat gtg gcc gaa gct gct ctt tac ttg          781
Gly Ala His Leu Val Pro Asn Asp Val Ala Glu Ala Ala Leu Tyr Leu
                225                 230                 235
gca ggt gat gag tcc aag tat gtt agt ggt cac aat ctt gtg ata gat          829
Ala Gly Asp Glu Ser Lys Tyr Val Ser Gly His Asn Leu Val Ile Asp
            240                 245                 250
gga ggg tac act gat gta aat gca gga ttt acc gtg ttt ggg cag tct          877
Gly Gly Tyr Thr Asp Val Asn Ala Gly Phe Thr Val Phe Gly Gln Ser
        255                 260                 265
cag taa aaattgtagc tgtgtcttca aagttaaatc attattgttg gtgtttgaag          933
Gln
    270
tgtctgtgtt tcgatggccc tgtagttcag aaacttgagc tgttagcatg tgtgaagaaa          993

aagaaggcaa attaatacag ggtgatatga tacctgcctt actgattatc gtggatgtgt          1053

gtgaagtgtc tgtattcgtg ctgttttggc tttcgtgact ctgtttcgat tgctttgaag          1113

ttcaaaaact ttataggatt atattaatat atatgcctca ctcttagcta aaaaaaaaaa          1173

aaa                                                                        1176

<210> 93
<211> 269
<212> PRT
<213> Glycine max

<400> 93
Met Ala Ser Val Ser Ser Val Leu Ala Pro Phe Arg Arg Leu Asp Gly

1                   5                   10                  15
Lys Val Ala Ile Ile Thr Gly Gly Ala Ser Gly Leu Gly Ala Ala Thr
                20                  25                  30
Ala Arg Leu Phe Ser Lys His Gly Ala Tyr Val Val Ile Ala Asp Ile
            35                  40                  45
Gln Asp Asp Leu Gly Leu Ser Val Ala Lys Glu Leu Glu Ser Ala Ser
    50                  55                  60
Tyr Val His Cys Asp Val Thr Lys Glu Glu Asp Val Glu Asn Cys Val
65                  70                  75                  80
Asn Thr Thr Val Ser Lys Tyr Gly Lys Leu Asp Ile Met Phe Asn Asn
                85                  90                  95
Ala Gly Val Ser Asp Glu Ile Lys Thr Ser Ile Leu Asp Asn Asn Lys
            100                 105                 110
Ser Asp Phe Glu Arg Val Ile Ser Val Asn Leu Val Gly Pro Phe Leu
        115                 120                 125
Gly Thr Lys His Ala Ala Arg Val Met Ile Pro Ala Lys Lys Gly Cys
    130                 135                 140
Ile Ile Asn Thr Ala Ser Val Ala Gly Cys Ile Gly Gly Gly Ala Thr
145                 150                 155                 160
His Ala Tyr Thr Ser Ser Lys His Ala Leu Ile Gly Leu Thr Lys Asn
                165                 170                 175
Thr Ala Val Glu Leu Gly Gln His Gly Ile Arg Val Asn Cys Leu Ser
            180                 185                 190
Pro Tyr Leu Val Val Thr Pro Leu Ser Lys Lys Tyr Phe Asn Ile Asp
        195                 200                 205
Glu Asp Lys Ile Arg Glu Ile Tyr Ser Asn Leu Lys Gly Ala His Leu
    210                 215                 220
Val Pro Asn Asp Val Ala Glu Ala Ala Leu Tyr Leu Ala Gly Asp Glu
225                 230                 235                 240
Ser Lys Tyr Val Ser Gly His Asn Leu Val Ile Asp Gly Gly Tyr Thr
                245                 250                 255
Asp Val Asn Ala Gly Phe Thr Val Phe Gly Gln Ser Gln
            260                 265

<210> 94
<211> 858
<212> DNA
<213> Brassica napus

<220>
<221> CDS
<222> (1)..(858)

<400> 94
atg agc agc aat cat cag act gtg atg aag caa cta gag cca tgg tgc      48
Met Ser Ser Asn His Gln Thr Val Met Lys Gln Leu Glu Pro Trp Cys
1               5                   10                  15
gag ctc aaa gac aaa gtg gtt ctc cta aca gga gct tca tcc ggc atc      96
Glu Leu Lys Asp Lys Val Val Leu Leu Thr Gly Ala Ser Ser Gly Ile
            20                  25                  30
gga aga gag atc tgc ctc gat cta gcc aaa tcc ggc tgc aag atc atc      144
Gly Arg Glu Ile Cys Leu Asp Leu Ala Lys Ser Gly Cys Lys Ile Ile
        35                  40                  45
gca gca gct cgt cgt ctc gac cgt ctc caa tcc ctc tgc tcc gag atc      192
Ala Ala Ala Arg Arg Leu Asp Arg Leu Gln Ser Leu Cys Ser Glu Ile
    50                  55                  60
aac gcc tta ttc tcc cca aca aaa acc aaa caa gcc gca cct ctc gag      240
Asn Ala Leu Phe Ser Pro Thr Lys Thr Lys Gln Ala Ala Pro Leu Glu
65                  70                  75                  80
cta gac gtc tcc tca gac tca tcc acc atc cga aac gca gtc aaa caa      288
Leu Asp Val Ser Ser Asp Ser Ser Thr Ile Arg Asn Ala Val Lys Gln
                85                  90                  95
gct tgg gac atc ttc gga aac atc gac gtc ttg atc aac aac gca ggc      336
Ala Trp Asp Ile Phe Gly Asn Ile Asp Val Leu Ile Asn Asn Ala Gly
            100                 105                 110
atc aga ggc aac gtc aag tcg agt ctg gac cta tcc gaa gaa gaa tgg      384
Ile Arg Gly Asn Val Lys Ser Ser Leu Asp Leu Ser Glu Glu Glu Trp
        115                 120                 125
gaa aga gtc ttc aga aca aac cta acc gga cct tgg cta gta tca aaa      432

Glu Arg Val Phe Arg Thr Asn Leu Thr Gly Pro Trp Leu Val Ser Lys
    130                 135                 140
cac gtc tgc gtt ctg atg cgc gac gcc aaa cgc cgc ggc gga gga tcg         480
His Val Cys Val Leu Met Arg Asp Ala Lys Arg Arg Gly Gly Gly Ser
145                 150                 155                 160
gtg ata aac gtt tcc tcc atc gcg ggg ctt cag cgc ggg aag cta ccc         528
Val Ile Asn Val Ser Ser Ile Ala Gly Leu Gln Arg Gly Lys Leu Pro
                165                 170                 175
ggc gcg ttg gcg tac gcg tgt tcg aaa gga ggt ctt gat att atg acg         576
Gly Ala Leu Ala Tyr Ala Cys Ser Lys Gly Gly Leu Asp Ile Met Thr
            180                 185                 190
aag atg atg gcg gtt gag ctg ggt gag tat ggt ata aga gtg aac tcg         624
Lys Met Met Ala Val Glu Leu Gly Glu Tyr Gly Ile Arg Val Asn Ser
        195                 200                 205
ata gct ccg ggg ctg ttt aag tcg gag atc acg gaa ggt ctg gtg agg         672
Ile Ala Pro Gly Leu Phe Lys Ser Glu Ile Thr Glu Gly Leu Val Arg
    210                 215                 220
aaa gag tgg atg aag aat gtg agg aag agg att gtt ccg ttg aag gtg         720
Lys Glu Trp Met Lys Asn Val Arg Lys Arg Ile Val Pro Leu Lys Val
225                 230                 235                 240
cag cag act gtg gac ccg ggg ctt acc tcg ctg gtt agg tat ctg att         768
Gln Gln Thr Val Asp Pro Gly Leu Thr Ser Leu Val Arg Tyr Leu Ile
                245                 250                 255
ccc cac tct tcc agg tat gtc tct cgc aat gtt tac att gtt gac gcg         816
Pro His Ser Ser Arg Tyr Val Ser Arg Asn Val Tyr Ile Val Asp Ala
            260                 265                 270
ggt gct aca ttg tct ggt cta acg att ttt tct tca ctt tga                 858
Gly Ala Thr Leu Ser Gly Leu Thr Ile Phe Ser Ser Leu
        275                 280                 285

<210> 95
<211> 285
<212> PRT
<213> Brassica napus

<400> 95
Met Ser Ser Asn His Gln Thr Val Met Lys Gln Leu Glu Pro Trp Cys
1               5                   10                  15
Glu Leu Lys Asp Lys Val Val Leu Leu Thr Gly Ala Ser Ser Gly Ile
            20                  25                  30
Gly Arg Glu Ile Cys Leu Asp Leu Ala Lys Ser Gly Cys Lys Ile Ile
        35                  40                  45
Ala Ala Ala Arg Arg Leu Asp Arg Leu Gln Ser Leu Cys Ser Glu Ile
    50                  55                  60
Asn Ala Leu Phe Ser Pro Thr Lys Thr Lys Gln Ala Ala Pro Leu Glu
65                  70                  75                  80
Leu Asp Val Ser Ser Asp Ser Ser Thr Ile Arg Asn Ala Val Lys Gln
                85                  90                  95
Ala Trp Asp Ile Phe Gly Asn Ile Asp Val Leu Ile Asn Asn Ala Gly
            100                 105                 110
Ile Arg Gly Asn Val Lys Ser Ser Leu Asp Leu Ser Glu Glu Glu Trp
        115                 120                 125
Glu Arg Val Phe Arg Thr Asn Leu Thr Gly Pro Trp Leu Val Ser Lys
    130                 135                 140
His Val Cys Val Leu Met Arg Asp Ala Lys Arg Arg Gly Gly Gly Ser
145                 150                 155                 160
Val Ile Asn Val Ser Ser Ile Ala Gly Leu Gln Arg Gly Lys Leu Pro
                165                 170                 175
Gly Ala Leu Ala Tyr Ala Cys Ser Lys Gly Gly Leu Asp Ile Met Thr
            180                 185                 190
Lys Met Met Ala Val Glu Leu Gly Glu Tyr Gly Ile Arg Val Asn Ser
        195                 200                 205
Ile Ala Pro Gly Leu Phe Lys Ser Glu Ile Thr Glu Gly Leu Val Arg
    210                 215                 220
Lys Glu Trp Met Lys Asn Val Arg Lys Arg Ile Val Pro Leu Lys Val
225                 230                 235                 240
Gln Gln Thr Val Asp Pro Gly Leu Thr Ser Leu Val Arg Tyr Leu Ile
                245                 250                 255
Pro His Ser Ser Arg Tyr Val Ser Arg Asn Val Tyr Ile Val Asp Ala

                260                 265                 270
Gly Ala Thr Leu Ser Gly Leu Thr Ile Phe Ser Ser Leu
        275                 280                 285

<210> 96
<211> 843
<212> DNA
<213> Zea mays

<220>
<221> CDS
<222> (1)..(843)

<400> 96
atg tcg tcg gcg ggg cca ccg ccg ccg ctg ccg ccg tgg agc agg ctg      48
Met Ser Ser Ala Gly Pro Pro Pro Pro Leu Pro Pro Trp Ser Arg Leu
1               5                   10                  15
gag ggg cag gtg gtg ctg gtg acg ggc gct tcc tcc ggc atc gga cgc      96
Glu Gly Gln Val Val Leu Val Thr Gly Ala Ser Ser Gly Ile Gly Arg
            20                  25                  30
gac ttc tgc ctc gac ctg gcg cgc gcc ggc tgc cgc gtc gtc gcc gcc      144
Asp Phe Cys Leu Asp Leu Ala Arg Ala Gly Cys Arg Val Val Ala Ala
        35                  40                  45
gcc cgc cgc gcc gat cgt ctc cgc tcg ctc tgc gac gag atc aac ccc      192
Ala Arg Arg Ala Asp Arg Leu Arg Ser Leu Cys Asp Glu Ile Asn Pro
    50                  55                  60
tcc gct gcc tcc cga gcc ccc cgc gcg gtg gct gtg gag gtc gac gtc      240
Ser Ala Ala Ser Arg Ala Pro Arg Ala Val Ala Val Glu Val Asp Val
65                  70                  75                  80
gcc gcc ggt ggt tcg gcc ctg gag gcg gcg gtg cag aag gcc tgg gac      288
Ala Ala Gly Gly Ser Ala Leu Glu Ala Ala Val Gln Lys Ala Trp Asp
                85                  90                  95
gcc ttt ggc cgt atc gac gcc ttg gtc aac aac gcc ggc ata cga ggt      336
Ala Phe Gly Arg Ile Asp Ala Leu Val Asn Asn Ala Gly Ile Arg Gly
            100                 105                 110
gca gtg cat tct cca tta gat tgg ccc gag gat gag tgg gac aga atc      384
Ala Val His Ser Pro Leu Asp Trp Pro Glu Asp Glu Trp Asp Arg Ile
        115                 120                 125
atc aag acg aac ctt acc gga tca tgg ctc gtg gcc aaa cat gtc tgt      432
Ile Lys Thr Asn Leu Thr Gly Ser Trp Leu Val Ala Lys His Val Cys
    130                 135                 140
cga cgc atg cgt gat gcc aag ctg aag ggt tca gtg gtt aac atc acc      480
Arg Arg Met Arg Asp Ala Lys Leu Lys Gly Ser Val Val Asn Ile Thr
145                 150                 155                 160
tct att gct ggc ctt aac cgt ggg cat ctg cct ggc tcc acg gga tac      528
Ser Ile Ala Gly Leu Asn Arg Gly His Leu Pro Gly Ser Thr Gly Tyr
                165                 170                 175
gca tcc tca aag gct gct gtg cat tat gcc acc aag att atg gct ttg      576
Ala Ser Ser Lys Ala Ala Val His Tyr Ala Thr Lys Ile Met Ala Leu
            180                 185                 190
gaa ttg ggc gcg gat cgc atc aga gtg aac tcg att gca cct gga ctc      624
Glu Leu Gly Ala Asp Arg Ile Arg Val Asn Ser Ile Ala Pro Gly Leu
        195                 200                 205
ttc aaa tca gag ata act gct cct ctg ttt caa aag agg tgg ttg agc      672
Phe Lys Ser Glu Ile Thr Ala Pro Leu Phe Gln Lys Arg Trp Leu Ser
    210                 215                 220
acc gtt gct tca aag ata gtg ccg ctt aag gag cat ggc gct act gat      720
Thr Val Ala Ser Lys Ile Val Pro Leu Lys Glu His Gly Ala Thr Asp
225                 230                 235                 240
cct gca ttg acg tcg ctg gtc cgt ttt ctg atc cat gaa gca tcg tcg      768
Pro Ala Leu Thr Ser Leu Val Arg Phe Leu Ile His Glu Ala Ser Ser
                245                 250                 255
tat gtg act ggc aac atc ttc att gta gac tca ggt gcc acc ata cct      816
Tyr Val Thr Gly Asn Ile Phe Ile Val Asp Ser Gly Ala Thr Ile Pro
            260                 265                 270
ggt gtt ccg ata ttc tca tcc ctg taa                                  843
Gly Val Pro Ile Phe Ser Ser Leu
        275                 280

<210> 97
<211> 280
<212> PRT
<213> Zea mays

<400> 97
Met Ser Ser Ala Gly Pro Pro Pro Pro Leu Pro Pro Trp Ser Arg Leu
1               5                   10                  15
Glu Gly Gln Val Val Leu Val Thr Gly Ala Ser Ser Gly Ile Gly Arg
            20                  25                  30
Asp Phe Cys Leu Asp Leu Ala Arg Ala Gly Cys Arg Val Val Ala Ala
        35                  40                  45
Ala Arg Arg Ala Asp Arg Leu Arg Ser Leu Cys Asp Glu Ile Asn Pro
    50                  55                  60
Ser Ala Ala Ser Arg Ala Pro Arg Ala Val Ala Val Glu Val Asp Val
65                  70                  75                  80
Ala Ala Gly Gly Ser Ala Leu Glu Ala Ala Val Gln Lys Ala Trp Asp
                85                  90                  95
Ala Phe Gly Arg Ile Asp Ala Leu Val Asn Asn Ala Gly Ile Arg Gly
            100                 105                 110
Ala Val His Ser Pro Leu Asp Trp Pro Glu Asp Glu Trp Asp Arg Ile
        115                 120                 125
Ile Lys Thr Asn Leu Thr Gly Ser Trp Leu Val Ala Lys His Val Cys
    130                 135                 140
Arg Arg Met Arg Asp Ala Lys Leu Lys Gly Ser Val Val Asn Ile Thr
145                 150                 155                 160
Ser Ile Ala Gly Leu Asn Arg Gly His Leu Pro Gly Ser Thr Gly Tyr
                165                 170                 175
Ala Ser Ser Lys Ala Ala Val His Tyr Ala Thr Lys Ile Met Ala Leu
            180                 185                 190
Glu Leu Gly Ala Asp Arg Ile Arg Val Asn Ser Ile Ala Pro Gly Leu
        195                 200                 205
Phe Lys Ser Glu Ile Thr Ala Pro Leu Phe Gln Lys Arg Trp Leu Ser
    210                 215                 220
Thr Val Ala Ser Lys Ile Val Pro Leu Lys Glu His Gly Ala Thr Asp
225                 230                 235                 240
Pro Ala Leu Thr Ser Leu Val Arg Phe Leu Ile His Glu Ala Ser Ser
                245                 250                 255
Tyr Val Thr Gly Asn Ile Phe Ile Val Asp Ser Gly Ala Thr Ile Pro
            260                 265                 270
Gly Val Pro Ile Phe Ser Ser Leu
        275                 280

<210> 98
<211> 22
<212> DNA
<213> Artificial sequence

<400> 98
atgagcaatc atcaaaccac gg                                                22

<210> 99
<211> 24
<212> DNA
<213> Artificial sequence

<400> 99
tcaaagagaa gaaaaaatag gcag                                              24


<210> 100
<211> 285
<212> PRT
<213> Artificial sequence

<220>
<221> Variant
<222> (2)..(3)
<223> xaa in position 2 to 3 is any amino acid

<220>
<221> Variant
<222> (6)..(8)
<223> xaa in position 6 to 8 is any amino acid
<220>
<221> Variant
<222> (9)..(13)
<223> xaa in position 9 to 13 is any or no amino acid
<220>
<221> Variant
<222> (17)..(19)
<223> xaa in position 17 to 19 is any amino acid
<220>
<221> Variant
<222> (21)..(26)
<223> xaa in position 21 to 26 is any amino acid
<220>
<221> Variant
<222> (29)..(65)
<223> xaa in position 29 to 65 is any amino acid
<220>
<221> Variant
<222> (66)..(90)
<223> xaa in position 66 to 90 is any or no amino acid
<220>
<221> Variant
<222> (92)..(92)
<223> xaa in position 92 is any amino acid
<220>
<221> Variant
<222> (93)..(95)
<223> xaa in position 93 to 95 is any or no amino acid
<220>
<221> Variant
<222> (97)..(97)
<223> xaa in position 97 is any amino acid
<220>
<221> Variant
<222> (99)..(99)
<223> xaa in position 99 is any amino acid
<220>
<221> Variant
<222> (104)..(124)
<223> xaa in position 104 to 124 is any amino acid
<220>
<221> Variant
<222> (125)..(131)
<223> xaa in position 125 to 131 is any or no amino acid
<220>
<221> Variant
<222> (134)..(146)
<223> xaa in position 134 to 146 is any amino acid
<220>
<221> Variant
<222> (148)..(151)
<223> xaa in position 148 to 151 is any amino acid
<220>
<221> Variant
<222> (152)..(157)
<223> xaa in position 152 to 157 is any or no amino acid
<220>
<221> Variant
<222> (159)..(159)
<223> xaa in position 159 is any amino acid
<220>
<221> Variant
<222> (161)..(161)
<223> xaa in position 161 is any amino acid
<220>

<221> Variant
<222> (163)..(164)
<223> xaa in position 163 to 164 is any amino acid
<220>
<221> Variant
<222> (166)..(176)
<223> xaa in position 166 to 176 is any amino acid
<220>
<221> Variant
<222> (177)..(179)
<223> xaa in position 177 to 179 is any or no amino acid
<220>
<221> Variant
<222> (181)..(183)
<223> xaa in position 181 to 183 is any amino acid
<220>
<221> Variant
<222> (185)..(194)
<223> xaa in position 185 to 194 is any amino acid
<220>
<221> Variant
<222> (196)..(196)
<223> xaa in position 196 is any amino acid
<220>
<221> Variant
<222> (198)..(205)
<223> xaa in position 198 to 205 is any amino acid
<220>
<221> Variant
<222> (207)..(209)
<223> xaa in position 207 to 209 is any amino acid
<220>
<221> Variant
<222> (212)..(213)
<223> xaa in position 212 to 213 is any amino acid
<220>
<221> Variant
<222> (214)..(214)
<223> xaa in position 214 is any or no amino acid
<220>
<221> Variant
<222> (216)..(229)
<223> xaa in position 216 to 229 is any amino acid
<220>
<221> Variant
<222> (230)..(248)
<223> xaa in position 230 to 248 is any or no amino acid
<220>
<221> Variant
<222> (250)..(251)
<223> xaa in position 250 to 251 is any amino acid
<220>
<221> Variant
<222> (253)..(253)
<223> xaa in position 253 is any amino acid
<220>
<221> Variant
<222> (255)..(259)
<223> xaa in position 255 to 259 is any amino acid
<220>
<221> Variant
<222> (261)..(265)
<223> xaa in position 261 to 265 is any amino acid
<220>
<221> Variant
<222> (269)..(272)
<223> xaa in position 269 to 272 is any amino acid
<220>
<221> Variant

<222> (273)..(275)
<223> xaa in position 273 to 275 is any or no amino acid
<220>
<221> Variant
<222> (278)..(283)
<223> xaa in position 278 to 283 is any amino acid

<400> 100
Ala xaa xaa Thr Gly xaa xaa xaa xaa xaa xaa xaa xaa Gly Ile Gly
1               5                   10                  15
xaa xaa xaa Ala xaa xaa xaa xaa xaa xaa Gly Ala xaa xaa xaa xaa
            20                  25                  30
xaa xaa xaa xaa xaa xaa xaa xaa xaa xaa xaa xaa xaa xaa xaa xaa
        35                  40                  45
xaa xaa xaa xaa xaa xaa xaa xaa xaa xaa xaa xaa xaa xaa xaa xaa
    50                  55                  60
xaa xaa xaa xaa xaa xaa xaa xaa xaa xaa xaa xaa xaa xaa xaa xaa
65                  70                  75                  80
xaa xaa xaa xaa xaa xaa xaa xaa xaa xaa Gly xaa xaa xaa xaa Asp
                85                  90                  95
xaa Leu xaa Asn Asn Ala Gly xaa xaa xaa xaa xaa xaa xaa xaa xaa
            100                 105                 110
xaa xaa xaa xaa xaa xaa xaa xaa xaa xaa xaa xaa xaa xaa xaa xaa
        115                 120                 125
xaa xaa xaa Asn Leu xaa xaa xaa xaa xaa xaa xaa xaa xaa xaa xaa
    130                 135                 140
xaa xaa Met xaa xaa xaa xaa xaa xaa xaa xaa xaa xaa Gly xaa Ile
145                 150                 155                 160
xaa Asn xaa xaa Ser xaa xaa xaa xaa xaa xaa xaa xaa xaa xaa xaa
                165                 170                 175
xaa xaa xaa Tyr xaa xaa xaa Lys xaa xaa xaa xaa xaa xaa xaa xaa
            180                 185                 190
xaa xaa Ala xaa Glu xaa xaa xaa xaa xaa xaa xaa xaa Asn xaa xaa
        195                 200                 205
xaa Pro Gly xaa xaa xaa Thr xaa xaa xaa xaa xaa xaa xaa xaa xaa
    210                 215                 220
xaa xaa xaa xaa xaa xaa xaa xaa xaa xaa xaa xaa xaa xaa xaa xaa
225                 230                 235                 240
xaa xaa xaa xaa xaa xaa xaa xaa Pro xaa xaa Arg xaa Gly xaa xaa
                245                 250                 255
xaa xaa xaa Ala xaa xaa xaa xaa xaa Leu Ala Ser xaa xaa xaa xaa
            260                 265                 270
xaa xaa xaa Thr Gly xaa xaa xaa xaa xaa xaa Gly Gly
        275                 280                 285


<210> 101
<211> 26
<212> PRT
<213> Artificial sequence

<220>
<221> Variant
<222> (1)..(1)
<223> xaa in position 1 is Leu, Ile, Val, Phe or Met
<220>
<221> Variant
<222> (2)..(2)
<223> xaa in position 2 is Val, Ile or Leu
<220>
<221> Variant
<222> (3)..(3)
<223> xaa in position 3 is Thr or Ser
<220>
<221> Variant
<222> (4)..(4)
<223> xaa in position 4 is Gly or Ala
<220>
<221> Variant

<222> (5)..(5)
<223> xaa in position 5 is Ala, Gly or Ser
<220>
<221> Variant
<222> (6)..(6)
<223> xaa in position 6 is Ser, Ala, Gly, Thr or Leu
<220>
<221> Variant
<222> (7)..(7)
<223> xaa in position 7 is any amino acid
<220>
<221> Variant
<222> (8)..(9)
<223> xaa in position 8 to 9 is any or no amino acid
<220>
<221> Variant
<222> (11)..(11)
<223> xaa in position 11 is Ile, Leu or Met
<220>
<221> Variant
<222> (13)..(15)
<223> xaa in position 13 to 15 is any amino acid
<220>
<221> Variant
<222> (16)..(16)
<223> xaa in position 16 is Ala, Cys, Val or Gly
<220>
<221> Variant
<222> (17)..(18)
<223> xaa in position 17 to 18 is any amino acid
<220>
<221> Variant
<222> (19)..(19)
<223> xaa in position 19 is Leu, Phe, Tyr or Met
<220>
<221> Variant
<222> (20)..(22)
<223> xaa in position 20 to 22 is any amino acid
<220>
<221> Variant
<222> (24)..(25)
<223> xaa in position 24 to 25 is any amino acid
<220>
<221> Variant
<222> (26)..(26)
<223> xaa in position 26 is Val, Ile or Leu

<400> 101
   Xaa Xaa Xaa Xaa Xaa Xaa Xaa Xaa Xaa Gly Xaa Gly Xaa Xaa Xaa Xaa
   1               5                   10                  15
   Xaa Xaa Xaa Xaa Xaa Xaa Gly Xaa Xaa Xaa
               20                  25


<210> 102
<211> 9
<212> PRT
<213> Artificial sequence

<220>
<221> Variant
<222> (1)..(1)
<223> xaa in position 1 is Ile, Val, Leu, Phe or Ala
<220>
<221> Variant
<222> (2)..(2)
<223> xaa in position 2 is Asp, His, Ser or Asn
<220>
<221> Variant

<222> (3)..(3)
<223> xaa in position 3 is Ile, Val or Phe
<220>
<221> Variant
<222> (4)..(4)
<223> xaa in position 4 is Leu, Val, Met, Ala or Ile
<220>
<221> Variant
<222> (5)..(5)
<223> xaa in position 5 is Val, Phe, Ile, Cys, His or Tyr
<220>
<221> Variant
<222> (6)..(6)
<223> xaa in position 6 is Asn or Ala
<220>
<221> Variant
<222> (7)..(7)
<223> xaa in position 7 is Asn, Cys or Val

<400> 102
   Xaa Xaa Xaa Xaa Xaa Xaa Xaa Ala Gly
   1               5


<210> 103
<211> 58
<212> PRT
<213> Artificial sequence

<220>
<221> Variant
<222> (2)..(2)
<223> xaa in position 2 is Arg, Ser, Ala, Lys, Val or Asn
<220>
<221> Variant
<222> (3)..(3)
<223> xaa in position 3 is Ile or Val
<220>
<221> Variant
<222> (4)..(4)
<223> xaa in position 4 is Ile, Val or Leu
<220>
<221> Variant
<222> (5)..(6)
<223> xaa in position 5 to 6 is any amino acid
<220>
<221> Variant
<222> (7)..(7)
<223> xaa in position 7 is Ser, Ala, Thr or Gly
<220>
<221> Variant
<222> (9)..(10)
<223> xaa in position 9 to 10 is any amino acid
<220>
<221> Variant
<222> (11)..(11)
<223> xaa in position 11 is Gly, Ser, Ala, Arg or Trp
<220>
<221> Variant
<222> (12)..(19)
<223> xaa in position 12 to 19 is any amino acid
<220>
<221> Variant
<222> (20)..(22)
<223> xaa in position 20 to 22 is any or no amino acid
<220>
<221> Variant
<222> (24)..(24)
<223> xaa in position 24 is any amino acid

<220>
<221> Variant
<222> (25)..(25)
<223> Xaa in position 25 is Ala, Thr, Ser, Val or Leu
<220>
<221> Variant
<222> (26)..(26)
<223> Xaa in position 26 is Ala, Ser or Thr
<220>
<221> Variant
<222> (28)..(28)
<223> Xaa in position 28 is Ala, Gly, His, Phe or Ser
<220>
<221> Variant
<222> (29)..(29)
<223> Xaa in position 29 is Gly or Ala
<220>
<221> Variant
<222> (30)..(30)
<223> Xaa in position 30 is Val, Leu, Ile, Met or Gln
<220>
<221> Variant
<222> (31)..(32)
<223> Xaa in position 31 to 32 is any amino acid
<220>
<221> Variant
<222> (33)..(33)
<223> Xaa in position 33 is Leu, Phe, Met or Ile
<220>
<221> Variant
<222> (34)..(34)
<223> Xaa in position 34 is Thr, Ser, Ile or Ala
<220>
<221> Variant
<222> (35)..(35)
<223> Xaa in position 35 is Lys, Arg, Gln or Met
<220>
<221> Variant
<222> (36)..(37)
<223> Xaa in position 36 to 37 is any amino acid
<220>
<221> Variant
<222> (38)..(38)
<223> Xaa in position 38 is Ala, Gly or Ser
<220>
<221> Variant
<222> (39)..(39)
<223> Xaa in position 39 is any amino acid
<220>
<221> Variant
<222> (40)..(40)
<223> Xaa in position 40 is Glu, Asp or Ala
<220>
<221> Variant
<222> (41)..(41)
<223> Xaa in position 41 is any amino acid
<220>
<221> Variant
<222> (42)..(42)
<223> Xaa in position 42 is Ala, Gly, Val, Ile or Ser
<220>
<221> Variant
<222> (43)..(44)
<223> Xaa in position 43 to 44 is any amino acid
<220>
<221> Variant
<222> (45)..(45)
<223> Xaa in position 45 is Gly, Asn, Lys or Arg
<220>

<221> Variant
<222> (46)..(46)
<223> Xaa in position 46 is Ile or Val
<220>
<221> Variant
<222> (47)..(47)
<223> Xaa in position 47 is Thr, Arg, Asn or Cys
<220>
<221> Variant
<222> (48)..(48)
<223> Xaa in position 48 is Val, Cys, Ala or Ile
<220>
<221> Variant
<222> (50)..(50)
<223> Xaa in position 50 is Ala, Cys, Val, Ser, Thr or Gly
<220>
<221> Variant
<222> (51)..(51)
<223> Xaa in position 51 is Ile, Val or Leu
<220>
<221> Variant
<222> (52)..(52)
<223> Xaa in position 52 is any amino acid
<220>
<221> Variant
<222> (55)..(55)
<223> Xaa in position 55 is any amino acid
<220>
<221> Variant
<222> (56)..(56)
<223> Xaa in position 56 is Ile, Val, Thr or Met
<220>
<221> Variant
<222> (57)..(57)
<223> Xaa in position 57 is any amino acid
<220>
<221> Variant
<222> (58)..(58)
<223> Xaa in position 58 is Thr or Ser

<400> 103
Gly Xaa Xaa Xaa Xaa Xaa Xaa Ser Xaa Xaa Xaa Xaa Xaa Xaa Xaa Xaa
1               5                   10                  15
Xaa Xaa Xaa Xaa Xaa Xaa Tyr Xaa Xaa Xaa Lys Xaa Xaa Xaa Xaa Xaa
            20                  25                  30
Xaa Xaa Xaa Xaa Xaa Xaa Xaa Xaa Xaa Xaa Xaa Xaa Xaa Xaa Xaa Xaa
        35                  40                  45
Asn Xaa Xaa Xaa Pro Gly Xaa Xaa Xaa Xaa
    50                  55


<210> 104
<211> 37
<212> PRT
<213> Artificial sequence

<220>
<221> Variant
<222> (1)..(1)
<223> Xaa in position 1 is Pro, Lys, Gln or Trp
<220>
<221> Variant
<222> (2)..(5)
<223> Xaa in position 2 to 5 is any amino acid
<220>
<221> Variant
<222> (6)..(6)
<223> Xaa in position 6 is Gly, Ala, Ile or Val
<220>

<221> Variant
<222> (7)..(9)
<223> xaa in position 7 to 9 is any amino acid
<220>
<221> Variant
<222> (10)..(10)
<223> xaa in position 10 is Glu, Asp, Gln or His
<220>
<221> Variant
<222> (11)..(11)
<223> xaa in position 11 is Ile, Val or Leu
<220>
<221> Variant
<222> (12)..(12)
<223> xaa in position 12 is Ala, Gly or Ser
<220>
<221> Variant
<222> (13)..(16)
<223> xaa in position 13 to 16 is any amino acid
<220>
<221> Variant
<222> (17)..(17)
<223> xaa in position 17 is Phe, Tyr or Trp
<220>
<221> Variant
<222> (18)..(18)
<223> xaa in position 18 is Leu or Phe
<220>
<221> Variant
<222> (19)..(19)
<223> xaa in position 19 is Ala, Cys, Val, Gly or Leu
<220>
<221> Variant
<222> (20)..(20)
<223> xaa in position 20 is any amino acid
<220>
<221> Variant
<222> (21)..(23)
<223> xaa in position 21 to 23 is any or no amino acid
<220>
<221> Variant
<222> (24)..(24)
<223> xaa in position 24 is Ala, Ser, Gln or Gly
<220>
<221> Variant
<222> (25)..(25)
<223> xaa in position 25 is any amino acid
<220>
<221> Variant
<222> (26)..(26)
<223> xaa in position 26 is Tyr, Phe, Trp, Gln, Gly or Asn
<220>
<221> Variant
<222> (27)..(27)
<223> xaa in position 27 is Ile, Val, Met, Thr, Leu or Ala
<220>
<221> Variant
<222> (28)..(28)
<223> xaa in position 28 is Thr, Asn, Ser or Arg
<220>
<221> Variant
<222> (29)..(29)
<223> xaa in position 29 is Gly or Ala
<220>
<221> Variant
<222> (30)..(33)
<223> xaa in position 30 to 33 is any amino acid
<220>
<221> Variant

<222> (34)..(34)
<223> xaa in position 34 is Val, Ile, Leu, Ala or Met
<220>
<221> Variant
<222> (35)..(35)
<223> xaa in position 35 is Asp, Asn, Ser, Thr or Cys
<220>
<221> Variant
<222> (36)..(36)
<223> xaa in position 36 is Gly or Ala

<400> 104
xaa xaa xaa xaa xaa xaa xaa xaa xaa xaa xaa xaa xaa xaa xaa xaa
1               5                   10                  15
xaa xaa xaa xaa xaa xaa xaa xaa xaa xaa xaa xaa xaa xaa xaa xaa
            20                  25                  30
xaa xaa xaa xaa Gly
        35

<210> 105
<211> 1687
<212> DNA
<213> Hordeum vulgare

<220>
<221> CDS
<222> (165)..(1409)

<400> 105
gaattcccgg gtcgacagcg aaggctcctc ccatcccctc cccccaccgc ctccaccgac     60

gcatcgatcg aatccgggac acgatctcgg attcgctcgg ccaatcgacg cgcaccagcc     120

ccccggatcg tcgttcgtac tcccccaccg cgagcgcttc agcc atg gca gga atg     176
                                                 Met Ala Gly Met
                                                 1
gca cca gag ggt tcc cag ttt gat gct aag cac tat gac agt aag atg     224
Ala Pro Glu Gly Ser Gln Phe Asp Ala Lys His Tyr Asp Ser Lys Met
5                   10                  15                  20
act gag tta ctg aac caa ggt gac act gag gag ttt ttc acc tca tat     272
Thr Glu Leu Leu Asn Gln Gly Asp Thr Glu Glu Phe Phe Thr Ser Tyr
                25                  30                  35
gat gag gtg cat gaa agt ttt gat gac atg ggc ctc caa gag aac ctt     320
Asp Glu Val His Glu Ser Phe Asp Asp Met Gly Leu Gln Glu Asn Leu
            40                  45                  50
ctt aga ggc atc tat gct tat ggt ttt gag aag cca tca gcc att cag     368
Leu Arg Gly Ile Tyr Ala Tyr Gly Phe Glu Lys Pro Ser Ala Ile Gln
        55                  60                  65
caa aga gga att gtt ccc ttc tgc aag ggt ctt gat gtg att caa caa     416
Gln Arg Gly Ile Val Pro Phe Cys Lys Gly Leu Asp Val Ile Gln Gln
    70                  75                  80
gct cag tct ggt aca gga aaa act gcc acc ttc tgt tct gga atc ttg     464
Ala Gln Ser Gly Thr Gly Lys Thr Ala Thr Phe Cys Ser Gly Ile Leu
85                  90                  95                  100
cag cag ctt gac tat gga ttg gtt gag tgc cag gca ttg gtc ctt gct     512
Gln Gln Leu Asp Tyr Gly Leu Val Glu Cys Gln Ala Leu Val Leu Ala
                105                 110                 115
ccg acc cgt gag ctt gca cag cag att gag aag gtc atg cgt gct ctt     560
Pro Thr Arg Glu Leu Ala Gln Gln Ile Glu Lys Val Met Arg Ala Leu
            120                 125                 130
ggt gac tac tta ggc gtc aag gtg cat gca tgt gtt ggt gga act tct     608
Gly Asp Tyr Leu Gly Val Lys Val His Ala Cys Val Gly Gly Thr Ser
        135                 140                 145
gtc cgt gag gat cag agg att ctt gcc agt ggt gtg cat gtt gtt gtc     656
Val Arg Glu Asp Gln Arg Ile Leu Ala Ser Gly Val His Val Val Val
    150                 155                 160
ggc aca cct ggt cgt gtg ttc gat atg ttg cgc agg caa tcc ctc cgc     704

Gly Thr Pro Gly Arg Val Phe Asp Met Leu Arg Arg Gln Ser Leu Arg
165                 170                 175                 180
cca gat aac atc aag atg ttt gtg ttg gat gaa gct gat gag atg cta      752
Pro Asp Asn Ile Lys Met Phe Val Leu Asp Glu Ala Asp Glu Met Leu
                185                 190                 195
tca cgt ggt ttc aag gat cag atc tat gat atc ttc cag ctt ctt cca      800
Ser Arg Gly Phe Lys Asp Gln Ile Tyr Asp Ile Phe Gln Leu Leu Pro
            200                 205                 210
tca aag att cag gtt ggg gtg ttc tct gct acc atg cct cct gag gct      848
Ser Lys Ile Gln Val Gly Val Phe Ser Ala Thr Met Pro Pro Glu Ala
        215                 220                 225
ctt gag ata acc cgc aag ttc atg aac aag ccc gtg agg att ctt gtc      896
Leu Glu Ile Thr Arg Lys Phe Met Asn Lys Pro Val Arg Ile Leu Val
    230                 235                 240
aag aga gat gag ctt acc ctt gag ggt atc aag caa ttc tat gtc aat      944
Lys Arg Asp Glu Leu Thr Leu Glu Gly Ile Lys Gln Phe Tyr Val Asn
245                 250                 255                 260
gtt gag aag gaa gag tgg aag ctg gac aca ctc tgt gac ctg tac gag      992
Val Glu Lys Glu Glu Trp Lys Leu Asp Thr Leu Cys Asp Leu Tyr Glu
                265                 270                 275
acc ttg gca att acc cag agt gtc atc ttt gtg aac acc cgc cgc aag      1040
Thr Leu Ala Ile Thr Gln Ser Val Ile Phe Val Asn Thr Arg Arg Lys
            280                 285                 290
gtg gac tgg ctc acc gac aag atg agg gga agg gac cac act gtc tct      1088
Val Asp Trp Leu Thr Asp Lys Met Arg Gly Arg Asp His Thr Val Ser
        295                 300                 305
gca aca cac gga gac atg gac cag aac act agg gac atc atc atg agg      1136
Ala Thr His Gly Asp Met Asp Gln Asn Thr Arg Asp Ile Ile Met Arg
    310                 315                 320
gag ttc aga tct ggc tct tct cgt gtg ctc atc acc acc gac ctg ctg      1184
Glu Phe Arg Ser Gly Ser Ser Arg Val Leu Ile Thr Thr Asp Leu Leu
325                 330                 335                 340
gcc cgt ggt att gat gtg cag caa gtc tcc ctt gtc atc aac tac gac      1232
Ala Arg Gly Ile Asp Val Gln Gln Val Ser Leu Val Ile Asn Tyr Asp
                345                 350                 355
ctg ccg acc cag cct gag aac tac ctc cat cgc att ggt cgt agt ggt      1280
Leu Pro Thr Gln Pro Glu Asn Tyr Leu His Arg Ile Gly Arg Ser Gly
            360                 365                 370
cgg ttc ggg agg aag gga gtt gcc atc aac ttc gtc acc cgt gaa gat      1328
Arg Phe Gly Arg Lys Gly Val Ala Ile Asn Phe Val Thr Arg Glu Asp
        375                 380                 385
gag agg atg ctg ttc gac atc cag aag ttc tac aac gtg gtc att gag      1376
Glu Arg Met Leu Phe Asp Ile Gln Lys Phe Tyr Asn Val Val Ile Glu
    390                 395                 400
gag ctc ccg gcc aat gtc gcc gac ctt ctc tag attattatca taggaatgca      1429
Glu Leu Pro Ala Asn Val Ala Asp Leu Leu
405                 410                 415
gtaaggtgct cgataaagtt cgtttttgtt gaatatctcc caactctcag ttttggagtg      1489

gtaaggtctg gttctgtttg aatcgcggtt aaggatcgag tttttttctt tttcttgtta      1549

tgcaatgtcc ggaaattttt gtagtgcgta ggctgtgcca actcccgggc tagctgggat      1609

agagtgacac tgatggagtt tttacttctc gttgtgaagc tattttattg cccctctgtt      1669

tttaaaaaaa aaaaaaaa                                                    1687

<210> 106
<211> 414
<212> PRT
<213> Hordeum vulgare

<400> 106

Met Ala Gly Met Ala Pro Glu Gly Ser Gln Phe Asp Ala Lys His Tyr
1               5                   10                  15
Asp Ser Lys Met Thr Glu Leu Leu Asn Gln Gly Asp Thr Glu Glu Phe
            20                  25                  30
Phe Thr Ser Tyr Asp Glu Val His Glu Ser Phe Asp Asp Met Gly Leu
        35                  40                  45
Gln Glu Asn Leu Leu Arg Gly Ile Tyr Ala Tyr Gly Phe Glu Lys Pro
    50                  55                  60
Ser Ala Ile Gln Gln Arg Gly Ile Val Pro Phe Cys Lys Gly Leu Asp
65                  70                  75                  80
Val Ile Gln Gln Ala Gln Ser Gly Thr Gly Lys Thr Ala Thr Phe Cys
                85                  90                  95
Ser Gly Ile Leu Gln Gln Leu Asp Tyr Gly Leu Val Glu Cys Gln Ala
            100                 105                 110
Leu Val Leu Ala Pro Thr Arg Glu Leu Ala Gln Gln Ile Glu Lys Val
        115                 120                 125
Met Arg Ala Leu Gly Asp Tyr Leu Gly Val Lys Val His Ala Cys Val
    130                 135                 140
Gly Gly Thr Ser Val Arg Glu Asp Gln Arg Ile Leu Ala Ser Gly Val
145                 150                 155                 160
His Val Val Val Gly Thr Pro Gly Arg Val Phe Asp Met Leu Arg Arg
                165                 170                 175
Gln Ser Leu Arg Pro Asp Asn Ile Lys Met Phe Val Leu Asp Glu Ala
            180                 185                 190
Asp Glu Met Leu Ser Arg Gly Phe Lys Asp Gln Ile Tyr Asp Ile Phe
        195                 200                 205
Gln Leu Leu Pro Ser Lys Ile Gln Val Gly Val Phe Ser Ala Thr Met
    210                 215                 220
Pro Pro Glu Ala Leu Glu Ile Thr Arg Lys Phe Met Asn Lys Pro Val
225                 230                 235                 240
Arg Ile Leu Val Lys Arg Asp Glu Leu Thr Leu Glu Gly Ile Lys Gln
                245                 250                 255
Phe Tyr Val Asn Val Glu Lys Glu Glu Trp Lys Leu Asp Thr Leu Cys
            260                 265                 270
Asp Leu Tyr Glu Thr Leu Ala Ile Thr Gln Ser Val Ile Phe Val Asn
        275                 280                 285
Thr Arg Arg Lys Val Asp Trp Leu Thr Asp Lys Met Arg Gly Arg Asp
    290                 295                 300
His Thr Val Ser Ala Thr His Gly Asp Met Asp Gln Asn Thr Arg Asp
305                 310                 315                 320
Ile Ile Met Arg Glu Phe Arg Ser Gly Ser Ser Arg Val Leu Ile Thr
                325                 330                 335
Thr Asp Leu Leu Ala Arg Gly Ile Asp Val Gln Gln Val Ser Leu Val
            340                 345                 350
Ile Asn Tyr Asp Leu Pro Thr Gln Pro Glu Asn Tyr Leu His Arg Ile
        355                 360                 365
Gly Arg Ser Gly Arg Phe Gly Arg Lys Gly Val Ala Ile Asn Phe Val
    370                 375                 380
Thr Arg Glu Asp Glu Arg Met Leu Phe Asp Ile Gln Lys Phe Tyr Asn
385                 390                 395                 400
Val Val Ile Glu Glu Leu Pro Ala Asn Val Ala Asp Leu Leu
                405                 410

<210> 107
<211> 1239
<212> DNA
<213> Brassica napus

<220>
<221> CDS
<222> (1)..(1239)

<400> 107
atg gca gga tcc gca cca gaa ggc act cag ttt gat gct cgt cag ttc      48
Met Ala Gly Ser Ala Pro Glu Gly Thr Gln Phe Asp Ala Arg Gln Phe
1               5                   10                  15
gac cag aag ttg aac gaa gtt ctt gag gga cag gat gag ttc ttc acc      96
Asp Gln Lys Leu Asn Glu Val Leu Glu Gly Gln Asp Glu Phe Phe Thr
            20                  25                  30

tct tac gat gaa tcc cac gac agc ttt gac gcc atg ggt ctc caa gag 144
Ser Tyr Asp Glu Ser His Asp Ser Phe Asp Ala Met Gly Leu Gln Glu
        35                  40                  45
aat ctc ctc agg ggt atc tac gct tac ggt ttc gag aag cct tct gct 192
Asn Leu Leu Arg Gly Ile Tyr Ala Tyr Gly Phe Glu Lys Pro Ser Ala
    50                  55                  60
atc cag caa aga gga atc gtt ccc ttt tgc aaa ggc ctc gac gtg atc 240
Ile Gln Gln Arg Gly Ile Val Pro Phe Cys Lys Gly Leu Asp Val Ile
65                  70                  75                  80
cag cag gcc cag tcc ggt acc ggc aaa acc gcc act ttc tgc tcc ggc 288
Gln Gln Ala Gln Ser Gly Thr Gly Lys Thr Ala Thr Phe Cys Ser Gly
                85                  90                  95
gtc ctc cag cag cta gac tac tcc ctc gtc cag tgc caa gcc ctc gtc 336
Val Leu Gln Gln Leu Asp Tyr Ser Leu Val Gln Cys Gln Ala Leu Val
            100                 105                 110
ctc gcc ccg acc aga gag ctc gcc cag cag atc gag aag gtc atg aga 384
Leu Ala Pro Thr Arg Glu Leu Ala Gln Gln Ile Glu Lys Val Met Arg
        115                 120                 125
gcc ctc ggc gac tac ctc ggc gtc aag gtc cac gcc tgc gtc ggc ggg 432
Ala Leu Gly Asp Tyr Leu Gly Val Lys Val His Ala Cys Val Gly Gly
    130                 135                 140
acc agc gtc cgc gag gac cag cgc atc ctc cag gcc ggc gtc cac gtc 480
Thr Ser Val Arg Glu Asp Gln Arg Ile Leu Gln Ala Gly Val His Val
145                 150                 155                 160
gtc gtc ggc acc ccg ggg cgc gtg ttc gac atg ctg aag cgc cag tcc 528
Val Val Gly Thr Pro Gly Arg Val Phe Asp Met Leu Lys Arg Gln Ser
                165                 170                 175
ctc cgc gcc gac aac atc aag atg ttc gtc ctg gac gag gcc gac gag 576
Leu Arg Ala Asp Asn Ile Lys Met Phe Val Leu Asp Glu Ala Asp Glu
            180                 185                 190
atg ctc tcc cgc ggg ttc aag gac cag atc tac gac atc ttc cag ctc 624
Met Leu Ser Arg Gly Phe Lys Asp Gln Ile Tyr Asp Ile Phe Gln Leu
        195                 200                 205
ctc ccg ccg aag atc cag gtg gga gtc ttc tcg gcg acc atg ccg ccg 672
Leu Pro Pro Lys Ile Gln Val Gly Val Phe Ser Ala Thr Met Pro Pro
    210                 215                 220
gag gct ctc gag atc acg agg aag ttc atg agc aag ccg gtg agg atc 720
Glu Ala Leu Glu Ile Thr Arg Lys Phe Met Ser Lys Pro Val Arg Ile
225                 230                 235                 240
ctg gtg aag cgc gac gag ctg acc ttg gaa ggt atc aag cag ttt tac 768
Leu Val Lys Arg Asp Glu Leu Thr Leu Glu Gly Ile Lys Gln Phe Tyr
                245                 250                 255
gtc aac gtt gag aag gaa gac tgg aag ctc gag acg ctc tgc gac ctc 816
Val Asn Val Glu Lys Glu Asp Trp Lys Leu Glu Thr Leu Cys Asp Leu
            260                 265                 270
tac gag acg ctg gcc atc act cag agc gtc atc ttc gtg aac act agg 864
Tyr Glu Thr Leu Ala Ile Thr Gln Ser Val Ile Phe Val Asn Thr Arg
        275                 280                 285
cgg aag gtt gac tgg ctc act gat aaa atg agg ggg cgt gac cac acg 912
Arg Lys Val Asp Trp Leu Thr Asp Lys Met Arg Gly Arg Asp His Thr
    290                 295                 300
gtc tcg gcg act cac gga gac atg gac cag aac act aga gac att atc 960
Val Ser Ala Thr His Gly Asp Met Asp Gln Asn Thr Arg Asp Ile Ile
305                 310                 315                 320
atg aga gag ttc agg tct ggc tct tcg cgt gtg ctg atc acc act gat 1008
Met Arg Glu Phe Arg Ser Gly Ser Ser Arg Val Leu Ile Thr Thr Asp
                325                 330                 335
ctc ttg gct cgt ggt atc gat gtt cag cag gtg tct ttg gtg atc aac 1056
Leu Leu Ala Arg Gly Ile Asp Val Gln Gln Val Ser Leu Val Ile Asn
            340                 345                 350
tac gat ctg ccg act cag cct gag aac tac ctt cac cgt att gga aga 1104
Tyr Asp Leu Pro Thr Gln Pro Glu Asn Tyr Leu His Arg Ile Gly Arg
        355                 360                 365
agt ggg agg ttt ggg agg aaa ggt gtg gcg atc aac ttt gtg acc aag 1152
Ser Gly Arg Phe Gly Arg Lys Gly Val Ala Ile Asn Phe Val Thr Lys
    370                 375                 380
gat gat gag agg atg ctg ttt gat att cag aag ttt tac aac gtg gtt 1200
Asp Asp Glu Arg Met Leu Phe Asp Ile Gln Lys Phe Tyr Asn Val Val
385                 390                 395                 400

gtt gag gag ctg cct tcc aac gtt gct gat ctg ctg tga                    1239
Val Glu Glu Leu Pro Ser Asn Val Ala Asp Leu Leu
                405                 410

<210> 108
<211> 412
<212> PRT
<213> Brassica napus

<400> 108
Met Ala Gly Ser Ala Pro Glu Gly Thr Gln Phe Asp Ala Arg Gln Phe
1               5                   10                  15
Asp Gln Lys Leu Asn Glu Val Leu Glu Gly Gln Asp Glu Phe Phe Thr
            20                  25                  30
Ser Tyr Asp Glu Ser His Asp Ser Phe Asp Ala Met Gly Leu Gln Glu
        35                  40                  45
Asn Leu Leu Arg Gly Ile Tyr Ala Tyr Gly Phe Glu Lys Pro Ser Ala
    50                  55                  60
Ile Gln Gln Arg Gly Ile Val Pro Phe Cys Lys Gly Leu Asp Val Ile
65                  70                  75                  80
Gln Gln Ala Gln Ser Gly Thr Gly Lys Thr Ala Thr Phe Cys Ser Gly
                85                  90                  95
Val Leu Gln Gln Leu Asp Tyr Ser Leu Val Gln Cys Gln Ala Leu Val
            100                 105                 110
Leu Ala Pro Thr Arg Glu Leu Ala Gln Gln Ile Glu Lys Val Met Arg
        115                 120                 125
Ala Leu Gly Asp Tyr Leu Gly Val Lys Val His Ala Cys Val Gly Gly
    130                 135                 140
Thr Ser Val Arg Glu Asp Gln Arg Ile Leu Gln Ala Gly Val His Val
145                 150                 155                 160
Val Val Gly Thr Pro Gly Arg Val Phe Asp Met Leu Lys Arg Gln Ser
                165                 170                 175
Leu Arg Ala Asp Asn Ile Lys Met Phe Val Leu Asp Glu Ala Asp Glu
            180                 185                 190
Met Leu Ser Arg Gly Phe Lys Asp Gln Ile Tyr Asp Ile Phe Gln Leu
        195                 200                 205
Leu Pro Pro Lys Ile Gln Val Gly Val Phe Ser Ala Thr Met Pro Pro
    210                 215                 220
Glu Ala Leu Glu Ile Thr Arg Lys Phe Met Ser Lys Pro Val Arg Ile
225                 230                 235                 240
Leu Val Lys Arg Asp Glu Leu Thr Leu Glu Gly Ile Lys Gln Phe Tyr
                245                 250                 255
Val Asn Val Glu Lys Glu Asp Trp Lys Leu Glu Thr Leu Cys Asp Leu
            260                 265                 270
Tyr Glu Thr Leu Ala Ile Thr Gln Ser Val Ile Phe Val Asn Thr Arg
        275                 280                 285
Arg Lys Val Asp Trp Leu Thr Asp Lys Met Arg Gly Arg Asp His Thr
    290                 295                 300
Val Ser Ala Thr His Gly Asp Met Asp Gln Asn Thr Arg Asp Ile Ile
305                 310                 315                 320
Met Arg Glu Phe Arg Ser Gly Ser Ser Arg Val Leu Ile Thr Thr Asp
                325                 330                 335
Leu Leu Ala Arg Gly Ile Asp Val Gln Gln Val Ser Leu Val Ile Asn
            340                 345                 350
Tyr Asp Leu Pro Thr Gln Pro Glu Asn Tyr Leu His Arg Ile Gly Arg
        355                 360                 365
Ser Gly Arg Phe Gly Arg Lys Gly Val Ala Ile Asn Phe Val Thr Lys
    370                 375                 380
Asp Asp Glu Arg Met Leu Phe Asp Ile Gln Lys Phe Tyr Asn Val Val
385                 390                 395                 400
Val Glu Glu Leu Pro Ser Asn Val Ala Asp Leu Leu
                405                 410

<210> 109
<211> 1227
<212> DNA
<213> Brassica napus

<220>

<221> CDS
<222> (1)..(1227)

<400> 109
atg gcg gaa gcg aat cct ggc cgt gga ggt gga aga aga agc ggc gga          48
Met Ala Glu Ala Asn Pro Gly Arg Gly Gly Gly Arg Arg Ser Gly Gly
1               5                   10                  15
cca atg gac gac gac aaa ttg gtc ttc gag acg acg gaa ggg atc gag          96
Pro Met Asp Asp Asp Lys Leu Val Phe Glu Thr Thr Glu Gly Ile Glu
            20                  25                  30
ccc atc acg aat ttc aac gac atg ggg atc aag gag gac gtt ctc cgc          144
Pro Ile Thr Asn Phe Asn Asp Met Gly Ile Lys Glu Asp Val Leu Arg
        35                  40                  45
ggc gtc tac gag tac ggt ttc gag aag ccc tcc gcg att cag cag aga          192
Gly Val Tyr Glu Tyr Gly Phe Glu Lys Pro Ser Ala Ile Gln Gln Arg
    50                  55                  60
gcg gtc atg ccg att ctc caa ggc cga gat gtg atc gct cag gct cag          240
Ala Val Met Pro Ile Leu Gln Gly Arg Asp Val Ile Ala Gln Ala Gln
65                  70                  75                  80
tcc ggt acc gga aag acc tca atg att gct ctc tcc gtc tgc caa atc          288
Ser Gly Thr Gly Lys Thr Ser Met Ile Ala Leu Ser Val Cys Gln Ile
                85                  90                  95
gtt gac act tcg tct aga gaa gtt cag gcg ttg ata ttg tcg cca acg          336
Val Asp Thr Ser Ser Arg Glu Val Gln Ala Leu Ile Leu Ser Pro Thr
            100                 105                 110
agg gag ctg gct tca cag acg gag aag act att caa gct att ggg ttg          384
Arg Glu Leu Ala Ser Gln Thr Glu Lys Thr Ile Gln Ala Ile Gly Leu
        115                 120                 125
cac gct aat att cag gcg cat gcg tgt att ggt ggg aag agc gtt gga          432
His Ala Asn Ile Gln Ala His Ala Cys Ile Gly Gly Lys Ser Val Gly
    130                 135                 140
gag gat atc agg aag ctg gag aat ggt gtc cat gtt gtg tct ggg acg          480
Glu Asp Ile Arg Lys Leu Glu Asn Gly Val His Val Val Ser Gly Thr
145                 150                 155                 160
cct ggg cgt gtt tgt gat atg att aag agg agg agt ctg cgt acc aga          528
Pro Gly Arg Val Cys Asp Met Ile Lys Arg Arg Ser Leu Arg Thr Arg
                165                 170                 175
gct att aag ctt ttg att ctt gat gaa tct gat gag atg ctg agc aga          576
Ala Ile Lys Leu Leu Ile Leu Asp Glu Ser Asp Glu Met Leu Ser Arg
            180                 185                 190
ggg ttc aag gac caa ata tat gat gtt tac aga tat ctt cca cct gat          624
Gly Phe Lys Asp Gln Ile Tyr Asp Val Tyr Arg Tyr Leu Pro Pro Asp
        195                 200                 205
ctt cag gtt tgc ttg gtt tct gca act ctt cct cac gag att ttg gag          672
Leu Gln Val Cys Leu Val Ser Ala Thr Leu Pro His Glu Ile Leu Glu
    210                 215                 220
atg aca tca aag ttt atg acg gaa cca gtg aag ata ctt gtg aag cgt          720
Met Thr Ser Lys Phe Met Thr Glu Pro Val Lys Ile Leu Val Lys Arg
225                 230                 235                 240
gat gag ttg act ctt gaa ggc att aaa caa ttt ttc gtt gct gtt gag          768
Asp Glu Leu Thr Leu Glu Gly Ile Lys Gln Phe Phe Val Ala Val Glu
                245                 250                 255
aaa gag gaa tgg aaa ttt gat aca ctc tgt gat ctt tat gac acg ctt          816
Lys Glu Glu Trp Lys Phe Asp Thr Leu Cys Asp Leu Tyr Asp Thr Leu
            260                 265                 270
act atc act caa gct gtt atc ttc tgc aac act aaa cga aag gtg gat          864
Thr Ile Thr Gln Ala Val Ile Phe Cys Asn Thr Lys Arg Lys Val Asp
        275                 280                 285
tgg cta agt gag aaa atg agg act aac aac ttc aca gtc tca tca atg          912
Trp Leu Ser Glu Lys Met Arg Thr Asn Asn Phe Thr Val Ser Ser Met
    290                 295                 300
cac ggt gac atg cct cag aag gaa aga gac gaa atc atg aat cag ttc          960
His Gly Asp Met Pro Gln Lys Glu Arg Asp Glu Ile Met Asn Gln Phe
305                 310                 315                 320
cgg tca ggc gac agt cgt gtt ttg atc acg aca gat gta tgg gca cgt          1008
Arg Ser Gly Asp Ser Arg Val Leu Ile Thr Thr Asp Val Trp Ala Arg
                325                 330                 335
ggg att gac gtg caa caa gtt tct ctc gtc atc aat tat gat ctc ccc          1056
Gly Ile Asp Val Gln Gln Val Ser Leu Val Ile Asn Tyr Asp Leu Pro

                340                 345                 350
aac aac cgt gag ctc tac atc cat cgt att gga cga tct ggt cgt ttc        1104
Asn Asn Arg Glu Leu Tyr Ile His Arg Ile Gly Arg Ser Gly Arg Phe
            355                 360                 365
ggc cgt aag ggt gtt gcg atc aac ttt gtt aaa agc gac gac atc aag        1152
Gly Arg Lys Gly Val Ala Ile Asn Phe Val Lys Ser Asp Asp Ile Lys
    370                 375                 380
atc ctc cgg gac att gag cag tac tac agt act cag att gac gag atg        1200
Ile Leu Arg Asp Ile Glu Gln Tyr Tyr Ser Thr Gln Ile Asp Glu Met
385                 390                 395                 400
cca atg aat gta gct gat ctt atc taa                                    1227
Pro Met Asn Val Ala Asp Leu Ile
                405

<210> 110
<211> 408
<212> PRT
<213> Brassica napus

<400> 110
Met Ala Glu Ala Asn Pro Gly Arg Gly Gly Gly Arg Arg Ser Gly Gly
1               5                   10                  15
Pro Met Asp Asp Asp Lys Leu Val Phe Glu Thr Thr Glu Gly Ile Glu
            20                  25                  30
Pro Ile Thr Asn Phe Asn Asp Met Gly Ile Lys Glu Asp Val Leu Arg
        35                  40                  45
Gly Val Tyr Glu Tyr Gly Phe Glu Lys Pro Ser Ala Ile Gln Gln Arg
    50                  55                  60
Ala Val Met Pro Ile Leu Gln Gly Arg Asp Val Ile Ala Gln Ala Gln
65                  70                  75                  80
Ser Gly Thr Gly Lys Thr Ser Met Ile Ala Leu Ser Val Cys Gln Ile
                85                  90                  95
Val Asp Thr Ser Ser Arg Glu Val Gln Ala Leu Ile Leu Ser Pro Thr
            100                 105                 110
Arg Glu Leu Ala Ser Gln Thr Glu Lys Thr Ile Gln Ala Ile Gly Leu
        115                 120                 125
His Ala Asn Ile Gln Ala His Ala Cys Ile Gly Gly Lys Ser Val Gly
    130                 135                 140
Glu Asp Ile Arg Lys Leu Glu Asn Gly Val His Val Val Ser Gly Thr
145                 150                 155                 160
Pro Gly Arg Val Cys Asp Met Ile Lys Arg Arg Ser Leu Arg Thr Arg
                165                 170                 175
Ala Ile Lys Leu Leu Ile Leu Asp Glu Ser Asp Glu Met Leu Ser Arg
            180                 185                 190
Gly Phe Lys Asp Gln Ile Tyr Asp Val Tyr Arg Tyr Leu Pro Pro Asp
        195                 200                 205
Leu Gln Val Cys Leu Val Ser Ala Thr Leu Pro His Glu Ile Leu Glu
    210                 215                 220
Met Thr Ser Lys Phe Met Thr Glu Pro Val Lys Ile Leu Val Lys Arg
225                 230                 235                 240
Asp Glu Leu Thr Leu Glu Gly Ile Lys Gln Phe Phe Val Ala Val Glu
                245                 250                 255
Lys Glu Glu Trp Lys Phe Asp Thr Leu Cys Asp Leu Tyr Asp Thr Leu
            260                 265                 270
Thr Ile Thr Gln Ala Val Ile Phe Cys Asn Thr Lys Arg Lys Val Asp
        275                 280                 285
Trp Leu Ser Glu Lys Met Arg Thr Asn Asn Phe Thr Val Ser Ser Met
    290                 295                 300
His Gly Asp Met Pro Gln Lys Glu Arg Asp Glu Ile Met Asn Gln Phe
305                 310                 315                 320
Arg Ser Gly Asp Ser Arg Val Leu Ile Thr Thr Asp Val Trp Ala Arg
                325                 330                 335
Gly Ile Asp Val Gln Gln Val Ser Leu Val Ile Asn Tyr Asp Leu Pro
            340                 345                 350
Asn Asn Arg Glu Leu Tyr Ile His Arg Ile Gly Arg Ser Gly Arg Phe
        355                 360                 365
Gly Arg Lys Gly Val Ala Ile Asn Phe Val Lys Ser Asp Asp Ile Lys
    370                 375                 380
Ile Leu Arg Asp Ile Glu Gln Tyr Tyr Ser Thr Gln Ile Asp Glu Met

385                 390                 395                 400
Pro Met Asn Val Ala Asp Leu Ile
                405

<210> 111
<211> 1233
<212> DNA
<213> Brassica napus

<220>
<221> CDS
<222> (1)..(1233)

<400> 111
atg gca gga tcg gca aca cag ttt gat gat gcg cgt cag ttt gat cag      48
Met Ala Gly Ser Ala Thr Gln Phe Asp Asp Ala Arg Gln Phe Asp Gln
1               5                   10                  15
gca ctc agt gaa atc ctg gag gga cac gat gag ttc ttc acc tcc tat      96
Ala Leu Ser Glu Ile Leu Glu Gly His Asp Glu Phe Phe Thr Ser Tyr
            20                  25                  30
gat gag gtt tat gag agt ttt gat ggc atg ggt ttg caa gag aac ctt      144
Asp Glu Val Tyr Glu Ser Phe Asp Gly Met Gly Leu Gln Glu Asn Leu
        35                  40                  45
ctt agg ggt atc tat gct tat ggt ttt gaa aag cct tct gct att cag      192
Leu Arg Gly Ile Tyr Ala Tyr Gly Phe Glu Lys Pro Ser Ala Ile Gln
    50                  55                  60
caa aga gga atc gta ccc ttt tgc aag ggt ctt gat gtg atc cag cag      240
Gln Arg Gly Ile Val Pro Phe Cys Lys Gly Leu Asp Val Ile Gln Gln
65                  70                  75                  80
gca cag tct ggt act gga aaa acc gca act ttc tgt tct ggt gtc ttg      288
Ala Gln Ser Gly Thr Gly Lys Thr Ala Thr Phe Cys Ser Gly Val Leu
                85                  90                  95
cag cag ctt gac gtc acc ctt gtc cag tgc caa gct ctg gtc ttg gct      336
Gln Gln Leu Asp Val Thr Leu Val Gln Cys Gln Ala Leu Val Leu Ala
            100                 105                 110
ccc acc agg gag ctt gct cag cag att gag aag gtc atg cgt gct ctc      384
Pro Thr Arg Glu Leu Ala Gln Gln Ile Glu Lys Val Met Arg Ala Leu
        115                 120                 125
gga gac tac att ggc gtc aag gtt cat gcc tgt gtt ggt ggt acc agt      432
Gly Asp Tyr Ile Gly Val Lys Val His Ala Cys Val Gly Gly Thr Ser
    130                 135                 140
gtt ctt gac gat aag cgt atc ctc caa gct ggt gtc cat gtt gtt gtt      480
Val Leu Asp Asp Lys Arg Ile Leu Gln Ala Gly Val His Val Val Val
145                 150                 155                 160
gga act cca ggt cgt gtc ttt gac atg ctc caa aga cag tct ctt cgc      528
Gly Thr Pro Gly Arg Val Phe Asp Met Leu Gln Arg Gln Ser Leu Arg
                165                 170                 175
tct gac tgc atc aag atg ttt gtc ctt gat gaa gct gat gag atg ctc      576
Ser Asp Cys Ile Lys Met Phe Val Leu Asp Glu Ala Asp Glu Met Leu
            180                 185                 190
tcc cgt ggt ttc aag gat cag atc tat gac ata ttc cag ctt ctc cca      624
Ser Arg Gly Phe Lys Asp Gln Ile Tyr Asp Ile Phe Gln Leu Leu Pro
        195                 200                 205
cca aag att cag gtc gga gtg ttc tca gct aca atg cca cca gaa gct      672
Pro Lys Ile Gln Val Gly Val Phe Ser Ala Thr Met Pro Pro Glu Ala
    210                 215                 220
ctc gag atc aca agg aag ttc atg agc aaa cca gtg aga atc ctg gtg      720
Leu Glu Ile Thr Arg Lys Phe Met Ser Lys Pro Val Arg Ile Leu Val
225                 230                 235                 240
aag cgt gac gag cta acg ctc gaa ggt atc aag cag ttc tac gtg aac      768
Lys Arg Asp Glu Leu Thr Leu Glu Gly Ile Lys Gln Phe Tyr Val Asn
                245                 250                 255
gtg gag aaa gaa gag tgg aag ctc gaa act ctc tgt gac ctc tac gag      816
Val Glu Lys Glu Glu Trp Lys Leu Glu Thr Leu Cys Asp Leu Tyr Glu
            260                 265                 270
acc tta gcc atc act cag agc gtc atc ttc gtc aac act cgc cgc aag      864
Thr Leu Ala Ile Thr Gln Ser Val Ile Phe Val Asn Thr Arg Arg Lys
        275                 280                 285
gtg gac tgg ctc aca gac aaa atg aga agc cgt gac cac acg gtc tca      912

Val Asp Trp Leu Thr Asp Lys Met Arg Ser Arg Asp His Thr Val Ser
    290                 295                 300
gcc act cac gga gac atg gac caa aac acg aga gac atc atc atg aga        960
Ala Thr His Gly Asp Met Asp Gln Asn Thr Arg Asp Ile Ile Met Arg
305                 310                 315                 320
gag ttc agg tct ggt tct tct cgt gtt ctc atc aca acc gat ctc ttg       1008
Glu Phe Arg Ser Gly Ser Ser Arg Val Leu Ile Thr Thr Asp Leu Leu
                325                 330                 335
gct cgt ggt atc gat gtg cag caa gtg tct ctg gtc atc aac ttt gac       1056
Ala Arg Gly Ile Asp Val Gln Gln Val Ser Leu Val Ile Asn Phe Asp
            340                 345                 350
ctc cca act cag ccg gag aat tac ctt cac cgt atc gga aga agt gga       1104
Leu Pro Thr Gln Pro Glu Asn Tyr Leu His Arg Ile Gly Arg Ser Gly
        355                 360                 365
agg ttt ggg agg aaa ggt gcg gcg att aac ttt gtg act cgt gat gat       1152
Arg Phe Gly Arg Lys Gly Ala Ala Ile Asn Phe Val Thr Arg Asp Asp
    370                 375                 380
gag aga atg ctt gct gat att cag aaa ttc tac aat gtt gtt gtt gag       1200
Glu Arg Met Leu Ala Asp Ile Gln Lys Phe Tyr Asn Val Val Val Glu
385                 390                 395                 400
gag ctt cca tca aac cta gcc gat ctg ctt tga                           1233
Glu Leu Pro Ser Asn Leu Ala Asp Leu Leu
                405                 410

<210> 112
<211> 410
<212> PRT
<213> Brassica napus

<400> 112
Met Ala Gly Ser Ala Thr Gln Phe Asp Asp Ala Arg Gln Phe Asp Gln
1               5                   10                  15
Ala Leu Ser Glu Ile Leu Glu Gly His Asp Glu Phe Phe Thr Ser Tyr
            20                  25                  30
Asp Glu Val Tyr Glu Ser Phe Asp Gly Met Gly Leu Gln Glu Asn Leu
        35                  40                  45
Leu Arg Gly Ile Tyr Ala Tyr Gly Phe Glu Lys Pro Ser Ala Ile Gln
    50                  55                  60
Gln Arg Gly Ile Val Pro Phe Cys Lys Gly Leu Asp Val Ile Gln Gln
65                  70                  75                  80
Ala Gln Ser Gly Thr Gly Lys Thr Ala Thr Phe Cys Ser Gly Val Leu
                85                  90                  95
Gln Gln Leu Asp Val Thr Leu Val Gln Cys Gln Ala Leu Val Leu Ala
            100                 105                 110
Pro Thr Arg Glu Leu Ala Gln Gln Ile Glu Lys Val Met Arg Ala Leu
        115                 120                 125
Gly Asp Tyr Ile Gly Val Lys Val His Ala Cys Val Gly Gly Thr Ser
    130                 135                 140
Val Leu Asp Asp Lys Arg Ile Leu Gln Ala Gly Val His Val Val Val
145                 150                 155                 160
Gly Thr Pro Gly Arg Val Phe Asp Met Leu Gln Arg Gln Ser Leu Arg
                165                 170                 175
Ser Asp Cys Ile Lys Met Phe Val Leu Asp Glu Ala Asp Glu Met Leu
            180                 185                 190
Ser Arg Gly Phe Lys Asp Gln Ile Tyr Asp Ile Phe Gln Leu Leu Pro
        195                 200                 205
Pro Lys Ile Gln Val Gly Val Phe Ser Ala Thr Met Pro Pro Glu Ala
    210                 215                 220
Leu Glu Ile Thr Arg Lys Phe Met Ser Lys Pro Val Arg Ile Leu Val
225                 230                 235                 240
Lys Arg Asp Glu Leu Thr Leu Glu Gly Ile Lys Gln Phe Tyr Val Asn
                245                 250                 255
Val Glu Lys Glu Glu Trp Lys Leu Glu Thr Leu Cys Asp Leu Tyr Glu
            260                 265                 270
Thr Leu Ala Ile Thr Gln Ser Val Ile Phe Val Asn Thr Arg Arg Lys
        275                 280                 285
Val Asp Trp Leu Thr Asp Lys Met Arg Ser Arg Asp His Thr Val Ser
    290                 295                 300
Ala Thr His Gly Asp Met Asp Gln Asn Thr Arg Asp Ile Ile Met Arg

305                 310                 315                 320
Glu Phe Arg Ser Gly Ser Ser Arg Val Leu Ile Thr Thr Asp Leu Leu
                325                 330                 335
Ala Arg Gly Ile Asp Val Gln Gln Val Ser Leu Val Ile Asn Phe Asp
            340                 345                 350
Leu Pro Thr Gln Pro Glu Asn Tyr Leu His Arg Ile Gly Arg Ser Gly
        355                 360                 365
Arg Phe Gly Arg Lys Gly Ala Ala Ile Asn Phe Val Thr Arg Asp Asp
    370                 375                 380
Glu Arg Met Leu Ala Asp Ile Gln Lys Phe Tyr Asn Val Val Val Glu
385                 390                 395                 400
Glu Leu Pro Ser Asn Leu Ala Asp Leu Leu
                405                 410

<210> 113
<211> 1227
<212> DNA
<213> Brassica napus

<220>
<221> CDS
<222> (1)..(1227)

<400> 113
atg gcg gaa gcg aat cct ggc cgt gga ggt gga aga aga ggc ggc gga      48
Met Ala Glu Ala Asn Pro Gly Arg Gly Gly Gly Arg Arg Gly Gly Gly
1               5                   10                  15
cca atg gac gac gac aaa ttg gtc ttc gag acg acg gaa ggg atc gag      96
Pro Met Asp Asp Asp Lys Leu Val Phe Glu Thr Thr Glu Gly Ile Glu
            20                  25                  30
ccc atc acg aat ttc aac gat atg ggg atc aag gag gac gtt ctc cgc      144
Pro Ile Thr Asn Phe Asn Asp Met Gly Ile Lys Glu Asp Val Leu Arg
        35                  40                  45
ggc gtc tac gag tac ggt ttc gag aag ccc tcc gcg att cag cag aga      192
Gly Val Tyr Glu Tyr Gly Phe Glu Lys Pro Ser Ala Ile Gln Gln Arg
    50                  55                  60
gcg gtc atg ccg att ctc caa ggt cga gat gtg atc gct cag gct cag      240
Ala Val Met Pro Ile Leu Gln Gly Arg Asp Val Ile Ala Gln Ala Gln
65                  70                  75                  80
tcc ggt acc gga aag acc tcc atg att gct ctc tcc gtc tgc caa atc      288
Ser Gly Thr Gly Lys Thr Ser Met Ile Ala Leu Ser Val Cys Gln Ile
                85                  90                  95
gtt gac act tcg tct aga gaa gtt cag gcg ttg ata ttg tcc ccg acg      336
Val Asp Thr Ser Ser Arg Glu Val Gln Ala Leu Ile Leu Ser Pro Thr
            100                 105                 110
aga gag ctg gct tcg cag acg gag aag act att caa gct att ggg ttg      384
Arg Glu Leu Ala Ser Gln Thr Glu Lys Thr Ile Gln Ala Ile Gly Leu
        115                 120                 125
cac gct aat att cag gcg cat gcg tgt att ggt ggg aag agc gtt gga      432
His Ala Asn Ile Gln Ala His Ala Cys Ile Gly Gly Lys Ser Val Gly
    130                 135                 140
gag gat atc agg aag ctg gag aat ggt gtc cat gtt gtg tct ggg acg      480
Glu Asp Ile Arg Lys Leu Glu Asn Gly Val His Val Val Ser Gly Thr
145                 150                 155                 160
cct ggg cgt gta tgt gat atg att aag agg agg agt ctg cgt acc aga      528
Pro Gly Arg Val Cys Asp Met Ile Lys Arg Arg Ser Leu Arg Thr Arg
                165                 170                 175
gct att aag ctt ttg att ctt gat gaa tct gat gag atg ctg agc agg      576
Ala Ile Lys Leu Leu Ile Leu Asp Glu Ser Asp Glu Met Leu Ser Arg
            180                 185                 190
ggg ttt aag gac cag ata tat gat gtt tac aga tat ctt ccg cct gat      624
Gly Phe Lys Asp Gln Ile Tyr Asp Val Tyr Arg Tyr Leu Pro Pro Asp
        195                 200                 205
ctt cag gtt tgt ttg gtt tct gca act ctt cct cac gag att ttg gag      672
Leu Gln Val Cys Leu Val Ser Ala Thr Leu Pro His Glu Ile Leu Glu
    210                 215                 220
atg aca tca aag ttt atg acg gag cca gtg aag ata ctt gtg aag cgt      720
Met Thr Ser Lys Phe Met Thr Glu Pro Val Lys Ile Leu Val Lys Arg
225                 230                 235                 240

gat gag ttg act ctt gaa ggc att aaa caa ttt ttc gtt gct gtt gag 768
Asp Glu Leu Thr Leu Glu Gly Ile Lys Gln Phe Phe Val Ala Val Glu
                245                 250                 255
aaa gag gag tgg aaa ttt gat acg ctc tgt gat ctt tat gac act ctt 816
Lys Glu Glu Trp Lys Phe Asp Thr Leu Cys Asp Leu Tyr Asp Thr Leu
            260                 265                 270
act atc act caa gct gtt atc ttc tgc aac act aaa cga aag gtg gat 864
Thr Ile Thr Gln Ala Val Ile Phe Cys Asn Thr Lys Arg Lys Val Asp
        275                 280                 285
tgg cta agt gag aaa atg agg act aac aac ttc aca gtc tca tca atg 912
Trp Leu Ser Glu Lys Met Arg Thr Asn Asn Phe Thr Val Ser Ser Met
    290                 295                 300
cat ggt gac atg cct cag aag gag aga gac gag atc atg aat cag ttt 960
His Gly Asp Met Pro Gln Lys Glu Arg Asp Glu Ile Met Asn Gln Phe
305                 310                 315                 320
cgg tca ggg gac agt cgt gtt ttg atc acg aca gat gta tgg gca cgt 1008
Arg Ser Gly Asp Ser Arg Val Leu Ile Thr Thr Asp Val Trp Ala Arg
                325                 330                 335
ggg att gat gtg cag caa gtt tct ctt gtg atc aat tat gat ctc ccc 1056
Gly Ile Asp Val Gln Gln Val Ser Leu Val Ile Asn Tyr Asp Leu Pro
            340                 345                 350
aac aac cgt gag ctc tac atc cat cgt att gga cga tct ggt cgt ttc 1104
Asn Asn Arg Glu Leu Tyr Ile His Arg Ile Gly Arg Ser Gly Arg Phe
        355                 360                 365
ggc cgt aag ggt gtt gcg atc aac ttt gtt aaa agc gac gac atc aag 1152
Gly Arg Lys Gly Val Ala Ile Asn Phe Val Lys Ser Asp Asp Ile Lys
    370                 375                 380
atc ctc cgg gac att gag cag tac tac agt act cag att gac gag atg 1200
Ile Leu Arg Asp Ile Glu Gln Tyr Tyr Ser Thr Gln Ile Asp Glu Met
385                 390                 395                 400
cca atg aat gta gct gat ctt atc taa 1227
Pro Met Asn Val Ala Asp Leu Ile
                405

<210> 114
<211> 408
<212> PRT
<213> Brassica napus

<400> 114
Met Ala Glu Ala Asn Pro Gly Arg Gly Gly Gly Arg Arg Gly Gly Gly
1               5                   10                  15
Pro Met Asp Asp Asp Lys Leu Val Phe Glu Thr Thr Glu Gly Ile Glu
            20                  25                  30
Pro Ile Thr Asn Phe Asn Asp Met Gly Ile Lys Glu Asp Val Leu Arg
        35                  40                  45
Gly Val Tyr Glu Tyr Gly Phe Glu Lys Pro Ser Ala Ile Gln Gln Arg
    50                  55                  60
Ala Val Met Pro Ile Leu Gln Gly Arg Asp Val Ile Ala Gln Ala Gln
65                  70                  75                  80
Ser Gly Thr Gly Lys Thr Ser Met Ile Ala Leu Ser Val Cys Gln Ile
                85                  90                  95
Val Asp Thr Ser Ser Arg Glu Val Gln Ala Leu Ile Leu Ser Pro Thr
            100                 105                 110
Arg Glu Leu Ala Ser Gln Thr Glu Lys Thr Ile Gln Ala Ile Gly Leu
        115                 120                 125
His Ala Asn Ile Gln Ala His Ala Cys Ile Gly Gly Lys Ser Val Gly
    130                 135                 140
Glu Asp Ile Arg Lys Leu Glu Asn Gly Val His Val Val Ser Gly Thr
145                 150                 155                 160
Pro Gly Arg Val Cys Asp Met Ile Lys Arg Arg Ser Leu Arg Thr Arg
                165                 170                 175
Ala Ile Lys Leu Leu Ile Leu Asp Glu Ser Asp Glu Met Leu Ser Arg
            180                 185                 190
Gly Phe Lys Asp Gln Ile Tyr Asp Val Tyr Arg Tyr Leu Pro Pro Asp
        195                 200                 205
Leu Gln Val Cys Leu Val Ser Ala Thr Leu Pro His Glu Ile Leu Glu
    210                 215                 220
Met Thr Ser Lys Phe Met Thr Glu Pro Val Lys Ile Leu Val Lys Arg

225                 230                 235                 240
Asp Glu Leu Thr Leu Glu Gly Ile Lys Gln Phe Phe Val Ala Val Glu
                245                 250                 255
Lys Glu Glu Trp Lys Phe Asp Thr Leu Cys Asp Leu Tyr Asp Thr Leu
            260                 265                 270
Thr Ile Thr Gln Ala Val Ile Phe Cys Asn Thr Lys Arg Lys Val Asp
        275                 280                 285
Trp Leu Ser Glu Lys Met Arg Thr Asn Asn Phe Thr Val Ser Ser Met
    290                 295                 300
His Gly Asp Met Pro Gln Lys Glu Arg Asp Glu Ile Met Asn Gln Phe
305                 310                 315                 320
Arg Ser Gly Asp Ser Arg Val Leu Ile Thr Thr Asp Val Trp Ala Arg
                325                 330                 335
Gly Ile Asp Val Gln Gln Val Ser Leu Val Ile Asn Tyr Asp Leu Pro
            340                 345                 350
Asn Asn Arg Glu Leu Tyr Ile His Arg Ile Gly Arg Ser Gly Arg Phe
        355                 360                 365
Gly Arg Lys Gly Val Ala Ile Asn Phe Val Lys Ser Asp Asp Ile Lys
    370                 375                 380
Ile Leu Arg Asp Ile Glu Gln Tyr Tyr Ser Thr Gln Ile Asp Glu Met
385                 390                 395                 400
Pro Met Asn Val Ala Asp Leu Ile
                405

<210> 115
<211> 1245
<212> DNA
<213> Zea mays

<220>
<221> CDS
<222> (1)..(1245)

<400> 115
atg gca gga atg gca ccc gag ggt tct cag ttt gat gct aag cac tat      48
Met Ala Gly Met Ala Pro Glu Gly Ser Gln Phe Asp Ala Lys His Tyr
1               5                   10                  15
gat tct aaa atg cag gag ctg ctg agc act ggt gag act gag gag ttc      96
Asp Ser Lys Met Gln Glu Leu Leu Ser Thr Gly Glu Thr Glu Glu Phe
            20                  25                  30
ttc act tca tat gat gaa gtt ttt gaa agt ttt gat gac atg ggc ctt      144
Phe Thr Ser Tyr Asp Glu Val Phe Glu Ser Phe Asp Asp Met Gly Leu
        35                  40                  45
caa gag aat ctt ctg aga ggc att tat gca tat ggt ttt gag aaa cca      192
Gln Glu Asn Leu Leu Arg Gly Ile Tyr Ala Tyr Gly Phe Glu Lys Pro
    50                  55                  60
tct gca att cag cag aga gga att gtt ccc ttc tgc aag ggg ctt gat      240
Ser Ala Ile Gln Gln Arg Gly Ile Val Pro Phe Cys Lys Gly Leu Asp
65                  70                  75                  80
gtc att cag caa gca caa tct ggt aca gga aag aca gca acc ttc tgt      288
Val Ile Gln Gln Ala Gln Ser Gly Thr Gly Lys Thr Ala Thr Phe Cys
                85                  90                  95
tct ggg atc ttg cag cag ctt gac tat ggt ctg gtt gaa tgc cag gcc      336
Ser Gly Ile Leu Gln Gln Leu Asp Tyr Gly Leu Val Glu Cys Gln Ala
            100                 105                 110
ctg gtc ctt gct ccc acc cgt gag ctt gca cag caa atc gag aaa gtc      384
Leu Val Leu Ala Pro Thr Arg Glu Leu Ala Gln Gln Ile Glu Lys Val
        115                 120                 125
atg cgc gcg ctt ggt gac tac ctt ggt gtt aag gtc cat gca tgt gtg      432
Met Arg Ala Leu Gly Asp Tyr Leu Gly Val Lys Val His Ala Cys Val
    130                 135                 140
gga gga aca tcc gtc cgt gag gac caa agg att ctc gcc agt ggt gtg      480
Gly Gly Thr Ser Val Arg Glu Asp Gln Arg Ile Leu Ala Ser Gly Val
145                 150                 155                 160
cat gtt gtt gtt ggt aca cct ggt cgt gtg ttt gac atg ctg cgt aga      528
His Val Val Val Gly Thr Pro Gly Arg Val Phe Asp Met Leu Arg Arg
                165                 170                 175
cag tcc ctc cgc cct gat aac atc aag atg ttt gtt ctt gat gaa gct      576
Gln Ser Leu Arg Pro Asp Asn Ile Lys Met Phe Val Leu Asp Glu Ala

            180                 185                 190
gat gaa atg ctt tct cgt ggt ttc aag gat cag att tat gac atc ttc 624
Asp Glu Met Leu Ser Arg Gly Phe Lys Asp Gln Ile Tyr Asp Ile Phe
        195                 200                 205
cag ctt ctg cca tcc aag att cag gtt gga gtc ttc tct gct acc atg 672
Gln Leu Leu Pro Ser Lys Ile Gln Val Gly Val Phe Ser Ala Thr Met
    210                 215                 220
cca cct gag gcc ctt gag att acc cgc aag ttc atg aac aaa cca gtg 720
Pro Pro Glu Ala Leu Glu Ile Thr Arg Lys Phe Met Asn Lys Pro Val
225                 230                 235                 240
aga att ctt gtg aag aga gat gag ctg acc ctt gag ggt atc aag cag 768
Arg Ile Leu Val Lys Arg Asp Glu Leu Thr Leu Glu Gly Ile Lys Gln
                245                 250                 255
ttc tat gtg aat gtg gac aag gaa gac tgg aag ctg gac aca ctg tgt 816
Phe Tyr Val Asn Val Asp Lys Glu Asp Trp Lys Leu Asp Thr Leu Cys
            260                 265                 270
gac ctg tat gag acc ctg gcc att acc cag agt gtc atc ttt gtc aac 864
Asp Leu Tyr Glu Thr Leu Ala Ile Thr Gln Ser Val Ile Phe Val Asn
        275                 280                 285
acc cgc cgc aag gtg gac tgg ctc act gac aag atg agg agc agg gac 912
Thr Arg Arg Lys Val Asp Trp Leu Thr Asp Lys Met Arg Ser Arg Asp
    290                 295                 300
cac acc gtt tct gcc act cat ggt gac atg gac cag aac act aga gac 960
His Thr Val Ser Ala Thr His Gly Asp Met Asp Gln Asn Thr Arg Asp
305                 310                 315                 320
atc atc atg agg gag ttc cgg tct ggc tcc tcc cgt gtg ctc atc acc 1008
Ile Ile Met Arg Glu Phe Arg Ser Gly Ser Ser Arg Val Leu Ile Thr
                325                 330                 335
acc gac ctg ctt gct cgt ggt att gat gtt cag cag gtg tcc ctg gtc 1056
Thr Asp Leu Leu Ala Arg Gly Ile Asp Val Gln Gln Val Ser Leu Val
            340                 345                 350
atc aac tat gat ctc ccc acc cag cca gag aat tac ctg cat cgc att 1104
Ile Asn Tyr Asp Leu Pro Thr Gln Pro Glu Asn Tyr Leu His Arg Ile
        355                 360                 365
ggt cgt agt ggt cgt ttc ggt agg aag ggt gtt gcc atc aac ttt gtg 1152
Gly Arg Ser Gly Arg Phe Gly Arg Lys Gly Val Ala Ile Asn Phe Val
    370                 375                 380
acc cgc gac gac gag cgg atg ctg ttc gac atc cag aag ttc tac aac 1200
Thr Arg Asp Asp Glu Arg Met Leu Phe Asp Ile Gln Lys Phe Tyr Asn
385                 390                 395                 400
gtg ttg att gag gag ctg cct gcc aac gtc gct gac ctt ctg tga 1245
Val Leu Ile Glu Glu Leu Pro Ala Asn Val Ala Asp Leu Leu
                405                 410

<210> 116
<211> 414
<212> PRT
<213> Zea mays

<400> 116
Met Ala Gly Met Ala Pro Glu Gly Ser Gln Phe Asp Ala Lys His Tyr
1               5                   10                  15
Asp Ser Lys Met Gln Glu Leu Leu Ser Thr Gly Glu Thr Glu Glu Phe
            20                  25                  30
Phe Thr Ser Tyr Asp Glu Val Phe Glu Ser Phe Asp Asp Met Gly Leu
        35                  40                  45
Gln Glu Asn Leu Leu Arg Gly Ile Tyr Ala Tyr Gly Phe Glu Lys Pro
    50                  55                  60
Ser Ala Ile Gln Gln Arg Gly Ile Val Pro Phe Cys Lys Gly Leu Asp
65                  70                  75                  80
Val Ile Gln Gln Ala Gln Ser Gly Thr Gly Lys Thr Ala Thr Phe Cys
                85                  90                  95
Ser Gly Ile Leu Gln Gln Leu Asp Tyr Gly Leu Val Glu Cys Gln Ala
            100                 105                 110
Leu Val Leu Ala Pro Thr Arg Glu Leu Ala Gln Gln Ile Glu Lys Val
        115                 120                 125
Met Arg Ala Leu Gly Asp Tyr Leu Gly Val Lys Val His Ala Cys Val
    130                 135                 140
Gly Gly Thr Ser Val Arg Glu Asp Gln Arg Ile Leu Ala Ser Gly Val

145                 150                 155                 160
His Val Val Val Gly Thr Pro Gly Arg Val Phe Asp Met Leu Arg Arg
                165                 170                 175
Gln Ser Leu Arg Pro Asp Asn Ile Lys Met Phe Val Leu Asp Glu Ala
            180                 185                 190
Asp Glu Met Leu Ser Arg Gly Phe Lys Asp Gln Ile Tyr Asp Ile Phe
        195                 200                 205
Gln Leu Leu Pro Ser Lys Ile Gln Val Gly Val Phe Ser Ala Thr Met
    210                 215                 220
Pro Pro Glu Ala Leu Glu Ile Thr Arg Lys Phe Met Asn Lys Pro Val
225                 230                 235                 240
Arg Ile Leu Val Lys Arg Asp Glu Leu Thr Leu Glu Gly Ile Lys Gln
                245                 250                 255
Phe Tyr Val Asn Val Asp Lys Glu Asp Trp Lys Leu Asp Thr Leu Cys
            260                 265                 270
Asp Leu Tyr Glu Thr Leu Ala Ile Thr Gln Ser Val Ile Phe Val Asn
        275                 280                 285
Thr Arg Arg Lys Val Asp Trp Leu Thr Asp Lys Met Arg Ser Arg Asp
    290                 295                 300
His Thr Val Ser Ala Thr His Gly Asp Met Asp Gln Asn Thr Arg Asp
305                 310                 315                 320
Ile Ile Met Arg Glu Phe Arg Ser Gly Ser Ser Arg Val Leu Ile Thr
                325                 330                 335
Thr Asp Leu Leu Ala Arg Gly Ile Asp Val Gln Gln Val Ser Leu Val
            340                 345                 350
Ile Asn Tyr Asp Leu Pro Thr Gln Pro Glu Asn Tyr Leu His Arg Ile
        355                 360                 365
Gly Arg Ser Gly Arg Phe Gly Arg Lys Gly Val Ala Ile Asn Phe Val
    370                 375                 380
Thr Arg Asp Asp Glu Arg Met Leu Phe Asp Ile Gln Lys Phe Tyr Asn
385                 390                 395                 400
Val Leu Ile Glu Glu Leu Pro Ala Asn Val Ala Asp Leu Leu
                405                 410

<210> 117
<211> 1224
<212> DNA
<213> Zea mays

<220>
<221> CDS
<222> (1)..(1224)

<400> 117
atg gcg gcg ccc acc acc tcc cgc cgc ggc ccc ggc ggc gcg cgc aac      48
Met Ala Ala Pro Thr Thr Ser Arg Arg Gly Pro Gly Gly Ala Arg Asn
1               5                   10                  15
atg gac gac gag aac ctc acc ttc gag acc tcc ccg ggg gtc gag gtc      96
Met Asp Asp Glu Asn Leu Thr Phe Glu Thr Ser Pro Gly Val Glu Val
            20                  25                  30
gtc agc agc ttc gac cag atg ggc atc aag gac gac ctc ctc cgc ggc      144
Val Ser Ser Phe Asp Gln Met Gly Ile Lys Asp Asp Leu Leu Arg Gly
        35                  40                  45
atc tac ggc tac ggt ttc gag aag ccc tcc gcc atc cag cag cgc gcc      192
Ile Tyr Gly Tyr Gly Phe Glu Lys Pro Ser Ala Ile Gln Gln Arg Ala
    50                  55                  60
gtg ctc ccc atc atc aac ggc cgc gac gtc atc gcg cag gcc cag tcc      240
Val Leu Pro Ile Ile Asn Gly Arg Asp Val Ile Ala Gln Ala Gln Ser
65                  70                  75                  80
ggc acc ggc aag acg tcc atg atc tcc ctc acc gtc tgc cag atc gtc      288
Gly Thr Gly Lys Thr Ser Met Ile Ser Leu Thr Val Cys Gln Ile Val
                85                  90                  95
gac acc gcc gtg cgc gag gtg cag gct ttg att ctc tcg cct act agg      336
Asp Thr Ala Val Arg Glu Val Gln Ala Leu Ile Leu Ser Pro Thr Arg
            100                 105                 110
gag ctt gct tcc caa aca gag aga gtt atg ctg gct att ggg gac cac      384
Glu Leu Ala Ser Gln Thr Glu Arg Val Met Leu Ala Ile Gly Asp His
        115                 120                 125
ctc aac gtc caa gta cat gct tgc att ggt gga aaa agt atc ggt gag      432

Leu Asn Val Gln Val His Ala Cys Ile Gly Gly Lys Ser Ile Gly Glu
    130                 135                 140
gat atc agg agg ctc gag aat ggg gtg cat gtt gtc tcg gga act cca      480
Asp Ile Arg Arg Leu Glu Asn Gly Val His Val Val Ser Gly Thr Pro
145                 150                 155                 160
ggc aga gtc tgt gat atg atc aag agg agg acc ttg cga aca aga gcc      528
Gly Arg Val Cys Asp Met Ile Lys Arg Arg Thr Leu Arg Thr Arg Ala
                165                 170                 175
atc aag ctt cta gtt ctg gat gaa gct gat gag atg ttg agt aga ggc      576
Ile Lys Leu Leu Val Leu Asp Glu Ala Asp Glu Met Leu Ser Arg Gly
            180                 185                 190
ttt aag gat cag att tat gat gtc tac aga tat ctc ccg cca gaa ctt      624
Phe Lys Asp Gln Ile Tyr Asp Val Tyr Arg Tyr Leu Pro Pro Glu Leu
        195                 200                 205
cag gtg gtt ttg atc tct gca act ctt cct cat gag atc ttg gag atg      672
Gln Val Val Leu Ile Ser Ala Thr Leu Pro His Glu Ile Leu Glu Met
    210                 215                 220
act agt aag ttc atg acc gaa ccg gtt agg atc ctt gtg aag cgt gat      720
Thr Ser Lys Phe Met Thr Glu Pro Val Arg Ile Leu Val Lys Arg Asp
225                 230                 235                 240
gag ctg acc ctg gag ggc atc aaa caa ttc ttt gtt gct gtt gag aaa      768
Glu Leu Thr Leu Glu Gly Ile Lys Gln Phe Phe Val Ala Val Glu Lys
                245                 250                 255
gag gag tgg aag ttt gat aca ctt tgt gat ctt tat gat aca ctc acc      816
Glu Glu Trp Lys Phe Asp Thr Leu Cys Asp Leu Tyr Asp Thr Leu Thr
            260                 265                 270
atc acc caa gct gtt att ttc tgc aat act aag aga aag gtg gat tgg      864
Ile Thr Gln Ala Val Ile Phe Cys Asn Thr Lys Arg Lys Val Asp Trp
        275                 280                 285
ctc act gaa aaa atg cgt aca aat aac ttc acc gta tca gct atg cac      912
Leu Thr Glu Lys Met Arg Thr Asn Asn Phe Thr Val Ser Ala Met His
    290                 295                 300
ggt gac atg ccc caa caa gaa agg gat gcc att atg ggt gag ttc agg      960
Gly Asp Met Pro Gln Gln Glu Arg Asp Ala Ile Met Gly Glu Phe Arg
305                 310                 315                 320
tct ggc gca act cgt gtg cta ata act aca gat gtt tgg gct cga gga      1008
Ser Gly Ala Thr Arg Val Leu Ile Thr Thr Asp Val Trp Ala Arg Gly
                325                 330                 335
ctg gac gtt cag cag gtt tca ctt gtc ata aat tat gat ctc cca aat      1056
Leu Asp Val Gln Gln Val Ser Leu Val Ile Asn Tyr Asp Leu Pro Asn
            340                 345                 350
aat cgt gag ctt tac atc cat cgc att ggt cgt tct ggt cgt ttt ggg      1104
Asn Arg Glu Leu Tyr Ile His Arg Ile Gly Arg Ser Gly Arg Phe Gly
        355                 360                 365
cgc aag ggt gtg gcg atc aat ttc gtg cgc aag gat gac atc cgt ata      1152
Arg Lys Gly Val Ala Ile Asn Phe Val Arg Lys Asp Asp Ile Arg Ile
    370                 375                 380
ctg aga gat ata gag cag tac tac agc aca caa att gac gag atg cca      1200
Leu Arg Asp Ile Glu Gln Tyr Tyr Ser Thr Gln Ile Asp Glu Met Pro
385                 390                 395                 400
atg aat gtt gct gat ctt att tga                                      1224
Met Asn Val Ala Asp Leu Ile
                405

<210> 118
<211> 407
<212> PRT
<213> Zea mays

<400> 118
Met Ala Ala Pro Thr Thr Ser Arg Arg Gly Pro Gly Gly Ala Arg Asn
1               5                   10                  15
Met Asp Asp Glu Asn Leu Thr Phe Glu Thr Ser Pro Gly Val Glu Val
            20                  25                  30
Val Ser Ser Phe Asp Gln Met Gly Ile Lys Asp Asp Leu Leu Arg Gly
        35                  40                  45
Ile Tyr Gly Tyr Gly Phe Glu Lys Pro Ser Ala Ile Gln Gln Arg Ala
    50                  55                  60
Val Leu Pro Ile Ile Asn Gly Arg Asp Val Ile Ala Gln Ala Gln Ser

65                  70                  75                  80
Gly Thr Gly Lys Thr Ser Met Ile Ser Leu Thr Val Cys Gln Ile Val
                85                  90                  95
Asp Thr Ala Val Arg Glu Val Gln Ala Leu Ile Leu Ser Pro Thr Arg
            100                 105                 110
Glu Leu Ala Ser Gln Thr Glu Arg Val Met Leu Ala Ile Gly Asp His
        115                 120                 125
Leu Asn Val Gln Val His Ala Cys Ile Gly Gly Lys Ser Ile Gly Glu
    130                 135                 140
Asp Ile Arg Arg Leu Glu Asn Gly Val His Val Val Ser Gly Thr Pro
145                 150                 155                 160
Gly Arg Val Cys Asp Met Ile Lys Arg Arg Thr Leu Arg Thr Arg Ala
                165                 170                 175
Ile Lys Leu Leu Val Leu Asp Glu Ala Asp Glu Met Leu Ser Arg Gly
            180                 185                 190
Phe Lys Asp Gln Ile Tyr Asp Val Tyr Arg Tyr Leu Pro Pro Glu Leu
        195                 200                 205
Gln Val Val Leu Ile Ser Ala Thr Leu Pro His Glu Ile Leu Glu Met
    210                 215                 220
Thr Ser Lys Phe Met Thr Glu Pro Val Arg Ile Leu Val Lys Arg Asp
225                 230                 235                 240
Glu Leu Thr Leu Glu Gly Ile Lys Gln Phe Phe Val Ala Val Glu Lys
                245                 250                 255
Glu Glu Trp Lys Phe Asp Thr Leu Cys Asp Leu Tyr Asp Thr Leu Thr
            260                 265                 270
Ile Thr Gln Ala Val Ile Phe Cys Asn Thr Lys Arg Lys Val Asp Trp
        275                 280                 285
Leu Thr Glu Lys Met Arg Thr Asn Asn Phe Thr Val Ser Ala Met His
    290                 295                 300
Gly Asp Met Pro Gln Gln Glu Arg Asp Ala Ile Met Gly Glu Phe Arg
305                 310                 315                 320
Ser Gly Ala Thr Arg Val Leu Ile Thr Thr Asp Val Trp Ala Arg Gly
                325                 330                 335
Leu Asp Val Gln Gln Val Ser Leu Val Ile Asn Tyr Asp Leu Pro Asn
            340                 345                 350
Asn Arg Glu Leu Tyr Ile His Arg Ile Gly Arg Ser Gly Arg Phe Gly
        355                 360                 365
Arg Lys Gly Val Ala Ile Asn Phe Val Arg Lys Asp Asp Ile Arg Ile
    370                 375                 380
Leu Arg Asp Ile Glu Gln Tyr Tyr Ser Thr Gln Ile Asp Glu Met Pro
385                 390                 395                 400
Met Asn Val Ala Asp Leu Ile
                405

<210> 119
<211> 1500
<212> DNA
<213> Zea mays

<220>
<221> CDS
<222> (1)..(1500)

<400> 119
atg cac cag cct aga gct cgc tac cca ccc ggc tat ggc tcc ggc ggt      48
Met His Gln Pro Arg Ala Arg Tyr Pro Pro Gly Tyr Gly Ser Gly Gly
1               5                   10                  15
gga ggc gga gcc gga cgt ggc ggt gga ggc gga aat gga gga ggt ggc      96
Gly Gly Gly Ala Gly Arg Gly Gly Gly Gly Gly Asn Gly Gly Gly Gly
            20                  25                  30
gga aac cac aac tac tac ggc cga aac ccg cag caa cac cac tac cac      144
Gly Asn His Asn Tyr Tyr Gly Arg Asn Pro Gln Gln His His Tyr His
        35                  40                  45
cag cag cag cag tcg cag cac tct cat agg aac tct tcg tac cag caa      192
Gln Gln Gln Gln Ser Gln His Ser His Arg Asn Ser Ser Tyr Gln Gln
    50                  55                  60
cag tgg ctg cgg cga gac cag gcc ccc gcc gtt gcg ggg gca gcg tcc      240
Gln Trp Leu Arg Arg Asp Gln Ala Pro Ala Val Ala Gly Ala Ala Ser
65                  70                  75                  80

gga aac gct gtg gcc aag acg gcg tct cag ctt gac gcc gta ggc tcg 288
Gly Asn Ala Val Ala Lys Thr Ala Ser Gln Leu Asp Ala Val Gly Ser
                85                  90                  95
agc tcc caa gac tgg aag gct caa cta aat ata cca gct cca gat aca 336
Ser Ser Gln Asp Trp Lys Ala Gln Leu Asn Ile Pro Ala Pro Asp Thr
            100                 105                 110
cgc tac agg aca gag gat gtc act gca aca aaa ggc aat gag ttt gaa 384
Arg Tyr Arg Thr Glu Asp Val Thr Ala Thr Lys Gly Asn Glu Phe Glu
        115                 120                 125
gat tat ttc ttg aaa cgt gaa ctg ctt atg gga atc tac gaa aag gga 432
Asp Tyr Phe Leu Lys Arg Glu Leu Leu Met Gly Ile Tyr Glu Lys Gly
    130                 135                 140
ttt gaa aga cca tct cct att caa gaa gaa agc att cct att gcg ctg 480
Phe Glu Arg Pro Ser Pro Ile Gln Glu Glu Ser Ile Pro Ile Ala Leu
145                 150                 155                 160
act gga agt gat att ctt gca agg gca aaa aat ggc act gga aag act 528
Thr Gly Ser Asp Ile Leu Ala Arg Ala Lys Asn Gly Thr Gly Lys Thr
                165                 170                 175
gct gcc ttt tgc att ccg gca ctt gaa aaa att gat cct gaa aat aat 576
Ala Ala Phe Cys Ile Pro Ala Leu Glu Lys Ile Asp Pro Glu Asn Asn
            180                 185                 190
gca att caa gtt gtt ata cta gta cca aca agg gaa ctg gct ctg caa 624
Ala Ile Gln Val Val Ile Leu Val Pro Thr Arg Glu Leu Ala Leu Gln
        195                 200                 205
act tca caa gtg tgt aaa gag ctt ggg aaa tac cta aac att gaa gtt 672
Thr Ser Gln Val Cys Lys Glu Leu Gly Lys Tyr Leu Asn Ile Glu Val
    210                 215                 220
atg gtt agc act gga gga acc agc ttg aag gat gac atc atg cgt tta 720
Met Val Ser Thr Gly Gly Thr Ser Leu Lys Asp Asp Ile Met Arg Leu
225                 230                 235                 240
tac caa cct gtt cat cta ctc gtt ggg act cct ggc cga ata ctt gat 768
Tyr Gln Pro Val His Leu Leu Val Gly Thr Pro Gly Arg Ile Leu Asp
                245                 250                 255
ctt acc agg aag ggc att tgt gtg ctg aaa gat tgt tca atg ctt gtc 816
Leu Thr Arg Lys Gly Ile Cys Val Leu Lys Asp Cys Ser Met Leu Val
            260                 265                 270
atg gat gag gca gac aag cta tta gct cca gag ttc cag cct tct gtg 864
Met Asp Glu Ala Asp Lys Leu Leu Ala Pro Glu Phe Gln Pro Ser Val
        275                 280                 285
gag gca ctt att cat ttc ctt cca cct agt cgg cag ctg ttg atg ttt 912
Glu Ala Leu Ile His Phe Leu Pro Pro Ser Arg Gln Leu Leu Met Phe
    290                 295                 300
tca gca acc ttt cct gta act gtc aag gaa ttc aaa gag aaa tat ctg 960
Ser Ala Thr Phe Pro Val Thr Val Lys Glu Phe Lys Glu Lys Tyr Leu
305                 310                 315                 320
cct aga cct tat gtg att aat cta atg gac gaa ctg acc ctg aaa gga 1008
Pro Arg Pro Tyr Val Ile Asn Leu Met Asp Glu Leu Thr Leu Lys Gly
                325                 330                 335
atc aca caa tat tat gct ttt gtt gaa gaa agg cag aaa gtt cat tgc 1056
Ile Thr Gln Tyr Tyr Ala Phe Val Glu Glu Arg Gln Lys Val His Cys
            340                 345                 350
ctg aat aca ctt ttc tca aag ctt caa att aat caa tcc att ata ttc 1104
Leu Asn Thr Leu Phe Ser Lys Leu Gln Ile Asn Gln Ser Ile Ile Phe
        355                 360                 365
tgc aac tct gtt aat aga gtt gag ctg ctg gct aaa aaa ata act gaa 1152
Cys Asn Ser Val Asn Arg Val Glu Leu Leu Ala Lys Lys Ile Thr Glu
    370                 375                 380
ctc ggt tat tca tgc ttc tac att cat gct aag atg ttg caa gac cac 1200
Leu Gly Tyr Ser Cys Phe Tyr Ile His Ala Lys Met Leu Gln Asp His
385                 390                 395                 400
aga aat cga gta ttt cat gat ttt cgt aat ggt gct tgc aga aac ctt 1248
Arg Asn Arg Val Phe His Asp Phe Arg Asn Gly Ala Cys Arg Asn Leu
                405                 410                 415
gtg tgc aca gat ctg ttt acc aga gga att gat atc cag gct gtt aat 1296
Val Cys Thr Asp Leu Phe Thr Arg Gly Ile Asp Ile Gln Ala Val Asn
            420                 425                 430
gtg gtc att aat ttt gac ttc cct aag aca tct gag aca tac tta cac 1344
Val Val Ile Asn Phe Asp Phe Pro Lys Thr Ser Glu Thr Tyr Leu His
        435                 440                 445

agg gtt ggc cgt tct ggg aga tat gga cac ctt ggt ttg gca gtg aac 1392
Arg Val Gly Arg Ser Gly Arg Tyr Gly His Leu Gly Leu Ala Val Asn
    450                 455                 460
ttg atc act tat gaa gac cgt ttc aac tta tat agg att gag caa gaa 1440
Leu Ile Thr Tyr Glu Asp Arg Phe Asn Leu Tyr Arg Ile Glu Gln Glu
465                 470                 475                 480
ctt gga acg gaa att aag aca ata ccc cca cag att gac ctg tcc ata 1488
Leu Gly Thr Glu Ile Lys Thr Ile Pro Pro Gln Ile Asp Leu Ser Ile
                485                 490                 495
tac tgc caa tga 1500
Tyr Cys Gln

<210> 120
<211> 499
<212> PRT
<213> Zea mays

<400> 120
Met His Gln Pro Arg Ala Arg Tyr Pro Pro Gly Tyr Gly Ser Gly Gly
1               5                   10                  15
Gly Gly Gly Ala Gly Arg Gly Gly Gly Gly Gly Asn Gly Gly Gly Gly
            20                  25                  30
Gly Asn His Asn Tyr Tyr Gly Arg Asn Pro Gln Gln His His Tyr His
        35                  40                  45
Gln Gln Gln Gln Ser Gln His Ser His Arg Asn Ser Ser Tyr Gln Gln
    50                  55                  60
Gln Trp Leu Arg Arg Asp Gln Ala Pro Ala Val Ala Gly Ala Ala Ser
65                  70                  75                  80
Gly Asn Ala Val Ala Lys Thr Ala Ser Gln Leu Asp Ala Val Gly Ser
                85                  90                  95
Ser Ser Gln Asp Trp Lys Ala Gln Leu Asn Ile Pro Ala Pro Asp Thr
            100                 105                 110
Arg Tyr Arg Thr Glu Asp Val Thr Ala Thr Lys Gly Asn Glu Phe Glu
        115                 120                 125
Asp Tyr Phe Leu Lys Arg Glu Leu Leu Met Gly Ile Tyr Glu Lys Gly
    130                 135                 140
Phe Glu Arg Pro Ser Pro Ile Gln Glu Glu Ser Ile Pro Ile Ala Leu
145                 150                 155                 160
Thr Gly Ser Asp Ile Leu Ala Arg Ala Lys Asn Gly Thr Gly Lys Thr
                165                 170                 175
Ala Ala Phe Cys Ile Pro Ala Leu Glu Lys Ile Asp Pro Glu Asn Asn
            180                 185                 190
Ala Ile Gln Val Val Ile Leu Val Pro Thr Arg Glu Leu Ala Leu Gln
        195                 200                 205
Thr Ser Gln Val Cys Lys Glu Leu Gly Lys Tyr Leu Asn Ile Glu Val
    210                 215                 220
Met Val Ser Thr Gly Gly Thr Ser Leu Lys Asp Asp Ile Met Arg Leu
225                 230                 235                 240
Tyr Gln Pro Val His Leu Leu Val Gly Thr Pro Gly Arg Ile Leu Asp
                245                 250                 255
Leu Thr Arg Lys Gly Ile Cys Val Leu Lys Asp Cys Ser Met Leu Val
            260                 265                 270
Met Asp Glu Ala Asp Lys Leu Leu Ala Pro Glu Phe Gln Pro Ser Val
        275                 280                 285
Glu Ala Leu Ile His Phe Leu Pro Pro Ser Arg Gln Leu Leu Met Phe
    290                 295                 300
Ser Ala Thr Phe Pro Val Thr Val Lys Glu Phe Lys Glu Lys Tyr Leu
305                 310                 315                 320
Pro Arg Pro Tyr Val Ile Asn Leu Met Asp Glu Leu Thr Leu Lys Gly
                325                 330                 335
Ile Thr Gln Tyr Tyr Ala Phe Val Glu Glu Arg Gln Lys Val His Cys
            340                 345                 350
Leu Asn Thr Leu Phe Ser Lys Leu Gln Ile Asn Gln Ser Ile Ile Phe
        355                 360                 365
Cys Asn Ser Val Asn Arg Val Glu Leu Leu Ala Lys Lys Ile Thr Glu
    370                 375                 380
Leu Gly Tyr Ser Cys Phe Tyr Ile His Ala Lys Met Leu Gln Asp His
385                 390                 395                 400

Arg Asn Arg Val Phe His Asp Phe Arg Asn Gly Ala Cys Arg Asn Leu
                405                 410                 415
Val Cys Thr Asp Leu Phe Thr Arg Gly Ile Asp Ile Gln Ala Val Asn
            420                 425                 430
Val Val Ile Asn Phe Asp Phe Pro Lys Thr Ser Glu Thr Tyr Leu His
        435                 440                 445
Arg Val Gly Arg Ser Gly Arg Tyr Gly His Leu Gly Leu Ala Val Asn
    450                 455                 460
Leu Ile Thr Tyr Glu Asp Arg Phe Asn Leu Tyr Arg Ile Glu Gln Glu
465                 470                 475                 480
Leu Gly Thr Glu Ile Lys Thr Ile Pro Pro Gln Ile Asp Leu Ser Ile
                485                 490                 495
Tyr Cys Gln


<210> 121
<211> 1578
<212> DNA
<213> Zea mays

<220>
<221> CDS
<222> (1)..(1578)

<400> 121
atg gat cca cgc gcc agg tat cct tcg ggc atg ggc aac ggc cgc ggt      48
Met Asp Pro Arg Ala Arg Tyr Pro Ser Gly Met Gly Asn Gly Arg Gly
1               5                   10                  15
ggg aac ctc aac tac tac ggc cgc ggc cca ccg ccg caa cag ccg cat      96
Gly Asn Leu Asn Tyr Tyr Gly Arg Gly Pro Pro Pro Gln Gln Pro His
            20                  25                  30
cag cac cac aac cac cag cag acg tcg ggg gcg cac cac cac cag cag      144
Gln His His Asn His Gln Gln Thr Ser Gly Ala His His His Gln Gln
        35                  40                  45
acg tcg ggg gcg cac cac cac cag cag tac gcg cag agg cag cac cag      192
Thr Ser Gly Ala His His His Gln Gln Tyr Ala Gln Arg Gln His Gln
    50                  55                  60
cac cag cac cac ggc cac aat cgc cat cag caa cag cag cag cac cac      240
His Gln His His Gly His Asn Arg His Gln Gln Gln Gln His His
65                  70                  75                  80
att tac cag cag caa cag cag cag tgg ctg cgg cgg agc cag att gcc      288
Ile Tyr Gln Gln Gln Gln Gln Gln Trp Leu Arg Arg Ser Gln Ile Ala
                85                  90                  95
cgc gag acc tcg ggc gca gcc gtg acc agc gag ccg aag tcc ctg gcg      336
Arg Glu Thr Ser Gly Ala Ala Val Thr Ser Glu Pro Lys Ser Leu Ala
            100                 105                 110
cca tcc acg gcc gcc gat ggc gtc gat tcg agt tca caa gat tgg aag      384
Pro Ser Thr Ala Ala Asp Gly Val Asp Ser Ser Ser Gln Asp Trp Lys
        115                 120                 125
gcc caa ttg aag ctg ccg cct cca gat aca cgc tac agg aca gag gat      432
Ala Gln Leu Lys Leu Pro Pro Pro Asp Thr Arg Tyr Arg Thr Glu Asp
    130                 135                 140
gtt aca gct acc aag gga aat gag ttt gaa gac tat ttt ctc aag cgt      480
Val Thr Ala Thr Lys Gly Asn Glu Phe Glu Asp Tyr Phe Leu Lys Arg
145                 150                 155                 160
gaa ctc cta atg ggt ata tac gag aag gga ttt gaa aga ccg tct cct      528
Glu Leu Leu Met Gly Ile Tyr Glu Lys Gly Phe Glu Arg Pro Ser Pro
                165                 170                 175
atc caa gag gaa agc att cct att gct tta act ggt agt gac att ctt      576
Ile Gln Glu Glu Ser Ile Pro Ile Ala Leu Thr Gly Ser Asp Ile Leu
            180                 185                 190
gca aga gcg aag aac gga act ggc aag act gct gca ttt tgt att cct      624
Ala Arg Ala Lys Asn Gly Thr Gly Lys Thr Ala Ala Phe Cys Ile Pro
        195                 200                 205
gca ctg gaa aag att gat caa gaa aaa aat gct att caa gtt gtt ata      672
Ala Leu Glu Lys Ile Asp Gln Glu Lys Asn Ala Ile Gln Val Val Ile
    210                 215                 220
ttg gtt ccc aca agg gaa ttg gct ctg caa aca tca caa gtt tgc aag      720
Leu Val Pro Thr Arg Glu Leu Ala Leu Gln Thr Ser Gln Val Cys Lys

225                 230                 235                 240
gag ctt ggt aag cat ttg aag atc cag gtt atg gtc acc act ggt gga      768
Glu Leu Gly Lys His Leu Lys Ile Gln Val Met Val Thr Thr Gly Gly
                245                 250                 255
acc agt ttg aag gat gat att gtt cgt ctg tac caa cct gtg cac tta      816
Thr Ser Leu Lys Asp Asp Ile Val Arg Leu Tyr Gln Pro Val His Leu
            260                 265                 270
ctc gtt gga aca cct ggg cgt att ttg gat ctt aca aag aaa ggt gtt      864
Leu Val Gly Thr Pro Gly Arg Ile Leu Asp Leu Thr Lys Lys Gly Val
        275                 280                 285
tgc att ttg aac gac tgt tca atg ctt att atg gat gag gca gac aag      912
Cys Ile Leu Asn Asp Cys Ser Met Leu Ile Met Asp Glu Ala Asp Lys
    290                 295                 300
ctt ctc tct cct gag ttt caa cct tcc ata gaa cag ttg att cgc tac      960
Leu Leu Ser Pro Glu Phe Gln Pro Ser Ile Glu Gln Leu Ile Arg Tyr
305                 310                 315                 320
ctc cct gct agt cga cag att ttg atg ttt tca gcg aca ttc cct gtg      1008
Leu Pro Ala Ser Arg Gln Ile Leu Met Phe Ser Ala Thr Phe Pro Val
                325                 330                 335
act gtt aag gag ttt aag gac aaa tat ctg cct aaa cct tat gtg att      1056
Thr Val Lys Glu Phe Lys Asp Lys Tyr Leu Pro Lys Pro Tyr Val Ile
            340                 345                 350
aac ctt atg gac gaa ctt aca ctg aag gga att aca cag ttc tat gct      1104
Asn Leu Met Asp Glu Leu Thr Leu Lys Gly Ile Thr Gln Phe Tyr Ala
        355                 360                 365
ttt gtg gaa gaa aga cag aaa gtt cac tgt ctc aac act ctc ttt tcc      1152
Phe Val Glu Glu Arg Gln Lys Val His Cys Leu Asn Thr Leu Phe Ser
    370                 375                 380
aag ctt caa atc aac cag tca ata ata ttt tgt aac tct gta aat cgt      1200
Lys Leu Gln Ile Asn Gln Ser Ile Ile Phe Cys Asn Ser Val Asn Arg
385                 390                 395                 400
gtt gaa cta ttg gcg aaa aag atc aca gag ctt ggc tat tct tgc ttc      1248
Val Glu Leu Leu Ala Lys Lys Ile Thr Glu Leu Gly Tyr Ser Cys Phe
                405                 410                 415
tat att cat gca aag atg cta cag gac cac cgt aat aga gtt ttc cat      1296
Tyr Ile His Ala Lys Met Leu Gln Asp His Arg Asn Arg Val Phe His
            420                 425                 430
gat ttc cgt aat gga gca tgc agg aac ctt gtt tgc act gat ctt ttt      1344
Asp Phe Arg Asn Gly Ala Cys Arg Asn Leu Val Cys Thr Asp Leu Phe
        435                 440                 445
aca agg gga ata gat att caa gct gtg aat gtt gtt atc aat ttt gat      1392
Thr Arg Gly Ile Asp Ile Gln Ala Val Asn Val Val Ile Asn Phe Asp
    450                 455                 460
ttc ccc aag aac tct gaa aca tat ctc cac agg gtt ggt aga tct ggg      1440
Phe Pro Lys Asn Ser Glu Thr Tyr Leu His Arg Val Gly Arg Ser Gly
465                 470                 475                 480
aga ttt ggt cac ctt ggt ctg gcc gtt aat ttg atc acc tat gag gat      1488
Arg Phe Gly His Leu Gly Leu Ala Val Asn Leu Ile Thr Tyr Glu Asp
                485                 490                 495
cgg ttt aac ttg tat agg att gaa caa gag ctt ggg act gaa ata aaa      1536
Arg Phe Asn Leu Tyr Arg Ile Glu Gln Glu Leu Gly Thr Glu Ile Lys
            500                 505                 510
tca att cct cct cag ata gac cag gct ata tac tgc caa taa              1578
Ser Ile Pro Pro Gln Ile Asp Gln Ala Ile Tyr Cys Gln
        515                 520                 525

<210> 122
<211> 525
<212> PRT
<213> Zea mays

<400> 122
   Met Asp Pro Arg Ala Arg Tyr Pro Ser Gly Met Gly Asn Gly Arg Gly
   1               5                   10                  15
   Gly Asn Leu Asn Tyr Tyr Gly Arg Gly Pro Pro Pro Gln Gln Pro His
               20                  25                  30
   Gln His His Asn His Gln Gln Thr Ser Gly Ala His His His Gln Gln
           35                  40                  45
   Thr Ser Gly Ala His His His Gln Gln Tyr Ala Gln Arg Gln His Gln
   50                  55                  60
   His Gln His His Gly His Asn Arg His Gln Gln Gln Gln Gln His His
   65                  70                  75                  80
   Ile Tyr Gln Gln Gln Gln Gln Gln Trp Leu Arg Arg Ser Gln Ile Ala
                   85                  90                  95
   Arg Glu Thr Ser Gly Ala Ala Val Thr Ser Glu Pro Lys Ser Leu Ala
               100                 105                 110
   Pro Ser Thr Ala Ala Asp Gly Val Asp Ser Ser Ser Gln Asp Trp Lys
           115                 120                 125
   Ala Gln Leu Lys Leu Pro Pro Pro Asp Thr Arg Tyr Arg Thr Glu Asp
       130                 135                 140
   Val Thr Ala Thr Lys Gly Asn Glu Phe Glu Asp Tyr Phe Leu Lys Arg
   145                 150                 155                 160
   Glu Leu Leu Met Gly Ile Tyr Glu Lys Gly Phe Glu Arg Pro Ser Pro
                   165                 170                 175
   Ile Gln Glu Glu Ser Ile Pro Ile Ala Leu Thr Gly Ser Asp Ile Leu
               180                 185                 190
   Ala Arg Ala Lys Asn Gly Thr Gly Lys Thr Ala Ala Phe Cys Ile Pro
           195                 200                 205
   Ala Leu Glu Lys Ile Asp Gln Glu Lys Asn Ala Ile Gln Val Val Ile
       210                 215                 220
   Leu Val Pro Thr Arg Glu Leu Ala Leu Gln Thr Ser Gln Val Cys Lys
   225                 230                 235                 240
   Glu Leu Gly Lys His Leu Lys Ile Gln Val Met Val Thr Thr Gly Gly
                   245                 250                 255
   Thr Ser Leu Lys Asp Asp Ile Val Arg Leu Tyr Gln Pro Val His Leu
               260                 265                 270
   Leu Val Gly Thr Pro Gly Arg Ile Leu Asp Leu Thr Lys Lys Gly Val
           275                 280                 285
   Cys Ile Leu Asn Asp Cys Ser Met Leu Ile Met Asp Glu Ala Asp Lys
       290                 295                 300
   Leu Leu Ser Pro Glu Phe Gln Pro Ser Ile Glu Gln Leu Ile Arg Tyr
   305                 310                 315                 320
   Leu Pro Ala Ser Arg Gln Ile Leu Met Phe Ser Ala Thr Phe Pro Val
                   325                 330                 335
   Thr Val Lys Glu Phe Lys Asp Lys Tyr Leu Pro Lys Pro Tyr Val Ile
               340                 345                 350
   Asn Leu Met Asp Glu Leu Thr Leu Lys Gly Ile Thr Gln Phe Tyr Ala
           355                 360                 365
   Phe Val Glu Glu Arg Gln Lys Val His Cys Leu Asn Thr Leu Phe Ser
       370                 375                 380
   Lys Leu Gln Ile Asn Gln Ser Ile Ile Phe Cys Asn Ser Val Asn Arg
   385                 390                 395                 400
   Val Glu Leu Leu Ala Lys Lys Ile Thr Glu Leu Gly Tyr Ser Cys Phe
                   405                 410                 415
   Tyr Ile His Ala Lys Met Leu Gln Asp His Arg Asn Arg Val Phe His
               420                 425                 430
   Asp Phe Arg Asn Gly Ala Cys Arg Asn Leu Val Cys Thr Asp Leu Phe
           435                 440                 445
   Thr Arg Gly Ile Asp Ile Gln Ala Val Asn Val Val Ile Asn Phe Asp
       450                 455                 460
   Phe Pro Lys Asn Ser Glu Thr Tyr Leu His Arg Val Gly Arg Ser Gly
   465                 470                 475                 480
   Arg Phe Gly His Leu Gly Leu Ala Val Asn Leu Ile Thr Tyr Glu Asp
                   485                 490                 495
   Arg Phe Asn Leu Tyr Arg Ile Glu Gln Glu Leu Gly Thr Glu Ile Lys
               500                 505                 510
   Ser Ile Pro Pro Gln Ile Asp Gln Ala Ile Tyr Cys Gln
           515                 520                 525

<210> 123
<211> 2301
<212> DNA
<213> Zea mays

<220>
<221> CDS
<222> (1)..(2301)

<400> 123
atg tcg aag cgg ccg aag ctc gag ggt ttc agc atc ccg cgg ccc acc 48
Met Ser Lys Arg Pro Lys Leu Glu Gly Phe Ser Ile Pro Arg Pro Thr
1               5                   10                  15
tcc tac aac ttc gag cgc tcg cag tcc gtg cag cgg ctc tac cgt ccg 96
Ser Tyr Asn Phe Glu Arg Ser Gln Ser Val Gln Arg Leu Tyr Arg Pro
            20                  25                  30
acc gat gac cca gac ctc gac gac atc gcc ttc tcc gac gac gcc ccc 144
Thr Asp Asp Pro Asp Leu Asp Asp Ile Ala Phe Ser Asp Asp Ala Pro
        35                  40                  45
tca gat gcc caa gcc agc acc gcg gta gaa gga aag gcc gag aat gag 192
Ser Asp Ala Gln Ala Ser Thr Ala Val Glu Gly Lys Ala Glu Asn Glu
    50                  55                  60
gag gag atg gac ccg ctc gac gcc ttc atg gcc gag atc cag gag gag 240
Glu Glu Met Asp Pro Leu Asp Ala Phe Met Ala Glu Ile Gln Glu Glu
65                  70                  75                  80
atc cgt gcg ccg ccc ccg ccg ccc aag ccc gag gcg ctt cgc cgc acg 288
Ile Arg Ala Pro Pro Pro Pro Pro Lys Pro Glu Ala Leu Arg Arg Thr
                85                  90                  95
gat tcc gac gat gac gag gat gac ccc gtc gag agc ttc ctg cgg gcc 336
Asp Ser Asp Asp Asp Glu Asp Asp Pro Val Glu Ser Phe Leu Arg Ala
            100                 105                 110
aag aag gac gct ggc ctc acg ctg gcc gcc gac gtc atg cgc gct ggt 384
Lys Lys Asp Ala Gly Leu Thr Leu Ala Ala Asp Val Met Arg Ala Gly
        115                 120                 125
tac gac tcc gat gag gag gtc tac gcc gct gcc aag gcc gtc gac gcc 432
Tyr Asp Ser Asp Glu Glu Val Tyr Ala Ala Ala Lys Ala Val Asp Ala
    130                 135                 140
ggc atg atg gaa tac gac tct gat gac aac ccc ata gtc atc gac aag 480
Gly Met Met Glu Tyr Asp Ser Asp Asp Asn Pro Ile Val Ile Asp Lys
145                 150                 155                 160
aag aaa ata gag ccc ata cca gca ctc gat cac tcg acc att gag tat 528
Lys Lys Ile Glu Pro Ile Pro Ala Leu Asp His Ser Thr Ile Glu Tyr
                165                 170                 175
gac gcc ttc aca aaa gat ttc tac gag gag aag cca tcg att tca ggt 576
Asp Ala Phe Thr Lys Asp Phe Tyr Glu Glu Lys Pro Ser Ile Ser Gly
            180                 185                 190
atg agt gac caa gag gta aca gat tat atg aaa agt ttg tca att cgt 624
Met Ser Asp Gln Glu Val Thr Asp Tyr Met Lys Ser Leu Ser Ile Arg
        195                 200                 205
gtt tct ggt ttt gat gtg ccg agg cca ata aaa aac ttt gag gat tgt 672
Val Ser Gly Phe Asp Val Pro Arg Pro Ile Lys Asn Phe Glu Asp Cys
    210                 215                 220
ggg ttt cat gta cct cta atg aat gct att gcc aaa caa gct tat gaa 720
Gly Phe His Val Pro Leu Met Asn Ala Ile Ala Lys Gln Ala Tyr Glu
225                 230                 235                 240
aaa cca act aca att cag tgc cag gct tta cct att gta ctt tca ggg 768
Lys Pro Thr Thr Ile Gln Cys Gln Ala Leu Pro Ile Val Leu Ser Gly
                245                 250                 255
aga gat atc att ggt att gca aaa act ggc tct ggt aaa act gca gct 816
Arg Asp Ile Ile Gly Ile Ala Lys Thr Gly Ser Gly Lys Thr Ala Ala
            260                 265                 270
ttt gtg ctc cct atg att gtt cat att atg gat cag cct gag ctt gag 864
Phe Val Leu Pro Met Ile Val His Ile Met Asp Gln Pro Glu Leu Glu
        275                 280                 285
aaa gaa gaa ggt cca ata gga gtc att tgt gct cca aca aga gaa ttg 912
Lys Glu Glu Gly Pro Ile Gly Val Ile Cys Ala Pro Thr Arg Glu Leu
    290                 295                 300
gca cat cag ata tat ctc gaa gct aag aag ttt gca aag cct tac aac 960
Ala His Gln Ile Tyr Leu Glu Ala Lys Lys Phe Ala Lys Pro Tyr Asn
305                 310                 315                 320
ctt cga gtt gct gct ata tat ggt ggt gtt tcc aaa ttt gac cag ttt 1008
Leu Arg Val Ala Ala Ile Tyr Gly Gly Val Ser Lys Phe Asp Gln Phe
                325                 330                 335
aaa gaa ctg aaa gca ggt tgt gaa ata gtc att gca act cca ggg aga 1056
Lys Glu Leu Lys Ala Gly Cys Glu Ile Val Ile Ala Thr Pro Gly Arg
            340                 345                 350
tta ata gac ttg ctg aag atg aag gca ttg aag atg ttc agg gca act 1104
Leu Ile Asp Leu Leu Lys Met Lys Ala Leu Lys Met Phe Arg Ala Thr

        355                 360                 365
tat ctg gtt ctt gat gaa gct gat cga atg ttc gat ctt gga ttt gag 1152
Tyr Leu Val Leu Asp Glu Ala Asp Arg Met Phe Asp Leu Gly Phe Glu
    370                 375                 380
cca caa ata cga tcc att gtt ggt caa att aga cca gat cga caa act 1200
Pro Gln Ile Arg Ser Ile Val Gly Gln Ile Arg Pro Asp Arg Gln Thr
385                 390                 395                 400
tta ctt ttt tct gca aca atg cca tac aaa gta gag cgt ttg gca aga 1248
Leu Leu Phe Ser Ala Thr Met Pro Tyr Lys Val Glu Arg Leu Ala Arg
                405                 410                 415
gaa ata tta act gat cct att aga gtt aca gtt ggt caa gtt ggc ggt 1296
Glu Ile Leu Thr Asp Pro Ile Arg Val Thr Val Gly Gln Val Gly Gly
            420                 425                 430
gct aat gaa gac att aaa caa gtt gtt aat gta ctc cct tct gat gtt 1344
Ala Asn Glu Asp Ile Lys Gln Val Val Asn Val Leu Pro Ser Asp Val
        435                 440                 445
gag aaa atg cct tgg ctt ttg gag aaa ttg cct gga atg att gat gat 1392
Glu Lys Met Pro Trp Leu Leu Glu Lys Leu Pro Gly Met Ile Asp Asp
    450                 455                 460
gga gat gtt ctt gta ttc gca tct aaa aag gct aga gtg gac gag ata 1440
Gly Asp Val Leu Val Phe Ala Ser Lys Lys Ala Arg Val Asp Glu Ile
465                 470                 475                 480
gag aaa gag ttg aat cag aga gga ttc aga att gct gcg ctt cat ggc 1488
Glu Lys Glu Leu Asn Gln Arg Gly Phe Arg Ile Ala Ala Leu His Gly
                485                 490                 495
gac aag gat caa gct tct cgt atg gag acc ctg cag aag ttc aaa tct 1536
Asp Lys Asp Gln Ala Ser Arg Met Glu Thr Leu Gln Lys Phe Lys Ser
            500                 505                 510
ggg aca ttt cat gtt ctg gtt gca act gat gtt gct gca cga ggt ctt 1584
Gly Thr Phe His Val Leu Val Ala Thr Asp Val Ala Ala Arg Gly Leu
        515                 520                 525
gac atc aaa tcg att aaa aca gtt gtc aac ttt gat att gca aaa gag 1632
Asp Ile Lys Ser Ile Lys Thr Val Val Asn Phe Asp Ile Ala Lys Glu
    530                 535                 540
atg gat atg cat att cac cga att gga aga act ggt cgt gct ggc gat 1680
Met Asp Met His Ile His Arg Ile Gly Arg Thr Gly Arg Ala Gly Asp
545                 550                 555                 560
aaa gat ggc act gca tat act ctg att aca cag aag gag gca cat ttt 1728
Lys Asp Gly Thr Ala Tyr Thr Leu Ile Thr Gln Lys Glu Ala His Phe
                565                 570                 575
gct ggt gaa ctg gtt cac agt ttg att gct gct ggc cag gat gta ccc 1776
Ala Gly Glu Leu Val His Ser Leu Ile Ala Ala Gly Gln Asp Val Pro
            580                 585                 590
aat gaa ctc atg gat ctg gct atg aag gat gga aga ttc aga gca aaa 1824
Asn Glu Leu Met Asp Leu Ala Met Lys Asp Gly Arg Phe Arg Ala Lys
        595                 600                 605
cga gac tcc agg aaa ggt ggg aag aaa ggt ggc aaa gga aaa ggt ggt 1872
Arg Asp Ser Arg Lys Gly Gly Lys Lys Gly Gly Lys Gly Lys Gly Gly
    610                 615                 620
gtt ggc ggt gct ggg cgc ggg cgc ggt gtg cgt gga gtt gat ttt ggc 1920
Val Gly Gly Ala Gly Arg Gly Arg Gly Val Arg Gly Val Asp Phe Gly
625                 630                 635                 640
ctc ggc ata ggt tac aat gcc gaa tct ggt tca caa gtg cct gct gca 1968
Leu Gly Ile Gly Tyr Asn Ala Glu Ser Gly Ser Gln Val Pro Ala Ala
                645                 650                 655
aga tct gct gct gta aat tct ctg aag aca ggg atg ttg caa cga ttt 2016
Arg Ser Ala Ala Val Asn Ser Leu Lys Thr Gly Met Leu Gln Arg Phe
            660                 665                 670
aag agc agc ttt gtc tct gga tct tca aat aca cca agc agc agt gca 2064
Lys Ser Ser Phe Val Ser Gly Ser Ser Asn Thr Pro Ser Ser Ser Ala
        675                 680                 685
cct tcc ttt gta aga cca gca ctc cgt ggc ttt gtg tct ggc ggc acc 2112
Pro Ser Phe Val Arg Pro Ala Leu Arg Gly Phe Val Ser Gly Gly Thr
    690                 695                 700
att gga gga gac aca cgg cct gca cag gca gcc ccc act ttc atc cct 2160
Ile Gly Gly Asp Thr Arg Pro Ala Gln Ala Ala Pro Thr Phe Ile Pro
705                 710                 715                 720
gca tcc cgg cct gca cag cct gcc ccc gcg gtc cct gca tcc cag cct 2208
Ala Ser Arg Pro Ala Gln Pro Ala Pro Ala Val Pro Ala Ser Gln Pro

                    725                 730                 735
gca gga aat aac aac gaa aat ggg aac ccg aat cca gaa agt tca cgg        2256
Ala Gly Asn Asn Asn Glu Asn Gly Asn Pro Asn Pro Glu Ser Ser Arg
            740                 745                 750
gat cag ttc agg gag aga aaa cga cca tca ggt tgg gat cgc tag            2301
Asp Gln Phe Arg Glu Arg Lys Arg Pro Ser Gly Trp Asp Arg
        755                 760                 765

<210> 124
<211> 766
<212> PRT
<213> Zea mays

<400> 124
Met Ser Lys Arg Pro Lys Leu Glu Gly Phe Ser Ile Pro Arg Pro Thr
1               5                   10                  15
Ser Tyr Asn Phe Glu Arg Ser Gln Ser Val Gln Arg Leu Tyr Arg Pro
            20                  25                  30
Thr Asp Asp Pro Asp Leu Asp Asp Ile Ala Phe Ser Asp Asp Ala Pro
        35                  40                  45
Ser Asp Ala Gln Ala Ser Thr Ala Val Glu Gly Lys Ala Glu Asn Glu
    50                  55                  60
Glu Glu Met Asp Pro Leu Asp Ala Phe Met Ala Glu Ile Gln Glu Glu
65                  70                  75                  80
Ile Arg Ala Pro Pro Pro Pro Pro Lys Pro Glu Ala Leu Arg Arg Thr
                85                  90                  95
Asp Ser Asp Asp Asp Glu Asp Asp Pro Val Glu Ser Phe Leu Arg Ala
            100                 105                 110
Lys Lys Asp Ala Gly Leu Thr Leu Ala Ala Asp Val Met Arg Ala Gly
        115                 120                 125
Tyr Asp Ser Asp Glu Glu Val Tyr Ala Ala Ala Lys Ala Val Asp Ala
    130                 135                 140
Gly Met Met Glu Tyr Asp Ser Asp Asp Asn Pro Ile Val Ile Asp Lys
145                 150                 155                 160
Lys Lys Ile Glu Pro Ile Pro Ala Leu Asp His Ser Thr Ile Glu Tyr
                165                 170                 175
Asp Ala Phe Thr Lys Asp Phe Tyr Glu Glu Lys Pro Ser Ile Ser Gly
            180                 185                 190
Met Ser Asp Gln Glu Val Thr Asp Tyr Met Lys Ser Leu Ser Ile Arg
        195                 200                 205
Val Ser Gly Phe Asp Val Pro Arg Pro Ile Lys Asn Phe Glu Asp Cys
    210                 215                 220
Gly Phe His Val Pro Leu Met Asn Ala Ile Ala Lys Gln Ala Tyr Glu
225                 230                 235                 240
Lys Pro Thr Thr Ile Gln Cys Gln Ala Leu Pro Ile Val Leu Ser Gly
                245                 250                 255
Arg Asp Ile Ile Gly Ile Ala Lys Thr Gly Ser Gly Lys Thr Ala Ala
            260                 265                 270
Phe Val Leu Pro Met Ile Val His Ile Met Asp Gln Pro Glu Leu Glu
        275                 280                 285
Lys Glu Glu Gly Pro Ile Gly Val Ile Cys Ala Pro Thr Arg Glu Leu
    290                 295                 300
Ala His Gln Ile Tyr Leu Glu Ala Lys Lys Phe Ala Lys Pro Tyr Asn
305                 310                 315                 320
Leu Arg Val Ala Ala Ile Tyr Gly Gly Val Ser Lys Phe Asp Gln Phe
                325                 330                 335
Lys Glu Leu Lys Ala Gly Cys Glu Ile Val Ile Ala Thr Pro Gly Arg
            340                 345                 350
Leu Ile Asp Leu Leu Lys Met Lys Ala Leu Lys Met Phe Arg Ala Thr
        355                 360                 365
Tyr Leu Val Leu Asp Glu Ala Asp Arg Met Phe Asp Leu Gly Phe Glu
    370                 375                 380
Pro Gln Ile Arg Ser Ile Val Gly Gln Ile Arg Pro Asp Arg Gln Thr
385                 390                 395                 400
Leu Leu Phe Ser Ala Thr Met Pro Tyr Lys Val Glu Arg Leu Ala Arg
                405                 410                 415
Glu Ile Leu Thr Asp Pro Ile Arg Val Thr Val Gly Gln Val Gly Gly
            420                 425                 430
Ala Asn Glu Asp Ile Lys Gln Val Val Asn Val Leu Pro Ser Asp Val

435                 440                 445
Glu Lys Met Pro Trp Leu Leu Glu Lys Leu Pro Gly Met Ile Asp Asp
    450                 455                 460
Gly Asp Val Leu Val Phe Ala Ser Lys Lys Ala Arg Val Asp Glu Ile
465                 470                 475                 480
Glu Lys Glu Leu Asn Gln Arg Gly Phe Arg Ile Ala Ala Leu His Gly
                485                 490                 495
Asp Lys Asp Gln Ala Ser Arg Met Glu Thr Leu Gln Lys Phe Lys Ser
            500                 505                 510
Gly Thr Phe His Val Leu Val Ala Thr Asp Val Ala Ala Arg Gly Leu
        515                 520                 525
Asp Ile Lys Ser Ile Lys Thr Val Val Asn Phe Asp Ile Ala Lys Glu
    530                 535                 540
Met Asp Met His Ile His Arg Ile Gly Arg Thr Gly Arg Ala Gly Asp
545                 550                 555                 560
Lys Asp Gly Thr Ala Tyr Thr Leu Ile Thr Gln Lys Glu Ala His Phe
                565                 570                 575
Ala Gly Glu Leu Val His Ser Leu Ile Ala Ala Gly Gln Asp Val Pro
            580                 585                 590
Asn Glu Leu Met Asp Leu Ala Met Lys Asp Gly Arg Phe Arg Ala Lys
        595                 600                 605
Arg Asp Ser Arg Lys Gly Gly Lys Lys Gly Gly Lys Gly Lys Gly Gly
    610                 615                 620
Val Gly Gly Ala Gly Arg Gly Arg Gly Val Arg Gly Val Asp Phe Gly
625                 630                 635                 640
Leu Gly Ile Gly Tyr Asn Ala Glu Ser Gly Ser Gln Val Pro Ala Ala
                645                 650                 655
Arg Ser Ala Ala Val Asn Ser Leu Lys Thr Gly Met Leu Gln Arg Phe
            660                 665                 670
Lys Ser Ser Phe Val Ser Gly Ser Ser Asn Thr Pro Ser Ser Ser Ala
        675                 680                 685
Pro Ser Phe Val Arg Pro Ala Leu Arg Gly Phe Val Ser Gly Gly Thr
    690                 695                 700
Ile Gly Gly Asp Thr Arg Pro Ala Gln Ala Ala Pro Thr Phe Ile Pro
705                 710                 715                 720
Ala Ser Arg Pro Ala Gln Pro Ala Pro Ala Val Pro Ala Ser Gln Pro
                725                 730                 735
Ala Gly Asn Asn Asn Glu Asn Gly Asn Pro Asn Pro Glu Ser Ser Arg
            740                 745                 750
Asp Gln Phe Arg Glu Arg Lys Arg Pro Ser Gly Trp Asp Arg
        755                 760                 765

<210> 125
<211> 1422
<212> DNA
<213> Zea mays

<220>
<221> CDS
<222> (1)..(1422)

<400> 125
atg aac ccg tat gat ctc cgc ttc gct gac ccc tca tcc tac cac gac      48
Met Asn Pro Tyr Asp Leu Arg Phe Ala Asp Pro Ser Ser Tyr His Asp
1               5                   10                  15
agg cgc agc gac ttg acc gtg gct cct acg ttc gct ccg ccg gct ccc      96
Arg Arg Ser Asp Leu Thr Val Ala Pro Thr Phe Ala Pro Pro Ala Pro
            20                  25                  30
gtg gct gcg gcg aac ctg aac cct tac gcc gcc gcc tac ccg ctg cct      144
Val Ala Ala Ala Asn Leu Asn Pro Tyr Ala Ala Ala Tyr Pro Leu Pro
        35                  40                  45
gtg ccc gtt ccc gtg ccc gcg gcc ccc gcg ggc ggc gac tac cag cgc      192
Val Pro Val Pro Val Pro Ala Ala Pro Ala Gly Gly Asp Tyr Gln Arg
    50                  55                  60
tac ggg cag gga ggc aga ggc cgt ggt ggc ggt ggc aga ggc ggt gga      240
Tyr Gly Gln Gly Gly Arg Gly Arg Gly Gly Gly Gly Arg Gly Gly Gly
65                  70                  75                  80
gga ggg tac gga ggt ggc ggc aga gga gga ggt cgc ggg agg gac ggg      288
Gly Gly Tyr Gly Gly Gly Gly Arg Gly Gly Gly Arg Gly Arg Asp Gly

                85                  90                  95
ctt gac tcg ctc gcc ctc cct aag ccg gac ttt cgg agc ctg ata ccg 336
Leu Asp Ser Leu Ala Leu Pro Lys Pro Asp Phe Arg Ser Leu Ile Pro
            100                 105                 110
ttc gag aaa aac ttc tac gtg gag tgt ccc tcc gtc cag gca atg tcg 384
Phe Glu Lys Asn Phe Tyr Val Glu Cys Pro Ser Val Gln Ala Met Ser
        115                 120                 125
gag gcg gat gtg gcg cag tac cgc cgc ctc cgg gat atc acc atc gag 432
Glu Ala Asp Val Ala Gln Tyr Arg Arg Leu Arg Asp Ile Thr Ile Glu
    130                 135                 140
ggc cgg gat gtg ccc aag ccg gtt aga tac ttc cag gaa gcc aac ttc 480
Gly Arg Asp Val Pro Lys Pro Val Arg Tyr Phe Gln Glu Ala Asn Phe
145                 150                 155                 160
cca gat tac tgc atg caa gct att gcc aaa tct gga ttt gtg gag cca 528
Pro Asp Tyr Cys Met Gln Ala Ile Ala Lys Ser Gly Phe Val Glu Pro
                165                 170                 175
aca cct att caa tct caa ggt tgg cct atg gct ttg aag ggt agg gac 576
Thr Pro Ile Gln Ser Gln Gly Trp Pro Met Ala Leu Lys Gly Arg Asp
            180                 185                 190
ctg att ggt atc gct caa aca ggc tca ggg aaa aca tta tct tat ctg 624
Leu Ile Gly Ile Ala Gln Thr Gly Ser Gly Lys Thr Leu Ser Tyr Leu
        195                 200                 205
tta cct ggg ttg gtt cat gtt ggt gcc cag cct cgg cta gaa caa ggc 672
Leu Pro Gly Leu Val His Val Gly Ala Gln Pro Arg Leu Glu Gln Gly
    210                 215                 220
gat ggc cca att gta tta att ctc gct ccc acg aga gaa ctg gct gtt 720
Asp Gly Pro Ile Val Leu Ile Leu Ala Pro Thr Arg Glu Leu Ala Val
225                 230                 235                 240
caa ata cag gaa gaa tct aca aaa ttt gga tca tat tca aga act aga 768
Gln Ile Gln Glu Glu Ser Thr Lys Phe Gly Ser Tyr Ser Arg Thr Arg
                245                 250                 255
agc aca tgt gtc tat ggt ggt gca cct aag ggt cca caa ata cgg gac 816
Ser Thr Cys Val Tyr Gly Gly Ala Pro Lys Gly Pro Gln Ile Arg Asp
            260                 265                 270
ctt aga aga gga gtt gaa att gtc att gca act cct ggc cgg ttg att 864
Leu Arg Arg Gly Val Glu Ile Val Ile Ala Thr Pro Gly Arg Leu Ile
        275                 280                 285
gat atg ctg gaa gct ggt cac aca aac cta cgt aga gtg aca tac ctt 912
Asp Met Leu Glu Ala Gly His Thr Asn Leu Arg Arg Val Thr Tyr Leu
    290                 295                 300
gtg ttg gat gag gcg gac cgg atg cta gat atg ggg ttt gag ccc caa 960
Val Leu Asp Glu Ala Asp Arg Met Leu Asp Met Gly Phe Glu Pro Gln
305                 310                 315                 320
att cgg aaa ata gta gca cag atc cgc cca gac agg caa acc ttg tac 1008
Ile Arg Lys Ile Val Ala Gln Ile Arg Pro Asp Arg Gln Thr Leu Tyr
                325                 330                 335
tgg agt gcc aca tgg cct aga gaa gtt gaa gcc tta gca aga cag ttt 1056
Trp Ser Ala Thr Trp Pro Arg Glu Val Glu Ala Leu Ala Arg Gln Phe
            340                 345                 350
cta caa aat cct tac aag gta atc ata ggc tca cct gaa ctt aaa gct 1104
Leu Gln Asn Pro Tyr Lys Val Ile Ile Gly Ser Pro Glu Leu Lys Ala
        355                 360                 365
aat cat tcc ata caa caa att gtt gaa gtt att tca gat cat gag aag 1152
Asn His Ser Ile Gln Gln Ile Val Glu Val Ile Ser Asp His Glu Lys
    370                 375                 380
tat cca aga ctc agt aag ctc ttg tct gat cta atg gat ggg agc cgt 1200
Tyr Pro Arg Leu Ser Lys Leu Leu Ser Asp Leu Met Asp Gly Ser Arg
385                 390                 395                 400
atc ttg ata ttc ttc caa aca aaa aag gat tgc gat aag atc acc cgg 1248
Ile Leu Ile Phe Phe Gln Thr Lys Lys Asp Cys Asp Lys Ile Thr Arg
                405                 410                 415
cag ctt aga atg gat ggg tgg cca gca tta tct ata cat ggt gat aaa 1296
Gln Leu Arg Met Asp Gly Trp Pro Ala Leu Ser Ile His Gly Asp Lys
            420                 425                 430
gct caa gct gaa cgg gac tat gtt ctt gca gag ttt aag agc ggc aag 1344
Ala Gln Ala Glu Arg Asp Tyr Val Leu Ala Glu Phe Lys Ser Gly Lys
        435                 440                 445
agc ccc ata atg gct gcc aca gat gtg gca gca cgt ggt ctt ggt atg 1392
Ser Pro Ile Met Ala Ala Thr Asp Val Ala Ala Arg Gly Leu Gly Met

450                 455                 460
atg att tat ttg aat att agg att agg tag                                 1422
Met Ile Tyr Leu Asn Ile Arg Ile Arg
465                 470

<210> 126
<211> 473
<212> PRT
<213> Zea mays

<400> 126
Met Asn Pro Tyr Asp Leu Arg Phe Ala Asp Pro Ser Ser Tyr His Asp
1               5                   10                  15
Arg Arg Ser Asp Leu Thr Val Ala Pro Thr Phe Ala Pro Pro Ala Pro
            20                  25                  30
Val Ala Ala Ala Asn Leu Asn Pro Tyr Ala Ala Ala Tyr Pro Leu Pro
        35                  40                  45
Val Pro Val Pro Val Pro Ala Ala Pro Ala Gly Gly Asp Tyr Gln Arg
    50                  55                  60
Tyr Gly Gln Gly Gly Arg Gly Arg Gly Gly Gly Gly Arg Gly Gly Gly
65                  70                  75                  80
Gly Gly Tyr Gly Gly Gly Gly Arg Gly Gly Gly Arg Gly Arg Asp Gly
                85                  90                  95
Leu Asp Ser Leu Ala Leu Pro Lys Pro Asp Phe Arg Ser Leu Ile Pro
            100                 105                 110
Phe Glu Lys Asn Phe Tyr Val Glu Cys Pro Ser Val Gln Ala Met Ser
        115                 120                 125
Glu Ala Asp Val Ala Gln Tyr Arg Arg Leu Arg Asp Ile Thr Ile Glu
    130                 135                 140
Gly Arg Asp Val Pro Lys Pro Val Arg Tyr Phe Gln Glu Ala Asn Phe
145                 150                 155                 160
Pro Asp Tyr Cys Met Gln Ala Ile Ala Lys Ser Gly Phe Val Glu Pro
                165                 170                 175
Thr Pro Ile Gln Ser Gln Gly Trp Pro Met Ala Leu Lys Gly Arg Asp
            180                 185                 190
Leu Ile Gly Ile Ala Gln Thr Gly Ser Gly Lys Thr Leu Ser Tyr Leu
        195                 200                 205
Leu Pro Gly Leu Val His Val Gly Ala Gln Pro Arg Leu Glu Gln Gly
    210                 215                 220
Asp Gly Pro Ile Val Leu Ile Leu Ala Pro Thr Arg Glu Leu Ala Val
225                 230                 235                 240
Gln Ile Gln Glu Glu Ser Thr Lys Phe Gly Ser Tyr Ser Arg Thr Arg
                245                 250                 255
Ser Thr Cys Val Tyr Gly Gly Ala Pro Lys Gly Pro Gln Ile Arg Asp
            260                 265                 270
Leu Arg Arg Gly Val Glu Ile Val Ile Ala Thr Pro Gly Arg Leu Ile
        275                 280                 285
Asp Met Leu Glu Ala Gly His Thr Asn Leu Arg Arg Val Thr Tyr Leu
    290                 295                 300
Val Leu Asp Glu Ala Asp Arg Met Leu Asp Met Gly Phe Glu Pro Gln
305                 310                 315                 320
Ile Arg Lys Ile Val Ala Gln Ile Arg Pro Asp Arg Gln Thr Leu Tyr
                325                 330                 335
Trp Ser Ala Thr Trp Pro Arg Glu Val Glu Ala Leu Ala Arg Gln Phe
            340                 345                 350
Leu Gln Asn Pro Tyr Lys Val Ile Ile Gly Ser Pro Glu Leu Lys Ala
        355                 360                 365
Asn His Ser Ile Gln Gln Ile Val Glu Val Ile Ser Asp His Glu Lys
    370                 375                 380
Tyr Pro Arg Leu Ser Lys Leu Leu Ser Asp Leu Met Asp Gly Ser Arg
385                 390                 395                 400
Ile Leu Ile Phe Phe Gln Thr Lys Lys Asp Cys Asp Lys Ile Thr Arg
                405                 410                 415
Gln Leu Arg Met Asp Gly Trp Pro Ala Leu Ser Ile His Gly Asp Lys
            420                 425                 430
Ala Gln Ala Glu Arg Asp Tyr Val Leu Ala Glu Phe Lys Ser Gly Lys
        435                 440                 445
Ser Pro Ile Met Ala Ala Thr Asp Val Ala Ala Arg Gly Leu Gly Met
    450                 455                 460

Met Ile Tyr Leu Asn Ile Arg Ile Arg
465                 470

<210> 127
<211> 1500
<212> DNA
<213> Zea mays

<220>
<221> CDS
<222> (1)..(1500)

<400> 127
atg cac cag cct aga gct cgc tac cca ccc ggc tat ggc tcc ggc ggt       48
Met His Gln Pro Arg Ala Arg Tyr Pro Pro Gly Tyr Gly Ser Gly Gly
1               5                   10                  15
gga ggc gga gcc gga cgt ggc ggt gga ggc gga aat gga gga ggt ggc       96
Gly Gly Gly Ala Gly Arg Gly Gly Gly Gly Gly Asn Gly Gly Gly Gly
            20                  25                  30
gga aac cac aac tac tac ggc cga aac ccg cag caa cac cac tac cac      144
Gly Asn His Asn Tyr Tyr Gly Arg Asn Pro Gln Gln His His Tyr His
        35                  40                  45
cag cag cag cag tcg cag cac tct cat agg aac tct tcg tac cag caa      192
Gln Gln Gln Gln Ser Gln His Ser His Arg Asn Ser Ser Tyr Gln Gln
    50                  55                  60
cag tgg ctg cgg cga gac cag gcc ccc gcc gtt gcg ggg gca gcg tcc      240
Gln Trp Leu Arg Arg Asp Gln Ala Pro Ala Val Ala Gly Ala Ala Ser
65                  70                  75                  80
gga aac gct gtg gcc aag acg gcg tct cag ctt gac gcc gta ggc tcg      288
Gly Asn Ala Val Ala Lys Thr Ala Ser Gln Leu Asp Ala Val Gly Ser
                85                  90                  95
agc tcc caa gac tgg aag gct caa cta aat ata cca gct cca gat aca      336
Ser Ser Gln Asp Trp Lys Ala Gln Leu Asn Ile Pro Ala Pro Asp Thr
            100                 105                 110
cgc tac agg aca gag gat gtc act gca aca aaa ggc aat gag ttt gaa      384
Arg Tyr Arg Thr Glu Asp Val Thr Ala Thr Lys Gly Asn Glu Phe Glu
        115                 120                 125
gat tat ttc ttg aaa cgt gaa ctg ctt atg gga atc tac gaa aag gga      432
Asp Tyr Phe Leu Lys Arg Glu Leu Leu Met Gly Ile Tyr Glu Lys Gly
    130                 135                 140
ttt gaa aga cca tct cct att caa gaa gaa agc att cct att gcg ctg      480
Phe Glu Arg Pro Ser Pro Ile Gln Glu Glu Ser Ile Pro Ile Ala Leu
145                 150                 155                 160
act gga agt gat att ctt gca agg gca aaa aat ggc act gga aag act      528
Thr Gly Ser Asp Ile Leu Ala Arg Ala Lys Asn Gly Thr Gly Lys Thr
                165                 170                 175
gct gcc ttt tgc att ccg gca ctt gaa aaa att gat cct gaa aaa aat      576
Ala Ala Phe Cys Ile Pro Ala Leu Glu Lys Ile Asp Pro Glu Lys Asn
            180                 185                 190
gct att caa gtt gtt ata cta gta cca aca agg gaa ctg gct ctg caa      624
Ala Ile Gln Val Val Ile Leu Val Pro Thr Arg Glu Leu Ala Leu Gln
        195                 200                 205
act tca caa gtc tgt aaa gag ctc ggg aaa tac cta aac att caa gtt      672
Thr Ser Gln Val Cys Lys Glu Leu Gly Lys Tyr Leu Asn Ile Gln Val
    210                 215                 220
atg gtt agc act gga gga act agc ttg aag gat gac atc atg cgt tta      720
Met Val Ser Thr Gly Gly Thr Ser Leu Lys Asp Asp Ile Met Arg Leu
225                 230                 235                 240
tac caa cct gtt cat tta ctt gtt ggg act cct ggc cga ata ctt gat      768
Tyr Gln Pro Val His Leu Leu Val Gly Thr Pro Gly Arg Ile Leu Asp
                245                 250                 255
ctt acc agg aag ggc att tgt gtg ttg aaa gac tgt tca atg ctt gtc      816
Leu Thr Arg Lys Gly Ile Cys Val Leu Lys Asp Cys Ser Met Leu Val
            260                 265                 270
atg gat gag gca gac aag cta tta gct cca gag ttc cag cct tct gtg      864
Met Asp Glu Ala Asp Lys Leu Leu Ala Pro Glu Phe Gln Pro Ser Val
        275                 280                 285
gag gca ctt att cat ttc ctt cca cct agt cgg cag ctg ttg atg ttt      912
Glu Ala Leu Ile His Phe Leu Pro Pro Ser Arg Gln Leu Leu Met Phe

290                 295                 300
tca gca acc ttt cct gta act gtc aag gaa ttc aaa gat aaa tat ctg         960
Ser Ala Thr Phe Pro Val Thr Val Lys Glu Phe Lys Asp Lys Tyr Leu
305                 310                 315                 320
cct aga cct tat gtg att aat cta atg gat gaa ctg acc ctg aaa gga         1008
Pro Arg Pro Tyr Val Ile Asn Leu Met Asp Glu Leu Thr Leu Lys Gly
                325                 330                 335
atc aca caa tat tat gct ttt gtt gaa gaa agg caa aaa gtt cat tgc         1056
Ile Thr Gln Tyr Tyr Ala Phe Val Glu Glu Arg Gln Lys Val His Cys
            340                 345                 350
ctg aat aca ctt ttc tca aag ctt caa att aat caa tcc att ata ttc         1104
Leu Asn Thr Leu Phe Ser Lys Leu Gln Ile Asn Gln Ser Ile Ile Phe
        355                 360                 365
tgc aac tct gtt aat aga gtt gag ctg ctg gct aaa aaa ata act gaa         1152
Cys Asn Ser Val Asn Arg Val Glu Leu Leu Ala Lys Lys Ile Thr Glu
    370                 375                 380
ctc ggt tat tca tgc ttc tac att cat gct aag atg ttg caa gac cac         1200
Leu Gly Tyr Ser Cys Phe Tyr Ile His Ala Lys Met Leu Gln Asp His
385                 390                 395                 400
aga aac cga gtg ttt cat gat ttt cgt aat ggt gct tgc aga aac ctt         1248
Arg Asn Arg Val Phe His Asp Phe Arg Asn Gly Ala Cys Arg Asn Leu
                405                 410                 415
gtg tgc aca gat ctg ttt acc aga gga att gat atc cag gct gtt aat         1296
Val Cys Thr Asp Leu Phe Thr Arg Gly Ile Asp Ile Gln Ala Val Asn
            420                 425                 430
gtg gtc att aat ttt gac ttc cct aag aca tct gag aca tac tta cac         1344
Val Val Ile Asn Phe Asp Phe Pro Lys Thr Ser Glu Thr Tyr Leu His
        435                 440                 445
agg gtt ggc cgt tct ggg aga tat gga cac ctt ggt ttg gca gtg aac         1392
Arg Val Gly Arg Ser Gly Arg Tyr Gly His Leu Gly Leu Ala Val Asn
    450                 455                 460
ttg atc act tat gaa gac cgt ttc aac tta tat agg att gag caa gaa         1440
Leu Ile Thr Tyr Glu Asp Arg Phe Asn Leu Tyr Arg Ile Glu Gln Glu
465                 470                 475                 480
ctt gga acg gaa att aag aca ata ccc cca cag att gac ctg tcc ata         1488
Leu Gly Thr Glu Ile Lys Thr Ile Pro Pro Gln Ile Asp Leu Ser Ile
                485                 490                 495
tac tgc caa tga                                                         1500
Tyr Cys Gln


<210> 128
<211> 499
<212> PRT
<213> Zea mays

<400> 128
Met His Gln Pro Arg Ala Arg Tyr Pro Pro Gly Tyr Gly Ser Gly Gly
1               5                   10                  15
Gly Gly Gly Ala Gly Arg Gly Gly Gly Gly Gly Asn Gly Gly Gly Gly
            20                  25                  30
Gly Asn His Asn Tyr Tyr Gly Arg Asn Pro Gln Gln His His Tyr His
        35                  40                  45
Gln Gln Gln Gln Ser Gln His Ser His Arg Asn Ser Ser Tyr Gln Gln
    50                  55                  60
Gln Trp Leu Arg Arg Asp Gln Ala Pro Ala Val Ala Gly Ala Ala Ser
65                  70                  75                  80
Gly Asn Ala Val Ala Lys Thr Ala Ser Gln Leu Asp Ala Val Gly Ser
                85                  90                  95
Ser Ser Gln Asp Trp Lys Ala Gln Leu Asn Ile Pro Ala Pro Asp Thr
            100                 105                 110
Arg Tyr Arg Thr Glu Asp Val Thr Ala Thr Lys Gly Asn Glu Phe Glu
        115                 120                 125
Asp Tyr Phe Leu Lys Arg Glu Leu Leu Met Gly Ile Tyr Glu Lys Gly
    130                 135                 140
Phe Glu Arg Pro Ser Pro Ile Gln Glu Glu Ser Ile Pro Ile Ala Leu
145                 150                 155                 160
Thr Gly Ser Asp Ile Leu Ala Arg Ala Lys Asn Gly Thr Gly Lys Thr
                165                 170                 175

Ala Ala Phe Cys Ile Pro Ala Leu Glu Lys Ile Asp Pro Glu Lys Asn
            180                 185                 190
Ala Ile Gln Val Val Ile Leu Val Pro Thr Arg Glu Leu Ala Leu Gln
        195                 200                 205
Thr Ser Gln Val Cys Lys Glu Leu Gly Lys Tyr Leu Asn Ile Gln Val
    210                 215                 220
Met Val Ser Thr Gly Gly Thr Ser Leu Lys Asp Asp Ile Met Arg Leu
225                 230                 235                 240
Tyr Gln Pro Val His Leu Leu Val Gly Thr Pro Gly Arg Ile Leu Asp
                245                 250                 255
Leu Thr Arg Lys Gly Ile Cys Val Leu Lys Asp Cys Ser Met Leu Val
            260                 265                 270
Met Asp Glu Ala Asp Lys Leu Leu Ala Pro Glu Phe Gln Pro Ser Val
        275                 280                 285
Glu Ala Leu Ile His Phe Leu Pro Pro Ser Arg Gln Leu Leu Met Phe
    290                 295                 300
Ser Ala Thr Phe Pro Val Thr Val Lys Glu Phe Lys Asp Lys Tyr Leu
305                 310                 315                 320
Pro Arg Pro Tyr Val Ile Asn Leu Met Asp Glu Leu Thr Leu Lys Gly
                325                 330                 335
Ile Thr Gln Tyr Tyr Ala Phe Val Glu Glu Arg Gln Lys Val His Cys
            340                 345                 350
Leu Asn Thr Leu Phe Ser Lys Leu Gln Ile Asn Gln Ser Ile Ile Phe
        355                 360                 365
Cys Asn Ser Val Asn Arg Val Glu Leu Leu Ala Lys Lys Ile Thr Glu
    370                 375                 380
Leu Gly Tyr Ser Cys Phe Tyr Ile His Ala Lys Met Leu Gln Asp His
385                 390                 395                 400
Arg Asn Arg Val Phe His Asp Phe Arg Asn Gly Ala Cys Arg Asn Leu
                405                 410                 415
Val Cys Thr Asp Leu Phe Thr Arg Gly Ile Asp Ile Gln Ala Val Asn
            420                 425                 430
Val Val Ile Asn Phe Asp Phe Pro Lys Thr Ser Glu Thr Tyr Leu His
        435                 440                 445
Arg Val Gly Arg Ser Gly Arg Tyr Gly His Leu Gly Leu Ala Val Asn
    450                 455                 460
Leu Ile Thr Tyr Glu Asp Arg Phe Asn Leu Tyr Arg Ile Glu Gln Glu
465                 470                 475                 480
Leu Gly Thr Glu Ile Lys Thr Ile Pro Pro Gln Ile Asp Leu Ser Ile
                485                 490                 495
Tyr Cys Gln

<210> 129
<211> 1506
<212> DNA
<213> Zea mays

<220>
<221> CDS
<222> (1)..(1506)

<400> 129
atg cgc gtg gcg gag cgg aag cgc gcg agg cga cgc ggc aac agg agg      48
Met Arg Val Ala Glu Arg Lys Arg Ala Arg Arg Arg Gly Asn Arg Arg
1               5                   10                  15
agg agg cca agg cta agg cga ccg cgg cag ccg aag cgg tgg cgg cgg      96
Arg Arg Pro Arg Leu Arg Arg Pro Arg Gln Pro Lys Arg Trp Arg Arg
            20                  25                  30
ctc gac gcg gcg tcc tct tcc tcc tcc atc acc gag ccg gcg gtg gag      144
Leu Asp Ala Ala Ser Ser Ser Ser Ser Ile Thr Glu Pro Ala Val Glu
        35                  40                  45
gag cag gcc aag cag atc gag gcg ctc tcc ctc gcc ccg tcg gtg gac      192
Glu Gln Ala Lys Gln Ile Glu Ala Leu Ser Leu Ala Pro Ser Val Asp
    50                  55                  60
gac gcc gca ggc gaa gag ggc ccg ccc ctc ctc gat gac tcc gac gac      240
Asp Ala Ala Gly Glu Glu Gly Pro Pro Leu Leu Asp Asp Ser Asp Asp
65                  70                  75                  80
tcg caa atc caa gcc gtg att tcg ggt ggc acg gtg tac gag tcg gcc      288

Ser Gln Ile Gln Ala Val Ile Ser Gly Gly Thr Val Tyr Glu Ser Ala
                85                  90                  95
acc acg ttc gag gat ctg aag ctg tcg ccc gag ctg ctt aag ggg ctg      336
Thr Thr Phe Glu Asp Leu Lys Leu Ser Pro Glu Leu Leu Lys Gly Leu
            100                 105                 110
cac gac gag atg ggg ttc agc cgc ccg agc aag atc cag gcg atc acc      384
His Asp Glu Met Gly Phe Ser Arg Pro Ser Lys Ile Gln Ala Ile Thr
        115                 120                 125
ctg ccc atg atc ctc acg ccg ccc tac aag gac ctc gtc gcg cag gcg      432
Leu Pro Met Ile Leu Thr Pro Pro Tyr Lys Asp Leu Val Ala Gln Ala
    130                 135                 140
cac aac ggc tct ggc aag acc acc tgt ttc gtc ctc ggc atg ctc agc      480
His Asn Gly Ser Gly Lys Thr Thr Cys Phe Val Leu Gly Met Leu Ser
145                 150                 155                 160
cgc gtc gac ccg caa cgc aag ata ccc cag gct atc tgc att tgc ccc      528
Arg Val Asp Pro Gln Arg Lys Ile Pro Gln Ala Ile Cys Ile Cys Pro
                165                 170                 175
acc agg gag ctt gcg cag cag aat aag gca gtt ctc atg agg atg ggc      576
Thr Arg Glu Leu Ala Gln Gln Asn Lys Ala Val Leu Met Arg Met Gly
            180                 185                 190
aag ttt act ggc att aca tgc gcc tgt gca atc ccg cca gct caa aaa      624
Lys Phe Thr Gly Ile Thr Cys Ala Cys Ala Ile Pro Pro Ala Gln Lys
        195                 200                 205
gac tac atg ccg atc tcg aaa atg gct ccg att act gac cag atc gtt      672
Asp Tyr Met Pro Ile Ser Lys Met Ala Pro Ile Thr Asp Gln Ile Val
    210                 215                 220
atc ggc aca tct ggt acg ctc atc aaa tgg att acc cat aag aag ctg      720
Ile Gly Thr Ser Gly Thr Leu Ile Lys Trp Ile Thr His Lys Lys Leu
225                 230                 235                 240
gcc aca agg gac atc aag ata ctt gtg ttt gat gag gca gat cat atg      768
Ala Thr Arg Asp Ile Lys Ile Leu Val Phe Asp Glu Ala Asp His Met
                245                 250                 255
ctt gct gag gat ggc ttt agg agt gat tct gaa agg atg atg agg gat      816
Leu Ala Glu Asp Gly Phe Arg Ser Asp Ser Glu Arg Met Met Arg Asp
            260                 265                 270
ata caa aga agc gct ggt ggt tgt cag gtg ctt ctc ttt tct gcg acc      864
Ile Gln Arg Ser Ala Gly Gly Cys Gln Val Leu Leu Phe Ser Ala Thr
        275                 280                 285
ttc aat gat agg gtg aag gat ttt gtt aca aag gtc att agg gat gga      912
Phe Asn Asp Arg Val Lys Asp Phe Val Thr Lys Val Ile Arg Asp Gly
    290                 295                 300
aat cag ata ttt gtg aag aag gaa gag ctt act ttg gaa aaa gtt aag      960
Asn Gln Ile Phe Val Lys Lys Glu Glu Leu Thr Leu Glu Lys Val Lys
305                 310                 315                 320
cag tac aaa gtt cag gta cct gat gaa tcg gca aaa ata gag gtt ata     1008
Gln Tyr Lys Val Gln Val Pro Asp Glu Ser Ala Lys Ile Glu Val Ile
                325                 330                 335
agg gac aag ata ttt gaa ttt gga cag aaa gtt ggc cag gtt atc ata     1056
Arg Asp Lys Ile Phe Glu Phe Gly Gln Lys Val Gly Gln Val Ile Ile
            340                 345                 350
ttt gtt aga aca aag caa agt act aag aat gtt cac aat gct ttg acg     1104
Phe Val Arg Thr Lys Gln Ser Thr Lys Asn Val His Asn Ala Leu Thr
        355                 360                 365
agg gag gac tat gtg tgc tcc tca atc caa gga tcc ctt gac caa tca     1152
Arg Glu Asp Tyr Val Cys Ser Ser Ile Gln Gly Ser Leu Asp Gln Ser
    370                 375                 380
gag agg gaa aag ata ata cag gaa ttc aaa gat ggc tac acc aag gtt     1200
Glu Arg Glu Lys Ile Ile Gln Glu Phe Lys Asp Gly Tyr Thr Lys Val
385                 390                 395                 400
ctt ata tca act gat gtt ctt gct cga ggt ttt gac caa gca cag gtt     1248
Leu Ile Ser Thr Asp Val Leu Ala Arg Gly Phe Asp Gln Ala Gln Val
                405                 410                 415
aac ctc gtc atc aac tac gac atg cca atc aaa ttt ggt aca aga gat     1296
Asn Leu Val Ile Asn Tyr Asp Met Pro Ile Lys Phe Gly Thr Arg Asp
            420                 425                 430
gaa cct gat tat gag gtg tac ttg cac aga att ggc aga gct ggg cgc     1344
Glu Pro Asp Tyr Glu Val Tyr Leu His Arg Ile Gly Arg Ala Gly Arg
        435                 440                 445
ttt ggc cgg aaa ggt gct gtg ttc aac ttg ctg tgt ggt caa acc gat     1392

Phe Gly Arg Lys Gly Ala Val Phe Asn Leu Leu Cys Gly Gln Thr Asp
    450                 455                 460
aat gtt gtg atg aag aag atc gag gac tat ttc cag cac agt gtg cct      1440
Asn Val Val Met Lys Lys Ile Glu Asp Tyr Phe Gln His Ser Val Pro
465                 470                 475                 480
gag gtt cgg aat tgg caa cgt gaa gaa gat ttt gag gct gct ctc aag      1488
Glu Val Arg Asn Trp Gln Arg Glu Glu Asp Phe Glu Ala Ala Leu Lys
                485                 490                 495
gat gca ggt tta ctt taa                                              1506
Asp Ala Gly Leu Leu
            500

<210> 130
<211> 501
<212> PRT
<213> Zea mays

<400> 130
Met Arg Val Ala Glu Arg Lys Arg Ala Arg Arg Arg Gly Asn Arg Arg
1               5                   10                  15
Arg Arg Pro Arg Leu Arg Arg Pro Arg Gln Pro Lys Arg Trp Arg Arg
            20                  25                  30
Leu Asp Ala Ala Ser Ser Ser Ser Ser Ile Thr Glu Pro Ala Val Glu
        35                  40                  45
Glu Gln Ala Lys Gln Ile Glu Ala Leu Ser Leu Ala Pro Ser Val Asp
    50                  55                  60
Asp Ala Ala Gly Glu Glu Gly Pro Pro Leu Leu Asp Asp Ser Asp Asp
65                  70                  75                  80
Ser Gln Ile Gln Ala Val Ile Ser Gly Gly Thr Val Tyr Glu Ser Ala
                85                  90                  95
Thr Thr Phe Glu Asp Leu Lys Leu Ser Pro Glu Leu Leu Lys Gly Leu
            100                 105                 110
His Asp Glu Met Gly Phe Ser Arg Pro Ser Lys Ile Gln Ala Ile Thr
        115                 120                 125
Leu Pro Met Ile Leu Thr Pro Pro Tyr Lys Asp Leu Val Ala Gln Ala
    130                 135                 140
His Asn Gly Ser Gly Lys Thr Thr Cys Phe Val Leu Gly Met Leu Ser
145                 150                 155                 160
Arg Val Asp Pro Gln Arg Lys Ile Pro Gln Ala Ile Cys Ile Cys Pro
                165                 170                 175
Thr Arg Glu Leu Ala Gln Gln Asn Lys Ala Val Leu Met Arg Met Gly
            180                 185                 190
Lys Phe Thr Gly Ile Thr Cys Ala Cys Ala Ile Pro Pro Ala Gln Lys
        195                 200                 205
Asp Tyr Met Pro Ile Ser Lys Met Ala Pro Ile Thr Asp Gln Ile Val
    210                 215                 220
Ile Gly Thr Ser Gly Thr Leu Ile Lys Trp Ile Thr His Lys Lys Leu
225                 230                 235                 240
Ala Thr Arg Asp Ile Lys Ile Leu Val Phe Asp Glu Ala Asp His Met
                245                 250                 255
Leu Ala Glu Asp Gly Phe Arg Ser Asp Ser Glu Arg Met Met Arg Asp
            260                 265                 270
Ile Gln Arg Ser Ala Gly Gly Cys Gln Val Leu Leu Phe Ser Ala Thr
        275                 280                 285
Phe Asn Asp Arg Val Lys Asp Phe Val Thr Lys Val Ile Arg Asp Gly
    290                 295                 300
Asn Gln Ile Phe Val Lys Lys Glu Glu Leu Thr Leu Glu Lys Val Lys
305                 310                 315                 320
Gln Tyr Lys Val Gln Val Pro Asp Glu Ser Ala Lys Ile Glu Val Ile
                325                 330                 335
Arg Asp Lys Ile Phe Glu Phe Gly Gln Lys Val Gly Gln Val Ile Ile
            340                 345                 350
Phe Val Arg Thr Lys Gln Ser Thr Lys Asn Val His Asn Ala Leu Thr
        355                 360                 365
Arg Glu Asp Tyr Val Cys Ser Ser Ile Gln Gly Ser Leu Asp Gln Ser
    370                 375                 380
Glu Arg Glu Lys Ile Ile Gln Glu Phe Lys Asp Gly Tyr Thr Lys Val
385                 390                 395                 400
Leu Ile Ser Thr Asp Val Leu Ala Arg Gly Phe Asp Gln Ala Gln Val

Asn Leu Val Ile Asn Tyr Asp Met Pro Ile Lys Phe Gly Thr Arg Asp
            405                 410                 415
Glu Pro Asp Tyr Glu Val Tyr Leu His Arg Ile Gly Arg Ala Gly Arg
            420                 425                 430
Phe Gly Arg Lys Gly Ala Val Phe Asn Leu Leu Cys Gly Gln Thr Asp
        435                 440                 445
Asn Val Val Met Lys Lys Ile Glu Asp Tyr Phe Gln His Ser Val Pro
    450                 455                 460
Glu Val Arg Asn Trp Gln Arg Glu Glu Asp Phe Glu Ala Ala Leu Lys
465                 470                 475                 480
Asp Ala Gly Leu Leu
            485                 490                 495
            500

<210> 131
<211> 1857
<212> DNA
<213> Zea mays

<220>
<221> CDS
<222> (1)..(1857)

<400> 131
atg tcc gcc atc gcg gcc tcc tcg gcc gcc gcc gcc gcg gcg tcg aac      48
Met Ser Ala Ile Ala Ala Ser Ser Ala Ala Ala Ala Ala Ala Ser Asn
1               5                   10                  15
tcc gat gag gaa gaa aac tat gag gag tac atc cca gtc gcc aag cgc      96
Ser Asp Glu Glu Glu Asn Tyr Glu Glu Tyr Ile Pro Val Ala Lys Arg
            20                  25                  30
cgg gcc atg gag gcc gag cgc ctc cgc cag ctc cgc ctc tcc aag tcg      144
Arg Ala Met Glu Ala Glu Arg Leu Arg Gln Leu Arg Leu Ser Lys Ser
        35                  40                  45
gcg ccg gcc tct gcc gtc tca tca ctc ccg atg cca cct ccc ccg cct      192
Ala Pro Ala Ser Ala Val Ser Ser Leu Pro Met Pro Pro Pro Pro Pro
    50                  55                  60
ctt ttg cca gcg cag acc tcc gtt ccc gac gcc gcc gcg aag ccc agc      240
Leu Leu Pro Ala Gln Thr Ser Val Pro Asp Ala Ala Ala Lys Pro Ser
65                  70                  75                  80
ctt ctc gtc aag gcc acg cag ctt aag cgc gcg gcc ccg gag gtg acc      288
Leu Leu Val Lys Ala Thr Gln Leu Lys Arg Ala Ala Pro Glu Val Thr
                85                  90                  95
gcc acg gag cag cgc atc cag cag gag aag gaa gtg att gaa aat ctc      336
Ala Thr Glu Gln Arg Ile Gln Gln Glu Lys Glu Val Ile Glu Asn Leu
            100                 105                 110
tca gac aac aaa tcg ctc agg tca gtc cgc gag atc gcc aag ggc atc      384
Ser Asp Asn Lys Ser Leu Arg Ser Val Arg Glu Ile Ala Lys Gly Ile
        115                 120                 125
atc tac acg gaa ccg atc cag acc ggg tgg aag ccg ccc ctc cgc ctc      432
Ile Tyr Thr Glu Pro Ile Gln Thr Gly Trp Lys Pro Pro Leu Arg Leu
    130                 135                 140
cgc cgc atg ccg ctc gcc aaa gcc aac gag ttc cgg cgc aag tgg cac      480
Arg Arg Met Pro Leu Ala Lys Ala Asn Glu Phe Arg Arg Lys Trp His
145                 150                 155                 160
atc atc gtc gac ggt gac gat ata ccg ccg cct gcg cgt gat ttc cgg      528
Ile Ile Val Asp Gly Asp Asp Ile Pro Pro Pro Ala Arg Asp Phe Arg
                165                 170                 175
gat ctc cgc ctc ccg gag ccc atg ctc cgg aag ctg cgc gag agg ggt      576
Asp Leu Arg Leu Pro Glu Pro Met Leu Arg Lys Leu Arg Glu Arg Gly
            180                 185                 190
atc gtc cag ccc acg ccg att cag gtg cag ggg ctg ccc gtt gtg ctc      624
Ile Val Gln Pro Thr Pro Ile Gln Val Gln Gly Leu Pro Val Val Leu
        195                 200                 205
tct ggg cgt gac atg ata ggc atc gct ttc acg ggg tcg ggg aag act      672
Ser Gly Arg Asp Met Ile Gly Ile Ala Phe Thr Gly Ser Gly Lys Thr
    210                 215                 220
ctg gtg ttc gtg ctg ccg ctt atc atg gtg gcg ctg cag gag gaa tca      720
Leu Val Phe Val Leu Pro Leu Ile Met Val Ala Leu Gln Glu Glu Ser
225                 230                 235                 240

ttg atg ccg atc gtg cct ggc gag ggc ccg ttt ggg atg atc ata tgc 768
Leu Met Pro Ile Val Pro Gly Glu Gly Pro Phe Gly Met Ile Ile Cys
                245                 250                 255
ccg tcg cgg gaa ctg gcc aag cag aca tat gat gtg att gag cag ttt 816
Pro Ser Arg Glu Leu Ala Lys Gln Thr Tyr Asp Val Ile Glu Gln Phe
            260                 265                 270
ctc att cca ctt aag gag gcc ggt tat ccg gag ata agg cat ttg ctt 864
Leu Ile Pro Leu Lys Glu Ala Gly Tyr Pro Glu Ile Arg His Leu Leu
        275                 280                 285
tgt att ggg ggt gtg gat atg agg aca cag ctc gat gtg ttg aag aag 912
Cys Ile Gly Gly Val Asp Met Arg Thr Gln Leu Asp Val Leu Lys Lys
    290                 295                 300
ggc gtg cac att gtg gtg gca acg cct ggt cgg ctc aag gat ttg ctt 960
Gly Val His Ile Val Val Ala Thr Pro Gly Arg Leu Lys Asp Leu Leu
305                 310                 315                 320
gcc aag aag aag atg aac ctt gac aac tgc agg tac tta acc tta gat 1008
Ala Lys Lys Lys Met Asn Leu Asp Asn Cys Arg Tyr Leu Thr Leu Asp
                325                 330                 335
gaa gcg gat agg ctg gtt gac cta gga ttt gaa gat gac atc aag gag 1056
Glu Ala Asp Arg Leu Val Asp Leu Gly Phe Glu Asp Asp Ile Lys Glu
            340                 345                 350
gtt ttc gac cat ttc aag gat caa agg caa act ctt ctc ttt tct gct 1104
Val Phe Asp His Phe Lys Asp Gln Arg Gln Thr Leu Leu Phe Ser Ala
        355                 360                 365
act atg cct cag aag att caa aat ttt gcg aag aac gcc ctt gtg aaa 1152
Thr Met Pro Gln Lys Ile Gln Asn Phe Ala Lys Asn Ala Leu Val Lys
    370                 375                 380
cca gtt act gtc aat gtg gga aga gct gga gca gca aat cta gac gtc 1200
Pro Val Thr Val Asn Val Gly Arg Ala Gly Ala Ala Asn Leu Asp Val
385                 390                 395                 400
att caa gaa gtc gaa tat gta aag gaa gat gct aga att ata tac ctc 1248
Ile Gln Glu Val Glu Tyr Val Lys Glu Asp Ala Arg Ile Ile Tyr Leu
                405                 410                 415
ctt gaa tgc ctc caa aag act cca cct cct gtt ctc att ttc tgt gaa 1296
Leu Glu Cys Leu Gln Lys Thr Pro Pro Pro Val Leu Ile Phe Cys Glu
            420                 425                 430
aac aag gct gat gtc gac tac atc cat gag tac ctt ctt cta aag gga 1344
Asn Lys Ala Asp Val Asp Tyr Ile His Glu Tyr Leu Leu Leu Lys Gly
        435                 440                 445
gtg gag gct gtt gct atc cat gga gga aaa gat caa gag gag aga cag 1392
Val Glu Ala Val Ala Ile His Gly Gly Lys Asp Gln Glu Glu Arg Gln
    450                 455                 460
aac gca att gac tcc ttc aag act ggt agg aag gat gtt ctg gtg gct 1440
Asn Ala Ile Asp Ser Phe Lys Thr Gly Arg Lys Asp Val Leu Val Ala
465                 470                 475                 480
act gat gtt gca tcg aag ggt ctt gat ttt cct gac att cag cat gtc 1488
Thr Asp Val Ala Ser Lys Gly Leu Asp Phe Pro Asp Ile Gln His Val
                485                 490                 495
att aac tat gac atg ccc gcc gaa ata gag aac tat gtc cac cga atc 1536
Ile Asn Tyr Asp Met Pro Ala Glu Ile Glu Asn Tyr Val His Arg Ile
            500                 505                 510
ggt cga act ggt cga tgt ggg aag act ggc ata gca act acc ttc atc 1584
Gly Arg Thr Gly Arg Cys Gly Lys Thr Gly Ile Ala Thr Thr Phe Ile
        515                 520                 525
aac aag aac caa act gag acc acg ctt ctc gat ctc aag cac ctg ctg 1632
Asn Lys Asn Gln Thr Glu Thr Thr Leu Leu Asp Leu Lys His Leu Leu
    530                 535                 540
aag gag gca aag cag agg att cca ccg gtg ttg gcg gag ctc aac gat 1680
Lys Glu Ala Lys Gln Arg Ile Pro Pro Val Leu Ala Glu Leu Asn Asp
545                 550                 555                 560
cca ttg gat gat gag gaa ata att gcc aag gag agt ggt gtc aaa ggt 1728
Pro Leu Asp Asp Glu Glu Ile Ile Ala Lys Glu Ser Gly Val Lys Gly
                565                 570                 575
tgt gcg tat tgt ggt ggg ctt ggc cat cgt gtc agt gac tgt ccg aag 1776
Cys Ala Tyr Cys Gly Gly Leu Gly His Arg Val Ser Asp Cys Pro Lys
            580                 585                 590
ttg gag cac caa aag tcc atg gca att gct ggc tcg aga aaa gac tac 1824
Leu Glu His Gln Lys Ser Met Ala Ile Ala Gly Ser Arg Lys Asp Tyr
        595                 600                 605

ttt ggc ggc ggg ggt tac cga gga gaa ata tga                                1857
Phe Gly Gly Gly Gly Tyr Arg Gly Glu Ile
    610                 615

<210> 132
<211> 618
<212> PRT
<213> Zea mays

<400> 132
Met Ser Ala Ile Ala Ala Ser Ser Ala Ala Ala Ala Ala Ala Ser Asn
1               5                   10                  15
Ser Asp Glu Glu Glu Asn Tyr Glu Glu Tyr Ile Pro Val Ala Lys Arg
            20                  25                  30
Arg Ala Met Glu Ala Glu Arg Leu Arg Gln Leu Arg Leu Ser Lys Ser
        35                  40                  45
Ala Pro Ala Ser Ala Val Ser Ser Leu Pro Met Pro Pro Pro Pro Pro
    50                  55                  60
Leu Leu Pro Ala Gln Thr Ser Val Pro Asp Ala Ala Ala Lys Pro Ser
65                  70                  75                  80
Leu Leu Val Lys Ala Thr Gln Leu Lys Arg Ala Ala Pro Glu Val Thr
                85                  90                  95
Ala Thr Glu Gln Arg Ile Gln Gln Glu Lys Glu Val Ile Glu Asn Leu
            100                 105                 110
Ser Asp Asn Lys Ser Leu Arg Ser Val Arg Glu Ile Ala Lys Gly Ile
        115                 120                 125
Ile Tyr Thr Glu Pro Ile Gln Thr Gly Trp Lys Pro Pro Leu Arg Leu
    130                 135                 140
Arg Arg Met Pro Leu Ala Lys Ala Asn Glu Phe Arg Arg Lys Trp His
145                 150                 155                 160
Ile Ile Val Asp Gly Asp Asp Ile Pro Pro Pro Ala Arg Asp Phe Arg
                165                 170                 175
Asp Leu Arg Leu Pro Glu Pro Met Leu Arg Lys Leu Arg Glu Arg Gly
            180                 185                 190
Ile Val Gln Pro Thr Pro Ile Gln Val Gln Gly Leu Pro Val Val Leu
        195                 200                 205
Ser Gly Arg Asp Met Ile Gly Ile Ala Phe Thr Gly Ser Gly Lys Thr
    210                 215                 220
Leu Val Phe Val Leu Pro Leu Ile Met Val Ala Leu Gln Glu Glu Ser
225                 230                 235                 240
Leu Met Pro Ile Val Pro Gly Glu Gly Pro Phe Gly Met Ile Ile Cys
                245                 250                 255
Pro Ser Arg Glu Leu Ala Lys Gln Thr Tyr Asp Val Ile Glu Gln Phe
            260                 265                 270
Leu Ile Pro Leu Lys Glu Ala Gly Tyr Pro Glu Ile Arg His Leu Leu
        275                 280                 285
Cys Ile Gly Gly Val Asp Met Arg Thr Gln Leu Asp Val Leu Lys Lys
    290                 295                 300
Gly Val His Ile Val Val Ala Thr Pro Gly Arg Leu Lys Asp Leu Leu
305                 310                 315                 320
Ala Lys Lys Lys Met Asn Leu Asp Asn Cys Arg Tyr Leu Thr Leu Asp
                325                 330                 335
Glu Ala Asp Arg Leu Val Asp Leu Gly Phe Glu Asp Asp Ile Lys Glu
            340                 345                 350
Val Phe Asp His Phe Lys Asp Gln Arg Gln Thr Leu Leu Phe Ser Ala
        355                 360                 365
Thr Met Pro Gln Lys Ile Gln Asn Phe Ala Lys Asn Ala Leu Val Lys
    370                 375                 380
Pro Val Thr Val Asn Val Gly Arg Ala Gly Ala Ala Asn Leu Asp Val
385                 390                 395                 400
Ile Gln Glu Val Glu Tyr Val Lys Glu Asp Ala Arg Ile Ile Tyr Leu
                405                 410                 415
Leu Glu Cys Leu Gln Lys Thr Pro Pro Pro Val Leu Ile Phe Cys Glu
            420                 425                 430
Asn Lys Ala Asp Val Asp Tyr Ile His Glu Tyr Leu Leu Leu Lys Gly
        435                 440                 445
Val Glu Ala Val Ala Ile His Gly Gly Lys Asp Gln Glu Glu Arg Gln
    450                 455                 460
Asn Ala Ile Asp Ser Phe Lys Thr Gly Arg Lys Asp Val Leu Val Ala

465                 470                 475                 480
Thr Asp Val Ala Ser Lys Gly Leu Asp Phe Pro Asp Ile Gln His Val
                485                 490                 495
Ile Asn Tyr Asp Met Pro Ala Glu Ile Glu Asn Tyr Val His Arg Ile
            500                 505                 510
Gly Arg Thr Gly Arg Cys Gly Lys Thr Gly Ile Ala Thr Thr Phe Ile
        515                 520                 525
Asn Lys Asn Gln Thr Glu Thr Thr Leu Leu Asp Leu Lys His Leu Leu
    530                 535                 540
Lys Glu Ala Lys Gln Arg Ile Pro Pro Val Leu Ala Glu Leu Asn Asp
545                 550                 555                 560
Pro Leu Asp Asp Glu Glu Ile Ile Ala Lys Glu Ser Gly Val Lys Gly
                565                 570                 575
Cys Ala Tyr Cys Gly Gly Leu Gly His Arg Val Ser Asp Cys Pro Lys
            580                 585                 590
Leu Glu His Gln Lys Ser Met Ala Ile Ala Gly Ser Arg Lys Asp Tyr
        595                 600                 605
Phe Gly Gly Gly Gly Tyr Arg Gly Glu Ile
    610                 615

<210> 133
<211> 1722
<212> DNA
<213> Zea mays

<220>
<221> CDS
<222> (1)..(1722)

<400> 133
atg agg cta agt att ggg caa gtc cat cga aat gtt ctt gcg ctg gca       48
Met Arg Leu Ser Ile Gly Gln Val His Arg Asn Val Leu Ala Leu Ala
1               5                   10                  15
tct tct cgg tca tgt ttt gtt ctg gga gat cgt gtt tcg ttc agg atg       96
Ser Ser Arg Ser Cys Phe Val Leu Gly Asp Arg Val Ser Phe Arg Met
            20                  25                  30
ctg tcc caa cca cgg gta gct gga ttt cat cag act gct tgg cgt ggt      144
Leu Ser Gln Pro Arg Val Ala Gly Phe His Gln Thr Ala Trp Arg Gly
        35                  40                  45
agt caa att ttg gaa gat aac agg gga gga cca ttg act ctt gct agc      192
Ser Gln Ile Leu Glu Asp Asn Arg Gly Gly Pro Leu Thr Leu Ala Ser
    50                  55                  60
cta gaa gtg caa agc agg gtt gaa tat gga aaa aag ggg aaa atg gaa      240
Leu Glu Val Gln Ser Arg Val Glu Tyr Gly Lys Lys Gly Lys Met Glu
65                  70                  75                  80
cga act gga ggt cca aag cca agt tca aga gct tct tcc ctc tat gtg      288
Arg Thr Gly Gly Pro Lys Pro Ser Ser Arg Ala Ser Ser Leu Tyr Val
                85                  90                  95
aaa cca aag gtt tct tca aat gtc aaa cca gca aag agt gcc ttg atg      336
Lys Pro Lys Val Ser Ser Asn Val Lys Pro Ala Lys Ser Ala Leu Met
            100                 105                 110
aag cca gct ggg ata aag aag aca ctg aag gta gat gag gct ctg ttt      384
Lys Pro Ala Gly Ile Lys Lys Thr Leu Lys Val Asp Glu Ala Leu Phe
        115                 120                 125
tcg gcc aag tca ttt gaa gaa ctt ggt ttg cca cct ttg ctt gtt gac      432
Ser Ala Lys Ser Phe Glu Glu Leu Gly Leu Pro Pro Leu Leu Val Asp
    130                 135                 140
cag ttg aac aag gaa ggt ctg act gcc cca act gag gtc caa tcc gca      480
Gln Leu Asn Lys Glu Gly Leu Thr Ala Pro Thr Glu Val Gln Ser Ala
145                 150                 155                 160
gca att cct ata ata gca caa aag cat gat gtt gtg atc cag tca tat      528
Ala Ile Pro Ile Ile Ala Gln Lys His Asp Val Val Ile Gln Ser Tyr
                165                 170                 175
act ggc tca ggg aaa act ctt gct tat ctt ctt cca att ctt tct gat      576
Thr Gly Ser Gly Lys Thr Leu Ala Tyr Leu Leu Pro Ile Leu Ser Asp
            180                 185                 190
ata ggc cct ctg aag agg act atg gaa caa gac cat tct gag aaa aga      624
Ile Gly Pro Leu Lys Arg Thr Met Glu Gln Asp His Ser Glu Lys Arg
        195                 200                 205

tca ggt ata gaa gca gtc atc gtt gct cct tcg aga gaa tta gga atg 672
Ser Gly Ile Glu Ala Val Ile Val Ala Pro Ser Arg Glu Leu Gly Met
    210                 215                 220
cag att gtg cga gaa gtg gag aag att ttg ggt cct aat gac aaa aga 720
Gln Ile Val Arg Glu Val Glu Lys Ile Leu Gly Pro Asn Asp Lys Arg
225                 230                 235                 240
ctg gtc cag caa ctt gtt ggc gga gct aat cgt tcc agg cag gaa gaa 768
Leu Val Gln Gln Leu Val Gly Gly Ala Asn Arg Ser Arg Gln Glu Glu
                245                 250                 255
gca ttg aag aag aac aaa cca atc att gtt gtc gga aca cct ggc cgt 816
Ala Leu Lys Lys Asn Lys Pro Ile Ile Val Val Gly Thr Pro Gly Arg
            260                 265                 270
att tct gaa att tct gca gca ggt aag cta cac act cat ggc tgc cgt 864
Ile Ser Glu Ile Ser Ala Ala Gly Lys Leu His Thr His Gly Cys Arg
        275                 280                 285
ttt ctt gta ctg gat gaa gtc gac cag ctt ctg tct ttt aat tac cgt 912
Phe Leu Val Leu Asp Glu Val Asp Gln Leu Leu Ser Phe Asn Tyr Arg
    290                 295                 300
gaa gat atg cat aga att ttg gag cat gtt gga agg aga cct gga ggc 960
Glu Asp Met His Arg Ile Leu Glu His Val Gly Arg Arg Pro Gly Gly
305                 310                 315                 320
aca tct agg gat att ctt ggc cca ctt gcg aga cga tct gag cgt cag 1008
Thr Ser Arg Asp Ile Leu Gly Pro Leu Ala Arg Arg Ser Glu Arg Gln
                325                 330                 335
act atc ctg gtt tct gca aca ata cca ttt tca gtt ata cga gca gca 1056
Thr Ile Leu Val Ser Ala Thr Ile Pro Phe Ser Val Ile Arg Ala Ala
            340                 345                 350
agg agt tgg ggt cat gat cca gtt ctc att aga gct aaa agt gta gtt 1104
Arg Ser Trp Gly His Asp Pro Val Leu Ile Arg Ala Lys Ser Val Val
        355                 360                 365
cca ctt gat tca atc act gtg cca aga cct gcg tta tcc caa agt gac 1152
Pro Leu Asp Ser Ile Thr Val Pro Arg Pro Ala Leu Ser Gln Ser Asp
    370                 375                 380
gct aac ccc agt tcg tca tcg cag tca gtg aac caa gct gct gtt ggc 1200
Ala Asn Pro Ser Ser Ser Ser Gln Ser Val Asn Gln Ala Ala Val Gly
385                 390                 395                 400
agc ttg ccg cca tct ttg gaa cac tac tat tgt acg gcc aag gcg cag 1248
Ser Leu Pro Pro Ser Leu Glu His Tyr Tyr Cys Thr Ala Lys Ala Gln
                405                 410                 415
cac aag gtt gac aca tta cgg agg tgc atc cat gct ctg gaa gca cag 1296
His Lys Val Asp Thr Leu Arg Arg Cys Ile His Ala Leu Glu Ala Gln
            420                 425                 430
aca gtg att gca ttt atg aac aac acc aag cca ctg aag gat gtt gtg 1344
Thr Val Ile Ala Phe Met Asn Asn Thr Lys Pro Leu Lys Asp Val Val
        435                 440                 445
ttt aag ttg gag gcc cgt ggt atc aaa gcc act gag cta cat gga gac 1392
Phe Lys Leu Glu Ala Arg Gly Ile Lys Ala Thr Glu Leu His Gly Asp
    450                 455                 460
ctt gga aag ctt gca agg tcg aca gtt ttg aaa aag ttc aag gat ggt 1440
Leu Gly Lys Leu Ala Arg Ser Thr Val Leu Lys Lys Phe Lys Asp Gly
465                 470                 475                 480
gaa ttc aga gtt ctc gtt acg aat gag ctg tct gcc aga gga ctg gac 1488
Glu Phe Arg Val Leu Val Thr Asn Glu Leu Ser Ala Arg Gly Leu Asp
                485                 490                 495
gtg cca gaa tgt gac ctt gtg gtt aat ctg gac ctt cca act gac tcc 1536
Val Pro Glu Cys Asp Leu Val Val Asn Leu Asp Leu Pro Thr Asp Ser
            500                 505                 510
aca cat tat gct cac aga gct gga aga act gga cgt ctt ggg cgg aaa 1584
Thr His Tyr Ala His Arg Ala Gly Arg Thr Gly Arg Leu Gly Arg Lys
        515                 520                 525
ggg atc gtg gtc aca ata tgc gaa gaa agc gag gga ttt gtt gta aga 1632
Gly Ile Val Val Thr Ile Cys Glu Glu Ser Glu Gly Phe Val Val Arg
    530                 535                 540
aaa atg cgc aag cag tta gat gtg gcc atc aag cca tgc gag ttc act 1680
Lys Met Arg Lys Gln Leu Asp Val Ala Ile Lys Pro Cys Glu Phe Thr
545                 550                 555                 560
gaa ggc gag ctt gtt gtc cac aag gag gaa gat gtg gaa taa 1722
Glu Gly Glu Leu Val Val His Lys Glu Glu Asp Val Glu
                565                 570

<210> 134
<211> 573
<212> PRT
<213> Zea mays

<400> 134
Met Arg Leu Ser Ile Gly Gln Val His Arg Asn Val Leu Ala Leu Ala
1               5                   10                  15
Ser Ser Arg Ser Cys Phe Val Leu Gly Asp Arg Val Ser Phe Arg Met
            20                  25                  30
Leu Ser Gln Pro Arg Val Ala Gly Phe His Gln Thr Ala Trp Arg Gly
        35                  40                  45
Ser Gln Ile Leu Glu Asp Asn Arg Gly Gly Pro Leu Thr Leu Ala Ser
    50                  55                  60
Leu Glu Val Gln Ser Arg Val Glu Tyr Gly Lys Lys Gly Lys Met Glu
65                  70                  75                  80
Arg Thr Gly Gly Pro Lys Pro Ser Ser Arg Ala Ser Ser Leu Tyr Val
                85                  90                  95
Lys Pro Lys Val Ser Ser Asn Val Lys Pro Ala Lys Ser Ala Leu Met
            100                 105                 110
Lys Pro Ala Gly Ile Lys Lys Thr Leu Lys Val Asp Glu Ala Leu Phe
        115                 120                 125
Ser Ala Lys Ser Phe Glu Glu Leu Gly Leu Pro Pro Leu Leu Val Asp
    130                 135                 140
Gln Leu Asn Lys Glu Gly Leu Thr Ala Pro Thr Glu Val Gln Ser Ala
145                 150                 155                 160
Ala Ile Pro Ile Ile Ala Gln Lys His Asp Val Val Ile Gln Ser Tyr
                165                 170                 175
Thr Gly Ser Gly Lys Thr Leu Ala Tyr Leu Leu Pro Ile Leu Ser Asp
            180                 185                 190
Ile Gly Pro Leu Lys Arg Thr Met Glu Gln Asp His Ser Glu Lys Arg
        195                 200                 205
Ser Gly Ile Glu Ala Val Ile Val Ala Pro Ser Arg Glu Leu Gly Met
    210                 215                 220
Gln Ile Val Arg Glu Val Glu Lys Ile Leu Gly Pro Asn Asp Lys Arg
225                 230                 235                 240
Leu Val Gln Gln Leu Val Gly Gly Ala Asn Arg Ser Arg Gln Glu Glu
                245                 250                 255
Ala Leu Lys Lys Asn Lys Pro Ile Ile Val Val Gly Thr Pro Gly Arg
            260                 265                 270
Ile Ser Glu Ile Ser Ala Ala Gly Lys Leu His Thr His Gly Cys Arg
        275                 280                 285
Phe Leu Val Leu Asp Glu Val Asp Gln Leu Leu Ser Phe Asn Tyr Arg
    290                 295                 300
Glu Asp Met His Arg Ile Leu Glu His Val Gly Arg Arg Pro Gly Gly
305                 310                 315                 320
Thr Ser Arg Asp Ile Leu Gly Pro Leu Ala Arg Arg Ser Glu Arg Gln
                325                 330                 335
Thr Ile Leu Val Ser Ala Thr Ile Pro Phe Ser Val Ile Arg Ala Ala
            340                 345                 350
Arg Ser Trp Gly His Asp Pro Val Leu Ile Arg Ala Lys Ser Val Val
        355                 360                 365
Pro Leu Asp Ser Ile Thr Val Pro Arg Pro Ala Leu Ser Gln Ser Asp
    370                 375                 380
Ala Asn Pro Ser Ser Ser Ser Gln Ser Val Asn Gln Ala Ala Val Gly
385                 390                 395                 400
Ser Leu Pro Pro Ser Leu Glu His Tyr Tyr Cys Thr Ala Lys Ala Gln
                405                 410                 415
His Lys Val Asp Thr Leu Arg Arg Cys Ile His Ala Leu Glu Ala Gln
            420                 425                 430
Thr Val Ile Ala Phe Met Asn Asn Thr Lys Pro Leu Lys Asp Val Val
        435                 440                 445
Phe Lys Leu Glu Ala Arg Gly Ile Lys Ala Thr Glu Leu His Gly Asp
    450                 455                 460
Leu Gly Lys Leu Ala Arg Ser Thr Val Leu Lys Lys Phe Lys Asp Gly
465                 470                 475                 480
Glu Phe Arg Val Leu Val Thr Asn Glu Leu Ser Ala Arg Gly Leu Asp
                485                 490                 495

Val Pro Glu Cys Asp Leu Val Val Asn Leu Asp Leu Pro Thr Asp Ser
            500                 505                 510
Thr His Tyr Ala His Arg Ala Gly Arg Thr Gly Arg Leu Gly Arg Lys
        515                 520                 525
Gly Ile Val Val Thr Ile Cys Glu Glu Ser Glu Gly Phe Val Val Arg
    530                 535                 540
Lys Met Arg Lys Gln Leu Asp Val Ala Ile Lys Pro Cys Glu Phe Thr
545                 550                 555                 560
Glu Gly Glu Leu Val Val His Lys Glu Glu Asp Val Glu
                565                 570

<210> 135
<211> 1224
<212> DNA
<213> Zea mays

<220>
<221> CDS
<222> (1)..(1224)

<400> 135
atg gcg gcg ccc acc acc tcc cgc cgc ggc ccc ggc ggc gcg cgc aac      48
Met Ala Ala Pro Thr Thr Ser Arg Arg Gly Pro Gly Gly Ala Arg Asn
1               5                   10                  15
atg gac gac gag aac ctc acc ttc gag acc tcc ccg ggg gtc gag gtc      96
Met Asp Asp Glu Asn Leu Thr Phe Glu Thr Ser Pro Gly Val Glu Val
            20                  25                  30
gtc agc agc ttc gac cag atg ggc atc aag gac gac ctc ctc cgc ggc      144
Val Ser Ser Phe Asp Gln Met Gly Ile Lys Asp Asp Leu Leu Arg Gly
        35                  40                  45
atc tac ggc tac ggt ttc gag aag ccc tcc gcc atc cag cag cgc gcc      192
Ile Tyr Gly Tyr Gly Phe Glu Lys Pro Ser Ala Ile Gln Gln Arg Ala
    50                  55                  60
gtg ctc ccc atc atc aac ggc cgc gac gtc atc gcg cag gcc cag tcc      240
Val Leu Pro Ile Ile Asn Gly Arg Asp Val Ile Ala Gln Ala Gln Ser
65                  70                  75                  80
ggt acc ggc aag acg tcc atg atc tcc ctc acc gtc tgc cag atc gtc      288
Gly Thr Gly Lys Thr Ser Met Ile Ser Leu Thr Val Cys Gln Ile Val
                85                  90                  95
gac acc gcc gtg cgc gag gtg cag gct ttg att ctc tcg cct act agg      336
Asp Thr Ala Val Arg Glu Val Gln Ala Leu Ile Leu Ser Pro Thr Arg
            100                 105                 110
gag ctt gct tcc caa aca gag aga gtt atg ctg gct att ggg gac cac      384
Glu Leu Ala Ser Gln Thr Glu Arg Val Met Leu Ala Ile Gly Asp His
        115                 120                 125
ctc aac gtc caa gtg cac gct tgc att ggt gga aag agt att ggt gag      432
Leu Asn Val Gln Val His Ala Cys Ile Gly Gly Lys Ser Ile Gly Glu
    130                 135                 140
gac atc agg agg ctc gag aat ggg gtg cat gtc gtc tct gga act cca      480
Asp Ile Arg Arg Leu Glu Asn Gly Val His Val Val Ser Gly Thr Pro
145                 150                 155                 160
ggc aga gtc tgt gat atg atc aag agg agg aca ctg cgg acc aga gcc      528
Gly Arg Val Cys Asp Met Ile Lys Arg Arg Thr Leu Arg Thr Arg Ala
                165                 170                 175
atc aag ctt cta gtt ctg gat gaa gct gat gag atg ttg agt aga ggc      576
Ile Lys Leu Leu Val Leu Asp Glu Ala Asp Glu Met Leu Ser Arg Gly
            180                 185                 190
ttt aag gat cag att tac gat gtc tac aga tat ctc cct cca gaa ctt      624
Phe Lys Asp Gln Ile Tyr Asp Val Tyr Arg Tyr Leu Pro Pro Glu Leu
        195                 200                 205
cag gtg gtt ttg atc tct gca act ctt cct cat gag atc ttg gag atg      672
Gln Val Val Leu Ile Ser Ala Thr Leu Pro His Glu Ile Leu Glu Met
    210                 215                 220
act agt aag ttc atg acc gaa ccg gtt agg atc ctt gtg aag cgt gat      720
Thr Ser Lys Phe Met Thr Glu Pro Val Arg Ile Leu Val Lys Arg Asp
225                 230                 235                 240
gag ctg acc ctg gag ggc atc aaa caa ttc ttt gtt gct gtt gag aaa      768
Glu Leu Thr Leu Glu Gly Ile Lys Gln Phe Phe Val Ala Val Glu Lys
                245                 250                 255

gag gag tgg aag ttt gat aca ctt tgt gat ctt tat gat aca ctc acc 816
Glu Glu Trp Lys Phe Asp Thr Leu Cys Asp Leu Tyr Asp Thr Leu Thr
            260                 265                 270
atc acc caa gct gtt att ttc tgc aat act aag aga aag gtg gat tgg 864
Ile Thr Gln Ala Val Ile Phe Cys Asn Thr Lys Arg Lys Val Asp Trp
        275                 280                 285
ctc act gaa aaa atg cgt aca aat aac ttc acc gta tca gct atg cac 912
Leu Thr Glu Lys Met Arg Thr Asn Asn Phe Thr Val Ser Ala Met His
    290                 295                 300
ggt gac atg ccc caa caa gaa agg gat gcc att atg ggt gag ttc agg 960
Gly Asp Met Pro Gln Gln Glu Arg Asp Ala Ile Met Gly Glu Phe Arg
305                 310                 315                 320
tct ggc gca act cgt gtg cta ata act aca gat gtt tgg gct cga gga 1008
Ser Gly Ala Thr Arg Val Leu Ile Thr Thr Asp Val Trp Ala Arg Gly
                325                 330                 335
ctg gac gtt cag cag gtt tca ctt gtc ata aat tat gat ctc cca aat 1056
Leu Asp Val Gln Gln Val Ser Leu Val Ile Asn Tyr Asp Leu Pro Asn
            340                 345                 350
aat cgt gag ctt tac atc cat cgc att ggt cgt tct ggt cgt ttt ggg 1104
Asn Arg Glu Leu Tyr Ile His Arg Ile Gly Arg Ser Gly Arg Phe Gly
        355                 360                 365
cgc aag ggt gtg gcg atc aat ttc gtg cgc aag gat gac atc cgt ata 1152
Arg Lys Gly Val Ala Ile Asn Phe Val Arg Lys Asp Asp Ile Arg Ile
    370                 375                 380
ctg aga gat ata gag cag tac tac agc aca caa att gac gag atg cca 1200
Leu Arg Asp Ile Glu Gln Tyr Tyr Ser Thr Gln Ile Asp Glu Met Pro
385                 390                 395                 400
atg aat gtt gct gat ctt att tga 1224
Met Asn Val Ala Asp Leu Ile
                405

<210> 136
<211> 407
<212> PRT
<213> Zea mays

<400> 136
Met Ala Ala Pro Thr Thr Ser Arg Arg Gly Pro Gly Gly Ala Arg Asn
1               5                   10                  15
Met Asp Asp Glu Asn Leu Thr Phe Glu Thr Ser Pro Gly Val Glu Val
            20                  25                  30
Val Ser Ser Phe Asp Gln Met Gly Ile Lys Asp Asp Leu Leu Arg Gly
        35                  40                  45
Ile Tyr Gly Tyr Gly Phe Glu Lys Pro Ser Ala Ile Gln Gln Arg Ala
    50                  55                  60
Val Leu Pro Ile Ile Asn Gly Arg Asp Val Ile Ala Gln Ala Gln Ser
65                  70                  75                  80
Gly Thr Gly Lys Thr Ser Met Ile Ser Leu Thr Val Cys Gln Ile Val
                85                  90                  95
Asp Thr Ala Val Arg Glu Val Gln Ala Leu Ile Leu Ser Pro Thr Arg
            100                 105                 110
Glu Leu Ala Ser Gln Thr Glu Arg Val Met Leu Ala Ile Gly Asp His
        115                 120                 125
Leu Asn Val Gln Val His Ala Cys Ile Gly Gly Lys Ser Ile Gly Glu
    130                 135                 140
Asp Ile Arg Arg Leu Glu Asn Gly Val His Val Val Ser Gly Thr Pro
145                 150                 155                 160
Gly Arg Val Cys Asp Met Ile Lys Arg Arg Thr Leu Arg Thr Arg Ala
                165                 170                 175
Ile Lys Leu Leu Val Leu Asp Glu Ala Asp Glu Met Leu Ser Arg Gly
            180                 185                 190
Phe Lys Asp Gln Ile Tyr Asp Val Tyr Arg Tyr Leu Pro Pro Glu Leu
        195                 200                 205
Gln Val Val Leu Ile Ser Ala Thr Leu Pro His Glu Ile Leu Glu Met
    210                 215                 220
Thr Ser Lys Phe Met Thr Glu Pro Val Arg Ile Leu Val Lys Arg Asp
225                 230                 235                 240
Glu Leu Thr Leu Glu Gly Ile Lys Gln Phe Phe Val Ala Val Glu Lys
                245                 250                 255

Glu Glu Trp Lys Phe Asp Thr Leu Cys Asp Leu Tyr Asp Thr Leu Thr
            260                 265                 270
Ile Thr Gln Ala Val Ile Phe Cys Asn Thr Lys Arg Lys Val Asp Trp
        275                 280                 285
Leu Thr Glu Lys Met Arg Thr Asn Asn Phe Thr Val Ser Ala Met His
    290                 295                 300
Gly Asp Met Pro Gln Gln Glu Arg Asp Ala Ile Met Gly Glu Phe Arg
305                 310                 315                 320
Ser Gly Ala Thr Arg Val Leu Ile Thr Thr Asp Val Trp Ala Arg Gly
                325                 330                 335
Leu Asp Val Gln Gln Val Ser Leu Val Ile Asn Tyr Asp Leu Pro Asn
            340                 345                 350
Asn Arg Glu Leu Tyr Ile His Arg Ile Gly Arg Ser Gly Arg Phe Gly
        355                 360                 365
Arg Lys Gly Val Ala Ile Asn Phe Val Arg Lys Asp Asp Ile Arg Ile
    370                 375                 380
Leu Arg Asp Ile Glu Gln Tyr Tyr Ser Thr Gln Ile Asp Glu Met Pro
385                 390                 395                 400
Met Asn Val Ala Asp Leu Ile
                405

<210> 137
<211> 2127
<212> DNA
<213> Zea mays

<220>
<221> CDS
<222> (1)..(2127)

<400> 137
atg tct ccc gcc ctt gcc gct ggc gtg gag ccc atg gcc gtc gac gat        48
Met Ser Pro Ala Leu Ala Ala Gly Val Glu Pro Met Ala Val Asp Asp
1               5                   10                  15
tcc gcc tcc aag aag gcc aag cgc aag cag ctc aag gcc gcc gcc gcc        96
Ser Ala Ser Lys Lys Ala Lys Arg Lys Gln Leu Lys Ala Ala Ala Ala
            20                  25                  30
gcc gcg gag gca gag gcg gag gcc gct tcg gtc aag aag aaa gag aag       144
Ala Ala Glu Ala Glu Ala Glu Ala Ala Ser Val Lys Lys Lys Glu Lys
        35                  40                  45
aaa gag aag aag cga aag gtg aag gag ccg tcc cct ccg ctc cca tcg       192
Lys Glu Lys Lys Arg Lys Val Lys Glu Pro Ser Pro Pro Leu Pro Ser
    50                  55                  60
gcc gcg tcc tct gat gag gaa gag aaa agc agc acc agc tcc gag gag       240
Ala Ala Ser Ser Asp Glu Glu Glu Lys Ser Ser Thr Ser Ser Glu Glu
65                  70                  75                  80
aca gca ccc gcg gcg aaa aaa gcg aag aag gag aag acg aag aag aat       288
Thr Ala Pro Ala Ala Lys Lys Ala Lys Lys Glu Lys Thr Lys Lys Asn
                85                  90                  95
gtt gat gtc tcg tct tca gcc tca gat gac gat ggc gag atc aca gcc       336
Val Asp Val Ser Ser Ser Ala Ser Asp Asp Asp Gly Glu Ile Thr Ala
            100                 105                 110
agc agc gat gag gac ccc gcg gac ccg aac gcg ctg acg aac ttc agg       384
Ser Ser Asp Glu Asp Pro Ala Asp Pro Asn Ala Leu Thr Asn Phe Arg
        115                 120                 125
ata tcg gag tcg ctg agg cag agc ctc agg tcc aag ggg atc aag tca       432
Ile Ser Glu Ser Leu Arg Gln Ser Leu Arg Ser Lys Gly Ile Lys Ser
    130                 135                 140
ctg ttc ccc att cag gcc acc act ttc gat ctc gta ctc gat ggc agc       480
Leu Phe Pro Ile Gln Ala Thr Thr Phe Asp Leu Val Leu Asp Gly Ser
145                 150                 155                 160
gac ttg gtt ggc cga gcg cgc acc ggt cag gga aaa act ttg gct ttt       528
Asp Leu Val Gly Arg Ala Arg Thr Gly Gln Gly Lys Thr Leu Ala Phe
                165                 170                 175
gtc ttg ccc ata ttg gag tct ttg gtt aat ggg gca aac aag gca tct       576
Val Leu Pro Ile Leu Glu Ser Leu Val Asn Gly Ala Asn Lys Ala Ser
            180                 185                 190
aga cgg act gaa cat ggc agg acc cca agt gtt ctc gtt cta cta cca       624
Arg Arg Thr Glu His Gly Arg Thr Pro Ser Val Leu Val Leu Leu Pro

        195                 200                 205
aca aga gag ctg gcc aat cag gtg cac gct gac ttt gag ttt tat ggt 672
Thr Arg Glu Leu Ala Asn Gln Val His Ala Asp Phe Glu Phe Tyr Gly
    210                 215                 220
gca aca ttt ggg ctc tct gca tgt tgt gtg tat ggg gga tca cct tat 720
Ala Thr Phe Gly Leu Ser Ala Cys Cys Val Tyr Gly Gly Ser Pro Tyr
225                 230                 235                 240
cgt cct caa gaa atg gca ttg aga aga ggt gtg gat att gtt gtt gga 768
Arg Pro Gln Glu Met Ala Leu Arg Arg Gly Val Asp Ile Val Val Gly
                245                 250                 255
act cct ggt cgt gtc aag gat ttc att gtt aaa gga act ctc aat ttg 816
Thr Pro Gly Arg Val Lys Asp Phe Ile Val Lys Gly Thr Leu Asn Leu
            260                 265                 270
aaa tgc ttg aaa ttc cgt gtc ctt gat gaa gct gat gag atg cta aac 864
Lys Cys Leu Lys Phe Arg Val Leu Asp Glu Ala Asp Glu Met Leu Asn
        275                 280                 285
atg ggt ttt gtt gat gat gtc gag ctc att ctt ggc aag gta gaa gat 912
Met Gly Phe Val Asp Asp Val Glu Leu Ile Leu Gly Lys Val Glu Asp
    290                 295                 300
gct acc aaa gta cag aca ctt ctg ttc agt gcc act ctg cca gat tgg 960
Ala Thr Lys Val Gln Thr Leu Leu Phe Ser Ala Thr Leu Pro Asp Trp
305                 310                 315                 320
gtg aat aag ctc tct atg agg ttt ctg aaa gtt gac agg aaa aca gtt 1008
Val Asn Lys Leu Ser Met Arg Phe Leu Lys Val Asp Arg Lys Thr Val
                325                 330                 335
gat ctt gtc ggt aac gag aaa ctg aag gcc agt gca tct gtt aag cac 1056
Asp Leu Val Gly Asn Glu Lys Leu Lys Ala Ser Ala Ser Val Lys His
            340                 345                 350
ctt gct ctt cct tgt aac aag gca gca agg gca caa ctt att cca gat 1104
Leu Ala Leu Pro Cys Asn Lys Ala Ala Arg Ala Gln Leu Ile Pro Asp
        355                 360                 365
att atc cga tgt tac agc cat gga ggc cga acc att atc ttc act gag 1152
Ile Ile Arg Cys Tyr Ser His Gly Gly Arg Thr Ile Ile Phe Thr Glu
    370                 375                 380
acg aag gat tct gca tca gag ctt tct ggt ttg att cct gga tcc cgt 1200
Thr Lys Asp Ser Ala Ser Glu Leu Ser Gly Leu Ile Pro Gly Ser Arg
385                 390                 395                 400
gcc ttg cat gga gat gtt gtg caa gct cag cgt gaa gtc att ctt gct 1248
Ala Leu His Gly Asp Val Val Gln Ala Gln Arg Glu Val Ile Leu Ala
                405                 410                 415
gga ttt cgt agc ggg aag ttc cag gtt ttg gtt gct aca aat gtg gcg 1296
Gly Phe Arg Ser Gly Lys Phe Gln Val Leu Val Ala Thr Asn Val Ala
            420                 425                 430
gct cgt ggt ctg gat att aat gat gtg cag ctt atc att cag tgt gaa 1344
Ala Arg Gly Leu Asp Ile Asn Asp Val Gln Leu Ile Ile Gln Cys Glu
        435                 440                 445
cct ccc cgg gac gtt gaa gct tac ata cac cgg tca ggt cgg aca ggg 1392
Pro Pro Arg Asp Val Glu Ala Tyr Ile His Arg Ser Gly Arg Thr Gly
    450                 455                 460
aga gca ggt aat act ggt gtt gct gtc atg ctt tat gag ccc aga tat 1440
Arg Ala Gly Asn Thr Gly Val Ala Val Met Leu Tyr Glu Pro Arg Tyr
465                 470                 475                 480
aag cac agt gtc agc aga cta gaa agg gag tct ggg gtt aaa ttt gag 1488
Lys His Ser Val Ser Arg Leu Glu Arg Glu Ser Gly Val Lys Phe Glu
                485                 490                 495
cat atc tct gcg cca caa ccc act gat gta gca caa tct gct ggc agt 1536
His Ile Ser Ala Pro Gln Pro Thr Asp Val Ala Gln Ser Ala Gly Ser
            500                 505                 510
gaa gct gca gat gca att gcg agt gtg tca gac agt gtt att cct atc 1584
Glu Ala Ala Asp Ala Ile Ala Ser Val Ser Asp Ser Val Ile Pro Ile
        515                 520                 525
ttc agg cag caa gca gag cag ttg cta agc tct tcc agt ctg tct gca 1632
Phe Arg Gln Gln Ala Glu Gln Leu Leu Ser Ser Ser Ser Leu Ser Ala
    530                 535                 540
gct gac ttg ctt gcc aaa gca ctt gca aag gca gtt ggt tac acg gac 1680
Ala Asp Leu Leu Ala Lys Ala Leu Ala Lys Ala Val Gly Tyr Thr Asp
545                 550                 555                 560
ata aag aaa aga tca ttg tta tct tcc ttg gag gat tac agt aca cta 1728
Ile Lys Lys Arg Ser Leu Leu Ser Ser Leu Glu Asp Tyr Ser Thr Leu

                    565                 570                 575
cat ctt caa act ggc aga ccg atg tgg tca cct ggg ttt gct ttt act        1776
His Leu Gln Thr Gly Arg Pro Met Trp Ser Pro Gly Phe Ala Phe Thr
            580                 585                 590
ata ttg aaa agg ttc atg cca gaa gat aaa ctt gca gat gta aag ggt        1824
Ile Leu Lys Arg Phe Met Pro Glu Asp Lys Leu Ala Asp Val Lys Gly
            595                 600                 605
gcc acc ctc acc gct gat gga aca gga gtt gta ttt gat gtt cct gca        1872
Ala Thr Leu Thr Ala Asp Gly Thr Gly Val Val Phe Asp Val Pro Ala
    610                 615                 620
gca gat gtt gaa gat tac att caa gct tcg gag agt gcc gca cag gtg        1920
Ala Asp Val Glu Asp Tyr Ile Gln Ala Ser Glu Ser Ala Ala Gln Val
625                 630                 635                 640
aca att gat gaa gtc aag caa ctg cca ccc ttg caa gag aag gac cag        1968
Thr Ile Asp Glu Val Lys Gln Leu Pro Pro Leu Gln Glu Lys Asp Gln
                645                 650                 655
tcg aga ggc aac tct ggg gga gga aga ttt ggc cat gga ggt ggc cgg        2016
Ser Arg Gly Asn Ser Gly Gly Gly Arg Phe Gly His Gly Gly Gly Arg
            660                 665                 670
aga ttt tct ggt ggt ggt ggc ggc ggc aga ggg ttt tct ggc cgg gga        2064
Arg Phe Ser Gly Gly Gly Gly Gly Gly Arg Gly Phe Ser Gly Arg Gly
        675                 680                 685
ggt ggc ggc aga ggg ttt tct ggc aga gga ggc ggc ggc agt aga ttt        2112
Gly Gly Gly Arg Gly Phe Ser Gly Arg Gly Gly Gly Gly Ser Arg Phe
    690                 695                 700
aac agg agg aat tag                                                    2127
Asn Arg Arg Asn
705

<210> 138
<211> 708
<212> PRT
<213> Zea mays

<400> 138
   Met Ser Pro Ala Leu Ala Ala Gly Val Glu Pro Met Ala Val Asp Asp
   1               5                   10                  15
   Ser Ala Ser Lys Lys Ala Lys Arg Lys Gln Leu Lys Ala Ala Ala Ala
               20                  25                  30
   Ala Ala Glu Ala Glu Ala Glu Ala Ala Ser Val Lys Lys Lys Glu Lys
           35                  40                  45
   Lys Glu Lys Lys Arg Lys Val Lys Glu Pro Ser Pro Pro Leu Pro Ser
       50                  55                  60
   Ala Ala Ser Ser Asp Glu Glu Glu Lys Ser Ser Thr Ser Ser Glu Glu
   65                  70                  75                  80
   Thr Ala Pro Ala Ala Lys Lys Ala Lys Lys Glu Lys Thr Lys Lys Asn
                   85                  90                  95
   Val Asp Val Ser Ser Ser Ala Ser Asp Asp Asp Gly Glu Ile Thr Ala
               100                 105                 110
   Ser Ser Asp Glu Asp Pro Ala Asp Pro Asn Ala Leu Thr Asn Phe Arg
           115                 120                 125
   Ile Ser Glu Ser Leu Arg Gln Ser Leu Arg Ser Lys Gly Ile Lys Ser
       130                 135                 140
   Leu Phe Pro Ile Gln Ala Thr Thr Phe Asp Leu Val Leu Asp Gly Ser
   145                 150                 155                 160
   Asp Leu Val Gly Arg Ala Arg Thr Gly Gln Gly Lys Thr Leu Ala Phe
                   165                 170                 175
   Val Leu Pro Ile Leu Glu Ser Leu Val Asn Gly Ala Asn Lys Ala Ser
               180                 185                 190
   Arg Arg Thr Glu His Gly Arg Thr Pro Ser Val Leu Val Leu Leu Pro
           195                 200                 205
   Thr Arg Glu Leu Ala Asn Gln Val His Ala Asp Phe Glu Phe Tyr Gly
       210                 215                 220
   Ala Thr Phe Gly Leu Ser Ala Cys Cys Val Tyr Gly Gly Ser Pro Tyr
   225                 230                 235                 240
   Arg Pro Gln Glu Met Ala Leu Arg Arg Gly Val Asp Ile Val Val Gly
                   245                 250                 255
   Thr Pro Gly Arg Val Lys Asp Phe Ile Val Lys Gly Thr Leu Asn Leu
               260                 265                 270
   Lys Cys Leu Lys Phe Arg Val Leu Asp Glu Ala Asp Glu Met Leu Asn
           275                 280                 285
   Met Gly Phe Val Asp Asp Val Glu Leu Ile Leu Gly Lys Val Glu Asp
       290                 295                 300
   Ala Thr Lys Val Gln Thr Leu Leu Phe Ser Ala Thr Leu Pro Asp Trp
   305                 310                 315                 320
   Val Asn Lys Leu Ser Met Arg Phe Leu Lys Val Asp Arg Lys Thr Val
                   325                 330                 335
   Asp Leu Val Gly Asn Glu Lys Leu Lys Ala Ser Ala Ser Val Lys His
               340                 345                 350
   Leu Ala Leu Pro Cys Asn Lys Ala Ala Arg Ala Gln Leu Ile Pro Asp
           355                 360                 365
   Ile Ile Arg Cys Tyr Ser His Gly Gly Arg Thr Ile Ile Phe Thr Glu
       370                 375                 380
   Thr Lys Asp Ser Ala Ser Glu Leu Ser Gly Leu Ile Pro Gly Ser Arg
   385                 390                 395                 400
   Ala Leu His Gly Asp Val Val Gln Ala Gln Arg Glu Val Ile Leu Ala
                   405                 410                 415
   Gly Phe Arg Ser Gly Lys Phe Gln Val Leu Val Ala Thr Asn Val Ala
               420                 425                 430
   Ala Arg Gly Leu Asp Ile Asn Asp Val Gln Leu Ile Ile Gln Cys Glu
           435                 440                 445
   Pro Pro Arg Asp Val Glu Ala Tyr Ile His Arg Ser Gly Arg Thr Gly
       450                 455                 460
   Arg Ala Gly Asn Thr Gly Val Ala Val Met Leu Tyr Glu Pro Arg Tyr
   465                 470                 475                 480
   Lys His Ser Val Ser Arg Leu Glu Arg Glu Ser Gly Val Lys Phe Glu
                   485                 490                 495
   His Ile Ser Ala Pro Gln Pro Thr Asp Val Ala Gln Ser Ala Gly Ser
               500                 505                 510
   Glu Ala Ala Asp Ala Ile Ala Ser Val Ser Asp Ser Val Ile Pro Ile
           515                 520                 525
   Phe Arg Gln Gln Ala Glu Gln Leu Leu Ser Ser Ser Ser Leu Ser Ala
       530                 535                 540
   Ala Asp Leu Leu Ala Lys Ala Leu Ala Lys Ala Val Gly Tyr Thr Asp
   545                 550                 555                 560
   Ile Lys Lys Arg Ser Leu Leu Ser Ser Leu Glu Asp Tyr Ser Thr Leu
                   565                 570                 575
   His Leu Gln Thr Gly Arg Pro Met Trp Ser Pro Gly Phe Ala Phe Thr
               580                 585                 590
   Ile Leu Lys Arg Phe Met Pro Glu Asp Lys Leu Ala Asp Val Lys Gly
           595                 600                 605
   Ala Thr Leu Thr Ala Asp Gly Thr Gly Val Val Phe Asp Val Pro Ala
       610                 615                 620
   Ala Asp Val Glu Asp Tyr Ile Gln Ala Ser Glu Ser Ala Ala Gln Val
   625                 630                 635                 640
   Thr Ile Asp Glu Val Lys Gln Leu Pro Pro Leu Gln Glu Lys Asp Gln
                   645                 650                 655
   Ser Arg Gly Asn Ser Gly Gly Gly Arg Phe Gly His Gly Gly Gly Arg
               660                 665                 670
   Arg Phe Ser Gly Gly Gly Gly Gly Gly Arg Gly Phe Ser Gly Arg Gly
           675                 680                 685
   Gly Gly Gly Arg Gly Phe Ser Gly Arg Gly Gly Gly Gly Ser Arg Phe
       690                 695                 700
   Asn Arg Arg Asn
   705

<210> 139
<211> 1290
<212> DNA
<213> Zea mays

<220>
<221> CDS
<222> (1)..(1290)

<400> 139
atg atg gga gag gcc aag gag aac gac gtt tat gag gag gag ctc ctg        48
Met Met Gly Glu Ala Lys Glu Asn Asp Val Tyr Glu Glu Glu Leu Leu

1                   5                   10                  15
gac tac gag gag gac gac gac aag acg gtc gat ggc tcc gct gct aag          96
Asp Tyr Glu Glu Asp Asp Asp Lys Thr Val Asp Gly Ser Ala Ala Lys
                20                  25                  30
ccc acc gga gag gtc gca aag aag ggc tac gtc ggg atc cac agt tcc          144
Pro Thr Gly Glu Val Ala Lys Lys Gly Tyr Val Gly Ile His Ser Ser
            35                  40                  45
ggt ttc aga gac ttc ctg ctc aag cca gag ctg ctc cgt gct atc cag          192
Gly Phe Arg Asp Phe Leu Leu Lys Pro Glu Leu Leu Arg Ala Ile Gln
    50                  55                  60
gat tgt ggt ttt gag cat cct tcc gaa gtg caa cac gag tgt atc cct          240
Asp Cys Gly Phe Glu His Pro Ser Glu Val Gln His Glu Cys Ile Pro
65                  70                  75                  80
caa gcc att ctt gga atg gat gtc atc tgt caa gct aaa tct ggg atg          288
Gln Ala Ile Leu Gly Met Asp Val Ile Cys Gln Ala Lys Ser Gly Met
                85                  90                  95
ggg aaa act gct gtt ttt gtc ctt tca tcc ctc caa caa att gac cct          336
Gly Lys Thr Ala Val Phe Val Leu Ser Ser Leu Gln Gln Ile Asp Pro
            100                 105                 110
gtt gcg ggt cag gta gca gca ctt gta ctg tgc cac aca agg gaa ctg          384
Val Ala Gly Gln Val Ala Ala Leu Val Leu Cys His Thr Arg Glu Leu
        115                 120                 125
gct tat cag ata tgc cac gaa ttt gag agg ttc agc aaa tat ctt act          432
Ala Tyr Gln Ile Cys His Glu Phe Glu Arg Phe Ser Lys Tyr Leu Thr
    130                 135                 140
gaa gtt aaa gtt gct gtc ttc tat gga ggt gtt cac atc agg aag cat          480
Glu Val Lys Val Ala Val Phe Tyr Gly Gly Val His Ile Arg Lys His
145                 150                 155                 160
aaa gat ttg ttg aag aat gaa tgc cct cat att gtg gtt ggc acg cct          528
Lys Asp Leu Leu Lys Asn Glu Cys Pro His Ile Val Val Gly Thr Pro
                165                 170                 175
ggt agg ata ctt gct ctt gcc agg gat aag gat ctc tcc ttg aag aat          576
Gly Arg Ile Leu Ala Leu Ala Arg Asp Lys Asp Leu Ser Leu Lys Asn
            180                 185                 190
gtg agg cac ttc att ctt gat gaa tgt gac aaa atg ctg gaa tca ctt          624
Val Arg His Phe Ile Leu Asp Glu Cys Asp Lys Met Leu Glu Ser Leu
        195                 200                 205
gac atg cgg aga gat gtt cag gag atc ttc aaa atg act cct cat gat          672
Asp Met Arg Arg Asp Val Gln Glu Ile Phe Lys Met Thr Pro His Asp
    210                 215                 220
aag caa gtc atg atg ttt tca gca acg ctc agc aag gag atc cgt cct          720
Lys Gln Val Met Met Phe Ser Ala Thr Leu Ser Lys Glu Ile Arg Pro
225                 230                 235                 240
gta tgc aag aaa ttt atg caa gat ccg atg gaa ata tat gtt gat gat          768
Val Cys Lys Lys Phe Met Gln Asp Pro Met Glu Ile Tyr Val Asp Asp
                245                 250                 255
gag gca aag ttg acc ctc cat ggt ctt gta cag cac tat ata aaa ctt          816
Glu Ala Lys Leu Thr Leu His Gly Leu Val Gln His Tyr Ile Lys Leu
            260                 265                 270
agc gag gca gag aag aac cgg aaa ctg aat gat ctc ttg gac gct ctt          864
Ser Glu Ala Glu Lys Asn Arg Lys Leu Asn Asp Leu Leu Asp Ala Leu
        275                 280                 285
gat ttc aac caa gtt gtg ata ttc gtg aag agt gtt agc aga gct gcc          912
Asp Phe Asn Gln Val Val Ile Phe Val Lys Ser Val Ser Arg Ala Ala
    290                 295                 300
gaa ctg aat aag tta ctt tgt gaa tgc aac ttc cca tca atc tgc att          960
Glu Leu Asn Lys Leu Leu Cys Glu Cys Asn Phe Pro Ser Ile Cys Ile
305                 310                 315                 320
cat tct gga atg act cag gaa gaa agg cta aca aga tat aag aac ttc          1008
His Ser Gly Met Thr Gln Glu Glu Arg Leu Thr Arg Tyr Lys Asn Phe
                325                 330                 335
aag gaa ggc cac aag aga att ctt gtg gct aca gat tta gtt ggc agg          1056
Lys Glu Gly His Lys Arg Ile Leu Val Ala Thr Asp Leu Val Gly Arg
            340                 345                 350
ggc att gac att gag cgt gtc aac att gtg ata aac tat gac atg cca          1104
Gly Ile Asp Ile Glu Arg Val Asn Ile Val Ile Asn Tyr Asp Met Pro
        355                 360                 365
gac tca gct gat act tat ttg cac agg gtt gga agg gct gga cgt ttt          1152
Asp Ser Ala Asp Thr Tyr Leu His Arg Val Gly Arg Ala Gly Arg Phe

370                 375                 380
ggc acg aag gga ctt gca att acc ttt gtt tcc tct gct tcg gat tct        1200
Gly Thr Lys Gly Leu Ala Ile Thr Phe Val Ser Ser Ala Ser Asp Ser
385                 390                 395                 400
gat gtt ctt aac cag gtg caa gaa agg ttt gag gta gac ata aag gaa        1248
Asp Val Leu Asn Gln Val Gln Glu Arg Phe Glu Val Asp Ile Lys Glu
                405                 410                 415
ctg cct gag caa att gac acc tcg acc tac atg cca tca taa                1290
Leu Pro Glu Gln Ile Asp Thr Ser Thr Tyr Met Pro Ser
            420                 425

<210> 140
<211> 429
<212> PRT
<213> Zea mays

<400> 140
Met Met Gly Glu Ala Lys Glu Asn Asp Val Tyr Glu Glu Glu Leu Leu
1               5                   10                  15
Asp Tyr Glu Glu Asp Asp Asp Lys Thr Val Asp Gly Ser Ala Ala Lys
            20                  25                  30
Pro Thr Gly Glu Val Ala Lys Lys Gly Tyr Val Gly Ile His Ser Ser
        35                  40                  45
Gly Phe Arg Asp Phe Leu Leu Lys Pro Glu Leu Leu Arg Ala Ile Gln
    50                  55                  60
Asp Cys Gly Phe Glu His Pro Ser Glu Val Gln His Glu Cys Ile Pro
65                  70                  75                  80
Gln Ala Ile Leu Gly Met Asp Val Ile Cys Gln Ala Lys Ser Gly Met
                85                  90                  95
Gly Lys Thr Ala Val Phe Val Leu Ser Ser Leu Gln Gln Ile Asp Pro
            100                 105                 110
Val Ala Gly Gln Val Ala Ala Leu Val Leu Cys His Thr Arg Glu Leu
        115                 120                 125
Ala Tyr Gln Ile Cys His Glu Phe Glu Arg Phe Ser Lys Tyr Leu Thr
    130                 135                 140
Glu Val Lys Val Ala Val Phe Tyr Gly Gly Val His Ile Arg Lys His
145                 150                 155                 160
Lys Asp Leu Leu Lys Asn Glu Cys Pro His Ile Val Val Gly Thr Pro
                165                 170                 175
Gly Arg Ile Leu Ala Leu Ala Arg Asp Lys Asp Leu Ser Leu Lys Asn
            180                 185                 190
Val Arg His Phe Ile Leu Asp Glu Cys Asp Lys Met Leu Glu Ser Leu
        195                 200                 205
Asp Met Arg Arg Asp Val Gln Glu Ile Phe Lys Met Thr Pro His Asp
    210                 215                 220
Lys Gln Val Met Met Phe Ser Ala Thr Leu Ser Lys Glu Ile Arg Pro
225                 230                 235                 240
Val Cys Lys Lys Phe Met Gln Asp Pro Met Glu Ile Tyr Val Asp Asp
                245                 250                 255
Glu Ala Lys Leu Thr Leu His Gly Leu Val Gln His Tyr Ile Lys Leu
            260                 265                 270
Ser Glu Ala Glu Lys Asn Arg Lys Leu Asn Asp Leu Leu Asp Ala Leu
        275                 280                 285
Asp Phe Asn Gln Val Val Ile Phe Val Lys Ser Val Ser Arg Ala Ala
    290                 295                 300
Glu Leu Asn Lys Leu Leu Cys Glu Cys Asn Phe Pro Ser Ile Cys Ile
305                 310                 315                 320
His Ser Gly Met Thr Gln Glu Glu Arg Leu Thr Arg Tyr Lys Asn Phe
                325                 330                 335
Lys Glu Gly His Lys Arg Ile Leu Val Ala Thr Asp Leu Val Gly Arg
            340                 345                 350
Gly Ile Asp Ile Glu Arg Val Asn Ile Val Ile Asn Tyr Asp Met Pro
        355                 360                 365
Asp Ser Ala Asp Thr Tyr Leu His Arg Val Gly Arg Ala Gly Arg Phe
    370                 375                 380
Gly Thr Lys Gly Leu Ala Ile Thr Phe Val Ser Ser Ala Ser Asp Ser
385                 390                 395                 400
Asp Val Leu Asn Gln Val Gln Glu Arg Phe Glu Val Asp Ile Lys Glu
                405                 410                 415

Leu Pro Glu Gln Ile Asp Thr Ser Thr Tyr Met Pro Ser
            420                 425

<210> 141
<211> 1554
<212> DNA
<213> Zea mays

<220>
<221> CDS
<222> (1)..(1554)

<400> 141
atg gat cca cgc gcc agg tat cct tcg ggc atg ggc aac ggc cgc ggt       48
Met Asp Pro Arg Ala Arg Tyr Pro Ser Gly Met Gly Asn Gly Arg Gly
1               5                   10                  15
ggg acc cct aac tac tac ggc cgc ggc cca ccg ccg caa cag ccg cat       96
Gly Thr Pro Asn Tyr Tyr Gly Arg Gly Pro Pro Pro Gln Gln Pro His
            20                  25                  30
cag cac cac cac cag cag cag cag cag aca tcg ggg gcg cat cac cac      144
Gln His His His Gln Gln Gln Gln Gln Thr Ser Gly Ala His His His
        35                  40                  45
cag cag tac gcg cag agg cag cag caa cag cac cgc cac agc cac aac      192
Gln Gln Tyr Ala Gln Arg Gln Gln Gln Gln His Arg His Ser His Asn
    50                  55                  60
cac cag cag caa cag cag cag cac cag agt cag cag cag cag cag cag      240
His Gln Gln Gln Gln Gln Gln His Gln Ser Gln Gln Gln Gln Gln Gln
65                  70                  75                  80
tgg ctg cgg cgg aat cag att gcc cgc gag gcc gcg ggc gca gcc gtg      288
Trp Leu Arg Arg Asn Gln Ile Ala Arg Glu Ala Ala Gly Ala Ala Val
                85                  90                  95
acc agc gag ccg aag gcc ctg gcg cca tcc acg gcc gcc gac ggc gtc      336
Thr Ser Glu Pro Lys Ala Leu Ala Pro Ser Thr Ala Ala Asp Gly Val
            100                 105                 110
aat tcg agt tcg caa gat tgg aag acc caa ttg aag ctt ccg cct cct      384
Asn Ser Ser Ser Gln Asp Trp Lys Thr Gln Leu Lys Leu Pro Pro Pro
        115                 120                 125
gat aca cgc tac agg aca gag gat gtt aca gcc acc aag gga aat gag      432
Asp Thr Arg Tyr Arg Thr Glu Asp Val Thr Ala Thr Lys Gly Asn Glu
    130                 135                 140
ttt gaa gac tat ttt ctc aag cgt gaa ctc cta atg ggt att tac gaa      480
Phe Glu Asp Tyr Phe Leu Lys Arg Glu Leu Leu Met Gly Ile Tyr Glu
145                 150                 155                 160
aag gga ttt gaa aga ccc tct cct att caa gag gaa agc att cct att      528
Lys Gly Phe Glu Arg Pro Ser Pro Ile Gln Glu Glu Ser Ile Pro Ile
                165                 170                 175
gct tta act ggt agt gac att ctt gca aga gcg aag aat gga act ggc      576
Ala Leu Thr Gly Ser Asp Ile Leu Ala Arg Ala Lys Asn Gly Thr Gly
            180                 185                 190
aag act gct gca ttt tgt att cct gca ctg gaa aag att gat caa gaa      624
Lys Thr Ala Ala Phe Cys Ile Pro Ala Leu Glu Lys Ile Asp Gln Glu
        195                 200                 205
aaa aat gct att caa gtt gtt ata ttg gtt ccc aca agg gaa ttg gct      672
Lys Asn Ala Ile Gln Val Val Ile Leu Val Pro Thr Arg Glu Leu Ala
    210                 215                 220
cta caa aca tca caa gtt tgt aag gag ctt ggt aaa cat ttg aag atc      720
Leu Gln Thr Ser Gln Val Cys Lys Glu Leu Gly Lys His Leu Lys Ile
225                 230                 235                 240
caa gtt atg gtc acc act ggt gga acc agc ttg aag gat gat att gtt      768
Gln Val Met Val Thr Thr Gly Gly Thr Ser Leu Lys Asp Asp Ile Val
                245                 250                 255
cgt ctg tac caa cct gtg cac tta att gtt gga aca cct ggc cgt att      816
Arg Leu Tyr Gln Pro Val His Leu Ile Val Gly Thr Pro Gly Arg Ile
            260                 265                 270
ttg gat ctt aca aag aaa ggt gtt tgc att ttg aag gac tgt tca atg      864
Leu Asp Leu Thr Lys Lys Gly Val Cys Ile Leu Lys Asp Cys Ser Met
        275                 280                 285
ctt att atg gat gag gca gac aag ctt ctc tct cct gag ttt caa cct      912
Leu Ile Met Asp Glu Ala Asp Lys Leu Leu Ser Pro Glu Phe Gln Pro

290                 295                 300
tcc ata gaa cag ttg att cgc tac ctc cct gct agt cga cag att ttg         960
Ser Ile Glu Gln Leu Ile Arg Tyr Leu Pro Ala Ser Arg Gln Ile Leu
305                 310                 315                 320
atg ttc tca gcg aca ttc cct gtg act gtt aag gag ttc aag gac aaa         1008
Met Phe Ser Ala Thr Phe Pro Val Thr Val Lys Glu Phe Lys Asp Lys
                325                 330                 335
tat ctt cct aaa cct tat gtg att aac ctt atg gat gaa ctt aca ctg         1056
Tyr Leu Pro Lys Pro Tyr Val Ile Asn Leu Met Asp Glu Leu Thr Leu
            340                 345                 350
aag gga att acg cag ttc tat gct ttt gtt gaa gaa cga cag aaa gtt         1104
Lys Gly Ile Thr Gln Phe Tyr Ala Phe Val Glu Glu Arg Gln Lys Val
        355                 360                 365
cac tgt ctc aac acc ctc ttt tcc aag ctt caa atc aat cag tca ata         1152
His Cys Leu Asn Thr Leu Phe Ser Lys Leu Gln Ile Asn Gln Ser Ile
    370                 375                 380
ata ttc tgt aac tct gta aat cgt gtt gaa cta ttg gcg aag aag atc         1200
Ile Phe Cys Asn Ser Val Asn Arg Val Glu Leu Leu Ala Lys Lys Ile
385                 390                 395                 400
aca gag ctt ggt tat tct tgc ttc tat att cat gca aag atg cta cag         1248
Thr Glu Leu Gly Tyr Ser Cys Phe Tyr Ile His Ala Lys Met Leu Gln
                405                 410                 415
gac cac cgc aat aga gtt ttc cat gat ttc cgt aat gga gcg tgc agg         1296
Asp His Arg Asn Arg Val Phe His Asp Phe Arg Asn Gly Ala Cys Arg
            420                 425                 430
aat ctt gtt tgt act gat ctt ttt aca agg gga ata gat att caa gct         1344
Asn Leu Val Cys Thr Asp Leu Phe Thr Arg Gly Ile Asp Ile Gln Ala
        435                 440                 445
gtg aat gtt gtt atc aat ttt gat ttc ccc aag aac tct gaa aca tat         1392
Val Asn Val Val Ile Asn Phe Asp Phe Pro Lys Asn Ser Glu Thr Tyr
    450                 455                 460
ctc cac agg gtt ggt aga tct ggg aga ttt ggc cac ctt ggt ctg gcc         1440
Leu His Arg Val Gly Arg Ser Gly Arg Phe Gly His Leu Gly Leu Ala
465                 470                 475                 480
gtt aat ttg atc acc tat gag gat cga ttt aac ttg tat agg att gaa         1488
Val Asn Leu Ile Thr Tyr Glu Asp Arg Phe Asn Leu Tyr Arg Ile Glu
                485                 490                 495
caa gag ctt ggg act gaa ata aaa cca att cct cct cag ata gac cag         1536
Gln Glu Leu Gly Thr Glu Ile Lys Pro Ile Pro Pro Gln Ile Asp Gln
            500                 505                 510
gct ata tat tgc caa tga                                                 1554
Ala Ile Tyr Cys Gln
        515

<210> 142
<211> 517
<212> PRT
<213> Zea mays

<400> 142
   Met Asp Pro Arg Ala Arg Tyr Pro Ser Gly Met Gly Asn Gly Arg Gly
   1               5                   10                  15
   Gly Thr Pro Asn Tyr Tyr Gly Arg Gly Pro Pro Pro Gln Gln Pro His
               20                  25                  30
   Gln His His His Gln Gln Gln Gln Gln Thr Ser Gly Ala His His His
           35                  40                  45
   Gln Gln Tyr Ala Gln Arg Gln Gln Gln Gln His Arg His Ser His Asn
       50                  55                  60
   His Gln Gln Gln Gln Gln Gln His Gln Ser Gln Gln Gln Gln Gln Gln
   65                  70                  75                  80
   Trp Leu Arg Arg Asn Gln Ile Ala Arg Glu Ala Ala Gly Ala Ala Val
                   85                  90                  95
   Thr Ser Glu Pro Lys Ala Leu Ala Pro Ser Thr Ala Ala Asp Gly Val
               100                 105                 110
   Asn Ser Ser Ser Gln Asp Trp Lys Thr Gln Leu Lys Leu Pro Pro Pro
           115                 120                 125
   Asp Thr Arg Tyr Arg Thr Glu Asp Val Thr Ala Thr Lys Gly Asn Glu
       130                 135                 140
   Phe Glu Asp Tyr Phe Leu Lys Arg Glu Leu Leu Met Gly Ile Tyr Glu
   145                     150                     155                     160
   Lys Gly Phe Glu Arg Pro Ser Pro Ile Gln Glu Glu Ser Ile Pro Ile
                   165                     170                     175
   Ala Leu Thr Gly Ser Asp Ile Leu Ala Arg Ala Lys Asn Gly Thr Gly
               180                     185                     190
   Lys Thr Ala Ala Phe Cys Ile Pro Ala Leu Glu Lys Ile Asp Gln Glu
           195                     200                     205
   Lys Asn Ala Ile Gln Val Val Ile Leu Val Pro Thr Arg Glu Leu Ala
       210                     215                     220
   Leu Gln Thr Ser Gln Val Cys Lys Glu Leu Gly Lys His Leu Lys Ile
   225                     230                     235                     240
   Gln Val Met Val Thr Thr Gly Gly Thr Ser Leu Lys Asp Asp Ile Val
                   245                     250                     255
   Arg Leu Tyr Gln Pro Val His Leu Ile Val Gly Thr Pro Gly Arg Ile
               260                     265                     270
   Leu Asp Leu Thr Lys Lys Gly Val Cys Ile Leu Lys Asp Cys Ser Met
           275                     280                     285
   Leu Ile Met Asp Glu Ala Asp Lys Leu Leu Ser Pro Glu Phe Gln Pro
       290                     295                     300
   Ser Ile Glu Gln Leu Ile Arg Tyr Leu Pro Ala Ser Arg Gln Ile Leu
   305                     310                     315                     320
   Met Phe Ser Ala Thr Phe Pro Val Thr Val Lys Glu Phe Lys Asp Lys
                   325                     330                     335
   Tyr Leu Pro Lys Pro Tyr Val Ile Asn Leu Met Asp Glu Leu Thr Leu
               340                     345                     350
   Lys Gly Ile Thr Gln Phe Tyr Ala Phe Val Glu Glu Arg Gln Lys Val
           355                     360                     365
   His Cys Leu Asn Thr Leu Phe Ser Lys Leu Gln Ile Asn Gln Ser Ile
       370                     375                     380
   Ile Phe Cys Asn Ser Val Asn Arg Val Glu Leu Leu Ala Lys Lys Ile
   385                     390                     395                     400
   Thr Glu Leu Gly Tyr Ser Cys Phe Tyr Ile His Ala Lys Met Leu Gln
                   405                     410                     415
   Asp His Arg Asn Arg Val Phe His Asp Phe Arg Asn Gly Ala Cys Arg
               420                     425                     430
   Asn Leu Val Cys Thr Asp Leu Phe Thr Arg Gly Ile Asp Ile Gln Ala
           435                     440                     445
   Val Asn Val Val Ile Asn Phe Asp Phe Pro Lys Asn Ser Glu Thr Tyr
       450                     455                     460
   Leu His Arg Val Gly Arg Ser Gly Arg Phe Gly His Leu Gly Leu Ala
   465                     470                     475                     480
   Val Asn Leu Ile Thr Tyr Glu Asp Arg Phe Asn Leu Tyr Arg Ile Glu
                   485                     490                     495
   Gln Glu Leu Gly Thr Glu Ile Lys Pro Ile Pro Pro Gln Ile Asp Gln
               500                     505                     510
   Ala Ile Tyr Cys Gln
           515

<210> 143
<211> 1926
<212> DNA
<213> Zea mays

<220>
<221> CDS
<222> (1)..(1926)

<400> 143
   atg gca gca tcc acg gcg gcg gcg gcc gcg cgg aag gga gcg ctg acg       48
   Met Ala Ala Ser Thr Ala Ala Ala Ala Ala Arg Lys Gly Ala Leu Thr
   1               5                   10                  15
   gat cag agg ttc tcg gag ctt tcc ccc gcg cta tcc ccg gag gtg gtg      96
   Asp Gln Arg Phe Ser Glu Leu Ser Pro Ala Leu Ser Pro Glu Val Val
               20                  25                  30
   gag gca ctg gac cgc ggc ggc ttc cag cgg tgc acg ccc gtg cag gcg      144
   Glu Ala Leu Asp Arg Gly Gly Phe Gln Arg Cys Thr Pro Val Gln Ala
           35                  40                  45
   gcg gcc atc ccg cac ctg ctg tcg cac aag gac gtc gcc gtc gac gcc      192
   Ala Ala Ile Pro His Leu Leu Ser His Lys Asp Val Ala Val Asp Ala
   50                  55                  60
   gcc aca ggc tcc ggg aag acc ctt gcc ttc atc gtc ccc gtc gtt gag          240
   Ala Thr Gly Ser Gly Lys Thr Leu Ala Phe Ile Val Pro Val Val Glu
   65                  70                  75                  80
   atc ctc cgc cgc cgc tcc tcc ccg ccc aag tca cac gag gtc ctt gct          288
   Ile Leu Arg Arg Arg Ser Ser Pro Pro Lys Ser His Glu Val Leu Ala
                   85                  90                  95
   ctg att atc tct ccc acg aga gaa ttg tcc tcg caa ata ttc aat gtg          336
   Leu Ile Ile Ser Pro Thr Arg Glu Leu Ser Ser Gln Ile Phe Asn Val
               100                 105                 110
   gcc cag ccc ttc ttc gcg acc ctg aat ggc gtg tcg tcc atg ctc ctg          384
   Ala Gln Pro Phe Phe Ala Thr Leu Asn Gly Val Ser Ser Met Leu Leu
           115                 120                 125
   gtg ggt ggg ctg gat atc aaa gca gag ctc aag aag gta gag gaa gag          432
   Val Gly Gly Leu Asp Ile Lys Ala Glu Leu Lys Lys Val Glu Glu Glu
       130                 135                 140
   ggc gca aac ata ttg gtg ggc act ccc ggg aag ctg ttt gat ata atg          480
   Gly Ala Asn Ile Leu Val Gly Thr Pro Gly Lys Leu Phe Asp Ile Met
   145                 150                 155                 160
   cac act gat gct cta gag tac aag aat ctt gag att ttg atc cta gat          528
   His Thr Asp Ala Leu Glu Tyr Lys Asn Leu Glu Ile Leu Ile Leu Asp
                   165                 170                 175
   gag gct gat agg ctc ttg gat atg gga ttc cag aaa cat att aac ttc          576
   Glu Ala Asp Arg Leu Leu Asp Met Gly Phe Gln Lys His Ile Asn Phe
               180                 185                 190
   ata ctt tca atg ttg cca aag ctg agg agg act ggg ctt ttc tca gct          624
   Ile Leu Ser Met Leu Pro Lys Leu Arg Arg Thr Gly Leu Phe Ser Ala
           195                 200                 205
   acc cag acg aaa gct gtt gcg gac cta tcc aaa gca ggg ttg aga aat          672
   Thr Gln Thr Lys Ala Val Ala Asp Leu Ser Lys Ala Gly Leu Arg Asn
       210                 215                 220
   cct ata agg gtt gag gtt aag aca gag gca aag tcc aca agc aaa gat          720
   Pro Ile Arg Val Glu Val Lys Thr Glu Ala Lys Ser Thr Ser Lys Asp
   225                 230                 235                 240
   gct gga cag caa gag ctt ggt cca tcc ata aca cca tta ggg ctt cgg          768
   Ala Gly Gln Gln Glu Leu Gly Pro Ser Ile Thr Pro Leu Gly Leu Arg
                   245                 250                 255
   ttg gag tat atg ata tgt gaa gca tca aag aag tca tct cag ctt gtt          816
   Leu Glu Tyr Met Ile Cys Glu Ala Ser Lys Lys Ser Ser Gln Leu Val
               260                 265                 270
   gat ttc ctt gtg cag aat agt ggg aag aaa ata atg gta tat ttt gca          864
   Asp Phe Leu Val Gln Asn Ser Gly Lys Lys Ile Met Val Tyr Phe Ala
           275                 280                 285
   aca tgt gct tgt gta gat tat tgg gct gtt gtt ctt cca cta att aag          912
   Thr Cys Ala Cys Val Asp Tyr Trp Ala Val Val Leu Pro Leu Ile Lys
       290                 295                 300
   tcg ctt aaa ggt tct cca ata att gct tac cat ggg aag atg aaa cag          960
   Ser Leu Lys Gly Ser Pro Ile Ile Ala Tyr His Gly Lys Met Lys Gln
   305                 310                 315                 320
   agc ctt cga gag aaa gct ttg gca tca ttt tct gct ctt tca agt ggc          1008
   Ser Leu Arg Glu Lys Ala Leu Ala Ser Phe Ser Ala Leu Ser Ser Gly
                   325                 330                 335
   gtt cta gtc tgc act gat gtt gca gca agg ggt ctt gac ata cca agt          1056
   Val Leu Val Cys Thr Asp Val Ala Ala Arg Gly Leu Asp Ile Pro Ser
               340                 345                 350
   gtt gat ctg att gtg cag tat gat cca cct caa gat ccc aac gtt ttc          1104
   Val Asp Leu Ile Val Gln Tyr Asp Pro Pro Gln Asp Pro Asn Val Phe
           355                 360                 365
   ata cat aga gct ggc cgt aca gca cgc tat gat caa gaa ggg gat gct          1152
   Ile His Arg Ala Gly Arg Thr Ala Arg Tyr Asp Gln Glu Gly Asp Ala
       370                 375                 380
   att gtt ttt ctc tta cca aag gag gat act tat gtt gag ttc ttg aag          1200
   Ile Val Phe Leu Leu Pro Lys Glu Asp Thr Tyr Val Glu Phe Leu Lys
   385                 390                 395                 400
   ctt cga ggt gtt cct ctc aca gaa agg gaa tgc ctc gca aac act gat          1248
   Leu Arg Gly Val Pro Leu Thr Glu Arg Glu Cys Leu Ala Asn Thr Asp
                   405                 410                 415
   gat gtc ata ccg cag att aga agt gct gcg ctc gaa gat cgt aat gtc          1296
   Asp Val Ile Pro Gln Ile Arg Ser Ala Ala Leu Glu Asp Arg Asn Val
                   420                 425                 430
   atg gag aaa gga ctt aga gcg ttt gtt tcg ttt gtc cga gca tac aag      1344
   Met Glu Lys Gly Leu Arg Ala Phe Val Ser Phe Val Arg Ala Tyr Lys
               435                 440                 445
   gag cac cac tgc tct tac att ttt agg tgg aaa gat ctc gag att gga      1392
   Glu His His Cys Ser Tyr Ile Phe Arg Trp Lys Asp Leu Glu Ile Gly
           450                 455                 460
   aaa cta gct atg gag tat ggt tta ctc cag atc cca tcc atg cca gaa      1440
   Lys Leu Ala Met Glu Tyr Gly Leu Leu Gln Ile Pro Ser Met Pro Glu
   465                 470                 475                 480
   gtg aag cat cat tct ctt tca cta gag gga ttt att tct gtt gat gat      1488
   Val Lys His His Ser Leu Ser Leu Glu Gly Phe Ile Ser Val Asp Asp
                   485                 490                 495
   gtt gat att aca caa atc aag tac aag gat aaa gct cga gaa aaa caa      1536
   Val Asp Ile Thr Gln Ile Lys Tyr Lys Asp Lys Ala Arg Glu Lys Gln
               500                 505                 510
   cgc aag aag gcg ctg aaa agg aaa gca gaa gag gag gct caa aat cca      1584
   Arg Lys Lys Ala Leu Lys Arg Lys Ala Glu Glu Glu Ala Gln Asn Pro
           515                 520                 525
   aag ccg gag agg aag aga cct cca gag aag ccg gag aag cca aag cgg      1632
   Lys Pro Glu Arg Lys Arg Pro Pro Glu Lys Pro Glu Lys Pro Lys Arg
       530                 535                 540
   aag aag acg ggc aag cag cgt cag tca atc caa act aag gag gac ctg      1680
   Lys Lys Thr Gly Lys Gln Arg Gln Ser Ile Gln Thr Lys Glu Asp Leu
   545                 550                 555                 560
   gat gag ctg gcg cac gag tac cgg ctt ctg aag aag ctc aaa cgt gga      1728
   Asp Glu Leu Ala His Glu Tyr Arg Leu Leu Lys Lys Leu Lys Arg Gly
                   565                 570                 575
   gac atc gat gaa gag gag tac gag aag ctc act ggg ttc gga gat tct      1776
   Asp Ile Asp Glu Glu Glu Tyr Glu Lys Leu Thr Gly Phe Gly Asp Ser
               580                 585                 590
   gat ggc gaa gct tcc gat ggg gat ggg agc aac ttg gac gaa agg aag      1824
   Asp Gly Glu Ala Ser Asp Gly Asp Gly Ser Asn Leu Asp Glu Arg Lys
           595                 600                 605
   aag ggc aac aag gcg cag aaa aag ccc aag cag aga gga aaa tgc aaa      1872
   Lys Gly Asn Lys Ala Gln Lys Lys Pro Lys Gln Arg Gly Lys Cys Lys
       610                 615                 620
   agt ggg tct aag aag ttc gaa ggg agg agt aaa ttg agg agc aaa aga      1920
   Ser Gly Ser Lys Lys Phe Glu Gly Arg Ser Lys Leu Arg Ser Lys Arg
   625                 630                 635                 640
   aga tag                                                              1926
   Arg


<210> 144
<211> 641
<212> PRT
<213> Zea mays

<400> 144
Met Ala Ala Ser Thr Ala Ala Ala Ala Ala Arg Lys Gly Ala Leu Thr
1               5                   10                  15
Asp Gln Arg Phe Ser Glu Leu Ser Pro Ala Leu Ser Pro Glu Val Val
            20                  25                  30
Glu Ala Leu Asp Arg Gly Gly Phe Gln Arg Cys Thr Pro Val Gln Ala
        35                  40                  45
Ala Ala Ile Pro His Leu Leu Ser His Lys Asp Val Ala Val Asp Ala
    50                  55                  60
Ala Thr Gly Ser Gly Lys Thr Leu Ala Phe Ile Val Pro Val Val Glu
65                  70                  75                  80
Ile Leu Arg Arg Arg Ser Ser Pro Pro Lys Ser His Glu Val Leu Ala
                85                  90                  95
Leu Ile Ile Ser Pro Thr Arg Glu Leu Ser Ser Gln Ile Phe Asn Val
            100                 105                 110
Ala Gln Pro Phe Phe Ala Thr Leu Asn Gly Val Ser Ser Met Leu Leu
        115                 120                 125
Val Gly Gly Leu Asp Ile Lys Ala Glu Leu Lys Lys Val Glu Glu Glu
    130                 135                 140
Gly Ala Asn Ile Leu Val Gly Thr Pro Gly Lys Leu Phe Asp Ile Met

145                 150                 155                 160
His Thr Asp Ala Leu Glu Tyr Lys Asn Leu Glu Ile Leu Ile Leu Asp
                165                 170                 175
Glu Ala Asp Arg Leu Leu Asp Met Gly Phe Gln Lys His Ile Asn Phe
            180                 185                 190
Ile Leu Ser Met Leu Pro Lys Leu Arg Arg Thr Gly Leu Phe Ser Ala
        195                 200                 205
Thr Gln Thr Lys Ala Val Ala Asp Leu Ser Lys Ala Gly Leu Arg Asn
    210                 215                 220
Pro Ile Arg Val Glu Val Lys Thr Glu Ala Lys Ser Thr Ser Lys Asp
225                 230                 235                 240
Ala Gly Gln Gln Glu Leu Gly Pro Ser Ile Thr Pro Leu Gly Leu Arg
                245                 250                 255
Leu Glu Tyr Met Ile Cys Glu Ala Ser Lys Lys Ser Ser Gln Leu Val
            260                 265                 270
Asp Phe Leu Val Gln Asn Ser Gly Lys Lys Ile Met Val Tyr Phe Ala
        275                 280                 285
Thr Cys Ala Cys Val Asp Tyr Trp Ala Val Val Leu Pro Leu Ile Lys
    290                 295                 300
Ser Leu Lys Gly Ser Pro Ile Ile Ala Tyr His Gly Lys Met Lys Gln
305                 310                 315                 320
Ser Leu Arg Glu Lys Ala Leu Ala Ser Phe Ser Ala Leu Ser Ser Gly
                325                 330                 335
Val Leu Val Cys Thr Asp Val Ala Ala Arg Gly Leu Asp Ile Pro Ser
            340                 345                 350
Val Asp Leu Ile Val Gln Tyr Asp Pro Pro Gln Asp Pro Asn Val Phe
        355                 360                 365
Ile His Arg Ala Gly Arg Thr Ala Arg Tyr Asp Gln Glu Gly Asp Ala
    370                 375                 380
Ile Val Phe Leu Leu Pro Lys Glu Asp Thr Tyr Val Glu Phe Leu Lys
385                 390                 395                 400
Leu Arg Gly Val Pro Leu Thr Glu Arg Glu Cys Leu Ala Asn Thr Asp
                405                 410                 415
Asp Val Ile Pro Gln Ile Arg Ser Ala Ala Leu Glu Asp Arg Asn Val
            420                 425                 430
Met Glu Lys Gly Leu Arg Ala Phe Val Ser Phe Val Arg Ala Tyr Lys
        435                 440                 445
Glu His His Cys Ser Tyr Ile Phe Arg Trp Lys Asp Leu Glu Ile Gly
    450                 455                 460
Lys Leu Ala Met Glu Tyr Gly Leu Leu Gln Ile Pro Ser Met Pro Glu
465                 470                 475                 480
Val Lys His His Ser Leu Ser Leu Glu Gly Phe Ile Ser Val Asp Asp
                485                 490                 495
Val Asp Ile Thr Gln Ile Lys Tyr Lys Asp Lys Ala Arg Glu Lys Gln
            500                 505                 510
Arg Lys Lys Ala Leu Lys Arg Lys Ala Glu Glu Glu Ala Gln Asn Pro
        515                 520                 525
Lys Pro Glu Arg Lys Arg Pro Pro Glu Lys Pro Glu Lys Pro Lys Arg
    530                 535                 540
Lys Lys Thr Gly Lys Gln Arg Gln Ser Ile Gln Thr Lys Glu Asp Leu
545                 550                 555                 560
Asp Glu Leu Ala His Glu Tyr Arg Leu Leu Lys Lys Leu Lys Arg Gly
                565                 570                 575
Asp Ile Asp Glu Glu Glu Tyr Glu Lys Leu Thr Gly Phe Gly Asp Ser
            580                 585                 590
Asp Gly Glu Ala Ser Asp Gly Asp Gly Ser Asn Leu Asp Glu Arg Lys
        595                 600                 605
Lys Gly Asn Lys Ala Gln Lys Lys Pro Lys Gln Arg Gly Lys Cys Lys
    610                 615                 620
Ser Gly Ser Lys Lys Phe Glu Gly Arg Ser Lys Leu Arg Ser Lys Arg
625                 630                 635                 640
Arg

<210> 145
<211> 1233
<212> DNA
<213> Zea mays

<220>
<221> CDS
<222> (1)..(1233)

<400> 145
atg gca gga ttg gca cca gaa ggg tcg caa ttt gat gac aag cag tat 48
Met Ala Gly Leu Ala Pro Glu Gly Ser Gln Phe Asp Asp Lys Gln Tyr
1               5                   10                  15
gac aag aaa atg cag gag ata tta act gaa gac ttc ttc act tca tat 96
Asp Lys Lys Met Gln Glu Ile Leu Thr Glu Asp Phe Phe Thr Ser Tyr
            20                  25                  30
gat gat gtt tgt gaa agt ttt gac agc atg gga ctg caa gag aac ctt 144
Asp Asp Val Cys Glu Ser Phe Asp Ser Met Gly Leu Gln Glu Asn Leu
        35                  40                  45
ctg aga ggc atc tat gcc tat ggt ttt gag aag cca tcg gct att cag 192
Leu Arg Gly Ile Tyr Ala Tyr Gly Phe Glu Lys Pro Ser Ala Ile Gln
    50                  55                  60
caa agg gga ata gtt ccc ttc tgc aag ggg ctt gac gtg att cag caa 240
Gln Arg Gly Ile Val Pro Phe Cys Lys Gly Leu Asp Val Ile Gln Gln
65                  70                  75                  80
gct cag tct gga act gga aaa aca gct acc ttc tgt tct gga atc ttg 288
Ala Gln Ser Gly Thr Gly Lys Thr Ala Thr Phe Cys Ser Gly Ile Leu
                85                  90                  95
cag caa ctt gat tat gga ttg gtt gaa tgc cag gca ttg gtt ctt gct 336
Gln Gln Leu Asp Tyr Gly Leu Val Glu Cys Gln Ala Leu Val Leu Ala
            100                 105                 110
cca acc cgt gag ctt gct cag caa att gag aag gtt atg cgt gct ctt 384
Pro Thr Arg Glu Leu Ala Gln Gln Ile Glu Lys Val Met Arg Ala Leu
        115                 120                 125
ggg gac tat ttg ggt gtc aaa gtg cat gcc tgt gtt ggt gga aca tct 432
Gly Asp Tyr Leu Gly Val Lys Val His Ala Cys Val Gly Gly Thr Ser
    130                 135                 140
gtt cgt gag gac caa agg att ctt gcc agt ggt gtg cat gtt gtt gtg 480
Val Arg Glu Asp Gln Arg Ile Leu Ala Ser Gly Val His Val Val Val
145                 150                 155                 160
ggc aca cca gga cgt gtg ttt gat atg ttg cgc agg cag tct ctc cgt 528
Gly Thr Pro Gly Arg Val Phe Asp Met Leu Arg Arg Gln Ser Leu Arg
                165                 170                 175
ccc gac aac atc aag atg ttt gtg ttg gat gag gct gat gag atg ctc 576
Pro Asp Asn Ile Lys Met Phe Val Leu Asp Glu Ala Asp Glu Met Leu
            180                 185                 190
tcc cgt gga ttc aag gat cag atc tat gat atc ttc cag ctt ctt cca 624
Ser Arg Gly Phe Lys Asp Gln Ile Tyr Asp Ile Phe Gln Leu Leu Pro
        195                 200                 205
tcc aag att cag gtc ggt gtc ttc tca gct acc atg ccc cct gag gcc 672
Ser Lys Ile Gln Val Gly Val Phe Ser Ala Thr Met Pro Pro Glu Ala
    210                 215                 220
ctt gag atc acg cgc aag ttc atg aac aaa cct gtg agg att ctc gtc 720
Leu Glu Ile Thr Arg Lys Phe Met Asn Lys Pro Val Arg Ile Leu Val
225                 230                 235                 240
aag aga gat gag ctc acc ctt gag ggt atc aag caa ttc tat gtc aat 768
Lys Arg Asp Glu Leu Thr Leu Glu Gly Ile Lys Gln Phe Tyr Val Asn
                245                 250                 255
atc gac aag gaa gat tgg aag ctc gac act ctg tgt gac ctg tat gag 816
Ile Asp Lys Glu Asp Trp Lys Leu Asp Thr Leu Cys Asp Leu Tyr Glu
            260                 265                 270
acc ctg gca atc acc cag agt gtc atc ttt gtg aac aca cgc cgc aag 864
Thr Leu Ala Ile Thr Gln Ser Val Ile Phe Val Asn Thr Arg Arg Lys
        275                 280                 285
gtg gac tgg ctc acg gac aag atg agg agc agg gac cac acc gtg tca 912
Val Asp Trp Leu Thr Asp Lys Met Arg Ser Arg Asp His Thr Val Ser
    290                 295                 300
gct aca cac ggt gat atg gac cag aac acc agg gac atc atc atg agg 960
Ala Thr His Gly Asp Met Asp Gln Asn Thr Arg Asp Ile Ile Met Arg
305                 310                 315                 320
gag ttc agg tcc ggg tcc tct cgt gtg ctc atc acc acc gac ctg ctc 1008
Glu Phe Arg Ser Gly Ser Ser Arg Val Leu Ile Thr Thr Asp Leu Leu
                325                 330                 335
gcc cgt ggt atc gac gtc cag cag gtc tca ctt gtc atc aac tac gac 1056

Ala Arg Gly Ile Asp Val Gln Gln Val Ser Leu Val Ile Asn Tyr Asp
            340                 345                 350
ctg ccg acc cag cca gag aac tac ctc cac cgc atc ggc cgt agt ggt      1104
Leu Pro Thr Gln Pro Glu Asn Tyr Leu His Arg Ile Gly Arg Ser Gly
        355                 360                 365
cgg ttt gga agg aag ggt gtt gcc atc aac ttc gtc acc cgc gac gac      1152
Arg Phe Gly Arg Lys Gly Val Ala Ile Asn Phe Val Thr Arg Asp Asp
    370                 375                 380
gag cga atg ctg ttt gac atc cag agg ttc tac aac gtg acc gtt gag      1200
Glu Arg Met Leu Phe Asp Ile Gln Arg Phe Tyr Asn Val Thr Val Glu
385                 390                 395                 400
gag ctg ccg gcc aat gtc gcc gac ctt ctc tag                          1233
Glu Leu Pro Ala Asn Val Ala Asp Leu Leu
                405                 410

<210> 146
<211> 410
<212> PRT
<213> Zea mays

<400> 146
Met Ala Gly Leu Ala Pro Glu Gly Ser Gln Phe Asp Asp Lys Gln Tyr
1               5                   10                  15
Asp Lys Lys Met Gln Glu Ile Leu Thr Glu Asp Phe Phe Thr Ser Tyr
            20                  25                  30
Asp Asp Val Cys Glu Ser Phe Asp Ser Met Gly Leu Gln Glu Asn Leu
        35                  40                  45
Leu Arg Gly Ile Tyr Ala Tyr Gly Phe Glu Lys Pro Ser Ala Ile Gln
    50                  55                  60
Gln Arg Gly Ile Val Pro Phe Cys Lys Gly Leu Asp Val Ile Gln Gln
65                  70                  75                  80
Ala Gln Ser Gly Thr Gly Lys Thr Ala Thr Phe Cys Ser Gly Ile Leu
                85                  90                  95
Gln Gln Leu Asp Tyr Gly Leu Val Glu Cys Gln Ala Leu Val Leu Ala
            100                 105                 110
Pro Thr Arg Glu Leu Ala Gln Gln Ile Glu Lys Val Met Arg Ala Leu
        115                 120                 125
Gly Asp Tyr Leu Gly Val Lys Val His Ala Cys Val Gly Gly Thr Ser
    130                 135                 140
Val Arg Glu Asp Gln Arg Ile Leu Ala Ser Gly Val His Val Val Val
145                 150                 155                 160
Gly Thr Pro Gly Arg Val Phe Asp Met Leu Arg Arg Gln Ser Leu Arg
                165                 170                 175
Pro Asp Asn Ile Lys Met Phe Val Leu Asp Glu Ala Asp Glu Met Leu
            180                 185                 190
Ser Arg Gly Phe Lys Asp Gln Ile Tyr Asp Ile Phe Gln Leu Leu Pro
        195                 200                 205
Ser Lys Ile Gln Val Gly Val Phe Ser Ala Thr Met Pro Pro Glu Ala
    210                 215                 220
Leu Glu Ile Thr Arg Lys Phe Met Asn Lys Pro Val Arg Ile Leu Val
225                 230                 235                 240
Lys Arg Asp Glu Leu Thr Leu Glu Gly Ile Lys Gln Phe Tyr Val Asn
                245                 250                 255
Ile Asp Lys Glu Asp Trp Lys Leu Asp Thr Leu Cys Asp Leu Tyr Glu
            260                 265                 270
Thr Leu Ala Ile Thr Gln Ser Val Ile Phe Val Asn Thr Arg Arg Lys
        275                 280                 285
Val Asp Trp Leu Thr Asp Lys Met Arg Ser Arg Asp His Thr Val Ser
    290                 295                 300
Ala Thr His Gly Asp Met Asp Gln Asn Thr Arg Asp Ile Ile Met Arg
305                 310                 315                 320
Glu Phe Arg Ser Gly Ser Ser Arg Val Leu Ile Thr Thr Asp Leu Leu
                325                 330                 335
Ala Arg Gly Ile Asp Val Gln Gln Val Ser Leu Val Ile Asn Tyr Asp
            340                 345                 350
Leu Pro Thr Gln Pro Glu Asn Tyr Leu His Arg Ile Gly Arg Ser Gly
        355                 360                 365
Arg Phe Gly Arg Lys Gly Val Ala Ile Asn Phe Val Thr Arg Asp Asp
    370                 375                 380

Glu Arg Met Leu Phe Asp Ile Gln Arg Phe Tyr Asn Val Thr Val Glu
385                 390                 395                 400
Glu Leu Pro Ala Asn Val Ala Asp Leu Leu
                405                 410

<210> 147
<211> 1224
<212> DNA
<213> Zea mays

<220>
<221> CDS
<222> (1)..(1224)

<400> 147
atg gcg gcg ccc acc acc tcc cgc cgc ggc ccc ggc ggc gcg cgc aac      48
Met Ala Ala Pro Thr Thr Ser Arg Arg Gly Pro Gly Gly Ala Arg Asn
1               5                   10                  15
atg gac gac gag aac cta acc ttc gag acc tcc ccg ggg gtc gag gtc      96
Met Asp Asp Glu Asn Leu Thr Phe Glu Thr Ser Pro Gly Val Glu Val
            20                  25                  30
gtc agc agc ttc gac cag atg ggc atc aag gac gac ctc ctc cgc ggc      144
Val Ser Ser Phe Asp Gln Met Gly Ile Lys Asp Asp Leu Leu Arg Gly
        35                  40                  45
atc tac ggc tac ggt ttc gag aag ccc tcc gcc atc cag cag cgc gcc      192
Ile Tyr Gly Tyr Gly Phe Glu Lys Pro Ser Ala Ile Gln Gln Arg Ala
    50                  55                  60
gtg ctc ccc atc atc aac ggc cgc gac gtc atc gcg cag gcc cag tcc      240
Val Leu Pro Ile Ile Asn Gly Arg Asp Val Ile Ala Gln Ala Gln Ser
65                  70                  75                  80
ggt acc ggc aag acg tcc atg atc tcc ctc acc gtc tgc cag atc gtc      288
Gly Thr Gly Lys Thr Ser Met Ile Ser Leu Thr Val Cys Gln Ile Val
                85                  90                  95
gac acc gcc gtg cgc gag gtg cag gct ttg att ctc tcg cct act agg      336
Asp Thr Ala Val Arg Glu Val Gln Ala Leu Ile Leu Ser Pro Thr Arg
            100                 105                 110
gag ctt gct tcc caa aca gag aga gtt atg ctg gct att ggg gac cac      384
Glu Leu Ala Ser Gln Thr Glu Arg Val Met Leu Ala Ile Gly Asp His
        115                 120                 125
ctc aac gtc caa gtg cac gct tgc att ggt gga aag agt att ggt gag      432
Leu Asn Val Gln Val His Ala Cys Ile Gly Gly Lys Ser Ile Gly Glu
    130                 135                 140
gac atc agg agg ctc gag aat ggg gtg cat gtc gtc tct gga act cca      480
Asp Ile Arg Arg Leu Glu Asn Gly Val His Val Val Ser Gly Thr Pro
145                 150                 155                 160
ggc aga gtc tgt gat atg atc aag agg agg aca ctg cgg acc aga gcc      528
Gly Arg Val Cys Asp Met Ile Lys Arg Arg Thr Leu Arg Thr Arg Ala
                165                 170                 175
atc aag ctt cta gtt ctg gat gaa gct gat gag atg ttg agt aga ggc      576
Ile Lys Leu Leu Val Leu Asp Glu Ala Asp Glu Met Leu Ser Arg Gly
            180                 185                 190
ttt aag gat cag att tac gat gtc tac aga tat ctc cct cca gaa ctt      624
Phe Lys Asp Gln Ile Tyr Asp Val Tyr Arg Tyr Leu Pro Pro Glu Leu
        195                 200                 205
cag gtg gtt ttg atc tct gca act ctt cct cat gag atc ttg gag atg      672
Gln Val Val Leu Ile Ser Ala Thr Leu Pro His Glu Ile Leu Glu Met
    210                 215                 220
act agt aag ttc atg acc gaa ccg gtt agg atc ctt gtg aag cgt gat      720
Thr Ser Lys Phe Met Thr Glu Pro Val Arg Ile Leu Val Lys Arg Asp
225                 230                 235                 240
gag ctg acc ctg gag ggc atc aaa caa ttc ttt gtt gct gtt gag aaa      768
Glu Leu Thr Leu Glu Gly Ile Lys Gln Phe Phe Val Ala Val Glu Lys
                245                 250                 255
gag gag tgg aag ttt gat aca ctt tgt gat ctt tat gat aca ctc acc      816
Glu Glu Trp Lys Phe Asp Thr Leu Cys Asp Leu Tyr Asp Thr Leu Thr
            260                 265                 270
atc acc caa gct gtt att ttc tgc aat act aag aga aag gtg gat tgg      864
Ile Thr Gln Ala Val Ile Phe Cys Asn Thr Lys Arg Lys Val Asp Trp
        275                 280                 285

ctc act gaa aaa atg cgt aca aat aac ttc acc gta tca gct atg cac 912
Leu Thr Glu Lys Met Arg Thr Asn Asn Phe Thr Val Ser Ala Met His
    290                 295                 300
ggt gac atg ccc caa caa gaa agg gat gcc att atg ggt gag ttc agg 960
Gly Asp Met Pro Gln Gln Glu Arg Asp Ala Ile Met Gly Glu Phe Arg
305                 310                 315                 320
tct ggc gca act cgt gtg cta ata act aca gat gtt tgg gct cga gga 1008
Ser Gly Ala Thr Arg Val Leu Ile Thr Thr Asp Val Trp Ala Arg Gly
                325                 330                 335
ctg gac gtt cag cag gtt tca ctt gtc ata aat tat gat ctc cca aat 1056
Leu Asp Val Gln Gln Val Ser Leu Val Ile Asn Tyr Asp Leu Pro Asn
            340                 345                 350
aat cgt gag ctt tac atc cat cgc att ggt cgt tct ggt cgt ttt ggg 1104
Asn Arg Glu Leu Tyr Ile His Arg Ile Gly Arg Ser Gly Arg Phe Gly
        355                 360                 365
cgc aag ggt gtg gcg atc aat ttc gtg cgc aag gat gac atc cgt ata 1152
Arg Lys Gly Val Ala Ile Asn Phe Val Arg Lys Asp Asp Ile Arg Ile
    370                 375                 380
ctg aga gat ata gag cag tac tac agc aca caa att gac gag atg cca 1200
Leu Arg Asp Ile Glu Gln Tyr Tyr Ser Thr Gln Ile Asp Glu Met Pro
385                 390                 395                 400
atg aat gtt gct gat ctt att tga 1224
Met Asn Val Ala Asp Leu Ile
                405

<210> 148
<211> 407
<212> PRT
<213> Zea mays

<400> 148
Met Ala Ala Pro Thr Thr Ser Arg Arg Gly Pro Gly Gly Ala Arg Asn
1               5                   10                  15
Met Asp Asp Glu Asn Leu Thr Phe Glu Thr Ser Pro Gly Val Glu Val
            20                  25                  30
Val Ser Ser Phe Asp Gln Met Gly Ile Lys Asp Asp Leu Leu Arg Gly
        35                  40                  45
Ile Tyr Gly Tyr Gly Phe Glu Lys Pro Ser Ala Ile Gln Gln Arg Ala
    50                  55                  60
Val Leu Pro Ile Ile Asn Gly Arg Asp Val Ile Ala Gln Ala Gln Ser
65                  70                  75                  80
Gly Thr Gly Lys Thr Ser Met Ile Ser Leu Thr Val Cys Gln Ile Val
                85                  90                  95
Asp Thr Ala Val Arg Glu Val Gln Ala Leu Ile Leu Ser Pro Thr Arg
            100                 105                 110
Glu Leu Ala Ser Gln Thr Glu Arg Val Met Leu Ala Ile Gly Asp His
        115                 120                 125
Leu Asn Val Gln Val His Ala Cys Ile Gly Gly Lys Ser Ile Gly Glu
    130                 135                 140
Asp Ile Arg Arg Leu Glu Asn Gly Val His Val Val Ser Gly Thr Pro
145                 150                 155                 160
Gly Arg Val Cys Asp Met Ile Lys Arg Arg Thr Leu Arg Thr Arg Ala
                165                 170                 175
Ile Lys Leu Leu Val Leu Asp Glu Ala Asp Glu Met Leu Ser Arg Gly
            180                 185                 190
Phe Lys Asp Gln Ile Tyr Asp Val Tyr Arg Tyr Leu Pro Pro Glu Leu
        195                 200                 205
Gln Val Val Leu Ile Ser Ala Thr Leu Pro His Glu Ile Leu Glu Met
    210                 215                 220
Thr Ser Lys Phe Met Thr Glu Pro Val Arg Ile Leu Val Lys Arg Asp
225                 230                 235                 240
Glu Leu Thr Leu Glu Gly Ile Lys Gln Phe Phe Val Ala Val Glu Lys
                245                 250                 255
Glu Glu Trp Lys Phe Asp Thr Leu Cys Asp Leu Tyr Asp Thr Leu Thr
            260                 265                 270
Ile Thr Gln Ala Val Ile Phe Cys Asn Thr Lys Arg Lys Val Asp Trp
        275                 280                 285
Leu Thr Glu Lys Met Arg Thr Asn Asn Phe Thr Val Ser Ala Met His
    290                 295                 300

Gly Asp Met Pro Gln Gln Glu Arg Asp Ala Ile Met Gly Glu Phe Arg
305                 310                 315                 320
Ser Gly Ala Thr Arg Val Leu Ile Thr Thr Asp Val Trp Ala Arg Gly
                325                 330                 335
Leu Asp Val Gln Gln Val Ser Leu Val Ile Asn Tyr Asp Leu Pro Asn
            340                 345                 350
Asn Arg Glu Leu Tyr Ile His Arg Ile Gly Arg Ser Gly Arg Phe Gly
        355                 360                 365
Arg Lys Gly Val Ala Ile Asn Phe Val Arg Lys Asp Asp Ile Arg Ile
    370                 375                 380
Leu Arg Asp Ile Glu Gln Tyr Tyr Ser Thr Gln Ile Asp Glu Met Pro
385                 390                 395                 400
Met Asn Val Ala Asp Leu Ile
                405

<210> 149
<211> 1944
<212> DNA
<213> Zea mays

<220>
<221> CDS
<222> (1)..(1944)

<400> 149
atg cga tct tca tgg gct gac tca gtt gcg aac gcc gag gaa tcg gcg      48
Met Arg Ser Ser Trp Ala Asp Ser Val Ala Asn Ala Glu Glu Ser Ala
1               5                   10                  15
ccc gcg act gct gct gct aat ggc tct gtt gca act cat agc acc tcg      96
Pro Ala Thr Ala Ala Ala Asn Gly Ser Val Ala Thr His Ser Thr Ser
            20                  25                  30
cgc cct acg cgc agc tcc tac gtg cct cca cat ctt cgt ggc cgc tca      144
Arg Pro Thr Arg Ser Ser Tyr Val Pro Pro His Leu Arg Gly Arg Ser
        35                  40                  45
gct ggt gct gct gtt gaa gct caa gca ggc tta gta gca cca gca caa      192
Ala Gly Ala Ala Val Glu Ala Gln Ala Gly Leu Val Ala Pro Ala Gln
    50                  55                  60
ggt gga cca ctg cca ttg gct gct gca caa cct tct ggt cag ggt gct      240
Gly Gly Pro Leu Pro Leu Ala Ala Ala Gln Pro Ser Gly Gln Gly Ala
65                  70                  75                  80
gct gtt ggt ggc cct cgc tgg gct ggc att gtg aat ggt ggt ggt ggt      288
Ala Val Gly Gly Pro Arg Trp Ala Gly Ile Val Asn Gly Gly Gly Gly
                85                  90                  95
ggt ggc agc att ggt gct cct cgc cag ggc cat ggt ggc gga ggc ggg      336
Gly Gly Ser Ile Gly Ala Pro Arg Gln Gly His Gly Gly Gly Gly Gly
            100                 105                 110
ggc cgt ggt gct tgg aac tcc cgt cct ggt ggt tgg gac cgc aga gac      384
Gly Arg Gly Ala Trp Asn Ser Arg Pro Gly Gly Trp Asp Arg Arg Asp
        115                 120                 125
cgg gag cca gat ccg ttt gcg aaa gcc gag gct gaa gaa att gat ttc      432
Arg Glu Pro Asp Pro Phe Ala Lys Ala Glu Ala Glu Glu Ile Asp Phe
    130                 135                 140
gac ggc cag gag aat act ggc atc aat ttt gat gcc tat gaa gac atc      480
Asp Gly Gln Glu Asn Thr Gly Ile Asn Phe Asp Ala Tyr Glu Asp Ile
145                 150                 155                 160
cct gtt gag acc agt ggc cat gat gtg cct gca cca gtc aac aca ttt      528
Pro Val Glu Thr Ser Gly His Asp Val Pro Ala Pro Val Asn Thr Phe
                165                 170                 175
gca gag att gat ttg ggt gat gca ttg aat gac aat atc cgg agg tgc      576
Ala Glu Ile Asp Leu Gly Asp Ala Leu Asn Asp Asn Ile Arg Arg Cys
            180                 185                 190
aag tat gtg aaa cca aca cca gtg cag cgt tac gcc atc cca atc tcc      624
Lys Tyr Val Lys Pro Thr Pro Val Gln Arg Tyr Ala Ile Pro Ile Ser
        195                 200                 205
att gct ggg cgg gat ctc atg gcc tgc gca cag act gga tcc gga aaa      672
Ile Ala Gly Arg Asp Leu Met Ala Cys Ala Gln Thr Gly Ser Gly Lys
    210                 215                 220
acc gcc gca ttc tgt ttt cca atc atc agt ggg atc ttg aag tca cca      720
Thr Ala Ala Phe Cys Phe Pro Ile Ile Ser Gly Ile Leu Lys Ser Pro

225                 230                 235                 240
aag cca cac cag agg tca cgg agt aca agg acc gct tgc cct cta gct      768
Lys Pro His Gln Arg Ser Arg Ser Thr Arg Thr Ala Cys Pro Leu Ala
                245                 250                 255
ctg atc tta tca ccc act cgt gag ctt tca gtc caa atc cat gaa gaa      816
Leu Ile Leu Ser Pro Thr Arg Glu Leu Ser Val Gln Ile His Glu Glu
            260                 265                 270
gca agg aag ttt gca tac cag act ggt gtc aga gtt gtg gtt gca tat      864
Ala Arg Lys Phe Ala Tyr Gln Thr Gly Val Arg Val Val Val Ala Tyr
        275                 280                 285
ggt ggc gca cca ata act aac cag ctg agg gag tta gag aga ggt gtg      912
Gly Gly Ala Pro Ile Thr Asn Gln Leu Arg Glu Leu Glu Arg Gly Val
    290                 295                 300
gaa atc ctg gtg gca act cct ggt cgc ttg atg gat ctg ttg gag agg      960
Glu Ile Leu Val Ala Thr Pro Gly Arg Leu Met Asp Leu Leu Glu Arg
305                 310                 315                 320
gct aga gtc tca ctt caa atg ata aag tat tta gct ctt gat gaa gct      1008
Ala Arg Val Ser Leu Gln Met Ile Lys Tyr Leu Ala Leu Asp Glu Ala
                325                 330                 335
gat cgg atg ctt gat atg ggt ttt gag cca cag ata cgt aaa att gtt      1056
Asp Arg Met Leu Asp Met Gly Phe Glu Pro Gln Ile Arg Lys Ile Val
            340                 345                 350
gag ggc atg gac atg cct caa cgt ggt gag agg cag aca atg ttg ttc      1104
Glu Gly Met Asp Met Pro Gln Arg Gly Glu Arg Gln Thr Met Leu Phe
        355                 360                 365
agt gca aca ttc cca aaa gag ata cag agg atg gcc gca gat ttc ctt      1152
Ser Ala Thr Phe Pro Lys Glu Ile Gln Arg Met Ala Ala Asp Phe Leu
    370                 375                 380
gct gat tac atc ttt ctt gct gtt ggg aga gtt ggt tca agc acc gat      1200
Ala Asp Tyr Ile Phe Leu Ala Val Gly Arg Val Gly Ser Ser Thr Asp
385                 390                 395                 400
ttg att gtt cag agg gtg gag ttt gtc ctc gct tca gac aaa cga agc      1248
Leu Ile Val Gln Arg Val Glu Phe Val Leu Ala Ser Asp Lys Arg Ser
                405                 410                 415
tac ctc atg gat ctt ctg cat gca caa aag gct aat ggc aca cat gga      1296
Tyr Leu Met Asp Leu Leu His Ala Gln Lys Ala Asn Gly Thr His Gly
            420                 425                 430
aag cac gct ctt aca ttg gtc ttt gtg gag aca aag agg ggg gct gat      1344
Lys His Ala Leu Thr Leu Val Phe Val Glu Thr Lys Arg Gly Ala Asp
        435                 440                 445
gcc ttg gaa gac ggg ttt ttt aga aat gga cat cca tgg act agc att      1392
Ala Leu Glu Asp Gly Phe Phe Arg Asn Gly His Pro Trp Thr Ser Ile
    450                 455                 460
cat gga gac agg aca caa cag gaa agg gag cat gcc ctt agg tcc ttc      1440
His Gly Asp Arg Thr Gln Gln Glu Arg Glu His Ala Leu Arg Ser Phe
465                 470                 475                 480
aag agc gga gca act ccc atc ctt gtg gcg act gat gtt gct gct cgt      1488
Lys Ser Gly Ala Thr Pro Ile Leu Val Ala Thr Asp Val Ala Ala Arg
                485                 490                 495
ggt ctt gac ata cca cat gtt gcc cat gtg att aat ttt gac ctc cct      1536
Gly Leu Asp Ile Pro His Val Ala His Val Ile Asn Phe Asp Leu Pro
            500                 505                 510
aat gat ata gat gac tat gtt cat cgg att gga agg act gga cgt gct      1584
Asn Asp Ile Asp Asp Tyr Val His Arg Ile Gly Arg Thr Gly Arg Ala
        515                 520                 525
ggg aaa tct ggt ctt gcg act gca ttc ttc aac gag agc aac act acg      1632
Gly Lys Ser Gly Leu Ala Thr Ala Phe Phe Asn Glu Ser Asn Thr Thr
    530                 535                 540
ctt gca agg ccg ttg agt gat ctc atg aaa gag gcc aac cag gag gtt      1680
Leu Ala Arg Pro Leu Ser Asp Leu Met Lys Glu Ala Asn Gln Glu Val
545                 550                 555                 560
cct aag tgg ctt gag gga tac gct gcc cga tca gcc tac gga ggt gga      1728
Pro Lys Trp Leu Glu Gly Tyr Ala Ala Arg Ser Ala Tyr Gly Gly Gly
                565                 570                 575
ggt ggt aga aac cgc aga caa ggt agc agt gcc aga ttt ggt ggc cgt      1776
Gly Gly Arg Asn Arg Arg Gln Gly Ser Ser Ala Arg Phe Gly Gly Arg
            580                 585                 590
gat ttc cgg cga gat agg ggc agt ggt gga gga tat ggt ggt ggt tcc      1824
Asp Phe Arg Arg Asp Arg Gly Ser Gly Gly Gly Tyr Gly Gly Gly Ser

                595                 600                 605
tac gga gga ggt ggt ggt ggt gga tat ggg gga tca tca gga tac ggt        1872
Tyr Gly Gly Gly Gly Gly Gly Gly Tyr Gly Gly Ser Ser Gly Tyr Gly
    610                 615                 620
ggt gcc tat ggc ggc ggt ggc agc agc ggc ggc ggc tat ggt ggt ggt        1920
Gly Ala Tyr Gly Gly Gly Gly Ser Ser Gly Gly Gly Tyr Gly Gly Gly
625                 630                 635                 640
cag agt acg agt tct tgg gac tga                                        1944
Gln Ser Thr Ser Ser Trp Asp
                645

<210> 150
<211> 647
<212> PRT
<213> Zea mays

<400> 150
Met Arg Ser Ser Trp Ala Asp Ser Val Ala Asn Ala Glu Glu Ser Ala
1               5                   10                  15
Pro Ala Thr Ala Ala Ala Asn Gly Ser Val Ala Thr His Ser Thr Ser
            20                  25                  30
Arg Pro Thr Arg Ser Ser Tyr Val Pro Pro His Leu Arg Gly Arg Ser
        35                  40                  45
Ala Gly Ala Ala Val Glu Ala Gln Ala Gly Leu Val Ala Pro Ala Gln
    50                  55                  60
Gly Gly Pro Leu Pro Leu Ala Ala Ala Gln Pro Ser Gly Gln Gly Ala
65                  70                  75                  80
Ala Val Gly Gly Pro Arg Trp Ala Gly Ile Val Asn Gly Gly Gly Gly
                85                  90                  95
Gly Gly Ser Ile Gly Ala Pro Arg Gln Gly His Gly Gly Gly Gly Gly
            100                 105                 110
Gly Arg Gly Ala Trp Asn Ser Arg Pro Gly Gly Trp Asp Arg Arg Asp
        115                 120                 125
Arg Glu Pro Asp Pro Phe Ala Lys Ala Glu Ala Glu Glu Ile Asp Phe
    130                 135                 140
Asp Gly Gln Glu Asn Thr Gly Ile Asn Phe Asp Ala Tyr Glu Asp Ile
145                 150                 155                 160
Pro Val Glu Thr Ser Gly His Asp Val Pro Ala Pro Val Asn Thr Phe
                165                 170                 175
Ala Glu Ile Asp Leu Gly Asp Ala Leu Asn Asp Asn Ile Arg Arg Cys
            180                 185                 190
Lys Tyr Val Lys Pro Thr Pro Val Gln Arg Tyr Ala Ile Pro Ile Ser
        195                 200                 205
Ile Ala Gly Arg Asp Leu Met Ala Cys Ala Gln Thr Gly Ser Gly Lys
    210                 215                 220
Thr Ala Ala Phe Cys Phe Pro Ile Ile Ser Gly Ile Leu Lys Ser Pro
225                 230                 235                 240
Lys Pro His Gln Arg Ser Arg Ser Thr Arg Thr Ala Cys Pro Leu Ala
                245                 250                 255
Leu Ile Leu Ser Pro Thr Arg Glu Leu Ser Val Gln Ile His Glu Glu
            260                 265                 270
Ala Arg Lys Phe Ala Tyr Gln Thr Gly Val Arg Val Val Val Ala Tyr
        275                 280                 285
Gly Gly Ala Pro Ile Thr Asn Gln Leu Arg Glu Leu Glu Arg Gly Val
    290                 295                 300
Glu Ile Leu Val Ala Thr Pro Gly Arg Leu Met Asp Leu Leu Glu Arg
305                 310                 315                 320
Ala Arg Val Ser Leu Gln Met Ile Lys Tyr Leu Ala Leu Asp Glu Ala
                325                 330                 335
Asp Arg Met Leu Asp Met Gly Phe Glu Pro Gln Ile Arg Lys Ile Val
            340                 345                 350
Glu Gly Met Asp Met Pro Gln Arg Gly Glu Arg Gln Thr Met Leu Phe
        355                 360                 365
Ser Ala Thr Phe Pro Lys Glu Ile Gln Arg Met Ala Ala Asp Phe Leu
    370                 375                 380
Ala Asp Tyr Ile Phe Leu Ala Val Gly Arg Val Gly Ser Ser Thr Asp
385                 390                 395                 400
Leu Ile Val Gln Arg Val Glu Phe Val Leu Ala Ser Asp Lys Arg Ser
                405                 410                 415

Tyr Leu Met Asp Leu Leu His Ala Gln Lys Ala Asn Gly Thr His Gly
            420                 425                 430
Lys His Ala Leu Thr Leu Val Phe Val Glu Thr Lys Arg Gly Ala Asp
        435                 440                 445
Ala Leu Glu Asp Gly Phe Phe Arg Asn Gly His Pro Trp Thr Ser Ile
    450                 455                 460
His Gly Asp Arg Thr Gln Gln Glu Arg Glu His Ala Leu Arg Ser Phe
465                 470                 475                 480
Lys Ser Gly Ala Thr Pro Ile Leu Val Ala Thr Asp Val Ala Ala Arg
                485                 490                 495
Gly Leu Asp Ile Pro His Val Ala His Val Ile Asn Phe Asp Leu Pro
            500                 505                 510
Asn Asp Ile Asp Asp Tyr Val His Arg Ile Gly Arg Thr Gly Arg Ala
        515                 520                 525
Gly Lys Ser Gly Leu Ala Thr Ala Phe Phe Asn Glu Ser Asn Thr Thr
    530                 535                 540
Leu Ala Arg Pro Leu Ser Asp Leu Met Lys Glu Ala Asn Gln Glu Val
545                 550                 555                 560
Pro Lys Trp Leu Glu Gly Tyr Ala Ala Arg Ser Ala Tyr Gly Gly Gly
                565                 570                 575
Gly Gly Arg Asn Arg Arg Gln Gly Ser Ser Ala Arg Phe Gly Gly Arg
            580                 585                 590
Asp Phe Arg Arg Asp Arg Gly Ser Gly Gly Gly Tyr Gly Gly Gly Ser
        595                 600                 605
Tyr Gly Gly Gly Gly Gly Gly Gly Tyr Gly Gly Ser Ser Gly Tyr Gly
    610                 615                 620
Gly Ala Tyr Gly Gly Gly Gly Ser Ser Gly Gly Gly Tyr Gly Gly Gly
625                 630                 635                 640
Gln Ser Thr Ser Ser Trp Asp
                645

<210> 151
<211> 1584
<212> DNA
<213> Zea mays

<220>
<221> CDS
<222> (1)..(1584)

<400> 151
atg gat cca cgc gcc agg tat cct tcg ggc atg ggc aac ggc cgc ggt      48
Met Asp Pro Arg Ala Arg Tyr Pro Ser Gly Met Gly Asn Gly Arg Gly
1               5                   10                  15
ggg aac ctc aac tac tac ggc cgc ggc cca ccg ccg caa cag ccg cat      96
Gly Asn Leu Asn Tyr Tyr Gly Arg Gly Pro Pro Pro Gln Gln Pro His
            20                  25                  30
cag cac cac aac cac cag cag acg tcg ggg gcg cac cac cac cag cag      144
Gln His His Asn His Gln Gln Thr Ser Gly Ala His His His Gln Gln
        35                  40                  45
acg tcg ggg gcg cac cac cac cag cag tac gcg cag agg cag cac cag      192
Thr Ser Gly Ala His His His Gln Gln Tyr Ala Gln Arg Gln His Gln
    50                  55                  60
cac cag cac cag cac cac ggc cac aat cgc cat cag caa cag cag cag      240
His Gln His Gln His His Gly His Asn Arg His Gln Gln Gln Gln Gln
65                  70                  75                  80
cac cac att tac cag cag caa cag cag cag tgg ctg cgg cgg agc cag      288
His His Ile Tyr Gln Gln Gln Gln Gln Gln Trp Leu Arg Arg Ser Gln
                85                  90                  95
att gcc cgc gag acc tcg ggc gca gcc gtg acc agc gag ccg aag tcc      336
Ile Ala Arg Glu Thr Ser Gly Ala Ala Val Thr Ser Glu Pro Lys Ser
            100                 105                 110
ctg gcg cca tcc acg gcc gcc gat ggc gtc gat tcg agt tca caa gat      384
Leu Ala Pro Ser Thr Ala Ala Asp Gly Val Asp Ser Ser Ser Gln Asp
        115                 120                 125
tgg aag gcc caa ttg aag ctg ccg cct cca gat aca cgc tac agg aca      432
Trp Lys Ala Gln Leu Lys Leu Pro Pro Pro Asp Thr Arg Tyr Arg Thr
    130                 135                 140
gag gat gtt aca gct acc aag gga aat gag ttt gaa gac tat ttt ctc      480

Glu Asp Val Thr Ala Thr Lys Gly Asn Glu Phe Glu Asp Tyr Phe Leu
145                 150                 155                 160
aag cgt gaa ctc cta atg ggt ata tac gag aag gga ttt gaa aga ccg     528
Lys Arg Glu Leu Leu Met Gly Ile Tyr Glu Lys Gly Phe Glu Arg Pro
                165                 170                 175
tct cct atc caa gag gaa agc att cct att gct tta act ggt agt gac     576
Ser Pro Ile Gln Glu Glu Ser Ile Pro Ile Ala Leu Thr Gly Ser Asp
            180                 185                 190
att ctt gca aga gcg aag aac gga act ggc aag act gct gca ttt tgt     624
Ile Leu Ala Arg Ala Lys Asn Gly Thr Gly Lys Thr Ala Ala Phe Cys
        195                 200                 205
att cct gca ctg gaa aag att gat caa gaa aaa aat gct att caa gtt     672
Ile Pro Ala Leu Glu Lys Ile Asp Gln Glu Lys Asn Ala Ile Gln Val
    210                 215                 220
gtt ata ttg gtt ccc aca agg gaa ttg gct ctg caa aca tca caa gtt     720
Val Ile Leu Val Pro Thr Arg Glu Leu Ala Leu Gln Thr Ser Gln Val
225                 230                 235                 240
tgc aag gag ctt ggt aag cat ttg aag atc cag gtt atg gtc acc act     768
Cys Lys Glu Leu Gly Lys His Leu Lys Ile Gln Val Met Val Thr Thr
                245                 250                 255
ggt gga acc agt ttg aag gat gat att gtt cgt ctg tac caa cct gtg     816
Gly Gly Thr Ser Leu Lys Asp Asp Ile Val Arg Leu Tyr Gln Pro Val
            260                 265                 270
cac tta ctc gtt gga aca cct ggg cgt att ttg gat ctt aca aag aaa     864
His Leu Leu Val Gly Thr Pro Gly Arg Ile Leu Asp Leu Thr Lys Lys
        275                 280                 285
ggt gtt tgc att ttg aac gac tgt tca atg ctt att atg gat gag gca     912
Gly Val Cys Ile Leu Asn Asp Cys Ser Met Leu Ile Met Asp Glu Ala
    290                 295                 300
gac aag ctt ctc tct cct gag ttt caa cct tcc ata gaa cag ttg att     960
Asp Lys Leu Leu Ser Pro Glu Phe Gln Pro Ser Ile Glu Gln Leu Ile
305                 310                 315                 320
cgc tac ctc cct gct agt cga cag att ttg atg ttt tca gcg aca ttc     1008
Arg Tyr Leu Pro Ala Ser Arg Gln Ile Leu Met Phe Ser Ala Thr Phe
                325                 330                 335
cct gtg act gtt aag gag ttt aag gac aaa tat ctg cct aaa cct tat     1056
Pro Val Thr Val Lys Glu Phe Lys Asp Lys Tyr Leu Pro Lys Pro Tyr
            340                 345                 350
gtg att aac ctt atg gac gaa ctt aca ctg aag gga att aca cag ttc     1104
Val Ile Asn Leu Met Asp Glu Leu Thr Leu Lys Gly Ile Thr Gln Phe
        355                 360                 365
tat gct ttt gtg gaa gaa aga cag aaa gtt cac tgt ctc aac act ctc     1152
Tyr Ala Phe Val Glu Glu Arg Gln Lys Val His Cys Leu Asn Thr Leu
    370                 375                 380
ttt tcc aag ctt caa atc aac cag tca ata ata ttt tgt aac tct gta     1200
Phe Ser Lys Leu Gln Ile Asn Gln Ser Ile Ile Phe Cys Asn Ser Val
385                 390                 395                 400
aat cgt gtt gaa cta ttg gcg aaa aag atc aca gag ctt ggc tat tct     1248
Asn Arg Val Glu Leu Leu Ala Lys Lys Ile Thr Glu Leu Gly Tyr Ser
                405                 410                 415
tgc ttc tat att cat gca aag atg cta cag gac cac cgt aat aga gtt     1296
Cys Phe Tyr Ile His Ala Lys Met Leu Gln Asp His Arg Asn Arg Val
            420                 425                 430
ttc cat gat ttc cgt aat gga gca tgc agg aac ctt gtt tgc act gat     1344
Phe His Asp Phe Arg Asn Gly Ala Cys Arg Asn Leu Val Cys Thr Asp
        435                 440                 445
ctt ttt aca agg gga ata gat att caa gct gtg aat gtt gtt atc aat     1392
Leu Phe Thr Arg Gly Ile Asp Ile Gln Ala Val Asn Val Val Ile Asn
    450                 455                 460
ttt gat ttc ccc aag aac tct gaa aca tat ctc cac agg gtt ggt aga     1440
Phe Asp Phe Pro Lys Asn Ser Glu Thr Tyr Leu His Arg Val Gly Arg
465                 470                 475                 480
tct ggg aga ttt ggt cac ctt ggt ctg gcc gtt aat ttg atc acc tat     1488
Ser Gly Arg Phe Gly His Leu Gly Leu Ala Val Asn Leu Ile Thr Tyr
                485                 490                 495
gag gat cgg ttt aac ttg tat agg att gaa caa gag ctt ggg act gaa     1536
Glu Asp Arg Phe Asn Leu Tyr Arg Ile Glu Gln Glu Leu Gly Thr Glu
            500                 505                 510
ata aaa tca att cct cct cag ata gac cag gct ata tac tgc caa         1581

Ile Lys Ser Ile Pro Pro Gln Ile Asp Gln Ala Ile Tyr Cys Gln
        515                 520                 525
taa                                                                 1584

<210> 152
<211> 527
<212> PRT
<213> Zea mays

<400> 152
   Met Asp Pro Arg Ala Arg Tyr Pro Ser Gly Met Gly Asn Gly Arg Gly
   1               5                   10                  15
   Gly Asn Leu Asn Tyr Tyr Gly Arg Gly Pro Pro Pro Gln Gln Pro His
               20                  25                  30
   Gln His His Asn His Gln Gln Thr Ser Gly Ala His His His Gln Gln
           35                  40                  45
   Thr Ser Gly Ala His His His Gln Gln Tyr Ala Gln Arg Gln His Gln
       50                  55                  60
   His Gln His Gln His His Gly His Asn Arg His Gln Gln Gln Gln Gln
   65                  70                  75                  80
   His His Ile Tyr Gln Gln Gln Gln Gln Gln Trp Leu Arg Arg Ser Gln
                   85                  90                  95
   Ile Ala Arg Glu Thr Ser Gly Ala Ala Val Thr Ser Glu Pro Lys Ser
               100                 105                 110
   Leu Ala Pro Ser Thr Ala Ala Asp Gly Val Asp Ser Ser Ser Gln Asp
           115                 120                 125
   Trp Lys Ala Gln Leu Lys Leu Pro Pro Pro Asp Thr Arg Tyr Arg Thr
       130                 135                 140
   Glu Asp Val Thr Ala Thr Lys Gly Asn Glu Phe Glu Asp Tyr Phe Leu
   145                 150                 155                 160
   Lys Arg Glu Leu Leu Met Gly Ile Tyr Glu Lys Gly Phe Glu Arg Pro
                   165                 170                 175
   Ser Pro Ile Gln Glu Glu Ser Ile Pro Ile Ala Leu Thr Gly Ser Asp
               180                 185                 190
   Ile Leu Ala Arg Ala Lys Asn Gly Thr Gly Lys Thr Ala Ala Phe Cys
           195                 200                 205
   Ile Pro Ala Leu Glu Lys Ile Asp Gln Glu Lys Asn Ala Ile Gln Val
       210                 215                 220
   Val Ile Leu Val Pro Thr Arg Glu Leu Ala Leu Gln Thr Ser Gln Val
   225                 230                 235                 240
   Cys Lys Glu Leu Gly Lys His Leu Lys Ile Gln Val Met Val Thr Thr
                   245                 250                 255
   Gly Gly Thr Ser Leu Lys Asp Asp Ile Val Arg Leu Tyr Gln Pro Val
               260                 265                 270
   His Leu Leu Val Gly Thr Pro Gly Arg Ile Leu Asp Leu Thr Lys Lys
           275                 280                 285
   Gly Val Cys Ile Leu Asn Asp Cys Ser Met Leu Ile Met Asp Glu Ala
       290                 295                 300
   Asp Lys Leu Leu Ser Pro Glu Phe Gln Pro Ser Ile Glu Gln Leu Ile
   305                 310                 315                 320
   Arg Tyr Leu Pro Ala Ser Arg Gln Ile Leu Met Phe Ser Ala Thr Phe
                   325                 330                 335
   Pro Val Thr Val Lys Glu Phe Lys Asp Lys Tyr Leu Pro Lys Pro Tyr
               340                 345                 350
   Val Ile Asn Leu Met Asp Glu Leu Thr Leu Lys Gly Ile Thr Gln Phe
           355                 360                 365
   Tyr Ala Phe Val Glu Glu Arg Gln Lys Val His Cys Leu Asn Thr Leu
       370                 375                 380
   Phe Ser Lys Leu Gln Ile Asn Gln Ser Ile Ile Phe Cys Asn Ser Val
   385                 390                 395                 400
   Asn Arg Val Glu Leu Leu Ala Lys Lys Ile Thr Glu Leu Gly Tyr Ser
                   405                 410                 415
   Cys Phe Tyr Ile His Ala Lys Met Leu Gln Asp His Arg Asn Arg Val
               420                 425                 430
   Phe His Asp Phe Arg Asn Gly Ala Cys Arg Asn Leu Val Cys Thr Asp
           435                 440                 445
   Leu Phe Thr Arg Gly Ile Asp Ile Gln Ala Val Asn Val Val Ile Asn
   450                 455                 460
   Phe Asp Phe Pro Lys Asn Ser Glu Thr Tyr Leu His Arg Val Gly Arg
   465                 470                 475                 480
   Ser Gly Arg Phe Gly His Leu Gly Leu Ala Val Asn Leu Ile Thr Tyr
                   485                 490                 495
   Glu Asp Arg Phe Asn Leu Tyr Arg Ile Glu Gln Glu Leu Gly Thr Glu
               500                 505                 510
   Ile Lys Ser Ile Pro Pro Gln Ile Asp Gln Ala Ile Tyr Cys Gln
           515                 520                 525

<210> 153
<211> 21
<212> DNA
<213> Artificial sequence

<400> 153
atggcaggaa tggcaccaga g                                                21

<210> 154
<211> 22
<212> DNA
<213> Artificial sequence

<400> 154
ctagagaagg tcggcgacat tg                                               22


<210> 155
<211> 402
<212> PRT
<213> Artificial sequence

<220>
<221> Variant
<222> (2)..(5)
<223> xaa in position 2 to 5 is any amino acid
<220>
<221> Variant
<222> (7)..(17)
<223> xaa in position 7 to 17 is any amino acid
<220>
<221> Variant
<222> (19)..(21)
<223> xaa in position 19 to 21 is any amino acid
<220>
<221> Variant
<222> (23)..(23)
<223> xaa in position 23 is any amino acid
<220>
<221> Variant
<222> (27)..(29)
<223> xaa in position 27 to 29 is any amino acid
<220>
<221> Variant
<222> (32)..(33)
<223> xaa in position 32 to 33 is any amino acid
<220>
<221> Variant
<222> (35)..(35)
<223> xaa in position 35 is any amino acid
<220>
<221> Variant
<222> (37)..(37)
<223> xaa in position 37 is any amino acid
<220>
<221> Variant
<222> (39)..(42)
<223> xaa in position 39 to 42 is any amino acid
<220>

<221> Variant
<222> (52)..(52)
<223> xaa in position 52 is any amino acid
<220>
<221> Variant
<222> (54)..(54)
<223> xaa in position 54 is any amino acid
<220>
<221> Variant
<222> (57)..(69)
<223> xaa in position 57 to 69 is any amino acid
<220>
<221> Variant
<222> (70)..(96)
<223> xaa in position 70 to 96 is any or no amino acid
<220>
<221> Variant
<222> (98)..(98)
<223> xaa in position 98 is any amino acid
<220>
<221> Variant
<222> (100)..(100)
<223> xaa in position 100 is any amino acid
<220>
<221> Variant
<222> (107)..(107)
<223> xaa in position 107 is any amino acid
<220>
<221> Variant
<222> (109)..(128)
<223> xaa in position 109 to 128 is any amino acid
<220>
<221> Variant
<222> (129)..(130)
<223> xaa in position 129 to 130 is any or no amino acid
<220>
<221> Variant
<222> (133)..(137)
<223> xaa in position 133 to 137 is any amino acid
<220>
<221> Variant
<222> (139)..(141)
<223> xaa in position 139 to 141 is any amino acid
<220>
<221> Variant
<222> (143)..(150)
<223> xaa in position 143 to 150 is any amino acid
<220>
<221> Variant
<222> (151)..(152)
<223> xaa in position 151 to 152 is any or no amino acid
<220>
<221> Variant
<222> (158)..(158)
<223> xaa in position 158 is any amino acid
<220>
<221> Variant
<222> (160)..(174)
<223> xaa in position 160 to 174 is any amino acid
<220>
<221> Variant
<222> (175)..(175)
<223> xaa in position 175 is any or no amino acid
<220>
<221> Variant
<222> (182)..(182)
<223> xaa in position 182 is any amino acid
<220>
<221> Variant

<222> (185)..(185)
<223> xaa in position 185 is any amino acid
<220>
<221> Variant
<222> (189)..(190)
<223> xaa in position 189 to 190 is any amino acid
<220>
<221> Variant
<222> (192)..(199)
<223> xaa in position 192 to 199 is any amino acid
<220>
<221> Variant
<222> (201)..(203)
<223> xaa in position 201 to 203 is any amino acid
<220>
<221> Variant
<222> (204)..(205)
<223> xaa in position 204 to 205 is any or no amino acid
<220>
<221> Variant
<222> (207)..(209)
<223> xaa in position 207 to 209 is any amino acid
<220>
<221> Variant
<222> (214)..(228)
<223> xaa in position 214 to 228 is any amino acid
<220>
<221> Variant
<222> (229)..(229)
<223> xaa in position 229 is any or no amino acid
<220>
<221> Variant
<222> (231)..(245)
<223> xaa in position 231 to 245 is any amino acid
<220>
<221> Variant
<222> (246)..(249)
<223> xaa in position 246 to 249 is any or no amino acid
<220>
<221> Variant
<222> (251)..(257)
<223> xaa in position 251 to 257 is any amino acid
<220>
<221> Variant
<222> (258)..(266)
<223> xaa in position 258 to 266 is any or no amino acid
<220>
<221> Variant
<222> (268)..(270)
<223> xaa in position 268 to 270 is any amino acid
<220>
<221> Variant
<222> (272)..(284)
<223> xaa in position 272 to 284 is any amino acid
<220>
<221> Variant
<222> (285)..(287)
<223> xaa in position 285 to 287 is any or no amino acid
<220>
<221> Variant
<222> (289)..(290)
<223> xaa in position 289 to 290 is any amino acid
<220>
<221> Variant
<222> (293)..(301)
<223> xaa in position 293 to 301 is any amino acid
<220>
<221> Variant
<222> (303)..(305)

<223> xaa in position 303 to 305 is any amino acid
<220>
<221> Variant
<222> (306)..(308)
<223> xaa in position 306 to 308 is any or no amino acid
<220>
<221> Variant
<222> (310)..(316)
<223> xaa in position 310 to 316 is any amino acid
<220>
<221> Variant
<222> (318)..(320)
<223> xaa in position 318 to 320 is any amino acid
<220>
<221> Variant
<222> (322)..(323)
<223> xaa in position 322 to 323 is any amino acid
<220>
<221> Variant
<222> (325)..(333)
<223> xaa in position 325 to 333 is any amino acid
<220>
<221> Variant
<222> (335)..(338)
<223> xaa in position 335 to 338 is any amino acid
<220>
<221> Variant
<222> (344)..(344)
<223> xaa in position 344 is any amino acid
<220>
<221> Variant
<222> (349)..(349)
<223> xaa in position 349 is any amino acid
<220>
<221> Variant
<222> (351)..(356)
<223> xaa in position 351 to 356 is any amino acid
<220>
<221> Variant
<222> (358)..(358)
<223> xaa in position 358 is any amino acid
<220>
<221> Variant
<222> (360)..(362)
<223> xaa in position 360 to 362 is any amino acid
<220>
<221> Variant
<222> (364)..(368)
<223> xaa in position 364 to 368 is any amino acid
<220>
<221> Variant
<222> (370)..(370)
<223> xaa in position 370 is any amino acid
<220>
<221> Variant
<222> (381)..(382)
<223> xaa in position 381 to 382 is any amino acid
<220>
<221> Variant
<222> (384)..(384)
<223> xaa in position 384 is any amino acid
<220>
<221> Variant
<222> (386)..(399)
<223> xaa in position 386 to 399 is any amino acid
<220>
<221> Variant
<222> (400)..(400)
<223> xaa in position 400 is any or no amino acid

<400> 155
Phe xaa xaa xaa xaa Leu xaa xaa xaa xaa xaa xaa xaa xaa xaa
1               5                   10                  15
xaa Gly xaa xaa xaa Pro xaa Pro Ile Gln xaa xaa xaa Ile Pro xaa
            20                  25                  30
xaa Leu xaa Gly xaa Asp xaa xaa xaa xaa Ala Gln Thr Gly Thr Gly
        35                  40                  45
Lys Thr Ala xaa Phe xaa Leu Pro xaa xaa xaa xaa xaa xaa xaa xaa
    50                  55                  60
xaa xaa xaa xaa xaa xaa xaa xaa xaa xaa xaa xaa xaa xaa xaa xaa
65                  70                  75                  80
xaa xaa xaa xaa xaa xaa xaa xaa xaa xaa xaa xaa xaa xaa xaa xaa
                85                  90                  95
Leu xaa Leu xaa Pro Thr Arg Glu Leu Ala xaa Gln xaa xaa xaa xaa
            100                 105                 110
xaa xaa xaa xaa xaa xaa xaa xaa xaa xaa xaa xaa xaa xaa xaa xaa
        115                 120                 125
xaa xaa Gly Gly xaa xaa xaa xaa xaa Gln xaa xaa xaa Leu xaa xaa
    130                 135                 140
xaa xaa xaa xaa xaa xaa xaa xaa Thr Pro Gly Arg Leu xaa Asp xaa
145                 150                 155                 160
xaa xaa xaa xaa xaa xaa xaa xaa xaa xaa xaa xaa xaa xaa Val
                165                 170                 175
Leu Asp Glu Ala Asp xaa Met Leu xaa Met Gly Phe xaa xaa Asp xaa
            180                 185                 190
xaa xaa xaa xaa xaa xaa xaa Pro xaa xaa xaa xaa xaa Gln xaa xaa
        195                 200                 205
xaa Phe Ser Ala Thr xaa xaa xaa xaa xaa xaa xaa xaa xaa xaa xaa
    210                 215                 220
xaa xaa xaa xaa xaa Pro xaa xaa xaa xaa xaa xaa xaa xaa xaa xaa
225                 230                 235                 240
xaa xaa xaa xaa xaa xaa xaa xaa xaa Gln xaa xaa xaa xaa xaa xaa
                245                 250                 255
xaa xaa xaa xaa xaa xaa xaa xaa xaa xaa Lys xaa xaa xaa Leu xaa
            260                 265                 270
xaa xaa xaa xaa xaa xaa xaa xaa xaa xaa xaa xaa xaa xaa xaa Phe
        275                 280                 285
xaa xaa Thr Lys xaa xaa xaa xaa xaa xaa xaa xaa xaa Leu xaa xaa
    290                 295                 300
xaa xaa xaa xaa Gly xaa xaa xaa xaa xaa xaa Gly xaa xaa xaa
305                 310                 315                 320
Gln xaa xaa Arg xaa xaa xaa xaa xaa xaa xaa xaa xaa Gly xaa xaa
                325                 330                 335
xaa xaa Leu Val Ala Thr Asp xaa Ala Ala Arg Gly xaa Asp xaa xaa
            340                 345                 350
xaa xaa xaa xaa Val xaa Asn xaa xaa xaa Pro xaa xaa xaa xaa xaa
        355                 360                 365
Tyr xaa His Arg Ile Gly Arg Thr Gly Arg Ala Gly xaa xaa Gly xaa
    370                 375                 380
Ala xaa xaa xaa xaa xaa xaa xaa xaa xaa xaa xaa xaa xaa xaa xaa
385                 390                 395                 400
Ile Glu

<210> 156
<211> 61
<212> PRT
<213> Artificial sequence

<220>
<221> Variant
<222> (1)..(1)
<223> xaa in position 1 is Phe or Trp
<220>
<221> Variant
<222> (2)..(3)
<223> xaa in position 2 to 3 is any amino acid

<220>
<221> Variant
<222> (4)..(4)
<223> Xaa in position 4 is Leu, Phe, Ile or Met
<220>
<221> Variant
<222> (5)..(5)
<223> Xaa in position 5 is any amino acid
<220>
<221> Variant
<222> (6)..(6)
<223> Xaa in position 6 is Leu, Ile, Phe, Met or Val
<220>
<221> Variant
<222> (7)..(9)
<223> Xaa in position 7 to 9 is any amino acid
<220>
<221> Variant
<222> (10)..(10)
<223> Xaa in position 10 is Leu, Ile, Val, Met or Phe
<220>
<221> Variant
<222> (11)..(13)
<223> Xaa in position 11 to 13 is any amino acid
<220>
<221> Variant
<222> (14)..(14)
<223> Xaa in position 14 is Leu, Val, Ile, Ala or Met
<220>
<221> Variant
<222> (15)..(17)
<223> Xaa in position 15 to 17 is any amino acid
<220>
<221> Variant
<222> (18)..(18)
<223> Xaa in position 18 is Gly, Asn, Lys, Asp or Glu
<220>
<221> Variant
<222> (19)..(19)
<223> Xaa in position 19 is Tyr, Phe, Ile or His
<220>
<221> Variant
<222> (20)..(21)
<223> Xaa in position 20 to 21 is any amino acid
<220>
<221> Variant
<222> (22)..(22)
<223> Xaa in position 22 is Pro, Ala or Cys
<220>
<221> Variant
<222> (23)..(23)
<223> Xaa in position 23 is Thr, Ser or Lys
<220>
<221> Variant
<222> (24)..(24)
<223> Xaa in position 24 is any amino acid
<220>
<221> Variant
<222> (25)..(25)
<223> Xaa in position 25 is Ile or Val
<220>
<221> Variant
<222> (27)..(28)
<223> Xaa in position 27 to 28 is any amino acid
<220>
<221> Variant
<222> (29)..(29)
<223> Xaa in position 29 is Ala, Thr, Ser or Cys
<220>

<221> Variant
<222> (30)..(30)
<223> Xaa in position 30 is Ile, Leu or Val
<220>
<221> Variant
<222> (32)..(33)
<223> Xaa in position 32 to 33 is any amino acid
<220>
<221> Variant
<222> (34)..(34)
<223> Xaa in position 34 is Leu, Met, Ile or Val
<220>
<221> Variant
<222> (35)..(35)
<223> Xaa in position 35 is any amino acid
<220>
<221> Variant
<222> (36)..(36)
<223> Xaa in position 36 is Gly, Arg, Gln, Met, Asn or Glu
<220>
<221> Variant
<222> (37)..(37)
<223> Xaa in position 37 is any amino acid
<220>
<221> Variant
<222> (39)..(39)
<223> Xaa in position 39 is Val, Leu, Ile, Met or Ala
<220>
<221> Variant
<222> (40)..(40)
<223> Xaa in position 40 is Leu, Met, Ile, Val or Ala
<220>
<221> Variant
<222> (41)..(41)
<223> Xaa in position 41 is Gly, Ala, Val, Ile or Leu
<220>
<221> Variant
<222> (42)..(42)
<223> Xaa in position 42 is any amino acid
<220>
<221> Variant
<222> (43)..(43)
<223> Xaa in position 43 is Ala or Ser
<220>
<221> Variant
<222> (44)..(44)
<223> Xaa in position 44 is Gln, Pro, Ala or Lys
<220>
<221> Variant
<222> (47)..(47)
<223> Xaa in position 47 is Thr or Ser
<220>
<221> Variant
<222> (51)..(51)
<223> Xaa in position 51 is Ala, Leu, Gly, Val, Phe or Met
<220>
<221> Variant
<222> (52)..(52)
<223> Xaa in position 52 is Ala, Gly or Ser
<220>
<221> Variant
<222> (53)..(53)
<223> Xaa in position 53 is Phe or Tyr
<220>
<221> Variant
<222> (54)..(54)
<223> Xaa in position 54 is any amino acid
<220>
<221> Variant

<222> (55)..(55)
<223> Xaa in position 55 is Leu, Ile or Val
<220>
<221> Variant
<222> (56)..(56)
<223> Xaa in position 56 is Pro or Ala
<220>
<221> Variant
<222> (57)..(57)
<223> Xaa in position 57 is Ile, Leu, Met, Ala, Val, Thr or Ser
<220>
<221> Variant
<222> (58)..(58)
<223> Xaa in position 58 is Leu, Ile, Val, Phe or Met
<220>
<221> Variant
<222> (59)..(60)
<223> Xaa in position 59 to 60 is any amino acid
<220>
<221> Variant
<222> (61)..(61)
<223> Xaa in position 61 is Leu, Ile, Val or Met

<400> 156
Xaa Xaa Xaa Xaa Xaa Xaa Xaa Xaa Xaa Xaa Xaa Xaa Xaa Xaa Xaa Xaa
1               5                   10                  15
Xaa Xaa Xaa Xaa Xaa Xaa Xaa Xaa Xaa Gln Xaa Xaa Xaa Xaa Pro Xaa
            20                  25                  30
Xaa Xaa Xaa Xaa Xaa Asp Xaa Xaa Xaa Xaa Xaa Xaa Thr Gly Xaa Gly
        35                  40                  45
Lys Thr Xaa Xaa Xaa Xaa Xaa Xaa Xaa Xaa Xaa Xaa Xaa
    50                  55                  60


<210> 157
<211> 113
<212> PRT
<213> Artificial sequence

<220>
<221> Variant
<222> (6)..(6)
<223> Xaa in position 6 is Ala, Val, Ser, Thr or Gly
<220>
<221> Variant
<222> (7)..(7)
<223> Xaa in position 7 is any amino acid
<220>
<221> Variant
<222> (9)..(9)
<223> Xaa in position 9 is Val, Ile, Thr, Ser or Ala
<220>
<221> Variant
<222> (10)..(15)
<223> Xaa in position 10 to 15 is any amino acid
<220>
<221> Variant
<222> (16)..(16)
<223> Xaa in position 16 is Tyr, Leu, Phe, Met, Ala or Val
<220>
<221> Variant
<222> (17)..(17)
<223> Xaa in position 17 is Gly, Ala, Ser, Thr, Val or Leu
<220>
<221> Variant
<222> (18)..(26)
<223> Xaa in position 18 to 26 is any amino acid
<220>
<221> Variant

<222> (27)..(28)
<223> xaa in position 27 to 28 is any or no amino acid
<220>
<221> Variant
<222> (29)..(29)
<223> xaa in position 29 is Ile, Val, Leu, Ala or Met
<220>
<221> Variant
<222> (30)..(30)
<223> xaa in position 30 is Tyr, Phe, Thr, Ile, Val, Leu or Met
<220>
<221> Variant
<222> (33)..(37)
<223> xaa in position 33 to 37 is any amino acid
<220>
<221> Variant
<222> (38)..(38)
<223> xaa in position 38 is Gln or His
<220>
<221> Variant
<222> (39)..(41)
<223> xaa in position 39 to 41 is any amino acid
<220>
<221> Variant
<222> (42)..(42)
<223> xaa in position 42 is Leu, Phe, Ile, Met or Val
<220>
<221> Variant
<222> (43)..(46)
<223> xaa in position 43 to 46 is any amino acid
<220>
<221> Variant
<222> (47)..(48)
<223> xaa in position 47 to 48 is any or no amino acid
<220>
<221> Variant
<222> (49)..(49)
<223> xaa in position 49 is Ile, Val, Leu or Phe
<220>
<221> Variant
<222> (50)..(50)
<223> xaa in position 50 is Leu, Val, Ile or Met
<220>
<221> Variant
<222> (51)..(51)
<223> xaa in position 51 is Val or Ile
<220>
<221> Variant
<222> (52)..(52)
<223> xaa in position 52 is Ala or Gly
<220>
<221> Variant
<222> (53)..(53)
<223> xaa in position 53 is Thr or Cys
<220>
<221> Variant
<222> (54)..(54)
<223> xaa in position 54 is Pro or Thr
<220>
<221> Variant
<222> (57)..(57)
<223> xaa in position 57 is Leu, Ile, Val, Met, Phe or Ala
<220>
<221> Variant
<222> (58)..(58)
<223> xaa in position 58 is Leu, Ile, Met, Val or Ala
<220>
<221> Variant
<222> (59)..(59)

<223> xaa in position 59 is Asp, Gln or Glu
<220>
<221> Variant
<222> (60)..(70)
<223> xaa in position 60 to 70 is any amino acid
<220>
<221> Variant
<222> (71)..(71)
<223> xaa in position 71 is any or no amino acid
<220>
<221> Variant
<222> (72)..(72)
<223> xaa in position 72 is Val, Leu, Ile, Thr, Cys or Pro
<220>
<221> Variant
<222> (73)..(74)
<223> xaa in position 73 to 74 is any amino acid
<220>
<221> Variant
<222> (75)..(75)
<223> xaa in position 75 is Leu, Phe, Val, Ile, Met or Cys
<220>
<221> Variant
<222> (76)..(76)
<223> xaa in position 76 is Val or Ile
<220>
<221> Variant
<222> (77)..(77)
<223> xaa in position 77 is Leu, Ile, Phe or Val
<220>
<221> Variant
<222> (80)..(80)
<223> xaa in position 80 is Ala, Ser or Gly
<220>
<221> Variant
<222> (82)..(82)
<223> xaa in position 82 is Arg, Glu or Gln
<220>
<221> Variant
<222> (83)..(83)
<223> xaa in position 83 is Met or Leu
<220>
<221> Variant
<222> (84)..(84)
<223> xaa in position 84 is Leu, Phe or Met
<220>
<221> Variant
<222> (85)..(85)
<223> xaa in position 85 is any amino acid
<220>
<221> Variant
<222> (86)..(86)
<223> xaa in position 86 is Met, Leu, Ile or Phe
<220>
<221> Variant
<222> (89)..(91)
<223> xaa in position 89 to 91 is any amino acid
<220>
<221> Variant
<222> (92)..(92)
<223> xaa in position 92 is Ile, Val, Leu, Ala or Met
<220>
<221> Variant
<222> (93)..(94)
<223> xaa in position 93 to 94 is any amino acid
<220>
<221> Variant
<222> (95)..(95)
<223> xaa in position 95 is Ile, Val, Leu or Met

<220>
<221> Variant
<222> (96)..(103)
<223> xaa in position 96 to 103 is any amino acid
<220>
<221> Variant
<222> (104)..(105)
<223> xaa in position 104 to 105 is any or no amino acid
<220>
<221> Variant
<222> (106)..(106)
<223> xaa in position 106 is Gln or His
<220>
<221> Variant
<222> (107)..(108)
<223> xaa in position 107 to 108 is any amino acid
<220>
<221> Variant
<222> (109)..(109)
<223> xaa in position 109 is Leu, Phe or Met
<220>
<221> Variant
<222> (110)..(110)
<223> xaa in position 110 is Phe or Tyr
<220>
<221> Variant
<222> (111)..(111)
<223> xaa in position 111 is Ser or Thr

<400> 157
Pro Thr Arg Glu Leu xaa xaa Gln xaa xaa xaa xaa xaa xaa xaa xaa
1               5                   10                  15
xaa xaa xaa xaa xaa xaa xaa xaa xaa xaa xaa xaa xaa xaa Gly Gly
            20                  25                  30
xaa xaa xaa xaa xaa xaa xaa xaa xaa xaa xaa xaa xaa xaa xaa xaa
        35                  40                  45
xaa xaa xaa xaa xaa xaa Gly Arg xaa xaa xaa xaa xaa xaa xaa xaa
    50                  55                  60
xaa xaa xaa xaa xaa xaa xaa xaa xaa xaa xaa xaa xaa Asp Glu xaa
65                  70                  75                  80
Asp xaa xaa xaa xaa xaa Gly Phe xaa xaa xaa xaa xaa xaa xaa xaa
                85                  90                  95
xaa xaa xaa xaa xaa xaa xaa xaa xaa xaa xaa xaa xaa xaa xaa Ala
            100                 105                 110
Thr

<210> 158
<211> 96
<212> PRT
<213> Artificial sequence

<220>
<221> Variant
<222> (2)..(4)
<223> xaa in position 2 to 4 is any amino acid
<220>
<221> Variant
<222> (5)..(5)
<223> xaa in position 5 is Lys, Arg or Gln
<220>
<221> Variant
<222> (6)..(10)
<223> xaa in position 6 to 10 is any amino acid
<220>
<221> Variant
<222> (11)..(11)
<223> xaa in position 11 is Leu, Val, Ile or Met

<220>
<221> Variant
<222> (12)..(14)
<223> Xaa in position 12 to 14 is any amino acid
<220>
<221> Variant
<222> (15)..(15)
<223> Xaa in position 15 is Leu, Met or Ile
<220>
<221> Variant
<222> (16)..(24)
<223> Xaa in position 16 to 24 is any amino acid
<220>
<221> Variant
<222> (25)..(27)
<223> Xaa in position 25 to 27 is any or no amino acid
<220>
<221> Variant
<222> (28)..(28)
<223> Xaa in position 28 is Ile, Leu, Met, Val or Phe
<220>
<221> Variant
<222> (29)..(29)
<223> Xaa in position 29 is any amino acid
<220>
<221> Variant
<222> (30)..(30)
<223> Xaa in position 30 is Gly, Ala or Ser
<220>
<221> Variant
<222> (31)..(33)
<223> Xaa in position 31 to 33 is any amino acid
<220>
<221> Variant
<222> (35)..(36)
<223> Xaa in position 35 to 36 is any amino acid
<220>
<221> Variant
<222> (38)..(40)
<223> Xaa in position 38 to 40 is any amino acid
<220>
<221> Variant
<222> (41)..(41)
<223> Xaa in position 41 is Leu, Ile, Val or Met
<220>
<221> Variant
<222> (42)..(43)
<223> Xaa in position 42 to 43 is any amino acid
<220>
<221> Variant
<222> (44)..(44)
<223> Xaa in position 44 is Phe, Leu, Met or Ile
<220>
<221> Variant
<222> (46)..(51)
<223> Xaa in position 46 to 51 is any amino acid
<220>
<221> Variant
<222> (52)..(52)
<223> Xaa in position 52 is Leu or Met
<220>
<221> Variant
<222> (53)..(53)
<223> Xaa in position 53 is Val, Ile or Leu
<220>
<221> Variant
<222> (54)..(54)
<223> Xaa in position 54 is Ala, Cys or Thr
<220>

<221> Variant
<222> (55)..(55)
<223> xaa in position 55 is Thr or Ser
<220>
<221> Variant
<222> (57)..(57)
<223> xaa in position 57 is Val or Ile
<220>
<221> Variant
<222> (59)..(59)
<223> xaa in position 59 is Ala, Ser or Gly
<220>
<221> Variant
<222> (62)..(62)
<223> xaa in position 62 is Leu or Ile
<220>
<221> Variant
<222> (63)..(63)
<223> xaa in position 63 is Asp or His
<220>
<221> Variant
<222> (64)..(64)
<223> xaa in position 64 is Ile, Val or Leu
<220>
<221> Variant
<222> (65)..(66)
<223> xaa in position 65 to 66 is any amino acid
<220>
<221> Variant
<222> (67)..(67)
<223> xaa in position 67 is Val, Leu, Ile or Met
<220>
<221> Variant
<222> (68)..(69)
<223> xaa in position 68 to 69 is any amino acid
<220>
<221> Variant
<222> (71)..(71)
<223> xaa in position 71 is Val, Ile, Phe, Tyr, Leu or Met
<220>
<221> Variant
<222> (73)..(73)
<223> xaa in position 73 is Phe, Tyr or Leu
<220>
<221> Variant
<222> (74)..(74)
<223> xaa in position 74 is Asp, Glu or His
<220>
<221> Variant
<222> (75)..(75)
<223> xaa in position 75 is Leu, Ile, Met or Val
<220>
<221> Variant
<222> (77)..(79)
<223> xaa in position 77 to 79 is any amino acid
<220>
<221> Variant
<222> (80)..(80)
<223> xaa in position 80 is Glu, Asp, Lys or Gln
<220>
<221> Variant
<222> (81)..(81)
<223> xaa in position 81 is any amino acid
<220>
<221> Variant
<222> (83)..(83)
<223> xaa in position 83 is Val, Ile, Leu or Met
<220>
<221> Variant

<222> (86)..(86)
<223> xaa in position 86 is Ile or Val
<220>
<221> Variant
<222> (89)..(89)
<223> xaa in position 89 is Thr or Ser
<220>
<221> Variant
<222> (90)..(90)
<223> xaa in position 90 is Gly or Ala
<220>
<221> Variant
<222> (92)..(92)
<223> xaa in position 92 is Ala, Asn, Gly or Leu
<220>
<221> Variant
<222> (93)..(93)
<223> xaa in position 93 is Gly, Ala, Asn or Glu
<220>
<221> Variant
<222> (94)..(95)
<223> xaa in position 94 to 95 is any amino acid

<400> 158
Phe xaa xaa xaa xaa xaa xaa xaa xaa xaa xaa xaa xaa xaa xaa xaa
1               5                   10                  15
xaa xaa xaa xaa xaa xaa xaa xaa xaa xaa xaa xaa xaa xaa xaa xaa
            20                  25                  30
xaa Gln xaa xaa Arg xaa xaa xaa xaa xaa xaa xaa Lys xaa xaa xaa
        35                  40                  45
xaa xaa xaa xaa xaa xaa xaa Asp xaa Ala xaa Arg Gly xaa xaa xaa
    50                  55                  60
xaa xaa xaa xaa xaa Val xaa Asn xaa xaa xaa Pro xaa xaa xaa xaa
65                  70                  75                  80
xaa Tyr xaa His Arg xaa Gly Arg xaa xaa Arg xaa xaa xaa xaa Gly
                85                  90                  95


<210> 159
<211> 1864
<212> DNA
<213> Brassica napus

<220>
<221> CDS
<222> (34)..(1596)

<400> 159
agtgaagctc ctcctctcga gtctcgaaca aca atg gcg aca atg gct gca atc      54
                                     Met Ala Thr Met Ala Ala Ile
                                     1               5
gga tcc tta aaa gtc cct tcc tct tcc tcg aac cac acc cgt aga tta      102
Gly Ser Leu Lys Val Pro Ser Ser Ser Ser Asn His Thr Arg Arg Leu
        10                  15                  20
tca tct tct tct caa cgg aag act ctc tcg ttc tcg tcg tct tct ctt      150
Ser Ser Ser Ser Gln Arg Lys Thr Leu Ser Phe Ser Ser Ser Ser Leu
    25                  30                  35
acc gga gag aaa ctc aac cca acg cag gag atc atc atc tcc aat ctc      198
Thr Gly Glu Lys Leu Asn Pro Thr Gln Glu Ile Ile Ile Ser Asn Leu
40                  45                  50                  55
cca cgt cgc aac gag aga aga acg cca tcg atc gtc tct cca aaa gca      246
Pro Arg Arg Asn Glu Arg Arg Thr Pro Ser Ile Val Ser Pro Lys Ala
                60                  65                  70
gtt tcc gat tcg caa aac tcg caa act tgc ctt gat cct gac gct agc      294
Val Ser Asp Ser Gln Asn Ser Gln Thr Cys Leu Asp Pro Asp Ala Ser
            75                  80                  85
aga agt gtg ttg ggg ata att ctg gga ggt ggt gct gga acg aga ttg      342
Arg Ser Val Leu Gly Ile Ile Leu Gly Gly Gly Ala Gly Thr Arg Leu
        90                  95                  100

tat ccg cta acg aag aag aga gcg aag cca gct gtt cct ctc ggc gcc 390
Tyr Pro Leu Thr Lys Lys Arg Ala Lys Pro Ala Val Pro Leu Gly Ala
    105                 110                 115
aac tac cgt ctc att gat atc cca gtg agc aat tgc ttg aac agt aac 438
Asn Tyr Arg Leu Ile Asp Ile Pro Val Ser Asn Cys Leu Asn Ser Asn
120                 125                 130                 135
atc tcc aag atc tat gtc ctt act cag ttc aac tcc gct tct ctc aac 486
Ile Ser Lys Ile Tyr Val Leu Thr Gln Phe Asn Ser Ala Ser Leu Asn
                140                 145                 150
cgc cat ctc tcc cga gct tac gcc agc aac atg ggt ggt tac aag aac 534
Arg His Leu Ser Arg Ala Tyr Ala Ser Asn Met Gly Gly Tyr Lys Asn
            155                 160                 165
gaa ggc ttc gtc gag gtt ctt gct gct caa cag agt ccc gag aat ccc 582
Glu Gly Phe Val Glu Val Leu Ala Ala Gln Gln Ser Pro Glu Asn Pro
        170                 175                 180
aat tgg ttt cag ggg act gct gat gca gtg agg cag tac ttg tgg ctg 630
Asn Trp Phe Gln Gly Thr Ala Asp Ala Val Arg Gln Tyr Leu Trp Leu
    185                 190                 195
ttt gaa gag cac aat gtg ttg gag ttt ttg gtt ctt gca ggg gat cat 678
Phe Glu Glu His Asn Val Leu Glu Phe Leu Val Leu Ala Gly Asp His
200                 205                 210                 215
ttg tac cgt atg gac tac gag aag ttc att caa gcg cat cgt gag acc 726
Leu Tyr Arg Met Asp Tyr Glu Lys Phe Ile Gln Ala His Arg Glu Thr
                220                 225                 230
gac gct gat atc act gtt gct gct ctt cct atg gat gag aaa cgt gcc 774
Asp Ala Asp Ile Thr Val Ala Ala Leu Pro Met Asp Glu Lys Arg Ala
            235                 240                 245
acg gct ttt gga ctt atg aag att gat gac gaa gga agg atc att gag 822
Thr Ala Phe Gly Leu Met Lys Ile Asp Asp Glu Gly Arg Ile Ile Glu
        250                 255                 260
ttt gct gag aag cct aaa gga gag cag tta aag gct atg aag gtt gat 870
Phe Ala Glu Lys Pro Lys Gly Glu Gln Leu Lys Ala Met Lys Val Asp
    265                 270                 275
aca aca atc ttg gga ctt gat gac gaa agg gcc aaa gag atg ccc ttt 918
Thr Thr Ile Leu Gly Leu Asp Asp Glu Arg Ala Lys Glu Met Pro Phe
280                 285                 290                 295
att gct agt atg ggg ata tat gtt gtt agc aag aat gtg atg ttg gac 966
Ile Ala Ser Met Gly Ile Tyr Val Val Ser Lys Asn Val Met Leu Asp
                300                 305                 310
ttg ctc cga gac cag ttc cct gga gct aat gac ttc ggg agt gaa gtt 1014
Leu Leu Arg Asp Gln Phe Pro Gly Ala Asn Asp Phe Gly Ser Glu Val
            315                 320                 325
atc cct ggt gct act gat ctt gga ctc aga gtg caa gct tat ctg tat 1062
Ile Pro Gly Ala Thr Asp Leu Gly Leu Arg Val Gln Ala Tyr Leu Tyr
        330                 335                 340
gat gga tac tgg gaa gat att ggt acc att gaa gcc ttt tac aat gct 1110
Asp Gly Tyr Trp Glu Asp Ile Gly Thr Ile Glu Ala Phe Tyr Asn Ala
    345                 350                 355
aat ctt ggg atc acc aag aaa cca gta cca gat ttc agc ttc tat gac 1158
Asn Leu Gly Ile Thr Lys Lys Pro Val Pro Asp Phe Ser Phe Tyr Asp
360                 365                 370                 375
cgt tca gca cca atc tac aca cag cct cgg tac tta cct cca tcc aag 1206
Arg Ser Ala Pro Ile Tyr Thr Gln Pro Arg Tyr Leu Pro Pro Ser Lys
                380                 385                 390
atg ctt gat gcc gat gtt acc gat agt gtg atc ggt gaa ggt tgt gtc 1254
Met Leu Asp Ala Asp Val Thr Asp Ser Val Ile Gly Glu Gly Cys Val
            395                 400                 405
atc aag aat tgc aaa atc cac cat tcc gtc att ggt ctt cgg tct tgc 1302
Ile Lys Asn Cys Lys Ile His His Ser Val Ile Gly Leu Arg Ser Cys
        410                 415                 420
ata tcg gag ggt gca atc ata gaa gac acc ttg ttg atg ggt gct gac 1350
Ile Ser Glu Gly Ala Ile Ile Glu Asp Thr Leu Leu Met Gly Ala Asp
    425                 430                 435
tac tac gag act gat gcg gat agg aca ctc ctg gct gca aaa ggc agc 1398
Tyr Tyr Glu Thr Asp Ala Asp Arg Thr Leu Leu Ala Ala Lys Gly Ser
440                 445                 450                 455
atc ccg att ggc att ggc cga gac tct cac att aaa aga gct atc att 1446
Ile Pro Ile Gly Ile Gly Arg Asp Ser His Ile Lys Arg Ala Ile Ile
                460                 465                 470

gac aag aat gct cgc att ggt gac aac gtc aag atc atc aac acg gac      1494
Asp Lys Asn Ala Arg Ile Gly Asp Asn Val Lys Ile Ile Asn Thr Asp
            475                 480                 485
aat gtg caa gaa gct gcc aga gag acg gat gga tac ttc atc aag agc      1542
Asn Val Gln Glu Ala Ala Arg Glu Thr Asp Gly Tyr Phe Ile Lys Ser
        490                 495                 500
ggc att gtt aca gtg atc aag gat gct ctg att cct agt gga act gtt      1590
Gly Ile Val Thr Val Ile Lys Asp Ala Leu Ile Pro Ser Gly Thr Val
    505                 510                 515
atc taa gagacaccac caccccgttt gacaatcttc ttaatatctc atggttttga      1646
Ile
520
cctcgagtta gcttccactg atgctatctc taatgttatc cgaggtcagg gcctatgcat      1706

cctcctgcct tatccctaaa taatattctc tactttgtat tctagatctt tggcgatata      1766

tgcgtatata tacatgcatt tttttttaaa aaaaaaagca caacattccc tctatgtaac      1826

aaaactatga accagaaatt gttaaaaaaa aaaaaaaa                            1864

<210> 160
<211> 520
<212> PRT
<213> Brassica napus

<400> 160
Met Ala Thr Met Ala Ala Ile Gly Ser Leu Lys Val Pro Ser Ser Ser
1               5                   10                  15
Ser Asn His Thr Arg Arg Leu Ser Ser Ser Ser Gln Arg Lys Thr Leu
            20                  25                  30
Ser Phe Ser Ser Ser Ser Leu Thr Gly Glu Lys Leu Asn Pro Thr Gln
        35                  40                  45
Glu Ile Ile Ile Ser Asn Leu Pro Arg Arg Asn Glu Arg Arg Thr Pro
    50                  55                  60
Ser Ile Val Ser Pro Lys Ala Val Ser Asp Ser Gln Asn Ser Gln Thr
65                  70                  75                  80
Cys Leu Asp Pro Asp Ala Ser Arg Ser Val Leu Gly Ile Ile Leu Gly
                85                  90                  95
Gly Gly Ala Gly Thr Arg Leu Tyr Pro Leu Thr Lys Lys Arg Ala Lys
            100                 105                 110
Pro Ala Val Pro Leu Gly Ala Asn Tyr Arg Leu Ile Asp Ile Pro Val
        115                 120                 125
Ser Asn Cys Leu Asn Ser Asn Ile Ser Lys Ile Tyr Val Leu Thr Gln
    130                 135                 140
Phe Asn Ser Ala Ser Leu Asn Arg His Leu Ser Arg Ala Tyr Ala Ser
145                 150                 155                 160
Asn Met Gly Gly Tyr Lys Asn Glu Gly Phe Val Glu Val Leu Ala Ala
                165                 170                 175
Gln Gln Ser Pro Glu Asn Pro Asn Trp Phe Gln Gly Thr Ala Asp Ala
            180                 185                 190
Val Arg Gln Tyr Leu Trp Leu Phe Glu Glu His Asn Val Leu Glu Phe
        195                 200                 205
Leu Val Leu Ala Gly Asp His Leu Tyr Arg Met Asp Tyr Glu Lys Phe
    210                 215                 220
Ile Gln Ala His Arg Glu Thr Asp Ala Asp Ile Thr Val Ala Ala Leu
225                 230                 235                 240
Pro Met Asp Glu Lys Arg Ala Thr Ala Phe Gly Leu Met Lys Ile Asp
                245                 250                 255
Asp Glu Gly Arg Ile Ile Glu Phe Ala Glu Lys Pro Lys Gly Glu Gln
            260                 265                 270
Leu Lys Ala Met Lys Val Asp Thr Thr Ile Leu Gly Leu Asp Asp Glu
        275                 280                 285
Arg Ala Lys Glu Met Pro Phe Ile Ala Ser Met Gly Ile Tyr Val Val
    290                 295                 300

Ser Lys Asn Val Met Leu Asp Leu Leu Arg Asp Gln Phe Pro Gly Ala
305                 310                 315                 320
Asn Asp Phe Gly Ser Glu Val Ile Pro Gly Ala Thr Asp Leu Gly Leu
                325                 330                 335
Arg Val Gln Ala Tyr Leu Tyr Asp Gly Tyr Trp Glu Asp Ile Gly Thr
            340                 345                 350
Ile Glu Ala Phe Tyr Asn Ala Asn Leu Gly Ile Thr Lys Lys Pro Val
        355                 360                 365
Pro Asp Phe Ser Phe Tyr Asp Arg Ser Ala Pro Ile Tyr Thr Gln Pro
    370                 375                 380
Arg Tyr Leu Pro Pro Ser Lys Met Leu Asp Ala Asp Val Thr Asp Ser
385                 390                 395                 400
Val Ile Gly Glu Gly Cys Val Ile Lys Asn Cys Lys Ile His His Ser
                405                 410                 415
Val Ile Gly Leu Arg Ser Cys Ile Ser Glu Gly Ala Ile Ile Glu Asp
            420                 425                 430
Thr Leu Leu Met Gly Ala Asp Tyr Tyr Glu Thr Asp Ala Asp Arg Thr
        435                 440                 445
Leu Leu Ala Ala Lys Gly Ser Ile Pro Ile Gly Ile Gly Arg Asp Ser
    450                 455                 460
His Ile Lys Arg Ala Ile Ile Asp Lys Asn Ala Arg Ile Gly Asp Asn
465                 470                 475                 480
Val Lys Ile Ile Asn Thr Asp Asn Val Gln Glu Ala Ala Arg Glu Thr
                485                 490                 495
Asp Gly Tyr Phe Ile Lys Ser Gly Ile Val Thr Val Ile Lys Asp Ala
            500                 505                 510
Leu Ile Pro Ser Gly Thr Val Ile
        515                 520

<210> 161
<211> 1878
<212> DNA
<213> Brassica napus

<220>
<221> CDS
<222> (98)..(1669)

<400> 161
cttcgcctcg ttctctcctt tgggtgcatg aaggacatat cacattaatt caaaaccaga      60

caatacgaat aaggtcttta caatccgctt aaaaaat atg gat tct tgt tgc tac      115
                                         Met Asp Ser Cys Cys Tyr
                                         1               5
ttg gga ttg ggg aca aac act gtc ttg ccc aaa gac ggt tac aag aat      163
Leu Gly Leu Gly Thr Asn Thr Val Leu Pro Lys Asp Gly Tyr Lys Asn
            10                  15                  20
ata gag aac agt ttt tgg ggt gag aag atc aaa gga agc ttc tct aaa      211
Ile Glu Asn Ser Phe Trp Gly Glu Lys Ile Lys Gly Ser Phe Ser Lys
        25                  30                  35
cct ttt gct tca gat tca agt tcc aag gag tta aga tat cga aag tcg      259
Pro Phe Ala Ser Asp Ser Ser Ser Lys Glu Leu Arg Tyr Arg Lys Ser
    40                  45                  50
ccg agg cca ggt gtt gct tat gcg gtt gct act tca aag aat gct aaa      307
Pro Arg Pro Gly Val Ala Tyr Ala Val Ala Thr Ser Lys Asn Ala Lys
55                  60                  65                  70
gag gct ttg aga cta cag cct tcg atg ttg ggg gag aga aga aaa gca      355
Glu Ala Leu Arg Leu Gln Pro Ser Met Leu Gly Glu Arg Arg Lys Ala
                75                  80                  85
gat cca aaa aac gtg gca gca atc att ctg gga gga ggc aat gga gct      403
Asp Pro Lys Asn Val Ala Ala Ile Ile Leu Gly Gly Gly Asn Gly Ala
            90                  95                  100
aaa ctc ttc cct ctc acc aag cga gct gca act cca gct gtt cct gtg      451
Lys Leu Phe Pro Leu Thr Lys Arg Ala Ala Thr Pro Ala Val Pro Val
        105                 110                 115
ggt gga tgc tat agg atg atc gac atc cct atg agt aat tgc att aac      499
Gly Gly Cys Tyr Arg Met Ile Asp Ile Pro Met Ser Asn Cys Ile Asn
    120                 125                 130

agt tgc atc aac aag atc ttc gtg ctc act cag ttt aac tcg gct tcc 547
Ser Cys Ile Asn Lys Ile Phe Val Leu Thr Gln Phe Asn Ser Ala Ser
135                 140                 145                 150
ctc aac cgc cac ttg gct cgt act tat ttc ggg aat ggc atc aac ttt 595
Leu Asn Arg His Leu Ala Arg Thr Tyr Phe Gly Asn Gly Ile Asn Phe
                155                 160                 165
gga gat ggt ttc gtt gag gtt cta gct gct aca cag act cct ggt gaa 643
Gly Asp Gly Phe Val Glu Val Leu Ala Ala Thr Gln Thr Pro Gly Glu
            170                 175                 180
gca ggg aag aaa tgg ttt caa gga aca gca gat gct gtc aga aag ttt 691
Ala Gly Lys Lys Trp Phe Gln Gly Thr Ala Asp Ala Val Arg Lys Phe
        185                 190                 195
ctc tgg gtg ttt gag gat gct aag aat agg aac att gag aat att atc 739
Leu Trp Val Phe Glu Asp Ala Lys Asn Arg Asn Ile Glu Asn Ile Ile
    200                 205                 210
atc ttg tct gga gat cat ctc tac aga atg aac tac atg gac ttt gtt 787
Ile Leu Ser Gly Asp His Leu Tyr Arg Met Asn Tyr Met Asp Phe Val
215                 220                 225                 230
cag tac cat gtg gat agc aac gcg gat att act ctt tcg tgc gcg cca 835
Gln Tyr His Val Asp Ser Asn Ala Asp Ile Thr Leu Ser Cys Ala Pro
                235                 240                 245
gtg ggt gag agt cgt gca tcg gat tat gga cta gtg aac att gat cga 883
Val Gly Glu Ser Arg Ala Ser Asp Tyr Gly Leu Val Asn Ile Asp Arg
            250                 255                 260
acc gga cgt gtg gtc cat ttc tgt gag aaa cca act ggc atc gat ctc 931
Thr Gly Arg Val Val His Phe Cys Glu Lys Pro Thr Gly Ile Asp Leu
        265                 270                 275
aaa tca atg caa agt gat acg aca atg ctt gga ctg tct caa caa gaa 979
Lys Ser Met Gln Ser Asp Thr Thr Met Leu Gly Leu Ser Gln Gln Glu
    280                 285                 290
gca gtg aaa tct ccg tac att gca tca atg gga gtt tac tgt ttc aaa 1027
Ala Val Lys Ser Pro Tyr Ile Ala Ser Met Gly Val Tyr Cys Phe Lys
295                 300                 305                 310
acg gaa gct ttg ctg aag ctt ctg aca cgg cgc tat ccg agt tcc aat 1075
Thr Glu Ala Leu Leu Lys Leu Leu Thr Arg Arg Tyr Pro Ser Ser Asn
                315                 320                 325
gac ttt gga tct gag atc atc cct gca gct ata aga gat cac aat gtt 1123
Asp Phe Gly Ser Glu Ile Ile Pro Ala Ala Ile Arg Asp His Asn Val
            330                 335                 340
caa gga tac gtc tac aga gac tat tgg gaa gat att gga act ata aag 1171
Gln Gly Tyr Val Tyr Arg Asp Tyr Trp Glu Asp Ile Gly Thr Ile Lys
        345                 350                 355
agc ttc tat gat gct aat ctt gct ctt gtc gag gag cat ccc aag ttt 1219
Ser Phe Tyr Asp Ala Asn Leu Ala Leu Val Glu Glu His Pro Lys Phe
    360                 365                 370
gag ttt tat gat cag gat aca cct ttc tac act tct cct cgg ttt cta 1267
Glu Phe Tyr Asp Gln Asp Thr Pro Phe Tyr Thr Ser Pro Arg Phe Leu
375                 380                 385                 390
cct ccc acc aaa act gag aaa tgc cgc ata gtg gat tcg ata ata tca 1315
Pro Pro Thr Lys Thr Glu Lys Cys Arg Ile Val Asp Ser Ile Ile Ser
                395                 400                 405
cac gga tgt ttc ttg gga gaa tgc agc atc caa cgt tcc atc att ggt 1363
His Gly Cys Phe Leu Gly Glu Cys Ser Ile Gln Arg Ser Ile Ile Gly
            410                 415                 420
gaa agg tca cgt ctt gac tat ggt gtt gag ctt cag gat act ttg atg 1411
Glu Arg Ser Arg Leu Asp Tyr Gly Val Glu Leu Gln Asp Thr Leu Met
        425                 430                 435
ttg gga gcg gat agt tac caa acc gaa tct gag ata gcg tct ttg ctc 1459
Leu Gly Ala Asp Ser Tyr Gln Thr Glu Ser Glu Ile Ala Ser Leu Leu
    440                 445                 450
gcg gaa ggc aat gta cca att ggc att ggc aga gac aca aag atc agg 1507
Ala Glu Gly Asn Val Pro Ile Gly Ile Gly Arg Asp Thr Lys Ile Arg
455                 460                 465                 470
aaa tgc atc att gac aag aat gca aag atc ggg aag aac gtg atg atc 1555
Lys Cys Ile Ile Asp Lys Asn Ala Lys Ile Gly Lys Asn Val Met Ile
                475                 480                 485
ttg aac agt gat gat gtg caa gaa gct gat agg cca gag gag ggg ttc 1603
Leu Asn Ser Asp Asp Val Gln Glu Ala Asp Arg Pro Glu Glu Gly Phe
            490                 495                 500

tac att agg tct ggg atc act gtg gtt gtg gaa aag gct acc att aaa 1651
Tyr Ile Arg Ser Gly Ile Thr Val Val Val Glu Lys Ala Thr Ile Lys
        505                 510                 515
gac ggc act gtc ata tga accaacaacg gagtgatctg attgagaaca 1699
Asp Gly Thr Val Ile
    520
ctcttgacca acgatcttga ttgaggtgtt tttgaagtgt tgtttttggg tggaagcagt 1759

gaaaacagag acagttgtga taatgttttt aatgtaaatt acttgagagt actacttaga 1819

accaaaagag agtttaagct taccaataag agtgatctac ctataaaaaa aaaaaaaaa 1878

<210> 162
<211> 523
<212> PRT
<213> Brassica napus

<400> 162
Met Asp Ser Cys Cys Tyr Leu Gly Leu Gly Thr Asn Thr Val Leu Pro
1               5                   10                  15
Lys Asp Gly Tyr Lys Asn Ile Glu Asn Ser Phe Trp Gly Glu Lys Ile
            20                  25                  30
Lys Gly Ser Phe Ser Lys Pro Phe Ala Ser Asp Ser Ser Ser Lys Glu
        35                  40                  45
Leu Arg Tyr Arg Lys Ser Pro Arg Pro Gly Val Ala Tyr Ala Val Ala
    50                  55                  60
Thr Ser Lys Asn Ala Lys Glu Ala Leu Arg Leu Gln Pro Ser Met Leu
65                  70                  75                  80
Gly Glu Arg Arg Lys Ala Asp Pro Lys Asn Val Ala Ala Ile Ile Leu
                85                  90                  95
Gly Gly Gly Asn Gly Ala Lys Leu Phe Pro Leu Thr Lys Arg Ala Ala
            100                 105                 110
Thr Pro Ala Val Pro Val Gly Gly Cys Tyr Arg Met Ile Asp Ile Pro
        115                 120                 125
Met Ser Asn Cys Ile Asn Ser Cys Ile Asn Lys Ile Phe Val Leu Thr
    130                 135                 140
Gln Phe Asn Ser Ala Ser Leu Asn Arg His Leu Ala Arg Thr Tyr Phe
145                 150                 155                 160
Gly Asn Gly Ile Asn Phe Gly Asp Gly Phe Val Glu Val Leu Ala Ala
                165                 170                 175
Thr Gln Thr Pro Gly Glu Ala Gly Lys Lys Trp Phe Gln Gly Thr Ala
            180                 185                 190
Asp Ala Val Arg Lys Phe Leu Trp Val Phe Glu Asp Ala Lys Asn Arg
        195                 200                 205
Asn Ile Glu Asn Ile Ile Ile Leu Ser Gly Asp His Leu Tyr Arg Met
    210                 215                 220
Asn Tyr Met Asp Phe Val Gln Tyr His Val Asp Ser Asn Ala Asp Ile
225                 230                 235                 240
Thr Leu Ser Cys Ala Pro Val Gly Glu Ser Arg Ala Ser Asp Tyr Gly
                245                 250                 255
Leu Val Asn Ile Asp Arg Thr Gly Arg Val Val His Phe Cys Glu Lys
            260                 265                 270
Pro Thr Gly Ile Asp Leu Lys Ser Met Gln Ser Asp Thr Thr Met Leu
        275                 280                 285
Gly Leu Ser Gln Gln Glu Ala Val Lys Ser Pro Tyr Ile Ala Ser Met
    290                 295                 300
Gly Val Tyr Cys Phe Lys Thr Glu Ala Leu Leu Lys Leu Leu Thr Arg
305                 310                 315                 320
Arg Tyr Pro Ser Ser Asn Asp Phe Gly Ser Glu Ile Ile Pro Ala Ala
                325                 330                 335
Ile Arg Asp His Asn Val Gln Gly Tyr Val Tyr Arg Asp Tyr Trp Glu
            340                 345                 350
Asp Ile Gly Thr Ile Lys Ser Phe Tyr Asp Ala Asn Leu Ala Leu Val
        355                 360                 365
Glu Glu His Pro Lys Phe Glu Phe Tyr Asp Gln Asp Thr Pro Phe Tyr

370                 375                 380
Thr Ser Pro Arg Phe Leu Pro Pro Thr Lys Thr Glu Lys Cys Arg Ile
385                 390                 395                 400
Val Asp Ser Ile Ile Ser His Gly Cys Phe Leu Gly Glu Cys Ser Ile
                405                 410                 415
Gln Arg Ser Ile Ile Gly Glu Arg Ser Arg Leu Asp Tyr Gly Val Glu
            420                 425                 430
Leu Gln Asp Thr Leu Met Leu Gly Ala Asp Ser Tyr Gln Thr Glu Ser
        435                 440                 445
Glu Ile Ala Ser Leu Leu Ala Glu Gly Asn Val Pro Ile Gly Ile Gly
    450                 455                 460
Arg Asp Thr Lys Ile Arg Lys Cys Ile Ile Asp Lys Asn Ala Lys Ile
465                 470                 475                 480
Gly Lys Asn Val Met Ile Leu Asn Ser Asp Asp Val Gln Glu Ala Asp
                485                 490                 495
Arg Pro Glu Glu Gly Phe Tyr Ile Arg Ser Gly Ile Thr Val Val Val
            500                 505                 510
Glu Lys Ala Thr Ile Lys Asp Gly Thr Val Ile
        515                 520

<210> 163
<211> 2212
<212> DNA
<213> Glycine max

<220>
<221> CDS
<222> (383)..(1978)

<400> 163
gaattcccgg gtcgacagcg aagtcagtga cagatcgaaa gacttcgcag gtttctcgtg      60

agtcgttaac gttagtgtag tgtcccttgc tatttgccaa tacaactcat tctacatttc      120

tatcttctcc ttctccatta ccaaattttt ctgtacgtag tttctacctc ctaggtccca      180

ccttcgtttc tctgaaaaat ctcatctttt tggggctctt ttcacttgaa tctccccagt      240

tatgattcct caacttatgg tacaaattgt ctgaaaagtt tggtacttta ttaaagtagt      300

tttgctcttc cctcatacaa attccattga gcagaaagtt accaaatcca aaaaggtttc      360

tatttccgtt attgatttag ca atg gat tca gct tgt gca acc ctg aat ggc      412
                         Met Asp Ser Ala Cys Ala Thr Leu Asn Gly
                         1               5                   10
cgc cat cta gcc aaa gtt agt gag gga att gga aga aac aga aca agt      460
Arg His Leu Ala Lys Val Ser Glu Gly Ile Gly Arg Asn Arg Thr Ser
                15                  20                  25
ggc ttc tgg ggt gag agt acg agg gga agt gtg aac aca aaa agg ttt      508
Gly Phe Trp Gly Glu Ser Thr Arg Gly Ser Val Asn Thr Lys Arg Phe
            30                  35                  40
ttg agt gtt caa tca tgc aag act tca cga acc aat agg aat ctt aga      556
Leu Ser Val Gln Ser Cys Lys Thr Ser Arg Thr Asn Arg Asn Leu Arg
        45                  50                  55
aac tcc aag cct gga agt gga att gca cgc gct gtt ctc aca tca gac      604
Asn Ser Lys Pro Gly Ser Gly Ile Ala Arg Ala Val Leu Thr Ser Asp
    60                  65                  70
atc gac gaa gat tcc atg gca ttt caa ggg gta ccc act ttt gag aaa      652
Ile Asp Glu Asp Ser Met Ala Phe Gln Gly Val Pro Thr Phe Glu Lys
75                  80                  85                  90
cct gaa gtg gac cca aaa agt gtg gct tcc atc ata ttg ggt gga ggt      700
Pro Glu Val Asp Pro Lys Ser Val Ala Ser Ile Ile Leu Gly Gly Gly
                95                  100                 105

gca gga act cga ctc ttt cct ctt act ggc aga aga gcc aag cca gcg 748
Ala Gly Thr Arg Leu Phe Pro Leu Thr Gly Arg Arg Ala Lys Pro Ala
            110                 115                 120
gtt cca att gga ggg tgt tat aga ctc ata gat atc ccc atg agc aat 796
Val Pro Ile Gly Gly Cys Tyr Arg Leu Ile Asp Ile Pro Met Ser Asn
        125                 130                 135
tgc atc aat agt ggc atc aga aaa att ttc atc ttg acg cag ttc aat 844
Cys Ile Asn Ser Gly Ile Arg Lys Ile Phe Ile Leu Thr Gln Phe Asn
    140                 145                 150
tct ttc tct ctc aac cgc cac ctg tcc cgt gca tac agc ttc gga aat 892
Ser Phe Ser Leu Asn Arg His Leu Ser Arg Ala Tyr Ser Phe Gly Asn
155                 160                 165                 170
ggc atg act ttt gga gat ggg ttt gtg gag gtc ttg gca gct act caa 940
Gly Met Thr Phe Gly Asp Gly Phe Val Glu Val Leu Ala Ala Thr Gln
                175                 180                 185
aca ccg ggt gag gct ggg aag aag tgg ttc caa ggg aca gct gat gct 988
Thr Pro Gly Glu Ala Gly Lys Lys Trp Phe Gln Gly Thr Ala Asp Ala
            190                 195                 200
gta aga caa ttt ata tgg gtt ttt gag gat gcc aag aac aag aat gtt 1036
Val Arg Gln Phe Ile Trp Val Phe Glu Asp Ala Lys Asn Lys Asn Val
        205                 210                 215
gag cat ata ttg ata ctt tct ggc gat cat ctt tac cgt atg gac tat 1084
Glu His Ile Leu Ile Leu Ser Gly Asp His Leu Tyr Arg Met Asp Tyr
    220                 225                 230
atg gac ttt gta cag aga cat gtt gac aca aat gcc gat atc aca gtt 1132
Met Asp Phe Val Gln Arg His Val Asp Thr Asn Ala Asp Ile Thr Val
235                 240                 245                 250
tca tgt gta ccc atg gat gac agt cgg gca tca gac tat gga ctg atg 1180
Ser Cys Val Pro Met Asp Asp Ser Arg Ala Ser Asp Tyr Gly Leu Met
                255                 260                 265
aaa att gat aaa aca gga cgg att ata cag ttt gca gaa aaa cct aag 1228
Lys Ile Asp Lys Thr Gly Arg Ile Ile Gln Phe Ala Glu Lys Pro Lys
            270                 275                 280
gga tca gat cta aag gca atg cgt gtt gac acc act ctt tta ggg tta 1276
Gly Ser Asp Leu Lys Ala Met Arg Val Asp Thr Thr Leu Leu Gly Leu
        285                 290                 295
ttg cca caa gaa gca gaa aaa cat cct tat att gca tcc atg ggt gtc 1324
Leu Pro Gln Glu Ala Glu Lys His Pro Tyr Ile Ala Ser Met Gly Val
    300                 305                 310
tac gtg ttt aga act gaa acc ttg ctg caa cta tta aga tgg aaa tgt 1372
Tyr Val Phe Arg Thr Glu Thr Leu Leu Gln Leu Leu Arg Trp Lys Cys
315                 320                 325                 330
tct tca tgc aat gac ttt gga tct gaa att atc cca tct gct gtg aat 1420
Ser Ser Cys Asn Asp Phe Gly Ser Glu Ile Ile Pro Ser Ala Val Asn
                335                 340                 345
gag cac aat gtc cag gca tat ttg ttc aat gac tac tgg gaa gat att 1468
Glu His Asn Val Gln Ala Tyr Leu Phe Asn Asp Tyr Trp Glu Asp Ile
            350                 355                 360
gga act ata aag tcc ttc ttt gat gca aat ctt gct cta aca gaa cag 1516
Gly Thr Ile Lys Ser Phe Phe Asp Ala Asn Leu Ala Leu Thr Glu Gln
        365                 370                 375
cct cct aaa ttt gaa ttc tat gat cca aag aca cct ttc ttc act tcc 1564
Pro Pro Lys Phe Glu Phe Tyr Asp Pro Lys Thr Pro Phe Phe Thr Ser
    380                 385                 390
ccc aga ttc cta cca cct acc aaa gta gaa aaa tgc aag att gtg gat 1612
Pro Arg Phe Leu Pro Pro Thr Lys Val Glu Lys Cys Lys Ile Val Asp
395                 400                 405                 410
gca att ata tct cat ggt tgc ttc ttg agg gag tgc agc gtt caa cat 1660
Ala Ile Ile Ser His Gly Cys Phe Leu Arg Glu Cys Ser Val Gln His
                415                 420                 425
tct att gtt gga gta cgc tca cgt ttg gag tct ggt gtg gag ctt cag 1708
Ser Ile Val Gly Val Arg Ser Arg Leu Glu Ser Gly Val Glu Leu Gln
            430                 435                 440
gat acg atg atg atg ggt gct gac tat tat caa act gag tat gaa att 1756
Asp Thr Met Met Met Gly Ala Asp Tyr Tyr Gln Thr Glu Tyr Glu Ile
        445                 450                 455
gca tct ctg gtg gca gaa ggg aag gtt cca att ggt gtc ggg gca aat 1804
Ala Ser Leu Val Ala Glu Gly Lys Val Pro Ile Gly Val Gly Ala Asn
    460                 465                 470

act aaa atc agg aat tgc ata atc gac aag aat gcc aag ata gga aga 1852
Thr Lys Ile Arg Asn Cys Ile Ile Asp Lys Asn Ala Lys Ile Gly Arg
475                 480                 485                 490
aat gtg atc ata gca aac acc gat ggt gtt caa gaa gct gac agg gca 1900
Asn Val Ile Ile Ala Asn Thr Asp Gly Val Gln Glu Ala Asp Arg Ala
                495                 500                 505
aag gaa gga ttc tac att agg tcg ggc atc aca gtt aca tta aaa aat 1948
Lys Glu Gly Phe Tyr Ile Arg Ser Gly Ile Thr Val Thr Leu Lys Asn
            510                 515                 520
gca aca atc aaa gat gga aca gtt ata tga agcacttcaa gttatccagc 1998
Ala Thr Ile Lys Asp Gly Thr Val Ile
        525                 530
aggccacttt ataatagttt tgacataagt acattcactt ccgacataat agtagatgaa 2058

atgctagctc gtacattaca aagtttctct cgatatatct ctataatagt tatgtttatt 2118

gcatttgtag gacacttcac ttgtaataac aggcaattct tgccactacg attaagttat 2178

aaattaaagt ttctaactta aaaaaaaaaa aaaa 2212

<210> 164
<211> 531
<212> PRT
<213> Glycine max

<400> 164
Met Asp Ser Ala Cys Ala Thr Leu Asn Gly Arg His Leu Ala Lys Val
1               5                   10                  15
Ser Glu Gly Ile Gly Arg Asn Arg Thr Ser Gly Phe Trp Gly Glu Ser
            20                  25                  30
Thr Arg Gly Ser Val Asn Thr Lys Arg Phe Leu Ser Val Gln Ser Cys
        35                  40                  45
Lys Thr Ser Arg Thr Asn Arg Asn Leu Arg Asn Ser Lys Pro Gly Ser
    50                  55                  60
Gly Ile Ala Arg Ala Val Leu Thr Ser Asp Ile Asp Glu Asp Ser Met
65                  70                  75                  80
Ala Phe Gln Gly Val Pro Thr Phe Glu Lys Pro Glu Val Asp Pro Lys
                85                  90                  95
Ser Val Ala Ser Ile Ile Leu Gly Gly Gly Ala Gly Thr Arg Leu Phe
            100                 105                 110
Pro Leu Thr Gly Arg Arg Ala Lys Pro Ala Val Pro Ile Gly Gly Cys
        115                 120                 125
Tyr Arg Leu Ile Asp Ile Pro Met Ser Asn Cys Ile Asn Ser Gly Ile
    130                 135                 140
Arg Lys Ile Phe Ile Leu Thr Gln Phe Asn Ser Phe Ser Leu Asn Arg
145                 150                 155                 160
His Leu Ser Arg Ala Tyr Ser Phe Gly Asn Gly Met Thr Phe Gly Asp
                165                 170                 175
Gly Phe Val Glu Val Leu Ala Ala Thr Gln Thr Pro Gly Glu Ala Gly
            180                 185                 190
Lys Lys Trp Phe Gln Gly Thr Ala Asp Ala Val Arg Gln Phe Ile Trp
        195                 200                 205
Val Phe Glu Asp Ala Lys Asn Lys Asn Val Glu His Ile Leu Ile Leu
    210                 215                 220
Ser Gly Asp His Leu Tyr Arg Met Asp Tyr Met Asp Phe Val Gln Arg
225                 230                 235                 240
His Val Asp Thr Asn Ala Asp Ile Thr Val Ser Cys Val Pro Met Asp
                245                 250                 255
Asp Ser Arg Ala Ser Asp Tyr Gly Leu Met Lys Ile Asp Lys Thr Gly
            260                 265                 270
Arg Ile Ile Gln Phe Ala Glu Lys Pro Lys Gly Ser Asp Leu Lys Ala
        275                 280                 285
Met Arg Val Asp Thr Thr Leu Leu Gly Leu Leu Pro Gln Glu Ala Glu
    290                 295                 300

Lys His Pro Tyr Ile Ala Ser Met Gly Val Tyr Val Phe Arg Thr Glu
305                 310                 315                 320
Thr Leu Leu Gln Leu Leu Arg Trp Lys Cys Ser Ser Cys Asn Asp Phe
                325                 330                 335
Gly Ser Glu Ile Ile Pro Ser Ala Val Asn Glu His Asn Val Gln Ala
            340                 345                 350
Tyr Leu Phe Asn Asp Tyr Trp Glu Asp Ile Gly Thr Ile Lys Ser Phe
        355                 360                 365
Phe Asp Ala Asn Leu Ala Leu Thr Glu Gln Pro Pro Lys Phe Glu Phe
    370                 375                 380
Tyr Asp Pro Lys Thr Pro Phe Phe Thr Ser Pro Arg Phe Leu Pro Pro
385                 390                 395                 400
Thr Lys Val Glu Lys Cys Lys Ile Val Asp Ala Ile Ile Ser His Gly
                405                 410                 415
Cys Phe Leu Arg Glu Cys Ser Val Gln His Ser Ile Val Gly Val Arg
            420                 425                 430
Ser Arg Leu Glu Ser Gly Val Glu Leu Gln Asp Thr Met Met Met Gly
        435                 440                 445
Ala Asp Tyr Tyr Gln Thr Glu Tyr Glu Ile Ala Ser Leu Val Ala Glu
    450                 455                 460
Gly Lys Val Pro Ile Gly Val Gly Ala Asn Thr Lys Ile Arg Asn Cys
465                 470                 475                 480
Ile Ile Asp Lys Asn Ala Lys Ile Gly Arg Asn Val Ile Ile Ala Asn
                485                 490                 495
Thr Asp Gly Val Gln Glu Ala Asp Arg Ala Lys Glu Gly Phe Tyr Ile
            500                 505                 510
Arg Ser Gly Ile Thr Val Thr Leu Lys Asn Ala Thr Ile Lys Asp Gly
        515                 520                 525
Thr Val Ile
    530

<210> 165
<211> 21
<212> DNA
<213> Artificial sequence

<400> 165
atggcgacaa tggctgcaat c                                                21

<210> 166
<211> 25
<212> DNA
<213> Artificial sequence

<400> 166
ttagataaca gttccactag gaatc                                            25


<210> 167
<211> 527
<212> PRT
<213> Artificial sequence

<220>
<221> Variant
<222> (2)..(4)
<223> xaa in position 2 to 4 is any amino acid
<220>
<221> Variant
<222> (10)..(10)
<223> xaa in position 10 is any amino acid
<220>
<221> Variant
<222> (13)..(13)
<223> xaa in position 13 is any amino acid
<220>
<221> Variant
<222> (15)..(15)
<223> xaa in position 15 is any amino acid

<220>
<221> Variant
<222> (19)..(20)
<223> xaa in position 19 to 20 is any amino acid
<220>
<221> Variant
<222> (23)..(23)
<223> xaa in position 23 is any amino acid
<220>
<221> Variant
<222> (28)..(28)
<223> xaa in position 28 is any amino acid
<220>
<221> Variant
<222> (30)..(31)
<223> xaa in position 30 to 31 is any amino acid
<220>
<221> Variant
<222> (39)..(39)
<223> xaa in position 39 is any amino acid
<220>
<221> Variant
<222> (43)..(43)
<223> xaa in position 43 is any amino acid
<220>
<221> Variant
<222> (46)..(46)
<223> xaa in position 46 is any amino acid
<220>
<221> Variant
<222> (48)..(48)
<223> xaa in position 48 is any amino acid
<220>
<221> Variant
<222> (50)..(50)
<223> xaa in position 50 is any amino acid
<220>
<221> Variant
<222> (52)..(53)
<223> xaa in position 52 to 53 is any amino acid
<220>
<221> Variant
<222> (62)..(62)
<223> xaa in position 62 is any amino acid
<220>
<221> Variant
<222> (67)..(75)
<223> xaa in position 67 to 75 is any amino acid
<220>
<221> Variant
<222> (77)..(81)
<223> xaa in position 77 to 81 is any amino acid
<220>
<221> Variant
<222> (83)..(83)
<223> xaa in position 83 is any amino acid
<220>
<221> Variant
<222> (90)..(90)
<223> xaa in position 90 is any amino acid
<220>
<221> Variant
<222> (92)..(92)
<223> xaa in position 92 is any amino acid
<220>
<221> Variant
<222> (94)..(99)
<223> xaa in position 94 to 99 is any amino acid
<220>

<221> Variant
<222> (110)..(112)
<223> xaa in position 110 to 112 is any amino acid
<220>
<221> Variant
<222> (114)..(114)
<223> xaa in position 114 is any amino acid
<220>
<221> Variant
<222> (117)..(127)
<223> xaa in position 117 to 127 is any amino acid
<220>
<221> Variant
<222> (129)..(129)
<223> xaa in position 129 is any amino acid
<220>
<221> Variant
<222> (131)..(131)
<223> xaa in position 131 is any amino acid
<220>
<221> Variant
<222> (133)..(140)
<223> xaa in position 133 to 140 is any amino acid
<220>
<221> Variant
<222> (149)..(152)
<223> xaa in position 149 to 152 is any amino acid
<220>
<221> Variant
<222> (154)..(154)
<223> xaa in position 154 is any amino acid
<220>
<221> Variant
<222> (156)..(159)
<223> xaa in position 156 to 159 is any amino acid
<220>
<221> Variant
<222> (164)..(167)
<223> xaa in position 164 to 167 is any amino acid
<220>
<221> Variant
<222> (169)..(197)
<223> xaa in position 169 to 197 is any amino acid
<220>
<221> Variant
<222> (200)..(202)
<223> xaa in position 200 to 202 is any amino acid
<220>
<221> Variant
<222> (205)..(205)
<223> xaa in position 205 is any amino acid
<220>
<221> Variant
<222> (209)..(210)
<223> xaa in position 209 to 210 is any amino acid
<220>
<221> Variant
<222> (213)..(215)
<223> xaa in position 213 to 215 is any amino acid
<220>
<221> Variant
<222> (217)..(217)
<223> xaa in position 217 is any amino acid
<220>
<221> Variant
<222> (223)..(224)
<223> xaa in position 223 to 224 is any amino acid
<220>
<221> Variant

<222> (226)..(227)
<223> xaa in position 226 to 227 is any amino acid
<220>
<221> Variant
<222> (229)..(229)
<223> xaa in position 229 is any amino acid
<220>
<221> Variant
<222> (233)..(234)
<223> xaa in position 233 to 234 is any amino acid
<220>
<221> Variant
<222> (238)..(242)
<223> xaa in position 238 to 242 is any amino acid
<220>
<221> Variant
<222> (244)..(246)
<223> xaa in position 244 to 246 is any amino acid
<220>
<221> Variant
<222> (254)..(255)
<223> xaa in position 254 to 255 is any amino acid
<220>
<221> Variant
<222> (258)..(263)
<223> xaa in position 258 to 263 is any amino acid
<220>
<221> Variant
<222> (265)..(265)
<223> xaa in position 265 is any amino acid
<220>
<221> Variant
<222> (268)..(286)
<223> xaa in position 268 to 286 is any amino acid
<220>
<221> Variant
<222> (288)..(289)
<223> xaa in position 288 to 289 is any amino acid
<220>
<221> Variant
<222> (296)..(296)
<223> xaa in position 296 is any amino acid
<220>
<221> Variant
<222> (299)..(299)
<223> xaa in position 299 is any amino acid
<220>
<221> Variant
<222> (301)..(308)
<223> xaa in position 301 to 308 is any amino acid
<220>
<221> Variant
<222> (311)..(314)
<223> xaa in position 311 to 314 is any amino acid
<220>
<221> Variant
<222> (323)..(324)
<223> xaa in position 323 to 324 is any amino acid
<220>
<221> Variant
<222> (326)..(327)
<223> xaa in position 326 to 327 is any amino acid
<220>
<221> Variant
<222> (331)..(332)
<223> xaa in position 331 to 332 is any amino acid
<220>
<221> Variant
<222> (334)..(337)

<223> xaa in position 334 to 337 is any amino acid
<220>
<221> Variant
<222> (339)..(339)
<223> xaa in position 339 is any amino acid
<220>
<221> Variant
<222> (341)..(341)
<223> xaa in position 341 is any amino acid
<220>
<221> Variant
<222> (345)..(347)
<223> xaa in position 345 to 347 is any amino acid
<220>
<221> Variant
<222> (349)..(350)
<223> xaa in position 349 to 350 is any amino acid
<220>
<221> Variant
<222> (352)..(353)
<223> xaa in position 352 to 353 is any amino acid
<220>
<221> Variant
<222> (355)..(355)
<223> xaa in position 355 is any amino acid
<220>
<221> Variant
<222> (359)..(359)
<223> xaa in position 359 is any amino acid
<220>
<221> Variant
<222> (361)..(369)
<223> xaa in position 361 to 369 is any amino acid
<220>
<221> Variant
<222> (371)..(372)
<223> xaa in position 371 to 372 is any amino acid
<220>
<221> Variant
<222> (374)..(375)
<223> xaa in position 374 to 375 is any amino acid
<220>
<221> Variant
<222> (378)..(382)
<223> xaa in position 378 to 382 is any amino acid
<220>
<221> Variant
<222> (384)..(386)
<223> xaa in position 384 to 386 is any amino acid
<220>
<221> Variant
<222> (389)..(390)
<223> xaa in position 389 to 390 is any amino acid
<220>
<221> Variant
<222> (392)..(392)
<223> xaa in position 392 is any amino acid
<220>
<221> Variant
<222> (395)..(398)
<223> xaa in position 395 to 398 is any amino acid
<220>
<221> Variant
<222> (400)..(403)
<223> xaa in position 400 to 403 is any amino acid
<220>
<221> Variant
<222> (405)..(407)
<223> xaa in position 405 to 407 is any amino acid

<220>
<221> Variant
<222> (412)..(412)
<223> xaa in position 412 is any amino acid
<220>
<221> Variant
<222> (414)..(414)
<223> xaa in position 414 is any amino acid
<220>
<221> Variant
<222> (416)..(421)
<223> xaa in position 416 to 421 is any amino acid
<220>
<221> Variant
<222> (423)..(425)
<223> xaa in position 423 to 425 is any amino acid
<220>
<221> Variant
<222> (427)..(428)
<223> xaa in position 427 to 428 is any amino acid
<220>
<221> Variant
<222> (432)..(432)
<223> xaa in position 432 is any amino acid
<220>
<221> Variant
<222> (434)..(434)
<223> xaa in position 434 is any amino acid
<220>
<221> Variant
<222> (436)..(437)
<223> xaa in position 436 to 437 is any amino acid
<220>
<221> Variant
<222> (439)..(470)
<223> xaa in position 439 to 470 is any amino acid
<220>
<221> Variant
<222> (477)..(477)
<223> xaa in position 477 is any amino acid
<220>
<221> Variant
<222> (480)..(481)
<223> xaa in position 480 to 481 is any amino acid
<220>
<221> Variant
<222> (483)..(483)
<223> xaa in position 483 is any amino acid
<220>
<221> Variant
<222> (485)..(485)
<223> xaa in position 485 is any amino acid
<220>
<221> Variant
<222> (487)..(490)
<223> xaa in position 487 to 490 is any amino acid
<220>
<221> Variant
<222> (495)..(495)
<223> xaa in position 495 is any amino acid
<220>
<221> Variant
<222> (497)..(499)
<223> xaa in position 497 to 499 is any amino acid
<220>
<221> Variant
<222> (501)..(504)
<223> xaa in position 501 to 504 is any amino acid
<220>

<221> Variant
<222> (506)..(506)
<223> xaa in position 506 is any amino acid
<220>
<221> Variant
<222> (510)..(515)
<223> xaa in position 510 to 515 is any amino acid
<220>
<221> Variant
<222> (517)..(520)
<223> xaa in position 517 to 520 is any amino acid
<220>
<221> Variant
<222> (522)..(523)
<223> xaa in position 522 to 523 is any amino acid
<220>
<221> Variant
<222> (525)..(526)
<223> xaa in position 525 to 526 is any amino acid

<400> 167
Val xaa xaa xaa Ile Leu Gly Gly Gly xaa Gly Thr xaa Leu xaa Pro
1               5                   10                  15
Leu Thr xaa xaa Arg Ala xaa Pro Ala Val Pro xaa Gly xaa xaa Tyr
            20                  25                  30
Arg Leu Ile Asp Ile Pro xaa Ser Asn Cys xaa Asn Ser xaa Ile xaa
        35                  40                  45
Lys xaa Ile xaa xaa Leu Thr Gln Phe Asn Ser Ala Ser xaa Leu Asn
    50                  55                  60
Arg His xaa xaa xaa xaa xaa xaa xaa xaa xaa Gly xaa xaa xaa xaa
65                  70                  75                  80
xaa Gly xaa Val Glu Val Leu Ala Ala xaa Gln xaa Pro xaa xaa xaa
                85                  90                  95
xaa xaa xaa Trp Phe Gln Gly Thr Ala Asp Ala Val Arg xaa xaa xaa
            100                 105                 110
Trp xaa Phe Glu xaa xaa xaa xaa xaa xaa xaa xaa xaa xaa xaa Leu
        115                 120                 125
xaa Leu xaa Gly xaa xaa xaa xaa xaa xaa xaa xaa Asp His Leu Tyr
    130                 135                 140
Arg Met Asp Tyr xaa xaa xaa xaa Gln xaa His xaa xaa xaa xaa Ala
145                 150                 155                 160
Asp Ile Thr xaa xaa xaa xaa Pro xaa xaa xaa xaa xaa xaa xaa xaa
                165                 170                 175
xaa xaa xaa xaa xaa xaa xaa xaa xaa xaa xaa xaa xaa xaa xaa xaa
            180                 185                 190
xaa xaa xaa xaa xaa Arg Ala xaa xaa xaa Gly Leu xaa Lys Ile Asp
        195                 200                 205
xaa xaa Gly Arg xaa xaa xaa Phe xaa Glu Lys Pro Lys Gly xaa xaa
    210                 215                 220
Leu xaa xaa Met xaa Val Asp Thr xaa xaa Leu Gly Leu xaa xaa xaa
225                 230                 235                 240
xaa xaa Ala xaa xaa xaa Pro Tyr Ile Ala Ser Met Gly xaa xaa Tyr
                245                 250                 255
Val xaa xaa xaa xaa xaa xaa Leu xaa Leu Leu xaa xaa xaa xaa xaa
            260                 265                 270
xaa xaa xaa xaa xaa xaa xaa xaa xaa xaa xaa xaa xaa xaa Pro xaa
        275                 280                 285
xaa Asn Asp Phe Gly Ser Glu xaa Ile Pro xaa Ala xaa xaa xaa xaa
    290                 295                 300
xaa xaa xaa xaa Ala Tyr xaa xaa xaa xaa Tyr Trp Glu Asp Ile Gly
305                 310                 315                 320
Thr Ile xaa xaa Phe xaa xaa Ala Asn Leu xaa xaa Thr xaa xaa xaa
                325                 330                 335
xaa Pro xaa Phe xaa Phe Tyr Asp xaa xaa xaa Pro xaa xaa Thr xaa
            340                 345                 350
xaa Arg xaa Leu Pro Pro xaa Lys xaa xaa xaa xaa xaa xaa xaa xaa
        355                 360                 365
xaa Asp xaa xaa Ile xaa xaa Gly Cys xaa xaa xaa xaa xaa Cys xaa
    370                 375                 380

xaa xaa His Ser xaa xaa Gly xaa Arg Ser xaa xaa xaa xaa Gly xaa
385                 390                 395                 400
xaa xaa xaa Asp xaa xaa xaa Met Gly Ala Asp xaa Tyr xaa Thr xaa
                405                 410                 415
xaa xaa xaa xaa xaa Leu xaa xaa xaa Gly xaa xaa Pro Ile Gly xaa
            420                 425                 430
Gly xaa Asn xaa xaa Ile xaa xaa xaa xaa xaa xaa xaa xaa xaa xaa
        435                 440                 445
xaa xaa xaa xaa xaa xaa xaa xaa xaa xaa xaa xaa xaa xaa xaa xaa
    450                 455                 460
xaa xaa xaa xaa xaa xaa Ile Ile Asp Lys Asn Ala xaa Ile Gly xaa
465                 470                 475                 480
xaa Val xaa Ile xaa Asn xaa xaa xaa xaa Val Gln Glu Ala xaa Arg
                485                 490                 495
xaa xaa xaa Gly xaa xaa xaa xaa Ile xaa Ser Gly Ile xaa xaa xaa
            500                 505                 510
xaa xaa xaa Ala xaa xaa xaa xaa Ile xaa xaa Gly xaa xaa Ile
        515                 520                 525

<210> 168
<211> 57
<212> PRT
<213> Artificial sequence

<220>
<221> Variant
<222> (1)..(1)
<223> xaa in position 1 is Gly, Arg or Phe
<220>
<221> Variant
<222> (2)..(2)
<223> xaa in position 2 is Thr, Ser, Ala, Lys or Val
<220>
<221> Variant
<222> (3)..(3)
<223> xaa in position 3 is any amino acid
<220>
<221> Variant
<222> (5)..(5)
<223> xaa in position 5 is Phe, Tyr or Gln
<220>
<221> Variant
<222> (6)..(6)
<223> xaa in position 6 is Pro or Leu
<220>
<221> Variant
<222> (8)..(8)
<223> xaa in position 8 is Thr or Arg
<220>
<221> Variant
<222> (9)..(11)
<223> xaa in position 9 to 11 is any amino acid
<220>
<221> Variant
<222> (13)..(13)
<223> xaa in position 13 is any amino acid
<220>
<221> Variant
<222> (14)..(14)
<223> xaa in position 14 is Pro, Arg or Ala
<220>
<221> Variant
<222> (18)..(18)
<223> xaa in position 18 is Leu, Ile, Met, Val, Phe or Ala
<220>
<221> Variant
<222> (20)..(20)
<223> xaa in position 20 is Gly or Ala

<220>
<221> Variant
<222> (21)..(21)
<223> Xaa in position 21 is any amino acid
<220>
<221> Variant
<222> (24)..(24)
<223> Xaa in position 24 is Leu, Met or Lys
<220>
<221> Variant
<222> (25)..(25)
<223> Xaa in position 25 is Ile or Asn
<220>
<221> Variant
<222> (27)..(27)
<223> Xaa in position 27 is Ile or Val
<220>
<221> Variant
<222> (29)..(29)
<223> Xaa in position 29 is Met, Ile or Val
<220>
<221> Variant
<222> (33)..(33)
<223> Xaa in position 33 is Leu, Ile or Phe
<220>
<221> Variant
<222> (36)..(36)
<223> Xaa in position 36 is any amino acid
<220>
<221> Variant
<222> (37)..(37)
<223> Xaa in position 37 is Ile or Val
<220>
<221> Variant
<222> (38)..(38)
<223> Xaa in position 38 is any amino acid
<220>
<221> Variant
<222> (40)..(40)
<223> Xaa in position 40 is any or no amino acid
<220>
<221> Variant
<222> (41)..(41)
<223> Xaa in position 41 is Ile or Val
<220>
<221> Variant
<222> (42)..(42)
<223> Xaa in position 42 is Tyr or Phe
<220>
<221> Variant
<222> (43)..(43)
<223> Xaa in position 43 is Val, Ile or Cys
<220>
<221> Variant
<222> (44)..(44)
<223> Xaa in position 44 is Leu, Met or Arg
<220>
<221> Variant
<222> (45)..(45)
<223> Xaa in position 45 is Thr or Ser
<220>
<221> Variant
<222> (47)..(47)
<223> Xaa in position 47 is Phe or Tyr
<220>
<221> Variant
<222> (49)..(49)
<223> Xaa in position 49 is Ser or Leu
<220>

<220>
<221> Variant
<222> (50)..(50)
<223> xaa in position 50 is any amino acid
<220>
<221> Variant
<222> (51)..(51)
<223> xaa in position 51 is Ser, Ala or Phe
<220>
<221> Variant
<222> (52)..(52)
<223> xaa in position 52 is any or no amino acid
<220>
<221> Variant
<222> (53)..(53)
<223> xaa in position 53 is Leu, Ile or Pro
<220>
<221> Variant
<222> (54)..(54)
<223> xaa in position 54 is Asn or Ile
<220>
<221> Variant
<222> (55)..(55)
<223> xaa in position 55 is Arg or Gly
<220>
<221> Variant
<222> (56)..(56)
<223> xaa in position 56 is His or Asn
<220>
<221> Variant
<222> (57)..(57)
<223> xaa in position 57 is Leu or Ile

<400> 168
   Xaa Xaa Xaa Leu Xaa Xaa Leu Xaa Xaa Xaa Xaa Ala Xaa Xaa Ala Val
   1               5                   10                  15
   Pro Xaa Gly Xaa Xaa Tyr Arg Xaa Xaa Asp Xaa Pro Xaa Ser Asn Cys
               20                  25                  30
   Xaa Asn Ser Xaa Xaa Xaa Lys Xaa Xaa Xaa Xaa Xaa Xaa Gln Xaa Asn
           35                  40                  45
   Xaa Xaa Xaa Xaa Xaa Xaa Xaa Xaa Xaa
       50                  55


<210> 169
<211> 68
<212> PRT
<213> Artificial sequence

<220>
<221> Variant
<222> (2)..(2)
<223> xaa in position 2 is any amino acid
<220>
<221> Variant
<222> (4)..(4)
<223> xaa in position 4 is Glu or Gln
<220>
<221> Variant
<222> (5)..(5)
<223> xaa in position 5 is Val, Ala or Gly
<220>
<221> Variant
<222> (6)..(6)
<223> xaa in position 6 is Leu or Phe
<220>
<221> Variant
<222> (8)..(8)
<223> xaa in position 8 is Ala, Gly or Asp
<220>

<221> Variant
<222> (9)..(9)
<223> Xaa in position 9 is any amino acid
<220>
<221> Variant
<222> (10)..(10)
<223> Xaa in position 10 is Gln or His
<220>
<221> Variant
<222> (11)..(16)
<223> Xaa in position 11 to 16 is any amino acid
<220>
<221> Variant
<222> (17)..(18)
<223> Xaa in position 17 to 18 is any or no amino acid
<220>
<221> Variant
<222> (21)..(21)
<223> Xaa in position 21 is Gln, Arg or Glu
<220>
<221> Variant
<222> (22)..(22)
<223> Xaa in position 22 is Gly or Ala
<220>
<221> Variant
<222> (23)..(23)
<223> Xaa in position 23 is any or no amino acid
<220>
<221> Variant
<222> (25)..(25)
<223> Xaa in position 25 is Asp or His
<220>
<221> Variant
<222> (26)..(26)
<223> Xaa in position 26 is Ala or Ser
<220>
<221> Variant
<222> (27)..(27)
<223> Xaa in position 27 is Val or Ile
<220>
<221> Variant
<222> (28)..(28)
<223> Xaa in position 28 is Arg or Thr
<220>
<221> Variant
<222> (29)..(29)
<223> Xaa in position 29 is any amino acid
<220>
<221> Variant
<222> (30)..(30)
<223> Xaa in position 30 is Phe, Tyr or Cys
<220>
<221> Variant
<222> (31)..(31)
<223> Xaa in position 31 is any amino acid
<220>
<221> Variant
<222> (33)..(33)
<223> Xaa in position 33 is Leu, Ile, Val or Ala
<220>
<221> Variant
<222> (34)..(34)
<223> Xaa in position 34 is any or no amino acid
<220>
<221> Variant
<222> (35)..(35)
<223> Xaa in position 35 is Phe or Leu
<220>
<221> Variant

<222> (36)..(36)
<223> xaa in position 36 is Glu or Asp
<220>
<221> Variant
<222> (37)..(37)
<223> xaa in position 37 is Asp, Glu or Asn
<220>
<221> Variant
<222> (38)..(39)
<223> xaa in position 38 to 39 is any amino acid
<220>
<221> Variant
<222> (40)..(43)
<223> xaa in position 40 to 43 is any or no amino acid
<220>
<221> Variant
<222> (44)..(44)
<223> xaa in position 44 is Val or Ile
<220>
<221> Variant
<222> (45)..(47)
<223> xaa in position 45 to 47 is any amino acid
<220>
<221> Variant
<222> (48)..(48)
<223> xaa in position 48 is Leu, Ile, Val or Ala
<220>
<221> Variant
<222> (49)..(49)
<223> xaa in position 49 is Ile or Val
<220>
<221> Variant
<222> (51)..(51)
<223> xaa in position 51 is Ala, Ser or Cys
<220>
<221> Variant
<222> (53)..(60)
<223> xaa in position 53 to 60 is any or no amino acid
<220>
<221> Variant
<222> (62)..(62)
<223> xaa in position 62 is any amino acid
<220>
<221> Variant
<222> (63)..(63)
<223> xaa in position 63 is Leu or Met
<220>
<221> Variant
<222> (65)..(65)
<223> xaa in position 65 is Arg or Gln
<220>
<221> Variant
<222> (67)..(67)
<223> xaa in position 67 is any amino acid

<400> 169
Gly xaa Val xaa xaa xaa Ala xaa xaa xaa xaa xaa xaa xaa xaa xaa
1               5                   10                  15
xaa xaa Trp Phe xaa xaa xaa Ala xaa xaa xaa xaa xaa xaa Trp
            20                  25                  30
xaa xaa xaa xaa xaa xaa xaa xaa xaa xaa xaa xaa xaa xaa xaa xaa
        35                  40                  45
xaa Leu xaa Gly xaa xaa xaa xaa xaa xaa xaa xaa Asp xaa xaa Tyr
    50                  55                  60
xaa Met xaa Tyr
65


<210> 170

<211> 31
<212> PRT
<213> Artificial sequence

<220>
<221> Variant
<222> (1)..(1)
<223> Xaa in position 1 is Arg, Glu or Leu
<220>
<221> Variant
<222> (2)..(2)
<223> Xaa in position 2 is Ala, Ser or Pro
<220>
<221> Variant
<222> (3)..(3)
<223> Xaa in position 3 is Ser or Thr
<220>
<221> Variant
<222> (4)..(4)
<223> Xaa in position 4 is any amino acid
<220>
<221> Variant
<222> (5)..(5)
<223> Xaa in position 5 is Phe, Tyr or Asn
<220>
<221> Variant
<222> (8)..(8)
<223> Xaa in position 8 is Met, Val or Leu
<220>
<221> Variant
<222> (9)..(9)
<223> Xaa in position 9 is Lys or Asn
<220>
<221> Variant
<222> (10)..(10)
<223> Xaa in position 10 is any amino acid
<220>
<221> Variant
<222> (12)..(13)
<223> Xaa in position 12 to 13 is any amino acid
<220>
<221> Variant
<222> (15)..(15)
<223> Xaa in position 15 is Arg, Lys, Ala or Asn
<220>
<221> Variant
<222> (16)..(16)
<223> Xaa in position 16 is Ile or Val
<220>
<221> Variant
<222> (17)..(18)
<223> Xaa in position 17 to 18 is any amino acid
<220>
<221> Variant
<222> (20)..(20)
<223> Xaa in position 20 is Ala, Ser, Leu, Phe or Cys
<220>
<221> Variant
<222> (22)..(22)
<223> Xaa in position 22 is Lys or Asn
<220>
<221> Variant
<222> (23)..(23)
<223> Xaa in position 23 is Pro or Gln
<220>
<221> Variant
<222> (24)..(27)
<223> Xaa in position 24 to 27 is any amino acid
<220>

<221> Variant
<222> (28)..(28)
<223> xaa in position 28 is Leu, Met, His or Arg
<220>
<221> Variant
<222> (29)..(30)
<223> xaa in position 29 to 30 is any amino acid

<400> 170
xaa xaa xaa xaa xaa Gly Leu xaa xaa xaa Asp xaa xaa Gly xaa xaa
1               5                   10                  15
xaa xaa Phe xaa Glu xaa xaa xaa xaa xaa xaa xaa xaa xaa Met
            20                  25                  30


<210> 171
<211> 68
<212> PRT
<213> Artificial sequence

<220>
<221> Variant
<222> (4)..(4)
<223> xaa in position 4 is Ser or Asn
<220>
<221> Variant
<222> (6)..(6)
<223> xaa in position 6 is Ile or Val
<220>
<221> Variant
<222> (7)..(7)
<223> xaa in position 7 is Ile or Leu
<220>
<221> Variant
<222> (9)..(9)
<223> xaa in position 9 is any amino acid
<220>
<221> Variant
<222> (10)..(10)
<223> xaa in position 10 is Ala or Ser
<220>
<221> Variant
<222> (11)..(20)
<223> xaa in position 11 to 20 is any amino acid
<220>
<221> Variant
<222> (21)..(21)
<223> xaa in position 21 is any or no amino acid
<220>
<221> Variant
<222> (22)..(22)
<223> xaa in position 22 is Phe or Tyr
<220>
<221> Variant
<222> (23)..(24)
<223> xaa in position 23 to 24 is any amino acid
<220>
<221> Variant
<222> (29)..(29)
<223> xaa in position 29 is Ile or Val
<220>
<221> Variant
<222> (31)..(31)
<223> xaa in position 31 is Thr or Ser
<220>
<221> Variant
<222> (32)..(32)
<223> xaa in position 32 is Ile or Val
<220>

<221> Variant
<222> (33)..(33)
<223> Xaa in position 33 is any amino acid
<220>
<221> Variant
<222> (34)..(34)
<223> Xaa in position 34 is Ser, Ala or Thr
<220>
<221> Variant
<222> (36)..(36)
<223> Xaa in position 36 is Tyr, Phe or Cys
<220>
<221> Variant
<222> (37)..(37)
<223> Xaa in position 37 is any amino acid
<220>
<221> Variant
<222> (38)..(38)
<223> Xaa in position 38 is Ala or Ser
<220>
<221> Variant
<222> (39)..(39)
<223> Xaa in position 39 is Asn or Ser
<220>
<221> Variant
<222> (40)..(40)
<223> Xaa in position 40 is Leu, Ile or Met
<220>
<221> Variant
<222> (41)..(41)
<223> Xaa in position 41 is Ala, Gly or Ser
<220>
<221> Variant
<222> (42)..(42)
<223> Xaa in position 42 is Leu or Ile
<220>
<221> Variant
<222> (43)..(48)
<223> Xaa in position 43 to 48 is any amino acid
<220>
<221> Variant
<222> (49)..(49)
<223> Xaa in position 49 is any or no amino acid
<220>
<221> Variant
<222> (51)..(51)
<223> Xaa in position 51 is any amino acid
<220>
<221> Variant
<222> (52)..(52)
<223> Xaa in position 52 is Phe or Leu
<220>
<221> Variant
<222> (53)..(53)
<223> Xaa in position 53 is Tyr or Asn
<220>
<221> Variant
<222> (54)..(54)
<223> Xaa in position 54 is Asp or Gly
<220>
<221> Variant
<222> (55)..(57)
<223> Xaa in position 55 to 57 is any amino acid
<220>
<221> Variant
<222> (58)..(58)
<223> Xaa in position 58 is Pro or Ala
<220>
<221> Variant

<222> (59)..(59)
<223> xaa in position 59 is any amino acid
<220>
<221> Variant
<222> (60)..(60)
<223> xaa in position 60 is Tyr, Phe or Asn
<220>
<221> Variant
<222> (62)..(63)
<223> xaa in position 62 to 63 is any amino acid
<220>
<221> Variant
<222> (64)..(64)
<223> xaa in position 64 is Arg or Gly
<220>
<221> Variant
<222> (65)..(65)
<223> xaa in position 65 is any amino acid
<220>
<221> Variant
<222> (66)..(66)
<223> xaa in position 66 is Leu or Cys

<400> 171
Asp Phe Gly xaa Glu xaa xaa Pro xaa xaa xaa xaa xaa xaa xaa xaa
1               5                   10                  15
xaa xaa xaa xaa xaa xaa xaa xaa Tyr Trp Glu Asp xaa Gly xaa xaa
            20                  25                  30
xaa xaa Phe xaa xaa xaa xaa xaa xaa xaa xaa xaa xaa xaa xaa xaa
        35                  40                  45
xaa Phe xaa xaa xaa xaa xaa xaa xaa xaa xaa xaa Thr xaa xaa xaa
    50                  55                  60
xaa xaa Pro Pro
65

<210> 172
<211> 901
<212> DNA
<213> Hordeum vulgare

<220>
<221> CDS
<222> (108)..(572)

<400> 172
ccaacctccg tccgtgctct gctccccgtg cggaaggcgg ctggccggcg gcagctacgg      60

cggcgcgcga ccggacgacg tacacgcaga tctgctaaag gagcaac atg gca aca       116
                                                    Met Ala Thr
                                                    1
ctt aaa gat gtg gca aca cgg aaa cct att cta gca aca atc cgc ctc       164
Leu Lys Asp Val Ala Thr Arg Lys Pro Ile Leu Ala Thr Ile Arg Leu
    5                   10                  15
ctg gtt cct gct gga gca gcc cgc ccg gca cca cct gtc gga cca gct       212
Leu Val Pro Ala Gly Ala Ala Arg Pro Ala Pro Pro Val Gly Pro Ala
20                  25                  30                  35
ctg ggt ttc tac agg ctc aat ctg atg gca ttc tgc aag gat ttc aac       260
Leu Gly Phe Tyr Arg Leu Asn Leu Met Ala Phe Cys Lys Asp Phe Asn
                40                  45                  50
gcc agg acg cag aag tac aag gca gag act ccg atg caa gtc acg ctg       308
Ala Arg Thr Gln Lys Tyr Lys Ala Glu Thr Pro Met Gln Val Thr Leu
            55                  60                  65
acc gcc tac aag gac agc acc ttc gag ttt gtg gtc aag tcg cca tcg       356
Thr Ala Tyr Lys Asp Ser Thr Phe Glu Phe Val Val Lys Ser Pro Ser
        70                  75                  80
gtc tca tgg ttc ctc aag aaa gcg gcg ggc atc gag agc gcg agc agc       404
Val Ser Trp Phe Leu Lys Lys Ala Ala Gly Ile Glu Ser Ala Ser Ser
    85                  90                  95

cgt ccg ggc cac aac atg gtg acg tcc ctc tcg ctc cgc cat gtc tat 452
Arg Pro Gly His Asn Met Val Thr Ser Leu Ser Leu Arg His Val Tyr
100                 105                 110                 115
gag atc gca aag ctg aag cag tcc gac ccg ttc tgc aag cac atg tcc 500
Glu Ile Ala Lys Leu Lys Gln Ser Asp Pro Phe Cys Lys His Met Ser
                120                 125                 130
ctc gag gcc ctg tgc aag tcc atc atc ggc acg gcc aag tcc atg ggc 548
Leu Glu Ala Leu Cys Lys Ser Ile Ile Gly Thr Ala Lys Ser Met Gly
            135                 140                 145
att gaa atc gtg aag gat ctc tga ccggtctggc tggactagct ctcaggcata 602
Ile Glu Ile Val Lys Asp Leu
150 155
tttttttatc ttgtgttcat ctacagccag tctagtttcg tctgatttga aagaaaggta 662

gggatgggat ttatctgtcg aaatctggcg gcgcccgtct tggtgatcag tttgtatcat 722

gtgacagttt tcttcagtgt aaccatttgg tggtggtcgg gtagtaagtt tctgcctccc 782

tctactactc cccctgttcc aaaataaatg tcatggtttt agttcaaatt tgaagtattt 842

tgcactgaac ttgttgaaat ggcaacttga gcgccttcca tgccaaaaaa aaaaaaaaa 901

<210> 173
<211> 154
<212> PRT
<213> Hordeum vulgare

<400> 173
Met Ala Thr Leu Lys Asp Val Ala Thr Arg Lys Pro Ile Leu Ala Thr
1               5                   10                  15
Ile Arg Leu Leu Val Pro Ala Gly Ala Ala Arg Pro Ala Pro Pro Val
            20                  25                  30
Gly Pro Ala Leu Gly Phe Tyr Arg Leu Asn Leu Met Ala Phe Cys Lys
        35                  40                  45
Asp Phe Asn Ala Arg Thr Gln Lys Tyr Lys Ala Glu Thr Pro Met Gln
    50                  55                  60
Val Thr Leu Thr Ala Tyr Lys Asp Ser Thr Phe Glu Phe Val Val Lys
65                  70                  75                  80
Ser Pro Ser Val Ser Trp Phe Leu Lys Lys Ala Ala Gly Ile Glu Ser
                85                  90                  95
Ala Ser Ser Arg Pro Gly His Asn Met Val Thr Ser Leu Ser Leu Arg
            100                 105                 110
His Val Tyr Glu Ile Ala Lys Leu Lys Gln Ser Asp Pro Phe Cys Lys
        115                 120                 125
His Met Ser Leu Glu Ala Leu Cys Lys Ser Ile Ile Gly Thr Ala Lys
    130                 135                 140
Ser Met Gly Ile Glu Ile Val Lys Asp Leu
145                 150

<210> 174
<211> 22
<212> DNA
<213> Artificial sequence

<400> 174
atggcaacac ttaaagatgt gg 22

<210> 175
<211> 21
<212> DNA
<213> Artificial sequence

<400> 175

tcagagatcc ttcacgattt c                                                    21

<210> 176
<211> 167
<212> PRT
<213> Artificial sequence

<220>
<221> Variant
<222> (2)..(4)
<223> Xaa in position 2 to 4 is any amino acid
<220>
<221> Variant
<222> (8)..(8)
<223> Xaa in position 8 is any amino acid
<220>
<221> Variant
<222> (10)..(10)
<223> Xaa in position 10 is any amino acid
<220>
<221> Variant
<222> (13)..(13)
<223> Xaa in position 13 is any amino acid
<220>
<221> Variant
<222> (15)..(15)
<223> Xaa in position 15 is any amino acid
<220>
<221> Variant
<222> (17)..(17)
<223> Xaa in position 17 is any or no amino acid
<220>
<221> Variant
<222> (18)..(19)
<223> Xaa in position 18 to 19 is Pro or no amino acid
<220>
<221> Variant
<222> (20)..(20)
<223> Xaa in position 20 is Val or no amino acid
<220>
<221> Variant
<222> (26)..(27)
<223> Xaa in position 26 to 27 is any amino acid
<220>
<221> Variant
<222> (32)..(33)
<223> Xaa in position 32 to 33 is any amino acid
<220>
<221> Variant
<222> (35)..(35)
<223> Xaa in position 35 is any amino acid
<220>
<221> Variant
<222> (37)..(37)
<223> Xaa in position 37 is any amino acid
<220>
<221> Variant
<222> (40)..(41)
<223> Xaa in position 40 to 41 is any amino acid
<220>
<221> Variant
<222> (43)..(46)
<223> Xaa in position 43 to 46 is any amino acid
<220>
<221> Variant
<222> (47)..(51)
<223> Xaa in position 47 to 51 is any or no amino acid
<220>

<221> Variant
<222> (53)..(54)
<223> xaa in position 53 to 54 is any amino acid
<220>
<221> Variant
<222> (58)..(58)
<223> xaa in position 58 is any amino acid
<220>
<221> Variant
<222> (61)..(63)
<223> xaa in position 61 to 63 is any amino acid
<220>
<221> Variant
<222> (65)..(66)
<223> xaa in position 65 to 66 is any or no amino acid
<220>
<221> Variant
<222> (70)..(70)
<223> xaa in position 70 is any amino acid
<220>
<221> Variant
<222> (72)..(73)
<223> xaa in position 72 to 73 is any amino acid
<220>
<221> Variant
<222> (75)..(75)
<223> xaa in position 75 is any amino acid
<220>
<221> Variant
<222> (78)..(80)
<223> xaa in position 78 to 80 is any amino acid
<220>
<221> Variant
<222> (83)..(83)
<223> xaa in position 83 is any amino acid
<220>
<221> Variant
<222> (85)..(87)
<223> xaa in position 85 to 87 is any amino acid
<220>
<221> Variant
<222> (89)..(90)
<223> xaa in position 89 to 90 is any amino acid
<220>
<221> Variant
<222> (91)..(98)
<223> xaa in position 91 to 98 is any or no amino acid
<220>
<221> Variant
<222> (101)..(102)
<223> xaa in position 101 to 102 is any amino acid
<220>
<221> Variant
<222> (104)..(107)
<223> xaa in position 104 to 107 is any amino acid
<220>
<221> Variant
<222> (108)..(120)
<223> xaa in position 108 to 120 is any or no amino acid
<220>
<221> Variant
<222> (123)..(123)
<223> xaa in position 123 is any amino acid
<220>
<221> Variant
<222> (126)..(128)
<223> xaa in position 126 to 128 is any amino acid
<220>
<221> Variant

<222> (130)..(131)
<223> xaa in position 130 to 131 is any amino acid
<220>
<221> Variant
<222> (134)..(135)
<223> xaa in position 134 to 135 is any amino acid
<220>
<221> Variant
<222> (137)..(138)
<223> xaa in position 137 to 138 is any amino acid
<220>
<221> Variant
<222> (140)..(143)
<223> xaa in position 140 to 143 is any or no amino acid
<220>
<221> Variant
<222> (145)..(148)
<223> xaa in position 145 to 148 is any amino acid
<220>
<221> Variant
<222> (150)..(153)
<223> xaa in position 150 to 153 is any amino acid
<220>
<221> Variant
<222> (155)..(155)
<223> xaa in position 155 is any amino acid
<220>
<221> Variant
<222> (157)..(157)
<223> xaa in position 157 is any amino acid
<220>
<221> Variant
<222> (166)..(166)
<223> xaa in position 166 is any amino acid

<400> 176
Val xaa xaa xaa Ile Lys Leu xaa Val xaa Ala Gly xaa Ala xaa Pro
1               5                   10                  15
xaa xaa xaa xaa Gly Pro Ala Leu Gly xaa xaa Gly Val Asn Ile xaa
            20                  25                  30
xaa Phe xaa Lys xaa Phe Asn xaa xaa Thr xaa xaa xaa xaa xaa xaa
        35                  40                  45
xaa xaa xaa Gly xaa xaa Ile Pro Val xaa Ile Thr xaa xaa xaa Asp
    50                  55                  60
xaa xaa Arg Ser Phe xaa Phe xaa xaa Lys xaa Pro Pro xaa xaa xaa
65                  70                  75                  80
Leu Leu xaa Lys xaa xaa xaa Ile xaa xaa xaa xaa xaa xaa xaa xaa
                85                  90                  95
xaa xaa Gly Ser xaa xaa Pro xaa xaa xaa xaa xaa xaa xaa xaa xaa
            100                 105                 110
xaa xaa xaa xaa xaa xaa xaa xaa Val Gly xaa Ile Thr xaa xaa xaa
        115                 120                 125
Val xaa xaa Ile Ala xaa xaa Lys xaa xaa Asp xaa xaa xaa xaa Leu
    130                 135                 140
xaa xaa xaa xaa Leu xaa xaa xaa xaa Arg xaa Ile xaa Gly Thr Ala
145                 150                 155                 160
Arg Ser Met Gly Ile xaa Val
                165


<210> 177
<211> 38
<212> PRT
<213> Artificial sequence

<220>
<221> Variant
<222> (1)..(1)
<223> xaa in position 1 is Ile, Leu, Val, Met, Ala or Phe

<220>
<221> Variant
<222> (2)..(2)
<223> Xaa in position 2 is Lys, Arg, Glu or Asn
<220>
<221> Variant
<222> (3)..(3)
<223> Xaa in position 3 is Leu, Ile, Val, Phe, Ala or Thr
<220>
<221> Variant
<222> (4)..(4)
<223> Xaa in position 4 is any amino acid
<220>
<221> Variant
<222> (5)..(5)
<223> Xaa in position 5 is Val, Ile, Leu, Phe or Cys
<220>
<221> Variant
<222> (6)..(6)
<223> Xaa in position 6 is any amino acid
<220>
<221> Variant
<222> (7)..(7)
<223> Xaa in position 7 is Ala, Gly or Ser
<220>
<221> Variant
<222> (8)..(8)
<223> Xaa in position 8 is Gly, Ala, Asn, Gln or Thr
<220>
<221> Variant
<222> (9)..(9)
<223> Xaa in position 9 is any amino acid
<220>
<221> Variant
<222> (11)..(11)
<223> Xaa in position 11 is any amino acid
<220>
<221> Variant
<222> (12)..(12)
<223> Xaa in position 12 is Pro, Thr, Ala or Ser
<220>
<221> Variant
<222> (13)..(13)
<223> Xaa in position 13 is Ala, Ser, Gly, Thr or no amino acid
<220>
<221> Variant
<222> (14)..(14)
<223> Xaa in position 14 is Pro or no amino acid
<220>
<221> Variant
<222> (15)..(15)
<223> Xaa in position 15 is Pro, Lys or no amino acid
<220>
<221> Variant
<222> (16)..(16)
<223> Xaa in position 16 is Val, Ile, Leu or no amino acid
<220>
<221> Variant
<222> (17)..(17)
<223> Xaa in position 17 is Gly or Ala
<220>
<221> Variant
<222> (18)..(18)
<223> Xaa in position 18 is Pro, Ser, Gln or Thr
<220>
<221> Variant
<222> (19)..(19)
<223> Xaa in position 19 is any amino acid
<220>

<221> Variant
<222> (20)..(20)
<223> Xaa in position 20 is Leu, Ile or Phe
<220>
<221> Variant
<222> (21)..(21)
<223> Xaa in position 21 is Gly, Ala or Ser
<220>
<221> Variant
<222> (22)..(23)
<223> Xaa in position 22 to 23 is any amino acid
<220>
<221> Variant
<222> (24)..(24)
<223> Xaa in position 24 is Gly, Pro, Gln, Lys, Arg or Asn
<220>
<221> Variant
<222> (25)..(25)
<223> Xaa in position 25 is Val, Ile, Leu or Ala
<220>
<221> Variant
<222> (26)..(26)
<223> Xaa in position 26 is Asn, Lys, Pro, Ser or Asp
<220>
<221> Variant
<222> (27)..(27)
<223> Xaa in position 27 is Ile, Val, Leu, Met, Ala or Ser
<220>
<221> Variant
<222> (28)..(29)
<223> Xaa in position 28 to 29 is any amino acid
<220>
<221> Variant
<222> (30)..(30)
<223> Xaa in position 30 is Phe or Val
<220>
<221> Variant
<222> (31)..(31)
<223> Xaa in position 31 is Cys, Val, Thr, Phe or Ile
<220>
<221> Variant
<222> (32)..(33)
<223> Xaa in position 32 to 33 is any amino acid
<220>
<221> Variant
<222> (34)..(34)
<223> Xaa in position 34 is Phe, Tyr or Ile
<220>
<221> Variant
<222> (36)..(37)
<223> Xaa in position 36 to 37 is any amino acid
<220>
<221> Variant
<222> (38)..(38)
<223> Xaa in position 38 is Thr or Ser

<400> 177
Xaa Xaa Xaa Xaa Xaa Xaa Xaa Xaa Xaa Ala Xaa Xaa Xaa Xaa Xaa Xaa
1               5                   10                  15
Xaa Xaa Xaa Xaa Xaa Xaa Xaa Xaa Xaa Xaa Xaa Xaa Xaa Xaa Xaa Xaa
            20                  25                  30
Xaa Xaa Asn Xaa Xaa Xaa
        35


<210> 178
<211> 39
<212> PRT
<213> Artificial sequence

<220>
<221> Variant
<222> (1)..(1)
<223> xaa in position 1 is Ile, Val, Leu, Thr, Met or Cys
<220>
<221> Variant
<222> (2)..(2)
<223> xaa in position 2 is Pro, Thr, Gln or Ala
<220>
<221> Variant
<222> (3)..(3)
<223> xaa in position 3 is Val, Thr, Cys or Ala
<220>
<221> Variant
<222> (4)..(4)
<223> xaa in position 4 is any amino acid
<220>
<221> Variant
<222> (5)..(5)
<223> xaa in position 5 is Ile, Val or Leu
<220>
<221> Variant
<222> (6)..(9)
<223> xaa in position 6 to 9 is any amino acid
<220>
<221> Variant
<222> (10)..(10)
<223> xaa in position 10 is Asp, Glu, Pro, Lys or Asn
<220>
<221> Variant
<222> (11)..(11)
<223> xaa in position 11 is Glu, Ser, Ala or no amino acid
<220>
<221> Variant
<222> (12)..(12)
<223> xaa in position 12 is Thr or no amino acid
<220>
<221> Variant
<222> (13)..(13)
<223> xaa in position 13 is Arg, Lys, Asn or Ser
<220>
<221> Variant
<222> (14)..(14)
<223> xaa in position 14 is Ser, Thr, Ala, Asn or Lys
<220>
<221> Variant
<222> (15)..(15)
<223> xaa in position 15 is Phe, Tyr, Val or Ile
<220>
<221> Variant
<222> (16)..(16)
<223> xaa in position 16 is any amino acid
<220>
<221> Variant
<222> (17)..(17)
<223> xaa in position 17 is Phe, Ile, Leu, Met, Tyr or Val
<220>
<221> Variant
<222> (18)..(18)
<223> xaa in position 18 is any amino acid
<220>
<221> Variant
<222> (19)..(19)
<223> xaa in position 19 is Thr, Leu, Val, Met, Ile or Ser
<220>
<221> Variant
<222> (20)..(21)
<223> xaa in position 20 to 21 is any amino acid

<220>
<221> Variant
<222> (22)..(22)
<223> Xaa in position 22 is Pro, Thr or Ser
<220>
<221> Variant
<222> (23)..(23)
<223> Xaa in position 23 is Pro, Thr, Ser, Gln or Lys
<220>
<221> Variant
<222> (24)..(24)
<223> Xaa in position 24 is Ala, Thr, Val, Met, Ile, Leu or Ser
<220>
<221> Variant
<222> (25)..(27)
<223> Xaa in position 25 to 27 is any amino acid
<220>
<221> Variant
<222> (28)..(28)
<223> Xaa in position 28 is Leu, Ile or Val
<220>
<221> Variant
<222> (29)..(33)
<223> Xaa in position 29 to 33 is any amino acid
<220>
<221> Variant
<222> (34)..(34)
<223> Xaa in position 34 is Ile, Leu, Val, Ala or Phe
<220>
<221> Variant
<222> (35)..(36)
<223> Xaa in position 35 to 36 is any amino acid
<220>
<221> Variant
<222> (37)..(37)
<223> Xaa in position 37 is any or no amino acid
<220>
<221> Variant
<222> (38)..(38)
<223> Xaa in position 38 is Gly or Ala
<220>
<221> Variant
<222> (39)..(39)
<223> Xaa in position 39 is Ser, Ala, Thr or His

<400> 178
Xaa Xaa Xaa Xaa Xaa Xaa Xaa Xaa Xaa Xaa Xaa Xaa Xaa Xaa Xaa Xaa
1               5                   10                  15
Xaa Xaa Xaa Xaa Xaa Xaa Xaa Xaa Xaa Xaa Xaa Xaa Xaa Xaa Xaa Xaa
            20                  25                  30
Xaa Xaa Xaa Xaa Xaa Xaa Xaa
        35


<210> 179
<211> 46
<212> PRT
<213> Artificial sequence

<220>
<221> Variant
<222> (1)..(1)
<223> Xaa in position 1 is Gly, Ala, Ser or Thr
<220>
<221> Variant
<222> (2)..(2)
<223> Xaa in position 2 is any amino acid
<220>
<221> Variant

<222> (3)..(3)
<223> Xaa in position 3 is Ile, Val, Leu, Ala or Met
<220>
<221> Variant
<222> (4)..(7)
<223> Xaa in position 4 to 7 is any amino acid
<220>
<221> Variant
<222> (8)..(8)
<223> Xaa in position 8 is Val, Leu, Ile, Ala or Cys
<220>
<221> Variant
<222> (9)..(10)
<223> Xaa in position 9 to 10 is any amino acid
<220>
<221> Variant
<222> (11)..(11)
<223> Xaa in position 11 is Ile, Val or Leu
<220>
<221> Variant
<222> (12)..(12)
<223> Xaa in position 12 is Ala or Val
<220>
<221> Variant
<222> (13)..(14)
<223> Xaa in position 13 to 14 is any amino acid
<220>
<221> Variant
<222> (16)..(17)
<223> Xaa in position 16 to 17 is any amino acid
<220>
<221> Variant
<222> (18)..(18)
<223> Xaa in position 18 is Asp, Gln, Ala or Ser
<220>
<221> Variant
<222> (19)..(22)
<223> Xaa in position 19 to 22 is any or no amino acid
<220>
<221> Variant
<222> (23)..(23)
<223> Xaa in position 23 is Leu, Met, Val, Ile, His or Cys
<220>
<221> Variant
<222> (24)..(27)
<223> Xaa in position 24 to 27 is any amino acid
<220>
<221> Variant
<222> (28)..(28)
<223> Xaa in position 28 is Leu, Ile, Val, Met or Glu
<220>
<221> Variant
<222> (29)..(34)
<223> Xaa in position 29 to 34 is any amino acid
<220>
<221> Variant
<222> (35)..(35)
<223> Xaa in position 35 is Ile, Val, Leu or Met
<220>
<221> Variant
<222> (36)..(36)
<223> Xaa in position 36 is any amino acid
<220>
<221> Variant
<222> (37)..(37)
<223> Xaa in position 37 is Gly or Ala
<220>
<221> Variant
<222> (38)..(38)

<223> xaa in position 38 is Thr, Ser, Gln or Val
<220>
<221> Variant
<222> (39)..(39)
<223> xaa in position 39 is Ala, Cys, Leu or Val
<220>
<221> Variant
<222> (40)..(40)
<223> xaa in position 40 is any amino acid
<220>
<221> Variant
<222> (41)..(41)
<223> xaa in position 41 is Ser, Asn, Ala, Pro, Gln, Thr or Arg
<220>
<221> Variant
<222> (42)..(42)
<223> xaa in position 42 is Met, Ile, Leu, Val or Cys
<220>
<221> Variant
<222> (44)..(44)
<223> xaa in position 44 is Ile, Leu, Val, Phe or Tyr
<220>
<221> Variant
<222> (45)..(45)
<223> xaa in position 45 is any amino acid
<220>
<221> Variant
<222> (46)..(46)
<223> xaa in position 46 is Val, Ile or Thr

<400> 179
xaa xaa xaa xaa xaa xaa xaa xaa xaa xaa xaa xaa xaa xaa Lys xaa
1               5                   10                  15
xaa xaa xaa xaa xaa xaa xaa xaa xaa xaa xaa xaa xaa xaa xaa xaa
            20                  25                  30
xaa xaa xaa xaa xaa xaa xaa xaa xaa xaa Gly xaa xaa xaa
        35                  40                  45

<210> 180
<211> 1029
<212> DNA
<213> Glycine max

<220>
<221> CDS
<222> (1)..(1029)

<400> 180
atg gac cac agc gct gat gca cat cgc acg gac ttg atg acc ata acg      48
Met Asp His Ser Ala Asp Ala His Arg Thr Asp Leu Met Thr Ile Thr
1               5                   10                  15
cgg ttc gtg ctg aac gag caa tcc aag cac ccc gag tca cgc ggc gat      96
Arg Phe Val Leu Asn Glu Gln Ser Lys His Pro Glu Ser Arg Gly Asp
            20                  25                  30
ttc acc atc ttg ctc agt cac att gtt ctc ggt tgc aag ttc gtt tgt      144
Phe Thr Ile Leu Leu Ser His Ile Val Leu Gly Cys Lys Phe Val Cys
        35                  40                  45
tcc gct gtc agc aag gct ggt ctt gct aaa ctt att gga ctc gct gga      192
Ser Ala Val Ser Lys Ala Gly Leu Ala Lys Leu Ile Gly Leu Ala Gly
    50                  55                  60
gaa acc aat gtt cag ggt gaa gaa cag aag aaa ctg gat gtt ctt tcc      240
Glu Thr Asn Val Gln Gly Glu Glu Gln Lys Lys Leu Asp Val Leu Ser
65                  70                  75                  80
aat gag gtt ttc atc aag gct ctc ata agc agt ggc cga aca tgc atc      288
Asn Glu Val Phe Ile Lys Ala Leu Ile Ser Ser Gly Arg Thr Cys Ile
                85                  90                  95
ttg gtt tct gaa gaa gat gaa gaa gcg ata att gtg gag cct tct aag      336
Leu Val Ser Glu Glu Asp Glu Glu Ala Ile Ile Val Glu Pro Ser Lys
            100                 105                 110

cgc gga aag tac tgt gtt tgc ttt gac cca ttg gat ggc tcg tcc aac          384
Arg Gly Lys Tyr Cys Val Cys Phe Asp Pro Leu Asp Gly Ser Ser Asn
        115                 120                 125
att gat tgt ggg gtt tcc att ggc aca atc ttt ggg gtt tat gtg ttg          432
Ile Asp Cys Gly Val Ser Ile Gly Thr Ile Phe Gly Val Tyr Val Leu
    130                 135                 140
aaa gat gtc cat gaa cca acc ata gaa gat gtc ctg cta cct ggg aag          480
Lys Asp Val His Glu Pro Thr Ile Glu Asp Val Leu Leu Pro Gly Lys
145                 150                 155                 160
aac atg gtg gca gct ggt tac tgt atg tat gga agc tct tgc acg ctt          528
Asn Met Val Ala Ala Gly Tyr Cys Met Tyr Gly Ser Ser Cys Thr Leu
                165                 170                 175
gtg tta agc act gga gca ggt gtt aat ggt ttc acc ctt gac cca tct          576
Val Leu Ser Thr Gly Ala Gly Val Asn Gly Phe Thr Leu Asp Pro Ser
            180                 185                 190
ctt ggt gag ttc atc cta act cac cct aac att aag atc cca aag aaa          624
Leu Gly Glu Phe Ile Leu Thr His Pro Asn Ile Lys Ile Pro Lys Lys
        195                 200                 205
ggt aaa att tat tca gtg aat gaa gga aat gcc aaa aac tgg gat cgt          672
Gly Lys Ile Tyr Ser Val Asn Glu Gly Asn Ala Lys Asn Trp Asp Arg
    210                 215                 220
cct act gct act tac gtt gaa aaa tgc aag ttt cct gaa gat ggt tca          720
Pro Thr Ala Thr Tyr Val Glu Lys Cys Lys Phe Pro Glu Asp Gly Ser
225                 230                 235                 240
tca cca aag tct cta aga tat att gga agc atg gta gct gat gtt cat          768
Ser Pro Lys Ser Leu Arg Tyr Ile Gly Ser Met Val Ala Asp Val His
                245                 250                 255
cgt acg ttg ctt tat gga ggc atc ttt ttg tac cct gct gac aaa aaa          816
Arg Thr Leu Leu Tyr Gly Gly Ile Phe Leu Tyr Pro Ala Asp Lys Lys
            260                 265                 270
agt cca aat gga aaa ctt cgt gtc ctg tat gaa gtc ttc cca atg tca          864
Ser Pro Asn Gly Lys Leu Arg Val Leu Tyr Glu Val Phe Pro Met Ser
        275                 280                 285
ttc ttg atg gaa cag gca gga gga cag tct ttc acg ggc aag gaa cgg          912
Phe Leu Met Glu Gln Ala Gly Gly Gln Ser Phe Thr Gly Lys Glu Arg
    290                 295                 300
gca ctt gat tta gtt cca acg aag ttg cac gag cga tct ccc ata ttc          960
Ala Leu Asp Leu Val Pro Thr Lys Leu His Glu Arg Ser Pro Ile Phe
305                 310                 315                 320
ctt ggt agc tat gat gat gtt gag gaa atc aaa gct ctt tat gct gct          1008
Leu Gly Ser Tyr Asp Asp Val Glu Glu Ile Lys Ala Leu Tyr Ala Ala
                325                 330                 335
gct gag ggt gaa caa gaa tga                                              1029
Ala Glu Gly Glu Gln Glu
            340

<210> 181
<211> 342
<212> PRT
<213> Glycine max

<400> 181
Met Asp His Ser Ala Asp Ala His Arg Thr Asp Leu Met Thr Ile Thr
1               5                   10                  15
Arg Phe Val Leu Asn Glu Gln Ser Lys His Pro Glu Ser Arg Gly Asp
            20                  25                  30
Phe Thr Ile Leu Leu Ser His Ile Val Leu Gly Cys Lys Phe Val Cys
        35                  40                  45
Ser Ala Val Ser Lys Ala Gly Leu Ala Lys Leu Ile Gly Leu Ala Gly
    50                  55                  60
Glu Thr Asn Val Gln Gly Glu Glu Gln Lys Lys Leu Asp Val Leu Ser
65                  70                  75                  80
Asn Glu Val Phe Ile Lys Ala Leu Ile Ser Ser Gly Arg Thr Cys Ile
                85                  90                  95
Leu Val Ser Glu Glu Asp Glu Glu Ala Ile Ile Val Glu Pro Ser Lys
            100                 105                 110
Arg Gly Lys Tyr Cys Val Cys Phe Asp Pro Leu Asp Gly Ser Ser Asn
        115                 120                 125
Ile Asp Cys Gly Val Ser Ile Gly Thr Ile Phe Gly Val Tyr Val Leu

130                 135                 140
Lys Asp Val His Glu Pro Thr Ile Glu Asp Val Leu Leu Pro Gly Lys
145                 150                 155                 160
Asn Met Val Ala Ala Gly Tyr Cys Met Tyr Gly Ser Ser Cys Thr Leu
                165                 170                 175
Val Leu Ser Thr Gly Ala Gly Val Asn Gly Phe Thr Leu Asp Pro Ser
            180                 185                 190
Leu Gly Glu Phe Ile Leu Thr His Pro Asn Ile Lys Ile Pro Lys Lys
        195                 200                 205
Gly Lys Ile Tyr Ser Val Asn Glu Gly Asn Ala Lys Asn Trp Asp Arg
    210                 215                 220
Pro Thr Ala Thr Tyr Val Glu Lys Cys Lys Phe Pro Glu Asp Gly Ser
225                 230                 235                 240
Ser Pro Lys Ser Leu Arg Tyr Ile Gly Ser Met Val Ala Asp Val His
                245                 250                 255
Arg Thr Leu Leu Tyr Gly Gly Ile Phe Leu Tyr Pro Ala Asp Lys Lys
            260                 265                 270
Ser Pro Asn Gly Lys Leu Arg Val Leu Tyr Glu Val Phe Pro Met Ser
        275                 280                 285
Phe Leu Met Glu Gln Ala Gly Gly Gln Ser Phe Thr Gly Lys Glu Arg
    290                 295                 300
Ala Leu Asp Leu Val Pro Thr Lys Leu His Glu Arg Ser Pro Ile Phe
305                 310                 315                 320
Leu Gly Ser Tyr Asp Asp Val Glu Glu Ile Lys Ala Leu Tyr Ala Ala
                325                 330                 335
Ala Glu Gly Glu Gln Glu
            340

<210> 182
<211> 20
<212> DNA
<213> Artificial sequence

<400> 182
atggaccaca gcgctgatgc                                                   20

<210> 183
<211> 21
<212> DNA
<213> Artificial sequence

<400> 183
tcattcttgt tcaccctcag c                                                 21


<210> 184
<211> 334
<212> PRT
<213> Artificial sequence

<220>
<221> Variant
<222> (2)..(13)
<223> xaa in position 2 to 13 is any amino acid
<220>
<221> Variant
<222> (14)..(23)
<223> xaa in position 14 to 23 is any or no amino acid
<220>
<221> Variant
<222> (25)..(26)
<223> xaa in position 25 to 26 is any amino acid
<220>
<221> Variant
<222> (28)..(29)
<223> xaa in position 28 to 29 is any amino acid
<220>
<221> Variant
<222> (31)..(31)

<223> xaa in position 31 is any amino acid
<220>
<221> Variant
<222> (32)..(38)
<223> xaa in position 32 to 38 is any or no amino acid
<220>
<221> Variant
<222> (40)..(43)
<223> xaa in position 40 to 43 is any amino acid
<220>
<221> Variant
<222> (47)..(67)
<223> xaa in position 47 to 67 is any amino acid
<220>
<221> Variant
<222> (71)..(78)
<223> xaa in position 71 to 78 is any amino acid
<220>
<221> Variant
<222> (79)..(90)
<223> xaa in position 79 to 90 is any or no amino acid
<220>
<221> Variant
<222> (92)..(92)
<223> xaa in position 92 is any amino acid
<220>
<221> Variant
<222> (94)..(97)
<223> xaa in position 94 to 97 is any amino acid
<220>
<221> Variant
<222> (106)..(106)
<223> xaa in position 106 is any amino acid
<220>
<221> Variant
<222> (108)..(112)
<223> xaa in position 108 to 112 is any amino acid
<220>
<221> Variant
<222> (114)..(114)
<223> xaa in position 114 is any amino acid
<220>
<221> Variant
<222> (117)..(124)
<223> xaa in position 117 to 124 is any amino acid
<220>
<221> Variant
<222> (125)..(152)
<223> xaa in position 125 to 152 is any or no amino acid
<220>
<221> Variant
<222> (154)..(157)
<223> xaa in position 154 to 157 is any amino acid
<220>
<221> Variant
<222> (162)..(163)
<223> xaa in position 162 to 163 is any amino acid
<220>
<221> Variant
<222> (166)..(170)
<223> xaa in position 166 to 170 is any amino acid
<220>
<221> Variant
<222> (172)..(174)
<223> xaa in position 172 to 174 is any amino acid
<220>
<221> Variant
<222> (176)..(176)
<223> xaa in position 176 is any amino acid

<220>
<221> Variant
<222> (178)..(178)
<223> xaa in position 178 is any or no amino acid
<220>
<221> Variant
<222> (180)..(181)
<223> xaa in position 180 to 181 is any amino acid
<220>
<221> Variant
<222> (183)..(183)
<223> xaa in position 183 is any amino acid
<220>
<221> Variant
<222> (186)..(186)
<223> xaa in position 186 is any amino acid
<220>
<221> Variant
<222> (187)..(192)
<223> xaa in position 187 to 192 is any or no amino acid
<220>
<221> Variant
<222> (194)..(194)
<223> xaa in position 194 is any amino acid
<220>
<221> Variant
<222> (196)..(196)
<223> xaa in position 196 is any amino acid
<220>
<221> Variant
<222> (198)..(210)
<223> xaa in position 198 to 210 is any amino acid
<220>
<221> Variant
<222> (211)..(214)
<223> xaa in position 211 to 214 is any or no amino acid
<220>
<221> Variant
<222> (216)..(239)
<223> xaa in position 216 to 239 is any amino acid
<220>
<221> Variant
<222> (240)..(247)
<223> xaa in position 240 to 247 is any or no amino acid
<220>
<221> Variant
<222> (249)..(251)
<223> xaa in position 249 to 251 is any amino acid
<220>
<221> Variant
<222> (253)..(253)
<223> xaa in position 253 is any amino acid
<220>
<221> Variant
<222> (255)..(255)
<223> xaa in position 255 is any amino acid
<220>
<221> Variant
<222> (257)..(257)
<223> xaa in position 257 is any amino acid
<220>
<221> Variant
<222> (260)..(260)
<223> xaa in position 260 is any amino acid
<220>
<221> Variant
<222> (262)..(263)
<223> xaa in position 262 to 263 is any amino acid
<220>

<221> Variant
<222> (265)..(265)
<223> xaa in position 265 is any or no amino acid
<220>
<221> Variant
<222> (267)..(269)
<223> xaa in position 267 to 269 is any amino acid
<220>
<221> Variant
<222> (272)..(272)
<223> xaa in position 272 is any amino acid
<220>
<221> Variant
<222> (273)..(281)
<223> xaa in position 273 to 281 is any or no amino acid
<220>
<221> Variant
<222> (286)..(287)
<223> xaa in position 286 to 287 is any amino acid
<220>
<221> Variant
<222> (290)..(291)
<223> xaa in position 290 to 291 is any amino acid
<220>
<221> Variant
<222> (293)..(297)
<223> xaa in position 293 to 297 is any amino acid
<220>
<221> Variant
<222> (303)..(306)
<223> xaa in position 303 to 306 is any amino acid
<220>
<221> Variant
<222> (308)..(311)
<223> xaa in position 308 to 311 is any amino acid
<220>
<221> Variant
<222> (312)..(312)
<223> xaa in position 312 is any or no amino acid
<220>
<221> Variant
<222> (314)..(316)
<223> xaa in position 314 to 316 is any amino acid
<220>
<221> Variant
<222> (318)..(320)
<223> xaa in position 318 to 320 is any amino acid
<220>
<221> Variant
<222> (322)..(322)
<223> xaa in position 322 is any amino acid
<220>
<221> Variant
<222> (324)..(328)
<223> xaa in position 324 to 328 is any amino acid
<220>
<221> Variant
<222> (331)..(333)
<223> xaa in position 331 to 333 is any amino acid

<400> 184
Lys xaa xaa xaa xaa xaa xaa xaa xaa xaa xaa xaa xaa xaa xaa xaa
1               5                   10                  15
xaa xaa xaa xaa xaa xaa xaa Gly xaa xaa Gly xaa xaa Asn xaa xaa
            20                  25                  30
xaa xaa xaa xaa xaa xaa Gly xaa xaa xaa xaa Lys Leu Asp xaa xaa
        35                  40                  45
xaa xaa xaa xaa xaa xaa xaa xaa xaa xaa xaa xaa xaa xaa xaa xaa
    50                  55                  60

xaa xaa xaa Ser Glu Glu xaa xaa xaa xaa xaa xaa xaa xaa xaa xaa
65                  70                  75                  80
xaa xaa xaa xaa xaa xaa xaa xaa xaa xaa Gly xaa Tyr xaa xaa xaa
                85                  90                  95
xaa Asp Pro Leu Asp Gly Ser Ser Asn xaa Asp xaa xaa xaa xaa xaa
            100                 105                 110
Gly xaa Ile Phe xaa xaa xaa xaa xaa xaa xaa xaa xaa xaa xaa xaa
        115                 120                 125
xaa xaa xaa xaa xaa xaa xaa xaa xaa xaa xaa xaa xaa xaa xaa xaa
    130                 135                 140
xaa xaa xaa xaa xaa xaa xaa xaa Gly xaa xaa xaa xaa Ala Ala Gly
145                 150                 155                 160
Tyr xaa xaa Tyr Gly xaa xaa xaa xaa xaa Val xaa xaa xaa Gly xaa
                165                 170                 175
Gly xaa Val xaa xaa Phe xaa Leu Asp xaa xaa xaa xaa xaa xaa xaa
            180                 185                 190
Gly xaa Phe xaa Leu xaa xaa xaa xaa xaa xaa xaa xaa xaa xaa xaa
        195                 200                 205
xaa xaa xaa xaa xaa xaa Asn xaa xaa xaa xaa xaa xaa xaa xaa xaa
    210                 215                 220
xaa xaa xaa xaa xaa xaa xaa xaa xaa xaa xaa xaa xaa xaa xaa xaa
225                 230                 235                 240
xaa xaa xaa xaa xaa xaa xaa Arg xaa xaa xaa Ser xaa Val xaa Asp
                245                 250                 255
xaa His Arg xaa Leu xaa xaa Gly xaa Gly xaa xaa xaa Tyr Pro xaa
            260                 265                 270
xaa xaa xaa xaa xaa xaa xaa xaa xaa Gly Lys Leu Arg xaa xaa Tyr
        275                 280                 285
Glu xaa xaa Pro xaa xaa xaa xaa xaa Glu Gln Ala Gly Gly xaa xaa
    290                 295                 300
xaa xaa Gly xaa xaa xaa xaa xaa Leu xaa xaa xaa Pro xaa xaa xaa
305                 310                 315                 320
His xaa Arg xaa xaa xaa xaa xaa Gly Ser xaa xaa xaa Val
                325                 330


<210> 185
<211> 80
<212> PRT
<213> Artificial sequence

<220>
<221> Variant
<222> (1)..(1)
<223> xaa in position 1 is Gly or Arg
<220>
<221> Variant
<222> (2)..(2)
<223> xaa in position 2 is Glu or Asp
<220>
<221> Variant
<222> (3)..(3)
<223> xaa in position 3 is any amino acid
<220>
<221> Variant
<222> (4)..(4)
<223> xaa in position 4 is Gln, Val or Pro
<220>
<221> Variant
<222> (5)..(5)
<223> xaa in position 5 is Lys, Gln, Met, Leu, Ala or Arg
<220>
<221> Variant
<222> (6)..(6)
<223> xaa in position 6 is any amino acid
<220>
<221> Variant
<222> (7)..(7)
<223> xaa in position 7 is Leu, Met, Ala or Ile

<220>
<221> Variant
<222> (9)..(9)
<223> Xaa in position 9 is Val, Leu, Ile or Glu
<220>
<221> Variant
<222> (10)..(10)
<223> Xaa in position 10 is any amino acid
<220>
<221> Variant
<222> (11)..(11)
<223> Xaa in position 11 is Ser, Ala, Gly, Thr or Cys
<220>
<221> Variant
<222> (12)..(12)
<223> Xaa in position 12 is Asn, Asp or His
<220>
<221> Variant
<222> (13)..(14)
<223> Xaa in position 13 to 14 is any amino acid
<220>
<221> Variant
<222> (15)..(15)
<223> Xaa in position 15 is Phe, Leu, Val, Ile or Met
<220>
<221> Variant
<222> (16)..(26)
<223> Xaa in position 16 to 26 is any amino acid
<220>
<221> Variant
<222> (27)..(27)
<223> Xaa in position 27 is any or no amino acid
<220>
<221> Variant
<222> (28)..(28)
<223> Xaa in position 28 is Leu, Ile, Met, Val, Phe, Ala or Tyr
<220>
<221> Variant
<222> (29)..(29)
<223> Xaa in position 29 is Ala, Val, Ser, Gly or Cys
<220>
<221> Variant
<222> (30)..(30)
<223> Xaa in position 30 is Ser, Glu or Thr
<220>
<221> Variant
<222> (32)..(32)
<223> Xaa in position 32 is Glu or Met
<220>
<221> Variant
<222> (33)..(33)
<223> Xaa in position 33 is any amino acid
<220>
<221> Variant
<222> (34)..(34)
<223> Xaa in position 34 is Asp, Glu, Ala or Lys
<220>
<221> Variant
<222> (35)..(41)
<223> Xaa in position 35 to 41 is any amino acid
<220>
<221> Variant
<222> (42)..(52)
<223> Xaa in position 42 to 52 is any or no amino acid
<220>
<221> Variant
<222> (53)..(53)
<223> Xaa in position 53 is Gly or Ala
<220>

<221> Variant
<222> (54)..(54)
<223> Xaa in position 54 is any amino acid
<220>
<221> Variant
<222> (55)..(55)
<223> Xaa in position 55 is Tyr, Leu, Phe, Ile or Val
<220>
<221> Variant
<222> (56)..(56)
<223> Xaa in position 56 is any amino acid
<220>
<221> Variant
<222> (57)..(57)
<223> Xaa in position 57 is Val, Leu or Ile
<220>
<221> Variant
<222> (58)..(59)
<223> Xaa in position 58 to 59 is any amino acid
<220>
<221> Variant
<222> (62)..(62)
<223> Xaa in position 62 is Leu or Ile
<220>
<221> Variant
<222> (65)..(65)
<223> Xaa in position 65 is Ser or Cys
<220>
<221> Variant
<222> (66)..(66)
<223> Xaa in position 66 is Ser, Ala or Arg
<220>
<221> Variant
<222> (67)..(67)
<223> Xaa in position 67 is Asn or Leu
<220>
<221> Variant
<222> (68)..(68)
<223> Xaa in position 68 is Ile, Leu, Val, Thr or Met
<220>
<221> Variant
<222> (69)..(69)
<223> Xaa in position 69 is Asp, Glu or Asn
<220>
<221> Variant
<222> (70)..(70)
<223> Xaa in position 70 is any amino acid
<220>
<221> Variant
<222> (71)..(71)
<223> Xaa in position 71 is Asn, Gly, Leu, Ala, Asp or Ser
<220>
<221> Variant
<222> (72)..(73)
<223> Xaa in position 72 to 73 is any amino acid
<220>
<221> Variant
<222> (74)..(74)
<223> Xaa in position 74 is Val, Ile, Thr or Leu
<220>
<221> Variant
<222> (76)..(76)
<223> Xaa in position 76 is Thr or Ser
<220>
<221> Variant
<222> (77)..(77)
<223> Xaa in position 77 is Ile or Val
<220>
<221> Variant

<222> (78)..(78)
<223> xaa in position 78 is Phe, Val or Trp
<220>
<221> Variant
<222> (79)..(79)
<223> xaa in position 79 is Ser, Gly or Ala
<220>
<221> Variant
<222> (80)..(80)
<223> xaa in position 80 is Ile, Val or Leu

<400> 185
   Xaa Xaa Xaa Xaa Xaa Xaa Xaa Asp Xaa Xaa Xaa Xaa Xaa Xaa Xaa Xaa
   1               5                   10                  15
   Xaa Xaa Xaa Xaa Xaa Xaa Xaa Xaa Xaa Xaa Xaa Xaa Xaa Xaa Glu Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Asp Pro Xaa Asp Gly
       50                  55                  60
   Xaa Xaa Xaa Xaa Xaa Xaa Xaa Xaa Xaa Xaa Gly Xaa Xaa Xaa Xaa Xaa
   65                  70                  75                  80

<210> 186
<211> 78
<212> PRT
<213> Artificial sequence

<220>
<221> Variant
<222> (1)..(1)
<223> xaa in position 1 is Gly or Ala
<220>
<221> Variant
<222> (2)..(3)
<223> xaa in position 2 to 3 is any amino acid
<220>
<221> Variant
<222> (4)..(4)
<223> xaa in position 4 is Gln, Met, Leu or Ile
<220>
<221> Variant
<222> (5)..(5)
<223> xaa in position 5 is Val, Leu, Ile, Thr or Arg
<220>
<221> Variant
<222> (6)..(6)
<223> xaa in position 6 is Ala, Cys or Ser
<220>
<221> Variant
<222> (7)..(7)
<223> xaa in position 7 is Ala, Ser or Gly
<220>
<221> Variant
<222> (8)..(8)
<223> xaa in position 8 is Gly, Cys or Leu
<220>
<221> Variant
<222> (9)..(9)
<223> xaa in position 9 is Tyr or Phe
<220>
<221> Variant
<222> (10)..(10)
<223> xaa in position 10 is any amino acid
<220>
<221> Variant
<222> (11)..(11)

<223> xaa in position 11 is Leu, Met, Val, Ile, Ser or Thr
<220>
<221> Variant
<222> (13)..(13)
<223> xaa in position 13 is Gly or Ser
<220>
<221> Variant
<222> (14)..(14)
<223> xaa in position 14 is Ser, Pro, Ala or Thr
<220>
<221> Variant
<222> (15)..(15)
<223> xaa in position 15 is any amino acid
<220>
<221> Variant
<222> (16)..(16)
<223> xaa in position 16 is Thr, Cys, Val, Ala, Ile or Leu
<220>
<221> Variant
<222> (17)..(17)
<223> xaa in position 17 is any amino acid
<220>
<221> Variant
<222> (18)..(18)
<223> xaa in position 18 is Leu, Phe, Met, Val or Ile
<220>
<221> Variant
<222> (19)..(19)
<223> xaa in position 19 is Val, Leu, Met, Ala or Ile
<220>
<221> Variant
<222> (20)..(20)
<223> xaa in position 20 is Leu, Tyr, Ile, Val, Phe, Met or Cys
<220>
<221> Variant
<222> (21)..(21)
<223> xaa in position 21 is Thr, Ser, Ala or Cys
<220>
<221> Variant
<222> (22)..(22)
<223> xaa in position 22 is any amino acid
<220>
<221> Variant
<222> (23)..(23)
<223> xaa in position 23 is Gly, Ala, Asp, Glu, Lys or Arg
<220>
<221> Variant
<222> (24)..(24)
<223> xaa in position 24 is any amino acid
<220>
<221> Variant
<222> (25)..(25)
<223> xaa in position 25 is Gly, Ala or Pro
<220>
<221> Variant
<222> (26)..(26)
<223> xaa in position 26 is any or no amino acid
<220>
<221> Variant
<222> (27)..(27)
<223> xaa in position 27 is Val, Leu or Thr
<220>
<221> Variant
<222> (28)..(29)
<223> xaa in position 28 to 29 is any amino acid
<220>
<221> Variant
<222> (30)..(30)
<223> xaa in position 30 is Phe, Tyr, Leu or Trp

<220>
<221> Variant
<222> (31)..(32)
<223> Xaa in position 31 to 32 is any amino acid
<220>
<221> Variant
<222> (33)..(33)
<223> Xaa in position 33 is Asp, Glu, Asn, Ser or His
<220>
<221> Variant
<222> (34)..(34)
<223> Xaa in position 34 is any amino acid
<220>
<221> Variant
<222> (35)..(40)
<223> Xaa in position 35 to 40 is any or no amino acid
<220>
<221> Variant
<222> (41)..(41)
<223> Xaa in position 41 is Gly, Ala, Asn, Gln, Lys, Arg or Met
<220>
<221> Variant
<222> (42)..(42)
<223> Xaa in position 42 is any amino acid
<220>
<221> Variant
<222> (43)..(43)
<223> Xaa in position 43 is Phe, Tyr or Trp
<220>
<221> Variant
<222> (44)..(44)
<223> Xaa in position 44 is any amino acid
<220>
<221> Variant
<222> (45)..(45)
<223> Xaa in position 45 is Leu, Ile or Val
<220>
<221> Variant
<222> (46)..(49)
<223> Xaa in position 46 to 49 is any amino acid
<220>
<221> Variant
<222> (50)..(50)
<223> Xaa in position 50 is Ile, Leu, Met or Val
<220>
<221> Variant
<222> (51)..(51)
<223> Xaa in position 51 is any amino acid
<220>
<221> Variant
<222> (52)..(52)
<223> Xaa in position 52 is Ile, Leu, Met, Val, Phe or Tyr
<220>
<221> Variant
<222> (53)..(55)
<223> Xaa in position 53 to 55 is any amino acid
<220>
<221> Variant
<222> (56)..(59)
<223> Xaa in position 56 to 59 is any or no amino acid
<220>
<221> Variant
<222> (60)..(60)
<223> Xaa in position 60 is Tyr, Phe or Ile
<220>
<221> Variant
<222> (61)..(61)
<223> Xaa in position 61 is Ser, Ala or Asn
<220>

<221> Variant
<222> (62)..(62)
<223> xaa in position 62 is any amino acid
<220>
<221> Variant
<222> (63)..(63)
<223> xaa in position 63 is Asn or Gly
<220>
<221> Variant
<222> (64)..(64)
<223> xaa in position 64 is any amino acid
<220>
<221> Variant
<222> (65)..(65)
<223> xaa in position 65 is Gly, Ser or Ala
<220>
<221> Variant
<222> (66)..(69)
<223> xaa in position 66 to 69 is any amino acid
<220>
<221> Variant
<222> (70)..(70)
<223> xaa in position 70 is Trp, Phe or Tyr
<220>
<221> Variant
<222> (71)..(76)
<223> xaa in position 71 to 76 is any amino acid
<220>
<221> Variant
<222> (77)..(77)
<223> xaa in position 77 is Tyr or Phe
<220>
<221> Variant
<222> (78)..(78)
<223> xaa in position 78 is Ile, Val, Leu, Phe, Met or Thr

<400> 186
   Xaa Xaa Xaa Xaa Xaa Xaa Xaa Xaa Xaa Xaa Xaa Tyr Xaa Xaa Xaa Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa
   65                  70                  75


<210> 187
<211> 90
<212> PRT
<213> Artificial sequence

<220>
<221> Variant
<222> (1)..(1)
<223> xaa in position 1 is Tyr, Phe, Lys or Leu
<220>
<221> Variant
<222> (2)..(2)
<223> xaa in position 2 is any amino acid
<220>
<221> Variant
<222> (3)..(3)
<223> xaa in position 3 is Ala, Met, Leu, Ser, Thr or Gly
<220>
<221> Variant
<222> (5)..(5)

<223> xaa in position 5 is Tyr or Trp
<220>
<221> Variant
<222> (6)..(6)
<223> xaa in position 6 is Ile, Val, Ser, Gly, Thr, Asn or Leu
<220>
<221> Variant
<222> (7)..(7)
<223> xaa in position 7 is Gly, Ala or Cys
<220>
<221> Variant
<222> (8)..(8)
<223> xaa in position 8 is Ser, Ala or Gly
<220>
<221> Variant
<222> (9)..(9)
<223> xaa in position 9 is Met, Leu or Phe
<220>
<221> Variant
<222> (11)..(11)
<223> xaa in position 11 is Ala, Gly, Ser or Pro
<220>
<221> Variant
<222> (12)..(12)
<223> xaa in position 12 is Asp or Glu
<220>
<221> Variant
<222> (13)..(13)
<223> xaa in position 13 is Val, Phe, Ile, Ala, Thr, Met or Leu
<220>
<221> Variant
<222> (14)..(14)
<223> xaa in position 14 is His, Tyr or Asn
<220>
<221> Variant
<222> (15)..(15)
<223> xaa in position 15 is Arg or Gln
<220>
<221> Variant
<222> (16)..(16)
<223> xaa in position 16 is Thr, Ile, Asn or Val
<220>
<221> Variant
<222> (17)..(17)
<223> xaa in position 17 is Leu, Ile, Met or Phe
<220>
<221> Variant
<222> (18)..(18)
<223> xaa in position 18 is any amino acid
<220>
<221> Variant
<222> (19)..(19)
<223> xaa in position 19 is Tyr, Arg, Lys, Gln or Thr
<220>
<221> Variant
<222> (21)..(21)
<223> xaa in position 21 is Lys, Gly or no amino acid
<220>
<221> Variant
<222> (23)..(23)
<223> xaa in position 23 is Ile, Val, Leu or Met
<220>
<221> Variant
<222> (24)..(24)
<223> xaa in position 24 is Phe, Tyr or Ala
<220>
<221> Variant
<222> (25)..(25)
<223> xaa in position 25 is any amino acid

<220>
<221> Variant
<222> (26)..(26)
<223> Xaa in position 26 is Tyr or Asn
<220>
<221> Variant
<222> (28)..(28)
<223> Xaa in position 28 is any amino acid
<220>
<221> Variant
<222> (29)..(37)
<223> Xaa in position 29 to 37 is any or no amino acid
<220>
<221> Variant
<222> (38)..(38)
<223> Xaa in position 38 is Gly, Asp or Leu
<220>
<221> Variant
<222> (39)..(39)
<223> Xaa in position 39 is Lys, Arg or His
<220>
<221> Variant
<222> (42)..(42)
<223> Xaa in position 42 is any amino acid
<220>
<221> Variant
<222> (43)..(43)
<223> Xaa in position 43 is Leu, Met, Val or Gln
<220>
<221> Variant
<222> (44)..(44)
<223> Xaa in position 44 is Tyr, Phe or Cys
<220>
<221> Variant
<222> (46)..(46)
<223> Xaa in position 46 is Ala, Cys, Val, Gly, Thr, Ser or Leu
<220>
<221> Variant
<222> (47)..(47)
<223> Xaa in position 47 is any amino acid
<220>
<221> Variant
<222> (49)..(49)
<223> Xaa in position 49 is Met, Ile, Leu, Val or Phe
<220>
<221> Variant
<222> (50)..(50)
<223> Xaa in position 50 is Ala, Ser or Gly
<220>
<221> Variant
<222> (51)..(51)
<223> Xaa in position 51 is Phe, Tyr, Met, Leu or Trp
<220>
<221> Variant
<222> (52)..(52)
<223> Xaa in position 52 is Leu, Ile or Val
<220>
<221> Variant
<222> (53)..(53)
<223> Xaa in position 53 is any amino acid
<220>
<221> Variant
<222> (54)..(54)
<223> Xaa in position 54 is Glu, Thr or Gln
<220>
<221> Variant
<222> (55)..(55)
<223> Xaa in position 55 is any amino acid
<220>

<221> Variant
<222> (56)..(56)
<223> xaa in position 56 is Ala or Val
<220>
<221> Variant
<222> (57)..(57)
<223> xaa in position 57 is Gly or Ser
<220>
<221> Variant
<222> (58)..(58)
<223> xaa in position 58 is Gly or Ala
<220>
<221> Variant
<222> (59)..(59)
<223> xaa in position 59 is any amino acid
<220>
<221> Variant
<222> (60)..(60)
<223> xaa in position 60 is Ala, Ser, Gly or Cys
<220>
<221> Variant
<222> (61)..(61)
<223> xaa in position 61 is Thr, Ser, Val, Phe or Ile
<220>
<221> Variant
<222> (62)..(62)
<223> xaa in position 62 is Thr, Asp or Asn
<220>
<221> Variant
<222> (63)..(64)
<223> xaa in position 63 to 64 is any amino acid
<220>
<221> Variant
<222> (65)..(65)
<223> xaa in position 65 is Gly, Met or Asn
<220>
<221> Variant
<222> (66)..(67)
<223> xaa in position 66 to 67 is any amino acid
<220>
<221> Variant
<222> (68)..(68)
<223> xaa in position 68 is Ile, Val, Leu, Ala or Met
<220>
<221> Variant
<222> (69)..(69)
<223> xaa in position 69 is Leu or Met
<220>
<221> Variant
<222> (70)..(70)
<223> xaa in position 70 is Asp, Glu, Ser, Gly, Arg or Leu
<220>
<221> Variant
<222> (71)..(71)
<223> xaa in position 71 is Ile, Leu, Val, Ala, Gln or Arg
<220>
<221> Variant
<222> (72)..(72)
<223> xaa in position 72 is any amino acid
<220>
<221> Variant
<222> (73)..(73)
<223> xaa in position 73 is Pro or Ala
<220>
<221> Variant
<222> (74)..(75)
<223> xaa in position 74 to 75 is any amino acid
<220>
<221> Variant

<222> (76)..(76)
<223> Xaa in position 76 is Leu, Ile or Val
<220>
<221> Variant
<222> (77)..(77)
<223> Xaa in position 77 is His or Asp
<220>
<221> Variant
<222> (78)..(78)
<223> Xaa in position 78 is Gln, Glu, Asp, Ala or Gly
<220>
<221> Variant
<222> (79)..(79)
<223> Xaa in position 79 is Arg, Lys or Thr
<220>
<221> Variant
<222> (80)..(80)
<223> Xaa in position 80 is any amino acid
<220>
<221> Variant
<222> (81)..(81)
<223> Xaa in position 81 is Pro, Ser, Gly or Ala
<220>
<221> Variant
<222> (82)..(83)
<223> Xaa in position 82 to 83 is any amino acid
<220>
<221> Variant
<222> (84)..(84)
<223> Xaa in position 84 is Leu, Ile, Val, Phe or Met
<220>
<221> Variant
<222> (86)..(86)
<223> Xaa in position 86 is Ser, Thr, Asn, Cys or Gly
<220>
<221> Variant
<222> (87)..(89)
<223> Xaa in position 87 to 89 is any amino acid
<220>
<221> Variant
<222> (90)..(90)
<223> Xaa in position 90 is Val, Ile or Met

<400> 187
Xaa Xaa Xaa Arg Xaa Xaa Xaa Xaa Xaa Val Xaa Xaa Xaa Xaa Xaa Xaa
1               5                   10                  15
Xaa Xaa Xaa Gly Xaa Gly Xaa Xaa Xaa Xaa Pro Xaa Xaa Xaa Xaa Xaa
            20                  25                  30
Xaa Xaa Xaa Xaa Xaa Xaa Xaa Leu Arg Xaa Xaa Xaa Glu Xaa Xaa Pro
        35                  40                  45
Xaa Xaa Xaa Xaa Xaa Xaa Xaa Xaa Xaa Xaa Xaa Xaa Xaa Xaa Xaa Xaa
    50                  55                  60
Xaa Xaa Xaa Xaa Xaa Xaa Xaa Xaa Xaa Xaa Xaa Xaa Xaa Xaa Xaa Xaa
65                  70                  75                  80
Xaa Xaa Xaa Xaa Gly Xaa Xaa Xaa Xaa Xaa
                85                  90

<210> 188
<211> 1300
<212> DNA
<213> Helianthus annuus

<220>
<221> CDS
<222> (51)..(1133)

<400> 188
cacaactaca cacctacact tacacaacta cgtacacagc tacacgtaca atg gca       56
                                                       Met Ala

                                                  1
acc tca ccg gaa tcc gag cat ccg atc aag gcg tat ggt tat gcg gcc          104
Thr Ser Pro Glu Ser Glu His Pro Ile Lys Ala Tyr Gly Tyr Ala Ala
            5                   10                  15
cgc gac acg tct gga tta ctt tcc cct ctc acc ttc tcc aga agg gct          152
Arg Asp Thr Ser Gly Leu Leu Ser Pro Leu Thr Phe Ser Arg Arg Ala
    20                  25                  30
act gga gag aaa gat gtg agg ttc aaa gtt ctt tac tgc gga atc tgt          200
Thr Gly Glu Lys Asp Val Arg Phe Lys Val Leu Tyr Cys Gly Ile Cys
35                  40                  45                  50
cat tcc gat ctt cac gtt gtc aaa aac gaa tgg gga ttt acc aag tac          248
His Ser Asp Leu His Val Val Lys Asn Glu Trp Gly Phe Thr Lys Tyr
                55                  60                  65
ccg att act cca ggg cat gag att gtt ggt gtg gtg aca gaa gta gga          296
Pro Ile Thr Pro Gly His Glu Ile Val Gly Val Val Thr Glu Val Gly
            70                  75                  80
agc aaa gtg gag aat ttc aag att gga gac aaa gtt ggg gtt ggg tgc          344
Ser Lys Val Glu Asn Phe Lys Ile Gly Asp Lys Val Gly Val Gly Cys
        85                  90                  95
ttg gtg gga tca tgt aga tca tgt caa agt tgt gct tct gac tat gaa          392
Leu Val Gly Ser Cys Arg Ser Cys Gln Ser Cys Ala Ser Asp Tyr Glu
    100                 105                 110
cag tat tgc gat aag cag gta caa act tat ggt ttc acc aac ttt gat          440
Gln Tyr Cys Asp Lys Gln Val Gln Thr Tyr Gly Phe Thr Asn Phe Asp
115                 120                 125                 130
ggc aca cag acg tac ggt gga tac tcc gat cac atg gtt tca gat gag          488
Gly Thr Gln Thr Tyr Gly Gly Tyr Ser Asp His Met Val Ser Asp Glu
                135                 140                 145
cat ttt gta ctc cgt tgg ccg gag aac tta cca ctt gat tct ggt gca          536
His Phe Val Leu Arg Trp Pro Glu Asn Leu Pro Leu Asp Ser Gly Ala
            150                 155                 160
ccg ttg cta tgt gct ggg atc aca act tac agc cct ctc aag tac ttt          584
Pro Leu Leu Cys Ala Gly Ile Thr Thr Tyr Ser Pro Leu Lys Tyr Phe
        165                 170                 175
gga ttg gac aag cct ggt acg aaa gtg ggc gtg gtt ggg ctc ggt ggg          632
Gly Leu Asp Lys Pro Gly Thr Lys Val Gly Val Val Gly Leu Gly Gly
    180                 185                 190
ctc ggt cat gtt gct gtg aag atg gct aag gca ttt ggg gct gaa gtt          680
Leu Gly His Val Ala Val Lys Met Ala Lys Ala Phe Gly Ala Glu Val
195                 200                 205                 210
act gtt ttt agt act tcc cct gct aag gag aaa gaa gca att cag gga          728
Thr Val Phe Ser Thr Ser Pro Ala Lys Glu Lys Glu Ala Ile Gln Gly
                215                 220                 225
ctt aaa gct gac cat ttc gta aac agt aaa gat gga ctt caa atg cag          776
Leu Lys Ala Asp His Phe Val Asn Ser Lys Asp Gly Leu Gln Met Gln
            230                 235                 240
gct gct aag ggc aca ctc gat ggc atc atc gac aca gtg tcc gca aat          824
Ala Ala Lys Gly Thr Leu Asp Gly Ile Ile Asp Thr Val Ser Ala Asn
        245                 250                 255
cat cca att gcg cca ttg ctt aat gca ctt aca ccc cat gga aag ctt          872
His Pro Ile Ala Pro Leu Leu Asn Ala Leu Thr Pro His Gly Lys Leu
    260                 265                 270
gtt ctt gtt ggt gca cct gag aag cca ctt gaa gta gca gca ttt tcg          920
Val Leu Val Gly Ala Pro Glu Lys Pro Leu Glu Val Ala Ala Phe Ser
275                 280                 285                 290
ttg atc atg gga agg aag ctt gtt gcg ggt agt aac att ggt gga att          968
Leu Ile Met Gly Arg Lys Leu Val Ala Gly Ser Asn Ile Gly Gly Ile
                295                 300                 305
aaa gag act cag gaa atg ctt gac ttt gca gca aag cat ggg ata act          1016
Lys Glu Thr Gln Glu Met Leu Asp Phe Ala Ala Lys His Gly Ile Thr
            310                 315                 320
gca gat ata gag gtt ata ccg ata gac tat gtg aac aca gcg atg gaa          1064
Ala Asp Ile Glu Val Ile Pro Ile Asp Tyr Val Asn Thr Ala Met Glu
        325                 330                 335
agg ctg ttg aaa tcc gat gtg agg tac cga ttt gtg atc gat gtg gcg          1112
Arg Leu Leu Lys Ser Asp Val Arg Tyr Arg Phe Val Ile Asp Val Ala
    340                 345                 350
aac tcg atc aag gct gaa tag gttgagtctt gaagttcttg ctgaatgttg          1163
Asn Ser Ile Lys Ala Glu

355                 360
tatttctctt tatagttttg agtatttgtg agcatgttat agagaagtga gagtcctgag     1223

gcttcttcgt ttgtatgcat ttgtttttgg tattgttaat aacttaatat atgtatcaaa     1283

ctaaaaaaaa aaaaaaa                                                    1300

<210> 189
<211> 360
<212> PRT
<213> Helianthus annuus

<400> 189
Met Ala Thr Ser Pro Glu Ser Glu His Pro Ile Lys Ala Tyr Gly Tyr
1               5                   10                  15
Ala Ala Arg Asp Thr Ser Gly Leu Leu Ser Pro Leu Thr Phe Ser Arg
            20                  25                  30
Arg Ala Thr Gly Glu Lys Asp Val Arg Phe Lys Val Leu Tyr Cys Gly
        35                  40                  45
Ile Cys His Ser Asp Leu His Val Val Lys Asn Glu Trp Gly Phe Thr
    50                  55                  60
Lys Tyr Pro Ile Thr Pro Gly His Glu Ile Val Gly Val Val Thr Glu
65                  70                  75                  80
Val Gly Ser Lys Val Glu Asn Phe Lys Ile Gly Asp Lys Val Gly Val
                85                  90                  95
Gly Cys Leu Val Gly Ser Cys Arg Ser Cys Gln Ser Cys Ala Ser Asp
            100                 105                 110
Tyr Glu Gln Tyr Cys Asp Lys Gln Val Gln Thr Tyr Gly Phe Thr Asn
        115                 120                 125
Phe Asp Gly Thr Gln Thr Tyr Gly Gly Tyr Ser Asp His Met Val Ser
    130                 135                 140
Asp Glu His Phe Val Leu Arg Trp Pro Glu Asn Leu Pro Leu Asp Ser
145                 150                 155                 160
Gly Ala Pro Leu Leu Cys Ala Gly Ile Thr Thr Tyr Ser Pro Leu Lys
                165                 170                 175
Tyr Phe Gly Leu Asp Lys Pro Gly Thr Lys Val Gly Val Val Gly Leu
            180                 185                 190
Gly Gly Leu Gly His Val Ala Val Lys Met Ala Lys Ala Phe Gly Ala
        195                 200                 205
Glu Val Thr Val Phe Ser Thr Ser Pro Ala Lys Glu Lys Glu Ala Ile
    210                 215                 220
Gln Gly Leu Lys Ala Asp His Phe Val Asn Ser Lys Asp Gly Leu Gln
225                 230                 235                 240
Met Gln Ala Ala Lys Gly Thr Leu Asp Gly Ile Ile Asp Thr Val Ser
                245                 250                 255
Ala Asn His Pro Ile Ala Pro Leu Leu Asn Ala Leu Thr Pro His Gly
            260                 265                 270
Lys Leu Val Leu Val Gly Ala Pro Glu Lys Pro Leu Glu Val Ala Ala
        275                 280                 285
Phe Ser Leu Ile Met Gly Arg Lys Leu Val Ala Gly Ser Asn Ile Gly
    290                 295                 300
Gly Ile Lys Glu Thr Gln Glu Met Leu Asp Phe Ala Ala Lys His Gly
305                 310                 315                 320
Ile Thr Ala Asp Ile Glu Val Ile Pro Ile Asp Tyr Val Asn Thr Ala
                325                 330                 335
Met Glu Arg Leu Leu Lys Ser Asp Val Arg Tyr Arg Phe Val Ile Asp
            340                 345                 350
Val Ala Asn Ser Ile Lys Ala Glu
        355                 360

<210> 190
<211> 1335
<212> DNA
<213> Brassica napus

<220>
<221> CDS
<222> (30)..(1112)

<400> 190
cctcactcac tcagacaaaa gagaaacaa atg gcg aaa tct cca gag aca gag 53
Met Ala Lys Ser Pro Glu Thr Glu
1               5
cat ccg aac aaa gcc ttt ggt tgg gct gcc aaa gac aaa tct ggt gta 101
His Pro Asn Lys Ala Phe Gly Trp Ala Ala Lys Asp Lys Ser Gly Val
    10                  15                  20
ctc tct cct ttc cat ttc tcc aga aga gac aat ggt gac aat gat gtg 149
Leu Ser Pro Phe His Phe Ser Arg Arg Asp Asn Gly Asp Asn Asp Val
25                  30                  35                  40
aca gtg aaa atc ttg tac tgt gga gtg tgc cac act gat tta cat acc 197
Thr Val Lys Ile Leu Tyr Cys Gly Val Cys His Thr Asp Leu His Thr
                45                  50                  55
atc aaa aac gac tgg ggt ttc tcc tat tat cct gta gtt ccc ggg cat 245
Ile Lys Asn Asp Trp Gly Phe Ser Tyr Tyr Pro Val Val Pro Gly His
            60                  65                  70
gaa ata gtt gga ata gct aca aaa gtt ggc aaa aac gtg act aaa ttc 293
Glu Ile Val Gly Ile Ala Thr Lys Val Gly Lys Asn Val Thr Lys Phe
        75                  80                  85
aga gaa gga gac cgt gtt gga gta gga gtg atc act ggt tct tgc cag 341
Arg Glu Gly Asp Arg Val Gly Val Gly Val Ile Thr Gly Ser Cys Gln
    90                  95                  100
tct tgc gaa tct tgt aac caa gat ctt gaa aac tac tgt ccc caa atg 389
Ser Cys Glu Ser Cys Asn Gln Asp Leu Glu Asn Tyr Cys Pro Gln Met
105                 110                 115                 120
tct ttc acc tac aac tcc ata gga tcc gat gga acc aag acc tac ggt 437
Ser Phe Thr Tyr Asn Ser Ile Gly Ser Asp Gly Thr Lys Thr Tyr Gly
                125                 130                 135
gga tat tcg gag tcc att gtg gtc gat caa cgt ttt gtc tta cag ttt 485
Gly Tyr Ser Glu Ser Ile Val Val Asp Gln Arg Phe Val Leu Gln Phe
            140                 145                 150
ccc gag gga ttg cct agc gat tcg ggt gcc ccc ttg ctg tgt gct ggt 533
Pro Glu Gly Leu Pro Ser Asp Ser Gly Ala Pro Leu Leu Cys Ala Gly
        155                 160                 165
atc act gtg tat agt cca atg aag tac tat ggt atg acc gag cca ggg 581
Ile Thr Val Tyr Ser Pro Met Lys Tyr Tyr Gly Met Thr Glu Pro Gly
    170                 175                 180
aag cat ttg ggc gtg gct gga ctt ggt ggg ctt ggt cat gtt gct gtc 629
Lys His Leu Gly Val Ala Gly Leu Gly Gly Leu Gly His Val Ala Val
185                 190                 195                 200
aag att ggt aaa gcc ttt ggt ttg aaa gtt act gtg att agt tct tct 677
Lys Ile Gly Lys Ala Phe Gly Leu Lys Val Thr Val Ile Ser Ser Ser
                205                 210                 215
cct agt aaa gaa gac gaa gct att aat cga ctt ggt gct gat tcg ttt 725
Pro Ser Lys Glu Asp Glu Ala Ile Asn Arg Leu Gly Ala Asp Ser Phe
            220                 225                 230
ctt gtc tca tct gat cct cag aag atg aag gct gca att gga act atg 773
Leu Val Ser Ser Asp Pro Gln Lys Met Lys Ala Ala Ile Gly Thr Met
        235                 240                 245
gac tat att atc gat acg gta tca gca gta cat gct ctg ttt cca ttg 821
Asp Tyr Ile Ile Asp Thr Val Ser Ala Val His Ala Leu Phe Pro Leu
    250                 255                 260
ctt ggt tta ctc aaa gtc aat gga aag ctt gtc acg tta ggc tta cct 869
Leu Gly Leu Leu Lys Val Asn Gly Lys Leu Val Thr Leu Gly Leu Pro
265                 270                 275                 280
gag aag cct ctt gag cta cca atc ttc cct ctt gtt ctc gga aga aaa 917
Glu Lys Pro Leu Glu Leu Pro Ile Phe Pro Leu Val Leu Gly Arg Lys
                285                 290                 295
atg gtg gga gga agt gac att gga ggg atg aag gag aca caa gag atg 965
Met Val Gly Gly Ser Asp Ile Gly Gly Met Lys Glu Thr Gln Glu Met
            300                 305                 310
ctt gag ttc tgc gct aag cat aac ata acg gca gat att gag ctg atc 1013
Leu Glu Phe Cys Ala Lys His Asn Ile Thr Ala Asp Ile Glu Leu Ile
        315                 320                 325
aag atg gat gag att aac act gcg atg gag agg ctt gct aag tct gat 1061

Lys Met Asp Glu Ile Asn Thr Ala Met Glu Arg Leu Ala Lys Ser Asp
    330                 335                 340
gtt agg tac agg ttc gtg atc gac gtg gct aac tcc ttg agc cct cca      1109
Val Arg Tyr Arg Phe Val Ile Asp Val Ala Asn Ser Leu Ser Pro Pro
345                 350                 355                 360
tga cttagccgaa cctaagagtt ggctctggag gagtttgtat ctttgggctt           1162

tttctgtgtt tgtttctctc gcgttatctc cgttttctac tttcttgtta gtgaactggt    1222

ttcttgtgga acaagtcacc taaattatca gagaacttta ctctgttttt cttttcatgt    1282

tttcgatatt gagatataat aagtctttct gattagagaa aaaaaaaaaa aaa           1335

<210> 191
<211> 360
<212> PRT
<213> Brassica napus

<400> 191
Met Ala Lys Ser Pro Glu Thr Glu His Pro Asn Lys Ala Phe Gly Trp
1               5                   10                  15
Ala Ala Lys Asp Lys Ser Gly Val Leu Ser Pro Phe His Phe Ser Arg
            20                  25                  30
Arg Asp Asn Gly Asp Asn Asp Val Thr Val Lys Ile Leu Tyr Cys Gly
        35                  40                  45
Val Cys His Thr Asp Leu His Thr Ile Lys Asn Asp Trp Gly Phe Ser
    50                  55                  60
Tyr Tyr Pro Val Val Pro Gly His Glu Ile Val Gly Ile Ala Thr Lys
65                  70                  75                  80
Val Gly Lys Asn Val Thr Lys Phe Arg Glu Gly Asp Arg Val Gly Val
                85                  90                  95
Gly Val Ile Thr Gly Ser Cys Gln Ser Cys Glu Ser Cys Asn Gln Asp
            100                 105                 110
Leu Glu Asn Tyr Cys Pro Gln Met Ser Phe Thr Tyr Asn Ser Ile Gly
        115                 120                 125
Ser Asp Gly Thr Lys Thr Tyr Gly Gly Tyr Ser Glu Ser Ile Val Val
    130                 135                 140
Asp Gln Arg Phe Val Leu Gln Phe Pro Glu Gly Leu Pro Ser Asp Ser
145                 150                 155                 160
Gly Ala Pro Leu Leu Cys Ala Gly Ile Thr Val Tyr Ser Pro Met Lys
                165                 170                 175
Tyr Tyr Gly Met Thr Glu Pro Gly Lys His Leu Gly Val Ala Gly Leu
            180                 185                 190
Gly Gly Leu Gly His Val Ala Val Lys Ile Gly Lys Ala Phe Gly Leu
        195                 200                 205
Lys Val Thr Val Ile Ser Ser Ser Pro Ser Lys Glu Asp Glu Ala Ile
    210                 215                 220
Asn Arg Leu Gly Ala Asp Ser Phe Leu Val Ser Ser Asp Pro Gln Lys
225                 230                 235                 240
Met Lys Ala Ala Ile Gly Thr Met Asp Tyr Ile Ile Asp Thr Val Ser
                245                 250                 255
Ala Val His Ala Leu Phe Pro Leu Leu Gly Leu Leu Lys Val Asn Gly
            260                 265                 270
Lys Leu Val Thr Leu Gly Leu Pro Glu Lys Pro Leu Glu Leu Pro Ile
        275                 280                 285
Phe Pro Leu Val Leu Gly Arg Lys Met Val Gly Gly Ser Asp Ile Gly
    290                 295                 300
Gly Met Lys Glu Thr Gln Glu Met Leu Glu Phe Cys Ala Lys His Asn
305                 310                 315                 320
Ile Thr Ala Asp Ile Glu Leu Ile Lys Met Asp Glu Ile Asn Thr Ala
                325                 330                 335
Met Glu Arg Leu Ala Lys Ser Asp Val Arg Tyr Arg Phe Val Ile Asp
            340                 345                 350
Val Ala Asn Ser Leu Ser Pro Pro

355                 360

<210> 192
<211> 1814
<212> DNA
<213> Glycine max

<220>
<221> CDS
<222> (166)..(1449)

<400> 192
tgaatattaa ggtagtaaga tgagacgtca agtttggatc tctatctcat tgttgattta      60

gtattaaatt tctatgtagt tgttgtttgt agtaatcatc aactcactca gttgccgtag     120

gttgtcgtga tccatcacaa cactttgtca ctgaaattca gaaaa atg gca tca gca     177
                                                  Met Ala Ser Ala
                                                  1
atg ttt cgt agg aac atc atc acc cac tac tcc aag ttt gct tct tct     225
Met Phe Arg Arg Asn Ile Ile Thr His Tyr Ser Lys Phe Ala Ser Ser
5                   10                  15                  20
ttg tct tcg ttg cct cgc cat tcc tca atc tac aac cct ctc tct atg     273
Leu Ser Ser Leu Pro Arg His Ser Ser Ile Tyr Asn Pro Leu Ser Met
                25                  30                  35
tct gtt tct tca ttt cat agc gaa agt gtt ggt ggt ggt ggt ggg ttg     321
Ser Val Ser Ser Phe His Ser Glu Ser Val Gly Gly Gly Gly Gly Leu
            40                  45                  50
cca tct tcc atg cgt gga gct gtg tat tgg gaa ccg aac aag ccg cta     369
Pro Ser Ser Met Arg Gly Ala Val Tyr Trp Glu Pro Asn Lys Pro Leu
        55                  60                  65
acc atc gag gaa ttt cac atg cct cgc ccc aag gcc ggt gaa gtt ctc     417
Thr Ile Glu Glu Phe His Met Pro Arg Pro Lys Ala Gly Glu Val Leu
    70                  75                  80
atc aaa aca aag gcc tgt gga gta tgc cac tct gat cta cat gtt atg     465
Ile Lys Thr Lys Ala Cys Gly Val Cys His Ser Asp Leu His Val Met
85                  90                  95                  100
aaa ggt gaa att ccg ttt acc agt cct tgt gtt gtg ggg cat gag att     513
Lys Gly Glu Ile Pro Phe Thr Ser Pro Cys Val Val Gly His Glu Ile
                105                 110                 115
acc ggt gag gtt gtt gaa cat gga gca ctc act gac agc aaa acc ata     561
Thr Gly Glu Val Val Glu His Gly Ala Leu Thr Asp Ser Lys Thr Ile
            120                 125                 130
gaa agg ctt cca gtt gga tct cga gtt gtt ggg gcc ttc atc atg cct     609
Glu Arg Leu Pro Val Gly Ser Arg Val Val Gly Ala Phe Ile Met Pro
        135                 140                 145
tgt ggc aac tgc tcc tac tgt tca aag ggc cat gat gat cta tgt gaa     657
Cys Gly Asn Cys Ser Tyr Cys Ser Lys Gly His Asp Asp Leu Cys Glu
    150                 155                 160
gct ttc ttt gcc tat aac cgg gca aaa gga acc ctc tat gat ggt gaa     705
Ala Phe Phe Ala Tyr Asn Arg Ala Lys Gly Thr Leu Tyr Asp Gly Glu
165                 170                 175                 180
act cga ctc ttt ttt cga aac agt gga aaa cca gca tac atg tat agt     753
Thr Arg Leu Phe Phe Arg Asn Ser Gly Lys Pro Ala Tyr Met Tyr Ser
                185                 190                 195
atg gga ggg ctt gct gaa tac tgt gtt gtg cca gca aat gga gtg tct     801
Met Gly Gly Leu Ala Glu Tyr Cys Val Val Pro Ala Asn Gly Val Ser
            200                 205                 210
gtg tta cca gac tca ttg cca tat aca gag tct gca att cta gga tgt     849
Val Leu Pro Asp Ser Leu Pro Tyr Thr Glu Ser Ala Ile Leu Gly Cys
        215                 220                 225
gct gtt ttt act gct tac ggc gct atg gct cat gca gct caa gtg cgt     897
Ala Val Phe Thr Ala Tyr Gly Ala Met Ala His Ala Ala Gln Val Arg
    230                 235                 240
cct ggt gat tct gtt gct gtt att gga act gga ggt gtt ggt tct agt     945
Pro Gly Asp Ser Val Ala Val Ile Gly Thr Gly Gly Val Gly Ser Ser
245                 250                 255                 260

tgt tta cag ata gct aga gcc ttt ggt gcc tca gat ata att gct gtg 993
Cys Leu Gln Ile Ala Arg Ala Phe Gly Ala Ser Asp Ile Ile Ala Val
                265                 270                 275
gat gta cgg gat gaa aag cta cag aaa gca aag aca ttt ggt gct act 1041
Asp Val Arg Asp Glu Lys Leu Gln Lys Ala Lys Thr Phe Gly Ala Thr
            280                 285                 290
cac act gta aat tca gca aag gaa gat ccc att gaa aag ata ctt gaa 1089
His Thr Val Asn Ser Ala Lys Glu Asp Pro Ile Glu Lys Ile Leu Glu
        295                 300                 305
att act ggt gga aag ggt gtt gat gtt gct gtg gaa gcc ttg ggg aaa 1137
Ile Thr Gly Gly Lys Gly Val Asp Val Ala Val Glu Ala Leu Gly Lys
    310                 315                 320
cca cag aca ttt gct caa tgt aca caa agt gtg aag gat ggg gga aaa 1185
Pro Gln Thr Phe Ala Gln Cys Thr Gln Ser Val Lys Asp Gly Gly Lys
325                 330                 335                 340
gct gtg atg att gga ctg gca caa gct ggt tct tta gga gag gtg gat 1233
Ala Val Met Ile Gly Leu Ala Gln Ala Gly Ser Leu Gly Glu Val Asp
                345                 350                 355
ata aat cgg ctt gtt cgc aga aag ata caa gtt att ggc tcc tat ggg 1281
Ile Asn Arg Leu Val Arg Arg Lys Ile Gln Val Ile Gly Ser Tyr Gly
            360                 365                 370
ggc agg gca agg caa gat ctt cca aag tta att aga ctg gca gaa aca 1329
Gly Arg Ala Arg Gln Asp Leu Pro Lys Leu Ile Arg Leu Ala Glu Thr
        375                 380                 385
ggt atc ttc aat ctt ggc cat gct gtt tca aga aca tac act ttt gag 1377
Gly Ile Phe Asn Leu Gly His Ala Val Ser Arg Thr Tyr Thr Phe Glu
    390                 395                 400
gag gcg gga aaa gca ttc caa gat ctc aat gaa gga aaa att gtc ggt 1425
Glu Ala Gly Lys Ala Phe Gln Asp Leu Asn Glu Gly Lys Ile Val Gly
405                 410                 415                 420
cga gct gtg att gag ata ata tag ctctatattc ccagaaatgc ttttctgcag 1479
Arg Ala Val Ile Glu Ile Ile
                425
cagcaatagc tatgcttttc acaccccaca agatatacat gctttggagt tgcaaggtgt 1539

tggtaccctg agaacataaa taaatagatg tcttctgatg tttcattgca ggggcatgtt 1599

caaatgaaaa ctgtttttct ttttgtgcat ttctttgctt ctaactcatg taatcaggta 1659

ggtacaccaa ggtgaagaat aatagtctct gaatgctcat cattggcgtg tcgtaaaact 1719

gatgatgaaa atgcaggaaa ataacagaaa agctagagat gctccaagaa agtaaattaa 1779

aaaaaaaatg tttccgacat aaaaaaaaaa aaaaa 1814

<210> 193
<211> 427
<212> PRT
<213> Glycine max

<400> 193
Met Ala Ser Ala Met Phe Arg Arg Asn Ile Ile Thr His Tyr Ser Lys
1               5                   10                  15
Phe Ala Ser Ser Leu Ser Ser Leu Pro Arg His Ser Ser Ile Tyr Asn
            20                  25                  30
Pro Leu Ser Met Ser Val Ser Ser Phe His Ser Glu Ser Val Gly Gly
        35                  40                  45
Gly Gly Gly Leu Pro Ser Ser Met Arg Gly Ala Val Tyr Trp Glu Pro
    50                  55                  60
Asn Lys Pro Leu Thr Ile Glu Glu Phe His Met Pro Arg Pro Lys Ala
65                  70                  75                  80
Gly Glu Val Leu Ile Lys Thr Lys Ala Cys Gly Val Cys His Ser Asp

                    85                  90                  95
Leu His Val Met Lys Gly Glu Ile Pro Phe Thr Ser Pro Cys Val Val
            100                 105                 110
Gly His Glu Ile Thr Gly Glu Val Val Glu His Gly Ala Leu Thr Asp
        115                 120                 125
Ser Lys Thr Ile Glu Arg Leu Pro Val Gly Ser Arg Val Val Gly Ala
    130                 135                 140
Phe Ile Met Pro Cys Gly Asn Cys Ser Tyr Cys Ser Lys Gly His Asp
145                 150                 155                 160
Asp Leu Cys Glu Ala Phe Phe Ala Tyr Asn Arg Ala Lys Gly Thr Leu
                165                 170                 175
Tyr Asp Gly Glu Thr Arg Leu Phe Phe Arg Asn Ser Gly Lys Pro Ala
            180                 185                 190
Tyr Met Tyr Ser Met Gly Gly Leu Ala Glu Tyr Cys Val Val Pro Ala
        195                 200                 205
Asn Gly Val Ser Val Leu Pro Asp Ser Leu Pro Tyr Thr Glu Ser Ala
    210                 215                 220
Ile Leu Gly Cys Ala Val Phe Thr Ala Tyr Gly Ala Met Ala His Ala
225                 230                 235                 240
Ala Gln Val Arg Pro Gly Asp Ser Val Ala Val Ile Gly Thr Gly Gly
                245                 250                 255
Val Gly Ser Ser Cys Leu Gln Ile Ala Arg Ala Phe Gly Ala Ser Asp
            260                 265                 270
Ile Ile Ala Val Asp Val Arg Asp Glu Lys Leu Gln Lys Ala Lys Thr
        275                 280                 285
Phe Gly Ala Thr His Thr Val Asn Ser Ala Lys Glu Asp Pro Ile Glu
    290                 295                 300
Lys Ile Leu Glu Ile Thr Gly Gly Lys Gly Val Asp Val Ala Val Glu
305                 310                 315                 320
Ala Leu Gly Lys Pro Gln Thr Phe Ala Gln Cys Thr Gln Ser Val Lys
                325                 330                 335
Asp Gly Gly Lys Ala Val Met Ile Gly Leu Ala Gln Ala Gly Ser Leu
            340                 345                 350
Gly Glu Val Asp Ile Asn Arg Leu Val Arg Arg Lys Ile Gln Val Ile
        355                 360                 365
Gly Ser Tyr Gly Gly Arg Ala Arg Gln Asp Leu Pro Lys Leu Ile Arg
    370                 375                 380
Leu Ala Glu Thr Gly Ile Phe Asn Leu Gly His Ala Val Ser Arg Thr
385                 390                 395                 400
Tyr Thr Phe Glu Glu Ala Gly Lys Ala Phe Gln Asp Leu Asn Glu Gly
                405                 410                 415
Lys Ile Val Gly Arg Ala Val Ile Glu Ile Ile
            420                 425

<210> 194
<211> 1226
<212> DNA
<213> Helianthus annuus

<220>
<221> CDS
<222> (25)..(1107)

<400> 194
cctctccaaa agcattaatt atca atg gcg aaa tca cct gaa tcc gag cat        51
                           Met Ala Lys Ser Pro Glu Ser Glu His
                           1               5
ccg atc aag gcc tat ggt tat gct gct cga gac act tcc gga gtt ctt        99
Pro Ile Lys Ala Tyr Gly Tyr Ala Ala Arg Asp Thr Ser Gly Val Leu
10                  15                  20                  25
tct cct ctc act ttc tca cga agg gct aca ggt gat aag gat gtg agg       147
Ser Pro Leu Thr Phe Ser Arg Arg Ala Thr Gly Asp Lys Asp Val Arg
                30                  35                  40
ttt aaa gtt ttg tat tgc ggg atc tgc cat tct gat ctc cac ttt gcc       195
Phe Lys Val Leu Tyr Cys Gly Ile Cys His Ser Asp Leu His Phe Ala
            45                  50                  55
aag aac gaa tgg ggc atg tct aca tat ccc att ctt cct ggg cat gag       243
Lys Asn Glu Trp Gly Met Ser Thr Tyr Pro Ile Leu Pro Gly His Glu
        60                  65                  70

atc gtg ggt gtg gtg aca gaa gta ggc acc aag gtg gag aaa ttc aaa 291
Ile Val Gly Val Val Thr Glu Val Gly Thr Lys Val Glu Lys Phe Lys
    75                  80                  85
gtt ggg gac act gta ggg gtt gga tgt ttg gtg gga tca tgc gga tct 339
Val Gly Asp Thr Val Gly Val Gly Cys Leu Val Gly Ser Cys Gly Ser
90                  95                  100                 105
tgt caa agt tgt gcc aat gat ctt gaa agc tac tgt ccc aaa cag att 387
Cys Gln Ser Cys Ala Asn Asp Leu Glu Ser Tyr Cys Pro Lys Gln Ile
                110                 115                 120
cta act tac ggt ttc ctg tac cat gat ggc aca ctt aca tac ggt ggc 435
Leu Thr Tyr Gly Phe Leu Tyr His Asp Gly Thr Leu Thr Tyr Gly Gly
            125                 130                 135
tac tcc gat cac atg gtg gca gat gag cat ttt gtg ctg cgt tgg ccc 483
Tyr Ser Asp His Met Val Ala Asp Glu His Phe Val Leu Arg Trp Pro
        140                 145                 150
gag aat ttg ccc ctt gat tcg ggt gca cca ttg tta tgt gcc ggc atc 531
Glu Asn Leu Pro Leu Asp Ser Gly Ala Pro Leu Leu Cys Ala Gly Ile
    155                 160                 165
aca act tat agc cca ctc aga cac ttt gga cta gac aag cct ggt atg 579
Thr Thr Tyr Ser Pro Leu Arg His Phe Gly Leu Asp Lys Pro Gly Met
170                 175                 180                 185
aag gtg ggt gtg gtt ggt ctt ggt ggg ctt ggt cat gtt gcg gtc aag 627
Lys Val Gly Val Val Gly Leu Gly Gly Leu Gly His Val Ala Val Lys
                190                 195                 200
ttt gcc aag gct ttt gga gcc gag gtt aca gtc atc agt acc agc tcg 675
Phe Ala Lys Ala Phe Gly Ala Glu Val Thr Val Ile Ser Thr Ser Ser
            205                 210                 215
gct aag aag caa gaa gcg ctt caa gga ctt aaa gct gac cat ttc att 723
Ala Lys Lys Gln Glu Ala Leu Gln Gly Leu Lys Ala Asp His Phe Ile
        220                 225                 230
gtt agc aaa gac cct gag caa atg cag tgt gca gca agt tct ttg gat 771
Val Ser Lys Asp Pro Glu Gln Met Gln Cys Ala Ala Ser Ser Leu Asp
    235                 240                 245
gga ata att gac act gtc tct gcg tct cac ccc att gaa cca ttg ttg 819
Gly Ile Ile Asp Thr Val Ser Ala Ser His Pro Ile Glu Pro Leu Leu
250                 255                 260                 265
agt gtg ctc aaa caa gat ggg aag ctt gtt ttt ctg ggt gta cca gag 867
Ser Val Leu Lys Gln Asp Gly Lys Leu Val Phe Leu Gly Val Pro Glu
                270                 275                 280
aag cca att gag ata tct acg tat tct tta att atg ggg aga aag atc 915
Lys Pro Ile Glu Ile Ser Thr Tyr Ser Leu Ile Met Gly Arg Lys Ile
            285                 290                 295
gtt act gga agt gcg acg gga ggg cta aag gag act cag gag atg ctt 963
Val Thr Gly Ser Ala Thr Gly Gly Leu Lys Glu Thr Gln Glu Met Leu
        300                 305                 310
gat ttt gcc gca aaa cat ggc gtg act gca gat att gag cta att ccc 1011
Asp Phe Ala Ala Lys His Gly Val Thr Ala Asp Ile Glu Leu Ile Pro
    315                 320                 325
ata gac tat gtg aac acg gct atg gat cgc gtc ttg aaa tcg gat gtt 1059
Ile Asp Tyr Val Asn Thr Ala Met Asp Arg Val Leu Lys Ser Asp Val
330                 335                 340                 345
aga tac cga ttt gtc att gat gtg gcg aac acc ctc aaa gct cca tag 1107
Arg Tyr Arg Phe Val Ile Asp Val Ala Asn Thr Leu Lys Ala Pro
                350                 355                 360
gtgaattgtt taattttgtg tttgttgttt aagaacttga gtcagtgttg gattgttgtc 1167

gctatatgtt ttatgaataa agactcggtt cctcttcagt tgataaaaaa aaaaaaaaa 1226

<210> 195
<211> 360
<212> PRT
<213> Helianthus annuus

<400> 195
Met Ala Lys Ser Pro Glu Ser Glu His Pro Ile Lys Ala Tyr Gly Tyr
1               5                   10                  15

Ala Ala Arg Asp Thr Ser Gly Val Leu Ser Pro Leu Thr Phe Ser Arg
            20                  25                  30
Arg Ala Thr Gly Asp Lys Asp Val Arg Phe Lys Val Leu Tyr Cys Gly
        35                  40                  45
Ile Cys His Ser Asp Leu His Phe Ala Lys Asn Glu Trp Gly Met Ser
    50                  55                  60
Thr Tyr Pro Ile Leu Pro Gly His Glu Ile Val Gly Val Val Thr Glu
65                  70                  75                  80
Val Gly Thr Lys Val Glu Lys Phe Lys Val Gly Asp Thr Val Gly Val
                85                  90                  95
Gly Cys Leu Val Gly Ser Cys Gly Ser Cys Gln Ser Cys Ala Asn Asp
            100                 105                 110
Leu Glu Ser Tyr Cys Pro Lys Gln Ile Leu Thr Tyr Gly Phe Leu Tyr
        115                 120                 125
His Asp Gly Thr Leu Thr Tyr Gly Gly Tyr Ser Asp His Met Val Ala
    130                 135                 140
Asp Glu His Phe Val Leu Arg Trp Pro Glu Asn Leu Pro Leu Asp Ser
145                 150                 155                 160
Gly Ala Pro Leu Leu Cys Ala Gly Ile Thr Thr Tyr Ser Pro Leu Arg
                165                 170                 175
His Phe Gly Leu Asp Lys Pro Gly Met Lys Val Gly Val Val Gly Leu
            180                 185                 190
Gly Gly Leu Gly His Val Ala Val Lys Phe Ala Lys Ala Phe Gly Ala
        195                 200                 205
Glu Val Thr Val Ile Ser Thr Ser Ser Ala Lys Lys Gln Glu Ala Leu
    210                 215                 220
Gln Gly Leu Lys Ala Asp His Phe Ile Val Ser Lys Asp Pro Glu Gln
225                 230                 235                 240
Met Gln Cys Ala Ala Ser Ser Leu Asp Gly Ile Ile Asp Thr Val Ser
                245                 250                 255
Ala Ser His Pro Ile Glu Pro Leu Leu Ser Val Leu Lys Gln Asp Gly
            260                 265                 270
Lys Leu Val Phe Leu Gly Val Pro Glu Lys Pro Ile Glu Ile Ser Thr
        275                 280                 285
Tyr Ser Leu Ile Met Gly Arg Lys Ile Val Thr Gly Ser Ala Thr Gly
    290                 295                 300
Gly Leu Lys Glu Thr Gln Glu Met Leu Asp Phe Ala Ala Lys His Gly
305                 310                 315                 320
Val Thr Ala Asp Ile Glu Leu Ile Pro Ile Asp Tyr Val Asn Thr Ala
                325                 330                 335
Met Asp Arg Val Leu Lys Ser Asp Val Arg Tyr Arg Phe Val Ile Asp
            340                 345                 350
Val Ala Asn Thr Leu Lys Ala Pro
        355                 360

<210> 196
<211> 1062
<212> DNA
<213> Brassica napus

<220>
<221> CDS
<222> (1)..(1062)

<400> 196
atg gga aag gag gca ttc gga ttg gcc gca aaa gac aat tct gga gtt      48
Met Gly Lys Glu Ala Phe Gly Leu Ala Ala Lys Asp Asn Ser Gly Val
1               5                   10                  15
ctc tcg act ttc cat ttc tct aga agg gag aca gga gag aag gat gtt      96
Leu Ser Thr Phe His Phe Ser Arg Arg Glu Thr Gly Glu Lys Asp Val
            20                  25                  30
agg ttc aaa gtt ttg ttc tgt gga atc tgt cac tct gat ttg cat atg      144
Arg Phe Lys Val Leu Phe Cys Gly Ile Cys His Ser Asp Leu His Met
        35                  40                  45
atc aag aat gag tgg gga ata tct act tac cct ctc gtc ccc ggg cat      192
Ile Lys Asn Glu Trp Gly Ile Ser Thr Tyr Pro Leu Val Pro Gly His
    50                  55                  60
gag att gtt ggt gtg gtg act gaa gtg gga agc aaa gtg act aaa ttc      240
Glu Ile Val Gly Val Val Thr Glu Val Gly Ser Lys Val Thr Lys Phe

65                  70                  75                  80
aaa act gga gac aaa gtc gga gta ggt tgc atg gtt ggc tcg tgc gga      288
Lys Thr Gly Asp Lys Val Gly Val Gly Cys Met Val Gly Ser Cys Gly
                85                  90                  95
tca tgt gat agc tgc acc gaa ggt ctc gag aac tac tgt cca aaa tcg      336
Ser Cys Asp Ser Cys Thr Glu Gly Leu Glu Asn Tyr Cys Pro Lys Ser
            100                 105                 110
atc caa acg tac gga ttc aag tat tac gac gac aca ata acc tac ggt      384
Ile Gln Thr Tyr Gly Phe Lys Tyr Tyr Asp Asp Thr Ile Thr Tyr Gly
        115                 120                 125
ggt tac tcc gac aac atg gtt tgc gac gaa ggt ttc atc atc cgt atg      432
Gly Tyr Ser Asp Asn Met Val Cys Asp Glu Gly Phe Ile Ile Arg Met
    130                 135                 140
ccc gaa aat ctc cca ttg gac gct gct gct ccg cta cta tgc gcg ggg      480
Pro Glu Asn Leu Pro Leu Asp Ala Ala Ala Pro Leu Leu Cys Ala Gly
145                 150                 155                 160
atc acg gtc tac tcc ccg cta aaa cat cac ggg ctc gac aag cct ggc      528
Ile Thr Val Tyr Ser Pro Leu Lys His His Gly Leu Asp Lys Pro Gly
                165                 170                 175
atg cac att ggt gtg gtt gga cta ggc ggt ttg ggt cac gtg gcg gtg      576
Met His Ile Gly Val Val Gly Leu Gly Gly Leu Gly His Val Ala Val
            180                 185                 190
aaa ttt gct aag gct atg ggt ctt aag gtt acg gtt att agt act tcg      624
Lys Phe Ala Lys Ala Met Gly Leu Lys Val Thr Val Ile Ser Thr Ser
        195                 200                 205
gat agt aag aga gac gaa gcg att aat agg ctt ggt gcg gac gcg ttc      672
Asp Ser Lys Arg Asp Glu Ala Ile Asn Arg Leu Gly Ala Asp Ala Phe
    210                 215                 220
gtg gtg agc cgt gac caa aaa cag att aag gat gca atg agg aca ttg      720
Val Val Ser Arg Asp Gln Lys Gln Ile Lys Asp Ala Met Arg Thr Leu
225                 230                 235                 240
gat ggt att att aat acc gta tct gcg act cat tcc ctt ctt cct ttg      768
Asp Gly Ile Ile Asn Thr Val Ser Ala Thr His Ser Leu Leu Pro Leu
                245                 250                 255
ctc ggt ttg cta aag cct aag gga aaa ctt gtt atg gtt ggt gca cca      816
Leu Gly Leu Leu Lys Pro Lys Gly Lys Leu Val Met Val Gly Ala Pro
            260                 265                 270
gaa aag cca ctc gag ctc ccg gtc atg cct ctc atc ttc gag agg aag      864
Glu Lys Pro Leu Glu Leu Pro Val Met Pro Leu Ile Phe Glu Arg Lys
        275                 280                 285
atg gta atg gga agt atg ata gga ggg ata aag gag act caa gag atg      912
Met Val Met Gly Ser Met Ile Gly Gly Ile Lys Glu Thr Gln Glu Met
    290                 295                 300
gtg gat atg gca gga aaa cac aac atc act gct gat att gag ctt atc      960
Val Asp Met Ala Gly Lys His Asn Ile Thr Ala Asp Ile Glu Leu Ile
305                 310                 315                 320
tct gcg gac tat gtc aac act gcc atg gaa cgg ctc gag aag gcc gac      1008
Ser Ala Asp Tyr Val Asn Thr Ala Met Glu Arg Leu Glu Lys Ala Asp
                325                 330                 335
gtt aga tac cgt ttt gtg att gat gtt gcc aac aca ttg aag cct act      1056
Val Arg Tyr Arg Phe Val Ile Asp Val Ala Asn Thr Leu Lys Pro Thr
            340                 345                 350
cct taa                                                              1062
Pro


<210> 197
<211> 353
<212> PRT
<213> Brassica napus

<400> 197
Met Gly Lys Glu Ala Phe Gly Leu Ala Ala Lys Asp Asn Ser Gly Val
1               5                   10                  15
Leu Ser Thr Phe His Phe Ser Arg Arg Glu Thr Gly Glu Lys Asp Val
            20                  25                  30
Arg Phe Lys Val Leu Phe Cys Gly Ile Cys His Ser Asp Leu His Met
        35                  40                  45
Ile Lys Asn Glu Trp Gly Ile Ser Thr Tyr Pro Leu Val Pro Gly His

    50                  55                  60
Glu Ile Val Gly Val Val Thr Glu Val Gly Ser Lys Val Thr Lys Phe
65                  70                  75                  80
Lys Thr Gly Asp Lys Val Gly Val Gly Cys Met Val Gly Ser Cys Gly
                85                  90                  95
Ser Cys Asp Ser Cys Thr Glu Gly Leu Glu Asn Tyr Cys Pro Lys Ser
            100                 105                 110
Ile Gln Thr Tyr Gly Phe Lys Tyr Tyr Asp Asp Thr Ile Thr Tyr Gly
        115                 120                 125
Gly Tyr Ser Asp Asn Met Val Cys Asp Glu Gly Phe Ile Ile Arg Met
    130                 135                 140
Pro Glu Asn Leu Pro Leu Asp Ala Ala Ala Pro Leu Leu Cys Ala Gly
145                 150                 155                 160
Ile Thr Val Tyr Ser Pro Leu Lys His His Gly Leu Asp Lys Pro Gly
                165                 170                 175
Met His Ile Gly Val Val Gly Leu Gly Gly Leu Gly His Val Ala Val
            180                 185                 190
Lys Phe Ala Lys Ala Met Gly Leu Lys Val Thr Val Ile Ser Thr Ser
        195                 200                 205
Asp Ser Lys Arg Asp Glu Ala Ile Asn Arg Leu Gly Ala Asp Ala Phe
    210                 215                 220
Val Val Ser Arg Asp Gln Lys Gln Ile Lys Asp Ala Met Arg Thr Leu
225                 230                 235                 240
Asp Gly Ile Ile Asn Thr Val Ser Ala Thr His Ser Leu Leu Pro Leu
                245                 250                 255
Leu Gly Leu Leu Lys Pro Lys Gly Lys Leu Val Met Val Gly Ala Pro
            260                 265                 270
Glu Lys Pro Leu Glu Leu Pro Val Met Pro Leu Ile Phe Glu Arg Lys
        275                 280                 285
Met Val Met Gly Ser Met Ile Gly Gly Ile Lys Glu Thr Gln Glu Met
    290                 295                 300
Val Asp Met Ala Gly Lys His Asn Ile Thr Ala Asp Ile Glu Leu Ile
305                 310                 315                 320
Ser Ala Asp Tyr Val Asn Thr Ala Met Glu Arg Leu Glu Lys Ala Asp
                325                 330                 335
Val Arg Tyr Arg Phe Val Ile Asp Val Ala Asn Thr Leu Lys Pro Thr
            340                 345                 350
Pro


<210> 198
<211> 1074
<212> DNA
<213> Brassica napus

<220>
<221> CDS
<222> (1)..(1074)

<400> 198
atg ggg aaa atg gag gta gag agg aaa aca aca ggc tgg gct gcg aga      48
Met Gly Lys Met Glu Val Glu Arg Lys Thr Thr Gly Trp Ala Ala Arg
1               5                   10                  15
gac cct tct ggg tta ctc tct cct tac act tac act ctc aga gag aca      96
Asp Pro Ser Gly Leu Leu Ser Pro Tyr Thr Tyr Thr Leu Arg Glu Thr
            20                  25                  30
gga cca gag gat gtt cac ata aga atc ata tgc tgt gga atc tgc cac      144
Gly Pro Glu Asp Val His Ile Arg Ile Ile Cys Cys Gly Ile Cys His
        35                  40                  45
aca gat ctc cat caa acc aaa aac gat ctt ggc atg tca aac tac ccc      192
Thr Asp Leu His Gln Thr Lys Asn Asp Leu Gly Met Ser Asn Tyr Pro
    50                  55                  60
atg gtt cct ggg cac gag gtt gtt ggg gaa gtg gtg gag gtg gga tca      240
Met Val Pro Gly His Glu Val Val Gly Glu Val Val Glu Val Gly Ser
65                  70                  75                  80
gat gtg agc aag ttc acc gca ggg gat ata gtt gga gtt ggt tgt ctc      288
Asp Val Ser Lys Phe Thr Ala Gly Asp Ile Val Gly Val Gly Cys Leu
                85                  90                  95
gtt gga tgc tgc gga ggt tgt agc ccc tgc gag aga gat ctg gaa cag      336

Val Gly Cys Cys Gly Gly Cys Ser Pro Cys Glu Arg Asp Leu Glu Gln
            100                 105                 110
tat tgt ccc aag aaa ata tgg agc tac aac gat gtt tac atc gat ggt      384
Tyr Cys Pro Lys Lys Ile Trp Ser Tyr Asn Asp Val Tyr Ile Asp Gly
        115                 120                 125
caa cca aca caa ggc ggt ttc gct aga gcc acc gtc gtt cac caa aag      432
Gln Pro Thr Gln Gly Gly Phe Ala Arg Ala Thr Val Val His Gln Lys
    130                 135                 140
ttt gtg gtg aag att cca gaa gga atg gct gtt gag cag gcg gca cca      480
Phe Val Val Lys Ile Pro Glu Gly Met Ala Val Glu Gln Ala Ala Pro
145                 150                 155                 160
cta ctg tgt gcc ggt gtg aca gtg tac agt cca ctg gct cac ttc ggt      528
Leu Leu Cys Ala Gly Val Thr Val Tyr Ser Pro Leu Ala His Phe Gly
                165                 170                 175
ctg aag cga cca ggc cta aga gga ggt ata cta ggg cta ggt gga gtt      576
Leu Lys Arg Pro Gly Leu Arg Gly Gly Ile Leu Gly Leu Gly Gly Val
            180                 185                 190
ggt cac atg ggt gtg aaa ata gcc aaa gca atg ggt cac cat gtg act      624
Gly His Met Gly Val Lys Ile Ala Lys Ala Met Gly His His Val Thr
        195                 200                 205
gtc ata agc tca tca aac aag aag aaa gaa gag gct ttg aaa gat ctt      672
Val Ile Ser Ser Ser Asn Lys Lys Lys Glu Glu Ala Leu Lys Asp Leu
    210                 215                 220
gga gca gat gat tac gtg atc gga tcc gac caa tcg aag atg aac gaa      720
Gly Ala Asp Asp Tyr Val Ile Gly Ser Asp Gln Ser Lys Met Asn Glu
225                 230                 235                 240
ttg gcg gat tca atg gat tac ata atc gac acg gtc cct gtt cat cat      768
Leu Ala Asp Ser Met Asp Tyr Ile Ile Asp Thr Val Pro Val His His
                245                 250                 255
gca ctt gag cct tac ttg tct ctg ctt agg ctt gat ggg aaa ctc ata      816
Ala Leu Glu Pro Tyr Leu Ser Leu Leu Arg Leu Asp Gly Lys Leu Ile
            260                 265                 270
ctc atg gga gtc ata agc aat cca tta caa ttt ctc act cct atg gtt      864
Leu Met Gly Val Ile Ser Asn Pro Leu Gln Phe Leu Thr Pro Met Val
        275                 280                 285
atg ctt ggg agg aaa gtg ata acg ggg agc ttc att ggg agc atg aag      912
Met Leu Gly Arg Lys Val Ile Thr Gly Ser Phe Ile Gly Ser Met Lys
    290                 295                 300
gaa aca gag gag atg ctt gag ttc tgt aaa gaa aag ggt ttg agt tcg      960
Glu Thr Glu Glu Met Leu Glu Phe Cys Lys Glu Lys Gly Leu Ser Ser
305                 310                 315                 320
atc atc gaa gta gtg aag atg gat tat gtg aac act gcg ttt gag aga      1008
Ile Ile Glu Val Val Lys Met Asp Tyr Val Asn Thr Ala Phe Glu Arg
                325                 330                 335
ctc gag aag aac gat gtg cgt tat agg ttc gtg gtt gat gtc gaa gga      1056
Leu Glu Lys Asn Asp Val Arg Tyr Arg Phe Val Val Asp Val Glu Gly
            340                 345                 350
agc aaa ctt gag gct tga                                              1074
Ser Lys Leu Glu Ala
        355

<210> 199
<211> 357
<212> PRT
<213> Brassica napus

<400> 199
   Met Gly Lys Met Glu Val Glu Arg Lys Thr Thr Gly Trp Ala Ala Arg
   1               5                   10                  15
   Asp Pro Ser Gly Leu Leu Ser Pro Tyr Thr Tyr Thr Leu Arg Glu Thr
               20                  25                  30
   Gly Pro Glu Asp Val His Ile Arg Ile Ile Cys Cys Gly Ile Cys His
           35                  40                  45
   Thr Asp Leu His Gln Thr Lys Asn Asp Leu Gly Met Ser Asn Tyr Pro
       50                  55                  60
   Met Val Pro Gly His Glu Val Val Gly Glu Val Val Glu Val Gly Ser
   65                  70                  75                  80
   Asp Val Ser Lys Phe Thr Ala Gly Asp Ile Val Gly Val Gly Cys Leu
                   85                  90                  95
   Val Gly Cys Cys Gly Gly Cys Ser Pro Cys Glu Arg Asp Leu Glu Gln
               100                 105                 110
   Tyr Cys Pro Lys Lys Ile Trp Ser Tyr Asn Asp Val Tyr Ile Asp Gly
           115                 120                 125
   Gln Pro Thr Gln Gly Gly Phe Ala Arg Ala Thr Val Val His Gln Lys
       130                 135                 140
   Phe Val Val Lys Ile Pro Glu Gly Met Ala Val Glu Gln Ala Ala Pro
   145                 150                 155                 160
   Leu Leu Cys Ala Gly Val Thr Val Tyr Ser Pro Leu Ala His Phe Gly
                   165                 170                 175
   Leu Lys Arg Pro Gly Leu Arg Gly Gly Ile Leu Gly Leu Gly Gly Val
               180                 185                 190
   Gly His Met Gly Val Lys Ile Ala Lys Ala Met Gly His His Val Thr
           195                 200                 205
   Val Ile Ser Ser Ser Asn Lys Lys Lys Glu Glu Ala Leu Lys Asp Leu
       210                 215                 220
   Gly Ala Asp Asp Tyr Val Ile Gly Ser Asp Gln Ser Lys Met Asn Glu
   225                 230                 235                 240
   Leu Ala Asp Ser Met Asp Tyr Ile Ile Asp Thr Val Pro Val His His
                   245                 250                 255
   Ala Leu Glu Pro Tyr Leu Ser Leu Leu Arg Leu Asp Gly Lys Leu Ile
               260                 265                 270
   Leu Met Gly Val Ile Ser Asn Pro Leu Gln Phe Leu Thr Pro Met Val
           275                 280                 285
   Met Leu Gly Arg Lys Val Ile Thr Gly Ser Phe Ile Gly Ser Met Lys
       290                 295                 300
   Glu Thr Glu Glu Met Leu Glu Phe Cys Lys Glu Lys Gly Leu Ser Ser
   305                 310                 315                 320
   Ile Ile Glu Val Val Lys Met Asp Tyr Val Asn Thr Ala Phe Glu Arg
                   325                 330                 335
   Leu Glu Lys Asn Asp Val Arg Tyr Arg Phe Val Val Asp Val Glu Gly
               340                 345                 350
   Ser Lys Leu Glu Ala
           355

<210> 200
<211> 1074
<212> DNA
<213> Brassica napus

<220>
<221> CDS
<222> (1)..(1074)

<400> 200
atg gga aag gtg cct gcg acg gag gcg ttc gga ttg gcc gcg aaa gac      48
Met Gly Lys Val Pro Ala Thr Glu Ala Phe Gly Leu Ala Ala Lys Asp
1               5                   10                  15
gaa tcc gga gtt ctc tcg cct ttc cgt ttc tca aga agg gag acg gga      96
Glu Ser Gly Val Leu Ser Pro Phe Arg Phe Ser Arg Arg Glu Thr Gly
            20                  25                  30
gag aag gat gtg agg tta aaa gtg ttg ttc tgt gga att tgc cac act      144
Glu Lys Asp Val Arg Leu Lys Val Leu Phe Cys Gly Ile Cys His Thr
        35                  40                  45
gat gta tgt atg gcc aga aac gag tgg gga ttt act act tac cct ctc      192
Asp Val Cys Met Ala Arg Asn Glu Trp Gly Phe Thr Thr Tyr Pro Leu
    50                  55                  60
gtc ccc ggg cat gag att gtt ggc gtg gtg act gaa gtc gga gct aaa      240
Val Pro Gly His Glu Ile Val Gly Val Val Thr Glu Val Gly Ala Lys
65                  70                  75                  80
gtg att aaa ttc aaa gcc gga gac aaa gtc gga gtt ggt tat atg ctc      288
Val Ile Lys Phe Lys Ala Gly Asp Lys Val Gly Val Gly Tyr Met Leu
                85                  90                  95
agc tcg tgc cgg tca tgt gac atc tgc acc gat gac caa gag aac cac      336
Ser Ser Cys Arg Ser Cys Asp Ile Cys Thr Asp Asp Gln Glu Asn His
            100                 105                 110
tgt cca aaa atg att atg acg tcc gga gga aag tac tac gat gac acc      384
Cys Pro Lys Met Ile Met Thr Ser Gly Gly Lys Tyr Tyr Asp Asp Thr
        115                 120                 125

atg act tat ggt ggt tac tct gac cac ctg gtt tgt gaa gag gat tac     432
Met Thr Tyr Gly Gly Tyr Ser Asp His Leu Val Cys Glu Glu Asp Tyr
    130                 135                 140
atc atc cgt att cct gaa aat ctc ccg tta gac gct gcc gcg ccg cta     480
Ile Ile Arg Ile Pro Glu Asn Leu Pro Leu Asp Ala Ala Ala Pro Leu
145                 150                 155                 160
ctc tgc gct ggg gtc acg gtt tat tca ccg ttg aag tct cac gga ctc     528
Leu Cys Ala Gly Val Thr Val Tyr Ser Pro Leu Lys Ser His Gly Leu
                165                 170                 175
gac aag ccc gga atc cac att ggt gtg gtg gga cta ggc ggt tta ggt     576
Asp Lys Pro Gly Ile His Ile Gly Val Val Gly Leu Gly Gly Leu Gly
            180                 185                 190
cac gta gca gtg aaa ttt gcc aag gct atg ggt act aag gtt acg gtt     624
His Val Ala Val Lys Phe Ala Lys Ala Met Gly Thr Lys Val Thr Val
        195                 200                 205
att agt tcg tca gag agt aag aga gac gag gcg att aat cgg ctt ggt     672
Ile Ser Ser Ser Glu Ser Lys Arg Asp Glu Ala Ile Asn Arg Leu Gly
    210                 215                 220
gcg gat ctg ttc tta gtg agc cgt gac ccg gaa gag atg aag gat gca     720
Ala Asp Leu Phe Leu Val Ser Arg Asp Pro Glu Glu Met Lys Asp Ala
225                 230                 235                 240
atg ggg act atg gat ggt att att gat aca gta tct gcc act cat ccg     768
Met Gly Thr Met Asp Gly Ile Ile Asp Thr Val Ser Ala Thr His Pro
                245                 250                 255
ctt ctt ccg ctt ctt ggt ttg cta aaa tat aag gga aaa tta att atg     816
Leu Leu Pro Leu Leu Gly Leu Leu Lys Tyr Lys Gly Lys Leu Ile Met
            260                 265                 270
gtt ggt gca ccc gat aaa cca ctt gac ctt cca gct tta ccg ctc atc     864
Val Gly Ala Pro Asp Lys Pro Leu Asp Leu Pro Ala Leu Pro Leu Ile
        275                 280                 285
tta ggg aag aag atg gtg gtg gga agt atg aca gga ggg ata aaa gag     912
Leu Gly Lys Lys Met Val Val Gly Ser Met Thr Gly Gly Ile Lys Glu
    290                 295                 300
tct caa gag atg gtt gat ttt gcc ggt aaa cac aac ata acg gca gat     960
Ser Gln Glu Met Val Asp Phe Ala Gly Lys His Asn Ile Thr Ala Asp
305                 310                 315                 320
att gag ctt atc tct gcg gat tat gtc aac atg gct atg gaa cgg cta     1008
Ile Glu Leu Ile Ser Ala Asp Tyr Val Asn Met Ala Met Glu Arg Leu
                325                 330                 335
gaa aag ggg gat gtt aga tac cgt ttt gtg att gat gtt gcc aac aca     1056
Glu Lys Gly Asp Val Arg Tyr Arg Phe Val Ile Asp Val Ala Asn Thr
            340                 345                 350
atg aag cct act cct tag                                             1074
Met Lys Pro Thr Pro
        355

<210> 201
<211> 357
<212> PRT
<213> Brassica napus

<400> 201
Met Gly Lys Val Pro Ala Thr Glu Ala Phe Gly Leu Ala Ala Lys Asp
1               5                   10                  15
Glu Ser Gly Val Leu Ser Pro Phe Arg Phe Ser Arg Arg Glu Thr Gly
            20                  25                  30
Glu Lys Asp Val Arg Leu Lys Val Leu Phe Cys Gly Ile Cys His Thr
        35                  40                  45
Asp Val Cys Met Ala Arg Asn Glu Trp Gly Phe Thr Thr Tyr Pro Leu
    50                  55                  60
Val Pro Gly His Glu Ile Val Gly Val Val Thr Glu Val Gly Ala Lys
65                  70                  75                  80
Val Ile Lys Phe Lys Ala Gly Asp Lys Val Gly Val Gly Tyr Met Leu
                85                  90                  95
Ser Ser Cys Arg Ser Cys Asp Ile Cys Thr Asp Asp Gln Glu Asn His
            100                 105                 110
Cys Pro Lys Met Ile Met Thr Ser Gly Gly Lys Tyr Tyr Asp Asp Thr
        115                 120                 125
Met Thr Tyr Gly Gly Tyr Ser Asp His Leu Val Cys Glu Glu Asp Tyr

130                 135                 140
Ile Ile Arg Ile Pro Glu Asn Leu Pro Leu Asp Ala Ala Ala Pro Leu
145                 150                 155                 160
Leu Cys Ala Gly Val Thr Val Tyr Ser Pro Leu Lys Ser His Gly Leu
                165                 170                 175
Asp Lys Pro Gly Ile His Ile Gly Val Val Gly Leu Gly Gly Leu Gly
            180                 185                 190
His Val Ala Val Lys Phe Ala Lys Ala Met Gly Thr Lys Val Thr Val
        195                 200                 205
Ile Ser Ser Ser Glu Ser Lys Arg Asp Glu Ala Ile Asn Arg Leu Gly
    210                 215                 220
Ala Asp Leu Phe Leu Val Ser Arg Asp Pro Glu Glu Met Lys Asp Ala
225                 230                 235                 240
Met Gly Thr Met Asp Gly Ile Ile Asp Thr Val Ser Ala Thr His Pro
                245                 250                 255
Leu Leu Pro Leu Leu Gly Leu Leu Lys Tyr Lys Gly Lys Leu Ile Met
            260                 265                 270
Val Gly Ala Pro Asp Lys Pro Leu Asp Leu Pro Ala Leu Pro Leu Ile
        275                 280                 285
Leu Gly Lys Lys Met Val Val Gly Ser Met Thr Gly Gly Ile Lys Glu
    290                 295                 300
Ser Gln Glu Met Val Asp Phe Ala Gly Lys His Asn Ile Thr Ala Asp
305                 310                 315                 320
Ile Glu Leu Ile Ser Ala Asp Tyr Val Asn Met Ala Met Glu Arg Leu
                325                 330                 335
Glu Lys Gly Asp Val Arg Tyr Arg Phe Val Ile Asp Val Ala Asn Thr
            340                 345                 350
Met Lys Pro Thr Pro
        355

<210> 202
<211> 1074
<212> DNA
<213> Brassica napus

<220>
<221> CDS
<222> (1)..(1074)

<400> 202
atg gga gag gtc ctt aag aag gag gcg tac gga ctg gct gta aaa gac      48
Met Gly Glu Val Leu Lys Lys Glu Ala Tyr Gly Leu Ala Val Lys Asp
1               5                   10                  15
gaa tct gga gtt atc tcg cct ttc cgt ttc tca aga agg gag aca gga      96
Glu Ser Gly Val Ile Ser Pro Phe Arg Phe Ser Arg Arg Glu Thr Gly
            20                  25                  30
gaa aat gat gtg agg tta aaa gtg ttg ttc tgt gga att tgc cac aca      144
Glu Asn Asp Val Arg Leu Lys Val Leu Phe Cys Gly Ile Cys His Thr
        35                  40                  45
gat tta agc atg gcc ata aac gag tgg ggg ttt act agt tac ccc ctt      192
Asp Leu Ser Met Ala Ile Asn Glu Trp Gly Phe Thr Ser Tyr Pro Leu
    50                  55                  60
gtc ccc ggg cat gaa ata gtg ggc gtg gtg act gaa gtc gga gcc aaa      240
Val Pro Gly His Glu Ile Val Gly Val Val Thr Glu Val Gly Ala Lys
65                  70                  75                  80
gtg act aaa ttc aac gcc gga gac aaa gtg gga gtt ggt tat atg gtc      288
Val Thr Lys Phe Asn Ala Gly Asp Lys Val Gly Val Gly Tyr Met Val
                85                  90                  95
agc tca tgc ggg tca tgt gaa acc tgc att gat gac caa gag aac tac      336
Ser Ser Cys Gly Ser Cys Glu Thr Cys Ile Asp Asp Gln Glu Asn Tyr
            100                 105                 110
tgt cca aaa atg atc cta acg tcc gga ggc aag tat tac gat gac act      384
Cys Pro Lys Met Ile Leu Thr Ser Gly Gly Lys Tyr Tyr Asp Asp Thr
        115                 120                 125
ata aca tat ggt ggt tac tcc gac cat atg gtt tgt gaa gag gac tac      432
Ile Thr Tyr Gly Gly Tyr Ser Asp His Met Val Cys Glu Glu Asp Tyr
    130                 135                 140
atc atc cgt att cca gaa aat ctt ccc tta gac gct acc gcg ccg cta      480
Ile Ile Arg Ile Pro Glu Asn Leu Pro Leu Asp Ala Thr Ala Pro Leu

145                 150                 155                 160
ctc tgc gct ggg acc acc gtc tat tcc ccg atg aag tat cac ggg ctc       528
Leu Cys Ala Gly Thr Thr Val Tyr Ser Pro Met Lys Tyr His Gly Leu
                165                 170                 175
gac aag ccg ggt atg cac att ggt gtg gtg gga cta ggc ggt tta ggt       576
Asp Lys Pro Gly Met His Ile Gly Val Val Gly Leu Gly Gly Leu Gly
            180                 185                 190
cat gta gct gtg aaa ttt gct aag gct atg ggt att aag gtt aca gtt       624
His Val Ala Val Lys Phe Ala Lys Ala Met Gly Ile Lys Val Thr Val
        195                 200                 205
att agt act tcg gat agg aag aga gac gag gcg tta act cgg ctt ggt       672
Ile Ser Thr Ser Asp Arg Lys Arg Asp Glu Ala Leu Thr Arg Leu Gly
    210                 215                 220
gcg gat ctg ttc ttg gtg agc cgt gac ccg gaa cag atg aag gat gca       720
Ala Asp Leu Phe Leu Val Ser Arg Asp Pro Glu Gln Met Lys Asp Ala
225                 230                 235                 240
atg ggt act atg gat ggt att att gat acc gta tct gct act cat cca       768
Met Gly Thr Met Asp Gly Ile Ile Asp Thr Val Ser Ala Thr His Pro
                245                 250                 255
gtt ctt ccg att ctt gat ttg ctt aaa tat aag ggt aaa ctt att atg       816
Val Leu Pro Ile Leu Asp Leu Leu Lys Tyr Lys Gly Lys Leu Ile Met
            260                 265                 270
gtt ggt gca cct gat aaa cca ctt gag ctt ccg gtt ctg cct ctc atc       864
Val Gly Ala Pro Asp Lys Pro Leu Glu Leu Pro Val Leu Pro Leu Ile
        275                 280                 285
ttt ggg aaa aag atg gtg gtg gga agt atg gta gga ggg ata aaa gag       912
Phe Gly Lys Lys Met Val Val Gly Ser Met Val Gly Gly Ile Lys Glu
    290                 295                 300
act caa gag atg atg gat ttg gcc gga aaa cac aac atc acg gca gat       960
Thr Gln Glu Met Met Asp Leu Ala Gly Lys His Asn Ile Thr Ala Asp
305                 310                 315                 320
att gag ctt atc tct gcg gat tat gtc aac act gcc atg gaa cgg ctc       1008
Ile Glu Leu Ile Ser Ala Asp Tyr Val Asn Thr Ala Met Glu Arg Leu
                325                 330                 335
cag aag gct gac gtt aga tac cgt ttt gtg att gat gtt gcc aac acc       1056
Gln Lys Ala Asp Val Arg Tyr Arg Phe Val Ile Asp Val Ala Asn Thr
            340                 345                 350
ttg aag cct agt cct taa                                               1074
Leu Lys Pro Ser Pro
        355

<210> 203
<211> 357
<212> PRT
<213> Brassica napus

<400> 203
Met Gly Glu Val Leu Lys Lys Glu Ala Tyr Gly Leu Ala Val Lys Asp
1               5                   10                  15
Glu Ser Gly Val Ile Ser Pro Phe Arg Phe Ser Arg Arg Glu Thr Gly
            20                  25                  30
Glu Asn Asp Val Arg Leu Lys Val Leu Phe Cys Gly Ile Cys His Thr
        35                  40                  45
Asp Leu Ser Met Ala Ile Asn Glu Trp Gly Phe Thr Ser Tyr Pro Leu
    50                  55                  60
Val Pro Gly His Glu Ile Val Gly Val Val Thr Glu Val Gly Ala Lys
65                  70                  75                  80
Val Thr Lys Phe Asn Ala Gly Asp Lys Val Gly Val Gly Tyr Met Val
                85                  90                  95
Ser Ser Cys Gly Ser Cys Glu Thr Cys Ile Asp Asp Gln Glu Asn Tyr
            100                 105                 110
Cys Pro Lys Met Ile Leu Thr Ser Gly Gly Lys Tyr Tyr Asp Asp Thr
        115                 120                 125
Ile Thr Tyr Gly Gly Tyr Ser Asp His Met Val Cys Glu Glu Asp Tyr
    130                 135                 140
Ile Ile Arg Ile Pro Glu Asn Leu Pro Leu Asp Ala Thr Ala Pro Leu
145                 150                 155                 160
Leu Cys Ala Gly Thr Thr Val Tyr Ser Pro Met Lys Tyr His Gly Leu
                165                 170                 175

Asp Lys Pro Gly Met His Ile Gly Val Val Gly Leu Gly Gly Leu Gly
            180                 185                 190
His Val Ala Val Lys Phe Ala Lys Ala Met Gly Ile Lys Val Thr Val
        195                 200                 205
Ile Ser Thr Ser Asp Arg Lys Arg Asp Glu Ala Leu Thr Arg Leu Gly
    210                 215                 220
Ala Asp Leu Phe Leu Val Ser Arg Asp Pro Glu Gln Met Lys Asp Ala
225                 230                 235                 240
Met Gly Thr Met Asp Gly Ile Ile Asp Thr Val Ser Ala Thr His Pro
                245                 250                 255
Val Leu Pro Ile Leu Asp Leu Leu Lys Tyr Lys Gly Lys Leu Ile Met
            260                 265                 270
Val Gly Ala Pro Asp Lys Pro Leu Glu Leu Pro Val Leu Pro Leu Ile
        275                 280                 285
Phe Gly Lys Lys Met Val Val Gly Ser Met Val Gly Gly Ile Lys Glu
    290                 295                 300
Thr Gln Glu Met Met Asp Leu Ala Gly Lys His Asn Ile Thr Ala Asp
305                 310                 315                 320
Ile Glu Leu Ile Ser Ala Asp Tyr Val Asn Thr Ala Met Glu Arg Leu
                325                 330                 335
Gln Lys Ala Asp Val Arg Tyr Arg Phe Val Ile Asp Val Ala Asn Thr
            340                 345                 350
Leu Lys Pro Ser Pro
        355

<210> 204
<211> 1083
<212> DNA
<213> Brassica napus

<220>
<221> CDS
<222> (1)..(1083)

<400> 204
atg gcg aaa tct cca gag aca gag cat ccg aac aaa gcc ttt ggt tgg      48
Met Ala Lys Ser Pro Glu Thr Glu His Pro Asn Lys Ala Phe Gly Trp
1               5                   10                  15
gct gcc aaa gac aaa tct ggt gta ctc act cct ttc cat ttc tcc aga      96
Ala Ala Lys Asp Lys Ser Gly Val Leu Thr Pro Phe His Phe Ser Arg
            20                  25                  30
aga gac aat ggt gac aat gat gtg aca gtg aag atc ttg tat tgt gga      144
Arg Asp Asn Gly Asp Asn Asp Val Thr Val Lys Ile Leu Tyr Cys Gly
        35                  40                  45
gtg tgc cac act gat tta cat acc atc aaa aac gac tgg ggc ttc tcg      192
Val Cys His Thr Asp Leu His Thr Ile Lys Asn Asp Trp Gly Phe Ser
    50                  55                  60
tat tat cct gta gtt cca ggg cat gaa ata gtt gga ata gct aca aaa      240
Tyr Tyr Pro Val Val Pro Gly His Glu Ile Val Gly Ile Ala Thr Lys
65                  70                  75                  80
gtt ggc aaa aac gtg act aaa ttc aga gaa gga gac cgt gta gga gta      288
Val Gly Lys Asn Val Thr Lys Phe Arg Glu Gly Asp Arg Val Gly Val
                85                  90                  95
gga gtg atc act ggt tct tgc caa tct tgc gaa tct tgt aac caa gat      336
Gly Val Ile Thr Gly Ser Cys Gln Ser Cys Glu Ser Cys Asn Gln Asp
            100                 105                 110
ctt gaa aac tac tgt ccc caa atg tct ttc acc tac aac tcc ata gga      384
Leu Glu Asn Tyr Cys Pro Gln Met Ser Phe Thr Tyr Asn Ser Ile Gly
        115                 120                 125
tcc gat gga acc aag acc tac ggt gga tat tcg gag tcc att gtg gtc      432
Ser Asp Gly Thr Lys Thr Tyr Gly Gly Tyr Ser Glu Ser Ile Val Val
    130                 135                 140
gat caa cgt ttt gtc ttg cag ttt ccc gag gga tta cct agc gat tcg      480
Asp Gln Arg Phe Val Leu Gln Phe Pro Glu Gly Leu Pro Ser Asp Ser
145                 150                 155                 160
ggt gcc ccc ttg ctg tgt gct gga atc act gtg tat agt cca atg aag      528
Gly Ala Pro Leu Leu Cys Ala Gly Ile Thr Val Tyr Ser Pro Met Lys
                165                 170                 175
tat tat ggt atg acc gag cct ggg aag cat ttg ggt gtg gct gga ctt      576

Tyr Tyr Gly Met Thr Glu Pro Gly Lys His Leu Gly Val Ala Gly Leu
            180                 185                 190
ggt ggg ctt ggt cat gtt gct gtc aag att ggt aaa gcc ttt ggt ttg         624
Gly Gly Leu Gly His Val Ala Val Lys Ile Gly Lys Ala Phe Gly Leu
        195                 200                 205
aaa gtc act gtg att agt tct tct cct agt aaa gaa gac gaa gct att         672
Lys Val Thr Val Ile Ser Ser Ser Pro Ser Lys Glu Asp Glu Ala Ile
    210                 215                 220
aat cga ctt ggt gct gat tcg ttt ctt gtc tca tct gat cct cag aag         720
Asn Arg Leu Gly Ala Asp Ser Phe Leu Val Ser Ser Asp Pro Gln Lys
225                 230                 235                 240
atg aag gct gca att gga act atg gac tat att atc gat acg gta tca         768
Met Lys Ala Ala Ile Gly Thr Met Asp Tyr Ile Ile Asp Thr Val Ser
                245                 250                 255
gca gta cat gct ctg ttt cca ttg ctt ggt tta ctc aaa gtc aat gga         816
Ala Val His Ala Leu Phe Pro Leu Leu Gly Leu Leu Lys Val Asn Gly
            260                 265                 270
aag ctt gtc acg tta ggc tta cct gag aag cct ctt gag cta cca atc         864
Lys Leu Val Thr Leu Gly Leu Pro Glu Lys Pro Leu Glu Leu Pro Ile
        275                 280                 285
ttc cct ctt gtt ctc gga aga aaa atg gtg gga gga agt gac att gga         912
Phe Pro Leu Val Leu Gly Arg Lys Met Val Gly Gly Ser Asp Ile Gly
    290                 295                 300
ggg atg aag gag aca caa gag atg ctt gaa ttc tgc gct aag cat aac         960
Gly Met Lys Glu Thr Gln Glu Met Leu Glu Phe Cys Ala Lys His Asn
305                 310                 315                 320
ata acg gca gat att gaa ctg atc aag atg gat gag att aac act gcg        1008
Ile Thr Ala Asp Ile Glu Leu Ile Lys Met Asp Glu Ile Asn Thr Ala
                325                 330                 335
atg gag agg ctt gct aag tct gat gtt agg tac agg ttc gtg atc gac        1056
Met Glu Arg Leu Ala Lys Ser Asp Val Arg Tyr Arg Phe Val Ile Asp
            340                 345                 350
gtc gct aac tcc ttg agc cct cca tga                                    1083
Val Ala Asn Ser Leu Ser Pro Pro
        355                 360

<210> 205
<211> 360
<212> PRT
<213> Brassica napus

<400> 205
Met Ala Lys Ser Pro Glu Thr Glu His Pro Asn Lys Ala Phe Gly Trp
1               5                   10                  15
Ala Ala Lys Asp Lys Ser Gly Val Leu Thr Pro Phe His Phe Ser Arg
            20                  25                  30
Arg Asp Asn Gly Asp Asn Asp Val Thr Val Lys Ile Leu Tyr Cys Gly
        35                  40                  45
Val Cys His Thr Asp Leu His Thr Ile Lys Asn Asp Trp Gly Phe Ser
    50                  55                  60
Tyr Tyr Pro Val Val Pro Gly His Glu Ile Val Gly Ile Ala Thr Lys
65                  70                  75                  80
Val Gly Lys Asn Val Thr Lys Phe Arg Glu Gly Asp Arg Val Gly Val
                85                  90                  95
Gly Val Ile Thr Gly Ser Cys Gln Ser Cys Glu Ser Cys Asn Gln Asp
            100                 105                 110
Leu Glu Asn Tyr Cys Pro Gln Met Ser Phe Thr Tyr Asn Ser Ile Gly
        115                 120                 125
Ser Asp Gly Thr Lys Thr Tyr Gly Gly Tyr Ser Glu Ser Ile Val Val
    130                 135                 140
Asp Gln Arg Phe Val Leu Gln Phe Pro Glu Gly Leu Pro Ser Asp Ser
145                 150                 155                 160
Gly Ala Pro Leu Leu Cys Ala Gly Ile Thr Val Tyr Ser Pro Met Lys
                165                 170                 175
Tyr Tyr Gly Met Thr Glu Pro Gly Lys His Leu Gly Val Ala Gly Leu
            180                 185                 190
Gly Gly Leu Gly His Val Ala Val Lys Ile Gly Lys Ala Phe Gly Leu
        195                 200                 205
Lys Val Thr Val Ile Ser Ser Ser Pro Ser Lys Glu Asp Glu Ala Ile

210                 215                 220
Asn Arg Leu Gly Ala Asp Ser Phe Leu Val Ser Ser Asp Pro Gln Lys
225                 230                 235                 240
Met Lys Ala Ala Ile Gly Thr Met Asp Tyr Ile Ile Asp Thr Val Ser
                245                 250                 255
Ala Val His Ala Leu Phe Pro Leu Leu Gly Leu Leu Lys Val Asn Gly
            260                 265                 270
Lys Leu Val Thr Leu Gly Leu Pro Glu Lys Pro Leu Glu Leu Pro Ile
        275                 280                 285
Phe Pro Leu Val Leu Gly Arg Lys Met Val Gly Gly Ser Asp Ile Gly
    290                 295                 300
Gly Met Lys Glu Thr Gln Glu Met Leu Glu Phe Cys Ala Lys His Asn
305                 310                 315                 320
Ile Thr Ala Asp Ile Glu Leu Ile Lys Met Asp Glu Ile Asn Thr Ala
                325                 330                 335
Met Glu Arg Leu Ala Lys Ser Asp Val Arg Tyr Arg Phe Val Ile Asp
            340                 345                 350
Val Ala Asn Ser Leu Ser Pro Pro
        355                 360

<210> 206
<211> 1074
<212> DNA
<213> Brassica napus

<220>
<221> CDS
<222> (1)..(1074)

<400> 206
atg ggg aaa atg gag gta gag agg aaa aca aca ggc tgg gct gcg aga      48
Met Gly Lys Met Glu Val Glu Arg Lys Thr Thr Gly Trp Ala Ala Arg
1               5                   10                  15
gac cct tct ggg tta ctc tct cct tac act tac act ctc aga gag aca      96
Asp Pro Ser Gly Leu Leu Ser Pro Tyr Thr Tyr Thr Leu Arg Glu Thr
            20                  25                  30
gga cca gag gat gtt cac ata aga atc ata tgc tgt gga atc tgc cac      144
Gly Pro Glu Asp Val His Ile Arg Ile Ile Cys Cys Gly Ile Cys His
        35                  40                  45
aca gat ctc cat caa acc aaa aac gat ctt ggc atg tca aac tac ccc      192
Thr Asp Leu His Gln Thr Lys Asn Asp Leu Gly Met Ser Asn Tyr Pro
    50                  55                  60
atg gtt cct ggg cac gag gtt gtt ggg gaa gtg gtg gag gtg gga tca      240
Met Val Pro Gly His Glu Val Val Gly Glu Val Val Glu Val Gly Ser
65                  70                  75                  80
gat gtg agc aag ttc acc gca ggg gat ata gtt gga gtt ggt tgt ctc      288
Asp Val Ser Lys Phe Thr Ala Gly Asp Ile Val Gly Val Gly Cys Leu
                85                  90                  95
gtt gga tgc tgc gga ggt tgt agc ccc tgc gag agg gat ctg gaa cag      336
Val Gly Cys Cys Gly Gly Cys Ser Pro Cys Glu Arg Asp Leu Glu Gln
            100                 105                 110
tat tgt ccc aag aaa ata tgg agc tac aac gat gtt tac atc gat ggt      384
Tyr Cys Pro Lys Lys Ile Trp Ser Tyr Asn Asp Val Tyr Ile Asp Gly
        115                 120                 125
caa cct aca caa ggc ggt ttc gct aga gcc acc gtc gtt cac caa aag      432
Gln Pro Thr Gln Gly Gly Phe Ala Arg Ala Thr Val Val His Gln Lys
    130                 135                 140
ttt gtg gtg aag att cca gaa gga atg gct gtt gag cag gcg gca cca      480
Phe Val Val Lys Ile Pro Glu Gly Met Ala Val Glu Gln Ala Ala Pro
145                 150                 155                 160
cta ctg tgt gcc ggt gtg aca gtg tac agt cca ctg gct cac ttc ggt      528
Leu Leu Cys Ala Gly Val Thr Val Tyr Ser Pro Leu Ala His Phe Gly
                165                 170                 175
ctg aag cga cca ggc cta aga gga ggt ata cta ggg cta ggt gga gtt      576
Leu Lys Arg Pro Gly Leu Arg Gly Gly Ile Leu Gly Leu Gly Gly Val
            180                 185                 190
ggt cac atg ggt gtg aaa ata gcc aaa gca atg ggt cac cat gtg act      624
Gly His Met Gly Val Lys Ile Ala Lys Ala Met Gly His His Val Thr
        195                 200                 205

gtc ata agc tca tca aac aag aag aaa gaa gag gct ttg aaa gat ctt        672
Val Ile Ser Ser Ser Asn Lys Lys Lys Glu Glu Ala Leu Lys Asp Leu
    210                 215                 220
gga gca gat gat tac gtg atc gga tcc gac caa tcg aag atg aac gaa        720
Gly Ala Asp Asp Tyr Val Ile Gly Ser Asp Gln Ser Lys Met Asn Glu
225                 230                 235                 240
ttg gcg gat tca atg gat tac ata atc gac acg gtc cct gtt cat cat        768
Leu Ala Asp Ser Met Asp Tyr Ile Ile Asp Thr Val Pro Val His His
                245                 250                 255
gca ctt gag cct tac ttg tct ctg ctt agg ctt gat ggg aaa ctc ata        816
Ala Leu Glu Pro Tyr Leu Ser Leu Leu Arg Leu Asp Gly Lys Leu Ile
            260                 265                 270
ctc atg gga gtc ata agc aat cca tta caa ttt ctc act cct atg gtt        864
Leu Met Gly Val Ile Ser Asn Pro Leu Gln Phe Leu Thr Pro Met Val
        275                 280                 285
atg ctt ggg agg aaa gtg ata acg ggg agc ttc att ggg agc atg aag        912
Met Leu Gly Arg Lys Val Ile Thr Gly Ser Phe Ile Gly Ser Met Lys
    290                 295                 300
gaa aca gag gag atg ctt gag ttc tgt aaa gaa aag ggt ttg agt tcg        960
Glu Thr Glu Glu Met Leu Glu Phe Cys Lys Glu Lys Gly Leu Ser Ser
305                 310                 315                 320
atc atc gaa gta gtg aag atg gat tat gtg aac act gcg ttt gag aga        1008
Ile Ile Glu Val Val Lys Met Asp Tyr Val Asn Thr Ala Phe Glu Arg
                325                 330                 335
ctc gag aag aac gat gtg cgt tat agg ttc gtg gtt gat gtc gaa gga        1056
Leu Glu Lys Asn Asp Val Arg Tyr Arg Phe Val Val Asp Val Glu Gly
            340                 345                 350
agc aaa ctt gag gct tga                                                1074
Ser Lys Leu Glu Ala
        355

<210> 207
<211> 357
<212> PRT
<213> Brassica napus

<400> 207
Met Gly Lys Met Glu Val Glu Arg Lys Thr Thr Gly Trp Ala Ala Arg
1               5                   10                  15
Asp Pro Ser Gly Leu Leu Ser Pro Tyr Thr Tyr Thr Leu Arg Glu Thr
            20                  25                  30
Gly Pro Glu Asp Val His Ile Arg Ile Ile Cys Cys Gly Ile Cys His
        35                  40                  45
Thr Asp Leu His Gln Thr Lys Asn Asp Leu Gly Met Ser Asn Tyr Pro
    50                  55                  60
Met Val Pro Gly His Glu Val Val Gly Glu Val Val Glu Val Gly Ser
65                  70                  75                  80
Asp Val Ser Lys Phe Thr Ala Gly Asp Ile Val Gly Val Gly Cys Leu
                85                  90                  95
Val Gly Cys Cys Gly Gly Cys Ser Pro Cys Glu Arg Asp Leu Glu Gln
            100                 105                 110
Tyr Cys Pro Lys Lys Ile Trp Ser Tyr Asn Asp Val Tyr Ile Asp Gly
        115                 120                 125
Gln Pro Thr Gln Gly Gly Phe Ala Arg Ala Thr Val Val His Gln Lys
    130                 135                 140
Phe Val Val Lys Ile Pro Glu Gly Met Ala Val Glu Gln Ala Ala Pro
145                 150                 155                 160
Leu Leu Cys Ala Gly Val Thr Val Tyr Ser Pro Leu Ala His Phe Gly
                165                 170                 175
Leu Lys Arg Pro Gly Leu Arg Gly Gly Ile Leu Gly Leu Gly Gly Val
            180                 185                 190
Gly His Met Gly Val Lys Ile Ala Lys Ala Met Gly His His Val Thr
        195                 200                 205
Val Ile Ser Ser Ser Asn Lys Lys Lys Glu Glu Ala Leu Lys Asp Leu
    210                 215                 220
Gly Ala Asp Asp Tyr Val Ile Gly Ser Asp Gln Ser Lys Met Asn Glu
225                 230                 235                 240
Leu Ala Asp Ser Met Asp Tyr Ile Ile Asp Thr Val Pro Val His His
                245                 250                 255

Ala Leu Glu Pro Tyr Leu Ser Leu Leu Arg Leu Asp Gly Lys Leu Ile
            260                 265                 270
Leu Met Gly Val Ile Ser Asn Pro Leu Gln Phe Leu Thr Pro Met Val
        275                 280                 285
Met Leu Gly Arg Lys Val Ile Thr Gly Ser Phe Ile Gly Ser Met Lys
    290                 295                 300
Glu Thr Glu Glu Met Leu Glu Phe Cys Lys Glu Lys Gly Leu Ser Ser
305                 310                 315                 320
Ile Ile Glu Val Val Lys Met Asp Tyr Val Asn Thr Ala Phe Glu Arg
                325                 330                 335
Leu Glu Lys Asn Asp Val Arg Tyr Arg Phe Val Val Asp Val Glu Gly
            340                 345                 350
Ser Lys Leu Glu Ala
        355

<210> 208
<211> 1074
<212> DNA
<213> Zea mays

<220>
<221> CDS
<222> (1)..(1074)

<400> 208
atg gcg gga ggc aag gag gcg cac ggg tgg gca gcc agg gat gtc tct      48
Met Ala Gly Gly Lys Glu Ala His Gly Trp Ala Ala Arg Asp Val Ser
1               5                   10                  15
ggt cac ctc tcc cct tac cac ttc tca cgg agg gtt cag aga gac gac      96
Gly His Leu Ser Pro Tyr His Phe Ser Arg Arg Val Gln Arg Asp Asp
            20                  25                  30
gac gtc acc atc aag gtg ctc ttc tgc ggg ctt tgc cac act gac ctc      144
Asp Val Thr Ile Lys Val Leu Phe Cys Gly Leu Cys His Thr Asp Leu
        35                  40                  45
cat gtc atc aag aac gag ttt ggc aac gcc aag tac ccc gtc gtt ccc      192
His Val Ile Lys Asn Glu Phe Gly Asn Ala Lys Tyr Pro Val Val Pro
    50                  55                  60
ggg cac gag atc gtc ggc gtc gtc acg gac gtc ggc tcc ggc gtc aca      240
Gly His Glu Ile Val Gly Val Val Thr Asp Val Gly Ser Gly Val Thr
65                  70                  75                  80
agc ttc aag ccc ggc gac acg gtg ggc gtg ggc tac ttc gtc gac tcc      288
Ser Phe Lys Pro Gly Asp Thr Val Gly Val Gly Tyr Phe Val Asp Ser
                85                  90                  95
tgc cgc agc tgc aac agc tgc agc aag ggg tac gag agc tac tgc ccg      336
Cys Arg Ser Cys Asn Ser Cys Ser Lys Gly Tyr Glu Ser Tyr Cys Pro
            100                 105                 110
cag ctc gtg gag acg tcc aac ggc gtg agc ctg gac gac gac ggc ggc      384
Gln Leu Val Glu Thr Ser Asn Gly Val Ser Leu Asp Asp Asp Gly Gly
        115                 120                 125
gcc acc acc aag ggc ggc ttc tcc gac gcc ctc gtc gtc cac cag cgc      432
Ala Thr Thr Lys Gly Gly Phe Ser Asp Ala Leu Val Val His Gln Arg
    130                 135                 140
tac gtg gtg cgg gtc ccg gcc agc ctg ccg ccc gcc ggg gcc gcg ccg      480
Tyr Val Val Arg Val Pro Ala Ser Leu Pro Pro Ala Gly Ala Ala Pro
145                 150                 155                 160
ctg ctg tgc gcc ggc gtc acc gtg ttc agc ccc atg gtg cag tac ggc      528
Leu Leu Cys Ala Gly Val Thr Val Phe Ser Pro Met Val Gln Tyr Gly
                165                 170                 175
ctg aac gcg ccg ggg aag cac ctg ggc gtc gtc ggc ctc ggc ggc ctc      576
Leu Asn Ala Pro Gly Lys His Leu Gly Val Val Gly Leu Gly Gly Leu
            180                 185                 190
ggc cac ctg gcc gtc cgc ttc ggc aag gcg ttc ggg atg aag gtc acc      624
Gly His Leu Ala Val Arg Phe Gly Lys Ala Phe Gly Met Lys Val Thr
        195                 200                 205
gtc atc agc acg tcg ctg ggc aag cgg gac gag gcc ctc ggc cgc ctc      672
Val Ile Ser Thr Ser Leu Gly Lys Arg Asp Glu Ala Leu Gly Arg Leu
    210                 215                 220
ggc gcc gac gcg ttc ctg gtc agc cgc gac ccc gag cag atg agg gcg      720
Gly Ala Asp Ala Phe Leu Val Ser Arg Asp Pro Glu Gln Met Arg Ala

225                 230                 235                 240
gcg gcg ggc acc ttg gac ggc gtc atc gac acg gtg tcg gcc gac cac       768
Ala Ala Gly Thr Leu Asp Gly Val Ile Asp Thr Val Ser Ala Asp His
                245                 250                 255
cct gtc gtg ccg ctg ctg gac ctg ctc aag ccg atg ggc cag atg gtc       816
Pro Val Val Pro Leu Leu Asp Leu Leu Lys Pro Met Gly Gln Met Val
            260                 265                 270
gtc gtc ggc ctg ccc acc aag ccg ctc cag gtg cct gcc ttc agc ctc       864
Val Val Gly Leu Pro Thr Lys Pro Leu Gln Val Pro Ala Phe Ser Leu
        275                 280                 285
gtc gcc ggc ggg aag cgc gtg gcc ggg agt gcc ggc ggc ggc gtc ggg       912
Val Ala Gly Gly Lys Arg Val Ala Gly Ser Ala Gly Gly Gly Val Gly
    290                 295                 300
gag tgc cag gcc atg ctc gac ttt gcc ggc gag cac ggg atc acc gcg       960
Glu Cys Gln Ala Met Leu Asp Phe Ala Gly Glu His Gly Ile Thr Ala
305                 310                 315                 320
gat gtg gag gtc gtc ggg atg gac tac gtc aat acc gcc atc cag cgc       1008
Asp Val Glu Val Val Gly Met Asp Tyr Val Asn Thr Ala Ile Gln Arg
                325                 330                 335
cta gag agg aac gat gtc agg tac cgc ttc gtt gtc gac gtc gcg ggc       1056
Leu Glu Arg Asn Asp Val Arg Tyr Arg Phe Val Val Asp Val Ala Gly
            340                 345                 350
agc aag att gga ggc tag                                               1074
Ser Lys Ile Gly Gly
        355

<210> 209
<211> 357
<212> PRT
<213> Zea mays

<400> 209
Met Ala Gly Gly Lys Glu Ala His Gly Trp Ala Ala Arg Asp Val Ser
1               5                   10                  15
Gly His Leu Ser Pro Tyr His Phe Ser Arg Arg Val Gln Arg Asp Asp
            20                  25                  30
Asp Val Thr Ile Lys Val Leu Phe Cys Gly Leu Cys His Thr Asp Leu
        35                  40                  45
His Val Ile Lys Asn Glu Phe Gly Asn Ala Lys Tyr Pro Val Val Pro
    50                  55                  60
Gly His Glu Ile Val Gly Val Val Thr Asp Val Gly Ser Gly Val Thr
65                  70                  75                  80
Ser Phe Lys Pro Gly Asp Thr Val Gly Val Gly Tyr Phe Val Asp Ser
                85                  90                  95
Cys Arg Ser Cys Asn Ser Cys Ser Lys Gly Tyr Glu Ser Tyr Cys Pro
            100                 105                 110
Gln Leu Val Glu Thr Ser Asn Gly Val Ser Leu Asp Asp Asp Gly Gly
        115                 120                 125
Ala Thr Thr Lys Gly Gly Phe Ser Asp Ala Leu Val Val His Gln Arg
    130                 135                 140
Tyr Val Val Arg Val Pro Ala Ser Leu Pro Pro Ala Gly Ala Ala Pro
145                 150                 155                 160
Leu Leu Cys Ala Gly Val Thr Val Phe Ser Pro Met Val Gln Tyr Gly
                165                 170                 175
Leu Asn Ala Pro Gly Lys His Leu Gly Val Val Gly Leu Gly Gly Leu
            180                 185                 190
Gly His Leu Ala Val Arg Phe Gly Lys Ala Phe Gly Met Lys Val Thr
        195                 200                 205
Val Ile Ser Thr Ser Leu Gly Lys Arg Asp Glu Ala Leu Gly Arg Leu
    210                 215                 220
Gly Ala Asp Ala Phe Leu Val Ser Arg Asp Pro Glu Gln Met Arg Ala
225                 230                 235                 240
Ala Ala Gly Thr Leu Asp Gly Val Ile Asp Thr Val Ser Ala Asp His
                245                 250                 255
Pro Val Val Pro Leu Leu Asp Leu Leu Lys Pro Met Gly Gln Met Val
            260                 265                 270
Val Val Gly Leu Pro Thr Lys Pro Leu Gln Val Pro Ala Phe Ser Leu
        275                 280                 285
Val Ala Gly Gly Lys Arg Val Ala Gly Ser Ala Gly Gly Gly Val Gly

290                 295                 300
Glu Cys Gln Ala Met Leu Asp Phe Ala Gly Glu His Gly Ile Thr Ala
305                 310                 315                 320
Asp Val Glu Val Val Gly Met Asp Tyr Val Asn Thr Ala Ile Gln Arg
                325                 330                 335
Leu Glu Arg Asn Asp Val Arg Tyr Arg Phe Val Val Asp Val Ala Gly
            340                 345                 350
Ser Lys Ile Gly Gly
        355

<210> 210
<211> 1245
<212> DNA
<213> Zea mays

<220>
<221> CDS
<222> (1)..(1245)

<400> 210
atg tca tat cat tgt cgc gtc ctc gtc ccc gtt cca ttt cat tat ccc      48
Met Ser Tyr His Cys Arg Val Leu Val Pro Val Pro Phe His Tyr Pro
1               5                   10                  15
ccc gga gct ggt gcg cga gtc gga gct ggt gca ggg tca ccc ttc ccc      96
Pro Gly Ala Gly Ala Arg Val Gly Ala Gly Ala Gly Ser Pro Phe Pro
            20                  25                  30
tcg gcc tca aga gct ctg cgg ttg ccg cgg cca agg gcg tcc gtg gag      144
Ser Ala Ser Arg Ala Leu Arg Leu Pro Arg Pro Arg Ala Ser Val Glu
        35                  40                  45
aag cgg gag cag gtg gcg gcg atg gaa gag caa ggc ggc cag gcg gcg      192
Lys Arg Glu Gln Val Ala Ala Met Glu Glu Gln Gly Gly Gln Ala Ala
    50                  55                  60
ctc ggg tgg gcg gcc agg gac gac tcc ggc gtc ctc tcc ccc tac agc      240
Leu Gly Trp Ala Ala Arg Asp Asp Ser Gly Val Leu Ser Pro Tyr Ser
65                  70                  75                  80
ttc tcc aga agg gtt cct aaa gac gac gat gtc acg atc aag gtg ctc      288
Phe Ser Arg Arg Val Pro Lys Asp Asp Asp Val Thr Ile Lys Val Leu
                85                  90                  95
tac tgc ggg atc tgc cac acc gac ctg cac gtc atc aag aac gac tgg      336
Tyr Cys Gly Ile Cys His Thr Asp Leu His Val Ile Lys Asn Asp Trp
            100                 105                 110
cga aac gcc atg tac cca gtc gtc ccg ggg cac gag atc gtg ggc gtg      384
Arg Asn Ala Met Tyr Pro Val Val Pro Gly His Glu Ile Val Gly Val
        115                 120                 125
gtg acc ggc gtc ggc ggc ggc gtc acg cgg ttc aag gcc ggc gac acg      432
Val Thr Gly Val Gly Gly Gly Val Thr Arg Phe Lys Ala Gly Asp Thr
    130                 135                 140
gtc ggc gtg ggc tac ttc gtg ggg tcc tgc cgc tcc tgc gac agc tgc      480
Val Gly Val Gly Tyr Phe Val Gly Ser Cys Arg Ser Cys Asp Ser Cys
145                 150                 155                 160
ggc aag ggg gac gac aac tac tgc gcg ggg atc gtg ctc acc tcc aac      528
Gly Lys Gly Asp Asp Asn Tyr Cys Ala Gly Ile Val Leu Thr Ser Asn
                165                 170                 175
ggc gtc gac cac gcg cac ggc ggc gcg ccc acc agg ggg gga ttc tcc      576
Gly Val Asp His Ala His Gly Gly Ala Pro Thr Arg Gly Gly Phe Ser
            180                 185                 190
gac gtc ctg gtc gcg agc gag cac tac gtg gtc cgc gtc ccc gac ggc      624
Asp Val Leu Val Ala Ser Glu His Tyr Val Val Arg Val Pro Asp Gly
        195                 200                 205
ctg gcg ctg gac cgc acc gcg ccg ctg ctc tgc gcc ggc gtc acc gtg      672
Leu Ala Leu Asp Arg Thr Ala Pro Leu Leu Cys Ala Gly Val Thr Val
    210                 215                 220
tac agc ccc atg atg cgc cac ggc ctc aac gag ccc ggc aag cac ctc      720
Tyr Ser Pro Met Met Arg His Gly Leu Asn Glu Pro Gly Lys His Leu
225                 230                 235                 240
ggc gtc gtc ggc ctc ggc ggc ctc ggc cac gtc gcc gtc aag ttc ggc      768
Gly Val Val Gly Leu Gly Gly Leu Gly His Val Ala Val Lys Phe Gly
                245                 250                 255
aag gcc ttc ggg atg aag gtc acc gtc atc agc acg tcc gcc agc aag      816

Lys Ala Phe Gly Met Lys Val Thr Val Ile Ser Thr Ser Ala Ser Lys
            260                 265                 270
cgc cag gag gcc atc gag aac ctc ggc gcg gac gag ttc ctc atc agc      864
Arg Gln Glu Ala Ile Glu Asn Leu Gly Ala Asp Glu Phe Leu Ile Ser
            275                 280                 285
cgg gac gag gac cag atg aag gcg gcg acg ggg acc atg gac ggc atc      912
Arg Asp Glu Asp Gln Met Lys Ala Ala Thr Gly Thr Met Asp Gly Ile
    290                 295                 300
atc gac acg gtg tcg gcg tgg cac ccg atc acg ccg ctg ctg gcg ctg      960
Ile Asp Thr Val Ser Ala Trp His Pro Ile Thr Pro Leu Leu Ala Leu
305                 310                 315                 320
ctg aag ccg ctg ggg cag atg gtg gtc gtg ggc gcg ccg agc aag ccg      1008
Leu Lys Pro Leu Gly Gln Met Val Val Val Gly Ala Pro Ser Lys Pro
            325                 330                 335
ctc gag ctg ccg gcc tac gcc atc gtg ccg ggc ggg aag ggc gtg gct      1056
Leu Glu Leu Pro Ala Tyr Ala Ile Val Pro Gly Gly Lys Gly Val Ala
            340                 345                 350
ggg aac aat gtc ggc agc gtc agg gac tgc cag gcc atg ctc gag ttc      1104
Gly Asn Asn Val Gly Ser Val Arg Asp Cys Gln Ala Met Leu Glu Phe
            355                 360                 365
gcg ggg aag cac ggc atc ggg gcc gag gtc gag gtc atc aag atg gac      1152
Ala Gly Lys His Gly Ile Gly Ala Glu Val Glu Val Ile Lys Met Asp
    370                 375                 380
tac gtc aac acg gcc atg gag cgg ctc gag aag aac gac gtc cgc tac      1200
Tyr Val Asn Thr Ala Met Glu Arg Leu Glu Lys Asn Asp Val Arg Tyr
385                 390                 395                 400
cgc ttc gtc atc gac gtc gcc ggc agc ctc ggc tct gcc gcc tag          1245
Arg Phe Val Ile Asp Val Ala Gly Ser Leu Gly Ser Ala Ala
            405                 410

<210> 211
<211> 414
<212> PRT
<213> Zea mays

<400> 211
Met Ser Tyr His Cys Arg Val Leu Val Pro Val Pro Phe His Tyr Pro
1               5                   10                  15
Pro Gly Ala Gly Ala Arg Val Gly Ala Gly Ala Gly Ser Pro Phe Pro
            20                  25                  30
Ser Ala Ser Arg Ala Leu Arg Leu Pro Arg Pro Arg Ala Ser Val Glu
            35                  40                  45
Lys Arg Glu Gln Val Ala Ala Met Glu Glu Gln Gly Gly Gln Ala Ala
    50                  55                  60
Leu Gly Trp Ala Ala Arg Asp Asp Ser Gly Val Leu Ser Pro Tyr Ser
65                  70                  75                  80
Phe Ser Arg Arg Val Pro Lys Asp Asp Asp Val Thr Ile Lys Val Leu
                85                  90                  95
Tyr Cys Gly Ile Cys His Thr Asp Leu His Val Ile Lys Asn Asp Trp
            100                 105                 110
Arg Asn Ala Met Tyr Pro Val Val Pro Gly His Glu Ile Val Gly Val
            115                 120                 125
Val Thr Gly Val Gly Gly Gly Val Thr Arg Phe Lys Ala Gly Asp Thr
    130                 135                 140
Val Gly Val Gly Tyr Phe Val Gly Ser Cys Arg Ser Cys Asp Ser Cys
145                 150                 155                 160
Gly Lys Gly Asp Asp Asn Tyr Cys Ala Gly Ile Val Leu Thr Ser Asn
                165                 170                 175
Gly Val Asp His Ala His Gly Gly Ala Pro Thr Arg Gly Gly Phe Ser
            180                 185                 190
Asp Val Leu Val Ala Ser Glu His Tyr Val Val Arg Val Pro Asp Gly
            195                 200                 205
Leu Ala Leu Asp Arg Thr Ala Pro Leu Leu Cys Ala Gly Val Thr Val
    210                 215                 220
Tyr Ser Pro Met Met Arg His Gly Leu Asn Glu Pro Gly Lys His Leu
225                 230                 235                 240
Gly Val Val Gly Leu Gly Gly Leu Gly His Val Ala Val Lys Phe Gly
                245                 250                 255
Lys Ala Phe Gly Met Lys Val Thr Val Ile Ser Thr Ser Ala Ser Lys

260                 265                 270
Arg Gln Glu Ala Ile Glu Asn Leu Gly Ala Asp Glu Phe Leu Ile Ser
        275                 280                 285
Arg Asp Glu Asp Gln Met Lys Ala Ala Thr Gly Thr Met Asp Gly Ile
    290                 295                 300
Ile Asp Thr Val Ser Ala Trp His Pro Ile Thr Pro Leu Leu Ala Leu
305                 310                 315                 320
Leu Lys Pro Leu Gly Gln Met Val Val Val Gly Ala Pro Ser Lys Pro
                325                 330                 335
Leu Glu Leu Pro Ala Tyr Ala Ile Val Pro Gly Gly Lys Gly Val Ala
            340                 345                 350
Gly Asn Asn Val Gly Ser Val Arg Asp Cys Gln Ala Met Leu Glu Phe
        355                 360                 365
Ala Gly Lys His Gly Ile Gly Ala Glu Val Glu Val Ile Lys Met Asp
    370                 375                 380
Tyr Val Asn Thr Ala Met Glu Arg Leu Glu Lys Asn Asp Val Arg Tyr
385                 390                 395                 400
Arg Phe Val Ile Asp Val Ala Gly Ser Leu Gly Ser Ala Ala
                405                 410

<210> 212
<211> 1065
<212> DNA
<213> Zea mays

<220>
<221> CDS
<222> (1)..(1065)

<400> 212
atg gct gct gaa tca gag aac ggc aac tgc aaa gct tgg gca gcg aga      48
Met Ala Ala Glu Ser Glu Asn Gly Asn Cys Lys Ala Trp Ala Ala Arg
1               5                   10                  15
gat cct tct gga gtt ctc tca cca cac agt ttt aac cgc aga gca gtg      96
Asp Pro Ser Gly Val Leu Ser Pro His Ser Phe Asn Arg Arg Ala Val
            20                  25                  30
caa agc ggc gat gtt tcg ctg aag atc ata tac tgt gga gtc tgt tat      144
Gln Ser Gly Asp Val Ser Leu Lys Ile Ile Tyr Cys Gly Val Cys Tyr
        35                  40                  45
gca gac gtt gct tgg acg cgg aat aag ctc aat gat tca aaa tat cct      192
Ala Asp Val Ala Trp Thr Arg Asn Lys Leu Asn Asp Ser Lys Tyr Pro
    50                  55                  60
ttg gtt cct ggg cat gag ata gct gga gtt gta act cag gtt ggt gca      240
Leu Val Pro Gly His Glu Ile Ala Gly Val Val Thr Gln Val Gly Ala
65                  70                  75                  80
gat gtc aaa ggc ttt aaa gtg ggt gac cat gta ggt gtt gga aca tat      288
Asp Val Lys Gly Phe Lys Val Gly Asp His Val Gly Val Gly Thr Tyr
                85                  90                  95
gtg aac tct tgc cgt gat tgt gag aac tgc aat atc tct cta gac aac      336
Val Asn Ser Cys Arg Asp Cys Glu Asn Cys Asn Ile Ser Leu Asp Asn
            100                 105                 110
cat tgc cca aaa gca gtt tat act ttc aat ggc att gat aca gat gga      384
His Cys Pro Lys Ala Val Tyr Thr Phe Asn Gly Ile Asp Thr Asp Gly
        115                 120                 125
act gtc aca aag ggg ggt tac tcc act cac att gta gtc cat gaa agg      432
Thr Val Thr Lys Gly Gly Tyr Ser Thr His Ile Val Val His Glu Arg
    130                 135                 140
tat tgc ttc aaa ata ccc gat ggc tac cct ttg gcg aag gca gca cct      480
Tyr Cys Phe Lys Ile Pro Asp Gly Tyr Pro Leu Ala Lys Ala Ala Pro
145                 150                 155                 160
ctt ctg tgt gct gga ata acc gtg tat act cca atg atg cga cac aac      528
Leu Leu Cys Ala Gly Ile Thr Val Tyr Thr Pro Met Met Arg His Asn
                165                 170                 175
atg aac caa cct gga aag tca ctt ggg gtc att ggt ctt ggt ggt ctg      576
Met Asn Gln Pro Gly Lys Ser Leu Gly Val Ile Gly Leu Gly Gly Leu
            180                 185                 190
ggt cac atg gct gtg aaa ttt ggt aaa gca ttt ggt ctt aag gtc aca      624
Gly His Met Ala Val Lys Phe Gly Lys Ala Phe Gly Leu Lys Val Thr
        195                 200                 205

gtt ttc agt aca agt gaa tca aag aga gat gaa gcc atc aac ctc ctt 672
Val Phe Ser Thr Ser Glu Ser Lys Arg Asp Glu Ala Ile Asn Leu Leu
    210                 215                 220
gga gca gat aat ttt gtt ata tca tca gac aca cag caa atg gag tcc 720
Gly Ala Asp Asn Phe Val Ile Ser Ser Asp Thr Gln Gln Met Glu Ser
225                 230                 235                 240
ctg aag aac tcc ctg cac ttc ata att gac act gct tcc ggt gac cat 768
Leu Lys Asn Ser Leu His Phe Ile Ile Asp Thr Ala Ser Gly Asp His
                245                 250                 255
cca ttt gat ccg tat ttc tct ctc ctt aag gtt ggt ggt gtg atg aca 816
Pro Phe Asp Pro Tyr Phe Ser Leu Leu Lys Val Gly Gly Val Met Thr
            260                 265                 270
ttg gta ggt ttt cca agt gag atc aaa atg cat cct gca agc ctt ata 864
Leu Val Gly Phe Pro Ser Glu Ile Lys Met His Pro Ala Ser Leu Ile
        275                 280                 285
ctt ggt gcg cgg acc ttg tct ggc agt gtt gtt gga ggt ata aaa gac 912
Leu Gly Ala Arg Thr Leu Ser Gly Ser Val Val Gly Gly Ile Lys Asp
    290                 295                 300
atc caa gaa atg gtt aac ttc tgt gca gcc aac aat atc tat ccg gag 960
Ile Gln Glu Met Val Asn Phe Cys Ala Ala Asn Asn Ile Tyr Pro Glu
305                 310                 315                 320
att gag atc att aag ata gat tat atc aac gag gct ctc gcg agg ctt 1008
Ile Glu Ile Ile Lys Ile Asp Tyr Ile Asn Glu Ala Leu Ala Arg Leu
                325                 330                 335
gtt aat cga gat gtg aaa tac cgc ttt gta atc gac atc gag aac tcc 1056
Val Asn Arg Asp Val Lys Tyr Arg Phe Val Ile Asp Ile Glu Asn Ser
            340                 345                 350
ttc aag tag 1065
Phe Lys

<210> 213
<211> 354
<212> PRT
<213> Zea mays

<400> 213
Met Ala Ala Glu Ser Glu Asn Gly Asn Cys Lys Ala Trp Ala Ala Arg
1               5                   10                  15
Asp Pro Ser Gly Val Leu Ser Pro His Ser Phe Asn Arg Arg Ala Val
            20                  25                  30
Gln Ser Gly Asp Val Ser Leu Lys Ile Ile Tyr Cys Gly Val Cys Tyr
        35                  40                  45
Ala Asp Val Ala Trp Thr Arg Asn Lys Leu Asn Asp Ser Lys Tyr Pro
    50                  55                  60
Leu Val Pro Gly His Glu Ile Ala Gly Val Val Thr Gln Val Gly Ala
65                  70                  75                  80
Asp Val Lys Gly Phe Lys Val Gly Asp His Val Gly Val Gly Thr Tyr
                85                  90                  95
Val Asn Ser Cys Arg Asp Cys Glu Asn Cys Asn Ile Ser Leu Asp Asn
            100                 105                 110
His Cys Pro Lys Ala Val Tyr Thr Phe Asn Gly Ile Asp Thr Asp Gly
        115                 120                 125
Thr Val Thr Lys Gly Gly Tyr Ser Thr His Ile Val Val His Glu Arg
    130                 135                 140
Tyr Cys Phe Lys Ile Pro Asp Gly Tyr Pro Leu Ala Lys Ala Ala Pro
145                 150                 155                 160
Leu Leu Cys Ala Gly Ile Thr Val Tyr Thr Pro Met Met Arg His Asn
                165                 170                 175
Met Asn Gln Pro Gly Lys Ser Leu Gly Val Ile Gly Leu Gly Gly Leu
            180                 185                 190
Gly His Met Ala Val Lys Phe Gly Lys Ala Phe Gly Leu Lys Val Thr
        195                 200                 205
Val Phe Ser Thr Ser Glu Ser Lys Arg Asp Glu Ala Ile Asn Leu Leu
    210                 215                 220
Gly Ala Asp Asn Phe Val Ile Ser Ser Asp Thr Gln Gln Met Glu Ser
225                 230                 235                 240
Leu Lys Asn Ser Leu His Phe Ile Ile Asp Thr Ala Ser Gly Asp His
                245                 250                 255

Pro Phe Asp Pro Tyr Phe Ser Leu Leu Lys Val Gly Gly Val Met Thr
            260                 265                 270
Leu Val Gly Phe Pro Ser Glu Ile Lys Met His Pro Ala Ser Leu Ile
        275                 280                 285
Leu Gly Ala Arg Thr Leu Ser Gly Ser Val Val Gly Gly Ile Lys Asp
    290                 295                 300
Ile Gln Glu Met Val Asn Phe Cys Ala Ala Asn Asn Ile Tyr Pro Glu
305                 310                 315                 320
Ile Glu Ile Ile Lys Ile Asp Tyr Ile Asn Glu Ala Leu Ala Arg Leu
                325                 330                 335
Val Asn Arg Asp Val Lys Tyr Arg Phe Val Ile Asp Ile Glu Asn Ser
            340                 345                 350
Phe Lys


<210> 214
<211> 1080
<212> DNA
<213> Zea mays

<220>
<221> CDS
<222> (1)..(1080)

<400> 214
atg gct ccc act ccc act cac acg gcg gcg gcg gtg gcg ctc gcc gcg      48
Met Ala Pro Thr Pro Thr His Thr Ala Ala Ala Val Ala Leu Ala Ala
1               5                   10                  15
cac gac tcg tcc ggc cac ctc gcg ccg ctc acc atc acc cgc agg agc      96
His Asp Ser Ser Gly His Leu Ala Pro Leu Thr Ile Thr Arg Arg Ser
            20                  25                  30
acc ggg gac gac gat gta gcg ata aag atc ctg tac tgt ggg atg tgt      144
Thr Gly Asp Asp Asp Val Ala Ile Lys Ile Leu Tyr Cys Gly Met Cys
        35                  40                  45
cac tct gac ctt cac agc atc aag aac gag tgg cac aac gcc acg tac      192
His Ser Asp Leu His Ser Ile Lys Asn Glu Trp His Asn Ala Thr Tyr
    50                  55                  60
ccc atg gtc cct ggt cac gag atc gcc gga gtg gtg acg gag gtg ggc      240
Pro Met Val Pro Gly His Glu Ile Ala Gly Val Val Thr Glu Val Gly
65                  70                  75                  80
aag aac gtg acc agc ttc aag gcc ggg gac cgc gtc ggc gtg ggc tgc      288
Lys Asn Val Thr Ser Phe Lys Ala Gly Asp Arg Val Gly Val Gly Cys
                85                  90                  95
atg gtg aac tcg tgc cag tcg tgc ggc agc tgc gac gag gac ttc gag      336
Met Val Asn Ser Cys Gln Ser Cys Gly Ser Cys Asp Glu Asp Phe Glu
            100                 105                 110
aac agc tgc cgg gcc gtc atc ttc acc tac aac tcc gtc gac cgc gac      384
Asn Ser Cys Arg Ala Val Ile Phe Thr Tyr Asn Ser Val Asp Arg Asp
        115                 120                 125
ggc acc gtc acc tac ggc ggc tac tcc agc agc gtg gtg gtg cac gag      432
Gly Thr Val Thr Tyr Gly Gly Tyr Ser Ser Ser Val Val Val His Glu
    130                 135                 140
cgc ttc gtg gtc cgg ttc ccc gac gcg atg ccg ctg gat cgg ggg gcg      480
Arg Phe Val Val Arg Phe Pro Asp Ala Met Pro Leu Asp Arg Gly Ala
145                 150                 155                 160
ccg ctg ctg tgc gcg ggc atc aca gtg tac agc ccc atg aag tac cac      528
Pro Leu Leu Cys Ala Gly Ile Thr Val Tyr Ser Pro Met Lys Tyr His
                165                 170                 175
ggc ctg aac gtg ccg ggg aag cac gtc ggc gtg atg ggg ctg gga ggc      576
Gly Leu Asn Val Pro Gly Lys His Val Gly Val Met Gly Leu Gly Gly
            180                 185                 190
ctg ggc cac gtc gcc gtc aag ttc gcc aag gcg ttc ggg gcc aag gtc      624
Leu Gly His Val Ala Val Lys Phe Ala Lys Ala Phe Gly Ala Lys Val
        195                 200                 205
acg gtc atc agc acg tcg cct ggg aag aag cag gag gcg ctc gac cgg      672
Thr Val Ile Ser Thr Ser Pro Gly Lys Lys Gln Glu Ala Leu Asp Arg
    210                 215                 220
ctt ggc gcc gac gcc ttc gtc gtc agc aag gac gcc gaa gaa atg aag      720
Leu Gly Ala Asp Ala Phe Val Val Ser Lys Asp Ala Glu Glu Met Lys

225                 230                 235                 240
gcg gct gcg agc acc atg gac ggc atc atc aac acg gtg tcg gcg aac       768
Ala Ala Ala Ser Thr Met Asp Gly Ile Ile Asn Thr Val Ser Ala Asn
                245                 250                 255
ctc acc ttg gcc ccg tac atg ggc ctg ctc aag ccc aac ggc aag atg       816
Leu Thr Leu Ala Pro Tyr Met Gly Leu Leu Lys Pro Asn Gly Lys Met
            260                 265                 270
atc atg gtc ggc ctg ccc acc aag cct ctc gag atc ccg ccc ttt gat       864
Ile Met Val Gly Leu Pro Thr Lys Pro Leu Glu Ile Pro Pro Phe Asp
        275                 280                 285
ctc atc att ggg aac aag acc ctg gcc ggg agc tgc atc ggc ggc atg       912
Leu Ile Ile Gly Asn Lys Thr Leu Ala Gly Ser Cys Ile Gly Gly Met
    290                 295                 300
agg gac acg cag gag atg atc aac gtg gcg gcc aag cac ggc gtg acg       960
Arg Asp Thr Gln Glu Met Ile Asn Val Ala Ala Lys His Gly Val Thr
305                 310                 315                 320
gcg gac ata gaa ctg gtg gcc gcg gac tac gtg aac acg gcc atg gag      1008
Ala Asp Ile Glu Leu Val Ala Ala Asp Tyr Val Asn Thr Ala Met Glu
                325                 330                 335
cgc ctg gcc aag gct gac gtc agg tac cgc ttc gtc atc gac atc ggc      1056
Arg Leu Ala Lys Ala Asp Val Arg Tyr Arg Phe Val Ile Asp Ile Gly
            340                 345                 350
aac acc ctc aag aat tca gag tga                                      1080
Asn Thr Leu Lys Asn Ser Glu
        355

<210> 215
<211> 359
<212> PRT
<213> Zea mays

<400> 215
Met Ala Pro Thr Pro Thr His Thr Ala Ala Ala Val Ala Leu Ala Ala
1               5                   10                  15
His Asp Ser Ser Gly His Leu Ala Pro Leu Thr Ile Thr Arg Arg Ser
            20                  25                  30
Thr Gly Asp Asp Asp Val Ala Ile Lys Ile Leu Tyr Cys Gly Met Cys
        35                  40                  45
His Ser Asp Leu His Ser Ile Lys Asn Glu Trp His Asn Ala Thr Tyr
    50                  55                  60
Pro Met Val Pro Gly His Glu Ile Ala Gly Val Val Thr Glu Val Gly
65                  70                  75                  80
Lys Asn Val Thr Ser Phe Lys Ala Gly Asp Arg Val Gly Val Gly Cys
                85                  90                  95
Met Val Asn Ser Cys Gln Ser Cys Gly Ser Cys Asp Glu Asp Phe Glu
            100                 105                 110
Asn Ser Cys Arg Ala Val Ile Phe Thr Tyr Asn Ser Val Asp Arg Asp
        115                 120                 125
Gly Thr Val Thr Tyr Gly Gly Tyr Ser Ser Ser Val Val Val His Glu
    130                 135                 140
Arg Phe Val Val Arg Phe Pro Asp Ala Met Pro Leu Asp Arg Gly Ala
145                 150                 155                 160
Pro Leu Leu Cys Ala Gly Ile Thr Val Tyr Ser Pro Met Lys Tyr His
                165                 170                 175
Gly Leu Asn Val Pro Gly Lys His Val Gly Val Met Gly Leu Gly Gly
            180                 185                 190
Leu Gly His Val Ala Val Lys Phe Ala Lys Ala Phe Gly Ala Lys Val
        195                 200                 205
Thr Val Ile Ser Thr Ser Pro Gly Lys Lys Gln Glu Ala Leu Asp Arg
    210                 215                 220
Leu Gly Ala Asp Ala Phe Val Val Ser Lys Asp Ala Glu Glu Met Lys
225                 230                 235                 240
Ala Ala Ala Ser Thr Met Asp Gly Ile Ile Asn Thr Val Ser Ala Asn
                245                 250                 255
Leu Thr Leu Ala Pro Tyr Met Gly Leu Leu Lys Pro Asn Gly Lys Met
            260                 265                 270
Ile Met Val Gly Leu Pro Thr Lys Pro Leu Glu Ile Pro Pro Phe Asp
        275                 280                 285
Leu Ile Ile Gly Asn Lys Thr Leu Ala Gly Ser Cys Ile Gly Gly Met

290                 295                 300
Arg Asp Thr Gln Glu Met Ile Asn Val Ala Ala Lys His Gly Val Thr
305                 310                 315                 320
Ala Asp Ile Glu Leu Val Ala Ala Asp Tyr Val Asn Thr Ala Met Glu
                325                 330                 335
Arg Leu Ala Lys Ala Asp Val Arg Tyr Arg Phe Val Ile Asp Ile Gly
            340                 345                 350
Asn Thr Leu Lys Asn Ser Glu
        355

<210> 216
<211> 1077
<212> DNA
<213> Zea mays

<220>
<221> CDS
<222> (1)..(1077)

<400> 216
atg gcg gga ggc aag gaa gcg cac ggg tgg gca gcc agg gat gtc tct      48
Met Ala Gly Gly Lys Glu Ala His Gly Trp Ala Ala Arg Asp Val Ser
1               5                   10                  15
ggt cac ctc tcc cct tac cac ttc tca cgg agg gtt cag aga gac gac      96
Gly His Leu Ser Pro Tyr His Phe Ser Arg Arg Val Gln Arg Asp Asp
            20                  25                  30
gac gtc acc atc aag gtg ctc ttc tgc ggg ctt tgc cac act gac ctc      144
Asp Val Thr Ile Lys Val Leu Phe Cys Gly Leu Cys His Thr Asp Leu
        35                  40                  45
cac gtc atc aag aac gag ttt ggc aac gcc aag tac ccc gtc gtt ccc      192
His Val Ile Lys Asn Glu Phe Gly Asn Ala Lys Tyr Pro Val Val Pro
    50                  55                  60
ggg cac gag atc gtc ggc gtc gtc acc gac gtc ggc tcc ggc gtc aca      240
Gly His Glu Ile Val Gly Val Val Thr Asp Val Gly Ser Gly Val Thr
65                  70                  75                  80
agc ttc aag ccc ggc gac acg gtg ggc gtg ggc tac ttc gtc gac tcc      288
Ser Phe Lys Pro Gly Asp Thr Val Gly Val Gly Tyr Phe Val Asp Ser
                85                  90                  95
tgc cgc agc tgc gac agc tgc agc aag ggg tac gag agc tac tgc ccg      336
Cys Arg Ser Cys Asp Ser Cys Ser Lys Gly Tyr Glu Ser Tyr Cys Pro
            100                 105                 110
cag ctc gtg gag acg tcc aac ggc gtg agc ctg gac gac gat gac ggc      384
Gln Leu Val Glu Thr Ser Asn Gly Val Ser Leu Asp Asp Asp Asp Gly
        115                 120                 125
ggc gcc acc acc aag ggc ggc ttc tcc gac gcc ctc gtc gtc cac cag      432
Gly Ala Thr Thr Lys Gly Gly Phe Ser Asp Ala Leu Val Val His Gln
    130                 135                 140
cgc tac gtg gtg cgg gtc ccg gcc agc ctg ccg ccc gcc ggg gcc gcg      480
Arg Tyr Val Val Arg Val Pro Ala Ser Leu Pro Pro Ala Gly Ala Ala
145                 150                 155                 160
ccg ctg ctg tgc gcc ggc gtc acc gtg ttc agc ccc atg gtg cag tac      528
Pro Leu Leu Cys Ala Gly Val Thr Val Phe Ser Pro Met Val Gln Tyr
                165                 170                 175
ggc ctg aac gcg ccg ggg aag cac ctg ggc gtc gtc ggc ctc ggc ggc      576
Gly Leu Asn Ala Pro Gly Lys His Leu Gly Val Val Gly Leu Gly Gly
            180                 185                 190
ctc ggc cac ctg gcc gtc cgc ttc ggc aag gcg ttc ggg atg aag gtc      624
Leu Gly His Leu Ala Val Arg Phe Gly Lys Ala Phe Gly Met Lys Val
        195                 200                 205
acc gtc atc agc acg tcg ctg ggc aag cgg gac gag gcc ctc ggc cgc      672
Thr Val Ile Ser Thr Ser Leu Gly Lys Arg Asp Glu Ala Leu Gly Arg
    210                 215                 220
ctc ggt gcc gac gcg ttc ctg gtc agc cgc gac ccc gag cag atg agg      720
Leu Gly Ala Asp Ala Phe Leu Val Ser Arg Asp Pro Glu Gln Met Arg
225                 230                 235                 240
gcg gcg gcg ggc acc ttg gac ggc gtc atc gac acg gtg tcg gcc gac      768
Ala Ala Ala Gly Thr Leu Asp Gly Val Ile Asp Thr Val Ser Ala Asp
                245                 250                 255
cac cct gtc gtg ccg ctg ctg gac ctg ctc aag ccg atg ggc cag atg      816

His Pro Val Val Pro Leu Leu Asp Leu Leu Lys Pro Met Gly Gln Met
                260                 265                 270
gtc gtc gtc ggc ctg ccc acc aag ccg ctc cag gtg cct gcc ttc agc      864
Val Val Val Gly Leu Pro Thr Lys Pro Leu Gln Val Pro Ala Phe Ser
            275                 280                 285
ctc gtc gcc ggc ggg aag cgc gtg gcc ggg agt gcc ggc ggc ggc gtc      912
Leu Val Ala Gly Gly Lys Arg Val Ala Gly Ser Ala Gly Gly Gly Val
    290                 295                 300
ggg gag tgc cag gcc atg ctc gac ttt gcc ggc gag cac ggg atc acc      960
Gly Glu Cys Gln Ala Met Leu Asp Phe Ala Gly Glu His Gly Ile Thr
305                 310                 315                 320
gcg gat gtg gag gtc gtc ggg atg gac tac gtc aat acc gcc atc cag      1008
Ala Asp Val Glu Val Val Gly Met Asp Tyr Val Asn Thr Ala Ile Gln
                325                 330                 335
cgc cta gag agg aac gat gtc agg tac cgc ttc gtt gtc gac gtc gcg      1056
Arg Leu Glu Arg Asn Asp Val Arg Tyr Arg Phe Val Val Asp Val Ala
            340                 345                 350
ggc agc aag att gga ggc tag                                          1077
Gly Ser Lys Ile Gly Gly
        355

<210> 217
<211> 358
<212> PRT
<213> Zea mays

<400> 217
Met Ala Gly Gly Lys Glu Ala His Gly Trp Ala Ala Arg Asp Val Ser
1               5                   10                  15
Gly His Leu Ser Pro Tyr His Phe Ser Arg Arg Val Gln Arg Asp Asp
            20                  25                  30
Asp Val Thr Ile Lys Val Leu Phe Cys Gly Leu Cys His Thr Asp Leu
        35                  40                  45
His Val Ile Lys Asn Glu Phe Gly Asn Ala Lys Tyr Pro Val Val Pro
    50                  55                  60
Gly His Glu Ile Val Gly Val Val Thr Asp Val Gly Ser Gly Val Thr
65                  70                  75                  80
Ser Phe Lys Pro Gly Asp Thr Val Gly Val Gly Tyr Phe Val Asp Ser
                85                  90                  95
Cys Arg Ser Cys Asp Ser Cys Ser Lys Gly Tyr Glu Ser Tyr Cys Pro
            100                 105                 110
Gln Leu Val Glu Thr Ser Asn Gly Val Ser Leu Asp Asp Asp Asp Gly
        115                 120                 125
Gly Ala Thr Thr Lys Gly Gly Phe Ser Asp Ala Leu Val Val His Gln
    130                 135                 140
Arg Tyr Val Val Arg Val Pro Ala Ser Leu Pro Pro Ala Gly Ala Ala
145                 150                 155                 160
Pro Leu Leu Cys Ala Gly Val Thr Val Phe Ser Pro Met Val Gln Tyr
                165                 170                 175
Gly Leu Asn Ala Pro Gly Lys His Leu Gly Val Val Gly Leu Gly Gly
            180                 185                 190
Leu Gly His Leu Ala Val Arg Phe Gly Lys Ala Phe Gly Met Lys Val
        195                 200                 205
Thr Val Ile Ser Thr Ser Leu Gly Lys Arg Asp Glu Ala Leu Gly Arg
    210                 215                 220
Leu Gly Ala Asp Ala Phe Leu Val Ser Arg Asp Pro Glu Gln Met Arg
225                 230                 235                 240
Ala Ala Ala Gly Thr Leu Asp Gly Val Ile Asp Thr Val Ser Ala Asp
                245                 250                 255
His Pro Val Val Pro Leu Leu Asp Leu Leu Lys Pro Met Gly Gln Met
            260                 265                 270
Val Val Val Gly Leu Pro Thr Lys Pro Leu Gln Val Pro Ala Phe Ser
        275                 280                 285
Leu Val Ala Gly Gly Lys Arg Val Ala Gly Ser Ala Gly Gly Gly Val
    290                 295                 300
Gly Glu Cys Gln Ala Met Leu Asp Phe Ala Gly Glu His Gly Ile Thr
305                 310                 315                 320
Ala Asp Val Glu Val Val Gly Met Asp Tyr Val Asn Thr Ala Ile Gln
                325                 330                 335

Arg Leu Glu Arg Asn Asp Val Arg Tyr Arg Phe Val Val Asp Val Ala
            340                 345                 350
Gly Ser Lys Ile Gly Gly
            355

<210> 218
<211> 1245
<212> DNA
<213> Zea mays

<220>
<221> CDS
<222> (1)..(1245)

<400> 218
atg tca tat cat tgt cgc gtc ctc gtc ccc gtt cca ttt cat tat ccc      48
Met Ser Tyr His Cys Arg Val Leu Val Pro Val Pro Phe His Tyr Pro
1               5                   10                  15
ccc gga gct ggt gcg cga gtc gga gct ggt gca ggg tca ccc ttc ccc      96
Pro Gly Ala Gly Ala Arg Val Gly Ala Gly Ala Gly Ser Pro Phe Pro
            20                  25                  30
tcg gcc tca aga gct ctg cgg ttg ccg cgg cca agg gcg tcc gtg gag      144
Ser Ala Ser Arg Ala Leu Arg Leu Pro Arg Pro Arg Ala Ser Val Glu
        35                  40                  45
aag cgg gag cag gtg gcg gcg atg gaa gag caa ggc ggc cag gcg gcg      192
Lys Arg Glu Gln Val Ala Ala Met Glu Glu Gln Gly Gly Gln Ala Ala
    50                  55                  60
ctc gga tgg gcg gcc agg gac gac tcc ggc gtc ctc tcc ccc tac agc      240
Leu Gly Trp Ala Ala Arg Asp Asp Ser Gly Val Leu Ser Pro Tyr Ser
65                  70                  75                  80
ttc tcc aga agg gtt cct aaa gac gac gat gtc acg atc aag gtg ctc      288
Phe Ser Arg Arg Val Pro Lys Asp Asp Asp Val Thr Ile Lys Val Leu
                85                  90                  95
tac tgc ggg atc tgc cac acc gac ctg cac gtc atc aag aac gac tgg      336
Tyr Cys Gly Ile Cys His Thr Asp Leu His Val Ile Lys Asn Asp Trp
            100                 105                 110
cga aac gcc atg tac cca gtc gtc ccg ggg cac gag atc gtg ggc gtg      384
Arg Asn Ala Met Tyr Pro Val Val Pro Gly His Glu Ile Val Gly Val
        115                 120                 125
gtg acc ggc gtc ggc ggc ggc gtc acg cgg ttc aag gcc ggc gac acg      432
Val Thr Gly Val Gly Gly Gly Val Thr Arg Phe Lys Ala Gly Asp Thr
    130                 135                 140
gtc ggc gtg ggc tac ttc gtg ggg tcc tgc cgc tcc tgc gac agc tgc      480
Val Gly Val Gly Tyr Phe Val Gly Ser Cys Arg Ser Cys Asp Ser Cys
145                 150                 155                 160
ggc aag ggg gac gac aac tac tgc gcg ggg atc gtg ctc acc tcc aac      528
Gly Lys Gly Asp Asp Asn Tyr Cys Ala Gly Ile Val Leu Thr Ser Asn
                165                 170                 175
ggc gtc gac cac gcg cac ggc ggc gcg ccc acc agg ggg gga ttc tcc      576
Gly Val Asp His Ala His Gly Gly Ala Pro Thr Arg Gly Gly Phe Ser
            180                 185                 190
gac gtc ctg gtc gcg agc gag cac tac gtg gtc cgc gtc ccc gac ggc      624
Asp Val Leu Val Ala Ser Glu His Tyr Val Val Arg Val Pro Asp Gly
        195                 200                 205
ctg gcg ctg gac cgc acc gcg ccg ctg ctc tgc gcc ggc gtc acc gtg      672
Leu Ala Leu Asp Arg Thr Ala Pro Leu Leu Cys Ala Gly Val Thr Val
    210                 215                 220
tac agc ccc atg atg cgc cac ggc ctc aac gag ccc ggc aag cac ctc      720
Tyr Ser Pro Met Met Arg His Gly Leu Asn Glu Pro Gly Lys His Leu
225                 230                 235                 240
ggc gtc gtc ggc ctc ggc ggc ctc ggc cac gtc gcc gtc aag ttc ggc      768
Gly Val Val Gly Leu Gly Gly Leu Gly His Val Ala Val Lys Phe Gly
                245                 250                 255
aag gcc ttc ggg atg aag gtc acc gtc atc agc acg tcc gcc agc aag      816
Lys Ala Phe Gly Met Lys Val Thr Val Ile Ser Thr Ser Ala Ser Lys
            260                 265                 270
cgc cag gag gcc atc gag aac ctc ggc gcg gac gag ttc ctc atc agc      864
Arg Gln Glu Ala Ile Glu Asn Leu Gly Ala Asp Glu Phe Leu Ile Ser
        275                 280                 285

cgg gac gag gac cag atg aag gcg gcg acg ggg acc atg gac ggc atc 912
Arg Asp Glu Asp Gln Met Lys Ala Ala Thr Gly Thr Met Asp Gly Ile
    290                 295                 300
atc gac acg gtg tcg gcg tgg cac ccg atc acg ccg ctg ctg gcg ctg 960
Ile Asp Thr Val Ser Ala Trp His Pro Ile Thr Pro Leu Leu Ala Leu
305                 310                 315                 320
ctg aag ccg ctg ggg cag atg gtg atc gtg ggc gcg ccg agc aag ccg 1008
Leu Lys Pro Leu Gly Gln Met Val Ile Val Gly Ala Pro Ser Lys Pro
                325                 330                 335
ctc gag ctg ccg gcc tac gcc atc gtg ccg ggc ggg aag ggc gtg gct 1056
Leu Glu Leu Pro Ala Tyr Ala Ile Val Pro Gly Gly Lys Gly Val Ala
            340                 345                 350
ggg aac aat gtc ggc agc gtc agg gac tgc cag gcc atg ctc gag ttc 1104
Gly Asn Asn Val Gly Ser Val Arg Asp Cys Gln Ala Met Leu Glu Phe
        355                 360                 365
gcg ggg aag cac ggc atc ggg gcc gag gtc gag gtc atc aag atg gac 1152
Ala Gly Lys His Gly Ile Gly Ala Glu Val Glu Val Ile Lys Met Asp
    370                 375                 380
tac gtc aac acg gcc atg gag cgg ctc gag aag aac gac gtc cgc tac 1200
Tyr Val Asn Thr Ala Met Glu Arg Leu Glu Lys Asn Asp Val Arg Tyr
385                 390                 395                 400
cgc ttc gtc atc gac gtc gcc ggc agc ctc ggc tct gcc gcc tag 1245
Arg Phe Val Ile Asp Val Ala Gly Ser Leu Gly Ser Ala Ala
                405                 410

<210> 219
<211> 414
<212> PRT
<213> Zea mays

<400> 219
Met Ser Tyr His Cys Arg Val Leu Val Pro Val Pro Phe His Tyr Pro
1               5                   10                  15
Pro Gly Ala Gly Ala Arg Val Gly Ala Gly Ala Gly Ser Pro Phe Pro
            20                  25                  30
Ser Ala Ser Arg Ala Leu Arg Leu Pro Arg Pro Arg Ala Ser Val Glu
        35                  40                  45
Lys Arg Glu Gln Val Ala Ala Met Glu Glu Gln Gly Gly Gln Ala Ala
    50                  55                  60
Leu Gly Trp Ala Ala Arg Asp Asp Ser Gly Val Leu Ser Pro Tyr Ser
65                  70                  75                  80
Phe Ser Arg Arg Val Pro Lys Asp Asp Asp Val Thr Ile Lys Val Leu
                85                  90                  95
Tyr Cys Gly Ile Cys His Thr Asp Leu His Val Ile Lys Asn Asp Trp
            100                 105                 110
Arg Asn Ala Met Tyr Pro Val Val Pro Gly His Glu Ile Val Gly Val
        115                 120                 125
Val Thr Gly Val Gly Gly Gly Val Thr Arg Phe Lys Ala Gly Asp Thr
    130                 135                 140
Val Gly Val Gly Tyr Phe Val Gly Ser Cys Arg Ser Cys Asp Ser Cys
145                 150                 155                 160
Gly Lys Gly Asp Asp Asn Tyr Cys Ala Gly Ile Val Leu Thr Ser Asn
                165                 170                 175
Gly Val Asp His Ala His Gly Gly Ala Pro Thr Arg Gly Gly Phe Ser
            180                 185                 190
Asp Val Leu Val Ala Ser Glu His Tyr Val Val Arg Val Pro Asp Gly
        195                 200                 205
Leu Ala Leu Asp Arg Thr Ala Pro Leu Leu Cys Ala Gly Val Thr Val
    210                 215                 220
Tyr Ser Pro Met Met Arg His Gly Leu Asn Glu Pro Gly Lys His Leu
225                 230                 235                 240
Gly Val Val Gly Leu Gly Gly Leu Gly His Val Ala Val Lys Phe Gly
                245                 250                 255
Lys Ala Phe Gly Met Lys Val Thr Val Ile Ser Thr Ser Ala Ser Lys
            260                 265                 270
Arg Gln Glu Ala Ile Glu Asn Leu Gly Ala Asp Glu Phe Leu Ile Ser
        275                 280                 285
Arg Asp Glu Asp Gln Met Lys Ala Ala Thr Gly Thr Met Asp Gly Ile
    290                 295                 300

Ile Asp Thr Val Ser Ala Trp His Pro Ile Thr Pro Leu Leu Ala Leu
305                 310                 315                 320
Leu Lys Pro Leu Gly Gln Met Val Ile Val Gly Ala Pro Ser Lys Pro
                325                 330                 335
Leu Glu Leu Pro Ala Tyr Ala Ile Val Pro Gly Gly Lys Gly Val Ala
            340                 345                 350
Gly Asn Asn Val Gly Ser Val Arg Asp Cys Gln Ala Met Leu Glu Phe
        355                 360                 365
Ala Gly Lys His Gly Ile Gly Ala Glu Val Glu Val Ile Lys Met Asp
    370                 375                 380
Tyr Val Asn Thr Ala Met Glu Arg Leu Glu Lys Asn Asp Val Arg Tyr
385                 390                 395                 400
Arg Phe Val Ile Asp Val Ala Gly Ser Leu Gly Ser Ala Ala
                405                 410

<210> 220
<211> 1104
<212> DNA
<213> Zea mays

<220>
<221> CDS
<222> (1)..(1104)

<400> 220
atg ggg agc ctg gcg tcc gag agg aag gtg gtc ggg tgg gcc gcc agg      48
Met Gly Ser Leu Ala Ser Glu Arg Lys Val Val Gly Trp Ala Ala Arg
1               5                   10                  15
gac gcc acc gga cac ctc tcc ccc tac tcc tac acc ctc agg aac aca      96
Asp Ala Thr Gly His Leu Ser Pro Tyr Ser Tyr Thr Leu Arg Asn Thr
            20                  25                  30
ggc cct gaa gat gtg gtg gtg aag gtg ctc tac tgc ggg atc tgc cac      144
Gly Pro Glu Asp Val Val Val Lys Val Leu Tyr Cys Gly Ile Cys His
        35                  40                  45
acg gac atc cac cag gcc aag aac cac ctc ggg gct tca aag tat cct      192
Thr Asp Ile His Gln Ala Lys Asn His Leu Gly Ala Ser Lys Tyr Pro
    50                  55                  60
atg gtc cct ggg cac gag gtg gtc ggc gag gtg gtg gag gtc ggg ccc      240
Met Val Pro Gly His Glu Val Val Gly Glu Val Val Glu Val Gly Pro
65                  70                  75                  80
gag gtg gcc aag tac ggc gtc ggc gac gtg gta ggc gtc ggg gtg atc      288
Glu Val Ala Lys Tyr Gly Val Gly Asp Val Val Gly Val Gly Val Ile
                85                  90                  95
gtt ggg tgc tgc cgc gag tgc agc ccc tgc aag gcc aac gtt gag cag      336
Val Gly Cys Cys Arg Glu Cys Ser Pro Cys Lys Ala Asn Val Glu Gln
            100                 105                 110
tac tgc aac aag aag atc tgg tca tac aac gac gtc tac act gat gga      384
Tyr Cys Asn Lys Lys Ile Trp Ser Tyr Asn Asp Val Tyr Thr Asp Gly
        115                 120                 125
cgg ccc acg cag ggt gga ttc gcc tcc acc atg gtc gtc gac cag aag      432
Arg Pro Thr Gln Gly Gly Phe Ala Ser Thr Met Val Val Asp Gln Lys
    130                 135                 140
ttt gtg gtg aag atc ccg gcg ggt ctg gct ccg gag caa gcg gcg ccg      480
Phe Val Val Lys Ile Pro Ala Gly Leu Ala Pro Glu Gln Ala Ala Pro
145                 150                 155                 160
ctg ctg tgc gct ggc gtg acg gtg tac agc ccg ctg aag cac ttt ggg      528
Leu Leu Cys Ala Gly Val Thr Val Tyr Ser Pro Leu Lys His Phe Gly
                165                 170                 175
ctg acg acc ccg ggc ctc cgt ggc ggc atc ctg ggc ctc ggc ggc gtg      576
Leu Thr Thr Pro Gly Leu Arg Gly Gly Ile Leu Gly Leu Gly Gly Val
            180                 185                 190
ggc cac atg ggc gtg aag gta gcc aag gcc atg ggc cac cac gtg acg      624
Gly His Met Gly Val Lys Val Ala Lys Ala Met Gly His His Val Thr
        195                 200                 205
gtg atc agc tcg tcg tcc aag aag cgc gcg gag gca atg gac cac ctc      672
Val Ile Ser Ser Ser Ser Lys Lys Arg Ala Glu Ala Met Asp His Leu
    210                 215                 220
ggc gcg gac gcg tac cta gtg agc tcg gac gcc gcg gcc atg gcg gcg      720
Gly Ala Asp Ala Tyr Leu Val Ser Ser Asp Ala Ala Ala Met Ala Ala

225                 230                 235                 240
gcc gcc gac tcg ctg gac tac atc atc gac acg gtg ccc gtg cac cac       768
Ala Ala Asp Ser Leu Asp Tyr Ile Ile Asp Thr Val Pro Val His His
                245                 250                 255
ccg ctg gag ccg tac ctg gcg ctg ctg aag ctg gac ggc aag ctc gtg       816
Pro Leu Glu Pro Tyr Leu Ala Leu Leu Lys Leu Asp Gly Lys Leu Val
            260                 265                 270
ctg ctg ggc gtc atc ggc gag ccc ctg agc ttc gtg tcg ccc atg gtg       864
Leu Leu Gly Val Ile Gly Glu Pro Leu Ser Phe Val Ser Pro Met Val
        275                 280                 285
atg ctg ggg cgg aag gcc atc acg ggg agc ttc atc ggc agc atc gac       912
Met Leu Gly Arg Lys Ala Ile Thr Gly Ser Phe Ile Gly Ser Ile Asp
    290                 295                 300
gag acc gct gag gtg ctt cag ttc tgc gtc gac aag ggg ctc acc tcc       960
Glu Thr Ala Glu Val Leu Gln Phe Cys Val Asp Lys Gly Leu Thr Ser
305                 310                 315                 320
cag atc gag gtg gtc aag atg ggg tac gtg aac gag gcg ctg gag cgg       1008
Gln Ile Glu Val Val Lys Met Gly Tyr Val Asn Glu Ala Leu Glu Arg
                325                 330                 335
ctg gag cgc aac gac gtc cgc tac cgc ttc gtc gtc gac gtc gcc ggt       1056
Leu Glu Arg Asn Asp Val Arg Tyr Arg Phe Val Val Asp Val Ala Gly
            340                 345                 350
agc aac gtc gag gcg gag gcg gcg gcg gcg gat gcg gcc agc aac           1101
Ser Asn Val Glu Ala Glu Ala Ala Ala Ala Asp Ala Ala Ser Asn
        355                 360                 365
tga                                                                   1104



<210> 221
<211> 367
<212> PRT
<213> Zea mays

<400> 221
Met Gly Ser Leu Ala Ser Glu Arg Lys Val Val Gly Trp Ala Ala Arg
1               5                   10                  15
Asp Ala Thr Gly His Leu Ser Pro Tyr Ser Tyr Thr Leu Arg Asn Thr
            20                  25                  30
Gly Pro Glu Asp Val Val Val Lys Val Leu Tyr Cys Gly Ile Cys His
        35                  40                  45
Thr Asp Ile His Gln Ala Lys Asn His Leu Gly Ala Ser Lys Tyr Pro
    50                  55                  60
Met Val Pro Gly His Glu Val Val Gly Glu Val Val Glu Val Gly Pro
65                  70                  75                  80
Glu Val Ala Lys Tyr Gly Val Gly Asp Val Val Gly Val Gly Val Ile
                85                  90                  95
Val Gly Cys Cys Arg Glu Cys Ser Pro Cys Lys Ala Asn Val Glu Gln
            100                 105                 110
Tyr Cys Asn Lys Lys Ile Trp Ser Tyr Asn Asp Val Tyr Thr Asp Gly
        115                 120                 125
Arg Pro Thr Gln Gly Gly Phe Ala Ser Thr Met Val Val Asp Gln Lys
    130                 135                 140
Phe Val Val Lys Ile Pro Ala Gly Leu Ala Pro Glu Gln Ala Ala Pro
145                 150                 155                 160
Leu Leu Cys Ala Gly Val Thr Val Tyr Ser Pro Leu Lys His Phe Gly
                165                 170                 175
Leu Thr Thr Pro Gly Leu Arg Gly Gly Ile Leu Gly Leu Gly Gly Val
            180                 185                 190
Gly His Met Gly Val Lys Val Ala Lys Ala Met Gly His His Val Thr
        195                 200                 205
Val Ile Ser Ser Ser Ser Lys Lys Arg Ala Glu Ala Met Asp His Leu
    210                 215                 220
Gly Ala Asp Ala Tyr Leu Val Ser Ser Asp Ala Ala Ala Met Ala Ala
225                 230                 235                 240
Ala Ala Asp Ser Leu Asp Tyr Ile Ile Asp Thr Val Pro Val His His
                245                 250                 255
Pro Leu Glu Pro Tyr Leu Ala Leu Leu Lys Leu Asp Gly Lys Leu Val
            260                 265                 270

Leu Leu Gly Val Ile Gly Glu Pro Leu Ser Phe Val Ser Pro Met Val
        275                 280                 285
Met Leu Gly Arg Lys Ala Ile Thr Gly Ser Phe Ile Gly Ser Ile Asp
    290                 295                 300
Glu Thr Ala Glu Val Leu Gln Phe Cys Val Asp Lys Gly Leu Thr Ser
305                 310                 315                 320
Gln Ile Glu Val Val Lys Met Gly Tyr Val Asn Glu Ala Leu Glu Arg
                325                 330                 335
Leu Glu Arg Asn Asp Val Arg Tyr Arg Phe Val Val Asp Val Ala Gly
            340                 345                 350
Ser Asn Val Glu Ala Glu Ala Ala Ala Ala Asp Ala Ala Ser Asn
        355                 360                 365

<210> 222
<211> 20
<212> DNA
<213> Artificial sequence

<400> 222
atggcaacct caccggaatc                                                   20

<210> 223
<211> 22
<212> DNA
<213> Artificial sequence

<400> 223
ctattcagcc ttgatcgagt tc                                                22


<210> 224
<211> 457
<212> PRT
<213> Artificial sequence

<220>
<221> Variant
<222> (2)..(2)
<223> xaa in position 2 is any amino acid
<220>
<221> Variant
<222> (5)..(5)
<223> xaa in position 5 is any amino acid
<220>
<221> Variant
<222> (7)..(7)
<223> xaa in position 7 is any amino acid
<220>
<221> Variant
<222> (9)..(16)
<223> xaa in position 9 to 16 is any amino acid
<220>
<221> Variant
<222> (17)..(31)
<223> xaa in position 17 to 31 is any or no amino acid
<220>
<221> Variant
<222> (35)..(36)
<223> xaa in position 35 to 36 is any amino acid
<220>
<221> Variant
<222> (38)..(38)
<223> xaa in position 38 is any amino acid
<220>
<221> Variant
<222> (40)..(42)
<223> xaa in position 40 to 42 is any amino acid
<220>
<221> Variant

<222> (44)..(46)
<223> xaa in position 44 to 46 is any amino acid
<220>
<221> Variant
<222> (47)..(48)
<223> xaa in position 47 to 48 is any or no amino acid
<220>
<221> Variant
<222> (50)..(54)
<223> xaa in position 50 to 54 is any amino acid
<220>
<221> Variant
<222> (57)..(57)
<223> xaa in position 57 is any amino acid
<220>
<221> Variant
<222> (60)..(66)
<223> xaa in position 60 to 66 is any amino acid
<220>
<221> Variant
<222> (68)..(69)
<223> xaa in position 68 to 69 is any amino acid
<220>
<221> Variant
<222> (70)..(74)
<223> xaa in position 70 to 74 is any or no amino acid
<220>
<221> Variant
<222> (76)..(77)
<223> xaa in position 76 to 77 is any amino acid
<220>
<221> Variant
<222> (79)..(82)
<223> xaa in position 79 to 82 is any amino acid
<220>
<221> Variant
<222> (84)..(85)
<223> xaa in position 84 to 85 is any amino acid
<220>
<221> Variant
<222> (87)..(98)
<223> xaa in position 87 to 98 is any amino acid
<220>
<221> Variant
<222> (99)..(115)
<223> xaa in position 99 to 115 is any or no amino acid
<220>
<221> Variant
<222> (117)..(131)
<223> xaa in position 117 to 131 is any amino acid
<220>
<221> Variant
<222> (133)..(139)
<223> xaa in position 133 to 139 is any amino acid
<220>
<221> Variant
<222> (140)..(148)
<223> xaa in position 140 to 148 is any or no amino acid
<220>
<221> Variant
<222> (151)..(151)
<223> xaa in position 151 is any amino acid
<220>
<221> Variant
<222> (156)..(156)
<223> xaa in position 156 is any amino acid
<220>
<221> Variant
<222> (158)..(158)

<223> xaa in position 158 is any amino acid
<220>
<221> Variant
<222> (160)..(169)
<223> xaa in position 160 to 169 is any amino acid
<220>
<221> Variant
<222> (170)..(173)
<223> xaa in position 170 to 173 is any or no amino acid
<220>
<221> Variant
<222> (175)..(180)
<223> xaa in position 175 to 180 is any amino acid
<220>
<221> Variant
<222> (182)..(182)
<223> xaa in position 182 is any amino acid
<220>
<221> Variant
<222> (188)..(192)
<223> xaa in position 188 to 192 is any amino acid
<220>
<221> Variant
<222> (194)..(196)
<223> xaa in position 194 to 196 is any amino acid
<220>
<221> Variant
<222> (198)..(215)
<223> xaa in position 198 to 215 is any amino acid
<220>
<221> Variant
<222> (216)..(222)
<223> xaa in position 216 to 222 is any or no amino acid
<220>
<221> Variant
<222> (225)..(261)
<223> xaa in position 225 to 261 is any amino acid
<220>
<221> Variant
<222> (262)..(301)
<223> xaa in position 262 to 301 is any or no amino acid
<220>
<221> Variant
<222> (303)..(307)
<223> xaa in position 303 to 307 is any amino acid
<220>
<221> Variant
<222> (309)..(325)
<223> xaa in position 309 to 325 is any amino acid
<220>
<221> Variant
<222> (326)..(358)
<223> xaa in position 326 to 358 is any or no amino acid
<220>
<221> Variant
<222> (361)..(362)
<223> xaa in position 361 to 362 is any amino acid
<220>
<221> Variant
<222> (364)..(405)
<223> xaa in position 364 to 405 is any amino acid
<220>
<221> Variant
<222> (406)..(454)
<223> xaa in position 406 to 454 is any or no amino acid
<220>
<221> Variant
<222> (456)..(456)
<223> xaa in position 456 is any amino acid

<400> 224
Gly xaa Cys His xaa Asp xaa His xaa xaa xaa xaa xaa xaa xaa xaa
1               5                   10                  15
xaa xaa xaa xaa xaa xaa xaa xaa xaa xaa xaa xaa xaa xaa xaa Gly
            20                  25                  30
His Glu xaa xaa Gly xaa Val xaa xaa xaa Gly xaa xaa xaa xaa xaa
        35                  40                  45
Val xaa xaa xaa xaa xaa Gly Asp xaa Val Gly xaa xaa xaa xaa xaa
    50                  55                  60
xaa xaa Cys xaa xaa xaa xaa xaa xaa xaa Cys xaa xaa Cys xaa xaa
65                  70                  75                  80
xaa xaa Glu xaa xaa Cys xaa xaa xaa xaa xaa xaa xaa xaa xaa xaa
                85                  90                  95
xaa xaa xaa xaa xaa xaa xaa xaa xaa xaa xaa xaa xaa xaa xaa xaa
            100                 105                 110
xaa xaa xaa Gly xaa xaa xaa xaa xaa xaa xaa xaa xaa xaa xaa xaa
        115                 120                 125
xaa xaa xaa Pro xaa xaa xaa xaa xaa xaa xaa xaa xaa xaa xaa xaa
    130                 135                 140
xaa xaa xaa xaa Ala Pro xaa Leu Cys Ala Gly xaa Thr xaa Tyr xaa
145                 150                 155                 160
xaa xaa xaa xaa xaa xaa xaa xaa xaa xaa xaa xaa Gly xaa xaa
                165                 170                 175
xaa xaa xaa xaa Gly xaa Gly Gly Leu Gly His xaa xaa xaa xaa xaa
            180                 185                 190
Ala xaa xaa xaa Gly xaa xaa xaa xaa xaa xaa xaa xaa xaa xaa xaa
        195                 200                 205
xaa xaa xaa xaa xaa xaa xaa xaa xaa xaa xaa xaa xaa xaa Gly Ala
    210                 215                 220
xaa xaa xaa xaa xaa xaa xaa xaa xaa xaa xaa xaa xaa xaa xaa xaa
225                 230                 235                 240
xaa xaa xaa xaa xaa xaa xaa xaa xaa xaa xaa xaa xaa xaa xaa xaa
                245                 250                 255
xaa xaa xaa xaa xaa xaa xaa xaa xaa xaa xaa xaa xaa xaa xaa xaa
            260                 265                 270
xaa xaa xaa xaa xaa xaa xaa xaa xaa xaa xaa xaa xaa xaa xaa xaa
        275                 280                 285
xaa xaa xaa xaa xaa xaa xaa xaa xaa xaa xaa xaa xaa Gly xaa xaa
    290                 295                 300
xaa xaa xaa Gly xaa xaa xaa xaa xaa xaa xaa xaa xaa xaa xaa xaa
305                 310                 315                 320
xaa xaa xaa xaa xaa xaa xaa xaa xaa xaa xaa xaa xaa xaa xaa xaa
                325                 330                 335
xaa xaa xaa xaa xaa xaa xaa xaa xaa xaa xaa xaa xaa xaa xaa xaa
            340                 345                 350
xaa xaa xaa xaa xaa xaa Gly Ser xaa xaa Gly xaa xaa xaa xaa xaa
        355                 360                 365
xaa xaa xaa xaa xaa xaa xaa xaa xaa xaa xaa xaa xaa xaa xaa xaa
    370                 375                 380
xaa xaa xaa xaa xaa xaa xaa xaa xaa xaa xaa xaa xaa xaa xaa xaa
385                 390                 395                 400
xaa xaa xaa xaa xaa xaa xaa xaa xaa xaa xaa xaa xaa xaa xaa xaa
                405                 410                 415
xaa xaa xaa xaa xaa xaa xaa xaa xaa xaa xaa xaa xaa xaa xaa xaa
            420                 425                 430
xaa xaa xaa xaa xaa xaa xaa xaa xaa xaa xaa xaa xaa xaa xaa xaa
        435                 440                 445
xaa xaa xaa xaa xaa xaa Arg xaa Val
    450                 455

<210> 225
<211> 93
<212> PRT
<213> Artificial sequence

<220>
<221> Variant

<222> (1)..(1)
<223> Xaa in position 1 is Ile, Val, Leu, Phe or Met
<220>
<221> Variant
<222> (2)..(2)
<223> Xaa in position 2 is any amino acid
<220>
<221> Variant
<222> (3)..(3)
<223> Xaa in position 3 is Ile, Val, Met or Leu
<220>
<221> Variant
<222> (4)..(5)
<223> Xaa in position 4 to 5 is any amino acid
<220>
<221> Variant
<222> (6)..(6)
<223> Xaa in position 6 is Cys, Ser, Ala or Thr
<220>
<221> Variant
<222> (7)..(7)
<223> Xaa in position 7 is Gly or Ala
<220>
<221> Variant
<222> (8)..(8)
<223> Xaa in position 8 is Val, Ile, Leu or Met
<220>
<221> Variant
<222> (10)..(10)
<223> Xaa in position 10 is any amino acid
<220>
<221> Variant
<222> (11)..(11)
<223> Xaa in position 11 is Ser, Thr or Ala
<220>
<221> Variant
<222> (12)..(12)
<223> Xaa in position 12 is Asp or Glu
<220>
<221> Variant
<222> (13)..(13)
<223> Xaa in position 13 is Leu, Ile, Val or Met
<220>
<221> Variant
<222> (14)..(18)
<223> Xaa in position 14 to 18 is any amino acid
<220>
<221> Variant
<222> (19)..(33)
<223> Xaa in position 19 to 33 is any or no amino acid
<220>
<221> Variant
<222> (34)..(34)
<223> Xaa in position 34 is Pro, Gly or Asn
<220>
<221> Variant
<222> (35)..(35)
<223> Xaa in position 35 is any amino acid
<220>
<221> Variant
<222> (36)..(36)
<223> Xaa in position 36 is Val, Ile, Thr, Cys or Ala
<220>
<221> Variant
<222> (37)..(37)
<223> Xaa in position 37 is any amino acid
<220>
<221> Variant
<222> (40)..(40)

<223> Xaa in position 40 is Glu or Gln
<220>
<221> Variant
<222> (41)..(41)
<223> Xaa in position 41 is Ile, Gly or Val
<220>
<221> Variant
<222> (42)..(42)
<223> Xaa in position 42 is Val, Ile, Ala or Thr
<220>
<221> Variant
<222> (44)..(44)
<223> Xaa in position 44 is any amino acid
<220>
<221> Variant
<222> (45)..(45)
<223> Xaa in position 45 is Val, Ile or Ala
<220>
<221> Variant
<222> (46)..(47)
<223> Xaa in position 46 to 47 is any amino acid
<220>
<221> Variant
<222> (48)..(48)
<223> Xaa in position 48 is Val, Leu, Ile, Thr or Met
<220>
<221> Variant
<222> (49)..(49)
<223> Xaa in position 49 is Gly or Ala
<220>
<221> Variant
<222> (50)..(57)
<223> Xaa in position 50 to 57 is any amino acid
<220>
<221> Variant
<222> (58)..(60)
<223> Xaa in position 58 to 60 is any or no amino acid
<220>
<221> Variant
<222> (62)..(62)
<223> Xaa in position 62 is Asp, Gln, Glu, Ser or Lys
<220>
<221> Variant
<222> (63)..(63)
<223> Xaa in position 63 is any amino acid
<220>
<221> Variant
<222> (64)..(64)
<223> Xaa in position 64 is Val or Ala
<220>
<221> Variant
<222> (65)..(65)
<223> Xaa in position 65 is Gly, Ser or Ala
<220>
<221> Variant
<222> (66)..(66)
<223> Xaa in position 66 is Val, Ile or Leu
<220>
<221> Variant
<222> (67)..(72)
<223> Xaa in position 67 to 72 is any amino acid
<220>
<221> Variant
<222> (73)..(73)
<223> Xaa in position 73 is Cys or Asp
<220>
<221> Variant
<222> (74)..(81)
<223> Xaa in position 74 to 81 is any or no amino acid

<220>
<221> Variant
<222> (83)..(84)
<223> xaa in position 83 to 84 is any amino acid
<220>
<221> Variant
<222> (86)..(92)
<223> xaa in position 86 to 92 is any amino acid

<400> 225
xaa xaa xaa xaa xaa xaa xaa xaa Cys xaa xaa xaa xaa xaa xaa xaa
1               5                   10                  15
xaa xaa xaa xaa xaa xaa xaa xaa xaa xaa xaa xaa xaa xaa xaa xaa
            20                  25                  30
xaa xaa xaa xaa xaa Gly His xaa xaa xaa Gly xaa xaa xaa xaa xaa
        35                  40                  45
xaa xaa xaa xaa xaa xaa xaa xaa xaa xaa xaa xaa Gly xaa xaa xaa
    50                  55                  60
xaa xaa xaa xaa xaa xaa xaa xaa xaa xaa xaa xaa xaa xaa xaa xaa
65                  70                  75                  80
xaa Cys xaa xaa Cys xaa xaa xaa xaa xaa xaa xaa Cys
                85                  90


<210> 226
<211> 76
<212> PRT
<213> Artificial sequence

<220>
<221> Variant
<222> (1)..(1)
<223> xaa in position 1 is Ala, Ser, Gly or Thr
<220>
<221> Variant
<222> (2)..(2)
<223> xaa in position 2 is Pro or Ser
<220>
<221> Variant
<222> (3)..(3)
<223> xaa in position 3 is Leu, Ile, Met, Val or Phe
<220>
<221> Variant
<222> (4)..(4)
<223> xaa in position 4 is any amino acid
<220>
<221> Variant
<222> (6)..(6)
<223> xaa in position 6 is Ala or Gly
<220>
<221> Variant
<222> (8)..(8)
<223> xaa in position 8 is Ile, Val, Leu, Ala or Ser
<220>
<221> Variant
<222> (10)..(10)
<223> xaa in position 10 is any amino acid
<220>
<221> Variant
<222> (11)..(11)
<223> xaa in position 11 is Tyr, Phe or Trp
<220>
<221> Variant
<222> (12)..(12)
<223> xaa in position 12 is any amino acid
<220>
<221> Variant
<222> (13)..(13)
<223> xaa in position 13 is Pro, Ala, Gly or Ser

<220>
<221> Variant
<222> (14)..(14)
<223> Xaa in position 14 is Leu, Met, Ile, Val or Phe
<220>
<221> Variant
<222> (15)..(23)
<223> Xaa in position 15 to 23 is any amino acid
<220>
<221> Variant
<222> (24)..(29)
<223> Xaa in position 24 to 29 is any or no amino acid
<220>
<221> Variant
<222> (30)..(30)
<223> Xaa in position 30 is Gly, Ala, Val, Ile, Leu or Met
<220>
<221> Variant
<222> (31)..(31)
<223> Xaa in position 31 is Ile, Val, Ser, Pro or Leu
<220>
<221> Variant
<222> (32)..(32)
<223> Xaa in position 32 is any amino acid
<220>
<221> Variant
<222> (33)..(33)
<223> Xaa in position 33 is Gly or Ala
<220>
<221> Variant
<222> (34)..(34)
<223> Xaa in position 34 is any amino acid
<220>
<221> Variant
<222> (36)..(36)
<223> Xaa in position 36 is Gly or Ala
<220>
<221> Variant
<222> (37)..(37)
<223> Xaa in position 37 is Leu, Val or Ile
<220>
<221> Variant
<222> (38)..(38)
<223> Xaa in position 38 is Gly or Ala
<220>
<221> Variant
<222> (39)..(39)
<223> Xaa in position 39 is any amino acid
<220>
<221> Variant
<222> (40)..(40)
<223> Xaa in position 40 is Met, Leu, Val, Ile or Phe
<220>
<221> Variant
<222> (41)..(41)
<223> Xaa in position 41 is Ala, Gly or Leu
<220>
<221> Variant
<222> (42)..(42)
<223> Xaa in position 42 is Val, Ile, Leu or Ala
<220>
<221> Variant
<222> (43)..(43)
<223> Xaa in position 43 is Lys, Gln, Leu or Met
<220>
<221> Variant
<222> (44)..(44)
<223> Xaa in position 44 is any amino acid
<220>

<221> Variant
<222> (45)..(45)
<223> xaa in position 45 is Ala, Gly, Leu or Ser
<220>
<221> Variant
<222> (46)..(48)
<223> xaa in position 46 to 48 is any amino acid
<220>
<221> Variant
<222> (49)..(49)
<223> xaa in position 49 is Gly or Phe
<220>
<221> Variant
<222> (50)..(63)
<223> xaa in position 50 to 63 is any amino acid
<220>
<221> Variant
<222> (64)..(73)
<223> xaa in position 64 to 73 is any or no amino acid
<220>
<221> Variant
<222> (74)..(74)
<223> xaa in position 74 is Leu, Met, Val, Ile or Phe
<220>
<221> Variant
<222> (75)..(75)
<223> xaa in position 75 is Gly or Ala
<220>
<221> Variant
<222> (76)..(76)
<223> xaa in position 76 is Ala, Gly or Val

<400> 226
   Xaa Xaa Xaa Xaa Cys Xaa Gly Xaa Thr Xaa Xaa Xaa Xaa Xaa Xaa Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Gly Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa
   65                  70                  75

<210> 227
<211> 602
<212> DNA
<213> Brassica napus

<220>
<221> CDS
<222> (30)..(431)

<400> 227
gaattcccgg gtcgacagcg aagggaacg atg aca atg atg aga tct ttc tcg      53
                                Met Thr Met Met Arg Ser Phe Ser
                                1               5
atg gca atg ttg ctc gtc gca cta gtt tca tcc atc tct att gtt tct      101
Met Ala Met Leu Leu Val Ala Leu Val Ser Ser Ile Ser Ile Val Ser
    10                  15                  20
tcg gct tct tca tcc cct gaa gcc gag ttt gtt aag aag acc atc tct      149
Ser Ala Ser Ser Ser Pro Glu Ala Glu Phe Val Lys Lys Thr Ile Ser
25                  30                  35                  40
tcc cac aag atc gtt atc ttc tcc aaa tcc tac tgc ccg tat tgc agg      197
Ser His Lys Ile Val Ile Phe Ser Lys Ser Tyr Cys Pro Tyr Cys Arg
                45                  50                  55
aga gcc aaa tct gtg ttc agt gag ctg gat cag gtt cct cat gtt gtg      245
Arg Ala Lys Ser Val Phe Ser Glu Leu Asp Gln Val Pro His Val Val
            60                  65                  70

gag ctt gat gaa aga gaa gat ggg tgg aac gtt cag agt gca ctt gga          293
Glu Leu Asp Glu Arg Glu Asp Gly Trp Asn Val Gln Ser Ala Leu Gly
        75                  80                  85
gag att gtt gga agg cga aca gta cca cag gtt ttc att aac gga aag          341
Glu Ile Val Gly Arg Arg Thr Val Pro Gln Val Phe Ile Asn Gly Lys
    90                  95                  100
cac att gga gga tca gac gat act gta gaa gcg cat gaa agc ggt gaa          389
His Ile Gly Gly Ser Asp Asp Thr Val Glu Ala His Glu Ser Gly Glu
105                 110                 115                 120
ctg gcc aag ctt ctc ggt ctt tcc acc aaa gct gaa ctc tag gttcaatgta       441
Leu Ala Lys Leu Leu Gly Leu Ser Thr Lys Ala Glu Leu
                125                 130
gttgtagttg gagcaatatt caggtgtaag cacttcaatt ttccagtttc atgataactt        501

gtaatgtgtt ctgaaggtta taaacgtctt gtcatggttt tgtgaaactg tattaaagac        561

tacgagttgg attgagatta aaaaaataaa aaaaaaaaaa a                            602

<210> 228
<211> 133
<212> PRT
<213> Brassica napus

<400> 228
Met Thr Met Met Arg Ser Phe Ser Met Ala Met Leu Leu Val Ala Leu
1               5                   10                  15
Val Ser Ser Ile Ser Ile Val Ser Ser Ala Ser Ser Ser Pro Glu Ala
            20                  25                  30
Glu Phe Val Lys Lys Thr Ile Ser Ser His Lys Ile Val Ile Phe Ser
        35                  40                  45
Lys Ser Tyr Cys Pro Tyr Cys Arg Arg Ala Lys Ser Val Phe Ser Glu
    50                  55                  60
Leu Asp Gln Val Pro His Val Val Glu Leu Asp Glu Arg Glu Asp Gly
65                  70                  75                  80
Trp Asn Val Gln Ser Ala Leu Gly Glu Ile Val Gly Arg Arg Thr Val
                85                  90                  95
Pro Gln Val Phe Ile Asn Gly Lys His Ile Gly Gly Ser Asp Asp Thr
            100                 105                 110
Val Glu Ala His Glu Ser Gly Glu Leu Ala Lys Leu Leu Gly Leu Ser
        115                 120                 125
Thr Lys Ala Glu Leu
    130

<210> 229
<211> 23
<212> DNA
<213> Artificial sequence

<400> 229
atgacaatga tgagatcttt ctc                                                23

<210> 230
<211> 20
<212> DNA
<213> Artificial sequence

<400> 230
ctagagttca gctttggtgg                                                    20

<210> 231
<211> 80
<212> PRT
<213> Artificial sequence

<220>
<221> Variant
<222> (2)..(3)
<223> xaa in position 2 to 3 is any amino acid
<220>
<221> Variant
<222> (5)..(53)
<223> xaa in position 5 to 53 is any amino acid
<220>
<221> Variant
<222> (57)..(58)
<223> xaa in position 57 to 58 is any amino acid
<220>
<221> Variant
<222> (60)..(65)
<223> xaa in position 60 to 65 is any amino acid
<220>
<221> Variant
<222> (68)..(77)
<223> xaa in position 68 to 77 is any amino acid
<220>
<221> Variant
<222> (79)..(79)
<223> xaa in position 79 is any amino acid

<400> 231
Lys xaa xaa Cys xaa xaa xaa xaa xaa xaa xaa xaa xaa xaa xaa xaa
1               5                   10                  15
xaa xaa xaa xaa xaa xaa xaa xaa xaa xaa xaa xaa xaa xaa xaa xaa
            20                  25                  30
xaa xaa xaa xaa xaa xaa xaa xaa xaa xaa xaa xaa xaa xaa xaa xaa
        35                  40                  45
xaa xaa xaa xaa xaa Thr Val Pro xaa xaa Phe xaa xaa xaa xaa xaa
    50                  55                  60
xaa Gly Gly xaa xaa xaa xaa xaa xaa xaa xaa xaa xaa Gly xaa Leu
65                  70                  75                  80


<210> 232
<211> 11
<212> PRT
<213> Artificial sequence

<220>
<221> Variant
<222> (1)..(1)
<223> xaa in position 1 is Val, Ile, Met or Ala
<220>
<221> Variant
<222> (2)..(2)
<223> xaa in position 2 is Val, Phe, Ala, Thr or Glu
<220>
<221> Variant
<222> (3)..(3)
<223> xaa in position 3 is Val, Met or Ile
<220>
<221> Variant
<222> (4)..(4)
<223> xaa in position 4 is Phe, Tyr or Ala
<220>
<221> Variant
<222> (5)..(5)
<223> xaa in position 5 is Ser, Ala, Val or Ile
<220>
<221> Variant
<222> (6)..(6)
<223> xaa in position 6 is Lys or Asn
<220>

<221> Variant
<222> (7)..(8)
<223> Xaa in position 7 to 8 is any amino acid
<220>
<221> Variant
<222> (9)..(9)
<223> Xaa in position 9 is Cys or Ile
<220>
<221> Variant
<222> (10)..(10)
<223> Xaa in position 10 is any amino acid
<220>
<221> Variant
<222> (11)..(11)
<223> Xaa in position 11 is Tyr, Phe, Met or Leu

<400> 232
Xaa Xaa Xaa Xaa Xaa Xaa Xaa Xaa Xaa Xaa Xaa
1               5                   10


<210> 233
<211> 14
<212> PRT
<213> Artificial sequence

<220>
<221> Variant
<222> (1)..(1)
<223> Xaa in position 1 is Thr, Ser or Ala
<220>
<221> Variant
<222> (2)..(2)
<223> Xaa in position 2 is Val, Met, Ile or Phe
<220>
<221> Variant
<222> (4)..(4)
<223> Xaa in position 4 is any amino acid
<220>
<221> Variant
<222> (5)..(5)
<223> Xaa in position 5 is Val, Ile or Leu
<220>
<221> Variant
<222> (6)..(6)
<223> Xaa in position 6 is Phe, Tyr, Ile or Val
<220>
<221> Variant
<222> (7)..(7)
<223> Xaa in position 7 is Ile, Val, Phe or Leu
<220>
<221> Variant
<222> (8)..(12)
<223> Xaa in position 8 to 12 is any amino acid
<220>
<221> Variant
<222> (14)..(14)
<223> Xaa in position 14 is Gly, Asp or Ser

<400> 233
Xaa Xaa Pro Xaa Xaa Xaa Xaa Xaa Xaa Xaa Xaa Xaa Gly Xaa
1               5                   10

<210> 234
<211> 735
<212> DNA
<213> Zea mays

<220>

<221> CDS
<222> (1)..(735)

<400> 234
atg aga cag aat ggc gcg gtg atg att cag ttt ggc cat cag atg cct      48
Met Arg Gln Asn Gly Ala Val Met Ile Gln Phe Gly His Gln Met Pro
1               5                   10                  15
gat tac gac tcc ccg gct acc cag tca acc agt gag acg agc cat caa      96
Asp Tyr Asp Ser Pro Ala Thr Gln Ser Thr Ser Glu Thr Ser His Gln
            20                  25                  30
gaa gcg tct gga atg agc gaa ggg agc ctc aac gag cat aat aat gac      144
Glu Ala Ser Gly Met Ser Glu Gly Ser Leu Asn Glu His Asn Asn Asp
        35                  40                  45
cat tca ggc aac ctt gat ggg tac tcg aag agt gac gaa aac aag atg      192
His Ser Gly Asn Leu Asp Gly Tyr Ser Lys Ser Asp Glu Asn Lys Met
    50                  55                  60
atg tca gcg tta tcc ctg ggc aat ccg gaa aca gct tac gca cat aat      240
Met Ser Ala Leu Ser Leu Gly Asn Pro Glu Thr Ala Tyr Ala His Asn
65                  70                  75                  80
ccg aag cct gac cgt act cag tcc ttc gcc ata tca tac cca tat gcc      288
Pro Lys Pro Asp Arg Thr Gln Ser Phe Ala Ile Ser Tyr Pro Tyr Ala
                85                  90                  95
gat cca tac tac ggt ggc gcg gtg gca gca gct tat ggc ccg cat gct      336
Asp Pro Tyr Tyr Gly Gly Ala Val Ala Ala Ala Tyr Gly Pro His Ala
            100                 105                 110
atc atg cac cct cag ctg gtt ggc atg gtt ccg tcc tct cga gtg cca      384
Ile Met His Pro Gln Leu Val Gly Met Val Pro Ser Ser Arg Val Pro
        115                 120                 125
ctg ccg atc gag cca gcc gct gaa gag ccc atc tat gtc aac gcg aag      432
Leu Pro Ile Glu Pro Ala Ala Glu Glu Pro Ile Tyr Val Asn Ala Lys
    130                 135                 140
cag tac cac gct att ctc cgg agg aga cag ctc cgt gca aag cta gag      480
Gln Tyr His Ala Ile Leu Arg Arg Arg Gln Leu Arg Ala Lys Leu Glu
145                 150                 155                 160
gcg gaa aac aag ctc gtg aaa agc cgc aag ccg tac ctc cac gag tct      528
Ala Glu Asn Lys Leu Val Lys Ser Arg Lys Pro Tyr Leu His Glu Ser
                165                 170                 175
cgg cac ctg cac gcg atg aag aga gct cgg gga aca ggc ggg cgg ttc      576
Arg His Leu His Ala Met Lys Arg Ala Arg Gly Thr Gly Gly Arg Phe
            180                 185                 190
ctg aac acg aag cag cag ccg gag tcc ccc ggc agc ggc ggc tcc tcg      624
Leu Asn Thr Lys Gln Gln Pro Glu Ser Pro Gly Ser Gly Gly Ser Ser
        195                 200                 205
gac gcg caa cgc gtg ccc gcg acc gcg agc ggc ggc ctg ttc acg aag      672
Asp Ala Gln Arg Val Pro Ala Thr Ala Ser Gly Gly Leu Phe Thr Lys
    210                 215                 220
cat gag cac agc ctg ccg ccc ggc ggt cgc cac cac tat cac gcg aga      720
His Glu His Ser Leu Pro Pro Gly Gly Arg His His Tyr His Ala Arg
225                 230                 235                 240
ggg ggc ggt gag tag                                                  735
Gly Gly Gly Glu


<210> 235
<211> 244
<212> PRT
<213> Zea mays

<400> 235
Met Arg Gln Asn Gly Ala Val Met Ile Gln Phe Gly His Gln Met Pro
1               5                   10                  15
Asp Tyr Asp Ser Pro Ala Thr Gln Ser Thr Ser Glu Thr Ser His Gln
            20                  25                  30
Glu Ala Ser Gly Met Ser Glu Gly Ser Leu Asn Glu His Asn Asn Asp
        35                  40                  45
His Ser Gly Asn Leu Asp Gly Tyr Ser Lys Ser Asp Glu Asn Lys Met
    50                  55                  60
Met Ser Ala Leu Ser Leu Gly Asn Pro Glu Thr Ala Tyr Ala His Asn
65                  70                  75                  80

Pro Lys Pro Asp Arg Thr Gln Ser Phe Ala Ile Ser Tyr Pro Tyr Ala
                85                  90                  95
Asp Pro Tyr Tyr Gly Gly Ala Val Ala Ala Ala Tyr Gly Pro His Ala
            100                 105                 110
Ile Met His Pro Gln Leu Val Gly Met Val Pro Ser Ser Arg Val Pro
        115                 120                 125
Leu Pro Ile Glu Pro Ala Ala Glu Glu Pro Ile Tyr Val Asn Ala Lys
    130                 135                 140
Gln Tyr His Ala Ile Leu Arg Arg Arg Gln Leu Arg Ala Lys Leu Glu
145                 150                 155                 160
Ala Glu Asn Lys Leu Val Lys Ser Arg Lys Pro Tyr Leu His Glu Ser
                165                 170                 175
Arg His Leu His Ala Met Lys Arg Ala Arg Gly Thr Gly Gly Arg Phe
            180                 185                 190
Leu Asn Thr Lys Gln Gln Pro Glu Ser Pro Gly Ser Gly Gly Ser Ser
        195                 200                 205
Asp Ala Gln Arg Val Pro Ala Thr Ala Ser Gly Gly Leu Phe Thr Lys
    210                 215                 220
His Glu His Ser Leu Pro Pro Gly Gly Arg His His Tyr His Ala Arg
225                 230                 235                 240
Gly Gly Gly Glu


<210> 236
<211> 591
<212> DNA
<213> Brassica napus

<220>
<221> CDS
<222> (1)..(591)

<400> 236
atg act tct tca gtg cat gag ctc tct gat aac aat gaa agt cat aag      48
Met Thr Ser Ser Val His Glu Leu Ser Asp Asn Asn Glu Ser His Lys
1               5                   10                  15
aag caa gaa cgt cca gat tcc caa act cca cca ccg gtt cct tca gga      96
Lys Gln Glu Arg Pro Asp Ser Gln Thr Pro Pro Pro Val Pro Ser Gly
            20                  25                  30
cca tgt tct gaa tct ata gat aca agc tcc gtc tac tca gaa ccg att      144
Pro Cys Ser Glu Ser Ile Asp Thr Ser Ser Val Tyr Ser Glu Pro Ile
        35                  40                  45
gcg cat ggg ctc tac ccg tat cca gat cct tac tac aga agc gtc ttt      192
Ala His Gly Leu Tyr Pro Tyr Pro Asp Pro Tyr Tyr Arg Ser Val Phe
    50                  55                  60
gca cat caa gcc tat ctt cca cat cca tat cct ggg gtc caa atg cag      240
Ala His Gln Ala Tyr Leu Pro His Pro Tyr Pro Gly Val Gln Met Gln
65                  70                  75                  80
tta atg gga atg cat caa caa gtg gtt cca tta cag tgt gat gca gtc      288
Leu Met Gly Met His Gln Gln Val Val Pro Leu Gln Cys Asp Ala Val
                85                  90                  95
gag gag cct gtg ttt gtt aac gca aag caa tac cac ggt ata ctc agg      336
Glu Glu Pro Val Phe Val Asn Ala Lys Gln Tyr His Gly Ile Leu Arg
            100                 105                 110
cgt agg caa tcc agg gca aaa ctt gag gct cgg aac aga gcc atc aag      384
Arg Arg Gln Ser Arg Ala Lys Leu Glu Ala Arg Asn Arg Ala Ile Lys
        115                 120                 125
tca aag aag cca tat atg cat gag tct cgg cat ttg cat gcg ata aac      432
Ser Lys Lys Pro Tyr Met His Glu Ser Arg His Leu His Ala Ile Asn
    130                 135                 140
cga cct aga gga tgt gga ggc cgg ttt ctc aat gcc aag aag aag aat      480
Arg Pro Arg Gly Cys Gly Gly Arg Phe Leu Asn Ala Lys Lys Lys Asn
145                 150                 155                 160
gga gag cgt aag gaa gag gag gag gca acc tct gat gag aac act tca      528
Gly Glu Arg Lys Glu Glu Glu Glu Ala Thr Ser Asp Glu Asn Thr Ser
                165                 170                 175
gaa gca agt tct agc atc agg tct gag aaa gca gcc atg ggt cct aat      576
Glu Ala Ser Ser Ser Ile Arg Ser Glu Lys Ala Ala Met Gly Pro Asn
            180                 185                 190

gtc agt aga tct tga                                                591
Val Ser Arg Ser
        195

<210> 237
<211> 196
<212> PRT
<213> Brassica napus

<400> 237
Met Thr Ser Ser Val His Glu Leu Ser Asp Asn Asn Glu Ser His Lys
1               5                   10                  15
Lys Gln Glu Arg Pro Asp Ser Gln Thr Pro Pro Pro Val Pro Ser Gly
            20                  25                  30
Pro Cys Ser Glu Ser Ile Asp Thr Ser Ser Val Tyr Ser Glu Pro Ile
        35                  40                  45
Ala His Gly Leu Tyr Pro Tyr Pro Asp Pro Tyr Tyr Arg Ser Val Phe
    50                  55                  60
Ala His Gln Ala Tyr Leu Pro His Pro Tyr Pro Gly Val Gln Met Gln
65                  70                  75                  80
Leu Met Gly Met His Gln Gln Val Val Pro Leu Gln Cys Asp Ala Val
                85                  90                  95
Glu Glu Pro Val Phe Val Asn Ala Lys Gln Tyr His Gly Ile Leu Arg
            100                 105                 110
Arg Arg Gln Ser Arg Ala Lys Leu Glu Ala Arg Asn Arg Ala Ile Lys
        115                 120                 125
Ser Lys Lys Pro Tyr Met His Glu Ser Arg His Leu His Ala Ile Asn
    130                 135                 140
Arg Pro Arg Gly Cys Gly Gly Arg Phe Leu Asn Ala Lys Lys Lys Asn
145                 150                 155                 160
Gly Glu Arg Lys Glu Glu Glu Glu Ala Thr Ser Asp Glu Asn Thr Ser
                165                 170                 175
Glu Ala Ser Ser Ser Ile Arg Ser Glu Lys Ala Ala Met Gly Pro Asn
            180                 185                 190
Val Ser Arg Ser
        195

<210> 238
<211> 21
<212> DNA
<213> Artificial sequence

<400> 238
atgagacaga atggcgcggt g                                                21

<210> 239
<211> 20
<212> DNA
<213> Artificial sequence

<400> 239
ctactcaccg ccccctctcg                                                  20


<210> 240
<211> 56
<212> PRT
<213> Artificial sequence

<220>
<221> Variant
<222> (2)..(3)
<223> xaa in position 2 to 3 is any amino acid
<220>
<221> Variant
<222> (9)..(11)
<223> xaa in position 9 to 11 is any amino acid
<220>
<221> Variant

<222> (13)..(14)
<223> xaa in position 13 to 14 is any amino acid
<220>
<221> Variant
<222> (17)..(18)
<223> xaa in position 17 to 18 is any amino acid
<220>
<221> Variant
<222> (20)..(22)
<223> xaa in position 20 to 22 is any amino acid
<220>
<221> Variant
<222> (24)..(34)
<223> xaa in position 24 to 34 is any amino acid
<220>
<221> Variant
<222> (35)..(35)
<223> xaa in position 35 is any or no amino acid
<220>
<221> Variant
<222> (37)..(37)
<223> xaa in position 37 is any amino acid
<220>
<221> Variant
<222> (43)..(43)
<223> xaa in position 43 is any amino acid
<220>
<221> Variant
<222> (46)..(47)
<223> xaa in position 46 to 47 is any amino acid
<220>
<221> Variant
<222> (49)..(49)
<223> xaa in position 49 is any amino acid
<220>
<221> Variant
<222> (51)..(52)
<223> xaa in position 51 to 52 is any amino acid

<400> 240
Pro xaa xaa Val Asn Ala Lys Gln xaa xaa xaa Ile xaa xaa Arg Arg
1               5                   10                  15
xaa xaa Arg xaa xaa xaa Glu xaa xaa xaa xaa xaa xaa xaa xaa xaa
            20                  25                  30
xaa xaa xaa Tyr xaa His Glu Ser Arg His xaa His Ala xaa xaa Arg
        35                  40                  45
xaa Arg xaa xaa Gly Gly Arg Phe
    50                  55


<210> 241
<211> 27
<212> PRT
<213> Artificial sequence

<220>
<221> Variant
<222> (2)..(2)
<223> xaa in position 2 is any amino acid
<220>
<221> Variant
<222> (3)..(3)
<223> xaa in position 3 is Tyr or Phe
<220>
<221> Variant
<222> (9)..(9)
<223> xaa in position 9 is Tyr or Phe
<220>
<221> Variant

<222> (10)..(10)
<223> xaa in position 10 is any amino acid
<220>
<221> Variant
<222> (11)..(11)
<223> xaa in position 11 is Arg, Ala or Gly
<220>
<221> Variant
<222> (13)..(13)
<223> xaa in position 13 is Leu or Met
<220>
<221> Variant
<222> (14)..(14)
<223> xaa in position 14 is Lys or Arg
<220>
<221> Variant
<222> (17)..(17)
<223> xaa in position 17 is any amino acid
<220>
<221> Variant
<222> (18)..(18)
<223> xaa in position 18 is Ala or Ser
<220>
<221> Variant
<222> (20)..(20)
<223> xaa in position 20 is Ala or Gln
<220>
<221> Variant
<222> (21)..(21)
<223> xaa in position 21 is Lys or Arg
<220>
<221> Variant
<222> (22)..(22)
<223> xaa in position 22 is Leu or Ala
<220>
<221> Variant
<222> (24)..(24)
<223> xaa in position 24 is Glu, Ala or Leu
<220>
<221> Variant
<222> (25)..(26)
<223> xaa in position 25 to 26 is any amino acid
<220>
<221> Variant
<222> (27)..(27)
<223> xaa in position 27 is Arg or Lys

<400> 241
Pro xaa xaa Val Asn Ala Lys Gln xaa xaa xaa Ile xaa xaa Arg Arg
1               5                   10                  15
xaa xaa Arg xaa xaa xaa Glu xaa xaa xaa xaa
            20                  25


<210> 242
<211> 26
<212> PRT
<213> Artificial sequence

<220>
<221> Variant
<222> (1)..(1)
<223> xaa in position 1 is Lys or Arg
<220>
<221> Variant
<222> (2)..(2)
<223> xaa in position 2 is any amino acid
<220>
<221> Variant

<222> (3)..(3)
<223> xaa in position 3 is Arg or Lys
<220>
<221> Variant
<222> (4)..(4)
<223> xaa in position 4 is Lys or Arg
<220>
<221> Variant
<222> (5)..(5)
<223> xaa in position 5 is Pro, Lys or Arg
<220>
<221> Variant
<222> (7)..(7)
<223> xaa in position 7 is Leu or Met
<220>
<221> Variant
<222> (13)..(13)
<223> xaa in position 13 is any amino acid
<220>
<221> Variant
<222> (16)..(16)
<223> xaa in position 16 is Met, Ile or Leu
<220>
<221> Variant
<222> (17)..(17)
<223> xaa in position 17 is Arg or Asn
<220>
<221> Variant
<222> (19)..(19)
<223> xaa in position 19 is Pro, Ala or Ile
<220>
<221> Variant
<222> (21)..(21)
<223> xaa in position 21 is Gly, Ala or Ser
<220>
<221> Variant
<222> (22)..(22)
<223> xaa in position 22 is any amino acid

<400> 242
xaa xaa xaa xaa xaa Tyr xaa His Glu Ser Arg His xaa His Ala xaa
1               5                   10                  15
xaa Arg xaa Arg xaa xaa Gly Gly Arg Phe
            20                  25

<210> 243
<211> 1099
<212> DNA
<213> Brassica napus

<220>
<221> CDS
<222> (54)..(923)

<400> 243
aacacattta agaagaaaac caaaaacaaa aaaatcaaga aagaagcaga aga atg      56
                                                            Met
                                                            1
gcc att aac ggt gac gat aag gtt tca gga gaa gaa aca gct tcg tcg      104
Ala Ile Asn Gly Asp Asp Lys Val Ser Gly Glu Glu Thr Ala Ser Ser
            5                   10                  15
aac cgg ccg gtt aga gtc tac gcc gat gga atc tat gat ttg ttc cac      152
Asn Arg Pro Val Arg Val Tyr Ala Asp Gly Ile Tyr Asp Leu Phe His
        20                  25                  30
ttc ggt cac gct cgc gcc atc gaa caa gcc aag aaa tcg ttt ccg aac      200
Phe Gly His Ala Arg Ala Ile Glu Gln Ala Lys Lys Ser Phe Pro Asn
    35                  40                  45
acg tat ctg ctc gta gga tgt tgt aac gac gag atc aca aac aaa ttc      248
Thr Tyr Leu Leu Val Gly Cys Cys Asn Asp Glu Ile Thr Asn Lys Phe

50                  55                  60                  65
aaa gga aag acc gtg atg aca gag tcc gaa cgc tat gaa tct ctc cga      296
Lys Gly Lys Thr Val Met Thr Glu Ser Glu Arg Tyr Glu Ser Leu Arg
                70                  75                  80
cat tgc aag tgg gtc gat gaa gtg att cca gat gca cca tgg gtt ctc      344
His Cys Lys Trp Val Asp Glu Val Ile Pro Asp Ala Pro Trp Val Leu
                85                  90                  95
acc acc gag ttt ctc gat aag cat cag atc gac tac gta gct cat gat      392
Thr Thr Glu Phe Leu Asp Lys His Gln Ile Asp Tyr Val Ala His Asp
            100                 105                 110
gct ctt cca tat gcg gat gct agt gga gct gga aac gat gtc tat gag      440
Ala Leu Pro Tyr Ala Asp Ala Ser Gly Ala Gly Asn Asp Val Tyr Glu
        115                 120                 125
ttt gta aag tcg att ggg aag ttt aaa gaa act aaa cga aca gag ggg      488
Phe Val Lys Ser Ile Gly Lys Phe Lys Glu Thr Lys Arg Thr Glu Gly
    130                 135                 140                 145
ata tcg aca tca gat ata atc atg aga att gtg aaa gat tat aat cag      536
Ile Ser Thr Ser Asp Ile Ile Met Arg Ile Val Lys Asp Tyr Asn Gln
                150                 155                 160
tat gtg ttg cgt aac ttg gac cga gga tat tcc aga gaa gag ctc ggt      584
Tyr Val Leu Arg Asn Leu Asp Arg Gly Tyr Ser Arg Glu Glu Leu Gly
                165                 170                 175
gtc agc ttt gtt aag gag aag agg ctt aga gtg aac gtg aaa ctg aag      632
Val Ser Phe Val Lys Glu Lys Arg Leu Arg Val Asn Val Lys Leu Lys
            180                 185                 190
aaa cta caa gag aaa gtg aaa gaa caa caa gaa aag ata caa acc gta      680
Lys Leu Gln Glu Lys Val Lys Glu Gln Gln Glu Lys Ile Gln Thr Val
        195                 200                 205
gca aaa act gct ggg atg cac cgc aat gag tgg gtt gaa aat gct gat      728
Ala Lys Thr Ala Gly Met His Arg Asn Glu Trp Val Glu Asn Ala Asp
210                 215                 220                 225
cgt tgg gtt gct ggg ttt ctt gaa atg ttt gag gaa ggt tgt cat aaa      776
Arg Trp Val Ala Gly Phe Leu Glu Met Phe Glu Glu Gly Cys His Lys
                230                 235                 240
atg gga aca gcc atc aga gat ggg atc cag caa cgg cta ctg agg cag      824
Met Gly Thr Ala Ile Arg Asp Gly Ile Gln Gln Arg Leu Leu Arg Gln
                245                 250                 255
gaa tca gaa gat gcc gag gat gat gag agc gaa gaa att tct gat gag      872
Glu Ser Glu Asp Ala Glu Asp Asp Glu Ser Glu Glu Ile Ser Asp Glu
            260                 265                 270
gat gag gtt gca gaa ggg gat gaa aac tat cat gtc agg aag gtt tct      920
Asp Glu Val Ala Glu Gly Asp Glu Asn Tyr His Val Arg Lys Val Ser
        275                 280                 285
taa atgagaaggg caacaaagag aatgggatag tgaagaaatg agagttttgg      973

290
ttcagcattc aagaatttcc agatgcggaa gaccaaagtg taacagattc cacagttatg      1033

gactagaatt gtttgttatg ttcaagtgaa tacatgaaaa ctgtacttcc agaaaaaaaa      1093

aaaaaa      1099

<210> 244
<211> 289
<212> PRT
<213> Brassica napus

<400> 244
Met Ala Ile Asn Gly Asp Asp Lys Val Ser Gly Glu Glu Thr Ala Ser
1               5                   10                  15
Ser Asn Arg Pro Val Arg Val Tyr Ala Asp Gly Ile Tyr Asp Leu Phe
            20                  25                  30
His Phe Gly His Ala Arg Ala Ile Glu Gln Ala Lys Lys Ser Phe Pro
        35                  40                  45
Asn Thr Tyr Leu Leu Val Gly Cys Cys Asn Asp Glu Ile Thr Asn Lys

50                      55                      60
Phe Lys Gly Lys Thr Val Met Thr Glu Ser Glu Arg Tyr Glu Ser Leu
65                  70                  75                  80
Arg His Cys Lys Trp Val Asp Glu Val Ile Pro Asp Ala Pro Trp Val
                85                  90                  95
Leu Thr Thr Glu Phe Leu Asp Lys His Gln Ile Asp Tyr Val Ala His
            100                 105                 110
Asp Ala Leu Pro Tyr Ala Asp Ala Ser Gly Ala Gly Asn Asp Val Tyr
        115                 120                 125
Glu Phe Val Lys Ser Ile Gly Lys Phe Lys Glu Thr Lys Arg Thr Glu
    130                 135                 140
Gly Ile Ser Thr Ser Asp Ile Ile Met Arg Ile Val Lys Asp Tyr Asn
145                 150                 155                 160
Gln Tyr Val Leu Arg Asn Leu Asp Arg Gly Tyr Ser Arg Glu Glu Leu
                165                 170                 175
Gly Val Ser Phe Val Lys Glu Lys Arg Leu Arg Val Asn Val Lys Leu
            180                 185                 190
Lys Lys Leu Gln Glu Lys Val Lys Glu Gln Gln Glu Lys Ile Gln Thr
        195                 200                 205
Val Ala Lys Thr Ala Gly Met His Arg Asn Glu Trp Val Glu Asn Ala
    210                 215                 220
Asp Arg Trp Val Ala Gly Phe Leu Glu Met Phe Glu Glu Gly Cys His
225                 230                 235                 240
Lys Met Gly Thr Ala Ile Arg Asp Gly Ile Gln Gln Arg Leu Leu Arg
                245                 250                 255
Gln Glu Ser Glu Asp Ala Glu Asp Asp Glu Ser Glu Glu Ile Ser Asp
            260                 265                 270
Glu Asp Glu Val Ala Glu Gly Asp Glu Asn Tyr His Val Arg Lys Val
        275                 280                 285
Ser


<210> 245
<211> 24
<212> DNA
<213> Artificial sequence

<400> 245
atggccatta acggtgacga taag                                              24

<210> 246
<211> 25
<212> DNA
<213> Artificial sequence

<400> 246
ttaagaaacc ttcctgacat gatag                                             25


<210> 247
<211> 144
<212> PRT
<213> Artificial sequence

<220>
<221> Variant
<222> (3)..(4)
<223> xaa in position 3 to 4 is any amino acid
<220>
<221> Variant
<222> (7)..(8)
<223> xaa in position 7 to 8 is any amino acid
<220>
<221> Variant
<222> (10)..(11)
<223> xaa in position 10 to 11 is any amino acid
<220>
<221> Variant
<222> (13)..(13)

<223> xaa in position 13 is any amino acid
<220>
<221> Variant
<222> (16)..(18)
<223> xaa in position 16 to 18 is any amino acid
<220>
<221> Variant
<222> (20)..(20)
<223> xaa in position 20 is any amino acid
<220>
<221> Variant
<222> (24)..(36)
<223> xaa in position 24 to 36 is any amino acid
<220>
<221> Variant
<222> (38)..(38)
<223> xaa in position 38 is any amino acid
<220>
<221> Variant
<222> (41)..(43)
<223> xaa in position 41 to 43 is any amino acid
<220>
<221> Variant
<222> (45)..(50)
<223> xaa in position 45 to 50 is any amino acid
<220>
<221> Variant
<222> (53)..(55)
<223> xaa in position 53 to 55 is any amino acid
<220>
<221> Variant
<222> (57)..(60)
<223> xaa in position 57 to 60 is any amino acid
<220>
<221> Variant
<222> (63)..(71)
<223> xaa in position 63 to 71 is any amino acid
<220>
<221> Variant
<222> (76)..(78)
<223> xaa in position 76 to 78 is any amino acid
<220>
<221> Variant
<222> (81)..(97)
<223> xaa in position 81 to 97 is any amino acid
<220>
<221> Variant
<222> (99)..(101)
<223> xaa in position 99 to 101 is any amino acid
<220>
<221> Variant
<222> (103)..(115)
<223> xaa in position 103 to 115 is any amino acid
<220>
<221> Variant
<222> (117)..(117)
<223> xaa in position 117 is any amino acid
<220>
<221> Variant
<222> (119)..(121)
<223> xaa in position 119 to 121 is any amino acid
<220>
<221> Variant
<222> (123)..(124)
<223> xaa in position 123 to 124 is any amino acid
<220>
<221> Variant
<222> (126)..(131)
<223> xaa in position 126 to 131 is any amino acid

<220>
<221> Variant
<222> (134)..(134)
<223> xaa in position 134 is any amino acid
<220>
<221> Variant
<222> (136)..(136)
<223> xaa in position 136 is any amino acid
<220>
<221> Variant
<222> (139)..(139)
<223> xaa in position 139 is any amino acid
<220>
<221> Variant
<222> (141)..(143)
<223> xaa in position 141 to 143 is any amino acid

<400> 247
Arg Val xaa xaa Asp Gly xaa xaa Asp xaa xaa His xaa Gly His xaa
1               5                   10                  15
xaa xaa Leu xaa Gln Ala Lys xaa xaa xaa xaa xaa xaa xaa xaa xaa
            20                  25                  30
xaa xaa xaa xaa Leu xaa Val Gly xaa xaa xaa Asp xaa xaa xaa xaa
        35                  40                  45
xaa xaa Lys Gly xaa xaa xaa Val xaa xaa xaa xaa Glu Arg xaa xaa
    50                  55                  60
xaa xaa xaa xaa xaa xaa xaa Val Asp Glu Val xaa xaa xaa Ala Pro
65                  70                  75                  80
xaa xaa xaa xaa xaa xaa xaa xaa xaa xaa xaa xaa xaa xaa xaa xaa
                85                  90                  95
xaa Asp xaa xaa xaa His xaa xaa xaa xaa xaa xaa xaa xaa xaa xaa
            100                 105                 110
xaa xaa xaa Asp xaa Tyr xaa xaa xaa Lys xaa xaa Gly xaa xaa xaa
        115                 120                 125
xaa xaa xaa Arg Thr xaa Gly xaa Ser Thr xaa Asp xaa xaa xaa Arg
    130                 135                 140

<210> 248
<211> 146
<212> PRT
<213> Artificial sequence

<220>
<221> Variant
<222> (1)..(1)
<223> xaa in position 1 is Arg or Lys
<220>
<221> Variant
<222> (2)..(2)
<223> xaa in position 2 is Val, Ile or Leu
<220>
<221> Variant
<222> (3)..(3)
<223> xaa in position 3 is Tyr or Trp
<220>
<221> Variant
<222> (4)..(4)
<223> xaa in position 4 is any amino acid
<220>
<221> Variant
<222> (7)..(7)
<223> xaa in position 7 is Ile, Cys or Val
<220>
<221> Variant
<222> (8)..(8)
<223> xaa in position 8 is Phe, Tyr or Met
<220>

<221> Variant
<222> (10)..(10)
<223> Xaa in position 10 is Leu, Met or Phe
<220>
<221> Variant
<222> (11)..(11)
<223> Xaa in position 11 is any amino acid
<220>
<221> Variant
<222> (13)..(13)
<223> Xaa in position 13 is any amino acid
<220>
<221> Variant
<222> (14)..(14)
<223> Xaa in position 14 is Gly or Ala
<220>
<221> Variant
<222> (15)..(15)
<223> Xaa in position 15 is His or Asn
<220>
<221> Variant
<222> (16)..(18)
<223> Xaa in position 16 to 18 is any amino acid
<220>
<221> Variant
<222> (19)..(19)
<223> Xaa in position 19 is Leu, Ile, Val, Met or Phe
<220>
<221> Variant
<222> (20)..(20)
<223> Xaa in position 20 is any amino acid
<220>
<221> Variant
<222> (21)..(21)
<223> Xaa in position 21 is Gln or Pro
<220>
<221> Variant
<222> (22)..(22)
<223> Xaa in position 22 is any amino acid
<220>
<221> Variant
<222> (23)..(23)
<223> Xaa in position 23 is Lys or Arg
<220>
<221> Variant
<222> (24)..(25)
<223> Xaa in position 24 to 25 is any amino acid
<220>
<221> Variant
<222> (26)..(26)
<223> Xaa in position 26 is Phe, Gly, Ile or Val
<220>
<221> Variant
<222> (27)..(28)
<223> Xaa in position 27 to 28 is any amino acid
<220>
<221> Variant
<222> (29)..(36)
<223> Xaa in position 29 to 36 is any or no amino acid
<220>
<221> Variant
<222> (37)..(37)
<223> Xaa in position 37 is Leu or Val
<220>
<221> Variant
<222> (38)..(38)
<223> Xaa in position 38 is Ile, Val, Leu, Phe or Tyr
<220>
<221> Variant

<222> (39)..(39)
<223> xaa in position 39 is Val, Cys, Ala or Ile
<220>
<221> Variant
<222> (41)..(41)
<223> xaa in position 41 is Val, Cys, Ile or Leu
<220>
<221> Variant
<222> (42)..(43)
<223> xaa in position 42 to 43 is any amino acid
<220>
<221> Variant
<222> (44)..(44)
<223> xaa in position 44 is Asp or Ser
<220>
<221> Variant
<222> (45)..(46)
<223> xaa in position 45 to 46 is any amino acid
<220>
<221> Variant
<222> (47)..(47)
<223> xaa in position 47 is Thr, Ile, Ala or Leu
<220>
<221> Variant
<222> (48)..(50)
<223> xaa in position 48 to 50 is any amino acid
<220>
<221> Variant
<222> (51)..(51)
<223> xaa in position 51 is Lys or Gly
<220>
<221> Variant
<222> (52)..(52)
<223> xaa in position 52 is Gly or Ile
<220>
<221> Variant
<222> (53)..(53)
<223> xaa in position 53 is any amino acid
<220>
<221> Variant
<222> (54)..(54)
<223> xaa in position 54 is any or no amino acid
<220>
<221> Variant
<222> (55)..(55)
<223> xaa in position 55 is Thr, Pro or Leu
<220>
<221> Variant
<222> (56)..(56)
<223> xaa in position 56 is Val or Ile
<220>
<221> Variant
<222> (57)..(60)
<223> xaa in position 57 to 60 is any amino acid
<220>
<221> Variant
<222> (61)..(61)
<223> xaa in position 61 is Glu or Gln
<220>
<221> Variant
<222> (63)..(65)
<223> xaa in position 63 to 65 is any amino acid
<220>
<221> Variant
<222> (66)..(66)
<223> xaa in position 66 is Val, Ile, Leu, Ala or Thr
<220>
<221> Variant
<222> (67)..(69)

<223> xaa in position 67 to 69 is any amino acid
<220>
<221> Variant
<222> (70)..(70)
<223> xaa in position 70 is Lys or Arg
<220>
<221> Variant
<222> (71)..(71)
<223> xaa in position 71 is Trp or Tyr
<220>
<221> Variant
<222> (72)..(72)
<223> xaa in position 72 is Val or Cys
<220>
<221> Variant
<222> (73)..(73)
<223> xaa in position 73 is Asp, Ser or Thr
<220>
<221> Variant
<222> (74)..(74)
<223> xaa in position 74 is Glu, Gln, Asp or Lys
<220>
<221> Variant
<222> (75)..(75)
<223> xaa in position 75 is Val, Ile or Ser
<220>
<221> Variant
<222> (76)..(76)
<223> xaa in position 76 is Ile, Val, Leu or Tyr
<220>
<221> Variant
<222> (77)..(78)
<223> xaa in position 77 to 78 is any amino acid
<220>
<221> Variant
<222> (79)..(79)
<223> xaa in position 79 is Ala or Val
<220>
<221> Variant
<222> (81)..(81)
<223> xaa in position 81 is Trp, Tyr or Phe
<220>
<221> Variant
<222> (82)..(87)
<223> xaa in position 82 to 87 is any amino acid
<220>
<221> Variant
<222> (88)..(88)
<223> xaa in position 88 is Leu, Met, Ile or Val
<220>
<221> Variant
<222> (89)..(98)
<223> xaa in position 89 to 98 is any or no amino acid
<220>
<221> Variant
<222> (99)..(99)
<223> xaa in position 99 is Tyr, Phe, Ile or no amino acid
<220>
<221> Variant
<222> (100)..(100)
<223> xaa in position 100 is any or no amino acid
<220>
<221> Variant
<222> (101)..(101)
<223> xaa in position 101 is Ala, Val, Ile or no amino acid
<220>
<221> Variant
<222> (102)..(102)
<223> xaa in position 102 is His or no amino acid

<220>
<221> Variant
<222> (103)..(103)
<223> Xaa in position 103 is Asp, Gly or no amino acid
<220>
<221> Variant
<222> (104)..(104)
<223> Xaa in position 104 is any or no amino acid
<220>
<221> Variant
<222> (105)..(105)
<223> Xaa in position 105 is Asp, Ile, Leu or no amino acid
<220>
<221> Variant
<222> (106)..(106)
<223> Xaa in position 106 is Pro, Ile or no amino acid
<220>
<221> Variant
<222> (107)..(109)
<223> Xaa in position 107 to 109 is any amino acid
<220>
<221> Variant
<222> (110)..(115)
<223> Xaa in position 110 to 115 is any or no amino acid
<220>
<221> Variant
<222> (116)..(116)
<223> Xaa in position 116 is Asp, Ala, Cys or Lys
<220>
<221> Variant
<222> (117)..(117)
<223> Xaa in position 117 is any amino acid
<220>
<221> Variant
<222> (118)..(118)
<223> Xaa in position 118 is Tyr or Pro
<220>
<221> Variant
<222> (119)..(121)
<223> Xaa in position 119 to 121 is any amino acid
<220>
<221> Variant
<222> (122)..(122)
<223> Xaa in position 122 is Lys, Arg, Gln or Glu
<220>
<221> Variant
<222> (123)..(124)
<223> Xaa in position 123 to 124 is any amino acid
<220>
<221> Variant
<222> (125)..(125)
<223> Xaa in position 125 is Gly, Asn, Gln or Asp
<220>
<221> Variant
<222> (126)..(126)
<223> Xaa in position 126 is any amino acid
<220>
<221> Variant
<222> (127)..(127)
<223> Xaa in position 127 is Phe, Tyr or Leu
<220>
<221> Variant
<222> (128)..(131)
<223> Xaa in position 128 to 131 is any amino acid
<220>
<221> Variant
<222> (132)..(132)
<223> Xaa in position 132 is Arg or Lys
<220>

<221> Variant
<222> (133)..(133)
<223> Xaa in position 133 is Thr, Ser or Lys
<220>
<221> Variant
<222> (134)..(134)
<223> Xaa in position 134 is any amino acid
<220>
<221> Variant
<222> (135)..(135)
<223> Xaa in position 135 is Gly, His or Ser
<220>
<221> Variant
<222> (136)..(136)
<223> Xaa in position 136 is Val, Ile or Met
<220>
<221> Variant
<222> (137)..(137)
<223> Xaa in position 137 is Ser or Glu
<220>
<221> Variant
<222> (138)..(138)
<223> Xaa in position 138 is Thr or Ser
<220>
<221> Variant
<222> (139)..(139)
<223> Xaa in position 139 is Ser, Thr or Asp
<220>
<221> Variant
<222> (140)..(140)
<223> Xaa in position 140 is Asp, Glu, Gly or Thr
<220>
<221> Variant
<222> (141)..(141)
<223> Xaa in position 141 is Ile, Leu, Ser or Val
<220>
<221> Variant
<222> (142)..(142)
<223> Xaa in position 142 is Ile, Val or Leu
<220>
<221> Variant
<222> (143)..(143)
<223> Xaa in position 143 is any amino acid
<220>
<221> Variant
<222> (144)..(144)
<223> Xaa in position 144 is Arg or Lys
<220>
<221> Variant
<222> (145)..(145)
<223> Xaa in position 145 is Ile, Met or Leu
<220>
<221> Variant
<222> (146)..(146)
<223> Xaa in position 146 is Met, Leu, Ile or Val

<400> 248
Xaa Xaa Xaa Xaa Asp Gly Xaa Xaa Asp Xaa Xaa His Xaa Xaa Xaa Xaa
1               5                   10                  15
Xaa Xaa Xaa Xaa Xaa Xaa Xaa Xaa Xaa Xaa Xaa Xaa Xaa Xaa Xaa Xaa
            20                  25                  30
Xaa Xaa Xaa Xaa Xaa Xaa Xaa Gly Xaa Xaa Xaa Xaa Xaa Xaa Xaa Xaa
        35                  40                  45
Xaa Xaa Xaa Xaa Xaa Xaa Xaa Xaa Xaa Xaa Xaa Xaa Xaa Arg Xaa Xaa
    50                  55                  60
Xaa Xaa Xaa Xaa Xaa Xaa Xaa Xaa Xaa Xaa Xaa Xaa Xaa Xaa Xaa Pro
65                  70                  75                  80
Xaa Xaa Xaa Xaa Xaa Xaa Xaa Xaa Xaa Xaa Xaa Xaa Xaa Xaa Xaa Xaa
                85                  90                  95

xaa xaa xaa xaa xaa xaa xaa xaa xaa xaa xaa xaa xaa xaa xaa xaa
            100                 105                 110
xaa xaa xaa xaa xaa xaa xaa xaa xaa xaa xaa xaa xaa xaa xaa xaa
        115                 120                 125
xaa xaa xaa xaa xaa xaa xaa xaa xaa xaa xaa xaa xaa xaa xaa xaa
    130                 135                 140
xaa xaa
145

<210> 249
<211> 993
<212> DNA
<213> Brassica napus

<220>
<221> CDS
<222> (85)..(837)

<400> 249
gaattcccgg gtcgaccgag caagaatcaa agaagaggcg atcagagatc gaaagctgtc      60

ccttcctctc aggtttgtta gaag atg tct cga aga tat gat agc cgc acc        111
                           Met Ser Arg Arg Tyr Asp Ser Arg Thr
                           1               5
aca atc ttc tcc cca gaa ggc cgt ctc tac caa gtc gag tac gcc atg       159
Thr Ile Phe Ser Pro Glu Gly Arg Leu Tyr Gln Val Glu Tyr Ala Met
10                  15                  20                  25
gag gcc att ggc aac gcc ggc tcc gcc atc ggt atc tta tcc aaa gac       207
Glu Ala Ile Gly Asn Ala Gly Ser Ala Ile Gly Ile Leu Ser Lys Asp
                30                  35                  40
ggc gtc gtt tta atc ggc gag aag aaa gtc acc tcc aag ctt ctc caa       255
Gly Val Val Leu Ile Gly Glu Lys Lys Val Thr Ser Lys Leu Leu Gln
            45                  50                  55
acc tcc acc tcc tct gag aaa atg tac aag atc gac gac cac gtc gcc       303
Thr Ser Thr Ser Ser Glu Lys Met Tyr Lys Ile Asp Asp His Val Ala
        60                  65                  70
tgt gct gtc gct ggt atc atg tct gac gcc aac atc ctc atc aac act       351
Cys Ala Val Ala Gly Ile Met Ser Asp Ala Asn Ile Leu Ile Asn Thr
    75                  80                  85
gct aga gtc caa gct caa cgc tac acc ttc atg tac cag gag ccc atg       399
Ala Arg Val Gln Ala Gln Arg Tyr Thr Phe Met Tyr Gln Glu Pro Met
90                  95                  100                 105
cct gtt gag cag ctt gtt cag tct ctc tgt gat aca aag caa ggg tac       447
Pro Val Glu Gln Leu Val Gln Ser Leu Cys Asp Thr Lys Gln Gly Tyr
                110                 115                 120
acg cag ttc ggt ggt ctt cgt ccc ttt gga gtt tcg ttt ctt ttt gca       495
Thr Gln Phe Gly Gly Leu Arg Pro Phe Gly Val Ser Phe Leu Phe Ala
            125                 130                 135
ggg tgg gac aag aac cat ggg ttt cag ctg tat atg agt gac ccc agt       543
Gly Trp Asp Lys Asn His Gly Phe Gln Leu Tyr Met Ser Asp Pro Ser
        140                 145                 150
ggg aac tac ggt ggc tgg aaa gct gcg gct gtg gga gct aac aac cag       591
Gly Asn Tyr Gly Gly Trp Lys Ala Ala Ala Val Gly Ala Asn Asn Gln
    155                 160                 165
gcg gct cag tct att ctg aaa caa gat tac aag gat gat gca act aga       639
Ala Ala Gln Ser Ile Leu Lys Gln Asp Tyr Lys Asp Asp Ala Thr Arg
170                 175                 180                 185
gaa gag gcg gtg gag ctt gct ctg aag gtt ttg agc aag acc atg gac       687
Glu Glu Ala Val Glu Leu Ala Leu Lys Val Leu Ser Lys Thr Met Asp
                190                 195                 200
agc act agc ttg acg tcg gag aag ctt gag ctt gct gag gtg ttt ctg       735
Ser Thr Ser Leu Thr Ser Glu Lys Leu Glu Leu Ala Glu Val Phe Leu
            205                 210                 215
aca ccg tca ggg agt gtt aag tac cat gtt cac tct cct gac tcg ctc       783
Thr Pro Ser Gly Ser Val Lys Tyr His Val His Ser Pro Asp Ser Leu
        220                 225                 230
acc aag ctg ttg gtg aag cac ggt gtg act cag ccc gct gct gaa tca       831
Thr Lys Leu Leu Val Lys His Gly Val Thr Gln Pro Ala Ala Glu Ser

235                 240                 245
tcc taa actgttgtca gagtcttgat ctctgcctgg tgctgtgtcc cctttatcta      887
Ser
250
tcttgttagt tctctgtttc acagctttca tttttagttt gatgttttta ttagactatg      947

acgatgaaac tgaaatttca caatgttatg agaaaaaaaa aaaaaa                     993

<210> 250
<211> 250
<212> PRT
<213> Brassica napus

<400> 250
Met Ser Arg Arg Tyr Asp Ser Arg Thr Thr Ile Phe Ser Pro Glu Gly
1               5                   10                  15
Arg Leu Tyr Gln Val Glu Tyr Ala Met Glu Ala Ile Gly Asn Ala Gly
            20                  25                  30
Ser Ala Ile Gly Ile Leu Ser Lys Asp Gly Val Val Leu Ile Gly Glu
        35                  40                  45
Lys Lys Val Thr Ser Lys Leu Leu Gln Thr Ser Thr Ser Ser Glu Lys
    50                  55                  60
Met Tyr Lys Ile Asp Asp His Val Ala Cys Ala Val Ala Gly Ile Met
65                  70                  75                  80
Ser Asp Ala Asn Ile Leu Ile Asn Thr Ala Arg Val Gln Ala Gln Arg
                85                  90                  95
Tyr Thr Phe Met Tyr Gln Glu Pro Met Pro Val Glu Gln Leu Val Gln
            100                 105                 110
Ser Leu Cys Asp Thr Lys Gln Gly Tyr Thr Gln Phe Gly Gly Leu Arg
        115                 120                 125
Pro Phe Gly Val Ser Phe Leu Phe Ala Gly Trp Asp Lys Asn His Gly
    130                 135                 140
Phe Gln Leu Tyr Met Ser Asp Pro Ser Gly Asn Tyr Gly Gly Trp Lys
145                 150                 155                 160
Ala Ala Ala Val Gly Ala Asn Asn Gln Ala Ala Gln Ser Ile Leu Lys
                165                 170                 175
Gln Asp Tyr Lys Asp Asp Ala Thr Arg Glu Glu Ala Val Glu Leu Ala
            180                 185                 190
Leu Lys Val Leu Ser Lys Thr Met Asp Ser Thr Ser Leu Thr Ser Glu
        195                 200                 205
Lys Leu Glu Leu Ala Glu Val Phe Leu Thr Pro Ser Gly Ser Val Lys
    210                 215                 220
Tyr His Val His Ser Pro Asp Ser Leu Thr Lys Leu Leu Val Lys His
225                 230                 235                 240
Gly Val Thr Gln Pro Ala Ala Glu Ser Ser
                245                 250

<210> 251
<211> 1016
<212> DNA
<213> Brassica napus

<220>
<221> CDS
<222> (85)..(837)

<400> 251
acacatcttt ggtctggagc aaattctcag cgaaaaagag atcaaaatca aagtcctttc      60

agctctctct caaaagtcaa aaac atg tct cga aga tac gat agc cgc acg      111
                           Met Ser Arg Arg Tyr Asp Ser Arg Thr
                           1               5

aca atc ttc tcc cca gaa ggg cgt ctc tac caa gtc gag tac gca atg      159
Thr Ile Phe Ser Pro Glu Gly Arg Leu Tyr Gln Val Glu Tyr Ala Met
10                  15                  20                  25

gaa gcc atc ggc aac gcc ggc tcc gcc atc ggt atc tta tcc aaa gac     207
Glu Ala Ile Gly Asn Ala Gly Ser Ala Ile Gly Ile Leu Ser Lys Asp
                30                  35                  40
ggc gtc gtt ctg atc ggc gag aag aaa gtc acc tcc aag ctt ctc caa     255
Gly Val Val Leu Ile Gly Glu Lys Lys Val Thr Ser Lys Leu Leu Gln
            45                  50                  55
acc tcc acc tcc tcc gag aag atg tac aag atc gac gac cac gtg gcc     303
Thr Ser Thr Ser Ser Glu Lys Met Tyr Lys Ile Asp Asp His Val Ala
        60                  65                  70
tgc gcc gtc gcc ggc atc atg tcc gac gca aac atc ctc atc aac acg     351
Cys Ala Val Ala Gly Ile Met Ser Asp Ala Asn Ile Leu Ile Asn Thr
    75                  80                  85
gct cgt gtc caa gcc cag cgc tac acc ttc atg tac cag gag cct atg     399
Ala Arg Val Gln Ala Gln Arg Tyr Thr Phe Met Tyr Gln Glu Pro Met
90                  95                  100                 105
cct gtt gag cag ctg gtt cag tct ctc tgc gac acc aag caa ggg tac     447
Pro Val Glu Gln Leu Val Gln Ser Leu Cys Asp Thr Lys Gln Gly Tyr
                110                 115                 120
acc cag ttc ggt ggt ctt cgt ccc ttt gga gtg tcc ttt ctc ttt gcc     495
Thr Gln Phe Gly Gly Leu Arg Pro Phe Gly Val Ser Phe Leu Phe Ala
            125                 130                 135
ggg tgg gat aag aac cat ggg ttt cag ctg tat atg agt gat ccg agt     543
Gly Trp Asp Lys Asn His Gly Phe Gln Leu Tyr Met Ser Asp Pro Ser
        140                 145                 150
ggg aac tac ggt ggg tgg aaa gct gca gct gtg gga gcg aac aac cag     591
Gly Asn Tyr Gly Gly Trp Lys Ala Ala Ala Val Gly Ala Asn Asn Gln
    155                 160                 165
gcg gct cag tct att ctg aaa caa gac tac aag gag gat gca act aga     639
Ala Ala Gln Ser Ile Leu Lys Gln Asp Tyr Lys Glu Asp Ala Thr Arg
170                 175                 180                 185
gaa gag gcg gtc gag ctc gcc ttg aag gtt ctg agc aag act atg gac     687
Glu Glu Ala Val Glu Leu Ala Leu Lys Val Leu Ser Lys Thr Met Asp
                190                 195                 200
agc acg agc ttg aca tct gag aag ctt gag ctt gct gag gtg ttt ttg     735
Ser Thr Ser Leu Thr Ser Glu Lys Leu Glu Leu Ala Glu Val Phe Leu
            205                 210                 215
act ccg tcg ggg agt gtt aag tac cat gtt cac tct cct gac tcg ctt     783
Thr Pro Ser Gly Ser Val Lys Tyr His Val His Ser Pro Asp Ser Leu
        220                 225                 230
acc aag ctg ttg ctc aag cat ggt gtg act caa cct gct gct gaa tct     831
Thr Lys Leu Leu Leu Lys His Gly Val Thr Gln Pro Ala Ala Glu Ser
    235                 240                 245
tcc tga gcacaaaact atgtccgagt tgtctcatct ctgcttggtg ctattattat     887
Ser
250
atctcctccc atctatcttg ttagttctct gtttttaatc tctcatgttt agtttgatgt     947

ttaattaaca atggcgatga aactagaatt ttatctgaag gtactttgaa ttgagaaaaa     1007

aaaaaaaaa                                                             1016

<210> 252
<211> 250
<212> PRT
<213> Brassica napus

<400> 252
Met Ser Arg Arg Tyr Asp Ser Arg Thr Thr Ile Phe Ser Pro Glu Gly
1               5                   10                  15
Arg Leu Tyr Gln Val Glu Tyr Ala Met Glu Ala Ile Gly Asn Ala Gly
            20                  25                  30
Ser Ala Ile Gly Ile Leu Ser Lys Asp Gly Val Val Leu Ile Gly Glu
        35                  40                  45
Lys Lys Val Thr Ser Lys Leu Leu Gln Thr Ser Thr Ser Ser Glu Lys
    50                  55                  60

Met Tyr Lys Ile Asp Asp His Val Ala Cys Ala Val Ala Gly Ile Met
65                  70                  75                  80
Ser Asp Ala Asn Ile Leu Ile Asn Thr Ala Arg Val Gln Ala Gln Arg
                85                  90                  95
Tyr Thr Phe Met Tyr Gln Glu Pro Met Pro Val Glu Gln Leu Val Gln
            100                 105                 110
Ser Leu Cys Asp Thr Lys Gln Gly Tyr Thr Gln Phe Gly Gly Leu Arg
        115                 120                 125
Pro Phe Gly Val Ser Phe Leu Phe Ala Gly Trp Asp Lys Asn His Gly
    130                 135                 140
Phe Gln Leu Tyr Met Ser Asp Pro Ser Gly Asn Tyr Gly Gly Trp Lys
145                 150                 155                 160
Ala Ala Ala Val Gly Ala Asn Asn Gln Ala Ala Gln Ser Ile Leu Lys
                165                 170                 175
Gln Asp Tyr Lys Glu Asp Ala Thr Arg Glu Glu Ala Val Glu Leu Ala
            180                 185                 190
Leu Lys Val Leu Ser Lys Thr Met Asp Ser Thr Ser Leu Thr Ser Glu
        195                 200                 205
Lys Leu Glu Leu Ala Glu Val Phe Leu Thr Pro Ser Gly Ser Val Lys
    210                 215                 220
Tyr His Val His Ser Pro Asp Ser Leu Thr Lys Leu Leu Leu Lys His
225                 230                 235                 240
Gly Val Thr Gln Pro Ala Ala Glu Ser Ser
                245                 250

<210> 253
<211> 1018
<212> DNA
<213> Brassica napus

<220>
<221> CDS
<222> (10)..(762)

<400> 253
gtttccaac atg tct cgg agg tat gac agt cgc acc acc atc ttc tca ccg      51
          Met Ser Arg Arg Tyr Asp Ser Arg Thr Thr Ile Phe Ser Pro
          1               5                   10
gaa ggt cgt ctc tac caa gtt gag tac gct atg gaa gcc att ggc aac      99
Glu Gly Arg Leu Tyr Gln Val Glu Tyr Ala Met Glu Ala Ile Gly Asn
15                  20                  25                  30
gct ggc tcc gct att gga atc ttg gcc aaa gac gga gtt gtc ctg att      147
Ala Gly Ser Ala Ile Gly Ile Leu Ala Lys Asp Gly Val Val Leu Ile
                35                  40                  45
ggt gaa aag aaa gtc acc tct aag ctt ctt caa acc tcc aca tcc gct      195
Gly Glu Lys Lys Val Thr Ser Lys Leu Leu Gln Thr Ser Thr Ser Ala
            50                  55                  60
gag aaa atg tac aag atc gat gac cac gtg gcc tgt gct gtc gct ggt      243
Glu Lys Met Tyr Lys Ile Asp Asp His Val Ala Cys Ala Val Ala Gly
        65                  70                  75
atc atg tct gac gcc aac att ctc atc aac acc gct cga gtc caa gct      291
Ile Met Ser Asp Ala Asn Ile Leu Ile Asn Thr Ala Arg Val Gln Ala
    80                  85                  90
cag cgt tac acc ttc atg tac caa gag cct atg ccg gtt gag cag ctg      339
Gln Arg Tyr Thr Phe Met Tyr Gln Glu Pro Met Pro Val Glu Gln Leu
95                  100                 105                 110
gtt cag tct cta tgt gat acc aag caa ggg tac aca cag ttt ggt ggt      387
Val Gln Ser Leu Cys Asp Thr Lys Gln Gly Tyr Thr Gln Phe Gly Gly
                115                 120                 125
ctt cgt ccc ttt gga gtt tct ttt ctt ttt gca gga tgg gac aag aac      435
Leu Arg Pro Phe Gly Val Ser Phe Leu Phe Ala Gly Trp Asp Lys Asn
            130                 135                 140
cat ggg ttt caa ctc tat atg agt gac ccg agt ggg aac tac ggt gga      483
His Gly Phe Gln Leu Tyr Met Ser Asp Pro Ser Gly Asn Tyr Gly Gly
        145                 150                 155
tgg aaa gct gct gcc gtt ggt gct aat aat caa gct gct cag tct att      531
Trp Lys Ala Ala Ala Val Gly Ala Asn Asn Gln Ala Ala Gln Ser Ile
    160                 165                 170
ctt aag caa gac tac aag gat gat gcg tcc agg gaa gag gtg gtt gag      579

Leu Lys Gln Asp Tyr Lys Asp Asp Ala Ser Arg Glu Glu Val Val Glu
175                 180                 185                 190
ctc gct ctc aag gtt ttg agc aag acg atg gac agc acg agc ttg aca       627
Leu Ala Leu Lys Val Leu Ser Lys Thr Met Asp Ser Thr Ser Leu Thr
                195                 200                 205
tct gag aag ctc gag ctt gct gag gtg ttt ctg act cca tca ggg agt       675
Ser Glu Lys Leu Glu Leu Ala Glu Val Phe Leu Thr Pro Ser Gly Ser
            210                 215                 220
gtt cag tac cat gtt cac tcg cct gac tct ctc act aag ctt ttg gtt       723
Val Gln Tyr His Val His Ser Pro Asp Ser Leu Thr Lys Leu Leu Val
        225                 230                 235
aag cat ggt gtg acg caa cca gct gca gaa act ccc taa gcttgaaacc        772
Lys His Gly Val Thr Gln Pro Ala Ala Glu Thr Pro
    240                 245                 250
actagacttg tacggtaatc gacattctgt gtttgtggtt tcccatttgc ttttttttcc      832

tttggacttg aagatactgt ctcggaatca attactactc tggttgtgtc atgaagttat     892

atgttgttat gaatcgaatc tgagcacttt tatttttgca atcctgtttt gtccgtttct     952

ccacaaatga atttcacatc aaaatgataa atgcaatgta ttgtttttcg ccaaaaaaaa     1012

aaaaaa                                                                1018

<210> 254
<211> 250
<212> PRT
<213> Brassica napus

<400> 254
Met Ser Arg Arg Tyr Asp Ser Arg Thr Thr Ile Phe Ser Pro Glu Gly
1               5                   10                  15
Arg Leu Tyr Gln Val Glu Tyr Ala Met Glu Ala Ile Gly Asn Ala Gly
            20                  25                  30
Ser Ala Ile Gly Ile Leu Ala Lys Asp Gly Val Val Leu Ile Gly Glu
        35                  40                  45
Lys Lys Val Thr Ser Lys Leu Leu Gln Thr Ser Thr Ser Ala Glu Lys
    50                  55                  60
Met Tyr Lys Ile Asp Asp His Val Ala Cys Ala Val Ala Gly Ile Met
65                  70                  75                  80
Ser Asp Ala Asn Ile Leu Ile Asn Thr Ala Arg Val Gln Ala Gln Arg
                85                  90                  95
Tyr Thr Phe Met Tyr Gln Glu Pro Met Pro Val Glu Gln Leu Val Gln
            100                 105                 110
Ser Leu Cys Asp Thr Lys Gln Gly Tyr Thr Gln Phe Gly Gly Leu Arg
        115                 120                 125
Pro Phe Gly Val Ser Phe Leu Phe Ala Gly Trp Asp Lys Asn His Gly
    130                 135                 140
Phe Gln Leu Tyr Met Ser Asp Pro Ser Gly Asn Tyr Gly Gly Trp Lys
145                 150                 155                 160
Ala Ala Ala Val Gly Ala Asn Asn Gln Ala Ala Gln Ser Ile Leu Lys
                165                 170                 175
Gln Asp Tyr Lys Asp Asp Ala Ser Arg Glu Glu Val Val Glu Leu Ala
            180                 185                 190
Leu Lys Val Leu Ser Lys Thr Met Asp Ser Thr Ser Leu Thr Ser Glu
        195                 200                 205
Lys Leu Glu Leu Ala Glu Val Phe Leu Thr Pro Ser Gly Ser Val Gln
    210                 215                 220
Tyr His Val His Ser Pro Asp Ser Leu Thr Lys Leu Leu Val Lys His
225                 230                 235                 240
Gly Val Thr Gln Pro Ala Ala Glu Thr Pro
                245                 250

<210> 255
<211> 1039
<212> DNA
<213> Glycine max

<220>
<221> CDS
<222> (84)..(836)

<400> 255
cctcacttcg tttccgtcgc agctttttac acactccacc gctgcttccc cttcacctcc      60

gatttcgtta atcagagatc aaa atg tct cga aga tat gac agc cgt aca aca      113
                          Met Ser Arg Arg Tyr Asp Ser Arg Thr Thr
                          1               5                   10
atc ttc tcc ccg gaa gga cgt ctt tac caa gtg gaa tat gca atg gag      161
Ile Phe Ser Pro Glu Gly Arg Leu Tyr Gln Val Glu Tyr Ala Met Glu
                15                  20                  25
gct att ggg aat gct ggt acc gca ata ggg atc tta tca aaa gat ggg      209
Ala Ile Gly Asn Ala Gly Thr Ala Ile Gly Ile Leu Ser Lys Asp Gly
            30                  35                  40
gta gtg ctg gtt ggt gaa aag aag gtc aca tcc aag ctg ctc caa acc      257
Val Val Leu Val Gly Glu Lys Lys Val Thr Ser Lys Leu Leu Gln Thr
        45                  50                  55
tca act tcc acc gag aaa atg tac aag att gat gat cat gtt gca tgc      305
Ser Thr Ser Thr Glu Lys Met Tyr Lys Ile Asp Asp His Val Ala Cys
    60                  65                  70
gct gtg gct ggg ata atg tct gat gcc aac atc cta atc aat acc gca      353
Ala Val Ala Gly Ile Met Ser Asp Ala Asn Ile Leu Ile Asn Thr Ala
75                  80                  85                  90
agg gtc caa gcc caa cgc tac aca tac gcc tat caa gag ccc atg cca      401
Arg Val Gln Ala Gln Arg Tyr Thr Tyr Ala Tyr Gln Glu Pro Met Pro
                95                  100                 105
gtt gag cag tta gtt cag tct ctc tgc gat acc aag caa ggt tac acc      449
Val Glu Gln Leu Val Gln Ser Leu Cys Asp Thr Lys Gln Gly Tyr Thr
            110                 115                 120
caa ttc ggt ggt ctt cgg cca ttt gga gtt tcg ttc cta ttt gca gga      497
Gln Phe Gly Gly Leu Arg Pro Phe Gly Val Ser Phe Leu Phe Ala Gly
        125                 130                 135
tgg gac aaa aac ttt ggc ttt cag ctt tac atg agc gac cct agt gga      545
Trp Asp Lys Asn Phe Gly Phe Gln Leu Tyr Met Ser Asp Pro Ser Gly
    140                 145                 150
aat tat ggt ggc tgg aaa gct gga gcc att ggg gca aat aac cag gca      593
Asn Tyr Gly Gly Trp Lys Ala Gly Ala Ile Gly Ala Asn Asn Gln Ala
155                 160                 165                 170
gca caa tca ata ctg aaa cag gac tac aag gat gac atc aca aga gaa      641
Ala Gln Ser Ile Leu Lys Gln Asp Tyr Lys Asp Asp Ile Thr Arg Glu
                175                 180                 185
gaa gca gta caa ctt gca ttg aag gtg tta agt aaa acc atg gac agc      689
Glu Ala Val Gln Leu Ala Leu Lys Val Leu Ser Lys Thr Met Asp Ser
            190                 195                 200
aca agt cta acc tca gaa aag ctt gaa ctt gca gag gtt ttc ctt tca      737
Thr Ser Leu Thr Ser Glu Lys Leu Glu Leu Ala Glu Val Phe Leu Ser
        205                 210                 215
ccc tct ggg aaa gtc aag tat gaa gtt tgc tcc cca gag gcc ctg act      785
Pro Ser Gly Lys Val Lys Tyr Glu Val Cys Ser Pro Glu Ala Leu Thr
    220                 225                 230
aag atg ttg gtg aag tct ggt gtt acc caa cct gca aca gac act gcc      833
Lys Met Leu Val Lys Ser Gly Val Thr Gln Pro Ala Thr Asp Thr Ala
235                 240                 245                 250
tag tttgctattc catgcctggt gcactttaat tatccctatc ttatttattt      886

gtggtgcttt tggcattaga agacagatat tggctcaaac atttttcaag cttaaacatt      946

ttgcatgttg gtaatgctaa tggctttgta ttgggtacat cttgatttta aagagagatt      1006

gatggtatgc gttctattta aaaaaaaaaa aaa                                    1039

<210> 256
<211> 250
<212> PRT
<213> Glycine max

<400> 256
Met Ser Arg Arg Tyr Asp Ser Arg Thr Thr Ile Phe Ser Pro Glu Gly
1               5                   10                  15
Arg Leu Tyr Gln Val Glu Tyr Ala Met Glu Ala Ile Gly Asn Ala Gly
            20                  25                  30
Thr Ala Ile Gly Ile Leu Ser Lys Asp Gly Val Val Leu Val Gly Glu
        35                  40                  45
Lys Lys Val Thr Ser Lys Leu Leu Gln Thr Ser Thr Ser Thr Glu Lys
    50                  55                  60
Met Tyr Lys Ile Asp Asp His Val Ala Cys Ala Val Ala Gly Ile Met
65                  70                  75                  80
Ser Asp Ala Asn Ile Leu Ile Asn Thr Ala Arg Val Gln Ala Gln Arg
                85                  90                  95
Tyr Thr Tyr Ala Tyr Gln Glu Pro Met Pro Val Glu Gln Leu Val Gln
            100                 105                 110
Ser Leu Cys Asp Thr Lys Gln Gly Tyr Thr Gln Phe Gly Gly Leu Arg
        115                 120                 125
Pro Phe Gly Val Ser Phe Leu Phe Ala Gly Trp Asp Lys Asn Phe Gly
    130                 135                 140
Phe Gln Leu Tyr Met Ser Asp Pro Ser Gly Asn Tyr Gly Gly Trp Lys
145                 150                 155                 160
Ala Gly Ala Ile Gly Ala Asn Asn Gln Ala Ala Gln Ser Ile Leu Lys
                165                 170                 175
Gln Asp Tyr Lys Asp Asp Ile Thr Arg Glu Glu Ala Val Gln Leu Ala
            180                 185                 190
Leu Lys Val Leu Ser Lys Thr Met Asp Ser Thr Ser Leu Thr Ser Glu
        195                 200                 205
Lys Leu Glu Leu Ala Glu Val Phe Leu Ser Pro Ser Gly Lys Val Lys
    210                 215                 220
Tyr Glu Val Cys Ser Pro Glu Ala Leu Thr Lys Met Leu Val Lys Ser
225                 230                 235                 240
Gly Val Thr Gln Pro Ala Thr Asp Thr Ala
                245                 250

<210> 257
<211> 1129
<212> DNA
<213> Glycine max

<220>
<221> CDS
<222> (86)..(835)

<400> 257
agcgaaaaag aaaaggcttg acttgagaga aatagagctt cgaatcgaaa ctcagaatta      60

gaaaaaaaca caaatcacaa tcaca atg gca agg tac gac cga gca atc acc      112
                            Met Ala Arg Tyr Asp Arg Ala Ile Thr
                            1               5
gtt ttc tcc ccc gac ggc cac ctc ttc cag gtc gag tat gcc ctc gaa      160
Val Phe Ser Pro Asp Gly His Leu Phe Gln Val Glu Tyr Ala Leu Glu
10                  15                  20                  25
gcc gtc cgc aag ggc aac gcc gcc gtc ggc gtc cgc ggc acc gac aac      208
Ala Val Arg Lys Gly Asn Ala Ala Val Gly Val Arg Gly Thr Asp Asn
                30                  35                  40
gtc gtc ctc ggc gtc gag aag aaa tcc acc gcc aag ctc caa gac tcc      256
Val Val Leu Gly Val Glu Lys Lys Ser Thr Ala Lys Leu Gln Asp Ser
            45                  50                  55

agg tcg gtg agg aag att gtg aat ctg gac aac cac att gcg ctg gca 304
Arg Ser Val Arg Lys Ile Val Asn Leu Asp Asn His Ile Ala Leu Ala
        60                  65                  70
tgc gcg ggg ctg aag gcc gac gca cgt gtg ctc ata aac agg gca cgt 352
Cys Ala Gly Leu Lys Ala Asp Ala Arg Val Leu Ile Asn Arg Ala Arg
    75                  80                  85
gtg gag tgc cag agc cac agg ctc acc gtg gag gat ccc gtc acg gtt 400
Val Glu Cys Gln Ser His Arg Leu Thr Val Glu Asp Pro Val Thr Val
90                  95                  100                 105
gag tac ata acg cgg tac att gct ggg ctc cag cag aag tac acg cag 448
Glu Tyr Ile Thr Arg Tyr Ile Ala Gly Leu Gln Gln Lys Tyr Thr Gln
                110                 115                 120
agc ggc ggt gtg cga ccc ttt ggg ctt tcg act ctc att gtc ggg ttt 496
Ser Gly Gly Val Arg Pro Phe Gly Leu Ser Thr Leu Ile Val Gly Phe
            125                 130                 135
gat ccc tac act ggc tcg ccc tcc ttg tac cag acg gat ccc tct ggc 544
Asp Pro Tyr Thr Gly Ser Pro Ser Leu Tyr Gln Thr Asp Pro Ser Gly
        140                 145                 150
acc ttc tcg gcg tgg aag gcg aac gcc acc ggg agg aac tca aat tcg 592
Thr Phe Ser Ala Trp Lys Ala Asn Ala Thr Gly Arg Asn Ser Asn Ser
    155                 160                 165
att agg gag ttc ctc gag aag aac tat aag gac acc tct ggc cag gag 640
Ile Arg Glu Phe Leu Glu Lys Asn Tyr Lys Asp Thr Ser Gly Gln Glu
170                 175                 180                 185
act gtc aag ttg gct att cgc gca ttg ctt gaa gtt gtg gag agc gga 688
Thr Val Lys Leu Ala Ile Arg Ala Leu Leu Glu Val Val Glu Ser Gly
                190                 195                 200
ggc aag aac ata gaa gtt gct gtg atg act aag gag cat ggt ctg cat 736
Gly Lys Asn Ile Glu Val Ala Val Met Thr Lys Glu His Gly Leu His
            205                 210                 215
caa ctg gag gaa gct gaa att gat gcc atc gtt gct gag att gaa gca 784
Gln Leu Glu Glu Ala Glu Ile Asp Ala Ile Val Ala Glu Ile Glu Ala
        220                 225                 230
gag aaa gca gct gct gag gct gct aag aaa gcc cct cct aag gag aca 832
Glu Lys Ala Ala Ala Glu Ala Ala Lys Lys Ala Pro Pro Lys Glu Thr
    235                 240                 245
taa ttttctgttt tgtgttacgc ggggcattgt tcttgctacg tgttggctgt 885

250
actcactgaa gctaaaattg atgccattgt gtctaaagat tgaaacagag aaaacggctg 945

ccgaggctgc aaagaatacc ctttctaagg agacatgatt ttattttact tctgtttagc 1005

tcgtgaattg ttcttgctag atgttggcct aacttaaaac aactgcttta ttgtttttgg 1065

gtactgtcat atagtgtttg gtcaaattaa ttgcatgtca ttattattcg aaaaaaaaaa 1125

aaaa 1129

<210> 258
<211> 249
<212> PRT
<213> Glycine max

<400> 258
Met Ala Arg Tyr Asp Arg Ala Ile Thr Val Phe Ser Pro Asp Gly His
1               5                   10                  15
Leu Phe Gln Val Glu Tyr Ala Leu Glu Ala Val Arg Lys Gly Asn Ala
            20                  25                  30
Ala Val Gly Val Arg Gly Thr Asp Asn Val Val Leu Gly Val Glu Lys
        35                  40                  45
Lys Ser Thr Ala Lys Leu Gln Asp Ser Arg Ser Val Arg Lys Ile Val
    50                  55                  60

Asn Leu Asp Asn His Ile Ala Leu Ala Cys Ala Gly Leu Lys Ala Asp
65                  70                  75                  80
Ala Arg Val Leu Ile Asn Arg Ala Arg Val Glu Cys Gln Ser His Arg
                85                  90                  95
Leu Thr Val Glu Asp Pro Val Thr Val Glu Tyr Ile Thr Arg Tyr Ile
            100                 105                 110
Ala Gly Leu Gln Gln Lys Tyr Thr Gln Ser Gly Gly Val Arg Pro Phe
        115                 120                 125
Gly Leu Ser Thr Leu Ile Val Gly Phe Asp Pro Tyr Thr Gly Ser Pro
    130                 135                 140
Ser Leu Tyr Gln Thr Asp Pro Ser Gly Thr Phe Ser Ala Trp Lys Ala
145                 150                 155                 160
Asn Ala Thr Gly Arg Asn Ser Asn Ser Ile Arg Glu Phe Leu Glu Lys
                165                 170                 175
Asn Tyr Lys Asp Thr Ser Gly Gln Glu Thr Val Lys Leu Ala Ile Arg
            180                 185                 190
Ala Leu Leu Glu Val Val Glu Ser Gly Gly Lys Asn Ile Glu Val Ala
        195                 200                 205
Val Met Thr Lys Glu His Gly Leu His Gln Leu Glu Glu Ala Glu Ile
    210                 215                 220
Asp Ala Ile Val Ala Glu Ile Glu Ala Glu Lys Ala Ala Ala Glu Ala
225                 230                 235                 240
Ala Lys Lys Ala Pro Pro Lys Glu Thr
                245

<210> 259
<211> 931
<212> DNA
<213> Triticum aestivum

<220>
<221> CDS
<222> (56)..(805)

<400> 259
ccgccgcccg ggaagcccaa accaaaccct aagcctccgc gaccggccgc cggcg atg      58
                                                             Met
                                                             1
gcc cgc tac gac cgc gcg atc acc gtc ttc tcc ccc gac ggc cac ctc      106
Ala Arg Tyr Asp Arg Ala Ile Thr Val Phe Ser Pro Asp Gly His Leu
            5                   10                  15
ttc cag gtc gag tac gcc ctc gag gcc gtc cgc aag ggc aac gct gcc      154
Phe Gln Val Glu Tyr Ala Leu Glu Ala Val Arg Lys Gly Asn Ala Ala
        20                  25                  30
gtc ggc gtc cgc ggc acc gac acc gtc gtc ctc ggc gtc gag aag aag      202
Val Gly Val Arg Gly Thr Asp Thr Val Val Leu Gly Val Glu Lys Lys
    35                  40                  45
tcc acc ccc aag ctc cag gac tcc agg tcc gtg cgg aag atc gcg agc      250
Ser Thr Pro Lys Leu Gln Asp Ser Arg Ser Val Arg Lys Ile Ala Ser
50                  55                  60                  65
ctg gac acg cac gtc gcg ctg gcg tgc gcg ggg ctc aag gcg gac gcg      298
Leu Asp Thr His Val Ala Leu Ala Cys Ala Gly Leu Lys Ala Asp Ala
                70                  75                  80
cgg gtg ctc atc aac cgc gcc cgc gtc gag tgc cag agc cac cgc ctc      346
Arg Val Leu Ile Asn Arg Ala Arg Val Glu Cys Gln Ser His Arg Leu
            85                  90                  95
acc gtc gag gac ccc gtc acc gtc gag tac atc act cgc tac atc gcc      394
Thr Val Glu Asp Pro Val Thr Val Glu Tyr Ile Thr Arg Tyr Ile Ala
        100                 105                 110
ggc ctg cag cag aag tac acc cag agc ggt gga gtc cgc ccc ttc ggc      442
Gly Leu Gln Gln Lys Tyr Thr Gln Ser Gly Gly Val Arg Pro Phe Gly
    115                 120                 125
cta tcc acc ctc atc gtc ggc ttc gat ccg tac acg gac aag ccc gcc      490
Leu Ser Thr Leu Ile Val Gly Phe Asp Pro Tyr Thr Asp Lys Pro Ala
130                 135                 140                 145
ctg tac cag acc gac ccc tcc ggc acc ttc tcc gcc tgg aag gcc aac      538
Leu Tyr Gln Thr Asp Pro Ser Gly Thr Phe Ser Ala Trp Lys Ala Asn
                150                 155                 160
gcc acg ggg agg aac tcc aac tcc atg cgg gag ttc ctg gag aag aac      586

Ala Thr Gly Arg Asn Ser Asn Ser Met Arg Glu Phe Leu Glu Lys Asn
            165                 170                 175
tac aag gac acc tcc ggc aag gag acc atc aag ctc acc atc cgc gct      634
Tyr Lys Asp Thr Ser Gly Lys Glu Thr Ile Lys Leu Thr Ile Arg Ala
        180                 185                 190
ctg cta gag gtt gtc gag agt ggt ggg aag aac ata gag att gca gtg      682
Leu Leu Glu Val Val Glu Ser Gly Gly Lys Asn Ile Glu Ile Ala Val
    195                 200                 205
atg aca aag aag gat ggc ctt cgg caa ctc gag gag act gaa atc gat      730
Met Thr Lys Lys Asp Gly Leu Arg Gln Leu Glu Glu Thr Glu Ile Asp
210                 215                 220                 225
gaa tat gtt gcc gag atc gag gca gag aag gcc gcg gca gag gcg gca      778
Glu Tyr Val Ala Glu Ile Glu Ala Glu Lys Ala Ala Ala Glu Ala Ala
            230                 235                 240
aag aag agt ggc ccg aag gat acc taa ttactggatg tgctatatct            825
Lys Lys Ser Gly Pro Lys Asp Thr
            245                 250
tcagtctgtc agccgaaacc ttttagtgtg tcacctgtta cgctacataa ctttctttaa    885

actgctttaa caagatattg aagtcgttcg caaaaaaaaa aaaaaa                   931

<210> 260
<211> 249
<212> PRT
<213> Triticum aestivum

<400> 260
Met Ala Arg Tyr Asp Arg Ala Ile Thr Val Phe Ser Pro Asp Gly His
1               5                   10                  15
Leu Phe Gln Val Glu Tyr Ala Leu Glu Ala Val Arg Lys Gly Asn Ala
            20                  25                  30
Ala Val Gly Val Arg Gly Thr Asp Thr Val Val Leu Gly Val Glu Lys
        35                  40                  45
Lys Ser Thr Pro Lys Leu Gln Asp Ser Arg Ser Val Arg Lys Ile Ala
    50                  55                  60
Ser Leu Asp Thr His Val Ala Leu Ala Cys Ala Gly Leu Lys Ala Asp
65                  70                  75                  80
Ala Arg Val Leu Ile Asn Arg Ala Arg Val Glu Cys Gln Ser His Arg
                85                  90                  95
Leu Thr Val Glu Asp Pro Val Thr Val Glu Tyr Ile Thr Arg Tyr Ile
            100                 105                 110
Ala Gly Leu Gln Gln Lys Tyr Thr Gln Ser Gly Gly Val Arg Pro Phe
        115                 120                 125
Gly Leu Ser Thr Leu Ile Val Gly Phe Asp Pro Tyr Thr Asp Lys Pro
    130                 135                 140
Ala Leu Tyr Gln Thr Asp Pro Ser Gly Thr Phe Ser Ala Trp Lys Ala
145                 150                 155                 160
Asn Ala Thr Gly Arg Asn Ser Asn Ser Met Arg Glu Phe Leu Glu Lys
                165                 170                 175
Asn Tyr Lys Asp Thr Ser Gly Lys Glu Thr Ile Lys Leu Thr Ile Arg
            180                 185                 190
Ala Leu Leu Glu Val Val Glu Ser Gly Gly Lys Asn Ile Glu Ile Ala
        195                 200                 205
Val Met Thr Lys Lys Asp Gly Leu Arg Gln Leu Glu Glu Thr Glu Ile
    210                 215                 220
Asp Glu Tyr Val Ala Glu Ile Glu Ala Glu Lys Ala Ala Ala Glu Ala
225                 230                 235                 240
Ala Lys Lys Ser Gly Pro Lys Asp Thr
                245

<210> 261
<211> 963
<212> DNA
<213> Hordeum vulgare

<220>

<221> CDS
<222> (65)..(814)

<400> 261
accaccacct ccgccgccgg gaagcccaaa ccaaacccta agcctcaccg accggccgcc      60

ggcg atg gcc cgc tac gac cgc gcg atc acg gtc ttc tcc ccc gac ggc      109
     Met Ala Arg Tyr Asp Arg Ala Ile Thr Val Phe Ser Pro Asp Gly
     1               5                   10                  15
cac ctc ttc cag gtc gag tac gcc ctc gag gcc gtc cgc aag ggc aac      157
His Leu Phe Gln Val Glu Tyr Ala Leu Glu Ala Val Arg Lys Gly Asn
                20                  25                  30
gcc gcc gtc ggc gtc cgc ggc acc gac acc gtc gtc ctc ggc gtc gag      205
Ala Ala Val Gly Val Arg Gly Thr Asp Thr Val Val Leu Gly Val Glu
            35                  40                  45
aag aag tcc acc ccc aag ctc cag gac tcc agg tcc gtg cgg aag atc      253
Lys Lys Ser Thr Pro Lys Leu Gln Asp Ser Arg Ser Val Arg Lys Ile
        50                  55                  60
gcg agc ctg gac acg cac gtc gcg ctg gcg tgc gcg ggg ctc aag gcg      301
Ala Ser Leu Asp Thr His Val Ala Leu Ala Cys Ala Gly Leu Lys Ala
    65                  70                  75
gac gcg cgg gtg ctc atc aac cgc gcg cgc gtc gag tgc cag agc cac      349
Asp Ala Arg Val Leu Ile Asn Arg Ala Arg Val Glu Cys Gln Ser His
80                  85                  90                  95
cgc ctc acc gtc gag gac ccc gtc acc gtc gag tac atc acc cgc tac      397
Arg Leu Thr Val Glu Asp Pro Val Thr Val Glu Tyr Ile Thr Arg Tyr
                100                 105                 110
atc gcc ggc ctg cag cag aag tac acc cag agc ggc gga gtc cgc ccc      445
Ile Ala Gly Leu Gln Gln Lys Tyr Thr Gln Ser Gly Gly Val Arg Pro
            115                 120                 125
ttc ggc ctc tcc acc ctc atc gtc ggc ttc gat ccg tac acg gac aag      493
Phe Gly Leu Ser Thr Leu Ile Val Gly Phe Asp Pro Tyr Thr Asp Lys
        130                 135                 140
ccc gcc ctg tac cag acc gac ccc tcc ggc acc ttc tcc gcc tgg aag      541
Pro Ala Leu Tyr Gln Thr Asp Pro Ser Gly Thr Phe Ser Ala Trp Lys
    145                 150                 155
gcc aac gcc acg ggg agg aac tcc aac tcc atg agg gag ttc ctg gag      589
Ala Asn Ala Thr Gly Arg Asn Ser Asn Ser Met Arg Glu Phe Leu Glu
160                 165                 170                 175
aag aac tac aag gac acc tcc ggc aag gag acc atc aag ctc acc atc      637
Lys Asn Tyr Lys Asp Thr Ser Gly Lys Glu Thr Ile Lys Leu Thr Ile
                180                 185                 190
cga gcc ctt cta gag gtt gtt gag agt ggc ggg aag aac atc gag atc      685
Arg Ala Leu Leu Glu Val Val Glu Ser Gly Gly Lys Asn Ile Glu Ile
            195                 200                 205
gca gtg atg aca aag aag gat ggc ctt cgg caa ctt gag gag act gaa      733
Ala Val Met Thr Lys Lys Asp Gly Leu Arg Gln Leu Glu Glu Thr Glu
        210                 215                 220
atc gat gaa tat gtt gcg gag att gag gca gag aag gcc gcg gca gag      781
Ile Asp Glu Tyr Val Ala Glu Ile Glu Ala Glu Lys Ala Ala Ala Glu
    225                 230                 235
gcg gca aag aag agt ggc ccg aag gac acc taa ttactggatg tgctatatct      834
Ala Ala Lys Lys Ser Gly Pro Lys Asp Thr
240                 245                 250
tcattctgtc agccgaaacc ttttagcgtg tcacctgtta cgttccataa ctttatttga      894

accgctttaa caagatatcg aagtcattcg taaatgaatt gccttgtttt cttgaaaaaa      954

aaaaaaaaa                                                              963


<210> 262
<211> 249
<212> PRT
<213> Hordeum vulgare

<400> 262
Met Ala Arg Tyr Asp Arg Ala Ile Thr Val Phe Ser Pro Asp Gly His
1               5                   10                  15
Leu Phe Gln Val Glu Tyr Ala Leu Glu Ala Val Arg Lys Gly Asn Ala
            20                  25                  30
Ala Val Gly Val Arg Gly Thr Asp Thr Val Val Leu Gly Val Glu Lys
        35                  40                  45
Lys Ser Thr Pro Lys Leu Gln Asp Ser Arg Ser Val Arg Lys Ile Ala
    50                  55                  60
Ser Leu Asp Thr His Val Ala Leu Ala Cys Ala Gly Leu Lys Ala Asp
65                  70                  75                  80
Ala Arg Val Leu Ile Asn Arg Ala Arg Val Glu Cys Gln Ser His Arg
                85                  90                  95
Leu Thr Val Glu Asp Pro Val Thr Val Glu Tyr Ile Thr Arg Tyr Ile
            100                 105                 110
Ala Gly Leu Gln Gln Lys Tyr Thr Gln Ser Gly Gly Val Arg Pro Phe
        115                 120                 125
Gly Leu Ser Thr Leu Ile Val Gly Phe Asp Pro Tyr Thr Asp Lys Pro
    130                 135                 140
Ala Leu Tyr Gln Thr Asp Pro Ser Gly Thr Phe Ser Ala Trp Lys Ala
145                 150                 155                 160
Asn Ala Thr Gly Arg Asn Ser Asn Ser Met Arg Glu Phe Leu Glu Lys
                165                 170                 175
Asn Tyr Lys Asp Thr Ser Gly Lys Glu Thr Ile Lys Leu Thr Ile Arg
            180                 185                 190
Ala Leu Leu Glu Val Val Glu Ser Gly Gly Lys Asn Ile Glu Ile Ala
        195                 200                 205
Val Met Thr Lys Lys Asp Gly Leu Arg Gln Leu Glu Glu Thr Glu Ile
    210                 215                 220
Asp Glu Tyr Val Ala Glu Ile Glu Ala Glu Lys Ala Ala Ala Glu Ala
225                 230                 235                 240
Ala Lys Lys Ser Gly Pro Lys Asp Thr
                245

<210> 263
<211> 22
<212> DNA
<213> Artificial sequence

<400> 263
atgtctcgaa gatatgatag cc                                              22

<210> 264
<211> 21
<212> DNA
<213> Artificial sequence

<400> 264
ttaggatgat tcagcagcgg g                                               21


<210> 265
<211> 258
<212> PRT
<213> Artificial sequence

<220>
<221> Variant
<222> (3)..(7)
<223> xaa in position 3 to 7 is any amino acid
<220>
<221> Variant
<222> (11)..(11)
<223> xaa in position 11 is any amino acid
<220>
<221> Variant
<222> (13)..(13)
<223> xaa in position 13 is any amino acid

<220>
<221> Variant
<222> (15)..(15)
<223> xaa in position 15 is any amino acid
<220>
<221> Variant
<222> (21)..(22)
<223> xaa in position 21 to 22 is any amino acid
<220>
<221> Variant
<222> (24)..(32)
<223> xaa in position 24 to 32 is any amino acid
<220>
<221> Variant
<222> (34)..(51)
<223> xaa in position 34 to 51 is any amino acid
<220>
<221> Variant
<222> (53)..(86)
<223> xaa in position 53 to 86 is any amino acid
<220>
<221> Variant
<222> (88)..(90)
<223> xaa in position 88 to 90 is any amino acid
<220>
<221> Variant
<222> (92)..(99)
<223> xaa in position 92 to 99 is any amino acid
<220>
<221> Variant
<222> (101)..(138)
<223> xaa in position 101 to 138 is any amino acid
<220>
<221> Variant
<222> (140)..(144)
<223> xaa in position 140 to 144 is any amino acid
<220>
<221> Variant
<222> (147)..(147)
<223> xaa in position 147 is any amino acid
<220>
<221> Variant
<222> (149)..(154)
<223> xaa in position 149 to 154 is any amino acid
<220>
<221> Variant
<222> (156)..(156)
<223> xaa in position 156 is any amino acid
<220>
<221> Variant
<222> (158)..(199)
<223> xaa in position 158 to 199 is any amino acid
<220>
<221> Variant
<222> (201)..(204)
<223> xaa in position 201 to 204 is any amino acid
<220>
<221> Variant
<222> (208)..(213)
<223> xaa in position 208 to 213 is any amino acid
<220>
<221> Variant
<222> (215)..(217)
<223> xaa in position 215 to 217 is any amino acid
<220>
<221> Variant
<222> (219)..(257)
<223> xaa in position 219 to 257 is any amino acid

<400> 265
Tyr Asp xaa xaa xaa xaa xaa Phe Ser Pro xaa Gly xaa Leu xaa Gln
1               5                   10                  15
Val Glu Tyr Ala xaa xaa Ala xaa xaa xaa xaa xaa xaa xaa xaa xaa
            20                  25                  30
Gly xaa xaa xaa xaa xaa xaa xaa xaa xaa xaa xaa xaa xaa xaa xaa
        35                  40                  45
xaa xaa xaa Leu xaa xaa xaa xaa xaa xaa xaa xaa xaa xaa xaa xaa
    50                  55                  60
xaa xaa xaa xaa xaa xaa xaa xaa xaa xaa xaa xaa xaa xaa xaa xaa
65                  70                  75                  80
xaa xaa xaa xaa xaa xaa Gly xaa xaa xaa Asp xaa xaa xaa xaa xaa
                85                  90                  95
xaa xaa xaa Arg xaa xaa xaa xaa xaa xaa xaa xaa xaa xaa xaa xaa
            100                 105                 110
xaa xaa xaa xaa xaa xaa xaa xaa xaa xaa xaa xaa xaa xaa xaa xaa
        115                 120                 125
xaa xaa xaa xaa xaa xaa xaa xaa xaa xaa Thr xaa xaa xaa xaa xaa
    130                 135                 140
Arg Pro xaa Gly xaa xaa xaa xaa xaa xaa Gly xaa Asp xaa xaa xaa
145                 150                 155                 160
xaa xaa xaa xaa xaa xaa xaa xaa xaa xaa xaa xaa xaa xaa xaa xaa
                165                 170                 175
xaa xaa xaa xaa xaa xaa xaa xaa xaa xaa xaa xaa xaa xaa xaa xaa
            180                 185                 190
xaa xaa xaa xaa xaa xaa xaa Leu xaa xaa xaa xaa Pro Ser Gly xaa
        195                 200                 205
xaa xaa xaa xaa xaa Ala xaa xaa xaa Gly xaa xaa xaa xaa xaa xaa
    210                 215                 220
xaa xaa xaa xaa xaa xaa xaa xaa xaa xaa xaa xaa xaa xaa xaa xaa
225                 230                 235                 240
xaa xaa xaa xaa xaa xaa xaa xaa xaa xaa xaa xaa xaa xaa xaa xaa
                245                 250                 255
xaa Leu

<210> 266
<211> 16
<212> PRT
<213> Artificial sequence

<220>
<221> Variant
<222> (1)..(1)
<223> xaa in position 1 is Phe, Tyr or Trp
<220>
<221> Variant
<222> (2)..(2)
<223> xaa in position 2 is Ser or Thr
<220>
<221> Variant
<222> (3)..(3)
<223> xaa in position 3 is Pro, Ser, Glu or Ala
<220>
<221> Variant
<222> (4)..(4)
<223> xaa in position 4 is any amino acid
<220>
<221> Variant
<222> (6)..(6)
<223> xaa in position 6 is any amino acid
<220>
<221> Variant
<222> (7)..(7)
<223> xaa in position 7 is Leu, Val or Ile
<220>
<221> Variant
<222> (8)..(8)

<223> xaa in position 8 is any amino acid
<220>
<221> Variant
<222> (10)..(10)
<223> xaa in position 10 is Val, Ile or Leu
<220>
<221> Variant
<222> (11)..(11)
<223> xaa in position 11 is Glu, Asp, Asn or Arg
<220>
<221> Variant
<222> (12)..(12)
<223> xaa in position 12 is Tyr, His or Phe
<220>
<221> Variant
<222> (13)..(13)
<223> xaa in position 13 is Ala, Gly, Ile or Ser
<220>
<221> Variant
<222> (14)..(15)
<223> xaa in position 14 to 15 is any amino acid
<220>
<221> Variant
<222> (16)..(16)
<223> xaa in position 16 is Ala, Ser or Gly

<400> 266
xaa xaa xaa xaa Gly xaa xaa xaa Gln xaa xaa xaa xaa xaa xaa xaa
1               5                   10                  15


<210> 267
<211> 77
<212> PRT
<213> Artificial sequence

<220>
<223> protein pattern

<220>
<221> Variant
<222> (1)..(1)
<223> Xaa in position 1 is Arg or Pro

<220>
<221> Variant
<222> (2)..(2)
<223> Xaa in position 2 is Pro or Ser

<220>
<221> Variant
<222> (7)..(7)
<223> Xaa in position 7 is Gly or Ala

<220>
<221> Variant
<222> (8)..(10)
<223> Xaa in position 8 to 10 is any amino acid

<220>
<221> Variant
<222> (11)..(11)
<223> Xaa in position 11 is Leu, Ile, Val, Met or Thr

<220>
<221> Variant
<222> (12)..(13)
<223> Xaa in position 12 to 13 is any amino acid

<220>
<221> Variant
<222> (14)..(14)
<223> Xaa in position 14 is Gly, Ser or Ala

<220>
<221> Variant
<222> (15)..(20)
<223> Xaa in position 15 to 20 is any amino acid

<220>
<221> Variant
<222> (21)..(58)
<223> Xaa in position 21 to 58 is any or no amino acid

<220>
<221> Variant
<222> (59)..(59)
<223> Xaa in position 59 is Leu, Ile or Val

<220>
<221> Variant
<222> (60)..(60)
<223> Xaa in position 60 is Tyr, Phe or Trp

<220>
<221> Variant
<222> (61)..(63)
<223> Xaa in position 61 to 63 is any amino acid

<220>
<221> Variant
<222> (64)..(64)
<223> Xaa in position 64 is Pro or Ala

<220>
<221> Variant
<222> (65)..(65)
<223> Xaa in position 65 is Ser, Ala or Gly

<220>
<221> Variant
<222> (66)..(66)
<223> Xaa in position 66 is Gly or Ala

<220>
<221> Variant
<222> (67)..(72)
<223> Xaa in position 67 to 72 is any amino acid

<220>
<221> Variant
<222> (73)..(73)
<223> Xaa in position 73 is Ala or Gly

<220>
<221> Variant
<222> (74)..(76)
<223> Xaa in position 74 to 76 is any amino acid

<400> 267
Xaa Xaa Phe Tyr Leu Met Xaa Xaa Xaa Xaa Xaa Xaa Xaa Xaa Xaa Xaa
1               5                   10                  15
Xaa Xaa Xaa Xaa Xaa Xaa Xaa Xaa Xaa Xaa Xaa Xaa Xaa Xaa Xaa Xaa
            20                  25                  30
Xaa Xaa Xaa Xaa Xaa Xaa Xaa Xaa Xaa Xaa Xaa Xaa Xaa Xaa Xaa Xaa
        35                  40                  45
Xaa Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Xaa Xaa Gly
65                  70                  75

<210> 268
<211> 246
<212> DNA
<213> Zea mays

<220>
<221> CDS
<222> (1)..(246)

<400> 268
atg cag aac gag gag ggt aag act gtg gac ctc tat gtc ccc agg aag       48
Met Gln Asn Glu Glu Gly Lys Thr Val Asp Leu Tyr Val Pro Arg Lys
1               5                   10                  15
tgc tcg gcc aca aac agg atc atc act gcc aag gac cat gcc tct gtc       96
Cys Ser Ala Thr Asn Arg Ile Ile Thr Ala Lys Asp His Ala Ser Val
            20                  25                  30
cag atc aac att ggc cac ttg gat gcg aat ggc ctg tat gat ggt cac       144
Gln Ile Asn Ile Gly His Leu Asp Ala Asn Gly Leu Tyr Asp Gly His
        35                  40                  45
ttc aca acg ttt gct ctc tct ggg ttt gtc cgt gct cag ggc gac gca       192
Phe Thr Thr Phe Ala Leu Ser Gly Phe Val Arg Ala Gln Gly Asp Ala
    50                  55                  60
gac agc tcc ttg gac agg ctg tgg cag aag aag aag gct gat atc aag       240
Asp Ser Ser Leu Asp Arg Leu Trp Gln Lys Lys Lys Ala Asp Ile Lys
65                  70                  75                  80
cag tag                                                               246
Gln


<210> 269
<211> 81
<212> PRT
<213> Zea mays

<400> 269
Met Gln Asn Glu Glu Gly Lys Thr Val Asp Leu Tyr Val Pro Arg Lys
1               5                   10                  15
Cys Ser Ala Thr Asn Arg Ile Ile Thr Ala Lys Asp His Ala Ser Val
            20                  25                  30
Gln Ile Asn Ile Gly His Leu Asp Ala Asn Gly Leu Tyr Asp Gly His
        35                  40                  45
Phe Thr Thr Phe Ala Leu Ser Gly Phe Val Arg Ala Gln Gly Asp Ala
    50                  55                  60
Asp Ser Ser Leu Asp Arg Leu Trp Gln Lys Lys Lys Ala Asp Ile Lys
65                  70                  75                  80
Gln


<210> 270
<211> 246
<212> DNA
<213> Zea mays

<220>
<221> CDS
<222> (1)..(246)

<400> 270
atg cag aac gag gag ggt aag act gtg gac ctc tat gtc ccc agg aag       48
Met Gln Asn Glu Glu Gly Lys Thr Val Asp Leu Tyr Val Pro Arg Lys
1               5                   10                  15
tgc tcg gcc aca aac agg atc atc act gcc aag gac cat gcc tct gtc       96
Cys Ser Ala Thr Asn Arg Ile Ile Thr Ala Lys Asp His Ala Ser Val
            20                  25                  30
cag atc aac att ggc cac ttg gat gcg aat ggc ctg tat gat ggt cac       144
Gln Ile Asn Ile Gly His Leu Asp Ala Asn Gly Leu Tyr Asp Gly His

35                  40                  45
ttc aca acg ttt gct ctc tct ggg ttt gtc cgt gct cag ggc gac gca        192
Phe Thr Thr Phe Ala Leu Ser Gly Phe Val Arg Ala Gln Gly Asp Ala
    50                  55                  60
gac agc tcc ttg gac agg ttg tgg cag aag aag aag gtt gat atc aag        240
Asp Ser Ser Leu Asp Arg Leu Trp Gln Lys Lys Lys Val Asp Ile Lys
65                  70                  75                  80
cag tag                                                                246
Gln

<210> 271
<211> 81
<212> PRT
<213> Zea mays

<400> 271
Met Gln Asn Glu Glu Gly Lys Thr Val Asp Leu Tyr Val Pro Arg Lys
1               5                   10                  15
Cys Ser Ala Thr Asn Arg Ile Ile Thr Ala Lys Asp His Ala Ser Val
            20                  25                  30
Gln Ile Asn Ile Gly His Leu Asp Ala Asn Gly Leu Tyr Asp Gly His
        35                  40                  45
Phe Thr Thr Phe Ala Leu Ser Gly Phe Val Arg Ala Gln Gly Asp Ala
    50                  55                  60
Asp Ser Ser Leu Asp Arg Leu Trp Gln Lys Lys Lys Val Asp Ile Lys
65                  70                  75                  80
Gln

<210> 272
<211> 246
<212> DNA
<213> Zea mays

<220>
<221> CDS
<222> (1)..(246)

<400> 272
atg cag aac gag gag ggt aag act gtg gac ctc tat gtc ccc agg aag        48
Met Gln Asn Glu Glu Gly Lys Thr Val Asp Leu Tyr Val Pro Arg Lys
1               5                   10                  15
tgc tcg gcc aca aac agg atc atc act gcc aag gac cat gcc tct gtc        96
Cys Ser Ala Thr Asn Arg Ile Ile Thr Ala Lys Asp His Ala Ser Val
            20                  25                  30
cag atc aac att ggc cac ttg gat gcg aat ggc ctg tat gat ggt cac        144
Gln Ile Asn Ile Gly His Leu Asp Ala Asn Gly Leu Tyr Asp Gly His
        35                  40                  45
ttc aca acg ttt gct ctc tct ggg ttt gtc cgt gct cag ggc gac gca        192
Phe Thr Thr Phe Ala Leu Ser Gly Phe Val Arg Ala Gln Gly Asp Ala
    50                  55                  60
gac agc tcc ttg gac agg ctg tgg cag aag aag aag gct gat atc aag        240
Asp Ser Ser Leu Asp Arg Leu Trp Gln Lys Lys Lys Ala Asp Ile Lys
65                  70                  75                  80
cag tag                                                                246
Gln

<210> 273
<211> 81
<212> PRT
<213> Zea mays

<400> 273
Met Gln Asn Glu Glu Gly Lys Thr Val Asp Leu Tyr Val Pro Arg Lys
1               5                   10                  15
Cys Ser Ala Thr Asn Arg Ile Ile Thr Ala Lys Asp His Ala Ser Val
            20                  25                  30

Gln Ile Asn Ile Gly His Leu Asp Ala Asn Gly Leu Tyr Asp Gly His
        35                  40                  45
Phe Thr Thr Phe Ala Leu Ser Gly Phe Val Arg Ala Gln Gly Asp Ala
    50                  55                  60
Asp Ser Ser Leu Asp Arg Leu Trp Gln Lys Lys Lys Ala Asp Ile Lys
65                  70                  75                  80
Gln

<210> 274
<211> 21
<212> DNA
<213> Artificial sequence

<400> 274
atgcagaacg aggagggtaa g                                                21

<210> 275
<211> 22
<212> DNA
<213> Artificial sequence

<400> 275
ctactgcttg atatcagcct tc                                               22

<210> 276
<211> 76
<212> PRT
<213> Artificial sequence

<220>
<221> Variant
<222> (2)..(2)
<223> xaa in position 2 is any amino acid
<220>
<221> Variant
<222> (4)..(5)
<223> xaa in position 4 to 5 is any amino acid
<220>
<221> Variant
<222> (7)..(8)
<223> xaa in position 7 to 8 is any amino acid
<220>
<221> Variant
<222> (13)..(13)
<223> xaa in position 13 is any amino acid
<220>
<221> Variant
<222> (20)..(20)
<223> xaa in position 20 is any amino acid
<220>
<221> Variant
<222> (23)..(23)
<223> xaa in position 23 is any amino acid
<220>
<221> Variant
<222> (25)..(25)
<223> xaa in position 25 is any amino acid
<220>
<221> Variant
<222> (32)..(32)
<223> xaa in position 32 is any amino acid
<220>
<221> Variant
<222> (34)..(38)
<223> xaa in position 34 to 38 is any amino acid
<220>
<221> Variant

<222> (41)..(57)
<223> xaa in position 41 to 57 is any amino acid
<220>
<221> Variant
<222> (59)..(60)
<223> xaa in position 59 to 60 is any amino acid
<220>
<221> Variant
<222> (62)..(63)
<223> xaa in position 62 to 63 is any amino acid
<220>
<221> Variant
<222> (65)..(66)
<223> xaa in position 65 to 66 is any amino acid
<220>
<221> Variant
<222> (68)..(69)
<223> xaa in position 68 to 69 is any amino acid
<220>
<221> Variant
<222> (71)..(74)
<223> xaa in position 71 to 74 is any amino acid

<400> 276
Met xaa Asn xaa xaa Gly xaa xaa Val Asp Leu Tyr xaa Pro Arg Lys
1               5                   10                  15
Cys Ser Ala xaa Asn Arg xaa Ile xaa Ala Lys Asp His Ala Ser xaa
            20                  25                  30
Gln xaa xaa xaa xaa xaa Val Asp xaa xaa xaa xaa xaa xaa xaa xaa
        35                  40                  45
xaa xaa xaa xaa xaa xaa xaa xaa xaa Ala xaa xaa Gly xaa xaa Arg
    50                  55                  60
xaa xaa Gly xaa xaa Asp xaa xaa xaa xaa Arg Leu
65                  70                  75


<210> 277
<211> 33
<212> PRT
<213> Artificial sequence

<220>
<221> Variant
<222> (2)..(11)
<223> xaa in position 2 to 11 is any amino acid
<220>
<221> Variant
<222> (13)..(13)
<223> xaa in position 13 is any amino acid
<220>
<221> Variant
<222> (15)..(15)
<223> xaa in position 15 is any amino acid
<220>
<221> Variant
<222> (19)..(28)
<223> xaa in position 19 to 28 is any amino acid
<220>
<221> Variant
<222> (30)..(32)
<223> xaa in position 30 to 32 is any amino acid

<400> 277
Met xaa xaa xaa xaa xaa xaa xaa xaa xaa xaa Tyr xaa Pro xaa Lys
1               5                   10                  15
Cys Ser xaa xaa xaa xaa xaa xaa xaa xaa xaa xaa His xaa xaa xaa
            20                  25                  30
Gln

<210> 278
<211> 1566
<212> DNA
<213> Glycine max

<220>
<221> CDS
<222> (1)..(1566)

<400> 278
atg ttg ctg gaa ctt gca ctt ggt ttg ttt gtg tta gct ttg ttt ctg 48
Met Leu Leu Glu Leu Ala Leu Gly Leu Phe Val Leu Ala Leu Phe Leu
1               5                   10                  15
cac ttg cgt ccc aca cca agt gca aaa tca aaa gca ctt cgc cac ctc 96
His Leu Arg Pro Thr Pro Ser Ala Lys Ser Lys Ala Leu Arg His Leu
            20                  25                  30
cca aac cct cca agc cca aag cct cgt ctt ccc ttc att ggc cac ctt 144
Pro Asn Pro Pro Ser Pro Lys Pro Arg Leu Pro Phe Ile Gly His Leu
        35                  40                  45
cac ctc tta aaa gat aaa ctt ctc cac tat gca ctc atc gat ctc tcc 192
His Leu Leu Lys Asp Lys Leu Leu His Tyr Ala Leu Ile Asp Leu Ser
    50                  55                  60
aaa aag cat ggc ccc tta ttc tct ctc tcc ttc ggc tcc atg cca acc 240
Lys Lys His Gly Pro Leu Phe Ser Leu Ser Phe Gly Ser Met Pro Thr
65                  70                  75                  80
gtc gtt gcc tcc acc cct gag ttg ttc aag ctc ttc ctc caa acc cac 288
Val Val Ala Ser Thr Pro Glu Leu Phe Lys Leu Phe Leu Gln Thr His
                85                  90                  95
gag gca act tcc ttc aac aca agg ttc caa acc tct gcc ata aga cgc 336
Glu Ala Thr Ser Phe Asn Thr Arg Phe Gln Thr Ser Ala Ile Arg Arg
            100                 105                 110
ctc act tac gac aac tct gtg gcc atg gtt cca ttc gga cct tac tgg 384
Leu Thr Tyr Asp Asn Ser Val Ala Met Val Pro Phe Gly Pro Tyr Trp
        115                 120                 125
aag ttc gtg agg aag ctc atc atg aac gac ctt ctc aac gcc acc acc 432
Lys Phe Val Arg Lys Leu Ile Met Asn Asp Leu Leu Asn Ala Thr Thr
    130                 135                 140
gtc aac aag ctc agg cct ttg agg acc caa cag atc cgc aag ttc ctt 480
Val Asn Lys Leu Arg Pro Leu Arg Thr Gln Gln Ile Arg Lys Phe Leu
145                 150                 155                 160
agg gtt atg gcc caa agc gca gag gcc cag aag ccc ctt gac gtc acc 528
Arg Val Met Ala Gln Ser Ala Glu Ala Gln Lys Pro Leu Asp Val Thr
                165                 170                 175
gag gag ctt ctc aaa tgg acc aac agc acc atc tcc atg atg atg ctc 576
Glu Glu Leu Leu Lys Trp Thr Asn Ser Thr Ile Ser Met Met Met Leu
            180                 185                 190
ggc gag gct gag gag atc aga gac atc gct cgc gag gtt ctt aag atc 624
Gly Glu Ala Glu Glu Ile Arg Asp Ile Ala Arg Glu Val Leu Lys Ile
        195                 200                 205
ttc ggc gaa tac agc ctc act gac ttc atc tgg cct ttg aag tat ctc 672
Phe Gly Glu Tyr Ser Leu Thr Asp Phe Ile Trp Pro Leu Lys Tyr Leu
    210                 215                 220
aag gtt gga aag tat gag aag agg att gat gac atc ttg aac aag ttc 720
Lys Val Gly Lys Tyr Glu Lys Arg Ile Asp Asp Ile Leu Asn Lys Phe
225                 230                 235                 240
gac cct gtc gtt gaa agg gtc atc aag aag cgc cgt gag atc gtc aga 768
Asp Pro Val Val Glu Arg Val Ile Lys Lys Arg Arg Glu Ile Val Arg
                245                 250                 255
agg aga aag aac gga gaa gtt gtt gag ggc gag gtc agc ggg gtt ttc 816
Arg Arg Lys Asn Gly Glu Val Val Glu Gly Glu Val Ser Gly Val Phe
            260                 265                 270
ctt gac act ttg ctt gaa ttc gct gag gat gag acc atg gag atc aaa 864
Leu Asp Thr Leu Leu Glu Phe Ala Glu Asp Glu Thr Met Glu Ile Lys
        275                 280                 285
atc acc aag gac cac atc aag ggt ctt gtt gtc gac ttt ttc tcg gca 912
Ile Thr Lys Asp His Ile Lys Gly Leu Val Val Asp Phe Phe Ser Ala
    290                 295                 300
gga aca gac tcc aca gcg gtg gca aca gag tgg gca ttg gca gaa ctc 960

Gly Thr Asp Ser Thr Ala Val Ala Thr Glu Trp Ala Leu Ala Glu Leu
305                 310                 315                 320
atc aac aat cct aag gtg ttg gaa aag gct cgt gag gag gtc tac agt      1008
Ile Asn Asn Pro Lys Val Leu Glu Lys Ala Arg Glu Glu Val Tyr Ser
                325                 330                 335
gtt gtg gga aag gac aga ctt gtg gac gaa gtt gac act caa aac ctt      1056
Val Val Gly Lys Asp Arg Leu Val Asp Glu Val Asp Thr Gln Asn Leu
            340                 345                 350
cct tac att aga gca atc gtg aag gag aca ttc cgc atg cac ccg cca      1104
Pro Tyr Ile Arg Ala Ile Val Lys Glu Thr Phe Arg Met His Pro Pro
        355                 360                 365
ctc cca gtg gtc aaa aga aag tgc aca gaa gag tgt gag att aat gga      1152
Leu Pro Val Val Lys Arg Lys Cys Thr Glu Glu Cys Glu Ile Asn Gly
    370                 375                 380
tat gtg atc cca gag gga gca ttg att ctc ttc aat gta tgg caa gta      1200
Tyr Val Ile Pro Glu Gly Ala Leu Ile Leu Phe Asn Val Trp Gln Val
385                 390                 395                 400
gga aga gac ccc aaa tac tgg gac aga cca tcg gag ttc cgt cct gag      1248
Gly Arg Asp Pro Lys Tyr Trp Asp Arg Pro Ser Glu Phe Arg Pro Glu
                405                 410                 415
agg ttc cta gag aca ggg gct gaa ggg gaa gca ggg cct ctt gat ctt      1296
Arg Phe Leu Glu Thr Gly Ala Glu Gly Glu Ala Gly Pro Leu Asp Leu
            420                 425                 430
agg gga caa cat ttt caa ctt ctc cca ttt ggg tct ggg agg aga atg      1344
Arg Gly Gln His Phe Gln Leu Leu Pro Phe Gly Ser Gly Arg Arg Met
        435                 440                 445
tgc cct gga gtc aat ctg gct act tcg gga atg gca aca ctt ctt gca      1392
Cys Pro Gly Val Asn Leu Ala Thr Ser Gly Met Ala Thr Leu Leu Ala
    450                 455                 460
tct ctt att cag tgc ttc gac ttg caa gtg ctg ggt cca caa gga cag      1440
Ser Leu Ile Gln Cys Phe Asp Leu Gln Val Leu Gly Pro Gln Gly Gln
465                 470                 475                 480
ata ttg aag ggt ggt gac gcc aaa gtt agc atg gaa gag aga gcc ggc      1488
Ile Leu Lys Gly Gly Asp Ala Lys Val Ser Met Glu Glu Arg Ala Gly
                485                 490                 495
ctc act gtt cca agg gca cat agt ctt gtc tgt gtt cca ctt gca agg      1536
Leu Thr Val Pro Arg Ala His Ser Leu Val Cys Val Pro Leu Ala Arg
            500                 505                 510
atc ggc gtt gca tct aaa ctc ctt tct taa                              1566
Ile Gly Val Ala Ser Lys Leu Leu Ser
        515                 520

<210> 279
<211> 521
<212> PRT
<213> Glycine max

<400> 279
Met Leu Leu Glu Leu Ala Leu Gly Leu Phe Val Leu Ala Leu Phe Leu
1               5                   10                  15
His Leu Arg Pro Thr Pro Ser Ala Lys Ser Lys Ala Leu Arg His Leu
            20                  25                  30
Pro Asn Pro Pro Ser Pro Lys Pro Arg Leu Pro Phe Ile Gly His Leu
        35                  40                  45
His Leu Leu Lys Asp Lys Leu Leu His Tyr Ala Leu Ile Asp Leu Ser
    50                  55                  60
Lys Lys His Gly Pro Leu Phe Ser Leu Ser Phe Gly Ser Met Pro Thr
65                  70                  75                  80
Val Val Ala Ser Thr Pro Glu Leu Phe Lys Leu Phe Leu Gln Thr His
                85                  90                  95
Glu Ala Thr Ser Phe Asn Thr Arg Phe Gln Thr Ser Ala Ile Arg Arg
            100                 105                 110
Leu Thr Tyr Asp Asn Ser Val Ala Met Val Pro Phe Gly Pro Tyr Trp
        115                 120                 125
Lys Phe Val Arg Lys Leu Ile Met Asn Asp Leu Leu Asn Ala Thr Thr
    130                 135                 140
Val Asn Lys Leu Arg Pro Leu Arg Thr Gln Gln Ile Arg Lys Phe Leu
145                 150                 155                 160
Arg Val Met Ala Gln Ser Ala Glu Ala Gln Lys Pro Leu Asp Val Thr

                165                 170                 175
Glu Glu Leu Leu Lys Trp Thr Asn Ser Thr Ile Ser Met Met Met Leu
            180                 185                 190
Gly Glu Ala Glu Glu Ile Arg Asp Ile Ala Arg Glu Val Leu Lys Ile
        195                 200                 205
Phe Gly Glu Tyr Ser Leu Thr Asp Phe Ile Trp Pro Leu Lys Tyr Leu
    210                 215                 220
Lys Val Gly Lys Tyr Glu Lys Arg Ile Asp Asp Ile Leu Asn Lys Phe
225                 230                 235                 240
Asp Pro Val Val Glu Arg Val Ile Lys Lys Arg Arg Glu Ile Val Arg
                245                 250                 255
Arg Arg Lys Asn Gly Glu Val Val Glu Gly Glu Val Ser Gly Val Phe
            260                 265                 270
Leu Asp Thr Leu Leu Glu Phe Ala Glu Asp Glu Thr Met Glu Ile Lys
        275                 280                 285
Ile Thr Lys Asp His Ile Lys Gly Leu Val Val Asp Phe Phe Ser Ala
    290                 295                 300
Gly Thr Asp Ser Thr Ala Val Ala Thr Glu Trp Ala Leu Ala Glu Leu
305                 310                 315                 320
Ile Asn Asn Pro Lys Val Leu Glu Lys Ala Arg Glu Glu Val Tyr Ser
                325                 330                 335
Val Val Gly Lys Asp Arg Leu Val Asp Glu Val Asp Thr Gln Asn Leu
            340                 345                 350
Pro Tyr Ile Arg Ala Ile Val Lys Glu Thr Phe Arg Met His Pro Pro
        355                 360                 365
Leu Pro Val Val Lys Arg Lys Cys Thr Glu Glu Cys Glu Ile Asn Gly
    370                 375                 380
Tyr Val Ile Pro Glu Gly Ala Leu Ile Leu Phe Asn Val Trp Gln Val
385                 390                 395                 400
Gly Arg Asp Pro Lys Tyr Trp Asp Arg Pro Ser Glu Phe Arg Pro Glu
                405                 410                 415
Arg Phe Leu Glu Thr Gly Ala Glu Gly Glu Ala Gly Pro Leu Asp Leu
            420                 425                 430
Arg Gly Gln His Phe Gln Leu Leu Pro Phe Gly Ser Gly Arg Arg Met
        435                 440                 445
Cys Pro Gly Val Asn Leu Ala Thr Ser Gly Met Ala Thr Leu Leu Ala
    450                 455                 460
Ser Leu Ile Gln Cys Phe Asp Leu Gln Val Leu Gly Pro Gln Gly Gln
465                 470                 475                 480
Ile Leu Lys Gly Gly Asp Ala Lys Val Ser Met Glu Glu Arg Ala Gly
                485                 490                 495
Leu Thr Val Pro Arg Ala His Ser Leu Val Cys Val Pro Leu Ala Arg
            500                 505                 510
Ile Gly Val Ala Ser Lys Leu Leu Ser
        515                 520

<210> 280
<211> 1566
<212> DNA
<213> Glycine max

<220>
<221> CDS
<222> (1)..(1566)

<400> 280
atg ttg ctt gaa ctt gca ctt ggt tta ttg gtt ttg gct ctg ttt ctg      48
Met Leu Leu Glu Leu Ala Leu Gly Leu Leu Val Leu Ala Leu Phe Leu
1               5                   10                  15
cac ttg cgt ccc aca ccc act gca aaa tca aaa gca ctt cgc cat ctc      96
His Leu Arg Pro Thr Pro Thr Ala Lys Ser Lys Ala Leu Arg His Leu
            20                  25                  30
cca aac cca cca agc cca aag cct cgt ctt ccc ttc ata gga cac ctt      144
Pro Asn Pro Pro Ser Pro Lys Pro Arg Leu Pro Phe Ile Gly His Leu
        35                  40                  45
cat ctc tta aaa gac aaa ctt ctc cac tac gca ctc atc gac ctc tcc      192
His Leu Leu Lys Asp Lys Leu Leu His Tyr Ala Leu Ile Asp Leu Ser
    50                  55                  60
aaa aaa cat ggt ccc tta ttc tct ctc tac ttt ggc tcc atg cca acc      240

Lys Lys His Gly Pro Leu Phe Ser Leu Tyr Phe Gly Ser Met Pro Thr
65                  70                  75                  80
gtt gtt gcc tcc aca cca gaa ttg ttc aag ctc ttc ctc caa acg cac      288
Val Val Ala Ser Thr Pro Glu Leu Phe Lys Leu Phe Leu Gln Thr His
                85                  90                  95
gag gca act tcc ttc aac aca agg ttc caa acc tca gcc ata aga cgc      336
Glu Ala Thr Ser Phe Asn Thr Arg Phe Gln Thr Ser Ala Ile Arg Arg
            100                 105                 110
ctc acc tat gat agc tca gtg gcc atg gtt ccc ttc gga cct tac tgg      384
Leu Thr Tyr Asp Ser Ser Val Ala Met Val Pro Phe Gly Pro Tyr Trp
        115                 120                 125
aag ttc gtg agg aag ctc atc atg aac gac ctt ctc aac gcc acc act      432
Lys Phe Val Arg Lys Leu Ile Met Asn Asp Leu Leu Asn Ala Thr Thr
    130                 135                 140
gta aac aag ttg agg cct ttg agg acc caa cag atc cgc aag ttc ctt      480
Val Asn Lys Leu Arg Pro Leu Arg Thr Gln Gln Ile Arg Lys Phe Leu
145                 150                 155                 160
agg gtt atg gcc caa ggc gca gag gca cag aag ccc ctt gac ttg acc      528
Arg Val Met Ala Gln Gly Ala Glu Ala Gln Lys Pro Leu Asp Leu Thr
                165                 170                 175
gag gag ctt ctg aaa tgg acc aac agc acc atc tcc atg atg atg ctc      576
Glu Glu Leu Leu Lys Trp Thr Asn Ser Thr Ile Ser Met Met Met Leu
            180                 185                 190
ggc gag gct gag gag atc aga gac atc gct cgc gag gtt ctt aag atc      624
Gly Glu Ala Glu Glu Ile Arg Asp Ile Ala Arg Glu Val Leu Lys Ile
        195                 200                 205
ttt ggc gaa tac agc ctc act gac ttc atc tgg cca ttg aag cat ctc      672
Phe Gly Glu Tyr Ser Leu Thr Asp Phe Ile Trp Pro Leu Lys His Leu
    210                 215                 220
aag gtt gga aag tat gag aag agg atc gac gac atc ttg aac aag ttc      720
Lys Val Gly Lys Tyr Glu Lys Arg Ile Asp Asp Ile Leu Asn Lys Phe
225                 230                 235                 240
gac cct gtc gtt gaa agg gtc atc aag aag cgc cgt gag atc gtg agg      768
Asp Pro Val Val Glu Arg Val Ile Lys Lys Arg Arg Glu Ile Val Arg
                245                 250                 255
agg aga aag aac gga gag gtt gtt gag ggt gag gtc agc ggg gtt ttc      816
Arg Arg Lys Asn Gly Glu Val Val Glu Gly Glu Val Ser Gly Val Phe
            260                 265                 270
ctt gac act ttg ctt gaa ttc gct gag gat gag acc atg gag atc aaa      864
Leu Asp Thr Leu Leu Glu Phe Ala Glu Asp Glu Thr Met Glu Ile Lys
        275                 280                 285
atc acc aag gac cac atc aag ggt ctt gtt gtc gac ttt ttc tcg gca      912
Ile Thr Lys Asp His Ile Lys Gly Leu Val Val Asp Phe Phe Ser Ala
    290                 295                 300
gga aca gac tcc aca gcg gtg gca aca gag tgg gca ttg gca gaa ctc      960
Gly Thr Asp Ser Thr Ala Val Ala Thr Glu Trp Ala Leu Ala Glu Leu
305                 310                 315                 320
atc aac aat cct aag gtg ttg gaa aag gct cgt gag gag gtc tac agt     1008
Ile Asn Asn Pro Lys Val Leu Glu Lys Ala Arg Glu Glu Val Tyr Ser
                325                 330                 335
gtt gtg gga aag gac aga ctt gtg gac gaa gtt gac act caa aac ctt     1056
Val Val Gly Lys Asp Arg Leu Val Asp Glu Val Asp Thr Gln Asn Leu
            340                 345                 350
cct tac att aga gca atc gtg aag gag aca ttc cgc atg cac ccg cca     1104
Pro Tyr Ile Arg Ala Ile Val Lys Glu Thr Phe Arg Met His Pro Pro
        355                 360                 365
ctc cca gtg gtc aaa aga aag tgc aca gaa gag tgt gag att aat gga     1152
Leu Pro Val Val Lys Arg Lys Cys Thr Glu Glu Cys Glu Ile Asn Gly
    370                 375                 380
tat gtg atc cca gag gga gca ttg att ctc ttc aat gta tgg caa gta     1200
Tyr Val Ile Pro Glu Gly Ala Leu Ile Leu Phe Asn Val Trp Gln Val
385                 390                 395                 400
gga aga gac ccc aaa tac tgg gac aga cca tcg gag ttc cgt cct gag     1248
Gly Arg Asp Pro Lys Tyr Trp Asp Arg Pro Ser Glu Phe Arg Pro Glu
                405                 410                 415
agg ttc cta gag aca ggg gct gaa ggg gaa gca ggg cct ctt gat ctt     1296
Arg Phe Leu Glu Thr Gly Ala Glu Gly Glu Ala Gly Pro Leu Asp Leu
            420                 425                 430
agg gga caa cat ttt caa ctt ctc cca ttt ggg tct ggg agg aga atg     1344

Arg Gly Gln His Phe Gln Leu Leu Pro Phe Gly Ser Gly Arg Arg Met
            435                 440                 445
tgc cct gga gtc aat ctg gct act tcg gga atg gca aca ctt ctt gca      1392
Cys Pro Gly Val Asn Leu Ala Thr Ser Gly Met Ala Thr Leu Leu Ala
    450                 455                 460
tct ctt att cag tgc ttc gac ttg caa gtg ctg ggt cca caa gga cag      1440
Ser Leu Ile Gln Cys Phe Asp Leu Gln Val Leu Gly Pro Gln Gly Gln
465                 470                 475                 480
ata ttg aag ggt ggt gac gcc aaa gtt agc atg gaa gag aga gcc ggc      1488
Ile Leu Lys Gly Gly Asp Ala Lys Val Ser Met Glu Glu Arg Ala Gly
            485                 490                 495
ctc act gtt cca agg gca cat agt ctt gtc tgt gtt cca ctt gca agg      1536
Leu Thr Val Pro Arg Ala His Ser Leu Val Cys Val Pro Leu Ala Arg
            500                 505                 510
atc ggc gtt gca tct aaa ctc ctt tct taa                              1566
Ile Gly Val Ala Ser Lys Leu Leu Ser
            515                 520

<210> 281
<211> 521
<212> PRT
<213> Glycine max

<400> 281
Met Leu Leu Glu Leu Ala Leu Gly Leu Leu Val Leu Ala Leu Phe Leu
1               5                   10                  15
His Leu Arg Pro Thr Pro Thr Ala Lys Ser Lys Ala Leu Arg His Leu
            20                  25                  30
Pro Asn Pro Pro Ser Pro Lys Pro Arg Leu Pro Phe Ile Gly His Leu
        35                  40                  45
His Leu Leu Lys Asp Lys Leu Leu His Tyr Ala Leu Ile Asp Leu Ser
    50                  55                  60
Lys Lys His Gly Pro Leu Phe Ser Leu Tyr Phe Gly Ser Met Pro Thr
65                  70                  75                  80
Val Val Ala Ser Thr Pro Glu Leu Phe Lys Leu Phe Leu Gln Thr His
                85                  90                  95
Glu Ala Thr Ser Phe Asn Thr Arg Phe Gln Thr Ser Ala Ile Arg Arg
            100                 105                 110
Leu Thr Tyr Asp Ser Ser Val Ala Met Val Pro Phe Gly Pro Tyr Trp
        115                 120                 125
Lys Phe Val Arg Lys Leu Ile Met Asn Asp Leu Leu Asn Ala Thr Thr
    130                 135                 140
Val Asn Lys Leu Arg Pro Leu Arg Thr Gln Gln Ile Arg Lys Phe Leu
145                 150                 155                 160
Arg Val Met Ala Gln Gly Ala Glu Ala Gln Lys Pro Leu Asp Leu Thr
                165                 170                 175
Glu Glu Leu Leu Lys Trp Thr Asn Ser Thr Ile Ser Met Met Met Leu
            180                 185                 190
Gly Glu Ala Glu Glu Ile Arg Asp Ile Ala Arg Glu Val Leu Lys Ile
        195                 200                 205
Phe Gly Glu Tyr Ser Leu Thr Asp Phe Ile Trp Pro Leu Lys His Leu
    210                 215                 220
Lys Val Gly Lys Tyr Glu Lys Arg Ile Asp Asp Ile Leu Asn Lys Phe
225                 230                 235                 240
Asp Pro Val Val Glu Arg Val Ile Lys Lys Arg Arg Glu Ile Val Arg
                245                 250                 255
Arg Arg Lys Asn Gly Glu Val Val Glu Gly Glu Val Ser Gly Val Phe
            260                 265                 270
Leu Asp Thr Leu Leu Glu Phe Ala Glu Asp Glu Thr Met Glu Ile Lys
        275                 280                 285
Ile Thr Lys Asp His Ile Lys Gly Leu Val Val Asp Phe Phe Ser Ala
    290                 295                 300
Gly Thr Asp Ser Thr Ala Val Ala Thr Glu Trp Ala Leu Ala Glu Leu
305                 310                 315                 320
Ile Asn Asn Pro Lys Val Leu Glu Lys Ala Arg Glu Glu Val Tyr Ser
                325                 330                 335
Val Val Gly Lys Asp Arg Leu Val Asp Glu Val Asp Thr Gln Asn Leu
            340                 345                 350
Pro Tyr Ile Arg Ala Ile Val Lys Glu Thr Phe Arg Met His Pro Pro

355                 360                 365
Leu Pro Val Val Lys Arg Lys Cys Thr Glu Glu Cys Glu Ile Asn Gly
    370                 375                 380
Tyr Val Ile Pro Glu Gly Ala Leu Ile Leu Phe Asn Val Trp Gln Val
385                 390                 395                 400
Gly Arg Asp Pro Lys Tyr Trp Asp Arg Pro Ser Glu Phe Arg Pro Glu
                405                 410                 415
Arg Phe Leu Glu Thr Gly Ala Glu Gly Glu Ala Gly Pro Leu Asp Leu
            420                 425                 430
Arg Gly Gln His Phe Gln Leu Leu Pro Phe Gly Ser Gly Arg Arg Met
        435                 440                 445
Cys Pro Gly Val Asn Leu Ala Thr Ser Gly Met Ala Thr Leu Leu Ala
    450                 455                 460
Ser Leu Ile Gln Cys Phe Asp Leu Gln Val Leu Gly Pro Gln Gly Gln
465                 470                 475                 480
Ile Leu Lys Gly Gly Asp Ala Lys Val Ser Met Glu Glu Arg Ala Gly
                485                 490                 495
Leu Thr Val Pro Arg Ala His Ser Leu Val Cys Val Pro Leu Ala Arg
            500                 505                 510
Ile Gly Val Ala Ser Lys Leu Leu Ser
        515                 520

<210> 282
<211> 1584
<212> DNA
<213> Zea mays

<220>
<221> CDS
<222> (1)..(1584)

<400> 282
atg gcg acc tgc gtt ctg ctg atg ctg cgg gaa gtc tct ccg tgg gcg      48
Met Ala Thr Cys Val Leu Leu Met Leu Arg Glu Val Ser Pro Trp Ala
1               5                   10                  15
ctg gcc agc gtg gtg gcg tcc gtg tcg ctg ttg tgg ctg gtg gtc tgg      96
Leu Ala Ser Val Val Ala Ser Val Ser Leu Leu Trp Leu Val Val Trp
            20                  25                  30
acg ctg gag tgg gcc tgg tgg acg cct tgg cgg ctc gag cgg gcc ctg      144
Thr Leu Glu Trp Ala Trp Trp Thr Pro Trp Arg Leu Glu Arg Ala Leu
        35                  40                  45
cgg gtc cag ggc ctc aag ggc acc agg tac cgc ctc ttc acc ggc gac      192
Arg Val Gln Gly Leu Lys Gly Thr Arg Tyr Arg Leu Phe Thr Gly Asp
    50                  55                  60
ctg agg gaa acc gcc cgg gct aac cgg gag gct cgc aag aag ccg ctg      240
Leu Arg Glu Thr Ala Arg Ala Asn Arg Glu Ala Arg Lys Lys Pro Leu
65                  70                  75                  80
ccg ctc ggc agc cac gac atc gcc cca cgc gtg cag ccc atg cat cac      288
Pro Leu Gly Ser His Asp Ile Ala Pro Arg Val Gln Pro Met His His
                85                  90                  95
agc acc atc aag gaa tac ggg aaa cta tcg ttc acc tgg ttc ggc cca      336
Ser Thr Ile Lys Glu Tyr Gly Lys Leu Ser Phe Thr Trp Phe Gly Pro
            100                 105                 110
aca cca agg gtg atg att cct gac cca gag tta gtc aaa gag gtg ctg      384
Thr Pro Arg Val Met Ile Pro Asp Pro Glu Leu Val Lys Glu Val Leu
        115                 120                 125
tct aat aag ttt ggc cac ttt ggc aaa cca agg agt aac cgc att ggg      432
Ser Asn Lys Phe Gly His Phe Gly Lys Pro Arg Ser Asn Arg Ile Gly
    130                 135                 140
agg ttg cta gcc aac ggg ctt gta aat cat gat ggt gaa aaa tgg gca      480
Arg Leu Leu Ala Asn Gly Leu Val Asn His Asp Gly Glu Lys Trp Ala
145                 150                 155                 160
aag cac agg aga att ctt aat cct gca ttt cac cat gaa aaa ata aag      528
Lys His Arg Arg Ile Leu Asn Pro Ala Phe His His Glu Lys Ile Lys
                165                 170                 175
ggg atg atg cca gta ttt tct acc tgc tgt att gaa atg att act aga      576
Gly Met Met Pro Val Phe Ser Thr Cys Cys Ile Glu Met Ile Thr Arg
            180                 185                 190
tgg gat aat tca atg cct tct gag gga tct tct gag ata gat gtc tgg      624

Trp Asp Asn Ser Met Pro Ser Glu Gly Ser Ser Glu Ile Asp Val Trp
        195                 200                 205
cct gag ttc cag aat ctt act gga gat gtt atc tca aga act gcg ttt        672
Pro Glu Phe Gln Asn Leu Thr Gly Asp Val Ile Ser Arg Thr Ala Phe
    210                 215                 220
ggg agc aac tat caa gaa ggg agg aga att ttc gag ctg caa gga gaa        720
Gly Ser Asn Tyr Gln Glu Gly Arg Arg Ile Phe Glu Leu Gln Gly Glu
225                 230                 235                 240
cta gct gaa cgc ctc atc caa tct gtt cag aca ata ttt atc cca ggc        768
Leu Ala Glu Arg Leu Ile Gln Ser Val Gln Thr Ile Phe Ile Pro Gly
                245                 250                 255
tat tgg ttc ttg ccc acc aaa aac aac aga agg atg aga gcg atc gat        816
Tyr Trp Phe Leu Pro Thr Lys Asn Asn Arg Arg Met Arg Ala Ile Asp
            260                 265                 270
gta gag atc cgc aaa att ctc cgt gaa ata att ggg aag aga gag aag        864
Val Glu Ile Arg Lys Ile Leu Arg Glu Ile Ile Gly Lys Arg Glu Lys
        275                 280                 285
gat act aaa aac aga gaa aca aat aac gac gac ttg ctg ggc tta tta        912
Asp Thr Lys Asn Arg Glu Thr Asn Asn Asp Asp Leu Leu Gly Leu Leu
    290                 295                 300
ctg gag tca aac aca agg caa tca aat gga aat gca agc ctg gga ttg        960
Leu Glu Ser Asn Thr Arg Gln Ser Asn Gly Asn Ala Ser Leu Gly Leu
305                 310                 315                 320
aca aca gaa gat gtg att gag gaa tgc aag tta ttt tac ttt gca ggt        1008
Thr Thr Glu Asp Val Ile Glu Glu Cys Lys Leu Phe Tyr Phe Ala Gly
                325                 330                 335
atg gag aca aca tca gtc ctt ctt act tgg aca ctt att gtg cta agc        1056
Met Glu Thr Thr Ser Val Leu Leu Thr Trp Thr Leu Ile Val Leu Ser
            340                 345                 350
atg cac cca gaa tgg caa gag aga gca aga gaa gag gtt ttg agc cac        1104
Met His Pro Glu Trp Gln Glu Arg Ala Arg Glu Glu Val Leu Ser His
        355                 360                 365
ttt gga aga acc aca ccg gat tat gat agc ttg agc cgc ctc aag act        1152
Phe Gly Arg Thr Thr Pro Asp Tyr Asp Ser Leu Ser Arg Leu Lys Thr
    370                 375                 380
ata acc atg att cta cat gag gtc ctt agg ttg tac cca ccg gca acc        1200
Ile Thr Met Ile Leu His Glu Val Leu Arg Leu Tyr Pro Pro Ala Thr
385                 390                 395                 400
ttt cta aca aga aga act tat aag gaa atg gag ctc ggt gga atc aaa        1248
Phe Leu Thr Arg Arg Thr Tyr Lys Glu Met Glu Leu Gly Gly Ile Lys
                405                 410                 415
tat cct gca gga gtt gac ctc ctt cta ccc gtc atc ttc att cac cat        1296
Tyr Pro Ala Gly Val Asp Leu Leu Leu Pro Val Ile Phe Ile His His
            420                 425                 430
gat cct gac att tgg gga aaa gac gca agc gag ttc aac cca gaa agg        1344
Asp Pro Asp Ile Trp Gly Lys Asp Ala Ser Glu Phe Asn Pro Glu Arg
        435                 440                 445
ttt gcc aac ggc atc tcc agc gca acc agg cat cag gct gct ttc ttt        1392
Phe Ala Asn Gly Ile Ser Ser Ala Thr Arg His Gln Ala Ala Phe Phe
    450                 455                 460
ccg ttc gga ggg ggg cct aga atc tgc atc ggc cag agc ttt gcg ttg        1440
Pro Phe Gly Gly Gly Pro Arg Ile Cys Ile Gly Gln Ser Phe Ala Leu
465                 470                 475                 480
ctg gaa gcc aag atg acg cta tgc acc atc ctc caa cgc ttc tcg ttc        1488
Leu Glu Ala Lys Met Thr Leu Cys Thr Ile Leu Gln Arg Phe Ser Phe
                485                 490                 495
gag ctc tcg cca tcc tac acc cac gcg ccg tac acc gtg ata aca ctg        1536
Glu Leu Ser Pro Ser Tyr Thr His Ala Pro Tyr Thr Val Ile Thr Leu
            500                 505                 510
cac cct caa cat ggt gct cag ata agg ctc aaa aag ctt tct ccg            1581
His Pro Gln His Gly Ala Gln Ile Arg Leu Lys Lys Leu Ser Pro
        515                 520                 525
tga                                                                    1584

<210> 283
<211> 527
<212> PRT

<213> Zea mays

<400> 283
Met Ala Thr Cys Val Leu Leu Met Leu Arg Glu Val Ser Pro Trp Ala
1               5                   10                  15
Leu Ala Ser Val Val Ala Ser Val Ser Leu Leu Trp Leu Val Val Trp
            20                  25                  30
Thr Leu Glu Trp Ala Trp Trp Thr Pro Trp Arg Leu Glu Arg Ala Leu
        35                  40                  45
Arg Val Gln Gly Leu Lys Gly Thr Arg Tyr Arg Leu Phe Thr Gly Asp
    50                  55                  60
Leu Arg Glu Thr Ala Arg Ala Asn Arg Glu Ala Arg Lys Lys Pro Leu
65                  70                  75                  80
Pro Leu Gly Ser His Asp Ile Ala Pro Arg Val Gln Pro Met His His
                85                  90                  95
Ser Thr Ile Lys Glu Tyr Gly Lys Leu Ser Phe Thr Trp Phe Gly Pro
            100                 105                 110
Thr Pro Arg Val Met Ile Pro Asp Pro Glu Leu Val Lys Glu Val Leu
        115                 120                 125
Ser Asn Lys Phe Gly His Phe Gly Lys Pro Arg Ser Asn Arg Ile Gly
    130                 135                 140
Arg Leu Leu Ala Asn Gly Leu Val Asn His Asp Gly Glu Lys Trp Ala
145                 150                 155                 160
Lys His Arg Arg Ile Leu Asn Pro Ala Phe His His Glu Lys Ile Lys
                165                 170                 175
Gly Met Met Pro Val Phe Ser Thr Cys Cys Ile Glu Met Ile Thr Arg
            180                 185                 190
Trp Asp Asn Ser Met Pro Ser Glu Gly Ser Ser Glu Ile Asp Val Trp
        195                 200                 205
Pro Glu Phe Gln Asn Leu Thr Gly Asp Val Ile Ser Arg Thr Ala Phe
    210                 215                 220
Gly Ser Asn Tyr Gln Glu Gly Arg Arg Ile Phe Glu Leu Gln Gly Glu
225                 230                 235                 240
Leu Ala Glu Arg Leu Ile Gln Ser Val Gln Thr Ile Phe Ile Pro Gly
                245                 250                 255
Tyr Trp Phe Leu Pro Thr Lys Asn Asn Arg Arg Met Arg Ala Ile Asp
            260                 265                 270
Val Glu Ile Arg Lys Ile Leu Arg Glu Ile Ile Gly Lys Arg Glu Lys
        275                 280                 285
Asp Thr Lys Asn Arg Glu Thr Asn Asn Asp Asp Leu Leu Gly Leu Leu
    290                 295                 300
Leu Glu Ser Asn Thr Arg Gln Ser Asn Gly Asn Ala Ser Leu Gly Leu
305                 310                 315                 320
Thr Thr Glu Asp Val Ile Glu Glu Cys Lys Leu Phe Tyr Phe Ala Gly
                325                 330                 335
Met Glu Thr Thr Ser Val Leu Leu Thr Trp Thr Leu Ile Val Leu Ser
            340                 345                 350
Met His Pro Glu Trp Gln Glu Arg Ala Arg Glu Glu Val Leu Ser His
        355                 360                 365
Phe Gly Arg Thr Thr Pro Asp Tyr Asp Ser Leu Ser Arg Leu Lys Thr
    370                 375                 380
Ile Thr Met Ile Leu His Glu Val Leu Arg Leu Tyr Pro Pro Ala Thr
385                 390                 395                 400
Phe Leu Thr Arg Arg Thr Tyr Lys Glu Met Glu Leu Gly Gly Ile Lys
                405                 410                 415
Tyr Pro Ala Gly Val Asp Leu Leu Leu Pro Val Ile Phe Ile His His
            420                 425                 430
Asp Pro Asp Ile Trp Gly Lys Asp Ala Ser Glu Phe Asn Pro Glu Arg
        435                 440                 445
Phe Ala Asn Gly Ile Ser Ser Ala Thr Arg His Gln Ala Ala Phe Phe
    450                 455                 460
Pro Phe Gly Gly Gly Pro Arg Ile Cys Ile Gly Gln Ser Phe Ala Leu
465                 470                 475                 480
Leu Glu Ala Lys Met Thr Leu Cys Thr Ile Leu Gln Arg Phe Ser Phe
                485                 490                 495
Glu Leu Ser Pro Ser Tyr Thr His Ala Pro Tyr Thr Val Ile Thr Leu
            500                 505                 510
His Pro Gln His Gly Ala Gln Ile Arg Leu Lys Lys Leu Ser Pro
        515                 520                 525

<210> 284
<211> 1590
<212> DNA
<213> Zea mays

<220>
<221> CDS
<222> (1)..(1590)

<400> 284
atg gtt ctt gga gcg ttg gcg agt gca gtc gcc tcg atg agc ctc ctc 48
Met Val Leu Gly Ala Leu Ala Ser Ala Val Ala Ser Met Ser Leu Leu
1               5                   10                  15
atc gtc tac ggt gtc ctg ggc gct ctg ctc ctg tgg aag gcc gcc cgg 96
Ile Val Tyr Gly Val Leu Gly Ala Leu Leu Leu Trp Lys Ala Ala Arg
            20                  25                  30
ctg ctg gat cgg ctg tgg tgg gag ccg cgg cgg ctg gag cgg gcg ctg 144
Leu Leu Asp Arg Leu Trp Trp Glu Pro Arg Arg Leu Glu Arg Ala Leu
        35                  40                  45
cgc gcg cag ggc ctg cgg ggc acg tcg tac cgc ttc ctc acg ggc gac 192
Arg Ala Gln Gly Leu Arg Gly Thr Ser Tyr Arg Phe Leu Thr Gly Asp
    50                  55                  60
ctc agg gag tac cgc cgg tca aaa gag gag gcc tgg gcc agg ccc ctg 240
Leu Arg Glu Tyr Arg Arg Ser Lys Glu Glu Ala Trp Ala Arg Pro Leu
65                  70                  75                  80
ccg ctg cgg tgc cac gac atc gcc ggc cac gtc gag ccg ttc atc cac 288
Pro Leu Arg Cys His Asp Ile Ala Gly His Val Glu Pro Phe Ile His
                85                  90                  95
ggc gcc gtc ctg gag cac ggc aag acg tgc ttc acc tgg ttc ggc ccc 336
Gly Ala Val Leu Glu His Gly Lys Thr Cys Phe Thr Trp Phe Gly Pro
            100                 105                 110
gtc ccc agg gtg acc gtc gtc gac ccg gac ctg gcg agg gat gtg atg 384
Val Pro Arg Val Thr Val Val Asp Pro Asp Leu Ala Arg Asp Val Met
        115                 120                 125
gcc aac aag ttc ggc cac ttc gag aag ccc aag ttc ccg gcg ctc acc 432
Ala Asn Lys Phe Gly His Phe Glu Lys Pro Lys Phe Pro Ala Leu Thr
    130                 135                 140
aag ctg ttc tcc gac ggt gtg gcc aac cac gaa ggc gag aag tgg gtc 480
Lys Leu Phe Ser Asp Gly Val Ala Asn His Glu Gly Glu Lys Trp Val
145                 150                 155                 160
aag cac agg agg atc ctc aac cct gcg ttc cac ctc gag aag ctc aag 528
Lys His Arg Arg Ile Leu Asn Pro Ala Phe His Leu Glu Lys Leu Lys
                165                 170                 175
ctc atg ctg ccg gcg ttc tct gcg tgc tgt gaa gag ctc gtc agc cga 576
Leu Met Leu Pro Ala Phe Ser Ala Cys Cys Glu Glu Leu Val Ser Arg
            180                 185                 190
tgg gcg cag tcg ctt ggc cct gac ggc tcg tgc gag ctg gac gtg gac 624
Trp Ala Gln Ser Leu Gly Pro Asp Gly Ser Cys Glu Leu Asp Val Asp
        195                 200                 205
ccg gag ctt cag acc ctc acc ggc gat gtc atc tct cgc acc gcg ttc 672
Pro Glu Leu Gln Thr Leu Thr Gly Asp Val Ile Ser Arg Thr Ala Phe
    210                 215                 220
ggc agc agc tac ctc gaa gga aga aag att ttc cag ctg cag gcc gag 720
Gly Ser Ser Tyr Leu Glu Gly Arg Lys Ile Phe Gln Leu Gln Ala Glu
225                 230                 235                 240
caa gct gag cgc ctc atg tcc atc att gac aaa ttc gcc gtt ccg gga 768
Gln Ala Glu Arg Leu Met Ser Ile Ile Asp Lys Phe Ala Val Pro Gly
                245                 250                 255
tac atg tcc ttg cct acg aaa aac aac cga agg atg cgc caa atc aaa 816
Tyr Met Ser Leu Pro Thr Lys Asn Asn Arg Arg Met Arg Gln Ile Lys
            260                 265                 270
agc gag atc gac tcg att ctt cgg ggc ctg atc ggt aaa aga atg caa 864
Ser Glu Ile Asp Ser Ile Leu Arg Gly Leu Ile Gly Lys Arg Met Gln
        275                 280                 285
gct atg aaa caa ggt gag agc gac aaa gat gac ctg ctg ggc tta ctg 912
Ala Met Lys Gln Gly Glu Ser Asp Lys Asp Asp Leu Leu Gly Leu Leu
    290                 295                 300
ctg gag tca aac gcg aga gag aca ggc gat cag agc ggc cag ccc ggc 960

Leu Glu Ser Asn Ala Arg Glu Thr Gly Asp Gln Ser Gly Gln Pro Gly
305                 310                 315                 320
caa gga ttg acg atg gaa gaa gtc atg gag gag tgc aag ctg ttc tac      1008
Gln Gly Leu Thr Met Glu Glu Val Met Glu Glu Cys Lys Leu Phe Tyr
                325                 330                 335
ttc gcg gga atg gag acg acg tcg gtg ctg ctc acg tgg acg atg gtc      1056
Phe Ala Gly Met Glu Thr Thr Ser Val Leu Leu Thr Trp Thr Met Val
            340                 345                 350
ctg cta agc atg cac ccg gag tgg cag gac cgt gcg agg gag gaa gtc      1104
Leu Leu Ser Met His Pro Glu Trp Gln Asp Arg Ala Arg Glu Glu Val
        355                 360                 365
ctc gga ctg ttc ggg aag aag aac caa ccg gga tac gac ggc ctg agt      1152
Leu Gly Leu Phe Gly Lys Lys Asn Gln Pro Gly Tyr Asp Gly Leu Ser
    370                 375                 380
cgc ctc aaa aca gtg acc atg atc ctc tac gag gtc ctc cgg ctg tac      1200
Arg Leu Lys Thr Val Thr Met Ile Leu Tyr Glu Val Leu Arg Leu Tyr
385                 390                 395                 400
ccg ccg gcc atc gcg ttc agc agg aaa acg tac aag gag atg gtg gtc      1248
Pro Pro Ala Ile Ala Phe Ser Arg Lys Thr Tyr Lys Glu Met Val Val
                405                 410                 415
gga gac gtc acg tac cct gcc ggc gtg acc ctg gag ctg ccc gtg ctg      1296
Gly Asp Val Thr Tyr Pro Ala Gly Val Thr Leu Glu Leu Pro Val Leu
            420                 425                 430
ttc atc cac cac gac ccc gac atc tgg gga agc gat gcg cac gag ttc      1344
Phe Ile His His Asp Pro Asp Ile Trp Gly Ser Asp Ala His Glu Phe
        435                 440                 445
agg ccg gag agg ttc gcc gag ggg gtg gcc agg gcg tcc aag gat agg      1392
Arg Pro Glu Arg Phe Ala Glu Gly Val Ala Arg Ala Ser Lys Asp Arg
    450                 455                 460
cta gcg ttc ttc ccc ttc ggc tgg ggc ccg cgg ata tgc atc ggc cag      1440
Leu Ala Phe Phe Pro Phe Gly Trp Gly Pro Arg Ile Cys Ile Gly Gln
465                 470                 475                 480
aac ttc gcc ctt ctg gag gcc aag atg gcg ctg agc atg atc ctt cag      1488
Asn Phe Ala Leu Leu Glu Ala Lys Met Ala Leu Ser Met Ile Leu Gln
                485                 490                 495
cgc ttc cag ttc gag ctc gcg ccg acg tat acc cat gcg ccg cgc cga      1536
Arg Phe Gln Phe Glu Leu Ala Pro Thr Tyr Thr His Ala Pro Arg Arg
            500                 505                 510
gtt ata atg ctg cgc ccg atg cat ggt gcc cag atc aag ctc aga gcg      1584
Val Ile Met Leu Arg Pro Met His Gly Ala Gln Ile Lys Leu Arg Ala
        515                 520                 525
ata tga                                                              1590
Ile

<210> 285
<211> 529
<212> PRT
<213> Zea mays

<400> 285
Met Val Leu Gly Ala Leu Ala Ser Ala Val Ala Ser Met Ser Leu Leu
1               5                   10                  15
Ile Val Tyr Gly Val Leu Gly Ala Leu Leu Leu Trp Lys Ala Ala Arg
            20                  25                  30
Leu Leu Asp Arg Leu Trp Trp Glu Pro Arg Arg Leu Glu Arg Ala Leu
        35                  40                  45
Arg Ala Gln Gly Leu Arg Gly Thr Ser Tyr Arg Phe Leu Thr Gly Asp
    50                  55                  60
Leu Arg Glu Tyr Arg Arg Ser Lys Glu Glu Ala Trp Ala Arg Pro Leu
65                  70                  75                  80
Pro Leu Arg Cys His Asp Ile Ala Gly His Val Glu Pro Phe Ile His
                85                  90                  95
Gly Ala Val Leu Glu His Gly Lys Thr Cys Phe Thr Trp Phe Gly Pro
            100                 105                 110
Val Pro Arg Val Thr Val Val Asp Pro Asp Leu Ala Arg Asp Val Met
        115                 120                 125
Ala Asn Lys Phe Gly His Phe Glu Lys Pro Lys Phe Pro Ala Leu Thr
    130                 135                 140

Lys Leu Phe Ser Asp Gly Val Ala Asn His Glu Gly Glu Lys Trp Val
145                 150                 155                 160
Lys His Arg Arg Ile Leu Asn Pro Ala Phe His Leu Glu Lys Leu Lys
                165                 170                 175
Leu Met Leu Pro Ala Phe Ser Ala Cys Cys Glu Glu Leu Val Ser Arg
            180                 185                 190
Trp Ala Gln Ser Leu Gly Pro Asp Gly Ser Cys Glu Leu Asp Val Asp
        195                 200                 205
Pro Glu Leu Gln Thr Leu Thr Gly Asp Val Ile Ser Arg Thr Ala Phe
    210                 215                 220
Gly Ser Ser Tyr Leu Glu Gly Arg Lys Ile Phe Gln Leu Gln Ala Glu
225                 230                 235                 240
Gln Ala Glu Arg Leu Met Ser Ile Ile Asp Lys Phe Ala Val Pro Gly
                245                 250                 255
Tyr Met Ser Leu Pro Thr Lys Asn Asn Arg Arg Met Arg Gln Ile Lys
            260                 265                 270
Ser Glu Ile Asp Ser Ile Leu Arg Gly Leu Ile Gly Lys Arg Met Gln
        275                 280                 285
Ala Met Lys Gln Gly Glu Ser Asp Lys Asp Asp Leu Leu Gly Leu Leu
    290                 295                 300
Leu Glu Ser Asn Ala Arg Glu Thr Gly Asp Gln Ser Gly Gln Pro Gly
305                 310                 315                 320
Gln Gly Leu Thr Met Glu Glu Val Met Glu Glu Cys Lys Leu Phe Tyr
                325                 330                 335
Phe Ala Gly Met Glu Thr Thr Ser Val Leu Leu Thr Trp Thr Met Val
            340                 345                 350
Leu Leu Ser Met His Pro Glu Trp Gln Asp Arg Ala Arg Glu Glu Val
        355                 360                 365
Leu Gly Leu Phe Gly Lys Lys Asn Gln Pro Gly Tyr Asp Gly Leu Ser
    370                 375                 380
Arg Leu Lys Thr Val Thr Met Ile Leu Tyr Glu Val Leu Arg Leu Tyr
385                 390                 395                 400
Pro Pro Ala Ile Ala Phe Ser Arg Lys Thr Tyr Lys Glu Met Val Val
                405                 410                 415
Gly Asp Val Thr Tyr Pro Ala Gly Val Thr Leu Glu Leu Pro Val Leu
            420                 425                 430
Phe Ile His His Asp Pro Asp Ile Trp Gly Ser Asp Ala His Glu Phe
        435                 440                 445
Arg Pro Glu Arg Phe Ala Glu Gly Val Ala Arg Ala Ser Lys Asp Arg
    450                 455                 460
Leu Ala Phe Phe Pro Phe Gly Trp Gly Pro Arg Ile Cys Ile Gly Gln
465                 470                 475                 480
Asn Phe Ala Leu Leu Glu Ala Lys Met Ala Leu Ser Met Ile Leu Gln
                485                 490                 495
Arg Phe Gln Phe Glu Leu Ala Pro Thr Tyr Thr His Ala Pro Arg Arg
            500                 505                 510
Val Ile Met Leu Arg Pro Met His Gly Ala Gln Ile Lys Leu Arg Ala
        515                 520                 525
Ile

<210> 286
<211> 22
<212> DNA
<213> Artificial sequence

<400> 286
atgttgctgg aacttgcact tg                                              22

<210> 287
<211> 22
<212> DNA
<213> Artificial sequence

<400> 287
ttaagaaagg agtttagatg ca                                              22

<210> 288

<211> 40
<212> PRT
<213> Artificial sequence

<220>
<221> Variant
<222> (1)..(1)
<223> Xaa in position 1 is Ile, Leu, Met or Val
<220>
<221> Variant
<222> (2)..(7)
<223> Xaa in position 2 to 7 is any amino acid
<220>
<221> Variant
<222> (8)..(8)
<223> Xaa in position 8 is Phe, Leu, Ile, Val or Tyr
<220>
<221> Variant
<222> (9)..(9)
<223> Xaa in position 9 is Leu, Ile, Met or Phe
<220>
<221> Variant
<222> (10)..(10)
<223> Xaa in position 10 is any amino acid
<220>
<221> Variant
<222> (11)..(11)
<223> Xaa in position 11 is Ala, Gly, Glu or Ser
<220>
<221> Variant
<222> (12)..(12)
<223> Xaa in position 12 is Gly, Ser or Ala
<220>
<221> Variant
<222> (13)..(13)
<223> Xaa in position 13 is Gln, His, Tyr, Phe, Arg or Lys
<220>
<221> Variant
<222> (14)..(14)
<223> Xaa in position 14 is Asp, Glu or His
<220>
<221> Variant
<222> (15)..(15)
<223> Xaa in position 15 is Thr, Ala or Ser
<220>
<221> Variant
<222> (16)..(16)
<223> Xaa in position 16 is Thr, Ser, Val or Leu
<220>
<221> Variant
<222> (17)..(17)
<223> Xaa in position 17 is Ser, Ala, Thr or Gly
<220>
<221> Variant
<222> (18)..(18)
<223> Xaa in position 18 is Ser, Thr, Ala, His or Asn
<220>
<221> Variant
<222> (19)..(20)
<223> Xaa in position 19 to 20 is any amino acid
<220>
<221> Variant
<222> (21)..(21)
<223> Xaa in position 21 is Ser, Thr, Cys, Met, Ala or Leu
<220>
<221> Variant
<222> (22)..(22)
<223> Xaa in position 22 is Trp, Phe, Ser, Met or Leu
<220>

<221> Variant
<222> (23)..(26)
<223> xaa in position 23 to 26 is any amino acid
<220>
<221> Variant
<222> (27)..(27)
<223> xaa in position 27 is Leu, Val or Ile
<220>
<221> Variant
<222> (28)..(30)
<223> xaa in position 28 to 30 is any amino acid
<220>
<221> Variant
<222> (31)..(31)
<223> xaa in position 31 is Pro or Thr
<220>
<221> Variant
<222> (32)..(33)
<223> xaa in position 32 to 33 is any amino acid
<220>
<221> Variant
<222> (34)..(34)
<223> xaa in position 34 is Leu, Gln, Val, Met or Ile
<220>
<221> Variant
<222> (35)..(35)
<223> xaa in position 35 is any amino acid
<220>
<221> Variant
<222> (36)..(36)
<223> xaa in position 36 is Lys, Arg, Glu, Ala or Asn
<220>
<221> Variant
<222> (37)..(37)
<223> xaa in position 37 is Leu, Val, Ile, Cys or Ala
<220>
<221> Variant
<222> (38)..(38)
<223> xaa in position 38 is any amino acid
<220>
<221> Variant
<222> (39)..(39)
<223> xaa in position 39 is Glu, Ala, Gln, Asp or Lys
<220>
<221> Variant
<222> (40)..(40)
<223> xaa in position 40 is Glu or Asp

<400> 288
xaa xaa xaa xaa xaa xaa xaa xaa xaa xaa xaa xaa xaa xaa xaa xaa
1               5                   10                  15
xaa xaa xaa xaa xaa xaa xaa xaa xaa xaa xaa xaa xaa xaa xaa xaa
            20                  25                  30
xaa xaa xaa xaa xaa xaa xaa xaa
        35                  40


<210> 289
<211> 21
<212> PRT
<213> Artificial sequence

<220>
<221> Variant
<222> (1)..(1)
<223> xaa in position 1 is Leu, Ile, Pro, Val or Ala
<220>
<221> Variant
<222> (2)..(5)

<223> xaa in position 2 to 5 is any amino acid
<220>
<221> Variant
<222> (6)..(6)
<223> xaa in position 6 is Leu, Ile, Val, Met, Tyr or Thr
<220>
<221> Variant
<222> (7)..(8)
<223> xaa in position 7 to 8 is any amino acid
<220>
<221> Variant
<222> (9)..(9)
<223> xaa in position 9 is Met, Leu or Ile
<220>
<221> Variant
<222> (10)..(10)
<223> xaa in position 10 is any amino acid
<220>
<221> Variant
<222> (11)..(11)
<223> xaa in position 11 is Tyr, Leu, Phe or Thr
<220>
<221> Variant
<222> (12)..(12)
<223> xaa in position 12 is Thr, Leu, Met, His or Ile
<220>
<221> Variant
<222> (13)..(14)
<223> xaa in position 13 to 14 is any amino acid
<220>
<221> Variant
<222> (15)..(15)
<223> xaa in position 15 is Val, Cys, Ile or Ala
<220>
<221> Variant
<222> (16)..(16)
<223> xaa in position 16 is Ile, Val, Ala or Leu
<220>
<221> Variant
<222> (17)..(17)
<223> xaa in position 17 is any amino acid
<220>
<221> Variant
<222> (19)..(19)
<223> xaa in position 19 is Thr, Ser, Val, Ala or Cys
<220>
<221> Variant
<222> (20)..(20)
<223> xaa in position 20 is Leu, Val, Ser or Met

<400> 289
xaa xaa xaa xaa xaa xaa xaa xaa xaa xaa xaa xaa xaa xaa xaa xaa
1               5                   10                  15
xaa Glu xaa xaa Arg
            20


<210> 290
<211> 33
<212> PRT
<213> Artificial sequence

<220>
<221> Variant
<222> (1)..(1)
<223> xaa in position 1 is Ile, Val, Ala or Leu
<220>
<221> Variant
<222> (3)..(3)

<223> Xaa in position 3 is Lys, Ala or Thr
<220>
<221> Variant
<222> (4)..(4)
<223> Xaa in position 4 is Gly, Asp or Ser
<220>
<221> Variant
<222> (5)..(6)
<223> Xaa in position 5 to 6 is any amino acid
<220>
<221> Variant
<222> (7)..(7)
<223> Xaa in position 7 is Val, Ile, Leu or Cys
<220>
<221> Variant
<222> (8)..(14)
<223> Xaa in position 8 to 14 is any amino acid
<220>
<221> Variant
<222> (15)..(15)
<223> Xaa in position 15 is His, Leu, Ser, Asn, Gln or Gly
<220>
<221> Variant
<222> (16)..(18)
<223> Xaa in position 16 to 18 is any amino acid
<220>
<221> Variant
<222> (19)..(20)
<223> Xaa in position 19 to 20 is any or no amino acid
<220>
<221> Variant
<222> (21)..(21)
<223> Xaa in position 21 is Tyr, Phe or Trp
<220>
<221> Variant
<222> (22)..(23)
<223> Xaa in position 22 to 23 is any amino acid
<220>
<221> Variant
<222> (24)..(24)
<223> Xaa in position 24 is any or no amino acid
<220>
<221> Variant
<222> (25)..(25)
<223> Xaa in position 25 is Pro, Ala, Lys or Val
<220>
<221> Variant
<222> (26)..(27)
<223> Xaa in position 26 to 27 is any amino acid
<220>
<221> Variant
<222> (28)..(28)
<223> Xaa in position 28 is Phe, Tyr or Trp
<220>
<221> Variant
<222> (29)..(29)
<223> Xaa in position 29 is any amino acid
<220>
<221> Variant
<222> (31)..(31)
<223> Xaa in position 31 is any amino acid
<220>
<221> Variant
<222> (33)..(33)
<223> Xaa in position 33 is Phe or Trp

<400> 290
Xaa Pro Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Xaa Xaa Xaa Xaa Xaa Xaa Pro Xaa Arg
            20                  25                  30
Xaa

<210> 291
<211> 25
<212> PRT
<213> Artificial sequence

<220>
<221> Variant
<222> (1)..(1)
<223> Xaa in position 1 is Phe, Tyr or Trp
<220>
<221> Variant
<222> (2)..(2)
<223> Xaa in position 2 is Leu, Met, Ile or Phe
<220>
<221> Variant
<222> (3)..(3)
<223> Xaa in position 3 is Pro, Val, Ala or Thr
<220>
<221> Variant
<222> (5)..(5)
<223> Xaa in position 5 is Gly or Ser
<220>
<221> Variant
<222> (6)..(6)
<223> Xaa in position 6 is any amino acid
<220>
<221> Variant
<222> (8)..(8)
<223> Xaa in position 8 is any amino acid
<220>
<221> Variant
<222> (9)..(9)
<223> Xaa in position 9 is Arg or His
<220>
<221> Variant
<222> (10)..(10)
<223> Xaa in position 10 is any amino acid
<220>
<221> Variant
<222> (12)..(12)
<223> Xaa in position 12 is Ile, Leu, Pro or Val
<220>
<221> Variant
<222> (14)..(15)
<223> Xaa in position 14 to 15 is any amino acid
<220>
<221> Variant
<222> (16)..(16)
<223> Xaa in position 16 is Phe, Tyr or Leu
<220>
<221> Variant
<222> (17)..(17)
<223> Xaa in position 17 is Ala, Val or Gly
<220>
<221> Variant
<222> (18)..(20)
<223> Xaa in position 18 to 20 is any amino acid
<220>
<221> Variant
<222> (21)..(21)
<223> Xaa in position 21 is Leu, Met, Ile, Phe, Ala or Thr
<220>
<221> Variant

<222> (22)..(24)
<223> xaa in position 22 to 24 is any amino acid
<220>
<221> Variant
<222> (25)..(25)
<223> xaa in position 25 is Leu, Ile, Val, Met, Ala or Thr

<400> 291
xaa xaa xaa Phe xaa xaa Gly xaa xaa xaa Cys xaa Gly xaa xaa xaa
1               5                   10                  15
xaa xaa xaa xaa xaa xaa xaa xaa xaa
            20                  25

<210> 292
<211> 813
<212> DNA
<213> Zea mays

<220>
<221> CDS
<222> (103)..(549)

<400> 292
gaattcccgg gtcgacttcg ctgtcgacga tttcgtgtta taaaggcaca gctcttctgt      60

ctccacgaga ttgttgaatt catctctgcc ctagcaccag cc atg gcg aag ccg      114
                                               Met Ala Lys Pro
                                               1
cag ctt ctt ctc tgt ttc cta ctc gcc gcc tcc gcg ccg cta atc gtc      162
Gln Leu Leu Leu Cys Phe Leu Leu Ala Ala Ser Ala Pro Leu Ile Val
5                   10                  15                  20
gcc acg gcc aag aaa tcc ggg gac gtg tcg gag ctc cag atc ggc gtc      210
Ala Thr Ala Lys Lys Ser Gly Asp Val Ser Glu Leu Gln Ile Gly Val
                25                  30                  35
aag tac aag ccg gaa tca tgc agc att caa gct cac aaa ggt gac aag      258
Lys Tyr Lys Pro Glu Ser Cys Ser Ile Gln Ala His Lys Gly Asp Lys
            40                  45                  50
gtt aaa gtt cac tac cgt gga aaa ctt act gat gga acg gtt ttt gat      306
Val Lys Val His Tyr Arg Gly Lys Leu Thr Asp Gly Thr Val Phe Asp
        55                  60                  65
tca agc tat gaa aga ggt gac ccg att gaa ttt gaa ttg ggc act gga      354
Ser Ser Tyr Glu Arg Gly Asp Pro Ile Glu Phe Glu Leu Gly Thr Gly
    70                  75                  80
caa gtg atc aaa gga tgg gat cag ggt ctc ctg gga atg tgc gtt ggt      402
Gln Val Ile Lys Gly Trp Asp Gln Gly Leu Leu Gly Met Cys Val Gly
85                  90                  95                  100
gaa aag cgg aag ttg aag atc cca tca aag ctt ggc tat ggg cct caa      450
Glu Lys Arg Lys Leu Lys Ile Pro Ser Lys Leu Gly Tyr Gly Pro Gln
                105                 110                 115
ggc tcg cca cct act att cct ggt gga gca acc ctc ata ttt gac acg      498
Gly Ser Pro Pro Thr Ile Pro Gly Gly Ala Thr Leu Ile Phe Asp Thr
            120                 125                 130
gag ctt gtc gct gtc aat gga gaa cca acc aac caa tct gac gag ctt      546
Glu Leu Val Ala Val Asn Gly Glu Pro Thr Asn Gln Ser Asp Glu Leu
        135                 140                 145
tag attcatcaaa caaatgaatc cattcacctt tttactcgga actcagcaga      599

tgtaatactt tccaagaaat gagccttcat tccctgccac tttcatagtt tctttcgctg      659

ttccacactg acatctatga tttttggagc aaactctgat ctgttttgac tagtttacac      719

gtttgtgtgt ggaaaaaaaa agaaaaaaaa aaaaaaaaaa aaaaaaaaaa aaaaaaaaaa      779

aaaaaaagaa aaaaaaaaaa aaaaaagcgg ccgc                                  813

<210> 293
<211> 148
<212> PRT
<213> Zea mays

<400> 293
Met Ala Lys Pro Gln Leu Leu Leu Cys Phe Leu Leu Ala Ala Ser Ala
1               5                   10                  15
Pro Leu Ile Val Ala Thr Ala Lys Lys Ser Gly Asp Val Ser Glu Leu
            20                  25                  30
Gln Ile Gly Val Lys Tyr Lys Pro Glu Ser Cys Ser Ile Gln Ala His
        35                  40                  45
Lys Gly Asp Lys Val Lys Val His Tyr Arg Gly Lys Leu Thr Asp Gly
    50                  55                  60
Thr Val Phe Asp Ser Ser Tyr Glu Arg Gly Asp Pro Ile Glu Phe Glu
65                  70                  75                  80
Leu Gly Thr Gly Gln Val Ile Lys Gly Trp Asp Gln Gly Leu Leu Gly
                85                  90                  95
Met Cys Val Gly Glu Lys Arg Lys Leu Lys Ile Pro Ser Lys Leu Gly
            100                 105                 110
Tyr Gly Pro Gln Gly Ser Pro Pro Thr Ile Pro Gly Gly Ala Thr Leu
        115                 120                 125
Ile Phe Asp Thr Glu Leu Val Ala Val Asn Gly Glu Pro Thr Asn Gln
    130                 135                 140
Ser Asp Glu Leu
145

<210> 294
<211> 339
<212> DNA
<213> Glycine max

<220>
<221> CDS
<222> (1)..(339)

<400> 294
atg gga gtg gag aag cag ttg ttg agg tct ggc acc ggt ccg aag cca       48
Met Gly Val Glu Lys Gln Leu Leu Arg Ser Gly Thr Gly Pro Lys Pro
1               5                   10                  15
att ccc ggt cag aac gta acc gtt cac tgc act ggt ttc ggg aag aac       96
Ile Pro Gly Gln Asn Val Thr Val His Cys Thr Gly Phe Gly Lys Asn
            20                  25                  30
ggt gac ctt tct cag aaa ttc tgg agc aca aag gat cct ggc cag aac      144
Gly Asp Leu Ser Gln Lys Phe Trp Ser Thr Lys Asp Pro Gly Gln Asn
        35                  40                  45
cca ttc act ttc aaa atc ggc caa ggt tct gtt atc aaa gga tgg gat      192
Pro Phe Thr Phe Lys Ile Gly Gln Gly Ser Val Ile Lys Gly Trp Asp
    50                  55                  60
gaa ggt gtg ctt ggc atg caa att ggt gaa gtt gct cgt ctc cgg tgc      240
Glu Gly Val Leu Gly Met Gln Ile Gly Glu Val Ala Arg Leu Arg Cys
65                  70                  75                  80
tct ccg gat tat gct tat ggt gct ggt ggc ttt cct gct tgg gga ata      288
Ser Pro Asp Tyr Ala Tyr Gly Ala Gly Gly Phe Pro Ala Trp Gly Ile
                85                  90                  95
cgg ccc aac tct gtc ttg gaa ttt gaa atc gaa gtc tta agt gcg caa      336
Arg Pro Asn Ser Val Leu Glu Phe Glu Ile Glu Val Leu Ser Ala Gln
            100                 105                 110
tga                                                                  339

<210> 295
<211> 112
<212> PRT
<213> Glycine max

<400> 295
Met Gly Val Glu Lys Gln Leu Leu Arg Ser Gly Thr Gly Pro Lys Pro
1               5                   10                  15
Ile Pro Gly Gln Asn Val Thr Val His Cys Thr Gly Phe Gly Lys Asn
            20                  25                  30
Gly Asp Leu Ser Gln Lys Phe Trp Ser Thr Lys Asp Pro Gly Gln Asn
        35                  40                  45
Pro Phe Thr Phe Lys Ile Gly Gln Gly Ser Val Ile Lys Gly Trp Asp
    50                  55                  60
Glu Gly Val Leu Gly Met Gln Ile Gly Glu Val Ala Arg Leu Arg Cys
65                  70                  75                  80
Ser Pro Asp Tyr Ala Tyr Gly Ala Gly Gly Phe Pro Ala Trp Gly Ile
                85                  90                  95
Arg Pro Asn Ser Val Leu Glu Phe Glu Ile Glu Val Leu Ser Ala Gln
            100                 105                 110

<210> 296
<211> 339
<212> DNA
<213> Glycine max

<220>
<221> CDS
<222> (1)..(339)

<400> 296
atg gga gtg gag aag cag ttg ttg agg tct ggc acc ggt ccg aag cca      48
Met Gly Val Glu Lys Gln Leu Leu Arg Ser Gly Thr Gly Pro Lys Pro
1               5                   10                  15
att ccc ggt cag aac gta acc gtt cac tgc act ggt ttc ggg aag aac      96
Ile Pro Gly Gln Asn Val Thr Val His Cys Thr Gly Phe Gly Lys Asn
            20                  25                  30
ggt gac ctt tct cag aaa ttc tgg agc aca aag gat cct ggc cag aac      144
Gly Asp Leu Ser Gln Lys Phe Trp Ser Thr Lys Asp Pro Gly Gln Asn
        35                  40                  45
cca ttc act ttc aaa atc ggc caa ggt tct gtt atc aaa gga tgg gat      192
Pro Phe Thr Phe Lys Ile Gly Gln Gly Ser Val Ile Lys Gly Trp Asp
    50                  55                  60
gaa ggt gtg ctt ggc atg caa att ggt gaa gtt gct cgt ctc cgg tgc      240
Glu Gly Val Leu Gly Met Gln Ile Gly Glu Val Ala Arg Leu Arg Cys
65                  70                  75                  80
tct ccg gat tat gct tat ggt gct ggt ggc ttt cct tct tgg gga ata      288
Ser Pro Asp Tyr Ala Tyr Gly Ala Gly Gly Phe Pro Ser Trp Gly Ile
                85                  90                  95
cag ccc aac tct gtc ttg gaa ttt gaa atc gaa gtc tta agt gcg caa      336
Gln Pro Asn Ser Val Leu Glu Phe Glu Ile Glu Val Leu Ser Ala Gln
            100                 105                 110
tga                                                                  339

<210> 297
<211> 112
<212> PRT
<213> Glycine max

<400> 297
Met Gly Val Glu Lys Gln Leu Leu Arg Ser Gly Thr Gly Pro Lys Pro
1               5                   10                  15
Ile Pro Gly Gln Asn Val Thr Val His Cys Thr Gly Phe Gly Lys Asn
            20                  25                  30
Gly Asp Leu Ser Gln Lys Phe Trp Ser Thr Lys Asp Pro Gly Gln Asn
        35                  40                  45
Pro Phe Thr Phe Lys Ile Gly Gln Gly Ser Val Ile Lys Gly Trp Asp
    50                  55                  60
Glu Gly Val Leu Gly Met Gln Ile Gly Glu Val Ala Arg Leu Arg Cys
65                  70                  75                  80
Ser Pro Asp Tyr Ala Tyr Gly Ala Gly Gly Phe Pro Ser Trp Gly Ile

85                  90                  95
Gln Pro Asn Ser Val Leu Glu Phe Glu Ile Glu Val Leu Ser Ala Gln
            100                 105                 110

<210> 298
<211> 22
<212> DNA
<213> Artificial sequence

<400> 298
atggcgaagc cgcagcttct tc                                                22

<210> 299
<211> 22
<212> DNA
<213> Artificial sequence

<400> 299
ctaaagctcg tcagattggt tg                                                22


<210> 300
<211> 160
<212> PRT
<213> Artificial sequence

<220>
<221> Variant
<222> (2)..(6)
<223> xaa in position 2 to 6 is any amino acid
<220>
<221> Variant
<222> (9)..(9)
<223> xaa in position 9 is any amino acid
<220>
<221> Variant
<222> (11)..(21)
<223> xaa in position 11 to 21 is any amino acid
<220>
<221> Variant
<222> (23)..(24)
<223> xaa in position 23 to 24 is any amino acid
<220>
<221> Variant
<222> (29)..(30)
<223> xaa in position 29 to 30 is any amino acid
<220>
<221> Variant
<222> (32)..(46)
<223> xaa in position 32 to 46 is any amino acid
<220>
<221> Variant
<222> (48)..(49)
<223> xaa in position 48 to 49 is any amino acid
<220>
<221> Variant
<222> (51)..(52)
<223> xaa in position 51 to 52 is any amino acid
<220>
<221> Variant
<222> (54)..(56)
<223> xaa in position 54 to 56 is any amino acid
<220>
<221> Variant
<222> (59)..(59)
<223> xaa in position 59 is any amino acid
<220>
<221> Variant
<222> (62)..(63)

<223> xaa in position 62 to 63 is any amino acid
<220>
<221> Variant
<222> (65)..(78)
<223> xaa in position 65 to 78 is any amino acid
<220>
<221> Variant
<222> (80)..(81)
<223> xaa in position 80 to 81 is any amino acid
<220>
<221> Variant
<222> (83)..(86)
<223> xaa in position 83 to 86 is any amino acid
<220>
<221> Variant
<222> (88)..(94)
<223> xaa in position 88 to 94 is any amino acid
<220>
<221> Variant
<222> (97)..(98)
<223> xaa in position 97 to 98 is any amino acid
<220>
<221> Variant
<222> (100)..(147)
<223> xaa in position 100 to 147 is any amino acid
<220>
<221> Variant
<222> (149)..(153)
<223> xaa in position 149 to 153 is any amino acid
<220>
<221> Variant
<222> (155)..(155)
<223> xaa in position 155 is any amino acid
<220>
<221> Variant
<222> (157)..(158)
<223> xaa in position 157 to 158 is any amino acid

<400> 300
Gly xaa xaa xaa xaa xaa His Tyr xaa Gly xaa xaa xaa xaa xaa xaa
1               5                   10                  15
xaa xaa xaa xaa xaa Gly xaa xaa Phe Asp Ser Ser xaa xaa Arg xaa
            20                  25                  30
xaa xaa xaa xaa xaa xaa xaa xaa xaa xaa xaa xaa xaa xaa Pro xaa
        35                  40                  45
xaa Phe xaa xaa Gly xaa xaa xaa Val Ile xaa Gly Trp xaa xaa Gly
    50                  55                  60
xaa xaa xaa xaa xaa xaa xaa xaa xaa xaa xaa xaa xaa xaa Met xaa
65                  70                  75                  80
xaa Gly xaa xaa xaa xaa Leu xaa xaa xaa xaa xaa xaa xaa Tyr Gly
                85                  90                  95
xaa xaa Gly xaa xaa xaa xaa xaa xaa xaa xaa xaa xaa xaa xaa xaa
            100                 105                 110
xaa xaa xaa xaa xaa xaa xaa xaa xaa xaa xaa xaa xaa xaa xaa xaa
        115                 120                 125
xaa xaa xaa xaa xaa xaa xaa xaa xaa xaa xaa xaa xaa xaa xaa xaa
    130                 135                 140
xaa xaa xaa Ile xaa xaa xaa xaa xaa Leu xaa Phe xaa xaa Glu Leu
145                 150                 155                 160

<210> 301
<211> 89
<212> PRT
<213> Artificial sequence

<220>
<221> Variant

<222> (1)..(1)
<223> xaa in position 1 is Tyr, Cys or Phe
<220>
<221> Variant
<222> (2)..(2)
<223> xaa in position 2 is any amino acid
<220>
<221> Variant
<222> (3)..(3)
<223> xaa in position 3 is Gly, Ala or Leu
<220>
<221> Variant
<222> (4)..(7)
<223> xaa in position 4 to 7 is any amino acid
<220>
<221> Variant
<222> (8)..(14)
<223> xaa in position 8 to 14 is any or no amino acid
<220>
<221> Variant
<222> (15)..(15)
<223> xaa in position 15 is Gly, Thr, Asp or Gln
<220>
<221> Variant
<222> (16)..(17)
<223> xaa in position 16 to 17 is any amino acid
<220>
<221> Variant
<222> (18)..(18)
<223> xaa in position 18 is Phe, Trp, Met, Ile or Val
<220>
<221> Variant
<222> (19)..(19)
<223> xaa in position 19 is Asp, Ser or Trp
<220>
<221> Variant
<222> (20)..(20)
<223> xaa in position 20 is Ser, Asn, Thr or Ala
<220>
<221> Variant
<222> (21)..(21)
<223> xaa in position 21 is Ser, Thr, Ile or Lys
<220>
<221> Variant
<222> (22)..(31)
<223> xaa in position 22 to 31 is any amino acid
<220>
<221> Variant
<222> (32)..(44)
<223> xaa in position 32 to 44 is any or no amino acid
<220>
<221> Variant
<222> (45)..(45)
<223> xaa in position 45 is Leu, Met, Ile, Val or Ala
<220>
<221> Variant
<222> (46)..(46)
<223> xaa in position 46 is any amino acid
<220>
<221> Variant
<222> (47)..(49)
<223> xaa in position 47 to 49 is any or no amino acid
<220>
<221> Variant
<222> (50)..(50)
<223> xaa in position 50 is Val, Ile, Leu or Thr
<220>
<221> Variant
<222> (51)..(51)

<223> xaa in position 51 is Ile, Val or Phe
<220>
<221> Variant
<222> (52)..(52)
<223> xaa in position 52 is any amino acid
<220>
<221> Variant
<222> (53)..(53)
<223> xaa in position 53 is Gly, Ala or Cys
<220>
<221> Variant
<222> (54)..(54)
<223> xaa in position 54 is Trp, Phe, Leu or Cys
<220>
<221> Variant
<222> (55)..(60)
<223> xaa in position 55 to 60 is any amino acid
<220>
<221> Variant
<222> (61)..(71)
<223> xaa in position 61 to 71 is any or no amino acid
<220>
<221> Variant
<222> (72)..(72)
<223> xaa in position 72 is Met, Ile, Leu, Ala or His
<220>
<221> Variant
<222> (73)..(74)
<223> xaa in position 73 to 74 is any amino acid
<220>
<221> Variant
<222> (76)..(86)
<223> xaa in position 76 to 86 is any amino acid
<220>
<221> Variant
<222> (87)..(87)
<223> xaa in position 87 is Ala or Gly
<220>
<221> Variant
<222> (88)..(88)
<223> xaa in position 88 is Tyr or Phe
<220>
<221> Variant
<222> (89)..(89)
<223> xaa in position 89 is Gly or Pro

<400> 301
xaa xaa xaa xaa xaa xaa xaa xaa xaa xaa xaa xaa xaa xaa xaa xaa
1               5                   10                  15
xaa xaa xaa xaa xaa xaa xaa xaa xaa xaa xaa xaa xaa xaa xaa xaa
            20                  25                  30
xaa xaa xaa xaa xaa xaa xaa xaa xaa xaa xaa xaa xaa xaa xaa xaa
        35                  40                  45
xaa xaa xaa xaa xaa xaa xaa xaa xaa xaa xaa xaa xaa xaa xaa xaa
    50                  55                  60
xaa xaa xaa xaa xaa xaa xaa xaa xaa xaa Gly xaa xaa xaa xaa xaa
65                  70                  75                  80
xaa xaa xaa xaa xaa xaa xaa xaa xaa
                85

<210> 302
<211> 1286
<212> DNA
<213> Zea mays

<220>
<221> CDS
<222> (174)..(1088)

<400> 302
ccgggggatc acgtcgttat cagcgaccag attcagacgc gagccgagcg ggggaggcgg      60

accacactag aactagaacc ctctctacgg ctctactacg ctacgcccca tcccgcccgg     120

accggaccgg acccaccttc ctccacctct ctgcccgccg ccggccgacc cga atg        176
                                                           Met
                                                           1
ggg gcg gga acc aat aat ggc ctg agc aac ggc gcc gcc gca ggg cag       224
Gly Ala Gly Thr Asn Asn Gly Leu Ser Asn Gly Ala Ala Ala Gly Gln
            5                   10                  15
cgc gcg gac gac ggg acc acg gtg ttc cgg ggc acg gcg tac tcg ccg       272
Arg Ala Asp Asp Gly Thr Thr Val Phe Arg Gly Thr Ala Tyr Ser Pro
        20                  25                  30
cta cgg acc acg gtg gcg ctc gcg ctg tgg ctc ggg gcc atc cac ttc       320
Leu Arg Thr Thr Val Ala Leu Ala Leu Trp Leu Gly Ala Ile His Phe
    35                  40                  45
aac gcc ttc ctc gtc ctc gcc tcg ctc ttc ctc ttc ccg cgc cgc gtc       368
Asn Ala Phe Leu Val Leu Ala Ser Leu Phe Leu Phe Pro Arg Arg Val
50                  55                  60                  65
gcc gca ctg gtg ctg gcg acg cag ctc ttc ttc atg ttc ctg ccg ctc       416
Ala Ala Leu Val Leu Ala Thr Gln Leu Phe Phe Met Phe Leu Pro Leu
                70                  75                  80
agt gat aag agc aga ctg ggc cgc aag atc gcc agg ttc ata agc aag       464
Ser Asp Lys Ser Arg Leu Gly Arg Lys Ile Ala Arg Phe Ile Ser Lys
            85                  90                  95
tac gtc att ggg tat ttt ccc gtc act ttg cac gtg gaa gac tat ggc       512
Tyr Val Ile Gly Tyr Phe Pro Val Thr Leu His Val Glu Asp Tyr Gly
        100                 105                 110
gcc ttt gat ccc aac agg gct tat gtg ttc ggt tat gag cct cat tct       560
Ala Phe Asp Pro Asn Arg Ala Tyr Val Phe Gly Tyr Glu Pro His Ser
    115                 120                 125
gtt ttg ccc ata gct gtt ggg atc ctc ggg gac ctt gtt gga ttc atg       608
Val Leu Pro Ile Ala Val Gly Ile Leu Gly Asp Leu Val Gly Phe Met
130                 135                 140                 145
ccg cta cca aag atg aag att ctt gca agc agt gcg gtg ttc tac acc       656
Pro Leu Pro Lys Met Lys Ile Leu Ala Ser Ser Ala Val Phe Tyr Thr
                150                 155                 160
ccg ttc cta agg caa ata tgg aca tgg ttg ggg ttg gct cct gcg tcg       704
Pro Phe Leu Arg Gln Ile Trp Thr Trp Leu Gly Leu Ala Pro Ala Ser
            165                 170                 175
aga aag agt ttc tac tcc tac ctt gga gct ggt tat agc tgt att ata       752
Arg Lys Ser Phe Tyr Ser Tyr Leu Gly Ala Gly Tyr Ser Cys Ile Ile
        180                 185                 190
gtg cca gga ggt gtg cag gaa ata ctt cat atg gat cat gat tca gag       800
Val Pro Gly Gly Val Gln Glu Ile Leu His Met Asp His Asp Ser Glu
    195                 200                 205
agc tat gtt tac aaa tgg tgg agg cca ggt ggc aag tta att gtc aag       848
Ser Tyr Val Tyr Lys Trp Trp Arg Pro Gly Gly Lys Leu Ile Val Lys
210                 215                 220                 225
att gct aga gca atc aaa ttt tct cca ata atc ttc tgg gga aaa ctg       896
Ile Ala Arg Ala Ile Lys Phe Ser Pro Ile Ile Phe Trp Gly Lys Leu
                230                 235                 240
ggg act ccc atc cct ttt gca aca cca atg cat gtg att gtt gga agg       944
Gly Thr Pro Ile Pro Phe Ala Thr Pro Met His Val Ile Val Gly Arg
            245                 250                 255
cca att gag gtt gta aag aat cct caa cct acc att gat gag ata aac       992
Pro Ile Glu Val Val Lys Asn Pro Gln Pro Thr Ile Asp Glu Ile Asn
        260                 265                 270
caa gtc cac gga cag ttc gtt gtt gcg atg caa gat ctg ttc gag aaa      1040
Gln Val His Gly Gln Phe Val Val Ala Met Gln Asp Leu Phe Glu Lys
    275                 280                 285
tac aag agc aga act gga tac cct gat ctt cag tta aga gtt ctt tga      1088
Tyr Lys Ser Arg Thr Gly Tyr Pro Asp Leu Gln Leu Arg Val Leu
290                 295                 300                 305
tggcgtgctg ttccaatgaa aagctcttat cagctaggtt taagggataa aatggctgtt    1148

cagacccatc gactcaattg atcgtagcat ttgggctata atgttttttg agcacaggtc     1208

cattattgaa attctgtacc ccattccaaa ttagaagacg tttttttaat atgtagcttt     1268

tccaaaaaaa aaaaaaaa                                                  1286

<210> 303
<211> 304
<212> PRT
<213> Zea mays

<400> 303
Met Gly Ala Gly Thr Asn Asn Gly Leu Ser Asn Gly Ala Ala Ala Gly
1               5                   10                  15
Gln Arg Ala Asp Asp Gly Thr Thr Val Phe Arg Gly Thr Ala Tyr Ser
            20                  25                  30
Pro Leu Arg Thr Thr Val Ala Leu Ala Leu Trp Leu Gly Ala Ile His
        35                  40                  45
Phe Asn Ala Phe Leu Val Leu Ala Ser Leu Phe Leu Phe Pro Arg Arg
    50                  55                  60
Val Ala Ala Leu Val Leu Ala Thr Gln Leu Phe Phe Met Phe Leu Pro
65                  70                  75                  80
Leu Ser Asp Lys Ser Arg Leu Gly Arg Lys Ile Ala Arg Phe Ile Ser
                85                  90                  95
Lys Tyr Val Ile Gly Tyr Phe Pro Val Thr Leu His Val Glu Asp Tyr
            100                 105                 110
Gly Ala Phe Asp Pro Asn Arg Ala Tyr Val Phe Gly Tyr Glu Pro His
        115                 120                 125
Ser Val Leu Pro Ile Ala Val Gly Ile Leu Gly Asp Leu Val Gly Phe
    130                 135                 140
Met Pro Leu Pro Lys Met Lys Ile Leu Ala Ser Ser Ala Val Phe Tyr
145                 150                 155                 160
Thr Pro Phe Leu Arg Gln Ile Trp Thr Trp Leu Gly Leu Ala Pro Ala
                165                 170                 175
Ser Arg Lys Ser Phe Tyr Ser Tyr Leu Gly Ala Gly Tyr Ser Cys Ile
            180                 185                 190
Ile Val Pro Gly Gly Val Gln Glu Ile Leu His Met Asp His Asp Ser
        195                 200                 205
Glu Ser Tyr Val Tyr Lys Trp Trp Arg Pro Gly Gly Lys Leu Ile Val
    210                 215                 220
Lys Ile Ala Arg Ala Ile Lys Phe Ser Pro Ile Ile Phe Trp Gly Lys
225                 230                 235                 240
Leu Gly Thr Pro Ile Pro Phe Ala Thr Pro Met His Val Ile Val Gly
                245                 250                 255
Arg Pro Ile Glu Val Val Lys Asn Pro Gln Pro Thr Ile Asp Glu Ile
            260                 265                 270
Asn Gln Val His Gly Gln Phe Val Val Ala Met Gln Asp Leu Phe Glu
        275                 280                 285
Lys Tyr Lys Ser Arg Thr Gly Tyr Pro Asp Leu Gln Leu Arg Val Leu
    290                 295                 300

<210> 304
<211> 22
<212> DNA
<213> Artificial sequence

<400> 304
atgggggcgg gaaccaataa tg                                             22

<210> 305
<211> 24
<212> DNA
<213> Artificial sequence

<400> 305
tcaaagaact cttaactgaa gatc                                              24


<210> 306
<211> 379
<212> PRT
<213> Artificial sequence

<220>
<221> Variant
<222> (3)..(35)
<223> xaa in position 3 to 35 is any amino acid
<220>
<221> Variant
<222> (37)..(41)
<223> xaa in position 37 to 41 is any amino acid
<220>
<221> Variant
<222> (43)..(48)
<223> xaa in position 43 to 48 is any amino acid
<220>
<221> Variant
<222> (50)..(65)
<223> xaa in position 50 to 65 is any amino acid
<220>
<221> Variant
<222> (67)..(70)
<223> xaa in position 67 to 70 is any amino acid
<220>
<221> Variant
<222> (72)..(76)
<223> xaa in position 72 to 76 is any amino acid
<220>
<221> Variant
<222> (80)..(81)
<223> xaa in position 80 to 81 is any amino acid
<220>
<221> Variant
<222> (83)..(83)
<223> xaa in position 83 is any amino acid
<220>
<221> Variant
<222> (86)..(87)
<223> xaa in position 86 to 87 is any amino acid
<220>
<221> Variant
<222> (89)..(92)
<223> xaa in position 89 to 92 is any amino acid
<220>
<221> Variant
<222> (94)..(140)
<223> xaa in position 94 to 140 is any amino acid
<220>
<221> Variant
<222> (142)..(143)
<223> xaa in position 142 to 143 is any amino acid
<220>
<221> Variant
<222> (145)..(145)
<223> xaa in position 145 is any amino acid
<220>
<221> Variant
<222> (150)..(153)
<223> xaa in position 150 to 153 is any amino acid
<220>
<221> Variant
<222> (155)..(160)
<223> xaa in position 155 to 160 is any amino acid

<220>
<221> Variant
<222> (162)..(169)
<223> xaa in position 162 to 169 is any amino acid
<220>
<221> Variant
<222> (173)..(176)
<223> xaa in position 173 to 176 is any amino acid
<220>
<221> Variant
<222> (178)..(179)
<223> xaa in position 178 to 179 is any amino acid
<220>
<221> Variant
<222> (181)..(183)
<223> xaa in position 181 to 183 is any amino acid
<220>
<221> Variant
<222> (185)..(186)
<223> xaa in position 185 to 186 is any amino acid
<220>
<221> Variant
<222> (188)..(189)
<223> xaa in position 188 to 189 is any amino acid
<220>
<221> Variant
<222> (191)..(196)
<223> xaa in position 191 to 196 is any amino acid
<220>
<221> Variant
<222> (198)..(209)
<223> xaa in position 198 to 209 is any amino acid
<220>
<221> Variant
<222> (211)..(231)
<223> xaa in position 211 to 231 is any amino acid
<220>
<221> Variant
<222> (233)..(234)
<223> xaa in position 233 to 234 is any amino acid
<220>
<221> Variant
<222> (238)..(238)
<223> xaa in position 238 is any amino acid
<220>
<221> Variant
<222> (240)..(240)
<223> xaa in position 240 is any amino acid
<220>
<221> Variant
<222> (242)..(244)
<223> xaa in position 242 to 244 is any amino acid
<220>
<221> Variant
<222> (247)..(249)
<223> xaa in position 247 to 249 is any amino acid
<220>
<221> Variant
<222> (251)..(251)
<223> xaa in position 251 is any amino acid
<220>
<221> Variant
<222> (253)..(254)
<223> xaa in position 253 to 254 is any amino acid
<220>
<221> Variant
<222> (259)..(261)
<223> xaa in position 259 to 261 is any amino acid
<220>

<221> Variant
<222> (263)..(265)
<223> xaa in position 263 to 265 is any amino acid
<220>
<221> Variant
<222> (267)..(269)
<223> xaa in position 267 to 269 is any amino acid
<220>
<221> Variant
<222> (273)..(275)
<223> xaa in position 273 to 275 is any amino acid
<220>
<221> Variant
<222> (280)..(283)
<223> xaa in position 280 to 283 is any amino acid
<220>
<221> Variant
<222> (285)..(290)
<223> xaa in position 285 to 290 is any amino acid
<220>
<221> Variant
<222> (292)..(300)
<223> xaa in position 292 to 300 is any amino acid
<220>
<221> Variant
<222> (302)..(308)
<223> xaa in position 302 to 308 is any amino acid
<220>
<221> Variant
<222> (310)..(317)
<223> xaa in position 310 to 317 is any amino acid
<220>
<221> Variant
<222> (320)..(320)
<223> xaa in position 320 is any amino acid
<220>
<221> Variant
<222> (322)..(326)
<223> xaa in position 322 to 326 is any amino acid
<220>
<221> Variant
<222> (328)..(329)
<223> xaa in position 328 to 329 is any amino acid
<220>
<221> Variant
<222> (331)..(333)
<223> xaa in position 331 to 333 is any amino acid
<220>
<221> Variant
<222> (336)..(337)
<223> xaa in position 336 to 337 is any amino acid
<220>
<221> Variant
<222> (341)..(341)
<223> xaa in position 341 is any amino acid
<220>
<221> Variant
<222> (344)..(344)
<223> xaa in position 344 is any amino acid
<220>
<221> Variant
<222> (346)..(354)
<223> xaa in position 346 to 354 is any amino acid
<220>
<221> Variant
<222> (356)..(363)
<223> xaa in position 356 to 363 is any amino acid
<220>
<221> Variant

<222> (365)..(366)
<223> xaa in position 365 to 366 is any amino acid
<220>
<221> Variant
<222> (368)..(370)
<223> xaa in position 368 to 370 is any amino acid
<220>
<221> Variant
<222> (372)..(378)
<223> xaa in position 372 to 378 is any amino acid

<400> 306
Leu Gln xaa xaa xaa xaa xaa xaa xaa xaa xaa xaa xaa xaa xaa xaa
1               5                   10                  15
xaa xaa xaa xaa xaa xaa xaa xaa xaa xaa xaa xaa xaa xaa xaa xaa
            20                  25                  30
xaa xaa xaa Trp xaa xaa xaa xaa xaa Tyr xaa xaa xaa xaa xaa xaa
        35                  40                  45
Asp xaa xaa xaa xaa xaa xaa xaa xaa xaa xaa xaa xaa xaa xaa xaa
    50                  55                  60
xaa Arg xaa xaa xaa xaa Trp xaa xaa xaa xaa xaa Tyr Phe Pro xaa
65                  70                  75                  80
xaa Leu xaa Lys Thr xaa xaa Leu xaa xaa xaa xaa Asn xaa xaa xaa
                85                  90                  95
xaa xaa xaa xaa xaa xaa xaa xaa xaa xaa xaa xaa xaa xaa xaa xaa
            100                 105                 110
xaa xaa xaa xaa xaa xaa xaa xaa xaa xaa xaa xaa xaa xaa xaa xaa
        115                 120                 125
xaa xaa xaa xaa xaa xaa xaa xaa xaa xaa xaa xaa Tyr xaa xaa Gly
    130                 135                 140
xaa His Pro His Gly xaa xaa xaa xaa Gly xaa xaa xaa xaa xaa xaa
145                 150                 155                 160
Thr xaa xaa xaa xaa xaa xaa xaa xaa Phe Pro Gly xaa xaa xaa xaa
                165                 170                 175
Leu xaa xaa Leu xaa xaa xaa Phe xaa xaa Pro xaa xaa Arg xaa xaa
            180                 185                 190
xaa xaa xaa xaa Gly xaa xaa xaa xaa xaa xaa xaa xaa xaa xaa xaa
        195                 200                 205
xaa Leu xaa xaa xaa xaa xaa xaa xaa xaa xaa xaa xaa xaa xaa xaa
    210                 215                 220
xaa xaa xaa xaa xaa xaa xaa Ile xaa xaa Gly Gly Ala xaa Glu xaa
225                 230                 235                 240
Leu xaa xaa xaa Pro Gly xaa xaa xaa Leu xaa Leu xaa xaa Arg Lys
                245                 250                 255
Gly Phe xaa xaa xaa Ala xaa xaa xaa Gly xaa xaa xaa Leu Val Pro
            260                 265                 270
xaa xaa xaa Phe Gly Glu Asn xaa xaa xaa xaa Gln xaa xaa xaa xaa
        275                 280                 285
xaa xaa Ser xaa xaa xaa xaa xaa xaa xaa xaa xaa Gln xaa xaa xaa
    290                 295                 300
xaa xaa xaa xaa Gly xaa xaa xaa xaa xaa xaa xaa xaa Arg Gly xaa
305                 310                 315                 320
Phe xaa xaa xaa xaa xaa Gly xaa xaa Pro xaa xaa xaa Pro Ile xaa
                325                 330                 335
xaa Val Val Gly xaa Pro Ile xaa Val xaa xaa xaa xaa xaa xaa xaa
            340                 345                 350
xaa xaa Pro xaa xaa xaa xaa xaa xaa xaa xaa His xaa xaa Tyr xaa
        355                 360                 365
xaa xaa Leu xaa xaa xaa xaa xaa xaa xaa Lys
    370                 375


<210> 307
<211> 47
<212> PRT
<213> Artificial sequence

<220>
<221> Variant

<222> (2)..(3)
<223> Xaa in position 2 to 3 is any amino acid
<220>
<221> Variant
<222> (4)..(4)
<223> Xaa in position 4 is Trp, Tyr or Phe
<220>
<221> Variant
<222> (5)..(21)
<223> Xaa in position 5 to 21 is any amino acid
<220>
<221> Variant
<222> (22)..(24)
<223> Xaa in position 22 to 24 is any or no amino acid
<220>
<221> Variant
<222> (25)..(25)
<223> Xaa in position 25 is Arg or Gln
<220>
<221> Variant
<222> (26)..(29)
<223> Xaa in position 26 to 29 is any amino acid
<220>
<221> Variant
<222> (30)..(30)
<223> Xaa in position 30 is Trp, His, Phe or Asn
<220>
<221> Variant
<222> (31)..(32)
<223> Xaa in position 31 to 32 is any amino acid
<220>
<221> Variant
<222> (33)..(33)
<223> Xaa in position 33 is Phe, Tyr, Met or Leu
<220>
<221> Variant
<222> (34)..(35)
<223> Xaa in position 34 to 35 is any amino acid
<220>
<221> Variant
<222> (37)..(37)
<223> Xaa in position 37 is Phe or Tyr
<220>
<221> Variant
<222> (39)..(39)
<223> Xaa in position 39 is Ile, Val, Ala or Leu
<220>
<221> Variant
<222> (40)..(40)
<223> Xaa in position 40 is any amino acid
<220>
<221> Variant
<222> (41)..(41)
<223> Xaa in position 41 is Leu or Val
<220>
<221> Variant
<222> (42)..(42)
<223> Xaa in position 42 is Val, His or Ile
<220>
<221> Variant
<222> (44)..(44)
<223> Xaa in position 44 is Thr, Glu or Ser
<220>
<221> Variant
<222> (45)..(45)
<223> Xaa in position 45 is any amino acid
<220>
<221> Variant
<222> (46)..(46)

<223> xaa in position 46 is Asp, Glu or Asn
<220>
<221> Variant
<222> (47)..(47)
<223> xaa in position 47 is Leu or Met

<400> 307
Tyr xaa xaa xaa xaa xaa xaa xaa xaa xaa xaa xaa xaa xaa xaa xaa
1               5                   10                  15
xaa xaa xaa xaa xaa xaa xaa xaa xaa xaa xaa xaa xaa xaa xaa xaa
            20                  25                  30
xaa xaa xaa Tyr xaa Pro xaa xaa xaa xaa Lys xaa xaa xaa xaa
        35                  40                  45


<210> 308
<211> 70
<212> PRT
<213> Artificial sequence

<220>
<221> Variant
<222> (2)..(2)
<223> xaa in position 2 is Ile, Leu or Val
<220>
<221> Variant
<222> (3)..(3)
<223> xaa in position 3 is any amino acid
<220>
<221> Variant
<222> (4)..(4)
<223> xaa in position 4 is Gly, Ala or Ser
<220>
<221> Variant
<222> (5)..(5)
<223> xaa in position 5 is any amino acid
<220>
<221> Variant
<222> (6)..(6)
<223> xaa in position 6 is His or Phe
<220>
<221> Variant
<222> (10)..(10)
<223> xaa in position 10 is Ile, Val or Met
<220>
<221> Variant
<222> (11)..(13)
<223> xaa in position 11 to 13 is any amino acid
<220>
<221> Variant
<222> (14)..(14)
<223> xaa in position 14 is Gly or Ala
<220>
<221> Variant
<222> (15)..(18)
<223> xaa in position 15 to 18 is any amino acid
<220>
<221> Variant
<222> (19)..(19)
<223> xaa in position 19 is Phe, Met, Leu or Ile
<220>
<221> Variant
<222> (20)..(20)
<223> xaa in position 20 is Ala, Ser, Cys or Gly
<220>
<221> Variant
<222> (21)..(21)
<223> xaa in position 21 is any amino acid
<220>

<221> Variant
<222> (22)..(22)
<223> Xaa in position 22 is Glu, Asn, Asp or Lys
<220>
<221> Variant
<222> (23)..(29)
<223> Xaa in position 23 to 29 is any amino acid
<220>
<221> Variant
<222> (30)..(30)
<223> Xaa in position 30 is Phe, Tyr or Leu
<220>
<221> Variant
<222> (31)..(31)
<223> Xaa in position 31 is Pro or Ala
<220>
<221> Variant
<222> (32)..(32)
<223> Xaa in position 32 is any amino acid
<220>
<221> Variant
<222> (33)..(33)
<223> Xaa in position 33 is Ile, Phe, Val or Leu
<220>
<221> Variant
<222> (34)..(36)
<223> Xaa in position 34 to 36 is any amino acid
<220>
<221> Variant
<222> (37)..(37)
<223> Xaa in position 37 is Leu, Ile or Val
<220>
<221> Variant
<222> (38)..(38)
<223> Xaa in position 38 is any amino acid
<220>
<221> Variant
<222> (39)..(39)
<223> Xaa in position 39 is Thr, Val or Met
<220>
<221> Variant
<222> (40)..(40)
<223> Xaa in position 40 is Leu or Ala
<220>
<221> Variant
<222> (41)..(43)
<223> Xaa in position 41 to 43 is any amino acid
<220>
<221> Variant
<222> (45)..(46)
<223> Xaa in position 45 to 46 is any amino acid
<220>
<221> Variant
<222> (48)..(49)
<223> Xaa in position 48 to 49 is any amino acid
<220>
<221> Variant
<222> (51)..(51)
<223> Xaa in position 51 is Glu or Asp
<220>
<221> Variant
<222> (52)..(53)
<223> Xaa in position 52 to 53 is any amino acid
<220>
<221> Variant
<222> (54)..(54)
<223> Xaa in position 54 is Met, Leu, Arg or Ile
<220>
<221> Variant

<222> (55)..(56)
<223> xaa in position 55 to 56 is any amino acid
<220>
<221> Variant
<222> (58)..(62)
<223> xaa in position 58 to 62 is any amino acid
<220>
<221> Variant
<222> (63)..(63)
<223> xaa in position 63 is Arg or Lys
<220>
<221> Variant
<222> (64)..(64)
<223> xaa in position 64 is any amino acid
<220>
<221> Variant
<222> (65)..(65)
<223> xaa in position 65 is Ser, Thr, Ala or Asn
<220>
<221> Variant
<222> (66)..(69)
<223> xaa in position 66 to 69 is any amino acid

<400> 308
Tyr xaa xaa xaa xaa xaa Pro His Gly xaa xaa xaa xaa xaa xaa xaa
1               5                   10                  15
xaa xaa xaa xaa xaa xaa xaa xaa xaa xaa xaa xaa xaa xaa xaa xaa
            20                  25                  30
xaa xaa xaa xaa xaa xaa xaa xaa xaa xaa xaa Phe xaa xaa Pro xaa
        35                  40                  45
xaa Arg xaa xaa xaa xaa xaa xaa Gly xaa xaa xaa xaa xaa xaa xaa
    50                  55                  60
xaa xaa xaa xaa xaa Leu
65                  70


<210> 309
<211> 60
<212> PRT
<213> Artificial sequence

<220>
<221> Variant
<222> (1)..(1)
<223> xaa in position 1 is Ile or Leu
<220>
<221> Variant
<222> (2)..(2)
<223> xaa in position 2 is Val, Ile, Leu, Met or Ala
<220>
<221> Variant
<222> (3)..(3)
<223> xaa in position 3 is Val, Ile or Leu
<220>
<221> Variant
<222> (7)..(7)
<223> xaa in position 7 is any amino acid
<220>
<221> Variant
<222> (9)..(9)
<223> xaa in position 9 is Ala or Ser
<220>
<221> Variant
<222> (10)..(10)
<223> xaa in position 10 is Leu or Met
<220>
<221> Variant
<222> (11)..(11)
<223> xaa in position 11 is any amino acid

<220>
<221> Variant
<222> (12)..(12)
<223> Xaa in position 12 is Ala, Ser or Cys
<220>
<221> Variant
<222> (13)..(13)
<223> Xaa in position 13 is any amino acid
<220>
<221> Variant
<222> (14)..(14)
<223> Xaa in position 14 is Pro, Thr or Val
<220>
<221> Variant
<222> (15)..(15)
<223> Xaa in position 15 is Gly or Asn
<220>
<221> Variant
<222> (16)..(18)
<223> Xaa in position 16 to 18 is any amino acid
<220>
<221> Variant
<222> (19)..(19)
<223> Xaa in position 19 is Leu or Val
<220>
<221> Variant
<222> (20)..(20)
<223> Xaa in position 20 is any amino acid
<220>
<221> Variant
<222> (21)..(21)
<223> Xaa in position 21 is Leu or Ile
<220>
<221> Variant
<222> (22)..(23)
<223> Xaa in position 22 to 23 is any amino acid
<220>
<221> Variant
<222> (25)..(25)
<223> Xaa in position 25 is Lys or Arg
<220>
<221> Variant
<222> (28)..(28)
<223> Xaa in position 28 is Val, Ile or Cys
<220>
<221> Variant
<222> (29)..(29)
<223> Xaa in position 29 is Lys or Arg
<220>
<221> Variant
<222> (30)..(30)
<223> Xaa in position 30 is any amino acid
<220>
<221> Variant
<222> (32)..(32)
<223> Xaa in position 32 is Leu, Ile, Val or Met
<220>
<221> Variant
<222> (33)..(34)
<223> Xaa in position 33 to 34 is any amino acid
<220>
<221> Variant
<222> (36)..(36)
<223> Xaa in position 36 is Asn or no amino acid
<220>
<221> Variant
<222> (37)..(38)
<223> Xaa in position 37 to 38 is any amino acid
<220>

<221> Variant
<222> (39)..(39)
<223> Xaa in position 39 is Leu or Ile
<220>
<221> Variant
<222> (42)..(43)
<223> Xaa in position 42 to 43 is any amino acid
<220>
<221> Variant
<222> (44)..(44)
<223> Xaa in position 44 is Ser, Ala, Thr or Asn
<220>
<221> Variant
<222> (48)..(48)
<223> Xaa in position 48 is Asn, Val or Thr
<220>
<221> Variant
<222> (49)..(49)
<223> Xaa in position 49 is Asp, Glu or Asn
<220>
<221> Variant
<222> (50)..(50)
<223> Xaa in position 50 is any amino acid
<220>
<221> Variant
<222> (51)..(51)
<223> Xaa in position 51 is Tyr, Phe or Leu
<220>
<221> Variant
<222> (52)..(52)
<223> Xaa in position 52 is any amino acid
<220>
<221> Variant
<222> (53)..(53)
<223> Xaa in position 53 is Gln, Val, Ile or Leu
<220>
<221> Variant
<222> (54)..(59)
<223> Xaa in position 54 to 59 is any amino acid
<220>
<221> Variant
<222> (60)..(60)
<223> Xaa in position 60 is Ser or Thr

<400> 309
Xaa Xaa Xaa Gly Gly Ala Xaa Glu Xaa Xaa Xaa Xaa Xaa Xaa Xaa Xaa
1               5                   10                  15
Xaa Xaa Xaa Xaa Xaa Xaa Xaa Arg Xaa Gly Phe Xaa Xaa Xaa Ala Xaa
            20                  25                  30
Xaa Xaa Gly Xaa Xaa Xaa Xaa Val Pro Xaa Xaa Xaa Phe Gly Glu Xaa
        35                  40                  45
Xaa Xaa Xaa Xaa Xaa Xaa Xaa Xaa Xaa Xaa Xaa Xaa
    50                  55                  60


<210> 310
<211> 79
<212> PRT
<213> Artificial sequence

<220>
<221> Variant
<222> (2)..(4)
<223> Xaa in position 2 to 4 is any amino acid
<220>
<221> Variant
<222> (5)..(5)
<223> Xaa in position 5 is Lys, Gln or Tyr
<220>

<221> Variant
<222> (6)..(8)
<223> xaa in position 6 to 8 is any amino acid
<220>
<221> Variant
<222> (10)..(10)
<223> xaa in position 10 is Phe, Ile, Val or Leu
<220>
<221> Variant
<222> (11)..(12)
<223> xaa in position 11 to 12 is any amino acid
<220>
<221> Variant
<222> (13)..(13)
<223> xaa in position 13 is Pro, Cys, Leu or Ala
<220>
<221> Variant
<222> (14)..(16)
<223> xaa in position 14 to 16 is any amino acid
<220>
<221> Variant
<222> (17)..(17)
<223> xaa in position 17 is Gly or Ala
<220>
<221> Variant
<222> (18)..(18)
<223> xaa in position 18 is Arg or Cys
<220>
<221> Variant
<222> (19)..(19)
<223> xaa in position 19 is Gly or Ser
<220>
<221> Variant
<222> (20)..(20)
<223> xaa in position 20 is any amino acid
<220>
<221> Variant
<222> (21)..(21)
<223> xaa in position 21 is Phe or Thr
<220>
<221> Variant
<222> (22)..(25)
<223> xaa in position 22 to 25 is any amino acid
<220>
<221> Variant
<222> (26)..(26)
<223> xaa in position 26 is any or no amino acid
<220>
<221> Variant
<222> (28)..(28)
<223> xaa in position 28 is Leu, Phe, Ile or Tyr
<220>
<221> Variant
<222> (29)..(29)
<223> xaa in position 29 is Leu, Val, Met or Ile
<220>
<221> Variant
<222> (31)..(31)
<223> xaa in position 31 is any amino acid
<220>
<221> Variant
<222> (32)..(32)
<223> xaa in position 32 is Arg, Ser, Ala or Asn
<220>
<221> Variant
<222> (33)..(33)
<223> xaa in position 33 is any amino acid
<220>
<221> Variant

<222> (34)..(34)
<223> xaa in position 34 is Pro, Arg, Leu or Ala
<220>
<221> Variant
<222> (35)..(35)
<223> xaa in position 35 is Ile, Leu or Val
<220>
<221> Variant
<222> (36)..(36)
<223> xaa in position 36 is any amino acid
<220>
<221> Variant
<222> (37)..(37)
<223> xaa in position 37 is Thr, Val, Gln or Ile
<220>
<221> Variant
<222> (38)..(38)
<223> xaa in position 38 is Val or Ile
<220>
<221> Variant
<222> (39)..(39)
<223> xaa in position 39 is Val, Met or Leu
<220>
<221> Variant
<222> (41)..(41)
<223> xaa in position 41 is any amino acid
<220>
<221> Variant
<222> (43)..(43)
<223> xaa in position 43 is Ile, Leu or Val
<220>
<221> Variant
<222> (44)..(44)
<223> xaa in position 44 is any amino acid
<220>
<221> Variant
<222> (45)..(45)
<223> xaa in position 45 is Val or Ile
<220>
<221> Variant
<222> (46)..(50)
<223> xaa in position 46 to 50 is any amino acid
<220>
<221> Variant
<222> (51)..(54)
<223> xaa in position 51 to 54 is any or no amino acid
<220>
<221> Variant
<222> (55)..(55)
<223> xaa in position 55 is Pro or Thr
<220>
<221> Variant
<222> (56)..(57)
<223> xaa in position 56 to 57 is any amino acid
<220>
<221> Variant
<222> (58)..(59)
<223> xaa in position 58 to 59 is any or no amino acid
<220>
<221> Variant
<222> (60)..(60)
<223> xaa in position 60 is Ile, Val, Met or Leu
<220>
<221> Variant
<222> (61)..(63)
<223> xaa in position 61 to 63 is any amino acid
<220>
<221> Variant
<222> (65)..(66)

<223> xaa in position 65 to 66 is any amino acid
<220>
<221> Variant
<222> (67)..(67)
<223> xaa in position 67 is Tyr, Val or Phe
<220>
<221> Variant
<222> (68)..(70)
<223> xaa in position 68 to 70 is any amino acid
<220>
<221> Variant
<222> (71)..(71)
<223> xaa in position 71 is Leu or Val
<220>
<221> Variant
<222> (72)..(73)
<223> xaa in position 72 to 73 is any amino acid
<220>
<221> Variant
<222> (74)..(74)
<223> xaa in position 74 is Leu, Ile, Val or Met
<220>
<221> Variant
<222> (75)..(75)
<223> xaa in position 75 is Phe, Tyr or Trp
<220>
<221> Variant
<222> (76)..(77)
<223> xaa in position 76 to 77 is any amino acid
<220>
<221> Variant
<222> (78)..(78)
<223> xaa in position 78 is His, Tyr or Asn
<220>
<221> Variant
<222> (79)..(79)
<223> xaa in position 79 is Lys or Arg

<400> 310
   Gln Xaa Xaa Xaa Xaa Xaa Xaa Xaa Gly Xaa Xaa Xaa Xaa Xaa Xaa Xaa
   1               5                   10                  15
   Xaa Xaa Xaa Xaa Xaa Xaa Xaa Xaa Xaa Xaa Gly Xaa Xaa Pro Xaa Xaa
               20                  25                  30
   Xaa Xaa Xaa Xaa Xaa Xaa Xaa Gly Xaa Pro Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa His
       50                  55                  60
   Xaa Xaa Xaa Xaa Xaa Xaa Xaa Xaa Xaa Xaa Xaa Xaa Xaa Xaa Xaa
   65                  70                  75

<210> 311
<211> 11449
<212> DNA
<213> Unknown, artificial

<400> 311
gtttacccgc caatatatcc tgtcaaacac tgatagtttg tggaattcga gctcggtacc      60
cggggatcct ctagagtcga cctgcaggca tgcaagcttt gcagtgcagc gtgacccggt     120
cgtgcccctc tctagagata atgagcattg catgtctaag ttataaaaaa ttaccacata     180
ttttttttgt cacacttgtt tgaagtgcag tttatctatc tttatacata tatttaaact     240
ttactctacg aataatataa tctatagtac tacaataata tcagtgtttt agagaatcat     300
ataaatgaac agttagacat ggtctaaagg acaattgagt attttgacaa caggactcta     360
cagttttatc tttttagtgt gcatgtgttc tccttttttt ttgcaaatag cttcacctat     420
ataatacttc atccatttta ttagtacatc catttagggt ttagggttaa tggtttttat     480
agactaattt ttttagtaca tctattttat tctattttag cctctaaatt aagaaaacta     540
aaactctatt ttagtttttt tatttaatag tttagatata aaatagaata aaataaagtg     600
actaaaaatt aaacaaatac cctttaagaa attaaaaaaa ctaaggaaac atttttcttg     660

tttcgagtag ataatgccag cctgttaaac gccgtcgacg agtctaacgg acaccaacca 720
gcgaaccagc agcgtcgcgt cgggccaagc gaagcagacg gcacggcatc tctgtcgctg 780
cctctggacc cctctcgaga gttccgctcc accgttggac ttgctccgct gtcggcatcc 840
agaaattgcg tggcggagcg gcagacgtga gccggcacgg caggcggcct cctcctcctc 900
tcacggcacc ggcagctacg ggggattcct ttcccaccgc tccttcgctt tcccttcctc 960
gcccgccgta ataaatagac accccctcca caccctcttt ccccaacctc gtgttgttcg 1020
gagcgcacac acacacaacc agatctcccc caaatccacc cgtcggcacc tccgcttcaa 1080
ggtacgccgc tcgtcctccc cccccccccc cctctctacc ttctctagat cggcgttccg 1140
gtccatggtt agggcccggt agttctactt ctgttcatgt ttgtgttaga tccgtgtttg 1200
tgttagatcc gtgctgctag cgttcgtaca cggatgcgac ctgtacgtca gacacgttct 1260
gattgctaac ttgccagtgt ttctctttgg ggaatcctgg gatggctcta gccgttccgc 1320
agacgggatc gatttcatga ttttttttgt ttcgttgcat agggtttggt ttgccctttt 1380
cctttatttc aatatatgcc gtgcacttgt ttgtcgggtc atcttttcat gctttttttt 1440
gtcttggttg tgatgatgtg gtctggttgg gcggtcgttc tagatcggag tagaattctg 1500
tttcaaacta cctggtggat ttattaattt tggatctgta tgtgtgtgcc atacatattc 1560
atagttacga attgaagatg atggatggaa atatcgatct aggataggta tacatgttga 1620
tgcgggtttt actgatgcat atacagagat gctttttgtt cgcttggttg tgatgatgtg 1680
gtgtggttgg gcggtcgttc attcgttcta gatcggagta gaatactgtt tcaaactacc 1740
tggtgtattt attaattttg gaactgtatg tgtgtgtcat acatcttcat agttacgagt 1800
ttaagatgga tggaaatatc gatctaggat aggtatacat gttgatgtgg gttttactga 1860
tgcatataca tgatggcata tgcagcatct attcatatgc tctaaccttg agtacctatc 1920
tattataata aacaagtatg ttttataatt atttcgatct tgatatactt ggatgatggc 1980
atatgcagca gctatatgtg gattttttta gccctgcctt catacgctat ttatttgctt 2040
ggtactgttt cttttgtcga tgctcaccct gttgtttggt gttacttctg cagggtaccc 2100
ccggggatcc actagttcta gaaaccatgg ccaccgccgc cgccgcgtct accgcgctca 2160
ctggcgccac taccgctgcg cccaaggcga ggcgccgggc gcacctcctg gccacccgcc 2220
gcgccctcgc cgcgcccatc aggtgctcag cggcgtcacc cgccatgccg atggctcccc 2280
cggccacccc gctccggccg tggggcccca ccgatccccg caagggcgcc gacatcctcg 2340
tcgagtccct cgagcgctgc ggcgtccgcg acgtcttcgc ctaccccggc ggcgcgtcca 2400
tggagatcca ccaggcactc acccgctccc ccgtcatcgc caaccacctc ttccgccacg 2460
agcaagggga ggcctttgcg gcctccggct acgcgcgctc ctcgggccgc gtcggcgtct 2520
gcatcgccac ctccggcccc ggcgccacca accttgtctc cgcgctcgcc gacgcgctgc 2580
tcgattccgt ccccatggtc gccatcacgg gacaggtgcc gcgacgcatg attggcaccg 2640
acgccttcca ggagacgccc atcgtcgagg tcacccgctc catcaccaag cacaactacc 2700
tggtcctcga cgtcgacgac atcccccgcg tcgtgcagga ggctttcttc ctcgcctcct 2760
ctggtcgacc ggggccggtg cttgtcgaca tccccaagga catccagcag cagatggcgg 2820
tgcctgtctg ggacaagccc atgagtctgc ctgggtacat tgcgcgcctt cccaagcccc 2880
ctgcgactga gttgcttgag caggtgctgc gtcttgttgg tgaatcccgg cgccctgttc 2940
tttatgttgg cggtggctgc gcagcatctg gtgaggagtt gcgacgcttt gtggagctga 3000
ctggaatccc ggtcacaact actcttatgg gcctcggcaa cttccccagc gacgacccac 3060
tgtctctgcg catgctaggt atgcatggca cggtgtatgc aaattatgca gtggataagg 3120
ccgatctgtt gcttgcactt ggtgtgcggt ttgatgatcg tgtgacaggg aagattgagg 3180
cttttgcaag cagggctaag attgtgcacg ttgatattga tccggctgag attggcaaga 3240
acaagcagcc acatgtgtcc atctgtgcag atgttaagct tgctttgcag ggcatgaatg 3300
ctcttcttga aggaagcaca tcaaagaaga gctttgactt tggctcatgg aacgatgagt 3360
tggatcagca gaagagggaa ttcccccttg ggtataaaac atctaatgag gagatccagc 3420
cacaatatgc tattcaggtt cttgatgagc tgacgaaagg cgaggccatc atcggcacag 3480
gtgttgggca gcaccagatg tgggcggcac agtactacac ttacaagcgg ccaaggcagt 3540
ggttgtcttc agctggtctt ggggctatgg gatttggttt gccggctgct gctggtgctt 3600
ctgtggccaa cccaggtgtt actgttgttg acatcgatgg agatggtagc tttctcatga 3660
acgttcagga gctagctatg atccgaattg agaacctccc ggtgaaggtc tttgtgctaa 3720
acaaccagca cctggggatg gtggtgcagt gggaggacag gttctataag gccaacagag 3780
cgcacacata cttgggaaac ccagagaatg aaagtgagat atatccagat ttcgtgacga 3840
tcgccaaagg gttcaacatt ccagcggtcc gtgtgacaaa gaagaacgaa gtccgcgcag 3900
cgataaagaa gatgctcgag actccagggc cgtacctctt ggatataatc gtcccacacc 3960
aggagcatgt gttgcctatg atccctaatg gtggggcttt caaggatatg atcctggatg 4020
gtgatggcag gactgtgtac tgatctaaaa tccagcaagc aactgatcta aaatccagca 4080
agcaccgcct ccctgctagt acaagggtga tatgttttta tctgtgtgat gttctcctgt 4140
attctatctt tttttgtagg ccgtcagcta tctgttatgg taatcctatg tagcttccga 4200
ccttgtaatt gtgtagtctg ttgttttcct tctggcatgt gtcataagag atcatttaag 4260
tgccttttgc tacatataaa taagataata agcactgcta tgcagtggtt ctgaattggc 4320
ttctgttgcc aaatttaagt gtccaactgg tccttgcttt tgttttcgct atttttttcc 4380
ttttttagtt attattatat tggtaatttc aactcaacat atgatgtatg gaataatgct 4440
agggctgcaa tttcaaacta ttttacaaac cagaatggca ttttcgtggt ttgaggggag 4500
tgaaaaaaaa tgaggcattt gactgaatta gttacctgat ccattttcgt ggtttggatc 4560
attggaatta aattccattc taataatagt aattttggca tatatcaatt aagttaattc 4620
ggttttatgc aaaatatatt tgtatactat tattatcaag atgtcggaga tatttatatg 4680
ctacattttt actatacagg agtgagatga agagtgtcat gtaagttaca cagtagaaac 4740
aaattctatt aatgcataaa atcatttcca tcatccaccc tatgaatttg agatagacct 4800

atatctaaac tttgaaaagt ggttgaatat caaattccaa attaaataag ttattttatt 4860
gagtgaattc taatttctct aaaacgaagg gatctaaacg ccctctaaag ctaatttgga 4920
aactcaaact ttcttagcat tggaggggat tgagaaaaaa tattaattca ttttcatctc 4980
aatcattcaa tctccaaaga gatttgagtt ccttattagt ctgttccatg catcaaatcg 5040
gctcaatgtg tcattatttg ccatgacgat tgacgagttg ttctggggcc tagcgctttc 5100
cacgccgatg tgctggggcc tggtcctgga gaagacagct tgatatttaa agctatcaat 5160
tgtttcaatt gattcccact tcatttttct aaatgtagaa aacggtgacg tataagaaaa 5220
agaatgaatt aggactttta ttccgtacac taatctagag cggcccctta aggcgctgcg 5280
atcgcgttaa cagcttgctg aggaggcctc ggaccgttaa ttaacacgtg ggcgcgccac 5340
tagtcaattc agtacattaa aaacgtccgc aatgtgttat taagttgtct aagcgtcaat 5400
ttgtttacac cacaatatat cctgccacca gccagccaac agctccccga ccggcagctc 5460
ggcacaaaat caccactcga tacaggcagc ccatcagtcc gggacggcgt cagcgggaga 5520
gccgttgtaa ggcggcagac tttgctcatg ttaccgatgc tattcggaag aacggcaact 5580
aagctgccgg gtttgaaaca cggatgatct cgcggagggt agcatgttga ttgtaacgat 5640
gacagagcgt tgctgcctgt gatcaaatat catctccctc gcagagatcc gaattatcag 5700
ccttcttatt catttctcgc ttaaccgtga caggctgtcg atcttgagaa ctatgccgac 5760
ataataggaa atcgctggat aaagccgctg aggaagctga gtggcgctat ttctttagaa 5820
gtgaacgttg acgatcgtcg accgtacccc gatgaattaa ttcggacgta cgttctgaac 5880
acagctggat acttacttgg gcgattgtca tacatgacat caacaatgta cccgtttgtg 5940
taaccgtctc ttggaggttc gtatgacact agtggttccc ctcagcttgc gactagatgt 6000
tgaggcctaa cattttatta gagagcaggc tagttgctta gatacatgat cttcaggccg 6060
ttatctgtca gggcaagcga aaattggcca tttatgacga ccaatgcccc gcagaagctc 6120
ccatctttgc cgccatagac gccgcgcccc ccttttgggg tgtagaacat ccttttgcca 6180
gatgtggaaa agaagttcgt tgtcccattg ttggcaatga cgtagtagcc ggcgaaagtg 6240
cgagacccat ttgcgctata tataagccta cgatttccgt tgcgactatt gtcgtaattg 6300
gatgaactat tatcgtagtt gctctcagag ttgtcgtaat ttgatggact attgtcgtaa 6360
ttgcttatgg agttgtcgta gttgcttgga gaaatgtcgt agttggatgg ggagtagtca 6420
tagggaagac gagcttcatc cactaaaaca attggcaggt cagcaagtgc ctgccccgat 6480
gccatcgcaa gtacgaggct tagaaccacc ttcaacagat cgcgcatagt cttccccagc 6540
tctctaacgc ttgagttaag ccgcgccgcg aagcggcgtc ggcttgaacg aattgttaga 6600
cattatttgc cgactacctt ggtgatctcg cctttcacgt agtgaacaaa ttcttccaac 6660
tgatctgcgc gcgaggccaa gcgatcttct tgtccaagat aagcctgcct agcttcaagt 6720
atgacgggct gatactgggc cggcaggcgc tccattgccc agtcggcagc gacatccttc 6780
ggcgcgattt tgccggttac tgcgctgtac caaatgcggg acaacgtaag cactacattt 6840
cgctcatcgc cagcccagtc gggcggcgag ttccatagcg ttaaggtttc atttagcgcc 6900
tcaaatagat cctgttcagg aaccggatca aagagttcct ccgccgctgg acctaccaag 6960
gcaacgctat gttctcttgc ttttgtcagc aagatagcca gatcaatgtc gatcgtggct 7020
ggctcgaaga tacctgcaag aatgtcattg cgctgccatt ctccaaattg cagttcgcgc 7080
ttagctggat aacgccacgg aatgatgtcg tcgtgcacaa caatggtgac ttctacagcg 7140
cggagaatct cgctctctcc aggggaagcc gaagtttcca aaaggtcgtt gatcaaagct 7200
cgccgcgttg tttcatcaag ccttacggtc accgtaacca gcaaatcaat atcactgtgt 7260
ggcttcaggc cgccatccac tgcggagccg tacaaatgta cggccagcaa cgtcggttcg 7320
agatggcgct cgatgacgcc aactacctct gatagttgag tcgatacttc ggcgatcacc 7380
gcttccctca tgatgtttaa ctcctgaatt aagccgcgcc gcgaagcggt gtcggcttga 7440
atgaattgtt aggcgtcatc ctgtgctccc gagaaccagt accagtacat cgctgtttcg 7500
ttcgagactt gaggtctagt tttatacgtg aacaggtcaa tgccgccgag agtaaagcca 7560
cattttgcgt acaaattgca ggcaggtaca ttgttcgttt gtgtctctaa tcgtatgcca 7620
aggagctgtc tgcttagtgc ccactttttc gcaaattcga tgagactgtg cgcgactcct 7680
ttgcctcggt gcgtgtgcga cacaacaatg tgttcgatag aggctagatc gttccatgtt 7740
gagttgagtt caatcttccc gacaagctct tggtcgatga atgcgccata gcaagcagag 7800
tcttcatcag agtcatcatc cgagatgtaa tccttccggt aggggctcac acttctggta 7860
gatagttcaa agccttggtc ggataggtgc acatcgaaca cttcacgaac aatgaaatgg 7920
ttctcagcat ccaatgtttc cgccacctgc tcagggatca ccgaaatctt catatgacgc 7980
ctaacgcctg gcacagcgga tcgcaaacct ggcgcggctt ttggcacaaa aggcgtgaca 8040
ggtttgcgaa tccgttgctg ccacttgtta acccttttgc cagatttggt aactataatt 8100
tatgttagag gcgaagtctt gggtaaaaac tggcctaaaa ttgctgggga tttcaggaaa 8160
gtaaacatca ccttccggct cgatgtctat tgtagatata tgtagtgtat ctacttgatc 8220
gggggatctg ctgcctcgcg cgtttcggtg atgacggtga aaacctctga cacatgcagc 8280
tcccggagac ggtcacagct tgtctgtaag cggatgccgg gagcagacaa gcccgtcagg 8340
gcgcgtcagc gggtgttggc gggtgtcggg gcgcagccat gacccagtca cgtagcgata 8400
gcggagtgta tactggctta actatgcggc atcagagcag attgtactga gagtgcacca 8460
tatgcggtgt gaaataccgc acagatgcgt aaggagaaaa taccgcatca ggcgctcttc 8520
cgcttcctcg ctcactgact cgctgcgctc ggtcgttcgg ctgcggcgag cggtatcagc 8580
tcactcaaag gcggtaatac ggttatccac agaatcaggg gataacgcag gaaagaacat 8640
gtgagcaaaa ggccagcaaa aggccaggaa ccgtaaaaag gccgcgttgc tggcgttttt 8700
ccataggctc cgcccccctg acgagcatca caaaaatcga cgctcaagtc agaggtggcg 8760
aaacccgaca ggactataaa gataccaggc gtttccccct ggaagctccc tcgtgcgctc 8820
tcctgttccg accctgccgc ttaccggata cctgtccgcc tttctccctt cgggaagcgt 8880
ggcgctttct catagctcac gctgtaggta tctcagttcg gtgtaggtcg ttcgctccaa 8940

gctgggctgt gtgcacgaac cccccgttca gcccgaccgc tgcgccttat ccggtaacta 9000
tcgtcttgag tccaacccgg taagacacga cttatcgcca ctggcagcag ccactggtaa 9060
caggattagc agagcgaggt atgtaggcgg tgctacagag ttcttgaagt ggtggcctaa 9120
ctacggctac actagaagga cagtatttgg tatctgcgct ctgctgaagc cagttacctt 9180
cggaaaaaga gttggtagct cttgatccgg caaacaaacc accgctggta gcggtggttt 9240
ttttgtttgc aagcagcaga ttacgcgcag aaaaaaagga tctcaagaag atcctttgat 9300
cttttctacg gggtctgacg ctcagtggaa cgaaaactca cgttaaggga ttttggtcat 9360
gagattatca aaaaggatct tcacctagat ccttttaaat taaaaatgaa gttttaaatc 9420
aatctaaagt atatatgagt aaacttggtc tgacagttac caatgcttaa tcagtgaggc 9480
acctatctca gcgatctgtc tatttcgttc atccatagtt gcctgactcc ccgtcgtgta 9540
gataactacg atacgggagg gcttaccatc tggccccagt gctgcaatga taccgcgaga 9600
cccacgctca ccggctccag atttatcagc aataaaccag ccagccggaa gggccgagcg 9660
cagaagtggt cctgcaactt tatccgcctc catccagtct attaattgtt gccgggaagc 9720
tagagtaagt agttcgccag ttaatagttt gcgcaacgtt gttgccattg ctgcaggggg 9780
gggggggggg ggggacttcc attgttcatt ccacggacaa aaacagagaa aggaaacgac 9840
agaggccaaa aagcctcgct ttcagcacct gtcgtttcct ttcttttcag agggtatttt 9900
aaataaaaac attaagttat gacgaagaag aacggaaacg ccttaaaccg gaaaattttc 9960
ataaatagcg aaaacccgcg aggtcgccgc cccgtaacct gtcggatcac cggaaaggac 10020
ccgtaaagtg ataatgatta tcatctacat atcacaacgt gcgtggaggc catcaaacca 10080
cgtcaaataa tcaattatga cgcaggtatc gtattaattg atctgcatca acttaacgta 10140
aaaacaactt cagacaatac aaatcagcga cactgaatac ggggcaacct catgtccccc 10200
cccccccccc ccctgcaggc atcgtggtgt cacgctcgtc gtttggtatg gcttcattca 10260
gctccggttc ccaacgatca aggcgagtta catgatcccc catgttgtgc aaaaaagcgg 10320
ttagctcctt cggtcctccg atcgttgtca gaagtaagtt ggccgcagtg ttatcactca 10380
tggttatggc agcactgcat aattctctta ctgtcatgcc atccgtaaga tgcttttctg 10440
tgactggtga gtactcaacc aagtcattct gagaatagtg tatgcggcga ccgagttgct 10500
cttgcccggc gtcaacacgg gataataccg cgccacatag cagaacttta aaagtgctca 10560
tcattggaaa acgttcttcg gggcgaaaac tctcaaggat cttaccgctg ttgagatcca 10620
gttcgatgta acccactcgt gcacccaact gatcttcagc atcttttact ttcaccagcg 10680
tttctgggtg agcaaaaaca ggaaggcaaa atgccgcaaa aaagggaata agggcgacac 10740
ggaaatgttg aatactcata ctcttccttt ttcaatatta ttgaagcatt tatcagggtt 10800
attgtctcat gagcggatac atatttgaat gtatttagaa aaataaacaa ataggggttc 10860
cgcgcacatt tccccgaaaa gtgccacctg acgtctaaga aaccattatt atcatgacat 10920
taacctataa aaataggcgt atcacgaggc cctttcgtct tcaagaattg gtcgacgatc 10980
ttgctgcgtt cggatatttt cgtggagttc ccgccacaga cccggattga aggcgagatc 11040
cagcaactcg cgccagatca tcctgtgacg gaactttggc gcgtgatgac tggccaggac 11100
gtcggccgaa agagcgacaa gcagatcacg cttttcgaca gcgtcggatt tgcgatcgag 11160
gatttttcgg cgctgcgcta cgtccgcgac cgcgttgagg gatcaagcca cagcagccca 11220
ctcgaccttc tagccgaccc agacgagcca agggatcttt ttggaatgct gctccgtcgt 11280
caggctttcc gacgtttggg tggttgaaca gaagtcatta tcgtacggaa tgccaagcac 11340
tcccgagggg aaccctgtgg ttggcatgca catacaaatg gacgaacgga taaacctttt 11400
cacgcccttt taaatatccg ttattctaat aaacgctctt ttctcttag 11449

<210> 312
<211> 4510
<212> DNA
<213> Unknown, artificial

<400> 312
acacaggaaa cagctatgac catgattacg ccaagctatc gtttaaacct taaggcgatc 60
gcgctgaggc ggaccgcacg tggaattagc ttggcgcgcc aattcccgat ctagtaacat 120
agatgacacc gcgcgcgata atttatccta gtttgcgcgc tatattttgt tttctatcgc 180
gtattaaatg tataattgcg ggactctaat cataaaaacc catctcataa ataacgtcat 240
gcattacatg ttaattatta catgcttaac gtaattcaac agaaattata tgataatcat 300
cgcaagaccg gcaacaggat tcaatcttaa gaaactttat tgccaaatgt ttgaacgatc 360
ggggaaattc gagctccacc gcggtggcgg ccgctctaga actagtggat cccccgggct 420
gcaggaattc gatatcaagc ttatcgatac cgtcgacctc gagggggggc ccggtacctt 480
ggtgaactac cgatgatcgt aagagcttac agacggtgcc ttatataggc agagcgtcgg 540
aaggggggtg gtgtcacaca cgcactgcga tccttgctta caagctaagc aaaggcatcg 600
tggcagacaa ggaataaagt ggcacaggtg ccaaaagaaa gtggacagca cacttgtccg 660
aaaagcacac aataatacaa ctcagtggag catccactga atgggcccag tactctccgc 720
tgcggtcatc cctgacgtca atgcctcctc aatcgtcatt gcttacgtct tggagatcta 780
gcacatcttc tgcggctact ggcgacatag cccttggtgt ggcctggtat tcctcgaact 840
cctgctcttc tatcttcagc ttggtgactt ccttcataag ttcctcctcc attgtttgca 900
cgccaccggt cttcagaact tcaagattct tgagaaattc atcaattctg tcttgaatgt 960
gttcttcaat gagatctgcc cagtaccagc aatggcattt gttcattgcg cacttgaaga 1020
atttccttcc tgggttggca gatgttctgg acactaactg aattgcaggc ttcctgcatg 1080
cacagagtag aactggttcc tctgttagca taaaagcttg ctctgttctt tcgaacatgt 1140
ccttcttgaa tgtggggaat ggatccagaa ttttgtttcc ccattctctg agttgctcag 1200

tgtatggatg atctggataa ggaattactt cccttatggc ttgtgtaagc aggatcatct 1260
cttccgttgg ttcattctga gctaatttgg ctttgagcct ggacaagatg tcagctaaac 1320
cattgctctt cccttttatg tgttcaatga ctatctctgg tcctgcacca gtgatgtagt 1380
ccatgaacct gatccatctg atctcagaag gcttgtgttc agcactcttg ttgtagaacc 1440
tttcgattgc actactgtca gttctgactg tgatctctct tttgtccaag tagaacaatc 1500
tcatcttttc taagccattc ataaccccat agatttctgc atcacaggtt ccttttggct 1560
tatcaaattt tccactggca tacctacaga tttgctctgt atttcttggg tctgccttgt 1620
ttttcttcca cttgcatact gctccccatc cagttgcaca tgcatctgtt tcaatgataa 1680
tgtatgcatc ttctggtgga atagtgagat ttggaagcgt tctcaccatt gtcttgatcc 1740
tattgatcag cttccaatct tctgaattga gccttcgctc acctttctct gaggtctttg 1800
gatataatgg gccaagaagc ttgcccatat ctttgatgtg gtttctggca tagttcagtg 1860
ttgctagcca ggattaatta aaggcctgtt aacagcgctg ggcccgataa ttcactggcc 1920
gtcgttttac aacgtcgtga ctgggaaaac cctggcgtta cccaacttaa tcgccttgca 1980
gcacatcccc ctttcgccag ctggcgtaat agcgaagagg cccgcaccga tcgcccttcc 2040
caacagttgc gcagcctgaa tggcgaatgg cgcctgatgc ggtattttct ccttacgcat 2100
ctgtgcggta tttcacaccg catatggtgc actctcagta caatctgctc tgatgccgca 2160
tagttaagcc agccccgaca cccgccaaca cccgctgacg cgccctgacg ggcttgtctg 2220
ctcccggcat ccgcttacag acaagctgtg accgtctccg ggagctgcat gtgtcagagg 2280
ttttcaccgt catcaccgaa acgcgcgaga cgaaagggcc tcgtgatacg cctattttta 2340
taggttaatg tcatgataat aatggtttct tagacgtcag gtggcacttt tcggggaaat 2400
gtgcgcggaa cccctatttg tttatttttc taaatacatt caaatatgta tccgctcatg 2460
agacaataac cctgataaat gcttcaataa tattgaaaaa ggaagagtat gagtattcaa 2520
catttccgtg tcgcccttat tccctttttt gcggcatttt gccttcctgt ttttgctcac 2580
ccagaaacgc tggtgaaagt aaaagatgct gaagatcagt tgggtgcacg agtgggttac 2640
atcgaactgg atctcaacag cggtaagatc cttgagagtt ttcgccccga agaacgtttt 2700
ccaatgatga gcacttttaa agttctgcta tgtggcgcgg tattatcccg tattgacgcc 2760
gggcaagagc aactcggtcg ccgcatacac tattctcaga atgacttggt tgagtactca 2820
ccagtcacag aaaagcatct tacggatggc atgacagtaa gagaattatg cagtgctgcc 2880
ataaccatga gtgataacac tgcggccaac ttacttctga caacgatcgg aggaccgaag 2940
gagctaaccg cttttttgca caacatgggg gatcatgtaa ctcgccttga tcgttgggaa 3000
ccggagctga atgaagccat accaaacgac gagcgtgaca ccacgatgcc tgtagcaatg 3060
gcaacaacgt tgcgcaaact attaactggc gaactactta ctctagcttc ccggcaacaa 3120
ttaatagact ggatggaggc ggataaagtt gcaggaccac ttctgcgctc ggcccttccg 3180
gctggctggt ttattgctga taaatctgga gccggtgagc gtgggtctcg cggtatcatt 3240
gcagcactgg ggccagatgg taagccctcc cgtatcgtag ttatctacac gacggggagt 3300
caggcaacta tggatgaacg aaatagacag atcgctgaga taggtgcctc actgattaag 3360
cattggtaac tgtcagacca agtttactca tatatacttt agattgattt aaaacttcat 3420
ttttaattta aaaggatcta ggtgaagatc ctttttgata atctcatgac caaaatccct 3480
taacgtgagt tttcgttcca ctgagcgtca gaccccgtag aaaagatcaa aggatcttct 3540
tgagatcctt tttttctgcg cgtaatctgc tgcttgcaaa caaaaaaacc accgctacca 3600
gcggtggttt gtttgccgga tcaagagcta ccaactcttt ttccgaaggt aactggcttc 3660
agcagagcgc agataccaaa tactgttctt ctagtgtagc cgtagttagg ccaccacttc 3720
aagaactctg tagcaccgcc tacatacctc gctctgctaa tcctgttacc agtggctgct 3780
gccagtggcg ataagtcgtg tcttaccggg ttggactcaa gacgatagtt accggataag 3840
gcgcagcggt cgggctgaac ggggggttcg tgcacacagc ccagcttgga gcgaacgacc 3900
tacaccgaac tgagatacct acagcgtgag ctatgagaaa gcgccacgct tcccgaaggg 3960
agaaaggcgg acaggtatcc ggtaagcggc agggtcggaa caggagagcg cacgagggag 4020
cttccagggg gaaacgcctg gtatctttat agtcctgtcg ggtttcgcca cctctgactt 4080
gagcgtcgat ttttgtgatg ctcgtcaggg gggcggagcc tatggaaaaa cgccagcaac 4140
gcggcctttt tacggttcct ggccttttgc tggccttttg ctcacatgtt ctttcctgcg 4200
ttatcccctg attctgtgga taaccgtatt accgcctttg agtgagctga taccgctcgc 4260
cgcagccgaa cgaccgagcg cagcgagtca gtgagcgagg aagcggaaga gcgcccaata 4320
cgcaaaccgc ctctccccgc gcgttggccg attcattaat gcagctggca cgacaggttt 4380
cccgactgga aagcgggcag tgagcgcaac gcaattaatg tgagttagct cactcattag 4440
gcaccccagg ctttacactt tatgcttccg gctcgtatgt tgtgtggaat tgtgagcgga 4500
taacaatttc 4510

<210> 313
<211> 13170
<212> DNA
<213> Unknown, artificial

<400> 313
gtttacccgc caatatatcc tgtcaaacac tgatagtttg tggaattcga gctcggtacc 60
cggggatcct ctagagtcga cctgcaggca tgcaagcttc cggctgcagt gcagcgtgac 120
ccggtcgtgc ccctctctag agataatgag cattgcatgt ctaagttata aaaaattacc 180
acatattttt tttgtcacac ttgtttgaag tgcagtttat ctatctttat acatatattt 240

aaactttact ctacgaataa tataatctat agtactacaa taatatcagt gttttagaga 300
atcatataaa tgaacagtta gacatggtct aaaggacaat tgagtatttt gacaacagga 360
ctctacagtt ttatcttttt agtgtgcatg tgttctcctt tttttttgca aatagcttca 420
cctatataat acttcatcca ttttattagt acatccattt agggtttagg gttaatggtt 480
tttatagact aattttttta gtacatctat tttattctat tttagcctct aaattaagaa 540
aactaaaact ctattttagt ttttttattt aatagtttag atataaaata gaataaaata 600
aagtgactaa aaattaaaca aatacccttt aagaaattaa aaaaactaag gaaacatttt 660
tcttgtttcg agtagataat gccagcctgt taaacgccgt cgacgagtct aacggacacc 720
aaccagcgaa ccagcagcgt cgcgtcgggc caagcgaagc agacggcacg gcatctctgt 780
cgctgcctct ggacccctct cgagagttcc gctccaccgt tggacttgct ccgctgtcgg 840
catccagaaa ttgcgtggcg gagcggcaga cgtgagccgg cacggcaggc ggcctcctcc 900
tcctctcacg gcaccggcag ctacggggga ttcctttccc accgctcctt cgctttccct 960
tcctcgcccg ccgtaataaa tagacacccc ctccacaccc tctttcccca acctcgtgtt 1020
gttcggagcg cacacacaca caaccagatc tcccccaaat ccacccgtcg gcacctccgc 1080
ttcaaggtac gccgctcgtc ctcccccccc cccccctctc ctaccttctc tagatcggcg 1140
ttccggtcca tggttagggc ccggtagttc tacttctgtt catgtttgtg ttagatccgt 1200
gtttgtgtta gatccgtgct gctagcgttc gtacacggat gcgacctgta cgtcagacac 1260
gttctgattg ctaacttgcc agtgtttctc tttggggaat cctgggatgg ctctagccgt 1320
tccgcagacg ggatcgattt catgattttt tttgtttcgt tgcatagggt ttggtttgcc 1380
cttttccttt atttcaatat atgccgtgca cttgtttgtc gggtcatctt ttcatgcttt 1440
tttttgtctt ggttgtgatg atgtggtctg gttgggcggt cgttctagat cggagtagaa 1500
ttctgtttca aactacctgg tggatttatt aattttggat ctgtatgtgt gtgccataca 1560
tattcatagt tacgaattga agatgatgga tggaaatatc gatctaggat aggtatacat 1620
gttgatgcgg gttttactga tgcatataca gagatgcttt ttgttcgctt ggttgtgatg 1680
atgtggtgtg gttgggcggt cgttcattcg ttctagatcg gagtagaata ctgtttcaaa 1740
ctacctggtg tatttattaa ttttggaact gtatgtgtgt gtcatacatc ttcatagtta 1800
cgagtttaag atggatggaa atatcgatct aggataggta tacatgttga tgtgggtttt 1860
actgatgcat atacatgatg gcatatgcag catctattca tatgctctaa ccttgagtac 1920
ctatctatta taataaacaa gtatgtttta taattatttc gatcttgata tacttggatg 1980
atggcatatg cagcagctat atgtggattt ttttagccct gccttcatac gctatttatt 2040
tgcttggtac tgtttctttt gtcgatgctc accctgttgt ttggtgttac ttctgcaggg 2100
tacccccggg gatccactag ttctagaaac catggccacc gccgccgccg cgtctaccgc 2160
gctcactggc gccactaccg ctgcgcccaa ggcgaggcgc cgggcgcacc tcctggccac 2220
ccgccgcgcc ctcgccgcgc ccatcaggtg ctcagcggcg tcacccgcca tgccgatggc 2280
tcccccggcc accccgctcc ggccgtgggg ccccaccgat ccccgcaagg gcgccgacat 2340
cctcgtcgag tccctcgagc gctgcggcgt ccgcgacgtc ttcgcctacc ccggcggcgc 2400
gtccatggag atccaccagg cactcacccg ctcccccgtc atcgccaacc acctcttccg 2460
ccacgagcaa ggggaggcct ttgcggcctc cggctacgcg cgctcctcgg gccgcgtcgg 2520
cgtctgcatc gccacctccg gccccggcgc caccaacctt gtctccgcgc tcgccgacgc 2580
gctgctcgat tccgtcccca tggtcgccat cacgggacag gtgccgcgac gcatgattgg 2640
caccgacgcc ttccaggaga cgcccatcgt cgaggtcacc cgctccatca ccaagcacaa 2700
ctacctggtc ctcgacgtcg acgacatccc ccgcgtcgtg caggaggctt tcttcctcgc 2760
ctcctctggt cgaccggggc cggtgcttgt cgacatcccc aaggacatcc agcagcagat 2820
ggcggtgcct gtctgggaca agcccatgag tctgcctggg tacattgcgc gccttcccaa 2880
gccccctgcg actgagttgc ttgagcaggt gctgcgtctt gttggtgaat cccggcgccc 2940
tgttctttat gttggcggtg gctgcgcagc atctggtgag gagttgcgac gctttgtgga 3000
gctgactgga atcccggtca caactactct tatgggcctc ggcaacttcc ccagcgacga 3060
cccactgtct ctgcgcatgc taggtatgca tggcacggtg tatgcaaatt atgcagtgga 3120
taaggccgat ctgttgcttg cacttggtgt gcggtttgat gatcgtgtga cagggaagat 3180
tgaggctttt gcaagcaggg ctaagattgt gcacgttgat attgatccgg ctgagattgg 3240
caagaacaag cagccacatg tgtccatctg tgcagatgtt aagcttgctt tgcagggcat 3300
gaatgctctt cttgaaggaa gcacatcaaa gaagagcttt gactttggct catggaacga 3360
tgagttggat cagcagaaga gggaattccc ccttgggtat aaaacatcta atgaggagat 3420
ccagccacaa tatgctattc aggttcttga tgagctgacg aaaggcgagg ccatcatcgg 3480
cacaggtgtt gggcagcacc agatgtgggc ggcacagtac tacacttaca agcggccaag 3540
gcagtggttg tcttcagctg gtcttggggc tatgggattt ggtttgccgg ctgctgctgg 3600
tgcttctgtg gccaacccag gtgttactgt tgttgacatc gatggagatg gtagctttct 3660
catgaacgtt caggagctag ctatgatccg aattgagaac ctcccggtga aggtctttgt 3720
gctaaacaac cagcacctgg ggatggtggt gcagtgggag gacaggttct ataaggccaa 3780
cagagcgcac acatacttgg gaaacccaga gaatgaaagt gagatatatc cagatttcgt 3840
gacgatcgcc aaagggttca acattccagc ggtccgtgtg acaaagaaga acgaagtccg 3900
cgcagcgata aagaagatgc tcgagactcc agggccgtac ctcttggata taatcgtccc 3960
acaccaggag catgtgttgc ctatgatccc taatggtggg gctttcaagg atatgatcct 4020
ggatggtgat ggcaggactg tgtactgatc taaaatccag caagcaactg atctaaaatc 4080
cagcaagcac cgcctccctg ctagtacaag ggtgatatgt ttttatctgt gtgatgttct 4140
cctgtattct atcttttttt gtaggccgtc agctatctgt tatggtaatc ctatgtagct 4200
tccgaccttg taattgtgta gtctgttgtt ttccttctgg catgtgtcat aagagatcat 4260
ttaagtgcct tttgctacat ataaataaga taataagcac tgctatgcag tggttctgaa 4320
ttggcttctg ttgccaaatt taagtgtcca actggtcctt gcttttgttt tcgctatttt 4380

tttccttttt tagttattat tatattggta atttcaactc aacatatgat gtatggaata 4440
atgctagggc tgcaatttca aactatttta caaaccagaa tggcattttc gtggtttgag 4500
gggagtgaaa aaaaatgagg catttgactg aattagttac ctgatccatt ttcgtggttt 4560
ggatcattgg aattaaattc cattctaata atagtaattt tggcatatat caattaagtt 4620
aattcggttt tatgcaaaat atatttgtat actattatta tcaagatgtc ggagatattt 4680
atatgctaca tttttactat acaggagtga gatgaagagt gtcatgtaag ttacacagta 4740
gaaacaaatt ctattaatgc ataaaatcat ttccatcatc caccctatga atttgagata 4800
gacctatatc taaactttga aaagtggttg aatatcaaat tccaaattaa ataagttatt 4860
ttattgagtg aattctaatt tctctaaaac gaagggatct aaacgccctc taaagctaat 4920
ttggaaactc aaactttctt agcattggag gggattgaga aaaaatatta attcattttc 4980
atctcaatca ttcaatctcc aaagagattt gagttcctta ttagtctgtt ccatgcatca 5040
aatcggctca atgtgtcatt atttgccatg acgattgacg agttgttctg gggcctagcg 5100
ctttccacgc cgatgtgctg gggcctggtc ctggagaaga cagcttgata tttaaagcta 5160
tcaattgttt caattgattc ccacttcatt tttctaaatg tagaaaacgg tgacgtataa 5220
gaaaaagaat gaattaggac ttttattccg tacactaatc tagagcggcc ccttaaggcg 5280
ctgcgatcgc gttaacagct tgctgaggag gcctcggacc gttaattaat cctggctagc 5340
aacactgaac tatgccagaa accacatcaa agatatgggc aagcttcttg gcccattata 5400
tccaaagacc tcagagaaag gtgagcgaag gctcaattca gaagattgga agctgatcaa 5460
taggatcaag acaatggtga gaacgcttcc aaatctcact attccaccag aagatgcata 5520
cattatcatt gaaacagatg catgtgcaac tggatgggga gcagtatgca agtggaagaa 5580
aaacaaggca gacccaagaa atacagagca aatctgtagg tatgccagtg gaaaatttga 5640
taagccaaaa ggaacctgtg atgcagaaat ctatggggtt atgaatggct tagaaaagat 5700
gagattgttc tacttggaca aaagagagat cacagtcaga actgacagta gtgcaatcga 5760
aaggttctac aacaagagtg ctgaacacaa gccttctgag atcagatgga tcaggttcat 5820
ggactacatc actggtgcag gaccagagat agtcattgaa cacataaaag ggaagagcaa 5880
tggtttagct gacatcttgt ccaggctcaa agccaaatta gctcagaatg aaccaacgga 5940
agagatgatc ctgcttacac aagccataag ggaagtaatt ccttatccag atcatccata 6000
cactgagcaa ctcagagaat ggggaaacaa aattctggat ccattcccca cattcaagaa 6060
ggacatgttc gaaagaacag agcaagcttt tatgctaaca gaggaaccag ttctactctg 6120
tgcatgcagg aagcctgcaa ttcagttagt gtccagaaca tctgccaacc caggaaggaa 6180
attcttcaag tgcgcaatga acaaatgcca ttgctggtac tgggcagatc tcattgaaga 6240
acacattcaa gacagaattg atgaatttct caagaatctt gaagttctga agaccggtgg 6300
cgtgcaaaca atggaggagg aacttatgaa ggaagtcacc aagctgaaga tagaagagca 6360
ggagttcgag gaataccagg ccacaccaag ggctatgtcg ccagtagccg cagaagatgt 6420
gctagatctc caagacgtaa gcaatgacga ttgaggaggc attgacgtca gggatgaccg 6480
cagcggagag tactgggccc attcagtgga tgctccactg agttgtatta ttgtgtgctt 6540
ttcggacaag tgtgctgtcc actttctttt ggcacctgtg ccactttatt ccttgtctgc 6600
cacgatgcct ttgcttagct tgtaagcaag gatcgcagtg cgtgtgtgac caccaccccc 6660
ttccgacgct ctgcctatat aaggcaccgt ctgtaagctc ttacgatcat cggtagttca 6720
ccaaggtacg cccgggtcgc tcctacgcgt caatgatccg cggacgccga gcccgagctc 6780
gaatttcccc gatcgttcaa acatttggca ataaagtttc ttaagattga atcctgttgc 6840
cggtcttgcg atgattatca tataatttct gttgaattac gttaagcatg taataattaa 6900
catgtaatgc atgacgttat ttatgagatg ggtttttatg attagagtcc cgcaattata 6960
catttaatac gcgatagaaa acaaaatata gcgcgcaaac taggataaat tatcgcgcgc 7020
ggtgtcatct atgttactag atcgggaatt ggcgcgccac tagtcaattc agtacattaa 7080
aaacgtccgc aatgtgttat taagttgtct aagcgtcaat ttgtttacac cacaatatat 7140
cctgccacca gccagccaac agctccccga ccggcagctc ggcacaaaat caccactcga 7200
tacaggcagc ccatcagtcc gggacggcgt cagcgggaga gccgttgtaa ggcggcagac 7260
tttgctcatg ttaccgatgc tattcggaag aacggcaact aagctgccgg gtttgaaaca 7320
cggatgatct cgcggagggt agcatgttga ttgtaacgat gacagagcgt tgctgcctgt 7380
gatcaaatat catctccctc gcagagatcc gaattatcag ccttcttatt catttctcgc 7440
ttaaccgtga caggctgtcg atcttgagaa ctatgccgac ataataggaa atcgctggat 7500
aaagccgctg aggaagctga gtggcgctat ttctttagaa gtgaacgttg acgatcgtcg 7560
accgtacccc gatgaattaa ttcggacgta cgttctgaac acagctggat acttacttgg 7620
gcgattgtca tacatgacat caacaatgta cccgtttgtg taaccgtctc ttggaggttc 7680
gtatgacact agtggttccc ctcagcttgc gactagatgt tgaggcctaa cattttatta 7740
gagagcaggc tagttgctta gatacatgat cttcaggccg ttatctgtca gggcaagcga 7800
aaattggcca tttatgacga ccaatgcccc gcagaagctc ccatctttgc cgccatagac 7860
gccgcgcccc ccttttgggg tgtagaacat ccttttgcca gatgtggaaa agaagttcgt 7920
tgtcccattg ttggcaatga cgtagtagcc ggcgaaagtg cgagacccat ttgcgctata 7980
tataagccta cgatttccgt tgcgactatt gtcgtaattg gatgaactat tatcgtagtt 8040
gctctcagag ttgtcgtaat ttgatggact attgtcgtaa ttgcttatgg agttgtcgta 8100
gttgcttgga gaaatgtcgt agttggatgg ggagtagtca tagggaagac gagcttcatc 8160
cactaaaaca attggcaggt cagcaagtgc ctgccccgat gccatcgcaa gtacgaggct 8220
tagaaccacc ttcaacagat cgcgcatagt cttccccagc tctctaacgc ttgagttaag 8280
ccgcgccgcg aagcggcgtc ggcttgaacg aattgttaga cattatttgc cgactacctt 8340
ggtgatctcg cctttcacgt agtgaacaaa ttcttccaac tgatctgcgc gcgaggccaa 8400
gcgatcttct tgtccaagat aagcctgcct agcttcaagt atgacgggct gatactgggc 8460
cggcaggcgc tccattgccc agtcggcagc gacatccttc ggcgcgattt tgccggttac 8520

tgcgctgtac caaatgcggg acaacgtaag cactacattt cgctcatcgc cagcccagtc 8580
gggcggcgag ttccatagcg ttaaggtttc atttagcgcc tcaaatagat cctgttcagg 8640
aaccggatca aagagttcct ccgccgctgg acctaccaag gcaacgctat gttctcttgc 8700
ttttgtcagc aagatagcca gatcaatgtc gatcgtggct ggctcgaaga tacctgcaag 8760
aatgtcattg cgctgccatt ctccaaattg cagttcgcgc ttagctggat aacgccacgg 8820
aatgatgtcg tcgtgcacaa caatggtgac ttctacagcg cggagaatct cgctctctcc 8880
aggggaagcc gaagtttcca aaaggtcgtt gatcaaagct cgccgcgttg tttcatcaag 8940
ccttacggtc accgtaacca gcaaatcaat atcactgtgt ggcttcaggc cgccatccac 9000
tgcggagccg tacaaatgta cggccagcaa cgtcggttcg agatggcgct cgatgacgcc 9060
aactacctct gatagttgag tcgatacttc ggcgatcacc gcttccctca tgatgtttaa 9120
ctcctgaatt aagccgcgcc gcgaagcggt gtcggcttga atgaattgtt aggcgtcatc 9180
ctgtgctccc gagaaccagt accagtacat cgctgtttcg ttcgagactt gaggtctagt 9240
tttatacgtg aacaggtcaa tgccgccgag agtaaagcca cattttgcgt acaaattgca 9300
ggcaggtaca ttgttcgttt gtgtctctaa tcgtatgcca aggagctgtc tgcttagtgc 9360
ccactttttc gcaaattcga tgagactgtg cgcgactcct ttgcctcggt gcgtgtgcga 9420
cacaacaatg tgttcgatag aggctagatc gttccatgtt gagttgagtt caatcttccc 9480
gacaagctct tggtcgatga atgcgccata gcaagcagag tcttcatcag agtcatcatc 9540
cgagatgtaa tccttccggt aggggctcac acttctggta gatagttcaa agccttggtc 9600
ggataggtgc acatcgaaca cttcacgaac aatgaaatgg ttctcagcat ccaatgtttc 9660
cgccacctgc tcagggatca ccgaaatctt catatgacgc ctaacgcctg gcacagcgga 9720
tcgcaaacct ggcgcggctt ttggcacaaa aggcgtgaca ggtttgcgaa tccgttgctg 9780
ccacttgtta acccttttgc cagatttggt aactataatt tatgttagag gcgaagtctt 9840
gggtaaaaac tggcctaaaa ttgctgggga tttcaggaaa gtaaacatca ccttccggct 9900
cgatgtctat tgtagatata tgtagtgtat ctacttgatc gggggatctg ctgcctcgcg 9960
cgtttcggtg atgacggtga aaacctctga cacatgcagc tcccggagac ggtcacagct 10020
tgtctgtaag cggatgccgg gagcagacaa gcccgtcagg gcgcgtcagc gggtgttggc 10080
gggtgtcggg gcgcagccat gacccagtca cgtagcgata gcggagtgta tactggctta 10140
actatgcggc atcagagcag attgtactga gagtgcacca tatgcggtgt gaaataccgc 10200
acagatgcgt aaggagaaaa taccgcatca ggcgctcttc cgcttcctcg ctcactgact 10260
cgctgcgctc ggtcgttcgg ctgcggcgag cggtatcagc tcactcaaag gcggtaatac 10320
ggttatccac agaatcaggg gataacgcag gaaagaacat gtgagcaaaa ggccagcaaa 10380
aggccaggaa ccgtaaaaag gccgcgttgc tggcgttttt ccataggctc cgcccccctg 10440
acgagcatca caaaaatcga cgctcaagtc agaggtggcg aaacccgaca ggactataaa 10500
gataccaggc gtttccccct ggaagctccc tcgtgcgctc tcctgttccg accctgccgc 10560
ttaccggata cctgtccgcc tttctccctt cgggaagcgt ggcgctttct catagctcac 10620
gctgtaggta tctcagttcg gtgtaggtcg ttcgctccaa gctgggctgt gtgcacgaac 10680
cccccgttca gcccgaccgc tgcgccttat ccggtaacta tcgtcttgag tccaacccgg 10740
taagacacga cttatcgcca ctggcagcag ccactggtaa caggattagc agagcgaggt 10800
atgtaggcgg tgctacagag ttcttgaagt ggtggcctaa ctacggctac actagaagga 10860
cagtatttgg tatctgcgct ctgctgaagc cagttacctt cggaaaaaga gttggtagct 10920
cttgatccgg caaacaaacc accgctggta gcggtggttt ttttgtttgc aagcagcaga 10980
ttacgcgcag aaaaaaagga tctcaagaag atcctttgat cttttctacg gggtctgacg 11040
ctcagtggaa cgaaaactca cgttaaggga ttttggtcat gagattatca aaaaggatct 11100
tcacctagat ccttttaaat taaaaatgaa gttttaaatc aatctaaagt atatatgagt 11160
aaacttggtc tgacagttac caatgcttaa tcagtgaggc acctatctca gcgatctgtc 11220
tatttcgttc atccatagtt gcctgactcc ccgtcgtgta gataactacg atacgggagg 11280
gcttaccatc tggccccagt gctgcaatga taccgcgaga cccacgctca ccggctccag 11340
atttatcagc aataaaccag ccagccggaa gggccgagcg cagaagtggt cctgcaactt 11400
tatccgcctc catccagtct attaattgtt gccgggaagc tagagtaagt agttcgccag 11460
ttaatagttt gcgcaacgtt gttgccattg ctgcaggggg gggggggggg ggggacttcc 11520
attgttcatt ccacggacaa aaacagagaa aggaaacgac agaggccaaa aagcctcgct 11580
ttcagcacct gtcgtttcct ttcttttcag agggtatttt aaataaaaac attaagttat 11640
gacgaagaag aacggaaacg ccttaaaccg gaaaattttc ataaatagcg aaaacccgcg 11700
aggtcgccgc cccgtaacct gtcggatcac cggaaaggac ccgtaaagtg ataatgatta 11760
tcatctacat atcacaacgt gcgtggaggc catcaaacca cgtcaaataa tcaattatga 11820
cgcaggtatc gtattaattg atctgcatca acttaacgta aaaacaactt cagacaatac 11880
aaatcagcga cactgaatac ggggcaacct catgtccccc cccccccccc ccctgcaggc 11940
atcgtggtgt cacgctcgtc gtttggtatg gcttcattca gctccggttc ccaacgatca 12000
aggcgagtta catgatcccc catgttgtgc aaaaaagcgg ttagctcctt cggtcctccg 12060
atcgttgtca gaagtaagtt ggccgcagtg ttatcactca tggttatggc agcactgcat 12120
aattctctta ctgtcatgcc atccgtaaga tgcttttctg tgactggtga gtactcaacc 12180
aagtcattct gagaatagtg tatgcggcga ccgagttgct cttgcccggc gtcaacacgg 12240
gataataccg cgccacatag cagaacttta aaagtgctca tcattggaaa acgttcttcg 12300
gggcgaaaac tctcaaggat cttaccgctg ttgagatcca gttcgatgta acccactcgt 12360
gcacccaact gatcttcagc atcttttact ttcaccagcg tttctgggtg agcaaaaaca 12420
ggaaggcaaa atgccgcaaa aaagggaata agggcgacac ggaaatgttg aatactcata 12480
ctcttccttt ttcaatatta ttgaagcatt tatcagggtt attgtctcat gagcggatac 12540
atatttgaat gtatttagaa aaataaacaa ataggggttc cgcgcacatt tccccgaaaa 12600
gtgccacctg acgtctaaga aaccattatt atcatgacat taacctataa aaataggcgt 12660

atcacgaggc cctttcgtct tcaagaattg gtcgacgatc ttgctgcgtt cggatatttt 12720
cgtggagttc ccgccacaga cccggattga aggcgagatc cagcaactcg cgccagatca 12780
tcctgtgacg gaactttggc gcgtgatgac tggccaggac gtcggccgaa agagcgacaa 12840
gcagatcacg cttttcgaca gcgtcggatt tgcgatcgag gatttttcgg cgctgcgcta 12900
cgtccgcgac cgcgttgagg gatcaagcca cagcagccca ctcgaccttc tagccgaccc 12960
agacgagcca agggatcttt ttggaatgct gctccgtcgt caggctttcc gacgtttggg 13020
tggttgaaca gaagtcatta tcgtacggaa tgccaagcac tcccgagggg aaccctgtgg 13080
ttggcatgca catacaaatg gacgaacgga taaacctttt cacgcccttt taaatatccg 13140
attattctaa taaacgctct tttctcttag 13170

<210> 314
<211> 16
<212> DNA
<213> Primer

<400> 314
gggtcgctcc tacgcg 16

<210> 315
<211> 46
<212> DNA
<213> Primer

<400> 315
gggtcgctcc tacgcgtcaa tgatccgcgg acgccgagcc cgagct 46

<210> 316
<211> 42
<212> DNA
<213> Primer

<400> 316
cgggctcggc gtccgcggat cattgacgcg taggagcgac cc 42

<210> 317
<211> 16
<212> DNA
<213> Primer

<400> 317
ctcgggctcg gcgtcc 16

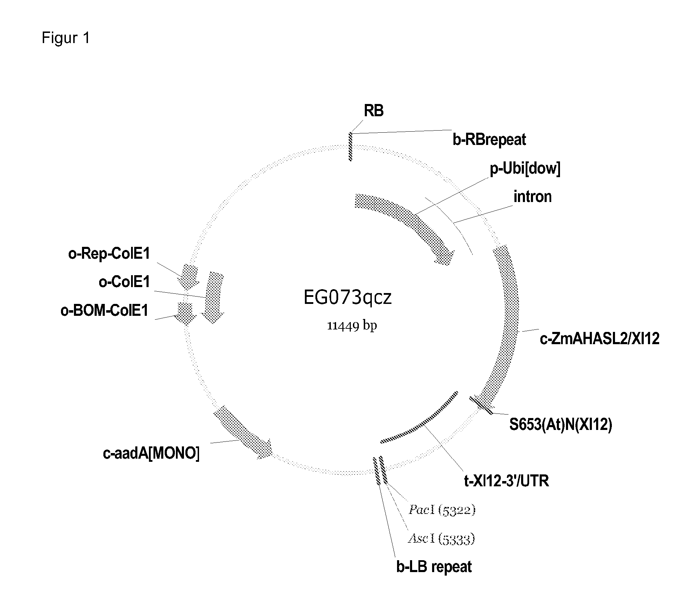

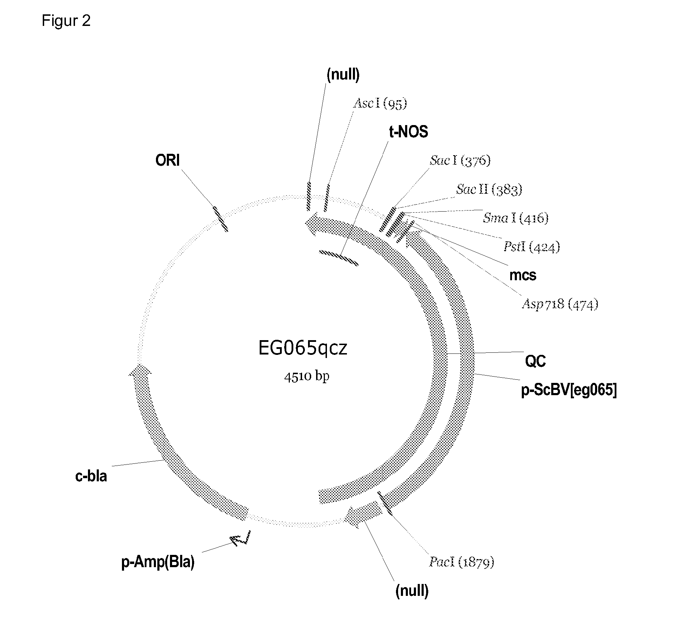

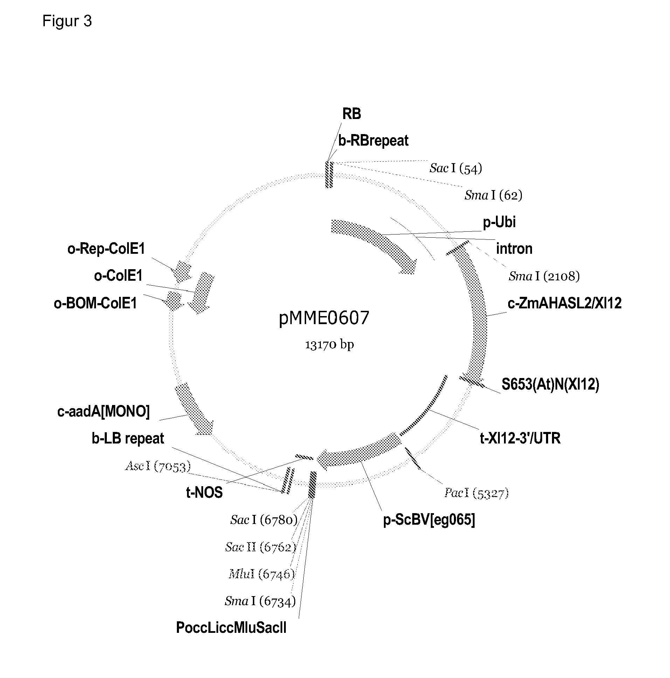

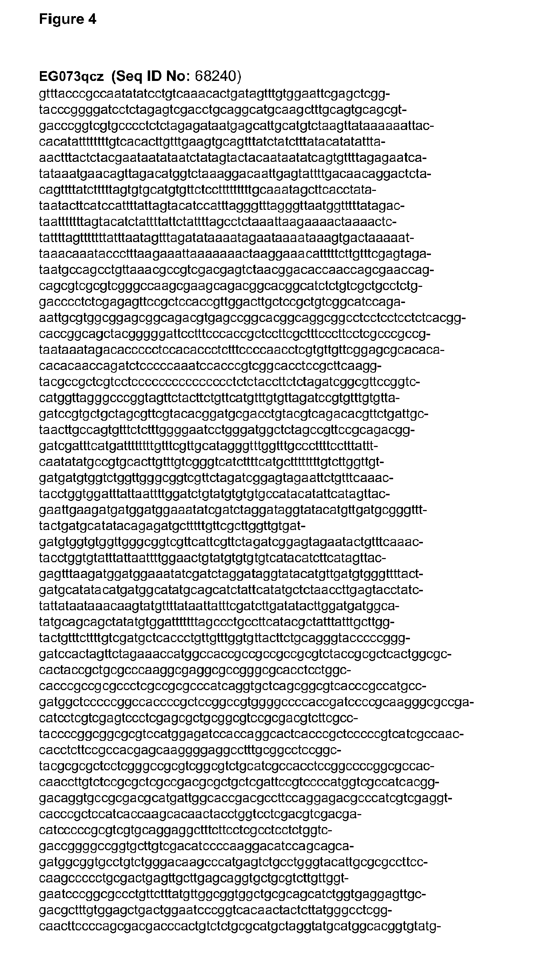

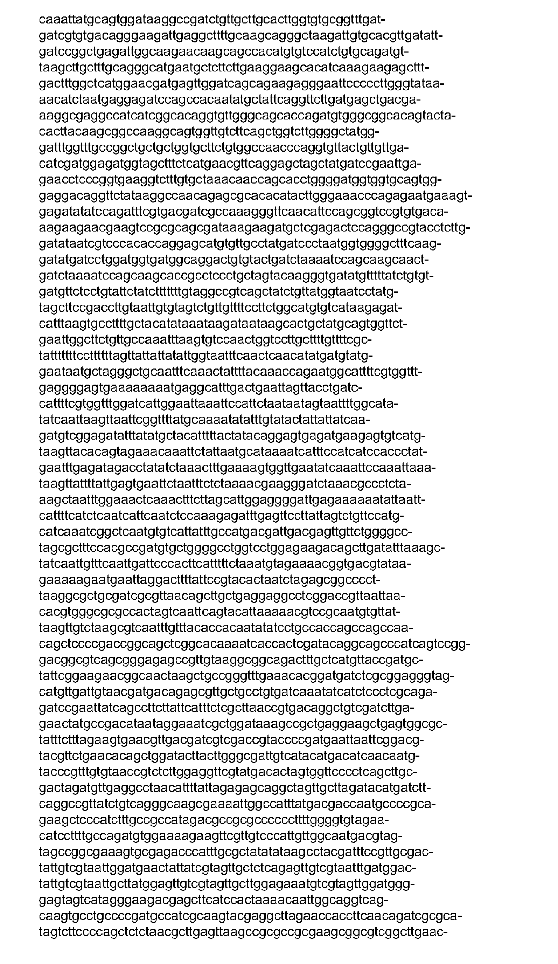

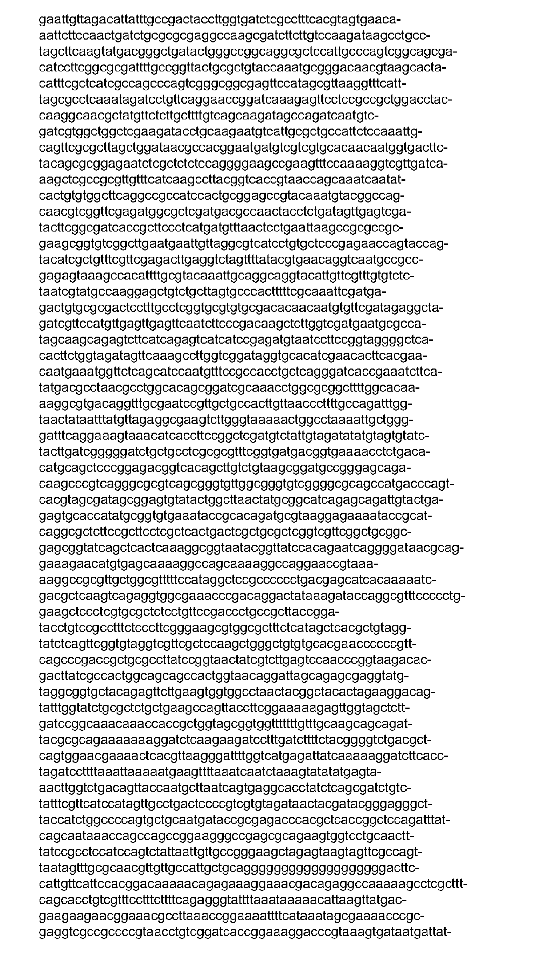

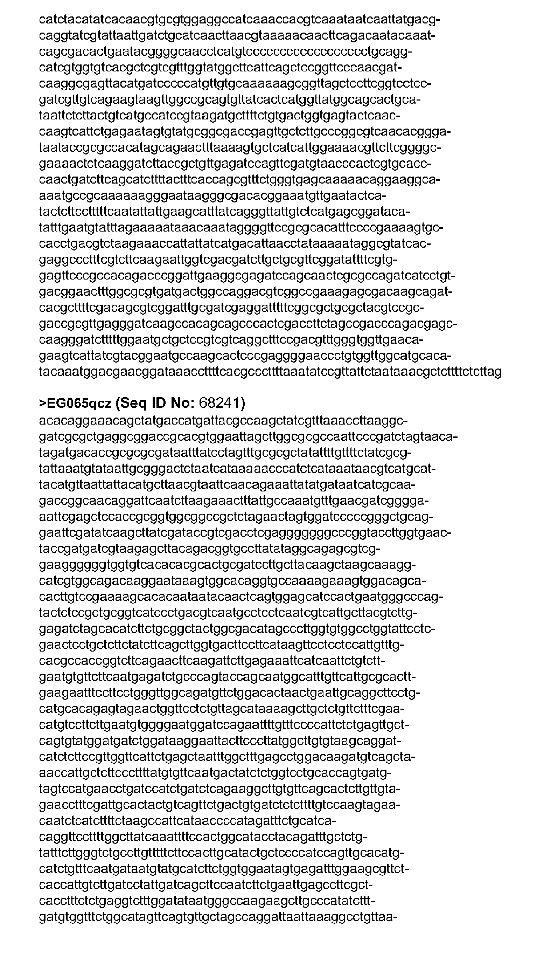

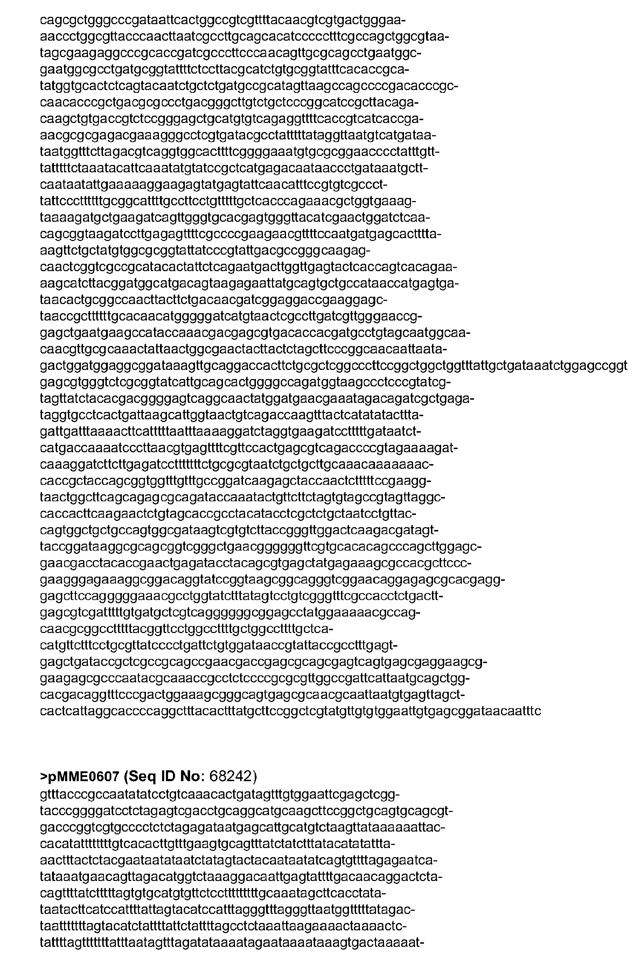

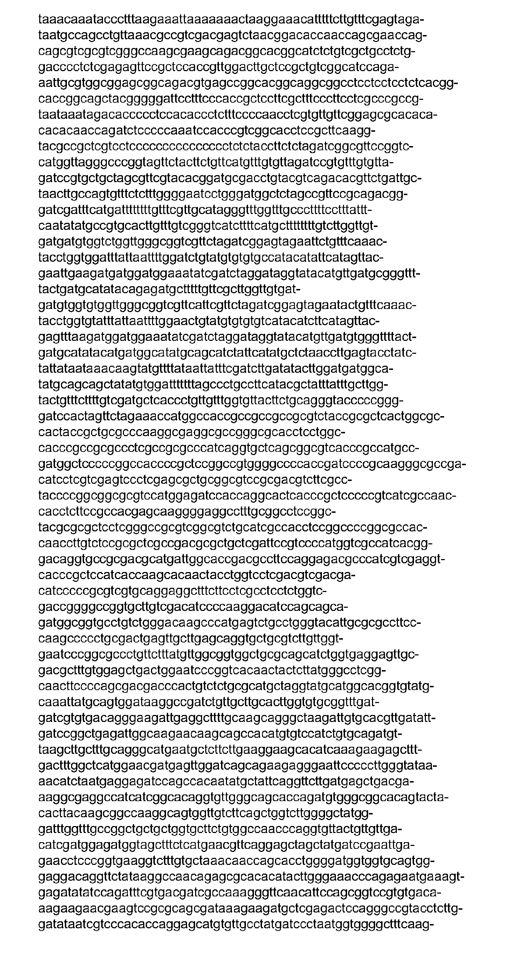

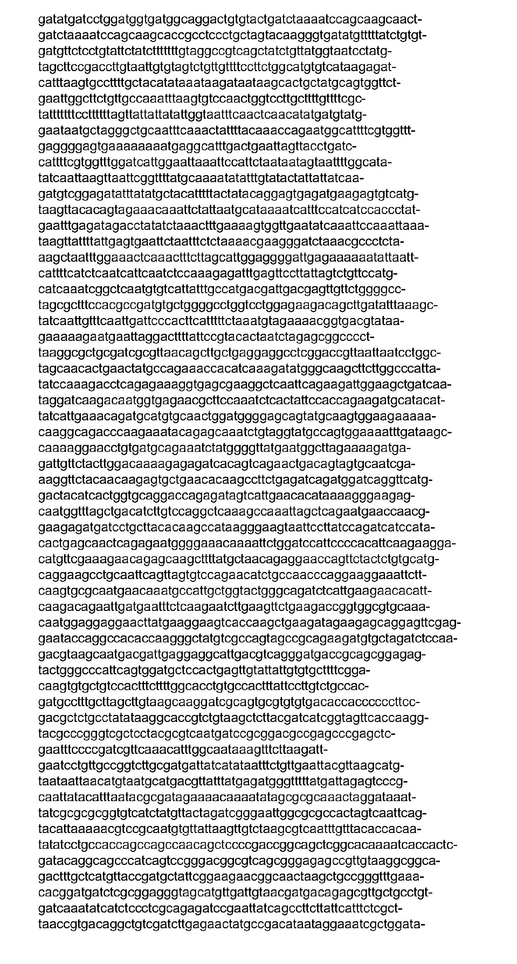

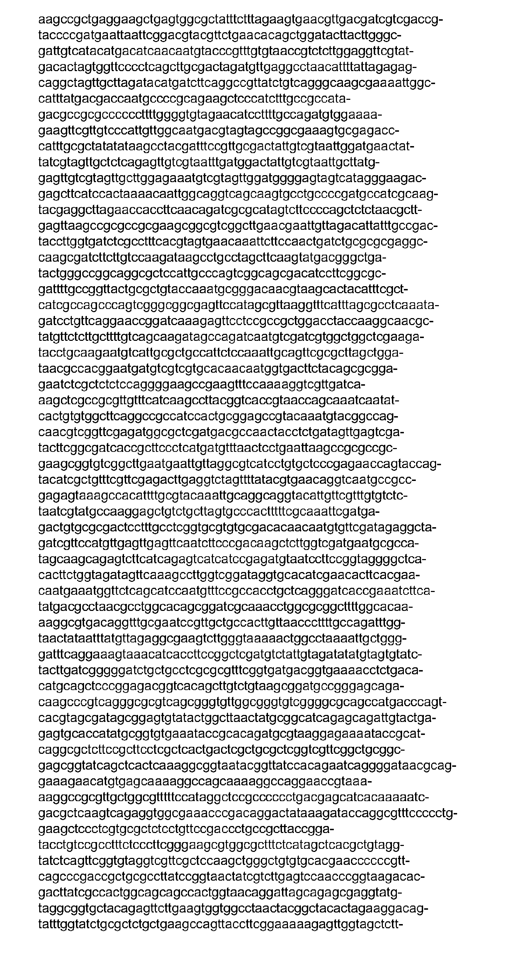

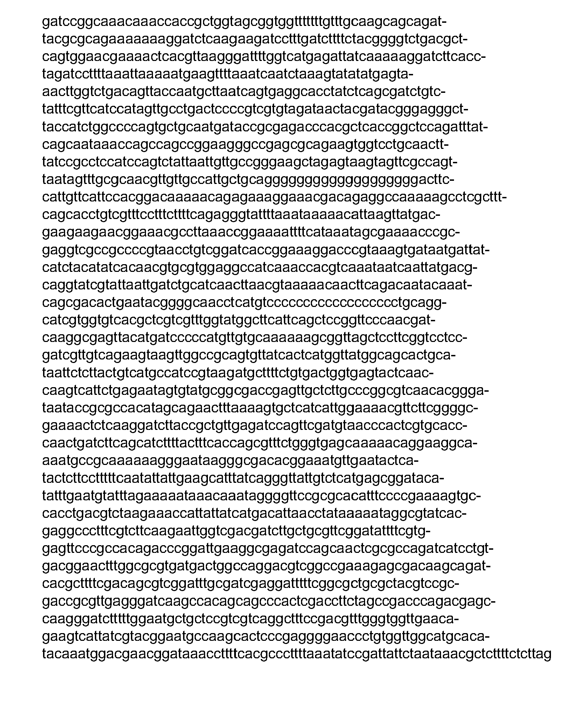

## Description

**[0001.0.0.0]** The present invention relates to a process for the production of a fine chemical in a microorgansm, a plant cell, a plant, a plant tissue or in one or more parts thereof. The invention furthermore relates to nucleic acid molecules, polypeptides, nucleic acid constructs, vectors, antisense molecules, antibodies, host cells, plant tissue, propagtion material, harvested material, plants, microorganisms as well as agricultural compositions and to their use.

**[0002.0.0.0]** Amino acids are used in many branches of industry, including the food, animal feed, cosmetics, pharmaceutical and chemical industries. Amino acids such as D,L-methionine, L-lysine or L-threonine are used in the animal feed industry. The essential amino acids valine, leucine, isoleucine, lysine, threonine, methionine, tyrosine, phenylalanine and tryptophan are particularly important for the nutrition of humans and a number of livestock species. Glycine, L-methionine and tryptophan are all used in the pharmaceutical industry. Glutamine, valine, leucine, isoleucine, histidine, arginine, proline, serine and alanine are used in the pharmaceutical and cosmetics industries. Threonine, tryptophan and D,L-methionine are widely used feed additives (Leuchtenberger, W. (1996) Amino acids - technical production and use, pp. 466-502 in Rehm et al., (Ed.) Biotechnology vol. 6, chapter 14a, VCH Weinheim). Moreover, amino acids are suitable for the chemical industry as precursors for the synthesis of synthetic amino acids and proteins, such as N-acetylcysteine, S-carboxymethyl-L-cysteine, (S)-5-hydroxytryptophan and other substances described in Ullmann's Encyclopedia of Industrial Chemistry, vol. A2, pp. 57-97, VCH Weinheim, 1985.

**[0003.0.0.0]** Over one million tons of amino acids are currently produced annually; their market value amounts to over 2.5 billion US dollars. They are currently produced by four competing processes: Extraction from protein hydrolysates, for example L-cystine, L-leucine or L-tyrosine, chemical synthesis, for example of D-, L-methionine, conversion of chemical precursors in an enzyme or cell reactor, for example L-phenylalanine, and fermentative production by growing, on an industrial scale, bacteria which have been developed to produce and secrete large amounts of the desired molecule in question. An organism, which is particularly suitable for this purpose is Corynebacterium glutamicum, which is used for example for the production of L-lysine or L-glutamic acid. Other amino acids which are produced by fermentation are, for example, L-threonine, L-tryptophan, L-aspartic acid and L-phenylalanine.

**[0004.0.0.0]** The biosynthesis of the natural amino acids in organisms capable of producing them, for example bacteria, has been characterized thoroughly; for a review of the bacterial amino acid biosynthesis and its regulation, see Umbarger, H.E. (1978) Ann. Rev. Biochem. 47: 533 - 606.

**[0005.0.0.0]** It is known that amino acids are produced by fermentation of strains of coryneform bacteria, in particular Corynebacterium glutamicum. Due to their great importance, the production processes are constantly being improved. Process improvements can relate to measures regarding technical aspects of the fermentation, such as, for example, stirring and oxygen supply, or the nutrient media composition, such as, for example, the sugar concentration during fermentation, or to the work-up to give the product, for example by ion exchange chromatography, or to the intrinsic performance properties of the microorganism itself. Bacteria from other genera such as Escherichia or Bacillus are also used for the production of amino acids. A number of mutant strains, which produce an assortment of desirable compounds from the group of the sulfur-containing fine chemicals, have been developed via strain selection. The performance properties of said microorganisms are improved with respect to the production of a particular molecule by applying methods of mutagenesis, selection and mutant selection. Methods for the production of methionine have also been developed. In this manner, strains are obtained which are, for example, resistant to antimetabolites, such as, for example, the methionine analogues -methylmethionine, ethionine, norleucine, N-acetylnorleucine, S-trifluoromethylhomocysteine, 2-amino-5-heprenoitic acid, selenomethionine, methionine sulfoximine, methoxine, 1-aminocyclopentanecarboxylic acid or which are auxotrophic for metabolites with regulatory importance and which produce sulfur-containing fine chemicals such as, for example, L-methionine. However, such processes developed for the production of methionine have the disadvantage that their yields are too low for being economically exploitable and that they are therefore not yet competitive with regard to chemical synthesis.

**[0006.0.0.0]** Zeh (Plant Physiol., Vol. 127, 2001: 792-802) describes increasing the methionine content in potato plants by inhibiting threonine synthase by what is known as antisense technology. This leads to a reduced threonine synthase activity without the threonine content in the plant being reduced. This technology is highly complex; the enzymatic activity must be inhibited in a very differentiated manner since otherwise auxotrophism for the amino acid occurs and the plant will no longer grow.

**[0007.0.0.0]** US 5,589,616 teaches the production of higher amounts of amino acids in plants by overexpressing a monocot storage protein in dicots. WO 96/38574, WO 97/07665, WO 97/28247, US 4,886,878, US 5,082,993 and US 5,670,635 are following this approach. That means in all the aforementioned intellectual property rights different proteins or polypeptides are expressed in plants. Said proteins or polypeptides should function as amino acid sinks. Other methods for increasing amino acids such as lysine are disclosed in WO 95/15392, WO 96/38574, WO 89/11789 or WO 93/19190. In this cases special enzymes in the amino acid biosynthetic pathway such as the diphydrodipicolinic acid synthase are deregulated. This leads to an increase in the production of lysine in the different plants. Another approach to increase the level of amino acids in plants is disclosed in EP-A-0 271 408. EP-A-0 271 408 teaches the mutagenesis of plant and selection afterwards with inhibitors of certain enzymes of amino acid biosynthetic pathway.

**[0008.0.0.0]** Methods of recombinant DNA technology have also been used for some years to improve Corynebacterium strains producing L-amino acids by amplifying individual amino acid biosynthesis genes and investigating the effect on amino acid production.

**[0009.0.0.0]** As described above, the essential amino acids are necessary for humans and many mammals, for example for livestock. L-methionine is important as methyl group donor for the biosynthesis of, for example, choline, creatine, adrenaline, bases and RNA and DNA, histidine, and for the transmethylation following the formation of S-adenosylmethionine or as a sulfhydryl group donor for the formation of cysteine. Moreover, L-methionine appears to have a positive effect in depression.

**[0010.0.0.0]** Improving the quality of foodstuffs and animal feeds is an important task of the food-and-feed industry. This is necessary since, for example, certain amino acids, which occur in plants are limited with regard to the supply of mammals. Especially advantageous for the quality of foodstuffs and animal feeds is as balanced as possible an amino acid profile since a great excess of an amino acid above a specific concentration in the food has no further positive effect on the utilization of the food since other amino acids suddenly become limiting. A further increase in quality is only possible via addition of further amino acids, which are limiting under these conditions. The targeted addition of the limiting amino acid in the form of synthetic products must be carried out with extreme caution in order to avoid amino acid imbalance. For example, the addition of an essential amino acid stimulates protein digestion, which may cause deficiency situations for the second or third limiting amino acid, in particular. In feeding experiments, for example casein feeding experiments, the additional provision of methionine, which is limiting in casein, has revealed the fatty degeneration of liver, which could only be alleviated after the additional provision of tryptophan.

**[0011.0.0.0]** To ensure a high quality of foods and animal feeds, it is therefore necessary to add a plurality of amino acids in a balanced manner to suit the organism.

**[0012.0.0.0]** It is an object of the present invention to develop an inexpensive process for the synthesis of L-methionine. L-methionine is with lysine or threonine (depending on the organism) one of the two amino acids which are most frequently limiting

**[0013.0.0.0]** It was now found that this object is achieved by providing the process according to the invention described herein and the embodiments characterized in the claims.

**[0014.0.0.0]** Accordingly, in a first embodiment, the invention relates to a process for the production of a fine chemical, whereby the fine chemical is methionine Accordingly, in the present invention, the term "the fine chemical" as used herein relates to "methadone". Further, in another embodiment the term "the fine chemicals" as used herein also relates to compositions of fine chemicals comprising methionine.

**[0015.0.0.0]** In one embodiment, the term "the fine chemical" or "the respective fine chemical" means L-methionine. Throughout the specification the term "the fine chemical" or "the respective fine chemical" means methionine, preferably L-methionine, its salts, ester or amids in free form or bound to proteins. In a preferred embodiment, the term "the fine chemical" means L-methionine in free form or its salts or bound to proteins. In one embodiment, the term "the fine chemical" and the term "the respective fine chemical" mean at least one chemical compound with an activity of the above mentioned fine chemical.

**[0016.0.0.0]** Accordingly, the present invention relates to a process for the production of the respective fine chemical comprising
(a) increasing or generating the activity of one or more
   of a protein as shown in table II, application no. 0, column 3 encoded by the nucleic acid sequences as shown in table I, application no. 0, column 5, in a non-human organism or in one or more parts thereof or
(b) growing the organism under conditions which permit the production of
   the fine chemical, thus amino acid of the invention or fine chemicals comprising amino acids of the invention, in said organism or in the culture medium surrounding the organism.

**[0017.0.0.0]** Comprises/comprising and grammatical variations thereof when used in this specification are to be taken to specify the presence of stated features, integers, steps or components or groups thereof, but not to preclude the presence or addition of one or more other features, integers, steps, components or groups thereof.
The term "Table I" used in this specification is to be taken to specify the content of Table I A and Table I B. The term "Table II" used in this specification is to be taken to specify the content of Table II A and Table II B. The term "Table I A" used in this specification is to be taken to specify the content of Table I A. The term "Table I B" used in this specification is to be taken to specify the content of Table I B. The term "Table II A" used in this specification is to be taken to specify the content of Table II A. The term "Table II B" used in this specification is to be taken to specify the content of Table II B. In one preferred embodiment, the term "Table I" means Table I B. In one preferred embodiment, the term "Table II" means Table II B.

**[0018.0.0.0]** Preferably, this process further comprises the step of recovering the fine chemical, which is synthesized by the organism from the organism and/or from the culture medium used for the growth or maintenance of the organism. The term "recovering" means the isolation of the fine chemical in different purities, that means on the one hand harvesting of the biological material, which contains the fine chemical without further purification and on the other hand purities of the fine chemical between 5% and 100% purity, preferred purities are in the range of 10% and 99%. In one embodiment, the purities are 20%, 30%, 40%, 50%, 60%, 70%, 80%, 90%, 95% or 99%.

**[0019.0.0.0]** Advantageously the process for the production of the respective fine chemical leads to an enhanced production of the fine respective chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% or more percent higher production of the respective fine chemical in comparison to the reference as defined below, e.g. that means in comparison to an organism without the aforementioned modification of the activity of a protein having the activity of a protein indicated in Table II, column 3, or encoded by nucleic acid molecule indicated in Table I, columns 5 or 7..

**[0020.0.0.0]** Surprisingly it was found, that the transgenic expression of the Linum usitatissimum protein as indicated in Table II, application no. 0, column 5, line 1 in a plant conferred an increase in methionine content of the transformed plants. Thus, in one embodiment, said protein or its homologs are used for the production of Methionine.
Surprisingly it was found, that the transgenic expression of the Glycine max protein as indicated in Table II, application no. 0, column 5, line 2 in a plant conferred an increase in methionine content of the transformed plants. Thus, in one embodiment, said protein or its homologs are used for the production of methionine.

**[0021.0.0.0]** In accordance with the invention, the term "organism" as understood herein relates always to a non-human organism, in particular to an animal or plant organism or to a microorganism. Further, the term "animal" as understood herein relates always to a non-human animal.
In accordance with the invention it is known to the skilled that anionic compounds such as acids are present in aqueous solutions in an equilibrium between the acid and its salts according to the pH present in the respective compartment of the cell or organism and the pK of the acid. Depending on the strength of the acid (pK) and the pH the salt or the free acid are predominant. Thus, the term "the fine chemical", the term "the respective fine chemical", or the term "acid" or the use of a denomination referring to a neutralized anionic compound relates to the anionic form as well as the neutralised status of that compound according to the milieu of the aqueous solution in which they are present.

**[0022.0.0.0]** The sequence of b1343 from Escherichia coli K12 has been published in Blattner, Science 277(5331), 1453-1474, 1997, and its activity is being defined as an ATP-dependent RNA helicase, stimulated by 23S rRNA. Accordingly, in one embodiment, the process of the present invention comprises the use of an "ATP-dependent RNA helicase, stimulated by 23S rRNA" from E. coli or its homolog, e.g. as shown herein, for the production of the fine chemical, meaning of methionine, in particular for increasing the amount of methionine in free or bound form in an organism or a part thereof, as mentioned. In one embodiment, in the process of the present invention the activity of a protein having an activity in rRNA processing or translation is increased or generated, e.g. from E. coli or a plant or a homolog thereof. Accordingly, in one embodiment, in the process of the present invention the activity of a ATP-dependent RNA helicase, stimulated by 23S rRNA or its homolog is increased for the production of the fine chemical, meaning of methionine, in particular for increasing the amount of methionine in free or bound form in an organism or a part thereof, as mentioned.
The sequence of b4232 from Escherichia coli K12 has been published in Blattner, Science 277(5331), 1453-1474, 1997, and its activity is being defined as a fructose-1,6-bisphosphatase. Accordingly, in one embodiment, the process of the present invention comprises the use of a "fructose-1,6-bisphosphatase" from E. coli or a plant or its homolog, e.g. as shown herein, for the production of the fine chemical, meaning of methionine, in particular for increasing the amount of methionine in free or bound form in an organism or a part thereof, as mentioned. In one embodiment, in the process of the present invention the activity of a protein of the superfamily "fructose-bisphosphatase", preferably having an activity in C-compound and carbohydrate metabolism, C-compound and carbohydrate utilization, energy, glycolysis and gluconeogenesis, plastid, photosynthesis, more preferred having an "fructose-1,6-bisphosphatase"-activity, is increased or generated, e.g. from E. coli or a homolog thereof. Accordingly, in one embodiment, in the process of the present invention the activity of a "fructose-1,6-bisphosphatase" or its homolog is increased for the production of the fine chemical, meaning of methionine, in particular for increasing the amount of methionine in free or bound form in an organism or a part thereof, as mentioned.

**[0022.1.0.0]** In one embodiment of the invention the polypeptides as shown in table II, column 5 and 7, or encoded by a nucleic acid molecule as shown in table I, column 5 and 7, and conferring an increase in the content of the fine chemical as shown in table I to IV, column 6 respectively, has preferably the activity of a E. coli or Saccharomyces cerevisae, respectively, protein as mentioned in table I to IV, column 3 respectively and as disclosed in paragraph [0022] of the respective invention.

**[0023.0.0.0]** Homologues ( = homologs) of the present gene products can be derived from any organisms as long as the homologue confers the herein mentioned activity, in particular, confers an increase in the respective fine chemical amount or content.
In one embodiment, the homolog of the any one of the polypeptides indicated in Table II, application no. 0, column 3, is a homolog having the same or a similar acitivity.
In particular an increase of activity confers an increase in the content of the respective fine chemical in the organisms. In one embodiment, the homolog is a homolog with a sequence as indicated in Table I or II, application no. 0, column 7.. In one embodiment, the homolog of one of the polypeptides indicated in Table II, application no. 0, column 3, is derived from an eukaryotic. In one embodiment, the homolog is derived from plants. In one embodiment, the homolog of a polypeptide indicated in Table II, application no. 0, column 3, is derived from a monocotyledonous plant. In one embodiment, the homolog of a polypeptide indicated in Table II, application no. 0, column 3, is derived from a dicotyledonous plant.

**[0023.1.0.0]** Homologs of the polypeptide disclosed in table II, application no. 0, column 5, may have the activity of a protein as disclosed in table I or II, application no. 0, column 3 and may be the polypetides encoded by the nucleic acid molecules indicated in table I , application no. 0, column 7, resp., or may be the polypeptides indicated in table II, application no. 0, column 7, resp.

**[0024.0.0.0]** Further homologs of are described herein below.

**[0025.0.0.0]** In accordance with the invention, a protein or polypeptide has the "activity of an protein as shown in table II, application no. 0, column 3 "if its de *novo* activity, or its increased expression directly or indirectly leads to an increased in the level of the fine chemical indicated in the respective line of table II, application no. 0, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 0, column 3, or which has at least 10% of the original enzymatic or biological activity, preferably 20%, particularly preferably 30%, most particularly preferably 40% in comparison to a protein as shown in the respective line of table II, application no. 0, column 3 of a a E. coli or Saccharomyces cerevisae, respectively, protein as mentioned in table I to IV, column 3 respectively and as disclosed in paragraph [0022] of the respective invention.

**[0025.1.0.0]** In one embodiment, the polypeptide of the invention or the polypeptide used in the method of the invention confers said activity, e.g. the increase of the fine chemical in an organism or a part thereof, if it is derived from an organism, which is evolutionary distant to the organism in which it is expressed. For example origin and expressing organism are derived from different families, orders, classes or phylums.

**[0025.2.0.0]** In one embodiment, the polypeptide of the invention or the polypeptide used in the method of the invention confers said activity, e.g. the increase of the fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0026.0.0.0]** The terms "increased", "rose", "extended", "enhanced", "improved" or "amplified" relate to a corresponding change of a property in an organism, a part of an organism such as a tissue, seed, root, leave, flower etc. or in a cell and are interchangeable. Preferably, the overall activity in the volume is increased or enhanced in cases if the increase or enhancement is related to the increase or enhancement of an activity of a gene product, independent whether the amount of gene product or the specific activity of the gene product or both is increased or enhanced or whether the amount, stability or translation efficacy of the nucleic acid sequence or gene encoding for the gene product is increased or enhanced. The terms "reduction", "decrease" or "deletion" relate to a corresponding change of a property in an organism, a part of an organism such as a tissue, seed, root, leave, flower etc. or in a cell. Preferably, the overall activity in the volume is reduced, decreased or deleted in cases if the reduction, decrease or deletion is related to the reduction, decrease or deletion of an activity of a gene product, independent whether the amount of gene product or the specific activity of the gene product or both is reduced, decreased or deleted or whether the amount, stability or translation efficacy of the nucleic acid sequence or gene encoding for the gene product is reduced, decreased or deleted.

**[0027.0.0.0]** The terms "increase" or "decrease" relate to a corresponding change of a property an organism or in a part of an organism, such as a tissue, seed, root, leave, flower etc. or in a cell. Preferably, the overall activity in the volume is increased in cases the increase relates to the increase of an activity of a gene product, independent whether the amount of gene product or the specific activity of the gene product or both is increased or generated or whether the amount, stability or translation efficacy of the nucleic acid sequence or gene encoding for the gene product is increased.

**[0028.0.0.0]** Under "change of a property" it is understood that the activity, expression level or amount of a gene product or the metabolite content is changed in a specific volume relative to a corresponding volume of a control, reference or wild type, including the de novo creation of the activity or expression.

**[0029.0.0.0]** The terms "increase" or "decrease" include the change or the modulation of said property in only parts of the subject of the present invention, for example, the modification can be found in compartment of a cell, like a organelle, or in a part of a plant, like tissue, seed, root, leave, flower etc. but is not detectable if the overall subject, i.e. complete cell or plant, is tested. Preferably, the increase or decrease is found cellular, thus the term "increase of an activity" or "increase of a metabolite content" relates to the cellular increase compared to the wild type cell. However, the terms increase or decrease as used herein also include the change or modulation of a property in the whole organism as mentioned.

**[0030.0.0.0]** Accordingly, the term "increase" or "decrease" means that the specific activity of an enzyme, preferably the amount of a compound or metabolite, e.g. of a polypeptide, a nucleic acid molecule or of the respective fine chemical of the invention or an encoding mRNA or DNA, can be increased or decreased in a volume.

**[0031.0.0.0]** The terms "wild type", "control" or "reference" are exchangeable and can be a cell or a part of organisms such as an organelle or a tissue, or an organism, in particular a microorganism or a plant, which was not modified or treated according to the herein described process according to the invention. Accordingly, the cell or a part of organisms such as an organelle or a tissue, or an organism, in particular a microorganism or a plant used as wild type, control or reference corresponds to the cell, organism or part thereof as much as possible and is in any other property but in the result of the process of the invention as identical to the subject matter of the invention as possible. Thus, the wild type, control, or reference is treated identically or as identical as possible, saying that only conditions or properties might be different which do not influence the quality of the tested property.

**[0032.0.0.0]** Preferably, any comparison is carried out under analogous conditions. The term "analogous conditions" means that all conditions such as, for example, culture or growing conditions, assay conditions (such as buffer composition, temperature, substrates, pathogen strain, concentrations and the like) are kept identical between the experiments to be compared.

**[0033.0.0.0]** The "reference", "control", or "wild type" is preferably a subject, e.g. an organelle, a cell, a tissue, an organism, in particular a plant or a microorganism, which was not modified or treated according to the herein described process of the invention and is in any other property as similar to the subject matter of the invention as possible. The reference, control, or wild type is in its genome, transcriptome, proteome or meta-bolome as similar as possible to the subject of the present invention. Preferably, the term "reference-" "control-" or "wild type-"-organelle, -cell, -tissue or-organism, in particular plant or microorganism, relates to an organelle, cell, tissue or organism, in particular plant or microorganism, which is nearly genetically identical to the organelle, cell, tissue or organism, in particular microorganism or plant, of the present invention or a part thereof preferably 95%, more preferred are 98%, even more preferred are 99,00%, in particular 99,10%, 99,30%, 99,50%, 99,70%, 99,90%, 99,99%, 99, 999% or more.. Most preferable the "reference", "control", or "wild type" is a subject, e.g. an organelle, a cell, a tissue, an organism, which is genetically identical to the organism, cell or organelle used according to the process of the invention except that the responsible or activity conferring nucleic acid molecules or the gene product encoded by them are amended, manipulated, exchanged or introduced according to the inventive process.

**[0034.0.0.0]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 0, column 3 its biochemical or genetical causes and the increased amount of the respective fine chemical.

**[0035.0.0.0]** In case, a control, reference or wild type differing from the subject of the present invention only by not being subject of the process of the invention can not be provided, a control, reference or wild type can be an organism in which the cause for the modulation of an activity conferring the increase of the fine chemical or expression of the nucleic acid molecule as described herein has been switched back or off, e.g. by knocking out the expression of responsible gene product, e.g. by antisense inhibition, by inactivation of an activator or agonist, by activation of an inhibitor or antagonist, by inhibition through adding inhibitory antibodies, by adding active compounds as e.g. hormones, by introducing negative dominant mutants, etc. A gene production can for example be knocked out by introducing inactivating point mutations, which lead to an enzymatic activity inhibition or a destabilization or an inhibition of the ability to bind to cofactors etc.

**[0036.0.0.0]** Accordingly, preferred reference subject is the starting subject of the present process of the invention. Preferably, the reference and the subject matter of the invention are compared after standardization and normalization, e.g. to the amount of total RNA, DNA, or Protein or activity or expression of reference genes, like housekeeping genes, such as ubiquitin, actin or ribosomal proteins.

**[0037.0.0.0]** A series of mechanisms exists via which a modification of a protein, e.g. the polypeptide of the invention or the polypeptide used in the method of the invention can directly or indirectly affect the yield, production and/or production efficiency of the fine chemical.

**[0038.0.0.0]** For example, the molecule number or the specific activity of the polypeptide or the nucleic acid molecule may be increased. Larger amounts of the fine chemical can be produced if the polypeptide or the nucleic acid of the invention is expressed de *novo* in an organism lacking the activity of said protein. However, it is also possible to increase the expression of the gene which is naturally present in the organisms, for example by amplifying the number of gene(s), by modifying the regulation of the gene, or by increasing the stability of the corresponding mRNA or of the corresponding gene product encoded by the nucleic acid molecule of the invention or the nucleic acid molecule used in the method of the invention, or by introducing homologous genes from other organisms which are differently regulated, e.g. not feedback sensitive.

**[0039.0.0.0]** This also applies analogously to the combined increased expression of the nucleic acid molecule of the present invention or its gene product with that of further enzymes or regulators of the biosynthesis pathways of the respective fine chemical, e.g. which are useful for the synthesis of the respective fine chemicals.

**[0040.0.0.0]** The increase, decrease or modulation according to this invention can be constitutive, e.g. due to a stable permanent transgenic expression or to a stable mutation in the corresponding endogenous gene encoding the nucleic acid molecule of the invention or the nucleic acid molecule used in the method of the invention or to a modulation of the expression or of the behaviour of a gene conferring the expression of the polypeptide of the invention or the polypeptide used in the method of the invention, or transient, e.g. due to an transient transformation or temporary addition of a modulator such as a agonist or antagonist or inducible, e.g. after transformation with a inducible construct carrying the nucleic acid molecule of the invention or the nucleic acid molecule used in the method of the invention under control of a inducible promoter and adding the inducer, e.g. tetracycline or as described herein below.

**[0041.0.0.0]** The increase in activity of the polypeptide amounts in a cell, a tissue, a organelle, an organ or an organism or a part thereof preferably to at least 5%, preferably to at least 20% or at to least 50%, especially preferably to at least 70%, 80%, 90% or more, very especially preferably are to at least 200%, most preferably are to at least 500% or more in comparison to the control, reference or wild type.

**[0042.0.0.0]** The specific activity of a polypeptide encoded by a nucleic acid molecule of the present invention or of the polypeptide of the present invention can be tested as described in the examples. In particular, the expression of a protein in question in a cell, e.g. a plant cell or a microorganism and the detection of an increase the respective fine chemical level in comparison to a control is an easy test and can be performed as described in the state of the art.

**[0043.0.0.0]** The term "increase" includes, that a compound or an activity is introduced into a cell de *novo* or that the compound or the activity has not been detectable before, in other words it is "generated".

**[0044.0.0.0]** Accordingly, in the following, the term "increasing" also comprises the term "generating" or "stimulating". The increased activity manifests itself in an increase of the fine chemical.

**[0045.0.0.0]** In one embodiment, the activity of the protein as indicated in Table II, columns 5 or 7, application no. 0, is increased conferring an increase of the respective fine chemical, indicated in Table II, application no. 0, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more

**[0046.0.0.0]** In one embodiment, the activity of the protein as indicated in Table II, columns 5 or 7, application no. 0, is increased conferring an increase of the respective fine chemical, indicated in Table II, application no. 0, column 6 of the respective line confers an increase of the respective fine chemical and of further amino acid or their precursors.

**[0047.0.0.0]** In this context, the respective fine chemical amount in a cell, preferably in a tissue, more preferred in a organism as a plant or a microorganism or part thereof, is increased by 3% or more, especially preferably are 10% or more, very especially preferably are more than 30% and most preferably are 70% or more, such as 100%, 300% or 500%.

**[0048.0.0.0]** The respective fine chemical can be contained in the organism either in its free form and/or bound to proteins or polypeptides or mixtures thereof. Accordingly, in one embodiment, the amount of the free form in a cell, preferably in a tissue, more preferred in a organism as a plant or a microorganism or part thereof, is increased by 3% or more, especially preferably are 10% or more, very especially preferably are more than 30% and most preferably are 70% or more, such as 100%, 300% or 500%. Accordingly, in an other embodiment, the amount of the bound the respective fine chemical in a cell, preferably in a tissue, more preferred in a organism as a plant or a microorganism or part thereof, is increased by 3% or more, especially preferably are 10% or more, very especially preferably are more than 30% and most preferably are 70% or more, such as 100%, 300% or 500%.

**[0049.0.0.0]** A protein having an activity conferring an increase in the amount or level of the fine chemical, has in one embodiment the structure of the polypeptide described herein, in particular of the polypeptides comprising the consensus sequence shown in table IV, application no. 0, column 7 or of the polypeptide as shown in the amino acid sequences as disclosed in table II, application no. 0, columns 5 and 7 or the functional homologues thereof as described herein, or is encoded by the nucleic acid molecule characterized herein or the nucleic acid molecule according to the invention, for example by the nucleic acid molecule as shown in table I, application no. 0, columns 5 and 7 or its herein described functional homologues and has the herein mentioned activity.

**[0050.0.0.0]** For the purposes of the present invention, the terms "L-methionine", "methionine", "homocysteine", "S-adenosylmethionine" and "threonine" also encompass the corresponding salts, such as, for example, methionine hydrochloride or methionine sulfate. Preferably the terms methionine or threonine are intended to encompass the terms L-methionine or L-threonine.

**[0051.0.0.0]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g. fine chemical compositions. Depending on the choice of the organism used for the process according to the present invention, for example a microorganism or a plant, compositions or mixtures of various fine chemicals, e.g. comprising further distinct amino acids, fatty acids, vitamins, hormones, sugars, lipids, etc. can be produced.

**[0052.0.0.0]** The term "expression" refers to the transcription and/or translation of a codogenic gene segment or gene. As a rule, the resulting product is an mRNA or a protein. However, expression products can also include functional RNAs such as, for example, antisense, nucleic acids, tRNAs, snRNAs, rRNAs, RNAi, siRNA, ribozymes etc. Expression may be systemic, local or temporal, for example limited to certain cell types, tissues organs or time periods.

**[0053.0.0.0]** In one embodiment, the process of the present invention comprises one or more of the following steps:
a) stabilizing a protein conferring the increased expression of a protein encoded by the nucleic acid molecule of the invention or of the polypeptide of the invention, e.g. of a polypeptide having the activity of a protein as indicated in table II, application no. 0, columns 5 and 7 or its homologs activity having herein-mentioned amino acid of the invention increasing activity; and/or
b) stabilizing a mRNA conferring the increased expression of a protein encoded by the nucleic acid molecule of the invention, as shown in table I, application no. 0, columns 5 and 7, e.g. a nucleic acid sequence encoding a polypeptide having the activity of a protein as indicated in table II, application no. 0, columns 5 and 7 or its homologs activity or of a mRNA encoding the polypeptide of the present invention having herein-mentioned amino acid of the invention increasing activity; and/or
c) increasing the specific activity of a protein conferring the increased expression of a protein encoded by the nucleic acid molecule of the invention or of the polypeptide of the present invention having herein-mentioned amino acid increasing activity, e.g. of a polypeptide having the activity of a protein as indicated in table II, application no. 0, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned amino acid of the invention increasing activity, e.g. of a polypeptide having the activity of a protein as indicated in table II, application no. 0, columns 5 and 7 or its homologs activity; and/or
e) stimulating activity of a protein conferring the increased expression of a protein encoded by the nucleic acid molecule of the present invention or a polypeptide of the present invention having herein-mentioned amino acid of the invention increasing activity, e.g. of a polypeptide having the activity of a protein as indicated in table II, application no. 0, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned amino acid of the invention increasing activity, e.g. of a polypeptide having the activity of a protein as indicated in table II, application no. 0, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned amino acid of the invention increasing activity, e.g. of a polypeptide having the activity of a protein as indicated in table II, application no. 0, columns 5 and 7 or its homologs activity; and/or
h) increasing the expression of the endogenous gene encoding the polypeptide of the invention, e.g. a polypeptide having the activity of a protein as indicated in table II, application no. 0, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with expecially high activity of the proteins of the invention from natural or from mutagenized resources and breeding them into the target organisms, eg the elite crops.

**[0054.0.0.0]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 0 in Table I to IV, resp., after increasing the expression or activity of the encoded polypeptide or having the activity of a polypeptide having an activity as the protein as shown in table II, application no. 0, column 3 or its homologs. In general, the amount of mRNA or polypeptide in a cell or a compartment of a organism correlates with the amount of encoded protein and thus with the overall activity of the encoded protein in said volume. Said correlation is not always linear, the activity in the volume is dependent on the stability of the molecules or the presence of activating or inhibiting co-factors. Further, product and educt inhibitions of enzymes are well known and described in Textbooks, e.g. Stryer, Biochemistry.

**[0055.0.0.0]** In general, the amount of mRNA or polypeptide in a cell or a compartment of a organism correlates with the amount of encoded protein and thus with the overall activity of the encoded protein in said volume. Said correlation is not always linear, the activity in the volume is dependent on the stability of the molecules or the presence of activating or inhibiting co-factors. Further, product and educt inhibitions of enzymes are well known and described in Textbooks, e.g. Stryer, Biochemistry.

**[0056.0.0.0]** In general, the amount of mRNA, polynucleotide or nucleic acid molecule in a cell or a compartment of an organism correlates with the amount of encoded protein and thus with the overall activity of the encoded protein in said volume. Said correlation is not always linear, the activity in the volume is dependent on the stability of the molecules, the degradation of the molecules or the presence of activating or inhibiting co-factors. Further, product and educt inhibitions of enzymes are well known, e.g. Zinser et al. "Enzyminhibitoren"/Enzyme inhibitors".

**[0057.0.0.0]** The activity of the abovementioned proteins and/or polypeptide encoded by the nucleic acid molecule of the present invention can be increased in various ways. For example, the activity in an organism or in a part thereof, like a cell, is increased via increasing the gene product number, e.g. by increasing the expression rate, like introducing a stronger promoter, or by increasing the stability of the mRNA expressed, thus increasing the translation rate, and/or increasing the stability of the gene product, thus reducing the proteins decayed. Further, the activity or turnover of enzymes can be influenced in such a way that a reduction or increase of the reaction rate or a modification (reduction or increase) of the affinity to the substrate results, is reached. A mutation in the catalytic centre of an polypeptide of the invention or the polypeptide used in the method of the invention, e.g. as enzyme, can modulate the turn over rate of the enzyme, e.g. a knock out of an essential amino acid can lead to a reduced or completely knock out activity of the enzyme, or the deletion or mutation of regulator binding sites can reduce a negative regulation like a feedback inhibition (or a substrate inhibition, if the substrate level is also increased). The specific activity of an enzyme of the present invention can be increased such that the turn over rate is increased or the binding of a co-factor is improved. Improving the stability of the encoding mRNA or the protein can also increase the activity of a gene product. The stimulation of the activity is also under the scope of the term "increased activity".

**[0058.0.0.0]** Moreover, the regulation of the abovementioned nucleic acid sequences may be modified so that gene expression is increased. This can be achieved advantageously by means of heterologous regulatory sequences or by modifying, for example mutating, the natural regulatory sequences which are present. The advantageous methods may also be combined with each other.

**[0059.0.0.0]** In general, an activity of a gene product in an organism or part thereof, in particular in a plant cell, a plant, or a plant tissue or a part thereof or in a microorganism can be increased by increasing the amount of the specific encoding mRNA or the corresponding protein in said organism or part thereof. "Amount of protein or mRNA" is understood as meaning the molecule number of polypeptides or mRNA molecules in an organism, a tissue, a cell, or a cell compartment. "Increase" in the amount of a protein means the quantitative increase of the molecule number of said protein in an organism, a tissue, a cell or a cell compartment or part thereof - for example by one of the methods described herein below - in comparison to a wild type, control or reference.

**[0060.0.0.0]** The increase in molecule number amounts preferably to at least 1%, preferably to more than 10%, more preferably to 30% or more, especially preferably to 50%, 70% or more, very especially preferably to 100%, most preferably to 500% or more. However, a de novo expression is also regarded as subject of the present invention.

**[0061.0.0.0]** A modification, i.e. an increase or decrease, can be caused by endogenous or exogenous factors. For example, an increase in activity in an organism or a part thereof can be caused by adding a gene product or a precursor or an activator or an agonist to the media or nutrition or can be caused by introducing said subjects into a organism, transient or stable.

**[0062.0.0.0]** In one embodiment the increase in the amount of the fine chemical in the organism or a part thereof, e.g. in a cell, a tissue, a organ, an organelle etc., is achieved by increasing the endogenous level of the polypeptide of the invention or the polypeptide used in the method of the invention. Accordingly, in an embodiment of the present invention, the present invention relates to a process wherein the gene copy number of a gene encoding the polynucleotide or nucleic acid molecule of the invention or the nucleic acid molecule used in the method of the invention as herein described is increased. Further, the endogenous level of the polypeptide of the invention or the polypeptide used in the method of the invention as described can for example be increased by modifying the transcriptional or translational regulation of the polypeptide.

**[0063.0.0.0]** In one embodiment the amount of the fine chemical in the organism or part thereof can be increase by targeted or random mutagenesis of the endogenous genes of the invention. For example homologous recombination can be used to either introduce positive regulatory elements like for plants the 35S enhancer into the promoter or to remove repressor elements form regulatory regions. In addition gene conversion like methods described by Kochevenko and Willmitzer (Plant Physiol. 2003 May; 132(1): 174-84) and citations therein can be used to disrupt repressor elements or to enhance to activity of positive regulatory elements.
Furthermore positive elements can be randomly introduced in (plant) genomes by T-DNA or transposon mutagenesis and lines can be screened for, in which the positive elements has be integrated near to a gene of the invention, the expression of which is thereby enhanced. The activation of plant genes by random integrations of enhancer elements has been described by Hayashi et al., 1992 (Science 258:1350-1353) or Weigel et al., 2000 (Plant Physiol. 122, 1003-1013) and others citied therein.
Reverse genetic strategies to identify insertions (which eventually carrying the activation elements) near in genes of interest have been described for various cases e.g. Krysan et al., 1999 (Plant Cell 1999, 11, 2283-2290); Sessions et al., 2002 (Plant Cell 2002, 14, 2985-2994); Young et al., 2001, (Plant Physiol. 2001, 125, 513-518); Koprek et al., 2000 (Plant J. 2000, 24, 253-263) ; Jeon et al., 2000 (Plant J. 2000, 22, 561-570) ; Tissier et al., 1999 (Plant Cell 1999, 11, 1841-1852); Speulmann et al., 1999 (Plant Cell 1999,11 , 1853-1866). Briefly material from all plants of a large T-DNA or transposon mutagenized plant population is harvested and genomic DNA prepared. Then the genomic DNA is pooled following specific architectures as described for example in Krysan et al., 1999 (Plant Cell 1999, 11, 2283-2290). Pools of genomics DNAs are then screened by specific multiplex PCR reactions detecting the combination of the insertional mutagen (e.g. T-DNA or Transposon) and the gene of interest. Therefore PCR reactions are run on the DNA pools with specific combinations of T-DNA or transposon border primers and gene specific primers. General rules for primer design can again be taken from Krysan et al., 1999 (Plant Cell 1999, 11, 2283-2290) Rescreening of lower levels DNA pools lead to the identification of individual plants in which the gene of interest is disrupted by the insertional mutagen.
The enhancement of positive regulatory elements or the disruption or weaking of negative regulatory elements can also be achieved through common mutagenesis techniques: The production of chemically or radiation mutated populations is a common technique and known to the skilled worker. Methods for plants are described by Koorneef et al. 1982 and the citations therein and by Lightner and Caspar in "Methods in Molecular Biology" Vol 82. These techniques usually induce pointmutations that can be identified in any known gene using methods such as tilling (Colbert et al. 2001).
Accordingly, the expression level can be increased if the endogenous genes encoding a polypeptide conferring an increased expression of the polypeptide of the present invention, in particular genes comprising the nucleic acid molecule of the present invention, are modified via homologous recombination, tilling approaches or gene conversion

**[0064.0.0.0]** Regulatory sequences can be operatively linked to the coding region of an endogenous protein and control its transcription and translation or the stability or decay of the encoding mRNA or the expressed protein. In order to modify and control the expression, promoter, UTRs, splicing sites, processing signals, polyadenylation sites, terminators, enhancers, repressors, post transcriptional or posttranslational modification sites can be changed, added or amended for example, the activation of plant genes by random integrations of enhancer elements has been described by Hayashi et al., 1992 (Science 258:1350-1353) or Weigel et al., 2000 (Plant Physiol. 122, 1003-1013) and others citied therein. For example, the expression level of the endogenous protein can be modulated by replacing the endogenous promoter with a stronger transgenic promoter or by replacing the endogenous 3'UTR with a 3'UTR, which provides more stability without amending the coding region. Further, the transcriptional regulation can be modulated by introduction of an artificial transcription factor as described in the examples. Alternative promoters, terminators and UTR are described below.

**[0065.0.0.0]** The activation of an endogenous polypeptide having above-mentioned activity, of the polypeptide of the invention or the polypeptide used in the method of the invention, e.g. conferring the increase of the respective fine chemical after increase of expression or activity can also be increased by introducing a synthetic transcription factor, which binds close to the coding region of an endogenous polypeptide of the invention or the polypeptide used in the method of the invention- or used in the process of the invention or its endogenous homolog -encoding gene and the synthetic transcription factor activates its transcription. A chimeric zinc finger protein can be construed, which comprises a specific DNA-binding domain and an activation domain as e.g. the VP16 domain of Herpes Simplex virus. The specific binding domain can bind to the regulatory region of the endogenous protein coding region. The expression of the chimeric transcription factor in a organism, in particular in a plant, leads to a specific expression of an endogenous polypeptid of the invention or used in the process of the invention, in particular a plant homolog thereof, see e.g. in WO01/52620, Oriz, Proc. Natl. Acad. Sci. USA, 2002, Vol. 99, 13290 or Guan, Proc. Natl. Acad. Sci. USA, 2002, Vol. 99, 13296.

**[0066.0.0.0]** In one further embodiment of the process according to the invention, organisms are used in which one of the abovementioned genes, or one of the abovementioned nucleic acids, is mutated in a way that the activity of the encoded gene products is less influenced by cellular factors, or not at all, in comparison with the unmutated proteins. For example, well known regulation mechanism of enzymic activity are substrate inhibition or feed back regulation mechanisms. Ways and techniques for the introduction of substitutions, deletions and additions of one or more bases, nucleotides or amino acids of a corresponding sequence are described herein below in the corresponding paragraphs and the references listed there, e.g. in Sambrook et al., Molecular Cloning, Cold Spring Habour, NY, 1989. The person skilled in the art will be able to identify regulation domains and binding sites of regulators by comparing the sequence of the nucleic acid molecule of the present invention or the expression product thereof with the state of the art by computer software means which comprise algorithms for the identifying of binding sites and regulation domains or by introducing into a nucleic acid molecule or in a protein systematically mutations and assaying for those mutations which will lead to an increased specific activity or an increased activity per volume, in particular per cell.

**[0067.0.0.0]** It is therefore advantageously to express in an organism a nucleic acid molecule of the invention or the nucleic acid molecule used in the method of the invention or a polypeptide of the invention or the polypeptide used in the method of the invention derived from a evolutionary distantly related organism, as e.g. using a prokaryotic gene in an eukaryotic host, as in these cases the regulation mechanism of the host cell may not weaken the activity (cellular or specific) of the gene or its expression product

**[0068.0.0.0]** The mutation is introduced in such a way that the production of the amino acids is not adversely affected.

**[0069.0.0.0]** Less influence on the regulation of a gene or its gene product is understood as meaning a reduced regulation of the enzymatic activity leading to an increased specific or cellular activity of the gene or its product. An increase of the enzymatic activity is understood as meaning an enzymatic activity, which is increased by at least 10%, advantageously at least 20, 30 or 40%, especially advantageously by at least 50, 60 or 70% in comparison with the starting organism. This leads to an increased productivity of the desired respective fine chemical(s).

**[0070.0.0.0]** Owing to the introduction of a gene or a plurality of genes conferring the expression of the nucleic acid molecule of the invention or the nucleic acid molecule used in the method of the invention or the polypeptide of the invention or the polypeptide used in the method of the invention as described below, for example the nucleic acid construct mentioned below, into an organism alone or in combination with other genes, it is possible not only to increase the biosynthetic flux towards the end product, but also to increase, modify or create de *novo* an advantageous, preferably novel metabolites composition in the organism, e.g. an advantageous amino acid composition comprising a higher content of (from a viewpoint of nutrional physiology limited) respective fine chemicals, in particular amino acids, likewise the fine chemical.

**[0071.0.0.0]** Preferably the composition further comprises higher amounts of metabolites positively affecting or lower amounts of metabolites negatively affecting the nutrition or health of animals or humans provided with said compositions or organisms of the invention or parts thereof. Likewise, the number or activity of further genes which are required for the import or export of nutrients or metabolites, including amino acids or its precursors, required for the cell's biosynthesis of amino acids may be increased so that the concentration of necessary or relevant precursors, cofactors or intermediates within the cell(s) or within the corresponding storage compartments is increased. Owing to the increased or novel generated activity of the polypeptide of the invention or the polypeptide used in the method of the invention or owing to the increased number of nucleic acid sequences of the invention and/or to the modulation of further genes which are involved in the biosynthesis of the amino acids, e.g. by increasing the activity of enzymes synthesizing precursors or by destroying the activity of one or more genes which are involved in the breakdown of the amino acids, it is possible to increase the yield, production and/or production efficiency of amino acids in the host organism, such as the plants or the microorganisms.

**[0072.0.0.0]** By influencing the metabolism thus, it is possible to produce, in the process according to the invention, further advantageous sulfur-containing compounds, which contain at least one sulfur atom bound covalently. Examples of such compounds are, in addition to methionine, homocysteine, S-adenosylmethionine, cysteine, advantageously methionine and S-adenosylmethionine.

**[0073.0.0.0]** Accordingly, in one embodiment, the process according to the invention relates to a process which comprises:
a) providing a non-human organism, preferably a microorganism, a non-human animal, a plant or animal cell, a plant or animal tissue or a plant;
b) increasing an activity of a polypeptide of the invention or the polypeptide used in the method of the invention or a homolog thereof, e.g. as indicated in Table II, application no. 0, columns 5 or 7, or of a polypeptide being encoded by the nucleic acid molecule of the present invention and described below, i.e. conferring an increase of the respective fine chemical in the organism, preferably in a microorganism, a non-human animal, a plant or animal cell, a plant or animal tissue or a plant,
c) growing the organism, preferably a microorganism, a non-human animal, a plant or animal cell, a plant or animal tissue or a plant, under conditions which permit the production of the respective fine chemical in the organism, preferably the microorganism, the plant cell, the plant tissue or the plant; and
d) if desired, recovering, optionally isolating, the free and/or bound the respective fine chemical and , optionally further free and/or bound amino acids synthesized by the organism, the microorganism, the non-human animal, the plant or animal cell, the plant or animal tissue or the plant.

**[0074.0.0.0]** The organism, in particular the microorganism, non-human animal, the plant or animal cell, the plant or animal tissue or the plant is advantageously grown in such a way that it is not only possible to recover, if desired isolate the free or bound the respective fine chemical or the free and bound the fine chemical but as option it is also possible to produce, recover and, if desired isolate, other free or/and bound amino acids, in particular lysine. Galili et al., Transgenic Res., 200, 9, 2, 137-144 describes that the heterologous expression of a bacterial gene for the amino acid biosynthesis confers the increase of free as well as of protein-bound amino acids.

**[0075.0.0.0]** After the above-described increasing (which as defined above also encompasses the generating of an activity in an organism, i.e. a de *novo* activity), for example after the introduction and the expression of the nucleic acid molecules of the invention or described in the methods or processes according to the invention, the organism according to the invention, advantageously, a microorganism, a non-human animal, a plant, plant or animal tissue or plant or animal cell, is grown and subsequently harvested.

**[0076.0.0.0]** Suitable organisms or host organisms (transgenic organism) for the nucleic acid molecule used according to the invention and for the inventive process, the nucleic acid construct or the vector (both as described below) are, in principle, all organisms which are capable of synthesizing the respective fine chemical, and which are suitable for the activation, introduction or stimulation genes. Examples which may be mentioned are plants, microorganisms such as fungi, bacteria, yeasts, alga or diatom, transgenic or obtained by site directed mutagenesis or random mutagenesis combined with specific selection procedures. Preferred organisms are those which are naturally capable of synthesizing the respective fine chemical in substantial amounts, like fungi, yeasts, bactria or plants. In principle, transgenic animals, for example Caenorhabditis elegans, are also suitable as host organisms.

**[0077.0.0.0]** In the event that the transgenic organism is a microorganism, such as a eukaryotic organism, for example a fungus, an alga, diatom or a yeast in particular a fungus, alga, diatom or yeast selected from the families Chaetomiaceae, Choanephoraceae, Cryptococcaceae, Cunninghamellaceae, Demetiaceae, Moniliaceae, Mortierellaceae, Mucoraceae, Pythiaceae, Sacharomycetaceae, Saprolegniaceae, Schizosacharomycetaceae, Sodariaceae, Sporobolomycetaceae Tuberculariaceae, Adelotheciaceae, Dinophyceae, Ditrichaceae or Prasinophyceae, or a prokaryotic organism, for example a bacterium or blue alga, in particular a bacterium from the families Actinomycetaceae, Bacillaceae, Brevibacteriaceae, Corynebacteriaceae, Enterobacteriacae, Gordoniaceae, Nocardiaceae, Micrococcaceae, Mycobacteriaceae, Pseudomonaceae, Rhizobiaceae or Streptomycetaceae, this microorganism is grown on a solid or in a liquid medium which is known to the skilled worker and suits the organism. After the growing phase, the organisms can be harvested.

**[0078.0.0.0]** The microorganisms or the recovered, and if desired isolated, respective fine chemical can then be processed further directly into foodstuffs or animal feeds or for other applications, for example according to the disclosures made in EP-B-0 533 039 or EP-A-0 615 693, which are expressly incorporated herein by reference. The fermentation broth or fermentation products can be purified in the customary manner by extraction and precipitation or via ion exchangers and other methods known to the person skilled in the art and described herein below. Products of these different work-up procedures are amino acids or amino acid compositions which still comprise fermentation broth and cell components in different amounts, advantageously in the range of from 0 to 99% by weight, preferably below 80% by weight, especially preferably between below 50% by weight.

**[0079.0.0.0]** Preferred microorganisms are selected from the group consisting of Chaetomiaceae such as the genera Chaetomium e.g. the species *Chaetomidium fimeti;* Choanephoraceae such as the genera Blakeslea, Choanephora e.g. the species *Blakeslea trispora, Choanephora cucurbitarum or Choanephora infundibulifera* var. *cucurbitarum;* Cryptococcaceae such as the genera Candida, Crytococcus, Rhodotorula, Torulopsis e.g. the species *Candida albicans, Candida albomarginata, Candida antarctica, Candida bacarum, Candida bogoriensis, Candida boidinii, Candida bovina, Candida brumptii, Candida cacaoi, Candida cariosilignicola, Candida catenulata, Candida chalmersii, Candida ciferrii, Candida cylindracea, Candida edax, Candida ernobii, Candida famata, Candida freyschussii, Candida friedrichii, Candida glabrata, Candida guilliermondii, Candida haemulonii, Candida humicola, Candida inconspicua, Candida ingens, Candida intermedia, Candida kefyr, Candida krusei, Candida lactiscondensi, Candida lambica, Candida lipolytica, Candida lusitaniae, Candida macedoniensis, Candida magnoliae, Candida membranaefaciens, Candida mesenterica, Candida multigemmis, Candida mycoderma, Candida nemodendra, Candida nitratophila, Candida norvegensis, Candida norvegica, Candida parapsilosis, Candida pelliculosa, Candida peltata, Candida pini, Candida pseudotropicalis, Candida pulcherrima, Candida punicea, Candida pustula, Candida ravautii, Candida reukaufii, Candida rugosa, Candida sake, Candida silvicola, Candida solani, Candida sp., Candida spandovensis, Candida succiphila, Candida tropicalis, Candida utilis, Candida valida, Candida versatilis, Candida vini, Candida zeylanoides, Cryptococcus albidus, Cryptococcus curvatus, Cryptococcus flavus, Cryptococcus humicola, Cryptococcus hungaricus, Cryptococcus kuetzingii, Cryptococcus laurentii, Cryptococcus macerans, Cryptococcus neoformans, Cryptococcus terreus, Cryptococcus uniguttulatus, Rhodotorula acheniorum, Rhodotorula bacarum, Rhodotorula bogoriensis, Rhodotorula flava, Rhodotorula glutinis, Rhodotorula macerans, Rhodotorula minuta, Rhodotorula mucilaginosa, Rhodotorula pilimanae, Rhodotorula pustula, Rhodotorula rubra, Rhodotorula tokyoensis, Torulopsis colliculosa, Torulopsis dattila or Torulopsis neoformans;* Cunninghamellaceae such as the genera Cunninghamella e.g. the species *Cunninghamella blakesleeana, Cunninghamella echinulata, Cunninghamella echinulata* var. *elegans, Cunninghamella elegans or Cunninghamella homothallica;* Demetiaceae such as the genera Alternaria, Bipolaris, Cercospora, Chalara, Cladosporium, Curvularia, Exophilia, Helicosporium, Helminthosporium, Orbimyces, Philalophora, Pithomyces, Spilocaea, Thielaviopsis, Wangiella e.g. the species *Curvularia affinis, Curvularia clavata, Curvularia fallax, Curvularia inaequalis, Curvularia indica, Curvularia lunata, Curvularia pallescens, Curvularia verruculosa or Helminothosporium sp.;* Moniliaceae such as the genera Arthrobotrys, Aspergillus, Epidermophyton, Geotrichum, Gliocladium, Histoplasma, Microsporum, Monilia, Oedocephalum, Oidium, Penicillium, Trichoderma, Trichophyton, Thrichoteclum, Verticillium e.g. the species *Aspergillus aculeatus, Aspergillus albus, Aspergillus alliaceus, Aspergillus asperescens , Aspergillus awamori, Aspergillus candidus, Aspergillus carbonarius, Aspergillus* carneus, *Aspergillus chevalieri, Aspergillus chevalieri* var. *intermedius, Aspergillus clavatus, Aspergillus ficuum, Aspergillus flavipes, Aspergillus flavus, Aspergillus foetidus, Aspergillus fumigatus, Aspergillus giganteus, Aspergillus humicola, Aspergillus intermedius, Aspergillus japonicus, Aspergillus nidulans, Aspergillus niger, Aspergillus niveus, Aspergillus ochraceus, Aspergillus oryzae, Aspergillus ostianus, Aspergillus parasiticus, Aspergillus parasiticus var. globosus, Aspergillus penicillioides, Aspergillus phoenicis, Aspergillus rugulosus, Aspergillus sclerotiorum, Aspergillus sojae* var. *gymnosardae, Aspergillus sydowi, Aspergillus tamarii, Aspergillus terreus, Aspergillus terricola, Aspergillus toxicarius, Aspergillus unguis, Aspergillus ustus, Aspergillus versicolor, Aspergillus vitricolae, Aspergillus wentii,·Penicillium adametzi, ·Penicillium albicans, Penicillium arabicum, Penicillium arenicola, Penicillium argillaceum, Penicillium arvense, Penicillium asperosporum,·Penicillium aurantiogriseum,·Penicillium avellaneum,·Penicillium* baarnense,*·Penicillium bacillisporum,·Penicillium brasilianum,·Penicillium brevicompactum,·Penicillium camemberti,·Penicillium canadense,·Penicillium canescens,·Penicillium caperatum,·Penicillium capsulatum,·Penicillium caseicolum,·Penicillium chrysogenum,·Penicillium citreonigrum,·Penicillium citrinum,·Penicillium claviforme,·Penicillium* commune,·*Penicillium corylophilum,·Penicillium corymbiferum,·Penicillium crustosum,·Penicillium cyclopium,·Penicillium daleae,·Penicillium decumbens,·Penicillium dierckxii, ·Penicillium digitatum,·Penicillium digitatum var. latum,·Penicillium divaricatum,·Penicillium diversum,·Penicillium duclauxii,·Penicillium echinosporum,·Penicillium expansum,·Penicillium fellutanum,·Penicillium frequentans,·Penicillium funiculosum,·Penicillium glabrum,·Penicillium gladioli,·Penicillium griseofulvum,·Penicillium hirsutum,·Penicillium hispanicum,·Penicillium islandicum,·Penicillium italicum,·Penicillium italicum var. avellaneum,·Penicillium janczewskii,·Penicillium janthinellum,·Penicillium japonicum,·Penicillium lavendulum,·Penicillium lilacinum,·Penicillium lividum, ·Penicillium martensii, Penicillium megasporum,·Penicillium miczynskii,·Penicillium nalgiovense,·Penicillium nigricans,·Penicillium notatum,·Penicillium ochrochloron,·Penicillium odoratum,*·*Penicillium oxalicum,·Penicillium paraherquei,*·*Penicillium patulum,·Penicillium pinophilum,·Penicillium piscarium, Penicillium pseudostromaticum,·Penicillium puberulum,·Penicillium purpurogenum, ·Penicillium raciborskii,·Penicillium roqueforti,·Penicillium rotundum,·Penicillium rubrum, Penicillium sacculum,·Penicillium simplicissimum, Penicillium sp., Penicillium spinulosum, Penicillium steckii, Penicillium stoloniferum, Penicillium striatisporum, Penicillium striatum, Penicillium tardum, Penicillium thomii, Penicillium turbatum, Penicillium variabile, Penicillium vermiculatum, Penicillium vermoesenii, Penicillium verrucosum, Penicillium verrucosum var. corymbiferum, Penicillium verrucosum var. cyclopium, Penicillium verruculosum, Penicillium vinaceum, Penicillium violaceum, Penicillium viridicatum, Penicillium vulpinum, Trichoderma hamatum, Trichoderma harzianum, Trichoderma koningii, Trichoderma longibrachiatum, Trichoderma polysporum, Trichoderma reesei, Trichoderma virens or Trichoderma viride;* Mortierellaceae such as the genera Mortierella e.g. the species *Mortierella isabellina, Mortierella polycephala , Mortierella ramanniana , Mortierella vinacea or Mortierella zonata;* Mucoraceae such as the genera Actinomucor, Mucor, Phycomyces, Rhizopus, Zygorhynchus e.g. the *species Mucor amphibiorum, Mucor circinelloides f. circinelloides, Mucor circinelloides var. griseocyanus, Mucor flavus, Mucor fuscus, Mucor griseocyanus, Mucor heterosporus, Mucor hiemalis, Mucor hiemalis f. hiemalis, Mucor inaequisporus, Mucor indicus, Mucor javanicus, Mucor mucedo, Mucor mucilagineus, Mucor piriformis, Mucor plasmaticus, Mucor plumbeus, Mucor racemosus, Mucor racemosus f. racemosus, Mucor racemosus f. sphaerosporus, Mucor rouxianus, Mucor rouxii, Mucor sinensis, Mucor sp., Mucor spinosus, Mucor tuberculisporus, Mucor variisporus, Mucor variosporus, Mucor wosnessenskii, Phycomyces blakesleeanus, Rhizopus achlamydosporus, Rhizopus arrhizus, Rhizopus chinensis, Rhizopus delemar, Rhizopus formosaensis, Rhizopus japonicus, Rhizopus javanicus, Rhizopus microsporus, Rhizopus microsporus var. chinensis, Rhizopus microsporus var. oligosporus, Rhizopus microsporus var. rhizopodiformis, Rhizopus nigricans, Rhizopus niveus, Rhizopus oligosporus, Rhizopus oryzae, Rhizopus pygmaeus, Rhizopus rhizopodiformis, Rhizopus semarangensis, Rhizopus sontii, Rhizopus stolonifer, Rhizopus thermosus, Rhizopus tonkinensis, Rhizopus tritici or Rhizopus usamii;* Pythiaceae such as the genera Phytium, Phytophthora e.g. the species *Pythium debaryanum, Pythium intermedium, Pythium irregulare, Pythium megalacanthum, Pythium paroecandrum, Pythium sylvaticum, Pythium ultimum, Phytophthora cactorum, Phytophthora cinnamomi, Phytophthora citricola, Phytophthora citrophthora, Phytophthora cryptogea, Phytophthora drechsleri, Phytophthora erythroseptica, Phytophthora lateralis, Phytophthora megasperma, Phytophthora nicotianae, Phytophthora nicotianae var. parasitica, Phytophthora palmivora, Phytophthora parasitica or Phytophthora syringae;* Sacharomycetaceae such as the genera Hansenula, Pichia, Saccharomyces, Saccharomycodes, Yarrowia e.g. the species *Hansenula anomala, Hansenula californica, Hansenula canadensis, Hansenula capsulata, Hansenula ciferrii, Hansenula glucozyma, Hansenula henricii, Hansenula holstii, Hansenula minuta, Hansenula nonfermentans, Hansenula philodendri, Hansenula polymorpha, Hansenula saturnus, Hansenula subpelliculosa, Hansenula wickerhamii, Hansenula wingei, Pichia alcoholophila, Pichia angusta, Pichia anomala, Pichia bispora, Pichia burtonii, Pichia canadensis, Pichia capsulata, Pichia carsonii, Pichia cellobiosa, Pichia ciferrii, Pichia farinosa, Pichia fermentans, Pichia finlandica, Pichia glucozyma, Pichia guilliermondii, Pichia haplophila, Pichia henricii, Pichia holstii, Pichia jadinii, Pichia lindnerii, Pichia membranaefaciens, Pichia methanolica, Pichia minuta var. minuta, Pichia minuta var. nonfermentans, Pichia norvegensis, Pichia ohmeri, Pichia pastoris, Pichia philodendri, Pichia pini, Pichia polymorpha, Pichia quercuum, Pichia rhodanensis, Pichia sargentensis, Pichia stipitis, Pichia strasburgensis, Pichia subpelliculosa, Pichia toletana, Pichia trehalophila, Pichia vini, Pichia xylosa, Saccharomyces aceti, Saccharomyces bailii, Saccharomyces bayanus, Saccharomyces bisporus, Saccharomyces capensis, Saccharomyces carlsbergensis, Saccharomyces cerevisiae, Saccharomyces cerevisiae var. ellipsoideus, Saccharomyces chevalieri, Saccharomyces delbrueckii, Saccharomyces diastaticus, Saccharomyces drosophilarum, Saccharomyces elegans, Saccharomyces ellipsoideus, Saccharomyces fermentati, Saccharomyces florentinus, Saccharomyces fragilis,* Saccharomyces *heterogenicus,* Saccharomyces *hienipiensis,* Saccharomyces *inusitatus, Saccharomyces italicus, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum, Saccharomycodes ludwigii or Yarrowia lipolytica;* Saprolegniaceae such as the genera Saprolegnia e.g. the species *Saprolegnia ferax;* Schizosacharomycetaceae such as the genera Schizosaccharomyces e.g. the species Schizosaccharomyces japonicus *var. japonicus, Schizosaccharomyces japonicus var. versatilis, Schizosaccharomyces malidevorans, Schizosaccharomyces octosporus, Schizosaccharomyces pombe var. malidevorans or Schizosaccharomyces pombe var. pombe;* Sodariaceae such as the genera Neurospora, Sordaria e.g. the species *Neurospora africana*, *Neurospora crassa*, Neurospora *intermedia, Neurospora sitophila, Neurospora tetrasperma, Sordaria fimicola or Sordaria macrospora;* Tuberculariaceae such as the genera Epicoccum, Fusarium, Myrothecium, Sphacelia, Starkeyomyces, Tubercularia e.g. the species *Fusarium acuminatum, Fusarium anthophilum, Fusarium aquaeductuum, Fusarium aquaeductuum var. medium, Fusarium avenaceum*, *Fusarium buharicum, Fusarium camptoceras*, *Fusarium cerealis*, *Fusarium chlamydosporum, Fusarium ciliatum, Fusarium coccophilum, Fusarium coeruleum, Fusarium concolor*, *Fusarium crookwellense*, *Fusarium culmorum, Fusarium dimerum, Fusarium diversisporum, Fusarium equiseti, Fusarium equiseti var. bullatum, Fusarium eumartii, Fusarium flocciferum, Fusarium fujikuroi, Fusarium graminearum, Fusarium graminum, Fusarium heterosporum,Fusarium incarnatum, Fusarium inflexum, Fusarium javanicum, Fusarium lateritium, Fusarium lateritium var. majus, Fusarium longipes, Fusarium melanochlorum, Fusarium merismoides, Fusarium merismoides var. chlamydosporale, Fusarium moniliforme, Fusarium moniliforme var. anthophilum, Fusarium moniliforme var. subglutinans, Fusarium nivale, Fusarium nivale var. majus, Fusarium oxysporum, Fusarium oxysporum f. sp. aechmeae, Fusarium oxysporum f. sp. cepae*, *Fusarium oxysporum f. sp. conglutinans, Fusarium oxysporum f. sp. cucumerinum, Fusarium oxysporum f. sp. cyclaminis, Fusarium oxysporum f. sp. dianthi, Fusarium oxysporum f. sp. lycopersici, Fusarium oxysporum f. sp. melonis, Fusarium oxysporum f. sp. passiflorae, Fusarium oxysporum f. sp. pisi, Fusarium oxysporum f. sp. tracheiphilum, Fusarium oxysporum f. sp. tuberosi, Fusarium oxysporum f. sp. tulipae, Fusarium oxysporum f. sp. vasinfectum, Fusarium pallidoroseum, Fusarium* poae, *Fusarium proliferatum, Fusarium proliferatum var. minus, Fusarium redolens, Fusarium redolens f. sp. dianthi, Fusarium reticulatum, Fusarium roseum, Fusarium sacchari var. elongatum, Fusarium sambucinum, Fusarium sambucinum var. coeruleum, Fusarium semitectum, Fusarium semitectum var. majus, Fusarium solani, Fusarium solani f. sp. pisi, Fusarium sporotrichioides, Fusarium sporotrichioides var. minus, Fusarium sublunatum, Fusarium succisae*, *Fusarium sulphureum, Fusarium tabacinum, Fusarium tricinctum, Fusarium udum, Fusarium ventricosum, Fusarium verticillioides, Fusarium xylarioides or Fusarium zonatum;* Sporobolomycetaceae such as the genera Bullera, Sporobolomyces, Itersonilia e.g. the species *Sporobolomyces holsaticus, Sporobolomyces odorus, Sporobolomyces puniceus, Sporobolomyces salmonicolor, Sporobolomyces singularis or Sporobolomyces tsugae;* Adelotheciaceae such as the genera e.g. the species *Physcomitrella patens;* Dinophyceae such as the genera Crypthecodinium, Phaeodactylum e.g. the species *Crypthecodinium cohnii or Phaeodactylum tricornutum;* Ditrichaceae such as the genera Ceratodon, Pleuridium, Astomiopsis, Ditrichum, Philibertiella, Ceratodon, Distichium, Skottsbergia e.g. the species *Ceratodon antarcticus, Ceratodon purpureus, Ceratodon purpureus ssp. convolutes or Ceratodon purpureus ssp. stenocarpus;* Prasinophyceae such as the genera Nephroselmis, Prasinococcus, Scherffelia, Tetraselmis, Mantoniella, Ostreococcus e.g. the species *Nephroselmis olivacea, Prasinococcus capsulatus, Scherffelia dubia, Tetraselmis chui, Tetraselmis suecica, Mantoniella squamata or Ostreococcus tauri;* Actinomycetaceae such as the genera Actinomyces, Actinobaculum, Arcanobacterium, Mobiluncus e.g. the species *Actinomyces bernardiae, Actinomyces bovis, Actinomyces bowdenii, Actinomyces canis, Actinomyces cardiffensis, Actinomyces catuli, Actinomyces coleocanis, Actinomyces denticolens, Actinomyces europaeus, Actinomyces funkei, Actinomyces georgiae, Actinomyces gerencseriae, Actinomyces hordeovulneris, Actinomyces howellii, Actinomyces humiferus, Actinomyces hyovaginalis, Actinomyces israelii, Actinomyces marimammalium, Actinomyces meyeri, Actinomyces naeslundii, Actinomyces nasicola, Actinomyces neuii* subsp. *anitratus, Actinomyces neuii* subsp. *neuii, Actinomyces odontolyticus, Actinomyces oricola, Actinomyces pyogenes, Actinomyces radicidentis, Actinomyces radingae, Actinomyces slackii, Actinomyces suimastitidis, Actinomyces suis, Actinomyces turicensis, Actinomyces urogenitalis, Actinomyces vaccimaxillae, Actinomyces viscosus, Actinobaculum schaalii, Actinobaculum suis, Actinobaculum urinale, Arcanobacterium bernardiae, Arcanobacterium haemolyticum, Arcanobacterium hippocoleae, Arcanobacterium phocae, Arcanobacterium pluranimalium, Arcanobacterium pyogenes, Mobiluncus curtisii* subsp. *curtisii, Mobiluncus curtisii* subsp. *holmesii or Mobiluncus mulieris;* Bacillaceae such as the genera Amphibacillus, Anoxybacillus, Bacillus, Exiguobacterium, Gracilibacillus, Holobacillus, Saccharococcus, Salibacillus, Virgibacillus e.g. the species *Amphibacillus fermentum, Amphibacillus tropicus, Amphibacillus xylanus, Anoxybacillus flavithermus, Anoxybacillus gonensis, Anoxybacillus pushchinoensis, Bacillus acidocaldarius, Bacillus acidoterrestris, Bacillus aeolius, Bacillus agaradhaerens, Bacillus agri, Bacillus alcalophilus, Bacillus alginolyticus, Bacillus alvei, Bacillus amyloliquefaciens, Bacillus amylolyticus, Bacillus aneurinilyticus, Bacillus aquimaris, Bacillus arseniciselenatis, Bacillus atrophaeus, Bacillus azotofixans, Bacillus azotoformans, Bacillus badius, Bacillus barbaricus, Bacillus benzoevorans, Bacillus borstelensis, Bacillus brevis, Bacillus carboniphilus, Bacillus centrosporus, Bacillus cereus, Bacillus chitinolyticus, Bacillus chondroitinus, Bacillus choshinensis, Bacillus circulans, Bacillus clarkii, Bacillus clausii, Bacillus coagulans, Bacillus cohnii, Bacillus curdlanolyticus, Bacillus cycloheptanicus, Bacillus decolorationis, Bacillus dipsosauri, Bacillus edaphicus, Bacillus ehimensis, Bacillus endophyticus, Bacillus fastidiosus, Bacillus firmus, Bacillus flexus, Bacillus formosus, Bacillus fumarioli, Bacillus funiculus, Bacillus fusiformis, Bacillus sphaericus subsp. fusiformis, Bacillus galactophilus, Bacillus globisporus, Bacillus globisporus subsp. marinus, Bacillus glucanolyticus, Bacillus gordonae, Bacillus halmapalus, Bacillus haloalkaliphilus, Bacillus halodenitrificans, Bacillus halodurans, Bacillus halophilus, Bacillus horikoshii, Bacillus horti, Bacillus infernos, Bacillus insolitus, Bacillus jeotgali, Bacillus kaustophilus, Bacillus kobensis, Bacillus krulwichiae, Bacillus laevolacticus, Bacillus larvae, Bacillus laterosporus, Bacillus lautus, Bacillus lentimorbus, Bacillus lentus, Bacillus licheniformis, Bacillus luciferensis, Bacillus macerans, Bacillus macquariensis, Bacillus marinus, Bacillus marisflavi, Bacillus marismortui, Bacillus megaterium, Bacillus methanolicus, Bacillus migulanus, Bacillus mojavensis, Bacillus mucilaginosus, Bacillus mycoides, Bacillus naganoensis, Bacillus nealsonii, Bacillus neidei, Bacillus niacini, Bacillus okuhidensis, Bacillus oleronius, Bacillus pabuli, Bacillus pallidus, Bacillus pantothenticus, Bacillus parabrevis, Bacillus pasteurii, Bacillus peoriae, Bacillus polymyxa, Bacillus popilliae, Bacillus pseudalcaliphilus, Bacillus pseudofirmus, Bacillus pseudomycoides, Bacillus psychrodurans, Bacillus psychrophilus, Bacillus psychrosaccharolyticus, Bacillus psychrotolerans, Bacillus pulvifaciens, Bacillus pumilus, Bacillus pycnus, Bacillus reuszeri, Bacillus salexigens, Bacillus schlegelii, Bacillus selenitireducens, Bacillus silvestris, Bacillus simplex, Bacillus siralis, Bacillus smithii, Bacillus sonorensis, Bacillus sphaericus, Bacillus sporothermodurans, Bacillus stearothermophilus , Bacillus subterraneus, Bacillus subtilis subsp. spizizenii, Bacillus subtilis subsp. subtilis, Bacillus thermantarcticus, Bacillus thermoaerophilus, Bacillus thermoamylovorans, Bacillus thermoantarcticus, Bacillus thermocatenulatus, Bacillus thermocloacae, Bacillus thermodenitrificans, Bacillus thermoglucosidasius, Bacillus thermoleovorans, Bacillus thermoruber, Bacillus thermosphaericus, Bacillus thiaminolyticus, Bacillus thuringiensis, Bacillus tusciae, Bacillus validus, Bacillus vallismortis, Bacillus vedderi, Bacillus vulcani, Bacillus weihenstephanensis, Exiguobacterium acetylicum, Exiguobacterium antarcticum, Exiguobacterium aurantiacum, Exiguobacterium undae, Gracilibacillus dipsosauri, Gracilibacillus halotolerans, Halobacillus halophilus, Halobacillus karajensis, Halobacillus litoralis, Halobacillus salinus, Halobacillus trueperi, Saccharococcus caldoxylosilyticus, Saccharococcus thermophilus, Salibacillus marismortui, Salibacillus salexigens, Virgibacillus carmonensis, Virgibacillus marismortui, Virgibacillus necropolis, Virgibacillus pantothenticus, Virgibacillus picturae, Virgibacillus proomii or Virgibacillus salexigens,* Brevibacteriaceae such as the genera Brevibacterium e.g. the species *Brevibacterium acetylicum, Brevibacterium albidum, Brevibacterium ammoniagenes, Brevibacterium avium, Brevibacterium casei, Brevibacterium citreum, Brevibacterium divaricatum, Brevibacterium epidermidis, Brevibacterium fermentans, Brevibacterium frigoritolerans, Brevibacterium halotolerans, Brevibacterium imperiale, Brevibacterium incertum, Brevibacterium iodinum, Brevibacterium linens, Brevibacterium liquefaciens, Brevibacterium lutescens, Brevibacterium luteum, Brevibacterium lyticum, Brevibacterium mcbrellneri, Brevibacterium otitidis, Brevibacterium oxydans, Brevibacterium paucivorans, Brevibacterium protophormiae, Brevibacterium pusillum, Brevibacterium saperdae, Brevibacterium stationis, Brevibacterium testaceum or Brevibacterium vitaeruminis;* Corynebacteriaceae such as the genera Corynebacterium e.g. the species *Corynebacterium accolens*, *Corynebacterium afermentans* subsp. *afermentans, Corynebacterium afermentans* subsp. *lipophilum, Corynebacterium* ammoniagenes, *Corynebacterium amycolatum, Corynebacterium appendicis, Corynebacterium aquilae, Corynebacterium argentoratense, Corynebacterium atypicum, Corynebacterium aurimucosum, Corynebacterium auris, Corynebacterium auriscanis, Corynebacterium betae, Corynebacterium beticola, Corynebacterium bovis, Corynebacterium callunae, Corynebacterium camporealensis*, *Corynebacterium capitovis, Corynebacterium casei, Corynebacterium confusum, Corynebacterium coyleae*, *Corynebacterium cystitidis, Corynebacterium durum, Corynebacterium efficiens, Corynebacterium equi, Corynebacterium falsenii, Corynebacterium fascians*, *Corynebacterium felinum, Corynebacterium flaccumfaciens*, *Corynebacterium flavescens*, *Corynebacterium freneyi, Corynebacterium glaucum, Corynebacterium glucuronolyticum, Corynebacterium glutamicum, Corynebacterium hoagii, Corynebacterium ilicis, Corynebacterium imitans, Corynebacterium insidiosum, Corynebacterium iranicum, Corynebacterium jeikeium, Corynebacterium kroppenstedtii, Corynebacterium kutscheri, Corynebacterium lilium, Corynebacterium lipophiloflavum, Corynebacterium macginleyi, Corynebacterium mastitidis, Corynebacterium matruchotii, Corynebacterium michiganense, Corynebacterium michiganense* subsp. *tessellarius, Corynebacterium minutissimum, Corynebacterium mooreparkense, Corynebacterium mucifaciens, Corynebacterium mycetoides, Corynebacterium nebraskense, Corynebacterium oortii, Corynebacterium paurometabolum, Corynebacterium phocae, Corynebacterium pilosum, Corynebacterium poinsettiae, Corynebacterium propinquum, Corynebacterium pseudodiphtheriticum, Corynebacterium pseudotuberculosis, Corynebacterium pyogenes, Corynebacterium rathayi, Corynebacterium renale, Corynebacterium riegelii, Corynebacterium seminale, Corynebacterium sepedonicum, Corynebacterium simulans, Corynebacterium singulare, Corynebacterium sphenisci, Corynebacterium spheniscorum, Corynebacterium striatum, Corynebacterium suicordis, Corynebacterium sundsvallense, Corynebacterium terpenotabidum, Corynebacterium testudinoris, Corynebacterium thomssenii, Corynebacterium tritici, Corynebacterium ulcerans, Corynebacterium urealyticum, Corynebacterium variabile, Corynebacterium vitaeruminis or Corynebacterium xerosis;* Enterobacteriacae such as the genera Alterococcus, Arsenophonus, Brenneria, Buchnera, Budvicia, Buttiauxella, Calymmatobacterium, Cedecea, Citrobacter, Edwardsiella, Enterobacter, Erwinia, Escherichia, Ewingella, Hafnia, Klebsiella, Kluyvera, Leclercia, Leminorella, Moellerella, Morganella, Obesumbacterium, Pantoea, Pectobacterium, Photorhabdus, Plesiomonas, Pragia, Proteus, Providencia, Rahnella, Saccharobacter, Salmonella, Shigella, Serratia, Sodalis, Tatumella, Trabulsiella, Wigglesworthia, Xenorhabdus,Yersinia and Yokenella e.g. the species *Arsenophonus nasoniae, Brenneria alni, Brenneria nigrifluens, Brenneria quercina, Brenneria rubrifaciens, Brenneria salicis, Budvicia aquatica, Buttiauxella agrestis, Buttiauxella brennerae, Buttiauxella ferragutiae, Buttiauxella gaviniae, Buttiauxella izardii, Buttiauxella noackiae, Buttiauxella warmboldiae, Cedecea davisae, Cedecea lapagei, Cedecea neteri, Citrobacter amalonaticus, Citrobacter diversus, Citrobacter freundii, Citrobacter genomospecies, Citrobacter gillenii, Citrobacter intermedium, Citrobacter koseri, Citrobacter murliniae, Citrobacter sp., Edwardsiella hoshinae, Edwardsiella ictaluri, Edwardsiella tarda, Erwinia alni, Erwinia amylovora, Erwinia ananatis, Erwinia aphidicola, Erwinia billingiae, Erwinia cacticida, Erwinia cancerogena, Erwinia carnegieana, Erwinia carotovora* subsp. *atroseptica, Erwinia carotovora* subsp. *betavasculorum, Erwinia carotovora* subsp. *odorifera, Erwinia carotovora* subsp. *wasabiae, Erwinia chrysanthemi, Erwinia cypripedii, Erwinia dissolvens, Erwinia herbicola, Erwinia mallotivora, Erwinia milletiae, Erwinia nigrifluens, Erwinia nimipressuralis, Erwinia persicina, Erwinia psidii, Erwinia pyrifoliae, Erwinia quercina, Erwinia rhapontici, Erwinia rubrifaciens, Erwinia salicis, Erwinia stewartii, Erwinia tracheiphila, Erwinia uredovora, Escherichia adecarboxylata, Escherichia anindolica, Escherichia aurescens, Escherichia blattae, Escherichia coli, Escherichia coli var. communior, Escherichia coli-mutabile, Escherichia fergusonii, Escherichia hermannii, Escherichia sp., Escherichia vulneris, Ewingella americana, Hafnia alvei, Klebsiella aerogenes, Klebsiella edwardsii subsp. atlantae, Klebsiella ornithinolytica, Klebsiella oxytoca, Klebsiella planticola, Klebsiella pneumoniae, Klebsiella pneumoniae subsp. pneumoniae, Klebsiella sp., Klebsiella terrigena, Klebsiella trevisanii, Kluyvera ascorbata, Kluyvera citrophila, Kluyvera cochleae, Kluyvera cryocrescens, Kluyvera georgiana, Kluyvera noncitrophila, Kluyvera sp., Leclercia adecarboxylata, Leminorella grimontii, Leminorella richardii, Moellerella wisconsensis, Morganella morganii, Morganella morganii subsp. morganii, Morganella morganii subsp. sibonii, Obesumbaterium proteus, Pantoea agglomerans, Pantoea ananatis, Pantoea citrea, Pantoea dispersa, Pantoea punctata, Pantoea stewartii subsp. stewartii, Pantoea terrea, Pectobacterium atrosepticum, Pectobacterium carotovorum subsp. atrosepticum, Pectobacterium carotovorum subsp. carotovorum, Pectobacterium chrysanthemi, Pectobacterium cypripedii, Photorhabdus asymbiotica, Photorhabdus luminescens, Photorhabdus luminescens subsp. akhurstii, Photorhabdus luminescens subsp. laumondii, Photorhabdus luminescens subsp. luminescens, Photorhabdus sp., Photorhabdus temperata, Plesiomonas shigelloides, Pragia fontium, Proteus hauseri, Proteus ichthyosmius, Proteus inconstans, Proteus mirabilis, Proteus morganii, Proteus myxofaciens, Proteus penneri, Proteus rettgeri, Proteus shigelloides, Proteus vulgaris, Providencia alcalifaciens, Providencia friedericiana, Providencia heimbachae, Providencia rettgeri, Providencia rustigianii, Providencia stuartii, Rahnella aquatilis, Salmonella abony, Salmonella arizonae, Salmonella bongori, Salmonella choleraesuis subsp. arizonae, Salmonella choleraesuis subsp. bongori, Salmonella choleraesuis subsp. cholereasuis, Salmonella choleraesuis subsp. diarizonae, Salmonella choleraesuis subsp. houtenae, Salmonella choleraesuis subsp. indica, Salmonella choleraesuis subsp. salamae, Salmonella daressalaam, Salmonella enterica subsp. houtenae, Salmonella enterica subsp. salamae, Salmonella enteritidis, Salmonella gallinarum, Salmonella heidelberg, Salmonella panama, Salmonella senftenberg, Salmonella typhimurium, Serratia entomophila, Serratia ficaria, Serratia fonticola, Serratia grimesii, Serratia liquefaciens, Serratia marcescens, Serratia marcescens subsp. marcescens, Serratia marinorubra, Serratia odorifera, Serratia plymouthensis, Serratia plymuthica, Serratia proteamaculans, Serratia proteamaculans subsp. quinovora, Serratia quinivorans, Serratia rubidaea, Shigella boydii, Shigella flexneri, Shigella paradysenteriae, Shigella sonnei, Tatumella ptyseos, Xenorhabdus beddingii, Xenorhabdus bovienii, Xenorhabdus luminescens, Xenorhabdus nematophila, Xenorhabdus nematophila subsp. beddingii, Xenorhabdus nematophila subsp. bovienii, Xenorhabdus nematophila subsp. poinarii or Xenorhabdus poinarii;* Gordoniaceae such as the genera Gordonia, Skermania e.g. the species *Gordonia aichiensis, Gordonia alkanivorans, Gordonia amarae, Gordonia amicalis, Gordonia bronchialis, Gordonia desulfuricans, Gordonia hirsuta, Gordonia hydrophobica, Gordonia namibiensis, Gordonia nitida, Gordonia paraffinivorans, Gordonia polyisoprenivorans, Gordonia rhizosphera, Gordonia rubripertincta, Gordonia sihwensis, Gordonia sinesedis, Gordonia sputi, Gordonia* terrae or *Gordonia westfalica*; Micrococcaceae such as the genera Micrococcus, Arthrobacter, Kocuria, Nesterenkonia, Renibacterium, Rothia, Stomatococcus e.g. the species *Micrococcus agilis, Micrococcus antarcticus, Micrococcus halobius, Micrococcus kristinae, Micrococcus luteus, Micrococcus lylae, Micrococcus nishinomiyaensis, Micrococcus roseus, Micrococcus sedentarius, Micrococcus varians, Arthrobacter agilis, Arthrobacter albus, Arthrobacter atrocyaneus, Arthrobacter aurescens, Arthrobacter chlorophenolicus, Arthrobacter citreus, Arthrobacter creatinolyticus, Arthrobacter crystallopoietes, Arthrobacter cumminsii, Arthrobacter duodecadis, Arthrobacter flavescens, Arthrobacter flavus, Arthrobacter gandavensis, Arthrobacter globiformis, Arthrobacter histidinolovorans, Arthrobacter ilicis, Arthrobacter koreensis, Arthrobacter luteolus, Arthrobacter methylotrophus, Arthrobacter mysorens, Arthrobacter nasiphocae, Arthrobacter nicotianae, Arthrobacter nicotinovorans, Arthrobacter oxydans, Arthrobacter pascens, Arthrobacter picolinophilus, Arthrobacter polychromogenes, Arthrobacter protophormiae, Arthrobacter psychrolactophilus, Arthrobacter radiotolerans, Arthrobacter ramosus, Arthrobacter rhombi, Arthrobacter roseus, Arthrobacter siderocapsulatus, Arthrobacter simplex, Arthrobacter sulfonivorans, Arthrobacter sulfureus, Arthrobacter terregens, Arthrobacter tumescens, Arthrobacter uratoxydans, Arthrobacter ureafaciens, Arthrobacter variabilis, Arthrobacter viscosus, Arthrobacter woluwensis, Kocuria erythromyxa, Kocuria kristinae, Kocuria palustris, Kocuria polaris, Kocuria rhizophila, Kocuria rosea, Kocuria varians, Nesterenkonia halobia, Nesterenkonia lacusekhoensis, Renibacterium salmoninarum, Rothia amarae, Rothia dentocariosa, Rothia mucilaginosa, Rothia nasimurium or Stomatococcus mucilaginosus;* Mycobacteriaceae such as the genera Mycobacterium e.g. the species *Mycobacterium africanum, Mycobacterium agri, Mycobacterium aichiense, Mycobacterium alvei, Mycobacterium asiaticum, Mycobacterium aurum, Mycobacterium austroafricanum, Mycobacterium bohemicum, Mycobacterium botniense, Mycobacterium brumae, Mycobacterium chelonae* subsp. *abscessus, Mycobacterium chitae, Mycobacterium chlorophenolicum, Mycobacterium chubuense, Mycobacterium confluentis, Mycobacterium cookii, Mycobacterium diernhoferi, Mycobacterium doricum, Mycobacterium duvalii, Mycobacterium fallax, Mycobacterium farcinogenes, Mycobacterium flavescens, Mycobacterium frederiksbergense, Mycobacterium gadium, Mycobacterium gilvum, Mycobacterium gordonae, Mycobacterium hassiacum, Mycobacterium hiberniae, Mycobacterium hodleri, Mycobacterium holsaticum, Mycobacterium komossense, Mycobacterium lacus, Mycobacterium madagascariense, Mycobacterium mageritense, Mycobacterium montefiorense, Mycobacterium moriokaense, Mycobacterium murale, Mycobacterium neoaurum, Mycobacterium nonchromogenicum, Mycobacterium obuense, Mycobacterium palustre, Mycobacterium parafortuitum, Mycobacterium peregrinum, Mycobacterium phlei, Mycobacterium pinnipedii, Mycobacterium poriferae, Mycobacterium pulveris, Mycobacterium rhodesiae, Mycobacterium shottsii, Mycobacterium sphagni, Mycobacterium terrae, Mycobacterium thermoresistibile, Mycobacterium tokaiense, Mycobacterium triviale, Mycobacterium tusciae or Mycobacterium vanbaalenii;* Nocardiaceae such as the genera Nocardia, Rhodococcus e.g. the species *Nocardia abscessus, Nocardia africana, Nocardia amarae, Nocardia asteroides, Nocardia autotrophica, Nocardia beijingensis, Nocardia brasiliensis, Nocardia brevicatena, Nocardia caishijiensis, Nocardia calcarea, Nocardia carnea, Nocardia cellulans, Nocardia cerradoensis, Nocardia coeliaca, Nocardia corynebacterioides, Nocardia crassostreae, Nocardia cummidelens, Nocardia cyriacigeorgica, Nocardia farcinica, Nocardia flavorosea, Nocardia fluminea, Nocardia globerula, Nocardia hydrocarbonoxydans, Nocardia ignorata, Nocardia mediterranei, Nocardia nova, Nocardia orientalis, Nocardia otitidis-caviarum, Nocardia otitidiscaviarum, Nocardia paucivorans, Nocardia petroleophila, Nocardia pinensis, Nocardia pseudobrasiliensis, Nocardia pseudovaccinii, Nocardia puris, Nocardia restricta, Nocardia rugosa, Nocardia salmonicida, Nocardia saturnea, Nocardia seriolae, Nocardia soli, Nocardia sulphurea, Nocardia transvalensis, Nocardia uniformis, Nocardia vaccinii, Nocardia veterana or Nocardia vinacea;* Pseudomonaceae such as the genera Azomonas, Azotobacter, Cellvibrio, Chryseomonas, Flaviomonas, Lampropedia, Mesophilobacter, Morococcus, Oligella, Pseudomonas, Rhizobacter, Rugamonas, Serpens, Thermoleophilum, Xylophilus e.g. the species *Azomonas agilis, Azomonas insignis, Azomonas macrocytogenes, Azotobacter agilis, Azotobacter agilis subsp. armeniae, Azotobacter armeniacus, Azotobacter beijerinckii, Azotobacter chroococcum, Azotobacter indicum, Azotobacter macrocytogenes, Azotobacter miscellum, Azotobacter nigricans subsp. nigricans, Azotobacter paspali, Azotobacter salinestris, Azotobacter sp., Azotobacter vinelandii, Flavimonas oryzihabitans, Mesophilobacter marinus, Oligella urethralis, Pseudomonas acidovorans, Pseudomonas aeruginosa, Pseudomonas agarici, Pseudomonas alcaligenes, Pseudomonas aminovorans, Pseudomonas amygdali, Pseudomonas andropogonis, Pseudomonas anguilliseptica, Pseudomonas antarctica, Pseudomonas antimicrobica, Pseudomonas antimycetica, Pseudomonas aptata, Pseudomonas arvilla, Pseudomonas asplenii, Pseudomonas atlantica, Pseudomonas atrofaciens, Pseudomonas aureofaciens, Pseudomonas avellanae, Pseudomonas azelaica Pseudomonas azotocolligans, Pseudomonas balearica, Pseudomonas barkeri, Pseudomonas bathycetes, Pseudomonas beijerinckii, Pseudomonas brassicacearum, Pseudomonas brenneri, Pseudomonas butanovora, Pseudomonas carboxydoflava, Pseudomonas carboxydohydrogena, Pseudomonas carboxydovorans, Pseudomonas carrageenovora, Pseudomonas caryophylli, Pseudomonas cepacia, Pseudomonas chloritidismutans, Pseudomonas chlororaphis, Pseudomonas cichorii, Pseudomonas citronellolis, Pseudomonas cocovenenans, Pseudomonas compransoris, Pseudomonas congelans, Pseudomonas coronafaciens, Pseudomonas corrugata, Pseudomonas dacunhae, Pseudomonas delafieldii, Pseudomonas delphinii, Pseudomonas denitrificans, Pseudomonas desmolytica, Pseudomonas diminuta, Pseudomonas doudoroffii, Pseudomonas echinoides, Pseudomonas elongata, Pseudomonas extorquens, Pseudomonas extremorientalis, Pseudomonas facilis, Pseudomonas ficuserectae, Pseudomonas flava, Pseudomonas flavescens, Pseudomonas fluorescens, Pseudomonas fragi, Pseudomonas frederiksbergensis, Pseudomonas fulgida, Pseudomonas fuscovaginae, Pseudomonas gazotropha, Pseudomonas gladioli, Pseudomonas glathei, Pseudomonas glumae, Pseudomonas graminis, Pseudomonas halophila, Pseudomonas helianthi, Pseudomonas huttiensis, Pseudomonas hydrogenothermophila, Pseudomonas hydrogenovora, Pseudomonas indica, Pseudomonas indigofera, Pseudomonas iodinum, Pseudomonas kilonensis, Pseudomonas lachrymans, Pseudomonas lapsa, Pseudomonas lemoignei, Pseudomonas lemonnieri, Pseudomonas lundensis, Pseudomonas luteola, Pseudomonas maltophilia, Pseudomonas marginalis, Pseudomonas marginata, Pseudomonas marina, Pseudomonas meliae, Pseudomonas mendocina, Pseudomonas mesophilica, Pseudomonas mixta, Pseudomonas monteilii, Pseudomonas morsprunorum, Pseudomonas multivorans, Pseudomonas natriegens, Pseudomonas nautica, Pseudomonas nitroreducens, Pseudomonas oleovorans, Pseudomonas oryzihabitans, Pseudomonas ovalis, Pseudomonas oxalaticus, Pseudomonas palleronii, Pseudomonas paucimobilis, Pseudomonas phaseolicola, Pseudomonas phenazinium, Pseudomonas pickettii, Pseudomonas pisi, Pseudomonas plantarii, Pseudomonas plecoglossicida, Pseudomonas poae, Pseudomonas primulae, Pseudomonas proteolytica, Pseudomonas pseudoalcaligenes, Pseudomonas pseudoalcaligenes* subsp. *konjaci, Pseudomonas pseudoalcaligenes* subsp. *pseudoalcaligenes, Pseudomonas pseudoflava, Pseudomonas putida, Pseudomonas putida* var. *naraensis, Pseudomonas putrefaciens, Pseudomonas pyrrocinia, Pseudomonas radiora, Pseudomonas reptilivora, Pseudomonas rhodesiae, Pseudomonas rhodos, Pseudomonas riboflavina, Pseudomonas rubescens, Pseudomonas rubrisubalbicans, Pseudomonas ruhlandii, Pseudomonas saccharophila, Pseudomonas savastanoi, Pseudomonas savastanoi* pvar. *glycinea, Pseudomonas savastanoi* pvar. *phaseolicola, Pseudomonas solanacearum, Pseudomonas* sp., *Pseudomonas spinosa, Pseudomonas stanieri, Pseudomonas stutzeri, Pseudomonas syringae, Pseudomonas syringae* pvar. *aptata, Pseudomonas syringae* pvar. *atrofaciens, Pseudomonas syringae* pvar. *coronafaciens, Pseudomonas syringae* pvar. *delphinii, Pseudomonas syringae* pvar. *glycinea, Pseudomonas syringae* pvar. *helianthi, Pseudomonas syringae* pvar. *lachrymans, Pseudomonas syringae* pvar. *lapsa, Pseudomonas syringae* pvar. *morsprunorum, Pseudomonas syringae* pvar. *phaseolicola, Pseudomonas syringae* pvar. *primulae, Pseudomonas syringae* pvar. *syringae, Pseudomonas syringae* pvar. *tabaci, Pseudomonas syringae* pvar. *tomato, Pseudomonas syringae* subsp. *glycinea, Pseudomonas syringae* subsp. *savastanoi, Pseudomonas syringae* subsp. *syringae, Pseudomonas syzygii, Pseudomonas tabaci, Pseudomonas taeniospiralis, Pseudomonas testosteroni, Pseudomonas thermocarboxydovorans, Pseudomonas thermotolerans, Pseudomonas thivervalensis, Pseudomonas tomato, Pseudomonas trivialis, Pseudomonas veronii, Pseudomonas vesicularis, Pseudomonas viridiflava, Pseudomonas viscogena, Pseudomonas woodsii, Rhizobacter dauci, Rhizobacter daucus or Xylophilus ampelinus;* Rhizobiaceae such as the genera Agrobacterium, Carbophilus, Chelatobacter, Ensifer, Rhizobium, Sinorhizobium e.g. the species *Agrobacterium atlanticum, Agrobacterium ferrugineum, Agrobacterium gelatinovorum, Agrobacterium larrymoorei, Agrobacterium meteori, Agrobacterium radiobacter, Agrobacterium rhizogenes, Agrobacterium rubi, Agrobacterium stellulatum, Agrobacterium tumefaciens, Agrobacterium vitis, Carbophilus carboxidus, Chelatobacter heintzii, Ensifer adhaerens, Ensifer arboris, Ensifer fredii, Ensifer kostiensis, Ensifer kummerowiae, Ensifer medicae, Ensifer meliloti, Ensifer saheli, Ensifer terangae, Ensifer xinjiangensis, Rhizobium ciceri Rhizobium etli, Rhizobium fredii, Rhizobium galegae, Rhizobium gallicum, Rhizobium giardinii, Rhizobium hainanense, Rhizobium huakuii, Rhizobium huautlense, Rhizobium indigoferae, Rhizobium japonicum, Rhizobium leguminosarum, Rhizobium loessense, Rhizobium loti, Rhizobium lupini, Rhizobium mediterraneum, Rhizobium meliloti, Rhizobium mongolense, Rhizobium phaseoli, Rhizobium radiobacter, Rhizobium rhizogenes, Rhizobium rubi, Rhizobium sullae, Rhizobium tianshanense, Rhizobium trifolii, Rhizobium tropici, Rhizobium undicola, Rhizobium vitis, Sinorhizobium adhaerens, Sinorhizobium arboris, Sinorhizobium fredii, Sinorhizobium kostiense, Sinorhizobium kummerowiae, Sinorhizobium medicae, Sinorhizobium meliloti, Sinorhizobium morelense, Sinorhizobium saheli or Sinorhizobium xinjiangense;* Streptomycetaceae such as the genera Kitasatosprora, Streptomyces, Streptoverticillium e.g. the species *Streptomyces abikoensis, Streptomyces aburaviensis, Streptomyces achromogenes subsp. achromogenes, Streptomyces achromogenes subsp. rubradiris, Streptomyces acidiscabies, Streptomyces acrimycini, Streptomyces aculeolatus, Streptomyces afghaniensis, Streptomyces alanosinicus, Streptomyces albaduncus, Streptomyces albiaxialis, Streptomyces albidochromogenes, Streptomyces albidoflavus, Streptomyces albireticuli, Streptomyces albofaciens, Streptomyces alboflavus, Streptomyces albogriseolus, Streptomyces albolongus, Streptomyces alboniger, Streptomyces albospinus, Streptomyces albosporeus subsp. albosporeus, Streptomyces albosporeus subsp. labilomyceticus, Streptomyces alboverticillatus, Streptomyces albovinaceus, Streptomyces alboviridis, Streptomyces albulus, Streptomyces albus subsp. albus, Streptomyces albus subsp. pathocidicus, Streptomyces almquistii, Streptomyces althioticus, Streptomyces amakusaensis, Streptomyces ambofaciens, Streptomyces aminophilus, Streptomyces anandii, Streptomyces anthocyanicus, Streptomyces antibioticus, Streptomyces antimycoticus, Streptomyces anulatus, Streptomyces arabicus, Streptomyces ardus, Streptomyces arenae, Streptomyces argenteolus, Streptomyces armeniacus, Streptomyces asiaticus, Streptomyces asterosporus, Streptomyces atratus, Streptomyces atroaurantiacus, Streptomyces atroolivaceus, Streptomyces atrovirens, Streptomyces aurantiacus, Streptomyces aurantiogriseus, Streptomyces aureocirculatus, Streptomyces aureofaciens, Streptomyces aureorectus, Streptomyces aureoversilis, Streptomyces aureoverticillatus, Streptomyces aureus, Streptomyces avellaneus, Streptomyces avermectinius, Streptomyces avermitilis, Streptomyces avidinii, Streptomyces azaticus, Streptomyces azureus, Streptomyces baarnensis, Streptomyces bacillaris, Streptomyces badius, Streptomyces baldaccii, Streptomyces bambergiensis, Streptomyces beijiangensis, Streptomyces bellus, Streptomyces bikiniensis, Streptomyces biverticillatus, Streptomyces blastmyceticus, Streptomyces bluensis, Streptomyces bobili, Streptomyces bottropensis, Streptomyces brasiliensis, Streptomyces bungoensis, Streptomyces cacaoi subsp. asoensis, Streptomyces cacaoi subsp. cacaoi, Streptomyces caelestis, Streptomyces caeruleus, Streptomyces californicus, Streptomyces calvus, Streptomyces canaries, Streptomyces candidus, Streptomyces canescens, Streptomyces cangkringensis, Streptomyces caniferus, Streptomyces canus, Streptomyces capillispiralis, Streptomyces capoamus, Streptomyces carpaticus, Streptomyces carpinensis, Streptomyces catenulae, Streptomyces caviscabies, Streptomyces cavourensis subsp. cavourensis, Streptomyces cavourensis subsp. washingtonensis, Streptomyces cellostaticus, Streptomyces celluloflavus, Streptomyces cellulolyticus, Streptomyces cellulosae,Streptomyces champavatii, Streptomyces chartreuses, Streptomyces chattanoogensis, Streptomyces chibaensis, Streptomyces chrestomyceticus, Streptomyces chromofuscus, Streptomyces chryseus, Streptomyces chrysomallus subsp. chrysomallus, Streptomyces chrysomallus subsp. fumigatus, Streptomyces cinereorectus, Streptomyces cinereoruber subsp. cinereoruber, Streptomyces cinereoruber subsp. fructofermentans, Streptomyces cinereospinus, Streptomyces cinereus, Streptomyces cinerochromogenes, Streptomyces cinnabarinus, Streptomyces cinnamonensis, Streptomyces cinnamoneus, Streptomyces cinnamoneus subsp. albosporus, Streptomyces cinnamoneus subsp. cinnamoneus, Streptomyces cinnamoneus subsp. lanosus, Streptomyces cinnamoneus subsp. sparsus, Streptomyces cirratus, Streptomyces ciscaucasicus, Streptomyces citreofluorescens, Streptomyces clavifer, Streptomyces clavuligerus, Streptomyces cochleatus, Streptomyces coelescens, Streptomyces coelicoflavus, Streptomyces coelicolor, Streptomyces coeruleoflavus, Streptomyces coeruleofuscus, Streptomyces coeruleoprunus, Streptomyces coeruleorubidus, Streptomyces coerulescens, Streptomyces collinus, Streptomyces colombiensis, Streptomyces corchorusii, Streptomyces costaricanus, Streptomyces cremeus, Streptomyces crystallinus, Streptomyces curacoi, Streptomyces cuspidosporus, Streptomyces cyaneofuscatus, Streptomyces cyaneus, Streptomyces cyanoalbus, Streptomyces cystargineus, Streptomyces daghestanicus, Streptomyces diastaticus subsp. ardesiacus, Streptomyces diastaticus subsp. diastaticus, Streptomyces diastatochromogenes, Streptomyces distallicus, Streptomyces djakartensis, Streptomyces durhamensis, Streptomyces echinatus, Streptomyces echinoruber, Streptomyces ederensis, Streptomyces ehimensis, Streptomyces endus, Streptomyces enissocaesilis, Streptomyces erumpens, Streptomyces erythraeus, Streptomyces erythrogriseus, Streptomyces eurocidicus, Streptomyces europaeiscabiei, Streptomyces eurythermus, Streptomyces exfoliates, Streptomyces felleus, Streptomyces fervens, Streptomyces fervens subsp. fervens, Streptomyces fervens subsp. melrosporus, Streptomyces filamentosus, Streptomyces filipinensis, Streptomyces fimbriatus, Streptomyces fimicarius, Streptomyces finlayi, Streptomyces flaveolus, Streptomyces flaveus, Streptomyces flavidofuscus, Streptomyces flavidovirens, Streptomyces flaviscleroticus, Streptomyces flavofungini, Streptomyces flavofuscus, Streptomyces flavogriseus, Streptomyces flavopersicus, Streptomyces flavotricini, Streptomyces flavovariabilis, Streptomyces flavovirens, Streptomyces flavoviridis, Streptomyces flocculus, Streptomyces floridae, Streptomyces fluorescens, Streptomyces fradiae, Streptomyces fragilis, Streptomyces fulvissimus, Streptomyces fulvorobeus, Streptomyces fumanus, Streptomyces fumigatiscleroticus, Streptomyces galbus, Streptomyces galilaeus, Streptomyces gancidicus, Streptomyces gardneri, Streptomyces gelaticus, Streptomyces geysiriensis, Streptomyces ghanaensis, Streptomyces gibsonii, Streptomyces glaucescens, Streptomyces glaucosporus, Streptomyces glaucus, Streptomyces globisporus subsp. caucasicus, Streptomyces globisporus subsp. flavofuscus, Streptomyces globisporus subsp. globisporus, Streptomyces globosus, Streptomyces glomeratus, Streptomyces glomeroaurantiacus, Streptomyces gobitricini, Streptomyces goshikiensis, Streptomyces gougerotii, Streptomyces graminearus, Streptomyces graminofaciens, Streptomyces griseinus, Streptomyces griseoaurantiacus, Streptomyces griseobrunneus, Streptomyces griseocarneus, Streptomyces griseochromogenes, Streptomyces griseoflavus, Streptomyces griseofuscus, Streptomyces griseoincarnatus, Streptomyces griseoloalbus, Streptomyces griseolosporeus, Streptomyces griseolus, Streptomyces griseoluteus, Streptomyces griseomycini, Streptomyces griseoplanus, Streptomyces griseorubens, Streptomyces griseoruber, Streptomyces griseorubiginosus, Streptomyces griseosporeus, Streptomyces griseostramineus, Streptomyces griseoverticillatus, Streptomyces griseoviridis, Streptomyces griseus subsp. alpha, Streptomyces griseus subsp. cretosus, Streptomyces griseus subsp. griseus, Streptomyces griseus subsp. solvifaciens, Streptomyces hachijoensis, Streptomyces halstedii, Streptomyces hawaiiensis, Streptomyces heliomycini, Streptomyces helvaticus, Streptomyces herbaricolor, Streptomyces hiroshimensis, Streptomyces hirsutus, Streptomyces humidus, Streptomyces humiferus, Streptomyces hydrogenans, Streptomyces hygroscopicus subsp. angustmyceticus, Streptomyces hygroscopicus subsp. decoyicus, Streptomyces hygroscopicus subsp. glebosus, Streptomyces hygroscopicus subsp. hygroscopicus, Streptomyces hygroscopicus subsp. ossamyceticus, Streptomyces iakyrus, Streptomyces indiaensis, Streptomyces indigoferus, Streptomyces indonesiensis, Streptomyces intermedius, Streptomyces inusitatus, Streptomyces ipomoeae, Streptomyces janthinus, Streptomyces javensis, Streptomyces kanamyceticus, Streptomyces kashmirensis, Streptomyces kasugaensis, Streptomyces katrae, Streptomyces kentuckensis, Streptomyces kifunensis, Streptomyces kishiwadensis, Streptomyces kunmingensis, Streptomyces kurssanovii, Streptomyces labedae, Streptomyces laceyi, Streptomyces ladakanum, Streptomyces lanatus, Streptomyces lateritius, Streptomyces laurentii, Streptomyces lavendofoliae, Streptomyces lavendulae subsp. grasserius, Streptomyces lavendulae subsp. lavendulae, Streptomyces lavenduligriseus, Streptomyces lavendulocolor, Streptomyces levis, Streptomyces libani* subsp. *libani, Streptomyces libani* subsp. *rufus, Streptomyces lienomycini, Streptomyces lilacinus, Streptomyces limosus, Streptomyces lincolnensis, Streptomyces lipmanii, Streptomyces litmocidini, Streptomyces lomondensis, Streptomyces longisporoflavus, Streptomyces longispororuber, Streptomyces longisporus, Streptomyces longwoodensis, Streptomyces lucensis, Streptomyces luridiscabiei, Streptomyces luridus, Streptomyces lusitanus, Streptomyces luteireticuli, Streptomyces luteogriseus, Streptomyces luteosporeus, Streptomyces luteoverticillatus, Streptomyces lydicus, Streptomyces macrosporus, Streptomyces malachitofuscus, Streptomyces malachitospinus, Streptomyces malaysiensis, Streptomyces mashuensis, Streptomyces massasporeus, Streptomyces matensis, Streptomyces mauvecolor, Streptomyces mediocidicus, Streptomyces mediolani, Streptomyces megasporus, Streptomyces melanogenes, Streptomyces melanosporofaciens, Streptomyces mexicanus, Streptomyces michiganensis, Streptomyces microflavus, Streptomyces minutiscleroticus, Streptomyces mirabilis, Streptomyces misakiensis, Streptomyces misionensis, Streptomyces mobaraensis, Streptomyces monomycini, Streptomyces morookaensis, Streptomyces murinus, Streptomyces mutabilis, Streptomyces mutomycini, Streptomyces naganishii, Streptomyces narbonensis, Streptomyces nashvillensis, Streptomyces netropsis, Streptomyces neyagawaensis, Streptomyces niger, Streptomyces nigrescens, Streptomyces nigrifaciens, Streptomyces nitrosporeus, Streptomyces niveiciscabiei, Streptomyces niveoruber, Streptomyces niveus, Streptomyces noboritoensis, Streptomyces nodosus, Streptomyces nogalater, Streptomyces nojiriensis, Streptomyces noursei, Streptomyces novaecaesareae, Streptomyces ochraceiscleroticus, Streptomyces odorifer, Streptomyces olivaceiscleroticus, Streptomyces olivaceoviridis, Streptomyces olivaceus, Streptomyces olivochromogenes, Streptomyces olivomycini, Streptomyces olivoreticuli, Streptomyces olivoreticuli subsp. cellulophilus, Streptomyces olivoreticuli subsp. olivoreticuli, Streptomyces olivoverticillatus, Streptomyces olivoviridis, Streptomyces omiyaensis, Streptomyces orinoci, Streptomyces pactum, Streptomyces paracochleatus, Streptomyces paradoxus, Streptomyces parvisporogenes, Streptomyces parvulus, Streptomyces parvus, Streptomyces peucetius, Streptomyces phaeochromogenes, Streptomyces phaeofaciens, Streptomyces phaeopurpureus, Streptomyces phaeoviridis, Streptomyces phosalacineus, Streptomyces pilosus, Streptomyces platensis, Streptomyces plicatus, Streptomyces pluricolorescens, Streptomyces polychromogenes, Streptomyces poonensis, Streptomyces praecox, Streptomyces prasinopilosus, Streptomyces prasinosporus, Streptomyces prasinus, Streptomyces prunicolor, Streptomyces psammoticus, Streptomyces pseudoechinosporeus, Streptomyces pseudogriseolus, Streptomyces pseudovenezuelae, Streptomyces pulveraceus, Streptomyces puniceus, Streptomyces puniciscabiei, Streptomyces purpeofuscus, Streptomyces purpurascens, Streptomyces purpureus, Streptomyces purpurogeneiscleroticus, Streptomyces racemochromogenes, Streptomyces rameus, Streptomyces ramulosus, Streptomyces rangoonensis, Streptomyces recifensis, Streptomyces rectiverticillatus, Streptomyces rectiviolaceus, Streptomyces regensis, Streptomyces resistomycificus, Streptomyces reticuliscabiei, Streptomyces rhizosphaericus, Streptomyces rimosus subsp. paromomycinus, Streptomyces rimosus subsp. rimosus, Streptomyces rishiriensis, Streptomyces rochei, Streptomyces roseiscleroticus, Streptomyces roseodiastaticus, Streptomyces roseoflavus, Streptomyces roseofulvus, Streptomyces roseolilacinus, Streptomyces roseolus, Streptomyces roseosporus, Streptomyces roseoverticillatus, Streptomyces roseoviolaceus, Streptomyces roseoviridis, Streptomyces rubber, Streptomyces rubiginosohelvolus, Streptomyces rubiginosus, Streptomyces rubrogriseus, Streptomyces rutgersensis subsp. castelarensis, Streptomyces rutgersensis subsp. rutgersensis, Streptomyces salmonis, Streptomyces sampsonii, Streptomyces sanglieri, Streptomyces sannanensis, Streptomyces sapporonensis, Streptomyces scabiei, Streptomyces sclerotialus, Streptomyces scopiformis, Streptomyces seoulensis, Streptomyces septatus, Streptomyces setae, Streptomyces setonii, Streptomyces showdoensis, Streptomyces sindenensis, Streptomyces sioyaensis, Streptomyces somaliensis, Streptomyces sparsogenes, Streptomyces spectabilis, Streptomyces speibonae, Streptomyces speleomycini, Streptomyces spheroids, Streptomyces spinoverrucosus, Streptomyces spiralis, Streptomyces spiroverticillatus, Streptomyces spitsbergensis, Streptomyces sporocinereus, Streptomyces sporoclivatus, Streptomyces spororaveus, Streptomyces sporoverrucosus, Streptomyces stelliscabiei, Streptomyces stramineus, Streptomyces subrutilus, Streptomyces sulfonofaciens, Streptomyces sulphurous, Streptomyces syringium, Streptomyces tanashiensis, Streptomyces tauricus, Streptomyces tendae, Streptomyces termitum, Streptomyces thermoalcalitolerans, Streptomyces thermoautotrophicus, Streptomyces thermocarboxydovorans, Streptomyces thermocarboxydus, Streptomyces thermocoprophilus, Streptomyces thermodiastaticus, Streptomyces thermogriseus, Streptomyces thermolineatus, Streptomyces thermonitrificans, Streptomyces thermospinosisporus, Streptomyces thermoviolaceus subsp. apingens, Streptomyces thermoviolaceus subsp. thermoviolaceus, Streptomyces thermovulgaris, Streptomyces thioluteus, Streptomyces torulosus, Streptomyces toxytricini, Streptomyces tricolor, Streptomyces tubercidicus, Streptomyces tuirus, Streptomyces turgidiscabies, Streptomyces umbrinus, Streptomyces variabilis, Streptomyces variegates, Streptomyces varsoviensis, Streptomyces vastus, Streptomyces venezuelae, Streptomyces vinaceus, Streptomyces vinaceusdrappus, Streptomyces violaceochromogenes, Streptomyces violaceolatus,Streptomyces violaceorectus, Streptomyces violaceoruber, Streptomyces violaceorubidus, Streptomyces violaceus, Streptomyces violaceusniger, Streptomyces violarus, Streptomyces violascens, Streptomyces violatus, Streptomyces violens, Streptomyces virens, Streptomyces virginiae, Streptomyces viridiflavus, Streptomyces viridiviolaceus, Streptomyces viridobrunneus, Streptomyces viridochromogenes, Streptomyces viridodiastaticus, Streptomyces viridosporus, Streptomyces vitaminophileus, Streptomyces vitaminophilus, Streptomyces wedmorensis, Streptomyces werraensis, Streptomyces willmorei, Streptomyces xanthochromogenes, Streptomyces xanthocidicus, Streptomyces xantholiticus, Streptomyces xanthophaeus, Streptomyces yatensis, Streptomyces yerevanensis, Streptomyces yogyakartensis, Streptomyces yokosukanensis, Streptomyces yunnanensis, Streptomyces zaomyceticus, Streptoverticillium abikoense, Streptoverticillium albireticuli, Streptoverticillium alboverticillatum, Streptoverticillium album, Streptoverticillium ardum, Streptoverticillium aureoversale, Streptoverticillium aureoversile, Streptoverticillium baldaccii , Streptoverticillium biverticillatum, Streptoverticillium blastmyceticum, Streptoverticillium cinnamoneum* subsp. *albosporum, Streptomyces cinnamoneus* subsp. *albosporus, Streptoverticillium cinnamoneum* subsp. *cinnamoneum, Streptoverticillium cinnamoneum* subsp. *lanosum, Streptoverticillium cinnamoneum* subsp. *sparsum, Streptoverticillium distallicum, Streptoverticillium ehimense, Streptoverticillium eurocidicum, Streptoverticillium fervens* subsp. *fervens, Streptoverticillium fervens* subsp. *melrosporus, Streptoverticillium flavopersicum, Streptoverticillium griseocarneum, Streptoverticillium griseoverticillatum, Streptoverticillium hachijoense, Streptoverticillium hiroshimense, Streptoverticillium kashmirense, Streptoverticillium kentuckense, Streptoverticillium kishiwadense, Streptoverticillium ladakanum, Streptoverticillium lavenduligriseum, Streptoverticillium lilacinum, Streptoverticillium luteoverticillatum, Streptoverticillium mashuense, Streptoverticillium mobaraense, Streptoverticillium morookaense, Streptoverticillium netropsis, Streptoverticillium olivomycini, Streptomyces olivomycini, Streptoverticillium olivoreticuli* subsp. *cellulophilum, Streptoverticillium olivoreticuli* subsp. *olivoreticuli, Streptoverticillium olivoreticulum, Streptoverticillium olivoreticulum* subsp. *cellulophilum, Streptoverticillium olivoverticillatum, Streptoverticillium orinoci, Streptoverticillium parvisporogenes, Streptoverticillium parvisporogenum, Streptoverticillium rectiverticillatum, Streptoverticillium reticulum* subsp. *protomycicum, Streptoverticillium roseoverticillatum, Streptoverticillium salmonis, Streptoverticillium sapporonense, Streptoverticillium septatum, Streptoverticillium syringium, Streptoverticillium thioluteum, Streptoverticillium verticillium* subsp. *quantum, Streptoverticillium verticillium* subsp. *tsukushiense or Streptoverticillium viridoflavum.*

**[0080.0.0.0]** Particular preferred strains are strains selected from the group consisting of Bacillaceae, Brevibacteriaceae, Corynebacteriaceae, Nocardiaceae, Mycobacteriaceae, Streptomycetaceae, Enterobacteriaceae such as *Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum* (= *Micrococcus glutamicum*), *Corynebacterium melassecola, Corynebacterium sp., Nocardia rhodochrous (Rhodococcus rhodochrous) , Mycobacterium rhodochrous, Streptomyces lividans* and *Escherichia coli* especially *Escherichia coli K12.*

**[0081.0.0.0]** In addition particular preferred strains are strains selected from the group consisting of Cryptococcaceae, Saccharomycetaceae, Schizosaccharo-mycetacease such as the genera Candida, Hansenula, Pichia, Saccharomyces and Schizosaccharomyces preferred are strains selected from the group consisting of the species *Rhodotorula rubra, Rhodotorula glutinis, Rhodotorula graminis, Yarrowia lipolytica, Sporobolomyces salmonicolor, Sporobolomyces shibatanus, Saccharomyces cerevisiae, Candida boidinii, Candida bombicola, Candida cylindracea, Candida parapsilosis, Candida rugosa, Candida tropicalis, Pichia methanolica and Pichia pastoris.*

**[0082.0.0.0]** Anacardiaceae such as the genera Pistacia, Mangifera, Anacardium e.g. the species *Pistacia vera* [pistachios, Pistazie], *Mangifer indica* [Mango] or *Anacardium occidentale* [Cashew]; Asteraceae such as the genera Calendula, Carthamus, Centaurea, Cichorium, Cynara, Helianthus, Lactuca, Locusta, Tagetes, Valeriana e.g. the species *Calendula officinalis* [Marigold], *Carthamus tinctorius* [safflower], *Centaurea cyanus* [cornflower], *Cichorium intybus* [blue daisy], *Cynara scolymus* [Artichoke], *Helianthus annus* [sunflower], Lactuca sativa, *Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [lettuce], *Tagetes lucida, Tagetes erecta or Tagetes tenuifolia* [Marigold]; Apiaceae such as the genera Daucus e.g. the species *Daucus carota* [carrot]; Betulaceae such as the genera Corylus e.g. the species *Corylus avellana* or *Corylus colurna* [hazelnut]; Boraginaceae such as the genera Borago e.g. the species *Borago officinalis* [borage]; Brassicaceae such as the genera Brassica, Melanosinapis, Sinapis, Arabadopsis e.g. the species *Brassica napus, Brassica rapa* ssp. [canola, oilseed rape, turnip rape], *Sinapis arvensis Brassica juncea, Brassica juncea var. juncea, Brassica juncea var. crispifolia, Brassica juncea var. foliosa, Brassica nigra, Brassica sinapioides, Melanosinapis communis* [mustard], *Brassica oleracea* [fodder beet] or Arabidopsis thaliana; Bromeliaceae such as the genera Anana, Bromelia e.g. the species *Anana comosus, Ananas ananas* or *Bromelia comosa* [pineapple]; Caricaceae such as the genera Carica e.g. the species *Carica papaya* [papaya]; Cannabaceae such as the genera Cannabis e.g. the species *Cannabis sative* [hemp], Convolvulaceae such as the genera Ipomea, Convolvulus e.g. the species *Ipomoea batatus, Ipomoea pandurata, Convolvulus batatas, Convolvulus tiliaceus, Ipomoea fastigiata, Ipomoea tiliacea, Ipomoea triloba* or *Convolvulus panduratus* [sweet potato, Man of the Earth, wild potato], Chenopodiaceae such as the genera Beta, i.e. the species *Beta vulgaris, Beta vulgaris var. altissima, Beta vulgaris var. Vulgaris, Beta maritima, Beta vulgaris var. perennis, Beta vulgaris var. conditiva* or *Beta vulgaris var. esculenta* [sugar beet]; Cucurbitaceae such as the genera Cucubita e.g. the species *Cucurbita maxima, Cucurbita mixta, Cucurbita pepo* or *Cucurbita moschata* [pumpkin, squash]; Elaeagnaceae such as the genera Elaeagnus e.g. the species *Olea europaea* [olive]; Ericaceae such as the genera Kalmia e.g. the species *Kalmia latifolia, Kalmia angustifolia, Kalmia microphylla, Kalmia polifolia, Kalmia occidentalis, Cistus chamaerhodendros* or *Kalmia lucida* [American laurel, broad-leafed laurel, calico bush, spoon wood, sheep laurel, alpine laurel, bog laurel, western bog-laurel, swamp-laurel]; Euphorbiaceae such as the genera Manihot, Janipha, Jatropha, Ricinus e.g. the species *Manihot utilissima, Janipha manihot" Jatropha manihot., Manihot aipil, Manihot dulcis, Manihot manihot, Manihot melanobasis, Manihot esculenta* [manihot, arrowroot, tapioca, cassava] or *Ricinus communis* [castor bean, Castor Oil Bush, Castor Oil Plant, Palma Christi, Wonder Tree]; Fabaceae such as the genera Pisum, Albizia, Cathormion, Feuillea, Inga, Pithecolobium, Acacia, Mimosa, Medicajo, Glycine, Dolichos, Phaseolus, Soja e.g. the species *Pisum sativum, Pisum arvense*, *Pisum humile* [pea], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [bastard logwood, silk tree, East Indian Walnut], *Medicago sativa, Medicago falcata, Medicago varia* [alfalfa] *Glycine max Dolichos soja, Glycine gracilis, Glycine hispida, Phaseolus max, Soja hispida* or *Soja max* [soybean]; Geraniaceae such as the genera Pelargonium, Cocos, Oleum e.g. the species Cocos *nucifera, Pelargonium grossularioides* or *Oleum cocois* [coconut]; Gramineae such as the genera Saccharum e.g. the species *Saccharum officinarum;* Juglandaceae such as the genera Juglans, Wallia e.g. the species *Juglans regia, Juglans ailanthifolia, Juglans sieboldiana, Juglans cinerea*, *Wallia cinerea*, *Juglans bixbyi, Juglans californica, Juglans hindsii, Juglans intermedia, Juglans jamaicensis, Juglans major, Juglans microcarpa, Juglans nigra* or *Wallia nigra* [walnut, black walnut, common walnut, persian walnut, white walnut, butternut, black walnut]; Lauraceae such as the genera Persea, Laurus e.g. the species laurel *Laurus nobilis* [bay, laurel, bay laurel, sweet bay], *Persea americana Persea americana, Persea gratissima* or *Persea persea* [avocado]; Leguminosae such as the genera Arachis e.g. the species Arachis *hypogaea* [peanut]; Linaceae such as the genera Linum, Adenolinum e.g. the species *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* or *Linum trigynum* [flax, linseed]; Lythrarieae such as the genera Punica e.g. the species *Punica granatum* [pomegranate]; Malvaceae such as the genera Gossypium e.g. the species *Gossypium hirsutum, Gossypium arboreum, Gossypium barbadense, Gossypium herbaceum* or *Gossypium thurberi* [cotton]; Musaceae such as the genera Musa e.g. the species *Musa nana, Musa acuminata, Musa paradisiaca, Musa* spp. [banana]; Onagraceae such as the genera Camissonia, Oenothera e.g. the species *Oenothera biennis* or *Camissonia brevipes* [primrose, evening primrose]; Palmae such as the genera Elacis e.g. the species *Elaeis guineensis* [oil plam]; Papaveraceae such as the genera Papaver e.g. the species *Papaver orientale, Papaver rhoeas*, *Papaver dubium* [poppy, oriental poppy, corn poppy, field poppy, shirley poppies, field poppy, long-headed poppy, long-pod poppy]; Pedaliaceae such as the genera Sesamum e.g. the species *Sesamum indicum* [sesame]; Piperaceae such as the genera Piper, Artanthe, Peperomia, Steffensia e.g. the species *Piper aduncum, Piper amalago, Piper angustifolium, Piper auritum, Piper betel, Piper cubeba, Piper longum, Piper nigrum, Piper retrofractum, Artanthe* adunca, *Artanthe elongata, Peperomia elongata, Piper elongatum, Steffensia elongata.* [Cayenne pepper, wild pepper]; Poaceae such as the genera Hordeum, Secale, Avena, Sorghum, Andropogon, Holcus, Panicum, Oryza, Zea, Triticum e.g. the species *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [barley, pearl barley, foxtail barley, wall barley, meadow barley], *Secale cereale* [rye], Avena sativa, *Avena fatua, Avena byzantina, Avena fatua var. sativa, Avena hybrida* [oat], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum* millet, *Panicum militaceum [Sorghum, millet], Oryza sativa, Oryza latifolia* [rice], *Zea mays* [corn, maize] *Triticum aestivum, Triticum durum, Triticum turgidum, Triticum hybernum, Triticum macha, Triticum sativum* or *Triticum vulgare* [wheat, bread wheat, common wheat], Proteaceae such as the genera Macadamia e.g. the species *Macadamia intergrifolia* [macadamia]; Rubiaceae such as the genera Coffea e.g. the species Cofea spp., *Coffea arabica, Coffea canephora* or *Coffea liberica* [coffee]; Scrophulariaceae such as the genera Verbascum e.g. the species *Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* or *Verbascum thapsus* [mullein, white moth mullein, nettle-leaved mullein, dense-flowered mullein, silver mullein, long-leaved mullein, white mullein, dark mullein, greek mullein, orange mullein, purple mullein, hoary mullein, great mullein]; Solanaceae such as the genera Capsicum, Nicotiana, Solanum, Lycopersicon e.g. the species *Capsicum annuum, Capsicum annuum var. glabriusculum, Capsicum frutescens* [pepper], *Capsicum annuum* [paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [tobacco], *Solanum tuberosum* [potato], *Solanum melongena* [egg-plant] (*Lycopersicon esculentum, Lycopersicon lycopersicum., Lycopersicon pyriforme, Solanum integrifolium* or *Solanum lycopersicum* [*tomato*]; Sterculiaceae such as the genera Theobroma e.g. the species *Theobroma cacao* [cacao]; Theaceae such as the genera Camellia e.g. the species *Camellia sinensis*) [tea].
All abovementioned organisms can in princible also function as host organisms.

**[0083.0.0.0]** Particular preferred plants are plants selected from the group consisting of Asteraceae such as the genera Helianthus, Tagetes e.g. the species *Helianthus annus* [sunflower], *Tagetes lucida, Tagetes erecta or Tagetes tenuifolia* [Marigold], Brassicaceae such as the genera Brassica, Arabadopsis e.g. the species *Brassica napus, Brassica rapa* ssp. [canola, oilseed rape, turnip rape] or Arabidopsis thaliana. Fabaceae such as the genera Glycine e.g. the species *Glycine max, Soja hispida* or *Soja max* [soybean] (wobei ich nicht sicher bin, ob es Soja max überhaupt gibt, die heißt eigentlich Glycine max). Linaceae such as the genera Linum e.g. the species *Linum usitatissimum,* [flax, linseed]; Poaceae such as the genera Hordeum, Secale, Avena, Sorghum, Oryza, Zea, Triticum e.g. the species *Hordeum vulgare* [barley]; *Secale cereale* [rye], Avena sativa, *Avena fatua, Avena byzantina,* Avena *fatua var. sativa, Avena hybrida* [oat], *Sorghum bicolor* [Sorghum, millet], *Oryza sativa, Oryza latifolia* [rice], *Zea mays* [corn, maize] *Triticum aestivum, Triticum durum, Triticum turgidum, Triticum hybernum, Triticum macha, Triticum sativum* or *Triticum vulgare* [wheat, bread wheat, common wheat]; Solanaceae such as the genera Solanum, Lycopersicon e.g. the species *Solanum tuberosum* [potato], *Lycopersicon esculentum, Lycopersicon lycopersicum., Lycopersicon pyriforme, Solanum integrifolium* or *Solanum lycopersicum* [*tomato*].

**[0084.0.0.0]** All abovementioned organisms can in princible also function as host organisms.

**[0085.0.0.0]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, application no. 0, columns 5 or 7, or a derivative thereof, or
b) a genetic regulatory element, for example a promoter, which is functionally linked to the nucleic acid sequence as indicated in Table I, application no. 0, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.0]** The use of the nucleic acid sequence according to the invention or of the nucleic acid construct according to the invention for the generation of transgenic plants is therefore also subject matter of the invention.

**[0087.0.0.0]** The respective fine chemical, which is synthesized in the organism, in particular the microorganism, the cell, the tissue or the plant, of the invention can be isolated if desired. Depending on the use of the respective fine chemical, different purities resulting from the purification may be advantageous as will be described herein below.

**[0088.0.0.0]** In an advantageous embodiment of the invention, the organism takes the form of a plant whose amino acid content is modified advantageously owing to the nucleic acid molecule of the present invention expressed. This is important for plant breeders since, for example, the nutritional value of plants for monogastric animals is limited by a few essential amino acids such as lysine, threonine or methionine.

**[0088.1.0.0]** In one embodiment, after an activity of a polypeptide of the present invention or used in the process of the present invention has been increased or generated, or after the expression of a nucleic acid molecule or polypeptide according to the invention has been generated or increased, the transgenic plant generated can be grown on or in a nutrient medium or else in the soil and subsequently harvested.

**[0089.0.0.0]** The plants or parts thereof, e.g. the leaves, roots, flowers, and/or stems and/or other harvestable material as described below, can then be used directly as foodstuffs or animal feeds or else be further processed. Again, the amino acids can be purified further in the customary manner via extraction and precipitation or via ion exchangers and other methods known to the person skilled in the art and described herein below. Products which are suitable for various applications and which result from these different processing procedures are amino acids or amino acid compositions which can still comprise further plant components in different amounts, advantageously in the range of from 0 to 99% by weight, preferably from below 90% by weight, especially preferably below 80% by weight. The plants can also advantageously be used directly without further processing, e.g. as feed or for extraction.

**[0090.0.0.0]** The chemically pure respective fine chemical or chemically pure compositions comprising the respective fine chemical may also be produced by the process described above. To this end, the respective fine chemical or the compositions are isolated in the known manner from an organism according to the invention, such as the microorganisms, non-human animal or the plants, and/or their culture medium in which or on which the organisms had been grown,. These chemically pure respective fine chemical or said compositions are advantageous for applications in the field of the food industry, the cosmetics industry or the pharmaceutical industry.

**[0091.0.0.0]** Thus, the content of plant components and preferably also further impurities is as low as possible, and the abovementioned respective fine chemical is obtained in as pure form as possible. In these applications, the content of plant components advantageously amounts to less than 10%, preferably 1%, more preferably 0.1%, very especially preferably 0.01% or less.

**[0092.0.0.0]** Accordingly, the respective fine chemical produced by the present invention is at least 0,1% by weight pure, preferably more than 1% by weight pure, more preferred 10% by weight pure, even more preferred are more than 50, 60, 70 or 80% by weight purity, even more preferred are more than 90 weight-% purity, most preferred are 95% by weight, 99% by weight or more.

**[0093.0.0.0]** In this context, the amount of the respective fine chemical in a cell of the invention may be increased according to the process of the invention by at least a factor of 1.1, preferably at least a factor of 1.5; 2; or 5, especially preferably by at least a factor of 10 or 30, very especially preferably by at least a factor of 50, in comparison with the wild type, control or reference. Preferably, said increase is found a tissue, more preferred in an organism or in a harvestable part thereof.

**[0094.0.0.0]** In principle, the respective fine chemicals produced can be increased in two ways by the process according to the invention. The pool of free respective fine chemicals, in particular of the free respective fine chemical, and/or the content of protein-bound respective fine chemicals, in particular of the protein-bound respective fine chemical may advantageously be increased.

**[0095.0.0.0]** It may be advantageous to increase the pool of free amino acids in the transgenic organisms by the process according to the invention in order to isolate high amounts of the pure respective fine chemical.

**[0096.0.0.0]** In another preferred embodiment of the invention a combination of the increased expression of the nucleic acid sequence or the protein of the invention together with the transformation of a protein or polypeptid, which functions as a sink for the desired amino acid for example methionine, lysine or threonine in the organism is useful to increase the production of the respective fine chemical (see US 5,589,616, WO 96/38574, WO 97/07665, WO 97/28247, US 4,886,878, US 5,082,993 and US 5,670,635). Galili et al., Transgenic Res. 2000 showed, that enhancing the synthesis of threonine by a feed back insensitive aspartate kinase did not lead only to in increase in free threonine but also in protein bound threonine.

**[0097.0.0.0]** In may also be advantageous to increase the content of the protein-bound respective fine chemical.

**[0098.0.0.0]** In a preferred embodiment, the respective fine chemical (methionine) and/or threonine are produced in accordance with the invention and, if desired, are isolated. The production of further amino acids such as lysine and of amino acid mixtures by the process according to the invention is advantageous.

**[0099.0.0.0]** In the case of the fermentation of microorganisms, the abovementioned amino acids may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. This concentrated fermentation broth can subsequently be processed by lyophilization, spray drying, spray granulation or by other methods.

**[0100.0.0.0]** To purify an amino acid, a product-containing fermentation broth from which the biomass has been separated may be subjected to chromatography with a suitable resin such as ion exchange resin for example anion or cation exchange resin, hydrophobic resin or hydrophilic resin for example epoxy resin, polyurethane resin or polyacrylamid resin, or resin for separation according to the molecular weight of the compounds for example polyvinyl chloride homopolymer resin or resins composed for example of polymers of acrylic acid, crosslinked with polyalkenyl ethers or divinyl glycol such as Carbopol®, Pemulen® and Noveon®. If necessary these chromatography steps may be repeated using the same or other chromatography resins. The skilled worker is familiar with the choice of suitable chromatography resins and their most effective use. The purified product may be concentrated by filtration or ultrafiltration and stored at a temperature, which ensures the maximum stability of the product.

**[0101.0.0.0]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.0.0]** Amino acids can for example be detected advantageously via HPLC separation in ethanolic extract as described by Geigenberger et al. (Plant Cell & Environ, 19, 1996: 43-55). Amino acids can be extracted with hot water. After filtration the extracts are diluted with water containing 20 mg/mL sodium acide. The separation and detection of the amino acids is performed using an anion exchange column and an electrochemical detector. Technical details can be taken from Y. Ding et al., 2002, Direct determination of free amino acids and sugars in green tea by anion-exchange chromatography with integrated pulsed amperometric detection, J Chromatogr A, (2002) 982; 237-244, or e.g. from Karchi et al., 1993, Plant J. 3: 721-727; Matthews MJ, 1997 (Lysine, threonine and methionine biosynthesis. In BK Singh, ed, Plant Amino Acids: Biochemistry and Biotechnology. Dekker, New York, pp 205-225; H Hesse and R Hoefgen. (2003) Molecular aspects of methionine biosynthesis. TIPS 8(259-262.

**[0103.0.0.0]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of a polypeptide having a sequence as indicated in Table II, application no. 0, columns 5 or 7, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 0, columns 5 or 7;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, application no. 0, columns 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence having a sequences as indicated in Table IV, application no. 0, columns 7, and conferring an increase in the amount of the respective fine chemical in an organism or a part thereof ;
k) nucleic acid molecule comprising one or more of the nucleic acid molecule encoding the amino acid sequence of a polypeptide encoding a domain of a polypeptide indicated in Table II, application no. 0, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[0103.1.0.0.]** In one embodiment, the nucleic acid molecule used in the process of the invention distinguishes over the sequence indicated in Table I, application no. 0, columns 5 or 7, by one or more nucleotides. In one embodiment, the nucleic acid molecule used in the process of the invention does not consist of the sequence shown in indicated in Table I, application no. 0, columns 5 or 7. In one embodiment, the nucleic acid molecule used in the process of the invention is less than 100%, 99,999%, 99,99%, 99,9% or 99% identical to a sequence indicated in Table I, application no. 0, columns 5 or 7. In another embodiment, the nucleic acid molecule does not encode a polypeptide of a sequence indicated in Table II, application no. 0, columns 5 or 7.

**[0104.0.0.0]** In one embodiment, the nucleic acid molecule of the invention or used in the process of the invention distinguishes over the sequence indicated in Table I, application no. 0, columns 5 or 7, by one or more nucleotides. In one embodiment, the nucleic acid molecule of the invention or the nucleic acid used in the process of the invention does not consist of the sequence shown in indicated in Table I, application no. 0, columns 5 or 7: In one embodiment, the nucleic acid molecule of the present invention is less than 100%, 99,999%, 99,99%, 99,9% or 99% identical to a sequence indicated in Table I, application no. 0, columns 5 or 7. In another embodiment, the nucleic acid molecule does not encode a polypeptide of a sequence indicated in Table II, application no. 0, columns 5 or 7.

**[0105.0.0.0]** Unless otherwise specified, the terms "polynucleotides", "nucleic acid" and "nucleic acid molecule" are interchangeably in the present context. Unless otherwise specified, the terms "peptide", "polypeptide" and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)", "polynucleotide", "nucleic acid sequence", "nucleotide sequence", or "nucleic acid molecule(s)" as used herein refers to a polymeric form of nucleotides of any length, either ribonucleotides or deoxyribonucleotides. The terms refer only to the primary structure of the molecule.

**[0106.0.0.0]** Thus, the terms "gene(s)", "polynucleotide", "nucleic acid sequence", "nucleotide sequence", or "nucleic acid molecule(s)" as used herein include double-and single-stranded DNA and RNA. They also include known types of modifications, for example, methylation, "caps", substitutions of one or more of the naturally occurring nucleotides with an analog. Preferably, the DNA or RNA sequence of the invention comprises a coding sequence encoding the herein defined polypeptide.

**[0107.0.0.0]** A "coding sequence" is a nucleotide sequence, which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleotide sequences or genomic DNA, while introns may be present as well under certain circumstances.

**[0108.0.0.0]** Nucleic acid molecules with the sequence as indicated in Table I, application no. 0, columns 5 or 7, nucleic acid molecules which are derived from a amino acid sequences as indicated in Table II, application no. 0, columns 5 or 7, or from polypeptides comprising the consensus sequence as indicated in Table IV, application no. 0, columns 7, or their derivatives or homologues encoding polypeptides with the enzymatic or biological activity of a polypeptide as indicated in Table II, application no. 0, column 3, 5 or 7, or e.g. conferring a increase of the respective fine chemical after increasing its expression or activity are advantageously increased in the process according to the invention.

**[0109.0.0.0]** In one embodiment, said sequences are cloned into nucleic acid constructs, either individually or in combination. These nucleic acid constructs enable an optimal synthesis of the respective fine chemical produced in the process according to the invention.

**[0110.0.0.0]** Nucleic acid molecules, which are advantageous for the process according to the invention and which encode polypeptides with an activity of a polypeptide of the invention or the polypeptide used in the method of the invention or used in the process of the invention, e.g. of a protein as indicated in Table II, application no. 0, column 5, or being encoded by a nucleic acid molecule indicated in Table I, application no. 0, column 5, or of its homologs, e.g. as indicated in Table II, application no. 0, column 7, can be determined from generally accessible databases.

**[0111.0.0.0]** Those, which must be mentioned, in particular in this context are general gene databases such as the EMBL database (Stoesser G. et al., Nucleic Acids Res 2001, Vol. 29, 17-21), the GenBank database (Benson D.A. et al., Nucleic Acids Res 2000, Vol. 28,15-18), or the PIR database (Barker W. C. et al., Nucleic Acids Res. 1999, Vol. 27, 39-43). It is furthermore possible to use organism-specific gene databases for determining advantageous sequences, in the case of yeast for example advantageously the SGD database (Cherry J. M. et al., Nucleic Acids Res. 1998, Vol. 26, 73-80) or the MIPS database (Mewes H.W. et al., Nucleic Acids Res. 1999, Vol. 27, 44-48), in the case of E. coli the GenProtEC database (http://web.bham.ac.uk/bcm4ght6/res.html), and in the case of Arabidopsis the TAIR-database (Huala, E. et al., Nucleic Acids Res. 2001 Vol. 29(1), 102-5) or the MIPS database.

**[0112.0.0.0]** The nucleic acid molecules used in the process according to the invention take the form of isolated nucleic acid sequences, which encode polypeptides with an activity of a polypeptide as indicated in Table I, application no. 0, column3, or having the sequence of a polypeptide as indicated in Table II, application no. 0, columns 5 and 7, and conferring an increase of the respective fine chemical.

**[0113.0.0.0]** The nucleic acid sequence(s) used in the process for the production of the respective fine chemical in transgenic organisms originate advantageously from an eukaryote but may also originate from a prokaryote or an archebacterium, thus it can derived from e.g. a microorganism, an animal or a plant.

**[0114.0.0.0]** For the purposes of the invention, as a rule the plural is intended to encompass the singular and vice versa.

**[0115.0.0.0]** In order to improve the introduction of the nucleic acid sequences and the expression of the sequences in the transgenic organisms, which are used in the process, the nucleic acid sequences are incorporated into a nucleic acid construct and/or a vector. In addition to the herein described sequences which are used in the process according to the invention, further nucleic acid sequences, advantageously of biosynthesis genes of the respective fine chemical produced in the process according to the invention, may additionally be present in the nucleic acid construct or in the vector and may be introduced into the organism together. However, these additional sequences may also be introduced into the organisms via other, separate nucleic acid constructs or vectors.

**[0116.0.0.0]** Using the herein mentioned cloning vectors and transformation methods such as those which are published and cited in: Plant Molecular Biology and Biotechnology (CRC Press, Boca Raton, Florida), chapter 6/7, pp. 71-119 (1993); F.F. White, Vectors for Gene Transfer in Higher Plants; in: Transgenic Plants, vol. 1, Engineering and Utilization, Ed.: Kung and R. Wu, Academic Press, 1993, 15-38; B. Jenes et al., Techniques for Gene Transfer, in: Transgenic Plants, vol. 1, Engineering and Utilization, Ed.: Kung and R. Wu, Academic Press (1993), 128-143; Potrykus, Annu. Rev. Plant Physiol. Plant Molec. Biol. 42 (1991), 205-225)) and further cited below, the nucleic acids may be used for the recombinant modification of a wide range of organisms, in particular prokaryotic or eukaryotic microorganisms or plants, so that they become a better and more efficient producer of the respective fine chemical produced in the process according to the invention. This improved production, or production efficiency, of the respective fine chemical or products derived there from, such as modified proteins, can be brought about by a direct effect of the manipulation or by an indirect effect of this manipulation.

**[0117.0.0.0]** In one embodiment, the nucleic acid molecule according to the invention originates from a plant, such as a plant selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, lridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants and in particular plants mentioned herein above as host plants such as the families and genera mentioned above for example preferred the species *Anacardium occidentale, Calendula officinalis, Carthamus tinctorius, Cichorium intybus, Cynara scolymus, Helianthus annus, Tagetes lucida, Tagetes erecta, Tagetes tenuifolia; Daucus carota; Corylus avellana, Corylus colurna, Borago officinalis; Brassica napus, Brassica rapa* ssp., *Sinapis arvensis Brassica juncea, Brassica juncea var. juncea, Brassica juncea var. crispifolia, Brassica juncea var. foliosa, Brassica nigra, Brassica sinapioides, Melanosinapis communis, Brassica oleracea,* Arabidopsis thaliana, *Anana comosus, Ananas ananas, Bromelia comosa, Carica papaya, Cannabis sative, Ipomoea batatus, Ipomoea pandurata, Convolvulus batatas, Convolvulus tiliaceus, Ipomoea fastigiata, Ipomoea tiliacea, Ipomoea triloba, Convolvulus panduratus, Beta vulgaris, Beta vulgaris var. altissima, Beta vulgaris var. vulgaris, Beta maritima, Beta vulgaris var. perennis, Beta vulgaris var. conditiva, Beta vulgaris var. esculenta, Cucurbita maxima, Cucurbita mixta, Cucurbita pepo, Cucurbita moschata, Olea europaea, Manihot utilissima, Janipha manihot,, Jatropha manihot., Manihot aipil, Manihot dulcis, Manihot manihot, Manihot melanobasis, Manihot esculenta, Ricinus communis, Pisum sativum, Pisum arvense, Pisum humile, Medicago sativa, Medicago falcata, Medicago varia, Glycine max Dolichos soja, Glycine gracilis, Glycine hispida, Phaseolus max, Soja hispida, Soja max, Cocos nucifera, Pelargonium grossularioides, Oleum cocoas, Laurus nobilis, Persea americana, Arachis hypogaea, Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense, Linum trigynum, Punica granatum, Gossypium hirsutum, Gossypium arboreum, Gossypium barbadense, Gossypium herbaceum, Gossypium thurberi, Musa nana, Musa acuminata, Musa paradisiaca, Musa* spp., *Elaeis guineensis, Papaver orientale, Papaver rhoeas, Papaver dubium, Sesamum indicum, Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata, , Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum,* Avena sativa, *Avena fatua, Avena byzantina, Avena fatua var. sativa, Avena hybrida, Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum* millet, *Panicum militaceum, Zea mays, Triticum aestivum, Triticum durum, Triticum turgidum, Triticum hybernum, Triticum macha, Triticum sativum* or *Triticum vulgare, Cofea spp., Coffea arabica, Coffea canephora, Coffea liberica, Capsicum annuum, Capsicum annuum var. glabriusculum, Capsicum frutescens, Capsicum annuum, Nicotiana tabacum, Solanum tuberosum, Solanum melongena, Lycopersicon esculentum, Lycopersicon lycopersicum., Lycopersicon pyriforme, Solanum integrifolium, Solanum lycopersicum Theobroma cacao* or *Camellia sinensis.*

**[0118.0.0.0]** In one embodiment, the nucleic acid molecule sequence originates advantageously from a microorganism as mentioned above under host organism such as a fungus for example the genera Aspergillus, Penicillium or Claviceps or from yeasts such as the genera Pichia, Torulopsis, Hansenula, Schizosaccharomyces, Candida, Rhodotorula or Saccharomyces, very especially advantageously from the yeast of the family Saccharomycetaceae, such as the advantageous genus Saccharomyces and the very advantageous genus and species Saccharomyces cerevisiae for the production of the respective fine chemical in microorganims.

**[0119.0.0.0]** The skilled worker knows other suitable sources for the production of respective fine chemicals, which present also useful nucleic acid molecule sources. They include in general all prokaryotic or eukaryotic cells, preferably unicellular microorganisms, such as fungi like the genus Claviceps or Aspergillus or gram-positive bacteria such as the genera Bacillus, Corynebacterium, Micrococcus, Brevibacterium, Rhodococcus, Nocardia, Caseobacter or Arthrobacter or gram-negative bacteria such as the genera Escherichia, Flavobacterium or Salmonella, or yeasts such as the genera Rhodotorula, Hansenula or Candida.

**[0120.0.0.0]** Production strains which are especially advantageously selected in the process according to the invention are microorganisms selected from the group of the families Actinomycetaceae, Bacillaceae, Brevibacteriaceae, Corynebacteriaceae, Enterobacteriacae, Gordoniaceae, Micrococcaceae, Mycobacteriaceae, Nocardiaceae, Pseudomonaceae, Rhizobiaceae, Streptomycetaceae, Chaetomiaceae, Choanephoraceae, Cryptococcaceae, Cunninghamellaceae, Demetiaceae, Moniliaceae, Mortierellaceae, Mucoraceae, Pythiaceae, Sacharomycetaceae, Saprolegniaceae, Schizosacharomycetaceae, Sodariaceae, Sporobolomycetaceae, Tuberculariaceae, Adelotheciaceae, Dinophyceae, Ditrichaceae and Prasinophyceaeor of the genera and species consisting of Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Escherichia coli K12 and its described strains.

**[0121.0.0.0]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 0, columns 5 or 7, or the functional homologues thereof as described herein, preferably conferring above-mentioned activity, i.e. conferring a increase of the respective fine chemical after increasing its activity.

**[0122.0.0.0]** In the process according to the invention nucleic acid sequences can be used, which, if appropriate, contain synthetic, non-natural or modified nucleotide bases, which can be incorporated into DNA or RNA. Said synthetic, non-natural or modified bases can for example increase the stability of the nucleic acid molecule outside or inside a cell. The nucleic acid molecules of the invention can contain the same modifications as aforementioned.

**[0123.0.0.0]** As used in the present context the term "nucleic acid molecule" may also encompass the untranslated sequence located at the 3' and at the 5' end of the coding gene region, for example at least 500, preferably 200, especially preferably 100, nucleotides of the sequence upstream of the 5' end of the coding region and at least 100, preferably 50, especially preferably 20, nucleotides of the sequence downstream of the 3' end of the coding gene region. It is often advantageous only to choose the coding region for cloning and expression purposes.

**[0124.0.0.0]** Preferably, the nucleic acid molecule used in the process according to the invention or the nucleic acid molecule of the invention is an isolated nucleic acid molecule.

**[0125.0.0.0]** An "isolated" polynucleotide or nucleic acid molecule is separated from other polynucleotides or nucleic acid molecules, which are present in the natural source of the nucleic acid molecule. An isolated nucleic acid molecule may be a chromosomal fragment of several kb, or preferably, a molecule only comprising the coding region of the gene. Accordingly, an isolated nucleic acid molecule of the invention may comprise chromosomal regions, which are adjacent 5' and 3' or further adjacent chromosomal regions, but preferably comprises no such sequences which naturally flank the nucleic acid molecule sequence in the genomic or chromosomal context in the organism from which the nucleic acid molecule originates (for example sequences which are adjacent to the regions encoding the 5'- and 3'-UTRs of the nucleic acid molecule). In various embodiments, the isolated nucleic acid molecule used in the process according to the invention may, for example comprise less than approximately 5 kb, 4 kb, 3 kb, 2 kb, 1 kb, 0.5 kb or 0.1 kb nucleotide sequences which naturally flank the nucleic acid molecule in the genomic DNA of the cell from which the nucleic acid molecule originates.

**[0126.0.0.0]** The nucleic acid molecules used in the process, for example the polynucleotides of the invention or of a part thereof can be isolated using molecular-biological standard techniques and the sequence information provided herein. Also, for example a homologous sequence or homologous, conserved sequence regions at the DNA or amino acid level can be identified with the aid of comparison algorithms. The former can be used as hybridization probes under standard hybridization techniques (for example those described in Sambrook et al., Molecular Cloning: A Laboratory Manual. 2nd Ed., Cold Spring Harbor Laboratory, Cold Spring Harbor Laboratory Press, Cold Spring Harbor, NY, 1989) for isolating further nucleic acid sequences useful in this process.

**[0127.0.0.0]** A nucleic acid molecule encompassing a complete sequence of the nucleic acid molecules used in the process, for example the polynucleotide of the invention, or a part thereof may additionally be isolated by polymerase chain reaction, oligonucleotide primers based on this sequence or on parts thereof being used. For example, a nucleic acid molecule comprising the complete sequence or part thereof can be isolated by polymerase chain reaction using oligonucleotide primers which have been generated on the basis of this sequence for example, mRNA can be isolated from cells (for example by means of the guanidinium thiocyanate extraction method of Chirgwin et al. (1979) Biochemistry 18:5294-5299) and cDNA can be generated by means of reverse transcriptase (for example Moloney MLV reverse transcriptase, available from Gibco/BRL, Bethesda, MD, or AMV reverse transcriptase, obtainable from Seikagaku America, Inc., St.Petersburg, FL).

**[0128.0.0.0]** Synthetic oligonucleotide primers for the amplification, e.g. as the pairs indicated in Table III, application no. 0, columns 7, by means of polymerase chain reaction can be generated on the basis of a sequence shown herein, for example the sequence as indicated in Table I, application no. 0, columns 5 or 7, or the sequences derived from sequences as indicated in Table II, application no. 0, columns 5 or 7.

**[0129.0.0.0]** Moreover, it is possible to identify conserved regions from various organisms by carrying out protein sequence alignments with the polypeptide used in the process of the invention, in particular with sequences of the polypeptide of the invention or the polypeptide used in the method of the invention, from which conserved regions, and in turn, degenerate primers can be derived. Conserved region for the polypeptide of the invention or the polypeptide used in the method of the invention are indicated in the alignments shown in the figures. Conserved regions are those, which show a very little variation in the amino acid sequence in one particular position of several homologs from different origin. The consensus sequences indicated in Table IV, application no. 0, columns 7, are derived from said alignments.

**[0130.0.0.0]** Degenerated primers can then be utilized by PCR for the amplification of fragments of novel proteins having above-mentioned activity, e.g. conferring the increase of the respective fine chemical after increasing its expression or activity or further functional homologs of the polypeptide of the invention or the polypeptide used in the method of the invention from other organisms.

**[0131.0.0.0]** These fragments can then be utilized as hybridization probe for isolating the complete gene sequence. As an alternative, the missing 5' and 3' sequences can be isolated by means of RACE-PCR (rapid amplification of cDNA ends). A nucleic acid molecule according to the invention can be amplified using cDNA or, as an alternative, genomic DNA as template and suitable oligonucleotide primers, following standard PCR amplification techniques. The nucleic acid molecule amplified thus can be cloned into a suitable vector and characterized by means of DNA sequence analysis. Oligonucleotides, which correspond to one of the nucleic acid molecules used in the process, can be generated by standard synthesis methods, for example using an automatic DNA synthesizer.

**[0132.0.0.0]** Nucleic acid molecules which are advantageously for the process according to the invention can be isolated based on their homology to the nucleic acid molecules disclosed herein using the sequences or part thereof as hybridization probe and following standard hybridization techniques under stringent hybridization conditions. In this context, it is possible to use, for example, isolated nucleic acid molecules of at least 15, 20, 25, 30, 35, 40, 50, 60 or more nucleotides, preferably of at least 15, 20 or 25 nucleotides in length which hybridize under stringent conditions with the above-described nucleic acid molecules, in particular with those which encompass a nucleotide sequence of the nucleic acid molecule used in the process of the invention or encoding a protein used in the invention or of the nucleic acid molecule of the invention. Nucleic acid molecules with 30, 50, 100, 250 or more nucleotides may also be used.

**[0133.0.0.0]** The term "homology" means that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other plant varieties or species, or mutations. These mutations may occur naturally or may be obtained by mutagenesis techniques. The allelic variations may be naturally occurring allelic variants as well as synthetically produced or genetically engineered variants. Structurally equivalents can, for example, be identified by testing the binding of said polypeptide to antibodies or computer based predictions. Structurally equivalent have the similar immunological characteristic, e.g. comprise similar epitopes.

**[0134.0.0.0]** By "hybridizing" it is meant that such nucleic acid molecules hybridize under conventional hybridization conditions, preferably under stringent conditions such as described by, e.g., Sambrook (Molecular Cloning; A Laboratory Manual, 2nd Edition, Cold Spring Harbor Laboratory Press, Cold Spring Harbor, NY (1989)) or in Current Protocols in Molecular Biology, John Wiley & Sons, N. Y. (1989), 6.3.1-6.3.6.

**[0135.0.0.0]** According to the invention, DNA as well as RNA molecules of the nucleic acid of the invention can be used as probes. Further, as template for the identification of functional homologues Northern blot assays as well as Southern blot assays can be performed. The Northern blot assay advantageously provides further information about the expressed gene product: e.g. expression pattern, occurrence of processing steps, like splicing and capping, etc. The Southern blot assay provides additional information about the chromosomal localization and organization of the gene encoding the nucleic acid molecule of the invention.

**[0136.0.0.0]** A preferred, nonlimiting example of stringent hydridization conditions are hybridizations in 6 x sodium chloride/sodium citrate (= SSC) at approximately 45°C, followed by one or more wash steps in 0.2 x SSC, 0.1% SDS at 50 to 65°C, for example at 50°C, 55°C or 60°C. The skilled worker knows that these hybridization conditions differ as a function of the type of the nucleic acid and, for example when organic solvents are present, with regard to the temperature and concentration of the buffer. The temperature under "standard hybridization conditions" differs for example as a function of the type of the nucleic acid between 42°C and 58°C, preferably between 45°C and 50°C in an aqueous buffer with a concentration of 0.1 x 0.5 x, 1 x, 2x, 3x, 4x or 5 x SSC (pH 7.2). If organic solvent(s) is/are present in the abovementioned buffer, for example 50% formamide, the temperature under standard conditions is approximately 40°C, 42°C or 45°C. The hybridization conditions for DNA:DNA hybrids are preferably for example 0.1 x SSC and 20°C, 25°C, 30°C, 35°C, 40°C or 45°C, preferably between 30°C and 45°C. The hybridization conditions for DNA:RNA hybrids are preferably for example 0.1 x SSC and 30°C, 35°C, 40°C, 45°C, 50°C or 55°C, preferably between 45°C and 55°C. The abovementioned hybridization temperatures are determined for example for a nucleic acid approximately 100 bp (= base pairs) in length and a G + C content of 50% in the absence of formamide. The skilled worker knows to determine the hybridization conditions required with the aid of textbooks, for example the ones mentioned above, or from the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford.

**[0137.0.0.0]** A further example of one such stringent hybridization condition is hybridization at 4XSSC at 65°C, followed by a washing in 0.1XSSC at 65°C for one hour. Alternatively, an exemplary stringent hybridization condition is in 50 % formamide, 4XSSC at 42°C. Further, the conditions during the wash step can be selected from the range of conditions delimited by low-stringency conditions (approximately 2X SSC at 50°C) and high-stringency conditions (approximately 0.2X SSC at 50°C, preferably at 65°C) (20X SSC: 0.3M sodium citrate, 3M NaCl, pH 7.0). In addition, the temperature during the wash step can be raised from low-stringency conditions at room temperature, approximately 22°C, to higher-stringency conditions at approximately 65°C. Both of the parameters salt concentration and temperature can be varied simultaneously, or else one of the two parameters can be kept constant while only the other is varied. Denaturants, for example formamide or SDS, may also be employed during the hybridization. In the presence of 50% formamide, hybridization is preferably effected at 42°C. Relevant factors like i) length of treatment, ii) salt conditions, iii) detergent conditions, iv) competitor DNAs, v) temperature and vi) probe selection can be combined case by case so that not all possibilities can be mentioned herein.
Thus, in a preferred embodiment, Northern blots are prehybridized with Rothi-Hybri-Quick buffer (Roth, Karlsruhe) at 68°C for 2h. Hybridization with radioactive labelled probe is done overnight at 68°C. Subsequent washing steps are performed at 68°C with 1xSSC.
For Southern blot assays the membrane is prehybridized with Rothi-Hybri-Quick buffer (Roth, Karlsruhe) at 68°C for 2h. The hybridization with radioactive labelled probe is conducted over night at 68°C. Subsequently the hybridization buffer is discarded and the filter shortly washed using 2xSSC; 0,1% SDS. After discarding the washing buffer new 2xSSC; 0,1% SDS buffer is added and incubated at 68°C for 15 minutes. This washing step is performed twice followed by an additional washing step using 1xSSC; 0,1% SDS at 68°C for 10 min.

**[0138.0.0.0]** Some further examples of conditions for DNA hybridization (Southern blot assays) and wash step are shown herein below:
(1) Hybridization conditions can be selected, for example, from the following conditions:
   a) 4X SSC at 65°C,
   b) 6X SSC at 45°C,
   c) 6X SSC, 100 mg/ml denatured fragmented fish sperm DNA at 68°C,
   d) 6X SSC, 0.5% SDS, 100 mg/ml denatured salmon sperm DNA at 68°C,
   e) 6X SSC, 0.5% SDS, 100 mg/ml denatured fragmented salmon sperm DNA, 50% formamide at 42°C,
   f) 50% formamide, 4X SSC at 42°C,
   g) 50% (vol/vol) formamide, 0.1% bovine serum albumin, 0.1% Ficoll, 0.1% polyvinylpyrrolidone, 50 mM sodium phosphate buffer pH 6.5, 750 mM NaCl, 75 mM sodium citrate at 42°C,
   h) 2X or 4X SSC at 50°C (low-stringency condition), or
   i) 30 to 40% formamide, 2X or 4X SSC at 42°C (low-stringency condition).
(2) Wash steps can be selected, for example, from the following conditions:
   a) 0.015 M NaCI/0.0015 M sodium citrate/0.1% SDS at 50°C.
   b) 0.1X SSC at 65°C.
   c) 0.1X SSC, 0.5 % SDS at 68°C.
   d) 0.1X SSC, 0.5% SDS, 50% formamide at 42°C.
   e) 0.2X SSC, 0.1% SDS at 42°C.
   f) 2X SSC at 65°C (low-stringency condition).

**[0139.0.0.0]** Polypeptides having above-mentioned activity, i.e. conferring the respective fine chemical increase, derived from other organisms, can be encoded by other DNA sequences which hybridize to a sequences indicated in Table I, application no. 0, columns 5 or 7, under relaxed hybridization conditions and which code on expression for peptides having the methionine increasing activity.

**[0140.0.0.0]** Further, some applications have to be performed at low stringency hybridisation conditions, without any consequences for the specificity of the hybridisation. For example, a Southern blot analysis of total DNA could be probed with a nucleic acid molecule of the present invention and washed at low stringency (55°C in 2xSSPE0,1% SDS). The hybridisation analysis could reveal a simple pattern of only genes encoding polypeptides of the present invention or used in the process of the invention, e.g. having herein-mentioned activity of increasing the respective fine chemical. A further example of such low-stringent hybridization conditions is 4XSSC at 50°C or hybridization with 30 to 40% formamide at 42°C. Such molecules comprise those which are fragments, analogues or derivatives of the polypeptide of the invention or used in the process of the invention and differ, for example, by way of amino acid and/or nucleotide deletion(s), insertion(s), substitution (s), addition(s) and/or recombination (s) or any other modification(s) known in the art either alone or in combination from the above-described amino acid sequences or their underlying nucleotide sequence(s). However, it is preferred to use high stringency hybridisation conditions.

**[0141.0.0.0]** Hybridization should advantageously be carried out with fragments of at least 5, 10, 15, 20, 25, 30, 35 or 40 bp, advantageously at least 50, 60, 70 or 80 bp, preferably at least 90, 100 or 110 bp. Most preferably are fragments of at least 15, 20, 25 or 30 bp. Preferably are also hybridizations with at least 100 bp or 200, very especially preferably at least 400 bp in length. In an especially preferred embodiment, the hybridization should be carried out with the entire nucleic acid sequence with conditions described above.

**[0142.0.0.0]** The terms "fragment", "fragment of a sequence" or "part of a sequence" mean a truncated sequence of the original sequence referred to. The truncated sequence (nucleic acid or protein sequence) can vary widely in length; the minimum size being a sequence of sufficient size to provide a sequence with at least a comparable function and/or activity of the original sequence referred to or hybridising with the nucleic acid molecule of the invention or the nucleic acid molecule used in the method of the invention or used in the process of the invention under stringent conditions, while the maximum size is not critical. In some applications, the maximum size usually is not substantially greater than that required to provide the desired activity and/or function(s) of the original sequence.

**[0143.0.0.0]** Typically, the truncated amino acid sequence will range from about 5 to about 310 amino acids in length. More typically, however, the sequence will be a maximum of about 250 amino acids in length, preferably a maximum of about 200 or 100 amino acids. It is usually desirable to select sequences of at least about 10, 12 or 15 amino acids, up to a maximum of about 20 or 25 amino acids.

**[0144.0.0.0]** The term "epitope" relates to specific immunoreactive sites within an antigen, also known as antigenic determinates. These epitopes can be a linear array of monomers in a polymeric composition - such as amino acids in a protein - or consist of or comprise a more complex secondary or tertiary structure. Those of skill will recognize that immunogens (i.e., substances capable of eliciting an immune response) are antigens; however, some antigen, such as haptens, are not immunogens but may be made immunogenic by coupling to a carrier molecule. The term "antigen" includes references to a substance to which an antibody can be generated and/or to which the antibody is specifically immunoreactive.

**[0145.0.0.0]** In one embodiment the present invention relates to a epitope of the polypeptide of the present invention or used in the process of the present invention and conferring above mentioned activity, preferably conferring an increase in the respective fine chemical.

**[0146.0.0.0]** The term "one or several amino acids" relates to at least one amino acid but not more than that number of amino acids, which would result in a homology of below 50% identity. Preferably, the identity is more than 70% or 80%, more preferred are 85%, 90%, 91%, 92%, 93%, 94% or 95%, even more preferred are 96%, 97%, 98%, or 99% identity.

**[0147.0.0.0]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 0, columns 5 or 7 is one which is sufficiently complementary to one of said nucleotide sequences such that it can hybridize to one of said nucleotide sequences thereby forming a stable duplex. Preferably, the hybridisation is performed under stringent hybridization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.0.0]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 0, columns 5 or 7, or a functional portion thereof and preferably has above mentioned activity, in particular has the-fine-chemical-increasing activity after increasing its activity or an activity of a product of a gene encoding said sequence or its homologs.

**[0149.0.0.0]** The nucleic acid molecule of the invention or the nucleic acid molecule used in the method of the invention comprises a nucleotide sequence which hybridises, preferably hybridises under stringent conditions as defined herein, to one of the nucleotide sequences indicated in Table I, application no. 0, columns 5 or 7, or a portion thereof and encodes a protein having above-mentioned activity and as indicated in indicated in Table II, application no. 0, columns 5 or 7, e.g. conferring an increase of the respective fine chemical.

**[0149.1.0.0]** Optionally, the nucleotide sequence, which hybridises to one of the nucleotide sequences indicated in Table I, application no. 0, columns 5 or 7, has further one or more of the activities annotated or known for the a protein as indicated in Table II, application no. 0, column 3.

**[0150.0.0.0]** Moreover, the nucleic acid molecule of the invention or used in the process of the invention can comprise only a portion of the coding region of one of the sequences indicated in Table I, application no. 0, columns 5 or 7, for example a fragment which can be used as a probe or primer or a fragment encoding a biologically active portion of the polypeptide of the present invention or of a polypeptide used in the process of the present invention, i.e. having above-mentioned activity, e.g. conferring an increase of methionine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, application no. 0, columns 5 or 7, an anti-sense sequence of one of the sequences indicated in Table I, application no. 0, columns 5 or 7, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 0, column 7, will result in a fragment of a polynucleotide sequence as indicated in Table I, application no. 0, columns 5 or 7.

**[0151.0.0.0]** Primer sets are interchangeable. The person skilled in the art knows to combine said primers to result in the desired product, e.g. in a full-length clone or a partial sequence. Probes based on the sequences of the nucleic acid molecule of the invention or used in the process of the present invention can be used to detect transcripts or genomic sequences encoding the same or homologous proteins. The probe can further comprise a label group attached thereto, e.g. the label group can be a radioisotope, a fluorescent compound, an enzyme, or an enzyme co-factor. Such probes can be used as a part of a genomic marker test kit for identifying cells which express an polypeptide of the invention or used in the process of the present invention, such as by measuring a level of an encoding nucleic acid molecule in a sample of cells, e.g., detecting mRNA levels or determining, whether a genomic gene comprising the sequence of the polynucleotide of the invention or used in the processes of the present invention has been mutated or deleted.

**[0152.0.0.0]** The nucleic acid molecule of the invention or the nucleic acid molecule used in the method of the invention encodes a polypeptide or portion thereof which includes an amino acid sequence which is sufficiently homologous to an amino acid sequence as indicated in Table II, application no. 0, columns 5 or 7, such that the protein or portion thereof maintains the ability to participate in the respective fine chemical production, in particular a methionine increasing activity as mentioned above or as described in the examples in plants or microorganisms is comprised.

**[0153.0.0.0]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 0, columns 5 or 7, such that the protein or portion thereof is able to participate in the increase of the respective fine chemical production. In one embodiment, a protein or portion thereof as indicated in Table II, application no. 0, columns 5 or 7, has for example an activity of a polypeptide indicated in Table II, application no. 0, column 3.

**[0154.0.0.0]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, application no. 0, columns 5 or 7, and has above-mentioned activity, e.g. conferring preferably the increase of the respective fine chemical.

**[0155.0.0.0]** Portions of proteins encoded by the nucleic acid molecule of the invention or the nucleic acid molecule used in the method of the invention are preferably biologically active, preferably having above-mentioned annotated activity, e.g. conferring a increase the respective fine chemical after increase of activity.

**[0156.0.0.0]** As mentioned herein, the term "biologically active portion" is intended to include a portion, e.g., a domain/motif, that confers increase of the respective fine chemical or has an immunological activity such that it is binds to an antibody binding specifically to the polypeptide of the present invention or a polypeptide used in the process of the present invention for producing the respective fine chemical;

**[0157.0.0.0]** The invention further relates to nucleic acid molecules that differ from one of the nucleotide sequences indicated in Table I, application no. 0, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, application no. 0, columns 5 or 7, or of the polypeptide as indicated in Table II, application no. 0, columns 5 or 7, or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, application no. 0, 7, or of the polypeptide as indicated in Table II, application no. 0, columns 5 or 7or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, application no. 0, columns 7, or of a polypeptide as indicated in Table II, application no. 0, columns 5 or 7, or the functional homologues thereof. However, in a one embodiment, the nucleic acid molecule of the present invention does not consist of a sequence as indicated in Table I, application no. 0, columns 5 or 7,

**[0158.0.0.0]** In addition, it will be appreciated by those skilled in the art that DNA sequence polymorphisms that lead to changes in the amino acid sequences may exist within a population. Such genetic polymorphism in the gene encoding the polypeptide of the invention or the polypeptide used in the method of the invention or comprising the nucleic acid molecule of the invention or the nucleic acid molecule used in the method of the invention may exist among individuals within a population due to natural variation.

**[0159.0.0.0]** As used herein, the terms "gene" and "recombinant gene" refer to nucleic acid molecules comprising an open reading frame encoding the polypeptide of the invention or the polypeptide used in the method of the invention or comprising the nucleic acid molecule of the invention or the nucleic acid molecule used in the method of the invention or encoding the polypeptide used in the process of the present invention, preferably from a crop plant or from a microorganism useful for the production of respective fine chemicals, in particular for the production of the respective fine chemical. Such natural variations can typically result in 1-5% variance in the nucleotide sequence of the gene. Any and all such nucleotide variations and resulting amino acid polymorphisms in genes encoding a polypeptide of the invention or the polypeptide used in the method of the invention or comprising a the nucleic acid molecule of the invention or the nucleic acid molecule used in the method of the invention that are the result of natural variation and that do not alter the functional activity as described are intended to be within the scope of the invention.

**[0160.0.0.0]** Nucleic acid molecules corresponding to natural variants homologues of a nucleic acid molecule of the invention or the nucleic acid molecule used in the method of the invention, which can also be a cDNA, can be isolated based on their homology to the nucleic acid molecules disclosed herein using the nucleic acid molecule of the invention or the nucleic acid molecule used in the method of the invention, or a portion thereof, as a hybridization probe according to standard hybridization techniques under stringent hybridization conditions.

**[0161.0.0.0]** Accordingly, in another embodiment, a nucleic acid molecule of the invention or the nucleic acid molecule used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 0, columns 5 or 7. The nucleic acid molecule is preferably at least 20, 30, 50, 100, 250 or more nucleotides in length.

**[0162.0.0.0]** The term "hybridizes under stringent conditions" is defined above. In one embodiment, the term "hybridizes under stringent conditions" is intended to describe conditions for hybridization and washing under which nucleotide sequences at least 30 %, 40 %, 50 % or 65% identical to each other typically remain hybridized to each other. Preferably, the conditions are such that sequences at least about 70%, more preferably at least about 75% or 80%, and even more preferably at least about 85%, 90% or 95% or more identical to each other typically remain hybridized to each other.

**[0163.0.0.0]** Preferably, the nucleic acid molecule of the invention or the nucleic acid molecule used in the method of the invention that hybridizes under stringent conditions to a sequence as indicated in Table I, application no. 0, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of an protein of the invention or used in the process of the invention.

**[0164.0.0.0]** In addition to naturally-occurring variants of the sequences of the polypeptide or nucleic acid molecule of the invention as well as of the polypeptide or nucleic acid molecule used in the process of the invention that may exist in the population, the skilled artisan will further appreciate that changes can be introduced by mutation into a nucleotide sequence of the nucleic acid molecule encoding the polypeptide of the invention or used in the process of the present invention, thereby leading to changes in the amino acid sequence of the encoded said polypeptide, without altering the functional ability of the polypeptide, preferably not decreasing said activity.

**[0165.0.0.0]** For example, nucleotide substitutions leading to amino acid substitutions at "non-essential" amino acid residues can be made in a sequence of the nucleic acid molecule of the invention or used in the process of the invention, e.g. as indicated in Table I, application no. 0, columns 5 or 7.

**[0166.0.0.0]** A "non-essential" amino acid residue is a residue that can be altered from the wild-type sequence of one without altering the activity of said polypeptide, whereas an "essential" amino acid residue is required for an activity as mentioned above, e.g. leading to an increase in the respective fine chemical in an organism after an increase of activity of the polypeptide. Other amino acid residues, however, (e.g., those that are not conserved or only semi-conserved in the domain having said activity) may not be essential for activity and thus are likely to be amenable to alteration without altering said activity.

**[0167.0.0.0]** Further, a person skilled in the art knows that the codon usage between organism can differ. Therefore, he may adapt the codon usage in the nucleic acid molecule of the present invention to the usage of the organism in which the polynucleotide or polypeptide is expressed.

**[0168.0.0.0]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table II, application no. 0, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 0, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 0, columns 5 or 7, more preferably at least about 70% identical to one of the sequences as indicated in Table II, application no. 0, columns 5 or 7, even more preferably at least about 80%, 90%, or 95% homologous to a sequence as indicated in Table II, application no. 0, columns 5 or 7, and most preferably at least about 96%, 97%, 98%, or 99% identical to the sequence as indicated in Table II, application no. 0, columns 5 or 7.

**[0169.0.0.0]** To determine the percentage homology(= identity) of two amino acid sequences or of two nucleic acid molecules, the sequences are written one underneath the other for an optimal comparison (for example gaps may be inserted into the sequence of a protein or of a nucleic acid in order to generate an optimal alignment with the other protein or the other nucleic acid).

**[0170.0.0.0]** The amino acid residues or nucleic acid molecules at the corresponding amino acid positions or nucleotide positions are then compared. If a position in one sequence is occupied by the same amino acid residue or the same nucleic acid molecule as the corresponding position in the other sequence, the molecules are homologous at this position (i.e. amino acid or nucleic acid "homology" as used in the present context corresponds to amino acid or nucleic acid "identity". The percentage homology between the two sequences is a function of the number of identical positions shared by the sequences (i.e. % homology = number of identical positions/total number of positions x 100). The terms "homology" and "identity" are thus to be considered as synonyms.

**[0171.0.0.0]** For the determination of the percentage homology (=identity) of two or more amino acids or of two or more nucleotide sequences several computer software programs have been developed. The homology of two or more sequences can be calculated with for example the software fasta, which presently has been used in the version fasta 3 (W. R. Pearson and D. J. Lipman (1988), Improved Tools for Biological Sequence Comparison. PNAS 85:2444- 2448; W. R. Pearson (1990) Rapid and Sensitive Sequence Comparison with FASTP and FASTA, Methods in Enzymology 183:63 - 98; W. R. Pearson and D. J. Lipman (1988) Improved Tools for Biological Sequence Comparison. PNAS 85:2444- 2448; W. R. Pearson (1990); Rapid and Sensitive Sequence Comparison with FASTP and FASTA Methods in Enzymology 183:63 - 98). Another useful program for the calculation of homologies of different sequences is the standard blast program, which is included in the Biomax pedant software (Biomax, Munich, Federal Republic of Germany). This leads unfortunately sometimes to suboptimal results since blast does not always include complete sequences of the subject and the query. Nevertheless as this program is very efficient it can be used for the comparison of a huge number of sequences. The following settings are typically used for such a comparisons of sequences: -p Program Name [String]; -d Database [String]; default = nr; -i Query File [File In]; default = stdin; -e Expectation value (E) [Real]; default = 10.0; -m alignment view options: 0 = pairwise; 1 = query-anchored showing identities; 2 = query-anchored no identities; 3 = flat query-anchored, show identities; 4 = flat query-anchored, no identities; 5 = query-anchored no identities and blunt ends; 6 = flat query-anchored, no identities and blunt ends; 7 = XML Blast output; 8 = tabular; 9 tabular with comment lines [Integer]; default = 0; -o BLAST report Output File [File Out] Optional; default = stdout; -F Filter query sequence (DUST with blastn, SEG with others) [String]; default = T; -G Cost to open a gap (zero invokes default behavior) [Integer]; default = 0; -E Cost to extend a gap (zero invokes default behavior) [Integer]; default = 0; -X X dropoff value for gapped alignment (in bits) (zero invokes default behavior); blastn 30, megablast 20, tblastx 0, all others 15 [Integer]; default = 0; -I Show GI's in deflines [T/F]; default = F; -q Penalty for a nucleotide mismatch (blastn only) [Integer]; default = -3; -r Reward for a nucleotide match (blastn only) [Integer]; default = 1; -v Number of database sequences to show one-line descriptions for (V) [Integer]; default = 500; -b Number of database sequence to show alignments for (B) [Integer]; default = 250; -f Threshold for extending hits, default if zero; blastp 11, blastn 0, blastx 12, tblastn 13; tblastx 13, megablast 0 [Integer]; default = 0; -g Perfom gapped alignment (not available with tblastx) [T/F]; default = T; -Q Query Genetic code to use [Integer]; default = 1; -D DB Genetic code (for tblast[nx] only) [Integer]; default = 1; -a Number of processors to use [Integer]; default = 1; -O SeqAlign file [File Out] Optional; -J Believe the query defline [T/F]; default = F; -M Matrix [String]; default = BLOSUM62; - W Word size, default if zero (blastn 11, megablast 28, all others 3) [Integer]; default = 0; -z Effective length of the database (use zero for the real size) [Real]; default = 0; -K Number of best hits from a region to keep (off by default, if used a value of 100 is recommended) [Integer]; default = 0; -P 0 for multiple hit, 1 for single hit [Integer]; default = 0; -Y Effective length of the search space (use zero for the real size) [Real]; default = 0; -S Query strands to search against database (for blast[nx], and tblastx) ; 3 is both, 1 is top, 2 is bottom [Integer]; default = 3; -T Produce HTML output [T/F]; default = F; -I Restrict search of database to list of GI's [String] Optional; -U Use lower case filtering of FASTA sequence [T/F] Optional; default = F; -y X dropoff value for ungapped extensions in bits (0.0 invokes default behavior); blastn 20, megablast 10, all others 7 [Real]; default = 0.0; -Z X dropoff value for final gapped alignment in bits (0.0 invokes default behavior); blastn/megablast 50, tblastx 0, all others 25 [Integer]; default = 0; -R PSI-TBLASTN checkpoint file [File In] Optional; -n MegaBlast search [T/F]; default = F; -L Location on query sequence [String] Optional; -A Multiple Hits window size, default if zero (blastn/megablast 0, all others 40 [Integer]; default = 0; -w Frame shift penalty (OOF algorithm for blastx) [Integer]; default = 0; -t Length of the largest intron allowed in tblastn for linking HSPs (0 disables linking) [Integer]; default = 0.

**[0172.0.0.0]** Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351-360, 1987, Higgins et al., CABIOS, 5 1989: 151-153) or preferably with the programs Gap and BestFit, which are respectively based on the algorithms of Needleman and Wunsch [J. Mol. Biol. 48; 443-453 (1970)] and Smith and Waterman [Adv. Appl. Math. 2; 482-489 (1981)]. Both programs are part of the GCG software-package [Genetics Computer Group, 575 Science Drive, Madison, Wisconsin, USA 53711 (1991); Altschul et al. (1997) Nucleic Acids Res. 25:3389 et seq.]. Therefore preferably the calculations to determine the percentages of sequence homology are done with the program Gap over the whole range of the sequences. The following standard adjustments for the comparison of nucleic acid sequences were used: gap weight: 50, length weight: 3, average match: 10.000, average mismatch: 0.000. **[0173.0.0.0]** For example a sequence which has a 80% homology with sequence SEQ ID NO: 25at the nucleic acid level is understood as meaning a sequence which, upon comparison with the sequence SEQ ID NO: 25 by the above Gap program algorithm with the above parameter set, has a 80% homology.

**[0174.0.0.0]** In the state of the art, homology between two polypeptides is also understood as meaning the identity of the amino acid sequence over in each case the entire sequence length which is calculated by comparison with the aid of the program algorithm GAP (Wisconsin Package Version 10.0, University of Wisconsin, Genetics Computer Group (GCG), Madison, USA), setting the following parameters:

| | | | |
|---|---|---|---|
| Gap weight: | 8 | Length weight: | 2 |
| Average match: | 2,912 | Average mismatch: | -2,003 |

**[0175.0.0.0]** For example a sequence which has a 80% homology with sequence SEQ ID NO: 26 at the protein level is understood as meaning a sequence which, upon comparison with the sequence SEQ ID NO: 26 by the above program algorithm with the above parameter set, has a 80% homology.

**[0176.0.0.0]** Functional equivalents derived from one of the polypeptides as indicated in Table II, application no. 0, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 0, columns 5 or 7, according to the invention and are distinguished by essentially the same properties as a polypeptide as indicated in Table II, application no. 0, columns 5 or 7.

**[0177.0.0.0]** Functional equivalents derived from a nucleic acid sequence as indicated in Table I, application no. 0, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptide as indicated in Table II, application no. 0, columns 5 or 7, according to the invention and encode polypeptides having essentially the same properties as a polypeptide as indicated in Table II, application no. 0, columns 5 or 7. **[0178.0.0.0]** "Essentially the same properties" of a functional equivalent is above all understood as meaning that the functional equivalent has above mentioned activity, e.g. conferring an increase in the respective fine chemical amount while increasing the amount of protein, activity or function of said functional equivalent in an organism, e.g. a microorganism, a plant or plant or animal tissue, plant or animal cells or a part of the same.

**[0179.0.0.0]** A nucleic acid molecule encoding a homologous to a protein sequence of as indicated in Table II, application no. 0, columns 5 or 7, can be created by introducing one or more nucleotide substitutions, additions or deletions into a nucleotide sequence of the nucleic acid molecule of the present invention, in particular as indicated in Table I, application no. 0, columns 5 or 7, such that one or more amino acid substitutions, additions or deletions are introduced into the encoded protein. Mutations can be introduced into the encoding sequences for example into sequences as indicated in Table I, application no. 0, columns 5 or 7, by standard techniques, such as site-directed mutagenesis and PCR-mediated mutagenesis.

**[0180.0.0.0]** Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

**[0181.0.0.0]** Thus, a predicted nonessential amino acid residue in a polypeptide of the invention or a polypeptide used in the process of the invention is preferably replaced with another amino acid residue from the same family. Alternatively, in another embodiment, mutations can be introduced randomly along all or part of a coding sequence of a nucleic acid molecule of the invention or used in the process of the invention, such as by saturation mutagenesis, and the resultant mutants can be screened for activity described herein to identify mutants that retain or even have increased above mentioned activity, e.g. conferring an increase in content of the respective fine chemical.

**[0182.0.0.0]** Following mutagenesis of one of the sequences shown herein, the encoded protein can be expressed recombinantly and the activity of the protein can be determined using, for example, assays described herein (see Examples).

**[0183.0.0.0]** The highest homology of the nucleic acid molecule used in the process according to the invention was found for the following database entries by Gap search.

**[0184.0.0.0]** Homologues of the nucleic acid sequences used, with a sequence as indicated in Table I, application no. 0, columns 5 or 7, or of the nucleic acid sequences derived from a sequences as indicated in Table II, application no. 0, columns 5 or 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 1 to 5 and/or lines 334 to 338, or from the derived nucleic acid sequences, the intention being, however, that the enzyme activity or the biological activity of the resulting proteins synthesized is advantageously retained or increased.

**[0185.0.0.0]** In one embodiment of the present invention, the nucleic acid molecule of the invention or used in the process of the invention comprises one or more sequences as indicated in Table I, application no. 0, columns 5 or 7. In one embodiment, it is preferred that the nucleic acid molecule comprises as little as possible other nucleotide sequences not shown in any one of sequences as indicated in Table I, application no. 0, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 0, columns 5 or 7.

**[0186.0.0.0]** Also preferred is that one or more nucleic acid molecule(s) used in the process of the invention encode a polypeptide comprising a sequence as indicated in Table II, application no. 0, columns 5 or 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 0, columns 5 or 7.

**[0187.0.0.0]** In one embodiment, the nucleic acid molecule of the invention or used in the process encodes a polypeptide comprising a sequence as indicated in Table II, application no. 0, columns 5 or 7, comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 0, columns 5 or 7.

**[0188.0.0.0]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 0, columns 5 or 7, preferably compared to a sequence as indicated in Table II, application no. 0, column 5, and expressed under identical conditions.

**[0189.0.0.0]** Homologues of a sequence as indicated in Table I, application no. 0, columns 5 or 7, or of a derived sequence as indicated in Table II, application no. 0, columns 5 or 7, also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.0]** In a further embodiment, the process according to the present invention comprises the following steps:
(a) selecting an organism or a part thereof expressing the polypeptide of this invention;
(b) mutagenizing the selected organism or the part thereof;
(c) comparing the activity or the expression level of said polypeptide in the mutagenized organism or the part thereof with the activity or the expression of said polypeptide in the selected organisms or the part thereof;
(d) selecting the mutagenized organisms or parts thereof, which comprise an increased activity or expression level of said polypeptide compared to the selected organism (a) or the part thereof;
(e) optionally, growing and cultivating the organisms or the parts thereof; and
(f) recovering, and optionally isolating, the free or bound respective fine chemical produced by the selected mutated organisms or parts thereof.

**[0191.0.0.0]** The organisms or part thereof produce according to the herein mentioned process of the invention an increased level of free and/or-bound respective fine chemical compared to said control or selected organisms or parts thereof.

**[0191.1.0.0]** In one embodiment, the organisms or part thereof produce according to the herein mentioned process of the invention an increased level of protein-bound respective fine chemical compared to said control or selected organisms or parts thereof.

**[0192.0.0.0]** Advantageously the selected organisms are mutagenized according to the invention. According to the invention mutagenesis is any change of the genetic information in the genome of an organism, that means any structural or compositional change in the nucleic acid preferably DNA of an organism that is not caused by normal segregation or genetic recombination processes. Such mutations may occur spontaneously, or may be induced by mutagens as described below. Such change can be induced either randomly or selectively. In both cases the genetic information of the organism is modified. In general this lead to the situation that the activity of the gene product of the relevant genes inside the cells or inside the organism is increased.

**[0193.0.0.0]** In case of the specific or so called site directed mutagenesis a distinct gene is mutated and thereby its activity and/or the activity or the encoded gene product is repressed, reduced or increased, preferably increased. In the event of a random mutagenesis one or more genes are mutated by chance and their activities and/or the activities of their gene products are repressed, reduced or increased, preferably increased.

**[0194.0.0.0]** For the purpose of a mutagenesis of a huge population of organisms, such population can be transformed with a DNA construct, which is useful for the activation of as much as possible genes of an organism, preferably all genes. For example the construct can contain a strong promoter or one or more enhancers, which are capable of transcriptionally activate genes in the vicinity of their integration side. With this method it is possible to statistically mutagenize, e.g. activate nearly all genes of an organism by the random integration of an activation construct. Afterwards the skilled worker can identify those mutagenized lines in which a gene of the invention has been activated, which in turns leads to the desired increase in the respective fine chemical production .

**[0195.0.0.0]** The genes of the invention can also be activated by mutagenesis, either of regulatory or coding regions. In the event of a random mutagenesis a huge number of organisms are treated with a mutagenic agent. The amount of said agent and the intensity of the treatment will be chosen in such a manner that statistically nearly every gene is mutated once. The process for the random mutagenesis as well as the respective agens is well known by the skilled person. Such methods are disclosed for example by A.M. van Harten [(1998), "Mutation breeding: theory and practical applications", Cambridge University Press, Cambridge, UK], E Friedberg, G Walker, W Siede [(1995), "DNA Repair and Mutagenesis", Blackwell Publishing], or K. Sankaranarayanan, J. M. Gentile, L. R. Ferguson [(2000) "Protocols in Mutagenesis", Elsevier Health Sciences]. As the skilled worker knows the spontaneous mutation rate in the cells of an organism is very low and that a large number of chemical, physical or biological agents are available for the mutagenesis of organisms. These agents are named as mutagens or mutagenic agents. As mentioned before three different kinds of mutagens (chemical, physical or biological agents) are available.

**[0196.0.0.0]** There are different classes of chemical mutagens, which can be separated by their mode of action. For example base analogues such as 5-bromouracil, 2-amino purin. Other chemical mutagens are interacting with the DNA such as sulphuric acid, nitrous acid, hydroxylamine; or other alkylating agents such as monofunctional agents like ethyl methanesulfonate, dimethylsulfate, methyl methanesulfonate), bifunctional like dichloroethyl sulphide, Mitomycin, Nitrosoguanidine - dialkylnitrosamine, N-Nitrosoguanidin derivatives, N-alkyl-N-nitro-N-nitroso-guanidine-), ntercalating dyes like Acridine, ethidium bromide).

**[0197.0.0.0]** Physical mutagens are for example ionizing irradiation (X ray), UV irradiation. Different forms of irradiation are available and they are strong mutagens. Two main classes of irradiation can be distinguished: a) non-ionizing irradiation such as UV light or ionizing irradiation such as X ray. Biological mutagens are for example transposable elements for example IS elements such as IS100, transposons such as Tn5, Tn10, Tn916 or Tn1000 or phages like Muamplac, P1, T5, λplac etc. Methods for introducing this phage DNA into the appropriate microorganism are well known to the skilled worker (see Microbiology, Third Edition, Eds. Davis, B.D., Dulbecco, R., Eisen, H.N. and Ginsberg, H.S., Harper International Edition, 1980). The common procedure of a transposon mutagenesis is the insertion of a transposable element within a gene or nearby for example in the promotor or terminator region and thereby leading to a loss of the gene function. Procedures to localize the transposon within the genome of the organisms are well known by a person skilled in the art.

**[0198.0.0.0]** Preferably a chemical or biochemical procedure is used for the mutagenesis of the organisms. A preferred chemical method is the mutagenesis with N-methyl-N-nitro-nitrosoguanidine.

**[0199.0.0.0]** Other biological method are disclosed by Spee et al. (Nucleic Acids Research, Vol. 21, No. 3, 1993: 777 - 778). Spee et al. teaches a PCR method using dITP for the random mutagenesis. This method described by Spee et al. was further improved by Rellos et al. (Protein Expr. Purif., 5, 1994 : 270 - 277). The use of an in vitro recombination technique for molecular mutagenesis is described by Stemmer (Proc. Natl. Acad. Sci. USA, Vol. 91, 1994: 10747 - 10751). Moore et al. (Nature Biotechnology Vol. 14, 1996: 458-467) describe the combination of the PCR and recombination methods for increasing the enzymatic activity of an esterase toward a para-nitrobenzyl ester. Another route to the mutagenesis of enzymes is described by Greener et al. in Methods in Molecular Biology (Vol. 57, 1996: 375 - 385). Greener et al. use the specific Escherichia coli strain XL1-Red to generate Escherichia coli mutants which have increased antibiotic resistance.

**[0200.0.0.0]** In one embodiment, the protein according to the invention or the nucleic acid molecule characterized herein originates from a eukaryotic or prokaryotic organism such as a non-human animal, a plant, a microorganism such as a fungi, a yeast, an alga, a diatom or a bacterium. Nucleic acid molecules, which advantageously can be used in the process of the invention originate from yeasts, for example the family Saccharomycetaceae, in particular the genus Saccharomyces, or yeast genera such as Candida, Hansenula, Pichia, Yarrowia, Rhodotorula or Schizosaccharomyces and the especially advantageous from the species Saccharomyces cerevisiae.

**[0201.0.0.0]** In one embodiment, nucleic acid molecules, which advantageously can be used in the process of the invention originate from bacteria, for example from Proteobacteria, in particular from Gammaproteobacteria, more preferred from Enterobacteriales, e.g. from the family Enterobacteriaceae, particularly from genera Escherichia, Salmonella, Klebsiella, advantageously form the species Escherichia coli K12.

**[0202.0.0.0]** If, in the process according to the invention, plants are selected as the donor organism, this plant may, in principle, be in any phylogenetic relation of the recipient plant. Donor and recipient plant may belong to the same family, genus, species, variety or line, resulting in an increasing homology between the nucleic acids to be integrated and corresponding parts of the genome of the recipient plant. This also applies analogously to microorganisms as donor and recipient organism.
It might also be advantageously to use nuclei acids molecules from very distinct species, since these might exhibit reduced sensitivity against endogenous regulatory mechanisms and such sequences might not be recognized by endogenous silencing mechanisms.

**[0203.0.0.0]** Accordingly, one embodiment of the application relates to the use of nucleic acid molecules in the process of the invention from plants, e.g. crop plants, e.g. from: B. napus; Glycine max; sunflower linseed or maize or their homologues.

**[0204.0.0.0]** Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 0, columns 5 or 7, or a fragment thereof conferring an increase in the amount of the respective fine chemical in an organism or a part thereof
b) nucleic acid molecule comprising, preferably at least the mature form, of a nucleic acid molecule as indicated in Table I, application no. 0, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using primers or primer pairs as indicated in Table III, application no. 0, column 7, and conferring an increase in the amount of the respective fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, application no. 0, columns 7, and conferring an increase in the amount of the respective fine chemical in an organism or a part thereof;
k) nucleic acid molecule encoding the amino acid sequence of a polypeptide encoding a domain of a polypeptide as indicated in Table II, application no. 0, columns 5 or 7, and conferring an increase in the amount of the respective fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 0, columns 5 or 7, or a nucleic acid molecule encoding, preferably at least the mature form of, the polypeptide as indicated in Table II, application no. 0, columns 5 or 7, and conferring an increase in the amount of the respective fine chemical in an organism or a part thereof;
or which encompasses a sequence which is complementary thereto; whereby, preferably, the nucleic acid molecule according to (a) to (I) distinguishes over the sequence indicated in Table I, application no. 0, columns 5 or 7, by one or more nucleotides. In one embodiment, the nucleic acid molecule does not consist of the sequence shown and indicated in Table I, application no. 0, columns 5 or 7: In one embodiment, the nucleic acid molecule is less than 100%, 99,999%, 99,99%, 99,9% or 99% identical to a sequence indicated in Table I, application no. 0, columns 5 or 7. In another embodiment, the nucleic acid molecule does not encode a polypeptide of a sequence indicated in Table II, application no. 0, columns 5 or 7. In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 0, columns 5 or 7. In a further embodiment the nucleic acid molecule does not encode a polypeptide sequence as indicated in Table II, application no. 0, columns 5 or 7. Accordingly, in one embodiment, the nucleic acid molecule of the differs at least in one or more residues from a nucleic acid molecule indicated in Table I, application no. 0, columns 5 or 7. Accordingly, in one embodiment, the nucleic acid molecule of the present invention encodes a polypeptide, which differs at least in one or more amino acids from a polypeptide indicated in Table II, application no. 0, columns 5 or 7. In another embodiment, a nucleic acid molecule indicated in Table I, application no. 0, columns 5 or 7, does not encode a protein of a sequence indicated in Table II, application no. 0, columns 5 or 7. Accordingly, in one embodiment, the protein encoded by a sequences of a nucleic acid according to (a) to (I) does not consist of a sequence as indicated in Table II, application no. 0, columns 5 or 7. In a further embodiment, the protein of the present invention is at least 30 %, 40 %, 50%, or 60% identical to a protein sequence indicated in Table II, application no. 0, columns 5 or 7, and less than 100%, preferably less than 99,999%, 99,99% or 99,9%, more preferably less than 99%, 98%, 97%, 96% or 95% identical to a sequence as indicated in Table I, application no. 0, columns 5 or 7.

**[0205.0.0.0]** The nucleic acid sequences used in the process are advantageously introduced in a nucleic acid construct, preferably an expression cassette which makes possible the expression of the nucleic acid molecules in an organism, advantageously a plant or a microorganism.

**[0206.0.0.0]** Accordingly, the invention also relates to an nucleic acid construct, preferably to an expression construct, comprising the nucleic acid molecule of the present invention functionally linked to one or more regulatory elements or signals.

**[0207.0.0.0]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes are genes of the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.0]** In principle, the nucleic acid construct can comprise the herein described regulator sequences and further sequences relevant for the expression of the comprised genes. Thus, the nucleic acid construct of the invention can be used as expression cassette and thus can be used directly for introduction into the plant, or else they may be introduced into a vector. Accordingly in one embodiment the nucleic acid construct is an expression cassette comprising a microorganism promoter or a microorganism terminator or both. In another embodiment the expression cassette encompasses a plant promoter or a plant terminator or both.

**[0209.0.0.0]** Accordingly, in one embodiment, the process according to the invention comprises the following steps:
(a) introducing of a nucleic acid construct comprising the nucleic acid molecule of the invention or used in the process of the invention or encoding the polypeptide of the present invention or used in the process of the invention; or
(b) introducing of a nucleic acid molecule, including regulatory sequences or factors, which expression increases the expression of the nucleic acid molecule of the invention or used in the process of the invention or encoding the polypeptide of the present invention or used in the process of the invention;
   in a cell, or an organism or a part thereof, preferably in a plant, plant cell or a microorganism, and
(c) expressing of the gene product encoded by the nucleic acid construct or the nucleic acid molecule mentioned under (a) or (b) in the cell or the organism.

**[0210.0.0.0]** After the introduction and expression of the nucleic acid construct the transgenic organism or cell is advantageously cultured and subsequently harvested. The transgenic organism or cell may be a prokaryotic or eukaryotic organism such as a microorganism, a non-human animal and plant for example a plant or animal cell, a plant or animal tissue, preferably a crop plant, or a part thereof.

**[0211.0.0.0]** To introduce a nucleic acid molecule into a nucleic acid construct, e.g. as part of an expression cassette, the codogenic gene segment is advantageously subjected to an amplification and ligation reaction in the manner known by a skilled person. It is preferred to follow a procedure similar to the protocol for the Pfu DNA polymerase or a Pfu/Taq DNA polymerase mixture. The primers are selected according to the sequence to be amplified. The primers should expediently be chosen in such a way that the amplificate comprise the codogenic sequence from the start to the stop codon. After the amplification, the amplificate is expediently analyzed. For example, the analysis may consider quality and quantity and be carried out following separation by gel electrophoresis. Thereafter, the amplificate can be purified following a standard protocol (for example Qiagen). An aliquot of the purified amplificate is then available for the subsequent cloning step. Suitable cloning vectors are generally known to the skilled worker.

**[0212.0.0.0]** They include, in particular, vectors which are capable of replication in easy to handle cloning systems like as bacterial yeast or insect cell based (e.g. baculovirus expression) systems, that is to say especially vectors which ensure efficient cloning in E. coli, and which make possible the stable transformation of plants. Vectors, which must be mentioned in particular are various binary and cointegrated vector systems which are suitable for the T-DNA-mediated transformation. Such vector systems are generally characterized in that they contain at least the vir genes, which are required for the Agrobacterium-mediated transformation, and the T-DNA border sequences.

**[0213.0.0.0]** In general, vector systems preferably also comprise further cis-regulatory regions such as promoters and terminators and/or selection markers by means of which suitably transformed organisms can be identified. While vir genes and T-DNA sequences are located on the same vector in the case of cointegrated vector systems, binary systems are based on at least two vectors, one of which bears vir genes, but no T-DNA, while a second one bears T-DNA, but no vir gene. Owing to this fact, the last-mentioned vectors are relatively small, easy to manipulate and capable of replication in E. coli and in Agrobacterium. These binary vectors include vectors from the series pBIB-HYG, pPZP, pBecks, pGreen. Those which are preferably used in accordance with the invention are Bin19, pBI101, pBinAR, pGPTV and pCAMBIA. An overview of binary vectors and their use is given by Hellens et al, Trends in Plant Science (2000) 5, 446-451.

**[0214.0.0.0]** For a vector preparation, vectors may first be linearized using restriction endonuclease(s) and then be modified enzymatically in a suitable manner. Thereafter, the vector is purified, and an aliquot is employed in the cloning step. In the cloning step, the enzyme-cleaved and, if required, purified amplificate is cloned together with similarly prepared vector fragments, using ligase. In this context, a specific nucleic acid construct, or vector or plasmid construct, may have one or else more codogenic gene segments. The codogenic gene segments in these constructs are preferably linked operably to regulatory sequences. The regulatory sequences include, in particular, plant sequences like the above-described promoters and terminators. The constructs can advantageously be propagated stably in microorganisms, in particular Escherichia coli and/or Agrobacterium tumefaciens, under selective conditions and enable the transfer of heterologous DNA into plants or other microorganisms. In accordance with a particular embodiment, the constructs are based on binary vectors (overview of a binary vector: Hellens et al., 2000). As a rule, they contain prokaryotic regulatory sequences, such as replication origin and selection markers, for the multiplication in microorganisms such as Escherichia coli and Agrobacterium tumefaciens. Vectors can further contain agrobacterial T-DNA sequences for the transfer of DNA into plant genomes or other eukaryotic regulatory sequences for transfer into other eukaryotic cells, e.g. Saccharomyces sp. or other prokaryotic regulatory sequences for the transfer into other prokaryotic cells, e.g. Corynebacterium sp. or Bacillus sp. For the transformation of plants, the right border sequence, which comprises approximately 25 base pairs, of the total agrobacterial T-DNA sequence is advantageously included. Usually, the plant transformation vector constructs according to the invention contain T-DNA sequences both from the right and from the left border region, which contain expedient recognition sites for site-specific acting enzymes which, in turn, are encoded by some of the vir genes.

**[0215.0.0.0]** Suitable host organisms are known to the skilled worker. Advantageous organisms are described further above in the present application. They include in particular eukaryotes or eubacteria, e.g. prokaryotes or archae bacteria. Advantageously host organisms are microorganisms selected from the group consisting of Actinomycetaceae, Bacillaceae, Brevibacteriaceae, Corynebacteriaceae, Enterobacteriacae, Gordoniaceae, Micrococcaceae, Mycobacteriaceae, Nocardiaceae, Pseudomonaceae, Rhizobiaceae, Streptomycetaceae, Chaetomiaceae, Choanephoraceae, Cryptococcaceae, Cunninghamellaceae, Demetiaceae, Moniliaceae, Mortierellaceae, Mucoraceae, Pythiaceae, Sacharomycetaceae, Saprolegniaceae, Schizosacharomycetaceae, Sodariaceae, Sporobolomycetaceae, Tuberculariaceae, Adelotheciaceae, Dinophyceae, Ditrichaceae and Prasinophyceae. Preferably are unicellular, microorganisms, e.g. fungi, bacteria or protoza, such as fungi like the genus Claviceps or Aspergillus or gram-positive bacteria such as the genera Bacillus, Corynebacterium, Micrococcus, Brevibacterium, Rhodococcus, Nocardia, Caseobacter or Arthrobacter or gram-negative bacteria such as the genera Escherichia, Flavobacterium or Salmonella, or yeasts such as the genera Rhodotorula, Hansenula, Pichia, Yerrowia, Saccharomyces, Schizosaccharomyces or Candida.

**[0216.0.0.0]** Host organisms which are especially advantageously selected in the process according to the invention are microorganisms selected from the group of the genera and species consisting of Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Escherichia coli K12 and its described strains.

**[0217.0.0.0]** Advantageously preferred in accordance with the invention are host organisms of the genus Agrobacterium tumefaciens or plants. Preferred plants are selected from among the families Aceraceae, Anacardiaceae, Apiaceae, Asteraceae, Apiaceae, Betulaceae, Boraginaceae, Brassicaceae, Bromeliaceae, Cactaceae, Caricaceae, Caryophyllaceae, Cannabaceae, Convolvulaceae, Chenopodiaceae, Elaeagnaceae, Geraniaceae, Gramineae, Juglandaceae, Lauraceae, Leguminosae, Linaceae, Cucurbitaceae, Cyperaceae, Euphorbiaceae, Fabaceae, Malvaceae, Nymphaeaceae, Papaveraceae, Rosaceae, Salicaceae, Solanaceae, Arecaceae, Iridaceae, Liliaceae, Orchidaceae, Gentianaceae, Labiaceae, Magnoliaceae, Ranunculaceae, Carifolaceae, Rubiaceae, Scrophulariaceae, Ericaceae, Polygonaceae, Violaceae, Juncaceae, Poaceae, perennial grass, fodder crops, vegetables and ornamentals.

**[0218.0.0.0]** Especially preferred are plants selected from the groups of the families Apiaceae, Asteraceae, Brassicaceae, Cucurbitaceae, Fabaceae, Papaveraceae, Rosaceae, Solanaceae, Liliaceae or Poaceae. Especially advantageous are, in particular, crop plants. Accordingly, an advantageous plant preferably belongs to the group of the genus peanut, oilseed rape, canola, sunflower, safflower, olive, sesame, hazelnut, almond, avocado, bay, pumpkin/squash, linseed, soya, pistachio, borage, maize, wheat, rye, oats, sorghum and millet, triticale, rice, barley, cassava, potato, sugarbeet, fodder beet, egg plant, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, alfalfa, dwarf bean, lupin, clover and lucerne.

**[0219.0.0.0]** In order to introduce, into a plant, the nucleic acid molecule of the invention or used in the process according to the invention, it has proved advantageous first to transfer them into an intermediate host, for example a bacterium or a eukaryotic unicellular cell. The transformation into E. coli, which can be carried out in a manner known per se, for example by means of heat shock or electroporation, has proved itself expedient in this context. Thus, the transformed E. coli colonies can be analysed for their cloning efficiency. This can be carried out with the aid of a PCR. Here, not only the identity, but also the integrity, of the plasmid construct can be verified with the aid of a defined colony number by subjecting an aliquot of the colonies to said PCR. As a rule, universal primers which are derived from vector sequences are used for this purpose, it being possible, for example, for a forward primer to be arranged upstream of the start ATG and a reverse primer to be arranged downstream of the stop codon of the codogenic gene segment. The amplificates are separated by electrophoresis and assessed with regard to quantity and quality.

**[0220.0.0.0]** The nucleic acid constructs, which are optionally verified, are subsequently used for the transformation of the plants or other hosts, e.g. other eukaryotic cells or other prokaryotic cells. To this end, it may first be necessary to obtain the constructs from the intermediate host. For example, the constructs may be obtained as plasmids from bacterial hosts by a method similar to conventional plasmid isolation.

**[0221.0.0.0]** The nucleic acid molecule of the invention or used in the process according to the invention can also be introduced into modified viral vectors like baculovirus vectors for expression in insect cells or plant viral vectors like tobacco mosaic virus or potato virus X-based vectors. Approaches leading to the expression of proteins from the modified viral genome including the the nucleic acid molecule of the invention or used in the process according to the invention involve for example the inoculation of tobacco plants with infectious RNA transcribed in vitro from a cDNA copy of the recombinant viral genome. Another approach utilizes the transfection of whole plants from wounds inoculated with Agrobacterium tumefaciens containing cDNA copies of recombinant plus-sense RNA viruses. Different vectors and virus are known to the skilled worker for expression in different target eg. production plants.

**[0222.0.0.0]** A large number of methods for the transformation of plants are known. Since, in accordance with the invention, a stable integration of heterologous DNA into the genome of plants is advantageous, the T-DNA-mediated transformation has proved expedient in particular. For this purpose, it is first necessary to transform suitable vehicles, in particular agrobacteria, with a codogenic gene segment or the corresponding plasmid construct comprising the nucleic acid molecule of the invention. This can be carried out in a manner known per se. For example, said nucleic acid construct of the invention, or said expression construct or said plasmid construct, which has been generated in accordance with what has been detailed above, can be transformed into competent agrobacteria by means of electroporation or heat shock. In principle, one must differentiate between the formation of cointegrated vectors on the one hand and the transformation with binary vectors on the other hand. In the case of the first alternative, the constructs, which comprise the codogenic gene segment or the nucleic acid molecule of the invention or the nucleic acid molecule used in the method of the invention have no T-DNA sequences, but the formation of the cointegrated vectors or constructs takes place in the agrobacteria by homologous recombination of the construct with T-DNA. The T-DNA is present in the agrobacteria in the form of Ti or Ri plasmids in which exogenous DNA has expediently replaced the oncogenes. If binary vectors are used, they can be transferred to agrobacteria either by bacterial conjugation or by direct transfer. These agrobacteria expediently already comprise the vector bearing the vir genes (currently referred to as helper Ti(Ri) plasmid).

**[0223.0.0.0]** One or more markers may expediently also be used together with the nucleic acid construct, or the vector of the invention and, if plants or plant cells shall be transformed together with the T-DNA, with the aid of which the isolation or selection of transformed organisms, such as agrobacteria or transformed plant cells, is possible. These marker genes enable the identification of a successful transfer of the nucleic acid molecules according to the invention via a series of different principles, for example via visual identification with the aid of fluorescence, luminescence or in the wavelength range of light which is discernible for the human eye, by a resistance to herbicides or antibiotics, via what are known as nutritive markers (auxotrophism markers) or antinutritive markers, via enzyme assays or via phytohormones. Examples of such markers which may be mentioned are GFP (= green fluorescent protein); the luciferin/luceferase system, the -galactosidase with its colored substrates, for example X-Gal, the herbicide resistances to, for example, imidazolinone, glyphosate, phosphinothricin or sulfonylurea, the antibiotic resistances to, for example, bleomycin, hygromycin, streptomycin, kanamycin, tetracyclin, chloramphenicol, ampicillin, gentamycin, geneticin (G418), spectinomycin or blasticidin, to mention only a few, nutritive markers such as the utilization of mannose or xylose, or antinutritive markers such as the resistance to 2-deoxyglucose. This list is a small number of possible markers. The skilled worker is very familiar with such markers. Different markers are preferred, depending on the organism and the selection method.

**[0224.0.0.0]** As a rule, it is desired that the plant nucleic acid constructs are flanked by T-DNA at one or both sides of the codogenic gene segment. This is particularly useful when bacteria of the species Agrobacterium tumefaciens or Agrobacterium rhizogenes are used for the transformation. A method, which is preferred in accordance with the invention, is the transformation with the aid of Agrobacterium tumefaciens. However, biolistic methods may also be used advantageously for introducing the sequences in the process according to the invention, and the introduction by means of PEG is also possible. The transformed agrobacteria can be grown in the manner known per se and are thus available for the expedient transformation of the plants. The plants or plant parts to be transformed are grown or provided in the customary manner. The transformed agrobacteria are subsequently allowed to act on the plants or plant parts until a sufficient transformation rate is reached. Allowing the agrobacteria to act on the plants or plant parts can take different forms. For example, a culture of morphogenic plant cells or tissue may be used. After the T-DNA transfer, the bacteria are, as a rule, eliminated by antibiotics, and the regeneration of plant tissue is induced. This is done in particular using suitable plant hormones in order to initially induce callus formation and then to promote shoot development.

**[0225.0.0.0]** The transfer of foreign genes into the genome of a plant is called transformation. In doing this the methods described for the transformation and regeneration of plants from plant tissues or plant cells are utilized for transient or stable transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least the flower primordia. The plant is subsequently grown on until the seeds of the treated plant are obtained (Clough and Bent, Plant J. (1998) 16, 735-743). To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Further advantageous transformation methods, in particular for plants, are known to the skilled worker and are described hereinbelow.

**[0226.0.0.0]** Further advantageous and suitable methods are protoplast transformation by poly(ethylene glycol)-induced DNA uptake, the "biolistic" method using the gene cannon - referred to as the particle bombardment method, electroporation, the incubation of dry embryos in DNA solution, microinjection and gene transfer mediated by Agrobacterium. Said methods are described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, in particular of crop plants such as by way of example tobacco plants, for example by bathing bruised leaves or chopped leaves in an agrobacterial solution and then culturing them in suitable media. The transformation of plants by means of Agrobacterium tumefaciens is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

**[0227.0.0.0]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence indicated in Table I, application no. 0, columns 5 or 7, or its derivatives, it is advantageous additionally to express and/or mutate further genes in the organisms. Especially advantageously, additionally at least one further gene of the amino acid biosynthetic pathway such as for L-lysine, L-threonine and/or L-methionine is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine a sequence as indicated in Table I, application no. 0, columns 5 or 7, with genes which generally support or enhances to growth or yield of the target organismen, for example genes which lead to faster growth rate of microorganisms or genes which produces stress-, pathogen, or herbicide resistant plants.

**[0228.0.0.0]** In a further embodiment of the process of the invention, therefore, organisms are grown, in which there is simultaneous overexpression of at least one nucleic acid or one of the genes which code for proteins involved in the amino acid metabolism, in particular in amino acid synthesis.

**[0229.0.0.0]** A further advantageous nucleic acid sequence which can be expressed in combination with the sequences used in the process and/or the abovementioned biosynthesis genes is the sequence of the ATP/ADP translocator as described in WO 01/20009. This ATP/ADP translocator leads to an increased synthesis of the essential amino acids lysine and/or methionine. Furthermore, an advantageous nucleic acid sequence coexpressed can be threonine adlolase and/or lysine decarboxylase as described in the state of the art.

**[0230.0.0.0]** In a further advantageous embodiment of the process of the invention, the organisms used in the process are those in which simultaneously at least one of the aforementioned genes or one of the aforementioned nucleic acids is mutated so that the activity of the corresponding proteins is influenced by metabolites to a smaller extent compared with the unmutated proteins, or not at all, and that in particular the production according to the invention of the respective fine chemical is not impaired, or so that their specific enzymatic activity is increased. Less influence means in this connection that the regulation of the enzymic activity is less by at least 10%, advantageously at least 20, 30 or 40%, particularly advantageously by at least 50, 60, 70, 80 or 90%, compared with the starting organism, and thus the activity of the enzyme is increased by these figures mentioned compared with the starting organism. An increase in the enzymatic activity means an enzymatic activity which is increased by at least 10%, advantageously at least 20, 30, 40 or 50%, particularly advantageously by at least 60, 70, 80, 90, 100, 200, 300, 500 or 1000%, compared with the starting organism. This leads to an increased productivity of the desired respective fine chemical or of the desired respective fine chemicals.

**[0231.0.0.0]** In a further advantageous embodiment of the process of the invention, the organisms used in the process are those in which simultaneously a methionine degrading protein is attenuated, in particular by reducing the rate of expression of the corresponding gene.

**[0232.0.0.0]** In another embodiment of the process of the invention, the organisms used in the process are those in which simultaneously at least one of the aforementioned nucleic acids or of the aforementioned genes is mutated in such a way that the enzymatic activity of the corresponding protein is partially reduced or completely blocked. A reduction in the enzymatic activity means an enzymatic activity, which is reduced by at least 10%, advantageously at least 20, 30 or 40%, particularly advantageously by at least 50, 60 or 70%, preferably more, compared with the starting organism.

**[0233.0.0.0]** If it is intended to transform the host cell, in particular the plant cell, with several constructs or vectors, the marker of a preceding transformation must be removed or a further marker employed in a following transformation. The markers can be removed from the host cell, in particular the plant cell, as described hereinbelow via methods with which the skilled worker is familiar. In particular plants without a marker, in particular without resistance to antibiotics, are an especially preferred embodiment of the present invention.

**[0234.0.0.0]** In the process according to the invention, the nucleic acid sequences used in the process according to the invention are advantageously linked operably to one or more regulatory signals in order to increase gene expression. These regulatory sequences are intended to enable the specific expression of the genes and the expression of protein. Depending on the host organism for example plant or microorganism, this may mean, for example, that the gene is expressed and/or overexpressed after induction only, or that it is expressed and/or overexpressed constitutively. These regulatory sequences are, for example, sequences to which the inductors or repressors bind and which thus regulate the expression of the nucleic acid. In addition to these novel regulatory sequences, or instead of these sequences, the natural regulation of these sequences may still be present before the actual structural genes and, if appropriate, may have been genetically modified so that the natural regulation has been switched off and gene expression has been increased. However, the nucleic acid construct of the invention suitable as expression cassette (= expression construct = gene construct) can also be simpler in construction, that is to say no additional regulatory signals have been inserted before the nucleic acid sequence or its derivatives, and the natural promoter together with its regulation has not been removed. Instead, the natural regulatory sequence has been mutated in such a way that regulation no longer takes place and/or gene expression is increased. These modified promoters can also be introduced on their own before the natural gene in the form of part sequences (= promoter with parts of the nucleic acid sequences according to the invention) in order to increase the activity. Moreover, the gene construct can advantageously also comprise one or more of what are known as enhancer sequences in operable linkage with the promoter, and these enable an increased expression of the nucleic acid sequence. Also, it is possible to insert additional advantageous sequences at the 3' end of the DNA sequences, such as, for example, further regulatory elements or terminators.

**[0235.0.0.0]** The nucleic acid molecules, which encode proteins according to the invention and nucleic acid molecules, which encode other polypeptides may be present in one nucleic acid construct or vector or in several ones. Advantageously, only one copy of the nucleic acid molecule of the invention or the nucleic acid molecule used in the method of the invention or its encoding genes is present in the nucleic acid construct or vector. Several vectors or nucleic acid construct or vector can be expressed together in the host organism. The nucleic acid molecule or the nucleic acid construct or vectoraccording to the invention can be inserted in a vector and be present in the cell in a free form. If a stable transformation is preferred, a vector is used, which is stably duplicated over several generations or which is else be inserted into the genome. In the case of plants, integration into the plastid genome or, in particular, into the nuclear genome may have taken place. For the insertion of more than one gene in the host genome the genes to be expressed are present together in one gene construct, for example in above-described vectors bearing a plurality of genes.

**[0236.0.0.0]** As a rule, regulatory sequences for the expression rate of a gene are located upstream (5'), within, and/or downstream (3') relative to to the coding sequence of the nucleic acid molecule of the invention or the nucleic acid molecule used in the method of the invention or another codogenic gene segment. They control in particular transcription and/or translation and/or the transcript stability. The expression level is dependent on the conjunction of further cellular regulatory systems, such as the protein biosynthesis and degradation systems of the cell.

**[0237.0.0.0]** Regulatory sequences include transcription and translation regulating sequences or signals, e.g. sequences located upstream (5'), which concern in particular the regulation of transcription or translation initiation, such as promoters or start codons, and sequences located downstream (3'), which concern in particular the regulation of transcription or translation termination and transcript stability, such as polyadenylation signals or stop codons. Regulatory sequences can also be present in transcribed coding regions as well in transcribed non-coding regions, e.g. in introns, as for example splicing sites. Promoters for the regulation of expression of the nucleic acid molecule according to the invention in a cell and which can be employed are, in principle, all those which are capable of stimulating the transcription of genes in the organisms in question, such as microorganisms or plants. Suitable promoters, which are functional in these organisms are generally known. They may take the form of constitutive or inducible promoters. Suitable promoters can enable the development-and/or tissue-specific expression in multi-celled eukaryotes; thus, leaf-, root-, flower-, seed-, stomata-, tuber- or fruit-specific promoters may advantageously be used in plants.

**[0238.0.0.0]** The regulatory sequences or factors can, as described above, have a positive effect on, the expression of the genes introduced, thus increasing their expression. Thus, an enhancement of the expression can advantageously take place at the transcriptional level by using strong transcription signals such as strong promoters and/or strong enhancers. In addition, enhancement of expression on the translational level is also possible, for example by introducing translation enhancer sequences, e.g., the enhancer e.g. improving the ribosomal binding to the transcript, or by increasing the stability of the mRNA, e.g. by replacing the 3'UTR coding region by a region encoding a 3'UTR known as conferring an high stability of the transcript or by stabilization of the transcript through the elimination of transcript instability, so that the mRNA molecule is translated more often than the wild type. For example in plants AU-rich elements (AREs) and DST (downstream) elements destabilized transcripts. Mutagenesis studies have demonstrated that residues within two of the conserved domains, the ATAGAT and the GTA regions, are necessary for instability function. Therefore removal or mutation of such elements would obviously lead to more stable transcripts, higher transcript rates and higher protein activity. Translation enhancers are also the "overdrive sequence", which comprises the tobacco mosaic virus 5'-untranslated leader sequence and which increases the protein/RNA ratio (Gallie et al., 1987, Nucl. Acids Research 15:8693-8711)
Enhancers are generally defined as cis active elements, which can stimulate gene transcription independent of position and orientation. Different enhancers have been identified in plants, which can either stimulate transcription constitutively or tissue or stimuli specific. Well known examples for constitutive enhancers are the enhancer from the 35S promoter (Odell et al., 1985, Nature 313:810-812) or the ocs enhancer (Fromm et al., 1989, Plant Cell 1: 977:984) Another examples are the G-Box motif tetramer which confers high-level constitutive expression in dicot and monocot plants (Ishige et al., 1999, Plant Journal, 18, 443-448) or the petE, a A/T-rich sequence which act as quantitative enhancers of gene expression in transgenic tobacco and potato plants (Sandhu et al., 1998; Plant Mol Biol. 37(5):885-96). Beside that, a large variety of cis-active elements have been described which contribute to specific expression pattern, like organ specific expression or induced expression in response to biotic or abiotic stress. Examples are elements which provide pathogen or wound-induced expression (Rushton, 2002, Plant Cell, 14, 749-762) or guard cell-specific expression (Plesch, 2001, Plant Journal 28, 455-464).

**[0239.0.0.0]** Advantageous regulatory sequences for the expression of the nucleic acid molecule according to the invention in microorganisms are present for example in promoters such as the cos, tac, rha, trp, tet, trp-tet, Ipp, lac, Ipp-lac, lacl^{q-}, T7, T5, T3, gal, trc, ara, SP6, λ-P_{R} or λ-P_{L} promoter, which are advantageously used in Gram-negative bacteria. Further advantageous regulatory sequences are present for example in the Gram-positive promoters amy, dnaK, xyls and SPO2, in the yeast or fungal promoters ADC1, MFa, AC, P-60, UASH, MCB, PHO, CYC1, GAPDH, TEF, rp28, ADH. Promoters, which are particularly advantageous, are constitutive, tissue or compartment specific and inducible promoters. In general, "promoter" is understood as meaning, in the present context, a regulatory sequence in a nucleic acid molecule, which mediates the expression of a coding sequence segment of a nucleic acid molecule. In general, the promoter is located upstream to the coding sequence segment. Some elements, for example expression-enhancing elements such as enhancer may, however, also be located downstream or even in the transcribed region.

**[0240.0.0.0]** In principle, it is possible to use natural promoters together with their regulatory sequences, such as those mentioned above, for the novel process. It is also possible advantageously to use synthetic promoters, either additionally or alone, in particular when they mediate seed-specific expression such as described in, for example, WO 99/16890.

**[0241.0.0.0]** The expression of the nucleic acid molecules used in the process may be desired alone or in combination with other genes or nucleic acids. Multiple nucleic acid molecules conferring the expression of advantageous genes can be introduced via the simultaneous transformation of several individual suitable nucleic acid constructs, i.e. expression constructs, or, preferably, by combining several expression cassettes on one construct. It is also possible to transform several vectors with in each case several expression cassettes stepwise into the recipient organisms.

**[0242.0.0.0]** As described above the transcription of the genes introduced should advantageously be terminated by suitable terminators at the 3' end of the biosynthesis genes introduced (behind the stop codon). A terminator, which may be used for this purpose is, for example, the OCS1 terminator, the nos3 terminator or the 35S terminator. As is the case with the promoters, different terminator sequences should be used for each gene. Terminators, which are useful in microorganism are for example the fimA terminator, txn terminator or trp terminator. Such terminators can be rho-dependent or rho-independent.

**[0243.0.0.0]** Different plant promoters such as, for example, the USP, the LegB4-, the DC3 promoter or the ubiquitin promoter from parsley or other herein mentioned promoter and different terminators may advantageously be used in the nucleic acid construct.

**[0244.0.0.0]** In order to ensure the stable integration, into the transgenic plant, of nucleic acid molecules used in the process according to the invention in combination with further biosynthesis genes over a plurality of generations, each of the coding regions used in the process should be expressed under the control of its own, preferably unique, promoter since repeating sequence motifs may lead to recombination events or to silencing or, in plants, to instability of the T-DNA.

**[0245.0.0.0]** The nucleic acid construct is advantageously constructed in such a way that a promoter is followed by a suitable cleavage site for insertion of the nucleic acid to be expressed, advantageously in a polylinker, followed, if appropriate, by a terminator located behind the polylinker. If appropriate, this order is repeated several times so that several genes are combined in one construct and thus can be introduced into the transgenic plant in order to be expressed. The sequence is advantageously repeated up to three times. For the expression, the nucleic acid sequences are inserted via the suitable cleavage site, for example in the polylinker behind the promoter. It is advantageous for each nucleic acid sequence to have its own promoter and, if appropriate, its own terminator, as mentioned above. However, it is also possible to insert several nucleic acid sequences behind a promoter and, if appropriate, before a terminator if a polycistronic transcription is possible in the host or target cells. In this context, the insertion site, or the sequence of the nucleic acid molecules inserted, in the nucleic acid construct is not decisive, that is to say a nucleic acid molecule can be inserted in the first or last position in the cassette without this having a substantial effect on the expression. However, it is also possible to use only one promoter type in the construct. However, this may lead to undesired recombination events or silencing effects, as said.

**[0246.0.0.0]** Accordingly, in a preferred embodiment, the nucleic acid construct according to the invention confers expression of the nucleic acid molecule of the invention or the nucleic acid molecule used in the method of the invention, and, optionally further genes, in a plant and comprises one or more plant regulatory elements. Said nucleic acid construct according to the invention advantageously encompasses a plant promoter or a plant terminator or a plant promoter and a plant terminator.

**[0247.0.0.0]** A "plant" promoter comprises regulatory elements, which mediate the expression of a coding sequence segment in plant cells. Accordingly, a plant promoter need not be of plant origin, but may originate from viruses or microorganisms, in particular for example from viruses which attack plant cells.

**[0248.0.0.0]** The plant promoter can also originates from a plant cell, e.g. from the plant, which is transformed with the nucleic acid construct or vector as described herein.
This also applies to other "plant" regulatory signals, for example in "plant" terminators.

**[0249.0.0.0]** A nucleic acid construct suitable for plant expression preferably comprises regulatory elements which are capable of controlling the expression of genes in plant cells and which are operably linked so that each sequence can fulfill its function. Accordingly, the nucleic acid construct can also comprise transcription terminators. Examples for transcriptional termination arepolyadenylation signals. Preferred polyadenylation signals are those which originate from Agrobacterium tumefaciens T-DNA, such as the gene 3 of the Ti plasmid pTiACH5, which is known as octopine synthase (Gielen et al., EMBO J. 3 (1984) 835 et seq.) or functional equivalents thereof, but all the other terminators which are functionally active in plants are also suitable.

**[0250.0.0.0]** The nucleic acid construct suitable for plant expression preferably also comprises other operably linked regulatory elements such as translation enhancers, for example the overdrive sequence, which comprises the tobacco mosaic virus 5'-untranslated leader sequence, which increases the protein/RNA ratio (Gallie et al., 1987, Nucl. Acids Research 15:8693-8711).

**[0251.0.0.0]** Other preferred sequences for use in operable linkage in gene expression constructs are targeting sequences, which are required for targeting the gene product into specific cell compartments (for a review, see Kermode, Crit. Rev. Plant Sci. 15, 4 (1996) 285-423 and references cited therein), for example into the vacuole, the nucleus, all types of plastids, such as amyloplasts, chloroplasts, chromoplasts, the extracellular space, the mitochondria, the endoplasmic reticulum, elaioplasts, peroxisomes, glycosomes, and other compartments of cells or extracellular. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell Targeting sequences are also known for eukaryotic and to a lower extent for prokaryotic organisms and can advantageously be operable linked with the nucleic acid molecule of the present invention to achieve an expression in one of said compartments or extracellular.

**[0252.0.0.0]** For expression in plants, the nucleic acid molecule must, as described above, be linked operably to or comprise a suitable promoter which expresses the gene at the right point in time and in a cell- or tissue-specific manner. Usable promoters are constitutive promoters (Benfey et al., EMBO J. 8 (1989) 2195-2202), such as those which originate from plant viruses, such as 35S CAMV (Franck et al., Cell 21 (1980) 285-294), 19S CaMV (see also US 5352605 and WO 84/02913), 34S FMV (Sanger et al., Plant. Mol. Biol., 14, 1990: 433-443), the parsley ubiquitin promoter, or plant promoters such as the Rubisco small subunit promoter described in US 4,962,028 or the plant promoters PRP1 [Ward et al., Plant. Mol. Biol. 22 (1993)], SSU, PGEL1, OCS [Leisner (1988) Proc Natl Acad Sci USA 85(5):2553-2557], lib4, usp, mas [Comai (1990) Plant Mol Biol 15 (3):373-381], STLS1, ScBV (Schenk (1999) Plant Mol Biol 39(6):1221-1230), B33, SAD1 or SAD2 (flax promoters, Jain et al., Crop Science, 39 (6), 1999: 1696-1701) or nos [Shaw et al. (1984) Nucleic Acids Res. 12(20):7831-7846]. Stable, constitutive expression of the proteins according to the invention a plant can be advantageous. However, inducible expression of the polypeptide of the invention or the polypeptide used in the method of the invention is advantageous, if a late expression before the harvest is of advantage, as metabolic manipulation may lead to a plant growth retardation.

**[0253.0.0.0]** The expression of plant genes can also be facilitated as described above via a chemical inducible promoter (for a review, see Gatz 1997, Annu. Rev. Plant Physiol. Plant Mol. Biol., 48:89-108). Chemically inducible promoters are particularly suitable when it is desired to express the gene in a time-specific manner. Examples of such promoters are a salicylic acid inducible promoter (WO 95/19443), and abscisic acid-inducible promoter (EP 335 528), a tetracyclin-inducible promoter (Gatz et al. (1992) Plant J. 2, 397-404), a cyclohexanol- or ethanol-inducible promoter (WO 93/21334) or others as described herein.

**[0254.0.0.0]** Other suitable promoters are those which react to biotic or abiotic stress conditions, for example the pathogen-induced PRP1 gene promoter (Ward et al., Plant. Mol. Biol. 22 (1993) 361-366), the tomato heat-inducible hsp80 promoter (US 5,187,267), the potato chill-inducible alpha-amylase promoter (WO 96/12814) or the wound-inducible pinII promoter (EP-A-0 375 091) or others as described herein.

**[0255.0.0.0]** Preferred promoters are in particular those which bring about gene expression in tissues and organs in which the biosynthesis of amino acids takes place, in seed cells, such as endosperm cells and cells of the developing embryo. Suitable promoters are the oilseed rape napin gene promoter (US 5,608,152), the Vicia faba USP promoter (Baeumlein et al., Mol Gen Genet, 1991, 225 (3):459-67), the Arabidopsis oleosin promoter (WO 98/45461), the Phaseolus vulgaris phaseolin promoter (US 5,504,200), the Brassica Bce4 promoter (WO 91/13980), the bean arc5 promoter, the carrot DcG3 promoter, or the Legumin B4 promoter (LeB4; Baeumlein et al., 1992, Plant Journal, 2 (2):233-9), and promoters which bring about the seed-specific expression in monocotyledonous plants such as maize, barley, wheat, rye, rice and the like. Advantageous seed-specific promoters are the sucrose binding protein promoter (WO 00/26388), the phaseolin promoter and the napin promoter. Suitable promoters which must be considered are the barley Ipt2 or Ipt1 gene promoter (WO 95/15389 and WO 95/23230), and the promoters described in WO 99/16890 (promoters from the barley hordein gene, the rice glutelin gene, the rice oryzin gene, the rice prolamin gene, the wheat gliadin gene, the wheat glutelin gene, the maize zein gene, the oat glutelin gene, the sorghum kasirin gene and the rye secalin gene). Further suitable promoters are Amy32b, Amy 6-6 and Aleurain [US 5,677,474], Bce4 (oilseed rape) [US 5,530,149], glycinin (soya) [EP 571 741], phosphoenolpyruvate carboxylase (soya) [JP 06/62870], ADR12-2 (soya) [WO 98/08962], isocitrate lyase (oilseed rape) [US 5,689,040] or α-amylase (barley) [EP 781 849]. Other promoters which are available for the expression of genes in plants are leaf-specific promoters such as those described in DE-A 19644478 or light-regulated promoters such as, for example, the pea petE promoter.

**[0256.0.0.0]** Further suitable plant promoters are the cytosolic FBPase promoter or the potato ST-LSI promoter (Stockhaus et al., EMBO J. 8, 1989, 2445), the Glycine max phosphoribosylpyrophosphate amidotransferase promoter (GenBank Accession No. U87999) or the node-specific promoter described in EP-A-0 249 676.

**[0257.0.0.0]** Other promoters, which are particularly suitable, are those which bring about plastid-specific expression. Suitable promoters such as the viral RNA polymerase promoter are described in WO 95/16783 and WO 97/06250, and the Arabidopsis clpP promoter, which is described in WO 99/46394.

**[0258.0.0.0]** Other promoters, which are used for the strong expression of heterologous sequences in as many tissues as possible, in particular also in leaves, are, in addition to several of the abovementioned viral and bacterial promoters, preferably, plant promoters of actin or ubiquitin genes such as, for example, the rice actin1 promoter. Further examples of constitutive plant promoters are the sugarbeet V-ATPase promoters (WO 01/14572). Examples of synthetic constitutive promoters are the Super promoter (WO 95/14098) and promoters derived from G-boxes (WO 94/12015). If appropriate, chemical inducible promoters may furthermore also be used, compare EP-A 388186, EP-A 335528, WO 97/06268.

**[0259.0.0.0]** As already mentioned herein, further regulatory sequences, which may be expedient, if appropriate, also include sequences, which target the transport and/or the localization of the expression products. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell.

**[0260.0.0.0]** Preferred recipient plants are, as described above, in particular those plants, which can be transformed in a suitable manner. These include monocotyledonous and dicotyledonous plants. Plants which must be mentioned in particular are agriculturally useful plants such as cereals and grasses, for example Triticum spp., Zea mays, Hordeum vulgare, oats, Secale cereale, Oryza sativa, Pennisetum glaucum, Sorghum bicolor, Triticale, Agrostis spp., Cenchrus ciliaris, Dactylis glomerata, Festuca arundinacea, Lolium spp., Medicago spp. and Saccharum spp., legumes and oil crops, for example Brassica juncea, Brassica napus, Glycine max, Arachis hypogaea, Gossypium hirsutum, Cicer arietinum, Helianthus annuus, Lens culinaris, Linum usitatissimum, Sinapis alba, Trifolium repens and Vicia narbonensis, vegetables and fruits, for example bananas, grapes, Lycopersicon esculentum, asparagus, cabbage, watermelons, kiwi fruit, Solanum tuberosum, Beta vulgaris, cassava and chicory, trees, for example Coffea species, Citrus spp., Eucalyptus spp., Picea spp., Pinus spp. and Populus spp., medicinal plants and trees, and flowers.

**[0261.0.0.0]** One embodiment of the present invention also relates to a method for generating a vector, which comprises the insertion, into a vector, of the nucleic acid molecule characterized herein, the nucleic acid molecule according to the invention or the expression cassette according to the invention. The vector can, for example, be introduced in to a cell, e.g. a microorganism or a plant cell, as described herein for the nucleic acid construct, or below under transformation or transfection or shown in the examples. A transient or stable transformation of the host or target cell is possible, however, a stable transformation is preferred. The vector according to the invention is preferably a vector, which is suitable for expressing the polypeptide according to the invention in a plant. The method can thus also encompass one or more steps for integrating regulatory signals into the vector, in particular signals, which mediate the expression in microorganisms or plants.

**[0262.0.0.0]** Accordingly, the present invention also relates to a vector comprising the nucleic acid molecule characterized herein as part of a nucleic acid construct suitable for plant expression or the nucleic acid molecule according to the invention.

**[0263.0.0.0]** The advantageous vectors of the inventioncomprise the nucleic acid molecules which encode proteins according to the invention, nucleic acid molecules which are used in the process, or nucleic acid construct suitable for plant expression comprising the nucleic acid molecules used, either alone or in combination with further genes such as the biosynthesis or regulatory genes of the respective fine chemical metabolism e.g. with the genes mentioned herein above. In accordance with the invention, the term "vector" refers to a nucleic acid molecule, which is capable of transporting another nucleic acid to which it is linked. One type of vector is a "plasmid", which means a circular double-stranded DNA loop into which additional DNA segments can be ligated. A further type of vector is a viral vector, it being possible to ligate additional nucleic acids segments into the viral genome. Certain vectors are capable of autonomous replication in a host cell into which they have been introduced (for example bacterial vectors with bacterial replication origin). Other preferred vectors are advantageously completely or partly integrated into the genome of a host cell when they are introduced into the host cell and thus replicate together with the host genome. Moreover, certain vectors are capable of controlling the expression of genes with which they are in operable linkage. In the present context, these vectors are referred to as "expression vectors". As mentioned above, they are capable of autonomous replication or may be integrated partly or completely into the host genome. Expression vectors, which are suitable for DNA recombination techniques usually take the form of plasmids. In the present description, "plasmid" and "vector" can be used interchangeably since the plasmid is the most frequently used form of a vector. However, the invention is also intended to encompass these other forms of expression vectors, such as viral vectors, which exert similar functions. The term vector is furthermore also to encompass other vectors which are known to the skilled worker, such as phages, viruses such as SV40, CMV, TMV, transposons, IS elements, phasmids, phagemids, cosmids, and linear or circular DNA.

**[0264.0.0.0]** The recombinant expression vectors which are advantageously used in the process comprise the nucleic acid molecules according to the invention or the nucleic acid construct according to the invention in a form which is suitable for expressing, in a host cell, the nucleic acid molecules according to the invention or described herein. Accordingly, the the recombinant expression vectors comprise one or more regulatory signals selected on the basis of the host cells to be used for the expression, in operable linkage with the nucleic acid sequence to be expressed.

**[0265.0.0.0]** In a recombinant expression vector, "operable linkage" means that the nucleic acid molecule of interest is linked to the regulatory signals in such a way that expression of the nucleic acid molecule is possible: they are linked to one another in such a way that the two sequences fulfill the predicted function assigned to the sequence (for example in an in-vitro transcription/translation system, or in a host cell if the vector is introduced into the host cell).

**[0266.0.0.0]** The term "regulatory sequence" is intended to comprise promoters, enhancers and other expression control elements (for example polyadenylation signals These regulatory sequences are described, for example, in Goeddel: Gene Expression Technology: Methods in Enzymology 185, Academic Press, San Diego, CA (1990), or see: Gruber and Crosby, in: Methods in Plant Molecular Biology and Biotechnolgy, CRC Press, Boca Raton, Florida, Ed.: Glick and Thompson, chapter 7, 89-108, including the references cited therein. Regulatory sequences encompass those, which control the constitutive expression of a nucleotide sequence in many types of host cells and those which control the direct expression of the nucleotide sequence in specific host cells only, and under specific conditions. The skilled worker knows that the design of the expression vector may depend on factors such as the selection of the host cell to be transformed, the extent to which the desired protein is expressed, and the like. A preferred selection of regulatory sequences is described above, for example promoters, terminators, enhancers and the like. The term regulatory sequence is to be considered as being encompassed by the term regulatory signal. Several advantageous regulatory sequences, in particular promoters and terminators are described above. In general, the regulatory sequences described as advantageous for nucleic acid construct suitable for expression are also applicable for vectors.

**[0267.0.0.0]** The recombinant expression vectors used can be designed specifically for the expression, in prokaryotic and/or eukaryotic cells, of nucleic acid molecules used in the process. This is advantageous since intermediate steps of the vector construction are frequently carried out in microorganisms for the sake of simplicity. For example, the genes according to the invention and other genes can be expressed in bacterial cells, insect cells (using baculovirus expression vectors), yeast cells and other fungal cells [Romanos (1992), Yeast 8:423-488; van den Hondel, (1991), in: More Gene Manipulations in Fungi, J.W. Bennet & L.L. Lasure, Ed., pp. 396-428: Academic Press: San Diego; and van den Hondel, C.A.M.J.J. (1991), in: Applied Molecular Genetics of Fungi, Peberdy, J.F., et al., Ed., pp. 1-28, Cambridge University Press: Cambridge], algae [Falciatore et al., 1999, Marine Biotechnology.1, 3:239-251] using vectors and following a transformation method as described in WO 98/01572, and preferably in cells of multi-celled plants [see Schmidt, R. and Willmitzer, L. (1988) Plant Cell Rep.:583-586; Plant Molecular Biology and Biotechnology, C Press, Boca Raton, Florida, chapter 6/7, pp.71-119 (1993); F.F. White, in: Transgenic Plants, Bd. 1, Engineering and Utilization, Ed.: Kung and R. Wu, Academic Press (1993), 128-43; Potrykus, Annu. Rev. Plant Physiol. Plant Molec. Biol. 42 (1991), 205-225 (and references cited therein)]. Suitable host cells are furthermore discussed in Goeddel, Gene Expression Technology: Methods in Enzymology 185, Academic Press, San Diego, CA (1990). As an alternative, the sequence of the recombinant expression vector can be transcribed and translated in vitro, for example using T7 promotor-regulatory sequences and T7 polymerase.

**[0268.0.0.0]** Proteins can be expressed in prokaryotes using vectors comprising constitutive or inducible promoters, which control the expression of fusion proteins or nonfusion proteins. Typical fusion expression vectors are, inter alia, pGEX (Pharmacia Biotech Inc; Smith, D.B., and Johnson, K.S. (1988) Gene 67:31-40), pMAL (New England Biolabs, Beverly, MA) and pRlT5 (Pharmacia, Piscataway, NJ), in which glutathione-S-transferase (GST), maltose-E-binding protein or protein A is fused with the recombinant target protein. Examples of suitable inducible nonfusion E. coli expression vectors are, inter alia, pTrc (Amann et al. (1988) Gene 69:301-315) and pET 11d [Studier et al., Gene Expression Technology: Methods in Enzymology 185, Academic Press, San Diego, California (1990) 60-89]. The target gene expression of the pTrc vector is based on the transcription of a hybrid trp-lac fusion promoter by the host RNA polymerase. The target gene expression from the pET 11d vector is based on the transcription of a T7-gn 10-lac fusion promoter, which is mediated by a coexpressed viral RNA polymerase (T7 gn1). This viral polymerase is provided by the host strains BL21 (DE3) or HMS174 (DE3) by a resident λ-prophage which harbors a T7 gn1 gene under the transcriptional control of the lacUV 5 promoter.

**[0269.0.0.0]** Other vectors which are suitable in prokaryotic organisms are known to the skilled worker; these vectors are for example in E. coli pLG338, pACYC184, the pBR series, such as pBR322, the pUC series such as pUC18 or pUC19, the M113mp series, pKC30, pRep4, pHS1, pHS2, pPLc236, pMBL24, pLG200, pUR290, pIN-III113-B1, λgt11 or pBdCl, in Streptomyces pIJ101, pIJ364, pIJ702 or pIJ361, in Bacillus pUB110, pC194 or pBD214, in Corynebacterium pSA77 or pAJ667.

**[0270.0.0.0]** In a further embodiment, the expression vector is a yeast expression vector. Examples of vectors for expression in the yeasts S. cerevisiae encompass pYeDesaturasec1 (Baldari et al. (1987) Embo J. 6:229-234), pMFa (Kurjan and Herskowitz (1982) Cell 30:933-943), pJRY88 (Schultz et al. (1987) Gene 54:113-123) and pYES2 (Invitrogen Corporation, San Diego, CA). Vectors and methods for the construction of vectors which are suitable for use in other fungi, such as the filamentous fungi, encompass those which are described in detail in: van den Hondel, C.A.M.J.J. [(1991), J.F. Peberdy, Ed., pp. 1-28, Cambridge University Press: Cambridge; or in: More Gene Manipulations in Fungi; J.W. Bennet & L.L. Lasure, Ed., pp. 396-428: Academic Press: San Diego]. Examples of other suitable yeast vectors are 2µM, pAG-1, YEp6, YEp13 or pEMBLYe23. [0271.0.0.0] Further vectors, which may be mentioned by way of example, are pALS1, pIL2 or pBB116 in fungi or pLGV23, pGHlac⁺, pBIN19, pAK2004 or pDH51 in plants.

**[0272.0.0.0]** As an alternative, the nucleic acid sequences can be expressed in insect cells using baculovirus expression vectors. Baculovirus vectors, which are available for expressing proteins in cultured insect cells (for example Sf9 cells) encompass the pAc series (Smith et al. (1983) Mol. Cell Biol. 3:2156-2165) and the pVL series (Lucklow and Summers (1989) Virology 170:31-39).

**[0273.0.0.0]** The abovementioned vectors are only a small overview of potentially suitable vectors. Further plasmids are known to the skilled worker and are described, for example, in: Cloning Vectors (Ed. Pouwels, P.H., et al., Elsevier, Amsterdam-New York-Oxford, 1985, ISBN 0 444 904018). Further suitable expression systems for prokaryotic and eukaryotic cells, see the chapters 16 and 17 by Sambrook, J., Fritsch, E.F., and Maniatis, T., Molecular Cloning: A Laboratory Manual, 2nd Edition, Cold Spring Harbor Laboratory, Cold Spring Harbor Laboratory Press, Cold Spring Harbor, NY, 1989.

**[0274.0.0.0]** Accordingly, one embodiment of the invention relates to a vector where the nucleic acid molecule according to the invention is linked operably to regulatory sequences which permit the expression in a prokaryotic or eukaryotic or in a prokaryotic and eukaryotic host.

**[0275.0.0.0]** Accordingly, one embodiment of the invention relates to a host cell, which has been transformed stably or transiently with the vector according to the invention or the nucleic acid molecule according to the invention or the nucleic acid construct according to the invention.

**[0276.0.0.0]** Depending on the host organism, the organisms used in the process according to the invention are cultured or grown in a manner with which the skilled worker is familiar. As a rule, microorganisms are grown in a liquid medium comprising a carbon source, usually in the form of sugars, a nitrogen source, usually in the form of organic nitrogen sources such as yeast extract or salts such as ammonium sulfate, trace elements such as iron salts, manganese salts, magnesium salts, and, if appropriate, vitamins, at temperatures between 0°C and 100°C, preferably between 10°C and 60°C, while passing in oxygen. In the event the microorganism is anaerobe, no oxygen is blown through the culture medium. The pH value of the liquid nutrient medium may be kept constant, that is to say regulated during the culturing phase, or not. The organisms may be cultured batchwise, semibatchwise or continuously. Nutrients may be provided at the beginning of the fermentation or fed in semicontinuously or continuously.

**[0277.0.0.0]** The amino acids produced can be isolated from the organism by methods with which the skilled worker is familiar. For example via extraction, salt precipitation and/or ion-exchange chromatography. To this end, the organisms may be disrupted beforehand. The process according to the invention can be conducted batchwise, semibatchwise or continuously. A summary of known culture and isolation techniques can be found in the textbook by Chmiel [Bioprozeßtechnik 1, Einführung in die Bioverfahrenstechnik (Gustav Fischer Verlag, Stuttgart, 1991)], Demain et al. (Industrial Microbiology and Biotechnology, second edition, ASM Press, Washington, D.C., 1999, ISBN 1-55581-128-0] or in the textbook by Storhas (Bioreaktoren und periphere Einrichtungen (Vieweg Verlag, Braunschweig/Wiesbaden, 1994)).

**[0278.0.0.0]** In one embodiment, the present invention relates to a polypeptide encoded by the nucleic acid molecule according to the present invention, preferably conferring an increase in the respective fine chemical content in an organism or cell after increasing the expression or activity.

**[0279.0.0.0]** The present invention also relates to a process for the production of a polypeptide according to the present invention, the polypeptide being expressed in a host cell according to the invention, preferably in a microorganism or a transgenic plant cell.

**[0280.0.0.0]** In one embodiment, the nucleic acid molecule used in the process for the production of the polypeptide is derived from a microorganism, preferably from a prokaryotic or protozoic cell with an eukaryotic organism as host cell. E.g., in one embodiment the polypeptide is produced in a plant cell or plant with a nucleic acid molecule derived from a prokaryote or a fungus or an alga or an other microorganism but not from plant.

**[0281.0.0.0]** The skilled worker knows that protein and DNA expressed in different organisms differ in many respects and properties, e.g. DNA modulation and imprinting, such as methylation or post-translational modification, as for example glucosylation, phosphorylation, acetylation, myristoylation, ADP-ribosylation, farnesylation, carboxylation, sulfation, ubiquination, etc. though having the same coding sequence. Preferably, the cellular expression control of the corresponding protein differs accordingly in the control mechanisms controlling the activity and expression of an endogenous protein or another eukaryotic protein. One major difference between proteins expressed in prokaryotic or eukaryotic organisms is the amount and pattern of glycosylation. For example in E. coli there are no glycosylated proteins. Proteins expressed in yeasts have high mannose content in the glycosylated proteins, whereas in plants the glycosylation pattern is complex.

**[0282.0.0.0]** The polypeptide of the present invention is preferably produced by recombinant DNA techniques. For example, a nucleic acid molecule encoding the protein is cloned into a vector (as described above), the vector is introduced into a host cell (as described above) and said polypeptide is expressed in the host cell. Said polypeptide can then be isolated from the cells by an appropriate purification scheme using standard protein purification techniques. Alternative to recombinant expression, the polypeptide or peptide of the present invention can be synthesized chemically using standard peptide synthesis techniques.

**[0283.0.0.0]** Moreover, a native polypeptide conferring the increase of the respective fine chemical in an organism or part thereof can be isolated from cells (e.g., endothelial cells), for example using the antibody of the present invention as described below, in particular, an antibody against a protein as indicated in Table II, application no. 0, column 3. E.g. an antibody against a polypeptide as indicated in Table II, application no. 0, columns 5 or 7, or an antigenic part thereof which can be produced by standard techniques utilizing polypeptides comprising or consisting of above mentioned sequences, e.g. the polypeptid of the present invention or fragment thereof,. Preferred are monoclonal antibodies specifically binding to polypeptide as indicated in Table II, application no. 0, columns 5 or 7.

**[0284.0.0.0]** In one embodiment, the present invention relates to a polypeptide having the amino acid sequence encoded by a nucleic acid molecule of the invention or the nucleic acid molecule used in the method of the invention or obtainable by a process of the invention. Said polypeptide confers preferably the aforementioned activity, in particular, the polypeptide confers the increase of the respective fine chemical in a cell or an organism or a part thereof after increasing the cellular activity, e.g. by increasing the expression or the specific activity of the polypeptide.

**[0285.0.0.0]** In one embodiment, the present invention relates to a polypeptide having a sequence as indicated in Table II, application no. 0, columns 5 or 7, or as encoded by a nucleic acid molecule as indicated in Table I, application no. 0, columns 5 or 7, or functional homologues thereof.

**[0286.0.0.0]** In one advantageous embodiment, in the method of the present invention the activity of a polypeptide is increased which comprises or consists of a consensus sequence as indicated in Table IV, application no. 0, column 7, and in one another embodiment, the present invention relates to a polypeptide comprising or consisting of a consensus sequence as indicated in Table IV, application no. 0, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, application no. 0, column 7.

**[0287.0.0.0]** In one embodiment not more than 15%, preferably 10%, even more preferred 5%, 4%, 3%, or 2%, most preferred 1% or 0% of the amino acid position indicated by a letter are/is replaced another amino acid or, in an other embodiment, are/is absent and/or replaced. In another embodiment the stretches of non-conserved amino acids, indicated by (X)n [whereas n indicates the number of X], vary in their length by 20%, preferably by 15 or 10 %, even more preferred by 5%, 4%, 3%, 2% or most preferred by only 1%.

**[0288.0.0.0]** In one embodiment 20 or less, preferably 15 or 10, preferably 9, 8, 7, or 6, more preferred 5 or 4, even more preferred 3, even more preferred 2, even more preferred 1, most preferred 0 amino acids are inserted into the consensus sequence or, in an other embodiment, are absent and/or replaced.

**[0289.0.0.0]** The consensus sequence shown herein was derived from a multiple alignment of the sequences as listed in table II. The consensus sequences of specified domains were derived from a multiple alignment of all sequences. The letters represent the one letter amino acid code and indicate that the amino acids are conserved in all aligned proteins. The letter X stands for amino acids, which are not conserved in all sequences.
In one example, in the cases where only a small selected subset of amino acids are possible at a certain position these amino acids are given in brackets. The number of given X indicates the distances between conserved amino acid residues, e.g. YX(21-23)F means that conserved tyrosine and phenylalanine residues are separated from each other by minimum 21 and maximum 23 amino acid residues in all investigated sequences.

**[0290.0.0.0]** The alignment was performed with the Software AlignX (sept 25, 2002) a component of Vector NTI Suite 8.0, InforMax^{™}, Invitrogen^{™} life science software, U.S. Main Office, 7305 Executive Way,Frederick, MD 21704,USA with the following settings: For pairwise alignments: gap opening penality: 10,0; gap extension penality 0,1. For multiple alignments: Gap opening penalty: 10,0; Gap extension penalty: 0,1; Gap separation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids.

**[0291.0.0.0]** In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of plant or microorganism specific consensus sequences.
In one embodiment, said polypeptide of the invention distinguishes over a sequence as indicated in Table II, application no. 0, columns 5 or 7, by one or more amino acids. In one embodiment, polypeptide distinguishes form a sequence as indicated in Table II, application no. 0, columns 5 or 7, by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 0, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II, application no. 0, columns 5 or 7.

**[0292.0.0.0]** In one embodiment, the polypeptide of the invention comprises any one of the sequences not known to the public before. In one embodiment, the polypeptide of the invention originates from a non-plant cell, in particular from a microorganism, and was expressed in a plant cell. In one embodiment, the present invention relates to a polypeptide encoded by the nucleic acid molecule of the invention or used in the process of the invention for which an activity has not been described yet.

**[0293.0.0.0]** In one embodiment, the invention relates to polypeptide conferring an increase in the respective fine chemical in an organism or part being encoded by the nucleic acid molecule of the invention or by a nucleic acid molecule used in the process of the invention.
In one embodiment, the polypeptide of the invention has a sequence which distinguishes from a sequence as indicated in Table II, application no. 0, columns 5 or 7, by one or more amino acids. In an other embodiment, said polypeptide of the invention does not consist of the sequence as indicated in Table II, application no. 0, columns 5 or 7. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 0, columns 5 or 7.

**[0294.0.0.0]** In one embodiment, the present invention relates to a polypeptide having an activity of a protein as indicated in Table II, application no. 0, column 3, which distinguishes over a sequence as indicated in Table II, application no. 0, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.0]** The terms "protein" and "polypeptide" used in this application are interchangeable. "Polypeptide" refers to a polymer of amino acids (amino acid sequence) and does not refer to a specific length of the molecule. Thus peptides and oligopeptides are included within the definition of polypeptide. This term does also refer to or include post-translational modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring.

**[0296.0.0.0]** Preferably, the polypeptide is isolated. An "isolated" or "purified" protein or nucleic acid molecule or biologically active portion thereof is substantially free of cellular material when produced by recombinant DNA techniques or chemical precursors or other chemicals when chemically synthesized.

**[0297.0.0.0]** The language "substantially free of cellular material" includes preparations of the polypeptide of the invention in which the protein is separated from cellular components of the cells in which it is naturally or recombinantly produced. In one embodiment, the language "substantially free of cellular material" includes preparations having less than about 30% (by dry weight) of "contaminating protein", more preferably less than about 20% of "contaminating protein", still more preferably less than about 10% of "contaminating protein", and most preferably less than about 5% "contaminating protein". The term "Contaminating protein" relates to polypeptides, which are not polypeptides of the present invention. When the polypeptide of the present invention or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations in which the polypeptide of the present invention is separated from chemical precursors or other chemicals, which are involved in the synthesis of the protein. The language "substantially free of chemical precursors or other chemicals" includes preparations having less than about 30% (by dry weight) of chemical precursors or non-polypeptide of the invention-chemicals, more preferably less than about 20% chemical precursors or non-polypeptide of the invention-chemicals, still more preferably less than about 10% chemical precursors or non-polypeptide of the invention-chemicals, and most preferably less than about 5% chemical precursors or non- polypeptide of the invention-chemicals. In preferred embodiments, isolated proteins or biologically active portions thereof lack contaminating proteins from the same organism from which the polypeptide of the present invention is derived. Typically, such proteins are produced by recombinant techniques.

**[0297.1.0.0]** Non-polypeptide of the invention-chemicals are e.g. polypeptides having not the activity and/or amino acid sequence of a polypeptide indicated in Table II, application no. 0, columns 3, 5 or 7.

**[0298.0.0.0]** A polypeptide of the invention can participate in the process of the present invention. The polypeptide or a portion thereof comprises preferably an amino acid sequence which is sufficiently homologous to an amino acid sequence as indicated in Table II, application no. 0, columns 5 or 7, such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, application no. 0, columns 5 or 7.

**[0299.0.0.0]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, application no. 0, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 0, columns 5 or 7, or which is homologous thereto, as defined above.

**[0300.0.0.0]** Accordingly the polypeptide of the present invention can vary from a sequence as indicated in Table II, application no. 0, columns 5 or 7, in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II, application no. 0, columns 5 or 7.

**[0301.0.0.0]** For the comparison of amino acid sequences the same algorithms as described above or nucleic acid sequences can be used. Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351-360, 1987, Higgins et al., CABIOS, 5 1989: 151-153) or preferably with the programs Gap and BestFit, which are respectively based on the algorithms of Needleman and Wunsch [J. Mol. Biol. 48; 443-453 (1970)] and Smith and Waterman [Adv. Appl. Math. 2; 482-489 (1981)]. Both programs are part of the GCG software-package [Genetics Computer Group, 575 Science Drive, Madison, Wisconsin, USA 53711 (1991); Altschul et al. (1997) Nucleic Acids Res. 25:3389 et seq.]. Therefore preferably the calculations to determine the percentages of sequence homology are done with the program Gap over the whole range of the sequences. The following standard adjustments for the comparison of amino acid sequences were used: gap weight: 8, length weight: 2, average match: 2.912, average mismatch: -2.003.

**[0302.0.0.0]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 0, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention .

**[0303.0.0.0]** Typically, biologically (or immunologically) active portions i.e. peptides, e.g., peptides which are, for example, 5, 10, 15, 20, 30, 35, 36, 37, 38, 39, 40, 50, 100 or more amino acids in length comprise a domain or motif with at least one activity or epitope of a polypeptide of the present invention or used in the process of the present invention. Moreover, other biologically active portions, in which other regions of the polypeptide are deleted, can be prepared by recombinant techniques and evaluated for one or more of the activities described herein.

**[0304.0.0.0]** Manipulation of the nucleic acid molecule of the invention or the nucleic acid molecule used in the method of the invention may result in the production of a protein having essentially the activity of the polypeptides as indicated in Table II, application no. 0, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.0]** Any mutagenesis strategies for the polypeptide of the present invention or the polypeptide used in the process of the present invention to result in increasing said activity are not meant to be limiting; variations on these strategies will be readily apparent to one skilled in the art. Using such strategies, and incorporating the mechanisms disclosed herein, the nucleic acid molecule and polypeptide of the invention or the polypeptide used in the method of the invention may be utilized to generate plants or parts thereof, expressing one or more wildtype protein(s) or one or more mutated protein encoding nucleic acid molecule(s) or polypeptide molecule(s) of the invention such that the yield, production, and/or efficiency of production of a desired compound is improved.

**[0306.0.0.0]** This desired compound may be any natural product of plants, which includes the final products of biosynthesis pathways and intermediates of naturally-occurring metabolic pathways, as well as molecules which do not naturally occur in the metabolism of said cells, but which are produced by a said cells of the invention. Preferably, the compound is a composition comprising the respective fine chemical or a recovered respective fine chemical, in particular, the fine chemical, free or in protein-bound form.

**[0306.1.0.0]** Preferably, the compound is a composition comprising the methionine or a recovered methionine, in particular, the fine chemical, free or in protein-bound form.

**[0307.0.0.0]** The invention also provides chimeric or fusion proteins.

**[0308.0.0.0]** As used herein, an "chimeric protein" or "fusion protein" comprises an polypeptide operatively linked to a polypeptide which does not confer above-mentioned activity, in particular, which does not confer an increase of content of the respective fine chemical in a cell or an organism or a part thereof, if its activity is increased.

**[0309.0.0.0]** In one embodiment, an reference to a " protein (= polypeptide) of the invention" or as indicated in Table II, application no. 0, columns 5 or 7, refers to a polypeptide having an amino acid sequence corresponding to the polypeptide of the invention or used in the process of the invention, whereas a "non-polypeptide of the invention" or "other polypeptide" not being indicated in Table II, application no. 0, columns 5 or 7, refers to a polypeptide having an amino acid sequence corresponding to a protein which is not substantially homologous a polypeptide of the invention, preferably which is not substantially homologous to a polypeptide as indicated in Table II, application no. 0, columns 5 or 7, e.g., a protein which does not confer the activity described herein or annotated or known for as indicated in Table II, application no. 0, column 3, and which is derived from the same or a different organism. In one embodiment, a "non-polypeptide of the invention" or "other polypeptide" not being indicated in Table II, application no. 0, columns 5 or 7, does not confer an increase of the respective fine chemical in an organism or part thereof.

**[0310.0.0.0]** Within the fusion protein, the term "operatively linked" is intended to indicate that the polypeptide of the invention or a polypeptide used in the process of the invention and the "other polypeptide" or a part thereof are fused to each other so that both sequences fulfil the proposed function addicted to the sequence used. The "other polypeptide" can be fused to the N-terminus or C-terminus of the polypeptide of the invention or used in the process of the invention. For example, in one embodiment the fusion protein is a GST-LMRP fusion protein in which the sequences of the polypeptide of the invention or the polypeptide used in the process of the invention are fused to the C-terminus of the GST sequences. Such fusion proteins can facilitate the purification of recombinant polypeptides of the invention or a polypeptide useful in the process of the invention.

**[0311.0.0.0]** In another embodiment, the fusion protein is a polypeptide of the invention or a polypeptide used in the process of the invention containing a heterologous signal sequence at its N-terminus. In certain host cells (e.g., mammalian host cells), expression and/or secretion of a polypeptide of the invention or a polypeptide used in the process of the invention can be increased through use of a heterologous signal sequence. As already mentioned above, targeting sequences, are required for targeting the gene product into specific cell compartment (for a review, see Kermode, Crit. Rev. Plant Sci. 15, 4 (1996) 285-423 and references cited therein), for example into the vacuole, the nucleus, all types of plastids, such as amyloplasts, chloroplasts, chromoplasts, the extracellular space, the mitochondria, the endoplasmic reticulum, elaioplasts, peroxisomes, glycosomes, and other compartments of cells or extracellular. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell. Targeting sequences are also known for eukaryotic and to a lower extent for prokaryotic organisms and can advantageously be operable linked with the nucleic acid molecule of the present invention to achieve an expression in one of said compartments or extracellular.

**[0312.0.0.0]** Preferably, a chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. The fusion gene can be synthesized by conventional techniques including automated DNA synthesizers. Alternatively, PCR amplification of gene fragments can be carried out using anchor primers, which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (e.g., a GST polypeptide). The nucleic acid molecule of the invention or the nucleic acid molecule used in the method of the invention can be cloned into such an expression vector such that the fusion moiety is linked in-frame to the encoded protein.

**[0313.0.0.0]** Furthermore, folding simulations and computer redesign of structural motifs of the protein of the invention can be performed using appropriate computer programs (Olszewski, Proteins 25 (1996), 286-299; Hoffman, Comput. Appl. Biosci. 11 (1995), 675-679). Computer modelling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). The appropriate programs can be used for the identification of interactive sites the polypeptide of the invention or polypeptides used in the process of the invention and its substrates or binding factors or other interacting proteins by computer assistant searches for complementary peptide sequences (Fassina, Immunomethods (1994), 114-120). Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used for, e.g., the preparation of peptidomimetics of the protein of the invention or fragments thereof. Such pseudopeptide analogues of the, natural amino acid sequence of the protein may very efficiently mimic the parent protein (Benkirane, J. Biol. Chem. 271 (1996), 33218-33224). For example, incorporation of easily available achiral Q-amino acid residues into a protein of the invention or a fragment thereof results in the substitution of amide bonds by polymethylene units of an aliphatic chain, thereby providing a convenient strategy for constructing a peptidomimetic (Banerjee, Biopolymers 39 (1996), 769-777).

**[0314.0.0.0]** Superactive peptidomimetic analogues of small peptide hormones in other systems are described in the prior art (Zhang, Biochem. Biophys. Res. Commun. 224 (1996), 327-331). Appropriate peptidomimetics of the protein of the present invention can also be identified by the synthesis of peptidomimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. **[0315.0.0.0]** Furthermore, a three-dimensional and/or crystallographic structure of the protein of the invention can be used for the design of peptidomimetic inhibitors of the biological activity of the protein of the invention (Rose, Biochemistry 35 (1996), 12933-12944; Rutenber, Bioorg. Med. Chem. 4 (1996),1545-1558).

**[0316.0.0.0]** Furthermore, a three-dimensional and/or crystallographic structure of the protein of the invention and the identification of interactive sites the polypeptide of the invention or the polypeptide used in the method of the invention and its substrates or binding factors can be used for the identification or design of mutants with modulated binding or turn over activities. For example, the active centre of the polypeptide of the present invention can be modelled and amino acid residues participating in the catalytic reaction can be modulated to increase or decrease the binding of the substrate to activate or improve the polypeptide. The identification of the active centre and the amino acids involved in the catalytic reaction facilitates the screening for mutants having an increased activity.

**[0317.0.0.0]** The sequences shown in column 5 of the Tables I to IV herein have also been described under their Gene/ORF Locus Name as described in the Table I, II, III or IV, column 3.

**[0318.0.0.0]** In an especially preferred embodiment, the polypeptide according to the invention furthermore also does not have the sequences of those proteins which are encoded by the sequences shown in the known listed activity of Gene/ORF Locus Names or as described in the Tables, column 3.

**[0319.0.0.0]** One embodiment of the invention also relates to an antibody, which binds specifically to the polypeptide according to the invention or parts, i.e. specific fragments or epitopes of such a protein.

**[0320.0.0.0]** The antibodies of the invention can be used to identify and isolate the polypeptide according to the invention and encoding genes in any organism, preferably plants, prepared in plants described herein. These antibodies can be monoclonal antibodies, polyclonal antibodies or synthetic antibodies as well as fragments of antibodies, such as Fab, Fv or scFv fragments etc. Monoclonal antibodies can be prepared, for example, by the techniques as originally described in Köhler and Milstein, Nature 256 (1975), 495, and Galfr6, Meth. Enzymol. 73 (1981), 3, which comprise the fusion of mouse myeloma cells to spleen cells derived from immunized mammals.

**[0321.0.0.0]** Furthermore, antibodies or fragments thereof to the aforementioned peptides can be obtained by using methods, which are described, e.g., in Harlow and Lane "Antibodies, A Laboratory Manual", CSH Press, Cold Spring Harbor, 1988. These antibodies can be used, for example, for the immunoprecipitation and immunolocalization of proteins according to the invention as well as for the monitoring of the synthesis of such proteins, for example, in recombinant organisms, and for the identification of compounds interacting with the protein according to the invention. For example, surface plasmon resonance as employed in the BlAcore system can be used to increase the efficiency of phage antibodies selections, yielding a high increment of affinity from a single library of phage antibodies, which bind to an epitope of the protein of the invention (Schier, Human Antibodies Hybridomas 7 (1996), 97-105; Malmborg, J. Immunol. Methods 183 (1995), 7-13). In many cases, the binding phenomena of antibodies to antigens are equivalent to other ligand/anti-ligand binding.

**[0322.0.0.0]** In one embodiment, the present invention relates to an antisense nucleic acid molecule comprising the complementary sequence of the nucleic acid molecule of the present invention.

**[0323.0.0.0]** Methods to modify the expression levels and/or the activity are known to persons skilled in the art and include for instance overexpression, co-suppression, the use of ribozymes, sense and anti-sense strategies or other gene silencing approaches like RNA interference (RNAi) or promoter methylation. "Sense strand" refers to the strand of a double-stranded DNA molecule that is homologous to an mRNA transcript thereof. The "anti-sense strand" contains an inverted sequence, which is complementary to that of the "sense strand".
In addition the expression levels and/or the activity can be modified by the introduction of mutations in the regulatory or coding regions of the nucleic acids of the invention. Furthermore antibodies can be expressed which specifically binds to a polypeptide of interest and thereby blocks it activity. The protein-binding factors can, for example, also be aptamers [Famulok M and Mayer G (1999) Curr. Top Microbiol. Immunol. 243: 123-36] or antibodies or antibody fragments or single-chain antibodies. Obtaining these factors has been described, and the skilled worker is familiar therewith. For example, a cytoplasmic scFv antibody has been employed for modulating activity of the phytochrome A protein in genetically modified tobacco plants [Owen M et al. (1992) Biotechnology (NY) 10(7): 790-794; Franken E et al. (1997) Curr. Opin. Biotechnol. 8(4): 411-416; Whitelam (1996) Trend Plant Sci. 1: 286-272].

**[0324.0.0.0]** An "antisense" nucleic acid molecule comprises a nucleotide sequence, which is complementary to a "sense" nucleic acid molecule encoding a protein, e.g., complementary to the coding strand of a double-stranded cDNA molecule or complementary to an encoding mRNA sequence. Accordingly, an antisense nucleic acid molecule can bond via hydrogen bonds to a sense nucleic acid molecule. The antisense nucleic acid molecule can be complementary to an entire coding strand of a nucleic acid molecule conferring the expression of the polypeptide of the invention or used in the process of the present invention, as the nucleic acid molecule of the invention or the nucleic acid molecule used in the method of the invention coding strand, or to only a portion thereof. Accordingly, an antisense nucleic acid molecule can be antisense to a "coding region" of the coding strand of a nucleotide sequence of a nucleic acid molecule of the present invention. The term "coding region" refers to the region of the nucleotide sequence comprising codons, which are translated into amino acid residues. Further, the antisense nucleic acid molecule is antisense to a "noncoding region" of the coding strand of a nucleotide sequence encoding the polypeptide of the invention or a polypeptide used in the process of the invention. The term "noncoding region" refers to 5' and 3' sequences which flank the coding region that are not translated into a polypeptide, i.e., also referred to as 5' and 3' untranslated regions (5'-UTR or3'-UTR).

**[0325.0.0.0]** Given the coding strand sequences encoding the polypeptide of the present invention antisense nucleic acid molecules of the invention can be designed according to the rules of Watson and Crick base pairing.

**[0326.0.0.0]** The antisense nucleic acid molecule can be complementary to the entire coding region of the mRNA encoding the nucleic acid molecule to the invention or used in the process of the present invention, but can also be an oligonucleotide which is antisense to only a portion of the coding or noncoding region of said mRNA. For example, the antisense oligonucleotide can be complementary to the region surrounding the translation start site of said mRNA. An antisense oligonucleotide can be, for example, about 5, 10, 15, 20, 25, 30, 35, 40, 45, 50, 100 or 200 nucleotides in length. An antisense nucleic acid molecule of the invention or the nucleic acid molecule used in the method of the invention can be constructed using chemical synthesis and enzymatic ligation reactions using procedures known in the art. For example, an antisense nucleic acid molecule (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid molecule has been subcloned in an antisense orientation (i.e., RNA transcribed from the inserted nucleic acid molecule will be of an antisense orientation to a target nucleic acid molecule of interest, described further in the following subsection).

**[0327.0.0.0]** The antisense nucleic acid molecules of the invention are typically administered to a cell or generated *in situ* such that they hybridize with or bind to cellular mRNA and/or genomic DNA encoding a polypeptide of the invention or the polypeptide used in the method of the invention having aforementioned the respective fine chemical increasing activity to thereby inhibit expression of the protein, e.g., by inhibiting transcription and/or translation.

**[0328.0.0.0]** The hybridization can be by conventional nucleotide complementarity to form a stable duplex, or, for example, in the case of an antisense nucleic acid molecule which binds to DNA duplexes, through specific interactions in the major groove of the double helix. The antisense nucleic acid molecule can also be delivered to cells using the vectors described herein. To achieve sufficient intracellular concentrations of the antisense molecules, vector in which the antisense nucleic acid molecule is placed under the control of a strong prokaryotic, viral, or eukaryotic including plant promoters are preferred.

**[0329.0.0.0]** In a further embodiment, the antisense nucleic acid molecule of the invention or the nucleic acid molecule used in the method of the invention can be an - anomeric nucleic acid molecule. An α-anomeric nucleic acid molecule forms specific double-stranded hybrids with complementary RNA in which, contrary to the usual units, the strands run parallel to each other (Gaultier et al. (1987) Nucleic Acids. Res. 15:6625-6641). The antisense nucleic acid molecule can also comprise a 2'-o-methyl-ribonucleotide (Inoue et al. (1987) Nucleic Acids Res. 15:6131-6148) or a chimeric RNA-DNA analogue (Inoue et al. (1987) FEBS Lett. 215:327-330).

**[0330.0.0.0]** Further the antisense nucleic acid molecule of the invention or the nucleic acid molecule used in the method of the invention can be also a ribozyme. Ribozymes are catalytic RNA molecules with ribonuclease activity, which are capable of cleaving a single-stranded nucleic acid, such as an mRNA, to which they have a complementary region. Thus, ribozymes (e.g., hammerhead ribozymes (described in Haselhoff and Gerlach (1988) Nature 334:585-591)) can be used to catalytically cleave mRNA transcripts encoding the polypeptide of the invention or the polypeptide used in the method of the invention to thereby inhibit translation of said mRNA. A ribozyme having specificity for a nucleic acid molecule encoding the polypeptide of the invention or used in the process of the invention can be designed based upon the nucleotide sequence of the nucleic acid molecule of the invention or the nucleic acid molecule used in the method of the invention or coding a protein used in the process of the invention or on the basis of a heterologous sequence to be isolated according to methods taught in this invention. For example, a derivative of a *Tetrahymena* L-19 IVS RNA can be constructed in which the nucleotide sequence of the active site is complementary to the nucleotide sequence to be cleaved in an encoding mRNA. See, e.g., Cech et al. U.S. Patent No. 4,987,071 and Cech et al. U.S. Patent No. 5,116,742. Alternatively, mRNA encoding the polypeptide of the invention or a polypeptide used in the process of the invention can be used to select a catalytic RNA having a specific ribonuclease activity from a pool of RNA molecules. See, e.g., Bartel, D. and Szostak, J.W. (1993) Science 261:1411-1418.

**[0331.0.0.0]** The antisense molecule of the present invention comprises also a nucleic acid molecule comprising a nucleotide sequences complementary to the regulatory region of an nucleotide sequence encoding the natural occurring polypeptide of the invention or the polypeptide used in the method of the invention, e.g. the polypeptide sequences shown in the sequence listing, or identified according to the methods described herein, e.g., its promoter and/or enhancers, e.g. to form triple helical structures that prevent transcription of the gene in target cells. See generally, Helene, C. (1991) Anticancer Drug Des. 6(6): 569-84; Helene, C. et al. (1992) Ann. N.Y. Acad. Sci. 660:27-36; and Maher, L.J. (1992) Bioassays 14(12): 807-15.

**[0332.0.0.0]** Furthermore the present invention relates to a double stranded RNA molecule capable for the reduction or inhibition of the activity of the gene product of a gene encoding the polypeptide of the invention, a polypeptide used in the process of the invention, the nucleic acid molecule of the invention or a nucleic acid molecule used in the process of the invention encoding.

**[0333.0.0.0]** The method of regulating genes by means of double-stranded RNA ("double-stranded RNA interference"; dsRNAi) has been described extensively for animal, yeast, fungi and plant organisms such as Neurospora, zebrafish, Drosophila, mice, planaria, humans, Trypanosoma, petunia or Arabidopsis (for example Matzke MA et al. (2000) Plant Mol. Biol. 43: 401-415; Fire A. et al. (1998) Nature 391: 806-811; WO 99/32619; WO 99/53050; WO 00/68374; WO 00/44914; WO 00/44895; WO 00/49035; WO 00/63364). In addition RNAi is also documented as an advantageously tool for the repression of genes in bacteria such as E. coli for example by Tchurikov et al. [J. Biol. Chem., 2000, 275 (34): 26523 - 26529]. Fire et al. named the phenomenon RNAi for "RNA interference". The techniques and methods described in the above references are expressly referred to. Efficient gene suppression can also be observed in the case of transient expression or following transient transformation, for example as the consequence of a biolistic transformation (Schweizer P et al. (2000) Plant J 2000 24: 895-903). dsRNAi methods are based on the phenomenon that the simultaneous introduction of complementary strand and counterstrand of a gene transcript brings about highly effective suppression of the expression of the gene in question. The resulting phenotype is very similar to that of an analogous knock-out mutant (Waterhouse PM et al. (1998) Proc. Natl. Acad. Sci. USA 95: 13959-64).

**[0334.0.0.0]** Tuschl et al. [Gens Dev., 1999, 13 (24): 3191 - 3197] was able to show that the efficiency of the RNAi method is a function of the length of the duplex, the length of the 3'-end overhangs, and the sequence in these overhangs. Based on the work of Tuschl et al. the following guidelines can be given to the skilled worker: To achieve good results the 5' and 3' untranslated regions of the used nucleic acid sequence and regions close to the start codon should be avoided as this regions are richer in regulatory protein binding sites and interactions between RNAi sequences and such regulatory proteins might lead to undesired interactions. Preferably a region of the used mRNA is selected, which is 50 to 100 nt (= nucleotides or bases) downstream of the AUG start codon. Only dsRNA (= double-stranded RNA) sequences from exons are useful for the method, as sequences from introns have no effect. The G/C content in this region should be greater than 30% and less than 70% ideally around 50%. A possible secondary structure of the target mRNA is less important for the effect of the RNAi method.

**[0335.0.0.0]** The dsRNAi method has proved to be particularly effective and advantageous for reducing the expression of a nucleic acid sequences as indicated in Table I, application no. 0, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, application no. 0, columns 5 or 7, and/or homologs thereof. In a double-stranded RNA molecule for reducing the expression of an protein encoded by a nucleic acid sequence of one of the sequences as indicated in Table I, application no. 0, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.0]** The term "essentially identical" refers to the fact that the dsRNA sequence may also include insertions, deletions and individual point mutations in comparison to the target sequence while still bringing about an effective reduction in expression. Preferably, the homology as defined above amounts to at least 30%, preferably at least 40%, 50%, 60%, 70% or 80%, very especially preferably at least 90%, most preferably 100%, between the "sense" strand of an inhibitory dsRNA and a part-segment of a nucleic acid sequence of the invention (or between the "antisense" strand and the complementary strand of a nucleic acid sequence, respectively). The part-segment amounts to at least 10 bases, preferably at least 17, 18, 19, 20, 21, 22, 23, 24, 25, 26, 27, 28, 29 or 30 bases, especially preferably at least 40, 50, 60, 70, 80 or 90 bases, very especially preferably at least 100, 200, 300 or 400 bases, most preferably at least 500, 600, 700, 800, 900 or more bases or at least 1000 or 2000 bases or more in length. In another preferred embodiment of the invention the part-segment amounts to 17, 18, 19, 20, 21, 22, 23, 24, 25, 26 or 27 bases, preferably to 20, 21, 22, 23, 24 or 25 bases. These short sequences are preferred in animals and plants. The longer sequences preferably between 200 and 800 bases are preferred in non-mammalian animals, preferably in invertebrates, in yeast, fungi or bacteria, but they are also useable in plants. Long double-stranded RNAs are processed in the organisms into many siRNAs (= small/short interfering RNAs) for example by the protein Dicer, which is a ds-specific Rnase III enzyme. As an alternative, an "essentially identical" dsRNA may also be defined as a nucleic acid sequence, which is capable of hybridizing with part of a gene transcript (for example in 400 mM NaCl, 40 mM PIPES pH 6.4, 1 mM EDTA at 50°C or 70°C for 12 to 16 h).

**[0337.0.0.0]** The dsRNA may consist of one or more strands of polymerized ribonucleotides. Modification of both the sugar-phosphate backbone and of the nucleosides may furthermore be present. For example, the phosphodiester bonds of the natural RNA can be modified in such a way that they encompass at least one nitrogen or sulfur heteroatom. Bases may undergo modification in such a way that the activity of, for example, adenosine deaminase is restricted. These and other modifications are described herein below in the methods for stabilizing antisense RNA.

**[0338.0.0.0]** The dsRNA can be prepared enzymatically; it may also be synthesized chemically, either in full or in part.

**[0339.0.0.0]** The double-stranded structure can be formed starting from a single, self-complementary strand or starting from two complementary strands. In a single, self-complementary strand, "sense" and "antisense" sequence can be linked by a linking sequence ("linker") and form for example a hairpin structure. Preferably, the linking sequence may take the form of an intron, which is spliced out following dsRNA synthesis. The nucleic acid sequence encoding a dsRNA may contain further elements such as, for example, transcription termination signals or polyadenylation signals. If the two strands of the dsRNA are to be combined in a cell or an organism advantageously in a plant, this can be brought about in a variety of ways.

**[0340.0.0.0]** Formation of the RNA duplex can be initiated either outside the cell or within the cell. As shown in WO 99/53050, the dsRNA may also encompass a hairpin structure, by linking the "sense" and "antisense" strands by a "linker" (for example an intron). The self-complementary dsRNA structures are preferred since they merely require the expression of a construct and always encompass the complementary strands in an equimolar ratio.

**[0341.0.0.0]** The expression cassettes encoding the "antisense" or the "sense" strand of the dsRNA or the self-complementary strand of the dsRNA are preferably inserted into a vector and stably inserted into the genome of a plant, using the methods described herein below (for example using selection markers), in order to ensure permanent expression of the dsRNA.

**[0342.0.0.0]** The dsRNA can be introduced using an amount which makes possible at least one copy per cell. A larger amount (for example at least 5, 10, 100, 500 or 1 000 copies per cell) may bring about more efficient reduction.

**[0343.0.0.0]** As has already been described, 100 % sequence identity between the dsRNA and a gene transcript of a nucleic acid sequence as indicated in Table I, application no. 0, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 0, columns 5 or 7, or homologs thereof of the one organism, may be used to suppress the corresponding expression in another organism.

**[0344.0.0.0]** Due to the high degree of sequence homology between sequences from various organisms (e. g. plants), allows the conclusion that these proteins may be conserved to a high degree within, for example other, plants, it is optionally possible that the expression of a dsRNA derived from one of the disclosed sequences as shown herein or homologs thereof should also have has an advantageous effect in other plant species. Preferably the consensus sequences shown herein can be used for the construction of useful dsRNA molecules.

**[0345.0.0.0]** The dsRNA can be synthesized either in vivo or in vitro. To this end, a DNA sequence encoding a dsRNA can be introduced into an expression cassette under the control of at least one genetic control element (such as, for example, promoter, enhancer, silencer, splice donor or splice acceptor or polyadenylation signal). Suitable advantageous constructs are described herein below. Polyadenylation is not required, nor do elements for initiating translation have to be present.

**[0346.0.0.0]** A dsRNA can be synthesized chemically or enzymatically. Cellular RNA polymerases or bacteriophage RNA polymerases (such as, for example T3, T7 or SP6 RNA polymerase) can be used for this purpose. Suitable methods for the in-vitro expression of RNA are described (WO 97/32016; US 5,593,874; US 5,698,425, US 5,712,135, US 5,789,214, US 5,804,693). Prior to introduction into a cell, tissue or organism, a dsRNA which has been synthesized in vitro either chemically or enzymatically can be isolated to a higher or lesser degree from the reaction mixture, for example by extraction, precipitation, electrophoresis, chromatography or combinations of these methods. The dsRNA can be introduced directly into the cell or else be applied extracellularly (for example into the interstitial space).

**[0347.0.0.0]** Advantageously the RNAi method leads to only a partial loss of gene function and therefore enables the skilled worker to study a gene dose effect in the desired organism and to fine tune the process of the invention. Furthermore it enables a person skilled in the art to study multiple functions of a gene.

**[0348.0.0.0]** Stable transformation of the plant with an expression construct, which brings about the expression of the dsRNA is preferred, however. Suitable methods are described herein below.

**[0349.0.0.0]** A further embodiment of the invention also relates to a method for the generation of a transgenic host or host cell, e.g. a eukaryotic or prokaryotic cell, preferably a transgenic microorganism, a transgenic plant cell or a transgenic plant tissue or a transgenic plant, which comprises introducing, into the plant, the plant cell or the plant tissue, the nucleic acid construct according to the invention, the vector according to the invention, or the nucleic acid molecule according to the invention.

**[0350.0.0.0]** A further embodiment of the invention also relates to a method for the transient generation of a host or host cell, eukaryotic or prokaryotic cell, preferably a transgenic microorganism, a transgenic plant cell or a transgenic plant tissue or a transgenic plant, which comprises introducing, into the plant, the plant cell or the plant tissue, the nucleic acid construct according to the invention, the vector according to the invention, the nucleic acid molecule characterized herein as being contained in the nucleic acid construct of the invention or the nucleic acid molecule according to the invention, whereby the introduced nucleic acid molecules, nucleic acid construct and/or vector is not integrated into the genome of the host or host cell. Therefore the transformants are not stable during the propagation of the host in respect of the introduced nucleic acid molecules, nucleic acid construct and/or vector.

**[0351.0.0.0]** In the process according to the invention, transgenic organisms are also to be understood as meaning - if they take the form of plants - plant cells, plant tissues, plant organs such as root, shoot, stem, seed, flower, tuber or leaf, or intact plants which are grown for the production of the respective fine chemical.

**[0352.0.0.0]** Growing is to be understood as meaning for example culturing the transgenic plant cells, plant tissue or plant organs on or in a nutrient medium or the intact plant on or in a substrate, for example in hydroponic culture, potting compost or on a field soil.

**[0353.0.0.0]** In a further advantageous embodiment of the process, the nucleic acid molecules can be expressed in single-celled plant cells (such as algae), see Falciatore et al., 1999, Marine Biotechnology 1 (3): 239-251 and references cited therein, and plant cells from higher plants (for example spermatophytes such as crops). Examples of plant expression vectors encompass those which are described in detail herein or in: Becker, D. [(1992) Plant Mol. Biol. 20:1195-1197] and Bevan, M.W. [(1984), Nucl. Acids Res. 12:8711-8721; Vectors for Gene Transfer in Higher Plants; in: Transgenic Plants, Vol. 1, Engineering and Utilization, Ed.: Kung and R. Wu, Academic Press, 1993, pp. 15-38]. An overview of binary vectors and their use is also found in Hellens, R. [(2000), Trends in Plant Science, Vol. 5 No.10, 446-451.

**[0354.0.0.0]** Vector DNA can be introduced into prokaryotic or eukaryotic cells via conventional transformation or transfection techniques. The terms "transformation" and "transfection" include conjugation and transduction and, as used in the present context, are intended to encompass a multiplicity of prior-art methods for introducing foreign nucleic acid molecules (for example DNA) into a host cell, including calcium phosphate coprecipitation or calcium chloride coprecipitation, DEAE-dextran-mediated transfection, PEG-mediated transfection, lipofection, natural competence, chemically mediated transfer, electroporation or particle bombardment. Suitable methods for the transformation or transfection of host cells, including plant cells, can be found in Sambrook et al. (Molecular Cloning: A Laboratory Manual., 2nd Ed., Cold Spring Harbor Laboratory, Cold Spring Harbor Laboratory Press, Cold Spring Harbor, NY, 1989) and in other laboratory handbooks such as Methods in Molecular Biology, 1995, Vol. 44, Agrobacterium protocols, Ed.: Gartland and Davey, Humana Press, Totowa, New Jersey.

**[0355.0.0.0]** The above-described methods for the transformation and regeneration of plants from plant tissues or plant cells are exploited for transient or stable transformation of plants. Suitable methods are the transformation of protoplasts by polyethylene-glycol-induced DNA uptake, the biolistic method with the gene gun - known as the particle bombardment method -, electroporation, the incubation of dry embryos in DNA-containing solution, microinjection and the Agrobacterium-mediated gene transfer. The abovementioned methods are described for example in B. Jenes, Techniques for Gene Transfer, in: Transgenic Plants, Vol. 1, Engineering and Utilization, edited by S.D. Kung and R. Wu, Academic Press (1993) 128-143 and in Potrykus Annu. Rev. Plant Physiol. Plant Molec. Biol. 42 (1991) 205-225. The construct to be expressed is preferably cloned into a vector, which is suitable for transforming Agrobacterium tumefaciens, for example pBin19 (Bevan, Nucl. Acids Res. 12 (1984) 8711). Agrobacteria transformed with such a vector can then be used in the known manner for the transformation of plants, in particular crop plants, such as, for example, tobacco plants, for example by bathing scarified leaves or leaf segments in an agrobacterial solution and subsequently culturing them in suitable media. The transformation of plants with Agrobacterium tumefaciens is described for example by Höfgen and Willmitzer in Nucl. Acid Res. (1988) 16, 9877 or known from, inter alia, F.F. White, Vectors for Gene Transfer in Higher Plants; in Transgenic Plants, Vol. 1, Engineering and Utilization, edited by S.D. Kung and R. Wu, Academic Press, 1993, pp. 15-38.

**[0356.0.0.0]** To select for the successful transfer of the nucleic acid molecule, vector or nucleic acid construct of the invention according to the invention into a host organism, it is advantageous to use marker genes as have already been described above in detail. It is known of the stable or transient integration of nucleic acids into plant cells that only a minority of the cells takes up the foreign DNA and, if desired, integrates it into its genome, depending on the expression vector used and the transfection technique used. To identify and select these integrants, a gene encoding for a selectable marker (as described above, for example resistance to antibiotics) is usually introduced into the host cells together with the gene of interest. Preferred selectable markers in plants comprise those, which confer resistance to an herbicide such as glyphosate or gluphosinate. Other suitable markers are, for example, markers, which encode genes involved in biosynthetic pathways of, for example, sugars or amino acids, such as β-galactosidase, ura3 or ilv2. Markers, which encode genes such as luciferase, gfp or other fluorescence genes, are likewise suitable. These markers and the aforementioned markers can be used in mutants in whom these genes are not functional since, for example, they have been deleted by conventional methods. Furthermore, nucleic acid molecules, which encode a selectable marker, can be introduced into a host cell on the same vector as those, which encode the polypeptides of the invention or used in the process or else in a separate vector. Cells which have been transfected stably with the nucleic acid introduced can be identified for example by selection (for example, cells which have integrated the selectable marker survive whereas the other cells die).

**[0357.0.0.0]** Since the marker genes, as a rule specifically the gene for resistance to antibiotics and herbicides, are no longer required or are undesired in the transgenic host cell once the nucleic acids have been introduced successfully, the process according to the invention for introducing the nucleic acids advantageously employs techniques which enable the removal, or excision, of these marker genes. One such a method is what is known as cotransformation. The cotransformation method employs two vectors simultaneously for the transformation, one vector bearing the nucleic acid according to the invention and a second bearing the marker gene(s). A large proportion of transformants receives or, in the case of plants, comprises (up to 40% of the transformants and above), both vectors. In case of transformation with Agrobacteria, the transformants usually receive only a part of the vector, the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase resource or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases, the marker gene must be eliminated by performing crosses. In microbiology, techniques were developed which make possible, or facilitate, the detection of such events. A further advantageous method relies on what are known as recombination systems, whose advantage is that elimination by crossing can be dispensed with. The best-known system of this type is what is known as the Cre/lox system. Cre1 is a recombinase, which removes the sequences located between the loxP sequences. If the marker gene is integrated between the loxP sequences, it is removed, once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

**[0358.0.0.0]** Agrobacteria transformed with an expression vector according to the invention may also be used in the manner known per se for the transformation of plants such as experimental plants like Arabidopsis or crop plants, such as, for example, cereals, maize, oats, rye, barley, wheat, soya, rice, cotton, sugarbeet, canola, sunflower, flax, hemp, potato, tobacco, tomato, carrot, bell peppers, oilseed rape, tapioca, cassava, arrow root, tagetes, alfalfa, lettuce and the various tree, nut, and grapevine species, in particular oil-containing crop plants such as soya, peanut, castor-oil plant, sunflower, maize, cotton, flax, oilseed rape, coconut, oil palm, safflower (Carthamus tinctorius) or cocoa beans, for example by bathing scarified leaves or leaf segments in an agrobacterial solution and subsequently growing them in suitable media.

**[0359.0.0.0]** In addition to the transformation of somatic cells, which then has to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic (Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289). Alternative methods are based on the repeated removal of the influorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension (Bechthold, N (1993). C R Acad Sci Paris Life Sci, 316: 1194-1199), while in the case of the"floral dip" method the developing floral tissue is incubated briefly with a surfactant-treated agrobacterial suspension (Clough, SJ und Bent, AF (1998). The Plant J. 16, 735-743). A certain proportion of transgenic seeds are harvested in both cases, and these seeds can be distinguished from nontransgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process, which has been schematically displayed in Klaus et al., 2004 (Nature Biotechnology 22(2), 225-229). Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview can be taken from Bock (2001) Transgenic plastids in basic research and plant biotechnology. J Mol Biol. 2001 Sep 21; 312 (3): 425-38 or Maliga, P (2003) Progress towards commercialization of plastid transformation technology. Trends Biotechnol. 21, 20-28. Further biotechnological progress has recently been reported in form of marker free plastid transformants, which can be produced by a transient cointegrated maker gene (Klaus et al., 2004, Nature Biotechnology 22 (2), 225-229).

**[0360.0.0.0]** The genetically modified plant cells can be regenerated via all methods with which the skilled worker is familiar. Suitable methods can be found in the abovementioned publications by S.D. Kung and R. Wu, Potrykus or Höfgen and Willmitzer.

**[0361.0.0.0]** Accordingly, the present invention thus also relates to a plant cell comprising the nucleic acid construct according to the invention, the nucleic acid molecule according to the invention or the vector according to the invention.

**[0362.0.0.0]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention or the polypeptide used in the method of the invention, e.g. the polypeptide as indicated in Table II, application no. 0, columns 5 or 7 cell or an organism is increased. For example, due to modulation or manipulation, the cellular activity of the polypeptide of the invention or the polypeptide used in the method of the invention or the nucleic acid molecule of the invention or the nucleic acid molecule used in the method of the invention is increased, e.g. due to an increased expression or specific activity of the subject matters of the invention in a cell or an organism or a part thereof. In one embodiment, transgenic for a polypeptide having an activity of a polypeptide as indicated in Table II, application no. 0, columns 5 or 7, means herein that due to modulation or manipulation of the genome, an activity as annotated for a polypeptide as indicated in Table II, application no. 0, column 3, e.g. having a sequence as indicated in Table II, application no. 0, columns 5 or 7, is increased in a cell or an organism or a part thereof. Examples are described above in context with the process of the invention

**[0363.0.0.0]** "Transgenic", for example regarding a nucleic acid molecule, an nucleic acid construct or a vector comprising said nucleic acid molecule or an organism transformed with said nucleic acid molecule, nucleic acid construct or vector, refers to all those subjects originating by recombinant methods in which either
a) the nucleic acid sequence, or
b) a genetic control sequence linked operably to the nucleic acid sequence, for example a promoter, or
c) (a) and (b)
are not located in their natural genetic environment or have been modified by recombinant methods, an example of a modification being a substitution, addition, deletion, inversion or insertion of one or more nucleotide residues. Natural genetic environment refers to the natural chromosomal locus in the organism of origin, or to the presence in a genomic library. In the case of a genomic library, the natural genetic environment of the nucleic acid sequence is preferably retained, at least in part. The environment flanks the nucleic acid sequence at least at one side and has a sequence of at least 50 bp, preferably at least 500 bp, especially preferably at least 1000 bp, very especially preferably at least 5000 bp, in length.

**[0364.0.0.0]** A naturally occurring expression cassette - for example the naturally occurring combination of a promoter of a polypeptide of the invention with the corresponding protein-encoding sequence - becomes a transgenic expression cassette when it is modified by non-natural, synthetic "artificial" methods such as, for example, mutagenization. Such methods have been described (US 5,565,350; WO 00/15815; also see above).

**[0365.0.0.0]** Further, the plant cell, plant tissue or plant can also be transformed such that further enzymes and proteins are (over)expressed which expression supports an increase of the respective fine chemical.

**[0366.0.0.0]** However, transgenic also means that the nucleic acids according to the invention are located at their natural position in the genome of an organism, but that the sequence has been modified in comparison with the natural sequence and/or that the regulatory sequences of the natural sequences have been modified. Preferably, transgenic/recombinant is to be understood as meaning the transcription of the nucleic acids used in the process according to the invention occurs at a non-natural position in the genome, that is to say the expression of the nucleic acids is homologous or, preferably, heterologous. This expression can be transiently or of a sequence integrated stably into the genome.

**[0367.0.0.0]** The term "transgenic plants" used in accordance with the invention also refers to the progeny of a transgenic plant, for example the T₁, T₂, T₃ and subsequent plant generations or the BC₁, BC₂, BC₃ and subsequent plant generations. Thus, the transgenic plants according to the invention can be raised and selfed or crossed with other individuals in order to obtain further transgenic plants according to the invention. Transgenic plants may also be obtained by propagating transgenic plant cells vegetatively. The present invention also relates to transgenic plant material, which can be derived from a transgenic plant population according to the invention. Such material includes plant cells and certain tissues, organs and parts of plants in all their manifestations, such as seeds, leaves, anthers, fibers, tubers, roots, root hairs, stems, embryo, calli, cotelydons, petioles, harvested material, plant tissue, reproductive tissue and cell cultures, which are derived from the actual transgenic plant and/or can be used for bringing about the transgenic plant.

**[0368.0.0.0]** Any transformed plant obtained according to the invention can be used in a conventional breeding scheme or in *in vitro* plant propagation to produce more transformed plants with the same characteristics and/or can be used to introduce the same characteristic in other varieties of the same or related species. Such plants are also part of the invention. Seeds obtained from the transformed plants genetically also contain the same characteristic and are part of the invention. As mentioned before, the present invention is in principle applicable to any plant and crop that can be transformed with any of the transformation method known to those skilled in the art.

**[0369.0.0.0]** In an especially preferred embodiment, the organism, the host cell, plant cell, plant, microorganism or plant tissue according to the invention is transgenic.

**[0370.0.0.0]** Accordingly, the invention therefore relates to transgenic organisms transformed with at least one nucleic acid molecule, nucleic acid construct or vector according to the invention, and to cells, cell cultures, tissues, parts - such as, for example, in the case of plant organisms, plant tissue, for example leaves, roots and the like - or propagation material derived from such organisms, or intact plants. The terms "recombinant (host)", and "transgenic (host)" are used interchangeably in this context. Naturally, these terms refer not only to the host organism or target cell in question, but also to the progeny, or potential progeny, of these organisms or cells. Since certain modifications may occur in subsequent generations owing to mutation or environmental effects, such progeny is not necessarily identical with the parental cell, but still comes within the scope of the term as used herein.

**[0371.0.0.0]** Suitable organisms for the process according to the invention or as hosts are all these eukaryotic or prokaryotic organisms, which are capable of synthesizing the respective fine chemical. The organisms used as hosts are microorganisms, such as bacteria, fungi, yeasts or algae, non-human animals, or plants, such as dictotyledonous or monocotyledonous plants.

**[0372.0.0.0]** In principle all plants can be used as host organism, especially the plants mentioned above as source organism. Preferred transgenic plants are, for example, selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants such as plants advantageously selected from the group of the genus peanut, oilseed rape, canola, sunflower, safflower, olive, sesame, hazelnut, almond, avocado, bay, pumpkin/squash, linseed, soya, pistachio, borage, maize, wheat, rye, oats, sorghum and millet, triticale, rice, barley, cassava, potato, sugarbeet, egg plant, alfalfa, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, dwarf bean, lupin, clover and Lucerne for mentioning only some of them.

**[0373.0.0.0]** Preferred plant cells, plant organs, plant tissues or parts of plants originate from the under source organism mentioned plant families, preferably from the abovementioned plant genus, more preferred from abovementioned plants species.

**[0374.0.0.0]** Transgenic plants comprising the amino acids synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. The amino acids produced in the process according to the invention may, however, also be isolated from the plant in the form of their free amino acids or bound in proteins. Amino acids produced by this process can be harvested by harvesting the organisms either from the culture in which they grow or from the field. This can be done via expressing, grinding and/or extraction, salt precipitation and/or ion-exchange chromatography of the plant parts, preferably the plant seeds, plant fruits, plant tubers and the like.

**[0375.0.0.0]** In a further embodiment, the present invention relates to a process for the generation of a microorganism, comprising the introduction, into the microorganism or parts thereof, of the nucleic acid construct of the invention, or the vector of the invention or the nucleic acid molecule of the invention or the nucleic acid molecule used in the method of the invention.

**[0376.0.0.0]** In another embodiment, the present invention relates also to a transgenic microorganism comprising the nucleic acid molecule of the invention or the nucleic acid molecule used in the method of the invention, the nucleic acid construct of the invention or the vector as of the invention. Appropriate microorganisms have been described herein before under source organism, preferred are in particular aforementioned strains suitable for the production of fine chemicals.

**[0377.0.0.0]** Accordingly, the present invention relates also to a process according to the present invention whereby the produced amino acid composition or the produced respective fine chemical is isolated.

**[0378.0.0.0]** In this manner, more than 50% by weight, advantageously more than 60% by weight, preferably more than 70% by weight, especially preferably more than 80% by weight, very especially preferably more than 90% by weight, of the fine chemicals produced in the process can be isolated. The resulting fine chemicals can, if appropriate, subsequently be further purified, if desired mixed with other active ingredients such as vitamins, amino acids, carbohydrates, antibiotics and the like, and, if appropriate, formulated.

**[0379.0.0.0]** In one embodiment, the fatty acid is the fine chemical.

**[0380.0.0.0]** The amino acids obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of a pharmaceuticals, food stuff, animal feeds, nutrients or cosmetics comprising the steps of the process according to the invention, including the isolation of the amino acid composition produced or the fine chemical produced if desired and formulating the product with a pharmaceutical acceptable carrier or formulating the product in a form acceptable for an application in agriculture. A further embodiment according to the invention is the use of the amino acids produced in the process or of the transgenic organisms in animal feeds, foodstuffs, medicines, food supplements, cosmetics or pharmaceuticals.

**[0381.0.0.0]** In principle all microorganisms can be used as host organism especially the ones mentioned under source organism above. It is advantageous to use in the process of the invention transgenic microorganisms such as fungi such as the genus Claviceps or Aspergillus or Gram-positive bacteria such as the genera Bacillus, Corynebacterium, Micrococcus, Brevibacterium, Rhodococcus, Nocardia, Caseobacter or Arthrobacter or Gram-negative bacteria such as the genera Escherichia, Flavobacterium or Salmonella or yeasts such as the genera Rhodotorula, Hansenula or Candida. Particularly advantageous organisms are selected from the group of genera Corynebacterium, Brevibacterium, Escherichia, Bacillus, Rhodotorula, Hansenula, Candida, Claviceps or Flavobacterium. It is very particularly advantageous to use in the process of the invention microorganisms selected from the group of genera and species consisting of Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Escherichia coli K12 and its described strains.

**[0382.0.0.0]** The process of the invention is, when the host organisms are microorganisms, advantageously carried out at a temperature between 0°C and 95°C, preferably between 10°C and 85°C, particularly preferably between 15°C and 75°C, very particularly preferably between 15°C and 45°C. The pH is advantageously kept at between pH 4 and 12, preferably between pH 6 and 9, particularly preferably between pH 7 and 8, during this. The process of the invention can be operated batchwise, semibatchwise or continuously. A summary of known cultivation methods is to be found in the textbook by Chmiel (Bioprozeßtechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)). The culture medium to be used must meet the requirements of the respective strains in a suitable manner. Descriptions of culture media for various microorganisms are present in the handbook "Manual of Methods for General Bacteriology" of the American Society for Bacteriology (Washington D. C., USA, 1981). These media, which can be employed according to the invention include, as described above, usually one or more carbon sources, nitrogen sources, inorganic salts, vitamins and/or trace elements. Preferred carbon sources are sugars such as mono-, di- or polysaccharides. Examples of very good carbon sources are glucose, fructose, mannose, galactose, ribose, sorbose, ribulose, lactose, maltose, sucrose, raffinose, starch or cellulose. Sugars can also be added to the media via complex compounds such as molasses, or other byproducts of sugar refining. It may also be advantageous to add mixtures of various carbon sources. Other possible carbon sources are oils and fats such as, for example, soybean oil, sunflower oil, peanut oil and/or coconut fat, fatty acids such as, for example, palmitic acid, stearic acid and/or linoleic acid, alcohols and/or polyalcohols such as, for example, glycerol, methanol and/or ethanol and/or organic acids such as, for example, acetic acid and/or lactic acid. Nitrogen sources are usually organic or inorganic nitrogen compounds or materials, which contain these compounds. Examples of nitrogen sources include ammonia in liquid or gaseous form or ammonium salts such as ammonium sulfate, ammonium chloride, ammonium phosphate, ammonium carbonate or ammonium nitrate, nitrates, urea, amino acids or complex nitrogen sources such as corn steep liquor, soybean meal, soybean protein, yeast extract, meat extract and others. The nitrogen sources may be used singly or as a mixture. Inorganic salt compounds, which may be present in the media include the chloride, phosphorus or sulfate salts of calcium, magnesium, sodium, cobalt, molybdenum, potassium, manganese, zinc, copper and iron.

**[0383.0.0.0]** For preparing sulfur-containing fine chemicals, in particular the respective fine chemical, e.g. amino acids containing sulfur it is possible to use as sulfur source inorganic sulfur-containing compounds such as, for example, sulfates, sulfites, dithionites, tetrathionates, thiosulfates, sulfides or else organic sulfur compounds such as mercaptans and thiols.

**[0384.0.0.0]** It is possible to use as phosphorus source phosphoric acid, potassium dihydrogenphosphate or dipotassium hydrogenphosphate or the corresponding sodium-containing salts. Chelating agents can be added to the medium in order to keep the metal ions in solution. Particularly suitable chelating agents include dihydroxyphenols such as catechol or protocatechuate, or organic acids such as citric acid. The fermentation media employed according to the invention for cultivating microorganisms normally also contain other growth factors such as vitamins or growth promoters, which include, for example, biotin, riboflavin, thiamine, folic acid, nicotinic acid, pantothenate and pyridoxine. Growth factors and salts are often derived from complex media components such as yeast extract, molasses, corn steep liquor and the like. Suitable precursors can moreover be added to the culture medium. The exact composition of the media compounds depends greatly on the particular experiment and is chosen individually for each specific case. Information about media optimization is obtainable from the textbook "Applied Microbiol. Physiology, A Practical Approach" (editors P.M. Rhodes, P.F. Stanbury, IRL Press (1997) pp. 53-73, ISBN 0 19 963577 3). Growth media can also be purchased from commercial suppliers such as Standard 1 (Merck) or BHI (Brain heart infusion, DIFCO) and the like. All media components are sterilized either by heat (1.5 bar and 121°C for 20 min) or by sterilizing filtration. The components can be sterilized either together or, if necessary, separately. All media components can be present at the start of the cultivation or optionally be added continuously or batchwise. The temperature of the culture is normally between 15°C and 45°C, preferably at 25°C to 40°C, and can be kept constant or changed during the experiment. The pH of the medium should be in the range from 5 to 8.5, preferably around 7. The pH for the cultivation can be controlled during the cultivation by adding basic compounds such as sodium hydroxide, potassium hydroxide, ammonia or aqueous ammonia or acidic compounds such as phosphoric acid or sulfuric acid. Foaming can be controlled by employing antifoams such as, for example, fatty acid polyglycol esters. The stability of plasmids can be maintained by adding to the medium suitable substances having a selective effect, for example antibiotics. Aerobic conditions are maintained by introducing oxygen or oxygen-containing gas mixtures such as, for example, ambient air into the culture. The temperature of the culture is normally from 20°C to 45°C and preferably from 25°C to 40°C. The culture is continued until formation of the desired product is at a maximum. This aim is normally achieved within 10 hours to 160 hours.

**[0385.0.0.0]** The fermentation broths obtained in this way, containing in particular L-methionine, L-threonine and/or L-lysine, normally have a dry matter content of from 7.5 to 25% by weight. Sugar-limited fermentation is additionally advantageous, at least at the end, but especially over at least 30% of the fermentation time. This means that the concentration of utilizable sugar in the fermentation medium is kept at, or reduced to, 0 to 3 g/I during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.0.0]** However, it is also possible to purify the amino acid produced further. For this purpose, the product-containing composition is subjected to a chromatography on a suitable resin, in which case the desired product or the impurities are retained wholly or partly on the chromatography resin. These chromatography steps can be repeated if necessary, using the same or different chromatography resins. The skilled worker is familiar with the choice of suitable chromatography resins and their most effective use. The purified product can be concentrated by filtration or ultrafiltration and stored at a temperature at which the stability of the product is a maximum.

**[0387.0.0.0]** The identity and purity of the isolated compound(s) can be determined by prior art techniques. These include high performance liquid chromatography (HPLC), spectroscopic methods, mass spectrometry (MS), staining methods, thin-layer chromatography, NIRS, enzyme assay or microbiological assays. These analytical methods are summarized in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Vol. A27, VCH: Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Vol. 17.

**[0388.0.0.0]** In yet another aspect, the invention also relates to harvestable parts and to propagation material of the transgenic plants according to the invention which either contain transgenic plant cells expressing a nucleic acid molecule according to the invention or which contains cells which show an increased cellular activity of the polypeptide of the invention or the polypeptide used in the method of the invention, e.g. an increased expression level or higher activity of the described protein.

**[0389.0.0.0]** Harvestable parts can be in principle any useful parts of a plant, for example, flowers, pollen, seedlings, tubers, leaves, stems, fruit, seeds, roots etc. Propagation material includes, for example, seeds, fruits, cuttings, seedlings, tubers, rootstocks etc. Preferred are seeds, fruits, seedlings or tubers as harvestable or propagation material.

**[0390.0.0.0]** The invention furthermore relates to the use of the transgenic organisms according to the invention and of the cells, cell cultures, parts - such as, for example, roots, leaves and the like as mentioned above in the case of transgenic plant organisms - derived from them, and to transgenic propagation material such as seeds or fruits and the like as mentioned above, for the production of foodstuffs or feeding stuffs, pharmaceuticals or fine chemicals.

**[0391.0.0.0]** Accordingly in another embodiment, the present invention relates to the use of the nucleic acid molecule, the organism, e.g. the microorganism, the plant, plant cell or plant tissue, the vector, or the polypeptide of the present invention for making fatty acids, carotenoids, isoprenoids, vitamins, lipids, wax esters, (poly)saccharides and/or polyhydroxyalkanoates, and/or its metabolism products, in particular, steroid hormones, cholesterol, prostaglandin, triacylglycerols, bile acids and/or ketone bodies producing cells, tissues and/or plants. There are a number of mechanisms by which the yield, production, and/or efficiency of production of fatty acids, carotenoids, isoprenoids, vitamins, wax esters, lipids, (poly)saccharides and/or polyhydroxyalkanoates, and/or its metabolism products, in particular, steroid hormones, cholesterol, triacylglycerols, prostaglandin, bile acids and/or ketone bodies or further of above defined fine chemicals incorporating such an altered protein can be affected. In the case of plants, by e.g. increasing the expression of acetyl-CoA which is the basis for many products, e.g., fatty acids, carotenoids, isoprenoids, vitamines, lipids, (poly)saccharides, wax esters, and/or polyhydroxyalkanoates, and/or its metabolism products, in particular, prostaglandin, steroid hormones, cholesterol, triacylglycerols, bile acids and/or ketone bodies in a cell, it may be possible to increase the amount of the produced said compounds thus permitting greater ease of harvesting and purification or in case of plants more efficient partitioning. Further, one or more of said metabolism products, increased amounts of the cofactors, precursor molecules, and intermediate compounds for the appropriate biosynthetic pathways maybe required. Therefore, by increasing the number and/or activity of transporter proteins involved in the import of nutrients, such as carbon sources (i.e., sugars), nitrogen sources (i.e., amino acids, ammonium salts), phosphate, and sulfur, it may be possible to improve the production of acetyl CoA and its metabolism products as mentioned above, due to the removal of any nutrient supply limitations on the biosynthetic process. In particular, it may be possible to increase the yield, production, and/or efficiency of production of said compounds, e.g. fatty acids, carotenoids, isoprenoids, vitamins, was esters, lipids, (poly)saccharides, and/or polyhydroxyalkanoates, and/or its metabolism products, in particular, steroid hormones, cholesterol, prostaglandin, triacylglycerols, bile acids and/or ketone bodies molecules etc. in plants.

**[0392.0.0.0]** Furthermore preferred is a method for the recombinant production of pharmaceuticals or fine chemicals in host organisms, wherein a host organism is transformed with one of the above-described nucleic acid constructs comprising one or more structural genes which encode the desired fine chemical or catalyze the biosynthesis of the desired fine chemical, the transformed host organism is cultured, and the desired fine chemical is isolated from the culture medium. This method can be applied widely to fine chemicals such as enzymes, vitamins, amino acids, sugars, fatty acids, and natural and synthetic flavourings, aroma substances and colorants or compositions comprising these. Especially preferred is the additional production of further amino acids, tocopherols and tocotrienols and carotenoids or compositions comprising said compounds. The transformed host organisms are cultured and the products are recovered from the host organisms or the culture medium by methods known to the skilled worker or the organism itself servers as food or feed supplement. The production of pharmaceuticals such as, for example, antibodies or vaccines, is described by Hood EE, Jilka JM. Curr Opin Biotechnol. 1999 Aug; 10(4):382-6; Ma JK, Vine ND. Curr Top Microbiol Immunol. 1999; 236:275-92.

**[0393.0.0.0]** In one embodiment, the present invention relates to a method for the identification of a gene product conferring an increase in the respective fine chemical production in a cell, comprising the following steps:
(a) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to a nucleic acid molecule sequence as indicated in Table I, application no. 0, columns 5 or 7, and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the respective fine chemical level in the host cells; and
(f) identifying the nucleic acid molecule and its gene product which expression confers an increase in the the respective fine chemical level in the host cell after expression compared to the wild type.

**[0394.0.0.0]** Relaxed hybridisation conditions are: After standard hybridisation procedures washing steps can be performed at low to medium stringency conditions usually with washing conditions of 40°-55°C and salt conditions between 2xSSC and 0,2x SSC with 0,1% SDS in comparison to stringent washing conditions as e.g. 60°-68°C with 0,1% SDS. Further examples can be found in the references listed above for the stringent hybridization conditions. Usually washing steps are repeated with increasing stringency and length until a useful signal to noise ratio is detected and depend on many factors as the target, e.g. its purity, GC-content, size etc, the probe, e.g. its length, is it a RNA or a DNA probe, salt conditions, washing or hybridisation temperature, washing or hybridisation time etc.

**[0395.0.0.0]** In an other embodiment, the present invention relates to a method for the identification of a gene product conferring an increase in the respective fine chemical production in a cell, comprising the following steps:
(a) identifying nucleic acid molecules of an organism; which can contain a candidate gene encoding a gene product conferring an increase in the respective fine chemical after expression, which are at least 20%, preferably 25%, more preferably 30%, even more preferred are 35%. 40% or 50%, even more preferred are 60%, 70% or 80%, most preferred are 90% or 95% or more homology to the nucleic acid molecule of the present invention, for example via homology search in a data bank;
(b) introducing the candidate nucleic acid molecules in host cells, preferably in a plant cells or microorganisms, appropriate for producing the respective fine chemical;
(c) expressing the identified nucleic acid molecules in the host cells;
(d) assaying the the respective fine chemical level in the host cells; and
(e) identifying the nucleic acid molecule and its gene product which expression confers an increase in the the respective fine chemical level in the host cell after expression compared to the wild type.
   Eventually gene products conferring the increase in the respective fine chemical production can also be identify according to a identical or similar 3D structure in step (a) and by the above described method.

**[0396.0.0.0]** The nucleic acid molecules identified can then be used for the production of the respective fine chemical in the same way as the nucleic acid molecule of the present invention. Accordingly, in one embodiment, the present invention relates to a process for the production of the respective fine chemical, comprising (a) identifying a nucleic acid molecule according to aforementioned steps (a) to (f) or (a) to (e) and recovering the free or bound fine chemical from a organism having an increased cellular activity of a polypeptide encoded by the isolated nucleic acid molecule compared to a wild type.

**[0397.0.0.0]** Furthermore, in one embodiment, the present invention relates to a method for the identification of a compound stimulating production of the respective fine chemical to said plant comprising:
a) contacting cells which express the polypeptide of the present invention or its mRNA with a candidate compound under cell cultivation conditions;
b) assaying an increase in expression of said polypeptide or said mRNA;
c) comparing the expression level to a standard response made in the absence of said candidate compound; whereby, an increased expression over the standard indicates that the compound is stimulating production of the respective fine chemical.

**[0398.0.0.0]** Furthermore, in one embodiment, the present invention relates to a method for the screening for agonists or an antagonist of the activity of the polypeptide of the present invention or used in the process of the present invention, e.g. a polypeptide conferring an increase of the respective fine chemical in an organism or a part thereof after increasing the activity in an organism or a part thereof, comprising:
(a) contacting cells, tissues , plants or microorganisms which express the polypeptide according to the invention with a candidate compound or a sample comprising a plurality of compounds under conditions which permit the expression the polypeptide of the present invention or used in the process of the present invention;
(b) assaying the respective fine chemical level or the polypeptide expression level in the cell, tissue, plant or microorganism or the media the cell, tissue, plant or microorganisms is cultured or maintained in; and
(c) identifying a agonist or antagonist by comparing the measured the respective fine chemical level or polypeptide of the invention or used in the invention expression level with a standard the respective fine chemical or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.

**[0399.0.0.0]** Furthermore, in one embodiment, the present invention relates to process for the identification of a compound conferring increased the respective fine chemical production in a plant or microorganism, comprising the steps:
(a) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(b) identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in fine chemical production.

**[0399.1.0.0]** One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, or a homolog thereof, e.g. comparing the phenotype of nearly identical organisms with low and high activity of a protein as indicated in Table II, columns 5 or 7, after incubation with the drug.

**[0400.0.0.0]** Said compound may be chemically synthesized or microbiologically produced and/or comprised in, for example, samples, e.g., cell extracts from, e.g., plants, animals or microorganisms, e.g. pathogens. Furthermore, said compound(s) may be known in the art but hitherto not known to be capable of suppressing or activating the polypeptide of the present invention. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994), in particular Chapter 17. The compounds may be, e.g., added to the reaction mixture, culture medium, injected into the cell or sprayed onto the plant.

**[0401.0.0.0]** If a sample containing a compound is identified in the method of the invention, then it is either possible to isolate the compound from the original sample identified as containing the compound capable of activating or increasing the content of the respective fine chemical in an organism or part thereof, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. Preferably, the compound identified according to the above described method or its derivative is further formulated in a form suitable for the application in plant breeding or plant cell and tissue culture.

**[0402.0.0.0]** The compounds which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Said compounds can also be functional derivatives or analogues of known inhibitors or activators. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art. Furthermore, peptidomimetics and/or computer aided design of appropriate derivatives and analogues can be used, for example, according to the methods described above. The cell or tissue that may be employed in the method of the invention preferably is a host cell, plant cell or plant tissue of the invention described in the embodiments hereinbefore.

**[0403.0.0.0]** Thus, in a further embodiment the invention relates to a compound obtained or identified according to the method for identifying an agonist of the invention said compound being an agonist of the polypeptide of the present invention or used in the process of the present invention.

**[0404.0.0.0]** Accordingly, in one embodiment, the present invention further relates to a compound identified by the method for identifying a compound of the present invention.

**[0405.0.0.0]** Said compound is, for example, a homologous of the polypeptide of the present invention. Homologues of the polypeptid of the present invention can be generated by mutagenesis, e.g., discrete point mutation or truncation of the polypeptide of the present invention. As used herein, the term "homologue" refers to a variant form of the protein, which acts as an agonist of the activity of the polypeptide of the present invention. An agonist of said protein can retain substantially the same, or a subset, of the biological activities of the polypeptide of the present invention. In particular, said agonist confers the increase of the expression level of the polypeptide of the present invention and/or the expression of said agonist in an organisms or part thereof confers the increase of free and/or bound the respective fine chemical in the organism or part thereof.

**[0406.0.0.0]** In one embodiment, the invention relates to an antibody specifically recognizing the compound or agonist of the present invention.

**[0407.0.0.0]** The invention also relates to a diagnostic composition comprising at least one of the aforementioned nucleic acid molecules, vectors, proteins, antibodies or compounds of the invention and optionally suitable means for detection.

**[0408.0.0.0]** The diagnostic composition of the present invention is suitable for the isolation of mRNA from a cell and contacting the mRNA so obtained with a probe comprising a nucleic acid probe as described above under hybridizing conditions, detecting the presence of mRNA hybridized to the probe, and thereby detecting the expression of the protein in the cell. Further methods of detecting the presence of a protein according to the present invention comprise immunotechniques well known in the art, for example enzyme linked immunosorbent assay. Furthermore, it is possible to use the nucleic acid molecules according to the invention as molecular markers or primer in plant breeding. Suitable means for detection are well known to a person skilled in the arm, e.g. buffers and solutions for hydridization assays, e.g. the aforementioned solutions and buffers, further and means for Southern-, Western-, Northern- etc. -blots, as e.g. described in Sambrook et al. are known.

**[0409.0.0.0]** In another embodiment, the present invention relates to a kit comprising the nucleic acid molecule, the vector, the host cell, the polypeptide, the antisense nucleic acid, the antibody, plant cell, the plant or plant tissue, the harvestable part, the propagation material and/or the compound or agonist or antagonists identified according to the method of the invention.

**[0410.0.0.0]** The compounds of the kit of the present invention may be packaged in containers such as vials, optionally with/in buffers and/or solution. If appropriate, one or more of said components might be packaged in one and the same container. Additionally or alternatively, one or more of said components might be adsorbed to a solid support as, e.g. a nitrocellulose filter, a glass plate, a chip, or a nylon membrane or to the well of a micro titerplate. The kit can be used for any of the herein described methods and embodiments, e.g. for the production of the host cells, transgenic plants, pharmaceutical compositions, detection of homologous sequences, identification of antagonists or agonists, as food or feed or as a supplement thereof, as supplement for the treating of plants, etc.

**[0411.0.0.0]** Further, the kit can comprise instructions for the use of the kit for any of said embodiments, in particular for the use for producing organisms or part thereof having an increased free or bound the respective fine chemical content.

**[0412.0.0.0]** In one embodiment said kit comprises further a nucleic acid molecule encoding one or more of the aforementioned protein, and/or an antibody, a vector, a host cell, an antisense nucleic acid, a plant cell or plant tissue or a plant.

**[0413.0.0.0]** In a further embodiment, the present invention relates to a method for the production of a agricultural composition providing the nucleic acid molecule, the vector or the polypeptide of the invention or the polypeptide used in the method of the invention or comprising the steps of the method according to the invention for the identification of said compound, agonist or antagonist; and formulating the nucleic acid molecule, the vector or the polypeptide of the invention or the polypeptide used in the method of the invention or the agonist, or compound identified according to the methods or processes of the present invention or with use of the subject matters of the present invention in a form applicable as plant agricultural composition.

**[0414.0.0.0]** In another embodiment, the present invention relates to a method for the production of a "the respective fine chemical"-production supporting plant culture composition comprising the steps of the method for of the present invention; and formulating the compound identified in a form acceptable as agricultural composition.

**[0415.0.0.0]** Under "acceptable as agricultural composition" is understood, that such a composition is in agreement with the laws regulating the content of fungicides, plant nutrients, herbicides, etc. Preferably such a composition is without any harm for the protected plants and the animals (humans included) fed therewith.

**[0416.0.0.0]** The present invention also pertains to several embodiments relating to further uses and methods. The nucleic acid molecule, polypeptide, protein homologues, fusion proteins, primers, vectors, host cells, described herein can be used in one or more of the following methods: identification of plants useful for the respective fine chemical production as mentioned and related organisms; mapping of genomes; identification and localization of sequences of interest; evolutionary studies; determination of regions required for function; modulation of an activity.

**[0417.0.0.0]** The nucleic acid molecule of the invention or the nucleic acid molecule used in the method of the invention, the vector of the invention or the nucleic acid construct of the invention may also be useful for the production of organisms resistant to inhibitors of the amino acid production biosynthesis pathways. In particular, the overexpression of the polypeptide of the present invention may protect plants against herbicides, which block the amino acid, in particular the respective fine chemical, synthesis in said plant. Inhibitors may inhibit one or more of the steps for the synthesis of methionine. The first committed step for the synthesis of Lys, Met and Thr is the first step, in which aspartate is phosphorylated to aspartyl-b-phosphate, catalyzed by aspartokinase: E.coli has 3 isozymes of aspartokinase that respond differently to each of the 3 amino acids, with regard to enzyme inhibition and feedback inhibition. The biosynthesis of lysine, methionine and threonine are not, then, controlled as a group. The pathway from aspartate to lysine has 10 steps. The pathway from aspartate to threonine has 5 steps. The pathway from aspartate to methionine has 7 steps. Regulation of the three pathways also occurs at the two branch points:
- b-Aspartate-semialdehyde (homoserine and lysine)
- Homoserine (threonine and methionine)
The regulation results from feedback inhibition by the amino acid products of the branches, indicated in the brackets above. One important step in the synthesis of this group of 3 amino acids is the step in which homocysteine is converted to methionine, catalyzed by the enzyme methionine synthase: 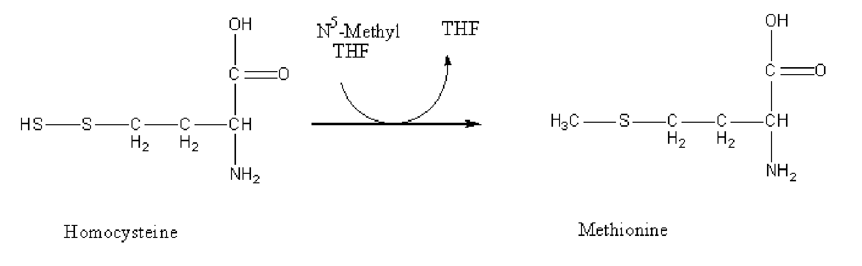 In this reaction, homocysteine is methylated to methionine, and the C1 donor is N5-methyl-THF. Thus, inhibition of one or more of the methionine synthesis enzymes, including also the provision of donor molecules, can inhibit the synthesis of methionine.
Examples of herbicides blocking the amino acid synthesis in plants are for example sulfonylurea and imidazolinone herbicides, which catalyze the first step in branched-chain amino acid biosynthesis. Inhibitors of the methionine synthesis may for example described in Danishpajooh IO, 2001 Nitric oxide inhibits methionine synthase activity in vivo and disrupts carbon flow through the folate pathway. J. Biol. Chem. 276: 27296-27303; Datko AH, 1982 Methionine biosynthesis in Lemna - inhibitor studies. Plant Physiol. 69: 1070-1076; Lavrador K, 1998 A new series of cyclic amino acids as inhibitors of S-adenosyl L-methionine synthetase. Bioorg. Med. Chem. Lett. 8: 1629-1634; Thompson GA, 1982 Methionine synthesis in Lemna - inhibition of cystathionine gamma-synthase by propargylglycine. Plant Physiol. 70: 1347-1352. In some organisms the methionine synthesis is inhibited by ethanol, lead, mercury, aluminium, thimerosal, cupper, N2O, as e.g. discussed in M. Waly, H. Oleteanu et al., 2004, Molecular Psychiatry, 1-13.
Interestingly, Arabidopsis seed germination was strongly delayed in the presence of DL-propargylglycine, a specific inhibitor of methionine synthesis. Furthermore, this compound totally inhibited seedling growth. These phenotypic effects were largely alleviated upon methionine supplementation in the germination medium. The results indicated that methionine synthase and S-adenosylmethionine synthetase are fundamental components controlling metabolism in the transition from a quiescent to a highly active state during seed germination. Moreover, the observed temporal patterns of accumulation of these proteins are consistent with an essential role of endogenous ethylene in Arabidopsis only after radicle protrusion; s. Gallarado, K., 2002, Importance of methionine biosynthesis for Arabidopsis seed germination and seedling growth, Physiolgia Plantarum, 116(2), pp 238-247. Accordingly, the overexpression of a polypeptide of the present invention in a plant may protect the plant against a herbicide inhibiting methionine synthesis. **[0418.0.0.0]** Accordingly, the nucleic acid molecules of the present invention have a variety of uses. First, they may be used to identify an organism or a close relative thereof. Also, they may be used to identify the presence thereof or a relative thereof in a mixed population of microorganisms or plants. By probing the extracted genomic DNA of a culture of a unique or mixed population of plants under stringent conditions with a probe spanning a region of the gene of the present invention which is unique to this, one can ascertain whether the present invention has been used or whether it or a close relative is present.

**[0419.0.0.0]** Further, the nucleic acid molecule of the invention or the nucleic acid molecule used in the method of the invention may be sufficiently homologous to the sequences of related species such that these nucleic acid molecules may serve as markers for the construction of a genomic map in related organism.

**[0420.0.0.0]** Accordingly, the present invention relates to a method for breeding plants for the production of the respective fine chemical, comprising
(a) providing a first plant variety produced according to the process of the invention preferably (over)expressing the nucleic acid molecule of the invention or the nucleic acid molecule used in the method of the invention;
(b) crossing the first plant variety with a second plant variety; and
(c) selecting the offspring plants which overproduce the respective fine chemical by means of analysis the distribution of a molecular marker in the offspring representing the first plant variety and its capability to (over)produce the respective fine chemical.
Details about the use of molecular markers in breeding can be found in Kumar et al., 1999 (Biotech Adv., 17:143-182) and Peleman and van der Voort 2003 (Trends Plant Sci. 2003 Jul;8(7):330-334)
The molecular marker can e.g. relate to the nucleic acid molecule of the invention or the nucleic acid molecule used in the method of the invention and/or its expression level. Accordingly, the molecular marker can be a probe or a PCR primer set useful for identification of the genomic existence or genomic localisation of the nucleic acid molecule of the invention or the nucleic acid molecule used in the method of the invention, e.g. in a Southern blot analysis or a PCR or its expression level, i.g. in a Northern Blot analysis or a quantitative PCR.
Accordingly, in one embodiment, the present invention relates to the use of the nucleic acid molecule of the present invention or encoding the polypeptide of the present invention as molecular marker for breeding, especially for breeding for a high or low respective fine chemical production.

**[0421.0.0.0]** The nucleic acid molecules of the invention are also useful for evolutionary and protein structural studies. By comparing the sequences of the invention or used in the process of the invention to those encoding similar enzymes from other organisms, the evolutionary relatedness of the organisms can be assessed. Similarly, such a comparison permits an assessment of which regions of the sequence are conserved and which are not, which may aid in determining those regions of the protein which are essential for the functioning of the enzyme. This type of determination is of value for protein engineering studies and may give an indication of what the protein can tolerate in terms of mutagenesis without losing function.

**[0422.0.0.0]** Accordingly, the nucleic acid molecule of the invention or the nucleic acid molecule used in the method of the invention can be used for the identification of other nucleic acids conferring an increase of the respective fine chemical after expression.

**[0423.0.0.0]** Further, the nucleic acid molecule of the invention or the nucleic acid molecule used in the method of the invention or a fragment of a gene conferring the expression of the polypeptide of the invention or the polypeptide used in the method of the invention, preferably comprising the nucleic acid molecule of the invention, can be used for marker assisted breeding or association mapping of the respective fine chemical derived traits

**[0424.0.0.0]** Accordingly, the nucleic acid of the invention, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the fine chemical and one or more other amino acids, in particular Threoinine, Alanine, Glutamin, Glutamic acid, Valine, Asparagine, Phenylalanine, Leucine, Proline , Tryptophan Tyrosine, Valine, Isoleucine and Arginine. Accordingly, the nucleic acid of the invention, or the nucleic acid molecule identified with the method of the present invention or the complement sequences thereof, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.0]** Further, the nucleic acid of the invention, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the antagonist or the agonist identified with the method of the invention, the antibody of the present invention, the antisense molecule of the present invention or the nucleic acid molecule identified with the method of the present invention, can be used for the preparation of an agricultural composition.

**[0426.0.0.0]** Furthermore, the nucleic acid of the invention, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, antagonist or the agonist identified with the method of the invention, the antibody of the present invention, the antisense molecule of the present invention or the nucleic acid molecule identified with the method of the present invention, can be used for the identification and production of compounds capable of conferring a modulation of the respective fine chemical levels in an organism or parts thereof, preferably to identify and produce compounds conferring an increase of the respective fine chemical levels in an organism or parts thereof, if said identified compound is applied to the organism or part thereof, i.e. as part of its food, or in the growing or culture media.

**[0427.0.0.0]** These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under hftp://www.ncbi.nlm.nih.gov/PubMed/medline.html. Further databases and addresses, such as hftp://www.ncbi.nlm.nih.gov/, hftp://www.infobiogen.fr/, hftp://www.fmi.ch/biology/research-tools.html, 1, hftp://www.tigr.org/, are known to the person skilled in the art and can also be obtained using, e.g., hftp://www.lycos.com. An overview of patent information in biotechnology and a survey of relevant sources of patent information useful for retrospective searching and for current awareness is given in Berks, TIBTECH 12 (1994), 352-364.

**[0428.0.0.0]** %

**[0429.0.0.0]** The present invention is illustrated by the examples, which follow. The present examples illustrate the basic invention without being intended as limiting the subject of the invention. The content of all of the references, patent applications, patents and published patent applications cited in the present patent application is herewith incorporated by reference.

**[0430.0.0.0]** Examples

**[0431.0.0.0]** Example 1: Cloning into in Escherichia coli

**[0432.0.0.0]** A DNA polynucleotide with a sequence as indicated in Table I, application no. 0, column 5 and encoding a polypeptide as listed in Table 1 below, was cloned into the plasmids pBR322 (Sutcliffe, J.G. (1979) Proc. Natl Acad. Sci. USA, 75: 3737-3741); pACYC177 (Change & Cohen (1978) J. Bacteriol. 134: 1141-1156); plasmids of the pBS series (pBSSK+, pBSSK- and others; Stratagene, LaJolla, USA) or cosmids such as SuperCos1 (Stratagene, LaJolla, USA) or Lorist6 (Gibson, T.J. Rosenthal, A., and Waterson, R.H. (1987) Gene 53: 283-286) for expression in E. coli using known, well-established procedures (see, for example, Sambrook, J. et al. (1989) "Molecular Cloning: A Laboratory Manual". Cold Spring Harbor Laboratory Press or Ausubel, F.M. et al. (1994) "Current Protocols in Molecular Biology", John Wiley & Sons).

**[0433.0.0.0]** Example 2: DNA sequencing and computerized functional analysis

**[0434.0.0.0]** The DNA was sequenced by standard procedures, in particular the chain determination method, using ABI377 sequencers (see, for example, Fleischman, R.D. et al. (1995) "Whole-genome Random Sequencing and Assembly of Haemophilus Influenzae Rd., Science 269; 496-512)".

**[0435.0.0.0]** Example 3: In-vivo and in-vitro mutagenesis

**[0436.0.0.0]** An in vivo mutagenesis of Corynebacterium glutamicum for the production of the respective fine chemical can be carried out by passing a plasmid DNA (or another vector DNA) through E. coli and other microorganisms (for example Bacillus spp. or yeasts such as Saccharomyces cerevisiae), which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.

**[0436.1.0.0]** In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widly used as chemical agents for random in-vitro mutagenesis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.0.0]** Example 4: DNA transfer between Escherichia coli and Corynebacterium glutamicum

**[0438.0.0.0]** Several Corynebacterium and Brevibacterium species comprise endogenous plasmids (such as, for example, pHM1519 or pBL1) which replicate autonomously (for a review, see, for example, Martin, J.F. et al. (1987) Biotechnology 5: 137-146). Shuttle vectors for Escherichia coli and Corynebacterium glutamicum can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons), which have a replication origin for, and suitable marker from, Corynebacterium glutamicum added. Such replication origins are preferably taken from endogenous plasmids, which have been isolated from Corynebacterium and Brevibacterium species. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology, VCH, Weinheim). There are many examples in the literature of the preparation of a large multiplicity of shuttle vectors which are replicated in E. coli and C. glutamicum and which can be used for various purposes including the overexpression of genes (see, for example, Yoshihama, M. et al. (1985) J. Bacteriol. 162: 591-597, Martin, J.F. et al., (1987) Biotechnology, 5: 137-146 and Eikmanns, B.J. et al. (1992) Gene 102: 93-98). Suitable vectors, which replicate in coryneform bacteria are, for example, pZ1 (Menkel et al., Appl. Environ. Microbiol., 64, 1989: 549 - 554) pEkEx1 (Eikmanns et al., Gene 102, 1991: 93 - 98) or pHS2-1 (Sonnen et al, Gene 107, 1991: 69 - 74). These vectors are based on the cryptic plasmids pHM1519, pBL1 or pGA1. Other plasmid vectors such as, for example, those based on pCG4 (US 4,489,160), pNG2 (Serwold-Davis et al., FEMS Microbiol. Lett., 66, 1990: 119 - 124) or pAG1 (US 5,158,891) can be used in the same manner.

**[0439.0.0.0]** Using standard methods, it is possible to clone a gene of interest into one of the above-described shuttle vectors and to introduce such hybrid vectors into Corynebacterium glutamicum strains. The transformation of C. glutamicum can be achieved by protoplast transformation (Kastsumata, R. et al., (1984) J. Bacteriol. 159, 306-311), electroporation (Liebl, E. et al., (1989) FEMS Microbiol. Letters, 53: 399-303) and in those cases where specific vectors are used also by conjugation (such as, for example, described in Schäfer, A., et al. (1990) J. Bacteriol. 172: 1663-1666). Likewise, it is possible to transfer the shuttle vectors for C. glutamicum to E. coli by preparing plasmid DNA from C. glutamicum (using standard methods known in the art) and transforming it into E. coli. This transformation step can be carried out using standard methods, but preferably using an Mcr-deficient E. coli strain, such as NM522 (Gough & Murray (1983) J. Mol. Biol. 166: 1-19).

**[0440.0.0.0]** If the transformed sequence(s) is/are to be integrated advantageously into the genome of the coryneform bacteria, standard techniques known to the skilled worker also exist for this purpose. Examples, which are used for this purpose are plasmid vectors as they have been described by Remscheid et al. (Appl. Environ. Microbiol., 60, 1994: 126-132) for the duplication and amplification of the hom-thrB operon. In this method, the complete gene is cloned into a plasmid vector which is capable of replication in a host such as E. coli, but not in C. glutamicum. Suitable vectors are, for example, pSUP301 (Simon et al., Bio/Technology 1, 1983: 784-791), pKIBmob or pK19mob (Schäfer et al., Gene 145, 1994: 69-73), pGEM-T (Promega Corp., Madison, WI, USA), pCR2.1-TOPO (Schuman, J. Biol. Chem., 269, 1994: 32678-32684, US 5,487,993), pCR^{®}Blunt (Invitrogen, Groningen, the Netherlands) or pEM1 (Schrumpf et al., J. Bacteriol., 173, 1991: 4510-4516).

**[0441.0.0.0]** Example 5: Determining the expression of the mutant/transgenic protein

**[0442.0.0.0]** The observations of the activity of a mutated, or transgenic, protein in a transformed host cell are based on the fact that the protein is expressed in a similar manner and in a similar quantity as the wild-type protein. A suitable method for determining the transcription quantity of the mutant, or transgenic, gene (a sign for the amount of mRNA which is available for the translation of the gene product) is to carry out a Northern blot (see, for example, Ausubel et al., (1988) Current Protocols in Molecular Biology, Wiley: New York), where a primer which is designed in such a way that it binds to the gene of interest is provided with a detectable marker (usually a radioactive or chemiluminescent marker) so that, when the total RNA of a culture of the organism is extracted, separated on a gel, applied to a stable matrix and incubated with this probe, the binding and quantity of the binding of the probe indicates the presence and also the amount of mRNA for this gene. Another method is a quantitative PCR. This information detects the extent to which the gene has been transcribed. Total cell RNA can be isolated from Corynebacterium glutamicum or other microorganisms by a variety of methods, which are known in the art, e.g. as described in Bormann, E.R. et al., (1992) Mol. Microbiol. 6: 317-326.

**[0443.0.0.0]** Standard techniques, such as Western blot, may be employed to determine the presence or relative amount of protein translated from this mRNA (see, for example, Ausubel et al. (1988) "Current Protocols in Molecular Biology", Wiley, New York) . In this method, total cell proteins are extracted, separated by gel electrophoresis, transferred to a matrix such as nitrocellulose and incubated with a probe, such as an antibody, which binds specifically to the desired protein. This probe is usually provided directly or indirectly with a chemiluminescent or colorimetric marker, which can be detected readily. The presence and the observed amount of marker indicates the presence and the amount of the sought mutant protein in the cell. However, other methods are also known.

**[0444.0.0.0]** Example 6: Growth of genetically modified Corynebacterium glutamicum: media and culture conditions

**[0445.0.0.0]** Genetically modified Corynebacteria are grown in synthetic or natural growth media. A number of different growth media for Corynebacteria are known and widely available (Lieb et al. (1989) Appl. Microbiol. Biotechnol. 32: 205-210; von der Osten et al. (1998) Biotechnology Letters 11: 11-16; Patent DE 4 120 867; Liebl (1992) "The Genus Corynebacterium", in: The Procaryotes, Vol. II, Balows, A., et al., Ed. Springer-Verlag).

**[0446.0.0.0]** Said media, which can be used according to the invention usually consist of one or more carbon sources, nitrogen sources, inorganic salts, vitamins and trace elements. Preferred carbon sources are sugars such as mono-, di- or polysaccharides. Examples of very good carbon sources are glucose, fructose, mannose, galactose, ribose, sorbose, ribulose, lactose, maltose, sucrose, raffinose, starch or cellulose. Sugars may also be added to the media via complex compounds such as molasses or other by-products of sugar refining. It may also be advantageous to add mixtures of various carbon sources. Other possible carbon sources are alcohols and/or organic acids such as methanol, ethanol, acetic acid or lactic acid. Nitrogen sources are usually organic or inorganic nitrogen compounds or materials containing said compounds. Examples of nitrogen sources include ammonia gas, aqueous ammonia solutions or ammonium salts such as NH₄Cl, or (NH₄)₂SO₄, NH₄0H, nitrates, urea, amino acids or complex nitrogen sources such as cornsteep liquor, soybean flour, soybean protein, yeast extract, meat extract and others. Mixtures of the above nitrogen sources may be used advantageously.

**[0447.0.0.0]** Inorganic salt compounds, which may be included in the media comprise the chloride, phosphorus or sulfate salts of calcium, magnesium, sodium, cobalt, molybdenum, potassium, manganese, zinc, copper and iron. Chelating agents may be added to the medium in order to keep the metal ions in solution. Particularly suitable chelating agents include dihydroxyphenols such as catechol or protocatechulate or organic acids such as citric acid. The media usually also contain other growth factors such as vitamins or growth promoters, which include, for example, biotin, riboflavin, thiamine, folic acid, nicotinic acid, panthothenate and pyridoxine. Growth factors and salts are frequently derived from complex media components such as yeast extract, molasses, cornsteep liquor and the like. The exact composition of the compounds used in the media depends heavily on the particular experiment and is decided upon individually for each specific case. Information on the optimization of media can be found in the textbook "Applied Microbiol. Physiology, A Practical Approach" (Ed. P.M. Rhodes, P.F. Stanbury, IRL Press (1997) S. 53-73, ISBN 0 19 963577 3). Growth media can also be obtained from commercial suppliers, for example Standard 1 (Merck) or BHI (Brain heart infusion, DIFCO) and the like.

**[0448.0.0.0]** All media components are sterilized, either by heat (20 min at 1.5 bar und 121°C) or by filter sterilization. The components may be sterilized either together or, if required, separately. All media components may be present at the start of the cultivation or added continuously or batchwise, as desired.

**[0449.0.0.0]** The culture conditions are defined separately for each experiment. The temperature is normally between 15°C and 45°C and may be kept constant or may be altered during the experiment. The pH of the medium should be in the range from 5 to 8.5, preferably around 7.0, and can be maintained by adding buffers to the media. An example of a buffer for this purpose is a potassium phosphate buffer. Synthetic buffers such as MOPS, HEPES, ACES and the like may be used as an alternative or simultaneously. The culture pH value may also be kept constant during the culture period by addition of, for example, NaOH or NH₄OH. If complex media components such as yeast extract are used, additional buffers are required less since many complex compounds have a high buffer capacity. When using a fermenter for the culture of microorganisms, the pH value can also be regulated using gaseous ammonia.

**[0450.0.0.0]** The incubation period is generally in a range of from several hours to several days. This time period is selected in such a way that the maximum amount of product accumulates in the fermentation broth. The growth experiments, which are disclosed can be carried out in a multiplicity of containers such as microtiter plates, glass tubes, glass flasks or glass or metal fermenters of various sizes. To screen a large number of clones, the microorganisms should be grown in microtiter plates, glass tubes or shake flasks, either using simple flasks or baffle flasks. 100 ml shake flasks filled with 10% (based on the volume) of the growth medium required are preferably used. The flasks should be shaken on an orbital shaker (amplitude 25 mm) at a rate ranging from 100 to 300 rpm. Evaporation losses can be reduced by maintaining a humid atmosphere; as an alternative, a mathematical correction should be carried out for the evaporation losses.

**[0451.0.0.0]** If genetically modified clones are examined, an unmodified control clone, or a control clone, which contains the basic plasmid without insertion, should also be included in the tests. If a transgenic sequence is expressed, a control clone should advantageously again be included in these tests. The medium is advantageously inoculated to an OD600 of 0.5 to 1.5 using cells which have been grown on agar plates, such as CM plates (10 g/I glucose, 2.5 g/I NaCl, 2 g/I urea, 10 g/I polypeptone, 5 g/I yeast extract, 5 g/I meat extract, 22 g/I agar, pH value 6.8 established with 2M NaOH), which have been incubated at 30°C. The media are inoculated for example by introducing of a preculture of seed organisms..

**[0451.1.0.0]** For example, the media are inoculated by introducing of a saline solution of C. glutamicum cells from CM plates or by addition of a liquid preculture of this bacterium.

**[0452.0.0.0]** Example 7: In-vitro analysis of the function of the proteins encoded by the transformed sequences

**[0453.0.0.0]** The determination of the activities and kinetic parameters of enzymes is well known in the art. Experiments for determining the activity of a specific modified enzyme must be adapted to the specific activity of the wild-enzyme type, which is well within the capabilities of the skilled worker. Overviews of enzymes in general and specific details regarding the structure, kinetics, principles, methods, applications and examples for the determination of many enzyme activities can be found for example in the following literature: Dixon, M., and Webb, E.C: (1979) Enzymes, Longmans, London; Fersht (1985) Enzyme Structure and Mechanism, Freeman, New York; Walsh (1979) Enzymatic Reaction Mechanisms. Freeman, San Francisco; Price, N.C., Stevens, L. (1982) Fundamentals of Enzymology. Oxford Univ. Press: Oxford; Boyer, P.D: Ed. (1983) The Enzymes, 3rd Ed. Academic Press, New York; Bisswanger, H. (1994) Enzymkinetik, 2nd Ed. VCH, Weinheim (ISBN 3527300325); Bergmeyer, H.U., Bergmeyer, J., Graßl, M. Ed. (1983-1986) Methods of Enzymatic Analysis, 3rd Ed. Vol. I-XII, Verlag Chemie: Weinheim; and Ullmann's Encyclopedia of Industrial Chemistry (1987) Vol. A9, "Enzymes", VCH, Weinheim, pp. 352-363.

**[0454.0.0.0]** Example 8: Analysis of the effect of the nucleic acid molecule on the production of the amino acids

**[0455.0.0.0]** The effect of the genetic modification in C. glutamicum on the production of an amino acid can be determined by growing the modified microorganisms under suitable conditions (such as those described above) and analyzing the medium and/or the cellular components for the increased production of the amino acid. Such analytical techniques are well known to the skilled worker and encompass spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, pp. 89-90 and pp. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", pp. 469-714, VCH: Weinheim; Belter, P.A. et al. (1988) Bioseparations: downstream processing for Biotechnology, John Wiley and Sons; Kennedy, J.F. and Cabral, J.M.S. (1992) Recovery processes for biological Materials, John Wiley and Sons; Shaeiwitz, J.A. and Henry, J.D. (1988) Biochemical Separations, in Ullmann's Encyclopedia of Industrial Chemistry, Vol. B3; chapter 11, pp. 1-27, VCH: Weinheim; and Dechow, F.J. (1989) Separation and purification techniques in biotechnology, Noyes Publications).

**[0456.0.0.0]** In addition to the determination of the fermentation end product, other components of the metabolic pathways which are used for the production of the desired compound, such as intermediates and by-products, may also be analyzed in order to determine the total productivity of the organism, the yield and/or production efficiency of the compound. The analytical methods encompass determining the amounts of nutrients in the medium (for example sugars, hydrocarbons, nitrogen sources, phosphate and other ions), determining biomass composition and growth, analyzing the production of ordinary metabolites from biosynthetic pathways and measuring gases generated during the fermentation. Standard methods for these are described in Applied Microbial Physiology; A Practical Approach, P.M. Rhodes and P.F. Stanbury, Ed. IRL Press, pp. 103-129; 131-163 and 165-192 (ISBN: 0199635773) and the references cited therein.

**[0457.0.0.0]** Example 9: Purification of the amino acid

**[0458.0.0.0]** The amino acid can be recovered from cells or from the supernatant of the above-described culture by a variety of methods known in the art. For example, the culture supernatant is recovered first. To this end, the cells are harvested from the culture by slow centrifugation. Cells can generally be disrupted or lysed by standard techniques such as mechanical force or sonication. The cell debris is removed by centrifugation and the supernatant fraction, if appropriate together with the culture supernatant, is used for the further purification of the amino acid. However, it is also possible to process the supernatant alone if the amino acid is present in the supernatant in sufficiently high a concentration. In this case, the amino acid, or the amino acid mixture, can be purified further for example via extraction and/or salt precipitation or via ion-exchange chromatography.

**[0459.0.0.0]** If required and desired, further chromatography steps with a suitable resin may follow, the amino acid, but not many contaminants in the sample, being retained on the chromatography resin or the contaminants, but not the sample with the product (amino acid), being retained on the resin. If necessary, these chromatography steps may be repeated, using identical or other chromatography resins. The skilled worker is familiar with the selection of suitable chromatography resin and the most effective use for a particular molecule to be purified. The purified product can be concentrated by filtration or ultrafiltration and stored at a temperature at which maximum product stability is ensured. Many purification methods, which are not limited to the above purification method are known in the art. They are described, for example, in Bailey, J.E. & Ollis, D.F. Biochemical Engineering Fundamentals, McGraw-Hill: New York (1986).

**[0460.0.0.0]** Identity and purity of the amino acid isolated can be determined by standard techniques of the art. They encompass high-performance liquid chromatography (HPLC), spectroscopic methods, mass spectrometry (MS), staining methods, thin-layer chromatography, NIRS, enzyme assay or microbiological assays. These analytical methods are compiled in: Patek et al. (1994) Appl. Environ. Microbiol. 60: 133-140; Malakhova et al. (1996) Biotekhnologiya 11: 27-32; and Schmidt et al. (1998) Bioprocess Engineer. 19: 67-70. Ulmann's Encyclopedia of Industrial Chemistry (1996) Vol. A27, VCH: Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Vol. 17.

**[0461.0.0.0]** Example 10: Cloning SEQ ID NO: 25 for the expression in plants

**[0462.0.0.0]** Unless otherwise specified, standard methods as described in Sambrook et al., Molecular Cloning: A laboratory manual, Cold Spring Harbor 1989, Cold Spring Harbor Laboratory Press are used.

**[0463.0.0.0]** SEQ ID NO: 25 is amplified by PCR as described in the protocol of the Pfu Turbo or DNA Herculase polymerase (Stratagene).

**[0464.0.0.0]** The composition for the protocol of the Pfu Turbo DNA polymerase was as follows: 1x PCR buffer (Stratagene), 0.2 mM of each dNTP, 100 ng genomic DNA of Saccharomyces cerevisiae (strain S288C; Research Genetics, Inc., now Invitrogen) or Escherichia coli (strain MG1655; E. coli Genetic Stock Center), 50 pmol forward primer, 50 pmol reverse primer, 2.5 u Pfu Turbo DNA polymerase. The amplification cycles were as follows:

**[0465.0.0.0]** 1 cycle of 3 minutes at 94-95°C, followed by 25-36 cycles of in each case 1 minute at 95°C or 30 seconds at 94°C, 45 seconds at 50°C, 30 seconds at 50°C or 30 seconds at 55°C and 210-480 seconds at 72°C, followed by 1 cycle of 8 minutes at 72°C, then 4°C. The composition for the protocol of the Herculase polymerase was as follows: 1x PCR buffer (Stratagene), 0.2 mM of each dNTP, 100 ng genomic DNA of *Saccharomyces cerevisiae* (strain S288C; Research Genetics, Inc., now Invitrogen) or *Escherichia coli* (strain MG1655; *E. coli* Genetic Stock Center), 50 pmol forward primer, 50 pmol reverse primer, 2.5 u Herculase polymerase. The amplification cycles were as follows:

**[0466.0.0.0]** 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.0.0]** The following primer sequences were selected for the gene SEQ ID NO: 25:
i) forward primer SEQ ID NO: 75
ii) reverse primer SEQ ID NO: 76

**[0468.0.0.0]** Thereafter, the amplificate was purified over QIAquick columns following the standard protocol (Qiagen).

**[0469.0.0.0]** For the cloning of PCR-products, produced by Pfu Turbo DNA polymerase, the vector DNA (30 ng) was restricted with Smal following the standard protocol (MBI Fermentas) and stopped by addition of high-salt buffer. The restricted vector fragments were purified via Nucleobond columns using the standard protocol (Macherey-Nagel). Thereafter, the linearized vector was dephosphorylated following the standard protocol (MBI Fermentas).

**[0470.0.0.0]** The PCR-products, produced by Pfu Turbo DNA polymerase, were directly cloned into the processed binary vector. The PCR-products, produced by Pfu Turbo DNA polymerase, were phosphorylated using a T4 DNA polymerase using a standard protocol (e.g. MBI Fermentas) and cloned into the processed binary vector.

**[0471.0.0.0]** The DNA termini of the PCR-products, produced by Herculase DNA polymerase, were blunted in a second synthesis reaction using Pfu Turbo DNA polymerase. The composition for the protocol of the blunting the DNA-termini was as follows: 0.2 mM blunting dTTP and 1.25 u Pfu Turbo DNA polymerase. The reaction was incubated at 72°C for 30 minutes. Then the PCR-products were cloned into the processed vector as well. The DNA termini of the PCR-products, produced by Herculase DNA polymerase, were blunted in a second synthesis reaction using *Pfu* Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were phosphorylated using a T4 DNA polymerase using a standard protocol (e.g. MBI Fermentas) and cloned into the processed vector as well.

**[0472.0.0.0]** A binary vector comprising a selection cassette (promoter, selection marker, terminator) and an expression cassette with promoter, cloning cassette and terminator sequence between the T-DNA border sequences was used. In addition to those within the cloning cassette, the binary vector has no Smal cleavage site. Binary vectors which can be used are known to the skilled worker; an overview of binary vectors and their use can be found in Hellens, R., Mullineaux, P. and Klee H., [(2000) "A guide to Agrobacterium binary vectors", Trends in Plant Science, Vol. 5 No. 10, 446-451. Depending on the vector used, cloning may advantageously also be carried out via other restriction enzymes. Suitable advantageous cleavage sites can be added to the ORF by using suitable primers for the PCR amplification.

**[0473.0.0.0]** Approximately 30 ng of prepared vector and a defined amount of prepared amplificate were mixed and ligated by addition of ligase.

**[0474.0.0.0]** The ligated vectors were transformed in the same reaction vessel by addition of competent *E. coli* cells (strain DH5alpha) and incubation for 20 minutes at 1°C followed by a heat shock for 90 seconds at 42°C and cooling to 4°C. Then, complete medium (SOC) was added and the mixture was incubated for 45 minutes at 37°C. The entire mixture was subsequently plated onto an agar plate with antibiotics (selected as a function of the binary vector used) and incubated overnight at 37°C.

**[0475.0.0.0]** The outcome of the cloning step was verified by amplification with the aid of primers which bind upstream and downstream of the integration site, thus allowing the amplification of the insertion. In addition combinations of the above mentioned gene specific primers and upstream and downstream primers were used in PCR reactions to identify clones with the correct insert orientation. The amplifications were carried as described in the protocol of Taq DNA polymerase (Gibco-BRL).

**[0476.0.0.0]** The amplification cycles were as follows: 1 cycle of 5 minutes at 94°C, followed by 35 cycles of in each case 15 seconds at 94°C, 15 seconds at 50-66°C and 5 minutes at 72°C, followed by 1 cycle of 10 minutes at 72°C, then 4°C.

**[0477.0.0.0]** Several colonies were checked, but only one colony for which a PCR product of the expected size was detected was used in the following steps.

**[0478.0.0.0]** A portion of this positive colony was transferred into a reaction vessel filled with complete medium (LB) and incubated overnight at 37°C. The LB medium contained an antibiotic chosen to suit the binary vector (see above) used and the resistance gene present therein in order to select the clone.

**[0479.0.0.0]** The plasmid preparation was carried out as specified in the Qiaprep standard protocol (Qiagen).

**[0480.0.0.0]** Example 11: Generation of transgenic plants which express SEQ ID NO: 25

**[0481.0.0.0]** 1 ng of the plasmid DNA isolated was transformed by electroporation into competent cells of Agrobacterium tumefaciens, of strain GV 3101 pMP90 (Koncz and Schell, Mol. Gen. Gent. 204, 383-396, 1986). The choice of the agrobacterial strain depends on the choice of the binary vector. An overview of possible strains and their properties is found in Hellens, R., Mullineaux, P. and Klee H., (2000) " A guide to Agrobacterium binary vectors, Trends in Plant Science, Vol. 5 No.10, 446-451. Thereafter, complete medium (YEP) was added and the mixture was transferred into a fresh reaction vessel for 3 hours at 28°C. Thereafter, all of the reaction mixture was plated onto YEP agar plates supplemented with the respective antibiotics, for example rifampicin and gentamycin for GV31 01 pMP90, and a further antibiotic for the selection onto the binary vector, was plated, and incubated for 48 hours at 28°C.

**[0482.0.0.0]** The agrobacteria generated in Example 10, which contains the plasmid construct were then used for the transformation of plants.

**[0483.0.0.0]** A colony was picked from the agar plate with the aid of a pipette tip and taken up in 3 ml of liquid TB medium, which also contained suitable antibiotics, depending on the agrobacterial strain and the binary plasmid. The preculture was grown for 48 hours at 28°C and 120 rpm.

**[0484.0.0.0]** 400 ml of LB medium containing the same antibiotics as above were used for the main culture. The preculture was transferred into the main culture. It was grown for 18 hours at 28°C and 120 rpm. After centrifugation at 4 000 rpm, the pellet was resuspended in infiltration medium (MS medium, 10% sucrose).

**[0485.0.0.0]** In order to grow the plants for the transformation, dishes (Piki Saat 80, green, provided with a screen bottom, 30 x 20 x 4.5 cm, from Wiesauplast, Kunststofftechnik, Germany) were half-filled with a GS 90 substrate (standard soil, Werkverband E.V., Germany). The dishes were watered overnight with 0.05% Proplant solution (Chimac-Apriphar, Belgium). Arabidopsis thaliana C24 seeds (Nottingham Arabidopsis Stock Centre, UK ; NASC Stock N906) were scattered over the dish, approximately 1 000 seeds per dish. The dishes were covered with a hood and placed in the stratification facility (8 h, 110 µmol/m2/s-1, 22°C; 16 h, dark, 6°C). After 5 days, the dishes were placed into the short-day controlled environment chamber (8 h 130 µmol/m2/s-1, 22°C; 16 h, dark 20°C), where they remained for approximately 10 days until the first true leaves had formed.

**[0486.0.0.0]** The seedlings were transferred into pots containing the same substrate (Teku pots, 7 cm, LC series, manufactured by Pöppelmann GmbH & Co, Germany). Five plants were pricked out into each pot. The pots were then returned into the short-day controlled environment chamber for the plant to continue growing.

**[0487.0.0.0]** After 10 days, the plants were transferred into the greenhouse cabinet (supplementary illumination, 16 h, 340 E, 22°C; 8 h, dark, 20°C), where they were allowed to grow for further 17 days.

**[0488.0.0.0]** For the transformation, 6-week-old Arabidopsis plants which had just started flowering were immersed for 10 seconds into the above-described agrobacterial suspension which had previously been treated with 10 I Silwett L77 (Crompton S.A., Osi Specialties, Switzerland). The method in question is described in Clough and Bent, 1998 (Clough, JC and Bent, AF. 1998 Floral dip: a simplified method for Agrobacterium-mediated transformation of Arabidopsis thaliana, Plant J. 16:735-743.

**[0489.0.0.0]** The plants were subsequently placed for 18 hours into a humid chamber. Thereafter, the pots were returned to the greenhouse for the plants to continue growing. The plants remained in the greenhouse for another 10 weeks until the seeds were ready for harvesting.

**[0490.0.0.0]** Depending on the resistance marker used for the selection of the transformed plants the harvested seeds were planted in the greenhouse and subjected to a spray selection or else first sterilized and then grown on agar plates supplemented with the respective selection agent. In case of BASTA®-resistance, plantlets were sprayed four times at an interval of 2 to 3 days with 0.02 % BASTA® and transformed plants were allowed to set seeds. The seeds of the transgenic A. thaliana plants were stored in the freezer (at -20°C).

**[0491.0.0.0]** Example 12: Plant culture for bioanalytical analyses

**[0492.0.0.0]** For the bioanalytical analyses of the transgenic plants, the latter were grown uniformly a specific culture facility. To this end the GS-90 substrate as the compost mixture was introduced into the potting machine (Laible System GmbH, Singen, Germany) and filled into the pots. Thereafter, 35 pots were combined in one dish and treated with Previcur. For the treatment, 25 ml of Previcur were taken up in 10 I of tap water. This amount was sufficient for the treatment of approximately 200 pots. The pots were placed into the Previcur solution and additionally irrigated overhead with tap water without Previcur. They were used within four days..

**[0493.0.0.0]** For the sowing, the seeds, which had been stored in the refrigerator (at - 20°C), were removed from the Eppendorf tubes with the aid of a toothpick and transferred into the pots with the compost. In total, approximately 5 to 12 seeds were distributed in the middle of the pot.

**[0494.0.0.0]** After the seeds had been sown, the dishes with the pots were covered with matching plastic hood and placed into the stratification chamber for 4 days in the dark at 4°C. The humidity was approximately 90%. After the stratification, the test plants were grown for 22 to 23 days at a 16-h-light, 8-h-dark rhythm at 20°C, an atmospheric humidity of 60% and a CO₂ concentration of approximately 400 ppm. The light sources used were Powerstar HQI-T 250 W/D Daylight lamps from Osram, which generate a light resembling the solar color spectrum with a light intensity of approximately 220 E/m2/s-1.

**[0495.0.0.0]** When the plants were 8, 9 and 10 days old, they were subjected to selection for the resistance marker Approximately 1400 pots with transgenic plants were treated with 1l 0,015% vol/vol of Basta (Glufosinate-ammonium) solution in water (Aventis Cropsience, Germany). After a further 3 to 4 days, the transgenic, resistant seedlings (plantlets in the 4-leaf stage) could be distinguished clearly from the untransformed plantlets. The nontransgenic seedlings were bleached or dead. The transgenic resistance plants were thinned when they had reached the age of 14 days. The plants, which had grown best in the center of the pot were considered the target plants. All the remaining plants were removed carefully with the aid of metal tweezers and discarded.

**[0496.0.0.0]** During their growth, the plants received overhead irrigation with distilled water (onto the compost) and bottom irrigation into the placement grooves. Once the grown plants had reached the age of 23 days, they were harvested.

**[0497.0.0.0]** Example 13: Metabolic analysis of transformed plants

**[0498.0.0.0]** The modifications identified in accordance with the invention, in the content of above-described metabolites, were identified by the following procedure.

### a) sampling and storage of the samples

**[0499.0.0.0]** Sampling was performed directly in the controlled-environment chamber. The plants were cut using small laboratory scissors, rapidly weighed on laboratory scales, transferred into a pre-cooled extraction sleeve and placed into an aluminum rack cooled by liquid nitrogen. If required, the extraction sleeves can be stored in the freezer at -80°C. The time elapsing between cutting the plant to freezing it in liquid nitrogen amounted to not more than 10 to 20 seconds.

### b) Lyophilization

**[0500.0.0.0]** During the experiment, care was taken that the plants either remained in the deep-frozen state (temperatures < -40°C) or were freed from water by lyophilization until the first contact with solvents.

**[0501.0.0.0]** The aluminum rack with the plant samples in the extraction sleeves was placed into the pre-cooled (-40°C) lyophilization facility. The initial temperature during the main drying phase was-35°C and the pressure was 0.120 mbar. During the drying phase, the parameters were altered following a pressure and temperature program. The final temperature after 12 hours was +30°C and the final pressure was 0.001 to 0.004 mbar. After the vacuum pump and the refrigerating machine had been switched off, the system was flushed with air (dried via a drying tube) or argon.

### c) Extraction

**[0502.0.0.0]** Immediately after the lyophilization apparatus had been flushed, the extraction sleeves with the lyophilized plant material were transferred into the 5 ml extraction cartridges of the ASE device (Accelerated Solvent Extractor ASE 200 with Solvent Controller and AutoASE software (DIONEX)).

**[0503.0.0.0]** The 24 sample positions of an ASE device (Accelerated Solvent Extractor ASE 200 with Solvent Controller and AutoASE software (DIONEX)) were filled with plant samples, including some samples for testing quality control.

**[0504.0.0.0]** The polar substances were extracted with approximately 10 ml of methanol/water (80/20, v/v) at T = 70°C and p = 140 bar, 5 minutes heating-up phase, 1 minute static extraction. The more lipophilic substances were extracted with approximately 10 ml of methanol/dichloromethane (40/60, v/v) at T = 70°C and p = 140 bar, 5 minute heating-up phase, 1 minute static extraction. The two solvent mixtures were extracted into the same glass tubes (centrifuge tubes, 50 ml, equipped with screw cap and pierceable septum for the ASE (DIONEX)).

**[0505.0.0.0]** The solution was treated with internal standards: ribitol, L-glycine-2,2-d₂, L-alanine-2,3,3,3-d₄, methionine-methyl-d₃, and α-methylglucopyranoside and methyl nonadecanoate, methyl undecanoate, methyl tridecanoate, methyl pentadecanoate, methyl nonacosanoate.

**[0506.0.0.0]** The total extract was treated with 8 ml of water. The solid residue of the plant sample and the extraction sleeve were discarded.

**[0507.0.0.0]** The extract was shaken and then centrifuged for 5 to 10 minutes at least at 1 400 g in order to accelerate phase separation. 1 ml of the supernatant methanol/water phase ("polar phase", colorless) was removed for the further GC analysis, and 1 ml was removed for the LC analysis. The remainder of the methanol/water phase was discarded. 0.5 ml of the organic phase ("lipid phase", dark green) was removed for the further GC analysis and 0.5 ml was removed for the LC analysis. All the portions removed were evaporated to dryness using the IR Dancer infrared vacuum evaporator (Hettich). The maximum temperature during the evaporation process did not exceed 40°C. Pressure in the apparatus was not less than 10 mbar.

### d) Processing the lipid phase for the LC/MS or LC/MS/MS analysis

**[0508.0.0.0]** The lipid extract, which had been evaporated to dryness was taken up in mobile phase. The polar extract, which had been evaporated to dryness was taken up in mobile phase.

### e) LC-MS analyisis

**[0509.0.0.0]** The LC part was carried out on a commercially available LCMS system from Agilent Technologies, USA. For polar extracts 10 µl are injected into the system at a flow rate of 200µl/min. The separation column (Reversed Phase C18) was maintained at 15 °C during chromatography. For lipid extracts 5 µl are injected into the system at a flow rate of 200 µl/min. The separation column (Reversed Phase C18) was maintained at 30 °C. HPLC was performed with gradient elution
The mass spectrometric analysis was performed on a Applied Biosystems API 4000 triple quadrupole instrument with turbo ion spray source. For polar extracts the instrument measures in negative ion mode in fullscan mode from 100-1000 amu. For lipid extracts the instrument measures in postive ion mode in fullscan mode from 100-1000 amu.

### f) Derivatization of the lipid phase for the GC/MS analysis

**[0510.0.0.0]** For the transmethanolysis, a mixture of 140 µl of chloroform, 37 µl of hydrochloric acid (37% by weight HCl in water), 320 µl of methanol and 20 µl of toluene was added to the evaporated extract. The vessel was sealed tightly and heated for 2 hours at 100°C, with shaking. The solution was subsequently evaporated to dryness. The residue was dried completely. The methoximation of the carbonyl groups was carried out by reaction with methoxyamine hydrochloride (5 mg/ml in pyridine, 100 for 1.5 hours at 60°C) in a tightly sealed vessel. 20 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 100 µl of N-methyl-N-(trimethylsilyl)-2,2,2-trifluoroacetamide (MSTFA) was carried out for 30 minutes at 60°C, again in the tightly sealed vessel. The final volume before injection into the GC was 220 µl.

### g) Derivatization of the polar phase for GC/MS analysis

**[0511.0.0.0]** The methoximation of the carbonyl groups was carried out by reaction with methoxyamine hydrochloride (5 mg/ml in pyridine, 50 I for 1.5 hours at 60°C) in a tightly sealed vessel. 10 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 50 µl of N-methyl-N-(trimethylsilyl)-2,2,2-trifluoroacetamide (MSTFA) was carried out for 30 minutes at 60°C, again in the tightly sealed vessel. The final volume before injection into the GC was 110 µl. µl.

### h) GC-MS Analysis

**[0512.0.0.0]** The GC-MS systems consist of an Agilent 6890 GC coupled to an Agilent 5973 MSD. The autosamplers are CompiPal or GCPal from CTC. For the analysis usual commercial capillary separation columns (30 m x 0,25 mm x 0,25 µm) with different poly-methyl-siloxane stationary phases containing 0 % up to 35% of aromatic moieties, depending on the analysed sample materials and fractions from the phase separation step, are used (for example: DB-1 ms, HP-5ms, DB-XLB, DB-35ms, Agilent Technologies). Up to 1 µL of the final volume is injected splitless and the oven temperature program is started at 70 °C and ended at 340 °C with different heating rates depending on the sample material and fraction from the phase separation step in order to achieve a sufficient chromatographic separation and number of scans within each analyte peak. Usual GC-MS standard conditions, for example constant flow with nominal 1 to 1.7 ml/min. and helium as the mobile phase gas are used. lonisation is done by electron impact with 70 eV, scanning within a m/z range from 15 to 600 with scan rates from 2.5 to 3 scans/sec and standard tune conditions.

### i) Analysis of the various plant samples

**[0513.0.0.0]** The samples were measured in individual series of 20 plant samples each (also referred to as sequences), each sequence containing at least 5 wild-type plants as controls. The peak area of each analyte was divided by the peak area of the respective internal standard. The data were standardized for the fresh weight established for the plant. The values calculated thus were related to the wild-type control group by being divided by the mean of the corresponding data of the wild-type control group of the same sequence. The values obtained were referred to as ratio_by_WT, they are comparable between sequences and indicate how much the analyte concentration in the mutant differs in relation to the wild-type control. Appropiate controls were done before to proof that the vector and transformation procedure itself has no significant influence on the metabolic composition of the plants. Therefore the desribed changes in comparison with wildtypes were caused by the introduced genes.

**[0514.0.0.0]** As an alternative, the amino acids can be detected advantageously via HPLC separation in ethanolic extract as described by Geigenberger et al. (Plant Cell & Environ, 19, 1996: 43-55).

**[0515.0.0.0]** %

**[0516.0.0.0]** When the analyses were repeated independently, all results proved to be significant.

**[0517.0.0.0]** Example 14a: Engineering ryegrass plants by over-expressing the polynucleotide characterized in the invention, e.g. derived from plants or an other organism

**[0518.0.0.0]** Seeds of several different ryegrass varieties can be used as explant sources for transformation, including the commercial variety Gunne available from Svalof Weibull seed company or the variety Affinity. Seeds are surface-sterilized sequentially with 1% Tween-20 for 1 minute, 100 % bleach for 60 minutes, 3 rinses with 5 minutes each with de-ionized and distilled H2O, and then germinated for 3-4 days on moist, sterile filter paper in the dark. Seedlings are further sterilized for 1 minute with 1% Tween-20, 5 minutes with 75% bleach, and rinsed 3 times with ddH2O, 5 min each.

**[0519.0.0.0]** Surface-sterilized seeds are placed on the callus induction medium containing Murashige and Skoog basal salts and vitamins, 20 g/I sucrose, 150 mg/l asparagine, 500 mg/l casein hydrolysate, 3 g/I Phytagel, 10 mg/l BAP, and 5 mg/l dicamba. Plates are incubated in the dark at 25°C for 4 weeks for seed germination and embryogenic callus induction.

**[0520.0.0.0]** After 4 weeks on the callus induction medium, the shoots and roots of the seedlings are trimmed away, the callus is transferred to fresh media, is maintained in culture for another 4 weeks, and is then transferred to MSO medium in light for 2 weeks. Several pieces of callus (11-17 weeks old) are either strained through a 10 mesh sieve and put onto callus induction medium, or are cultured in 100 ml of liquid ryegrass callus induction media (same medium as for callus induction with agar) in a 250 ml flask. The flask is wrapped in foil and shaken at 175 rpm in the dark at 23°C for 1 week. Sieving the liquid culture with a 40-mesh sieve is collected the cells. The fraction collected on the sieve is plated and is cultured on solid ryegrass callus induction medium for 1 week in the dark at 25°C. The callus is then transferred to and is cultured on MS medium containing 1% sucrose for 2 weeks.

**[0521.0.0.0]** Transformation can be accomplished with either Agrobacterium or with particle bombardment methods. An expression vector is created containing a constitutive plant promoter and the cDNA of the gene in a pUC vector. The plasmid DNA is prepared from E. coli cells using with Qiagen kit according to manufacturer's instruction. Approximately 2 g of embryogenic callus is spread in the center of a sterile filter paper in a Petri dish. An aliquot of liquid MSO with 10 g/I sucrose is added to the filter paper. Gold particles (1.0 µm in size) are coated with plasmid DNA according to method of Sanford et al., 1993 and are delivered to the embryogenic callus with the following parameters: 500 µg particles and 2 µg DNA per shot, 1300 psi and a target distance of 8.5 cm from stopping plate to plate of callus and 1 shot per plate of callus.

**[0522.0.0.0]** After the bombardment, calli are transferred back to the fresh callus development medium and maintained in the dark at room temperature for a 1-week period. The callus is then transferred to growth conditions in the light at 25 °C to initiate embryo differentiation with the appropriate selection agent, e.g. 250 nM Arsenal, 5 mg/l PPT or 50 mg/L Kanamycin. Shoots resistant to the selection agent are appearing and once rooted are transferred to soil.

**[0523.0.0.0]** Samples of the primary transgenic plants (TO) are analyzed by PCR to confirm the presence of T-DNA. These results are confirmed by Southern hybridization in which DNA is electrophoresed on a 1% agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

**[0524.0.0.0]** Transgenic T0 ryegrass plants are propagated vegetatively by excising tillers. The transplanted tillers are maintained in the greenhouse for 2 months until well established. The shoots are defoliated and allowed to grow for 2 weeks.

**[0525.0.0.0]** Example 14b: Engineering soybean plants by over-expressing the polynucleotide characterized in the invention, e.g. derived from plants or another organism

**[0526.0.0.0]** Soybean can be transformed according to the following modification of the method described in the Texas A&M patent US 5,164,310. Several commercial soybean varieties are amenable to transformation by this method. The cultivar Jack (available from the Illinois Seed Foundation) is commonly used for transformation. Seeds are sterilized by immersion in 70% (v/v) ethanol for 6 min and in 25 % commercial bleach (NaOCl) supplemented with 0.1% (v/v) Tween for 20 min, followed by rinsing 4 times with sterile double distilled water. Removing the radicle, hypocotyl and one cotyledon from each seedling propagates seven-day seedlings. Then, the epicotyl with one cotyledon is transferred to fresh germination media in petri dishes and incubated at 25 °C under a 16-hr photoperiod (approx. 100 E-m-2s-1) for three weeks. Axillary nodes (approx. 4 mm in length) are cut from 3 - 4 week-old plants. Axillary nodes are excised and incubated in Agrobacterium LBA4404 culture.

**[0527.0.0.0]** Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used as described above, including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription as described above. In this example, the 34S promoter (GenBank Accession numbers M59930 and X16673) is used to provide constitutive expression of the trait gene.

**[0528.0.0.0]** After the co-cultivation treatment, the explants are washed and transferred to selection media supplemented with 500 mg/L timentin. Shoots are excised and placed on a shoot elongation medium. Shoots longer than 1 cm are placed on rooting medium for two to four weeks prior to transplanting to soil.

**[0529.0.0.0]** The primary transgenic plants (T0) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and is used as recommended by the manufacturer.

**[0530.0.0.0]** Example 14c: Engineering corn plants by over-expressing the polynucleotide characterized in the invention, e.g. derived from plants or another organism.

**[0530.1.0.0]** Amplification of for example SEQ ID NO: 25 was achieved as described in example 10 except that the upstream primer SEQ ID NO:75 and the reverse primer SEQ ID NO: 76 contained the following 5'extensions:
i) forward primer: 5'- GGGTCGCTCCTACGCG-3' SEQ ID NO: 314
ii) reverse primer 5'- CTCGGGCTCGGCGTCC-3' SEQ ID NO: 317

**[0530.2.0.0]:** Vector construction
The maize transformation vector for constitutive expression was constructed as follows.
As base vectors, the vectors EG073qcz (SEQ ID NO 311) and EG065qcz (SEQ ID NO: 312) were chosen. The MCS from EG065qcz was deleted by digestion of the vector with Asp718 and Pstl, followed by blunting of the vector using T4 DNA polymerase. The blunted vector was religated. The vector generated was called EG065-MCS. The LIC cassette was cloned in the vector EG065-MCS by hybridizing the following oligos, generating a DNA fragment with ends able to ligate into a Smal and Sacl digested vector. This fragment was ligated into the vector EG065-MCS that had been digested with Smal and Sacl. The generated vector was called EG065-LIC. The complete expression cassette comprising ScBV (Schenk (1999) Plant Mol Biol 39(6):1221-1230) promoter, LIC cassette and terminator was cut out of EG065-LIC with Ascl and Pacl and ligated into the vector EG073qcz that had previously been digested with Ascl and Pacl. The resulting binary vector for corn transformation was called pMME0607 (SEQ ID NO: 313).
Oligo POCCLicMIuISacllfw: gggtcgctcctacgcgtcaatgatccgcggacgccgagcccgagct (SEQ ID NO: 315)
Oligo POCCLicMIuISaclrev: cgggctcggcgtccgcggatcattgacgcgtaggagcgaccc (SEQ ID NO: 316)
For cloning of a polynucleotide of the invention, for example the ORF of SEQ ID NO: 25, from Linum usitatissimum the vector DNA was treated with the restriction enzyme MluI and SacII. The reaction was stopped by inactivation at 70°C for 20 minutes and purified over QlAquick columns following the standard protocol (Qiagen).
Then the PCR-product representing the amplified ORF and the vector DNA were treated with T4 DNA polymerase according to the standard protocol (MBI Fermentas) to produce single stranded overhangs with the parameters 1 unit T4 DNA polymerase at 37°C for 2-10 minutes for the vector and 1 u T4 DNA polymerase at 15°C for 10-60 minutes for the PCR product representing SEQ ID NO: 25.
The reaction was stopped by addition of high-salt buffer and purified over QIAquick columns following the standard protocol (Qiagen).
Approximately 30 ng of prepared vector and a defined amount of prepared amplificate were mixed and hybridized at 65 C for 15 minutes followed by 37 C 0,1 C/1 seconds, followed by 37 C 10 minutes, followed by 0,1 C/1 seconds, then 4 °C.
The ligated constructs were transformed in the same reaction vessel by addition of competent *E. coli* cells (strain DH5alpha) and incubation for 20 minutes at 1°C followed by a heat shock for 90 seconds at 42°C and cooling to 4°C. Then, complete medium (SOC) was added and the mixture was incubated for 45 minutes at 37°C. The entire mixture was subsequently plated onto an agar plate with 0.05 mg/ml kanamycine and incubated overnight at 37°C.
The outcome of the cloning step was verified by amplification with the aid of primers which bind upstream and downstream of the integration site, thus allowing the amplification of the insertion. The amplifications were carried as described in the protocol of *Taq* DNA polymerase (Gibco-BRL).
The amplification cycles were as follows: 1 cycle of 5 minutes at 94°C, followed by 35 cycles of in each case 15 seconds at 94°C, 15 seconds at 50-66°C and 5 minutes at 72°C, followed by 1 cycle of 10 minutes at 72°C, then 4°C.
Several colonies were checked, but only one colony for which a PCR product of the expected size was detected was used in the following steps.
A portion of this positive colony was transferred into a reaction vessel filled with complete medium (LB) supplemented with kanamycin () and incubated overnight at 37°C.
The plasmid preparation was carried out as specified in the Qiaprep standard protocol (Qiagen).

**[0530.3.0.0]** Example 14 c.a.: Corn Transformation
The preparation of the immature embryos and Agrobacterium were basically as stated in US 5,591,616. In brief, the Agrobacterium strain LBA4404 transformed with the plasmid by a standard method, such as the triple cross method or the electroporation, was grown on LB plates for 2 days prior to cocultivation. A loop of cells was resuspended in liquid infection media at an O.D. of approximately 1.0. Immature Embryos of about 1.5mm in size were incubated in the soln of agrobacterium for around 30 minutes. Excised embryos were removed from liquid and then co- cultivated in the dark at 22°C with Agrobacterium tumefaciens on solid MS-based callus induction medium containing 2 mg/l 2,4-D, 10um AgNO3, and 200um Acetosyringone. After several days of co-cultivation, embryos were transferred to MS-based media containing 2mg/l 2,4, 10um AgNO3 and 200mg/l Timentin the dark at 27°C for 1 week. Embryos were transferred to MS-based selection media containing imidazoline herbicide (500nM Pursuit) as a selection agent in the dark for 3 weeks. After 3 weeks putative transgenic events were transferred to an MS-based media containing 2mg/L Kinetin 500nM Pursuit, 200mg/l Timentin and incubated under cool white fluorescent light (100 uE/m2/s-1 with photoperiod of 16hrs) at 25 °Cfor 2-3 weeks, or until shoots develop. The shoots were transferred to MS-based rooting medium and incubated under light at 25 °C for 2 weeks. The rooted shoots were transplanted to 4 inch pots containing artificial soil mix. Metro-Mix® 360 in and grown in an environmental chamber for 1-2 weeks. The environmental chamber maintained 16-h-light, 8-h-dark cycles at 27°C day and 22 °C respectively. Light was supplied by a mixture of incandescent and cool white fluorescent bulbs with an intensity of - 400 uE/m2/s-1. After plants were grown to 4-6 leaf stage they were moved to 14 inch pots containing Metro-Mix® 360. Supplemental metal-halide lamps were used to maintain >800uE/m2/s-1 with a 16-h-light, 8-h-dark cycles at 28°C day and 22 °C. Transplantation occurs weekly on Tuesday. Peters 20-20-20 plus micronutrients (200ppm) is used to fertilize plants 2x weekly on Monday and Thursday after sampling of T0's is performed. T1 seeds were produced from plants that exhibit tolerance to the imidazolinone herbicides and which are PCR positive for the transgenes. T0 plants with single locus insertions of the T-DNA (self-pollinated) produced T1 generation that segregated for the transgene in a 3:1 ratio. Progeny containing copies of the transgene were tolerant of imidazolinone herbicides and could be detected by PCR analysis.

**[0530.4.0.0]** Example 14 c.b.: Growth of T0 corn plants for metabolic analysis
Plants were grown under the following standardized conditions to properly stage them for T0 sampling. T0 plantlets were transferred to 14" pots in the greenhouse after they grow to 4-6 leaf stage (1-3 weeks). pBSMM232 containing plants were produced carried along with each experiment to serve as controls for T0 samples. Plantlets were moved to 14" pots on Tuesday of each week. Plants were grown for 9 days until the 7-13 leaf stage is reached. On Thursday between 10am and 2 pm leaf sampling was performed on the 3rd youngest (1^{st} fully elongated). Within 30 seconds 250-500mg of leaf material (without midrib), were removed weighed and placed into pre-extracted glass thimbles in liquid nitrogen. A second sample (opposite side of the midrib) from each plant was sampled as described above for qPCR analysis.

**[0530.5.0.0]** Example 14 c.c.: Growth of T1 corn plant for metabolic analysis
For the bioanalytical analyses of the transgenic plants, the latter were grown uniformly in a specific culture facility. To this end the GS-90 substrate as the compost mixture was introduced into the potting machine (Laible System GmbH, Singen, Germany) and filled into the pots. Thereafter, 26 pots were combined in one dish and treated with Previcur. For the treatment, 25 ml of Previcur were taken up in 10 I of tap water. This amount was sufficient for the treatment of approximately 150 pots. The pots were placed into the Previcur solution and additionally irrigated overhead with tap water without Previcur. They were used within four days.
For the sowing, the seeds, which had been stored at room temperature were removed from the paper-bag and transferred into the pots with the soil. In total, approximately 1 to 3 seeds were distributed in the middle of the pot.
After the seeds had been sown, the dishes with the pots were covered with matching plastic hood and placed into growth chambers for 2 days. After this time the plastic hood was removed and plants were placed on the growth table and cultivated for 22 to 24 days under following growth conditions: 16-h-light, 8-h-dark rhythm at 20°C, an atmospheric humidity of 60% and a CO₂ concentration of approximately 400 ppm. The light sources used were Powerstar HQI-T 250 W/D Daylight lamps from Osram, which generate a light resembling the solar color spectrum with a light intensity of approximately 220 µE/m2/s-1.
When the plants were 7 days old, they were subjected to select transgenic plants. For this purposes pieces of plant leaves were sampled and a PCR reaction with the respective primers for the transgene were performed. Plants exhibiting the transgene were used for the metabolic analysis. The nontransgenic seedlings were removed. The transgenic plants were thinned when they had reached the age of 18 days. The transgenic plants, which had grown best in the center of the pot were considered the target plants. All the remaining plants were removed carefully with the aid of metal tweezers and discarded.
During their growth, the plants received overhead irrigation with distilled water (onto the compost) and bottom irrigation into the placement grooves. Once the grown plants had reached the age of 24 days, they were harvested.

**[0530.6.0.0]** Example 14 c.d.: Metabolic analysis of maize leaves.
The modifications identified in accordance with the invention, in the content of above-described metabolites, were identified by the following procedure.
a) Sampling and storage of the samples
   Sampling was performed in corridor next to the green house. The leaves were incised twice using small laboratory scissors and this part of the leave was removed manually from the middle rib. The sample was rapidly weighed on laboratory scales, transferred into a pre-cooled extraction sleeve and placed into kryo-box cooled by liquid nitrogen. The time elapsing between cutting the leave to freezing it in liquid nitrogen amounted to not more than 30 seconds. The boxes were stored in a freezer at -80 C, an shipped on dry ice.
b) Lyophilization
   During the experiment, care was taken that the plants either remained in the deep-frozen state (temperatures < -40°C) or were freed from water by lyophilization until the first contact with solvents. Before entering the analytical process the extraction sleeves with the samples were transferred to a pre-cooled aluminium rack.
   The aluminum rack with the plant samples in the extraction sleeves was placed into the pre-cooled (-40°C) lyophilization facility. The initial temperature during the main drying phase was-35°C and the pressure was 0.120 mbar. During the drying phase, the parameters were altered following a pressure and temperature program. The final temperature after 12 hours was +30°C and the final pressure was 0.001 to 0.004 mbar. After the vacuum pump and the refrigerating machine had been switched off, the system was flushed with air (dried via a drying tube) or argon.
c) Extraction
   Immediately after the lyophilization apparatus had been flushed, the extraction sleeves with the lyophilized plant material were transferred into the 5 ml extraction cartridges of the ASE device (Accelerated Solvent Extractor ASE 200 with Solvent Controller and AutoASE software (DIONEX)).
   Immediately after the lyophilization apparatus had been flushed, the extraction sleeves with the lyophilized plant material were transferred into the 5 ml extraction cartridges of the ASE device (Accelerated Solvent Extractor ASE 200 with Solvent Controller and AutoASE software (DIONEX)).
   The 24 sample positions of an ASE device (Accelerated Solvent Extractor ASE 200 with Solvent Controller and AutoASE software (DIONEX)) were filled with plant samples, including some samples for testing quality control.
   The polar substances were extracted with approximately 10 ml of methanol/water (80/20, v/v) at T = 70°C and p = 140 bar, 5 minutes heating-up phase, 1 minute static extraction. The more lipophilic substances were extracted with approximately 10 ml of methanol/dichloromethane (40/60, v/v) at T = 70°C and p = 140 bar, 5 minute heating-up phase, 1 minute static extraction. The two solvent mixtures were extracted into the same glass tubes (centrifuge tubes, 50 ml, equipped with screw cap and pierceable septum for the ASE (DIONEX)).
   The solution was treated with internal standards: ribitol, L-glycine-2,2-d₂, L-alanine-2,3,3,3-d₄, methionine-methyl-d₃, and α-methylglucopyranoside and methyl nonadecanoate, methyl undecanoate, methyl tridecanoate, methyl pentadecanoate, methyl nonacosanoate.
   The total extract was treated with 8 ml of water. The solid residue of the plant sample and the extraction sleeve were discarded.
   The extract was shaken and then centrifuged for 5 to 10 minutes at least at 1 400 g in order to accelerate phase separation. 0.5 ml of the supernatant methanol/water phase ("polar phase", colorless) was removed for the further GC analysis, and 0.5 ml was removed for the LC analysis. The remainder of the methanol/water phase of all samples was used for additional quality controls. 0.5 ml of the organic phase ("lipid phase", dark green) was removed for the further GC analysis and 0.5 ml was removed for the LC analysis. All the portions removed were evaporated to dryness using the IR Dancer infrared vacuum evaporator (Hettich). The maximum temperature during the evaporation process did not exceed 40°C. Pressure in the apparatus was not less than 10 mbar.
d) Processing the lipid phase for the LC/MS or LC/MS/MS analysis
   The lipid extract, which had been evaporated to dryness was taken up in mobile phase. The HPLC was run with gradient elution.
   The polar extract, which had been evaporated to dryness was taken up in mobile phase. The HPLC was run with gradient elution.
e) LC/MSMS analysis
   The LC part was carried out on a commercially available LCMS system from Agilent Technologies, USA. For polar extracts 10 µl are injected into the system at a flow rate of 200µl/min. The separation column (Reversed Phase C18) was maintained at 15 °C during chromatography. For lipid extracts 5 µl are injected into the system at a flow rate of 200 µl/min. The separation column (Reversed Phase C18) was maintained at 30 °C. HPLC was performed with gradient elution
   The mass spectrometric analysis was performed on a Applied Biosystems API 4000 triple quadrupole instrument with turbo ion spray source. For polar extracts the instrument measures in negative ion mode in fullscan mode from 100-1000 amu. For lipid extracts the instrument measures in postive ion mode in fullscan mode from 100-1000 amu.
f) Derivatization of the lipid phase for the GC/MS analysis
   For the transmethanolysis, a mixture of 140 µl of chloroform, 37 µl of hydrochloric acid (37% by weight HCl in water), 320 µl of methanol and 20 µl of toluene was added to the evaporated extract. The vessel was sealed tightly and heated for 2 hours at 100°C, with shaking. The solution was subsequently evaporated to dryness. The residue was dried completely.
   The methoximation of the carbonyl groups was carried out by reaction with methoxyamine hydrochloride (20 mg/ml in pyridine, 100 µl for 1.5 hours at 60°C) in a tightly sealed vessel. 20 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 100 µl of N-methyl-N-(trimethylsilyl)-2,2,2-trifluoroacetamide (MSTFA) was carried out for 30 minutes at 60°C, again in the tightly sealed vessel. The final volume before injection into the GC was 220 µl.
g) Derivatization of the polar phase for the GC/MS analysis
   The methoximation of the carbonyl groups was carried out by reaction with methoxyamine hydrochloride (20 mg/ml in pyridine, 50 µl for 1.5 hours at 60°C) in a tightly sealed vessel. 10 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 50 µl of N-methyl-N-(trimethylsilyl)-2,2,2-trifluoroacetamide (MSTFA) was carried out for 30 minutes at 60°C, again in the tightly sealed vessel. The final volume before injection into the GC was 110 µl.
h) GC/MS analysis
   The GC-MS systems consist of an Agilent 6890 GC coupled to an Agilent 5973 MSD. The autosamplers are CompiPal or GCPal from CTC. For the analysis usual commercial capillary separation columns (30 m x 0,25 mm x 0,25 µm) with different poly-methyl-siloxane stationary phases containing 0 % up to 35% of aromatic moieties, depending on the analysed sample materials and fractions from the phase separation step, are used (for example: DB-1 ms, HP-5ms, DB-XLB, DB-35ms, Agilent Technologies). Up to 1 µL of the final volume is injected splitless and the oven temperature program is started at 70 °C and ended at 340 °C with different heating rates depending on the sample material and fraction from the phase separation step in order to achieve a sufficient chromatographic separation and number of scans within each analyte peak. Usual GC-MS standard conditions, for example constant flow with nominal 1 to 1.7 ml/min. and helium as the mobile phase gas are used. lonisation is done by electron impact with 70 eV, scanning within a m/z range from 15 to 600 with scan rates from 2.5 to 3 scans/sec and standard tune conditions.
i) Analysis of the various plant samples
   The samples were measured in individual series of 20 plant (leaf) samples each (also referred to as sequences), each sequence containing at least 5 samples from individual control plants containing GUS. The peak area of each analyte was divided by the peak area of the respective internal standard. The data were standardized for the fresh weight established for the respective harvested sample. The values calculated were then related to the GUS-containing control group by being divided by the mean of the corresponding data of the control group of the same sequence. The values obtained were referred to as ratio_by_WT, they are comparable between sequences and indicate how much the analyte concentration in the mutant differs in relation to the control. The GUS-containing plants were chosen in order to assure that the vector and transformation procedure itself has no significant influence on the metabolic composition of the plants. Therefore the desribed changes in comparison with the controls were caused by the introduced genes.

**[0531.0.0.0]** Transformation of maize (Zea Mays L.) can also be performed with a modification of the method described by Ishida et al. (1996. Nature Biotech 14745-50). Transformation is genotype-dependent in corn and only specific genotypes are amenable to transformation and regeneration. The inbred line A188 (University of Minnesota) or hybrids with A188 as a parent are good sources of donor material for transformation (Fromm et al. 1990 Biotech 8:833-839), but other genotypes can be used successfully as well. Ears are harvested from corn plants at approximately 11 days after pollination (DAP) when the length of immature embryos is about 1 to 1.2 mm. Immature embryos are co-cultivated with Agrobacterium tumefaciens that carry "super binary" vectors and transgenic plants are recovered through organogenesis. The super binary vector system of Japan Tobacco is described in WO patents WO94/00977 and WO95/06722. Vectors can be constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. In this example, the 34S promoter (GenBank Accession numbers M59930 and X16673 can be used to provide constitutive expression of the trait gene.

**[0532.0.0.0]** Excised embryos can be grown on callus induction medium, then maize regeneration medium, containing imidazolinone as a selection agent. The Petri plates can be incubated in the light at 25 °C for 2-3 weeks, or until shoots develop. The green shoots can be transferred from each embryo to maize rooting medium and incubated at 25 °C for 2-3 weeks, until roots develop. The rooted shoots can be transplanted to soil in the greenhouse. T1 seeds can be produced from plants that exhibit tolerance to the imidazolinone herbicides and which can be PCR positive for the transgenes.

**[0533.0.0.0]** The T1 generation of single locus insertions of the T-DNA can segregate for the transgene in a 3:1 ratio. Those progeny containing one or two copies of the transgene can be tolerant of the imidazolinone herbicide. Homozygous T2 plants can exhibited similar phenotypes as the T1 plants. Hybrid plants (F1 progeny) of homozygous transgenic plants and non-transgenic plants can also exhibit increased similar phenotypes.

**[0534.0.0.0]** Example 14d: Engineering wheat plants by over-expressing the polynucleotide characterized in the invention, e.g. derived from plants or another organism

**[0535.0.0.0]** Transformation of wheat can be performed with the method described by Ishida et al. (1996 Nature Biotech. 14745-50). The cultivar Bobwhite (available from CYMMIT, Mexico) can commonly be used in transformation. Immature embryos can be co-cultivated with Agrobacterium tumefaciens that carry "super binary" vectors, and transgenic plants are recovered through organogenesis. The super binary vector system of Japan Tobacco is described in WO patents WO94/00977 and WO95/06722. Vectors can be constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. The 34S promoter (GenBank Accession numbers M59930 and X16673) can be used to provide constitutive expression of the trait gene.

**[0536.0.0.0]** After incubation with Agrobacterium, the embryos can be grown on callus induction medium, then regeneration medium, containing imidazolinone as a selection agent. The Petri plates can be incubated in the light at 25 °C for 2-3 weeks, or until shoots develop. The green shoots can be transferred from each embryo to rooting medium and incubated at 25 °C for 2-3 weeks, until roots develop. The rooted shoots can be transplanted to soil in the greenhouse. T1 seeds can be produced from plants that exhibit tolerance to the imidazolinone herbicides and which are PCR positive for the transgenes.

**[0537.0.0.0]** The T1 generation of single locus insertions of the T-DNA can segregate for the transgene in a 3:1 ratio. Those progeny containing one or two copies of the transgene can be tolerant of the imidazolinone herbicide. Homozygous T2 plants exhibited similar phenotypes.

**[0538.0.0.0]** Example 14e: Engineering Rapeseed/Canola plants by over-expressing the polynucleotide characterized in the invention, e.g. derived from plants or another organism

**[0539.0.0.0]** Cotyledonary petioles and hypocotyls of 5-6 day-old young seedlings can be used as explants for tissue culture and transformed according to Babic et al.(1998, Plant Cell Rep 17: 183-188). The commercial cultivar Westar (Agriculture Canada) can be the standard variety used for transformation, but other varieties can be used.

**[0540.0.0.0]** Agrobacterium tumefaciens LBA4404 containing a binary vector can be used for canola transformation. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette can consist of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription. The 34S promoter (GenBank Accession numbers M59930 and X16673) can be used to provide constitutive expression of the trait gene.

**[0541.0.0.0]** Canola seeds can be surface-sterilized in 70% ethanol for 2 min., and then in 30% Clorox with a drop of Tween-20 for 10 min, followed by three rinses with sterilized distilled water. Seeds can be then germinated in vitro 5 days on half strength MS medium without hormones, 1% sucrose, 0.7% Phytagar at 23oC, 16 hr. light. The cotyledon petiole explants with the cotyledon attached can be excised from the in vitro seedlings, and can be inoculated with Agrobacterium by dipping the cut end of the petiole explant into the bacterial suspension. The explants can be then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants can be transferred to MSBAP-3 medium containing 3 mg/l BAP, cefotaxime, carbenicillin, or timentin (300 mg/l) for 7 days, and can then be cultured on MSBAP-3 medium with cefotaxime, carbenicillin, or timentin and selection agent until shoot regeneration. When the shoots are 5 - 10 mm in length, they can be cut and transferred to shoot elongation medium (MSBAP-0.5, containing 0.5 mg/I BAP). Shoots of about 2 cm in length can be transferred to the rooting medium (MSO) for root induction.

**[0542.0.0.0]** Samples of the primary transgenic plants (TO) can be analyzed by PCR to confirm the presence of T-DNA. These results can be confirmed by Southern hybridization in which DNA is electrophoresed on a 1 % agarose gel and are transferred to a positively charged nylon membrane (Roche Diagnostics). The PCR DIG Probe Synthesis Kit (Roche Diagnostics) can be used to prepare a digoxigenin-labelled probe by PCR, and used as recommended by the manufacturer.

**[0543.0.0.0]** Example 14f: Engineering alfalfa plants by over-expressing the polynucleotide characterized in the invention, e.g. derived from plants or another organism.

**[0544.0.0.0]** A regenerating clone of alfalfa (Medicago sativa) can be transformed using the method of (McKersie et al., 1999 Plant Physiol 119: 839-847). Regeneration and transformation of alfalfa can be genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) can be selected for use in tissue culture (Walker et al., 1978 Am J Bot 65:654-659).

**[0545.0.0.0]** Petiole explants can be cocultivated with an overnight culture of Agrobacterium tumefaciens C58C1 pMP90 (McKersie et al., 1999 Plant Physiol 119: 839-847) or LBA4404 containing a binary vector. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette can consist of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene that provides constitutive, developmental, tissue or environmental regulation of gene transcription. The 34S promoter (GenBank Accession numbers M59930 and X16673) can be used to provide constitutive expression of the trait gene.

**[0546.0.0.0]** The explants can be cocultivated for 3 d in the dark on SH induction medium containing 288 mg/ L Pro, 53 mg/ L thioproline, 4.35 g/ L K2SO4, and 100 µm acetosyringinone. The explants can be washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos can be transferred to BOi2Y development medium containing no growth regulators, no antibiotics, and 50 g/ L sucrose. Somatic embryos are subsequently germinated on half-strength Murashige-Skoog medium. Rooted seedlings can be transplanted into pots and grown in a greenhouse.

**[0547.0.0.0]** The T0 transgenic plants are propagated by node cuttings and rooted in Turface growth medium. The plants are defoliated and grown to a height of about 10 cm (approximately 2 weeks after defoliation).

**[0548.0.0.0]** Example 14g: Engineering alfalfa plants by over-expressing the polynucleotide characterized in the invention, derived e.g. from Saccharomyces cerevisiae, E. coli or plants or another organism

**[0549.0.0.0]** A regenerating clone of alfalfa (Medicago sativa) can be transformed using the method of (McKersie et al., 1999 Plant Physiol 119: 839-847). Regeneration and transformation of alfalfa can be genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659).

**[0550.0.0.0]** Petiole explants can be cocultivated with an overnight culture of Agrobacterium tumefaciens C58C1 pMP90 (McKersie et al., 1999 Plant Physiol 119: 839-847) or LBA4404 containing a binary vector. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene that provides constitutive, developmental, tissue or environmental regulation of gene transcription. In this example, the 34S promoter (GenBank Accession numbers M59930 and X16673) can be used to provide constitutive expression of the trait gene.

**[0551.0.0.0]** The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings are transplanted into pots and grown in a greenhouse.

**[0552.0.0.0]** The T0 transgenic plants are propagated by node cuttings and rooted in Turface growth medium. The plants are defoliated and grown to a height of about 10 cm (approximately 2 weeks after defoliation).

**[0552.1.0.0]** ./.

**[0552.2.0.0]** Example 16: Preparation of homologous sequences from plants Different plants can be grown under standard or varying conditions in the greenhouse. RNA can be extracted following the protocol of Jones, Dunsmuir and Bedbrook (1985) EMBO J. 4: 2411-2418. Approx. 1 gram of tissue material from various organs is ground in liquid nitrogen. The powder is transferred to a 13 ml Falcon tube containing 4.5 ml NTES buffer (100 mM NaCl, 10 mM Tris/HCl pH 7.5, 1 mM EDTA, 1 % SDS; in RNase-free water) and 3 ml phenol/chloroform/isoamylalcohol (25/24/1), immediately mixed and stored on ice. The mixture is spun for 10 minutes at 7000 rpm using a centrifuge (Sorval; SM24 or SS34 rotor). The supernatant is transferred to a new tube, 1/10th volume of 3 M NaAcetate (pH 5.2; in RNase-free water) and 1 volume of isopropanol is added, mixed at stored for 1 hour or overnight at -20 °C. The mixture is spun for 10 minutes at 7000 rpm. The supernatant is discarded and the pellet washed with 70 % ethanol (v/v). The mixture is spun for 5 minutes at 7000 rpm, the supernatant is discarded and the pellet is air-dried. 1 ml RNase-free water is added and allow the DNA/RNA pellet to dissolve on ice at 4 C. The nucleic acid solution is transferred to a 2 ml Eppendorf tube and 1 ml of 4 M LiAcetate is added. After mixing the solution is kept for at least 3 hours, or overnight, at 4 C. The mixture is spun for 10 minutes at 14000 rpm, the supernatant discarded, the pellet washed with 70 % Ethanol, air-dried and dissolved in 200 µl of RNase-free water.
Total RNA can be used to construct a cDNA-library according to the manufacturer's protocol (for example using the ZAP-cDNA synthesis and cloning kit of Stratagene, La Jolla, USA). Basically, messenger RNA (mRNA) is primed in the first strand synthesis with a oligo(dT) linker-primer and is reverse-transcribed using reverse transcriptase. After second strand cDNA synthesis, the double-stranded cDNA is ligated into the Uni-ZAP XR vector. The Uni-ZAP XR vector allows in vivo excision of the pBluescript phagemid. The polylinker of the pBluescript phagemid has 21 unique cloning sites flanked by T3 and T7 promoters and a choice of 6 different primer sites for DNA sequencing. Systematic single run sequencing of the expected 5 prime end of the clones can allow preliminary annotation of the sequences for example with the help of the pedant pro Software package (Biomax, München). Clones for the nucleic acids of the invention or used in the process according to the invention can be identified based on homology search with standard algorithms like blastp or gap. Identified putative full length clones with identity or high homology can be subjected to further sequencing in order to obtain the complete sequence.
Additional new homologous sequences can be identified in a similar manner by preparing respective cDNA libraries from various plant sources as described above. Libraries can then be screened with available sequences of the invention under low stringency conditions for example as described in Sambrook et al., Molecular Cloning: A laboratory manual, Cold Spring Harbor 1989, Cold Spring Harbor Laboratory Press. Purified positive clones can be subjected to the in vivo excision and complete sequencing. A pairwise sequence alignment of the original and the new sequence using the blastp or gap program allows the identification of orthologs, meaning homologous sequences from different organisms, which should have a sequence identity of at least 30%. Furthermore the conservation of functionally important amino acid residues or domains, which can be identified by the alignment of several already available paralogs, can identify a new sequence as an new orthologs..
Alternatively libraries can be subjected to mass sequencing and obtained sequences can be stored in a sequence database, which then can be screened for putative orthologs by different search algorithms, for example the tbastn algorithm to search the obtained nucleic acid sequences with a amino acid sequence of the invention. Clones with the highest sequence identity are used for a complete sequence determination and orthologs can be identified as described above.

**[0553.0.0.0]** A process for the production of methionine, which comprises
(a) increasing or generating the activity of a protein as indicated in Table II, application no. 0, columns 5 or 7, or a functional equivalent thereof in a non-human organism, or in one or more parts thereof; and
(b) growing the organism under conditions which permit the production of methionine in said organism.
A process for the production of methionine, comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 0, columns 5 or 7, or a fragment thereof, which confers an increase in the amount of methionine in an organism or a part thereof;
b) nucleic acid molecule comprising of a nucleic acid molecule as indicated in Table I, application no. 0, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of methionine in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of methionine in an organism or a part thereof;
e) nucleic acid molecule which hybridizes with a nucleic acid molecule of (a) to (c) under stringent hybridisation conditions and conferring an increase in the amount of methionine in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 0, columns 7, and conferring an increase in the amount of the respective fine chemical in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of methionine in an organism or a part thereof;
h) nucleic acid molecule encoding a polypeptide comprising a consensus sequence as indicated in Table IV, application no. 0, columns 7, and conferring an increase in the amount of the respective fine chemical in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the respective fine chemical in an organism or a part thereof.
or comprising a sequence which is complementary thereto.
The process as mentioned above, comprising recovering of the free or bound methionine.
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound methionine produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 0, columns 5 or 7, or a fragment thereof, which confers an increase in the amount of methionine in an organism or a part thereof;
b) nucleic acid molecule comprising of a nucleic acid molecule as indicated in Table I, application no. 0, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of methionine in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of methionine in an organism or a part thereof;
e) nucleic acid molecule which hybridizes with a nucleic acid molecule of (a) to (c) under stringent hybridisation conditions and conferring an increase in the amount of methionine in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 0, columns 7, and conferring an increase in the amount of the respective fine chemical in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of methionine in an organism or a part thereof;
h) nucleic acid molecule encoding a polypeptide comprising a consensus sequence as indicated in Table IV, application no. 0, columns 7, and conferring an increase in the amount of the respective fine chemical in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the respective fine chemical in an organism or a part thereof.
whereby the nucleic acid molecule distinguishes over the sequence as indicated in Table I, application no. 0, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 0, columns 5 or 7, by one or more amino acids.
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of methionine in an organism or a part thereof comprising:
(a) contacting cells, tissues , plants or microorganisms which express the a polypeptide encoded by the nucleic acid molecule as mentioned above conferring an increase in the amount of methionine in an organism or a part thereof with a candidate compound or a sample comprising a plurality of compounds under conditions which permit the expression the polypeptide;
(b) assaying the methionine level or the polypeptide expression level in the cell, tissue, plant or microorganism or the media the cell, tissue, plant or microorganisms is cultured or maintained in; and
(c) identifying a agonist or antagonist by comparing the measured methionine level or polypeptide expression level with a standard methionine or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased methionine production in a plant or microorganism, comprising the steps:
(a) culturing a plant cell or tissue or microorganism or maintaining a plant expressing the polypeptide encoded by the nucleic acid molecule as mentioned above conferring an increase in the amount of methionine in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of methionine in an organism or a part thereof;
(b) identifying if the compound is an effective agonist by detecting the presence or absence or increase of a signal produced by said readout system.
A method for the identification of a gene product conferring an increase in methionine production in a cell, comprising the following steps:
(a) contacting the nucleic acid molecules of a sample, which can contain a candidate gene encoding a gene product conferring an increase in methionine after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing methionine;
(d) expressing the identified nucleic acid molecules in the host cells;
(e) assaying the methionine level in the host cells; and
(f) identifying nucleic acid molecule and its gene product which expression confers an increase in the methionine level in the host cell in the host cell after expression compared to the wild type.
A method for the identification of a gene product conferring an increase in methionine production in a cell, comprising the following steps:
(a) identifying in a data bank nucleic acid molecules of an organism; which can contain a candidate gene encoding a gene product conferring an increase in the methionine amount or level in an organism or a part thereof after expression, and which are at least 20% homolog to the nucleic acid molecule as mentioned above;
(b) introducing the candidate nucleic acid molecules in host cells appropriate for producing methionine;
(c) expressing the identified nucleic acid molecules in the host cells;
(d) assaying the methionine level in the host cells; and
(e) identifying nucleic acid molecule and its gene product which expression confers an increase in the methionine level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of methionine after expression.
Use of the polypeptide as mentioned above or the nucleic acid construct as mentioned above or the gene product identified according to the method as mentioned above for identifying compounds capable of conferring a modulation of methionine levels in an organism.
Food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.

### New genes for a process for the production of fine chemicals

**[0000.0.0.1]** In a further embodiment, the present invention relates to a further process for the production of fine chemicals as defined below and corresponding embodiments as described herein as follows.

**[0001.0.0.1] to [0007.0.0.1]: see [0001.0.0.0] to [0007.0.0.0]**

**[0007.1.0.1]** Following the approach of deregulating specific enzymes in the amino acid biosynthetic pathway an increase of the levels of free threonine is disclosed in US 5,942,660 which is achieved by overexpression of either a wild-type or deregulated aspartate kinase, homoserine dehydrogenase or threonine synthase.

**[0008.0.0.1] see [0008.0.0.0]**

**[0009.0.1.1]** As described above, the essential amino acids are necessary for humans and many mammals, for example for livestock. Threonine is an important constituent in many body proteins and is necessary for the formation of tooth enamel protein, collagen and elastin, which both needed for healthy skin and wound healing. It is a precursor to the amino acids glycine and serine. It acts as a lipotropic in controlling fat build-up in the liver. Threonine is an immune stimulant because it promotes thymus growth and activity. It is a component of digestive enzymes and immune secretions from the gut, particularly mucins. It has been used as a supplement to help alleviate anxiety and some cases of depression. In animal production, as an important essential amino acid, threonine is normally the second limiting amino acid for pigs and the third limiting amino acid for chicken (Gallus gallus f. domestica, e.g. laying hen or broiler).

**[0010.0.0.1] see [0010.0.0.0]**

**[0011.0.0.1] see [0011.0.0.0]**

**[0012.0.1.1]** It is an object of the present invention to develop an inexpensive process for the synthesis of threonine, preferably L-threonine. Threonine is together with lysine and methionine (depending on the organism) one of the amino acids which are most frequently limiting.

**[0013.0.0.1] see [0013.0.0.0]**

**[0014.0.1.1]** Accordingly, in a first embodiment, the invention relates to a process for the production of a fine chemical, whereby the fine chemical is threonine, preferably L-threonine. Accordingly, in the present invention, the term "the fine chemical" as used herein relates to "threonine". Further, the term "the fine chemicals" as used herein also relates to fine chemicals comprising threonine.

**[0015.0.1.1]** In one embodiment, the term "the fine chemical" means threonine, preferably L-threonine. Throughout the specification the term "the fine chemical" means threonine, preferably L-threonine, its salts, ester or amids in free form or bound to proteins. In a preferred embodiment, the term "the fine chemical" means threonine, preferably L-threonine, in free form or its salts or bound to proteins.

**[0016.0.1.1]** Accordingly, the present invention relates to a process for the production of the respective fine chemical comprising
(a) increasing or generating the activity of one or more of a protein as shown in table II, application no. 1, column 3 encoded by the nucleic acid sequences as shown in table I, application no. 1, column 5, in a non-human organism or in one or more parts thereof or
(b) growing the organism under conditions which permit the production of the fine chemical, thus amino acid of the invention or fine chemicals comprising amino acids of the invention, in said organism or in the culture medium surrounding the organism.

**[0017.0.0.1] see [0017.0.0.0]**

**[0018.0.0.1] to [0019.0.0.1] see [0018.0.0.0] to [0019.0.0.0]**

**[0020.0.1.1]** Surprisingly it was found, that the transgenic expression of the Zea mays protein as indicated in Table II, application no.1, column 5, line 3 in a plant conferred an increase in Threonine content of the transformed plants. Thus, in one embodiment, said protein or its homologs are used for the production of Threonine. Surprisingly it was found, that the transgenic expression of the Zea mays protein as indicated in Table II, application no.1, column 5, line 4 in thaliana plant conferred an increase in Threonine content of the transformed plants. Thus, in one embodiment, said protein or its homologs are used for the production of Threonine.

**[0021.0.0.1] see [0021.0.0.0]**

**[0022.0.1.1]** The sequence of YKR057W from Saccharomyces cerevisiae has been published in Dujon et al., Nature 369 (6479), 371-378, 1994 and Goffeau et al., Science 274 (5287), 546-547, 1996 and its activity is being defined as an ribosomal protein, similar to S21 ribosomal proteins, involved in ribosome biogenesis and translation. Accordingly, in one embodiment, the process of the present invention comprises the use of a ribosomal protein, similar to S21 ribosomal proteins, involved in ribosome biogenesis and translation from Saccaromyces cerevisiae or a plant or its homolog, e.g. as shown herein, for the production of the fine chemical, meaning of threonine, in particular for increasing the amount of threonine, preferably L-threonine in free or bound form in an organism or a part thereof, as mentioned.
The sequence of YOR245C from Saccharomyces cerevisiae has been published in Dujon, B. et al., Nature 387 (6632 Suppl), 98-102 (1997) and its activity is defined as a acyl-CoA:diacylglycerol acyltransferase. Accordingly, in one embodiment, the process of the present invention comprises the use of a gene product defined as a acyl-CoA:diacylglycerol acyltransferase from Saccaromyces cerevisiae or a plant or its homolog, e.g. as shown herein, for the production of the fine chemical, meaning threonine, in particular for increasing the amount of threonine, preferably L-threonine in free or bound form in an organism or a part thereof, as mentioned. In one embodiment, in the process of the present invention the activity of a acyl-CoA:diacylglycerol acyltransferase is increased or generated, e.g. from Saccharomyces cerevisiae or a homolog thereof.

**[0022.1.0.1] to [0023.0.0.1] see [0022.1.0.0] to [0023.0.0.0]**

**[0023.1.1.1]** Homologs of the polypeptide disclosed in table II, application no., column 5, may have the activity of a protein as disclosed in table I or II, application no.1, column 3 and may be the polypetides encoded by the nucleic acid molecules indicated in table I , application no. 1, column 7, resp., or may be the polypeptides indicated in table II, application no. 1, column 7, resp.

**[0024.0.0.1]: see [0024.0.0.0]**

**[0025.0.1.1]** In accordance with the invention, a protein or polypeptide has the "activity of an protein as shown in table II, application no. 1, column 3 "if its de *novo* activity, or its increased expression directly or indirectly leads to an increased in the level of the fine chemical indicated in the respective line of table II, application no. 1, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 1, column 3, or which has at least 10% of the original enzymatic or biological activity, preferably 20%, particularly preferably 30%, most particularly preferably 40% in comparison to a protein as shown in the respective line of table II, application no. 1, column 3 of a a E. coli or Saccharomyces cerevisae, respectively, protein as mentioned in table I to IV, column 3 respectively and as disclosed in paragraph [0022] of the respective invention.

**[0025.1.0.1]** In one embodiment, the polypeptide of the invention confers said activity, e.g. the increase of the fine chemical in an organism or a part thereof, if it is derived from an organism, which is evolutionary distant to the organism in which it is expressed. For example origin and expressing organism are derived from different families, orders, classes or phylums.

**[0026.0.0.1] to [0033.0.0.1]: see [0026.0.0.0] to [0033.0.0.0]**

**[0034.0.1.1]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 1, column 3 its biochemical or genetical causes and the increased amount of the respective fine chemical.

**[0035.0.0.1] to [0044.0.0.1]: see [0035.0.0.0] to [0044.0.0.0]**

**[0045.0.1.1]** In one embodiment, the activity of the protein. as indicated in Table II, columns 5 or 7, application no. 1, is increased conferring an increase of the respective fine chemical, indicated in Table II, application no. 1, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more

**[0046.0.1.1]** In one embodiment, the activity of the protein as indicated in Table II, columns 5 or 7, application no. 1, is increased conferring an increase of the respective fine chemical, indicated in Table II, application no. 1, column 6 of the respective line confers an increase of the respective fine chemical and of further amino acid or their precursors.

**[0047.0.0.1] see [0047.0.0.0]**

**[0048.0.0.1] see [0048.0.0.0]**

**[0049.0.1.1]** A protein having an activity conferring an increase in the amount or level of the fine chemical, has in one embodiment the structure of the polypeptide described herein, in particular of the polypeptides comprising the consensus sequence shown in table IV, application no. 1, column 7 or of the polypeptide as shown in the amino acid sequences as disclosed in table II, application no. 1, columns 5 and 7 or the functional homologues thereof as described herein, or is encoded by the nucleic acid molecule characterized herein or the nucleic acid molecule according to the invention, for example by the nucleic acid molecule as shown in table I, application no. 1, columns 5 and 7 or its herein described functional homologues and has the herein mentioned activity.

**[0050.0.1.1]** For the purposes of the present invention, the term "threonine" and "L-threonine" also encompass the corresponding salts, such as, for example, threonine hydrochloride or threonine sulfate. Preferably the term threonine is intended to encompass the term L-threonine.

**[0051.0.0.1] see [0051.0.0.0]**

**[0052.0.0.1] see [0052.0.0.0]**

**[0053.0.1.1]** In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 1, columns 5 and 7 or its homologs activity having herein-mentioned amino acid of the invention increasing activity; and/or
b) stabilizing a mRNA conferring the increased expression of a protein encoded by the nucleic acid molecule of the invention as shown in table I, application no. 1, columns 5 and 7, e.g. a nucleic acid sequence encoding a polypeptide having the activity of a protein as indicated in table II, application no. 1, columns 5 and 7 or its homologs activity or of a mRNA encoding the polypeptide of the present invention having herein-mentioned amino acid of the invention increasing activity; and/or
c) increasing the specific activity of a protein conferring the increased expression of a protein encoded by the nucleic acid molecule of the invention or of the polypeptide of the present invention having herein-mentioned amino acid increasing activity, e.g. of a polypeptide having the activity of a protein as indicated in table II, application no. 1, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned amino acid of the invention increasing activity, e.g. of a polypeptide having the activity of a protein as indicated in table II, application no. 1, columns 5 and 7 or its homologs activity; and/or
e) stimulating activity of a protein conferring the increased expression of a protein encoded by the nucleic acid molecule of the present invention or a polypeptide of the present invention having herein-mentioned amino acid of the invention increasing activity, e.g. of a polypeptide having the activity of a protein as indicated in table II, application no. 1, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned amino acid of the invention increasing activity, e.g. of a polypeptide having the activity of a protein as indicated in table II, application no. 1, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned amino acid of the invention increasing activity, e.g. of a polypeptide having the activity of a protein as indicated in table II, application no. 1, columns 5 and 7 or its homologs activity; and/or
h) increasing the expression of the endogenous gene encoding the polypeptide of the invention, e.g. a polypeptide having the activity of a protein as indicated in table II, application no. 1, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with expecially high activity of the proteins of the invention from natural or from mutagenized resources and breeding them into the target organisms, eg the elite crops.

**[0054.0.1.1]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 1 in Table I to IV, resp., after increasing the expression or activity of the encoded polypeptide or having the activity of a polypeptide having an activity as the protein as shown in table II, application no. 1, column 3 or its homologs.

**[0055.0.0.1] to [0064.0.0.1] see [0055.0.0.0] to [0064.0.0.0]**

**[0065.0.1.1]** The activation of an endogenous polypeptide having above-mentioned activity, of the polypeptide of the invention, e.g. conferring the increase of the fine chemical after increase of expression or activity can also be increased by introducing a synthetic transcription factor, which binds close to the coding region of an endogenous polypeptide of the invention- or its endogenous homolog-encoding gene and activates its transcription. A chimeric zinc finger protein can be construed, which comprises a specific DNA-binding domain and an activation domain as e.g. the VP16 domain of Herpes Simplex virus. The specific binding domain can bind to the regulatory region of the endogenous protein-coding region. The expression of the chimeric transcription factor in a organism, in particular in a plant, leads to a specific expression of an endogenous polypeptid of the invention, in particular a plant homolog thereof, see e.g. in WO01/52620, Oriz, Proc. Natl. Acad. Sci. USA, 2002, Vol. 99, 13290 or Guan, Proc. Natl. Acad. Sci. USA, 2002, Vol. 99, 13296.

**[0066.0.0.1] to [0069.0.0.1]: see [0066.0.0.0] to [0069.0.0.0]**

**[0070.0.1.1]** Owing to the introduction of a gene or a plurality of genes conferring the expression of the nucleic acid molecule of the invention or the polypeptide of the invention, for example the nucleic acid construct mentioned below, into an organism alone or in combination with other genes, it is possible not only to increase the biosynthetic flux towards the end product, but also to increase, modify or create de *novo* an advantageous, preferably novel metabolites composition in the organism, e.g. an advantageous amino acid composition comprising a higher content of (from a viewpoint of nutrional physiology limited) fine chemicals, in particular amino acids, likewise the fine chemical.

**[0071.0.0.1] see [0071.0.0.0]**

**[0072.0.1.1]** By influencing the metabolism thus, it is possible to produce, in the process according to the invention, further advantageous hydroxy containing compounds. Examples of such compounds are, in addition to threonine, serine, homoserine, phosphohomoserine or hydroxyproline or methionine.

**[0073.0.1.1]** Accordingly, in one embodiment, the process according to the invention relates to a process which comprises:
(a) providing a non-human organism, preferably a microorganism, a non-human animal, a plant or animal cell, a plant or animal tissue or a plant;
(b) increasing an activity of a polypeptide of the invention or a homolog thereof, e.g. as indicated in Table II, application no. 1, columns 5 or 7, or of a polypeptide being encoded by the nucleic acid molecule of the present invention and described below, e.g. conferring an increase of the fine chemical in the organism, preferably in a microorganism, a non-human animal, a plant or animal cell, a plant or animal tissue or a plant,
(c) growing the organism, preferably a microorganism, a non-human animal, a plant or animal cell, a plant or animal tissue or a plant under conditions which permit the production of the fine chemical in the organism, preferably the microorganism, the plant cell, the plant tissue or the plant; and
(d) if desired, revovering, optionally isolating, the free and/or bound the fine chemical and , optionally further free and/or bound amino acids synthetized by the organism, the microorganism, the non-human animal, the plant or animal cell, the plant or animal tissue or the plant.

**[0074.0.0.1] to [0084.0.0.1]: see [0074.0.0.0] to [0084.0.0.0]**

**[0085.0.1.1]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
(a) a nucleic acid sequence as indicated in Table I, application no. 1, columns 5 or 7, or
(b) a genetic regulatory element, for example a promoter, which is functionally linked to the nucleic acid sequence as indicated in Table I, application no. 1,columns 5 or 7, or a derivative thereof, or
(c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide radicals. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.1]: see [0086.0.0.0]**

**[0087.0.0.1]: see [0087.0.0.0]**

**[0088.0.1.1]** In an advantageous embodiment of the invention, the organism takes the form of a plant whose amino acid content is modified advantageously owing to the nucleic acid molecule of the present invention expressed. This is important for plant breeders since, for example, the nutritional value of plants for monogastric animals is limited by a few essential amino acids such as lysine, threonine or methionine.

**[0088.1.1.1]** In one embodiment, after an activity of a polypeptide of the present invention has been increased or generated, or after the expression of a nucleic acid molecule or polypeptide according to the invention has been generated or increased, the transgenic plant generated can be grown on or in a nutrient medium or else in the soil and subsequently harvested.

**[0089.0.0.1] to [0097.0.0.1]: see [0089.0.0.0] to [0097.0.0.0]**

**[0098.0.1.1]** In a further embodiment, the fine chemical threonine is produced in accordance with the invention and, if desired, is isolated. The production of further amino acids such as methionine, lysine and/or mixtures of amino acid by the process according to the invention is advantageous.

**[0099.0.0.1] to [0102.0.0.1]: see [0099.0.0.0] to [0102.0.0.0]**

**[0103.0.1.1]** In a preferred embodiment, the present invention relates to a process for the production of the fine chemical threonine comprising or generating in an organism or a part thereof the expression of at least one nucleic acid molecule comprising a nucleic acid molecule selected from the group consisting of:
(a) nucleic acid molecule encoding, preferably at least the mature form, of a polypeptide having a sequence as indicated in Table II, application no. 1,columns 5 or 7;
(b) nucleic acid molecule comprising, preferably at least the mature form, of a nucleic acid molecule having a sequence as indicated in Table I, application no. 1,columns 5 or 7;
(c) nucleic acid molecule whose sequence can be deduced from a polypeptide sequence encoded by a nucleic acid molecule of (a) or (b) as result of the degeneracy of the genetic code and conferring an increase in the amount of the fine chemical threonine in an organism or a part thereof;
(d) nucleic acid molecule encoding a polypeptide which has at least 50% identity with the amino acid sequence of the polypeptide encoded by the nucleic acid molecule of (a) to (c) and conferring an increase in the amount of the fine chemical threonine in an organism or a part thereof;
(e) nucleic acid molecule which hybidizes with a nucleic acid molecule of (a) to (c) under under stringent hybridisation conditions and conferring an increase in the amount of the fine chemical threonine in an organism or a part thereof;
(f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical threonine in an organism or a part thereof;
(g) nucleic acid molecule encoding a fragment or an epitope of a polypeptide which is encoded by one of the nucleic acid molecules of (a) to (e), preferably to (a) to (c) and and conferring an increase in the amount of the fine chemical threonine in an organism or a part thereof;
(h) nucleic acid molecule comprising a nucleic acid molecule which is obtained by amplifying nucleic acid molecules from a cDNA library or a genomic library using the primers pairs having a sequence as indicated in Table III, application no. 1, columns 7, and conferring an increase in the amount of the fine chemical threonine in an organism or a part thereof;
(i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the fine chemical threonine in an organism or a part thereof;
(j) nucleic acid molecule which encodes a polypeptide comprising the consensus sequence having a sequences as indicated in Table IV, application no. 1,column 7, and conferring an increase in the amount of the fine chemical threonine in an organism or a part thereof;
(k) nucleic acid molecule comprising one or more of the nucleic acid molecule encoding the amino acid sequence of a polypeptide encoding a domain of a polypeptide indicated in Table II, application no. 1,columns 5 or 7, and conferring an increase in the amount of the fine chemical threonine in an organism or a part thereof; and
(l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical threonine in an organism or a part thereof;
or which comprises a sequence which is complementary thereto.

**[0104.0.1.1]** In one embodiment, the nucleic acid molecule of the invention distinguishes over the sequence indicated in Table I, application no. 1, columns 5 or 7, by one or more nucleotides. In one embodiment, the nucleic acid molecule of the present invention does not consist of the sequence shown in indicated in Table I, application no. 1, columns 5 or 7. In another embodiment, the nucleic acid molecule does not encode a polypeptide of a sequence indicated in Table I, application no. 1,columns 5 or 7.

**[0105.0.0.1] to [0107.0.0.1]: see [0105.0.0.0] to [0107.0.0.0]**

**[0108.0.1.1]** Nucleic acid molecules with the sequence as indicated in Table I, application no. 1, columns 5 or 7, nucleic acid molecules which are derived from an amino acid sequences as indicated in Table II, application no. 1, columns 5 or 7, or from polypeptides comprising the consensus sequence as indicated in Table IV, application no. 1,column 7, or their derivatives or homologues encoding polypeptides with the enzymatic or biological activity of a polypeptide as indicated in Table II application no. 1,, column 3, 5 or 7, or e.g. conferring a increase of the fine chemical threonine after increasing its expression or activity are advantageously increased in the process according to the invention.

**[0109.0.0.1] see [0109.0.0.0]**

**[0110.0.1.1]** Nucleic acid molecules, which are advantageous for the process according to the invention and which encode polypeptides with an activity of a polypeptide of the invention can be determined from generally accessible databases.

**[0111.0.0.1] see [0111.0.0.0]**

**[0112.0.1.1]** The nucleic acid molecules used in the process according to the invention take the form of isolated nucleic acid sequences, which encode polypeptides with an activity of a polypeptide as indicated in Table IIa or II, application no. 1, column 3, or having the sequence of a polypeptide as indicated in Table II, application no. 1, columns 5 and 7, and conferring an increase of the fine chemical threonine.

**[0113.0.0.1] to [0120.0.0.1]: see [0113.0.0.0] to [0120.0.0.0]**

**[0121.0.1.1]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 1, columns 5 or 7, or the functional homologues thereof as described herein, preferably conferring above-mentioned activity, i.e. conferring a increase of the fine chemical threonine after increasing its activity

**[0122.0.0.1] to [127.0.0.1]: see [0122.0.0.0] to [0127.0.0.0]**

**[0128.0.1.1]** Synthetic oligonucleotide primers for the amplification, e.g. as the pairs indicated in Table III, application no. 1, column 7, by means of polymerase chain reaction can be generated on the basis of a sequence as indicated in Table I, application no. 1, columns 5 or 7, or the sequences derived from sequences as indicated in Table II, application no. 1, columns 5 or 7.

**[0129.0.1.1]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequences indicated in Table IV, application no. 1, column 7, are derived from said aligments.

**[0130.0.1.1]** Degenerated primers can then be utilized by PCR for the amplification of fragments of novel proteins having above-mentioned activity, e.g. conferring the increase of the fine chemical after increasing its expression or activity or further functional homologs of the polypeptide of the invention from other organisms.

**[0131.0.0.1] to [0138.0.0.1]: see [0131.0.0.0] to [0138.0.0.0]**

**[0139.0.1.1]** Polypeptides having above-mentioned activity, i.e. conferring a threonine increase, derived from other organisms, can be encoded by other DNA sequences which hybridize to a sequences indicated in Table I, application no. 1, columns 5 or 7, under relaxed hybridization conditions and which code on expression for peptides having the threonine increasing activity.

**[0140.0.0.1] to [0146.0.0.1]: see [0140.0.0.0] to [0146.0.0.0]**

**[0147.0.1.1]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 1, columns 5 or 7, is one which is sufficiently complementary to one of said nucleotide sequences such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.1.1]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 1, columns 5 or 7, or a functional portion thereof and preferably has above mentioned activity, in particular has the-fine-chemical-increasing activity after increasing its acitivity or an activity of a product of a gene encoding said sequence or its homog's.

**[0149.0.1.1]** The nucleic acid molecule of the invention comprises a nucleotide sequence which hybridizes, preferably hybridizes under stringent conditions as defined herein, to one of the nucleotide sequences indicated in Table I, application no. 1, columns 5 or 7, or a portion thereof and encodes a protein having above-mentioned activity, e.g. conferring an increase of the fine chemical.

**[00149.1.1.1]** Optionally, the nucleotide sequence, which hybridises to one of the nucleotide sequences indicated in Table I, application no. 1, columns 5 or 7, has further one or more of the activities annotated or known for the a protein as indicated in Table II, application no. 1, column 3.

**[0150.0.1.1]** Moreover, the nucleic acid molecule of the invention can comprise only a portion of the coding region of one of the sequences indicated in Table I, application no. 1, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of fine chemical threonine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, application no. 1, columns 5 or 7, an anti-sense sequence of one of the sequences indicated in Table I, application no. 1, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 1, column 7, will result in a fragment of a polynucleotide sequence as indicated in Table I, application no. 1, columns 5 or 7.

**[01 51 .0.0.1] see [0151.0.0.0]**

**[0152.0.1.1]** The nucleic acid molecule of the invention encodes a polypeptide or portion thereof which includes an amino acid sequence which is sufficiently homologous to an amino acid sequence as indicated in Table II, application no. 1, columns 5 or 7such that the protein or portion thereof maintains the ability to participate in threonine production, in particular a threonine increasing activity as mentioned above or as described in the examples in plants or microorganisms is comprised.

**[0153.0.1.1]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 1, columns 5 or 7such that the protein or portion thereof is able to participate in the increase of threonine production. In one embodiment, a protein or portion thereof as indicated in Table II, application no. 1, columns 5 or 7, has for example an activity of a polypeptide indicated in Table II, application no. 1, column 3.

**[0154.0.1.1]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91 %, 92%, 93% or 94% and most preferably at least about 95%, 97%, 98%, 99% or more homologous to an entire amino acid sequence as indicated in Table II, application no. 1, columns 5 or 7, and has above-mentioned activity, e.g.conferring preferably the increase of the fine chemical.

**[0155.0.0.1] see [0155.0.0.0]**

**[0156.0.0.1] see [0156.0.0.0]**

**[0157.0.1.1]** The invention further relates to nucleic acid molecules that differ from one of a nucleotide sequences as indicated in Table I, application no. 1, columns 5 or 7, (and portions thereof) due to degeneracy of the genetic code and thus encode a polypeptide of the present invention, in particular a polypeptide having above mentioned activity, e.g. conferring an increase in threonine in an organism, e.g. as that polypeptides comprising the consensus sequences as indicated in Table IV, application no. 1, columns 7, or of the polypeptide as indicated in Table II, application no. 1, columns 5 or 7, or their functional homologues. Advantageously, the nucleic acid molecule of the invention comprises, or in an other embodiment has, a nucleotide sequence encoding a protein comprising, or in another embodiment having, a consensus sequences as indicated in Table IV, application no. 1, columns 7, or of the polypeptide as as indicated in Table II, application no. 1, columns 5 or 7, or the functional homologues. In a still further embodiment, the nucleic acid molecule of the invention encodes a full length protein which is substantially homologous to an amino acid sequence comprising a consensus sequence as indicated in Table IV, application no. 1, column 7, or of a polypeptide as indicated in Table II, application no. 1, columns 5 or 7, or the functional homologues thereof. However, in a preferred embodiment, the nucleic acid molecule of the present invention does not consist of a sequence as indicated in Table I, application no. 1, columns 5 or 7.

**[0158.0.0.1] to [0160.0.0.1]: see [0158.0.0.0] to [0160.0.0.0]**

**[0161.0.1.1]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 1, columns 5 or 7. The nucleic acid molecule is preferably at least 20, 30, 50, 100, 250 or more nucleotides in length.

**[0162.0.0.1]: see [0162.0.0.0]**

**[0163.0.1.1]** Preferably, nucleic acid molecule of the invention that hybridizes under stringent conditions to a sequence as indicated in Table I, application no. 1, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the fine chemical increase after increasing the expression or activity thereof or the activity of a protein of the invention or used in the process of the invention.

**[0164.0.0.1]: see [0164.0.0.0]**

**[0165.0.1.1]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7.

**[0166.0.0.1]: see [0166.0.0.0]**

**[0167.0.0.1] see [0167.0.0.0]**

**[0168.0.1.1]** Accordingly, the invention relates to nucleic acid molecules encoding a polypeptide having above-mentioned activity, e.g. conferring an increase in the fine chemical in an organisms or parts thereof that contain changes in amino acid residues that are not essential for said activity. Such polypeptides differ in amino acid sequence from a sequence contained in a sequence as indicated in Table II, application no. 1, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 1, columns 5 or 7, and is capable of participation in the increase of production of the fine chemical after increasing its activity, e.g. its expression. Preferably, the protein encoded by the nucleic acid molecule is at least about 60% identical to a sequence as indicated in Table II, application no. 1, columns 5 or 7, more preferably at least about 70% identical to one of the sequences as indicated in Table II, application no. 1, columns 5 or 7, even more preferably at least about 80%, 90% or 95% homologous to a sequence as indicated in Table II, application no. 1, columns 5 or 7, and most preferably at least about 96%, 97%, 98%, or 99% identical to the sequence as indicated in Table II, application no. 1, columns 5 or 7.

**[0169.0.0.1] to [0172.0.0.1]: see [0169.0.0.0] to [0172.0.0.0]**

**[0173.0.1.1]** For example a sequence which has a 80% homology with sequence SEQ ID NO: 268at the nucleic acid level is understood as meaning a sequence which, upon comparison with the sequence SEQ ID NO: 268by the above Gap program algorithm with the above parameter set, has a 80% homology.

**[0174.0.0.1]: see [0174.0.0.0]**

**[0175.0.1.1]** For example a sequence which has a 80% homology with sequence SEQ ID NO: 269 at the protein level is understood as meaning a sequence which, upon comparison with the sequence SEQ ID NO: 269 by the above program algorithm with the above parameter set, has a 80% homology.

**[0176.0.1.1]** Functional equivalents derived from one of the polypeptides as indicated in Table II, application no. 1, columns 5 or 7, according to the invention by substitution, insertion or deletion have at least least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 1, columns 5 or 7, according to the invention and are distinguished by essentially the same properties as a polypeptide as indicated in Table II, application no. 1, columns 5 or 7.

**[0177.0.1.1]** Functional equivalents derived from a nucleic acid sequence as indicated in Table I, application no. 1, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptides as indicated in Table II, application no. 1, columns 5 or 7, according to the invention and encode polypeptides having essentially the same properties as a polypeptide as indicated in Table II, application no. 1, columns 5 or 7.

**[0178.0.0.1] see [0178.0.0.0]**

**[0179.0.1.1]** A nucleic acid molecule encoding an homologous to a protein sequence of as indicated in Table II, application no. 1, columns 5 or 7, can be created by introducing one or more nucleotide substitutions, additions or deletions into a nucleotide sequence of the nucleic acid molecule of the present invention, in particular as indicated in Table I, application no. 1, columns 5 or 7, such that one or more amino acid substitutions, additions or deletions are introduced into the encoded protein. Mutations can be introduced into the encoding sequences of sequences as indicated in Table I, application no. 1, columns 5 or 7, by standard techniques, such as site-directed mutagenesis and PCR-mediated mutagenesis.

**[0180.0.0.1] to [0183.0.0.1]: see [0180.0.0.0] to [0183.0.0.0]**

**[0184.0.1.1]** Homologues of the nucleic acid sequences used, with a sequence as indicated in Table I, application no. 1, columns 5 or 7, or of the nucleic acid sequences derived from a sequences as indicated in Table II, application no. 1, columns 5 or 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 1, columns 5 or 7, or from the derived nucleic acid sequences, the intention being, however, that the enzyme activity or the biological activity of the resulting proteins synthesized is advantageously retained or increased.

**[0185.0.1.1]** In one embodiment of the present invention, the nucleic acid molecule of the invention or used in the process of the invention comprises one more sequence as indicated in Table I, application no. 1, columns 5 or 7. In one embodiment it is preferred that the nucleic acid molecule comprises as little as possible other nucleotide sequences not shown in any one of sequences as indicated in Table I, application no. 1, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule use in the process of the invention is identical to a sequence as indicated in Table I, application no. 1, columns 5 or 7.

**[0186.0.1.1]** Also preferred is that one or more nucleic acid molecule(s) used in the process of the invention encodes a polypeptide comprising a sequence as indicated in Table II, application no. 1, columns 5 or 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polynucleotide used in the process of the invention is identical to the sequences as indicated in Table II, application no. 1, columns 5 or 7.

**[0187.0.1.1]** In one embodiment, the nucleic acid molecule of the invention or used in the process encodes a polypeptide comprising a sequence as indicated in Table II, application no. 1, columns 5 or 7, comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 1, columns 5 or 7.

**[0188.0.1.1]** Polypeptides (= proteins), which still have the essential enzymatic activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 1, columns 5 or 7, preferably compared to a sequence as indicated in Table II, application no. 1, column 5, and expressed under identical conditions.

**[0189.0.1.1]** Homologues of sequences as indicated in Table I, application no. 1, columns 5 or 7, or of derived sequences as indicated in Table II, application no. 1, columns 5 or 7, also mean truncated sequences, cDNA, single-stranded DNA or RNA of the coding and noncoding DNA sequence. Homologues of said sequences are also understood as meaning derivatives which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.1] to [0203.0.0.1]: see [0190.0.0.0] to [0203.0.0.0]**

**[0204.0.1.1]** Accordingly, in one embodiment, the invention relates to a nucleic acid molecule which comprises a nucleic acid molecule selected from the group consisting of:
(a) nucleic acid molecule encoding, preferably at least the mature form, of a polypeptide as indicated in Table II, application no. 1, columns 5 or 7, or a fragment thereof conferring an increase in the amount of the respective fine chemical threonine in an organism or a part thereof
(b) nucleic acid molecule comprising, preferably at least the mature form, of a nucleic acid molecule as indicated in Table I, application no. 1, columns 5 or 7, or a fragment thereof conferring an increase in the amount of the respective fine chemical threonine in an organism or a part thereof;
(c) nucleic acid molecule whose sequence can be deduced from a polypeptide sequence encoded by a nucleic acid molecule of (a) or (b) as result of the degeneracy of the genetic code and conferring an increase in the amount of the respective fine chemical threonine in an organism or a part thereof;
(d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical threonine in an organism or a part thereof;
(e) nucleic acid molecule which hybridizes with a nucleic acid molecule of (a) to (c) under under stringent hybridisation conditions and conferring an increase in the amount of the respective fine chemical threonine in an organism or a part thereof;
(f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical threonine in an organism or a part thereof;
(g) nucleic acid molecule encoding a fragment or an epitope of a polypeptide which is encoded by one of the nucleic acid molecules of (a) to (e), preferably to (a) to (c) and conferring an increase in the amount of the respective fine chemical threonine in an organism or a part thereof ;
(h) nucleic acid molecule comprising a nucleic acid molecule which is obtained by amplifying a cDNA library or a genomic library using primers or primer pairs as indicated in Table III, application no. 1, column 7, and conferring an increase in the amount of the respective fine chemical threonine in an organism or a part thereof;
(i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical threonine in an organism or a part thereof;
(j) nucleic acid molecule which encodes a polypeptide comprising the consensus sequence as indicated in Table IV, application no. 1, column 7, and conferring an increase in the amount of the respective fine chemical threonine in an organism or a part thereof;
(k) nucleic acid molecule encoding the amino acid sequence of a polypeptide encoding a domaine of a polypeptide as indicated in Table II, application no. 1, columns 5 or 7, and conferring an increase in the amount of the respective fine chemical threonine in an organism or a part thereof; and
(l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 1, columns 5 or 7, or a nucleic acid molecule encoding, preferably at least the mature form of, the polypeptide as indicated in Table II, application no. 1, columns 5 or 7, and conferring an increase in the amount of the respective fine chemical threonine in an organism or a part thereof;
or which encompasses a sequence which is complementary thereto;
whereby, preferably, the nucleic acid molecule according to (a) to (I) distinguishes over a sequence depicted in as indicated in Table I, columns 5 or 7, by one or more nucleotides. In one embodiment, the nucleic acid molecule of the invention does not consist of a sequence as indicated in Table I, application no. 1, columns 5 or 7. In one embodiment, the nucleic acid molecule is less than 100%, 99,999%, 99,99%, 99,9% or 99% identical to a sequence indicated in Table I, application no. 1, columns 5 or 7. In another embodiment, the nucleic acid molecule does not encode a polypeptide of a sequence indicated in Table II, application no. 1, columns 5 or 7. In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 1, columns 5 or 7. In a further embodiment the nucleic acid molecule does not encode a polypeptide sequence as indicated in Table II application no. 1,, columns 5 or 7. Accordingly, in one embodiment, the nucleic acid molecule of the differs at least in one or more residues from a nucleic acid molecule indicated in Table I, application no. 1, columns 5 or 7. Accordingly, in one embodiment, the nucleic acid molecule of the present invention encodes a polypeptide, which differs at least in one or more amino acids from a polypeptide indicated in Table II, columns 5 or 7. In another embodiment, a nucleic acid molecule indicated in Table I, columns 5 or 7, does not encode a protein of a sequence indicated in Table II, application no. 1, columns 5 or 7. Accordingly, in one embodiment, the protein encoded by a sequences of a nucleic acid accoriding to (a) to (I) does not consist of a sequence as indicated in Table II, application no. 1, columns 5 or 7. In a further embodiment, the protein of the present invention is at least 30 %, 40 %, 50%, or 60% identical to a protein sequence indicated in Table II, application no. 1, columns 5 or 7, and less than 100%, preferably less than 99,999%, 99,99% or 99,9%, more preferably less than 99%, 985, 97%, 96% or 95% identical to a sequence as indicated in Table I, application no. 1, columns 5 or 7.

**[0205.0.0.1] to [0226.0.0.1]: see [0205.0.0.0] to [0226.0.0.0]**

**[0227.0.1.1]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence as indicated in Table I, application no. 1, columns 5 or 7, or its derivatives, it is advantageous additionally to express and/or mutate further genes in the organisms. Especially advantageously, additionally at least one further gene of the amino acid biosynthetic pathway such as for L-lysine, L-methionine and/or L-threonine is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acid desired since, for example, feedback regulations no longer exist to the same extent or not at all. In addition it might be advantageously to combine sequences as indicated in Table I, application no. 1, columns 5 or 7, with genes which generally support or enhances to growth or yield of the target organismen, for example genes which lead to faster growth rate of microorganisms or genes which produces stress-, pathogen, or herbicide resistant plants.

**[0228.0.0.1] to [0230.0.0.1]: see [0228.0.0.0] to [0230.0.0.0]**

**[0231.0.1.1]** In a further advantageous embodiment of the process of the invention, the organisms used in the process are those in which simultaneously a threonine degrading protein is attenuated, in particular by reducing the rate of expression of the corresponding gene.

**[0232.0.0.1] to [0282.0.0.1]: see [0232.0.0.0] to [0282.0.0.0]**

**[0283.0.1.1]** Moreover, a native polypeptide conferring the increase of the fine chemical threonine in an organism or part thereof can be isolated from cells (e.g., endothelial cells), for example using the antibody of the present invention as described below, in particular, an antibody against a protein as indicated in Table II, application no. 1, column 3. E.g. an antibody against a polypeptide as indicated in Table II, application no. 1, columns 5 or 7, which can be produced by standard techniques utilizing polypeptides comprising or consisting of above mentioned sequences, e.g. the polypeptid of the present invention or fragment thereof. Preferred are monoclonal antibodies.

**[0284.0.0.1]: see [0284.0.0.0]**

**[0285.0.1.1]** In one embodiment, the present invention relates to a polypeptide having a sequence as indicated in Table II, application no. 1, columns 5 or 7, or as coded by a nucleic acid molecule as indicated in Table II, application no. 1, columns 5 or 7, or functional homologues thereof.

**[0286.0.1.1]** In one advantageous embodiment, in the method of the present invention the activity of a polypeptide is increased which comprises or consists of a consensus sequence as indicated in Table IV, application no. 1, column 7and in one other embodiment, the present invention relates to a polypeptide comprising or consisting of a consensus sequence as indicated in Table IV, application no. 1, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid.

**[0287.0.0.1] to [0290.0.0.1]: see [0287.0.0.0] to [0290.0.0.0]**

**[0291.0.1.1]** In one advantageous embodiment, the method of the present invention comprises the increasing of a polypeptide comprising or consisting of plant or microorganism specific consensus sequences.
Accordingly, in one embodiment, the present invention relates to a polypeptide comprising or consisting of plant or microorganism specific consensus sequences.
In one embodiment, said polypeptide of the invention distinguishes over a sequence as indicated in Table II, application no. 1, columns 5 or 7, by one or more amino acids. In one embodiment, the polypeptide distinguishes from a sequence as indicated in Table II, application no. 1, columns 5 or 7, by more than1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 1, columns 5 or 7, by not more than 80% or 70% of the amino acids, preferably not more than 60% or 50%, more preferred not more than 40% or 30%, even more preferred not more than 20% or 10%. In another embodiment, said polypeptide of the invention does not consist of a sequence as indicated in Table II, application no. 1, columns 5 or 7.

**[0292.0.0.1]: see [0292.0.0.0]**

**[0293.0.1.1]** In one embodiment, the invention relates to a polypeptide conferring an increase in the fine chemical threonine in an organism or part being encoded by the nucleic acid molecule of the invention or by the nucleic acid molecule of the invention used in the process of the invention.
In one embodiment, the poylpeptide of the invention is having a sequence which distinguishes from a sequence as indicated in Table II, application no. 1, columns 5 or 7, by one or more amino acids. In another embodiment, said polypeptide of the invention does not consist of the sequence as indicated in Table II, application no. 1, columns 5 or 7. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 1, columns 5 or 7.

**[0294.0.1.1]** In one embodiment, the present invention relates to a polypeptide having an activity of a protein as indicated in Table II, application no. 1, column 3, which distinguishes over a sequence as indicated in Table II, application no. 1, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.1] to [0297.0.0.1]: see [0295.0.0.0] to [0297.0.0.0]**

**[0297.1.0.1]** Non-polypeptide of the invention-chemicals are e.g. polypeptides having not the activity of a polypeptide indicated in Table II, application no. 1, columns 3, 5 or 7.

**[0298.0.1.1]** A polypeptide of the invention can participate in the process of the present invention. The polypeptide or a portion thereof comprises preferably an amino acid sequence which is sufficiently homologous to an amino acid sequence as indicated in Table II, application no. 1, columns 5 or 7, such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, application no. 1, columns 5 or 7.

**[0299.0.1.1]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, application no. 1, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 1, columns 5 or 7, or which is homologous thereto, as defined above.

**[0300.0.1.1]** Accordingly the polypeptide of the present invention can vary from a sequence as indicated in Table II, application no. 1, columns 5 or 7, in the amino acid sequence due to natural variation or mutagenesis, as described in detail herein. Accordingly, the polypeptide comprises an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II application no. 1 columns 5 or 7.

**[0301.0.0.1] see [0301.0.0.0]**

**[0302.0.1.1]** Biologically active portions of a polypeptide of the present invention include peptides comprising amino acid sequences derived from the amino acid sequence of the polypeptide of the present invention or used in the process of the present invention, e.g., an amino acid sequence as indicated in Table II, application no. 1, columns 5 or 7, or the amino acid sequence of a protein homologous thereto, which include fewer amino acids than a full length polypeptide of the present invention or used in the process of the present invention or the full length protein which is homologous to a polypeptide of the present invention or used in the process of the present invention depicted herein, and exhibit at least one activity of a polypeptide of the present invention or used in the process of the present invention.

**[0303.0.0.1]: see [0303.0.0.0]**

**[0304.0.1.1]** Manipulation of the nucleic acid molecule of the invention may result in the production of a protein having essentially the activity of the polypeptides as indicated in Table II, application no. 1, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.1] to [0308.0.0.1]: see [0305.0.0.0] to [0308.0.0.0]**

**[0309.0.1.1]** In one embodiment, an reference to a "protein (= polypeptide)" of the invention or as indicated in Table II, application no. 1, columns 5 or 7, refers to a polypeptide having an amino acid sequence corresponding to the polypeptide of the invention or used in the process of the invention, whereas a "non- polypeptide of the invention" or "other polypeptide" not being indicated in Table II, application no. 1, columns 5 or 7, refers to a polypeptide having an amino acid sequence corresponding to a protein which is not substantially homologous to a polypeptide of the invention, preferably which is not substantially homologous to a polypeptide as indicated in Table II, application no. 1, columns 5 or 7, e.g., a protein which does not confer the activity described herein or annotated or known for as indicated in Table II, application no. 1, column 3, and which is derived from the same or a different organism. In one embodiment a "non-polypeptide of the invention" or "other polypeptide" not being indicate in Table II, application no. 1, columns 5 or 7, does not confer an increase of the fine chemical in an organism or part thereof.

**[0310.0.0.1] to [0334.0.0.1]: see [0310.0.0.0] to [0334.0.0.0]**

**[0335.0.1.1]** The dsRNAi method has proved to be particularly effective and advantageous for reducing the expression of a nucleic acid sequences as indicated in Table II, application no. 1, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, application no. 1, columns 5 or 7, and/or homologs thereof. In a double-stranded RNA molecule for reducing the expression of an protein encoded by a nucleic acid sequence of one of the sequences as indicated in Table II, application no. 1, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.1] to [0342.0.0.1]: see [0336.0.0.0] to [0342.0.0.0]**

**[0343.0.1.1]** As has already been described, 100 % sequence identity between the dsRNA and a gene transcript of a nucleic acid sequence as indicated in Table I, application no. 1, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 1, columns 5 or 7, or homologs thereof of the one organism, may be used to suppress the corresponding expression in another organism.

**[0344.0.0.1] to [0361.0.0.1]: see [0344.0.0.0] to [0361.0.0.0]**

**[0362.0.1.1]** Accordingly the present invention relates to any cell transgenic for any nucleic acid characterized as part of the invention, e.g. conferring the increase of the fine chemical threonine in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 1, columns 5 or 7. Due to the above mentioned activity the fine chemical content in a cell or an organism is increased. For example, due to modulation or manupulation, the cellular activity of the polypeptide of the invention or nucleic acid molecule of the invention is increased, e.g. due to an increased expression or specific activity of the subject matters of the invention in a cell or an organism or a part thereof. In one embodiment transgenic for a polypeptide having an activity of a polypeptide as indicated in Table II, application no. 1, columns 5 or 7, means herein that due to modulation or manipulation of the genome, an activity as annotated for a polypeptide as indicated in Table II, application no. 1, columns 3, e.g. having a sequence as indicated in Table II, application no. 1, columns 5 or 7, is increased in a cell or an organism or a part thereof. Examples are described above in context with the process of the invention.

**[0363.0.0.1]: see [0363.0.0.0]**

**[0364.0.1.1]** A naturally occurring expression cassette - for example the naturally occurring combination of a promoter of a polypeptide of the invention with the corresponding protein-encoding-sequence - becomes a transgenic expression cassette when it is modified by non-natural, synthetic "artificial" methods such as, for example, mutagenization. Such methods have been described (US 5,565,350; WO 00/15815; also see above).

**[0365.0.0.1] to [0382.0.0.1]: see [0365.0.0.0] to [0382.0.0.0]**

**[0383.0.1.1]** For preparing hydroxy containing fine chemicals, in particular the fine chemical threonine, it is possible to use as hydroxy source organic hydroxy-containing compounds such as, for example, alcohols, hydroxy-containing organic acids, acetals, or compounds containing carbonyl or carboxyl groups to be reduced by known methods of the art.

**[0384.0.0.1] to [0392.0.0.1]: see [0384.0.0.0] to [0392.0.0.0]**

**[0393.0.1.1]** In one embodiment, the present invention relates to a method for the identification of a gene product conferring an increase in the fine chemical production in a cell, comprising the following steps:
(a) contacting-, e.g. hybridising, the nucleic acid molecules of a sample, e.g. cells, tissues, plants or microorganisms or a nucleic acid library, which can contain a candidate gene encoding a gene product conferring an increase in the fine chemical threonine after expression, with the nucleic acid molecule of the present invention;
(b) identifying the nucleic acid molecules, which hybridize under relaxed stringent conditions with the nucleic acid molecule of the present invention in particular to the nucleic acid molecule sequence as indicated in Table I, application no. 1, columns 5 or 7, and, optionally, isolating the full length cDNA clone or complete genomic clone;
(c) introducing the candidate nucleic acid molecules in host cells, preferably in a plant cell or a microorganism, appropriate for producing the fine chemical threonine;
(d) expressing the identified nucleic acid molecules in the host cells;
(e) assaying the the fine chemical level in the host cells; and
(f) identifying the nucleic acid molecule and its gene product which expression confers an increase in the the fine chemical level in the host cell after expression compared to the wild type.

**[0394.0.0.1] to [0399.0.0.1]: see [0394.0.0.0] to [0399.0.0.0]**

**[0399.1.1.1]** One can think to screen for increased production of the fine chemical threonine by for example searching for a resistance to a drug blocking the synthesis of the fine chemical threonine and looking whether this effect is dependent on the activity or expression of a polypeptide as indicated in Table II, application no. 1, columns 5 or 7, or a homolog thereof, e.g. comparing the phenotyp of nearly identical organisms with low and high acitivity of a protein as indicated in Table II, application no. 1, columns 5 or 7, after incubation with the drug.

**[0400.0.0.1] to [0430.0.0.1]: see [0400.0.0.1] to [0430.0.0.0]**

**[0431.0.1.1]** Example 1: Cloning SEQ ID NO: 268 or another DNA polynucleotide according the enclosed sequence listing encoding an ORF as shown in the below table in Escherichia coli

**[0432.0.1.1]** SEQ ID NO: 268 or another DNA polynucleotide according the enclosed sequence listing encoding an ORF as shown in the below table was cloned into the plasmids pBR322 (Sutcliffe, J.G. (1979) Proc. Natl Acad. Sci. USA, 75: 3737-3741); pACYC177 (Change & Cohen (1978) J. Bacteriol. 134: 1141-1156); plasmids of the pBS series (pBSSK+, pBSSK- and others; Stratagene, LaJolla, USA) or cosmids such as SuperCos1 (Stratagene, LaJolla, USA) or Lorist6 (Gibson, T.J. Rosenthal, A., and Waterson, R.H. (1987) Gene 53: 283-286) for expression in E. coli using known, well-established procedures (see, for example, Sambrook, J. et al. (1989) "Molecular Cloning: A Laboratory Manual". Cold Spring Harbor Laboratory Press or Ausubel, F.M. et al. (1994) "Current Protocols in Molecular Biology", John Wiley & Sons).

**[0433.0.0.1] to [0460.0.0.1]: see [0433.0.0.0] to [0460.0.0.0]**

**[0461.0.1.1]** Example 10: Cloning SEQ ID NO: 268 for the expression in plants

**[0462.0.0.1]: see [0462.0.0.0]**

**[0463.0.1.1]** SEQ ID NO: 268 is amplified by PCR as described in the protocol of the Pfu Turbo or DNA Herculase polymerase (Stratagene).

**[0464.0.0.1] to [0466.0.0.1]: see [0464.0.0.0] to [0466.0.0.0]**

**[0467.0.1.1]** The following primer sequences were selected for the gene SEQ ID NO: 81:
i) forward primer SEQ ID NO: 274 :
ii) reverse primer SEQ ID NO: 275 :

**[0468.0.0.1] to [0479.0.0.1]: see [0468.0.0.0] to [0479.0.0.0]**

**[0480.0.1.1]** Example 11: Generation of transgenic plants which express SEQ ID NO: 81

**[0481.0.0.1] to [0513.0.0.1]: see [0481.0.0.0] to [0513.0.0.0]**

[0514.0.1.1] As an alternative, the amino acids can be detected advantageously via HPLC separation in ethanolic extract as described by Geigenberger et al. (Plant Cell & Environ, 19, 1996: 43-55).
*Arabidopsis thaliana* plants were engineered as described in Example 11.

**[0515.0.0.1]: to [0552.0.0.1]: see [0515.0.0.0] to [0552.0.0.0]**

**[0552.1.1.1]:** ./.

**[0553.0.1.1]** A process for the production of threonine, which comprises
(a) increasing or generating the activity of a protein as indicated in Table II, application no. 1, columns 5 or 7, or a functional equivalent thereof in a non-human organism, or in one or more parts thereof; and
(b) growing the organism under conditions which permit the production of threonine in said organism.
A process for the production of threonine, comprising the increasing or generating in an organism or a part thereof the expression of at least one nucleic acid molecule comprising a nucleic acid molecule selected from the group consisting of:
(a) nucleic acid molecule encoding of a polypeptide as indicated in Table II, application no. 1, columns 5 or 7, or a fragment thereof, which confers an increase in the amount of threonine in an organism or a part thereof;
(b) nucleic acid molecule comprising of the nucleic acid molecule as indicated in Table I, application no. 1, columns 5 or 7;
(c) nucleic acid molecule whose sequence can be deduced from a polypeptide sequence encoded by a nucleic acid molecule of (a) or (b) as a result of the degeneracy of the genetic code and conferring an increase in the amount of threonine in an organism or a part thereof;
(d) nucleic acid molecule which encodes a polypeptide which has at least 50% identity with the amino acid sequence of the polypeptide encoded by the nucleic acid molecule of (a) to (c) and conferring an increase in the amount of threonine in an organism or a part thereof;
(e) nucleic acid molecule which hybidizes with a nucleic acid molecule of (a) to (c) under under stringent hybridisation conditions and conferring an increase in the amount of threonine in an organism or a part thereof;
(f) nucleic acid molecule which encompasses a nucleic acid molecule which is obtained by amplifying nucleic acid molecules from a cDNA library or a genomic library using the primers or primer pairs as indicated in Table III, columns 5 or 7, lines 6 to 15, 339 to 355 and conferring an increase in the amount of the fine chemical threonine in an organism or a part thereof;
(g) nucleic acid molecule encoding a polypeptide which is isolated with the aid of monoclonal antibodies against a polypeptide encoded by one of the nucleic acid molecules of (a) to (f) and conferring an increase in the amount of threonine in an organism or a part thereof;
(h) nucleic acid molecule encoding a polypeptide comprising a consensus sequence as indicated in Table IV, application no. 1, columns 5 or 7, and conferring an increase in the amount of the fine chemical threonine in an organism or a part thereof; and
(i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical threonine in an organism or a part thereof.
or comprising a sequence which is complementary thereto.
The process as mentioned above, comprising recovering of the free or bound threonine.
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound threonine produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
(a) nucleic acid molecule encoding a polypeptide as indicated in Table II, application no. 1, columns 5 or 7, or a fragment thereof, which confers an increase in the amount of threonine in an organism or a part thereof;
(b) nucleic acid molecule comprising a nucleic acid as indicated in Table I, application no. 1, columns 5 or 7;
(c) nucleic acid molecule whose sequence can be deduced from a polypeptide sequence encoded by a nucleic acid molecule of (a) or (b) as a result of the degeneracy of the genetic code and conferring an increase in the amount of threonine in an organism or a part thereof;
(d) nucleic acid molecule which encodes a polypeptide which has at least 50% identity with the amino acid sequence of the polypeptide encoded by the nucleic acid molecule of (a) to (c) and conferring an increase in the amount of threonine in an organism or a part thereof;
(e) nucleic acid molecule which hybidizes with a nucleic acid molecule of (a) to (c) under under stringent hybridisation conditions and conferring an increase in the amount of threonine in an organism or a part thereof;
(f) nucleic acid molecule which encompasses a nucleic acid molecule which is obtained by amplifying nucleic acid molecules from a cDNA library or a genomic library using the primers in Table III, application no. 1, column 8, and conferring an increase in the amount of the fine chemical threonine in an organism or a part thereof;
(g) nucleic acid molecule encoding a polypeptide which is isolated with the aid of monoclonal antibodies against a polypeptide encoded by one of the nucleic acid molecules of (a) to (f) and conferring an increase in the amount of threonine in an organism or a part thereof;
(h) nucleic acid molecule encoding a polypeptide comprising the consensus sequence shown in Table IV, application no. 1, column 8, and conferring an increase in the amount of the fine chemical in an organism or a part thereof; and
(i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
whereby the nucleic acid molecule distinguishes over the sequence as indicated in Table I, application no. 1, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a Sequence as indicated in Table II, application no. 1, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of threonine in an organism or a part thereof comprising:
(a) contacting cells, tissues , plants or microorganisms which express the a polypeptide encoded by the nucleic acid molecule as mentioned above conferring an increase in the amount of threonine in an organism or a part thereof with a candidate compound or a sample comprising a plurality of compounds under conditions which permit the expression the polypeptide;
(b) assaying the threonine level or the polypeptide expression level in the cell, tissue, plant or microorganism or the media the cell, tissue, plant or microorganisms is cultured or maintained in; and
(c) identifying a agonist or antagonist by comparing the measured threonine level or polypeptide expression level with a standard threonine or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased threonine production in a plant or microorganism, comprising the steps:
(a) culturing a plant cell or tissue or microorganism or maintaining a plant expressing the polypeptide encoded by the nucleic acid molecule as mentioned above conferring an increase in the amount of threonine in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of threonine in an organism or a part thereof;
(b) identifying if the compound is an effective agonist by detecting the presence or absence or increase of a signal produced by said readout system.
A method for the identification of a gene product conferring an increase in threonine production in a cell, comprising the following steps:
(a) contacting the nucleic acid molecules of a sample, which can contain a candidate gene encoding a gene product conferring an increase in threonine after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing threonine;
(d) expressing the identified nucleic acid molecules in the host cells;
(e) assaying the threonine level in the host cells; and
(f) identifying nucleic acid molecule and its gene product which expression confers an increase in the threonine level in the host cell after expression compared to the wild type.
A method for the identification of a gene product conferring an increase in threonine production in a cell, comprising the following steps:
(a) identifiying in a data bank nucleic acid molecules of an organism; which can contain a candidate gene encoding a gene product conferring an increase in the threonine amount or level in an organism or a part thereof after expression, and which are at least 20% homolog to the nucleic acid molecule as mentioned above;
(b) introducing the candidate nucleic acid molecules in host cells appropriate for producing threonine;
(c) expressing the identified nucleic acid molecules in the host cells;
(d) assaying the threonine level in the host cells; and
(e) identifying the nucleic acid molecule and its gene product which expression confers an increase in the threonine level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of threonine after expression.
Use of the polypeptide as mentioned above or the nucleic acid construct as mentioned above or the gene product identified according to the method as mentioned above for identifying compounds capable of conferring a modulation of threonine levels in an organism.
Food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.

### New genes for a process for the production of fine chemicals

**[0000.0.0.2]:** In a further embodiment, the present invention relates to a further process for the production of fine chemicals as defined below and corresponding embodiments as described herein as follows.

**[0001.0.0.2] to [0009.0.0.2]: see [0.0.01.0.0.0] to [0008.0.0.0]**

**[0009.0.2.2]** As described above, the essential amino acids are necessary for humans and many mammals, for example for livestock. Tryptophane (L-tryptophane) is one of the most reactive amino acids. At pH 4.0-6.0 tryptophane amino group reacts with aldehydes producing Schiff-bases. On the other hand if the amino group is blocked by acetylation, tryptophane reacts with aldehydes yielding carboline derivatives (carboline 1,2,3,4-tetrahydro-carboline-3-carboxylic acid). Tryptophane plays a unique role in defense against infection because of its relative scarcity compared to other amino acids. During infection, the body induces tryptophane-catabolizing enzymes which increase tryptophane's scarcity in an attempt to starve the infecting organisms [R. R. Brown, Y. Ozaki, S. P. Datta, et al., Implications of interferon-induced tryptophane catabolism in cancer, auto-immune diseases and AIDS. In: Kynurenine and Serotonin Pathways, R. Schwarcz, et al., (Eds.), Plenum Press, New York, 1991]. In most proteins, tryptophane is the least abundant essential amino acid, comprising approximately 1 % of plant proteins and 1.5% of animal proteins. Although the minimum daily requirement for tryptophane is 160 mg for women and 250 mg for men, 500-700 mg are recommended to ensure high-quality protein intake. Actual tryptophane utilization is substantially higher. Men use approximately 3.5 grams of tryptophane to make one days's worth of protein [J. C. Peters, Tryptophane Nutrition and Metabolism: an Overview. In: Kynurenine and Serotonin Pathways, R. Schwarcz, et al., (Eds.), Plenum Press, New York, 1991]. The balance is obtained by hepatic recycling of tryptophane from used (catabolized) proteins.
Dietary tryptophane is well absorbed intestinally. About 10% of the tryptophane circulating in the bloodstream is free, and 90% is bound to the protein albumin. The tryptophane binding site on albumin also has affinity for free fatty acids (FFAs), so tryptophane is displaced when FFAs rise, as when fasting.
Although tryptophane is not usually the limiting amino acid in protein synthesis, tryptophane may become insufficient for the normal functioning of other tryptophane-dependent pathways. Numerous lines of research point to tryptophane's central role in regulation of feeding and other behaviors. Tryptophane is not only typically the least abundant amino acid in the liver's free amino acid pool, but liver tryptophane-tRNA levels fall faster during food deprivation than other indispensable amino acids [ Q. R. Rogers, The nutritional and metabolic effects of amino acid imbalances. In: Protein Metabolism and Nutrition, D. J. A. Cole (Ed.), Butterworths, London, 1976]. Under fasting conditions, and possibly in wasting syndromes, tryptophane may become the rate-limiting amino acid for protein synthesis [Peters, 1991].

**[0010.0.0.2] and [0011.0.0.2]: see [0010.0.0.0] and [0011.0.0.0]**

**[0012.0.2.2]** It is an object of the present invention to develop an inexpensive process for the synthesis of tryptophane, preferably L-tryptophane. Tryptophane is together with methionine, lysine and threonine (depending on the organism) one of the amino acids which are most frequently limiting.

**[0013.0.0.2]: see [0013.0.0.0]**

**[0014.0.2.2]** Accordingly, in a first embodiment, the invention relates to a process for the production of a fine chemical, whereby the fine chemical is tryptophane, preferably L-tryptophane. Accordingly, in the present invention, the term "the fine chemical" as used herein relates to "tryptophane". Further, the term "the fine chemicals" as used herein also relates to fine chemicals comprising tryptophane.

**[0015.0.2.2]** In one embodiment, the term "the fine chemical" means tryptophane, preferably L-tryptophane. Throughout the specification the term "the fine chemical" means tryptophane, preferably L-tryptophane, its salts, ester or amids in free form or bound to proteins. In a preferred embodiment, the term "the fine chemical" means tryptophane, preferably L-tryptophane, in free form or its salts or bound to proteins.
In one embodiment, the term "the fine chemical" and the term "the respective fine chemical" mean at least one chemical compound with an activity of the above mentioned fine chemical.

**[0016.0.2.2]** Accordingly, the present invention relates to a process for the production of the respective fine chemical comprising
(a) increasing or generating the activity of one or more of a protein as shown in table II, application no. 2, column 3 encoded by the nucleic acid sequences as shown in table I, application no. 2, column 5, in a non-human organism or in one or more parts thereof or
(b) growing the organism under conditions which permit the production of the fine chemical, thus amino acid of the invention or fine chemicals comprising amino acids of the invention, in said organism or in the culture medium surrounding the organism.

**[0017.0.0.2] to [0019.0.0.2]: see [0017.0.0.0] and [0019.0.0.0]**

[0020.0.2.2] Surprisingly it was found, that the transgenic expression of the Zea mays protein as indicated in Table II, application no 2, column 5, line 5 in a plant conferred an increase in tryptophane content of the transformed plants. Thus, in one embodiment, said protein or its homologs are used for the production of tryptophane.

**[0021.0.0.2]: see [0021.0.0.0]**

**[0022.0.2.2]** The sequence of b3983 from Escherichia coli K12 has been published in Blattner et al., Science 277(5331), 1453-1474, 1997, and its activity is being defined as a 50S ribosomal subunit protein L12. Accordingly, in one embodiment, the process of the present invention comprises the use of a gene product with the activity of the Escherichia coli ribosomal protein L11 superfamily, preferably a protein with a 50S ribosomal subunit protein L12 activity from E. coli or a plant or its homolog, e.g. as shown herein, for the production of the fine chemical, meaning of tryptophane, in particular for increasing the amount of tryptophane in free or bound form in an organism or a part thereof, as mentioned.

**[0022.1.0.2]: to [0023.0.0.2]: see [0022.1.0.0] to [0023.0.0.0]**

**[0023.1.2.2]** Homologs of the polypeptide disclosed in table II, application no. 2, column 5, may have the activity of a protein as disclosed in table I or II, application no. 2, column 3 and may be the polypetides encoded by the nucleic acid molecules indicated in table I , application no. 2, column 7, resp., or may be the polypeptides indicated in table II, application no. 2, column 7, resp.

**[0024.0.0.2]: see [0024.0.0.0]**

**[0025.0.2.2]** In accordance with the invention, a protein or polypeptide has the "activity of an protein as shown in table II, application no. 2, column 3 "if its de *novo* activity, or its increased expression directly or indirectly leads to an increased in the level of the fine chemical indicated in the respective line of table II, application no. 2, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 2, column 3, or which has at least 10% of the original enzymatic or biological activity, preferably 20%, particularly preferably 30%, most particularly preferably 40% in comparison to a protein as shown in the respective line of table II, application no. 2, column 3 of a E. coli or Saccharomyces cerevisae, respectively, protein as mentioned in table I to IV, column 3 respectively and as disclosed in paragraph [0022] of the respective invention.

**[0025.1.0.2]: see [0025.1.0.0]**

**[0025.2.0.2]: see [0025.2.0.0]**

**[0026.0.0.2] to [0033.0.0.2]: see [0026.0.0.0] to [0033.0.0.0]**

**[0034.0.2.2]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 2, column 3 its biochemical or genetical causes and the increased amount of the respective fine chemical.

**[0035.0.0.2] to [0044.0.0.2]: see [0035.0.0.0] to [0044.0.0.0]**

**[0045.0.2.2]** In one embodiment, the activity of the protein. as indicated in Table II, columns 5 or 7, application no. 2, is increased conferring an increase of the respective fine chemical, indicated in Table II, application no. 2, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more

**[0046.0.2.2]** In one embodiment, the activity of the protein as indicated in Table II, columns 5 or 7, application no. 2, is increased conferring an increase of the respective fine chemical, indicated in Table II, application no. 2, column 6 of the respective line confers an increase of the respective fine chemical and of further amino acid or their precursors.

**[0047.0.0.2] and [0048.0.0.2]: see [0047.0.0.0] and [0048.0.0.0]**

**[0049.0.2.2]** A protein having an activity conferring an increase in the amount or level of the fine chemical, has in one embodiment the structure of the polypeptide described herein, in particular of the polypeptides comprising the consensus sequence shown in table IV, application no. 2, column 7 or of the polypeptide as shown in the amino acid sequences as disclosed in table II, application no. 2, columns 5 and 7 or the functional homologues thereof as described herein, or is encoded by the nucleic acid molecule characterized herein or the nucleic acid molecule according to the invention, for example by the nucleic acid molecule as shown in table I, application no. 2, columns 5 and 7 or its herein described functional homologues and has the herein mentioned activity.

**[0050.0.2.2]** For the purposes of the present invention, the term "tryptophane" and "L-tryptophane" also encompass the corresponding salts, such as, for example, tryptophane hydrochloride or tryptophane sulfate. Preferably the term tryptophane is intended to encompass the term L-tryptophane.

**[0051.0.0.2] and [0052.0.0.2]: see [0051.0.0.0] and [0052.0.0.0]**

**[0053.0.2.2]** In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 2, columns 5 and 7 or its homologs activity having herein-mentioned amino acid of the invention increasing activity; and/or
b) stabilizing a mRNA conferring the increased expression of a protein encoded by the nucleic acid molecule of the invention as shown in table I, application no. 2, columns 5 and 7, e.g. a nucleic acid sequence encoding a polypeptide having the activity of a protein as indicated in table II, application no. 2, columns 5 and 7 or its homologs activity or of a mRNA encoding the polypeptide of the present invention having herein-mentioned amino acid of the invention increasing activity; and/or
c) increasing the specific activity of a protein conferring the increased expression of a protein encoded by the nucleic acid molecule of the invention or of the polypeptide of the present invention having herein-mentioned amino acid increasing activity, e.g. of a polypeptide having the activity of a protein as indicated in table II, application no. 2, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned amino acid of the invention increasing activity, e.g. of a polypeptide having the activity of a protein as indicated in table II, application no. 2, columns 5 and 7 or its homologs activity; and/or
e) stimulating activity of a protein conferring the increased expression of a protein encoded by the nucleic acid molecule of the present invention or a polypeptide of the present invention having herein-mentioned amino acid of the invention increasing activity, e.g. of a polypeptide having the activity of a protein as indicated in table II, application no. 2, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned amino acid of the invention increasing activity, e.g. of a polypeptide having the activity of a protein as indicated in table II, application no. 2, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned amino acid of the invention increasing activity, e.g. of a polypeptide having the activity of a protein as indicated in table II, application no. 2, columns 5 and 7 or its homologs activity; and/or
h) increasing the expression of the endogenous gene encoding the polypeptide of the invention, e.g. a polypeptide having the activity of a protein as indicated in table II, application no. 2, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with expecially high activity of the proteins of the invention from natural or from mutagenized resources and breeding them into the target organisms, eg the elite crops.

**[0054.0.2.2]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 2 in Table I to IV, resp., after increasing the expression or activity of the encoded polypeptide or having the activity of a polypeptide having an activity as the protein as shown in table II, application no. 2, column 3 or its homologs.

**[0055.0.0.2] to [0071.0.0.2]: see [0055.0.0.0] to [0071.0.0.0]**

**[0072.0.2.2]** By influencing the metabolism thus, it is possible to produce, in the process according to the invention, further advantageous compounds. Examples of such compounds are, in addition to tryptophane chorismic acid, anthralinic acid, N-5'-Phosphoribosyl-anthranilate, 1-(o-Carboxyphenylamino)-1-deoxyribulose 5-phosphate., 1-(Indol-3-yl)-glycerin-3-phosphate, and 5-hydroxytrytophane.

**[0073.0.2.2]** Accordingly, in one embodiment, the process according to the invention relates to a process which comprises:
a) providing a non-human organism, preferably a microorganism, a non-human animal, a plant or animal cell, a plant or animal tissue or a plant;
b) increasing an activity of a polypeptide of the invention or the polypeptide used in the method of the invention or a homolog thereof, e.g. as indicated in Table II, application no. 2, columns 5 or 7, or of a polypeptide being encoded by the nucleic acid molecule of the present invention and described below, e.g. conferring an increase of the respective fine chemical in the organism, preferably in a microorganism, a non-human animal, a plant or animal cell, a plant or animal tissue or a plant,
c) growing the organism, preferably the microorganism, the non-human animal, the plant or animal cell, the plant or animal tissue or the plant under conditions which permit the production of the respective fine chemical in the organism, preferably the microorganism, the plant cell, the plant tissue or the plant; and
d) if desired, recovering, optionally isolating, the free and/or bound the fine chemical and, optionally further free and/or bound amino acids synthetized by the organism, the microorganism, the non-human animal, the plant or animal cell, the plant or animal tissue or the plant.

**[0074.0.0.2] to [0084.0.0.2]: see [0074.0.0.0] to [0084.0.0.0]**

**[0085.0.2.2]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, application no. 2, columns 5 or 7, a derivative thereof, or
b) a genetic regulatory element, for example a promoter, which is functionally linked to the nucleic acid sequence as indicated in Table I, application no. 2, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.2] and [0087.0.0.2]: see [0086.0.0.0] and [0087.0.0.0]**

**[0088.0.2.2]** In an advantageous embodiment of the invention, the organism takes the form of a plant whose amino acid content is modified advantageously owing to the nucleic acid molecule of the present invention expressed. This is important for plant breeders since, for example, the nutritional value of plants for monogastric animals is limited by a few essential amino acids such as lysine, threonine or methionine or tryptophane.

**[0088.1.0.2] to [0097.0.0.2]: see [0088.1.0.0] to [0097.0.0.0]**

**[0098.0.2.2]** In a preferred embodiment, the fine chemical (tryptophane) is produced in accordance with the invention and, if desired, is isolated. The production of further amino acids such as methionine, lysine and/or threonine mixtures of amino acid by the process according to the invention is advantageous.

**[0099.0.0.2] to [0102.0.0.2]: see [0099.0.0.0] to [0102.0.0.0]**

**[0103.0.2.2]** In a preferred embodiment, the present invention relates to a process for the production of the fine chemical comprising or generating in an organism or a part thereof the expression of at least one nucleic acid molecule comprising a nucleic acid molecule selected from the group consisting of:
(a) nucleic acid molecule encoding, preferably at least the mature form, of a polypeptide having a sequence as indicated in Table II, application no. 2, columns 5 or 7;
(b) nucleic acid molecule comprising, preferably at least the mature form, of a nucleic acid molecule having a sequence as indicated in Table I, application no. 2, columns 5 or 7;
(c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
(d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
(e) nucleic acid molecule which hybridizes with a nucleic acid molecule of (a) to (c) under r stringent hybridisation conditions and conferring an increase in the amount of the fine chemical in an organism or a part thereof;
(f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical in an organism or a part thereof;
(g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof;
(h) nucleic acid molecule comprising a nucleic acid molecule which is obtained by amplifying nucleic acid molecules from a cDNA library or a genomic library using the primers pairs having a sequence as indicated in Table III, application no. 2, columns 7, and conferring an increase in the amount of the fine chemical in an organism or a part thereof;
(i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the fine chemical in an organism or a part thereof;
(j) nucleic acid molecule which encodes a polypeptide comprising the consensus sequence having a sequences as indicated in Table IV, application no. 2, column7, and conferring an increase in the amount of the fine chemical in an organism or a part thereof;
(k) nucleic acid molecule comprising one or more of the nucleic acid molecule encoding the amino acid sequence of a polypeptide encoding a domain of a polypeptide indicated in Table II, application no. 2, columns 5 or 7, and conferring an increase in the amount of the fine chemical in an organism or a part thereof; and
(l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[00103.1.0.0.]** In one embodiment, the nucleic acid molecule used in the process of the invention distinguishes over the sequence indicated in Table I, application no. 2, columns 5 or 7, by one or more nucleotides. In one embodiment, the nucleic acid molecule used in the process of the invention does not consist of the sequence indicated in Table I, application no. 2, columns 5 or 7,: In one embodiment, the nucleic acid molecule used in the process of the invention is less than 100%, 99,999%, 99,99%, 99,9% or 99% identical to a sequence indicated in Table I, application no. 2, columns 5 or 7. In another embodiment, the nucleic acid molecule does not encode a polypeptide of a sequence indicated in Table II, application no. 2, columns 5 or 7.

**[00103.2.0.0.]** ./.

**[0104.0.2.2]** In one embodiment, the nucleic acid molecule of the invention or used in the process of the invention distinguishes over the sequence indicated in Table I, application no. 2, columns 5 or 7, by one or more nucleotides. In one embodiment, the nucleic acid molecule of the present invention or used in the process of the invention does not consist of the sequence n indicated in Table I, application no. 2, columns 5 or 7: In one embodiment, the nucleic acid molecule of the present invention is less than 100%, 99,999%, 99,99%, 99,9% or 99% identical to a sequence indicated in Table I, application no. 2, columns 5 or 7. In another embodiment, the nucleic acid molecule does not encode a polypeptide of a sequence indicated in Table I, application no. 2, columns 5 or 7.

**[0105.0.0.2] to [0107.0.0.2]: see [0105.0.0.0] to [0107.0.0.0]**

**[0108.0.2.2]** Nucleic acid molecules with the sequence as indicated in Table I, application no. 2, columns 5 or 7, nucleic acid molecules which are derived from a amino acid sequences as indicated in Table II, application no. 2, columns 5 or 7, or from polypeptides comprising the consensus sequence as indicated in Table IV, application no. 2, column 7, or their derivatives or homologues encoding polypeptides with the enzymatic or biological activity of a polypeptide as indicated in Table II, application no. 2, column 3, 5 or 7, or e.g. conferring a increase of the fine chemical after increasing its expression or activity are advantageously increased in the process according to the invention.

**[0109.0.0.2]: see [0109.0.0.0]**

**[0110.0.2.2]** Nucleic acid molecules, which are advantageous for the process according to the invention and which encode polypeptides with an activity of a polypeptide of the invention or the polypeptide used in the method of the invention or used in the process of the invention , e.g. of a protein as indicated in Table II, application no. 2, column 5, or being encoded by a nucleic acid molecule indicated in Table I, application no. 2, column 5, or of its homologs, e.g. as indicated in Table II, application no. 2, column 7, can be determined from generally accessible databases.

**[0111.0.0.2]: see [0111.0.0.0]**

**[0112.0.2.2]** The nucleic acid molecules used in the process according to the invention take the form of isolated nucleic acid sequences, which encode polypeptides with an activity of a polypeptide as indicated in Table II, application no. 2, column 3, or having the sequence of a polypeptide as indicated in Table II, application no. 2, columns 5 and 7, and conferring an tryptophane increase.

**[0113.0.0.2] to [120.0.0.2]: see [0113.0.0.0] to [0120.0.0.0]**

**[0121.0.2.2]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 2, columns 5 or 7, or the functional homologues thereof as described herein, preferably conferring above-mentioned activity, i.e. conferring a increase of the fine chemical after increasing its activity.

**[0122.0.0.2] to [0127.0.0.2]: see [0122.0.0.0] to [0127.0.0.0]**

**[0128.0.2.2]** Synthetic oligonucleotide primers for the amplification, e.g. as the pairs indicated in Table III, application no. 2, column 7, by means of polymerase chain reaction can be generated on the basis of a sequence as indicated in Table I, application no. 2, columns 5 or 7, or the sequences derived from sequences as indicated in Table II, application no. 2, columns 5 or 7.

**[0129.0.2.2]** Moreover, it is possible to identify conserved regions from various organisms by carrying out protein sequence alignments with the polypeptide used in the process of the invention, in particular with sequences of the polypeptide of the invention or the polypeptide used in the method of the invention, from which conserved regions, and in turn, degenerate primers can be derived. Conserved region for the polypeptide of the invention or the polypeptide used in the method of the invention are indicated in the alignments shown in the figures. Conserved regions are those, which show a very little variation in the amino acid in one particular position of several homologs from different origin. The consensus sequences indicated in Table IV, application no. 2, column 7, are derived from said alignments.

**[0130.0.2.2] to [0138.0.0.2]: see [0131.0.0.0] to [0138.0.0.0]**

**[0139.0.2.2]** Polypeptides having above-mentioned activity, i.e. conferring a tryptophane increase, derived from other organisms, can be encoded by other DNA sequences which hybridize to a sequences indicated in Table I, application no. 2, columns 5 or 7, under relaxed hybridization conditions and which code on expression for peptides having the tryptophane increasing activity.

**[0140.0.0.2] to [0146.0.0.2]: see [0140.0.0.0] to [0146.0.0.0]**

**[0147.0.2.2]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 2, columns 5 or 7, is one which is sufficiently complementary to one of said nucleotide sequences such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.2.2]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 2, columns 5 or 7, or a functional portion thereof and preferably has above mentioned activity, in particular has the-fine-chemical-increasing activity after increasing its activity or an activity of a product of a gene encoding said sequence or its homologs.

**[0149.0.2.2]** The nucleic acid molecule of the invention comprises a nucleotide sequence which hybridizes, preferably hybridizes under stringent conditions as defined herein, to one of the nucleotide sequences indicated in Table I, application no. 2, columns 5 or 7, or a portion thereof and encodes a protein having above-mentioned activity and as indicated in indicated in Table II, application no. 2, columns 5 or 7, e.g. conferring an increase of the fine chemical.

**[0149.1.2.2]** Optionally, the nucleotide sequence, which hybridises to one of the nucleotide sequences indicated in Table I, application no. 2, columns 5 or 7, has further one or more of the activities annotated or known for a protein as indicated in Table II, application no. 2, column 3.

**[0150.0.2.2]** Moreover, the nucleic acid molecule of the invention or used in the process of the invention can comprise only a portion of the coding region of one of the sequences indicated in Table I, application no. 2, columns 5 or 7, for example a fragment which can be used as a probe or primer or a fragment encoding a biologically active portion of the polypeptide of the present invention or of a polypeptide used in the process of the present invention, i.e. having above-mentioned activity, e.g. conferring an increase of tryptophane if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, application no. 2, columns 5 or 7, an anti-sense sequence of one of the sequences indicated in Table I, application no. 2, columns 5 or 7, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 2, column 7, will result in a fragment of a polynucleotide sequence as indicated in Table I, application no. 2, columns 5 or 7.

**[0151.0.0.2]: [see 0151.0.0.0]**

**[0152.0.2.2]** The nucleic acid molecule of the invention encodes a polypeptide or portion thereof which includes an amino acid sequence which is sufficiently homologous to an amino acid sequence as indicated in Table II, application no. 2, columns 5 or 7, such that the protein or portion thereof maintains the ability to participate in tryptophane production, in particular a tryptophane increasing activity as mentioned above or as described in the examples in plants or microorganisms is comprised.

**[0153.0.2.2]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 2, columns 5 or 7, such that the protein or portion thereof is able to participate in the increase of tryptophane production. In one embodiment, a protein or portion thereof as indicated in Table II, application no. 2, columns 5 or 7, has for example an activity of a polypeptide indicated in Table II, application no. 2, column 3.

**[0154.0.2.2]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, application no. 2, columns 5 or 7, and has above-mentioned activity, e.g. conferring preferably the increase of the fine chemical.

**[0155.0.0.2] and [0156.0.0.2]: see [0155.0.0.0] to [0156.0.0.0]**

**[0157.0.2.2]** The invention further relates to nucleic acid molecules that differ from one of a nucleotide sequences as indicated in Table I, application no. 2, columns 5 or 7, (and portions thereof) due to degeneracy of the genetic code and thus encode a polypeptide of the present invention, in particular a polypeptide having above mentioned activity, e.g. conferring an increase in tryptophane in a organism, e.g. as that polypeptides comprising the consensus sequences as indicated in Table IV, application no. 2, columns 7, or of the polypeptide as indicated in Table II, application no. 2, columns 5 or 7, or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, application no. 2, columns 7, or of the polypeptide as indicated in Table II, application no. 2, columns 5 or 7, or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, application no. 2, column 7, or of a polypeptide as indicated in Table II, application no. 2, columns 5 or 7, or the functional homologues thereof. However, in a preferred embodiment, the nucleic acid molecule of the present invention does not consist of a sequence as indicated in Table I, application no. 2, columns 5 or 7. Preferably the nucleic acid molecule of the invention is a functional homologue or identical to a nucleic acid molecule indicated in Table I, application no. 2, columns 5 or 7.

**[0158.0.0.2] to [0160.0.0.2]: see [0158.0.0.0] to [0160.0.0.0]**

**[0161.0.2.2]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 2, columns 5 or 7. The nucleic acid molecule is preferably at least 20, 30, 50, 100, 250 or more nucleotides in length.

**[0162.0.0.2]: see [0162.0.0.0]**

**[0163.0.2.2]** Preferably, nucleic acid molecule of the invention that hybridizes under stringent conditions to a sequence as indicated in Table I, application no. 2, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the fine chemical increase after increasing the expression or activity thereof or the activity of a protein of the invention or used in the process of the invention.

**[0164.0.0.2]: see [0164.0.0.0]**

**[0165.0.2.2]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7,lines 16 to 18 and/or lines 356 to 362.

**[0166.0.0.2] and [0167.0.0.2]:** see **[0166.0.0.0]** and **[0167.0.0.0]**

**[0168.0.2.2]** Accordingly, the invention relates to nucleic acid molecules encoding a polypeptide having above-mentioned activity, e.g. conferring an increase in the fine chemical in an organisms or parts thereof that contain changes in amino acid residues that are not essential for said activity. Such polypeptides differ in amino acid sequence from a sequence contained in as sequence as indicated in Table II, application no. 2, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 2, columns 5 or 7, and is capable of participation in the increase of production of the fine chemical after increasing its activity, e.g. its expression. Preferably, the protein encoded by the nucleic acid molecule is at least about 60% identical to a sequence as indicated in Table II, application no. 2, columns 5 or 7, more preferably at least about 70% identical to one of the sequences as indicated in Table II, application no. 2, columns 5 or 7, even more preferably at least about 80%, 90% or 95% homologous to a sequence as indicated in Table II, application no. 2, columns 5 or 7, and most preferably at least about 96%, 97%, 98%, or 99% identical to the sequence as indicated in Table II, application no. 2, columns 5 or 7.

**[0169.0.0.2] to [0172.0.0.2]: see [0169.0.0.0] to [0172.0.0.0]**

**[0173.0.2.2]** For example a sequence which has a 80% homology with sequence SEQ ID No: 172 at the nucleic acid level is understood as meaning a sequence which, upon comparison with the sequence SEQ ID No: 172 by the above Gap program algorithm with the above parameter set, has a 80% homology.

**[0174.0.0.2]: see [0174.0.0.0]**

**[0175.0.2.2]** For example a sequence which has a 80% homology with sequence SEQ ID No: 173 at the protein level is understood as meaning a sequence which, upon comparison with the sequence SEQ ID No: 173 by the above program algorithm with the above parameter set, has a 80% homology.

**[0176.0.2.2]** Functional equivalents derived from one of the polypeptides as indicated in Table II, application no. 2, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 2, columns 5 or 7, according to the invention and are distinguished by essentially the same properties as a polypeptide as indicated in Table II, application no. 2, columns 5 or 7.

**[0177.0.2.2]** Functional equivalents derived from a nucleic acid sequence as indicated in Table I, application no. 2, columns 5 or 7 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptides as indicated in Table II, application no. 2, columns 5 or 7, according to the invention and encode polypeptides having essentially the same properties as a polypeptide as indicated in Table II, application no. 2, columns 5 or 7.

**[0178.0.0.2]: see [0178.0.0.0]**

**[0179.0.2.2]** A nucleic acid molecule encoding an homologous to a protein sequence of as indicated in Table II, application no. 2, columns 5 or 7, can be created by introducing one or more nucleotide substitutions, additions or deletions into a nucleotide sequence of the nucleic acid molecule of the present invention, in particular as indicated in Table I, application no. 2, columns 5 or 7, such that one or more amino acid substitutions, additions or deletions are introduced into the encoded protein. Mutations can be introduced into the encoding sequences of a sequences as indicated in Table I, application no. 2, columns 5 or 7, by standard techniques, such as site-directed mutagenesis and PCR-mediated mutagenesis.

**[0180.0.0.2] to [0183.0.0.2]: see [0180.0.0.0] to [0183.0.0.0]**

**[0184.0.2.2]** Homologues of the nucleic acid sequences used, with a sequence as indicated in Table I, application no. 2, columns 5 or 7, or of the nucleic acid sequences derived from a sequences as indicated in Table II, application no. 2, columns 5 or 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 2, columns 5 or 7, or from the derived nucleic acid sequences, the intention being, however, that the enzyme activity or the biological activity of the resulting proteins synthesized is advantageously retained or increased.

**[0185.0.2.2]** In one embodiment of the present invention, the nucleic acid molecule of the invention or used in the process of the invention comprises one or more sequences as indicated in Table I, application no. 2, columns 5 or 7,. In one embodiment it is preferred that the nucleic acid molecule comprises as little as possible other nucleotide sequences not shown in any one of sequences as indicated in Table I, application no. 2, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule use in the process of the invention is identical to a sequences as indicated in Table I, application no. 2, columns 5 or 7.

**[0186.0.2.2]** Also preferred is that one or more nucleic acid molecule(s) used in the process of the invention encodes a polypeptide comprising a sequence as indicated in Table II, application no. 2, columns 5 or 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 2, columns 5 or 7.

**[0187.0.2.2]** In one embodiment, the nucleic acid molecule of the invention or used in the process encodes a polypeptide comprising a sequence as indicated in Table II, application no. 2, columns 5 or 7, and comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 2, columns 5 or 7.

**[0188.0.2.2]** Polypeptides (= proteins), which still have the essential enzymatic activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 2, columns 5 or 7, preferably compared to a sequence as indicated in Table II, application no. 2, column 5, and expressed under identical conditions.

**[0189.0.2.2]** Homologues of a sequences as indicated in Table I, application no. 2, columns 5 or 7, or of a derived sequences as indicated in Table II, application no. 2, columns 5 or 7, also mean truncated sequences, cDNA, single-stranded DNA or RNA of the coding and noncoding DNA sequence. Homologues of said sequences are also understood as meaning derivatives which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.2] to [0203.0.0.2]: see [0190.0.0.0] to [0203.0.0.0]**

**[0204.0.2.2]** Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 2, columns 5 or 7, or a fragment thereof conferring an increase in the amount of the fine chemical in an organism or a part thereof
b) nucleic acid molecule comprising, preferably at least the mature form, of a nucleic acid molecule as indicated in Table II, application no. 2, columns 5 or 7, or a fragment thereof conferring an increase in the amount of the fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof;
h) nucleic acid molecule comprising a nucleic acid molecule which is obtained by amplifying a cDNA library or a genomic library using primers or primer pairs as indicated in Table III, application no. 2, column 7, and conferring an increase in the amount of the fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising the consensus sequence as indicated in Table IV, application no. 2, column 7, and conferring an increase in the amount of the fine chemical in an organism or a part thereof;
k) nucleic acid molecule encoding the amino acid sequence of a polypeptide encoding a domain of a polypeptide as indicated in Table II, application no. 2, columns 5 or 7, and conferring an increase in the amount of the fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 2, columns 5 or 7, or a nucleic acid molecule encoding, preferably at least the mature form of, the polypeptide as indicated in Table II, application no. 2, columns 5 or 7, and conferring an increase in the amount of the fine chemical in an organism or a part thereof;
or which encompasses a sequence which is complementary thereto;
whereby, preferably, the nucleic acid molecule according to (a) to (I) distinguishes over the sequence indicated in Table I, application no. 2, columns 5 or 7, by one or more nucleotides. In one embodiment, the nucleic acid molecule does not consist of the sequence shown and in indicated in Table I, application no. 2, columns 5 or 7: In one embodiment, the nucleic acid molecule is less than 100%, 99,999%, 99,99%, 99,9% or 99% identical to a sequence indicated in Table I, application no. 2, columns 5 or 7. In another embodiment, the nucleic acid molecule does not encode a polypeptide of a sequence indicated in Table II, application no. 2, columns 5 or 7. In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 2, columns 5 or 7. In a further embodiment the nucleic acid molecule does not encode a polypeptide sequence as indicated in Table II, application no. 2, columns 5 or 7. Accordingly, in one embodiment, the nucleic acid molecule of the differs at least in one or more residues from a nucleic acid molecule indicated in Table I, application no. 2, columns 5 or 7. Accordingly, in one embodiment, the nucleic acid molecule of the present invention encodes a polypeptide, which differs at least in one or more amino acids from a polypeptide indicated in Table II, application no. 2, columns 5 or 7. In another embodiment, a nucleic acid molecule indicated in Table I, application no. 2, columns 5 or 7, does not encode a protein of a sequence indicated in Table II, application no. 2, columns 5 or 7. Accordingly, in one embodiment, the protein encoded by a sequences of a nucleic acid according to (a) to (I) does not consist of a sequence as indicated in Table II, application no. 2, columns 5 or 7. In a further embodiment, the protein of the present invention is at least 30 %, 40 %, 50%, or 60% identical to a protein sequence indicated in Table II, application no. 2, columns 5 or 7, and less than 100%, preferably less than 99,999%, 99,99% or 99,9%, more preferably less than 99%, 985, 97%, 96% or 95% identical to a sequence as indicated in Table I, application no. 2, columns 5 or 7.

**[0205.0.0.2] to [0226.0.0.2]: see [0205.0.0.0] to [0226.0.0.0]**

**[0227.0.2.2]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansim.
In addition to a sequence as indicated in Table I, application no. 2, columns 5 or 7, or its derivatives, it is advantageous additionally to express and/or mutate further genes in the organisms. Especially advantageously, additionally at least one further gene of the amino acid biosynthetic pathway such as for L-lysine, L-threonine and/or L-methionine or L-tryptophane is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all.
In addition it might be advantageously to combine a sequences as indicated in Table I, application no. 2, columns 5 or 7, with genes which generally support or enhances to growth or yield of the target organismn, for example genes which lead to faster growth rate of microorganisms or genes which produces stress-, pathogen, or herbicide resistant plants.

**[0228.0.0.2] to [0230.0.0.2]: see [0228.0.0.0] to [0230.0.0.0]**

**[0231.0.0.2]** In a further advantageous embodiment of the process of the invention, the organisms used in the process are those in which simultaneously a tryptophane degrading protein is attenuated, in particular by reducing the rate of expression of the corresponding gene.

**[0232.0.0.2] to [0282.0.0.2]: see [0232.0.0.0] to [0282.0.0.0]**

**[0283.0.2.2]** Moreover, a native polypeptide conferring the increase of the fine chemical in an organism or part thereof can be isolated from cells (e.g., endothelial cells), for example using the antibody of the present invention as described below, in particular, an antibody against a protein as indicated in Table II, application no. 2, column 3, E.g. an antibody against a polypeptide as indicated in Table II, application no. 2, columns 5 or 7, which can be produced by standard techniques utilizing polypeptides comprising or consisting of above mentioned sequences, e.g. the polypeptide of the present invention or fragment thereof, Preferred are monoclonal antibodies, specifically binding to polypeptide as indicated in Table II, application no. 2, columns 5 or 7.

**[0284.0.0.2] see [0284.0.0.0]**

**[0285.0.2.2]** In one embodiment, the present invention relates to a polypeptide having a sequence as indicated in Table II, application no. 2, columns 5 or 7, or as encoded by a nucleic acid molecule as indicated in Table I, application no. 2, columns 5 or 7, or functional homologues thereof.

**[0286.0.2.2]** In one advantageous embodiment, in the method of the present invention the activity of a polypeptide is increased which comprises or consists of a consensus sequence as indicated in Table IV, application no. 2, column 7, and in one another embodiment, the present invention relates to a polypeptide comprising or consisting of a consensus sequence as indicated in Table IV, application no. 2, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences as indicated in Table IV, application no. 2, column 7 amino acidamino acid

**[0287.0.0.2] to [290.0.0.2]: see [0287.0.0.0] to [0290.0.0.0]**

**[0291.0.2.2]** In one advantageous embodiment, the method of the present invention comprises the increasing of a polypeptide comprising or consisting of plant or microorganism specific consensus sequences.

Accordingly, in one embodiment, the present invention relates to a polypeptide comprising or consisting of plant or microorganism specific consensus sequences.

In one embodiment, said polypeptide of the invention distinguishes over a sequence as indicated in Table II, application no. 2, columns 5 or 7, by one or more amino acids. In one embodiment, polypeptide distinguishes form a sequence as indicated in Table II, application no. 2, columns 5 or 7by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 2, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II, application no. 2, columns 5 or 7.

**[0292.0.0.2]: see [0292.0.0.0]**

**[0293.0.2.2]** In one embodiment, the invention relates to polypeptide conferring an increase in the respective fine chemical in an organism or part being encoded by the nucleic acid molecule of the invention or by a nucleic acid molecule used in the process of the invention.

In one embodiment, the polypeptide of the invention has a sequence which distinguishes from a sequence as indicated in Table II, application no. 2, columns 5 or 7, by one or more amino acids. In an other embodiment, said polypeptide of the invention does not consist of the sequence as indicated in Table II, application no. 2, columns 5 or 7. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 2, columns 5 or 7.

**[0294.0.2.2]** In one embodiment, the present invention relates to a polypeptide having an activity of a protein as indicated in Table II, application no. 2, column 3, which distinguishes over a sequence as indicated in Table II, application no. 2, columns 5 or 7, to 362 by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.2] to [0297.0.0.2]: see [0295.0.0.0] to [0297.0.0.0]**

**[0297.1.0.2]** Non-polypeptide of the invention-chemicals are e.g. polypeptides having not the activity and/or amino acid sequence of a polypeptide indicated in Table II, application no. 2, columns 3, 5 or 7.

**[0298.0.2.2]** A polypeptide of the invention can participate in the process of the present invention. The polypeptide or a portion thereof comprises preferably an amino acid sequence which is sufficiently homologous to an amino acid sequence as indicated in Table II, application no. 2, columns 5 or 7, such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, application no. 2, columns 5 or 7.

**[0299.0.2.2]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, application no. 2, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 2, columns 5 or 7, or which is homologous thereto, as defined above.

**[0300.0.2.2]** Accordingly the polypeptide of the present invention can vary from a sequence as indicated in Table II, application no. 2, columns 5 or 7, in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II, application no. 2, columns 5 or 7.

**[0301.0.0.2]: see [0301.0.0.0]**

**[0302.0.2.2]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 2, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.2]: see [0303.0.0.0]**

**[0304.0.2.2]** Manipulation of the nucleic acid molecule of the invention may result in the production of a protein having essentially the activity of the polypeptides as indicated in Table II, application no. 2, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.2] to [0306.0.0.2]: see [0305.0.0.0] to [0306.0.0.0]**

**[00306.1.2.2]** Preferrably, the compound is a composition comrising the tryptophane or a recovered tryptophane, in particular, the fine chemical, free or in protein-bound form.

**[0307.0.0.2]: to [0308.0.0.2]: see [0305.0.0.0] to [03086.0.0.0]**

**[0309.0.2.2]** In one embodiment, a reference to a "protein (= polypeptide)" of the invention or as indicated in Table II, application no. 2, columns 5 or 7, refers to a polypeptide having an amino acid sequence corresponding to the polypeptide of the invention or used in the process of the invention , whereas a "non- polypeptide of the invention" or "other polypeptide"-not being indicated in Table II, application no. 2, columns 5 or 7, refers to a polypeptide having an amino acid sequence corresponding to a protein which is not substantially homologous a polypeptide of the invention, preferably which is not substantially homologous to a as indicated in Table II, application no. 2, columns 5 or 7, e.g., a protein which does not confer the activity described herein or annotated or known for as indicated in Table II, application no. 2, column 3, and which is derived from the same or a different organism. In one embodiment a "non-polypeptide of the invention" or "other polypeptide" not being indicate in Table II, application no. 2, columns 5 or 7, does not confer an increase of the fine chemical in an organism or part thereof.

**[0310.0.0.2] to [334.0.0.2]: see [0310.0.0.0] to [0334.0.0.0]**

**[0335.0.2.2]** The dsRNAi method has proved to be particularly effective and advantageous for reducing the expression of a nucleic acid sequences as indicated in Table II, application no. 2, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, application no. 2, columns 5 or 7, and/or homologs thereof. In a double-stranded RNA molecule for reducing the expression of an protein encoded by a nucleic acid sequence of one of the sequences as indicated in Table I, application no. 2, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.2] to [0342.0.0.2]: see [0336.0.0.0] to [0342.0.0.0]**

**[0343.0.2.2]** As has already been described, 100 % sequence identity between the dsRNA and a gene transcript of a nucleic acid sequence as indicated in Table I, application no. 2, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 2, columns 5 or 7, or homologs thereof of the one organism, may be used to suppress the corresponding expression in another organism.

**[0344.0.0.2] to [0361.0.0.2]: see [0344.0.0.0] to [0361.0.0.0]**

**[0362.0.2.2]** Accordingly the present invention relates to any cell transgenic for any nucleic acid characterized as part of the invention, e.g. conferring the increase of the fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention, e.g. the polypeptide as indicated in Table II, application no. 2, columns 5 or 7. Due to the above mentioned activity the fine chemical content in a cell or an organism is increased. For example, due to modulation or manipulation, the cellular activity of the polypeptide of the invention or the polypeptide used in the method of the invention or nucleic acid molecule of the invention or the nucleic acid molecule used in the method of the invention is increased, e.g. due to an increased expression or specific activity of the subject matters of the invention in a cell or an organism or a part thereof. In one embodiment transgenic for a polypeptide having an activity of a polypeptide as indicated in Table II, application no. 2, columns 5 or 7, means herein that due to modulation or manipulation of the genome, an activity as annotated for a polypeptide as indicated in Table II, application no. 2, columns 3, e.g. having a sequence as indicated in Table II, application no. 2, columns 5 or 7, is increased in a cell or an organism or a part thereof. Examples are described above in context with the process of the invention.

**[0363.0.0.2] to [0382.0.0.2]: see [0365.0.0.0] to [0382.0.0.0]**

**[0383.0.2.2]** For preparing aromatic compound-containing fine chemicals, in particular the fine chemical, it is possible to use as aromat source organic aromatic-containing compounds such as, for example, benzene, naphthaline, indole, pyrrole, furen, oxazole, imidazole, thiophene, pyrridin, pyrrimidine or else organic aromatic compounds such as benzoic acid and chorismic, shikimic, aminobenzoic, kynurenic acids or pyridoxidal.

**[0384.0.0.2]: see [0384.0.0.0]**

**[0385.0.2.2]** The fermentation broths obtained in this way, containing in particular L-tryptophane, L-methionine, L-threonine and/or L-lysine, normally have a dry matter content of from 7.5 to 25% by weight. Sugar-limited fermentation is additionally advantageous, at least at the end, but especially over at least 30% of the fermentation time. This means that the concentration of utilizable sugar in the fermentation medium is kept at, or reduced to, 0 to 3 g/I during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.0.2] to [392.0.0.2]: see [0386.0.0.0] to [0392.0.0.0]**

**[0393.0.2.2]** In one embodiment, the present invention relates to a method for the identification of a gene product conferring an increase in the fine chemical production in a cell, comprising the following steps:
(a) contacting, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence as indicated in Table I, application no. 2, columns 5 or 7, and, optionally, isolating the full length cDNA clone or complete genomic clone;
(c) introducing the candidate nucleic acid molecules in host cells, preferably in a plant cell or a microorganism, appropriate for producing the fine chemical;
(d) expressing the identified nucleic acid molecules in the host cells;
(e) assaying the fine chemical level in the host cells; and
(f) identifying the nucleic acid molecule and its gene product which expression confers an increase in the fine chemical level in the host cell after **expression** compared to the wild type.

**[0394.0.0.2] to [0399.0.0.2]: see [0394.0.0.0] to [0399.0.0.0]**

**[0399.1.2.2]** One can think to screen for increased production of the fine chemical by for example searching for a resistance to a drug blocking the synthesis of the fine chemical and looking whether this effect is dependent on the activity or expression of a polypeptide as indicated in Table II, application no. 2, columns 5 or 7, or a homolog thereof, e.g. comparing the phenotype of nearly identical organisms with low and high activity of a protein as indicated in Table II, application no. 2, columns 5 or 7, after incubation with the drug.

**[0400.0.0.2] to [0416.0.0.2]: see [0400.0.0.0] to [0416.0.0.0]**

**[0417.0.2.2]** The nucleic acid molecule of the invention, the vector of the invention or the nucleic acid construct of the invention may also be useful for the production of organisms resistant to inhibitors of the amino acid production biosynthesis pathways. In particular, the overexpression of the polypeptide of the present invention may protect plants against herbicides, which block the amino acid, in particular the fine chemical, synthesis in said plant. Examples of herbicides blocking the amino acid synthesis in plants are for example sulfonylurea and imidazolinone herbicides which catalyze the first step in branched-chain amino acid biosynthesis.

**[0418.0.0.2] to [0423.0.0.2]: see [0418.0.0.0] to [0423.0.0.0]**

**[0424.0.2.2]** Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the fine chemical or of the fine chemical and one or more other amino acids, in particular methionine, threonine, alanine, glutamine, glutamic acid, valine, asparagine, phenylalanine, leucine, proline, Tryptophan tyrosine, , isoleucine and arginine.
Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the fine chemical in a organism or part thereof, e.g. in a cell.

**[0425.0.0.2] to [0430.0.0.2]: see [0425.0.0.0] to [0430.0.0.0]**

**[0431.0.2.2]** Example 1: Cloning SEQ ID No: 172 in Escherichia coli

**[0432.0.0.0] to [0460.0.0.2]: see [0433.0.0.0] to [0460.0.0.0]**

**[0461.0.2.2]** Example 10: Cloning SEQ ID NO: 172 for the expression in plants

**[0462.0.0.2] see [0462.0.0.0]**

**[0463.0.2.2]** SEQ ID NO: 172 is amplified by PCR as described in the protocol of the Pfu Turbo or DNA Herculase polymerase (Stratagene).

**[0464.0.0.2] to [0466.0.0.2]: see [0464.0.0.0] to [0466.0.0.0]**

**[0467.0.2.2]** The following primer sequences were selected for the gene SEQ ID No: 172:
i) forward primer SEQ ID No: 174
ii) reverse primer SEQ ID No: 175

**[0468.0.0.2] to [0479.0.0.2]: see [0468.0.0.0] to [0479.0.0.0]**

**[0480.0.2.2]** Example 11: Generation of transgenic plants which express SEQ ID No: 172

**[0481.0.0.2] to [0513.0.0.2]: see [0481.0.0.0] to [0513.0.0.0]**

**[0514.0.2.2]** As an alternative, the amino acids can be detected advantageously via HPLC separation in ethanolic extract as described by Geigenberger et al. (Plant Cell & Environ, 19, 1996: 43-55).

**[0515.0.0.2] to [0552.2.0.2]: see [0515.0.0.0] to [0552.2.0.0]**

**[0553.0.2.2]** A process for the production of tryptophane, which comprises
increasing or generating the activity of a protein as indicated in Table II, application no. 2, columns 5 or 7, or a functional equivalent thereof in a non-human organism, or in one or more parts thereof; and
growing the organism under conditions which permit the production of tryptophane in said organism.
A process for the production of tryptophane, comprising the increasing or generating in an organism or a part thereof the expression of at least one nucleic acid molecule comprising a nucleic acid molecule selected from the group consisting of:
nucleic acid molecule encoding of a polypeptide as indicated in Table II, application no. 2, columns 5 or 7, or a fragment thereof, which confers an increase in the amount of tryptophane in an organism or a part thereof;
nucleic acid molecule comprising of the nucleic acid molecule as indicated in Table I, application no. 2, columns 5 or 7;
nucleic acid molecule whose sequence can be deduced from a polypeptide sequence encoded by a nucleic acid molecule of (a) or (b) as a result of the degeneracy of the genetic code and conferring an increase in the amount of tryptophane in an organism or a part thereof;
nucleic acid molecule which encodes a polypeptide which has at least 50% identity with the amino acid sequence of the polypeptide encoded by the nucleic acid molecule of (a) to (c) and conferring an increase in the amount of tryptophane in an organism or a part thereof;
nucleic acid molecule which hybidizes with a nucleic acid molecule of (a) to (c) under stringent hybridisation conditions and conferring an increase in the amount of tryptophane in an organism or a part thereof;
nucleic acid molecule which encompasses a nucleic acid molecule which is obtained by amplifying nucleic acid molecules from a cDNA library or a genomic library using the primers or primer pairs as indicated in Table III, application no. 2, column 7, and conferring an increase in the amount of the fine chemical in an organism or a part thereof;
nucleic acid molecule encoding a polypeptide which is isolated with the aid of monoclonal antibodies against a polypeptide encoded by one of the nucleic acid molecules of (a) to (f) and conferring an increase in the amount of tryptophane in an organism or a part thereof;
nucleic acid molecule encoding a polypeptide comprising a consensus sequence as indicated in Table IV, application no. 2, column 7, and conferring an increase in the amount of the fine chemical in an organism or a part thereof;
nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
or comprising a sequence which is complementary thereto.
The process as mentioned above, comprising recovering of the free or bound tryptophane.
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound tryptophane produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
(a) nucleic acid molecule encoding of a polypeptide as indicated in Table II, application no. 2, columns 5 or 7, or a fragment thereof, which confers an increase in the amount of tryptophane in an organism or a part thereof;
(b) nucleic acid molecule comprising of a nucleic acid as indicated in Table I, application no. 2, columns 5 or 7;
(c) nucleic acid molecule whose sequence can be deduced from a polypeptide sequence encoded by a nucleic acid molecule of (a) or (b) as a result of the degeneracy of the genetic code and conferring an increase in the amount of tryptophane in an organism or a part thereof;
(d) nucleic acid molecule which encodes a polypeptide which has at least 50% identity with the amino acid sequence of the polypeptide encoded by the nucleic acid molecule of (a) to (c) and conferring an increase in the amount of tryptophane in an organism or a part thereof;
(e) nucleic acid molecule which hybidizes with a nucleic acid molecule of (a) to (c) under under stringent hybridisation conditions and conferring an increase in the amount of tryptophane in an organism or a part thereof;
(f) nucleic acid molecule which encompasses a nucleic acid molecule which is obtained by amplifying nucleic acid molecules from a cDNA library or a genomic library using the primers or primer pairs as indicated in Table III, application no. 2, column 7, and conferring an increase in the amount of tryptophane in an organism or a part thereof;
(g) nucleic acid molecule encoding a polypeptide which is isolated with the aid of monoclonal antibodies against a polypeptide encoded by one of the nucleic acid molecules of (a) to (f) and conferring an increase in the amount of tryptophane in an organism or a part thereof;
(h) nucleic acid molecule encoding a polypeptide comprising the consensus sequence as indicated in Table IV, application no. 2, column 7, and conferring an increase in the amount of the fine chemical in an organism or a part thereof; and
(i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
whereby the nucleic acid molecule distinguishes over the sequence as indicated in Table I, application no. 2, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 2, columns 5 or 7, by one or more amino acids.
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of tryptophane in an organism or a part thereof comprising:
(a) contacting cells, tissues , plants or microorganisms which express the a polypeptide encoded by the nucleic acid molecule as mentioned above conferring an increase in the amount of tryptophane in an organism or a part thereof with a candidate compound or a sample comprising a plurality of compounds under conditions which permit the expression the polypeptide;
(b) assaying the tryptophane level or the polypeptide expression level in the cell, tissue, plant or microorganism or the media the cell, tissue, plant or microorganisms is cultured or maintained in; and
(c) identifying a agonist or antagonist by comparing the measured tryptophane level or polypeptide expression level with a standard tryptophane or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A method for the identification of a compound conferring increased tryptophane production in a plant or microorganism, comprising the steps:
a) culturing a plant cell or tissue or microorganism or maintaining a plant expressing the polypeptide encoded by the nucleic acid molecule as mentioned above conferring an increase in the amount of tryptophane in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of tryptophane in an organism or a part thereof;
b) identifying if the compound is an effective agonist by detecting the presence or absence or increase of a signal produced by said readout system.
A method for the identification of a gene product conferring an increase in tryptophane production in a cell, comprising the following steps:
a) contacting the nucleic acid molecules of a sample, which can contain a candidate gene encoding a gene product conferring an increase in tryptophane after expression with the nucleic acid molecule as mentioned above;
b) identifying the nucleic acid molecules, which hybridise under relaxed stringent conditions with the nucleic acid molecule as mentioned above;
c) introducing the candidate nucleic acid molecules in host cells appropriate for producing tryptophane ;
d) expressing the identified nucleic acid molecules in the host cells;
e) assaying the tryptophane level in the host cells; and
f)identifying nucleic acid molecule and its gene product which expression confers an increase in the tryptophane level in the host cell in the host cell after expression compared to the wild type.
A method for the identification of a gene product conferring an increase in tryptophane production in a cell, comprising the following steps:
a) identifying in a data bank nucleic acid molecules of an organism; which can contain a candidate gene encoding a gene product conferring an increase in the tryptophane amount or level in an organism or a part thereof after expression, and which are at least 20% homolog to the nucleic acid molecule as mentioned above;
b) introducing the candidate nucleic acid molecules in host cells appropriate for producing tryptophane ;
c) expressing the identified nucleic acid molecules in the host cells ;
d) assaying the tryptophane level in the host cells ; and
e) identifying nucleic acid molecule and its gene product which expression confers an increase in the tryptophane level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of tryptophane after expression.
Use of the polypeptide as mentioned above or the nucleic acid construct as mentioned above or the gene product identified according to the method as mentioned above for identifying compounds capable of conferring a modulation of tryptophane levels in an organism.
Food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.

Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the host cell as mentioned above or the gene product identified according to the method as mentioned above for the protection of a plant against a tryptophane synthesis inhibiting herbicide.

### New genes for a process for the production of fine chemicals

**[0000.0.0.3]** In a further embodiment, the present invention relates to a further process for the production of fine chemicals as defined below and corresponding embodiments as described herein as follows.

**[0001.0.0.3] to [0008.0.0.3]: see [0001.0.0.0] to [0008.0.0.0]**

**[0009.0.3.3]** As described above, the essential amino acids are necessary for humans and many mammals, for example for livestock. The branched-chain amino acids (BCAA) leucineand valine are among the nine dietary indispensable amino acids for humans. BCAA accounts for 35-40% of the dietary indispensable amino acids in body protein and 14% of the total amino acids in skeletal muscle (Ferrando et al., (1995) Oral branched chain amino acids decrease whole-body proteolysis. J. Parenter. Enteral Nutr. 19: 47-54.13). They share a common membrane transport system and enzymes for their transamination and irreversible oxidation (Block, K. P. (1989) Interactions among leucine and valine with special reference to the branched chain amino acid antagonism. In: Absorption and Utilization of Amino Acids (Friedman, M., ed.), pp. 229-244, CRC Press, Boca Raton, FL. and Champe, P. C. & Harvey, R. A. (1987) Amino acids: metabolism of carbon atoms. In: Biochemistry (Champ, P. C. & Harvery, P. A., eds.), pp.242-252, J. B. Lippincott, Philadelphia, PA.). Further, for patient suffering from Maple Syrup Urine Disease (MSUD) a reduced uptake of those branched-chain amino acids is essential.
Dietary sources of the branched-chain amino acids are principally derived from animal and vegetable proteins. The branched-chain amino acids (BCAA) leucineand valine are limiting for the growth of many mammals. Therefore the branched-chain amino acids are supplemented in the feed of broiler, leg hens, turkey, swine or cattle diets.

**[0010.0.0.3] see [0010.0.0.0]**

**[0011.0.0.3] see [0011.0.0.0]**

**[0012.0.3.3]** It is an object of the present invention to develop an inexpensive process for the synthesis of leucine and/or valine, preferably L- leucine and/or L-valine.

**[0013.0.0.3] see [0013.0.0.0]**

**[0014.0.3.3]** Accordingly, in a first embodiment, the invention relates to a process for the production of a fine chemical, whereby the fine chemical is leucine and/or valine, preferably L- leucine and/or L-valine. Accordingly, in the present invention, the term "the fine chemical" as used herein relates to "leucine and/or valine". Further, the term "the fine chemicals" as used herein also relates to fine chemicals comprising leucine and/or valine.

**[0015.0.3.3]** In one embodiment, the term "the fine chemical" means leucine and/or valine, preferably L- leucine and/or L-valine. Throughout the specification the term "the fine chemical" means leucine and/or valine, preferably L- leucine and/or L-valine, its salts, ester or amids in free form or bound to proteins. In a preferred embodiment, the term "the fine chemical" means leucine and/or valine, preferably L- leucine and/or L-valine, in free form or its salts or bound to proteins.

**[0016.0.3.3]** Accordingly, the present invention relates to a process for the production of the respective fine chemical comprising
(a) increasing or generating the activity of one or more of a protein as shown in table II, application no. 3, column 3 encoded by the nucleic acid sequences as shown in table I, application no. 3, column 5, in a non-human organism or in one or more parts thereof or
(b) growing the organism under conditions which permit the production of the fine chemical, thus amino acids of the invention or fine chemicals comprising amino acids of the invention, in said organism or in the culture medium surrounding the organism.

**[0016.1.3.3]** Accordingly, the term "the fine chemical" means in one embodiment "valine" in relation to all sequences listed in Tables I to IV, line 6 and/or 8 and/or 9 or homologs thereof and means in one embodiment "leucine" in relation to all sequences listed in Table I to IV, line 7 or homologs thereof.

**[0017.0.0.3] to [0019.0.0.3] see [0017.0.0.0] to [0019.0.0.0]**

**[0020.0.3.3]** Surprisingly it was found, that the transgenic expression of the Zea mays protein as indicated in Table II, column 5, line 6 in a plant conferred an increase in valine content of the transformed plants. Thus, in one embodiment, said protein or its homologs are used for the production of valine.
Surprisingly it was found, that the transgenic expression of the Zea mays protein as indicated in Table II, column 5, line 7 in a plant conferred an increase in leucine content of the transformed plants. Thus, in one embodiment, said protein or its homologs are used for the production of leucine.
Surprisingly it was found, that the transgenic expression of the Linum usitatissimum protein as indicated in Table II, column 5, line 8 in a plant conferred an increase in valine content of the transformed plants. Thus, in one embodiment, said protein or its homologs are used for the production of valine.
Surprisingly it was found, that the transgenic expression of the Hordeum vulgare protein as indicated in Table II, column 5, line 9 in a plant conferred an increase in valine content of the transformed plants. Thus, in one embodiment, said protein or its homologs are used for the production of valine.

**[0021.0.0.3] see [0021.0.0.0]**

**[0022.0.3.3]** The sequence of YKR057W from Saccharomyces cerevisiae has been published in Dujon et al., Nature 369 (6479), 371-378, 1994 and Goffeau et al., Science 274 (5287), 546-547, 1996 and its activity is beeing defined as a ribosomal protein, similar to S21A, S26A and/or YS25 ribosomal proteins, involved in ribosome biogenesis and translation. Accordingly, in one embodiment, the process of the present invention comprises the use of a ribosomal protein, similar to S21A, S26A and/or YS25 ribosomal proteins, involved in ribosome biogenesis and translation from Saccaromyces cerevisiae or a plant or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a ribosomal protein, similar to S21A, S26A and/or YS25 ribosomal proteins, involved in ribosome biogenesis and translation is increased or generated, e.g. from Saccharomyces cerevisiae or a plant or a homolog thereof.
The sequence of YNL135C from Saccharomyces cerevisiae has been published in Philippsen, P., Nature 387 (6632 Suppl), 93-98, 1997 and Goffeau, A., Science 274 (5287), 546-547, 1996, and its activity is beeing defined as a peptidylprolyl isomerase. Accordingly, in one embodiment, the process of the present invention comprises the use of a peptidylprolyl isomerase or its homolog, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a peptidylprolyl isomerase is increased or generated, e.g. from Saccharomyces cerevisiae or a plant or a homolog thereof.
The sequence of b1343 from Escherichia coli K12 has been published in Blattner et al., Science 277(5331), 1453-1474, 1997, and its activity is being defined as an ATP-dependent RNA helicase. Accordingly, in one embodiment, the process of the present invention comprises the use of a protein with an ATP-dependent RNA helicase from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of an ATP-dependent RNA helicase is increased or generated, e.g. from E. coli or a plant or a homolog thereof.
The sequence of b3938 from Escherichia coli K12 has been published in Blattner et al., Science 277(5331), 1453-1474, 1997, and its activity is being defined as a transcriptional repressor for methionine biosynthesis . Accordingly, in one embodiment, the process of the present invention comprises the use of a transcriptional repressor activity for methionine biosynthesis from E. coli or a plant or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a transcriptional repressor for methionine biosynthesis is increased or generated, e.g. from E. coli or a plant or a homolog thereof.

**[0022.1.0.3] see [0022.1.0.0]**

**[0023.0.0.3]** for the disclosure of the paragraph [0023.0.0.3] see paragraph [0023.0.0.0] above.

**[0023.1.0.3]** Homologs of the polypeptide disclosed in table II, application no. 3, column 5, may have the activity of a protein as disclosed in table I or II, application no. 3, column 3 and may be the polypetides encoded by the nucleic acid molecules indicated in table I , application no. 3, column 7, resp., or may be the polypeptides indicated in table II, application no. 3, column 7, resp.

**[0024.0.0.3]** Further homologs of are described herein below.

**[0025.0.3.3]** In accordance with the invention, a protein or polypeptide has the "activity of an protein as shown in table II, application no. 3, column 3" if its de *novo* activity, or its increased expression directly or indirectly leads to an increased in the level of the fine chemical indicated in the respective line of table II, application no. 3, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 3, column 3, or which has at least 10% of the original enzymatic or biological activity, preferably 20%, particularly preferably 30%, most particularly preferably 40% in comparison to a protein as shown in the respective line of table II, application no. 3, column 3 of a E. coli or Saccharomyces cerevisae, respectively, protein as mentioned in table I to IV, column 3 respectively and as disclosed in paragraph [0022] of the respective invention..

**[0025.1.0.3]** In one embodiment, the polypeptide of the invention or the polypeptide used in the method of the invention confers said activity, e.g. the increase of the fine chemical in an organism or a part thereof, if it is derived from an organism, which is evolutionary distant to the organism in which it is expressed. For example origin and expressing organism are derived from different families, orders, classes or phylums.

**[0025.2.0.3]** In one embodiment, the polypeptide of the invention or the polypeptide used in the method of the invention confers said activity, e.g. the increase of the fine chemical in an organism or a part thereof, if it is derived from an organism, which is evolutionary close to the organism indicated in Table I, application no. 3, column 4 and is expressed in an organism, which is evolutionary distant to the origin organism. For example origin and expressing organism are derived from different families, orders, classes or phylums whereas origin and the organsim indicated in Table I, application no. 3, column 4 are derived from the same families, orders, classes or phylums.

**[0026.0.0.3] to [0033.0.0.3]: see [0026.0.0.0] to [0033.0.0.0]**

**[0034.0.3.3]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 3, column 3 its biochemical or genetical causes and the increased amount of the respective fine chemical.

**[0035.0.0.3] to [0044.0.0.3]: see [0035.0.0.0] to [0044.0.0.0]**

**[0045.0.3.3]** In one embodiment, the activity of the protein. as indicated in Table II, columns 5 or 7, application no. 3, is increased conferring an increase of the respective fine chemical, indicated in Table II, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more.

**[0046.0.3.3]** In one embodiment, the activity of the protein as indicated in Table II, columns 5 or 7, application no. 3, is increased conferring an increase of the respective fine chemical, indicated in Table II, column 6 of the respective line confers an increase of the respective fine chemical and of further amino acids or their precursors.

**[0047.0.0.3] see [0047.0.0.0]**

**[0048.0.0.3] see [0048.0.0.0]**

**[0049.0.3.3]** A protein having an activity conferring an increase in the amount or level of the fine chemical, has in one embodiment the structure of the polypeptide described herein, in particular of the polypeptides comprising the consensus sequence shown in table IV, application no. 3, column 7 or of the polypeptide as shown in the amino acid sequences as disclosed in table II, application no. 3, columns 5 and 7 or the functional homologues thereof as described herein, or is encoded by the nucleic acid molecule characterized herein or the nucleic acid molecule according to the invention, for example by the nucleic acid molecule as shown in table I, application no. 3, columns 5 and 7 or its herein described functional homologues and has the herein mentioned activity.

**[0050.0.3.3]** For the purposes of the present invention, the term "leucine" and/or "valine" and "L- leucine" and/or "L-valine" also encompass the corresponding salts, such as, for example, leucine- and/or valine- hydrochloride or leucine and/or valine sulfate. Preferably the term leucine and/or valine is intended to encompass the term L-leucine and/or L- valine.

**[0051.0.0.3] see [0051.0.0.0]**

**[0052.0.0.3] see [0052.0.0.0]**

**[0053.0.3.3]** In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 3, columns 5 and 7 or its homologs activity having herein-mentioned amino acids of the invention increasing activity; and/or
b) stabilizing a mRNA conferring the increased expression of a protein encoded by the nucleic acid molecule of the invention as shown in table I, application no. 3, columns 5 and 7, e.g. a nucleic acid sequence encoding a polypeptide having the activity of a protein as indicated in table II, application no. 3, columns 5 and 7 or its homologs activity or of a mRNA encoding the polypeptide of the present invention having herein-mentioned amino acids of the invention increasing activity; and/or
c) increasing the specific activity of a protein conferring the increased expression of a protein encoded by the nucleic acid molecule of the invention or of the polypeptide of the present invention having herein-mentioned amino acid increasing activity, e.g. of a polypeptide having the activity of a protein as indicated in table II, application no. 3, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned amino acids of the invention increasing activity, e.g. of a polypeptide having the activity of a protein as indicated in table II, application no. 3, columns 5 and 7 or its homologs activity; and/or
e) stimulating activity of a protein conferring the increased expression of a protein encoded by the nucleic acid molecule of the present invention or a polypeptide of the present invention having herein-mentioned amino acids of the invention increasing activity, e.g. of a polypeptide having the activity of a protein as indicated in table II, application no. 3, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned amino acids of the invention increasing activity, e.g. of a polypeptide having the activity of a protein as indicated in table II, application no. 3, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned amino acids of the invention increasing activity, e.g. of a polypeptide having the activity of a protein as indicated in table II, application no. 3, columns 5 and 7 or its homologs activity; and/or
h) increasing the expression of the endogenous gene encoding the polypeptide of the invention, e.g. a polypeptide having the activity of a protein as indicated in table II, application no. 3, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with especially high activity of the proteins of the invention from natural or from mutagenized resources and breeding them into the target organisms, eg the elite crops.

**[0054.0.2.3]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 3 in Table I to IV, resp., after increasing the expression or activity of the encoded polypeptide or having the activity of a polypeptide having an activity as the protein as shown in table II, application no. 3, column 3 or its homologs.

**[0055.0.0.3] to [0064.0.0.3] see [0055.0.0.0] to [0064.0.0.0]**

**[0065.0.3.3]** The activation of an endogenous polypeptide having above-mentioned activity, e.g.having the activity of a YKR057W, YNL135C, b1343 and/or b3983 protein or of the polypeptide of the invention, e.g. conferring the increase of leucine and/or valine after increase of expression or activity can also be increased by introducing a synthetic transcription factor, which binds close to the coding region of YKR057W, YNL135C, b1343 and/or b3983 protein encoding gene and activates its transcription. A chimeric zinc finger protein can be construed, which comprises a specific DNA-binding domain and an activation domain as e.g. the VP16 domain of Herpes Simplex virus. The specific binding domain can bind to the regulatory region of the YKR057W, YNL135C, b1343 and/or b3983 protein encoding gene. The expression of the chimeric transcription factor in a organism, in particular in a plant, leads to a specific expression of YKR057W, YNL135C, b1343 and/or b3983 protein, see e.g. in WO01/52620, Oriz, Proc. Natl. Acad. Sci. USA, 2002, Vol. 99, 13290 or Guan, Proc. Natl. Acad. Sci. USA, 2002, Vol. 99, 13296.

**[0066.0.0.3] to [0069.0.0.3]: see [0066.0.0.0] to [0069.0.0.0]**

**[0070.0.3.3]** Owing to the introduction of a gene or a plurality of genes conferring the expression of the nucleic acid molecule of the invention or the polypeptide of the invention, for example the nucleic acid construct mentioned below, or encoding the YKR057W, YNL135C, b1343 and/or b3983 protein into an organism alone or in combination with other genes, it is possible not only to increase the biosynthetic flux towards the end product, but also to increase, modify or create de *novo* an advantageous, preferably novel metabolites composition in the organism, e.g. an advantageous amino acid composition comprising a higher content of (from a viewpoint of nutrional physiology limited) amino acids, like tryptophane, methionine, lysine and/or threonine.

**[0071.0.0.3] see [0071.0.0.0]**

**[0072.0.3.3]** By influencing the metabolism thus, it is possible to produce, in the process according to the invention, further advantageous compounds,. Examples of such compounds are, in addition to leucine and/or valine 2-Acetolactate, 2-3-Dihydroxyisovalerate, 2-Oxoisovalerate, 3-Hydroxyisobutyrate, 3-Hydroxy-isobutyryl-CoA, Methylacrylyl-CoA, Isobutyryl-CoA, 2-Aceto-2-hydroxybutyrate, 2:3-Di-OH-3-methylvalerate, 2-Oxo-3-methylvalerate, 2-Methylacetoacetyl-CoA, 2-Methyl-3-hydroxybutyryl-CoA, Tiglyl-CoA, 2 Methylbutyryl-CoA, 22-isopropylmalate, 3-Isopropylmalate, Oxoleucine, Isovaleryl-CoA, 3-Methylcrotonyl-CoA and/or 3-Methylglutaconyl-CoA.

**[0073.0.3.3]** Accordingly, in one embodiment, the process according to the invention relates to a process which comprises:
a) providing a non-human organism, preferably a microorganism, a non-human animal, a plant or animal cell, a plant or animal tissue or a plant;
b) increasing the YKR057W, YNL135C, b1343 and/or b3983 protein activity or of a polypeptide being encoded by the nucleic acid molecule of the present invention and described below, e.g. conferring an increase of the fine chemical in the organism, preferably in the microorganism, the non-human animal, the plant or animal cell, the plant or animal tissue or the plant,
c) growing the organism, preferably the microorganism, the non-human animal, the plant or animal cell, the plant or animal tissue or the plant under conditions which permit the production of the fine chemical in the organism, preferably the microorganism, the plant cell, the plant tissue or the plant; and
d) if desired, revovering, optionally isolating, the free and/or bound the fine chemical and , optionally further free and/or bound amino acids synthetized by the organism, the microorganism, the non-human animal, the plant or animal cell, the plant or animal tissue or the plant.

**[0074.0.0.3] to [0084.0.0.3]: see [0074.0.0.0] to [0084.0.0.0]**

**[0085.0.3.3]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as depicted in Table I, application no. 3, columns 5 and 7, or a derivative thereof, or
b) a genetic regulatory element, for example a promoter, which is functionally linked to the nucleic acid sequence as depicted in Table I, application no. 3, columns 5 and 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.3]: see [0086.0.0.0]**

**[0087.0.0.3]: see [0087.0.0.0]**

**[0088.0.3.3]** In an advantageous embodiment of the invention, the organism takes the form of a plant whose amino acid content is modified advantageously owing to the nucleic acid molecule of the present invention expressed. This is important for plant breeders since, for example, the nutritional value of plants for monogastric animals is limited by a few essential amino acids such as lysine, threonine or methionine or tryptophane. After the YKR057W, YNL135C, b1343 and/or b3983 protein activity has been increased or generated, or after the expression of nucleic acid molecule or polypeptide according to the invention has been generated or increased, the transgenic plant generated thus is grown on or in a nutrient medium or else in the soil and subsequently harvested.

**[0089.0.0.3] to [0097.0.0.3]: see [0089.0.0.0] to [0097.0.0.0]**

**[0098.0.3.3]** In a preferred embodiment, the fine chemical (leucine and/or valine) is produced in accordance with the invention and, if desired, is isolated. The production of further amino acids such as methionine, lysine and/or threonine mixtures of amino acid by the process according to the invention is advantageous.

**[0099.0.0.3] to [0102.0.0.3]: see [0099.0.0.0] to [0102.0.0.0]**

**[0103.0.3.3]** In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide shown in Table II, application no. 3, columns 5 and 7, or a fragment thereof, which confers an increase in the amount of the fine chemical in an organism or a part thereof;
b) nucleic acid molecule comprising, preferably at least the mature form, of the nucleic acid molecule shown in Table I, application no. 3, columns 5 and 7,
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the fine chemical in an organism or a part thereof;
h) nucleic acid molecule comprising a nucleic acid molecule which is obtained by amplifying nucleic acid molecules from a cDNA library or a genomic library using the primers in Table III, application no. 3, column 7, and conferring an increase in the amount of the fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the fine chemical in an organism or a part thereof;
j) nucleic acid molecule which encodes a polypeptide comprising the consensus sequence shown in Table IV, application no. 3, column7, and conferring an increase in the amount of the fine chemical in an organism or a part thereof;
k) nucleic acid molecule comprising one or more of the nucleic acid molecule encoding the amino acid sequence of a polypeptide encoding a domain of the polypeptide shown in Table I, application no. 3, columns 5 and 7, and conferring an increase in the amount of the fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[0103.1.3.3]** In one embodiment, the nucleic acid molecule used in the process of the invention distinguishes over the sequence indicated in Table I, application no. 3, columns 5 or 7 by one or more nucleotides. In one embodiment, the nucleic acid molecule used in the process of the invention does not consist of the sequence shown in indicated in Table I ,application no. 3, columns 5 or 7,. In one embodiment, the nucleic acid molecule used in the process of the invention is less than 100%, 99,999%, 99,99%, 99,9% or 99% identical to a sequence indicated in Table I , application no. 3, columns 5 or 7,. In another embodiment, the nucleic acid molecule does not encode a polypeptide of a sequence indicated in Table II, application no. 3, columns 5 or 7.

**[0104.0.3.3]** In one embodiment, the nucleic acid molecule used in the process distinguishes over the sequence depicted in Table I, application no. 3, columns 5 and 7, by one or more nucleotides or does not consist of the sequence shown in Table I, application no. 3, columns 5 and 7.. In one embodiment, the nucleic acid molecule of the present invention is less than 100%, 99,999%, 99,99%, 99,9% or 99% identical to the sequence shown in Table I.. In another embodiment, the nucleic acid molecule does not encode a polypeptide of the sequence shown in Table II, application no. 3, preferably of sequences as shown in Table I, application no. 3, columns 5 or 7.

**[0105.0.0.3] to [0107.0.0.3]: see [0105.0.0.0] to [0107.0.0.0]**

**[0108.0.3.3]** Nucleic acid molecules with the sequence shown in Table I, , nucleic acid molecules which are derived from the amino acid sequences shown in Table II, application no. 3, columns 5 or 7, or from polypeptides comprising the consensus sequence shown in Table IV, application no. 3, columns 7, or their derivatives or homologues encoding polypeptides with the enzymatic or biological activity of an YKR057W, YNL135C, b1343 and/or b3983 protein or conferring a leucine and/or valine increase after increasing its expression or activity are advantageously increased in the process according to the invention.

**[0109.0.0.3] see [0109.0.0.0]**

**[0110.0.3.3]** Nucleic acid molecules, which are advantageous for the process according to the invention and which encode polypeptides with YKR057W, YNL135C, b1343 and/or b3983 protein activity can be determined from generally accessible databases.

**[0111.0.0.3] see [0111.0.0.0]**

**[0112.0.3.3]** The nucleic acid molecules used in the process according to the invention take the form of isolated nucleic acid sequences, which encode polypeptides with YKR057W, YNL135C, b1343 and/or b3983 protein activity and conferring a leucine and/or valine increase.

**[0113.0.0.3] to [0120.0.0.3]: see [0113.0.0.0] to [0120.0.0.0]**

**[0121.0.3.3]** However, it is also possible to use artificial sequences, which differ in one or more bases from the nucleic acid sequences found in organisms, or in one or more amino acid molecules from polypeptide sequences found in organisms, in particular from the polypeptide sequences shown in Table II, application no. 3, columns 5 or 7, or the functional homologues thereof as described herein, preferably conferring above-mentioned activity, i.e. conferring a leucine and/or valine increase after increasing its activity, e.g. having the activity of an YKR057W, YNL135C, b1343 and/or b3983 protein.

**[0122.0.0.3] to [127.0.0.3]: see [0122.0.0.0] to [0127.0.0.0]**

**[0128.0.3.3]** Synthetic oligonucleotide primers for the amplification, e.g. as shown in Table III, application no. 3, column 7, by means of polymerase chain reaction can be generated on the basis of a sequence shown herein, for example the sequence shown in Table I, application no. 3, columns 5 or 7, or the sequences derived from Table II, application no. 3.

**[0129.0.3.3]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequence shown in Table IV, application no. 3, column 7, is derived from said aligments.

**[0130.0.3.3]** Degenerated primers can then be utilized by PCR for the amplification of fragments of novel proteins having above-mentioned activity, e.g. conferring the increase of leucine and/or valine after increasing the expression or activity or having an YKR057W, YNL135C, b1343 and/or b3983 activity or further functional homologs of the polypeptide of the invention from other organisms.

**[0131.0.0.3] to [0138.0.0.3]: see [0131.0.0.0] to [0138.0.0.0]**

**[0139.0.3.3]** Polypeptides having above-mentioned activity, i.e. conferring the fine chemical increase, derived from other organisms, can be encoded by other DNA sequences which hybridize to the sequences shown in Table I, application no. 3, columns 5 or 7, under relaxed hybridization conditions and which code on expression for peptides having the leucine and/or valine increasing activity.

**[0140.0.0.3] to [0146.0.0.3]: see [0140.0.0.0] to [0146.0.0.0]**

**[0147.0.3.3]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 3, columns 5 or 7, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.3.3]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 3, columns 5 or 7 or a portion thereof and preferably has above mentioned activity, in particular, of valine and/or leucine increasing activity after increasing the acitivity or an activity of a product of a gene encoding said sequences or their homologs.

**[0149.0.3.3]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 3, columns 5 or 7, or a portion thereof and encodes a protein having above-mentioned activity and as indicated in indicated in Table II.

**[0150.0.3.3]** Moreover, the nucleic acid molecule of the invention can comprise only a portion of the coding region of one of the sequences indicated in Table I, application no. 3, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of valine and/or leucine, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 3, columns 5 or 7, , an anti-sense sequence of one of the sequences, e.g., as indicated in Table I, application no. 3, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 3, column 7, will result in a fragment of a polynucleotide sequence as indicated in Table I, application no. 3, columns 5 or 7 or its gene product.

**[0151.0.0.3] see [0151.0.0.0]**

**[0152.0.3.3]** The nucleic acid molecule of the invention encodes a polypeptide or portion thereof which includes an amino acid sequence which is sufficiently homologous to the amino acid sequence of Table II, application no. 3, columns 5 or 7, such that the protein or portion thereof maintains the ability to participate in the fine chemical production, in particular a leucine and/or valine increasing the activity as mentioned above or as described in the examples in plants or microorganisms is comprised.

**[0153.0.3.3]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in Table II, application no. 3, columns 5 or 7, such that the protein or portion thereof is able to participate in the increase of the fine chemical production. For examples having an YKR057W, YNL135C, b1343 and/or b3983 activity are described herein.

**[0154.0.3.3]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence of Table II, application no. 3, columns 5 or 7, and having above-mentioned activity, e.g.conferring preferably the increase of the fine chemical.

**[0155.0.0.3] and [0156.0.0.3] : see [0155.0.0.0] and [0156.0.0.0]**

**[0157.0.3.3]** The invention further relates to nucleic acid molecules that differ from one of the nucleotide sequences shown in Table I, application no. 3, columns 5 or 7, (and portions thereof) due to degeneracy of the genetic code and thus encode a polypeptide of the present invention, in particular a polypeptide having above mentioned activity, e.g. conferring an increase in the fine chemical in a organism, e.g. comprising the consensus sequences as indicated in Table IV, application no. 3, column 7, or of the polypeptide as indicated in Table II, application no. 3, columns 5 or 7, or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, application no. 3, column 7, or of the polypeptide as indicated in Table II, application no. 3, columns 5 or 7, or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, application no. 3, column 7, or of a polypeptide as indicated in Table II, application no. 3, columns 5 or 7, or the functional homologues thereof. However, in a preferred embodiment, the nucleic acid molecule of the present invention does not consist of a sequence as indicated in Table I, application no. 3, columns 5 or 7,

**[0158.0.0.3] to [0160.0.0.3]: see [0158.0.0.0] to [0160.0.0.0]**

**[0161.0.3.3]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising the sequence shown in Table I, application no. 3, columns 5 or 7.. The nucleic acid molecule is preferably at least 20, 30, 50, 100, 250 or more nucleotides in length.

**[0162.0.0.3]: see [0162.0.0.0]**

**[0163.0.3.3]** Preferably, nucleic acid molecule of the invention that hybridizes under stringent conditions to a sequence of Table I, application no. 3, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the fine chemical increase after increasing the expression or activity thereof or the activity of a protein of the invention or used in the process of the invention.

**[0164.0.0.3]: see [0164.0.0.0]**

**[0165.0.3.3]** For example, nucleotide substitutions leading to amino acid substitutions at "non-essential" amino acid residues can be made in a sequence of the nucleic acid molecule of the invention or used in the process of the invention, e.g. in Table I, application no. 3, columns 5 or 7.

**[0166.0.0.3] and [0167.0.0.3]: see [0166.0.0.0] and [0167.0.0.0]**

**[0168.0.3.3]** Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 3, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 3, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 3, columns 5 or 7, more preferably at least about 70% identical to one of the sequences as indicated in Table II, application no. 3, columns 5 or 7, ,even more preferably at least about 80%, 90%, or 95% homologous to a sequence as indicated in Table II, application no. 3, columns 5 or 7, , and most preferably at least about 96%, 97%, 98%, or 99% identical to the sequence as indicated in Table II, application no. 3, columns 5 or 7.

**[0169.0.0.3] to [0172.0.0.3]: see [0169.0.0.0] to [0172.0.0.0]**

**[0173.0.3.3]** For example a sequence which has a 80% homology with sequence SEQ ID NO: 268 at the nucleic acid level is understood as meaning a sequence which, upon comparison with the sequence SEQ ID NO: 268 by the above Gap program algorithm with the above parameter set, has a 80% homology.

**[0174.0.0.3]: see [0174.0.0.0]**

**[0175.0.3.3]** For example a sequence which has a 80% homology with sequence SEQ ID NO: 269 at the protein level is understood as meaning a sequence which, upon comparison with the sequence SEQ ID NO: 269 by the above program algorithm with the above parameter set, has a 80% homology.

**[0176.0.3.3]** Functional equivalents derived from one of the polypeptides as shown in Table II, application no. 3, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, application no. 3, columns 5 or 7, according to the invention and are distinguished by essentially the same properties as the polypeptide as shown in Table II, application no. 3, columns 5 or 7.

**[0177.0.3.3]** Functional equivalents derived from a nucleic acid sequence as indicated in Table I, application no. 3, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptide as indicated in Table II, application no. 3, columns 5 or according to the invention and encode polypeptides having essentially the same properties as a polypeptide as indicated in Table II, application no. 3, columns 5 or 7,.

**[0178.0.0.3] see [0178.0.0.0]**

**[0179.0.3.3]** A nucleic acid molecule encoding an homologous to a protein sequence of Table I, application no. 3, columns 5 or 7, preferably of Table II, application no. 3, column 7, can be created by introducing one or more nucleotide substitutions, additions or deletions into a nucleotide sequence of the nucleic acid molecule of the present invention, in particular of Table I, application no. 3, columns 5 or 7, such that one or more amino acid substitutions, additions or deletions are introduced into the encoded protein. Mutations can be introduced into the encoding sequences of Table I, application no. 3, columns 5 or 7, by standard techniques, such as site-directed mutagenesis and PCR-mediated mutagenesis.

**[0180.0.0.3] to [0183.0.0.3]: see [0180.0.0.0] to [0183.0.0.0]**

**[0184.0.3.3]** Homologues of the nucleic acid sequences used, with the sequence shown in Table I, application no. 3, columns 5 or 7, or of the nucleic acid sequences derived from the sequences Table I, application no. 3, columns 5 or 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from Table I, application no. 3, columns 5 or 7, , or from the derived nucleic acid sequences, the intention being, however, that the enzyme activity or the biological activity of the resulting proteins synthesized is advantageously retained or increased.

**[0185.0.3.3]** In one embodiment of the present invention, the nucleic acid molecule of the invention or used in the process of the invention comprises one or more sequences as indicated in Table I, application no. 3, columns 5 or 7,. In one embodiment, it is preferred that the nucleic acid molecule comprises as little as possible other nucleotides not shown in any one of sequences as indicated in Table I, application no. 3, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 3, columns 5 or 7.

**[0186.0.3.3]** Also preferred is that the nucleic acid molecule used in the process of the invention encodes a polypeptide comprising the sequence shown in Table II, application no. 3, columns 5 or 7.,ln one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment used in the inventive process, the encoded polypeptide is identical to the sequences shown in Table II.

**[0187.0.3.3]** In one embodiment, the nucleic acid molecule of the invention or used in the process encodes a polypeptide comprising the sequence shown in Table II, application no. 3, columns 5 or 7, comprises less than 100 further nucleotides. In a further embodiment, said nucleic acid molecule comprises less than 30 further nucleotides. In one embodiment, the nucleic acid molecule used in the process is identical to a coding sequence of the sequences shown in Table I, application no. 3, columns 5 or 7.

**[0188.0.3.3]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 3, columns 5 or 7 and is expressed under identical conditions.

**[0189.0.3.3]** Homologues of a sequences as indicated in Table I, application no. 3, columns 5 or 7, or of a derived sequences as indicated in Table II, application no. 3, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.3] to [0203.0.0.3]: see [0190.0.0.0] to [0203.0.0.0]**

**[0204.0.0.3]** Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 3, columns 5 or 7, or a fragment thereof conferring an increase in the amount of the respective fine chemical, in particular according to table II, application no. 3, column 6 in an organism or a part thereof
b) nucleic acid molecule comprising, preferably at least the mature form, of a nucleic acid molecule as indicated in Table I, application no. 3, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, application no. 3, column 7, and conferring an increase in the amount of the respective fine chemical, in particular according to table II, application no. 3, column 6 in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, application no. 3, columns 5 or 7, and conferring an increase in the amount of the respective fine chemical, in particular according to table II, application no. 3, column 6 in an organism or a part thereof;
k) nucleic acid molecule encoding the amino acid sequence of a polypeptide encoding a domaine of a polypeptide as indicated in Table II, application no. 3, columns 5 or 7, and conferring an increase in the amount of the respective fine chemical, in particular accccording to table II, application no. 3, column 6 in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 3, columns 5 or 7, or a nucleic acid molecule encoding, preferably at least the mature form of, a polypeptide as indicated in Table II, application no. 3, columns 5 or 7, and conferring an increase in the amount of the respective fine chemical according to table II, application no. 3, column 6 in an organism or a part thereof;
   or which encompasses a sequence which is complementary thereto;
whereby, preferably, the nucleic acid molecule according to (a) to (I) distinguishes over the sequence indicated in Table I, application no. 3,columns 5 or 7, by one or more nucleotides. In one embodiment, the nucleic acid molecule does not consist of the sequence shown and indicated in Table I, application no. 3, columns 5 or 7,
In one embodiment, the nucleic acid molecule is less than 100%, 99,999%, 99,99%, 99,9% or 99% identical to a sequence indicated in Table I, application no. 3, columns 5 or 7,.
In another embodiment, the nucleic acid molecule does not encode a polypeptide of a sequence indicated in Table II, application no. 3, columns 5 or 7,.
In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 3, columns 5 or 7,.
In a further embodiment the nucleic acid molecule does not encode a polypeptide sequence as indicated in Table II, application no. 3, columns 5 or 7,.
Accordingly, in one embodiment, the nucleic acid molecule of the differs at least in one or more residues from a nucleic acid molecule indicated in Table I, application no. 3, columns 5 or 7,.
Accordingly, in one embodiment, the nucleic acid molecule of the present invention encodes a polypeptide, which differs at least in one or more amino acids from a polypeptide indicated in Table II, application no. 3, columns 5 or 7,.
In another embodiment, a nucleic acid molecule indicated in Table I, application no. 3, columns 5 or 7,.
Accordingly, in one embodiment, the protein encoded by a sequences of a nucleic acid accoriding to (a) to (I) does not consist of a sequence as indicated in Table II, application no. 3, columns 5 or 7,.
In a further embodiment, the protein of the present invention is at least 30 %, 40 %, 50%, or 60% identical to a protein sequence indicated in Table II, application no. 3, columns 5 or 7, and less than 100%, preferably less than 99,999%, 99,99% or 99,9%, more preferably less than 99%, 985, 97%, 96% or 95% identical to a sequence as indicated in Table I, columns 5 or 7.

**[0205.0.0.3] to [0226.0.0.3]: see [0205.0.0.0] to [0226.0.0.0]**

**[0227.0.3.3]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.

In addition to the sequence mentioned in Table I, application no. 3, columns 5 or 7, or its derivatives, it is advantageous additionally to express and/or mutate further genes in the organisms. Especially advantageously, additionally at least one further gene of the amino acid biosynthetic pathway such as for L-lysine, L-threonine and/or L-methionine or L-leucine and/or valine is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine the sequences shown in Table I, application no. 3, columns 5 or 7, with genes which generally support or enhances to growth or yield of the target organismen, for example genes which lead to faster growth rate of microorganisms or genes which produces stress-, pathogen, or herbicide resistant plants.

**[0228.0.0.3] to [0230.0.0.3]: see [0228.0.0.0] to [0230.0.0.0]**

**[0231.0.0.3]** In a further advantageous embodiment of the process of the invention, the organisms used in the process are those in which simultaneously a leucine and/or valine degrading protein is attenuated, in particular by reducing the rate of expression of the corresponding gene.

**[0232.0.0.3] to [0282.0.0.3]: see [0232.0.0.0] to [0282.0.0.0]**

**[0283.0.3.3]** Moreover, native polypeptide conferring the increase of the fine chemical in an organism or part thereof can be isolated from cells (e.g., endothelial cells), for example using the antibody of the present invention as described below, in particular, an anti- YKR057W, YNL135C, b1343 and/or b3983 protein antibody or an antibody against polypeptides as shown in Table II, application no. 3, columns 5 or 7, , which can be produced by standard techniques utilizing the polypeptid of the present invention or fragment thereof, i.e., the polypeptide of this invention. Preferred are monoclonal antibodies.

**[0284.0.0.3]: see [0284.0.0.0]**

**[0285.0.3.3]** In one embodiment, the present invention relates to a polypeptide having the sequence shown in Table II, application no. 3, columns 5 or 7, or as coded by the nucleic acid molecule shown in Table I" application no. 3, columns 5 or 7, or functional homologues thereof.

**[0286.0.3.3]** In one advantageous embodiment, in the method of the present invention the activity of a polypeptide is increased comprising or consisting of the consensus sequence shown in Table IV, application no. 3, and in one another embodiment, the present invention relates to a polypeptide comprising or consisting of the consensus sequence shown in Table IV, application no. 3,
whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent.

**[0287.0.0.3] to [0290.0.0.3]: see [0287.0.0.0] to [0290.0.0.0]**

**[0291.0.3.3]** In one advantageous embodiment, the method of the present invention comprises the increasing of a polypeptide comprising or consisting of plant or microorganism specific consensus sequences.

In one embodiment, said polypeptide of the invention distinguishes over a sequence as indicated in Table II, application no. 3, columns 5 or 7, by one or more amino acids. In one embodiment, polypeptide distinguishes form a sequence as indicated in Table II, application no. 3, columns 5 or 7, by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 3, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II, application no. 3, columns 5 or 7,.

**[0292.0.0.3]: see [0292.0.0.0]**

**[0293.0.3.3]** In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II, application no. 3, columns 5 or 7, by one or more amino acids. In an other embodiment, said polypeptide of the invention does not consist of the sequence as indicated in Table II, application no. 3, columns 5 or 7,.
In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 3, columns 5 or 7.

**[0294.0.3.3]** In one embodiment, the present invention relates to a polypeptide having an activity of a protein as indicated in Table II, application no. 3, column 3, which distinguishes over a sequence as indicated in Table II, application no. 3, columns 5 or 7, by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.3] to [0297.0.0.3]: see [0295.0.0.0] to [0297.0.0.0]**

**[0297.0.3.3]** The language "substantially free of cellular material" includes preparations of the polypeptide of the invention in which the protein is separated from cellular components of the cells in which it is naturally or recombinantly produced. In one embodiment, the language "substantially free of cellular material" includes preparations having less than about 30% (by dry weight) of "contaminating protein", more preferably less than about 20% of "contaminating protein", still more preferably less than about 10% of "contaminating protein", and most preferably less than about 5% "contaminating protein". The term "Contaminating protein" relates to polypeptides, which are not polypeptides of the present invention. When the polypeptide of the present invention or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations in which the polypeptide of the present invention is separated from chemical precursors or other chemicals which are involved in the synthesis of the protein. The language "substantially free of chemical precursors or other chemicals" includes preparations having less than about 30% (by dry weight) of chemical precursors or non- YKR057W, YNL135C, b1343 and/or b3983 chemicals, more preferably less than about 20% chemical precursors or non- YKR057W, YNL135C, b1343 and/or b3983 chemicals, still more preferably less than about 10% chemical precursors or non- YKR057W, YNL135C, b1343 and/or b3983 chemicals, and most preferably less than about 5% chemical precursors or non- YKR057W, YNL135C, b1343 and/or b3983 chemicals. In preferred embodiments, isolated proteins or biologically active portions thereof lack contaminating proteins from the same organism from which the polypeptide of the present invention is derived. Typically, such proteins are produced by recombinant techniques-.

**[00297.1.0.3]** Non-polypeptide of the invention-chemicals are e.g. polypeptides having not the activity and/or the amino acid sequence of a polypeptide indicated in Table II, application no. 3, columns 3, 5 or 7.

**[0298.0.3.3]** A polypeptide of the invention can participate in the process of the present invention. The polypeptide or a portion thereof comprises preferably an amino acid sequence which is sufficiently homologous to an amino acid sequence shown in Table II, application no. 3, columns 5 or 7, such that the protein or portion thereof maintains the ability to confer the activity of the present invention. The portion of the protein is preferably a biologically active portion as described herein. Preferably, the polypeptide used in the processof the invention has an amino acid sequence identical as shown in Table II, application no. 3, columns 5 or 7, .

**[0299.0.3.3]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences of Table II, application no. 3, columns 5 or 7, . The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table I, application no. 3, columns 5 or 7, or which is homologous thereto, as defined above.

**[0300.0.3.3]** In one embodiment, the present invention relates to a polypeptide having an activity of a protein as indicated in Table II, application no. 3, column 3, which distinguishes over a sequence as indicated in Table II, application no. 3, columns 5 or 7, by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0301.0.0.3] : see [0301.0.0.0]**

**[0302.0.3.3]** Biologically active portions of an polypeptide of the present invention include peptides comprising amino acid sequences derived from the amino acid sequence of the polypeptide of the present invention or used in the process of the present invention, e.g., the amino acid sequence shown in Table II, application no. 3, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.3]: see [0303.0.0.0]**

**[0304.0.3.3]** Manipulation of the nucleic acid molecule of the invention may result in the production of YKR057W, YNL135C, b1343 and/or b3983 protein having differences from the wild-type YKR057W, YNL135C, b1343 and/or b3983 protein. These proteins may be improved in efficiency or activity, may be present in greater numbers in the cell than is usual, or may be decreased in efficiency or activity in relation to the wild type protein.

**[0305.0.0.3] : see [0305.0.0.0]**

**[0305.0.3.3]** Any mutagenesis strategies for the polypeptide of the present invention or the polypeptide used in the process of the present invention to result in increasing said activity are not meant to be limiting; variations on these strategies will be readily apparent to one skilled in the art. Using such strategies, and incorporating the mechanisms disclosed herein, the nucleic acid molecule and polypeptide of the invention may be utilized to generate plants or parts thereof, expressing wildtype YKR057W, YNL135C, b1343 and/or b3983 proteins or mutated YKR057W, YNL135C, b1343 and/or b3983 protein encoding nucleic acid molecules and polypeptide molecules of the invention such that the yield, production, and/or efficiency of production of a desired compound is improved.

**[0306.0.0.3] to [0308.0.0.3]: see [0306.0.0.0] to [0308.0.0.0]**

**[0309.0.3.3]** In one embodiment, an " YKR057W, YNL135C, b1343 and/or b3983 protein (= polypeptide)" refers to a polypeptide having an amino acid sequence corresponding to the polypeptide of the invention or used in the process of the invention , whereas a "non- YKR057W, YNL135C, b1343 and/or b3983 polypeptide" or "other polypeptide" refers to a polypeptide having an amino acid sequence corresponding to a protein which is not substantially homologous a polypeptide of the invention, preferably which is not substantially homologous to a polypeptide having an YKR057W, YNL135C, b1343 and/or b3983 protein activity, e.g., a protein which does not confer the activity described herein and which is derived from the same or a different organism.

**[0310.0.0.3] to [0334.0.0.3]: see [0310.0.0.0] to [0334.0.0.0]**

**[0335.0.3.3]** The dsRNAi method has proved to be particularly effective and advantageous for reducing the expression of the nucleic acid sequences of the Table I, , application no. 3, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of the nucleic acid sequences of the Table I, application no. 3, columns 5 or 7, and/or homologs thereof. In a double-stranded RNA molecule for reducing the expression of an protein encoded by a nucleic acid sequence of one of the Table I, application no. 3, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.3] to [0342.0.0.3]: see [0336.0.0.0] to [0342.0.0.0]**

**[0343.0.3.3]** As has already been described, 100 % sequence identity between the dsRNA and a gene transcript of a nucleic acid sequence of one of the Table I, application no. 3, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of Table I, application no. 3, columns 5 or 7, or homologs thereof of the one organism, may be used to suppress the corresponding expression in another organism.

**[0344.0.0.3] to [0361.0.0.3]: see [0344.0.0.0] to [0361.0.0.0]**

**[0362.0.3.3]** Accordingly the present invention relates to any cell transgenic for any nucleic acid characterized as part of the invention, e.g. conferring the increase of the fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. encoding a polypeptide having an YKR057W, YNL135C, b1343 and/or b3983 protein activity. Due to the above mentioned activity the fine chemical content in a cell or an organism is increased. For example, due to modulation or manupulation, the cellular activity is increased, e.g. due to an increased expression or specific activity of the subject matters of the invention in a cell or an organism or a part thereof. Transgenic for a polypeptide having an YKR057W, YNL135C, b1343 and/or b3983 protein or activity means herein that due to modulation or manipulation of the genome, the activity of YKR057W, YNL135C, b1343 and/or b3983 or a YKR057W, YNL135C, b1343 and/or b3983 -like activity is increased in the cell or organism or part thereof. Examples are described above in context with the process of the invention.

**[0363.0.0.3]: see [0363.0.0.0]**

**[0364.0.3.3]** A naturally occurring expression cassette - for example the naturally occurring combination of the YKR057W, YNL135C, b1343 and/or b3983 protein promoter with the corresponding YKR057W, YNL135C, b1343 and/or b3983 protein gene - becomes a transgenic expression cassette when it is modified by non-natural, synthetic "artificial" methods such as, for example, mutagenization. Such methods have been described (US 5,565,350; WO 00/15815; also see above).

**[0365.0.0.3] to [0382.0.0.3]: see [0365.0.0.0] to [0382.0.0.0]**

**[0383.0.3.3]** For preparing branched-chain amino acid compound-containing fine chemicals, in particular the fine chemical, it is possible to use as branched-chain amino acid source organic branched-chain amino acid-containing compounds such as, for example, isovalerate, isopropylmalate, oxoisocaproate, isovaleryl-compounds, methyl-valerate, isobutyrate, methyl-butyryl-compounds, isopropyrate, isopropyl-compounds or else organic branched-chain amino acid precursor compounds.

**[0384.0.0.3] to [0392.0.0.3]: see [0384.0.0.0] to [0392.0.0.0]**

**[0393.0.3.3]** In one embodiment, the present invention relates to a method for the identification of a gene product conferring an increase in the fine chemical production in a cell, comprising the following steps:
a) contacting_{;} e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
b) identifying the nucleic acid molecules, which hybridize under relaxed stringent conditions with the nucleic acid molecule of the present invention in particular to the nucleic acid molecule sequence as indicated in Table I, application no. 3, columns 5 or 7, and, optionally, isolating the full length cDNA clone or complete genomic clone;
c) introducing the candidate nucleic acid molecules in host cells, preferably in a plant cell or a microorganism, appropriate for producing the fine chemical;
d) expressing the identified nucleic acid molecules in the host cells;
e) assaying the the fine chemical level in the host cells; and
f) identifying the nucleic acid molecule and its gene product which expression confers an increase in the the fine chemical level in the host cell after expression compared to the wild type.

### [0394.0.0.3] to [0552.0.0.3]: see [0394.0.0.1] to [0552.0.0.1]

**[00553.0.3.3]** A process for the production of valine and/or leucine resp., which comprises
(a) increasing or generating the activity of a protein as indicated in Table II, application no. 3, columns 5 or 7, or a functional equivalent thereof in a non-human organism, or in one or more parts thereof; and
(b) growing the organism under conditions which permit the production of valine and/or leucine resp. in said organism.
A process for the production of valine and/or leucine resp., comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 3, columns 5 or 7, , or a fragment thereof, which confers an increase in the amount of the fine chemical as indicated in table II, column 6, e.g of valine and/or leucine resp., in an organism or a part thereof;
b) nucleic acid molecule comprising of a nucleic acid molecule as indicated in Table I, application no. 3, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of valine and/or leucine resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of valine and/or leucine resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of valine and/or leucine resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 3, column 7, and conferring an increase in the amount of the fine chemical as indicated in table II, column 6, e.g of valine and/or leucine resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of valine and/or leucine resp., in an organism or a part thereof;
h) nucleic acid molecule encoding a polypeptide comprising a consensus sequence as indicated in Table IV, application no. 3, column 7, and conferring an increase in the amount of the fine chemical as indicated in table II, column 6, e.g of valine and/or leucine resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical as indicated in table II, column 6, e.g of valine and/or leucine resp., in an organism or a part thereof.
or comprising a sequence which is complementary thereto.
The process as mentioned above, comprising recovering of the free or bound valine and/or leucine resp..
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound valine and/or leucine resp., produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 3, columns 5 or 7, or a fragment thereof, which confers an increase in the amount of the fine chemical as indicated in table II, column 6, e.g of valine and/or leucine resp., in an organism or a part thereof;
b) nucleic acid molecule comprising of a nucleic acid molecule as indicated in Table I, application no. 3, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of valine and/or leucine resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of valine and/or leucine resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of valine and/or leucine resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 3, column 7, and conferring an increase in the amount of the fine chemical as indicated in table II, column 6, e.g of valine and/or leucine resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of valine and/or leucine resp., in an organism or a part thereof;
h) nucleic acid molecule encoding a polypeptide comprising a consensus sequence as indicated in Table IV, application no. 3, column 7, and conferring an increase in the amount of the fine chemical as indicated in table II, column 6, e.g of valine and/or leucine resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemiccal as indicated in table II, column 6, e.g of valine and/or leucine resp., in an organism or a part thereof.
whereby the nucleic acid molecule distinguishes over the sequence as indicated in Table I, application no. 3, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 3, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of valine and/or leucine resp., in an organism or a part thereof comprising:
(a) contacting cells, tissues , plants or microorganisms which express the a polypeptide encoded by the nucleic acid molecule as mentioned above conferring an increase in the amount of valine and/or leucine resp., in an organism or a part thereof with a candidate compound or a sample comprising a plurality of compounds under conditions which permit the expression the polypeptide;
(b) assaying the valine and/or leucine resp., level or the polypeptide expression level in the cell, tissue, plant or microorganism or the media the cell, tissue, plant or microorganisms is cultured or maintained in; and
(c) identifying a agonist or antagonist by comparing the measured valine and/or leucine resp., level or polypeptide expression level with a standard valine and/or leucine resp., or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased valine and/or leucine resp., production in a plant or microorganism, comprising the steps:
(a) culturing a plant cell or tissue or microorganism or maintaining a plant expressing the polypeptide encoded by the nucleic acid molecule as mentioned above conferring an increase in the amount of valine and/or leucine resp., in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of valine and/or leucine rersp., in an organism or a part thereof;
(b) identifying if the compound is an effective agonist by detecting the presence or absence or increase of a signal produced by said readout system.
A method for the identification of a gene product conferring an increase in valine and/or leucine resp., production in a cell, comprising the following steps:
(a) contacting the nucleic acid molecules of a sample, which can contain a candidate gene encoding a gene product conferring an increase in valine and/or leucine resp., after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing valine and/or leucine resp.;
(d) expressing the identified nucleic acid molecules in the host cells;
(e) assaying the valine and/or leucine rersp., level in the host cells; and
(f) identifying nucleic acid molecule and its gene product which expression confers an increase in the valine and/or leucine resp., level in the host cell in the host cell after expression compared to the wild type.
A method for the identification of a gene product conferring an increase in valine and/or leucine resp., production in a cell, comprising the following steps:
(a) identifiying in a data bank nucleic acid molecules of an organism; which can contain a candidate gene encoding a gene product conferring an increase in the valine and/or leucine resp., amount or level in an organism or a part thereof after expression, and which are at least 20% homolog to the nucleic acid molecule as mentioned above;
(b) introducing the candidate nucleic acid molecules in host cells appropriate for producing valine and/or leucine resp.,;
(c) expressing the identified nucleic acid molecules in the host cells;
(d) assaying the valine and/or leucine resp., level in the host cells; and
(e) identifying nucleic acid molecule and its gene product which expression confers an increase in the valine and/or leucine resp., level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of valine and/or leucine resp., after expression.
Use of the polypeptide as mentioned above or the nucleic acid construct as mentioned above or the gene product identified according to the method as mentioned above for identifying compounds capable of conferring a modulation of valine and/or leucine resp., levels in an organism.
Food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the protection of a plant against a valine and/or leucine synthesis inhibiting herbicide.

### New genes for a process for the production of fine chemicals

**[0000.0.0.4]** In a further embodiment, the present invention relates to a further process for the production of fine chemicals as defined below and corresponding embodiments as described herein as follows.

**[0001.0.0.4] to [0008.0.0.4]: see [0001.0.0.0] to [0008.0.0.0]**

**[0009.0.4.4]** As described above, the essential amino acids are necessary for humans and many mammals, for example for livestock. Arginine is a semi-essential amino acid involved in multiple areas of human physiology and metabolism. It is not considered essential because humans can synthesize it de novo from glutamine, glutamate, and proline. However, dietary intake remains the primary determinant of plasma arginine levels, since the rate of arginine biosynthesis does not increase to compensate for depletion or inadequate supply. Dietary arginine intake regulates whole body arginine synthesis from proline in the neonatal piglet. The maximal rate of arginine synthesis (0.68g/kg/d) is not enough to supply the whole body metabolic requirement for arginine in the young pig. In animals, glutamate functions as a neurotransmitter and activates glutamate receptor cation channels (iGluRs), which trigger electrical or Ca²⁺ signal cascades. In plants, amino acids are involved in signalling of both plant nitrogen status and plant nitrogen : carbon ratios. Endogenous glutamine has been implicated in feedback inhibition of root N uptake, via the suppression of transcription of genes encoding inorganic nitrogen transporters (Rawat et al., Plant Journal 19: 143-152, 1999; Zhuo et al., Plant Journal 17: 563-568, 1999). The nonessential amino acid, proline, is synthesized from L-ornithine or L-glutamate. The proline from L-ornithine is linked to protein metabolism in the urea cycle and the proline from L-glutamate is linked to carbohydrate metabolism. Collagen is the major reservoir for proline in the body. Vitamin C should be used with proline for collagen problems.

**[0010.0.0.4] to [0011.0.0.4]: see [0010.0.0.0] to [0011.0.0.0]:**

**[0012.0.4.4]** It is an object of the present invention to develop an inexpensive process for the synthesis of arginine and/or glutamate and/or glutamine and/or proline, preferably L- arginine and/or L- glutamate and/or L-glutamine and/or L- proline.

**[0013.0.0.4]: see [0013.0.0.0]**

**[0014.0.4.4]** Accordingly, in a first embodiment, the invention relates to a process for the production of a fine chemical, whereby the fine chemical is arginine and/or glutamate and/or glutamine and/or proline, preferably L- arginine and/or L- glutamate and/or L-glutamine and/or L- proline. Accordingly, in the present invention, the term "the fine chemical" as used herein relates to "arginine and/or glutamate and/or glutamine and/or proline". Further, the term "the fine chemicals" as used herein also relates to fine chemicals comprising arginine and/or glutamate and/or glutamine and/or proline.

**[0015.0.4.4]** In one embodiment, the term "the fine chemical" means arginine and/or glutamate and/or glutamine and/or proline, preferably L- arginine and/or L- glutamate and/or L-glutamine and/or L- proline. Throughout the specification the term "the fine chemical" means arginine and/or glutamate and/or glutamine and/or proline, preferably L- arginine and/or L- glutamate and/or L-glutamine and/or L- proline, its salts, ester or amids in free form or bound to proteins. In a preferred embodiment, the term "the fine chemical" means arginine and/or glutamate and/or glutamine and/or proline, preferably L- arginine and/or L- glutamate and/or L-glutamine and/or L- proline, in free form or its salts or bound to proteins.

**[0016.0.4.4]** Accordingly, the present invention relates to a process for the production of the respective fine chemical comprising
(a) increasing or generating the activity of one or more of a protein as shown in table II, application no. 4, column 3 encoded by the nucleic acid sequences as shown in table I, application no. 4, column 5, in a non-human organism or in one one or more parts thereof or
(b) growing the organism under conditions which permit the production of the fine chemical, thus amino acids of the invention or fine chemicals comprising amino acids of the invention, in said organism or in the culture medium surrounding the organism.

**[0016.1.4.4]** Accordingly, the term "the fine chemical" means in one embodiment "arginine" in relation to all sequences listed in Tables I to IV, line 10 or homologs thereof and
means in one embodiment "proline" in relation to all sequences listed in Table I to IV, line 11 or homologs thereof and
means in one embodiment "glutamine" in relation to all sequences listed in Table I to IV, lines 12 and/or 14 to 15 or homologs thereof and
means in one embodiment "glutamate" in relation to all sequences listed in Table I to IV, line 13 or homologs thereof.
Accordingly, in one embodiment the term "the fine chemical" means "arginine" and "glutamine" in relation to all sequences listed in Table I to IV, lines 10 and/or 12;
in one embodiment the term "the fine chemical" means "glutamate" and "glutamine" in relation to all sequences listed in Table I to IV, lines 13 and/or 14;

**[0017.0.0.4] to [0019.0.0.4]: see [0017.0.0.0]to [0019.0.0.0]**

**[0020.0.4.4]** Surprisingly it was found, that the transgenic expression of the Zea mays protein as indicated in Table II, column 5, lines 10 and 12 in a plant conferred an increase in arginine and/or glutamine (or the respective fine chemical) content of the transformed plants. Thus, in one embodiment, said protein or its homologs are used for the production of arginine; in one embodiment, said protein or its homologs are used for the production of glutamine, in one embodiment, said protein or its homologs are used for the production of one or more fine chemical selected from the group consisting of arginine and/or glutamine.

Surprisingly it was found, that the transgenic expression of the Brassica napus protein as indicated in Table II, column 5, line11 in a plant conferred an increase in proline content of the transformed plants. Thus, in one embodiment, said protein or its homologs are used for the production of proline.

Surprisingly it was found, that the transgenic expression of the Linum usitatissimum protein as indicated in Table II, column 5, lines 13 and 14 in a plant conferred an increase in glutamate and/or glutamine (or the respective fine chemical) content of the transformed plants. Thus, in one embodiment, said protein or its homologs are used for the production of glutamate; in one embodiment, said protein or its homologs are used for the production of glutamine, in one embodiment, said protein or its homologs are used for the production of one or more fine chemical selected from the group consisting of glutamate and/or glutamine.

Surprisingly it was found, that the transgenic expression of the Brassica napus protein as indicated in Table II, column 5, line 15 in a plant conferred an increase in glutamine content of the transformed plants. Thus, in one embodiment, said protein or its homologs are used for the production of glutamine.

**[0021.0.0.4]: see [0021.0.0.0]**

**[0022.0.4.4]** The sequence of YGR135W from Saccharomyces cerevisiae has been published in Goffeau st al., Science 274 (5287), 546-547, 1996 and Tettelin et al., Nature 387 (6632 Suppl), 81-84 (1997) and its activity is being defined as a "proteasome component Y13". Accordingly, in one embodiment, the process of the present invention comprises the use of a a gene product with an activity of multicatalytic endopeptidase complex chain C9 superfamily, preferably a protein with proteasome component Y13 activity, from Saccharomyces cerevisiae or a plant or its homolog, e.g. as shown herein, for the production of the fine chemical, meaning of proline, in particular for increasing the amount of proline, preferably proline in free or bound form in an organism or a part thereof, as mentioned.
The sequence of YKR057W from Saccharomyces cerevisiae has been published in Dujon et al., Nature 369 (6479), 371-378, 1994 and Goffeau et al., Science 274 (5287), 546-547, 1996 and its activity is being defined as a ribosomal protein, similar to S21 ribosomal proteins, involved in ribosome biogenesis and translation. Accordingly, in one embodiment, the process of the present invention comprises the use of a gene product with an activity of rat ribosomal protein S21 superfamily, preferably a protein with a ribosomal protein, similar to S21 ribosomal proteins, involved in ribosome biogenesis and translation activity from Saccharomyces cerevisiae or a plant or its homolog, e.g. as shown herein, for the production of the fine chemical, meaning of arginine and/or glutamine , in particular for increasing the amount of arginine and/or glutamine , in particular for increasing the amount of arginine, in particular for increasing the amount of glutamine, in particular for increasing the amount of arginine and glutamine, preferably arginine and/or glutamine in free or bound form in an organism or a part thereof, as mentioned.
The sequence of b1343 (Accession number NP_415490) from Escherichia coli K12 has been published in Blattner et al., Science 277 (5331), 1453-1474, 1997, and its activity is being defined as a ATP-dependent RNA helicase, stimulated by 23S rRNA. Accordingly, in one embodiment, the process of the present invention comprises the use of a gene product with an activity of Escherichia coli b1343 protein, preferably a protein with the activity of a ATP-dependent RNA helicase, stimulated by 23S rRNA from E. coli or a plant or its homolog, e.g. as shown herein, for the production of the fine chemical, meaning of glutamine and/or glutamate, in particular for increasing the amount of glutamine, in particular for increasing the amount of glutamate, in particular for increasing the amount of glutamine and glutamate, preferably glutamine and/or glutamate in free or bound form in an organism or a part thereof, as mentioned.
The sequence of b2426 (Accession number NP_416921) from Escherichia coli K12 has been published in Blattner et al., Science 277 (5331), 1453-1474, 1997, and its activity is being defined as a putative oxidoreductase with NAD(P)-binding domain. Accordingly, in one embodiment, the process of the present invention comprises the use of a gene product with an activity of ribitol dehydrogenase, short-chain alcohol dehydrogenase homology superfamily, preferably a protein with the activity of a putative oxidoreductase with NAD(P)-binding domain from E. coli or a plant or its homolog, e.g. as shown herein, for the production of the fine chemical, meaning of glutamine, in particular for increasing the amount of glutamine, preferably glutamine in free or bound form in an organism or a part thereof, as mentioned.

**[0022.1.0.7] to [0023.0.0.4] see [0022.1.0.0] to [0023.0.0.0]**

**[0023.1.4.4]** Homologs of the polypeptide disclosed in table II, application no. 4, column 5, may have the activity of a protein as disclosed in table I or II, application no. 4, column 3 and may be the polypetides encoded by the nucleic acid molecules indicated in table I , application no. 4, column 7, resp., or may be the polypeptides indicated in table II, application no. 4, column 7, resp.

### [0024.0.0.4]: see [0024.0.0.0]

**[0025.0.4.4]** In accordance with the invention, a protein or polypeptide has the "activity of an protein as shown in table II, application no. 4, column 3" if its de *novo* activity, or its increased expression directly or indirectly leads to an increased in the level of the fine chemical indicated in the respective line of table II, application no. 4, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 4, column 3, or which has at least 10% of the original enzymatic or biological activity, preferably 20%, particularly preferably 30%, most particularly preferably 40% in comparison to a protein as shown in the respective line of table II, application no. 4, column 3 of a E. coli or Saccharomyces cerevisae, respectively, protein as mentioned in table I to IV, column 3 respectively and as disclosed in paragraph [0022] of the respective invention.

**[0025.1.0.4] to [0033.0.0.4]: see [0025.1.0.0] to [0033.0.0.0]**

**[0034.0.4.4]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 4, column 3 its biochemical or genetical causes and the increased amount of the respective fine chemical.

**[0035.0.0.4] to [0044.0.0.4]: see [0035.0.0.0] to [0044.0.0.0]**

**[0045.0.4.4]** In one embodiment, the activity of the protein. as indicated in Table II, columns 5 or 7, application no. 4, is increased conferring an increase of the respective fine chemical, indicated in Table II, column 6 of the respective line, between 10% and

50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more.

**[0046.0.4.4]** In one embodiment, the activity of the protein as indicated in Table II, columns 5 or 7, application no. 4, is increased conferring an increase of the respective fine chemical, indicated in Table II, column 6 of the respective line confers an increase of the respective fine chemical and of further amino acids or their precursors.

**[0047.0.0.4] and [0048.0.0.4]: see [0047.0.0.0] and [0048.0.0.0]**

**[0049.0.4.4]** A protein having an activity conferring an increase in the amount or level of the fine chemical, has in one embodiment the structure of the polypeptide described herein, in particular of the polypeptides comprising the consensus sequence shown in table IV, application no. 4, column 7 or of the polypeptide as shown in the amino acid sequences as disclosed in table II, application no. 4, columns 5 and 7 or the functional homologues thereof as described herein, or is encoded by the nucleic acid molecule characterized herein or the nucleic acid molecule according to the invention, for example by the nucleic acid molecule as shown in table I, application no. 4, columns 5 and 7 or its herein described functional homologues and has the herein mentioned activity.

**[0050.0.4.4]** For the purposes of the present invention, the term "arginine" and/or "glutamate" and/or "glutamine" and/or "proline" and "L- arginine" and/or" L- glutamate" and/or "L-glutamine" and/or "L-proline" also encompass the corresponding salts, such as, for example, arginine- and/or glutamate- and/or glutamine- and/or proline- hydrochloride or arginine and/or glutamate and/or glutamine and/or proline sulfate. Preferably the term arginine and/or glutamate and/or glutamine and/or proline is intended to encompass the term L- arginine and/or L- glutamate and/or L-glutamine and/or L- proline.

**[0051.0.0.4] and [0052.0.0.4]: see [0051.0.0.0] and [0052.0.0.0]**

**[0053.0.4.4]** In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 4, columns 5 and 7 or its homologs activity having herein-mentioned amino acids of the invention increasing activity; and/or
b) stabilizing a mRNA conferring the increased expression of a protein encoded by the nucleic acid molecule of the invention as shown in table I, application no. 4, columns 5 and 7, e.g. a nucleic acid sequence encoding a polypeptide having the activity of a protein as indicated in table II, application no. 4, columns 5 and 7 or its homologs activity or of a mRNA encoding the polypeptide of the present invention having herein-mentioned amino acids of the invention increasing activity; and/or
c) increasing the specific activity of a protein conferring the increased expression of a protein encoded by the nucleic acid molecule of the invention or of the polypeptide of the present invention having herein-mentioned amino acid increasing activity, e.g. of a polypeptide having the activity of a protein as indicated in table II, application no. 4, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned amino acids of the invention increasing activity, e.g. of a polypeptide having the activity of a protein as indicated in table II, application no. 4, columns 5 and 7 or its homologs activity; and/or
e) stimulating activity of a protein conferring the increased expression of a protein encoded by the nucleic acid molecule of the present invention or a polypeptide of the present invention having herein-mentioned amino acids of the invention increasing activity, e.g. of a polypeptide having the activity of a protein as indicated in table II, application no. 4, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned amino acids of the invention increasing activity, e.g. of a polypeptide having the activity of a protein as indicated in table II, application no. 4, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned amino acids of the invention increasing activity, e.g. of a polypeptide having the activity of a protein as indicated in table II, application no. 4, columns 5 and 7 or its homologs activity; and/or
h) increasing the expression of the endogenous gene encoding the polypeptide of the invention, e.g. a polypeptide having the activity of a protein as indicated in table II, application no. 4, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with especially high activity of the proteins of the invention from natural or from mutagenized resources and breeding them into the target organisms, eg the elite crops.

**[0054.0.4.4]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 4 in Table I to IV, resp., after increasing the expression or activity of the encoded polypeptide or having the activity of a polypeptide having an activity as the protein as shown in table II, application no. 4, column 3 or its homologs.

**[0055.0.0.4] to [0071.0.0.4]: see [0055.0.0.0] to [0071.0.0.0]**

**[0072.0.4.4]** By influencing the metabolism thus, it is possible to produce, in the process according to the invention, further advantageous compounds,. Examples of such compounds are, in addition to arginine and/or glutamate and/or glutamine and/or proline Argininosuccinate, Citrulline, Ornithine, Urea, Pyrroline-5-carboxylate, Hydroxyproline, Hydroxypyrroline-carboxylate, 3-Hydroxypyrroline-5-carboxylate, Y-Glutamylcysteine, Glutathione, Hydroxyglutamate, 4-Hydroxyglutamate, Oxoglutarate, 4-Hydroxy-2-oxoglutarate, Glutamine.

**[0073.0.4.4]** Accordingly, in one embodiment, the process according to the invention relates to a process which comprises:
a) providing a non-human organism, preferably a microorganism, a non-human animal, a plant or animal cell, a plant or animal tissue or a plant;
b) increasing an activity of a polypeptide of the invention or a homolog thereof, e.g. as indicated in Table II, application no. 4, columns 5 or 7, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
c) growing the organism, preferably the microorganism, the non-human animal, the plant or animal cell, the plant or animal tissue or the plant under conditions which permit the production of the fine chemical in the organism, preferably the microorganism, the plant cell, the plant tissue or the plant; and
d) if desired, revovering, optionally isolating, the free and/or bound the fine chemical and , optionally further free and/or bound amino acids synthetized by the organism, the microorganism, the non-human animal, the plant or animal cell, the plant or animal tissue or the plant.

**[0074.0.0.4] to [0084.0.0.4]: see [0074.0.0.0] to [0084.0.0.0]**

**[0085.0.4.4]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as indicated in Table I, application no. 4, columns 5 or 7, or a derivative thereof, or
b) a genetic regulatory element, for example a promoter, which is functionally linked to the nucleic acid sequence as indicated in Table I, application no. 4, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.4] and [0088.1.0.4]: see [0086.0.0.0] and [0088.1.0.0]**

**[0089.0.0.4] to [0097.0.0.4]: see [0089.0.0.0] to [0097.0.0.0]**

**[0098.0.4.4]** In a preferred embodiment, the fine chemical (arginine and/or glutamate and/or glutamine and/or proline) is produced in accordance with the invention and, if desired, is isolated. The production of further amino acids such as methionine, lysine and/or threonine mixtures of amino acid by the process according to the invention is advantageous.

**[0099.0.0.4] to [0102.0.0.4]: see [0099.0.0.0] to [0102.0.0.0]**

**[0103.0.4.4]** In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, application no. 4, columns 5 or 7, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 4.
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, application no. 4; column 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the fine chemical in an organism or a part thereof;
j) nucleic acid molecule which encodes a polypeptide comprising the consensus sequence having a sequences as indicated in Table IV, application no. 4, column 7, and conferring an increase in the amount of the respective fine chemical in an organism or a part thereof ;
k) nucleic acid molecule comprising one or more of the nucleic acid molecule encoding the amino acid sequence of a polypeptide encoding a domain of the polypeptide indicated in Table II, application no. 4, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[00103.1.0.4.]** In one embodiment, the nucleic acid molecule used in the process of the invention distinguishes over the sequence indicated in Table I, application no. 4, columns 5 or 7 by one or more nucleotides. In one embodiment, the nucleic acid molecule used in the process of the invention does not consist of the sequence shown in indicated in Table I ,application no. 4, columns 5 or 7,. In one embodiment, the nucleic acid molecule used in the process of the invention is less than 100%, 99,999%, 99,99%, 99,9% or 99% identical to a sequence indicated in Table I , application no. 4, columns 5 or 7,. In another embodiment, the nucleic acid molecule does not encode a polypeptide of a sequence indicated in Table II, application no. 4, columns 5 or 7.

**[0104.0.4.4]** In one embodiment, the nucleic acid molecule used in the process distinguishes over the sequence indicated in Table I, application no. 4, columns 5 or 7, by one or more nucleotides. In one embodiment, the nucleic acid molecule used in the process of the invention does not consist of the sequence indicated in Table I, application no. 4, columns 5 or 7, In one embodiment, the nucleic acid molecule of the present invention is less than 100%, 99,999%, 99,99%, 99,9% or 99% identical to the sequence indicated in Table I, application no. 4, columns 5 or 7, In another embodiment, the nucleic acid molecule does not encode a polypeptide of a sequence indicated in Table II, application no. 4, columns 5 or 7

**[0105.0.0.4] to [0107.0.0.4]: see [0105.0.0.0] to [0107.0.0.0]**

**[0108.0.4.4]** Nucleic acid molecules with the sequence as indicated in Table I, application no. 4, columns 5 or 7, nucleic acid molecules which are derived from a amino acid sequences as indicated in Table II, application no. 4, columns 5 or 7, or from polypeptides comprising the consensus sequence as indicated in Table IV, application no. 4, column 7, or their derivatives or homologues encoding polypeptides with the enzymatic or biological activity of a polypeptide as indicated in Table I, application no. 4, column 3, 5 or 7, or e.g. conferring a increase of the fine chemical after increasing its expression or activity are advantageously increased in the process according to the invention.

**[0109.0.0.4]: see [0109.0.0.0]**

**[0110.0.4.4]** Nucleic acid molecules, which are advantageous for the process according to the invention and which encode polypeptides with an activity of a polypeptide of the invention or the polypeptide used in the method of the invention or used in the process of the invention, e.g. of a protein as indicated in Table II, application no. 4, column 5, or being encoded by a nucleic acid molecule indicated in Table I, application no. 4, column 5, or of its homologs, e.g. as indicated in Table II, application no. 4, column 7, can be determined from generally accessible databases.

**[0111.0.0.4]: see [0111.0.0.0]**

**[0112.0.4.4]** The nucleic acid molecules used in the process according to the invention take the form of isolated nucleic acid sequences, which encode polypeptides with protein activity of proteins as indicated in Table II, application no. 4, columns 5 or 7, and conferring a arginine and/or glutamate and/or proline and/or glutamine increase.

**[0113.0.0.4] to [0120.0.0.4]: [0113.0.0.0] to [0120.0.0.0]**

**[0121.0.4.4]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 4, columns 5 or 7, or the functional homologues thereof as described herein, preferably conferring above-mentioned activity, i.e. conferring a increase of the respective fine chemical after increasing its activity.

**[0122.0.0.4] to [0127.0.0.4]: see [0122.0.0.0] to [0127.0.0.0]**

**[0128.0.4.4]** Synthetic oligonucleotide primers for the amplification, e.g. as the pairs indicated in Table III, application no. 4, column 7, by means of polymerase chain reaction can be generated on the basis of a sequence shown herein, for example the sequence as indicated in Table I, application no. 4, columns 5 or 7, or the sequences derived from a sequence as indicated in Table II, application no. 4, columns 5 or 7.

**[0129.0.4.4]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequence indicated in Table IV, application no. 4, column 7, is derived from said aligments.

**[0130.0.0.4] to [0138.0.0.4]: see [0130.0.0.0] to [0138.0.0.0]**

**[0139.0.4.4]** Polypeptides having above-mentioned activity, i.e. conferring the increase of the respective fine chemical level, derived from other organisms, can be encoded by other DNA sequences which hybridize to a sequences indicated in Table I, application no. 4, columns 5 or 7, under relaxed hybridization conditions and which code on expression for peptides having the respective fine chemical, in particular, of arginine and/or glutamate and/or proline and/or glutamine, resp., increasing activity.

**[0140.0.0.4] to [0146.0.0.4]: see [0140.0.0.0] to [0146.0.0.0]**

**[0147.0.4.4]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 4, columns 5 or 7, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.4.4]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 4, columns 5 or 7 or a portion thereof and preferably has above mentioned activity, in particular, of arginine and/or glutamate and/or proline and/or glutamine increasing activity after increasing the acitivity or an activity of a product of a gene encoding said sequences or their homologs.

**[0149.0.4.4]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 4, columns 5 or 7, or a portion thereof and encodes a protein having above-mentioned activity and as indicated in indicated in Table II.

**[00149.1.4.4]** Optionally, the nucleotide sequence, which hybridises to one of the nucleotide sequences indicated in Table I, application no. 4, columns 5 or 7, has further one or more of the activities annotated or known for the a protein as indicated in Table II, application no. 4, column 3.

**[0150.0.4.4]** Moreover, the nucleic acid molecule of the invention can comprise only a portion of the coding region of one of the sequences indicated in Table I, application no. 4, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of arginine and/or glutamate and/or proline and/or glutamine, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 4, columns 5 or 7, , an anti-sense sequence of one of the sequences, e.g., as indicated in Table I, application no. 4, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 4, column 7, will result in a fragment of a polynucleotide sequence as indicated in Table I, application no. 4, columns 5 or 7 or its gene product.

**[0151.0.0.4]: see [0151.0.0.0]**

**[0152.0.4.4]** The nucleic acid molecule of the invention encodes a polypeptide or portion thereof which includes an amino acid sequence which is sufficiently homologous to an amino acid sequence as indicated in Table II, application no. 4, columns 5 or 7, such that the protein or portion thereof maintains the ability to participate in the respective fine chemical production, in particular an activity increasing the level of arginine and/or glutamate and/or proline and/or glutamine, resp., as mentioned above or as described in the examples in plants or microorganisms is comprised.

**[0153.0.4.4]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 4, columns 5 or 7, such that the protein or portion thereof is able to participate in the increase of the respective fine chemical production. In one embodiment, a protein or portion thereof as indicated in Table II, application no. 4, columns 5 or 7, has for example an activity of a polypeptide as indicaded in Table II, application no. 4, column 3.

**[0154.0.4.4]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, application no. 4, columns 5 or 7, and has above-mentioned activity, e.g.conferring preferably the increase of the respective fine chemical.

**[0155.0.0.4] and [0156.0.0.4]: see [0155.0.0.0] and [0156.0.0.0]**

**[0157.0.4.4]** The invention further relates to nucleic acid molecules that differ from one of the nucleotide sequences indicated in Table I, application no. 4, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, application no. 4, column 7 or of the polypeptide as indicated in Table II, application no. 4, columns 5 or 7, or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, application no. 4, column 7, or of the polypeptide as indicated in Table II, application no. 4, columns 5 or 7, or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, application no. 4, column 7, or of a polypeptide as indicated in Table II, application no. 4, columns 5 or 7, or the functional homologues thereof. However, in a preferred embodiment, the nucleic acid molecule of the present invention does not consist of a sequence as indicated in Table I, application no. 4, columns 5 or 7. Preferably the nucleic acid molecule of the invention is a functional homologue or identical to a nucleic acid molecule indicated in Table I, application no. 4, columns 5 or 7.

**[0158.0.0.4] to [0160.0.0.4]: see [0158.0.0.0] to [0160.0.0.0]**

**[0161.0.4.4]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 4, columns 5 or 7. The nucleic acid molecule is preferably at least 20, 30, 50, 100, 250 or more nucleotides in length.

**[0162.0.0.4]: see [0162.0.0.0]**

**[0163.0.4.4]** Preferably, nucleic acid molecule of the invention that hybridizes under stringent conditions to a sequence as indicated in Table I, application no. 4, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.4]: see [0164.0.0.0]**

**[0165.0.4.4]** For example, nucleotide substitutions leading to amino acid substitutions at "non-essential" amino acid residues can be made in a sequence of the nucleic acid molecule of the invention or used in the process of the invention, e.g. as indicated in Table I, application no. 4, columns 5 or 7.

**[0166.0.0.4] and [0167.0.0.4]: see [0166.0.0.0] and [0167.0.0.0]**

**[0168.0.4.4]** Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 4, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 4, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 4, columns 5 or 7, more preferably at least about 70% identical to one of the sequences as indicated in Table II, application no. 4, columns 5 or 7, ,even more preferably at least about 80%, 90%, or 95% homologous to a sequence as indicated in Table II, application no. 4, columns 5 or 7, , and most preferably at least about 96%, 97%, 98%, or 99% identical to the sequence as indicated in Table II, application no. 4, columns 5 or 7.

**[0169.0.0.4] to [0172.0.0.4]: see [0169.0.0.0] to [0172.0.0.0]**

**[0173.0.4.4]** For example a sequence which has a 80% homology with sequence SEQ ID NO: 268 at the nucleic acid level is understood as meaning a sequence which, upon comparison with the sequence SEQ ID NO: 268 by the above Gap program algorithm with the above parameter set, has a 80% homology.

**[0174.0.0.4]: see [0174.0.0.0]**

**[0175.0.4.4]** For example a sequence which has a 80% homology with sequence SEQ ID NO: 269 at the protein level is understood as meaning a sequence which, upon comparison with the sequence SEQ ID NO: 269 by the above program algorithm with the above parameter set, has a 80% homology.

**[0176.0.4.4]** Functional equivalents derived from one of the polypeptides as indicated in Table II, application no. 4, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 4, columns 5 or 7, , according to the invention and are distinguished by essentially the same properties as a polypeptide as indicated in Table II, application no. 4, columns 5 or 7.,

**[0177.0.4.4]** Functional equivalents derived from a nucleic acid sequence as indicated in Table I, application no. 4, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptide as indicated in Table II, application no. 4, columns 5 or according to the invention and encode polypeptides having essentially the same properties as a polypeptide as indicated in Table II, application no. 4, columns 5 or 7,.

**[0178.0.0.4]: see [0178.0.0.0]**

**[0179.0.4.4]** A nucleic acid molecule encoding an homologous to a protein sequence as indicated in Table II, application no. 4, columns 5 or 7, can be created by introducing one or more nucleotide substitutions, additions or deletions into a nucleotide sequence of the nucleic acid molecule of the present invention, in particular as indicated in Table I, application no. 4, columns 5 or 7, , such that one or more amino acid substitutions, additions or deletions are introduced into the encoded protein. Mutations can be introduced into the encoding sequences of a sequences as indicated in Table I, application no. 4, columns 5 or 7 by standard techniques, such as site-directed mutagenesis and PCR-mediated mutagenesis.

**[0180.0.0.4] to [0183.0.0.4]: see [0180.0.0.0] to [0183.0.0.0]**

**[0184.0.4.4]** Homologues of the nucleic acid sequences used, with a sequence as indicated in Table I, application no. 4, columns 5 or 7, or of the nucleic acid sequences derived from a sequences as indicated in Table II, application no. 4, columns 5 or 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 4, columns 5 or 7, or from the derived nucleic acid sequences, the intention being, however, that the enzyme activity or the biological activity of the resulting proteins synthesized is advantageously retained or increased.

**[0185.0.4.4]** In one embodiment of the present invention, the nucleic acid molecule of the invention or used in the process of the invention comprises one or more sequences as indicated in Table I, application no. 4" columns 5 or 7,. In one embodiment, it is preferred that the nucleic acid molecule comprises as little as possible other nucleotides not shown in any one of sequences as indicated in Table I" application no. 4, columns 5 or 7, In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 4, columns 5 or 7.

**[0186.0.4.4]** Also preferred is that one or more nucleic acid molecule(s) used in the process of the invention encodes a polypeptide comprising a sequence as indicated in Table II, application no. 4, columns 5 or 7,. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 4, columns 5 or 7.

**[0187.0.4.4]** In one embodiment, a nucleic acid molecule of the invention or used in the process encodes a polypeptide comprising the sequence as indicated in Table II, application no. 4, columns 5 or 7, comprises less than 100 further nucleotides. In a further embodiment, said nucleic acid molecule comprises less than 30 further nucleotides. In one embodiment, the nucleic acid molecule used in the process is identical to a coding sequence as indicated in Table II, application no. 4, columns 5 or 7.

**[0188.0.4.4]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 4, columns 5 or 7 and is expressed under identical conditions.

**[0189.0.4.4]** Homologues of a sequences as indicated in Table I, application no. 4, columns 5 or 7, or of a derived sequences as indicated in Table II, application no. 4, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.4] to [0203.0.0.4]: see [0190.0.0.0] to [0203.0.0.0]**

**[0204.0.4.4]** Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 4, columns 5 or 7, or a fragment thereof conferring an increase in the amount of the respective fine chemical, in particular according to table II, application no. 4, column 6 in an organism or a part thereof
b) nucleic acid molecule comprising, preferably at least the mature form, of a nucleic acid molecule as indicated in Table I, application no. 4, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, application no. 4, column 7, and conferring an increase in the amount of the respective fine chemical, in particular according to table II, application no. 4, column 6 in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, application no. 4, columns 5 or 7, and conferring an increase in the amount of the respective fine chemical, in particular according to table II, application no. 4, column 6 in an organism or a part thereof;
k) nucleic acid molecule encoding the amino acid sequence of a polypeptide encoding a domaine of a polypeptide as indicated in Table II, application no. 4, columns 5 or 7, and conferring an increase in the amount of the respective fine chemical, in particular accccording to table II, application no. 4, column 6 in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 4, columns 5 or 7, or a nucleic acid molecule encoding, preferably at least the mature form of, a polypeptide as indicated in Table II, application no. 4, columns 5 or 7, and conferring an increase in the amount of the respective fine chemical according to table II, application no. 4, column 6 in an organism or a part thereof; or which encompasses a sequence which is complementary thereto;
whereby, preferably, the nucleic acid molecule according to (a) to (I) distinguishes over the sequence indicated in Table I, application no. 4,columns 5 or 7, by one or more nucleotides. In one embodiment, the nucleic acid molecule does not consist of the sequence shown and indicated in Table I, application no. 4, columns 5 or 7,
In one embodiment, the nucleic acid molecule is less than 100%, 99,999%, 99,99%, 99,9% or 99% identical to a sequence indicated in Table I, application no. 4, columns 5 or 7,.
In another embodiment, the nucleic acid molecule does not encode a polypeptide of a sequence indicated in Table II, application no. 4, columns 5 or 7,.
In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 4, columns 5 or 7,.
In a further embodiment the nucleic acid molecule does not encode a polypeptide sequence as indicated in Table II, application no. 4, columns 5 or 7,.
Accordingly, in one embodiment, the nucleic acid molecule of the differs at least in one or more residues from a nucleic acid molecule indicated in Table I, application no. 4, columns 5 or 7,.
Accordingly, in one embodiment, the nucleic acid molecule of the present invention encodes a polypeptide, which differs at least in one or more amino acids from a polypeptide indicated in Table II, application no. 4, columns 5 or 7,.
In another embodiment, a nucleic acid molecule indicated in Table I, application no. 4, columns 5 or 7,.
Accordingly, in one embodiment, the protein encoded by a sequences of a nucleic acid accoriding to (a) to (I) does not consist of a sequence as indicated in Table II, application no. 4, columns 5 or 7,.
In a further embodiment, the protein of the present invention is at least 30 %, 40 %, 50%, or 60% identical to a protein sequence indicated in Table II, application no. 4, columns 5 or 7, and less than 100%, preferably less than 99,999%, 99,99% or 99,9%, more preferably less than 99%, 985, 97%, 96% or 95% identical to a sequence as indicated in Table I, columns 5 or 7.

**[0205.0.0.4] to [0226.0.0.4]: see [0205.0.0.0] to [0226.0.0.0]**

**[0227.0.4.4]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.

In addition to a sequence indicated in Table I, application no. 4, columns 5 or 7, or its derivatives, it is advantageous additionally to express and/or mutate further genes in the organisms. Especially advantageously, additionally at least one further gene of the amino acid biosynthetic pathway such as for L-lysine, L-threonine and/or L-methionine and/or L-leucine and/or isoleucine and/or valine is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine one or more of the sequences indicated in Table I, application no. 4, columns 5 or 7, with genes which generally support or enhances to growth or yield of the target organismen, for example genes which lead to faster growth rate of microorganisms or genes which produces stress-, pathogen, or herbicide resistant plants.

**[0228.0.0.4] to [0230.0.0.4]: see[0228.0.0.0] to [0230.0.0.0]**

**[0231.0.4.4]** In a further advantageous embodiment of the process of the invention, the organisms used in the process are those in which simultaneously an arginine and/or glutamate and/or glutamine and/or proline degrading protein is attenuated, in particular by reducing the rate of expression of the corresponding gene.

**[0232.0.0.4] to [0282.0.0.4]: see [0232.0.0.0] to [0282.0.0.0]**

**[0283.0.4.4]** Moreover, a native polypeptide conferring the increase of the respective fine chemical in an organism or part thereof can be isolated from cells (e.g., endothelial cells), for example using the antibody of the present invention as described below, e.g. an antibody against a protein as indicated in Table II, application no. 4, column 3, or an antibody against a polypeptide as indicated in Table II, application no. 4, columns 5 or 7, which can be produced by standard techniques utilizing the polypeptid of the present invention or fragment thereof, i.e., the polypeptide of this invention. Preferred are monoclonal antibodies.

**[0284.0.0.4]: see [0284.0.0.0].**

**[0285.0.4.4]** In one embodiment, the present invention relates to a polypeptide having a sequence as indicated in Table II, application no. 4, columns 5 or 7, or as encoded by a nucleic acid molecule as indicated in Table I, application no. 4, columns 5 or 7, or functional homologues thereof.

**[0286.0.4.4]** In one advantageous embodiment, in the method of the present invention the activity of a polypeptide is increased which comprises or consists of a consensus sequence as indicated in Table IV, application no. 4, column 7,. In another embodiment, the present invention relates to a polypeptide comprising or consisting of a consensus sequence as indicated in Table IV, application no. 4, column 7, , whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, application no. 4, column 7.

**[0287.0.0.4] to [0290.0.0.4]: see [0287.0.0.0] to [0290.0.0.0]**

**[0291.0.4.4]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II, application no. 4, columns 5 or 7, by one or more amino acids. In one embodiment, polypeptide distinguishes form a sequence as indicated in Table II, application no. 4, columns 5 or 7, by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 4, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II, application no. 4, columns 5 or 7.

**[0292.0.0.4]: see [0292.0.0.0]**

**[0293.0.4.4]** In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II, application no. 4, columns 5 or 7, by one or more amino acids. In an other embodiment, said polypeptide of the invention does not consist of the sequence as indicated in Table II, application no. 4, columns 5 or 7,.
In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 4, columns 5 or 7.

**[0294.0.4.4]** In one embodiment, the present invention relates to a polypeptide having an activity of a protein as indicated in Table II, application no. 4, column 3, which distinguishes over a sequence as indicated in Table II, application no. 4, columns 5 or 7, by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.4] and [0297.0.0.4]: see [0295.0.0.0]and [0297.0.0.0]**

**[0297.1.4.4]** Non polypeptide of the invention-chemicals are e.g. polypeptides having not the activity of a polypeptide indicated in Table II, application no. 4, columns 3, 5 or 7.

**[0298.0.4.4]** A polypeptide of the invention can participate in the process of the present invention. The polypeptide or a portion thereof comprises preferably an amino acid sequence which is sufficiently homologous to an amino acid sequence as indicated in Table II, application no. 4, columns 5 or 7,. The portion of the protein is preferably a biologically active portion as described herein. Preferably, the polypeptide used in the process of the invention has an amino acid sequence identical to a sequence as indicated in Table II, application no. 4, columns 5 or 7,.

**[0299.0.4.4]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, application no. 4, columns 5 or 7,. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 4, columns 5 or 7, , or which is homologous thereto, as defined above.

**[0300.0.4.4]** In one embodiment, the present invention relates to a polypeptide having an activity of a protein as indicated in Table II, application no. 4, column 3, which distinguishes over a sequence as indicated in Table II, application no. 4, columns 5 or 7, by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0301.0.0.4]: see [0301.0.0.0]**

**[0302.0.4.4]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 4, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.4]: see [0303.0.0.0]**

**[0304.0.4.4]** Manipulation of the nucleic acid molecule of the invention may result in the production of a protein having essentially the activity of the polypeptides as indicated in Table II, application no. 4, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.4] to [0308.0.0.4]: see [0306.0.0.0] to [0308.0.0.0]**

**[0309.0.4.4]** In one embodiment, a reference to a protein (= polypeptide) of the invention or as indicated in Table II, application no. 4, columns 5 or 7, refers to a polypeptide having an amino acid sequence corresponding to the polypeptide of the invention or used in the process of the invention , whereas a " non-polypeptide of the invention" or "other polypeptide" not being indicated in Table II, application no. 4, columns 5 or 7, refers to a polypeptide having an amino acid sequence corresponding to a protein which is not substantially homologous a polypeptide of the invention, preferably which is not substantially homologous to a polypeptide as indicated in Table II, application no. 4, columns 5 or 7, e.g., a protein which does not confer the activity described herein or annotated or known for as indicated in Table II, application no. 4, column 3, and which is derived from the same or a different organism. In one embodiment, a "non-polypeptide of the invention" or "other polypeptide" not being indicated in Table II, application no. 4, columns 5 or 7, does not confer an increase of the respective fine chemical in an organism or part therof.

**[0310.0.0.4] to [0334.0.0.4]: see [0310.0.0.0] to [0334.0.0.0]**

**[0335.0.4.4]** The dsRNAi method has proved to be particularly effective and advantageous for reducing the expression of a nucleic acid sequences as indicated in Table I, application no. 4, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, application no. 4, columns 5 or 7, and/or homologs thereof. In a double-stranded RNA molecule for reducing the expression of an protein encoded by a nucleic acid sequence sequences as indicated in Table I, application no. 4, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.4] to [0342.0.0.4]: see [0336.0.0.0] to [0342.0.0.0]**

**[0343.0.4.4]** As has already been described, 100 % sequence identity between the dsRNA and a gene transcript of a nucleic acid sequence as indicated in Table I, application no. 4, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 4, columns 5 or 7, or homologs thereof of the one organism, may be used to suppress the corresponding expression in another organism.

**[0344.0.0.4] to [0361.0.0.4]: see [0344.0.0.0] to [0361.0.0.0]**

**[0362.0.4.4]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 4, columns 5 or 7, e.g. encoding a polypeptide having protein activity, as indicated in Table II, application no. 4, columns 3,. Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, application no. 4, columns 5 or 7, means herein that due to modulation or manipulation of the genome, an activity as annotated for a polypeptide as indicated in Table II, application no. 4, column 3, e.g. having a sequence as indicated in Table II, application no. 4, columns 5 or 7, is increased in a cell or an organism or a part thereof. Examples are described above in context with the process of the invention

**[0363.0.0.4]: to [0382.0.0.4]: see [0363.0.0.0] to [0382.0.0.0]**

**[0383.0.4.4]** For preparing arginine and/or glutamate and/or glutamine and/or proline compound-containing fine chemicals, in particular the fine chemical, it is possible to use as arginine and/or glutamate and/or glutamine and/or proline amino acid source organic compounds such as, for example, citrulline, argininosuccinate, ornithine, aspartate, 2-Oxoglutarate, glutamyl, glutamic-semialdehyde, Pyrroline-5-carboxylate, Glutamine or else organic arginine and/or glutamate and/or glutamine and/or proline acid precursor compounds.

**[0384.0.0.4]: see [0384.0.0.0]**

**[0385.0.4.4]** The fermentation broths obtained in this way, containing in particular L-arginine and/or L-glutamate and/or L-proline and/or L-tryptophane, L-methionine, L-threonine and/or L-lysine, normally have a dry matter content of from 7.5 to 25% by weight. Sugar-limited fermentation is additionally advantageous, at least at the end, but especially over at least 30% of the fermentation time. This means that the concentration of utilizable sugar in the fermentation medium is kept at, or reduced to, 0 to 3 g/I during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.0.4] to [0392.0.0.4]: see [0386.0.0.0] to [0392.0.0.0]**

**[0393.0.4.4]** In one embodiment, the present invention relates to a method for the identification of a gene product conferring an increase in the fine chemical production in a cell, comprising the following steps:
a) contacting, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
b) identifying the nucleic acid molecules, which hybridize under relaxed stringent conditions with the nucleic acid molecule of the present invention in particular to the nucleic acid molecule sequence as indicated in Table I, application no. 4, columns 5 or 7, and, optionally, isolating the full length cDNA clone or complete genomic clone;
c) introducing the candidate nucleic acid molecules in host cells, preferably in a plant cell or a microorganism, appropriate for producing the fine chemical;
d) expressing the identified nucleic acid molecules in the host cells;
e) assaying the the fine chemical level in the host cells; and
f) identifying the nucleic acid molecule and its gene product which expression confers an increase in the the fine chemical level in the host cell after expression compared to the wild type.

**[0394.0.0.4] to [0552.0.0.4]: see [0394.0.0.1] to [0552.0.0.1]**

**[0553.0.4.4]** A process for the production of arginine, proline, glutamine and/or glutamate resp., which comprises
(a) increasing or generating the activity of a protein as indicated in Table II, application no. 4, columns 5 or 7, or a functional equivalent thereof in a non-human organism, or in one or more parts thereof; and
(b) growing the organism under conditions which permit the production of arginine, proline, glutamine and/or glutamate resp. in said organism.
A process for the production of arginine, proline, glutamine and/or glutamate resp., comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 4, columns 5 or 7, , or a fragment thereof, which confers an increase in the amount of the fine chemical as indicated in table II, column 6, e.g of arginine, proline, glutamine and/or glutamate resp., in an organism or a part thereof;
b) nucleic acid molecule comprising of a nucleic acid molecule as indicated in Table I, application no. 4, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of arginine, proline, glutamine and/or glutamate resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of arginine, proline, glutamine and/or glutamate resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of arginine, proline, glutamine and/or glutamate resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 4, column 7, and conferring an increase in the amount of the fine chemical as indicated in table II, column 6, e.g of arginine, proline, glutamine and/or glutamate resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of arginine, proline, glutamine and/or glutamate resp., in an organism or a part thereof;
h) nucleic acid molecule encoding a polypeptide comprising a consensus sequence as indicated in Table IV, application no. 4, column 7, and conferring an increase in the amount of the fine chemical as indicated in table II, column 6, e.g of arginine, proline, glutamine and/or glutamate resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical as indicated in table II, column 6, e.g of arginine, proline, glutamine and/or glutamate resp., in an organism or a part thereof.
or comprising a sequence which is complementary thereto.
The process as mentioned above, comprising recovering of the free or bound arginine, proline, glutamine and/or glutamate resp..
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound arginine, proline, glutamine and/or glutamate resp., produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 4, columns 5 or 7, or a fragment thereof, which confers an increase in the amount of the fine chemical as indicated in table II, column 6, e.g of arginine, proline, glutamine and/or glutamate resp., in an organism or a part thereof;
b) nucleic acid molecule comprising of a nucleic acid molecule as indicated in Table I, application no. 4, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of arginine, proline, glutamine and/or glutamate resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of arginine, proline, glutamine and/or glutamate resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of arginine, proline, glutamine and/or glutamate resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 4, column 7, and conferring an increase in the amount of the fine chemical as indicated in table II, column 6, e.g of arginine, proline, glutamine and/or glutamate resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of arginine, proline, glutamine and/or glutamate resp., in an organism or a part thereof;
h) nucleic acid molecule encoding a polypeptide comprising a consensus sequence as indicated in Table IV, application no. 4, column 7, and conferring an increase in the amount of the fine chemical as indicated in table II, column 6, e.g of arginine, proline, glutamine and/or glutamate resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemiccal as indicated in table II, column 6, e.g of arginine, proline, glutamine and/or glutamate resp., in an organism or a part thereof. whereby the nucleic acid molecule distinguishes over the sequence as indicated in Table I, application no. 4, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 4, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of arginine, proline, glutamine and/or glutamate resp., in an organism or a part thereof comprising:
(a) contacting cells, tissues , plants or microorganisms which express the a polypeptide encoded by the nucleic acid molecule as mentioned above conferring an increase in the amount of arginine, proline, glutamine and/or glutamate resp., in an organism or a part thereof with a candidate compound or a sample comprising a plurality of compounds under conditions which permit the expression the polypeptide;
(b) assaying the arginine, proline, glutamine and/or glutamate resp., level or the polypeptide expression level in the cell, tissue, plant or microorganism or the media the cell, tissue, plant or microorganisms is cultured or maintained in; and
(c) identifying a agonist or antagonist by comparing the measured arginine, proline, glutamine and/or glutamate resp., level or polypeptide expression level with a standard arginine, proline, glutamine and/or glutamate resp., or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased arginine, proline, glutamine and/or glutamate resp., production in a plant or microorganism, comprising the steps:
(a) culturing a plant cell or tissue or microorganism or maintaining a plant expressing the polypeptide encoded by the nucleic acid molecule as mentioned above conferring an increase in the amount of arginine, proline, glutamine and/or glutamate resp., in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of arginine, proline, glutamine and/or glutamate rersp., in an organism or a part thereof;
(b) identifying if the compound is an effective agonist by detecting the presence or absence or increase of a signal produced by said readout system.
A method for the identification of a gene product conferring an increase in arginine, proline, glutamine and/or glutamate resp., production in a cell, comprising the following steps:
(a) contacting the nucleic acid molecules of a sample, which can contain a candidate gene encoding a gene product conferring an increase in arginine, proline, glutamine and/or glutamate resp., after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing arginine, proline, glutamine and/or glutamate resp.;
(d) expressing the identified nucleic acid molecules in the host cells;
(e) assaying the arginine, proline, glutamine and/or glutamate rersp., level in the host cells; and
(f) identifying nucleic acid molecule and its gene product which expression confers an increase in the arginine, proline, glutamine and/or glutamate resp., level in the host cell in the host cell after expression compared to the wild type.
A method for the identification of a gene product conferring an increase in arginine, proline, glutamine and/or glutamate resp., production in a cell, comprising the following steps:
(a) identifiying in a data bank nucleic acid molecules of an organism; which can contain a candidate gene encoding a gene product conferring an increase in the arginine, proline, glutamine and/or glutamate resp., amount or level in an organism or a part thereof after expression, and which are at least 20% homolog to the nucleic acid molecule as mentioned above;
(b) introducing the candidate nucleic acid molecules in host cells appropriate for producing arginine, proline, glutamine and/or glutamate resp.,;
(c) expressing the identified nucleic acid molecules in the host cells;
(d) assaying the arginine, proline, glutamine and/or glutamate resp., level in the host cells; and
(e) identifying nucleic acid molecule and its gene product which expression confers an increase in the arginine, proline, glutamine and/or glutamate resp., level in the host cell after expression compared to the wild type.

A method for the production of an agricultural composition comprising the steps of the method as mentioned above and formulating the compound identified as mentioned above in a form acceptable for an application in agriculture.

A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.

Use of the nucleic acid molecule as mentioned above for the identification of a nucleic acid molecule conferring an increase of arginine, proline, glutamine and/or glutamate resp., after expression.

Use of the polypeptide as mentioned above or the nucleic acid construct as mentioned above or the gene product identified according to the method as mentioned above for identifying compounds capable of conferring a modulation of arginine, proline, glutamine and/or glutamate resp., levels in an organism.

Food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.

Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the protection of a plant against a arginine and/or proline and/or glutamine and/or glutamate synthesis inhibiting herbicide.

### New genes for a process for the production of fine chemicals

**[0000.0.0.7]** In a further embodiment, the present invention relates to a further process for the production of fine chemicals as defined below and the corresponding embodiments as described herein as follows.

**[0001.0.0.7]** for the disclosure of this paragraph see [0001.0.0.0].

**[0002.0.7.7]** Fatty acids and triglycerides have numerous applications in the food and feed industry, in cosmetics and in the drug sector. Depending on whether they are free saturated or unsaturated fatty acids or triglycerides with an increased content of saturated or unsaturated fatty acids, they are suitable for the most varied applications; thus, for example, polyunsaturated fatty acids (= PUFAs) are added to infant formula to increase its nutritional value. The various fatty acids and triglycerides are mainly obtained from microorganisms such as fungi or from oil-producing plants including phytoplankton and algae, such as soybean, oilseed rape, sunflower and others, where they are usually obtained in the form of their triacylglycerides.

**[0003.0.7.7]** Stearic acid (= octadecanoic acid) is one of the many useful types of saturated fatty acids that comes from many animal and vegetable fats and oils. It is a waxy solid that melts at around 70°C. Commenly stearic acid is either prepared by treating animal fat with water at a high pressure and temperature or starting with vegetable oils by hydrogenation of said oils. It is useful as an ingredient in making candles, soaps, and cosmetics and for softening rubber.

**[0004.0.7.7]** Principally microorganisms such as Mortierella or oil producing plants such as soybean, rapeseed or sunflower or algae such as Crytocodinium or Phaeodactylum are a common source for oils containing fatty acids, where they are usually obtained in the form of their triacyl glycerides. Alternatively, they are obtained advantageously from animals, such as fish. The free fatty acids are prepared advantageously by hydrolysis with a strong base such as potassium or sodium hydroxide.

**[0005.0.7.7]** Whether oils with unsaturated or with saturated fatty acids are preferred depends on the intended purpose; thus, for example, lipids with unsaturated fatty acids, specifically polyunsaturated fatty acids, are preferred in human nutrition since they have a positive effect on the cholesterol level in the blood and thus on the possibility of heart disease. They are used in a variety of dietetic foodstuffs or medicaments. In addition PUFAs are commonly used in food, feed and in the cosmetic industry. Poly unsaturated w-3- and/or ω-6-fatty acids are an important part of animal feed and human food. Because of the common composition of human food poly unsaturated ω-3-fatty acids, which are an essential component of fish oil, should be added to the food to increase the nutritional value of the food; thus, for example, polyunsaturated fatty acids such as DHA or EPA are added as mentioned above to infant formula to increase its nutritional value. The true essential fatty acids linoleic and linolenic fatty acid have a lot of positive effects in the human body such as a positive effect on healthy heart, arteries and skin. They bring for example relieve from eczema, diabetic neuropathy or PMS and cyclical breast pain.

**[0006.0.7.7]** Unlike most saturated fats, stearic acid does not seem to increase cholesterol levels in the blood, because liver enzymes convert it to an unsaturated fat during digestion.

**[0007.0.7.7]** Stearic acid is the most common one of the long-chain fatty acids. It is found in many foods, such as beef fat, and cocoa butter. It is widely used as mentioned above as a lubricant, in soaps, cosmetics, food packaging, deodorant sticks, toothpastes, and as a softener in rubber.

**[0008.0.7.7]** Encouraging research shows that stearic acid, one of the components of the fat found in the cocoa butter of chocolate, may have some positive effects on platelets. The mechanism believed to be responsible for the potential platelet activation by stearic acid involves Arachidonic metabolism, which includes thromboxane A2, a potent aggregating compound, and prostaglandin 12, a potent anti-aggregating compound.

**[0009.0.7.7]** As described above, fatty acids are used in a lot of different applications, for example in cosmetics, pharmaceuticals and in feed and food.

**[0010.0.7.7]** Therefore improving the productivity of such fatty acids and improving the quality of foodstuffs and animal feeds is an important task of the different industries.

**[0011.0.7.7]** To ensure a high productivity of certain fatty acids in plants or microorganism, it is necessary to manipulate the natural biosynthesis of fatty acids in said organism.

**[0012.0.7.7]** Accordingly, there is still a great demand for new and more suitable genes which encode enzymes which participate in the biosynthesis of fatty acids and make it possible to produce certain fatty acids specifically on an industrial scale without unwanted byproducts forming. In the selection of genes for biosynthesis two characteristics above all are particularly important. On the one hand, there is as ever a need for improved processes for obtaining the highest possible contents of fatty acids on the other hand as less as possible byproducts should be produced in the production process.

**[0013.0.0.7]** for the disclosure of this paragraph see [0013.0.0.0] above.

**[0014.0.7.7]** Accordingly, in a first embodiment, the invention relates to a process for the production of a fine chemical, whereby the fine chemical is stearic acid or tryglycerides, lipids, oils or fats containing stearic acid. Accordingly, in the present invention, the term "the fine chemical" as used herein relates to "stearic acid and/or tryglycerides, lipids, oils and/or fats containing stearic acid". Further, the term "the fine chemicals" as used herein also relates to fine chemicals comprising stearic acid and/or triglycerides, lipids, oils and/or fats containing stearic acid.

**[0015.0.7.7]** In one embodiment, the term "the fine chemical" means stearic acid and/or tryglycerides, lipids, oils and/or fats containing stearic acid. Throughout the specification the term "the fine chemical" means stearic acid and/or tryglycerides, lipids, oils and/or fats containing stearic acid, stearic acid and its salts, ester, thioester or stearic acid in free form or bound to other compounds such as triglycerides, glycolipids, phospholipids etc. In a preferred embodiment, the term "the fine chemical" means stearic acid, in free form or its salts or bound to triglycerides. Triglycerides, lipids, oils, fats or lipid mixture thereof shall mean any triglyceride, lipid, oil and/or fat containing any bound or free stearic acid for example sphingolipids, phosphoglycerides, lipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride or other fatty acid esters such as acetyl-Coenzym A thioester, which contain further saturated or unsaturated fatty acids in the fatty acid molecule.
In one embodiment, the term "the fine chemical" and the term "the respective fine chemical" mean at least one chemical compound with an activity of the above mentioned fine chemical.

**[0016.0.7.7]** Accordingly, the present invention relates to a process for the production of the respective fine chemical comprising
(a) increasing or generating the activity of one or more
   of a protein as shown in table II, application no. 7, column 3 encoded by the nucleic acid sequences as shown in table I, application no. 7, column 5, in a non-human organism or in one or more parts thereof or
(b) growing the organism under conditions which permit the production of the fine chemical, thus fatty acid of the invention or fine chemicals comprising the fatty acid of the invention, in said organism or in the culture medium surrounding the organism.

**[0017.0.0.7] and [0018.0.0.7]** for the disclosure of the paragraphs [0017.0.0.7] and [0018.0.0.7] see paragraphs [0017.0.0.0] and [0018.0.0.0] above.

**[0019.0.0.7]** for the disclosure of the paragraph [0019.0.0.7] see paragraph [0019.0.0.0] above.

**[0020.0.7.7]** Surprisingly it was found, that the transgenic expression of the Brassica napus protein as indicated in Table II, application no.7, column 5, line 16 in a plant conferred an increase in Stearic Acid C18:0 content of the transformed plants. Thus, in one embodiment, said protein or its homologs are used for the production of Stearic Acid C18:0.
Surprisingly it was found, that the transgenic expression of the Glycine max protein as indicated in Table II, application no.7, column 5, line 17 in thaliana plant conferred an increase in Stearic Acid C18:0 content of the transformed plants. Thus, in one embodiment, said protein or its homologs are used for the production of Stearic Acid C18:0.

**[0021.0.0.7]** for the disclosure of this paragraph see [0021.0.0.0] above.

**[0022.0.7.7]** The sequence of b1093 from Escherichia coli K12 has been published in Blattner et al., Science 277(5331), 1453-1474, 1997, and its activity is being defined as 3-oxoacyl-[acyl-carrier-protein] reductase. Accordingly, in one embodiment, the process of the present invention comprises the use of a 3-oxoacyl-[acyl-carrier-protein] reductase from E. coli or a plant or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a 3-oxoacyl-[acyl-carrier-protein] reductase is increased or generated, e.g. from E. coli or a plant or a homolog thereof.
The sequence of YDR513w from Saccharomyces cerevisiae has been published in Jacq et al., Nature 387 (6632 Suppl), 75-78 (1997) and in Goffeau et al., Science 274 (5287), 546-547, 1996 and its cellular activity has characterized as glutaredoxin (thioltransferase, glutathione reductase. Accordingly, in one embodiment, the process of the present invention comprises the use of YDR513w, from Saccharomyces cerevisiae or a plant or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a glutaredoxin is increased or generated, e.g. from Saccharomyces cerevisiae or a plant or a homolog thereof.

**[0022.1.0.7] see [0022.1.0.0]**

**[0023.0.0.7]** for the disclosure of the paragraph [0023.0.0.7] see paragraph [0023.0.0.0] above.

**[0023.1.7.7]** Homologs of the polypeptide disclosed in table II, application no. 7, column 5, may have the activity of a protein as disclosed in table I or II, application no. 7, column 3 and may be the polypetides encoded by the nucleic acid molecules indicated in table I, application no. 7, column 7, resp., or may be the polypeptides indicated in table II, application no. 7, column 7, resp.

**[0024.0.0.7]** for the disclosure of the paragraph [0024.0.0.7] see [0024.0.0.0] above.

**[0025.0.7.7]** In accordance with the invention, a protein or polypeptide has the "activity of an protein as shown in table II, application no. 7, column 3 "if its de novo activity, or its increased expression directly or indirectly leads to an increase in the level of the fine chemical indicated in the respective line of table II, application no. 7, column 6 "metabolite" in the organism or a part thereof, preferably in a cell of said organism. Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 7, column 3, or which has at least 10% of the original enzymatic or biological activity, preferably 20%, particularly preferably 30%, most particularly preferably 40% in comparison to a protein as shown in the respective line of table II, application no. 7, column 3 of a E. coli or Saccharomyces cerevisae, respectively, protein as mentioned in table I to IV, column 3 respectively and as disclosed in paragraph [0022] of the respective invention.

**[0025.1.0.7] and [0025.2.0.7]** for the disclosure of the paragraphs [0025.1.0.7] and [0025.2.0.7] see paragraphs [0025.1.0.0] and [0025.2.0.0] above.

**[0026.0.0.7] to [0033.0.0.7]** for the disclosure of the paragraphs [0026.0.0.7] to [0033.0.0.7] see paragraphs [0026.0.0.0] to [0033.0.0.0] above.

**[0034.0.7.7]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 7, column 3 its biochemical or genetical causes and the increased amount of the respective fine chemical.

**[0035.0.0.7] to [0038.0.0.7] and [0039.0.5.7]** for the disclosure of the paragraphs [0035.0.0.7] to [0038.0.0.7] and [0039.0.5.7] see paragraphs [0035.0.0.0] to [0039.0.0.0] above.

**[0040.0.0.7] to [0044.0.0.7]** for the disclosure of the paragraphs [0040.0.0.7] to [0044.0.0.7] see paragraphs [0035.0.0.0] and [0044.0.0.0] above.

**[0045.0.7.7]** In one embodiment, the activity of the protein. as indicated in Table II, columns 5 or 7, application no. 7, is increased conferring an increase of the respective fine chemical, indicated in Table II, application no. 7, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more

**[0046.0.7.7]** In one embodiment, the activity of the protein as indicated in Table II, columns 5 or 7, application no. 7, is increased conferring an increase of the respective fine chemical, indicated in Table II, application no. 7, column 6 of the respective line confers an increase of the respective fine chemical and of further fatty acid or their precursors.

**[0047.0.0.7] to [0048.0.0.7]** for the disclosure of the paragraphs [0047.0.0.7] and [0048.0.0.7] see paragraphs [0047.0.0.0] and [0048.0.0.0] above.

**[0049.0.7.7]** A protein having an activity conferring an increase in the amount or level of the fine chemical, has in one embodiment the structure of the polypeptide described herein, in particular of the polypeptides comprising the consensus sequence shown in table IV, application no. 7, column 7 or of the polypeptide as shown in the amino acid sequences as disclosed in table II, application no. 7, columns 5 and 7 or the functional homologues thereof as described herein, or is encoded by the nucleic acid molecule characterized herein or the nucleic acid molecule according to the invention, for example by the nucleic acid molecule as shown in table I, application no. 7, columns 5 and 7 or its herein described functional homologues and has the herein mentioned activity.

**[0050.0.7.7]** For the purposes of the present invention, the term "stearic acid" also encompasses the corresponding salts, such as, for example, the potassium or sodium salts of stearic acid or the salts of stearic acid with amines such as diethylamine.

**[0051.0.5.7] and [0052.0.0.7]** for the disclosure of the paragraphs [0051.0.5.7] and [0052.0.0.7] see paragraphs [0051.0.0.0] and [0052.0.0.0] above.

**[0053.0.7.7]** In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 7, columns 5 and 7 or its homologs activity having herein-mentioned fatty acid of the invention increasing activity; and/or
b) stabilizing a mRNA conferring the increased expression of a protein encoded by the nucleic acid molecule of the invention as shown in table I, application no. 7, columns 5 and 7, e.g. a nucleic acid sequence encoding a polypeptide having the activity of a protein as indicated in table II, application no. 7, columns 5 and 7 or its homologs activity or of a mRNA encoding the polypeptide of the present invention having herein-mentioned fatty acid of the invention increasing activity; and/or
c) increasing the specific activity of a protein conferring the increased expression of a protein encoded by the nucleic acid molecule of the invention or of the polypeptide of the present invention having herein-mentioned fatty acid increasing activity, e.g. of a polypeptide having the activity of a protein as indicated in table II, application no. 7, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned fatty acid of the invention increasing activity, e.g. of a polypeptide having the activity of a protein as indicated in table II, application no. 7, columns 5 and 7 or its homologs activity; and/or
e) stimulating activity of a protein conferring the increased expression of a protein encoded by the nucleic acid molecule of the present invention or a polypeptide of the present invention having herein-mentioned fatty acid of the invention increasing activity, e.g. of a polypeptide having the activity of a protein as indicated in table II, application no. 7, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned fatty acid of the invention increasing activity, e.g. of a polypeptide having the activity of a protein as indicated in table II, application no. 7, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned fatty acid of the invention increasing activity, e.g. of a polypeptide having the activity of a protein as indicated in table II, application no. 7, columns 5 and 7 or its homologs activity; and/or
h) increasing the expression of the endogenous gene encoding the polypeptide of the invention, e.g. a polypeptide having the activity of a protein as indicated in table II, application no. 7, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with expecially high activity of the proteins of the invention from natural or from mutagenized resources and breeding them into the target organisms, eg the elite crops.

**[0054.0.7.7]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 7 in Table I to IV, resp., after increasing the expression or activity of the encoded polypeptide or having the activity of a polypeptide having an activity as the protein as shown in table II, application no. 7, column 3 or its homologs.

**[0055.0.0.7] to [0067.0.0.7]** for the disclosure of the paragraphs [0055.0.0.7] to [0067.0.0.7] see paragraphs [0055.0.0.0] to [0067.0.0.0] above.

**[0068.0.5.7] and [0069.0.5.7]** for the disclosure of the paragraphs [0068.0.5.7] and [0069.0.5.7] see paragraphs [0068.0.0.0] and [0069.0.0.0] above.

**[0070.0.6.7]** Owing to the introduction of a gene or a plurality of genes conferring the expression of the nucleic acid molecule of the invention or the nucleic acid molecule used in the method of the invention or the polypeptide of the invention or the polypeptide used in the method of the invention as described below, for example the nucleic acid construct mentioned below into an organism alone or in combination with other genes, it is possible not only to increase the biosynthetic flux towards the end product, but also to increase, modify or create de *novo* an advantageous, preferably novel metabolites composition in the organism, e.g. an advantageous fatty acid composition comprising a higher content of (from a viewpoint of nutrional physiology limited) fatty acids, like palmitate, palmitoleate, stearate and/or oleate.

**[0071.0.5.7]** for the disclosure of the paragraph [0071.0.5.7] see paragraph [0071.0.0.0] above.

**[0072.0.7.7]** By influencing the metabolism thus, it is possible to produce, in the process according to the invention, further advantageous compounds. Examples of such compounds are, in addition to stearic acid, triglycerides, lipids, oils and/or fats containing stearic acid compounds such as palmitate, palmitoleate, stearate, oleate, α-linolenic acid and/or linoleic acid.

**[0073.0.7.7]** Accordingly, in one embodiment, the process according to the invention relates to a process, which comprises:
(a) providing a non-human organism, preferably a microorganism, a non-human animal, a plant or animal cell, a plant or animal tissue or a plant;
(b) increasing the activity of a protein having the activity of a polypeptide of the invention or the polypeptide used in the method of the invention or a homolog thereof, e.g. as shown in Table II, application no. 7, columns 5 or 7, or of a polypeptide being encoded by the nucleic acid molecule of the present invention and described below, i.e. conferring an increase of the respective fine chemical in the organism, preferably a microorganism, the a non-human animal, a plant or animal cell, a plant or animal tissue or the plant,
(c) growing the organism, preferably a microorganism, a non-human animal, a plant or animal cell, a plant or animal tissue or the plant under conditions which permit the production of the fine chemical in the organism, preferably a microorganism, a plant cell, a plant tissue or the plant; and
(d) if desired, revovering, optionally isolating, the free and/or bound the respective fine chemical and, optionally further free and/or bound fatty acids synthetized by the organism, the microorganism, the non-human animal, the plant or animal cell, the plant or animal tissue or the plant.

**[0074.0.5.7]** The organism, in particular the microorganism, non-human animal, the plant or animal cell, the plant or animal tissue or the plant is advantageously grown in such a way that it is not only possible to recover, if desired isolate the free or bound the fine chemical or the free and bound the fine chemical but as option it is also possible to produce, recover and, if desired isolate, other free or/and bound fatty acids, in particular oleic acid.

**[0075.0.0.7] to [0084.0.0.7]** for the disclosure of the paragraphs [0075.0.0.7] to [0084.0.0.7] see paragraphs [0075.0.0.0] to [0084.0.0.0] above.

**[0085.0.7.7]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as shown in table I, application no. 7, columns 5 and 7 or a derivative thereof, or
b) a genetic regulatory element, for example a promoter, which is functionally linked to the nucleic acid sequence as shown table I, application no. 7, columns 5 and 7 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.7] and [0087.0.0.7]** for the disclosure of the paragraphs [0086.0.0.7] and [0087.0.0.7] see paragraphs [0086.0.0.0] and [0087.0.0.0] above.

**[0088.0.7.7]** In an advantageous embodiment of the invention, the organism takes the form of a plant whose fatty acid content is modified advantageously owing to the nucleic acid molecule of the present invention expressed. This is important for plant breeders since, for example, the nutritional value of plants for poultry is dependent on the abovementioned essential fatty acids and the general amount of fatty acids as energy source in feed. After the activity of a protein as shown in Table II, application no. 7, columns 5 or 7, has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.7], [0089.0.0.7], [0090.0.0.7] and [0091.0.5.7]** for the disclosure of the paragraphs [0088.1.0.7], [0089.0.0.7], [0090.0.0.7] and [0091.0.5.7] see paragraphs [0088.1.0.0], [0089.0.0.0], [0090.0.0.0] and [0091.0.0.0] above.

**[0092.0.0.7] to [0094.0.0.7]** for the disclosure of the paragraphs [0092.0.0.7] to [0094.0.0.7] see paragraphs [0092.0.0.0] to [0094.0.0.0] above.

**[0095.0.7.7]** It may be advantageous to increase the pool of free fatty acids in the transgenic organisms by the process according to the invention in order to isolate high amounts of the pure fine chemical

**[0096.0.7.7]** In another preferred embodiment of the invention a combination of the increased expression of the nucleic acid sequence or the protein of the invention together with the transformation of a protein or polypeptid for example a fatty acid transporter protein or a compound, which functions as a sink for the desired fatty acid for example for linoleic or linolenic acid in the organism is useful to increase the production of the respective fine chemical (see Bao and Ohlrogge, Plant Physiol. 1999 August; 120 (4): 1057-1062). Such fatty acid transporter protein may serve as a link between the location of fatty acid synthesis and the socalled sink tissue, in which fatty acids, triglycerides, oils and fats are stored.

**[0097.0.7.7]** for the disclosure of the paragraph [0097.0.7.7] see paragraph [0097.0.0.0] above.

**[0098.0.7.7]** In a preferred embodiment, the fine chemical (stearic acid) is produced in accordance with the invention and, if desired, is isolated. The production of further fatty acids such as palmitic acid, stearic acid, palmitoleic acid, oleic acid and/or linoleic acid mixtures thereof or mixtures of other fatty acids by the process according to the invention is advantageous.

**[0099.0.7.7]** In the case of the fermentation of microorganisms, the abovementioned fatty acids may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, spray granulation or by other methods. Preferably the fatty acids or the fatty acid compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.7.7]** Transgenic plants which comprise the fatty acids such as saturated or polyunsaturated fatty acids synthesized in the process according to the invention can advantageously be marketed directly without there being any need for the oils, lipids or fatty acids synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue. However, the fine chemical produced in the process according to the invention can also be isolated from the organisms, advantageously plants, in the form of their oils, fats, lipids and/or free fatty acids. Fatty acids produced by this process can be obtained by harvesting the organisms, either from the crop in which they grow, or from the field. This can be done via pressing or extraction of the plant parts, preferably the plant seeds. To increase the efficiency of oil extraction it is beneficial to clean, to temper and if necessary to hull and to flake the plant material especially the seeds. In this context, the oils, fats, lipids and/or free fatty acids can be obtained by what is known as cold beating or cold pressing without applying heat. To allow for greater ease of disruption of the plant parts, specifically the seeds, they are previously comminuted, steamed or roasted. The seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. In this manner, more than 96% of the compounds produced in the process can be isolated. Thereafter, the resulting products are processed further, i.e. degummed and/or refined. In this process, substances such as the plant mucilages and suspended matter are first removed. What is known as desliming can be affected enzymatically or, for example, chemico-physically by addition of acid such as phosphoric acid. Thereafter otionally, the free fatty acids are removed by treatment with a base like alkali, for example aqueous KOH or NaOH, or acid hydrolysis, advantageously in the presence of an alcohol such as methanol or ethanol, or via enzymatic cleavage, and isolated via, for example, phase separation and subsequent acidification via, for example, H₂SO₄. The fatty acids can also be liberated directly without the above-described processing step. If desired the resulting product can be washed thoroughly with water to remove traces of soap and the alkali remaining in the product and then dried. To remove the pigment remaining in the product, the products can be subjected to bleaching, for example using filler's earth or active charcoal. At the end, the product can be deodorized, for example using steam distillation under vacuum. These chemically pure fatty acids or fatty acid compositions are advantageous for applications in the food industry sector, the cosmetic sector and especially the pharmacological industry sector.

**[00101.0.5.7]** for the disclosure of the paragraph [0101.0.5.7] see paragraph [0101.0.0.0] above.

**[0102.0.7.7]** Fatty acids can for example be detected advantageously via GC separation methods. The unambiguous detection for the presence of fatty acid products can be obtained by analyzing recombinant organisms using analytical standard methods: GC, GC-MS or TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353). One example is the analysis of fatty acids via FAME and GC-MS or TLC (abbreviations: FAME, fatty acid methyl ester; GC-MS, gas liquid chromatography/mass spectrometry; TLC, thin-layer chromatography. The material to be analyzed can be disrupted by sonication, grinding in a glass mill, liquid nitrogen and grinding or via other applicable methods. After disruption, the material must be centrifuged. The sediment is resuspended in distilled water, heated for 10 minutes at 100°C, cooled on ice and recentrifuged, followed by extraction for one hour at 90°C in 0.5 M sulfuric acid in methanol with 2% dimethoxypropane, which leads to hydrolyzed oil and lipid compounds, which give transmethylated lipids. These fatty acid methyl esters are extracted in petroleum ether and finally subjected to a GC analysis using a capillary column (Chrompack, WCOT Fused Silica, CP-Wax-52 CB, 25 µm, 0.32 mm) at a temperature gradient of between 170°C and 240°C for 20 minutes and 5 minutes at 240°C. The identity of the resulting fatty acid methyl esters must be defined using standards which are available from commercial sources (i.e. Sigma).

**[0103.0.7.7]** In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of a polypeptide having a sequence as shown in Table II, application no. 7, columns 5 or 7, in or a fragment thereof, which confers an increase in the amount of the respective fine chemical in an organism or a part thereof;
b) nucleic acid molecule comprising, preferably at least the mature form, of the nucleic acid molecule having a sequence as shown in Table I, application no. 7, columns 5 or 7,
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as shown in Table III, application no. 7, column 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequence as shown in Table IV, application no. 7, column 7, and conferring an increase in the amount of the respective fine chemical in an organism or a part thereof ;
k) nucleic acid molecule comprising one or more of the nucleic acid molecule encoding the amino acid sequence of a polypeptide encoding a domain of a polypeptide as shown in Table II, application no. 7, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof; or which comprises a sequence which is complementary thereto.

**[0103.1.0.7] and [0103.2.0.7]** for the disclosure of the paragraphs [0103.1.0.7] and [0103.2.0.7] see paragraphs [0103.1.0.0] and [0103.2.0.0] above.

**[0104.0.7.7]** In one embodiment, the nucleic acid molecule used in the process distinguishes over the sequence shown in Table I, application no. 7, columns 5 or 7, by one or more nucleotides or does not consist of the sequence shown in Table I, application no. 7, columns 5 or 7. In one embodiment, the nucleic acid molecule of the present invention is less than 100%, 99,999%, 99,99%, 99,9% or 99% identical to the sequence shown in Table I, application no. 7, columns 5 or 7. In another embodiment, the nucleic acid molecule does not encode a polypeptide of the sequence shown in Table II, application no. 7, columns 5 or 7.

**[0105.0.0.7] to [0107.0.0.7]** for the disclosure of the paragraphs [0105.0.0.7] to [0107.0.0.7] see paragraphs [0105.0.0.0] and [0107.0.0.0] above.

**[0108.0.7.7]** Nucleic acid molecules with the sequence shown in Table I, application no. 7, columns 5 or 7, nucleic acid molecules which are derived from the amino acid sequences shown in Table II, application no. 7, columns 5 or 7, or from polypeptides comprising the consensus sequence shown in Table IV, application no. 7, column 7, or their derivatives or homologues encoding polypeptides with the enzymatic or biological activity of a protein as shown in Table II, application no. 7, columns 5 or 7, or e.g. conferring a linoleic acid increase after increasing its expression or activity are advantageously increased in the process according to the invention.

**[0109.0.5.7]** for the disclosure of this paragraph see [0109.0.0.0] above.

**[0110.0.7.7]** Nucleic acid molecules, which are advantageous for the process according to the invention and which encode polypeptides with an activity of a polypeptide used in the method of the invention or used in the process of the invention, e.g. of a protein as shown in Table II, application no. 7, columns 5 or 7, or being encoded by a nucleic acid molecule indicated in Table I, application no. 7, columns 5 or 7, or of its homologs, e.g. as indicated in Table II, application no. 7, columns 5 or 7, can be determined from generally accessible databases.

**[0111.0.0.7]** for the disclosure of this paragraph see [0111.0.0.0] above.

**[0112.0.7.7]** The nucleic acid molecules used in the process according to the invention take the form of isolated nucleic acid sequences, which encode polypeptides with an activity of a polypeptide as indicated in Table II, application no. 7, column3, or having the sequence of a polypeptide as indicated in Table II, application no. 7, columns 5 and 7, and conferring an increase of the respective fine chemical.

**[0113.0.0.7] to [0120.0.0.7]** for the disclosure of the paragraphs [0113.0.0.7] to [0120.0.0.7] see paragraphs [0113.0.0.0] and [0120.0.0.0] above.

**[0121.0.7.7]** However, it is also possible to use artificial sequences, which differ in one or more bases from the nucleic acid sequences found in organisms, or in one or more amino acid molecules from polypeptide sequences found in organisms, in particular from the polypeptide sequences shown in Table II, application no. 7, columns 5 or 7, or the functional homologues thereof as described herein, preferably conferring above-mentioned activity, i.e. conferring an increase of the respective fine chemical after increasing its activity.

**[0122.0.0.7] to [0127.0.0.7]** for the disclosure of the paragraphs [0122.0.0.7] to [0127.0.0.7] see paragraphs [0122.0.0.0] and [0127.0.0.0] above.

**[0128.0.7.7]** Synthetic oligonucleotide primers for the amplification, e.g. as shown in Table III, application no. 7, column 7, by means of polymerase chain reaction can be generated on the basis of a sequence shown herein, for example the sequence shown in Table I, application no. 7, columns 5 or 7, or the sequences as shown in Table II, application no. 7, columns 5 or 7.

**[0129.0.7.7]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequence shown in Table IV, application no. 7, column 7, is derived from said aligments.

**[0130.0.7.7]** for the disclosure of this paragraph see [0130.0.0.0].

**[0131.0.0.7] to [0138.0.0.7]** for the disclosure of the paragraphs [0131.0.0.7] to [0138.0.0.7] see paragraphs [0131.0.0.0] to [0138.0.0.0] above.

**[0139.0.7.7]** Polypeptides having above-mentioned activity, i.e. conferring the respective fine chemical increase, derived from other organisms, can be encoded by other DNA sequences which hybridize to the sequences shown in Table I, application no. 7, columns 5 or 7, under relaxed hybridization conditions and which code on expression for peptides having the stearic acid increasing activity.

**[0140.0.0.7] to [0146.0.0.7]** for the disclosure of the paragraphs [0140.0.0.7] to [0146.0.0.7] see paragraphs [0140.0.0.0] and [0146.0.0.0] above.

**[0147.0.7.7]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 7, columns 5 or 7 is one which is sufficiently complementary to one of said nucleotide sequences such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.7.7]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table **I,** application no. 7, columns 5 or 7, or a functional portion thereof and preferably has above mentioned activity, in particular has the-fine-chemical-increasing activity after increasing its acitivity or an activity of a product of a gene encoding said sequence or its homologs.

**[0149.0.7.7]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 7, columns 5 or 7, or a portion thereof and encodes a protein having above-mentioned activity as indicated in Table II, application no. 7, columns 5 or 7, e.g. conferring an increase of the respective fine chemical.

**[00149.1.0.7]** Optionally, the nucleotide sequence, which hybridises to one of the nucleotide sequences indicated in Table I, application no. 7, columns 5 or 7, has further one or more of the activities annotated or known for the a protein as indicated in Table II, application no. 7, column 3.

**[0150.0.7.7]** Moreover, the nucleic acid molecule of the invention or used in the process of the invention can comprise only a portion of the coding region of one of the sequences indicated in Table I, application no. 7, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of methionine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, application no. 7, columns 5 or 7, an anti-sense sequence of one of the sequences indicated in Table I, application no. 7, columns 5 or 7, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 7, column 7, will result in a fragment of a polynucleotide sequence as indicated in Table I, application no. 7, columns 5 or 7.

**[0151.0.0.7]** for the disclosure of this paragraph see [0151.0.0.0] above.

**[0152.0.7.7]** The nucleic acid molecule of the invention encodes a polypeptide or portion thereof which includes an amino acid sequence which is sufficiently homologous to the amino acid sequence shown in Table II, application no. 7, columns 5 or 7, such that the protein or portion thereof maintains the ability to participate in the fine chemical production, in particular a stearic acid increasing the activity as mentioned above or as described in the examples in plants or microorganisms is comprised.

**[0153.0.7.7]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in Table II, application no. 7, columns 5 or 7, such that the protein or portion thereof is able to participate in the increase of the respective fine chemical production. In one embodiment, a protein or protion thereof as shown in Table II, application no. 7, columns 5 or 7, has for example an activity of a polypeptide as indicated in Table II, application no. 7, columns 5 or 7are described herein.

**[0154.0.7.7]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%,

91%, 92%, 93% or 94% and most preferably at least about 95%, 97%, 98%, 99% or more homologous to an entire amino acid sequence as shown in Table II, application no. 7, columns 5 or 7, and having above-mentioned activity, e.g.conferring preferably the increase of the respective fine chemical.

**[0155.0.0.7] and [0156.0.0.7]** for the disclosure of the paragraphs [0155.0.0.7] and [0156.0.0.7] see paragraphs [0155.0.0.0] and [0156.0.0.0] above.

**[0157.0.7.7]** The invention further relates to nucleic acid molecules that differ from one of the nucleotide sequences indicated in Table I, application no. 7, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, application no. 7, column 7, or of the polypeptide as indicated in Table II, application no. 7, columns 5 or 7, or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, application no. 7, column 7, or of the polypeptide as indicated in Table II, application no. 7, columns 5 or 7, or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, application no. 7, column 7, or of a polypeptide as indicated in Table II, application no. 7, columns 5 or 7, or the functional homologues thereof. However, in a preferred embodiment, the nucleic acid molecule of the present invention does not consist of a sequence as indicated in Table I, application no. 7, columns 5 or 7,

**[0158.0.0.7] to [0160.0.0.7]** for the disclosure of the paragraphs [0158.0.0.7] to [0160.0.0.7] see paragraphs [0158.0.0.0] to [0160.0.0.0] above.

**[0161.0.7.7]** Accordingly, in another embodiment, a nucleic acid molecule of the invention or the nucleic acid molecule used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising the sequence shown in Table I, application no. 7, columns 5 or 7. The nucleic acid molecule is preferably at least 20, 30, 50, 100, 250 or more nucleotides in length.

**[0162.0.0.7]** for the disclosure of this paragraph see paragraph [0162.0.0.0] above.

**[0163.0.7.7]** Preferably, nucleic acid molecule of the invention that hybridizes under stringent conditions to a sequence as indicated in Table I, application no. 7, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.7]** for the disclosure of this paragraph see paragraph [0164.0.0.0] above.

**[0165.0.7.7]** For example, nucleotide substitutions leading to amino acid substitutions at "non-essential" amino acid residues can be made in a sequence of the nucleic acid molecule of the invention or used in the process of the invention, e.g. as shown in Table I, application no. 7, columns 5 or 7.

**[0166.0.0.7] and [0167.0.0.7]** for the disclosure of the paragraphs [0166.0.0.7] and [0167.0.0.7] see paragraphs [0166.0.0.0] and [0167.0.0.0] above.

**[0168.0.7.7]** Accordingly, the invention relates to nucleic acid molecules encoding a polypeptide having above-mentioned activity, e.g. conferring an increase in the respective fine chemical in an organism or parts thereof that contain changes in amino acid residues that are not essential for said activity. Such polypeptides differ in amino
acid sequence from a sequence contained in a sequence as indicated in Table II, application no. 7, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 7, columns 5 or 7, and is capable of participation in the increase of production of the respective fine chemical after increasing its activity, e.g. its expression. Preferably, the protein encoded by the nucleic acid molecule is at least about 60% identical to the sequence as indicated in Table II, application no. 7, columns 5 or 7, more preferably at least about 70% identical to one of the sequences as indicated in Table II, application no. 7, columns 5 or 7, even more preferably at least about 80%, 90%, 95% homologous to the sequence as indicated in Table II, application no. 7, columns 5 or 7, and most preferably at least about 96%, 97%, 98%, or 99% identical to the sequence as indicated in Table II, application no. 7, columns 5 or 7.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 7, columns 5 or 7yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 7, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 7, columns 5 or 7, more preferably at least about 70% identical to one of the sequences as indicated in Table II, application no. 7, columns 5 or 7, even more preferably at least about 80%, 90%, or 95% homologous to a sequence as indicated in Table II, application no. 7, columns 5 or 7, and most preferably at least about 96%, 97%, 98%, or 99% identical to the sequence as indicated in Table II, application no. 7, columns 5 or 7.

**[0169.0.0.7] to [0175.0.5.7]** for the disclosure of the paragraphs [0169.0.0.7] to [0175.0.5.7] see paragraphs [0169.0.0.0] to [0175.0.0.0] above.

**[0176.0.7.7]** Functional equivalents derived from one of the polypeptides as shown in Table II, application no. 7, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, application no. 7, columns 5 or 7, according to the invention and are distinguished by essentially the same properties as the polypeptide as shown in Table II, application no. 7, columns 5 or 7.

**[0177.0.7.7]** Functional equivalents derived from the nucleic acid sequence as shown in Table I, application no. 7, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, application no. 7, columns 5 or 7, according to the invention and encode polypeptides having essentially the same properties as the polypeptide as shown in Table II, application no. 7, columns 5 or 7.

**[0178.0.0.7]** for the disclosure of this paragraph see [0178.0.0.0] above.

**[0179.0.7.7]** A nucleic acid molecule encoding a homologous to a protein sequence of as indicated in Table II, application no. 7, columns 5 or 7, can be created by introducing one or more nucleotide substitutions, additions or deletions into a nucleotide sequence of the nucleic acid molecule of the present invention, in particular as indicated in Table I, application no. 7, columns 5 or 7, such that one or more amino acid substitutions, additions or deletions are introduced into the encoded protein. Mutations can be introduced into the encoding sequences for example into a sequences as indicated in Table I, application no. 7, columns 5 or 7, by standard techniques, such as site-directed mutagenesis and PCR-mediated mutagenesis.

**[0180.0.0.7] to [0183.0.0.7]** for the disclosure of the paragraphs [0180.0.0.7] to [0183.0.0.7] see paragraphs [0180.0.0.0] to [0183.0.0.0] above.

**[0184.0.7.7]** Homologues of the nucleic acid sequences used, with a sequence as indicated in Table I, application no. 7, columns 5 or 7, or of the nucleic acid sequences derived from a sequences as indicated in Table II, application no. 7, columns 5 or 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 7, columns 5 or 7, or from the derived nucleic acid sequences, the intention being, however, that the enzyme activity or the biological activity of the resulting proteins synthesized is advantageously retained or increased.

**[0185.0.7.7]** In one embodiment of the present invention, the nucleic acid molecule of the invention or used in the process of the invention comprises one or more sequences as indicated in Table I, application no. 7, columns 5 or 7. In one embodiment, it is preferred that the nucleic acid molecule comprises as little as possible other nucleotide sequences not shown in any one of sequences as indicated in Table I, application no. 7, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 7, columns 5 or 7.

**[0186.0.7.7]** Also preferred is that one or more nucleic acid molecule(s) used in the process of the invention encode a polypeptide comprising a sequence as indicated in Table II, application no. 7, columns 5 or 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 7, columns 5 or 7.

**[0187.0.7.7]** In one embodiment, the nucleic acid molecule of the invention or used in the process encodes a polypeptide comprising a sequence as indicated in Table II, application no. 7, columns 5 or 7, comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 7, columns 5 or 7.

**[0188.0.7.7]** Polypeptides (= proteins), which still have the essential biological activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide shown in Table II, application no. 7, columns 5 or 7, expressed under identical conditions.

**[0189.0.7.7]** Homologues of sequences as indicated in Table I, application no. 7, columns 5 or 7, or of the derived sequences shown in Table II, application no. 7, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.7], [0191.0.5.7], [00191.1.0.7] and [0192.0.0.7] to [0203.0.0.7] for the** disclosure of the paragraphs [0190.0.0.7], [0191.0.5.7], [0191.1.0.7] and [0192.0.0.7] to [0203.0.0.7] see paragraphs [0190.0.0.0], [0191.0.0.0], [0191.1.0.0] and [0192.0.0.0] to [0203.0.0.0] above.

**[0204.0.7.7]** Accordingly, in one embodiment, the invention relates to a nucleic acid molecule, which comprises a nucleic acid molecule selected from the group consisting of:
a) nucleic acid molecule encoding, preferably at least the mature form, of the polypeptide shown in Table II, application no. 7, columns 5 or 7; or a fragment thereof conferring an increase in the amount of the fine chemical in an organism or a part thereof
b) nucleic acid molecule comprising, preferably at least the mature form, of the nucleic acid molecule shown in Table I, application no. 7, columns 5 or 7,; or a fragment thereof conferring an increase in the amount of the fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using the primers or primer pairs as indicated in Table III, application no. 7, column 7, and conferring an increase in the amount of the respective fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising the consensus sequence shown in Table IV, application no. 7, column 7, and conferring an increase in the amount of the respective fine chemical in an organism or a part thereof;
k) nucleic acid molecule encoding the amino acid sequence of a polypeptide encoding a domaine of the polypeptide shown in Table II, application no. 7, columns 5 or 7; and conferring an increase in the amount of the respective fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of the nucleic acid molecule shown in Table I, application no. 7, columns 5 or 7, or a nucleic acid molecule encoding, preferably at least the mature form of, the polypeptide shown in Table II, application no. 7, columns 5 or 7, and conferring an increase in the amount of the respective fine chemical in an organism or a part thereof; or which encompasses a sequence which is complementary thereto;
whereby, preferably, the nucleic acid molecule according to (a) to (I) distinguishes over the sequence as depicted in Table I, application no. 7, columns 5 or 7, by one or more nucleotides. In one embodiment, the nucleic acid molecule of the invention does not consist of the sequence shown in Table I, application no. 7, columns 5 or 7. In an other embodiment, the nucleic acid molecule of the present invention is at least 30 % identical and less than 100%, 99,999%, 99,99%, 99,9% or 99% identical to the sequence shown in Table I, application no. 7, columns 5 or 7. In a further embodiment the nucleic acid molecule does not encode the polypeptide sequence shown in Table II, application no. 7, columns 5 or 7. Accordingly, in one embodiment, the nucleic acid molecule of the present invention encodes in one embodiment a polypeptide which differs at least in one or more amino acids from the polypeptide as depicted in Table II, application no. 7, columns 5 or 7, and therefore does not encode a protein of the sequence shown in Table II, application no. 7, columns 5 or 7. Accordingly, in one embodiment, the protein encoded by a sequence of a nucleic acid according to (a) to (I) does not consist of the sequence shown in Table II, application no. 7, columns 5 or 7. In a further embodiment, the protein of the present invention is at least 30 % identical to protein sequence depicted in Table II, application no. 7, columns 5 or 7, and less than 100%, preferably less than 99,999%, 99,99% or 99,9%, more preferably less than 99%, 985, 97%, 96% or 95% identical to the sequence shown in Table II, application no. 7, columns 5 or 7.

**[0205.0.0.7] to [0226.0.0.7]** for the disclosure of the paragraphs [0205.0.0.7] to [0226.0.0.7] see paragraphs [0205.0.0.0] to [0226.0.0.0] above.

**[0227.0.7.7]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to the sequence mentioned in Table I, application no. 7, columns 5 or 7, or its derivatives, it is advantageous additionally to express and/or mutate further genes in the organisms. Especially advantageously, additionally at least one further gene of the fatty acid biosynthetic pathway such as for palmitate, palmitoleate, stearate and/or oleate is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine the sequences shown in Table I, application no. 7, columns 5 or 7, with genes which generally support or enhances to growth or yield of the target organismen, for example genes which lead to faster growth rate of microorganisms or genes which produces stress-, pathogen, or herbicide resistant plants.

**[0228.0.5.7] to [0230.0.5.7]** for the disclosure of the paragraphs [0228.0.5.7] to [0230.0.5.7] see paragraphs [0228.0.0.0] to [0230.0.0.0] above.

**[0231.0.7.7]** In a further advantageous embodiment of the process of the invention, the organisms used in the process are those in which simultaneously a stearic acid degrading protein is attenuated, in particular by reducing the rate of expression of the corresponding gene.

**[0232.0.0.7] to [0276.0.0.7]** for the disclosure of the paragraphs [0232.0.0.7] to [0276.0.0.7] see paragraphs [0232.0.0.0] to [0276.0.0.0] above.

**[0277.0.5.7]** for the disclosure of this paragraph see paragraph [0277.0.5.5] above.

**[0278.0.0.7] to [0282.0.0.7]** for the disclosure of the paragraphs [0278.0.0.7] to [0282.0.0.7] see paragraphs [0278.0.0.0] to [0282.0.0.0] above.

**[0283.0.7.7]** Moreover, native polypeptide conferring the increase of the respective fine chemical in an organism or part thereof can be isolated from cells (e.g., endothelial cells), for example using the antibody of the present invention as described below, in particular, an antibody against polypeptides as shown in Table II, application no. 7, columns 5 or 7, or an antigenic part thereof, which can be produced by standard techniques utilizing polypeptides comprising or consisting of abovementioned sequences, e.g. the polypeptid of the present invention or fragment thereof. Preferred are monoclonal antibodies specifically binding to polypeptides as indicated in Table II, application no. 7, columns 5 or 7.

**[0284.0.0.7]** for the disclosure of this paragraph see [0284.0.0.0] above.

**[0285.0.7.7]** In one embodiment, the present invention relates to a polypeptide having the sequence shown in Table II, application no. 7, columns 5 or 7, or as coded by the nucleic acid molecule shown in Table I, application no. 7, columns 5 or 7, or functional homologues thereof.

**[0286.0.7.7]** In one advantageous embodiment, in the method of the present invention the activity of a polypeptide is increased which comprises or consists of a consensus sequence as indicated in Table IV, application no. 7, column 7, and in one another embodiment, the present invention relates to a polypeptide comprising or consisting of a consensus sequence as indicated in Table IV, application no. 7, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a poylpeptide or to a polypeptide comprising more than one consensus sequences as indicated in Table IV, application no. 7, column 7.

**[0287.0.0.7] to [0290.0.0.7]** for the disclosure of the paragraphs [0287.0.0.7] to [0290.0.0.7] see paragraphs [0287.0.0.0] to [0290.0.0.0] above.

**[0291.0.7.7]** In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of a plant or microorganism specific consensus sequences.
In one embodiment, said polypeptide of the invention distinguishes over the sequence as indicated in Table, application no. 7, columns 5 or 7, by one or more amino acids. In one embodiment, polypeptide distinguishes form the sequence shown in Table II, application no. 7, columns 5 or 7, by more than 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from the sequence shown in Table II, application no. 7, columns 5 or 7, by not more than 80% or 70% of the amino acids, preferably not more than 60% or 50%, more preferred not more than 40% or 30%, even more preferred not more than 20% or 10%. In another embodiment, said polypeptide of the invention does not consist of the sequence shown in Table II, application no. 7, columns 5 or 7.

**[0292.0.0.7]** for the disclosure of this paragraph see [0292.0.0.0] above.

**[0293.0.7.7]** In one embodiment, the invention relates to polypeptide conferring an increase in the respective fine chemical in an organism or part being encoded by the nucleic acid molecule of the invention or by a nucleic acid molecule used in the process.
In one embodiment, the polypeptide of the invention has a sequence, which distinguishes from the sequence as shown in Table II, application no. 7, columns 5 or 7, by one or more amino acids. In another embodiment, said polypeptide of the invention does not consist of the sequence shown in Table II, application no. 7, columns 5 or 7. In a further embodiment, said polypeptide of the present invention is less than 100%, 99,999%, 99,99%, 99,9% or 99% identical. In one embodiment, said polypeptide does not consist of the sequence encoded by the nucleic acid molecules shown in Table I, application no. 7, columns 5 or 7.

**[0294.0.7.7]** In one embodiment, the present invention relates to a polypeptide having an activity of a protein as indicated in Table II, application no. 7, column 3, which distinguishes over the sequence as indicated in Table II, application no. 7, columns 5 or 7by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.7], [0296.0.0.7] and [0297.0.7.7]** for the disclosure of the paragraphs [0295.0.0.7], [0296.0.0.7] and [0297.0.7.7] see paragraphs [0295.0.0.0] to [0297.0.0.0] above.

**[00297.1.0.7]** Non-polypeptide of the invention-chemicals are e.g. polypeptides having not the activity and/or the amino acid sequence of a polypeptide indicated in Table II, application no. 7, columns 3, 5 or 7.

**[0298.0.7.7]** A polypeptide of the invention can participate in the process of the present invention. The polypeptide or a portion thereof comprises preferably an amino acid sequence which is sufficiently homologous to an amino acid sequence as indicated in Table II, application no. 7, columns 5 or 7, such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, application no. 7, columns 5 or 7.

**[0299.0.7.7]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences of Table II, application no. 7, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table I, application no. 7, columns 5 or 7, or which is homologous thereto, as defined above.

**[0300.0.7.7]** Accordingly the polypeptide of the present invention can vary from a sequence as indicated in Table II, columns 5 or 7, lines 72 to 81 and 460 to 462 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II, application no. 7, columns 5 or 7.

**[0301.0.0.7]** for the disclosure of this paragraph see [0301.0.0.0] above.

**[0302.0.7.7]** Biologically active portions of an polypeptide of the present invention include peptides comprising amino acid sequences derived from the amino acid sequence of the polypeptide of the present invention or used in the process of the present invention, e.g., an amino acid sequence shown in Table II, application no. 7, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.7]** for the disclosure of this paragraph see [0303.0.0.0] above.

**[0304.0.7.7]** Manipulation of the nucleic acid molecule of the invention or the nucleic acid molecule used in the method of the invention may result in the production of a protein having essentially the activity of the polypeptides as indicated in Table II, application no. 7, columns 5 or 7, but having differences in the sequence from said wild-type protein. These proteins may be improved in efficiency or activity, may be present in greater numbers in the cell than is usual, or may be decreased in efficiency or activity in relation to the wild type protein.

**[0305.0.5.7], [0306.0.5.7] and [0306.1.0.7]** for the disclosure of the paragraphs [0305.0.5.7], [0306.0.5.7] and [0306.1.0.7] see paragraphs [0305.0.0.0], [0306.0.0.0] and [0306.1.0.0] above.

**[0307.0.0.7] and [0308.0.0.7]** for the disclosure of the paragraphs [0307.0.0.7] and [0308.0.0.7] see paragraphs [0307.0.0.0 and [0308.0.0.0] above.

**[0309.0.7.7]** In one embodiment, a reference to protein (= polypeptide)" of the invention e.g. as indicated in Table II, application no. 7, columns 5 or 7, refers to a polypeptide having an amino acid sequence corresponding to the polypeptide of the invention or used in the process of the invention, whereas a "non- polypeptide" or "other polypeptide" e.g. not indicated in Table II, application no. 7, columns 5 or 7refers to a polypeptide having an amino acid sequence corresponding to a protein which is not substantially homologous to a polypeptide of the invention, preferably which is not substantially homologous to a polypeptide having a protein activity, e.g., a protein which does not confer the activity described herein and which is derived from the same or a different organism.

**[0310.0.0.7] to [0334.0.0.7]** for the disclosure of the paragraphs [0310.0.0.7] to [0334.0.0.7] see paragraphs [0310.0.0.0] to [0334.0.0.0] above.

**[0335.0.7.7]** The dsRNAi method has proved to be particularly effective and advantageous for reducing the expression of a nucleic acid sequence as indicated in Table I, application no. 7, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, application no. 7, columns 5 or 7, and/or homologs thereof. In a double-stranded RNA molecule for reducing the expression of an protein encoded by a nucleic acid sequence of one of the sequences as indicated in Table I, application no. 7, columns 5 or 7and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.7] to [0342.0.0.7]** for the disclosure of the paragraphs [0336.0.0.7] to [0342.0.0.7] see paragraphs [0336.0.0.0] to [0342.0.0.0] above.

**[0343.0.7.7]** As has already been described, 100 % sequence identity between the dsRNA and a gene transcript of a nucleic acid sequence as indicated in Table I, application no. 7, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of the sequences as indicated in Table I, application no. 7, columns 5 or 7, or homologs thereof of the one organism, may be used to suppress the corresponding expression in another organism.

**[0344.0.0.7] to [0350.0.0.7], [0351.0.7.7] and [0352.0.0.7] to [0361.0.0.7]** for the disclosure of the paragraphs [0344.0.0.7] to [0350.0.0.7], [0351.0.7.7] and [0352.0.0.7] to [0361.0.0.7] see paragraphs [0344.0.0.0] to [0361.0.0.0] above.

**[0362.0.7.7]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention, e.g. encoding a polypeptide having an activity of a protein such as the polypeptides as indicated in Table II, application no. 7, column 3. Due to the above mentioned activity the fine chemical content in a cell or an organism is increased. For example, due to modulation or manupulation, the cellular activity is increased, e.g. due to an increased expression or specific activity of the subject matters of the invention in a cell or an organism or a part thereof. Transgenic for a polypeptide having an activity of a protein such as the polypeptides as indicated in Table II, application no. 7, column 3. Activity means herein that due to modulation or manipulation of the genome, the activity of polypeptide having an activity of a protein such as the polypeptides as indicated in Table II, application no. 7, column 3, or a similar activity, which is increased in the cell or organism or part thereof. Examples are described above in context with the process of the invention.

**[0363.0.0.7], [0364.0.7.7] and [0365.0.0.7] to [0379.0.7.7]** for the disclosure of the paragraphs [0363.0.0.7], [0364.0.7.7] and [0365.0.0.7] to [0379.0.7.7] see paragraphs [0363.0.0.0] to [0379.0.0.0] above.

**[0380.0.5.7],** The fatty acids obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of a pharmaceuticals, food stuff, animal feeds, nutrients or cosmetics comprising the steps of the process according to the invention, including the isolation of the fatty acid composition produced or the fine chemical produced if desired and formulating the product with a pharmaceutical acceptable carrier or formulating the product in a form acceptable for an application in agriculture. A further embodiment according to the invention is the use of the fatty acids produced in the process or of the transgenic organisms in animal feeds, foodstuffs, medicines, food supplements, cosmetics or pharmaceuticals.

**[0381.0.0.7] and [0382.0.0.7]** for the disclosure of the paragraphs [0381.0.0.7] and [0382.0.0.7] see paragraphs [0381.0.0.0] and [0382.0.0.0] above.

**[0383.0.7.7]** For preparing fatty acid compound-containing fine chemicals, in particular the fine chemical, it is possible to use as fatty acid source organic compounds such as, for example, oils, fats and/or lipids comprising fatty acids such as fatty acids having a carbon back bone between C₁₀- to C₁₆- carbon atoms and/or small organic acids such acetic acid, propionic acid or butanoic acid as precursor compounds.

**[0384.0.0.7]** for the disclosure of the paragraph [0384.0.0.7see paragraph [0384.0.0.0] above.

**[0386.0.7.7]** However, it is also possible to purify the fatty acid produced further. For this purpose, the product-containing composition is subjected for example to a thin layer chromatography on silica gel plates or to a chromatography such as a Florisil column (Bouhours J.F., J. Chromatrogr. 1979, 169, 462), in which case the desired product or the impurities are retained wholly or partly on the chromatography resin. These chromatography steps can be repeated if necessary, using the same or different chromatography resins. The skilled worker is familiar with the choice of suitable chromatography resins and their most effective use. An alternative method to purify the fatty acids is for example a crystallization in the presence of urea. These methods can be combined with each other.

**[0387.0.0.7] to [0392.0.0.7]** for the disclosure of the paragraphs [0387.0.0.7] to [0392.0.0.7] see paragraphs [0387.0.0.0] to [0392.0.0.0] above.

**[0393.0.7.7]** In one embodiment, the present invention relates to a method for the identification of a gene product conferring an increase in the fine chemical production in a cell, comprising the following steps:
(a) contacting-, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence shown in Table I, application no. 7, columns 5 or 7, and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the respective fine chemical level in the host cells; and
(f) identifying the nucleic acid molecule and its gene product which expression confers an increase in the respective fine chemical level in the host cell after expression compared to the wild type.

**[0394.0.0.7] to [0415.0.0.7] and [0416.0.7.7]** for the disclosure of the paragraphs [0394.0.0.7] to [0415.0.0.7] and [0416.0.7.7] see paragraphs [0394.0.0.0] to [0416.0.0.0] above.

**[0417.0.7.7]** The nucleic acid molecule of the invention, the vector of the invention or the nucleic acid construct of the invention may also be useful for the production of organisms resistant to inhibitors of the fatty acid production biosynthesis pathways. In particular, the overexpression of the polypeptide of the present invention may protect an organism such as a microorganism or a plant against inhibitors, which block the fatty acid, in particular the fine chemical, synthesis in said organism. Examples of inhibitors or herbicides blocking the fatty acid synthesis in organism such as microorganism or plants are for example cerulenin, Thiolactomycin, Diazoborines or Triclosan, which inhibit the fatty acids (beta-ketoacyl thioester synthetase inhibitors) and sterol biosynthesis inhibitors, aryloxyphenoxypropionates such as diclofop, fenoxaprop, haloxyfop, fluazifop or quizalofop or cyclohexanediones such as clethodim or sethoxydim [(2-[1-{ethoxyimino}butyl]-5-[2-{ethylthio}propyl]-3-hydroxy-2-cyclohexen-1-one], which inhibit the plant acetyl-coenzyme A carboxylase or thiocarbamates such as butylate, EPTC [= S-ethyl dipropylcarbamothioat] or vernolate. **[0418.0.0.7] to [0430.0.0.7]** for the disclosure of the paragraphs [0418.0.0.7] to [0430.0.0.7] see paragraphs [0418.0.0.0] to [0430.0.0.0] above.

**[0431.0.7.7], [0432.0.7.7], [0433.0.0.7] and [0434.0.0.7]** for the disclosure of the paragraphs [0431.0.7.7], [0432.0.7.7], [0433.0.0.7] and [0434.0.0.7] see paragraphs [0431.0.0.0] to [0434.0.0.0] above.

**[0435.0.5.7]** Example 3: In-vivo and in-vitro mutagenesis

**[0436.0.7.7]** An *in vivo* mutagenesis of organisms such as Saccharomyces, Mortierella, Escherichia and others mentioned above, which are beneficial for the production of fatty acids can be carried out by passing a plasmid DNA (or another vector DNA) containing the desired nucleic acid sequence or nucleic acid sequences through E. coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired fine chemical.

**[0437.0.7.7]** Example 4: DNA transfer between Escherichia coli, Saccharomyces cerevisiae and Mortierella alpina

**[0438.0.7.7]** Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which alow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology, VCH, Weinheim) or for other antibiotic resistance genes such as for G418, gentamycin, neomycin, hygromycin or tetracycline resistance.

**[0439.0.7.7]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255269) or electroporation (Delorme E., Appl. Environ. Microbiol., vol. 55, no. 9, 1989 : 2242 - 2246).

**[0440.0.7.7]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.7]** for the disclosure of the paragraph [0441.0.0.7] see [0441.0.0.0] above.

**[0442.0.0.7]** The observations of the acivity of a mutated, or transgenic, protein in a transformed host cell are based on the fact that the protein is expressed in a similar manner and in a similar quantity as the wild-type protein. A suitable method for determining the transcription quantity of the mutant, or transgenic, gene (a sign for the amount of mRNA which is available for the translation of the gene product) is to carry out a Northern blot (see, for example, Ausubel et al., (1988) Current Protocols in Molecular Biology, Wiley: New York), where a primer which is designed in such a way that it binds to the gene of interest is provided with a detectable marker (usually a radioactive or chemiluminescent marker) so that, when the total RNA of a culture of the organism is extracted, separated on a gel, applied to a stable matrix and incubated with this probe, the binding and quantity of the binding of the probe indicates the presence and also the amount of mRNA for this gene. Another method is a quantitative PCR. This information detects the extent to which the gene has been transcribed. Total cell RNA can be isolated for example from yeasts or E. coli by a variety of methods, which are known in the art, for example with the Ambion kit according to the instructions of the manufacturer or as described in Edgington et al., Promega Notes Magazine Number41, 1993, p. 14.

**[0443.0.0.7]** for the disclosure of this paragraph see [0443.0.0.0] above.

**[0444.0.7.7]** Example 6: Growth of genetically modified organism: media and culture conditions

**[0445.0.7.7]** Genetically modified Yeast, Mortierella or Escherichia coli are grown in synthetic or natural growth media known by the skilled worker. A number of different growth media for Yeast, Mortierella or Escherichia coli are well known and widely available. A method for clulturing Mortierella is disclosed by Jang et al. [Bot. Bull. Acad. Sin. (2000) 41: 41-48]. Mortierella can be grown at 20°C in a culture medium containing: 10 g/I glucose, 5 g/I yeast extract at pH 6.5. Futhermore Jang et al. teaches a submerged basal medium containing 20 g/I soluble starch, 5 g/I Bacto yeast extract, 10 g/l KNO₃, 1 g/l KH₂PO₄, and 0.5 g/l MgSO₄•7H₂O, pH 6.5.

**[0446.0.0.7] to [0453.0.0.7]** for the disclosure of the paragraphs [0446.0.0.7] to **[0453.0.0.7]** see paragraphs [0446.0.0.0] to [0453.0.0.0] above.

**[0454.0.7.7]** Example 8: Analysis of the effect of the nucleic acid molecule on the production of the fatty acid

**[0455.0.7.7]** The effect of the genetic modification in plants, fungi, algae or ciliates on the production of a desired compound (such as a fatty acid) can be determined by growing the modified microorganisms or the modified plant under suitable conditions (such as those described above) and analyzing the medium and/or the cellular components for the elevated production of desired product (i.e. of the lipids or a fatty acid). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).

In addition to the abovementioned processes, plant lipids are extracted from plant material as described by Cahoon et al. (1999) Proc. Natl. Acad. Sci. USA 96 (22): 12935-12940 and Browse et al. (1986) Analytic Biochemistry 152:141-145. The qualitative and quantitative analysis of lipids or fatty acids is described by Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 pp. (Oily Press Lipid Library; 1); "Progress in Lipid Research, Oxford: Pergamon Press, 1 (1952) - 16 (1977) under the title: Progress in the Chemistry of Fats and Other Lipids CODEN.

**[0456.0.0.7]** for the disclosure of this paragraph see [0456.0.0.0] above.

**[0457.0.7.7]** Example 9: Purification of the fatty acid

**[0458.0.7.7]** One example is the analysis of fatty acids (abbreviations: FAME, fatty acid methyl ester; GC-MS, gas liquid chromatography/mass spectrometry; TAG, triacylglycerol; TLC, thin-layer chromatography).
The unambiguous detection for the presence of fatty acid products can be obtained by analyzing recombinant organisms using analytical standard methods: GC, GC-MS or TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353).
The total fatty acids produced in the organism for example in yeasts used in the inventive process can be analysed for example according to the following procedure: The material such as yeasts, E. coli or plants to be analyzed can be disrupted by sonication, grinding in a glass mill, liquid nitrogen and grinding or via other applicable methods. After disruption, the material must be centrifuged (1000 x g, 10 min., 4°C) and washed once with 100 mM NaHCO₃, pH 8.0 to remove residual medium and fatty acids. For preparation of the fatty acid methyl esters (FAMES) the sediment is resuspended in distilled water, heated for 10 minutes at 100°C, cooled on ice and recentrifuged, followed by extraction for one hour at 90°C in 0.5 M sulfuric acid in methanol with 2% dimethoxypropane, which leads to hydrolyzed oil and lipid compounds, which give transmethylated lipids.
The FAMES are then extracted twice with 2 ml petrolether, washed once with 100 mM NaHCO₃, pH 8.0 and once with distilled water and dried with Na₂SO₄. The organic solvent can be evaporated under a stream of Argon and the FAMES were dissolved in 50 µl of petrolether. The samples can be separated on a ZEBRON ZB-Wax capillary column (30 m, 0,32 mm, 0,25 µm; Phenomenex) in a Hewlett Packard 6850 gas chromatograph with a flame ionisation detector. The oven temperature is programmed from 70°C (1 min. hold) to 200°C at a rate of 20°C/min., then to 250°C (5 min. hold) at a rate of 5°C/min and finally to 260°C at a rate of 5°C/min. Nitrogen is used as carrier gas (4,5 ml/min. at 70°C). The identity of the resulting fatty acid methyl esters can be identified by comparison with retention times of FAME standards, which are available from commercial sources (i.e. Sigma).

Plant material is initially homogenized mechanically by comminuting in a pestle and mortar to make it more amenable to extraction.

This is followed by heating at 100°C for 10 minutes and, after cooling on ice, by resedimentation. The cell sediment is hydrolyzed for one hour at 90°C with 1 M methanolic sulfuric acid and 2% dimethoxypropane, and the lipids are transmethylated. The resulting fatty acid methyl esters (FAMEs) are extracted in petroleum ether. The extracted FAMEs are analyzed by gas liquid chromatography using a capillary column (Chrompack, WCOT Fused Silica, CP-Wax-52 CB, 25 m, 0.32 mm) and a temperature gradient of from 170°C to 240°C in 20 minutes and 5 minutes at 240°C. The identity of the fatty acid methyl esters is confirmed by comparison with corresponding FAME standards (Sigma). The identity and position of the double bond can be analyzed further by suitable chemical derivatization of the FAME mixtures, for example to give 4,4-dimethoxyoxazoline derivatives (Christie, 1998) by means of GC-MS.

The methodology is described for example in Napier and Michaelson, 2001, Lipids. 36(8):761-766; Sayanova et al., 2001, Journal of Experimental Botany. 52(360):1581-1585, Sperling et al., 2001, Arch. Biochem. Biophys. 388(2):293-298 and Michaelson et al., 1998, FEBS Letters. 439(3):215-218.

**[0459.0.7.7]** If required and desired, further chromatography steps with a suitable resin may follow. Advantageously the fatty acids can be further purified with a so-called RTHPLC. As eluent different an acetonitrile/water or chloroform/acetonitrile mixtures are advantageously is used. For example canola oil can be separated said HPLC method using an RP-18-column (ET 250/3 Nucleosil 120-5 C₁₈ Macherey und Nagel, Düren, Germany). A chloroform/acetonitrile mixture (v/v 30:70) is used as eluent. The flow rate is beneficial 0.8 ml/min. For the analysis of the fatty acids an ELSD detector (evaporative light-scattering detector) is used. MPLC, dry-flash chromatography or thin layer chromatography are other beneficial chromatography methods for the purification of fatty acids. If necessary, these chromatography steps may be repeated, using identical or other chromatography resins. The skilled worker is familiar with the selection of suitable chromatography resin and the most effective use for a particular molecule to be purified.

**[0460.0.7.7]** In addition depending on the produced fine chemical purification is also possible with cristalisation or destilation. Both methods are well known to a person skilled in the art.

**[0461.0.7.7]** Example 10: Cloning SEQ ID NO: 227 for the expression in plants

**[0462.0.0.7]** for the disclosure of this paragraph see [0462.0.0.0] above.

**[0463.0.7.7]** SEQ ID NO: 227 is amplified by PCR as described in the protocol of the Pfu Turbo or DNA Herculase polymerase (Stratagene).

**[0464.0.7.7]** The composition for the protocol of the *Pfu* Turbo DNA polymerase was as follows: 1x PCR buffer (Stratagene), 0.2 mM of each dNTP, 100 ng genomic DNA of *Saccharomyces cerevisiae* (strain S288C; Research Genetics, Inc., now Invitrogen) or *Escherichia coli* (strain MG1655; *E.coli* Genetic Stock Center), 50 pmol forward primer, 50 pmol reverse primer, 2.5 u *Pfu* Turbo DNA polymerase. The amplification cycles were as follows:

**[0465.0.7.7]** 1 cycle of 3 minutes at 94-95°C, followed by 25-36 cycles of in each case 1 minute at 95°C or 30 seconds at 94°C, 45 seconds at 50°C, 30 seconds at 50°C or 30 seconds at 55°C and 210-480 seconds at 72°C, followed by 1 cycle of 8 minutes at 72°C, then 4°C.

**[0466.0.0.7]** for the disclosure of the paragraphs [0466.0.0.7] see paragraphs [0466.0.0.0] above.

**[0467.0.7.7]** The following primer sequences were selected for the gene SEQ ID NO: 227:
i) forward primer SEQ ID NO: 229
ii) reverse primer SEQ ID NO: 230

**[0468.0.0.7] to [0479.0.0.7]** for the disclosure of the paragraphs [0468.0.0.7] to [0479.0.0.7] see paragraphs [0468.0.0.0] to [0479.0.0.0] above.

**[0480.0.7.7]** Example 11: Generation of transgenic plants which express SEQ ID NO: 227

**[0481.0.0.7]** for the disclosure of this paragraph see [0481.0.0.0] above.

**[0482.0.0.7] to [0513.0.0.7]** for the disclosure of the paragraphs [0482.0.0.7] to [0513.0.0.7] see paragraphs [0482.0.0.0] to [0513.0.0.0] above.

**[0514.0.7.7]** As an alternative, the fatty acids can be detected and purfied advantageously as described in I. Brondz, "Development of fatty acid analysis by high-performance liquid chromatography, gas chromatography, and related techniques [Review]", Analytica Chimica Acta 2002, 465, 1-37

**[0515.0.5.7] ./.**

**[0516.0.0.7] and [0517.0.7.7]** for the disclosure of the paragraphs [0516.0.0.7] and [0517.0.7.7] see paragraphs [0516.0.0.0] and [0517.0.0.0] above.

**[0518.0.0.7] to [0529.0.0.7] and [0530.0.7.7]** for the disclosure of the paragraphs [0518.0.0.7] to [0529.0.0.7] and [0530.0.7.7] see paragraphs [0518.0.0.0] to [0530.0.0.0] above.

**[0530.1.0.7] to [0530.6.0.7]** for the disclosure of the paragraphs [0530.1.0.7] to [0530.6.0.7] see paragraphs [0530.1.0.0] to [0530.6.0.0] above.

**[0531.0.0.7] to [0533.0.0.7] and [0534.0.5.7]** for the disclosure of the paragraphs [0531.0.0.7] to [0533.0.0.7] and [0534.0.5.7] see paragraphs [0531.0.0.0] to [0534.0.0.0] above.

**[0535.0.0.7] to [0537.0.0.7] and [0538.0.7.7]** for the disclosure of the paragraphs [0535.0.0.7] to [0537.0.0.7] and [0538.0.7.7] see paragraphs [0535.0.0.0] to [0538.0.0.0] above.

**[0539.0.0.7] to [0542.0.0.7] and [0543.0.7.7]** for the disclosure of the paragraphs [0539.0.0.7] to [0542.0.0.7] and [0543.0.7.7] see paragraphs [0539.0.0.0] to [0543.0.0.0] above.

**[0544.0.0.7] to [0547.0.0.7] and [0548.0.7.7] to [0552.0.0.7]** for the disclosure of the paragraphs [0544.0.0.7] to [0547.0.0.7] and [0548.0.7.7] to [0552.0.0.7] see paragraphs [0544.0.0.0] to [0552.0.0.0] above.

**[0553.0.7.7]** A process for the production of stearic acid, which comprises
a) increasing or generating the activity of a protein as indicated in Table II, application no. 7, columns 5 or 7, or a functional equivalent thereof in a non-human organism, or in one or more parts thereof; and
b) growing the organism under conditions which permit the production of stearic acid in said organism.
A process for the production of stearic acid, comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 7, columns 5 or 7, or a fragment thereof, which confers an increase in the amount of stearic acid in an organism or a part thereof;
b) nucleic acid molecule comprising of a nucleic acid molecule as indicated in Table I, application no. 7, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of stearic acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of stearic acid in an organism or a part thereof;
e) nucleic acid molecule which hybidizes with a nucleic acid molecule of (a) to (c) under stringent hybridisation conditions and conferring an increase in the amount of stearic acid in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 7, column 7, and conferring an increase in the amount of stearic acid in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of stearic acid in an organism or a part thereof;
h) nucleic acid molecule encoding a polypeptide comprising a consensus sequence as indicated in Table IV, application no. 7, column 7, and conferring an increase in the amount of stearic acid in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of stearic acid in an organism or a part thereof.
or comprising a sequence which is complementary thereto.
The process as mentioned above, comprising recovering of the free or bound stearic acid.
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound stearic acid produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 7, columns 5 or 7, or a fragment thereof, which confers an increase in the amount of stearic acid in an organism or a part thereof;
b) nucleic acid molecule comprising of a nucleic acid molecule as indicated in Table I, application no. 7, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of stearic acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of stearic acid in an organism or a part thereof;
e) nucleic acid molecule which hybidizes with a nucleic acid molecule of (a) to (c) under stringent hybridisation conditions and conferring an increase in the amount of stearic acid in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 7, column 7, and conferring an increase in the amount of stearic acid in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of stearic acid in an organism or a part thereof;
h) nucleic acid molecule encoding a polypeptide comprising a consensus sequence as indicated in Table IV, application no. 7, column 7, and conferring an increase in the amount of stearic acid in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of stearic acid in an organism or a part thereof. whereby the nucleic acid molecule distinguishes over the sequence as indicated in Table I, application no. 7, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 7, columns 5 or 7by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of stearic acid in an organism or a part thereof comprising:
(a) contacting cells, tissues , plants or microorganisms which express the a polypeptide encoded by the nucleic acid molecule as mentioned above conferring an increase in the amount of stearic acid in an organism or a part thereof with a candidate compound or a sample comprising a plurality of compounds under conditions which permit the expression the polypeptide;
(b) assaying the stearic acid level or the polypeptide expression level in the cell, tissue, plant or microorganism or the media the cell, tissue, plant or microorganisms is cultured or maintained in; and
(c) identifying a agonist or antagonist by comparing the measured stearic acid level or polypeptide expression level with a standard of stearic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased stearic acid production in a plant or microorganism, comprising the steps:
(a) culturing a plant cell or tissue or microorganism or maintaining a plant expressing the polypeptide encoded by the nucleic acid molecule as mentioned above conferring an increase in the amount of stearic acid in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of stearic acid in an organism or a part thereof;
(b) identifying if the compound is an effective agonist by detecting the presence or absence or increase of a signal produced by said readout system.
A method for the identification of a gene product conferring an increase in stearic acid production in a cell, comprising the following steps:
a) contacting the nucleic acid molecules of a sample, which can contain a candidate gene encoding a gene product conferring an increase in stearic acid after expression with the nucleic acid molecule as mentioned above;
b) identifying the nucleic acid molecules, which hybridise under relaxed stringent conditions with the nucleic acid molecule as mentioned above;
c) introducing the candidate nucleic acid molecules in host cells appropriate for producing stearic acid;
d) expressing the identified nucleic acid molecules in the host cells;
e) assaying the stearic acid level in the host cells; and
f)identifying nucleic acid molecule and its gene product which expression confers an increase in the stearic acid level in the host cell in the host cell after expression compared to the wild type.
A method for the identification of a gene product conferring an increase in stearic acid production in a cell, comprising the following steps:
(a) identifiying in a data bank nucleic acid molecules of an organism; which can contain a candidate gene encoding a gene product conferring an increase in the stearic acid amount or level in an organism or a part thereof after expression, and which are at least 20% homolog to the nucleic acid molecule as mentioned above;
(b) introducing the candidate nucleic acid molecules in host cells appropriate for producing stearic acid;
(c) expressing the identified nucleic acid molecules in the host cells;
(d) assaying the stearic acid level in the host cells; and
(e) identifying nucleic acid molecule and its gene product which expression confers an increase in the stearic acid level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of stearic acid after expression.
Use of the polypeptide as mentioned above or the nucleic acid construct as mentioned above or the gene product identified according to the method as mentioned above for identifying compounds capable of conferring a modulation of stearic acid levels in an organism.
Food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the host cell as mentioned above or the gene product identified according to the method as mentioned above for the protection of a plant against a stearic acid synthesis inhibiting herbicide. New genes for a process for the production of fine chemicals

**[0000.0.0.8]** In a further embodiment, the present invention relates to a further process for the production of fine chemicals as defined below and the corresponding embodiments as described herein as follows.

**[0001.0.0.8] and [0002.0.7.8]** for the disclosure of the paragraphs [0001.0.0.8] and [0002.0.7.8] see paragraphs [0001.0.0.0] and [0002.0.7.7] above.

**[0003.0.8.8]** Palmitic acid is a major component for manufacturing of soaps, lubricating oils and waterproofing materials. Furhermore it is used for the synthesis of metallic palmitates. Additional applications are as food additive and in the synthesis of food-grade additives; as a constituent of cosmetic formulations. Palmitic acid is a major component of many natural fats and oils in the form of a glyceryl ester, e.g. palm oil, and in most commercial- grade stearic acid products.

**[0004.0.8.8] and [0005.0.5.8]** for the disclosure of the paragraphs [0004.0.7.8] and [0005.0.5.8] see paragraphs [0004.0.7.7] and [0005.0.7.7] above.

**[0006.0.8.8]** Palmitic acid is as mentioned above the major fat in meat and dairy products.

**[0007.0.8.8]** Further uses or palmitic acid are as food ingredients raw material for emulsifiers or personal care emulsifier for facial creams and lotions. Palmitic acid is also used in shaving cream formulations, waxes or fruit wax formulations.

**[0008.0.8.8]** Palmitic acid is also used in shaving cream formulations, waxes or fruit wax formulations.

**[0009.0.7.8] to [0012.0.7.8]** for the disclosure of the paragraphs [0009.0.7.8] to [0012.0.7.8] see paragraphs [0009.0.7.7] and [0012.0.7.7] above.

**[0013.0.0.8]** for the disclosure of this paragraph see [0013.0.0.0] above.

**[0014.0.8.8]** Accordingly, in a first embodiment, the invention relates to a process for the production of a fine chemical, whereby the fine chemical is palmitic acid or tryglycerides, lipids, oils or fats containing palmitic acid. Accordingly, in the present invention, the term "the fine chemical" as used herein relates to "palmitic acid and/or tryglycerides, lipids, oils and/or fats containing palmitic acid". Further, the term "the fine chemicals" as used herein also relates to fine chemicals comprising palmitic acid and/or triglycerides, lipids, oils and/or fats containing palmitic acid.

**[0015.0.8.8]** In one embodiment, the term "the fine chemical" means palmitic acid and/or tryglycerides, lipids, oils and/or fats containing palmitic acid. Throughout the specification the term "the fine chemical" means palmitic acid and/or tryglycerides, lipids, oils and/or fats containing palmitic acid, palmitic acid and its salts, ester, thioester or palmitic acid in free form or bound to other compounds such as triglycerides, glycolipids, phospholipids etc. In a preferred embodiment, the term "the fine chemical" means palmitic acid, in free form or its salts or bound to triglycerides. Triglycerides, lipids, oils, fats or lipid mixture thereof shall mean any triglyceride, lipid, oil and/or fat containing any bound or free palmitic acid for example sphingolipids, phosphoglycerides, lipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride or other fatty acid esters such as acetyl-Coenzym A thioester, which contain further saturated or unsaturated fatty acids in the fatty acid molecule.
In one embodiment, the term "the fine chemical" and the term "the respective fine chemical" mean at least one chemical compound with an activity of the above mentioned fine chemical.

**[0016.0.8.8]** Accordingly, the present invention relates to a process for the production of the respective fine chemical comprising
(a) increasing or generating the activity of one or more of a protein as shown in table II, application no. 8, column 3 encoded by the nucleic acid sequences as shown in table I, application no. 8, column 5, in a non-human organism or in one or more parts thereof or
(b) growing the organism under conditions which permit the production of the fine chemical, thus fatty acid of the invention or fine chemicals comprising the fatty acid of the invention, in said organism or in the culture medium surrounding the organism.

**[0017.0.0.8] to [0019.0.0.8]** for the disclosure of the paragraphs [0017.0.0.8] to [0019.0.0.8] see paragraphs [0017.0.0.0] and [0019.0.0.0] above.

**[0020.0.8.8]** Surprisingly it was found, that the transgenic expression of the Brassica napus protein as indicated in Table II, application no. 8, column 5, line 18 in a plant conferred an increase in Palmitic Acid C16:0 content of the transformed plants. Thus, in one embodiment, said protein or its homologs are used for the production of Palmitic Acid C16 : 0.

**[0021.0.0.8]** for the disclosure of this paragraph see [0021.0.0.0] above.

**[0022.0.8.8]** ./.The sequence of YDR513W from Saccharomyces cerevisiae has been published in Jacq et al., Nature 387 (6632 Suppl), 75-78 (1997) and in Goffeau et al., Science 274 (5287), 546-547, 1996 and its cellular activity has characterized as glutaredoxin (thioltransferase, glutathione reductase). Accordingly, in one embodiment, the process of the present invention comprises the use of a glutaredoxin, from Saccharomyces cerevisiae or a plant or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a glutaredoxin is increased or generated, e.g. from Saccharomyces cerevisiae or a plant or a homolog thereof.

**[0022.1.0.8] to [0023.0.0.8]** for the disclosure of this paragraphs see [0022.1.0.0] to [0023.0.0.0] above.

**[0023.1.0.8]** Homologs of the polypeptide disclosed in table II, application no. 8, column 5, may have the activity of a protein as disclosed in table I or II, application no. 8, column 3 and may be the polypetides encoded by the nucleic acid molecules indicated in table I , application no. 8, column 7, resp., or may be the polypeptides indicated in table II, application no. 8, column 7, resp.

**[0024.0.0.8]** for the disclosure of the paragraphs [0024.0.0.8] see [0024.0.0.0] above.

**[0025.0.8.8]** In accordance with the invention, a protein or polypeptide has the "activity of an protein as shown in table II, application no. 8, column 3 "if its de *novo* activity, or its increased expression directly or indirectly leads to an increased in the level of the fine chemical indicated in the respective line of table II, application no. 8, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 8, column 3, or which has at least 10% of the original enzymatic or biological activity, preferably 20%, particularly preferably 30%, most particularly preferably 40% in comparison to a protein as shown in the respective line of table II, application no. 8, column 3 of a E. coli or Saccharomyces cerevisae, respectively, protein as mentioned in table I to IV, column 3 respectively and as disclosed in paragraph [0022] of the respective invention.

**[0025.1.0.8] and [0025.2.0.8]** for the disclosure of the paragraphs [0025.1.0.8] and [0025.2.0.8] see paragraphs [0025.1.0.0] and [0025.2.0.0] above.

**[0026.0.0.8] to [0033.0.0.8]** for the disclosure of the paragraphs [0026.0.0.8] to [0033.0.0.8] see paragraphs [0026.0.0.0] to [0033.0.0.0] above.

**[0034.0.8.8]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 8, column 3 its biochemical or genetical causes and the increased amount of the respective fine chemical.

**[0035.0.0.8] to [0038.0.0.8] and [0039.0.5.8]** for the disclosure of the paragraphs [0035.0.0.8] to [0038.0.0.8] and [0039.0.5.8] see paragraphs [0035.0.0.0] to [0039.0.0.0] above.

**[0040.0.0.8] to [0044.0.0.8]** for the disclosure of the paragraphs [0040.0.0.8] to [0044.0.0.8] see paragraphs [0035.0.0.0] and [0044.0.0.0] above.

**[0045.0.8.8]** In one embodiment, the activity of the protein. as indicated in Table II, columns 5 or 7, application no. 8, is increased conferring an increase of the respective fine chemical, indicated in Table II, application no. 8, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more **[0046.0.8.8]** In one embodiment, the activity of the protein as indicated in Table II, columns 5 or 7, application no. 8, is increased conferring an increase of the respective fine chemical, indicated in Table II, application no. 8, column 6 of the respective line confers an increase of the respective fine chemical and of further fatty acid or their precursors.

**[0047.0.0.8] to [0048.0.0.8]** for the disclosure of the paragraphs [0047.0.0.8] and [0048.0.0.8] see paragraphs [0047.0.0.0] and [0048.0.0.0] above.

**[0049.0.8.8]** A protein having an activity conferring an increase in the amount or level of the fine chemical, has in one embodiment the structure of the polypeptide described herein, in particular of the polypeptides comprising the consensus sequence shown in table IV, application no. 8, column 7 or of the polypeptide as shown in the amino acid sequences as disclosed in table II, application no. 8, columns 5 and 7 or the functional homologues thereof as described herein, or is encoded by the nucleic acid molecule characterized herein or the nucleic acid molecule according to the invention, for example by the nucleic acid molecule as shown in table I, application no. 8, columns 5 and 7 or its herein described functional homologues and has the herein mentioned activity.

**[0050.0.8.8]** For the purposes of the present invention, the term "palmitic acid" also encompasses the corresponding salts, such as, for example, the potassium or sodium salts of palmitic acid or the salts of palmitic acid with amines such as diethylamine.

**[0051.0.5.8] and [0052.0.0.8]** for the disclosure of the paragraphs [0051.0.5.8] and [0052.0.0.8] see paragraphs [0051.0.0.0] and [0052.0.0.0] above.

**[0053.0.8.8]** In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 8, columns 5 and 7 or its homologs activity having herein-mentioned fatty acid of the invention increasing activity; and/or
b) stabilizing a mRNA conferring the increased expression of a protein encoded by the nucleic acid molecule of the invention as shown in table I, application no. 8, columns 5 and 7, e.g. a nucleic acid sequence encoding a polypeptide having the activity of a protein as indicated in table II, application no. 8, columns 5 and 7 or its homologs activity or of a mRNA encoding the polypeptide of the present invention having herein-mentioned fatty acid of the invention increasing activity; and/or
c) increasing the specific activity of a protein conferring the increased expression of a protein encoded by the nucleic acid molecule of the invention or of the polypeptide of the present invention having herein-mentioned fatty acid increasing activity, e.g. of a polypeptide having the activity of a protein as indicated in table II, application no. 8, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned fatty acid of the invention increasing activity, e.g. of a polypeptide having the activity of a protein as indicated in table II, application no. 8, columns 5 and 7 or its homologs activity; and/or
e) stimulating activity of a protein conferring the increased expression of a protein encoded by the nucleic acid molecule of the present invention or a polypeptide of the present invention having herein-mentioned fatty acid of the invention increasing activity, e.g. of a polypeptide having the activity of a protein as indicated in table II, application no. 8, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned fatty acid of the invention increasing activity, e.g. of a polypeptide having the activity of a protein as indicated in table II, application no. 8, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned fatty acid of the invention increasing activity, e.g. of a polypeptide having the activity of a protein as indicated in table II, application no. 8, columns 5 and 7 or its homologs activity; and/or
h) increasing the expression of the endogenous gene encoding the polypeptide of the invention, e.g. a polypeptide having the activity of a protein as indicated in table II, application no. 8, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with expecially high activity of the proteins of the invention from natural or from mutagenized resources and breeding them into the target organisms, eg the elite crops.

**[0054.0.8.8]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of palmitic acid after increasing the expression or activity of the encoded polypeptide or having the activity of a polypeptide having an activity of a protein as indicated in Table II, application no. 8, column 3, or its homolog's activity, e.g. as indicated in Table II, application no. 8, columns 5 or 7. In general, the amount of mRNA or polypeptide in a cell or a compartment of a organism correlates with the amount of encoded protein and thus with the overall activity of the encoded protein in said volume. Said correlation is not always linear, the activity in the volume is dependent on the stability of the molecules or the presence of activating or inhibiting co-factors. Further, product and educt inhibitions of enzymes are well known and described in Textbooks, e.g. Stryer, Biochemistry.

**[0055.0.0.8] to [0067.0.0.8]** for the disclosure of the paragraphs [0055.0.0.8] to [0067.0.0.8] see paragraphs [0055.0.0.0] to [0067.0.0.0] above.

**[0068.0.5.8] and [0069.0.5.8]** for the disclosure of the paragraphs [0068.0.5.8] and [0069.0.5.8] see paragraphs [0068.0.0.0] and [0069.0.0.0] above.

**[0070.0.6.8] and [0071.0.5.8]** for the disclosure of the paragraphs [0070.0.6.8] and [0071.0.5.8] see paragraphs [0070.0.7.7] and [0071.0.0.0] above.

**[0072.0.8.8]** By influencing the metabolism thus, it is possible to produce, in the process according to the invention, further advantageous compounds. Examples of such compounds are, in addition to palmitic acid, triglycerides, lipids, oils and/or fats containing palmitic acid compounds such as palmitate, palmitoleate, stearate, oleate, α-linolenic acid and/or linoleic acid.

**[0073.0.8.8]** Accordingly, in one embodiment, the process according to the invention relates to a process, which comprises:
a) providing a non-human organism, preferably a microorganism, a non-human animal, a plant or animal cell, a plant or animal tissue or a plant;
b) increasing the activity of a protein having the activity of a polypeptide of the invention or the polypeptide used in the method of the invention or a homolog thereof, e.g. as shown in Table II, application no. 8, columns 5 or 7, or of a polypeptide being encoded by the nucleic acid molecule of the present invention and described below, i.e. conferring an increase of the respective fine chemical in the organism, preferably a microorganism, the a non-human animal, a plant or animal cell, a plant or animal tissue or the plant,
c) growing the organism, preferably a microorganism, a non-human animal, a plant or animal cell, a plant or animal tissue or the plant under conditions which permit the production of the fine chemical in the organism, preferably a microorganism, a plant cell, a plant tissue or the plant; and
d) if desired, recovering, optionally isolating, the free and/or bound the respective fine chemical and, optionally further free and/or bound fatty acids synthetized by the organism, the microorganism, the non-human animal, the plant or animal cell, the plant or animal tissue or the plant.

**[0074.0.5.8]** for the disclosure of this paragraph see [0074.0.7.7] above.

**[0075.0.0.8] to [0084.0.0.8]** for the disclosure of the paragraphs [0075.0.0.8] to [0084.0.0.8] see paragraphs [0075.0.0.0] to [0084.0.0.0] above.

**[0085.0.8.8]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as shown in table I, application no. 8, columns 5 and 7 or a derivative thereof, or
b) a genetic regulatory element, for example a promoter, which is functionally linked to the nucleic acid sequence as shown table I, application no. 8, columns 5 and 7 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.8] and [0087.0.0.8]** for the disclosure of the paragraphs [0086.0.0.8] and [0087.0.0.8] see paragraphs [0086.0.0.0] and [0087.0.0.0] above.

**[0088.0.8.8]** In an advantageous embodiment of the invention, the organism takes the form of a plant whose fatty acid content is modified advantageously owing to the nucleic acid molecule of the present invention expressed. This is important for plant breeders since, for example, the nutritional value of plants for poultry is dependent on the abovementioned essential fatty acids and the general amount of fatty acids as energy source in feed. After the activity of a protein as shown in Table II, application no. 8, columns 5 or 7, has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.8], [0089.0.0.8], [0090.0.0.8] and [0091.0.5.8]** for the disclosure of the paragraphs [0088.1.0.8], [0089.0.0.8], [0090.0.0.8] and [0091.0.5.8] see paragraphs [0088.1.0.0], [0089.0.0.0], [0090.0.0.0] and [0091.0.0.0] above.

**[0092.0.0.8] to [0094.0.0.8]** for the disclosure of the paragraphs [0092.0.0.8] to [0094.0.0.8] see paragraphs [0092.0.0.0] to [0094.0.0.0] above.

**[0095.0.5.8], [0096.0.5.8] and [0097.0.5.8]** for the disclosure of the paragraphs [0095.0.5.8], [0096.0.5.8] and [0097.0.5.8] see paragraphs [0095.0.7.7], [0096.0.7.7] and [0097.0.0.0] above.

**[0098.0.8.8]** In a preferred embodiment, the fine chemical (palmitic acid) is produced in accordance with the invention and, if desired, is isolated. The production of further fatty acids such as stearic acid, palmitoleic acid, oleic acid, α-linolenic acid and/or linoleic acid mixtures thereof or mixtures of other fatty acids by the process according to the invention is advantageous.

**[0099.0.5.8] and [0100.0.5.8]** for the disclosure of the paragraphs [0099.0.5.8] and [0100.0.5.8] see paragraphs [0099.0.7.7] and [0100.0.7.7] above.

**[0101.0.5.8] and [0102.0.5.8]** for the disclosure of the paragraphs [0101.0.5.8] and [0102.0.5.8] see [0101.0.0.0] and [0102.0.7.7] above.

**[0103.0.8.8]** In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of a polypeptide having a sequence as shown in Table II, application no. 8, columns 5 or 7, in or a fragment thereof, which confers an increase in the amount of the respective fine chemical in an organism or a part thereof;
b) nucleic acid molecule comprising, preferably at least the mature form, of the nucleic acid molecule having a sequence as shown in Table I, application no. 8, columns 5 or 7,
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as shown in Table III, application no. 8, column 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequence as shown in Table IV, application no. 8, column 7, and conferring an increase in the amount of the respective fine chemical in an organism or a part thereof ;
k) nucleic acid molecule comprising one or more of the nucleic acid molecule encoding the amino acid sequence of a polypeptide encoding a domain of a polypeptide as shown in Table II, application no. 8, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[0103.1.0.8] and [0103.2.0.8]** for the disclosure of the paragraphs [0103.1.0.8] and [0103.2.0.8] see paragraphs [0103.1.0.0] and [0103.2.0.0] above.

**[0104.0.8.8]** In one embodiment, the nucleic acid molecule used in the process distinguishes over the sequence shown in Table I, application no. 8, columns 5 or 7, by one or more nucleotides or does not consist of the sequence shown in Table I, application no. 8, columns 5 or 7, . In one embodiment, the nucleic acid molecule of the present invention is less than 100%, 99,999%, 99,99%, 99,9% or 99% identical to the sequence shown in Table I, application no. 8, columns 5 or 7,. In another embodiment, the nucleic acid molecule does not encode a polypeptide of the sequence shown in Table II, application no. 8, columns 5 or 7.

**[0105.0.0.8] to [0107.0.0.8]** for the disclosure of the paragraphs [0105.0.0.8] to [0107.0.0.8] see paragraphs [0105.0.0.0] and [0107.0.0.0] above.

**[0108.0.8.8]** Nucleic acid molecules with the sequence shown in Table I, application no. 8, columns 5 or 7, nucleic acid molecules which are derived from the amino acid sequences shown in Table II, application no. 8, columns 5 or 7, or from polypeptides comprising the consensus sequence shown in Table IV, application no. 8, column 7, or their derivatives or homologues encoding polypeptides with the enzymatic or biological activity of a protein as shown in Table II, application no. 8, columns 5 or 7, or e.g. conferring a linoleic acid increase after increasing its expression or activity are advantageously increased in the process according to the invention.

**[0109.0.5.8]** for the disclosure of this paragraph see [0109.0.0.0] above.

**[0110.0.8.8]** Nucleic acid molecules, which are advantageous for the process according to the invention and which encode polypeptides with an activity of a polypeptide used in the method of the invention or used in the process of the invention, e.g. of a protein as shown in Table II, application no. 8, columns 5 or 7 or being encoded by a nucleic acid molecule indicated in Table I, application no. 8, columns 5 or 7, or of its homologs, e.g. as indicated in Table II, application no. 8, columns 5 or 7, can be determined from generally accessible databases.

**[0111.0.0.8]** for the disclosure of this paragraph see [0111.0.0.0] above.

**[0112.0.8.8]** The nucleic acid molecules used in the process according to the invention take the form of isolated nucleic acid sequences, which encode polypeptides with an activity of a polypeptide as indicated in Table II, application no. 8, column3, or having the sequence of a polypeptide as indicated in Table II, application no. 8, columns 5 and 7, and conferring an increase of the respective fine chemical.

**[0113.0.0.8] to [0120.0.0.8]** for the disclosure of the paragraphs [0113.0.0.8] to [0120.0.0.8] see paragraphs [0113.0.0.0] and [0120.0.0.0] above.

**[0121.0.8.8]** However, it is also possible to use artificial sequences, which differ in one or more bases from the nucleic acid sequences found in organisms, or in one or more amino acid molecules from polypeptide sequences found in organisms, in particular from the polypeptide sequences shown in Table II, application no. 8, columns 5 or 7, or the functional homologues thereof as described herein, preferably conferring above-mentioned activity, i.e. conferring an increase of the respective fine chemical after increasing its activity.

**[0122.0.0.8] to [0127.0.0.8]** for the disclosure of the paragraphs [0122.0.0.8] to [0127.0.0.8] see paragraphs [0122.0.0.0] and [0127.0.0.0] above.

**[0128.0.8.8]** Synthetic oligonucleotide primers for the amplification, e.g. as shown in Table III, application no. 8, column 7, , by means of polymerase chain reaction can be generated on the basis of a sequence shown herein, for example the sequence shown in Table I, application no. 8, columns 5 or 7, or the sequences as shown in Table II, application no. 8, columns 5 or 7.

**[0129.0.8.8]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequences shown in Table IV, application no. 8, column 7, are derived from said aligments.

**[0130.0.8.8]** for the disclosure of this paragraph see [0130.0.0.0].

**[0131.0.0.8] to [0138.0.0.8]** for the disclosure of the paragraphs [0131.0.0.8] to [0138.0.0.8] see paragraphs [0131.0.0.0] to [0138.0.0.0] above.

**[0139.0.8.8]** Polypeptides having above-mentioned activity, i.e. conferring the respective fine chemical increase, derived from other organisms, can be encoded by other DNA sequences which hybridize to the sequences shown in Table I, application no. 8, columns 5 or 7, under relaxed hybridization conditions and which code on expression for peptides having the palmitic acid increasing activity.

**[0140.0.0.8] to [0146.0.0.8]** for the disclosure of the paragraphs [0140.0.0.8] to [0146.0.0.8] see paragraphs [0140.0.0.0] and [0146.0.0.0] above.

**[0147.0.8.8]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 8, columns 5 or 7, is one which is sufficiently complementary to one of said nucleotide sequences such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.8.8]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 8, columns 5 or 7, or a functional portion thereof and preferably has above mentioned activity, in particular has the-fine-chemical-increasing activity after increasing its acitivity or an activity of a product of a gene encoding said sequence or its homologs.

**[0149.0.8.8]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 8, columns 5 or 7, or a portion thereof and encodes a protein having above-mentioned activity as indicated in Table II, application no. 8, columns 5 or 7, e.g. conferring an increase of the respective fine chemical.

**[00149.1.0.8]** Optionally, the nucleotide sequence, which hybridises to one of the nucleotide sequences indicated in Table I, application no. 8, columns 5 or 7, has further one or more of the activities annotated or known for the a protein as indicated in Table II, application no. 8, column 3.

**[0150.0.8.8]** Moreover, the nucleic acid molecule of the invention or used in the process of the invention can comprise only a portion of the coding region of one of the sequences indicated in Table I, application no. 8, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of methionine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, application no. 8, columns 5 or 7, an anti-sense sequence of one of the sequences indicated in Table I, application no. 8, columns 5 or 7, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 8, column 7, will result in a fragment of a polynucleotide sequence as indicated in Table I, application no. 8, columns 5 or 7. Preferred is Table I, application no. 8, column 7.

**[0151.0.0.8]** for the disclosure of this paragraph see [0151.0.0.0] above.

**[0152.0.8.8]** The nucleic acid molecule of the invention encodes a polypeptide or portion thereof which includes an amino acid sequence which is sufficiently homologous to the amino acid sequence shown in Table II, application no. 8, columns 5 or 7, such that the protein or portion thereof maintains the ability to participate in the fine chemical production, in particular a palmitic acid increasing the activity as mentioned above or as described in the examples in plants or microorganisms is comprised.

**[0153.0.8.8]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in Table II, application no. 8, columns 5 or 7, such that the protein or portion thereof is able to participate in the increase of the respective fine chemical production. In one embodiment, a protein or portion thereof as shown in Table II, application no. 8, columns 5 or 7, has for example an activity of a polypeptide as indicated in Table II, application no. 8, columns 5 or 7.

**[0154.0.8.8]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as shown in Table II, application no. 8, columns 5 or 7, and having above-mentioned activity, e.g.conferring preferably the increase of the respective fine chemical.

**[0155.0.0.8] and [0156.0.0.8]** for the disclosure of the paragraphs [0155.0.0.8] and [0156.0.0.8] see paragraphs [0155.0.0.0] and [0156.0.0.0] above.

**[0157.0.8.8]** The invention further relates to nucleic acid molecules that differ from one of the nucleotide sequences indicated in Table I, application no. 8, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, application no. 8, column 7, or of the polypeptide as indicated in Table II, application no. 8, columns 5 or 7, or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, application no. 8, column 7, or of the polypeptide as indicated in Table II, application no. 8, columns 5 or 7, or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, application no. 8, column 7, or of a polypeptide as indicated in Table II, application no. 8, columns 5 or 7, or the functional homologues thereof. However, in a preferred embodiment, the nucleic acid molecule of the present invention does not consist of a sequence as indicated in Table I, application no. 8, columns 5 or 7,

**[0158.0.0.8] to [0160.0.0.8]** for the disclosure of the paragraphs [0158.0.0.8] to [0160.0.0.8] see paragraphs [0158.0.0.0] to [0160.0.0.0] above.

**[0161.0.8.8]** Accordingly, in another embodiment, a nucleic acid molecule of the invention or the nucleic acid molecule used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising the sequence shown in Table I, application no. 8, columns 5 or 7. The nucleic acid molecule is preferably at least 20, 30, 50, 100, 250 or more nucleotides in length.

**[0162.0.0.8]** for the disclosure of this paragraph see paragraph [0162.0.0.0] above.

**[0163.0.8.8]** Preferably, nucleic acid molecule of the invention that hybridizes under stringent conditions to a sequence as indicated in Table I, application no. 8, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.8]** for the disclosure of this paragraph see paragraph [0164.0.0.0] above.

**[0165.0.8.8]** For example, nucleotide substitutions leading to amino acid substitutions at "non-essential" amino acid residues can be made in a sequence of the nucleic acid molecule of the invention or used in the process of the invention, e.g. as shown in Table I, application no. 8, columns 5 or 7.

**[0166.0.0.8] and [0167.0.0.8]** for the disclosure of the paragraphs [0166.0.0.8] and [0167.0.0.8] see paragraphs [0166.0.0.0] and [0167.0.0.0] above.

**[0168.0.8.8]** Accordingly, the invention relates to nucleic acid molecules encoding a polypeptide having above-mentioned activity, e.g. conferring an increase in the respective fine chemical in an organism or parts thereof that contain changes in amino acid residues that are not essential for said activity. Such polypeptides differ in amino acid sequence from a sequence contained in a sequence as indicated in Table II, application no. 8, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 8, columns 5 or 7, and is capable of participation in the increase of production of the respective fine chemical after increasing its activity, e.g. its expression. Preferably, the protein encoded by the nucleic acid molecule is at least about 60% identical to the sequence as indicated in Table II, application no. 8, columns 5 or 7, more preferably at least about 70% identical to one of the sequences as indicated in Table II, application no. 8, columns 5 or 7, even more preferably at least about 80%, 90%, 95% homologous to the sequence as indicated in Table II, application no. 8, columns 5 or 7and most preferably at least about 96%, 97%, 98%, or 99% identical to the sequence as indicated in Table II, application no. 8, columns 5 or 7.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 8, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 8, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 8, columns 5 or 7, more preferably at least about 70% identical to one of the sequences as indicated in Table II, application no. 8, columns 5 or 7, even more preferably at least about 80%, 90%, or 95% homologous to a sequence as indicated in Table II, application no. 8, columns 5 or 7, and most preferably at least about 96%, 97%, 98%, or 99% identical to the sequence as indicated in Table II, application no. 8, columns 5 or 7.

**[0169.0.0.8] to [0175.0.5.8]** for the disclosure of the paragraphs [0169.0.0.8] to [0175.0.5.8] see paragraphs [0169.0.0.0] to [0175.0.0.0] above.

**[0176.0.8.8]** Functional equivalents derived from one of the polypeptides as shown in Table II, application no. 8, columns 5 or 7according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, application no. 8, columns 5 or 7, according to the invention and are distinguished by essentially the same properties as the polypeptide as shown in Table II, application no. 8, columns 5 or 7.

**[0177.0.8.8]** Functional equivalents derived from the nucleic acid sequence as shown in Table I, application no. 8, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, application no. 8, columns 5 or 7, according to the invention and encode polypeptides having essentially the same properties as the polypeptide as shown in Table II, application no. 8, columns 5 or 7.

**[0178.0.0.8]** for the disclosure of this paragraph see [0178.0.0.0] above.

**[0179.0.8.8]** A nucleic acid molecule encoding a homologous protein to a protein sequence of as indicated in Table II, application no. 8, columns 5 or 7, can be created by introducing one or more nucleotide substitutions, additions or deletions into a nucleotide sequence of the nucleic acid molecule of the present invention, in particular as indicated in Table I, application no. 8, columns 5 or 7, such that one or more amino acid substitutions, additions or deletions are introduced into the encoded protein. Mutations can be introduced into the encoding sequences for example into a sequences as indicated in Table I, application no. 8, columns 5 or 7, by standard techniques, such as site-directed mutagenesis and PCR-mediated mutagenesis.

**[0180.0.0.8] to [0183.0.0.8]** for the disclosure of the paragraphs [0180.0.0.8] to [0183.0.0.8] see paragraphs [0180.0.0.0] to [0183.0.0.0] above.

**[0184.0.8.8]** Homologues of the nucleic acid sequences used, with a sequence as indicated in Table I, application no. 8, columns 5 or 7, or of the nucleic acid sequences derived from a sequences as indicated in Table II, application no. 8, columns 5 or 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 8, columns 5 or 7, or from the derived nucleic acid sequences, the intention being, however, that the enzyme activity or the biological activity of the resulting proteins synthesized is advantageously retained or increased.

**[0185.0.8.8]** In one embodiment of the present invention, the nucleic acid molecule of the invention or used in the process of the invention comprises one or more sequences as indicated in Table I, application no. 8, columns 5 or 7. In one embodiment, it is preferred that the nucleic acid molecule comprises as little as possible other nucleotide sequences not shown in any one of sequences as indicated in Table I, application no. 8, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 8, columns 5 or 7.

**[0186.0.8.8]** Also preferred is that one or more nucleic acid molecule(s) used in the process of the invention encode a polypeptide comprising a sequence as indicated in Table II, application no. 8, columns 5 or 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 8, columns 5 or 7.

**[0187.0.8.8]** In one embodiment, the nucleic acid molecule of the invention or used in the process encodes a polypeptide comprising a sequence as indicated in Table II, application no. 8, columns 5 or 7, and comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 8, columns 5 or 7.

**[0188.0.8.8]** Polypeptides (= proteins), which still have the essential biological activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide shown in Table II, application no. 8, columns 5 or 7, expressed under identical conditions.

**[0189.0.8.8]** Homologues of sequences as indicated in Table I, application no. 8, columns 5 or 7, or of the derived sequences shown in Table II, application no. 8, columns 5 or 7, also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.8], [0191.0.5.8], [00191.1.0.8] and [0192.0.0.8] to [0203.0.0.8]** for the disclosure of the paragraphs [0190.0.0.8], [0191.0.5.8], [0191.1.0.8] and [0192.0.0.8] to [0203.0.0.8] see paragraphs [0190.0.0.0], [0191.0.7.7], [0191.1.0.0] and [0192.0.0.0] to [0203.0.0.0] above.

**[0204.0.8.8]** Accordingly, in one embodiment, the invention relates to a nucleic acid molecule, which comprises a nucleic acid molecule selected from the group consisting of:
a) nucleic acid molecule encoding, preferably at least the mature form, of the polypeptide shown in Table II, application no. 8, columns 5 or 7; or a fragment thereof conferring an increase in the amount of the fine chemical in an organism or a part thereof
b) nucleic acid molecule comprising, preferably at least the mature form, of the nucleic acid molecule shown in Table I, application no. 8, columns 5 or 7; or a fragment thereof conferring an increase in the amount of the fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using the primers or primer pairs as indicated in Table III, application no. 8, column 7, and conferring an increase in the amount of the respective fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising the consensus sequence shown in Table IV, application no. 8, column 7, and conferring an increase in the amount of the respective fine chemical in an organism or a part thereof;
k) nucleic acid molecule encoding the amino acid sequence of a polypeptide encoding a domaine of the polypeptide shown in Table II, application no. 8, columns 5 or 7,; and conferring an increase in the amount of the respective fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of the nucleic acid molecule shown in Table I, application no. 8, columns 5 or 7, or a nucleic acid molecule encoding, preferably at least the mature form of, the polypeptide shown in Table II, application no. 8, columns 5 or 7, , and conferring an increase in the amount of the respective fine chemical in an organism or a part thereof; or which encompasses a sequence which is complementary thereto;
whereby, preferably, the nucleic acid molecule according to (a) to (I) distinguishes over the sequence as depicted in Table I, application no. 8, columns 5 or 7, by one or more nucleotides. In one embodiment, the nucleic acid molecule of the invention does not consist of the sequence shown in Table I, application no. 8, columns 5 or 7. In an other embodiment, the nucleic acid molecule of the present invention is at least 30 % identical and less than 100%, 99,999%, 99,99%, 99,9% or 99% identical to the sequence shown in Table I, application no. 8, columns 5 or 7. In a further embodiment the nucleic acid molecule does not encode the polypeptide sequence shown in Table II, application no. 8, columns 5 or 7. Accordingly, in one embodiment, the nucleic acid molecule of the present invention encodes in one embodiment a polypeptide which differs at least in one or more amino acids from the polypeptide as depicted in Table II, application no. 8, columns 5 or 7, and therefore does not encode a protein of the sequence shown in Table II, application no. 8, columns 5 or 7. Accordingly, in one embodiment, the protein encoded by a sequence of a nucleic acid according to (a) to (I) does not consist of the sequence shown in Table II, application no. 8, columns 5 or 7. In a further embodiment, the protein of the present invention is at least 30 % identical to protein sequence depicted in Table II, application no. 8, columns 5 or 7, and less than 100%, preferably less than 99,999%, 99,99% or 99,9%, more preferably less than 99%, 985, 97%, 96% or 95% identical to the sequence shown in Table II, application no. 8, columns 5 or 7.

**[0205.0.0.8] to [0226.0.0.8]** for the disclosure of the paragraphs [0205.0.0.8] to [0226.0.0.8] see paragraphs [0205.0.0.0] to [0226.0.0.0] above.

**[0227.0.8.8]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to the sequence mentioned in Table I, application no. 8, columns 5 or 7, or its derivatives, it is advantageous additionally to express and/or mutate further genes in the organisms. Especially advantageously, additionally at least one further gene of the fatty acid biosynthetic pathway such as for palmitate, palmitoleate, stearate and/or oleate is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine the sequences shown in Table I, application no. 8, columns 5 or 7, with genes which generally support or enhances to growth or yield of the target organismen, for example genes which lead to faster growth rate of microorganisms or genes which produces stress-, pathogen, or herbicide resistant plants.

**[0228.0.5.8] to [0230.0.5.8]** for the disclosure of the paragraphs [0228.0.5.8] to [0230.0.5.8] see paragraphs [0228.0.0.0] to [0230.0.0.0] above.

**[0231.0.8.8]** In a further advantageous embodiment of the process of the invention, the organisms used in the process are those in which simultaneously a palmitic acid degrading protein is attenuated, in particular by reducing the rate of expression of the corresponding gene.

**[0232.0.0.8] to [0276.0.0.8]** for the disclosure of the paragraphs [0232.0.0.8] to [0276.0.0.8] see paragraphs [0232.0.0.0] to [0276.0.0.0] above.

**[0277.0.5.8]** for the disclosure of this paragraph see paragraph [0277.0.7.7] above.

**[0278.0.0.8] to [0282.0.0.8]** for the disclosure of the paragraphs [0278.0.0.8] to [0282.0.0.8] see paragraphs [0278.0.0.0] to [0282.0.0.0] above.

**[0283.0.8.8]** Moreover, native polypeptide conferring the increase of the respective fine chemical in an organism or part thereof can be isolated from cells (e.g., endothelial cells), for example using the antibody of the present invention as described below, in particular, an antibody against polypeptides as shown in Table II, application no. 8, columns 5 or 7, or an antigenic part thereof, which can be produced by standard techniques utilizing polypeptides comprising or consisting of abovementioned sequences, e.g. the polypeptid of the present invention or fragment thereof. Preferred are monoclonal antibodies specifically binding to polypeptides as indicated in Table II, application no. 8, columns 5 or 7.

**[0284.0.0.8]** for the disclosure of this paragraph see [0284.0.0.0] above.

**[0285.0.8.8]** In one embodiment, the present invention relates to a polypeptide having the sequence shown in Table II, application no. 8, columns 5 or 7, or as coded by the nucleic acid molecule shown in Table I, application no. 8, columns 5 or 7, or functional homologues thereof.

**[0286.0.8.8]** In one advantageous embodiment, in the method of the present invention the activity of a polypeptide is increased which comprises or consists of a consensus sequence as indicated in Table IV, application no. 8, column 7, and in one another embodiment, the present invention relates to a polypeptide comprising or consisting of a consensus sequence as indicated in Table IV, application no. 8, column 7, , whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a poylpeptide or to a polypeptide comprising more than one consensus sequences as indicated in Table IV, application no. 8, column 7.

**[0287.0.0.8] to [0290.0.0.8]** for the disclosure of the paragraphs [0287.0.0.8] to [0290.0.0.8] see paragraphs [0287.0.0.0] to [0290.0.0.0] above.

**[0291.0.8.8]** In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of a plant or microorganism specific consensus sequences.
In one embodiment, said polypeptide of the invention distinguishes over the sequence as indicated in Table II, application no. 8, columns 5 or 7, by one or more amino acids. In one embodiment, polypeptide distinguishes form the sequence shown in Table II, application no. 8, columns 5 or 7, by more than 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from the sequence shown in Table II, application no. 8, columns 5 or 7, by not more than 80% or 70% of the amino acids, preferably not more than 60% or 50%, more preferred not more than 40% or 30%, even more preferred not more than 20% or 10%. In another embodiment, said polypeptide of the invention does not consist of the sequence shown in Table II, application no. 8, columns 5 or 7.

**[0292.0.0.8]** for the disclosure of this paragraph see [0292.0.0.0] above.

**[0293.0.8.8]** In one embodiment, the invention relates to polypeptide conferring an increase in the respective fine chemical in an organism or part being encoded by the nucleic acid molecule of the invention or by a nucleic acid molecule used in the process.
In one embodiment, the polypeptide of the invention has a sequence, which distinguishes from the sequence as shown in Table II, application no. 8, columns 5 or 7, by one or more amino acids. In another embodiment, said polypeptide of the invention does not consist of the sequence shown in Table II, application no. 8, columns 5 or 7. In a further embodiment, said polypeptide of the present invention is less than 100%, 99,999%, 99,99%, 99,9% or 99% identical. In one embodiment, said polypeptide does not consist of the sequence encoded by the nucleic acid molecules shown in Table I, application no. 8, columns 5 or 7.

**[0294.0.8.8]** In one embodiment, the present invention relates to a polypeptide having an activity of a protein as indicated in Table II, application no. 8, column 3, which distinguishes over the sequence as indicated in Table II, columns 5 or 7, by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.8], [0296.0.0.8] and [0297.0.5.8]** for the disclosure of the paragraphs [0295.0.0.8], [0296.0.0.8] and [0297.0.5.8] see paragraphs [0295.0.0.0] to [0297.0.0.0] above.

**[00297.1.0.8]** Non-polypeptide of the invention-chemicals are e.g. polypeptides having not the activity and/or the amino acid sequence of a polypeptide indicated in Table II, application no. 8, columns 3, 5 or 7.

**[0298.0.8.8]** A polypeptide of the invention can participate in the process of the present invention. The polypeptide or a portion thereof comprises preferably an amino acid sequence which is sufficiently homologous to an amino acid sequence as indicated in Table II, application no. 8, columns 5 or 7, such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, application no. 8, columns 5 or 7.

**[0299.0.8.8]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences of Table II, application no. 8, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table I, application no. 8, columns 5 or 7, or which is homologous thereto, as defined above.

**[0300.0.8.8]** Accordingly the polypeptide of the present invention can vary from a sequence as indicated in Table II, application no. 8, columns 5 or 7in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II, application no. 8, columns 5 or 7.

**[0301.0.0.8]** for the disclosure of this paragraph see [0301.0.0.0] above.

**[0302.0.8.8]** Biologically active portions of an polypeptide of the present invention include peptides comprising amino acid sequences derived from the amino acid sequence of the polypeptide of the present invention or used in the process of the present invention, e.g., an amino acid sequence shown in Table II, application no. 8, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.8]** for the disclosure of this paragraph see [0303.0.0.0] above.

**[0304.0.8.8]** Manipulation of the nucleic acid molecule of the invention or the nucleic acid molecule used in the method of the invention may result in the production of a protein having essentially the activity of the polypeptides as indicated in Table II, application no. 8, columns 5 or 7, but having differences in the sequence from said wild-type protein. These proteins may be improved in efficiency or activity, may be present in greater numbers in the cell than is usual, or may be decreased in efficiency or activity in relation to the wild type protein.

**[0305.0.5.8], [0306.0.5.8] and [0306.1.0.8]** for the disclosure of the paragraphs [0305.0.5.8], [0306.0.5.8] and [0306.1.0.8] see paragraphs [0305.0.0.0], [0306.0.0.0] and [0306.1.0.0] above.

**[0307.0.0.8] and [0308.0.0.8]** for the disclosure of the paragraphs [0307.0.0.8] and [0308.0.0.8] see paragraphs [0307.0.0.0 and [0308.0.0.0] above.

**[0309.0.8.8]** In one embodiment, a reference to protein (= polypeptide)" of the invention e.g. as indicated in Table II, application no. 8, columns 5 or 7, refers to a polypeptide having an amino acid sequence corresponding to the polypeptide of the invention or used in the process of the invention, whereas a "non- polypeptide" or "other polypeptide" e.g. not indicated in Table II, application no. 8, columns 5 or 7, refers to a polypeptide having an amino acid sequence corresponding to a protein which is not substantially homologous to a polypeptide of the invention, preferably which is not substantially homologous to a polypeptide having a protein activity, e.g., a protein which does not confer the activity described herein and which is derived from the same or a different organism.

**[0310.0.0.8] to [0334.0.0.8]** for the disclosure of the paragraphs [0310.0.0.8] to [0334.0.0.8] see paragraphs [0310.0.0.0] to [0334.0.0.0] above.

**[0335.0.8.8]** The dsRNAi method has proved to be particularly effective and advantageous for reducing the expression of a nucleic acid sequence as indicated in Table I, application no. 8, columns 5 or 7and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, application no. 8, columns 5 or 7, and/or homologs thereof. In a double-stranded RNA molecule for reducing the expression of an protein encoded by a nucleic acid sequence of one of the sequences as indicated in Table I, application no. 8, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.8] to [0342.0.0.8]** for the disclosure of the paragraphs [0336.0.0.8] to [0342.0.0.8] see paragraphs [0336.0.0.0] to [0342.0.0.0] above.

**[0343.0.8.8]** As has already been described, 100 % sequence identity between the dsRNA and a gene transcript of a nucleic acid sequence as indicated in Table I, application no. 8, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of the sequences as indicated in Table I, application no. 8, columns 5 or 7, or homologs thereof of the one organism, may be used to suppress the corresponding expression in another organism.

**[0344.0.0.8] to [0350.0.0.8], [0351.0.5.8] and [0352.0.0.8] to [0361.0.0.8]** for the disclosure of the paragraphs [0344.0.0.8] to [0350.0.0.8], [0351.0.5.8] and [0352.0.0.8] to [0361.0.0.8] see paragraphs [0344.0.0.0] to [0361.0.0.0] above.

**[0362.0.8.8]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention, e.g. encoding a polypeptide having an activity of a protein such as the polypeptides as indicated in Table II, application no. 8, column 3. Due to the above mentioned activity the fine chemical content in a cell or an organism is increased. For example, due to modulation or manupulation, the cellular activity is increased, e.g. due to an increased expression or specific activity of the subject matters of the invention in a cell or an organism or a part thereof. Transgenic for a polypeptide having an activity of a protein such as the polypeptides as indicated in Table II, application no. 8, column 3. Activity means herein that due to modulation or manipulation of the genome, the activity of polypeptide having an activity of a protein such as the polypeptides as indicated in Table II, application no. 8, column 3, or a similar activity, which is increased in the cell or organism or part thereof. Examples are described above in context with the process of the invention.

**[0363.0.0.8], [0364.0.5.8] and [0365.0.0.8] to [0379.0.5.8]** for the disclosure of the paragraphs [0363.0.0.8], [0364.0.5.8] and [0365.0.0.8] to [0379.0.5.8] see paragraphs [0363.0.0.0] to [0379.0.0.0] above.

**[0380.0.5.8], [0381.0.0.8] and [0382.0.0.8]** for the disclosure of the paragraphs [0380.0.5.8], [0381.0.0.8] and [0382.0.0.8] see paragraphs [0380.0.7.7], [0381.0.0.0] and [0382.0.0.0] above.

**[0383.0.5.8], [0384.0.0.8], [0385.0.5.8] and [0386.0.5.8]** for the disclosure of the paragraphs [0383.0.5.8], [0384.0.0.8], [0385.0.5.8] and [0386.0.5.8] see paragraphs [0383.0.7.7], [0384.0.0.0], [0385.0.7.7] and [0386.0.7.7] above.

**[0387.0.0.8] to [0392.0.0.8]** for the disclosure of the paragraphs [0387.0.0.8] to [0392.0.0.8] see paragraphs [0387.0.0.0] to [0392.0.0.0] above.

**[0393.0.8.8]** In one embodiment, the present invention relates to a method for the identification of a gene product conferring an increase in the fine chemical production in a cell, comprising the following steps:
(a) contacting-, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence shown in Table I, application no. 8, columns 5 or 7and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the respective fine chemical level in the host cells; and
(f) identifying the nucleic acid molecule and its gene product which expression confers an increase in the respective fine chemical level in the host cell after expression compared to the wild type.

**[0394.0.0.8] to [0552.0.0.8]** for the disclosure of the paragraphs [0394.0.0.8] to [0552.0.0.8] see paragraphs [0394.0.0.7] to [0552.0.0.7] above.

**[0553.0.8.8]** A process for the production of palmitic acid, which comprises
(a) increasing or generating the activity of a protein as indicated in Table II, application no. 8, columns 5 or 7, or a functional equivalent thereof in a non-human organism, or in one or more parts thereof; and
(b) growing the organism under conditions which permit the production of palmitic acid in said organism.
A process for the production of palmitic acid, comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 8, columns 5 or 7, or a fragment thereof, which confers an increase in the amount of palmitic acid in an organism or a part thereof;
b) nucleic acid molecule comprising of a nucleic acid molecule as indicated in Table I, application no. 8, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of palmitic acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of palmitic acid in an organism or a part thereof;
e) nucleic acid molecule which hybidizes with a nucleic acid molecule of (a) to (c) under stringent hybridisation conditions and conferring an increase in the amount of palmitic acid in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 8, column 7, and conferring an increase in the amount of palmitic acid in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of palmitic acid in an organism or a part thereof;
h) nucleic acid molecule encoding a polypeptide comprising a consensus sequence as indicated in Table IV, application no. 8, column 7, and conferring an increase in the amount of palmitic acid in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of palmitic acid in an organism or a part thereof.
or comprising a sequence which is complementary thereto.
The process as mentioned above, comprising recovering of the free or bound palmitic acid.
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound palmitic acid produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 8, columns 5 or 7, or a fragment thereof, which confers an increase in the amount of palmitic acid in an organism or a part thereof;
b) nucleic acid molecule comprising of a nucleic acid molecule as indicated in Table I, application no. 8, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of palmitic acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of palmitic acid in an organism or a part thereof;
e) nucleic acid molecule which hybidizes with a nucleic acid molecule of (a) to (c) under stringent hybridisation conditions and conferring an increase in the amount of palmitic acid in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 8, column 7, and conferring an increase in the amount of palmitic acid in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of palmitic acid in an organism or a part thereof;
h) nucleic acid molecule encoding a polypeptide comprising a consensus sequence as indicated in Table IV, application no. 8, column 7, and conferring an increase in the amount of palmitic acid in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of palmitic acid in an organism or a part thereof. whereby the nucleic acid molecule distinguishes over the sequence as indicated in Table I, application no. 8, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 8, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of palmitic acid in an organism or a part thereof comprising:
(a) contacting cells, tissues , plants or microorganisms which express the a polypeptide encoded by the nucleic acid molecule as mentioned above conferring an increase in the amount of palmitic acid in an organism or a part thereof with a candidate compound or a sample comprising a plurality of compounds under conditions which permit the expression the polypeptide;
(b) assaying the palmitic acid level or the polypeptide expression level in the cell, tissue, plant or microorganism or the media the cell, tissue, plant or microorganisms is cultured or maintained in; and
(c) identifying a agonist or antagonist by comparing the measured palmitic acid level or polypeptide expression level with a standard of palmitic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased palmitic acid production in a plant or microorganism, comprising the steps:
(a) culturing a plant cell or tissue or microorganism or maintaining a plant expressing the polypeptide encoded by the nucleic acid molecule as mentioned above conferring an increase in the amount of palmitic acid in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of palmitic acid in an organism or a part thereof;
(b) identifying if the compound is an effective agonist by detecting the presence or absence or increase of a signal produced by said readout system.
A method for the identification of a gene product conferring an increase in palmitic acid production in a cell, comprising the following steps:
(a) contacting the nucleic acid molecules of a sample, which can contain a candidate gene encoding a gene product conferring an increase in palmitic acid after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing palmitic acid;
(d) expressing the identified nucleic acid molecules in the host cells;
(e) assaying the palmitic acid level in the host cells; and
(f) identifying nucleic acid molecule and its gene product which expression confers an increase in the palmitic acid level in the host cell in the host cell after expression compared to the wild type.
A method for the identification of a gene product conferring an increase in palmitic acid production in a cell, comprising the following steps:
(a) identifiying in a data bank nucleic acid molecules of an organism; which can contain a candidate gene encoding a gene product conferring an increase in the palmitic acid amount or level in an organism or a part thereof after expression, and which are at least 20% homolog to the nucleic acid molecule as mentioned above;
(b) introducing the candidate nucleic acid molecules in host cells appropriate for producing palmitic acid;
(c) expressing the identified nucleic acid molecules in the host cells;
(d) assaying the palmitic acid level in the host cells; and
(e) identifying nucleic acid molecule and its gene product which expression confers an increase in the palmitic acid level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of palmitic acid after expression.
Use of the polypeptide as mentioned above or the nucleic acid construct as mentioned above or the gene product identified according to the method as mentioned above for identifying compounds capable of conferring a modulation of palmitic acid levels in an organism.
Food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the host cell as mentioned above or the gene product identified according to the method as mentioned above for the protection of a plant against a palmitic acid synthesis inhibiting herbicide. New genes for a process for the production of fine chemicals

**[0001.0.0.12]** for the disclosure of this paragraph see [0001.0.0.0].

**[0002.0.12.12]** Sterols are a class of essential, natural compounds required by all eukaryotes to complete their life cycle. In animals, cholesterol is typically the major sterol while in fungi it is ergosterol. Plants produce a class of sterols called phytosterols. Phytosterols are natural components of many vegetables and grains. The term "phytosterols" covers plant sterols and plant stanols. Plant sterols are naturally occurring substances present in the diet as minor components of vegetable oils. The structures of these plant sterols are similar to that of cholesterol with an extra methyl or ethyl group and a double bond in the side chain. Saturated plant sterols, referred to as stanols, have no double bond in the ring structure.
Phytosterols (including plant sterols and stanols) are natural components of plant foods, especially plant oils, seeds and nuts, cereals and legumes specially of edible vegetable oils such as sunflower seed oil and, as such are natural constituents of the human diet. The most common phytosterols are beta-sitosterol, campesterol, and stigmasterol. Beta-sitosterol is found in high amounts in nuts.

**[0003.0.12.12]** A high concentration of cholesterol in serum, i.e., hypercholesterolemia, is a wellknown risk factor for coronary heart disease (CHD). Blood cholesterol levels can be decreased by following diets, which are low in saturated fat, high in polyunsaturated fat and low in cholesterol. Although considerable achievements have been made in terms of knowledge and education, consumers still find it difficult to follow healthy eating advice.
Both plant sterols and plant stanols are effective in lowering plasma total and low density lipoprotein (LDL) cholesterol and this occurs by inhibiting the absorption of cholesterol from the small intestine. The plasma cholesterol-lowering properties of plant sterols have been known since the 1950s (Pollak, Circulation, 7, 702-706.1953). They have been used as cholesterol-lowering agents, first in a free form (Pollak and Kritchevsky, Sitosterol. In: Monographs on Aherosclerosis. Clarkson TB, Kritchevsky D,Pollak OJ, eds. New York, Basel, Karger 1981; 1-219) and recently mainly as esterified phytosterols (Katan et al., Mayo Clin Proc 2003; 78: 965-978).
The consumption of plant sterols and plant stanols lowers blood cholesterol levels by inhibiting the absorption of dietary and endogenously-produced cholesterol from the small intestine and the plant sterols/stanols are only very poorly absorbed themselves. This inhibition is related to the similarity in physico-chemical properties of plant sterols and stanols and cholesterol. Plant sterols and plant stanols appear to be without hazard to health, having been shown without adverse effects in a large number of human studies. They show no evidence of toxicity even at high dose levels and gastro-intestinal absorption is low.

**[0004.0.12.12]** The most abundant sterols of vascular plants are campesterol, sitosterol and stigmasterol, all of which contain a double bond between the carbon atoms at positions 5 and 6 and are classified as delta-5 sterols.
Exemplary naturally occurring delta-5 plant sterols isofucosterol, sitosterol, stigmasterol, campesterol, cholesterol, and dihydrobrassicasterol. Exemplary naturally occurring non-delta-5 plant sterols are cycloartenol, 24-methylene cycloartenol, cycloeucalenol, and obtusifoliol.
The ratio of delta-5 to non-delta-5 sterols in plants can be an important factor relating to insect pest resistance. Insect pests are unable to synthesize de novo the steroid nucleus and depend upon external sources of sterols in their food source for production of necessary steroid compounds. In particular, insect pests require an external source of delta-5 sterols. By way of example, externally provided delta-5 sterols are necessary for the production of ecdysteroids, hormones that control reproduction and development. See, e.g., Costet et al., Proc. Natl. Acad. Sci. USA, 84:643 (1987) and Corio-Costet et al., Archives of Insect Biochem. Physiol., 11:47 (1989).

**[0005.0.12.12]** US 20020148006 and WO 98/45457 describes the modulation of phytosterol compositions to confer resistance to insects, nematodes, fungi and/or environmental stresses, and/or to improve the nutritional value of plants by using a DNA sequence encoding a first enzyme; which binds a first sterol and is preferably selected from the group consisting of S-adenosyl-L-methionine- ₂₄₍₂₅₎-sterol methyl transferase, a C-4 demethylase, a cycloeucalenol to obtusifoliol-isomerase, a 14- -demethylase, a ₈ to ₇-isomerase, a ₇-C-5-desaturase and a 24,25-reductase, and produces a second sterol and a 3' non-translated region which causes polyadenylation at the 3' end of the RNA.
WO 93/16187 discloses new plants containing in its genome one or more genes involved in the early stages of phytosterol biosynthesis, preferably the genes encode mevalonate kinase.
US 5,306,862, US 5,589,619, US 5,365,017, US 5,349,126 and US 20030150008 describe a method of increasing sterol (and squalene) accumulation in a plant based on an increased HMG-CoA reductase activity to increase the pest resistance of transgenic plants.
WO 97/48793 discloses a C-14 sterol reductase polypeptide for the genetic manipulation of a plant sterol biosynthetic pathway.
US 20040172680 disclose the use of a gene expressing a SMT1 (sterol methyltransferase) to increase the level of sterols in the seeds of plants. A DNA sequence encoding sterol methyltransferase isolated from Zea mays is disclosed in WO 00/08190. Bouvier-Nav et al in Eur. J. Biochem. 256, 88-96 (1988) describes two families of sterol methyl transferases (SMTs), The first (SMT1) applying to cycloartenol and the second (SMT2) to 24-methylene lophenol. Schaller et al (Plant Physiology (1998) 118: 461-169) describes the over-expression of SMT2 from Arabidopsis in tobacco resulting in a change in the ratio of 24-methyl cholesterol to sitosterol in the tobacco leaf.
US 6,723,837 and US 20040199940 disclose nucleic acid molecules encoding proteins and fragments of proteins associated with sterol and phytosterol metabolism as well as cells, that have been manipulated to contain increased levels or overexpress at least one sterol or phytosterol compound. The protein or fragment is selected from the group consisting of a HES1, HMGCoA reductase, squalene synthase, cycloartenol synthase, SMTI, SMTII and UPC, preferably from member of the KES1/HES1/OSH1 family of oxysterol-binding (OSBP) proteins comprising an oxysterol-binding protein consensus sequence--E(K, Q) xSH (H, R) PPx (S, T, A, C, F)A. One class of proteins, oxysterol-binding proteins, have been reported in humans and yeast (Jiang et al., Yeast 10: 341-353 (1994), the entirety of which is herein incorporated by reference). These proteins have been reported to modulate ergosterol levels in yeast (Jiang et al., Yeast 10: 341-353 (1994)). In particular, Jiang et al., reported three genes KES1, HES1 and OSH1, which encode proteins containing an oxysterol-binding region.

**[0006.0.12.12]** Transgenic plants having altered sterol profiles could be instrumental in establishing a dietary approach to cholesterol management and cardiovascular disease prevention. The altered phytosterol profile further leads to pest resistance.

**[0007.0.12.12]** Although people consume plant sterols every day in their normal diet, the amount is not great enough to have a significant blood cholesterol lowering effect. The intake of phytosterols varies among different populations according to the food products being consumed, but the average daily Western diet is reported to contain 150-300 mg of these sterols (de Vries et al., J Food Comp Anal 1997; 19: 115-141; Björkhem et al. Inborn errors in bile acid biosynthesis and storage of sterols other than cholesterol. In: The Metabolic and Molecular Bases of Inherited Disease. Scriver CS, Beaudet AL, Sly WS, Valle D, eds. New York, McGraw-Hill 2001; 2961-2988). In order to achieve a cholesterol-lowering benefit, approximately 1 g/day of plant sterols need to be consumed (Hendriks et al., European Journal of Clinical Nutrition, 53, 319-327.1999).

**[0008.0.12.12]** Phytosterols are found naturally in plant foods at low levels. The enrichment of foods such as margarines with plant sterols and stanols is one of the recent developments in functional foods to enhance the cholesterol-lowering ability of traditional food products. Incorporation of additional phytosterols into the diet may be an effective way of lowering total and LDL cholesterol levels. The non-esterified phytosterols can be used as novel food ingredients in:
(a) bakery products and cereals (eg, breakfast cereals, breakfast bars);
(b) dairy products such as low and reduced fat liquid milk, low and reduced fat yoghurt and yoghurt products, and dairy based desserts;
(c) non-carbonated soft drinks like low and reduced fat soy beverages and low and reduced fat soy-based yoghurts;
(d) meat products or edible fats and oils (eg, mayonnaise, spice sauces, salad dressings);
(e) margarine; and
table spreads or dietary fats.

**[0009.0.12.12]** When edible oils undergo normal refining, plant sterols are partially extracted. It is estimated that 2500 tonnes of vegetable oil needs to be refined to produce 1 tonne of plant sterols. Plant stanols are obtained by hydrogenation of the plant sterols.

**[0010.0.12.12]** Another source of plant sterols is tall oil, derived from the process of paper production from wood and approximately 2500 tonnes of pine is required to produce 1 tonne of plant sterols. Tall oil also contains a higher proportion of plant stanols (primarily b-sitostanol) than do vegetable oils.

**[0011.0.12.12]** As described above, phytosterols are used in a lot of different applications, for example in cosmetics, pharmaceuticals and in feed and food. Therefore improving the quality of foodstuffs and animal feeds is an important task of the food-and-feed industry. Especially advantageous for the quality of foodstuffs and animal feeds is as balanced as possible a sterol profile in the diet.

**[0012.0.12.12]** Accordingly, there is still a great demand for new and more suitable genes which encode enzymes which participate in the biosynthesis of phytosterols and make it possible to produce them specifically on an industrial scale without unwanted byproducts forming. In the selection of genes for or regulators of biosynthesis two characteristics above all are particularly important. On the one hand, there is as ever a need for improved processes for obtaining the highest possible contents of phytosterols; on the other hand as less as possible by products should be produced in the production process.

**[0013.0.0.12]** for the disclosure of this paragraph see [0013.0.0.0] above.

**[0014.0.12.12]** Accordingly, in a first embodiment, the invention relates to a process for the production of a fine chemical, whereby the fine chemical is/are phytosterols. The term "phytosterols" covers plant sterols and plant stanols. Accordingly, in the present invention, the term "the fine chemical" as used herein relates to "phytosterols". Further, the term "the fine chemicals" as used herein also relates to fine chemicals comprising plant sterols and plant stanols, preferably beta-sitosterol, campesterol, and/or stigmasterol.

**[0015.0.12.12]** In one embodiment, the term "the fine chemical" means phytosterols, plant sterols and plant stanols. Throughout the specification the term "the fine chemical" means phytosterols and ester, thioester or sterols in free form or bound to other compounds. For the purpose of this description, the term sterol/stanol refers both to free sterols/stanols and conjugated sterols/stanols, for example, where the 3-hydroxy group is esterified by a fatty acid chain or phenolic acid to give a steryl/stanyl ester. As used herein, the term "phytosterol" includes all phytosterols without limitation, for example: sitosterol, campesterol, stigmasterol, brassicasterol, desmosterol, chalinosterol, poriferasterol, clionasterol, the corresponding stanols and all natural or synthesized forms and derivatives thereof, including isomers. It is to be understood that modifications to the phytosterols i.e. to include side chains also falls within the purview of this invention. All those derivates forms are summarized as "conjugates". In an preferred embodiment, the term "the fine chemical" or the term "phytosterol" or the term "the respective fine chemical" means at least one chemical compound plant sterols and plant stanols selected from the group "beta-sitosterol, sitostanol, stigmasterol, brassicasterol, campestanol, isofucosterol and campesterol", preferred "beta-sitosterol, campesterol, and/or stigmasterol", most preferred "beta-sitosterol and/or campesterol". Also preferably, are esters of sterols/stanols with C10-24 fatty acids.
Increased phytosterol content normally means an increased total phytosterol content. However, an increased phytosterol content also means, in particular, a modified content of the above-described compounds ("beta-sitosterol, sitostanol, stigmasterol, brassicasterol, campestanol, isofucosterol and campesterol") with phytosterol activity, without the need for an inevitable increase in the total phytosterol content.

**[0016.0.12.12]** Accordingly, the present invention relates to a process for the production of the respective fine chemical comprising
(a) increasing or generating the activity of one or more of a protein as shown in table II, application no. 12, column 3 encoded by the nucleic acid sequences as shown in table I, application no. 12, column 5, in a non-human organism or in one one or more parts thereof or
(b) growing the organism under conditions which permit the production of the fine chemical, thus beta-sitosterol and/or campesterol of the invention or fine chemicals comprising beta-sitosterol and/or campesterol of the invention, in said organism or in the culture medium surrounding the organism.

**[0016.1.12.12]**Accordingly, the term "the fine chemical" means in one embodiment "beta-Sitosterol" in relation to all sequences listed in Tables I to IV, line 19 or homologs thereof and means in one embodiment "Campesterol" in relation to all sequences listed in Table I to IV, line 20 , or homologs thereof.
Accordingly, in one embodiment the term "the fine chemical" means "beta-Sitosterol" and " Campesterol " in relation to all sequences listed in Table I to IV, lines 19 and/or 20.

**[0017.0.0.12] and [0019.0.0.12]** for the disclosure of the paragraphs [0017.0.0.12] and [0019.0.0.12] see paragraphs [0017.0.0.0] and [0019.0.0.0] above.

**[0020.0.12.12]**Surprisingly it was found, that the transgenic expression of the Zea mays protein as indicated in Table II, column 5, lines 19 and 20 in a plant conferred an increase in beta-Sitosterol and/or Campesterol (or the respective fine chemical) content of the transformed plants. Thus, in one embodiment, said protein or its homologs are used for the production of beta-Sitosterol; in one embodiment, said protein or its homologs are used for the production of Campesterol, in one embodiment, said protein or its homologs are used for the production of one or more fine chemical selected from the group consisting of beta-Sitosterol and/or Campesterol.

**[0021.0.0.12]** for the disclosure of this paragraph see [0021.0.0.0] above.

**[0022.0.12.12]** The sequence of YKR057W from Saccharomyces cerevisiae has been published in Dujon et al., Nature 369 (6479), 371-378, 1994 and Goffeau et al., Science 274 (5287), 546-547, 1996 and its activity is being defined as a ribosomal protein, similar to S21 ribosomal proteins, involved in ribosome biogenesis and translation. Accordingly, in one embodiment, the process of the present invention comprises the use of a protein involved in the ribosome biogenesis and translation, in particular of the superfamiliy of the ribosomal protein, preferably having a S21 ribosomal protein activity or its homolog, e.g. as shown herein, for the production of the respective fine chemical, meaning of phytosterol, e.g. beta-sitosterol and/or campesterol and/or conjugates, preferably in free or bound form in an organism or a part thereof, as mentioned. In one further embodiment the YKR057W protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention said activity, e.g. of a protein of the yeast ribosomal protein superfamily or preferably having a activity involved in ribosome biogenesis and translation is increased or generated, e.g. from Saccharomyces cerevisiae or a plant or a homolog thereof.

**[0022.1.0.12] to [0023.0.12.12]** for the disclosure of this paragraphs see [0022.1.0.0] to [0023.0.0.0] above.

**[0023.1.12.12]**Homologs of the polypeptide disclosed in table II, application no. 12, column 5, may have the activity of a protein as disclosed in table I or II, application no. 12, column 3 and may be the polypetides encoded by the nucleic acid molecules indicated in table I , application no. 12, column 7, resp., or may be the polypeptides indicated in table II, application no. 12, column 7, resp.

**[0024.0.0.12]** for the disclosure of this paragraph see [0024.0.0.0] above.

**[0025.0.12.12]** In accordance with the invention, a protein or polypeptide has the "activity of an protein as shown in table II, application no. 12, column 3" if its de *novo* activity, or its increased expression directly or indirectly leads to an increased in the level of the fine chemical indicated in the respective line of table II, application no. 12, column 6 "metabolite" in the organism or a part thereof, preferably in a cell of said organism. The protein has the above mentioned activities of a protein as shown in table II, application no. 12, column 3, preferably in the event the nucleic acid sequences encoding said proteins is functionally joined to the nucleic acid sequence of a transit peptide. Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 12, column 3, or which has at least 10% of the original enzymatic or biological activity, preferably 20%, particularly preferably 30%, most particularly preferably 40% in comparison to a protein as shown in the respective line of table II, application no. 12, column 3 of a E. coli or Saccharomyces cerevisae, respectively, protein as mentioned in table I to IV, column 3 respectively and as disclosed in paragraph [0022] of the respective invention.

**[0025.1.0.12] and [0025.2.0.12]** for the disclosure of the paragraphs [0025.1.0.12] and [0025.2.0.12] see [0025.1.0.0] and [0025.2.0.0] above.

**[0026.0.0.12] to [0033.0.0.12]** for the disclosure of the paragraphs [0026.0.0.12] to [0033.0.0.12] see paragraphs [0026.0.0.0] to [0033.0.0.0] above.

**[0034.0.12.12]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 12, column 3 its biochemical or genetical causes and the increased amount of the respective fine chemical.

**[0035.0.0.12] to [0038.0.0.12] and [0039.0.5.12]** for the disclosure of the paragraphs [0035.0.0.12] to [0038.0.0.12] and [0039.0.5.12] see paragraphs [0035.0.0.0] to [0039.0.0.0] above.

**[0040.0.0.12] to [0044.0.0.12]** for the disclosure of the paragraphs [0040.0.0.12] to [0044.0.0.12] see paragraphs [0035.0.0.0] and [0044.0.0.0] above.

**[0045.0.12.12.]** In one embodiment, the activity of the protein. as indicated in Table II, columns 5 or 7, application no. 12, is increased conferring an increase of the respective fine chemical, indicated in Table II, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more.

**[0046.0.12.12]** In one embodiment, the activity of the protein as indicated in Table II, columns 5 or 7, application no. 12, is increased conferring an increase of the respective fine chemical, indicated in Table II, column 6 of the respective line confers an increase of the respective fine chemical or their precursors.

**[0047.0.0.12] to [0048.0.0.12]** for the disclosure of the paragraphs [0047.0.0.12] and [0048.0.0.12] see paragraphs [0047.0.0.0] and [0048.0.0.0] above.

**[0049.0.12.12]** A protein having an activity conferring an increase in the amount or level of the fine chemical, has in one embodiment the structure of the polypeptide described herein, in particular of the polypeptides comprising the consensus sequence shown in table IV, application no. 12, column 7 or of the polypeptide as shown in the amino acid sequences as disclosed in table II, application no. 12, columns 5 and 7 or the functional homologues thereof as described herein, or is encoded by the nucleic acid molecule characterized herein or the nucleic acid molecule according to the invention, for example by the nucleic acid molecule as shown in table I, application no. 12, columns 5 and 7 or its herein described functional homologues and has the herein mentioned activity.

**[0050.0.12.12]** For the purposes of the present invention, the term "the respective fine chemical" also encompass the corresponding salts, such as, for example, the potassium or sodium salts of phytosterols or their ester.

**[0051.0.0.12] and [0052.0.0.12]** for the disclosure of the paragraphs [0051.0.5.12] and [0052.0.0.12] see paragraphs [0051.0.0.0] and [0052.0.0.0] above.

**[0053.0.12.12]**In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 12, columns 5 and 7 or its homologs activity having herein-mentioned beta-sitosterol and/or campesterol of the invention increasing activity; and/or
b) stabilizing a mRNA conferring the increased expression of a protein encoded by the nucleic acid molecule of the invention as shown in table I, application no. 12, columns 5 and 7, e.g. a nucleic acid sequence encoding a polypeptide having the activity of a protein as indicated in table II, application no. 12, columns 5 and 7 or its homologs activity or of a mRNA encoding the polypeptide of the present invention having herein-mentioned beta-sitosterol and/or campesterol of the invention increasing activity; and/or
c) increasing the specific activity of a protein conferring the increased expression of a protein encoded by the nucleic acid molecule of the invention or of the polypeptide of the present invention having herein-mentioned beta-sitosterol and/or campesterol increasing activity, e.g. of a polypeptide having the activity of a protein as indicated in table II, application no. 12, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned beta-sitosterol and/or campesterol of the invention increasing activity, e.g. of a polypeptide having the activity of a protein as indicated in table II, application no. 12, columns 5 and 7 or its homologs activity; and/or
e) stimulating activity of a protein conferring the increased expression of a protein encoded by the nucleic acid molecule of the present invention or a polypeptide of the present invention having herein-mentioned beta-sitosterol and/or campesterol of the invention increasing activity, e.g. of a polypeptide having the activity of a protein as indicated in table II, application no. 12, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned beta-sitosterol and/or campesterol of the invention increasing activity, e.g. of a polypeptide having the activity of a protein as indicated in table II, application no. 12, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned beta-sitosterol and/or campesterol of the invention increasing activity, e.g. of a polypeptide having the activity of a protein as indicated in table II, application no. 12, columns 5 and 7 or its homologs activity; and/or
h) increasing the expression of the endogenous gene encoding the polypeptide of the invention, e.g. a polypeptide having the activity of a protein as indicated in table II, application no. 12, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
k) selecting of organisms with especially high activity of the proteins of the invention from natural or from mutagenized resources and breeding them into the target organisms, eg the elite crops.

**[0054.0.12.12]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 12 in Table I to IV, resp., after increasing the expression or activity of the encoded polypeptide or having the activity of a polypeptide having an activity as the protein as shown in table II, application no. 12, column 5 or 7 . In general, the amount of mRNA or polypeptide in a cell or a compartment of a organism correlates with the amount of encoded protein and thus with the overall activity of the encoded protein in said volume. Said correlation is not always linear, the activity in the volume is dependent on the stability of the molecules or the presence of activating or inhibiting co-factors. Further, product and educt inhibitions of enzymes are well known and described in Textbooks, e.g. Stryer, Biochemistry.

**[0055.0.0.12] to [0067.0.0.12]** for the disclosure of the paragraphs [0055.0.0.12] to [0067.0.0.12] see paragraphs [0055.0.0.0] to [0067.0.0.0] above.

**[0068.0.12.12]** The mutation is introduced in such a way that the production of the phytosterol(s) is not adversely affected.

**[0069.0.0.12]** for the disclosure of this paragraph see paragraphs [0069.0.0.0] above.

**[0070.0.12.12]** Owing to the introduction of a gene or a plurality of genes conferring the expression of the nucleic acid molecule of the invention or the polypeptide of the invention, for example the nucleic acid construct mentioned below, or encoding the YKR057W protein into an organism alone or in combination with other genes, it is possible not only to increase the biosynthetic flux towards the end product, but also to increase, modify or create de *novo* an advantageous, preferably novel metabolites composition in the organism, e.g. an advantageous fatty acid composition comprising a higher content of (from a viewpoint of nutrional physiology limited) phytosterol(s) etc..

**[0071.0.0.12]** for the disclosure of this paragraph see [0071.0.0.0] above.

**[0072.0.12.12]** By influencing the metabolism thus, it is possible to produce, in the process according to the invention, further advantageous compounds. Examples of such compounds are, in addition to phytosterols further sterols, stanols or squalene, squalene epoxide or cycloartenol.

**[0073.0.12.12]**Accordingly, in one embodiment, the process according to the invention relates to a process, which comprises:
a) providing a non-human organism, preferably a microorganism, a non-human animal, a plant or animal cell, a plant or animal tissue or a plant;
b) increasing an activity of a polypeptide of the invention or a homolog thereof, e.g. as indicated in Table II, application no. 12, columns 5 or 7, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
d) if desired, revovering, optionally isolating, the free and/or bound the respective fine chemical and, optionally further free and/or bound phytosterol or its conjugate synthesised by the organism, the microorganism, the non-human animal, the plant or animal cell, the plant or animal tissue or the plant.

**[0074.0.12.12]** The organism, in particular the microorganism, non-human animal, the plant or animal cell, the plant or animal tissue or the plant is advantageously grown in such a way that it is not only possible to recover, if desired isolate the free or bound the respective fine chemical or the free and bound respective fine chemical but as option it is also possible to produce, recover and, if desired isolate, other free or/and bound of phytosterol(s) or its/their conjugates.

**[0075.0.0.12] to [0077.0.0.12]** for the disclosure of the paragraphs [0075.0.0.12] to [0077.0.0.12] see paragraphs [0075.0.0.0] to [0077.0.0.0] above.

**[0078.0.12.12]**The organism such as microorganisms or plants or the recovered, and if desired isolated, respective fine chemical can then be processed further directly into foodstuffs or animal feeds or for other applications, for example according to the disclosures made in:
US 20040101829, which disclose a methods for treating hyperlipidemia and to reduce Low Density Lipoprotein ("LDL") levels in a subject,
US 20040047971, which disclose the preparation of a fat composition containing sterol esters characterised by direct interesterification of sterol with triglyceride,
US 5,965,449, which describes phytosterol-based compositions useful in preventing and treating cardiovascular disease and other disorders,
US 5,523,087, which is for a pharmaceutical composition containing beta-sitosterol for the treatment of diabetic male sexual dysfunction;
US 5,747,464, which discloses a composition for inhibiting absorption of fat and cholesterol from the gut comprising beta.-sitosterol bound irreversibly to pectin,
US 4,588,717, which describes a vitamin supplement which comprises a fatty acid ester of a phytosterol,
US 5,270,041, which teaches the use of small amounts of sterols, their fatty acid esters and glucosides for the treatment of tumours,
US 6,087,353, which comprises methods of making a composition suitable for incorporation into foods, beverages, pharmaceuticals, nutraceuticals and the like which comprises condensing a suitable aliphatic acid with a phytosterol to form a phytosterol ester and subsequently hydrogenating the phytosterol ester to form a hydrogenated phytosterol ester,
which are expressly incorporated herein by reference.
The fermentation broth, fermentation products, plants or plant products can be treated with water and a mixture of organic solvents (hexane and acetone) in order to extract the phytosterols. Crude phytosterols are obtained from the organic phase by removal of the solvents, complexation of the sterols in the extract with calcium chloride in methanol, separation of the sterol-complexes by centrifugation, dissociation of the complexes by heating in water and removal of the water. The crude phytosterols can be further purified by crystallisation from isopropanol. According to an other production process the tall oil soap is first subjected to fractional distillation which removes volatile compounds. The resulting residue (tall oil pitch) containing sterols in esterified form is treated with alkali to liberate these sterols. After neutralisation, the material is subjected to a two-stage distillation process. The distillate is then dissolved in methanol/methylethylketone solvent and the sterols crystallising from this solution are obtained by filtration, washed with solvent and dried. US 4,420,427 teaches the preparation of sterols from vegetable oil sludge using solvents such as methanol. Alternatively, phytosterols may be obtained from tall oil pitch or soap, by-products of
the forestry practise as described in PCT/CA95/00555, incorporated herein by reference. The extraction and crystallization may be performed by other methods known to the person skilled in the art and described herein below.
To form a phytosterol ester in accordance with the US 6,087,353, the selected phytosterol and aliphatic acid or its ester with volatile alcohol are mixed together under reaction conditions to permit condensation of the phytosterol with the aliphatic acid to produce an ester. A most preferred method of preparing these esters which is widely used in the edible fat and oil industry is described in U.S. Pat. No. 5,502,045 (which is incorporated herein by reference). The stanol and/or sterol esters with the desired fatty acid composition can also be produced by direct, preferably catalytic esterification methods, e.g. U.S. Pat. No. 5,892,068, between free fatty acids or fatty acid blends of the composition and the stanol and/or sterol. In addition, stanol and/or sterol esters can also be produced by enzymatic esterification e.g. as outlined in EP 195 311 (which are incorporated herein by reference).

Products of these different work-up procedures are phytosterols and/or esters and/or conjugates or compositions which still comprise fermentation broth, plant particles and cell components in different amounts, advantageously in the range of from 0 to 99% by weight, preferably below 80% by weight, especially preferably between below 50% by weight.

**[0079.0.0.12] to [0084.0.0.12]** for the disclosure of the paragraphs [0079.0.0.12] to [0084.0.0.12] see paragraphs [0079.0.0.0] to [0084.0.0.0] above.

**[0085.0.12.12]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, application no. 12, columns 5 or 7, or a derivative thereof, or
b) a genetic regulatory element, for example a promoter, which is functionally linked to the nucleic acid sequence as indicated in Table I, application no. 12, columns 5 or 7, or a derivative thereof, or
c) (a) and (b) is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.12] and [0087.0.0.12]** for the disclosure of the paragraphs [0086.0.0.12] and [0087.0.0.12] see paragraphs [0086.0.0.0] and [0087.0.0.0] above.

**[0088.0.12.12]** In an advantageous embodiment of the invention, the organism takes the form of a plant whose phytosterol content is modified advantageously owing to the nucleic acid molecule of the present invention expressed. This is important for plant breeders since, for example, the nutritional value of plants for poultry is dependent on the abovementioned phytosterol and the general amount of phytosterol as source in feed and/or food. Further, this is also important since, for example a balanced content of different phytosterols induces stress resistance to plants. After the YKR057W, protein activity has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.12], [0089.0.0.12] and [0090.0.0.12]** for the disclosure of the paragraphs [0088.1.0.12], [0089.0.0.12] and [0090.0.0.12] see paragraphs [0088.1.0.0], [0089.0.0.0] and [0090.0.0.0] above.

**[0091.0.12.12]** Thus, the content of plant components and preferably also further impurities is as low as possible, and the abovementioned phytosterols are obtained in as pure form as possible. In these applications, the content of plant components advantageously amounts to less than 10%, preferably 1%, more preferably 0.1%, very especially preferably 0.01 % or less.

**[0092.0.0.12] to [0094.0.0.12]** for the disclosure of the paragraphs [0092.0.0.12] to [0094.0.0.12] see paragraphs [0092.0.0.0] to [0094.0.0.0] above.

**[0095.0.12.12]** It may be advantageous to increase the pool of said phytosterols in the transgenic organisms by the process according to the invention in order to isolate high amounts of the pure respective fine chemical.

**[0096.0.12.12]** In another preferred embodiment of the invention a combination of the increased expression of the nucleic acid sequence or the protein of the invention together with the transformation of a protein or polypeptid or a compound, which functions as a sink for the desired fine chemical, for example beta-sitosterol or campesterol in the organism, is useful to increase the production of the respective fine chemical.

**[0097.0.0.12]** for the disclosure of this paragraph see paragraph [0097.0.0.0] above.

**[0098.0.12.12]** In a preferred embodiment, the respective fine chemical (beta-sitosterol or campesterol) is produced in accordance with the invention and, if desired, is isolated. The production of further phytosterols or conjugates or mixtures thereof or mixtures with other compounds by the process according to the invention is advantageous.

**[0099.0.12.12]** In the case of the fermentation of microorganisms, the abovementioned phytosterol (preferably beta-sitosterol and/or campesterol) accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical or the phytosterol (preferably beta-sitosterol and/or campesterol) comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.12.12]** Transgenic plants which comprise the phytosterol (preferably beta-sitosterol and/or campesterol) synthesized in the process according to the invention can advantageously be marketed directly without there being any need for the phytosterols (preferably beta-sitosterol and/or campesterol) (oils, lipids or fatty acids synthesized to be isolated). Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue. However, the respective fine chemical produced in the process according to the invention can also be isolated from the organisms, advantageously plants, in the form of their oils, fats, lipids, esters and/or as extracts, e.g. ether, alcohol, or other organic solvents or water containing extract and/or free phytosterol(s) . The respective fine chemical produced by this process can be obtained by harvesting the organisms, either from the crop in which they grow, or from the field. This can be done via pressing or extraction of the plant parts, preferably the plant seeds. To increase the efficiency of extraction it is beneficial to clean, to temper and if necessary to hull and to flake the plant material especially the seeds. In this context, the oils, fats, lipids, esters and/or free phytosterols can be obtained by what is known as cold beating or cold pressing without applying heat. To allow for greater ease of disruption of the plant parts, specifically the seeds, they are previously comminuted, steamed or roasted. The seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. In this manner, more than 96% of the compounds produced in the process can be isolated. Thereafter, the resulting products are processed further, i.e. degummed and/or refined. In this process, substances such as the plant mucilages and suspended matter are first removed. What is known as desliming can be affected enzymatically or, for example, chemico-physically by addition of acid such as phosphoric acid.
Plant sterols (phytosterols) are by-products of traditional vegetable oil refining. The source may be commonly a blend of crude edible oils, consisting of soy bean oil or of
other edible oils, e.g. corn, rapeseed, olive and palm oil in varying proportions. Hemp may also be a source of new oilseed, oil and food ingredients as well as Sea buckthorn (hippophaë rhamnoides). The crude oil, which is obtained by pressing or solvent extraction, may undergoes a series of refining processes to remove solvents, lecithins, free fatty acids, color bodies, off-odors and off-flavors. In one of these steps, the oil may be subjected to steam distillation at reduced pressure (deodorisation) and the resulting distillate contains the phytosterol fraction. From this fraction, fatty acids, lecithins and other compounds are removed by fractional distillation, ethanolysis/transesterification, distillation and crystallisation from a heptane solution, and the phytosterols are further purified by recrystallisation using food grade materials and good manufacturing practices. The extraction and purification steps are standard methods and similar to the procedures used traditionally by the food industry for the production of plant sterols. Phytosterol esters may be produced from the sterols using food grade vegetable oil-derived fatty acids or triglycerides and applying standard methods for esterification or transesterification commonly used in the fats and oils industry.
Phytosterol in microorganisms may be localized intracellularly, therefor their recovery essentials comes down to the isolation of the biomass. Well-establisthed approaches for the harvesting of cells include filtration, centrifugation and coagulation/flocculation as described herein. Determination of tocopherols in cells has been described by Tan and Tsumura 1989, see also Biotechnology of Vitamins, Pigments and Growth Factors, Edited by Erik J. Vandamme, London, 1989, p.96 to 103. Many further methods to determine the tocopherol content are known to the person skilled in the art.

**[0101.0.12.12]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.12.12]** Phytosterols can for example be analyzed advantageously via HPLC or GC separation methods and detected by MS oder MSMS methods. The unambiguous detection for the presence of beta-sitosterol and/or campesterol containing products can be obtained by analyzing recombinant organisms using analytical standard methods: GC, GC-MS or TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353). The material to be analyzed can be disrupted by sonication, grinding in a glass mill, liquid nitrogen and grinding, cooking, or via other applicable methods; see also Biotechnology of Vitamins, Pigments and Growth Factors, Edited by Erik J. Vandamme, London, 1989, p.96 to 103.
For the sterol isolation and analysis the German standard method F III (1) may be used. The method comprises: saponification of fat, isolation of unsaponifiable matter using an aliminium oxide column, separation of sterol fraction by preparative TLC and determination of the composition of sterols as trimetysilyl ethers by GLC.

**[0103.0.12.12]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, application no. 12, columns 5 or 7, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 12, columns 5 or 7.
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybidizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, application no. 12, column 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, application no. 12, column 7, and conferring an increase in the amount of the respective fine chemical in an organism or a part thereof ;
k) nucleic acid molecule comprising one or more of the nucleic acid molecule encoding the amino acid sequence of a polypeptide encoding a domain of the polypeptide indicated in Table II, application no. 12, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.0.12.]** In one embodiment, the nucleic acid molecule used in the process of the invention distinguishes over the sequence indicated in Table I, application no. 12, columns 5 or 7 by one or more nucleotides. In one embodiment, the nucleic acid molecule used in the process of the invention does not consist of the sequence shown in indicated in Table I ,application no. 12, columns 5 or 7,. In one embodiment, the nucleic acid molecule used in the process of the invention is less than 100%, 99,999%, 99,99%, 99,9% or 99% identical to a sequence indicated in Table I , application no. 12, columns 5 or 7,. In another embodiment, the nucleic acid molecule does not encode a polypeptide of a sequence indicated in Table II, application no. 12, columns 5 or 7.

**[0104.0.12.12]** In one embodiment, the nucleic acid molecule used in the process distinguishes over the sequence indicated in Table I, application no. 12, columns 5 or 7, y one or more nucleotides. In one embodiment, the nucleic acid molecule used in the process of the invention does not consist of the sequence indicated in Table I, application no. 12, columns 5 or 7, In one embodiment, the nucleic acid molecule of the present invention is less than 100%, 99,999%, 99,99%, 99,9% or 99% identical to the sequence indicated in Table I, application no. 12, columns 5 or 7. In another embodiment, the nucleic acid molecule does not encode a polypeptide of a sequence indicated in Table II, application no. 12, columns 5 or 7.

**[0105.0.0.12] to [0107.0.0.12]** for the disclosure of the paragraphs [0105.0.0.12] to [0107.0.0.12] see paragraphs [0105.0.0.0] and [0107.0.0.0] above.

**[0108.0.12.12]** Nucleic acid molecules with the sequence as indicated in Table I, application no. 12, columns 5 or 7, nucleic acid molecules which are derived from an amino acid sequences as indicated in Table II, application no. 12, columns 5 or 7, or from polypeptides comprising the consensus sequence as indicated in Table IV, application no. 12, column 7, or their derivatives or homologues encoding polypeptides with the enzymatic or biological activity of an activity of a polypeptide as indicated in Table II, application no. 12, column 3, 5 or 7, or conferring an increase of the respective fine chemical, meaning phytosterol, in particular, beta-sitosterol and/or campesterol after increasing its expression or activity are advantageously increased in the process according to the invention.

**[0109.0.0.12]** for the disclosure of this paragraph see [0109.0.0.0] above.

**[0110.0.12.12]** Nucleic acid molecules, which are advantageous for the process according to the invention and which encode polypeptides with an activity of a polypeptide used in the method of the invention or used in the process of the invention, e.g. of a protein as shown in Table II, application no. 12, columns 5 or 7, or being encoded by a nucleic acid molecule indicated in Table I, application no. 12, columns 5 or 7, or of its homologs, e.g. as indicated in Table II, application no. 12, columns 5 or 7, can be determined from generally accessible databases.

**[0111.0.0.12]** for the disclosure of this paragraph see [0111.0.0.0] above.

**[0112.0.12.12]** The nucleic acid molecules used in the process according to the invention take the form of isolated nucleic acid sequences, which encode polypeptides with an activity of a polypeptide as indicated in Table II, application no. 12, column 3, or having the sequence of a polypeptide as indicated in Table II, application no. 12, columns 5 and 7, and conferring an increase in the level of phytosterols, preferably beta-sitosterol and/or campesterol.

**[0113.0.0.12] to [0120.0.0.12]** for the disclosure of the paragraphs [0113.0.0.12] to [0120.0.0.12] see paragraphs [0113.0.0.0] and [0120.0.0.0] above.

**[0121.0.12.12]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 12, columns 5 or 7, or the functional homologues thereof as described herein, preferably conferring above-mentioned activity, i.e. conferring an increase in the level of beta-sitosterol after increasing the activity of the polypeptide sequences indicated in Table II, application no. 12, columns 5 or 7, , or conferring increase in the level of campesterol after increasing the activity of the polypeptide sequences indicated in Table II, application no. 12, columns 5 or 7,.

**[0122.0.0.12] to [0127.0.0.12]** for the disclosure of the paragraphs [0122.0.0.12] to [0127.0.0.12] see paragraphs [0122.0.0.0] and [0127.0.0.0] above.

**[0128.0.12.12]** Synthetic oligonucleotide primers for the amplification, e.g. as the pairs indicated in Table III, application no. 12, column 7, by means of polymerase chain reaction can be generated on the basis of a sequence shown herein, for example the sequence as indicated in Table I, application no. 12, columns 5 or 7, or the sequences derived from a sequence as indicated in Table II, application no. 12, columns 5 or 7,.

**[0129.0.12.12]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved regions are those, which show a very little variation in the amino acid in one particular position of several homologs from different origin. The consenus sequences indicated in Table IV, application no. 12, column 7, are derived from said aligments.

**[0130.0.12.12]** Degenerated primers can then be utilized by PCR for the amplification of fragments of novel proteins having above-mentioned activity, e.g. conferring the increase of phytosterol, preferably beta-sitosterol and/or campesterol resp after increasing the expression or activity of the protein comprising said fragment.

**[0131.0.0.12] to [0138.0.0.12]** for the disclosure of the paragraphs [0131.0.0.12] to [0138.0.0.12] see paragraphs [0131.0.0.0] to [0138.0.0.0] above.

**[0139.0.12.12]** Polypeptides having above-mentioned activity, i.e. conferring the respective fine chemical increase, derived from other organisms, can be encoded by other DNA sequences which hybridize to a sequences indicated in Table I, application no. 12, columns 5 or 7, under relaxed hybridization conditions and which code on expression for peptides having the phytosterol, preferably beta-sitosterol and/or campesterol increasing activity.

**[0140.0.0.12] to [0146.0.0.12]** for the disclosure of the paragraphs [0140.0.0.12] to **[0146.0.0.12]** see paragraphs [0140.0.0.0] to [0146.0.0.0] above.

**[0147.0.12.12]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 12, columns 5 or 7, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.12.12]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 12, columns 5 or 7 or a portion thereof and preferably has above mentioned activity, in particular, of beta-Sitosterol and/or Campesterol increasing activity after increasing the acitivity or an activity of a product of a gene encoding said sequences or their homologs.

**[0149.0.12.12]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 12, columns 5 or 7, or a portion thereof and encodes a protein having above-mentioned activity and as indicated in indicated in Table II.

**[00149.1.12.12]** Optionally, in one embodiment, the nucleotide sequence, which hybridises to one of the nucleotide sequences indicated in Table I, application no. 12, columns 5 or 7, has further one or more of the activities annotated or known for the a protein as indicated in Table II, application no. 12, column 3,.

**[0150.0.12.12]** Moreover, the nucleic acid molecule of the invention can comprise only a portion of the coding region of one of the sequences indicated in Table I, application no. 12, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of beta-Sitosterol and/or Campesterol, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 12, columns 5 or 7, , an anti-sense sequence of one of the sequences, e.g., as indicated in Table I, application no. 12, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 12, column 7, will result in a fragment of a polynucleotide sequence as indicated in Table I, application no. 12, columns 5 or 7 or its gene product.

**[0151.0.0.12]** for the disclosure of this paragraph see paragraph [0151.0.0.0] above.

**[0152.0.12.12]** The nucleic acid molecule of the invention encodes a polypeptide or portion thereof which includes an amino acid sequence which is sufficiently homologous to an amino acid sequence as indicated in Table II, application no. 12, columns 5 or 7, such that the protein or portion thereof maintains the ability to participate in the respective fine chemical production, in particular an activity increasing the level of phytosterol, in particular, of beta-sitosterol and/or campesterol, resp., as mentioned above or as described in the examples in plants or microorganisms is comprised.

**[0153.0.12.12]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 12, columns 5 or 7, such that the protein or portion thereof is able to participate in the increase of the respective fine chemical production. In one embodiment, a protein or portion thereof as indicated in Table II, application no. 12, columns 5 or 7, has for example an activity of a polypeptide as indicaded in Table II, application no. 12, column 3,.

**[0154.0.12.12]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, application no. 12, columns 5 or 7, and has above-mentioned activity, e.g.conferring preferably the increase of the respective fine chemical.

**[0155.0.0.12] and [0156.0.0.12]** for the disclosure of the paragraphs [0155.0.0.12] and [0156.0.0.12] see paragraphs [0155.0.0.0] and [0156.0.0.0] above.

**[0157.0.12.12]** The invention further relates to nucleic acid molecules that differ from one of the nucleotide sequences as indicated in Table I, application no. 12, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides encoded by the sequence as indicated in Table I, application no. 12, columns 5 or 7, or of the polypeptide as indicated in Table II, application no. 12, columns 5 or 7, or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, application no. 12, column 7, or of the polypeptide as indicated in Table II, application no. 12, columns 5 or 7, , or the functional homologues. In a still further embodiment, the nucleic acid molecule of the invention encodes a full length protein which is substantially homologous to an amino acid sequence comprising a consensus sequence as indicated in Table IV, application no. 12, column 7, or of a polypeptide as indicated in Table II, application no. 12, columns 5 or 7, or the functional homologues. However, in a preferred embodiment, the nucleic acid molecule of the present invention does not consist of a sequence as indicated in Table I, application no. 12, columns 5 or 7, Preferably the nucleic acid molecule of the invention is a functional homologue or identical to a nucleic acid molecule indicated in Table I, application no. 12, column 7,.

**[0158.0.0.12] to [0160.0.0.12]** for the disclosure of the paragraphs [0158.0.0.12] to [0160.0.0.12] see paragraphs [0158.0.0.0] to [0160.0.0.0] above.

**[0161.0.12.12]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 12, columns 5 or 7,. The nucleic acid molecule is preferably at least 20, 30, 50, 100, 250 or more nucleotides in length.

**[0162.0.0.12]** for the disclosure of this paragraph see paragraph [0162.0.0.0] above.

**[0163.0.12.12]** Preferably, nucleic acid molecule of the invention that hybridizes under stringent conditions to a sequence as indicated in Table I, application no. 12, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.12]** for the disclosure of this paragraph see paragraph [0164.0.0.0] above.

**[0165.0.12.12]** For example, nucleotide substitutions leading to amino acid substitutions at "non-essential" amino acid residues can be made in a sequence of the nucleic acid molecule of the invention or used in the process of the invention, e.g. as indicated in Table I, application no. 12, columns 5 or 7,.

**[0166.0.0.12] and [0167.0.0.12]** for the disclosure of the paragraphs [0166.0.0.12] and [0167.0.0.12] see paragraphs [0166.0.0.0] and [0167.0.0.0] above.

**[0168.0.12.12]** Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 12, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 12, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 12, columns 5 or 7, more preferably at least about 70% identical to one of the sequences as indicated in Table II, application no. 12, columns 5 or 7, ,even more preferably at least about 80%, 90%, or 95% homologous to a sequence as indicated in Table II, application no. 12, columns 5 or 7, , and most preferably at least about 96%, 97%, 98%, or 99% identical to the sequence as indicated in Table II, application no. 12, columns 5 or 7.

**[0169.0.0.12] to [0172.0.0.12]** for the disclosure of the paragraphs [0169.0.0.12] to [0172.0.0.12] see paragraphs [0169.0.0.0] to [0172.0.0.0] above.

**[0173.0.12.12]** For example a sequence which has a 80% homology with sequence SEQ ID NO: 268 at the nucleic acid level is understood as meaning a sequence which, upon comparison with the sequence SEQ ID NO: 268 by the above Gap program algorithm with the above parameter set, has a 80% homology.

**[0174.0.0.12]** for the disclosure of the paragraph [0174.0.0.12] see paragraph [0174.0.0.0] above.

**[0175.0.12.12]** For example a sequence which has a 80% homology with sequence SEQ ID NO: 269 at the protein level is understood as meaning a sequence which, upon comparison with the sequence SEQ ID NO: 269 by the above program algorithm with the above parameter set, has a 80% homology.

**[0176.0.12.12]** Functional equivalents derived from one of the polypeptides as indicated in Table II, application no. 12, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 12, columns 5 or 7, according to the invention and are distinguished by essentially the same properties as a polypeptide as indicated in Table II, application no. 12, columns 5 or 7,.

**[0177.0.12.12]** Functional equivalents derived from a nucleic acid sequence as indicated in Table I, application no. 12, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptide as indicated in Table II, application no. 12, columns 5 or according to the invention and encode polypeptides having essentially the same properties as a polypeptide as indicated in Table II, application no. 12, columns 5 or 7,.

**[0178.0.0.12]** for the disclosure of this paragraph see [0178.0.0.0] above.

**[0179.0.12.12]** A nucleic acid molecule encoding an homologous to a protein sequence as indicated in Table II, application no. 12, columns 5 or 7, can be created by introducing one or more nucleotide substitutions, additions or deletions into a nucleotide sequence of the nucleic acid molecule of the present invention, in particular as indicated in Table I, application no. 12, columns 5 or 7, such that one or more amino acid substitutions, additions or deletions are introduced into the encoded protein. Mutations can be introduced into the encoding sequences of a sequence as indicated in Table I, application no. 12, columns 5 or 7, by standard techniques, such as site-directed mutagenesis and PCR-mediated mutagenesis.

**[0180.0.0.12] to [0183.0.0.12]** for the disclosure of the paragraphs [0180.0.0.12] to [0183.0.0.12] see paragraphs [0180.0.0.0] to [0183.0.0.0] above.

**[0184.0.12.12]** Homologues of the nucleic acid sequences used, with a sequence as indicated in Table I, application no. 12, columns 5 or 7, or of the nucleic acid sequences derived from a sequences as indicated in Table II, application no. 12, columns 5 or 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 12, columns 5 or 7, or from the derived nucleic acid sequences, the intention being, however, that the enzyme activity or the biological activity of the resulting proteins synthesized is advantageously retained or increased.

**[0185.0.12.12]** In one embodiment of the present invention, the nucleic acid molecule of the invention or used in the process of the invention comprises one or more sequences as indicated in Table I, application no. 12, columns 5 or 7,. In one embodiment, it is preferred that the nucleic acid molecule comprises as little as possible other nucleotides not shown in any one of sequences as indicated in Table I, application no. 12, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 12, columns 5 or 7, ,

**[0186.0.12.12]** Also preferred is that one or more nucleic acid molecule(s) used in the process of the invention encodes a polypeptide comprising a sequence as indicated in Table II, application no. 12, columns 5 or 7,. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 12, columns 5 or 7,.

**[0187.0.12.12]** In one embodiment, a nucleic acid molecule of the invention or used in the process encodes a polypeptide comprising the sequence as indicated in Table II, application no. 12, columns 5 or 7, comprises less than 100 further nucleotides. In a further embodiment, said nucleic acid molecule comprises less than 30 further nucleotides. In one embodiment, the nucleic acid molecule used in the process is identical to a coding sequence of the amino sequences indicated in Table II, application no. 12, columns 5 or 7.

**[0188.0.12.12]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 12, columns 5 or 7 and is expressed under identical conditions.

**[0189.0.12.12]** Homologues of a sequences as indicated in Table I, application no. 12, columns 5 or 7, or of a derived sequences as indicated in Table II, application no. 12, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.12] to [0203.0.0.12]** for the disclosure of the paragraphs [0190.0.0.12] to [0203.0.0.12] see paragraphs [0190.0.0.0] to [0203.0.0.0] above.

**[0204.0.12.12]** Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 12, columns 5 or 7, or a fragment thereof conferring an increase in the amount of the respective fine chemical, in particular according to table II , application no. 12, column 6 in an organism or a part thereof
b) nucleic acid molecule comprising, preferably at least the mature form, of a nucleic acid molecule as indicated in Table I, application no. 12, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, application no. 12, column 7, and conferring an increase in the amount of the respective fine chemical, in particular according to table II, application no. 12, column 6 in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, application no. 12, columns 5 or 7, and conferring an increase in the amount of the respective fine chemical, in particular according to table II, application no. 12, column 6 in an organism or a part thereof;
k) nucleic acid molecule encoding the amino acid sequence of a polypeptide encoding a domaine of a polypeptide as indicated in Table II, application no. 12, columns 5 or 7, and conferring an increase in the amount of the respective fine chemical, in particular accccording to table II, application no. 12, column 6 in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 12, columns 5 or 7, or a nucleic acid molecule encoding, preferably at least the mature form of, a polypeptide as indicated in Table II, application no. 12, columns 5 or 7, and conferring an increase in the amount of the respective fine chemical according to table II, application no. 12, column 6 in an organism or a part thereof;
or which encompasses a sequence which is complementary thereto;
whereby, preferably, the nucleic acid molecule according to (a) to (I) distinguishes over the sequence indicated in Table I, application no. 12,columns 5 or 7, by one or more nucleotides. In one embodiment, the nucleic acid molecule does not consist of the sequence shown and indicated in Table I, application no. 12, columns 5 or 7,
In one embodiment, the nucleic acid molecule is less than 100%, 99,999%, 99,99%, 99,9% or 99% identical to a sequence indicated in Table I, application no. 12, columns 5 or 7,.
In another embodiment, the nucleic acid molecule does not encode a polypeptide of a sequence indicated in Table II, application no. 12, columns 5 or 7,.
In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 12, columns 5 or 7,.
In a further embodiment the nucleic acid molecule does not encode a polypeptide sequence as indicated in Table II, application no. 12, columns 5 or 7,.
Accordingly, in one embodiment, the nucleic acid molecule of the differs at least in one or more residues from a nucleic acid molecule indicated in Table I, application no. 12, columns 5 or 7,.
Accordingly, in one embodiment, the nucleic acid molecule of the present invention encodes a polypeptide, which differs at least in one or more amino acids from a polypeptide indicated in Table II, application no. 12, columns 5 or 7,.
In another embodiment, a nucleic acid molecule indicated in Table I, application no. 12, columns 5 or 7,.
Accordingly, in one embodiment, the protein encoded by a sequences of a nucleic acid accoriding to (a) to (I) does not consist of a sequence as indicated in Table II, application no. 12, columns 5 or 7,.
In a further embodiment, the protein of the present invention is at least 30 %, 40 %, 50%, or 60% identical to a protein sequence indicated in Table II, application no. 12, columns 5 or 7, and less than 100%, preferably less than 99,999%, 99,99% or 99,9%, more preferably less than 99%, 985, 97%, 96% or 95% identical to a sequence as indicated in Table I, columns 5 or 7.

**[0205.0.0.12] and [0206.0.0.12]** for the disclosure of the paragraphs [0205.0.0.12] and [0206.0.0.12] see paragraphs [0205.0.0.0] and [0206.0.0.0] above.

**[0207.0.12.12]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the sterol metabolism, the squalen metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.12] to [0226.0.0.12]** for the disclosure of the paragraphs [0208.0.0.12] to [0226.0.0.12] see paragraphs [0208.0.0.0] to [0226.0.0.0] above.

**[0227.0.12.12]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.

In addition to a sequence indicated in Table I, application no. 12, columns 5 or 7, or its derivatives, it is advantageous additionally to express and/or mutate further genes in the organisms. Especially advantageously, additionally at least one further gene of the sterol biosynthetic pathway such as for a phytosterol precursor, for example squalene epoxide is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine one or more of the sequences indicated in Table I, application no. 12, columns 5 or 7, with genes which generally support or enhances to growth or yield of the target organismen, for example genes which lead to faster growth rate of microorganisms or genes which produces stress-, pathogen, or herbicide resistant plants.

**[0228.0.12.12]** In a further embodiment of the process of the invention, therefore, organisms are grown, in which there is simultaneous overexpression of at least one nucleic acid or one of the genes which code for proteins involved in the sterol/phytosterol metabolism, in particular in synthesis of of beta-sitosterol and/or campesterol.

**[0229.0.12.12]** Further advantageous nucleic acid sequences which can be expressed in combination with the sequences used in the process and/or the abovementioned biosynthesis genes are the sequences encoding further genes of the phytosterol biosynthetic pathway, such as the enzymes catalyzing the production of acetyl CoA_HMGCoA, mevalonate, mevalonate 5 phosphate, mevalonate 5-pyrophosphate, isopentyl diphosphate, 5-pyrophosphatemevalonate, isopentyl pyrophosphate (PIP), dimethylallyl pyrophosphate (DMAPP), PIP+DMAPP, geranyl pyrophosphate+IPP, farnesyl pyrophosphate, 2 farnesyl pyrophosphate, squalene (squalene synthase) and squalene epoxide, or cycloartenol synthase controling the cyclization of squalene epoxide, S-adenosyl-L-meth ion ine:sterol C-24 methyl transferase (EC 2.1.1.41) (SMT1) catalyzing the transfer of a methyl group from a cofactor, SMT2 catalyzing the second methyl transfer reaction, sterol C-14 demethylase catalyzing the demethylation at C-14, removing the methyl group and creating a double bond. These genes can lead to an increased synthesis of the essential phytosterol, in particular, of beta-sitosterol and/or campesterol resp..

**[0230.0.0.12]** for the disclosure of this paragraph see paragraph [0230.0.0.0] above.

**[0231.0.12.12]** In a further advantageous embodiment of the process of the invention, the organisms used in the process are those in which simultaneously a protein degrading phytosterol, in particular, of beta-sitosterol and/or campesterol, resp., is attenuated, in particular by reducing the rate of expression of the corresponding gene.

**[0232.0.0.5] to [0276.0.0.5]** for the disclosure of the paragraphs [0232.0.0.5] to [0276.0.0.5] see paragraphs [0232.0.0.0] to [0276.0.0.0] above.

**[0277.0.12.12]** The respective fine chemical produced can be isolated from the organism by methods with which the skilled worker is familiar. For example, via extraction, salt precipitation, and/or different chromatography methods. The process according to the invention can be conducted batchwise, semibatchwise or continuously. The respective fine chemical produced by this process can be obtained by harvesting the organisms, either from the crop in which they grow, or from the field. This can be done via pressing or extraction of the plant parts.

**[0278.0.0.12] to [0282.0.0.12]** for the disclosure of the paragraphs [0278.0.0.12] to [0282.0.0.12] see paragraphs [0278.0.0.0] to [0282.0.0.0] above.

**[0283.0.12.12]** Moreover, a native polypeptide conferring the increase of the respective fine chemical in an organism or part thereof can be isolated from cells (e.g., endothelial cells), for example using the antibody of the present invention as described below, e.g. an antibody against a protein as indicated in Table II, application no. 12, column 3, or an antibody against a polypeptide as indicated in Table II, application no. 12, columns 5 or 7, which can be produced by standard techniques utilizing the polypeptid of the present invention or fragment thereof, i.e., the polypeptide of this invention. Preferred are monoclonal antibodies.

**[0284.0.0.12]** for the disclosure of this paragraph see [0284.0.0.0] above.

**[0285.0.12.12]** In one embodiment, the present invention relates to a polypeptide having a sequence as indicated in Table II, application no. 12, columns 5 or 7, or as encoded by a nucleic acid molecule as indicated in Table I, application no. 12, columns 5 or 7, or functional homologues thereof.

**[0286.0.12.12]** In one advantageous embodiment, in the method of the present invention the activity of a polypeptide is increased which comprises or consists of a consensus sequence as indicated in Table IV, application no. 12, column 7,. In another embodiment, the present invention relates to a polypeptide comprising or consisting of a consensus sequence as indicated in Table IV, application no. 12, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, application no. 12, column 7,.

**[0287.0.0.12] to [0290.0.0.12]** for the disclosure of the paragraphs [0287.0.0.12] to [0290.0.0.12] see paragraphs [0287.0.0.0] to [0290.0.0.0] above.

**[0291.0.12.12]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II, application no. 12, columns 5 or 7, by one or more amino acids. In one embodiment, polypeptide distinguishes form a sequence as indicated in Table II, application no. 12, columns 5 or 7, by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 12, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II, application no. 12, columns 5 or 7,.

**[0292.0.0.12]** for the disclosure of this paragraph see [0292.0.0.0] above.

**[0293.0.12.12]** In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II, application no. 12, columns 5 or 7, by one or more amino acids. In an other embodiment, said polypeptide of the invention does not consist of the sequence as indicated in Table II, application no. 12, columns 5 or 7,.
In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 12, columns 5 or 7.

**[0294.0.12.12]** In one embodiment, the present invention relates to a polypeptide having an activity of a protein as indicated in Table II, application no. 12, column 3, which distinguishes over a sequence as indicated in Table II, application no. 12, columns 5 or 7, by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.12] to [0297.0.0.12]** for the disclosure of the paragraphs [0295.0.0.12] to [0297.0.0.12] see paragraphs [0295.0.0.0] to [0297.0.0.0] above.

**[0297.1.12.12]** Non polypeptide of the invention-chemicals are e.g. polypeptides having not the activity of a polypeptide indicated in Table II, application no. 12, columns 3, 5 or 7.

**[0298.0.12.12]** A polypeptide of the invention can participate in the process of the present invention. The polypeptide or a portion thereof comprises preferably an amino acid sequence, which is sufficiently homologous to an amino acid sequence as indicated in Table II, application no. 12, columns 5 or 7,. The portion of the protein is preferably a biologically active portion as described herein. Preferably, the polypeptide used in the process of the invention has an amino acid sequence identical to a sequence as indicated in Table II, application no. 12, columns 5 or 7,.

**[0299.0.12.12]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, application no. 12, columns 5 or 7,. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 12, or which is homologous thereto, as defined above.

**[0300.0.12.12]** In one embodiment, the present invention relates to a polypeptide having an activity of a protein as indicated in Table II, application no. 12, column 3, which distinguishes over a sequence as indicated in Table II, application no. 12, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0301.0.0.12]** for the disclosure of this paragraph see [0301.0.0.0] above.

**[0302.0.12.12]** Biologically active portions of an polypeptide of the present invention include peptides comprising amino acid sequences derived from the amino acid sequence of the polypeptide of the present invention or used in the process of the present invention, e.g., the amino acid sequence as indicated in Table II, application no. 12, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.12]** for the disclosure of this paragraph see [0303.0.0.0] above.

**[0304.0.12.12]** Manipulation of the nucleic acid molecule of the invention may result in the production of a protein having essentially the activity of the polypeptides as indicated in Table II, application no. 12, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.12], [0306.0.0.12] and [0306.1.0.12]** for the disclosure of the paragraphs [0305.0.0.12], [0306.0.0.12] and [0306.1.0.12] see paragraphs [0305.0.0.0], [0306.0.0.0] and [0306.1.0.0] above.

**[0307.0.0.12] and [0308.0.0.12]** for the disclosure of the paragraphs [0307.0.0.12] and [0308.0.0.12] see paragraphs [0307.0.0.0 and [0308.0.0.0] above.

**[0309.0.12.12]** In one embodiment, a reference to a protein (= polypeptide) of the invention or as indicated in Table II, application no. 12, columns 5 or 7, refers to a polypeptide having an amino acid sequence corresponding to the polypeptide of the invention or used in the process of the invention, whereas a " non-polypeptide of the invention" or "other polypeptide" not being indicated in Table II, application no. 12, columns 5 or 7, refers to a polypeptide having an amino acid sequence corresponding to a protein which is not substantially homologous a polypeptide of the invention, preferably which is not substantially homologous to a polypeptide as indicated in Table II, application no. 12, columns 5 or 7, e.g., a protein which does not confer the activity described herein or annotated or known for as indicated in Table II, application no. 12, column 3, and which is derived from the same or a different organism. In one embodmeint, a "non-polypeptide of the invention" or "other polypeptide" not being indicated in Table II, application no. 12, columns 5 or 7, does not confer an increase of the respective fine chemical in an organism or part therof.

**[0310.0.0.12] to [0334.0.0.12]** for the disclosure of the paragraphs [0310.0.0.12] to [0334.0.0.12] see paragraphs [0310.0.0.0] to [0334.0.0.0] above.

**[0335.0.12.12]** The dsRNAi method has proved to be particularly effective and advantageous for reducing the expression of a nucleic acid sequences as indicated in Table I, application no. 12, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, application no. 12, columns 5 or 7, and/or homologs thereof. In a double-stranded RNA molecule for reducing the expression of an protein encoded by a nucleic acid sequence sequences as indicated in Table I, application no. 12, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.12] to [0342.0.0.12]** for the disclosure of the paragraphs [0336.0.0.12] to [0342.0.0.12] see paragraphs [0336.0.0.0] to [0342.0.0.0] above.

**[0343.0.12.12]** As has already been described, 100 % sequence identity between the dsRNA and a gene transcript of a nucleic acid sequence as indicated in Table I, application no. 12, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 12, columns 5 or 7, or homologs thereof of the one organism, may be used to suppress the corresponding expression in another organism.

**[0344.0.0.12] to [0361.0.0.12]** for the disclosure of the paragraphs [0344.0.0.12] to [0361.0.0.12] see paragraphs [0344.0.0.0] to [0361.0.0.0] above.

**[0362.0.12.12]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 12, columns 5 or 7, e.g. encoding a polypeptide having protein activity, as indicated in Table II, application no. 12, column 3,. Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, application no. 12, columns 5 or 7, means herein that due to modulation or manipulation of the genome, an activity as annotated for a polypeptide as indicated in Table II, application no. 12, column 3, e.g. having a sequence as indicated in Table II, application no. 12, columns 5 or 7, is increased in a cell or an organism or a part thereof. Examples are described above in context with the process of the invention

**[0363.0.0.12]** for the disclosure of this paragraph see paragraph [0363.0.0.0] above.

**[0364.0.12.12]** A naturally occurring expression cassette - for example the naturally occurring combination of a promoter of a polypeptide of the invention as indicated in Table II, application no. 12, column 3, with the corresponding protein-encoding sequence as indicated in Table I, application no. 12, column 3, - becomes a transgenic expression cassette when it is modified by non-natural, synthetic "artificial" methods such as, for example, mutagenization. Such methods have been described (US 5,565,350; WO 00/15815; also see above).

**[0365.0.0.12] to [0373.0.0.12]** for the disclosure of the paragraphs [0365.0.0.12], to [0373.0.0.12] see paragraphs [0365.0.0.0] to [0373.0.0.0] above.

**[0374.0.12.12]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. Phytosterol, in particular, of beta-sitosterol and/or campesterol resp., produced in the process according to the invention may, however, also be isolated from the plant in the form of their free form or bound in or to compounds or moieties. Phytosterol, in particular, of beta-sitosterol and/or campesterol resp., produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.12] and [0376.0.0.12]** for the disclosure of the paragraphs [0375.0.0.12] and [0376.0.0.12] see paragraphs [0375.0.0.0] and [0376.0.0.0] above.

**[0377.0.12.12]** Accordingly, the present invention relates also to a process according to the present invention whereby the produced phytosterol, in particular, of beta-sitosterol and/or campesterol comprising composition or the produced the respective fine chemical is isolated.

**[0378.0.12.12]** In this manner, more than 50% by weight, advantageously more than 60% by weight, preferably more than 70% by weight, especially preferably more than 80% by weight, very especially preferably more than 90% by weight, of the phytosterol, in particular, of beta-sitosterol and/or campesterol, resp., produced in the process can be isolated. The resulting phytosterol, in particular, of beta-sitosterol and/or campesterol resp., can, if appropriate, subsequently be further purified, if desired mixed with other active ingredients such as vitamins, amino acids, carbohydrates, antibiotics and the like, and, if appropriate, formulated.

**[0379.0.12.12]** In one embodiment, the phytosterol, in particular, of beta-sitosterol and/or campesterol resp., is a mixture comprising of one or more the respective fine chemicals. In one embodiment, the respective fine chemical means here phytosterol, in particular, of beta-sitosterol and/or campesterol. In one embodiment, phytosterol means here a mixture of the respective fine chemicals.

**[0380.0.12.12]** The phytosterol, in particular, the beta-sitosterol and/or campesterol resp., obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the phytosterol comprising composition produced or the respective fine chemical produced if desired and formulating the product with a pharmaceutical acceptable carrier or formulating the product in a form acceptable for an application in agriculture. A further embodiment according to the invention is the use of the phytosterol resp., produced in the process or of the transgenic organisms in animal feeds, foodstuffs, medicines, food supplements, cosmetics or pharmaceuticals.

**[0381.0.0.12] and [0382.0.0.12]** for the disclosure of the paragraphs [0381.0.0.12] and [0382.0.0.12] see paragraphs [0381.0.0.0] and [0382.0.0.0] above.

**[0383.0.12.12]** For preparing sterol compound-containing fine chemicals, in particular the respective fine chemical of the invention, it is possible to use as source organic compounds such as, for example, acetyl CoA_HMGCoA, mevalonate, mevalonate 5 phosphate, mevalonate 5-pyrophosphate, isopentyl diphosphate, 5-pyrophosphatemevalonate, isopentyl pyrophosphate (PIP), dimethylallyl pyrophosphate (DMAPP), PIP+DMAPP, geranyl pyrophosphate+IPP, farnesyl pyrophosphate, 2 farnesyl pyrophosphate, squalene and squalene epoxide, cycloartenol and for preparing esters comprising the phytosterols of the invention oils, fats and/or lipids comprising fatty acids such as fatty acids having a carbon back bone between C₁₀- to C₁₆- carbon atoms and/or small organic acids such acetic acid, propionic acid or butanoic acid as precursor compounds.

**[0384.0.0.12]** for the disclosure of this paragraph see paragraph [0384.0.0.0] above.

**[0385.0.12.12]** The fermentation broths obtained in this way, containing in particular phytosterol, in particular, of beta-sitosterol and/or campesterol resp., in mixtures with other compounds, in particular with other sterols or vitamins, e.g. with carotenoids, e.g. with astaxanthin, or fatty acids or containing microorganisms or parts of microorganisms, like plastids, containing phytosterol, in particular, of beta-sitosterol and/or campesterol resp., in mixtures with other compounds, e.g. with vitamins, normally have a dry matter content of from 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 3 g/I during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.
As phytosterol is often localized in membranes or plastids, in one embodiment it is advantageous to avoid a leaching of the cells when the biomass is isolated entirely or partly by separation methods, such as, for example, centrifugation, filtration, decantation, coagulation/flocculation or a combination of these methods, from the fermentation broth. The dry biomass can directly be added to animal feed; provided the vitamin E concentration is sufficiently high and no toxic compounds are present.

**[0386.0.12.12]** Accordingly, it is possible to further purify the produced phytosterol, in particular, of beta-sitosterol and/or campesterol resp.. For this purpose, the product-containing composition, e.g. a total or partial lipid extraction fraction using organic solvents, e.g. as described above, is subjected for example to a saponification to remove triglycerides, partition between e.g. hexane/methanol (seperation of non-polar epiphase from more polar hypophasic derivates) and separation via e.g. an open column chromatography, preparative thin layer chromatography or HPLC in which case the desired product or the impurities are retained wholly or partly on the chromatography resin (see e.g. Kaluzny et al., J Lipid Res 1985; 26: 135-140). These chromatography steps can be repeated if necessary, using the same or different chromatography resins. The skilled worker is familiar with the choice of suitable chromatography resins and their most effective use.

**[0387.0.0.12] to [0392.0.0.12]** for the disclosure of the paragraphs [0387.0.0.12] to [0392.0.0.12] see paragraphs [0387.0.0.0] to [0392.0.0.0] above.

**[0393.0.12.12]** In one embodiment, the present invention relates to a method for the identification of a gene product conferring an increase in the fine chemical production in a cell, comprising the following steps:
a) contacting, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
b) identifying the nucleic acid molecules, which hybridize under relaxed stringent conditions with the nucleic acid molecule of the present invention in particular to the nucleic acid molecule sequence as indicated in Table I, application no. 12, columns 5 or 7, and, optionally, isolating the full length cDNA clone or complete genomic clone;
c) introducing the candidate nucleic acid molecules in host cells, preferably in a plant cell or a microorganism, appropriate for producing the fine chemical;
d) expressing the identified nucleic acid molecules in the host cells;
e) assaying the the fine chemical level in the host cells; and
f) identifying the nucleic acid molecule and its gene product which expression confers an increase in the the fine chemical level in the host cell after expression compared to the wild type.

**[0394.0.0.12] to [0399.0.0.12]** for the disclosure of the paragraphs [0394.0.0.12] to [0399.0.0.12] see paragraphs [0394.0.0.0] to [0399.0.0.0] above.

**[0399.1.12.12]**One can think to screen for increased production of the respective fine chemical by for example searching for a resistance to a drug blocking the synthesis of the respective fine chemical and looking whether this effect is dependent on the activity or expression of a polypeptide as indicated in Table II, application no. 12, columns 5 or 7, or a homolog thereof, e.g. comparing the phenotyp of nearly identical organisms with low and high activity of a protein as indicated in Table II, application no. 12, columns 5 or 7, after incubation with the drug.

**[0400.0.0.12] to [0416.0.0.12]** for the disclosure of the paragraphs [0400.0.0.12] to [0416.0.0.12] see paragraphs [0400.0.0.0] to [0416.0.0.0] above.

**[0417.0.12.12]**The nucleic acid molecule of the invention, the vector of the invention or the nucleic acid construct of the invention may also be useful for the production of organisms resistant to inhibitors of the phytosterol production biosynthesis pathways. In particular, the overexpression of the polypeptide of the present invention may protect an organism such as a microorganism or a plant against inhibitors, which block the phytosterol, in particular the respective fine chemical, synthesis in said organism. Examples of inhibitors or herbicides blocking the phytosterol synthesis in organism such as microorganism or plants are for example compounds which inhibit the cytochrom P450 such as Tetcyclasis, triazoles like Paclobutrazol or Epoxiconazol, pyridines like Obtusifoliol, demethylases inhibitors, or compounds like Mevilonin, which inhibits the HMG-CoA reductase.

**[0418.0.0.12] to [0423.0.0.12]** for the disclosure of the paragraphs [0418.0.0.12] to [0423.0.0.12] see paragraphs [0418.0.0.0] to [0423.0.0.0] above.

**[0424.0.12.12]** Accordingly, the nucleic acid of the invention, the polypeptide of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other sterols, phytosterols, carotenoids, vitamins or fatty acids. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.12] to [0430.0.0.12]** for the disclosure of the paragraphs [0425.0.0.12] to [0430.0.0.12] see paragraphs [0425.0.0.0] to [0430.0.0.0] above.

**[0431.0.12.12]** Example 1: Cloning SEQ ID NO: 12135 in Escherichia coli

**[0432.0.12.12]** SEQ ID NO: 12135 was cloned into the plasmids pBR322 (Sutcliffe, J.G. (1979) Proc. Natl Acad. Sci. USA, 75: 3737-3741); pACYC177 (Change & Cohen (1978) J. Bacteriol. 134: 1141-1156); plasmids of the pBS series (pBSSK+, pBSSK- and others; Stratagene, LaJolla, USA) or cosmids such as SuperCos1 (Stratagene, LaJolla, USA) or Lorist6 (Gibson, T.J. Rosenthal, A., and Waterson, R.H. (1987) Gene 53: 283-286) for expression in E. coli using known, well-established procedures (see, for example, Sambrook, J. et al. (1989) "Molecular Cloning: A Laboratory Manual". Cold Spring Harbor Laboratory Press or Ausubel, F.M. et al. (1994) "Current Protocols in Molecular Biology", John Wiley & Sons).

**[0433.0.0.12] and [0434.0.0.12]** for the disclosure of the paragraphs [0433.0.0.12] and [0434.0.0.12] see paragraphs [0433.0.0.0] and [0434.0.0.0] above.

**[0435.0.12.12]** Example 3: In-vivo and in-vitro mutagenesis

**[0436.0.12.12]** An *in vivo* mutagenesis of organisms such as green algae (e.g. Spongiococcum sp, e.g. Spongiococcum exentricum, Chlorella sp., Haematococcus, Phaedactylum tricornatum, Volvox or Dunaliella), Synchocytic spec. PLL 6803, Physocmetrella patens, Saccharomyces, Mortierella, Escherichia and others mentioned above, which are beneficial for the production of phytosterol can be carried out by passing a plasmid DNA (or another vector DNA) containing the desired nucleic acid sequences, e.g. the nucleic acid molecule of the invention or the vector of the invention, or nucleic acid sequences through E. coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.5.12] to [0440.0.5.12] and [0441.0.0.12]** for the disclosure of the paragraphs [0437.0.5.12] to [0440.0.5.12] and [0441.0.0.12] see paragraphs [0437.0.5.5] to [0444.0.5.5] and [0441.0.0.0]above.

**[0442.0.5.12], [0443.0.0.12], [0444.0.5.12] and [0445.0.5.12]** for the disclosure of the paragraphs [0442.0.5.12], [0443.0.0.12], [0444.0.5.12] and [0445.0.5.12] see paragraphs [0442.0.5.5], [0443.0.0.0], [0444.0.5.5] and [0445.0.5.5] above.

**[0446.0.0.12] to [0450.0.0.12] and [0451.0.5.12]** for the disclosure of the paragraphs [0446.0.0.12] to [0450.0.0.12] and [0451.0.5.12] see paragraphs [0446.0.0.0] to [0450.0.0.0] and [0451.0.5.5] above.

**[0452.0.0.12] to [0454.0.0.12], [0455.0.5.12] and [0456.0.0.12]** for the disclosure of the paragraphs [0452.0.0.12] to [0454.0.0.12], [0455.0.5.12] and [0456.0.0.12] see [0452.0.0.0] to [0454.0.0.0], [0455.0.5.5] and [0456.0.0.0] above.

**[0457.0.12.12]** Example 9: Purification of the phytosterol

**[0458.0.12.12]** One example is the analysis of phytosterol: the content of the phytosterols of the invention can be determinated by gas chromatography with flame
ionisation detection (GC-FID; column SAC-5, 30 m x 0.25 mm, 0.25 µm, samples not silylated) using standards for these phytosterols. Another method is the detection by gas chromatography-mass spectrometry (GC-MS) using the same type of column as indicated above.
For the analysis of the concentrations of sterols by gas chromatography mass spectrometry a Hewlett-Packard (HP) 5890 gas chromatograph equipped with an NB-54 fused-silica capillary column (15 m×0.20mm I.D.; Nordion, Helsinki, Finland) and interfaced with an HP 5970A mass spectrometry detector operating in electron impact mode (70 eV) can be used. The column oven is programmed from 230 °C to 285 °C at 10 °C/min and injector and detector should be at 285 °C. The lipids from the samples (200 µl) are extracted with chloroform/methanol (2:1) and transesterified with sodium methoxide. The released free sterols are trimethylsilylated as described previously (Gylling et al. J. Lipid Res 40: 593-600, 1999) and quantified by single ion monitoring technique using m/z 129 (cholesterol, campesterol and β-sitosterol), m/z 215 (β-sitostanol), m/z 343 (desmosterol), m/z 255 (lathosterol) and m/z 217 (5-α-cholestane, internal standard) as selected ions (Vaskonen, Dissertation, Biomedicum Helsinki, June 19, 2002).

**[0459.0.12.12]** If required and desired, further chromatography steps with a suitable resin may follow. Advantageously, the phytosterol, in particular, of beta-sitosterol and/or campesteroll, can be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.0.12]** for the disclosure of this paragraph see [0460.0.0.0] above.

**[0461.0.12.12]** Example 10: Cloning SEQ ID NO: 268 for the expression in plants

**[0462.0.0.12]** for the disclosure of this paragraph see [0462.0.0.0] above.

**[0463.0.12.12]** SEQ ID NO: 268 is amplified by PCR as described in the protocol of the Pfu Turbo or DNA Herculase polymerase (Stratagene).

**[0464.0.0.12] to [0466.0.0.12]** for the disclosure of the paragraphs [0464.0.0.12] to [0466.0.0.12] see paragraphs [0464.0.0.0] to [0466.0.0.0] above.

**[0467.0.12.12]** The following primer sequences were selected for the gene SEQ ID NO: 268:
i) forward primer: SEQ ID NO: 274
ii) reverse primer: SEQ ID NO: 275

**[0468.0.0.12] to [0479.0.0.12]** for the disclosure of the paragraphs [0468.0.0.12] to [0479.0.0.12] see paragraphs [0468.0.0.0] to [0479.0.0.0] above.

**[0480.0.12.12]** Example 11: Generation of transgenic plants which express SEQ ID NO: 268

**[0481.0.0.12] to [0513.0.0.12]** for the disclosure of the paragraphs [0481.0.0.12] to [0513.0.0.12] see paragraphs [0482.0.0.0] to [0513.0.0.0] above.

**[0514.0.12.12]** As an alternative, phytosterols can be detected via HPLC, e.g. reversed-phase HPLC, as described by Heftmann, E. and Hunter, I.R. (J Chromatogr 1979; 165: 283-299). As separating principles of HPLC and GC are complementary, preparative reversed-phase HPLC followed by GC-MS analysis of the obtained sterol fractions is a preferred method to analyze sterols from natural products (Bianchini, J.-P. et al.; J Chromatogr 1985; 329: 231-246).

**[0515.0.0.12] to [0552.0.0.12]** for the disclosure of the paragraphs [0515.0.0.12] to [0552.0.0.12] see paragraphs [0515.0.0.0] to [0552.0.0.0] above.

**[0553.0.12.12]** A process for the production of beta-Sitosterol and/or Campesterol resp., which comprises
(a) increasing or generating the activity of a protein as indicated in Table II, application no. 12, columns 5 or 7, or a functional equivalent thereof in a non-human organism, or in one or more parts thereof; and
(b) growing the organism under conditions which permit the production of beta-Sitosterol and/or Campesterol resp. in said organism.
A process for the production of beta-Sitosterol and/or Campesterol resp., comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 12, columns 5 or 7, , or a fragment thereof, which confers an increase in the amount of the fine chemical as indicated in table II, column 6, e.g of beta-Sitosterol and/or Campesterol resp., in an organism or a part thereof;
b) nucleic acid molecule comprising of a nucleic acid molecule as indicated in Table I, application no. 12, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of beta-Sitosterol and/or Campesterol resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of beta-Sitosterol and/or Campesterol resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of beta-Sitosterol and/or Campesterol resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 12, column 7, and conferring an increase in the amount of the fine chemical as indicated in table II, column 6, e.g of beta-Sitosterol and/or Campesterol resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of beta-Sitosterol and/or Campesterol resp., in an organism or a part thereof;
h) nucleic acid molecule encoding a polypeptide comprising a consensus sequence as indicated in Table IV, application no. 12, column 7, and conferring an increase in the amount of the fine chemical as indicated in table II, column 6, e.g of beta-Sitosterol and/or Campesterol resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical as indicated in table II, column 6, e.g of beta-Sitosterol and/or Campesterol resp., in an organism or a part thereof.
or comprising a sequence which is complementary thereto.
The process as mentioned above, comprising recovering of the free or bound beta-Sitosterol and/or Campesterol resp..
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound beta-Sitosterol and/or Campesterol resp., produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 12, columns 5 or 7, or a fragment thereof, which confers an increase in the amount of the fine chemical as indicated in table II, column 6, e.g of beta-Sitosterol and/or Campesterol resp., in an organism or a part thereof;
b) nucleic acid molecule comprising of a nucleic acid molecule as indicated in Table I, application no. 12, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of beta-Sitosterol and/or Campesterol resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of beta-Sitosterol and/or Campesterol resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of beta-Sitosterol and/or Campesterol resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 12, column 7, and conferring an increase in the amount of the fine chemical as indicated in table II, column 6, e.g beta-Sitosterol and/or Campesterol resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of beta-Sitosterol and/or Campesterol resp., in an organism or a part thereof;
h) nucleic acid molecule encoding a polypeptide comprising a consensus sequence as indicated in Table IV, application no. 12, column 7, and conferring an increase in the amount of the fine chemical as indicated in table II, column 6, e.g of beta-Sitosterol and/or Campesterol resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemiccal as indicated in table II, column 6, e.g of beta-Sitosterol and/or Campesterol resp., in an organism or a part thereof.
whereby the nucleic acid molecule distinguishes over the sequence as indicated in Table I, application no. 12, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 12, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of beta-Sitosterol and/or Campesterol resp., in an organism or a part thereof comprising:
(a) contacting cells, tissues , plants or microorganisms which express the a polypeptide encoded by the nucleic acid molecule as mentioned above conferring an increase in the amount of beta-Sitosterol and/or Campesterol resp., in an organism or a part thereof with a candidate compound or a sample comprising a plurality of compounds under conditions which permit the expression the polypeptide;
(b) assaying the beta-Sitosterol and/or Campesterol resp., level or the polypeptide expression level in the cell, tissue, plant or microorganism or the media the cell, tissue, plant or microorganisms is cultured or maintained in; and
(c) identifying a agonist or antagonist by comparing the measured beta-Sitosterol and/or Campesterol resp., level or polypeptide expression level with a standard beta-Sitosterol and/or Campesterol resp., or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased beta-Sitosterol and/or Campesterol resp., production in a plant or microorganism, comprising the steps:
(a) culturing a plant cell or tissue or microorganism or maintaining a plant expressing the polypeptide encoded by the nucleic acid molecule as mentioned above conferring an increase in the amount of beta-Sitosterol and/or Campesterol resp., in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of beta-Sitosterol and/or Campesterol rersp., in an organism or a part thereof;
(b) identifying if the compound is an effective agonist by detecting the presence or absence or increase of a signal produced by said readout system.
A method for the identification of a gene product conferring an increase in beta-Sitosterol and/or Campesterol resp., production in a cell, comprising the following steps:
(a) contacting the nucleic acid molecules of a sample, which can contain a candidate gene encoding a gene product conferring an increase in beta-Sitosterol and/or Campesterol resp., after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing beta-Sitosterol and/or Campesterol resp.;
(d) expressing the identified nucleic acid molecules in the host cells;
(e) assaying the beta-Sitosterol and/or Campesterol rersp., level in the host cells; and
(f) identifying nucleic acid molecule and its gene product which expression confers an increase in the beta-Sitosterol and/or Campesterol resp., level in the host cell in the host cell after expression compared to the wild type.
A method for the identification of a gene product conferring an increase in beta-Sitosterol and/or Campesterol resp., production in a cell, comprising the following steps:
(a) identifiying in a data bank nucleic acid molecules of an organism; which can contain a candidate gene encoding a gene product conferring an increase in the beta-Sitosterol and/or Campesterol resp., amount or level in an organism or a part thereof after expression, and which are at least 20% homolog to the nucleic acid molecule as mentioned above;
(b) introducing the candidate nucleic acid molecules in host cells appropriate for producing beta-Sitosterol and/or Campesterol resp.,;
(c) expressing the identified nucleic acid molecules in the host cells;
(d) assaying the beta-Sitosterol and/or Campesterol resp., level in the host cells; and
(e) identifying nucleic acid molecule and its gene product which expression confers an increase in the beta-Sitosterol and/or Campesterol resp., level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of beta-Sitosterol and/or Campesterol resp., after expression.
Use of the polypeptide as mentioned above or the nucleic acid construct as mentioned above or the gene product identified according to the method as mentioned above for identifying compounds capable of conferring a modulation of beta-Sitosterol and/or Campesterol resp., levels in an organism.
Food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the protection of a plant against a beta-Sitosterol and/or Campesterol synthesis inhibiting herbicide.

### Process for the production of fine chemicals

### Description

**[0000.0.0.13]** In a further embodiment, the present invention relates to a further process for the production of fine chemicals as defined below and corresponding embodiments as described herein as follows.

**[0001.0.0.13]: see [0001.0.0.0]**

**[0002.0.13.13]:** see **[0003.0.7.7]**

**[0003.0.13.13]** Straight- or normal-chain (even-numbered), monoenoic components, i.e. with one double bond, make up a high proportion of the total fatty acids in most natural lipids. Normally the double bond is of the *cis*- or Z-configuration, although some fatty acids with *trans-* or *E*-double bonds are known. The most abundant monoenoic fatty acids in animal and plant tissues are straight-chain compounds with 16 or 18 carbon atoms, but analogous fatty acids with 10 to 36 carbon atoms have been found in nature in esterified form. Very long-chain (20:1 upwards) cis-monoenoic fatty acids have relatively high melting points, but the more common C₁₈ monoenes tend to be liquid at room temperature. Triacylglycerols (or oils and fats) containing high proportions of monoenoic fatty acids are usually liquid at ambient temperature. Analogous fatty acids with *trans* double bonds are normally higher melting. Very-long-chain monoenoic fatty acids of the (*n*-9) family occur in a variety of natural sources, often accompanied by analogous fatty acids of the (*n*-7) family), especially in animal tissues. For example, monoenes from 20:1 to 26:1 are normal constituents of animal sphingolipids. Odd-numbered very-long chain monoenes (23:1 upwards) from brain belong to (*n*-8) and (*n*-1 0) families, presumably because they are formed by chain-elongation of 9-17:1 (17:1 (*n*-8)) and 9-19:1 (19:1 (*n*-10)), respectively (see below). An even wider range of chain-lengths is found in monoenes from plant waxes and sponge lipids.
In animals and yeasts, stearoyl-CoA is converted directly to oleoyl-CoA by a concerted removal of hydrogen atoms from carbons 9 and 10 (*D*-stereochemistry in each instance). Subsequently, oleate can be chain elongated by two carbon atoms to give longer-chain fatty acids of the (*n*-9) family, while palmitoleate is the precursor of the (*n-*7) family of fatty acids. Certain bacteria produce mono-unsaturated fatty acids by an anaerobic mechanism that involves the fatty acid synthetase. During the fourth cycle of chain elongation, a branch point occurs in fatty acid synthesis following the dehydrase step. Chain elongation can proceed as normal, or an isomerase can convert the *trans-*2-decanoyl-ACP too cis-3-decanoyl-ACP. The latter is not a substrate for the enoyl-ACP reductase, but it can be further elongated with eventual formation of a *cis*-11-18:1 fatty acid. For many years, this was thought to be the major pathway for biosynthesis of unsaturated fatty acids in bacteria, but it is now recognised that it is restricted to a few proteobacteria, such as *E. coli.* Aerobic mechanisms certainly exist, but other mechanisms and enzymes have yet to be adequately characterized for most bacterial species.

**[0004.0.13.13]** Principally microorganisms such as Mortierella or oil producing plants such as soybean, rapeseed or sunflower or algae such as Crytocodinium or Phaeodactylum are a common source for oils containing fatty acids, where they are usually obtained in the form of their triacyl glycerides. Alternatively, they are obtained advantageously from animals, such as fish. The free fatty acids are prepared advantageously by hydrolysis with a strong base such as potassium or sodium hydroxide.

**[0005.0.13.13]** ./.

**[0006.0.13.13]** 11-cis-Eicosenoic acid (gadoleic acid) is a common if minor constituent of animal tissues and fish oils, often accompanied by the 13-isomer. It is also found in rapeseed oil and seed oils of related species. *cis*-5-20:1 can amount to 67% of the total fatty acids in meadowfoam oil.

**[0007.0.13.13]** *cis*-*Monoenoic* acids obviously have desirable physical properties for membranes lipids, and they are now recognised by nutritionists as being beneficial in the human diet.

**[0008.0.13.13]** The *exception* is erucic acid as there is evidence from studies with laboratory rats that it may adversely affect the metabolism of the heart. Erucic acid is used in the manufacture of industrial oils, e.g. for production of polyethylene film.

**[0009.0.13.13]** As described above, *cis*-Monoenoic acids are used in a lot of different applications, for example in cosmetics, pharmaceuticals and in feed and food.

**[0010.0.13.13]** Therefore improving the productivity of such *cis*-Monoenoic acids and improving the quality of foodstuffs and animal feeds is an important task of the different industries.

**[0011.0.13.13]** To ensure a high productivity of certain *cis*-Monoenoic acids in plants or microorganism, it is necessary to manipulate the natural biosynthesis of fatty acids in said organism.

**[0012.0.13.13]** Accordingly, there is still a great demand for new and more suitable genes which encode enzymes or other regulators which participate in the biosynthesis of *cis*-Monoenoic acids and make it possible to produce certain *cis*-Monoenoic acids specifically on an industrial scale without unwanted byproducts forming. In the selection of genes for biosynthesis two characteristics above all are particularly important. On the one hand, there is as ever a need for improved processes for obtaining the highest possible contents of *cis*-Monoenoic acids on the other hand as less as possible byproducts should be produced in the production process.

**[0013.0.0.13] see [0013.0.0.0]**

**[0014.0.13.13]** It was found that the overexpression of the nucleic acid molecule characterized herein confers an increase in the content of Eicosenic acid (20:1) in plants. Accordingly, in a first embodiment, the invention relates to a process for the production of Eicosenic acid. In Arabidopsis thaliana, Eicosenic acid is predominately found in the stereoisomeric form 20:1 delta 9c (gadoleic acid). Accordingly, in a further embodiment, the invention relates to a process for the production of a fine chemical, whereby the fine chemical is gadoleic acid or tryglycerides, lipids, oils or fats containing gadoleic acid. Accordingly, in the present invention, the term "the fine chemical" as used herein relates to "gadoleic acid and/or tryglycerides, lipids, oils and/or fats containing gadoleic acid". Further, the term "the fine chemicals" as used herein also relates to fine chemicals comprising gadoleic acid and/or triglycerides, lipids, oils and/or fats containing gadoleic acid.

Advantageously, the increase of the Eicosenic acid (20:1 fatty acid) content, e.g. the gadoleic acid content, in a composition produced according to the process of the invention results in an incrase in the melting point of the composition, in particular if the composition is a fatty composition as a oil or a wax. As other monoenoic fatty acids, eicosenic acid, in particular gadoleic acid, may be used as anti-foaming agent in detergents or as an anti-blocking agent in the production of platics or used preservation agent, flouering agent, plastic softener, formulation agent, flotation agent, wetting agent emulsifer agend and/or lubricating agents, as e.g. erucic acid, arachinic acid, pelagonic acid, brassylic acid or erucic acid amidses. Furthermore, monounsaturated fatty acids as eicosenic acid, in particular gadolenic acid, are cholesterol lowering when they replace significant levels of saturated fatty acids in the diet. Some studies have found that diets high in monounsaturated fatty acids compared with polyunsaturated fatty acids decrease LDL cholsterol while maintaining HDL cholesterol levels. However, other studies suggested that the effect of consuming polyunsaturated fat and monounsaturated fat is similar and results in a decrease in both LDL and HDL cholesterol (see e.g. homepage of the Instiute of Shorting and Edible Oils for references).

**[0015.0.13.13]** In one embodiment, the term "the respective fine chemical" means gadoleic acid, eicosenic acid, C20:1 fatty acidand/or tryglycerides, lipids, oils and/or fats containing gadoleic acid. Throughout the specification the term "the respective fine chemical" means gadoleic acid and/or tryglycerides, lipids, oils and/or fats containing gadoleic acid, gadoleic acid and its salts, ester, thioester or gadoleic acid in free form or bound to other compounds such as triglycerides, glycolipids, phospholipids etc. In a preferred embodiment, the term "the respective fine chemical" means gadoleic acid, in free form or its salts or bound to triglycerides. Triglycerides, lipids, oils, fats or lipid mixture thereof shall mean any triglyceride, lipid, oil and/or fat containing any bound or free gadoleic acid for example sphingolipids, phosphoglycerides, lipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride or other fatty acid esters such as acetyl-Coenzym A thioester, which contain further saturated or unsaturated fatty acids in the fatty acid molecule.

**[0016.0.13.13]** Accordingly, the present invention relates to a process for the production of the respective fine chemical comprising
(a) increasing or generating the activity of one or more
   of a protein as shown in table II, application no. 13, column 3 encoded by the nucleic acid sequences as shown in table I, application no. 13, column 5, in a non-human organism or in one or more parts thereof or
(b) growing the organism under conditions which permit the production of the fine chemical, thus the acid of the invention or fine chemicals comprising the acid of the invention, in said organism or in the culture medium surrounding the organism.

**[0017.0.0.13]** to **[0019.0.0.13]: see [0017.0.0.0]** to **[0019.0.0.0]**

**[0020.0.13.13]** Surprisingly it was found, that the transgenic expression of the Brassica napus protein as indicated in Table II, application no. 13, column 5, line 21 in a plant conferred an increase in C20:1 fatty acid, putative content of the transformed plants. Thus, in one embodiment, said protein or its homologs are used for the production of C20:1 fatty acid, putative.

**[0021.0.0.13] see [0021.0.0.0]**

**[0022.0.13.13]** The sequence of YDR513W from Saccharomyces cerevisiae has been published in Jacq et al., Nature 387 (6632 Suppl), 75-78 (1997) and in Goffeau et al., Science 274 (5287), 546-547, 1996 and its cellular activity has characterized as glutathione reductase. Accordingly, in one embodiment, the process of the present invention comprises the use of glutathione reductase or a protein of the glutaredoxin superfamily for the production of gadoleic acid. Accordingly, in one embodiment, the process of the present invention comprises the use of YDR513W, from Saccharomyces cerevisiae, e.g. as indicated herein in Table II, line 125, columns 3 or 5, or its homologue, e.g. as shown herein in Table II, line 125, column 7, for the production of the respective fine chemical, meaning of gadoleic acid and/or tryglycerides, lipids, oils and/or fats containing gadoleic acid, in particular for increasing the amount of gadoleic acid and/or tryglycerides, lipids, oils and/or fats containing gadoleic acid, preferably gadoleic acid in free or bound form in an organism or a part thereof, as mentioned. In one embodiment, in the process of the present invention the activity of a glutathione reductase is increased or generated, e.g. from Saccharomyces cerevisiae or a plant or a homolog thereof.

**[0022.1.0.13]** to **[0023.0.0.13]: see [0022.1.0.0]** to **[0023.0.0.0]**

**[0023.1.13.13]**Homologs of the polypeptide disclosed in table II, application no. 13, column 5, may have the activity of a protein as disclosed in table I or II, application no. 13, column 3 and may be the polypetides encoded by the nucleic acid molecules indicated in table I , application no. 13, column 7, resp., or may be the polypeptides indicated in table II, application no. 13, column 7, resp.

**[0024.0.0.13] see [0024.0.0.0]**

**[0025.0.13.13]** In accordance with the invention, a protein or polypeptide has the "activity of an protein as shown in table II, application no. 13, column 3 "if its de *novo* activity, or its increased expression directly or indirectly leads to an increased in the level of the fine chemical indicated in the respective line of table II, application no. 13, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 13, column 3, or which has at least 10% of the original enzymatic or biological activity, preferably 20%, particularly preferably 30%, most particularly preferably 40% in comparison to a protein as shown in the respective line of table II, application no. 13, column 3 of a E. coli or Saccharomyces cerevisae, respectively, protein as mentioned in table I to IV, column 3 respectively and as disclosed in paragraph [0022] of the respective invention.

**[0025.1.0.13] see [0025.1.0.0]**

**[0026.0.0.13] to [0033.0.0.13]: see [0026.0.0.0] to [0033.0.0.0]**

**[0034.0.13.13]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 13, column 3 its biochemical or genetical causes and the increased amount of the respective fine chemical.

**[0035.0.0.13] to [0044.0.0.13]: see [0035.0.0.0] to [0044.0.0.0]**

**[0045.0.13.13]** In one embodiment, the activity of the protein. as indicated in Table II, columns 5 or 7, application no. 13, is increased conferring an increase of the respective fine chemical, indicated in Table II, application no. 13, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more

**[0046.0.13.13]**In one embodiment, the activity of the protein as indicated in Table II, columns 5 or 7, application no. 13, is increased conferring an increase of the respective fine chemical, indicated in Table II, application no. 13, column 6 of the respective line confers an increase of the respective fine chemical and of further acids, like erucic acid or their precursors.

**[0047.0.0.13] to [0048.0.0.13]: see [0047.0.0.0] to [0048.0.0.0]**

**[0049.0.13.13]A** protein having an activity conferring an increase in the amount or level of the fine chemical, has in one embodiment the structure of the polypeptide described herein, in particular of the polypeptides comprising the consensus sequence shown in table IV, application no. 13, column 7 or of the polypeptide as shown in the amino acid sequences as disclosed in table II, application no. 13, columns 5 and 7 or the functional homologues thereof as described herein, or is encoded by the nucleic acid molecule characterized herein or the nucleic acid molecule according to the invention, for example by the nucleic acid molecule as shown in table I, application no. 13, columns 5 and 7 or its herein described functional homologues and has the herein mentioned activity.

**[0050.0.13.13]** For the purposes of the present invention, the term "gadoleic acid" also encompasses the corresponding salts, such as, for example, the potassium or sodium salts of gadoleic acid or the salts of gadoleic acid with amines such as diethylamine.

**[0051.0.13.13]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g monoenoic fatty acid compositions. Depending on the choice of the organism used for the process according to the present invention, for example a microorganism or a plant, compositions or mixtures of various monoenoic fatty acid can be produced.

**[0052.0.0.13]: see [0052.0.0.0]**

**[0053.0.13.13]** In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 13, columns 5 and 7 or its homologs activity having herein-mentioned 229,230 of the invention increasing activity; and/or
b) stabilizing a mRNA conferring the increased expression of a protein encoded by the nucleic acid molecule of the invention as shown in table I, application no. 13, columns 5 and 7, e.g. a nucleic acid sequence encoding a polypeptide having the activity of a protein as indicated in table II, application no. 13, columns 5 and 7 or its homologs activity or of a mRNA encoding the polypeptide of the present invention having herein-mentioned 229,230of the invention increasing activity; and/or
c) increasing the specific activity of a protein conferring the increased expression of a protein encoded by the nucleic acid molecule of the invention or of the polypeptide of the present invention having herein-mentioned 229,230 increasing activity, e.g. of a polypeptide having the activity of a protein as indicated in table II, application no. 13, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned 229,230 of the invention increasing activity, e.g. of a polypeptide having the activity of a protein as indicated in table II, application no. 13, columns 5 and 7 or its homologs activity; and/or
e) stimulating activity of a protein conferring the increased expression of a protein encoded by the nucleic acid molecule of the present invention or a polypeptide of the present invention having herein-mentioned 229,230 of the invention increasing activity, e.g. of a polypeptide having the activity of a protein as indicated in table II, application no. 13, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned 229,230 of the invention increasing activity, e.g. of a polypeptide having the activity of a protein as indicated in table II, application no. 13, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned 229,230 of the invention increasing activity, e.g. of a polypeptide having the activity of a protein as indicated in table II, application no. 13, columns 5 and 7 or its homologs activity; and/or
h) increasing the expression of the endogenous gene encoding the polypeptide of the invention, e.g. a polypeptide having the activity of a protein as indicated in table II, application no. 13, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with expecially high activity of the proteins of the invention from natural or from mutagenized resources and breeding them into the target organisms, eg the elite crops.

**[0054.0.13.13]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 13 in Table I to IV, resp., after increasing the expression or activity of the encoded polypeptide or having the activity of a polypeptide having an activity as the protein as shown in table II, application no. 13, column 3 or its homologs.

**[0055.0.0.13] to [0067.0.0.13]: see [0055.0.0.0] to [0067.0.0.0]**

**[0068.0.13.13]** The mutation is introduced in such a way that the production of the fatty acids is not adversely affected.

**[0069.0.0.13] to [0071.0.0.13]: see [0069.0.0.7] to [0071.0.0.7]**

**[0072.0.13.13]** By influencing the metabolism thus, it is possible to produce, in the process according to the invention, further advantageous compounds. Examples of such compounds are, in addition to gadoleic acid, triglycerides, lipids, oils and/or fats containing gadoleic acid compounds such as palmitate, palmitoleate, oleate and/or linoleic acid.

**[0073.0.13.13] to [0084.0.0.13] see [0073.0.7.7]: [0084.0.0.7]**

**[0084.1.13.13]** It was found that the level of eicosenic acid is high in the following plants seeds with decreasing level: Selenia grandis , Teesdalia nudicaulis (L.) R. Br. , Teesdalia nudicaulis (L.) R. Br. , Cardiospermum canescens , Lesquerella fendleri , Leavenworthia torulosa , Leavenworthia torulosa , Cardiospermum grandiflorum , Cardiospermum halicacabum , Paullinia elegans , Cupania anacardioides , Koelreuteria elegans , Koelreuteria apiculata , Lobularia maritima , Iberis odorata, Alyssum maritimum , Cardiospermum corindum , Biscutella laevigata , Biscutella auriculata , Lobularia maritima, Dithyrea californica , Dithyrea wislizenii , Thysanocarpus radians , Cheirantus maritimus , Cordia verbenacea DC. , Conringia orientalis (L.) Dumort. , Tropaeolum majus , Conringia orientalis (L.) Dumort. , Tropaeolum majus , Arachis hypogaea , Neslia paniculata, Malcolmia maritima , Malcolmia flexuosa , Arachis peruviana , Sapindus mukorossi, Conringia orientalis , Arachis fastigiata , Arabidopsis thaliana , Arabidopsis thaliana Schur, Sapindus mukorossi , Azima tetracantha , Tropaeolum majus , Tropaeolum majus , Brassica repanda (Willd.) DC. , Calepina irregularis , Arabidopsis thaliana (L.) Heynh. Columbia , Crambe orientalis , Nerisyrenia camporum , Crambe maritima L. , Delphinium ajacis , Tropaeolum minus L. , Cardamine impatiens, Brassica oleracea L. ssp. robertiana (Gay) Rouy et Fouq. , Delphinium spp. , Camelina rumelica , Juniperus chinensis , Capsella rubella , Capsella rubella , Cordia myxa , Sapindus emarginatus , Lepidium cuneiforme , Caulanthus inflatus , Malcolmia chia , Camelina sativa , Cardamine bellidifolia L. , Atalaya hemiglauca , Aesculus assamica , Leonurus sibiricus , Capsella grandiflora , Capsella grandiflora , Sinapis arvensis L. , Erysimum perovskianum , Camelina microcarpa , Schleichera trijuga , Schleichera trijuga , Brassica juncea , Tropaeolum majus , Schleichera trijuga , Cardamine hirsuta , Cardamine amare , Lepidium lasiocarpum , Stanleyella texana, Sapindus mukorossi , Goldbachia laevigata D.C. , Descurainia bourgaeana Webb. , Crambe maritima , Consolida orientalis , Tropaeolum majus , Tropaeolum majus , Lunaria rediviva , Tristellateia australasica , Lepidium perfoliatum , Camelina sativa , Camelina sativa , Lepidium apetalum , Sapindus saponaria , Neslia paniculata , Sapindus saponaria , Brassica sp. , Brassica juncea , Coronopus didymus , Fezia pterocarpa pitard , Arabis glabra , Eruca sativa , Descurainia pinnata var. pinnata , Hornungia petraea , Descurainia sophia , Phoenix dactylifera L. , Descurainia sophia , Thlaspi alpinum , Capsella bursa-pastoris , Capsella bursa-pastoris , Capsella spp. , Capsella spp. , Descurainia sophia , Capsella spp. , Capsella spp. , Tropaeolum minus L. , Capsella bursa-pastoris , Brassica campestris , Lunaria rediviva , Capsella spp. , Descurainia sophia , Capsella spp. , Capsella bursa-pastoris , Clitoria rubiginosa , Arabis laevigata , Thlaspi alpestre L. , Camelina sativa , Crambe cordifolia , Capsella bursa-pastoris , Savignya parviflora , Brassica campestris , Lepidium sativum , Arabis virginica , Sophia ochroleuca , Sapindus emarginatus , Brassica cossoneana (Boiss. et Reuter) Maire , Parrya menziesii , Diplotaxis tenuifolia . For example, it was found that the following content of eicosenic acid is comprised in the oil of seeds: 58.50 , GLC-Area-% ,Selenia grandis , 56.10 , GLC-Area-% ,Teesdalia nudicaulis (L.) R. Br. , 56.00 , GLC-Area-% ,Teesdalia nudicaulis (L.) R. Br. , 55.70 , GLC area% , Cardiospermum canescens , 54.50 , GLC-Area-% ,Lesquerella fendleri , 53.00 , GLC-Area-% ,Leavenworthia torulosa , 53.00 , GLC-Area-% , Leavenworthia torulosa, 52.60 , GLC-Area-% ,Cardiospermum grandiflorum , 49.10 , GLC-Area-% ,Cardiospermum halicacabum, 48.70 , GLC-Area-% ,Paullinia elegans, 46.00 , GLC-Area-% ,Cupania anacardioides, 45.30 , GLC-Area-%, Koelreuteria elegans, 44.60 , GLC area% ,Koelreuteria apiculata , 42.00 , GLC-Area-% ,Lobularia maritima , 41.90 , GLC-Area-% ,Iberis odorata , 41.80 , GLC-Area-% ,Alyssum maritimum, 41.60 , GLC-Area-% ,Cardiospermum corindum , 40.00 , GLC-Area-% ,Biscutella laevigata, 36.10 , GLC-area-% ,Biscutella auriculata , 35.60 , GLC-area-% ,Lobularia maritima. A further list of plants with high gadoleic acid can be found on webpage of the Agricultural Research service (www.ars-grin.gov/cgibin/duke/chemical.pl?gadoleicacid) showing plant species with the highes amount of gadoleic acid. The content of fatty acids in the oil of plants and plants which have a high level of distinct fatty acids can also be identified on the webpage of the Institute of Chemistry and Physics of Lipids, http://www.bagkf.de/sofa/.
Thus, in on embodiment, the process of the present invention is performed in a plant of the above lists of identified in above webpages, preferably in a plant with a high amount of eicosenic acid, in particular with high gadoleic level and/or together with further genes isolated from a plant with a high eicosenic acid level, in particular a high gadoleic level. In a preferred embodiment, the process of the present invention is performed in a plant identified in above webpages with a low amount of erucic acid, in particular with high gadoleic level and/or together with further genes isolated from a plant with a high eicosenic acid level, in particular a high gadoleic level. In another embodiment, the process of the present invention is performed in a plant identified in above webpages, preferably in a plant with a high amount of erucic acid, in particular with high gadoleic level and/or together with further genes isolated from a plant with a high eicosenic acid level, in particular a high gadoleic level.
[0085.0.13.13] With *regard* to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as shown in table I, application no. 13, columns 5 or 7 or a derivative thereof, or
b) a genetic regulatory element, for example a promoter, which is functionally linked to the nucleic acid sequence as shown table I, application no. 13, columns 5 or 7 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.13] to [0087.0.0.13]: see [0086.0.0.0] to [0087.0.0.0]**

**[0088.0.13.13]** In an *advantageous* embodiment of the invention, the organism takes the form of a plant whose fatty acid content is modified advantageously owing to the nucleic acid molecule of the present invention expressed. This is important for plant breeders since, for example, the nutritional value of plants for poultry is dependent on the abovementioned essential fatty acids and the general amount of fatty acids as energy source in feed. After the YDR513W protein activity, e.g. of a protein as indicated in Table II, column 5 or 7, application no. 13 or being encoded by a nucleic acid molecule indicated in Table I, column 5, application no. 13 or of its homologs, e.g. as indicated in Table II, column 7, line 125, has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.13]: see [0088.1.0.0]**

**[0089.0.0.13] to [0102.0.0.13]: see [0089.0.0.7] to [0102.0.0.7]**

**[0103.0.13.13]** In a *preferred* embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide shown in table II, application no. 13, columns 5 or 7 or a fragment thereof, which confers an increase in the amount of the respective fine chemical in an organism or a part thereof;
b) nucleic acid molecule comprising, preferably at least the mature form, of the nucleic acid molecule shown in table I, application no. 13, columns 5 or 7;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybidizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers shown in table III, application no. 13, column 7 and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence shown in table IV, application no. 13, column 7 and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide shown in table II, application no. 13, columns 5 or 7 and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof; or which comprises a sequence which is complementary thereto.

**[0103.1.13.13.]** In one embodiment, the nucleic acid molecule used in the process of the invention distinguishes over the sequence indicated in Table I, columns 5 or 7, application no. 13, by one or more nucleotides. In one embodiment, the nucleic acid molecule used in the process of the invention does not consist of the sequence shown in indicated in Table I, columns 5 or 7, application no. 13. In one embodiment, the nucleic acid molecule used in the process of the invention is less than 100%, 99,999%, 99,99%, 99,9% or 99% identical to a sequence indicated in Table I, columns 5 or 7, application no. 13. In another embodiment, the nucleic acid molecule does not encode a polypeptide of a sequence indicated in Table II, columns 5 or 7, application no. 13.

**[0104.0.13.13]** In one *embodiment,* the nucleic acid molecule used in the process distinguishes over the sequence shown in table I, application no. 13, columns 5 or 7 by one or more nucleotides or does not consist of the sequence shown in table I, application no. 13, columns 5 or 7. In one embodiment, the nucleic acid molecule of the present invention is less than 100%, 99,999%, 99,99%, 99,9% or 99% identical to the sequence shown in table I, application no. 13, columns 5 or 7. In another embodiment, the nucleic acid molecule does not encode a polypeptide of the sequence shown in table II, application no. 13, columns 5 or 7.

**[0105.0.0.13] to [0107.0.0.13]: see [0105.0.0.0] to [0107.0.0.0]**

**[0108.0.13.13]** *Nucleic* acid molecules with the sequence shown in table I, application no. 13, columns 5 or 7, nucleic acid molecules which are derived from the amino acid sequences shown in table II, application no. 13, columns 5 or 7 or from polypeptides comprising the consensus sequence shown in table IV, application no. 13, column 7, or their derivatives or homologues encoding polypeptides with the enzymatic or biological activity of an YDR513W protein or conferring of eicosenic acid, in particular of gadoleic acid increase after increasing its expression or activity are advantageously increased in the process according to the invention.

**[0109.0.0.13]: see [0109.0.0.0]**

**[0110.0.13.13]** Nucleic acid molecules, which are advantageous for the process according to the invention and which encode polypeptides with YDR513W protein activity, e.g. of a protein as indicated in Table II, column 5, application no. 13 or being encoded by a nucleic acid molecule indicated in Table I, column 5, application no. 13 or of its homologs, e.g. as indicated in Table II, column 7, application no. 13, can be determined from generally accessible databases.

**[0111.0.0.13] see [0111.0.0.0]**

**[0112.0.13.13]** The *nucleic* acid molecules used in the process according to the invention take the form of isolated nucleic acid sequences, which encode polypeptides with YDR513W protein activity and conferring gadoleic acid increase, e.g. a protein as indicated in Table II, column 5, application no. 13 or being encoded by a nucleic acid molecule indicated in Table I, column 5, application no. 13, or of their homologs, e.g. as indicated in Table I or II, column 7, application no. 13,.

**[0113.0.0.13] to [0147.0.0.13]: see [0113.0.7.7] to [0147.0.7.7]**

**[0148.0.13.13]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence shown in table I, application no. 13, columns 5 or 7, or a functional portion thereof and preferably has above mentioned activity, in particular having a the fine chemical, in particular gadoleic acid-increasing activity after increasing the acitivity or an activity of an YDR513W gene product, e.g. a gene encoding a protein as indicated in Table II, column 5, application no. 13 or comprising or expressing a nucleic acid molecule indicated in Table I, column 5, application no. 13, or of their homologs, e.g. as indicated in Table I or II, column 7, application no. 13.

**[0149.0.13.13]** The nucleic acid molecule of the invention comprises a nucleotide sequence which hybridizes, preferably hybridizes under stringent conditions as defined herein, to one of the nucleotide sequences shown in table I, application no. 13, columns 5 or 7or a portion thereof and encodes a protein having above-mentioned activity, e.g. conferring an increase of the fine chemical, and optionally, the activity of YDR513W, e.g. of a protein as indicated in Table II, column 5, application no. 13 or being encoded by a nucleic acid molecule indicated in Table I, column 5, application no. 13, or of their homologs, e.g. as indicated in Table I or II, column 7, application no. 13.

**[00149.1.0.13]** Optionally, the nucleotide sequence, which hybridises to one of the nucleotide sequences indicated in Table I, columns 5 or 7, application no. 13, preferably of Table IB, columns 5 or 7, application no. 13 has further one or more of the activities annotated or known for the a protein as indicated in Table II, column 3, application no. 13.

**[0150.0.13.13]** Moreover, the nucleic acid molecule of the invention can comprise only a portion of the coding region of one of the sequences shown in table I, application no. 13, columns 5 or 7 for example a fragment which can be used as a probe or primer or a fragment encoding a biologically active portion of the polypeptide of the present invention or of a polypeptide used in the process of the present invention, i.e. having above-mentioned activity, e.g.conferring an increase of gadoleic acid if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., in table I, application no. 13, columns 5 or 7, an anti-sense sequence of one of the sequences, e.g., set forth in table I, application no. 13, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primers pairs shown in table III, application no. 13, column 7 will result in a fragment of gene product with the activity of a YDR513W protein, e.g. of a gene encoding of a protein as indicated in Table II, column 5, application no. 13 or expressing a nucleic acid molecule indicated in Table I, column 5, application no. 13 or of its homologs, e.g. as indicated in Table II, column 7, application no. 13..

**[0151.0.0.13] to [0230.0.0.13] see [0153.0.7.7] to [0230.0.5.7]**

**[0231.0.13.13]** In a further advantageous embodiment of the process of the invention, the organisms used in the process are those in which simultaneously a gadoleic acid degrading protein is attenuated, in particular by reducing the rate of expression of the corresponding gene.

**[0232.0.0.13] to [0552.0.0.13]: see [0232.0.7.7] to [0552.0.7.7]**

**[0553.0.13.13]** A process for the production of gadoleic acid, which comprises
(a) increasing or generating the activity of a protein as indicated in Table II, application no. 13, columns 5 or 7, or a functional equivalent thereof in a non-human organism, or in one or more parts thereof; and
(b) growing the organism under conditions which permit the production of gadoleic acid in said organism.
A process for the production of gadoleic acid, comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 13, columns 5 or 7, or a fragment thereof, which confers an increase in the amount of gadoleic acid in an organism or a part thereof;
b) nucleic acid molecule comprising of a nucleic acid molecule as indicated in Table I, application no. 13, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of gadoleic acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of gadoleic acid in an organism or a part thereof;
e) nucleic acid molecule which hybidizes with a nucleic acid molecule of (a) to (c) under under stringent hybridisation conditions and conferring an increase in the amount of gadoleic acid in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 13, columns 7, and conferring an increase in the amount of gadoleic acid in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of gadoleic acid in an organism or a part thereof;
h) nucleic acid molecule encoding a polypeptide comprising a consensus as indicated in Table IV, application no. 13, column 7, and conferring an increase in the amount of gadoleic acid in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of gadoleic acid in an organism or a part thereof. or comprising a sequence which is complementary thereto.
   The process as mentioned above, comprising recovering of the free or bound gadoleic acid.
   The process as mentioned above, comprising the following steps:
   (a) selecting an organism or a part thereof expressing a polypeptide encoded by the nucleic acid molecule characterized as mentioned above;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound gadoleic acid produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 13, columns 5 or 7, or a fragment thereof, which confers an increase in the amount of gadoleic acid in an organism or a part thereof;
b) nucleic acid molecule comprising of a nucleic acid molecule as indicated in Table I, application no. 13, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of gadoleic acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of gadoleic acid in an organism or a part thereof;
e) nucleic acid molecule which hybidizes with a nucleic acid molecule of (a) to (c) under stringent hybridisation conditions and conferring an increase in the amount of gadoleic acid in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 13, column 7, and conferring an increase in the amount of gadoleic acid in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of gadoleic acid in an organism or a part thereof;
h) nucleic acid molecule encoding a polypeptide comprising a consensus as indicated in Table IV, application no. 13, column 7, and conferring an increase in the amount of gadoleic acid in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of gadoleic acid in an organism or a part thereof.
whereby the nucleic acid molecule distinguishes over the sequence as indicated in Table I, application no. 13, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 13, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of gadoleic acid in an organism or a part thereof comprising:
(a) contacting cells, tissues , plants or microorganisms which express the a polypeptide encoded by the nucleic acid molecule as mentioned above conferring an increase in the amount of gadoleic acid in an organism or a part thereof with a candidate compound or a sample comprising a plurality of compounds under conditions which permit the expression the polypeptide ;
(b) assaying the gadoleic acid level or the polypeptide expression level in the cell, tissue, plant or microorganism or the media the cell, tissue, plant or microorganisms is cultured or maintained in; and
(c) identifying a agonist or antagonist by comparing the measured gadoleic acid level or polypeptide expression level with a standard gadoleic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased gadoleic acid production in a plant or microorganism, comprising the steps:
(a) culturing a plant cell or tissue or microorganism or maintaining a plant expressing the polypeptide encoded by the nucleic acid molecule as mentioned above conferring an increase in the amount of gadoleic acid in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of gadoleic acid in an organism or a part thereof;
(b) identifying if the compound is an effective agonist by detecting the presence or absence or increase of a signal produced by said readout system.
A method for the identification of a gene product conferring an increase in gadoleic acid production in a cell, comprising the following steps:
(a) contacting the nucleic acid molecules of a sample, which can contain a candidate gene encoding a gene product conferring an increase in gadoleic acid after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing gadoleic acid;
(d) expressing the identified nucleic acid molecules in the host cells;
(e) assaying the gadoleic acid level in the host cells; and
(f) identifying nucleic acid molecule and its gene product which expression confers an increase in the gadoleic acid level in the host cell in the host cell after expression compared to the wild type.
A method for the identification of a gene product conferring an increase in gadoleic acid production in a cell, comprising the following steps:
(a) identifiying in a data bank nucleic acid molecules of an organism; which can contain a candidate gene encoding a gene product conferring an increase in the gadoleic acidamount or level in an organism or a part thereof after expression, and which are at least 20% homolog to the nucleic acid molecule as mentioned above;
(b) introducing the candidate nucleic acid molecules in host cells appropriate for producing gadoleic acid;
(c) expressing the identified nucleic acid molecules in the host cells;
(d) assaying the gadoleic acidlevel in the host cells; and
(e) identifying nucleic acid molecule and its gene product which expression confers an increase in the gadoleic acid level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of gadoleic acid after expression.
Use of the polypeptide as mentioned above or the nucleic acid construct as mentioned above or the gene product identified according to the method as mentioned above for identifying compounds capable of conferring a modulation of gadoleic acid levels in an organism.
Cosmetical, pharmaceutical, food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the protection of vegetable fats, oils or waxes.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the production of industrial oils, fats or waxes.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the production of detergents, cleaning agents, cosmetics, dye additives, lubricating agents, hydraulic oils, preservation agents, flavoring agents, plastic softeners, formulation agents, flotation agents, wetting agents, emulsifiers or lubricating agents.
The plant as mentioned above, which has a low level of erucic acid.
The plant as mentioned above, which has a high level or erucic acid.
The plant as mentioned above, which has a low level of gluconsinolate.

### Process for the production of fine chemicals

**[0000.0.0.14]** In a further embodiment, the present invention relates to a further process for the production of fine chemicals as defined below and corresponding embodiments as described herein as follows.

**[0001.0.0.14] to [0002.0.0.14]: see [0001.0.0.0] to [0002.0.0.0]**

**[0002.1.14.14]** L-alanine is used in various pharmaceutical and veterinary applications. For example, it is included, together with other amino acids, in preparations for infusion solutions or preparations for parenteral administration as clinical preoperative and postoperative foods, as well as an animal feed supplement. Furthermore, alanine is used as a food additive on account of its sweet taste.
L-phenylalanine and L-aspartic acid have very important markets as key components in the manufacture of the sweetener aspartame. Aspartame (C₁₄H₁₈N₂O₅), L-aspartyl-L-phenylalanine methyl ester, is a compound of three components, which are methanol, aspartic acid and phenylalanine. L-aspartic acid is further used as a flavoring agent.
The amino acid L-citrulline is a metabolite in the urea cycle. Other amino acids in this cycle are L-arginine and L-ornithine.L-citrulline is involved in liver detoxification of ammonia, and has been shown to speed recover from fatigue. It has also been utilized in the treatment of Ornithine Transcarbamylase Deficiency and other Urea Cycle disorders. In cell metabolism, L-arginine and L-citrulline might serve as endogenous N sources (Ludwig et al., PLANT PHYSIOLOGY , Vol 101, Issue 2 429-434, 1993). Glycine is a valuable compound of wide use as food additives for processed foodstuffs and raw materials for agricultural chemicals and medicines. Glycine is the simplest amino acid, and is used in crop production as a chelating agent for micronutrients and has been used as a nitrogen fertilizer, at least on an experimental basis. As such, it is representative of amino acids used in crop production. Practically all commercial glycine is produced by synthetic processes such as the Strecker Synthesis, the reaction of formaldehyde, ammonia, and hydrogen cyanide, and hydrolysis of the resulting aminonitrile. Glycine is used as chelating / complexing agent for cation nutrients, plant growth regulators, substrate for microbiological products, fertilizer source of nitrogen.
Serine is a primary intermediate in the biosynthesis of a wide variety of cellular metabolites including such economically important compounds as choline, glycine, cysteine and tryptophan. In addition, serine acts as a single carbon donor and is responsible for 60% to 75% of the total need of the cell for C1 units through the production of 5,10-methylenetetrahydrofolate from tetrahydrofolate. These C1 units are used in a wide variety of biosynthetic pathways including the synthesis of methionine, inosine monophosphate, other purines and some pyrimidines (e.g., thymidine and hydroxymethyl cytidine).
The glycine-serine interconversion, catalysed by glycine decarboxylase and serine hydroxymethyltransferase, is an important reaction of primary metabolism in all organisms including plants, by providing one-carbon units for many biosynthetic reactions. In plants, in addition, it is an integral part of the photorespiratory metabolic pathway and produces large amounts of photorespiratory CO₂ within mitochondria (Bauwe et al., Journal of Experimental Botany, Vol. 54, No. 387, pp. 1523-1535, June 1,2003.)
The enzymatic conversion of phenylalanine to tyrosine is known in eukaryotes. Human phenylalanine hydroxylase is specifically expressed in the liver to convert L-phenylalanine to L-tyrosine (Wang et al. J. Biol. Chem. 269 (12): 9137-46 (1994)). Deficiency of the PAH enzyme causes classic phenylketonurea, a common genetic disorder.
Tyrosine and and their derivatives are also used in organic synthesis. For example, tyrosine is starting material in the synthesis of chatecolamines or DOPA (dihydroxyphenyl-alanine) as well as a precursor of adrenaline, dopamine and norepinepherine. A variety of beta-amino-gamma-keto acids can be prepared from commercially available I- .
5-Oxoproline, also named as pyroglutamic acid PCA and slats like sodium-PCA, is used as cosmetic ingredient, such as hair and skin conditioning agent. One optical isomer of PCA (the L form) is a naturally occurring component of mammalian tissue. 5-Oxoproline is further used as templates in the synthesis of homochiral glutamate antagonists.

**[0003.0.0.14] to [0008.0.0.14]: see [0003.0.0.0] to [0008.0.0.0]**

**[0008.1.14.14]**US 5,498,532 disclose the production of various L-amino acids like glutamic acid, glutamine, lysine, threonine, isoleucine, valine, leucine, tryptophan, phenylalanine, tyrosine, histidine, arginine, ornithine, citrulline and proline by direct fermentation using, coryneform bacteria belonging to the genus Corynbacterium or Brevibacterium, which are inherently unable to assimilate lactose, but due to recombinant DNA technology able to assimilate lactose,which represent the carbon source.
The coproduction of glutamic acid and other amino acids including lysine, aspartic acid, alanine by an auxotroph of Bacillus methanolicus is described in US 6,110,713.

According to the teaching of US 5,677,156 L-aspartic acid can be efficiently produced from maleic acid or fumaric acid by adding the aspartase-containing microorganism, like Brevibacterium flavum AB-41 strain (FERM BP-1498) and Eschirichia coli ATCC 11303.
US 5,354,672 discloses a method of producing tyrosine, methionine, or phenylalanine by transiently incorporating a DNA inversion gene into the host cell, Escherichia coli cells, which induce hypersecretion of amino acids.
Known is also the production of citrulline in the small intestine as a product of glutamine metabolism, or in the arginine biosynthetic pathway, where ornithine carbamoyltransferases catalyse the production of citrulline from carbamoyl-phosphate and ornithine. Benninghoff et al. disclose the production of citrulline and ornithine by interferon-gamma treated macrophages (International Immunology, Vol 3, 413-417, 1991).
There disclosed is a method for producing glycine in US 20030040085, which comprises subjecting an aqueous solution of glycinonitrile to a hydrolysis reaction in a hydrolysis reaction system under the action of a microbial enzyme, thereby converting the glycinonitrile to glycine while by-producing ammonia.
US 20040157290 discloses a process for preparing a serine-rich foreign protein comprising culturing a bacterium containing the cysteine synthase (cysK) gene and a gene encoding the foreign protein.
US 20030079255 disclose the production of Para-hydroxycinnamic acid by introducing genes encoding phenylalanine ammonia-lyase from C. violaceum or R. glutinis tyrosine into a host microorganism and as intermediates, tyrosine and cinnamic acid are also produced.
Production of single cell protein and selected amino acids by microbial fermentation is known, e.g., US 4,652,527. One amino acid which has been produced on an industrial scale is lysine, see Tosaka et al., Trends in Biotechnology, 1: 70-74 (1983), Tosaka and Takinami, Progress in Industrial Microbiology, Ch. 24, pp. 152-172 (Aida et al., 1986). Another example is glutamic acid which has been produced using bacteria of the genera Corynebacterium, Brevibacterium, Microbacterium, and Arothrobacter by fermentation on molasses and starch hydrozylates. Aspartic acid and alanine are produced by enzymatic means from fumaric acid and ammonia. Bacillus species have been used in fermentation processes to produce amino acids, Tosaka et al.; Tosaka and Takinami, as named above.

**[0009.0.14.14]**As described above, the amino acids are necessary for humans and many mammals, for example for livestock or other applications in the health care area. L-aspartic acid is one of the amino acids that is difficult to produce directly by fermentation. Consequently today the enzyme catalyzed addition of ammonia to fumaric acid is employed commercially in the production of aspartic acid.
As chelating agents, amino acids increase the biological availability of different metals. Chelating agents in general can enhance nutrient uptake, but may also increase the uptake of toxic metals if those are also present. If cation impurities are present in micronutrient sources (e.g. Cadmium), chelation of those metals would make those contaminants more readily assimilated by plants than in less available forms.

**[0010.0.0.14] to [0011.0.0.14]: see [0010.0.0.0] to [0011.0.0.0]**

**[0012.0.14.14]** It is an object of the present invention to develop an inexpensive process for the synthesis of 5-oxoproline, alanine, aspartic acid, citrulline, glycine, phenylalanine, serine and/or tyrosine. Amino acids are (depending on the organism) one of the most frequently limiting components of food or feed.

**[0013.0.0.14] see [0013.0.0.00]**

**[0014.0.14.14]** Accordingly, in a first embodiment, the invention relates to a process for the production of a fine chemical, whereby the fine chemical is 5-oxoproline, alanine, aspartic acid, citrulline, glycine, phenylalanine, serine and/or tyrosine. Accordingly, in the present invention, the term "the fine chemical" as used herein relates to "5-oxoproline, alanine, aspartic acid, citrulline, glycine, phenylalanine, serine and/or tyrosine". Further, the term "the fine chemicals" as used herein also relates to fine chemicals comprising 5-oxoproline, alanine, aspartic acid, citrulline, glycine, phenylalanine, serine and/or tyrosine.

**[001 5.0.14.14]**In one embodiment, the term "the fine chemical" or "the respective fine chemical" means 5-oxoproline, alanine, aspartic acid, citrulline, glycine, phenylalanine, serine and/or tyrosine, preferably the amino acid of the present invention in the L configuration, meaning L-5-oxoproline, L-alanine, L-aspartic acid, L-citrulline, L-glycine, L-phenylalanine, L-serine and/or L-tyrosine . Throughout the specification the term "the fine chemical" means 5-oxoproline, alanine, aspartic acid, citrulline, glycine, phenylalanine, serine and/or tyrosine, preferably the amino acid of the present invention in the L configuration, its salts, ester or amids in free form or bound to proteins. In a preferred embodiment, the term "the fine chemical" means L-5-oxoproline, L-alanine, L-aspartic acid, L-citrulline, L-glycine, L-phenylalanine, L-serine and/or L-tyrosine in free form or its salts or bound to proteins.

**[0016.0.14.14]**Accordingly, the present invention relates to a process for the production of the respective fine chemical comprising
(a) increasing or generating the activity of one or more of a protein as shown in table II, application no. 14, column 3 encoded by the nucleic acid sequences as shown in table I, application no. 14, column 5, in a non-human organism or in one or more parts thereof or
(b) growing the organism under conditions which permit the production of the fine chemical, thus amino acids of the invention or fine chemicals comprising amino acids of the invention, in said organism or in the culture medium surrounding the organism.

**[0016.1.14.14]** Accordingly, the term "the fine chemical" means in one embodiment "5-oxoproline" in relation to all sequences listed in Table I to IV, application no. 14 line 24 or homologs thereof and
means in one embodiment "alanine" in relation to all sequences listed in Tables I to IV, lines 25 and/or 26 or homologs thereof and
means in one embodiment "aspartic acid" in relation to all sequences listed in Table I to IV, lines 27 and/or 28, or homologs thereof and
means in one embodiment "citrulline" in relation to all sequences listed in Table I to IV, lines 22 and/or 29 to 30 or homologs thereof and
means in one embodiment "glycine" in relation to all sequences listed in Table I to IV, line 31 or homologs thereof and
means in one embodiment "phenylalanine" in relation to all sequences listed in Table I to IV, lines 32 and/or 33 or homologs thereof and
means in one embodiment "serine" in relation to all sequences listed in Table I to IV, line 23 or homologs thereof and
means in one embodiment "tyrosine" in relation to all sequences listed in Table I to IV, line34 or homologs thereof.
Accordingly, in one embodiment the term "the fine chemical" means "5-oxoproline" and "alanine", "5-oxoproline" and "aspartic acid", "aspartic acid" and "alanine" and/or "5-oxoproline" and "alanine" and "aspartic acid" in relation to all sequences listed in Table I to IV, lines 24, 25 and/or 27;
in one embodiment the term "the fine chemical" means "phenylalanineand "tyrosine" in relation to all sequences listed in Table I to IV, lines 33 and/or 34;
in one embodiment the term "the fine chemical" means one fine chemical or any combination of two or three fine chemicals selected from the group consisting of "glycine", and "phenylalanine" and "alanine" and "aspartic acid", in relation to all sequences listed in Table I to IV, lines 26, 28, 31 and/or 32;
Accordingly, the term "the fine chemical" can mean "5-oxoproline", "alanine", "aspartic acid", "citrulline", "glycine", " ", "phenylalanine", "serine" and/or "tyrosine" , owing to circumstances and the context. In order to illustrate that the meaning of the term "the fine chemical" means "5-oxoproline", "alanine", "aspartic acid", "citrulline", "glycine", " ", "phenylalanine", "serine" and/or "tyrosine" the term "the respective fine chemical" is also used.

**[0017.0.0.14] to [0019.0.0.14]: see [0017.0.0.0] to [0019.0.0.0]**

**[0020.0.14.14]** Surprisingly it was found, that the transgenic expression of the Zea mays protein as indicated in Table II, column 5, line22 in a plant conferred an increase in citrulline content of the transformed plants. Thus, in one embodiment, said protein or its homologs are used for the production of citrulline.

Surprisingly it was found, that the transgenic expression of the Zea mays protein as indicated in Table II, column 5, line23 in plant conferred an increase in serine content of the transformed plants. Thus, in one embodiment, said protein or its homologs are used for the production of serine.
Surprisingly it was found, that the transgenic expression of the Linum usitatissimum protein as indicated in Table II, column 5, line24, 25 and/or 27 in a plant conferred an increase in 5-oxoplroline, alanine and/or aspartic acid content of the transformed plants. Thus, in one embodiment, said protein or its homologs are used for the production of 5-oxoproline in one embodiment, said protein or its homologs are used for the production of alanine, in one embodiment, said protein or its homologs are used for the production of aspartic acid, in one embodiment, said protein or its homologs are used for the production of one or more fine chemical selected from the group consisting of: 5-oxoproline, alanine and/or aspartic acid.
Surprisingly it was found, that the transgenic expression of the Hordeum vulgare protein as indicated in Table II, column 5, lines26, 28, 31 and/or 32 in a plant conferred an increase in alanine, aspartic acid, glycine and/or phenylalanine. content of the transformed plants. Thus, in one embodiment, said protein or its homologs are used for the production of alaninein one embodiment, said protein or its homologs are used for the production of aspartic aicd, in one embodiment, said protein or its homologs are used for the production of glycine, in one embodiment, said protein or its homologs are used for the production of phenylalanine, in one embodiment, said protein or its homologs are used for the production of one or more fine chemical selected from the group consisting of: alanine, aspartic acid, glycine and/or phenylalanine..
Surprisingly it was found, that the transgenic expression of the Brassica napus protein as indicated in Table II, column 5, line29 in Arabidopsis thaliana conferred an increase in citrulline content of the transformed plants. Thus, in one embodiment, said protein or its homologs are used for the production of citrulline.
Surprisingly it was found, that the transgenic expression of the Helianthus annuus protein as indicated in Table II, column 5, lines 30 in thaliana plant conferred an increase in citrulline content of the transformed plants. Thus, in one embodiment, said protein or its homologs are used for the production of citrulline.
Surprisingly it was found, that the transgenic expression of the Hordeum vulgare protein as indicated in Table II, column 5, lines 33 and 34 in thaliana plant conferred an increase in phenylalanine and/or tyrosine (or the respective fine chemical) content of the transformed plants. Thus, in one embodiment, said protein or its homologs are used for the production of phenylalanine; in one embodiment, said protein or its homologs are used for the production of tyrosine, in one embodiment, said protein or its homologs are used for the production of one or more fine chemical selected from the group consisting of: phenylalanine and/or tyrosine.

### [0021.0.0.14] see [0021.0.0.0]

**[0022.0.14.14]** The sequence of YOR245C from Saccharomyces cerevisiae has been published in Dujon,et al., Nature 387 (6632 Suppl), 98-102 (1997), and Goffeau, Science 274 (5287), 546-547, 1996 and its activity is being defined as a Acyl-CoA:diacylglycerol acyltransferase, preferably of the Caenorhabditis elegans hypothetical protein K07B1.4 superfamily. Accordingly, in one embodiment, the process of the present invention comprises the use of a gene product with an activity of Caenorhabditis elegans hypothetical protein K07B1.4 superfamily, preferably such protein is having a Acyl-CoA:diacylglycerol acyltransferase activity or its homolog, e.g. as shown herein, for the production of the the respective fine chemical, meaning of citrulline, in particular for increasing the amount of citrulline in free or bound form in an organism or a part thereof, as mentioned. In one embodiment, in the process of the present invention the activity of a Acyl-CoA:diacylglycerol acyltransferase is increased or generated, e.g. from Saccharomyces cerevisiae or a plant or a homolog thereof.
The sequence of YKR057W from Saccharomyces cerevisiae has been published in Goffeau, Science 274 (5287), 546-547 (1996) and Dujon, Nature 369 (6479), 371-378 (1994) and its activity is being defined as a ribosomal protein, similar to S21 ribosomal proteins, involved in ribosome biogenesis and translation, preferably of the rat ribosomal protein S21 superfamily. Accordingly, in one embodiment, the process of the present invention comprises the use of a gene product with an activity of the rat ribosomal protein S21 superfamily, preferably such protein is having a ribosomal protein, similar to S21 ribosomal proteins, involved in ribosome biogenesis and translation activity or its homolog, e.g. as shown herein, for the production of the respective fine chemical, meaning of serine, in particular for increasing the amount of serine, preferably of serine in free or bound form in an organism or a part thereof, as mentioned. In one embodiment, in the process of the present invention the activity of a ribosomal protein, similar to S21 ribosomal proteins, involved in ribosome biogenesis and translation is increased or generated, e.g. from Saccharomyces cerevisiae or a plant or a homolog thereof.
The sequence of b1343 from Escherichia coli K12 has been published in Blattner et al., Science 277(5331), 1453-1474, 1997, and its activity has been defined as a ATP-dependent RNA helicase, stimulated by 23S rRNA. Accordingly, in one embodiment, the process of the present invention comprises the use of a gene product with an activity of the ATP-dependent RNA helicase, stimulated by 23S rRNA from E. coli or from a plant or its homolog, e.g. as shown herein, for the production of the respective fine chemical, meaning of alanine, 5-oxoproline and/or aspartic acid, in particular for increasing the amount of alanine, in particular for increasing the amount of 5-oxoproline, in particular for increasing the amount of aspartic acid, in particular for increasing the amount of alanine and 5-oxoproline, in particular for increasing the amount of alanine and aspartic acid, in particular for increasing the amount of 5-oxoproline and aspartic acid, in particular for increasing the amount of alanine and 5-oxoproline and aspartic acid, preferably of alanine, 5-oxoproline and/or aspartic acid in free or bound form in an organism or a part thereof, as mentioned.
The sequence of b2426 from Escherichia coli K12 has been published in Blattner et al., Science 277 (5331), 1453-1474, 1997, and its activity is being defined as a putative oxidoreductase with NAD(P)-binding domain, preferably of the ribitol dehydrogenase, short-chain alcohol dehydrogenase homology superfamily. Accordingly, in one embodiment, the process of the present invention comprises the use of a gene product with an activity of a putative oxidoreductase with NAD(P)-binding domain from E. coli or from a plant or its homolog, e.g. as shown herein, for the production of the respective fine chemical, meaning of citrulline, in particular for increasing the amount of citrulline, preferably citrulline in free or bound form in an organism or a part thereof, as mentioned.
The sequence of b2576 from Escherichia coli K12 has been published in Blattner et al., Science 277(5331), 1453-1474, 1997, and its activity has been defined as a ATP-dependent RNA helicase. Accordingly, in one embodiment, the process of the present invention comprises the use of a gene product with an activity of the ATP-dependent RNA helicase from E. coli or from a plant or its homolog, e.g. as shown herein, for the production of the respective fine chemical, meaning of alanine, glycine, aspartic acid and/or phenylalanine, in particular for increasing the amount of alanine, in particular for increasing the amount of glycine, in particular for increasing the amount of aspartic acid, in particular for increasing the amount of phenylalanine,in particular for increasing the amount of alanine and glycine, in particular for increasing the amount of alanine and aspartic acid, in particular for increasing the amount of alanine and phenylalanine, in particular for increasing the amount of glycine and aspartic acid, in particular for increasing the amount of glycine and phenylalanine, in particular for increasing the amount of aspartic acid and phenylalanine, in particular for increasing the amount of alanine and glycine and aspartic acid, in particular for increasing the amount of alanine and glycine and phenylalanine, in particular for increasing the amount of alanine and aspartic acid and phenylalanine, in particular for increasing the amount of glycine and aspartic acid and phenylalanine, in particular for increasing the amount of alanine and glycine and aspartic acid and phenylalanine, preferably of alanine, glycine, aspartic acid and/or phenylalanine in free or bound form in an organism or a part thereof, as mentioned.
The sequence of b3983 from Escherichia coli K12 has been published in Blattner et al., Science 277(5331), 1453-1474, 1997, and its activity has been defined as a 50S ribosomal subunit protein L12, preferably of the Escherichia coli ribosomal protein L11 superfamily. Accordingly, in one embodiment, the process of the present invention comprises the use of a gene product with an activity of the 50S ribosomal subunit protein L12 from E. coli or from a plant or its homolog, e.g. as shown herein, for the production of the respective fine chemical, meaning of phenylalanine and/or tyrosine, in particular for increasing the amount of phenylalanine, in particular for increasing the amount of tyrosine, in particular for increasing the amount of phenylalanine and tyrosine, preferably of phenylalanine and/or tyrosine in free or bound form in an organism or a part thereof, as mentioned.
The sequence of b4269 from Escherichia coli K12 has been published in Blattner et al., Science 277 (5331), 1453-1474, 1997, and its activity is being defined as a putative alcohol dehydrogenase with NAD(P)-binding and GroES domains. Accordingly, in one embodiment, the process of the present invention comprises the use of a gene product with an activity of a putative alcohol dehydrogenase with NAD(P)-binding and GroES domains from E. coli or from a plant or its homolog, e.g. as shown herein, for the production of the respective fine chemical, meaning of citrulline, in particular for increasing the amount of citrulline, preferably citrulline in free or bound form in an organism or a part thereof, as mentioned.

### [0022.1.0.14] to [0023.0.0.14] see [0022.1.0.14] to [0023.0.0.14]2

**[0023.1.14.14]** Homologs of the polypeptide disclosed in table II, application no. 14, column 5, may have the activity of a protein as disclosed in table I or II, application no. 14, column 3 and may be the polypetides encoded by the nucleic acid molecules indicated in table I , application no. 14, column 7, resp., or may be the polypeptides indicated in table II, application no. 14, column 7, resp.

**[0024.0.0.14] see [0024.0.0.0]**

**[0025.0.14.14]** In accordance with the invention, a protein or polypeptide has the "activity of an protein as shown in table II, application no. 14, column 3 "if its *de novo* activity, or its increased expression directly or indirectly leads to an increased in the level of the fine chemical indicated in the respective line of table II, application no. 14, column 6 "metabolite" in the organism or a part thereof, preferably in a cell of said organism. Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 14, column 3, or which has at least 10% of the original enzymatic or biological activity, preferably 20%, particularly preferably 30%, most particularly preferably 40% in comparison to a protein as shown in the respective line of table II, application no. 14, column 3 of of a E. coli or Saccharomyces cerevisae, respectively, protein as mentioned in table I to IV, column 3 respectively and as disclosed in paragraph [0022] of the respective invention.

**[0025.1.0.14] to [0033.0.0.14]: see [0025.1.0.0] to [0033.0.0.0]**

**[0034.0.14.14]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 14, column 3 its biochemical or genetical causes and the increased amount of the respective fine chemical.

**[0035.0.0.14] to [0044.0.0.14]: see [0035.0.0.0] to [0044.0.0.0]**

**[0045.0.14.14]** In one embodiment, the activity of the protein. as indicated in Table II, columns 5 or 7, application no. 14, is increased conferring an increase of the respective fine chemical, indicated in Table II, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more

**[0046.0.14.14]** In one embodiment, the activity of the protein as indicated in Table II, columns 5 or 7, application no. 14, is increased conferring an increase of the respective fine chemical, indicated in Table II, application no. 14, column 6 of the respective line confers an increase of the respective fine chemical and of further amino acids or their precursors.

**[0047.0.0.14] to [0048.0.0.14]: see [0047.0.0.0] to [0048.0.0.0]**

**[0049.0.14.14]** A protein having an activity conferring an increase in the amount or level of the fine chemical, has in one embodiment the structure of the polypeptide described herein, in particular of the polypeptides comprising the consensus sequence shown in table IV, application no. 14, column 7 or of the polypeptide as shown in the amino acid sequences as disclosed in table II, application no. 14, columns 5 and 7 or the functional homologues thereof as described herein, or is encoded by the nucleic acid molecule characterized herein or the nucleic acid molecule according to the invention, for example by the nucleic acid molecule as shown in table I, application no. 14, columns 5 and 7 or its herein described functional homologues and has the herein mentioned activity.

**[0050.0.14.14]**For the purposes of the present invention "the respective fine chemical" also encompass the corresponding salts, such as, for example, the potassium, amonium or sodium salts of the respective fine chemical or the respective amino acid hydrochloride or sulfateof 5-oxoproline, alanine, aspartic acid, citrulline, glycine, , phenylalanine, serine and/or tyrosine .

**[0051.0.0.14] to [0052.0.0.14]: see [0051.0.0.0] to [0052.0.0.0]**

**[0053.0.14.14]**In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 14, columns 5 and 7 or its homologs activity having herein-mentioned amino acids of the invention increasing activity; and/or
b) stabilizing a mRNA conferring the increased expression of a protein encoded by the nucleic acid molecule of the invention as shown in table I, application no. 14, columns 5 and 7, e.g. a nucleic acid sequence encoding a polypeptide having the activity of a protein as indicated in table II, application no. 14, columns 5 and 7 or its homologs activity or of a mRNA encoding the polypeptide of the present invention having herein-mentioned amino acids of the invention increasing activity; and/or
c) increasing the specific activity of a protein conferring the increased expression of a protein encoded by the nucleic acid molecule of the invention or of the polypeptide of the present invention having herein-mentioned amino acid increasing activity, e.g. of a polypeptide having the activity of a protein as indicated in table II, application no. 14, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned amino acids of the invention increasing activity, e.g. of a polypeptide having the activity of a protein as indicated in table II, application no. 14, columns 5 and 7 or its homologs activity; and/or
e) stimulating activity of a protein conferring the increased expression of a protein encoded by the nucleic acid molecule of the present invention or a polypeptide of the present invention having herein-mentioned amino acids of the invention increasing activity, e.g. of a polypeptide having the activity of a protein as indicated in table II, application no. 14, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned amino acids of the invention increasing activity, e.g. of a polypeptide having the activity of a protein as indicated in table II, application no. 14, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned amino acids of the invention increasing activity, e.g. of a polypeptide having the activity of a protein as indicated in table II, application no. 14, columns 5 and 7 or its homologs activity; and/or
h) increasing the expression of the endogenous gene encoding the polypeptide of the invention, e.g. a polypeptide having the activity of a protein as indicated in table II, application no. 14, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j)selecting of organisms with expecially high activity of the proteins of the invention from natural or from mutagenized resources and breeding them into the target organisms, eg the elite crops.

**[0054.0.14.14]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 14 in Table I to IV, resp., after increasing the expression or activity of the encoded polypeptide or having the activity of a polypeptide having an activity as the protein as shown in table II, application no. 14, column 3 or its homologs..

**[0055.0.0.14] to [0071.0.0.14]: see [0055.0.0.0] to [0071.0.0.0]**

**[0072.0.14.14]** By influencing the metabolism thus, it is possible to produce, in the process according to the invention, further advantageous compounds. Examples of such compounds are, in addition to 5-oxoproline, alanine, aspartic acid, citrulline, glycine, , phenylalanine, serine and/or tyrosine further amino acids or the respective precursors.

**[0073.0.14.14]**Accordingly, in one embodiment, the process according to the invention relates to a process which comprises:
a) providing a non-human organism, preferably a microorganism, a non-human animal, a plant or animal cell, a plant or animal tissue or a plant;
b) increasing an activity of a polypeptide of the invention or a homolog thereof, e.g. as indicated in Table II, application no. 14, columns 5 or 7, or of a polypeptide being encoded by the nucleic acid molecule of the present invention and described below, i.e. conferring an increase of the respective fine chemical in the organism, preferably in a microorganism, a non-human animal, a plant or animal cell, a plant or animal tissue or a plant,
c) growing the organism, preferably a microorganism, a non-human animal, a plant or animal cell, a plant or animal tissue or a plant, under conditions which permit the production of the respective fine chemical in the organism, preferably the microorganism, the plant cell, the plant tissue or the plant; and
d) if desired, recovering, optionally isolating, the free and/or bound the respective fine chemical as indicated in any one of Tables I to IV, application no. 14, column 6 "metabolite" and , optionally further free and/or bound amino acids synthetized by the organism, the microorganism, the non-human animal, the plant or animal cell, the plant or animal tissue or the plant.

### [0074.0.0.14] to [0084.0.0.14]: see [0075.0.0.0] to [0084.0.0.0]

**[0085.0.14.14]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, application no. 14, columns 5 or 7, or a derivative thereof, or
b) a genetic regulatory element, for example a promoter, which is functionally linked to the nucleic acid sequence as indicated in Table I, application no. 14, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.14] to [0088.1.0.14]: see [0086.0.0.0] to [0088.1.0.0]**

**[0089.0.0.14] to [0097.0.0.14]: see [0089.0.0.0] to [0097.0.0.0]**

**[0098.0.14.14]**In a preferred embodiment, the respective fine chemical (5-oxoproline, alanine, aspartic acid, citrulline, glycine, , phenylalanine, serine and/or tyrosine ) are produced in accordance with the invention and, if desired, are isolated. The production of further amino acids and of amino acid mixtures or mixtures with other compounds by the process according to the invention is advantageous.

**[0099.0.0.14] to [0102.0.0.14]: see [0099.0.0.0] to [0102.0.0.0]**

**[0103.0.14.14]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of a polypeptide having a sequence as indicated in Table II, application no. 14, columns 5 or 7, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 14, columns 5 or 7,.;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybidizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, application no. 14, columns 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, application no. 14, columns 7, and conferring an increase in the amount of the respective fine chemical in an organism or a part thereof ;
k) nucleic acid molecule comprising one or more of the nucleic acid molecule encoding the amino acid sequence of a polypeptide encoding a domaine of a polypeptide indicated in Table II, application no. 14, columns 5 or 7 and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.0.14.]** In one embodiment, the nucleic acid molecule used in the process of the invention distinguishes over the sequence indicated in Table I, application no. 14, columns 5 or 7 by one or more nucleotides. In one embodiment, the nucleic acid molecule used in the process of the invention does not consist of the sequence shown in indicated in Table I ,application no. 14, columns 5 or 7,. In one embodiment, the nucleic acid molecule used in the process of the invention is less than 100%, 99,999%, 99,99%, 99,9% or 99% identical to a sequence indicated in Table I , application no. 14, columns 5 or 7,. In another embodiment, the nucleic acid molecule does not encode a polypeptide of a sequence indicated in Table II , application no. 14, columns 5 or 7,.

**[0104.0.14.14]** In one embodiment, the nucleic acid molecule of the invention distinguishes over the sequence indicated in Table I, application no. 14, columns 5 or 7, , by one or more nucleotides. In one embodiment, the nucleic acid molecule of the invention does not consist of the sequence shown in indicated in Table I, application no. 14, columns 5 or 7,. In one embodiment, the nucleic acid molecule of the present invention is less than 100%, 99,999%, 99,99%, 99,9% or 99% identical to a sequence indicated in Table I, application no. 14, columns 5 or 7,. In another embodiment, the nucleic acid molecule does not encode a polypeptide of a sequence indicated in Table II, application no. 14, columns 5 or 7,.

**[0105.0.0.14] to [0107.0.0.14]: see [0105.0.0.0] to [0107.0.0.0]**

**[0108.0.14.14]** Nucleic acid molecules with the sequence as indicated in Table I, application no. 14, columns 5 or 7, , nucleic acid molecules which are derived from a amino acid sequences as indicated in Table II, application no. 14, columns 5 or 7, , or from polypeptides comprising the consensus sequence as indicated in Table IV, application no. 14, columns 7, , or their derivatives or homologues encoding polypeptides with the enzymatic or biological activity of a polypeptide as indicated in Table I, application no. 14, column 3, 5 or 7, , or e.g. conferring a increase of the respective fine chemical after increasing its expression or activity are advantageously increased in the process according to the invention.

**[0109.0.0.14]: see [0109.0.0.0]**

**[0110.0.14.14]** Nucleic acid molecules, which are advantageous for the process according to the invention and which encode polypeptides with an activity of a polypeptide of the invention or the polypeptide used in the method of the invention or used in the process of the invention, e.g. of a protein as indicated in Table II, application no. 14, column 5, , being encoded by a nucleic acid molecule indicated in Table I, application no. 14, column 5, , or of its homologs, e.g. as indicated in Table II, application no. 14, column 7 can be determined from generally accessible databases.

**[0111.0.0.14]: see [0111.0.0.0]**

**[0112.0.14.14]** The nucleic acid molecules used in the process according to the invention take the form of isolated nucleic acid sequences, which encode polypeptides with an activity of a polypeptide as indicated in Table I, application no. 14, column3, or having the sequence of a polypeptide as indicated in Table II, application no. 14, columns 5 and 7, and conferring a 5-oxoproline and/or alanine and/or aspartic acid and/or citrulline and/or glycine and/or phenylalanine and/or serine and/or tyrosine increase.

**[0113.0.0.14] to [0120.0.0.14]: see [0113.0.0.0] to [0120.0.0.0]**

**[0121.0.14.14]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 14, columns 5 or 7, or the functional homologues thereof as described herein, preferably conferring above-mentioned activity, i.e. conferring a increase of the respective fine chemical after increasing its activity.

**[0122.0.0.14] to [0127.0.0.14]: see [0122.0.0.0] to [0127.0.0.0]**

**[0128.0.14.14]** Synthetic oligonucleotide primers for the amplification, e.g. as the pairs indicated in Table III, application no. 14, column 7, , by means of polymerase chain reaction can be generated on the basis of a sequence shown herein, for example the sequence as indicated in Table I, application no. 14, columns 5 or 7, or the sequences derived from sequences as indicated in Table II, application no. 14, columns 5 or 7,.

**[0129.0.14.14]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consensus sequences indicated in Table IV, application no. 14, column 7, are derived from said alignments.

**[0130.0.14.14]**Degenerated primers can then be utilized by PCR for the amplification of fragments of novel proteins having above-mentioned activity, e.g. conferring the increase of amino acids, e.g. of 5-oxoproline, alanine, aspartic acid, citrulline, glycine, , phenylalanine, serine and/or tyrosine after increasing its expression or activity of the protein comprising said fragment.

**[0131.0.0.14] to [0138.0.0.14]: see [0131.0.0.0] to [0138.0.0.0]**

**[0139.0.14.14]** Polypeptides having above-mentioned activity, i.e. conferring an increase of the respective fine chemical level, derived from other organisms, can be encoded by other DNA sequences which hybridize to a sequences indicated in Table I, application no. 14, columns 5 or 7, under relaxed hybridization conditions and which code on expression for peptides having the respective fine chemical, in particular, of 5-oxoproline and/or alanine and/or aspartic acid and/or citrulline and/or glycine and/or phenylalanine and/or serine and/or tyrosine resp., increasing activity.

**[0140.0.0.14] to [0146.0.0.14]: see [0140.0.0.0] to [0146.0.0.0]**

**[0147.0.14.14]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 14, columns 5 or 7, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.14.14]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 14, columns 5 or 7 or a portion thereof and preferably has above mentioned activity, in particular, of arginine and/or glutamate and/or proline and/or glutamine increasing activity after increasing the acitivity or an activity of a product of a gene encoding said sequences or their homologs.

**[0149.0.14.14]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 14, columns 5 or 7, or a portion thereof and encodes a protein having above-mentioned activity and as indicated in indicated in Table II.

**[0150.0.14.14]** Moreover, the nucleic acid molecule of the invention can comprise only a portion of the coding region of one of the sequences indicated in Table I, application no. 14, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of arginine and/or glutamate and/or proline and/or glutamine, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 14, columns 5 or 7, , an anti-sense sequence of one of the sequences, e.g., as indicated in Table I, application no. 14, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 14, column 7, will result in a fragment of a polynucleotide sequence as indicated in Table I, application no. 14, columns 5 or 7 or its gene product.

**[0151.0.0.14] see [0151.0.0.0]**

**[0152.0.14.14]** The nucleic acid molecule of the invention encodes a polypeptide or portion thereof which includes an amino acid sequence which is sufficiently homologous to an amino acid sequence as indicated in Table II, application no. 14, columns 5 or 7, , such that the protein or portion thereof maintains the ability to participate in the respective fine chemical production, in particular an activity increasing the level of 5-oxoproline and/or alanine and/or aspartic acid and/or citrulline and/or glycine and/or phenylalanine and/or serine and/or tyrosine as mentioned above or as described in the examples in plants or microorganisms is comprised.

**[0153.0.14.14]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table I, application no. 14, columns 5 or 7, , such that the protein or portion thereof is able to participate in the increase of the respective fine chemical production. In one embodiment, a protein or portion thereof as indicated in Table II, application no. 14, columns 5 or 7, , has for example an activity of a polypeptide indicaded in Table II, application no. 14, column 3.

**[0154.0.14.14]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, application no. 14, columns 5 or 7, and has above-mentioned activity, e.g. conferring preferably the increase of the respective fine chemical.

**[0155.0.0.14] to [0156.0.0.14]: see [0155.0.0.0] to [0156.0.0.0]**

**[0157.0.14.14]** The invention further relates to nucleic acid molecules that differ from one of the nucleotide sequences indicated in Table I, application no. 14, columns 5 or 7 (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, application no. 14, column 7 or of the polypeptide as indicated in Table II, application no. 14, columns 5 or 7 or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, application no. 14, column 7, or of the polypeptide as indicated in Table II, application no. 14, columns 5 or 7, or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, application no. 14, column 7, or of a polypeptide as indicated in Table II, application no. 14, columns 5 or 7, or the functional homologues thereof. However, in a preferred embodiment, the nucleic acid molecule of the present invention does not consist of a sequence as indicated in Table I, application no. 14, columns 5 or 7,. Preferably the nucleic acid molecule of the invention is a functional homologue or identical to a nucleic acid molecule indicated in Table I, application no. 14, columns 5 or 7.

**[0158.0.0.14] to [0160.0.0.14]: see [0158.0.0.0] to [0160.0.0.0]**

**[0161.0.14.14]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 14, columns 5 or 7,. The nucleic acid molecule is preferably at least 20, 30, 50, 100, 250 or more nucleotides in length.

**[0162.0.0.14] see [0162.0.0.0]**

**[0163.0.14.14]** Preferably, nucleic acid molecule of the invention that hybridizes under stringent conditions to a sequence as indicated in Table I, application no. 14, columns 5 or 7, , corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of an protein of the invention or used in the process of the invention.

**[0164.0.0.14] see [0164.0.0.0]**

**[0165.0.14.14]** For example, nucleotide substitutions leading to amino acid substitutions at "non-essential" amino acid residues can be made in a sequence of the nucleic acid molecule of the invention or used in the process of the invention, e.g. as indicated in Table I, application no. 14, columns 5 or 7,.

**[0166.0.0.14] to [0167.0.0.14]: see [0166.0.0.0] to [0167.0.0.0]**

**[0168.0.14.14]** Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 14, columns 5 or 7, , yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 14, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 14, columns 5 or 7, , more preferably at least about 70% identical to one of the sequences as indicated in Table II, application no. 14, columns 5 or 7, , even more preferably at least about 80%, 90%, or 95% homologous to a sequence as indicated in Table II, application no. 14, columns 5 or 7, , and most preferably at least about 96%, 97%, 98%, or 99% identical to the sequence as indicated in Table II, application no. 14, columns 5 or 7,.

**[0169.0.0.14] to [0172.0.0.14]: see [0169.0.0.0] to [0172.0.0.0]**

**[0173.0.14.14]** For example a sequence which has a 80% homology with sequence SEQ ID NO: 302 at the nucleic acid level is understood as meaning a sequence which, upon comparison with the sequence SEQ ID NO: 302 by the above Gap program algorithm with the above parameter set, has a 80% homology.

**[0174.0.0.14] see [0174.0.0.0]**

**[0175.0.14.14]** For example a sequence which has a 80% homology with sequence SEQ ID NO: 303 at the protein level is understood as meaning a sequence which, upon comparison with the sequence SEQ ID NO: 303 by the above program algorithm with the above parameter set, has a 80% homology.

**[0176.0.14.14]** Functional equivalents derived from one of the polypeptides as indicated in Table II, application no. 14, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 14, columns 5 or 7, according to the invention and are distinguished by essentially the same properties as a polypeptide as indicated in Table II, application no. 14, columns 5 or 7,. [0177.0.14.14] Functional equivalents derived from a nucleic acid sequence as indicated in Table I, application no. 14, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of a polypeptide as indicated in Table II, application no. 14, columns 5 or according to the invention and encode polypeptides having essentially the same properties as a polypeptide as indicated in Table II, application no. 14, columns 5 or 7,.

**[0178.0.0.14]: see [0178.0.0.0]**

**[0179.0.14.14]** A nucleic acid molecule encoding an homologous to a protein sequence as indicated in Table II, application no. 14, columns 5 or 7, can be created by introducing one or more nucleotide substitutions, additions or deletions into a nucleotide sequence of the nucleic acid molecule of the present invention, in particular as indicated in Table I, application no. 14, columns 5 or 7, such that one or more amino acid substitutions, additions or deletions are introduced into the encoded protein. Mutations can be introduced into the encoding sequences of a sequences as indicated in Table I, application no. 14, columns 5 or 7, by standard techniques, such as site-directed mutagenesis and PCR-mediated mutagenesis.

[0180.0.0.14] to [0183.0.0.14]: see [0180.0.0.0] to [0183.0.0.0]

**[0184.0.14.14]** Homologues of the nucleic acid sequences used, with a sequence as indicated in Table I, application no. 14, columns 5 or 7, or of the nucleic acid sequences derived from a sequences as indicated in Table II, application no. 14, columns 5 or 7, comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 14, columns 5 or 7, or from the derived nucleic acid sequences, the intention being, however, that the enzyme activity or the biological activity of the resulting proteins synthesized is advantageously retained or increased.

**[0185.0.14.14]** In one embodiment of the present invention, the nucleic acid molecule of the invention or used in the process of the invention comprises one or more sequences as indicated in Table I, application no. 14, columns 5 or 7,. In one embodiment, it is preferred that the nucleic acid molecule comprises as little as possible other nucleotides not shown in any one of sequences as indicated in Table I, application no. 14, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 14, columns 5 or 7, ,

**[0186.0.14.14]** Also preferred is that one or more nucleic acid molecule(s) used in the process of the invention encodes a polypeptide comprising a sequence as indicated in Table II, application no. 14, columns 5 or 7,. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 14, columns 5 or 7,.

**[0187.0.14.14]** In one embodiment, a nucleic acid molecule of the invention or used in the process encodes a polypeptide comprising the sequence as indicated in Table II, application no. 14, columns 5 or 7, comprises less than 100 further nucleotides. In a further embodiment, said nucleic acid molecule comprises less than 30 further nucleotides. In one embodiment, the nucleic acid molecule used in the process is identical to a coding sequence as indicated in Table II, application no. 14, columns 5 or 7.

**[0188.0.14.14]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 14, columns 5 or 7 and is expressed under identical conditions.

**[0189.0.14.14** Homologues of a sequences as indicated in Table I, application no. 14, columns 5 or 7, or of a derived sequences as indicated in Table II, application no. 14, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.14] to [0203.0.0.14]: see [0190.0.0.0] to [0203.0.0.0]**

**[0204.0.14.14]** Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 14, columns 5 or 7, or a fragment thereof conferring an increase in the amount of the respective fine chemical, in particular according to table II B, application no. 14, column 6 in an organism or a part thereof
b) nucleic acid molecule comprising, preferably at least the mature form, of a nucleic acid molecule as indicated in Table I, application no. 14, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, application no. 14, column 7, and conferring an increase in the amount of the respective fine chemical, in particular according to table II B, application no. 14, column 6 in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, application no. 14, columns 5 or 7, and conferring an increase in the amount of the respective fine chemical, in particularaccording to table II, application no. 14, column 6 in an organism or a part thereof;
k) nucleic acid molecule encoding the amino acid sequence of a polypeptide encoding a domaine of a polypeptide as indicated in Table II, application no. 14, columns 5 or 7, and conferring an increase in the amount of the respective fine chemical, in particular according to table II, application no. 14, column 6 in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 14, columns 5 or 7, or a nucleic acid molecule encoding, preferably at least the mature form of, a polypeptide as indicated in Table II, application no. 14, columns 5 or 7, and conferring an increase in the amount of the respective fine chemical according to table II B, application no. 14, column 6 in an organism or a part thereof; or which encompasses a sequence which is complementary thereto;
whereby, preferably, the nucleic acid molecule according to (a) to (I) distinguishes over the sequence indicated in Table I, application no. 14,columns 5 or 7, by one or more nucleotides. In one embodiment, the nucleic acid molecule does not consist of the sequence shown and indicated in Table I, application no. 14, columns 5 or 7,
In one embodiment, the nucleic acid molecule is less than 100%, 99,999%, 99,99%, 99,9% or 99% identical to a sequence indicated in Table I, application no. 14, columns 5 or 7,.
In another embodiment, the nucleic acid molecule does not encode a polypeptide of a sequence indicated in Table II, application no. 14, columns 5 or 7,.
In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 14, columns 5 or 7,.
In a further embodiment the nucleic acid molecule does not encode a polypeptide sequence as indicated in Table II, application no. 14, columns 5 or 7,.
Accordingly, in one embodiment, the nucleic acid molecule of the differs at least in one or more residues from a nucleic acid molecule indicated in Table I, application no. 14, columns 5 or 7,.
Accordingly, in one embodiment, the nucleic acid molecule of the present invention encodes a polypeptide, which differs at least in one or more amino acids from a polypeptide indicated in Table II, application no. 14, columns 5 or 7,.
In another embodiment, a nucleic acid molecule indicated in Table I, application no. 14, columns 5 or 7,.
Accordingly, in one embodiment, the protein encoded by a sequences of a nucleic acid accoriding to (a) to (I) does not consist of a sequence as indicated in Table II, application no. 14, columns 5 or 7,.
In a further embodiment, the protein of the present invention is at least 30 %, 40 %, 50%, or 60% identical to a protein sequence indicated in Table II, application no. 14, columns 5 or 7, , and less than 100%, preferably less than 99,999%, 99,99% or 99,9%, more preferably less than 99%, 985, 97%, 96% or 95% identical to a sequence as indicated in Table I, columns 5 or 7,.

**[0205.0.0.14] to [0226.0.0.14]: see [0205.0.0.0] to [0226.0.0.0]**

**[0227.0.14.14]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table I, application no. 14, columns 5 or 7, or its derivatives, it is advantageous additionally to express and/or mutate further genes in the organisms. Especially advantageously, additionally at least one further gene of the amino acid biosynthetic pathway such as for amino acid precursors is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine a sequence as indicated in Table I, application no. 14, columns 5 or 7, with genes which generally support or enhances to growth or yield of the target organismen, for example genes which lead to faster growth rate of microorganisms or genes which produces stress-, pathogen, or herbicide resistant plants.

**[0228.0.0.14] to [0230.0.0.14]: see [0228.0.0.0] to [0230.0.0.0]**

**[00231.0.14.14]** In a further advantageous embodiment of the process of the invention, the organisms used in the process are those in which simultaneously a protein degrading 5-oxoproline and/or alanine and/or aspartic acid and/or citrulline and/or glycine and/or phenylalanine and/or serine and/or tyrosine resp., is attenuated, in particular by reducing the rate of expression of the corresponding gene.

**[0232.0.0.14] to [0282.0.0.14]: see [0232.0.0.0] to [0282.0.0.0]**

**[0283.0.14.14]** Moreover, a native polypeptide conferring the increase of the respective fine chemical in an organism or part thereof can be isolated from cells (e.g., endothelial cells), for example using the antibody of the present invention as described below, e.g. an antibody against a protein as indicated in Table II, application no. 14, column 3, or an antibody against a polypeptide as indicated in Table II, application no. 14, columns 5 or 7, which can be produced by standard techniques utilizing polypeptides comprising or consisting of above mentioned sequences, e.g. the polypeptid of the present invention or fragment thereof. Preferred are monoclonal antibodies.

**[0284.0.0.14] see [0284.0.0.0]**

**[0285.0.14.14]** In one embodiment, the present invention relates to a polypeptide having a sequence as indicated in Table II, application no. 14, columns 5 or 7, or as coded by a nucleic acid molecule as indicated in Table I, application no. 14, columns 5 or 7, or functional homologues thereof.

**[0286.0.14.14]** In one advantageous embodiment, in the method of the present invention the activity of a polypeptide is increased which comprises or consists of a consensus sequence as indicated in Table IV, application no. 14, column 7,. In another embodiment, the present invention relates to a polypeptide comprising or consisting of a consensus sequence as indicated in Table IV, application no. 14, column 7, , whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, application no. 14, column 7,.

**[0287.0.0.14] to [0290.0.0.14]: see [0287.0.0.0] to [0290.0.0.0]**

**[0291.0.14.14]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II, application no. 14, columns 5 or 7, by one or more amino acids. In one embodiment, polypeptide distinguishes form a sequence as indicated in Table II, application no. 14, columns 5 or 7, by more than 1, 2, 3, 4, 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from a sequence as indicated in Table II, application no. 14, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A, application no. 14, columns 5 or 7,.

**[0292.0.0.14]: see [0292.0.0.0]**

**[0293.0.14.14]** In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II, application no. 14, columns 5 or 7, by one or more amino acids. In an other embodiment, said polypeptide of the invention does not consist of the sequence as indicated in Table II, application no. 14, columns 5 or 7,.
In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 14, columns 5 or 7.

**[0294.0.14.14]** In one embodiment, the present invention relates to a polypeptide having an activity of a protein as indicated in Table II, application no. 14, column 3, which distinguishes over a sequence as indicated in Table II, application no. 14, columns 5 or 7, by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.14] to [0297.0.0.14]: see [0295.0.0.0] to [0297.0.0.0]**

**[0297.1.14.14]** Non-polypeptide of the invention-chemicals are e.g. polypeptides having not the activity of a polypeptide indicated in Table II, application no. 14, columns 3, 5 or 7,.

**[0298.0.14.14]** A polypeptide of the invention can participate in the process of the present invention. The polypeptide or a portion thereof comprises preferably an amino acid sequence which is sufficiently homologous to an amino acid sequence as indicated in Table II, application no. 14, columns 5 or 7, such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, application no. 14, columns 5 or 7,.

**[0299.0.14.14]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, application no. 14, columns 5 or 7.
The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 14, columns 5 or 7, or which is homologous thereto, as defined above.

**[0300.0.14.14]** Accordingly the polypeptide of the present invention can vary from a sequence as indicated in Table II, application no. 14, columns 5 or 7, in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II, application no. 14, columns 5 or 7,.

**[0301.0.0.14] see [0301.0.0.0]**

**[0302.0.14.14]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 14, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.14] see [0303.0.0.0]**

**[0304.0.14.14]** Manipulation of the nucleic acid molecule of the invention may result in the production of a protein having essentially the activity of the polypeptides as indicated in Table II, application no. 14, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.14] to [0308.0.0.14]: see [0305.0.0.0] to [0308.0.0.0]**

**[0309.0.14.14]** In one embodiment, an reference to a " protein (= polypeptide) " of the invention or as indicated in Table II, application no. 14, columns 5 or 7, refers to a polypeptide having an amino acid sequence corresponding to the polypeptide of the invention or used in the process of the invention , whereas a "non-polypeptide of the invention" or "other polypeptide" not being indicated in Table II, application no. 14, columns 5 or 7, refers to a polypeptide having an amino acid sequence corresponding to a protein which is not substantially homologous a polypeptide of the invention, preferably which is not substantially homologous to a as indicated in Table II, application no. 14, columns 5 or 7, , e.g., a protein which does not confer the activity described herein or annotated or known for as indicated in Table II, application no. 14, column 3, and which is derived from the same or a different organism. In one embodmeint, a "non-polypeptide of the invention" or "other polypeptide" not being indicated in Table II, application no. 14, columns 5 or 7, does not confer an increase of the respective fine chemical in an organism or part thereof.

**[0310.0.0.14] to [0334.0.0.14]: see [0310.0.0.0] to [0334.0.0.0]**

**[0335.0.14.14]** The dsRNAi method has proved to be particularly effective and advantageous for reducing the expression of a nucleic acid sequences as indicated in Table I, application no. 14, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, application no. 14, columns 5 or 7, and/or homologs thereof. In a double-stranded RNA molecule for reducing the expression of an protein encoded by a nucleic acid sequence of one of the sequences as indicated in Table I,application no. 14, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.14] to [0342.0.0.14]: see [0336.0.0.0] to [0342.0.0.0]**

**[0343.0.14.14]** As has already been described, 100 % sequence identity between the dsRNA and a gene transcript of a nucleic acid sequence as indicated in Table I, application no. 14, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 14, columns 5 or 7, or homologs thereof of the one organism, may be used to suppress the corresponding expression in another organism.

**[0344.0.0.14] to [0361.0.0.14]: see [0344.0.0.0] to [0361.0.0.0]**

**[0362.0.14.14]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 14, columns 5 or 7, e.g. encoding a polypeptide having protein activity, as indicated in Table II, application no. 14, columns 3,.
Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, application no. 14, columns 5 or 7, means herein that due to modulation or manipulation of the genome, an activity as annotated for a polypeptide as indicated in Table II, application no. 14, column 3, e.g. having a sequence as indicated in Table II, application no. 14, columns 5 or 7 is increased in a cell or an organism or a part thereof. Examples are described above in context with the process of the invention

### [0363.0.0.14] to [0384.0.0.14]: see [0363.0.0.0] to [0384.0.0.0]

**[0385.0.14.14]** The fermentation broths obtained in this way, containing in particular 5-oxoproline, alanine, aspartic acid, citrulline, glycine, , phenylalanine, serine and/or tyrosine, normally have a dry matter content of from 7.5 to 25% by weight. Sugar-limited fermentation is additionally advantageous, at least at the end, but especially over at least 30% of the fermentation time. This means that the concentration of utilizable sugar in the fermentation medium is kept at, or reduced to, 0 to 3 g/I during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.
An other method for purification the amino acids of the invention, in particular 5-oxoproline, alanine, aspartic acid, citrulline, glycine, , phenylalanine, serine and/or tyrosine is a process by means of electrodialysis as described in US 6,551,803.

**[0386.0.0.14] to [0392.0.0.14]: see [0386.0.0.0] to [0392.0.0.0]**

**[0393.0.14.14]** In one embodiment, the present invention relates to a method for the identification of a gene product conferring an increase in the fine chemical production in a cell, comprising the following steps:
(a) contacting, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence as indicated in Table I, application no. 14, columns 5 or 7, and, optionally, isolating the full length cDNA clone or complete genomic clone;
(c) introducing the candidate nucleic acid molecules in host cells, preferably in a plant cell or a microorganism, appropriate for producing the fine chemical;
(d) expressing the identified nucleic acid molecules in the host cells;
(e) assaying the the fine chemical level in the host cells; and
(f) identifying the nucleic acid molecule and its gene product which expression confers an increase in the the fine chemical level in the host cell after expression compared to the wild type.

**[0394.0.0.14] to [0552.0.0.14]: see [0394.0.0.0] to [0552.0.0.0]**

**[0553.0.14.14]** A process for the production of 5-oxoproline, alanine, aspartic acid, citrulline, glycine, phenylalanine, serine and/or tyrosine resp., which comprises
(a) increasing or generating the activity of a protein as indicated in Table II, columns 5 or 7, application no. 14 resp., or a functional equivalent thereof in a non-human organism, or in one or more parts thereof; and
(b) growing the organism under conditions which permit the production of 5-oxoproline, alanine, aspartic acid, citrulline, glycine, phenylalanine, serine and/or tyrosine resp. in said organism.
A process for the production of 5-oxoproline, alanine, aspartic acid, citrulline, glycine, phenylalanine, serine and/or tyrosine resp., comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, columns 5 or 7, application no. 14 resp., or a fragment thereof, which confers an increase in the amount of ccchemical, e.g of 5-oxoproline, anine, aspartic acid, citrulline, glycine, phenylalanine, serine and/or tyrosine resp., in an organism or a part thereof;
b) nucleic acid molecule comprising of a nucleic acid molecule as indicated in Table I, application no. 14, columns 5 or 7, application no. 14 resp.,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 14, column 7, and conferring an increase in the amount of the fine chemical as indicated in table II, column 6, e.g of the 5-oxoproline, alanine, aspartic acid, citrulline, glycine, phenylalanine, serine and/or tyrosine resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof;
h) nucleic acid molecule encoding a polypeptide comprising a consensus sequence as indicated in Table IV, application no. 14, column 7, resp., and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof.
or comprising a sequence which is complementary thereto.
The process as mentioned above, comprising recovering of the free or bound 5-oxoproline, alanine, aspartic acid, citrulline, glycine, phenylalanine, serine and/or tyrosine resp..
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound 5-oxoproline, alanine, aspartic acid, citrulline, glycine, phenylalanine, serine and/or tyrosine resp., produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 14, columns 5 or 7., or a fragment thereof, which confers an increase in the amount of the fine chemical as indicated in table II, column 6, e.g of 5-oxoproline, alanine, aspartic acid, citrulline, glycine, phenylalanine, serine and/or tyrosine resp., in an organism or a part thereof;
b) nucleic acid molecule comprising of a nucleic acid molecule as indicated in Table I, application no. 14, columns 5 or 7, , resp.,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 14, column 7, resp., and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof;
h) nucleic acid molecule encoding a polypeptide comprising a consensus sequence as indicated in Table IV, application no. 14, column 7, resp., and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical as indicated in table II, column 6, e.g of 5-oxoproline, alanine, aspartic acid, citrulline, glycine, phenylalanine, serine and/or tyrosine resp., in an organism or a part thereof.
   whereby the nucleic acid molecule distinguishes over the sequence as indicated in Table I, application no. 14, columns 5 or 7 resp., by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 14, columns 5 or 7, resp., by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of 5-oxoproline, alanine, aspartic acid, citrulline, glycine, phenylalanine, serine and/or tyrosine resp., in an organism or a part thereof comprising:
(a) contacting cells, tissues , plants or microorganisms which express the a polypeptide encoded by the nucleic acid molecule as mentioned above conferring an increase in the amount of 5-oxoproline, alanine, aspartic acid, citrulline, glycine, phenylalanine, serine and/or tyrosine resp., in an organism or a part thereof with a candidate compound or a sample comprising a plurality of compounds under conditions which permit the expression the polypeptide;
(b) assaying the 5-oxoproline, alanine, aspartic acid, citrulline, glycine, phenylalanine, serine and/or tyrosine resp., level or the polypeptide expression level in the cell, tissue, plant or microorganism or the media the cell, tissue, plant or microorganisms is cultured or maintained in; and
(c) identifying a agonist or antagonist by comparing the measured 5-oxoproline, alanine, aspartic acid, citrulline, glycine, phenylalanine, serine and/or tyrosine resp., level or polypeptide expression level with a standard 5-oxoproline, alanine, aspartic acid, citrulline, glycine, phenylalanine, serine and/or tyrosine resp., or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.

A process for the identification of a compound conferring increased 5-oxoproline, alanine, aspartic acid, citrulline, glycine, phenylalanine, serine and/or tyrosine resp., production in a plant or microorganism, comprising the steps:
(a) culturing a plant cell or tissue or microorganism or maintaining a plant expressing the polypeptide encoded by the nucleic acid molecule as mentioned above conferring an increase in the amount of 5-oxoproline, alanine, aspartic acid, citrulline, glycine, phenylalanine, serine and/or tyrosine resp., in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of 5-oxoproline, alanine, aspartic acid, citrulline, glycine, phenylalanine, serine and/or tyrosine rersp., in an organism or a part thereof;
(b) identifying if the compound is an effective agonist by detecting the presence or absence or increase of a signal produced by said readout system.
A method for the identification of a gene product conferring an increase in 5-oxoproline, alanine, aspartic acid, citrulline, glycine, phenylalanine, serine and/or tyrosine resp., production in a cell, comprising the following steps:
(a) contacting the nucleic acid molecules of a sample, which can contain a candidate gene encoding a gene product conferring an increase in 5-oxoproline, alanine, aspartic acid, citrulline, glycine, phenylalanine, serine and/or tyrosine resp., after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing 5-oxoproline, alanine, aspartic acid, citrulline, glycine, phenylalanine, serine and/or tyrosine resp.;
(d) expressing the identified nucleic acid molecules in the host cells;
(e) assaying the 5-oxoproline, alanine, aspartic acid, citrulline, glycine, phenylalanine, serine and/or tyrosine rersp., level in the host cells; and
(f) identifying nucleic acid molecule and its gene product which expression confers an increase in the 5-oxoproline, alanine, aspartic acid, citrulline, glycine, phenylalanine, serine and/or tyrosine resp., level in the host cell in the host cell after expression compared to the wild type.
A method for the identification of a gene product conferring an increase in 5-oxoproline, alanine, aspartic acid, citrulline, glycine, phenylalanine, serine and/or tyrosine resp., production in a cell, comprising the following steps:
(a) identifiying in a data bank nucleic acid molecules of an organism; which can contain a candidate gene encoding a gene product conferring an increase in the 5-oxoproline, alanine, aspartic acid, citrulline, glycine, phenylalanine, serine and/or tyrosine resp., amount or level in an organism or a part thereof after expression, and which are at least 20% homolog to the nucleic acid molecule as mentioned above;
(b) introducing the candidate nucleic acid molecules in host cells appropriate for producing 5-oxoproline, alanine, aspartic acid, citrulline, glycine, phenylalanine, serine and/or tyrosine resp.,;
(c) expressing the identified nucleic acid molecules in the host cells;
(d) assaying the 5-oxoproline, alanine, aspartic acid, citrulline, glycine, phenylalanine, serine and/or tyrosineresp., level in the host cells; and
(e) identifying nucleic acid molecule and its gene product which expression confers an increase in the 5-oxoproline, alanine, aspartic acid, citrulline, glycine, phenylalanine, serine and/or tyrosine resp., level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of 5-oxoproline, alanine, aspartic acid, citrulline, glycine, phenylalanine, serine and/or tyrosine resp., after expression.
Use of the polypeptide as mentioned above or the nucleic acid construct as mentioned above or the gene product identified according to the method as mentioned above for identifying compounds capable of conferring a modulation of 5-oxoproline, alanine, aspartic acid, citrulline, glycine, phenylalanine, serine and/or tyrosine resp., levels in an organism.
Food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the protection of a plant against a 5-oxoproline and/or alanine and/or aspartic acid and/or citrulline and/or glycine and/or homoserine and/or phenylalanine and/or serine and/or tyrosine synthesis inhibiting herbicide.

### New genes for a process for the production of fine chemicals

[0000.0.0.15] In a further embodiment, the present invention relates to a further process for the production of fine chemicals as defined below and the corresponding embodiments as described herein as follows.

[0001.0.0.15] **see [0001.0.0.0]**

[0002.0.15.15] Oils and fats, which chemically are glycerol esters of fatty acids (triacylglycerols (TAGs)), play a major role in nutrition but more and more in nonfood applications such as lubricants, hydraulic oil, biofuel, or oleochemicals for coatings, plasticizer, soaps, and detergents (W. Lohs and W. Friedt, in Designer Oil Crops, D. J. Murphy, Ed. (VCH, Weinheirn, Germany, 1993)). The ideal oil for industrial application would consist of a particular type of fatty acid that could be supplied constantly at a competitively low price as compared with raw materials based on mineral oil products. Furthermore, such a fatty acid may have a reactive group in addition to the carboxyl function to provide an additional target for chemical modifications (Töpfer et al., Science, Vol. 268, 681-686, 1995).

[0003.0.15.15] to **[0004.0.15.15]** [0003.0.7.7] **[0004.0.7.7]**

[0005.0.15.15] Further sources of fatty acids are membrane lipids of organisms. Preferably lipids are phopholipids and/or glycolipids, more preferably glycerophospholipids, galactolipids and/or sphingolipids.

[0006.0.15.15.] Margaric acid was first mentioned in the early 1800s. 1813 M. E. Chevreul discovered that fats are composed of fatty acids and named one of these "margaric acid" because it glistened with lustrous pearly drops that reminded him of the Greek word for pearl, margaron or margarites. In the middle of the 1800s W. H. Heintz showed that "margaric acid" discovered by Chevreul was an indefinite mixture of palmitic and stearic acids.
Today, the term "margaric acid" is the trivial name for heptadecanoic acid (17:0), which is naturally occurring in minor amounts.
The fatty acid with odd number of carbon atoms is present in trace amounts in plants, in triglycerides from Brazil-nut oil, Dracocephalum moldavica oil, Poppy-seed, Palm, Almond, Sunflower or Soyabean. Margaric acid can be isolated from tallow (1%), specially from subcutaneous adipose tissue in subcutaneous fat from lambs.
Margaric acid can be ingredient of satiety agents or fungicide composition. It is further used as ingredient in cosmetics, pharmaceuticals and in feed and food, like baking adjuvants as disclosed in US 20030143312 or accordind to US 20040097392 as component in surfactant systems.
The heptadecanoic acid is mainly used as an internal standard in quantification of fatty acids. It can be further useful in treatment of neurological diseases which may be caused by yeast, fungi or prions based on yeast or fungal etiology (US 6,652,866) or in antikeratolytic-wound healing compositions (US 5,641,814).Heptadecanoic acid was produced up to now in higher amount primarily by organic synthesis.

[0007.0.15.15.] ./.

[0008.0.15.15] **see [0005.0.7.7]**

**[0009.0.15.15.]** Further poly unsaturated ω--3- and/or ω-6-fatty acids important part of animal feed and human food are delta 7, 10 hexadecadienic acid (16:2(*n*-6)) and delta 7, 10, 13 hexadecatrienic acid (16:3(n-3)). Hexadecadienic acid is a minor component of some seed and fish oils, and of plant leaves but the precursor of hexadecatrienic acid 16:3(*n*-3), which is a common constituent of leaf lipids. This acid is known to occur in photosynthetic leaves, such as for example Arabidopsis thaliana, rape leaves, fern lipid, ginko leaves, potato leaves, tomato leaves and spinach. It may also occur in the leaves of Brassicaceae plants, such as horse radish, cabbage, turnip, Chinese mustard, cauliflower and watercress.
In higher plants, the galactolipids contain a high proportion of polyunsaturated fatty acids, up to 95% of which can be linolenic acid (18:3(n-3)). In this instance, the most abundant molecular species of mono- and digalactosyldiacylglycerols must have 18:3 at both sn-I and sn-2 positions of the glycerol backbone. Plants such as the pea, which have 18:3 as almost the only fatty acid in the monogalactosyldiacylglycerols, have been termed "18:3 plants". Other species, and Arabidopsis thaliana is an example, contain appreciable amounts of hexadecatrienoic acid (16:3(n-3)) in the monogalactosyldiacylglycerols, and they are termed "16:3 plants".
As mentioned, polyunsaturated fatty acid are further used in the cosmetic industry. The application US 20030039672 discloses a cosmetic method for treating aged, sensitive, dry, flaky, wrinkled and/or photodamaged skin through topical application of a composition which comprises an unsaturated C16 fatty acid having at least three double bonds, which may be preferably hexadecatrienoic acid.

**[0010.0.15.15.] to [0011.0.15.15] see [0007.0.7.7] to [0008.0.7.7]**

[0012.0.15.15] Accordingly, there is still a great demand for new and more suitable genes which encode enzymes which participate in the biosynthesis of fatty acids and make it possible to produce certain fatty acids specifically on an industrial scale without unwanted byproducts forming. In the selection of genes for biosynthesis two characteristics above all are particularly important. On the one hand, there is as ever a need for improved processes for obtaining the highest possible contents of polyunsaturated fatty acids on the other hand as less as possible byproducts should be produced in the production process.

[0013.0.0.15] **see [0013.0.0.0]**

[0014.0.15.15] Accordingly, in a first embodiment, the invention relates to a process for the production of a fine chemical, whereby the fine chemical is heptadecanoic acid (C17:0, margaric acid) and/or and/or hexadecatrienoic acid, preferably delta 7, 10, 13 hexadecatrienoic acid (C16:3 (n-3), cis 7- cis 10- cis 13-hexadecatrienoic acid, hiragonic acid) or tryglycerides, lipids, oils or fats containing heptadecanoic acid and/or hexadecatrienoic acid, preferably delta 7, 10, 13 hexadecatrienoic acid. Accordingly, in the present invention, the term "the fine chemical" as used herein relates to "heptadecanoic acid and/or hexadecatrienoic acid, preferably delta 7, 10, 13 hexadecatrienoic acid and/or tryglycerides, lipids, oils and/or fats containing heptadecanoic acid and/or hexadecatrienoic acid, preferably delta 7, 10, 13 hexadecatrienoic acid". Further, the term "the fine chemicals" as used herein also relates to fine chemicals comprising heptadecanoic acid and/or hexadecatrienoic acid, preferably delta 7, 10 ,13 hexadecatrienoic acid and/or triglycerides, lipids, oils and/or fats containing heptadecanoic acid and/or hexadecatrienoic acid, preferably delta 7, 10, 13 hexadecatrienoic acid.

[0015.0.15.15] In one embodiment, the term "the fine chemical" means heptadecanoic acid and/or hexadecatrienoic acid, preferably delta 7, 10, 13 hexadecatrienoic acid and/or tryglycerides, lipids, oils and/or fats containing heptadecanoic acid and/or hexadecatrienoic acid, preferably delta 7, 10, 13 hexadecatrienoic acid. Throughout the specification the term "the fine chemical" means heptadecanoic acid and/or hexadecatrienoic acid, preferably delta 7, 10, 13 hexadecatrienoic acid and/or tryglycerides, lipids, oils and/or fats containing heptadecanoic acid and/or hexadecatrienoic acid, preferably delta 7, 10 ,13 hexadecatrienoic and its salts, ester, thioester or heptadecanoic acid and/or hexadecatrienoic acid, preferably delta 7, 10 ,13 hexadecatrienoic acid in free form or bound to other compounds such as triglycerides, glycolipids, phospholipids etc. In a preferred embodiment, the term "the fine chemical" means heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10, 13 hexadecatrienoic acid, in free form or its salts or bound to a glycerol backbone or to glycerol-3-phosphate backbone or to a sphingosine-phosphate backbone or to sphingosine-mono- or oligosaccharide backbone or to a glycerol-3-mono- or disaccharide backbone. Triglycerides, lipids, oils, fats or lipid mixture thereof shall mean any triglyceride, lipid, oil and/or fat containing any bound or free heptadecanoic acid and/or hexadecatrienoic acid, preferably delta 7, 10 ,13 hexadecatrienoic acid for example sphingolipids, phosphoglycerides, lipids, glycerophospholipids, galactolipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride or other fatty acid esters such as acetyl-Coenzym A thioester, which contain further saturated or unsaturated fatty acids in the fatty acid molecule.

[0016.0.15.15] Accordingly, the present invention relates to a process for the production of the respective fine chemical comprising
(a) increasing or generating the activity of one or more
   of a protein as shown in table II, application no. 15, column 3 encoded by the nucleic acid sequences as shown in table I, application no. 15, column 5, in a non-human organism or in one or more parts thereof or
(b) growing the organism under conditions which permit the production of the fine chemical, thus fatty acid of the invention or fine chemicals comprising the fatty acids of the invention, in said organism or in the culture medium surrounding the organism.

**[0017.0.0.15]** to **[0019.0.0.15] see [0017.0.0.0] to** [0019.0.0.0]

[0020.0.15.15] Surprisingly it was found, that the transgenic expression of the Brassica napus protein as indicated in Table II, application no. 15, column 5, line 35 in a plant conferred an increase in Margaric Acid (C17:0) content of the transformed plants. Thus, in one embodiment, said protein or its homologs are used for the production of Margaric Acid (C17:0).
Surprisingly it was found, that the transgenic expression of the Brassica napus protein as indicated in Table II, application no. 15, column 5, line 36 in thaliana plant conferred an increase in Hexadeca-trienoic Acid (C16:3) content of the transformed plants. Thus, in one embodiment, said protein or its homologs are used for the production of Hexadeca-trienoic Acid (C16:3).

[0021.0.15.15] **see [0021.0.0.0]**

[0022.0.15.15] The sequence of YDR513W from Saccharomyces cerevisiae has been published in Jacq et al., Nature 387 (6632 Suppl), 75-78 (1997) and in Goffeau et al., Science 274 (5287), 546-547, 1996 and its cellular activity has been characterized as a glutathione reductase, preferably of the glutaredoxin superfamily. Accordingly, in one embodiment, the process of the present invention comprises the use of a gene product with an activity of the glutaredoxin superfamily, preferably a protein with a glutathione reductase activity or its homolog, e.g. as shown herein, from Saccharomyces cerevisiae or a plant or its homolog, e.g. as shown herein, for the production of the fine chemical, meaning of heptadecanoic acid and/or tryglycerides, lipids, preferably glycerophospholipids, sphingolipids and/or galactolipids, oils and/or fats containing heptadecanoic acid, in particular for increasing the amount of heptadecanoic acid and/or tryglycerides, lipids, preferably glycerophospholipids, sphingolipids and/or galactolipids, oils and/or fats containing margaric acid, preferably heptadecanoic acid in free or bound form in an organism or a part thereof, as mentioned. In one further embodiment the YDR513W protein expression is increased together with the increase of another gene of the lipid biosynthesis pathway, preferably with a gene encoding a protein being involved in the production of fatty acids or encoding a fatty acid transporter protein or a compound, which functions as a sink for the respective fatty acid. In one embodiment, in the process of the present invention the activity of a protein of the glutaredoxin superfamily, preferably of a glutathione reductase is increased or generated, e.g. from Saccharomyces cerevisiae or a plant or a homolog thereof.
The sequence of b3430 from Escherichia coli K12 has been published in Blattner, Science 277(5331), 1453-1474, 1997, and its activity is being defined as a glucose-1-phosphate adenylyltransferase, preferably of the a glucose-1-phosphate adenylyltransferase superfamily. Accordingly, in one embodiment, the process of the present invention comprises the use of a gene product with an activity of the a glucose-1-phosphate adenylyltransferase superfamily, preferably such protein is having a a glucose-1-phosphate adenylyltransferase acitivity from E. coli or a plant or its homolog, e.g. as shown herein, for the production of the respective fine chemical, meaning of hexydecatrienoic acid (C16:3, preferably C16:3 delta 7, 10, 13) and/or tryglycerides, lipids, preferably glycerophospholipids, sphingolipids and/or galactolipids, oils and/or fats containing hexydecatrienoic acid, preferably delta 7, 10,13 hexadecatrienoic acid, in particular for increasing the amount of hexydecatrienoic acid, preferably delta 7, 10, 13 hexadecatrienoic acid and/or tryglycerides, lipids, preferably glycerophospholipids, sphingolipids and/or galactolipids, oils and/or fats containing hexydecatrienoic acid, preferably delta 7, 10 ,13 hexadecatrienoic acid in free or bound form in an organism or a part thereof, as mentioned. In one further embodiment the b3430 protein expression is increased together with the increase of another gene of the lipid biosynthesis pathway, preferably with a gene encoding a protein being involved in the production of galactolipids, preferably of monogalactosyldiacylglycerol In one embodiment, in the process of the present invention the activity of a a glucose-1-phosphate adenylyltransferase is increased or generated, e.g. from E. coli or a plant or a homolog thereof.

**[0022.1.0.15] to [0023.0.0.15] see [0022.1.0.15] to [0023.0.0.15]**

**[0023.1.15.15]** Homologs of the polypeptide disclosed in table II, application no. 15, column 5, may have the activity of a protein as disclosed in table I or II, application no. 15, column 3 and may be the polypetides encoded by the nucleic acid molecules indicated in table I , application no. 15, column 7, resp., or may be the polypeptides indicated in table II, application no. 15, column 7, resp.

**[0024.0.0.15] see [0024.0.0.0]**

**[0025.0.15.15]** In accordance with the invention, a protein or polypeptide has the "activity of an protein as shown in table II, application no. 15, column 3 "if its de *novo* activity, or its increased expression directly or indirectly leads to an increased in the level of the fine chemical indicated in the respective line of table II, application no. 15, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 15, column 3, or which has at least 10% of the original enzymatic or biological activity, preferably 20%, particularly preferably 30%, most particularly preferably 40% in comparison to a protein as shown in the respective line of table II, application no. 15, column 3 of a E. coli or Saccharomyces cerevisae, respectively, protein as mentioned in table I to IV, column 3 respectively and as disclosed in paragraph [0022] of the respective invention.

**[0026.0.0.15] to [0033.0.0.15] see [0026.0.0.0] to [0033.0.0.0]**

**[0034.0.15.15]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 15, column 3 its biochemical or genetical causes and the increased amount of the respective fine chemical.

**[0035.0.0.15] to [0036.0.0.15] see [0035.0.0.0] to [0036.0.0.0]**

[0037.0.15.15] A series of mechanisms exists via which a modification of the a protein, e.g. the polypeptide of the invention can directly or indirectly affect the yield, production and/or production efficiency of the fatty acid.

**[0038.0.0.15] to [0044.0.0.15] see [0038.0.0.0] to [0044.0.0.0]**

**[0045.0.15.15]** In one embodiment, the activity of the protein, as indicated in Table II, columns 5 or 7, application no. 15, is increased conferring an increase of the respective fine chemical, indicated in Table II, application no. 15, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more [0046.0.15.15] In one embodiment, the activity of the protein as indicated in Table II, columns 5 or 7, application no. 15, is increased conferring an increase of the respective fine chemical, indicated in Table II, application no. 15, column 6 of the respective line confers an increase of the respective fine chemical and of further fatty acid or their precursors.

**[0047.0.0.15] and [0048.0.0.15] see [0047.0.0.0] and [0048.0.0.0]**

**[0049.0.15.15]** A protein having an activity conferring an increase in the amount or level of the fine chemical, has in one embodiment the structure of the polypeptide described herein, in particular of the polypeptides comprising the consensus sequence shown in table IV, application no. 15, column 7 or of the polypeptide as shown in the amino acid sequences as disclosed in table II, application no. 15, columns 5 and 7 or the functional homologues thereof as described herein, or is encoded by the nucleic acid molecule characterized herein or the nucleic acid molecule according to the invention, for example by the nucleic acid molecule as shown in table I, application no. 15, columns 5 and 7 or its herein described functional homologues and has the herein mentioned activity.

**[0050.0.15.15]** For the purposes of the present invention, the term "heptadecanoic acid",and/or "hexadecatrienoic acid", preferably "delta 7, 10 ,13 hexadecatrienoic acid" and/or C24:1 fatty acid also encompasses the corresponding salts, such as, for example, the potassium or sodium salts of the above named fatty acids or the salts of the above named fatty acids with amines such as diethylamine or the esters the above named fatty acids.

[0051.0.15.15] Owing to the biological activity of the proteins which are used in the process according to the invention and which are encoded by nucleic acid molecules according to the invention, it is possible to produce compositions comprising the fine chemical, i.e. an increased amount of the free chemical free or bound, e.g fatty acid compositions. Depending on the choice of the organism used for the process according to the present invention, for example a microorganism or a plant, compositions or mixtures of various fatty acids can be produced.

**[0052.0.0.15] see [0052.0.0.0]**

[0053.0.15.15] In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 15, columns 5 and 7 or its homologs activity having herein-mentioned fatty acid of the invention increasing activity; and/or
b) stabilizing a mRNA conferring the increased expression of a protein encoded by the nucleic acid molecule of the invention as shown in table I, application no. 15, columns 5 and 7, e.g. a nucleic acid sequence encoding a polypeptide having the activity of a protein as indicated in table II, application no. 15, columns 5 and 7 or its homologs activity or of a mRNA encoding the polypeptide of the present invention having herein-mentioned fatty acid of the invention increasing activity; and/or
c) increasing the specific activity of a protein conferring the increased expression of a protein encoded by the nucleic acid molecule of the invention or of the polypeptide of the present invention having herein-mentioned fatty acid increasing activity, e.g. of a polypeptide having the activity of a protein as indicated in table II, application no. 15, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned fatty acid of the invention increasing activity, e.g. of a polypeptide having the activity of a protein as indicated in table II, application no. 15, columns 5 and 7 or its homologs activity; and/or
e) stimulating activity of a protein conferring the increased expression of a protein encoded by the nucleic acid molecule of the present invention or a polypeptide of the present invention having herein-mentioned fatty acid of the invention increasing activity, e.g. of a polypeptide having the activity of a protein as indicated in table II, application no. 15, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned fatty acid of the invention increasing activity, e.g. of a polypeptide having the activity of a protein as indicated in table II, application no. 15, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned fatty acid of the invention increasing activity, e.g. of a polypeptide having the activity of a protein as indicated in table II, application no. 15, columns 5 and 7 or its homologs activity; and/or
h) increasing the expression of the endogenous gene encoding the polypeptide of the invention, e.g. a polypeptide having the activity of a protein as indicated in table II, application no. 15, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
k) selecting of organisms with expecially high activity of the proteins of the invention from natural or from mutagenized resources and breeding them into the target organisms, eg the elite crops.

**[0054.0.15.15]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein according to Table II, application no. 15, column 3, or its homologs activity, e.g. as indicated in Table II, application no. 15, columns 5 or 7.

**[0055.0.0.15] to [0069.0.0.15] see [0055.0.0.0] to [0069.0.0.0]**

[0070.0.15.15] Owing to the introduction of a gene or a plurality of genes conferring the expression of the nucleic acid molecule of the invention or the polypeptide of the invention, for example the nucleic acid construct mentioned below into an organism alone or in combination with other genes, it is possible not only to increase the biosynthetic flux towards the end product, but also to increase, modify or create de *novo* an advantageous, preferably novel metabolites composition in the organism, e.g. an advantageous fatty acid composition comprising a higher content of (from a viewpoint of nutritonal physiology limited) fatty acids, like heptadecanoic acid and/or hexadecatrienoic acid, preferably delta 7, 10 ,13 hexadecatrienoic acid.

**[0071.0.15.15] see [0071.0.7.7]**

[0072.0.15.15] By influencing the metabolism thus, it is possible to produce, in the process according to the invention, further advantageous compounds. Examples of such compounds are, in addition to heptadecanoic acid and/or hexadecatrienoic acid, preferably delta 7, 10 ,13 hexadecatrienoic acid, triglycerides, lipids, preferably glycerophospholipids, sphingolipids and/or galactolipids, oils and/or fats containing heptadecanoic acid and/or hexadecatrienoic acid, preferably delta 7, 10 ,13 hexadecatrienoic acid compounds like other fatty acids such as palmitate, stearate, palmitoleate, oleate, linoleate and/or linoleate or erucic acid and/or, arachidonic acid.

**[0073.0.15.15]** Accordingly, in one embodiment, the process according to the invention relates to a process, which comprises:
(a) providing a non-human organism, preferably a microorganism, a non-human animal, a plant or animal cell, a plant or animal tissue or a plant;
(b) increasing an activity of a polypeptide of the invention or a homolog thereof, e.g. as indicated in Table II, application no. 15, columns 5 or 7, , or of a polypeptide being encoded by the nucleic acid molecule of the present invention and described below, i.e. conferring an increase of the respective fine chemical in the organism, preferably in a microorganism, a non-human animal, a plant or animal cell, a plant or animal tissue or a plant,
(c) growing the organism, preferably the microorganism, the non-human animal, the plant or animal cell, the plant or animal tissue or the plant under conditions which permit the production of the fine chemical in the organism, preferably the microorganism, the plant cell, the plant tissue or the plant; and
(d) if desired, recovering, optionally isolating, the free and/or bound the fine chemical and, optionally further free and/or bound fatty acids synthesized by the organism, the microorganism, the non-human animal, the plant or animal cell, the plant or animal tissue or the plant.

**[0074.0.15.15]** The organism, in particular the microorganism, non-human animal, the plant or animal cell, the plant or animal tissue or the plant is advantageously grown in such a way that it is not only possible to recover, if desired isolate the free or bound the fine chemical or the free and bound the fine chemical but as option it is also possible to produce, recover and, if desired isolate, other free or/and bound fatty acids.

**[0075.0.0.15] to [0077.0.0.15] see [0075.0.0.0] to [0077.0.0.0]**

**[0078.0.0.15] to [0084.0.0.15]: see [0078.0.0.0] to [0084.0.0.0]**

[0085.0.15.15]With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as shown in table I, application no. 15, columns 5 or 7, or a derivative thereof, or
b) a genetic regulatory element, for example a promoter, which is functionally linked to the nucleic acid sequence as shown table I, application no. 15, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.15] to [0087.0.0.15] see [0086.0.0.0] to [0087.0.0.0]**

[0088.0.15.15] In an advantageous embodiment of the invention, the organism takes the form of a plant whose fatty acid content is modified advantageously owing to the nucleic acid molecule of the present invention expressed. This is important for plant breeders since, for example, the nutritional value of plants for animals is dependent on the abovementioned fatty acids and the general amount of fatty acids as energy source in feed. After the above mentioned protein activity has been increased or generated, or after the expression of nucleic acid molecule or polypeptide according to the invention has been generated or increased, the transgenic plant generated thus is grown on or in a nutrient medium or else in the soil and subsequently harvested.

**[0089.0.0.15] to [0097.0.0.15] see [0089.0.0.0] to [0097.0.7.7]**

[0098.0.15.15] In a preferred embodiment, the fine chemical (heptadecanoic acid and/or hexadecatrienoic acid, preferably delta 7, 10 ,13 hexadecatrienoic acid) is produced in accordance with the invention and, if desired, is isolated. The production of further fatty acids such as palmitoleic acid, palmitic acid, stearic acid, oleic acid, linoleic acid, nervonic acid and/or linolenic acid mixtures thereof or mixtures of other fatty acids by the process according to the invention is advantageous.

**[0099.0.15.15] to [0102.0.15.15] see [0099.0.7.7] [0102.0.7.7]**

**[0102.1.15.15]** An other analytical method is described by Summit et al, (Proceedings of the Ocean Drilling Program, Scientific Results Volume 169, 2000). The fatty acid methyl esters are analyzed by capillary gas chromatography with flame ionization detection. Various mono- and polyunsaturated fatty acids can further be individually distinguished and quantified in one sample without prior separation by semi-selective HSQC (heteronuclear single quantum coherence)-NMR (Willker et al. , Magn. Reson. Chem. 36, S79-S84 (1998)).
Fatty acid compositions, preferably of monogalactosyl diacylglycerol (MGDG) and digalactosyl diacylglycerol (DGDG) can be investigated using HPLC/ESI-MS combined with in-source (or cone voltage) fragmentation, negative-ion electrospray ionization (ESI) mass spectrometry interfaced with high performance liquid chromatography (HPLC) (Kim et al., Bull. Korean Chem. Soc. 2003, Vol. 24, No. 8).

**[0103.0.15.15]** ln a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide shown in Table II, application no. 15, columns 5 or 7, or a fragment thereof, which confers an increase in the amount of the fine chemical in an organism or a part thereof;
b) nucleic acid molecule comprising, preferably at least the mature form, of the nucleic acid molecule shown in Table I, application no. 15, columns 5 or 7,;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the fine chemical in an organism or a part thereof;
h) nucleic acid molecule comprising a nucleic acid molecule which is obtained by amplifying nucleic acid molecules from a cDNA library or a genomic library using the primers shown in Table III, application no. 15, column 7, and conferring an increase in the amount of the fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the fine chemical in an organism or a part thereof;
j) nucleic acid molecule which encodes a polypeptide comprising the consensus sequence shown in Table IV, application no. 15, columns 7, and conferring an increase in the amount of the fine chemical in an organism or a part thereof;
k) nucleic acid molecule comprising one or more of the nucleic acid molecule encoding the amino acid sequence of a polypeptide encoding a domain of the polypeptide shown in Table II, application no. 15, columns 5 or 7, and conferring an increase in the amount of the fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

[0104.0.15.15] In one embodiment, the nucleic acid molecule used in the process distinguishes over the sequence shown in Table I, application no. 15, columns 5 or 7, by one or more nucleotides or does not consist of the sequence shown in Table I, application no. 15, columns 5 or 7. In one embodiment, the nucleic acid molecule of the present invention is less than 100%, 99,999%, 99,99%, 99,9% or 99% identical to the sequence shown in Table I, application no. 15, columns 5 or 7. In another embodiment, the nucleic acid molecule does not encode a polypeptide of the sequence shown in Table II, application no. 15, columns 5 or 7.

**[0105.0.0.15] to [0107.0.0.15] see [0105.0.0.0] to [0107.0.0.0]**

[0108.0.15.15] Nucleic acid molecules with the sequence shown in Table I, application no. 15, columns 5 or 7, nucleic acid molecules which are derived from the amino acid sequences shown in Table II, application no. 15, columns 5 or 7, or from polypeptides comprising the consensus sequence shown in Table IV, application no. 15, columns 5 or 7, or their derivatives or homologues encoding polypeptides with the enzymatic or biological activity of a polypeptide as indicated in Table I, application no. 15, column 3, 5 or 7, or e.g. conferring a increase in the respective fine chemical after increasing its expression or activity are advantageously increased in the process according to the invention.

[0109.0.15.15] In one embodiment, said sequences are cloned into nucleic acid constructs, either individually or in combination. These nucleic acid constructs enable an optimal synthesis of the fatty acids produced in the process according to the invention.

**[0110.0.0.15] see [0110.0.0.0]**

**[0111.0.0.15] see [0111.0.0.0]**

**[0112.0.15.15]** The nucleic acid molecules used in the process according to the invention take the form of isolated nucleic acid sequences, which encode polypeptides with an activity of a polypeptide as indicated in Table I, application no. 15, column 3, or having the sequence of a polypeptide as indicated in Table II, application no. 15, columns 5 and 7, and conferring an increase of the respective fine chemical.

**[0113.0.0.15] to [0120.0.0.15] see [0113.0.0.0] to [0120.0.0.0]**

**[0121.0.15.15]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 15, columns 5 or 7, or the functional homologues thereof as described herein, preferably conferring above-mentioned activity, i.e.
conferring an increase in the level of heptadecanoic acid after increasing the activity of the polypeptide sequences indicated in Table II, columns 5 or 7, application no. 15;
or conferring increase in the level of hexadecatrienoic acid, preferably delta 7, 10 ,13 hexadecatrienoic acid after increasing the activity of the polypeptide sequences indicated in Table II, application no. 15, columns 5 or 7.

**[0122.0.0.15] to [0127.0.0.15] see [0122.0.0.0] to [0127.0.0.0]**

**[0128.0.15.15]** Synthetic oligonucleotide primers for the amplification, e.g. as the pairs indicated in Table III, application no. 15, columns 7, resp., by means of polymerase chain reaction can be generated on the basis of a sequence shown herein, for example the sequence as indicated in Table I, application no. 15, columns 5 or 7, resp., or the sequences derived from sequences as indicated in Table II, application no. 15, columns 5 or 7, resp..

**[0129.0.15.15]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consensus sequences indicated in Table IV, application no. 15, columns 7, resp., are derived from said alignments.

[0130.0.15.15] Degenerated primers can then be utilized by PCR for the amplification of fragments of novel proteins having above-mentioned activity, e.g. conferring the increase of fatty acids, e.g. of heptadecanoic acid and/or hexadecatrienoic acid, preferably delta 7, 10 ,13 hexadecatrienoic acid after increasing its expression or activity of the protein comprising said fragment.

**[0131.0.0.15] to [0138.0.0.15] see [0131.0.0.0] to [0138.0.0.0]**

[0139.0.15.15] Polypeptides having above-mentioned activity, i.e. conferring an increase of the respective fine chemical level, derived from other organisms, can be encoded by other DNA sequences which hybridize to a sequences indicated in Table I, columns 5 or 7resp., under relaxed hybridization conditions and which code on expression for peptides having the heptadecanoic acid and/or hexadecatrienoic acid, preferably delta 7, 10 ,13 hexadecatrienoic acid increasing activity.

**[0140.0.0.15] to [0146.0.0.15]: see [0140.0.0.0] to [0146.0.0.0]**

[0147.0.15.15] Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 15, columns 5 or 7, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.15.15]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence shown in Table I, application no. 15, columns 5 or 7, or a portion thereof and preferably has above mentioned activity, in particular having a heptadecanoic acid hexadecatrienoic acid, preferably delta 7, 10 ,13 hexadecatrienoic acid delta 15 fatty acid -increasing activity after increasing its activity or an activity of a product of a gene encoding said sequence or its homologs.

**[0149.0.15.15]** The nucleic acid molecule of the invention comprises a nucleotide sequence which hybridizes, preferably hybridizes under stringent conditions as defined herein, to one of the nucleotide sequences indicated in Table I, application no. 15, columns 5 or 7, or a portion thereof and encodes a protein having above-mentioned activity, e.g. conferring an increase of the respective fine chemical, e.g. of heptadecanoic acid and/or 2 and/or hexadecatrienoic acid, preferably delta 7, 10, 13 hexadecatrienoic acid and optionally the activity of a protein indicated in Table II, application no. 15, column 5,

**[00149.1.15.15]** Optionally, the nucleotide sequence, which hybridises to one of the nucleotide sequences indicated in Table I, columns 5 or 7, has further one or more of the activities annotated or known for the a protein as indicated in Table II, application no. 15, column 3,

**[0150.0.15.15]** Moreover, the nucleic acid molecule of the invention can comprise only a portion of the coding region of one of the sequences shown in Table I, application no. 15, columns 5 or 7, resp., for example a fragment which can be used as a probe or primer or a fragment encoding a biologically active portion of the polypeptide of the present invention or of a polypeptide used in the process of the present invention, i.e. having above-mentioned activity, e.g.conferring an increase of heptadecanoic acid and/or hexadecatrienoic acid, preferably delta 7, 10 ,13 hexadecatrienoic acid if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., in Table I, application no. 15, columns 5 or 7, an anti-sense sequence of one of the sequences, e.g., set forth in Table I, application no. 15, columns 5 or 7, resp., or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primers shown in table III, application no. 15, column 7, resp., will result in a fragment of a polynucleotide sequence as indicated in Table I, application no. 15, columns 5 or 7.

**[0151.0.0.15] see [0151.0.0.0]**

**[0152.0.15.15]** The nucleic acid molecule of the invention encodes a polypeptide or portion thereof which includes an amino acid sequence which is sufficiently homologous to an amino acid sequence as indicated in Table II, application no. 15, columns 5 or 7, resp.,such that the protein or portion thereof maintains the ability to participate in the respective fine chemical production, in particular an activity increasing the level of amino acids, in particular of heptadecanoic acid and/or hexadecatrienoic acid, preferably delta 7, 10, 13 hexadecatrienoic acid as mentioned above or as described in the examples in plants or microorganisms is comprised.

[0153.0.15.15] As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table I, application no. 15, columns 5 or 7, such that the protein or portion thereof is able to participate in the increase of the respective fine chemical production.
In one embodiment, a protein or portion thereof as indicated in Table II, application no. 15, columns 5 or 7, has for example an activity of a polypeptide indicated in Table II, application no. 15, column 3.

**[0154.0.15.15]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, application no. 15, columns 5 or 7, and has above-mentioned activity, e.g. conferring preferably the increase of the respective fine chemical.

**[0155.0.0.15] to [0156.0.0.15]: see [0155.0.0.0] to [0156.0.0.0]**

[0157.0.15.15] The invention further relates to nucleic acid molecules that differ from one of a nucleotide sequences as indicated in Table I, application no. 15, columns 5 or 7,,(and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides encoded by the sequences as indicated in Table IV, application no. 15, columns , or of the polypeptide as indicated in Table II, application no. 15, columns 5 or 7, or their functional homologues. Advantageously, the nucleic acid molecule of the invention comprises, or in an other embodiment has, a nucleotide sequence encoding a protein comprising, or in an other embodiment having, a consensus sequences as indicated in Table IV, application no. 15, columns 7, or of the polypeptide as as indicated in Table II, columns 5 or 7, or the functional homologues. In a still further embodiment, the nucleic acid molecule of the invention encodes a full length protein which is substantially homologous to an amino acid sequence comprising a consensus sequence as indicated in Table IV, application no. 15, columns 5 or 7, or of a polypeptide as indicated in Table II, application no. 15, columns 5 or 7, or the functional homologues thereof. However, in a preferred embodiment, the nucleic acid molecule of the present invention does not consist of a sequence as indicated in Table I, application no. 15, columns 5 or 7.

**[0158.0.0.15] to [0160.0.0.15]: see [0158.0.0.0] to [0160.0.0.0]**

[0161.0.15.15] Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 15, columns 5 or 7, The nucleic acid molecule is preferably at least 20, 30, 50, 100, 250 or more nucleotides in length.

**[0162.0.0.15] see [0162.0.0.0]**

[0163.0.15.15] Preferably, nucleic acid molecule of the invention that hybridizes under stringent conditions to a sequence as indicated in Table I, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.15] see [0164.0.0.0]**

[0165.0.15.15] For example, nucleotide substitutions leading to amino acid substitutions at "non-essential" amino acid residues can be made in a sequence of the nucleic acid molecule of the invention or used in the process of the invention, e.g. as indicated in Table I, application no. 15, columns 5 or 7,.

**[0166.0.0.15] to [0167.0.0.15] see [0166.0.0.0] to [0167.0.0.0]**

[0168.0.15.15] Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table II, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 15, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 15, columns 5 or 7more preferably at least about 70% identical to one of the sequences as indicated in Table II, application no. 15, columns 5 or 7, even more preferably at least about 80%, 90%, or 95% homologous to a sequence as indicated in Table II, application no. 15, columns 5 or 7and most preferably at least about 96%, 97%, 98%, or 99% identical to the sequence as indicated in Table II, application no. 15, columns 5 or 7.

**[0169.0.0.15] to [0172.0.0.15] see [0169.0.0.0] to [0172.0.0.0]**

**[0173.0.15.15] to [0175.0.15.15] see [0173.0.7.7] to [0175.0.7.7]**

**[0176.0.15.15]** Functional equivalents derived from one of the polypeptides as indicated in Table II, application no. 15, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 15, columns 5 or 7, according to the invention and are distinguished by essentially the same properties as a polypeptide as indicated in Table II, application no. 15, columns 5 or 7.

[0177.0.15.15] Functional equivalents derived from a nucleic acid sequence as indicated in Table I, application no. 15, columns 5 or 7, , according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of a polypeptides as indicated in Table II, application no. 15, columns 5 or 7according to the invention and encode polypeptides having essentially the same properties as a polypeptide as indicated in Table II, application no. 15, columns 5 or 7

**[0178.0.0.15] see [0178.0.0.0]**

[0179.0.15.15] A nucleic acid molecule encoding a homologous to a protein sequence of as indicated in Table II, application no. 15, columns 5 or 7, can be created by introducing one or more nucleotide substitutions, additions or deletions into a nucleotide sequence of the nucleic acid molecule of the present invention, in particular as indicated in Table IB , application no. 15, columns 5 or 7 such that one or more amino acid substitutions, additions or deletions are introduced into the encoded protein. Mutations can be introduced into the encoding sequences of a sequences as indicated in Table I, application no. 15, columns 5 or 7, by standard techniques, such as site-directed mutagenesis and PCR-mediated mutagenesis.

**[0180.0.0.15] to [0183.0.0.15] see [0180.0.0.0] to [0183.0.0.0]**

**[0184.0.15.15]** Homologues of the nucleic acid sequences used, with a sequence as indicated in Table I, application no. 15, columns 5 or 7, or of the nucleic acid sequences derived from a sequences as indicated in Table II, columns 5 or 7, comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, or from the derived nucleic acid sequences, the intention being, however, that the enzyme activity or the biological activity of the resulting proteins synthesized is advantageously retained or increased.

[0185.0.15.15] In one embodiment of the present invention, the nucleic acid molecule of the invention or used in the process of the invention comprises one or more sequences as indicated in Table I, columns 5 or 7, In one embodiment, it is preferred that the nucleic acid molecule comprises as little as possible other nucleotide sequences not shown in any one of sequences as indicated in Table I, application no. 15, columns 5 or 7, In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 15, columns 5 or 7,

[0186.0.15.15] Also preferred is that one or more nucleic acid molecule(s) used in the process of the invention encode a polypeptide comprising a sequence as indicated in Table II, application no. 15, columns 5 or 7, In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 15, columns 5 or 7,.

**[0187.0.15.15]** In one embodiment, the nucleic acid molecule of the invention or used in the process encodes a polypeptide comprising a sequence as indicated in Table II, application no. 15, columns 5 or 7, comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 15, columns 5 or 7,.

**[0188.0.15.15]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 15, columns 5 or 7, preferably compared to a sequence as indicated in Table II, application no. 15, column 5, and expressed under identical conditions.

**[0189.0.15.15]** Homologues of a sequences as indicated in Table I, application no. 15, columns 5 or 7, or of a derived sequences as indicated in Table II, application no. 15, columns 5 or 7, also mean truncated sequences, cDNA, single-stranded DNA or RNA of the coding and noncoding DNA sequence. Homologues of said sequences are also understood as meaning derivatives which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.15] to [0203.0.0.15] see [0192.0.0.0] to [0203.0.0.0]**

[0204.0.15.15] Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, application no. 15, columns 5 or 7 or a fragment thereof conferring an increase in the amount of the respective fine chemical, i.e. heptadecanoic acid and/or and/or hexadecatrienoic acid, preferably delta 7, 10 ,13 hexadecatrienoic acid in an organism or a part thereof;
b) nucleic acid molecule comprising, preferably at least the mature form, of a nucleic acid molecule as indicated in Table I, application no. 15, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof;
h) nucleic acid molecule comprising a nucleic acid molecule which is obtained by amplifying a cDNA library or a genomic library using primers or primer pairs as indicated in Table lll, application no. 15, column 7, and conferring an increase in the amount of the respective fine chemical, i.e. heptadecanoic acid and/or hexadecatrienoic acid, preferably delta 7, 10 ,13 hexadecatrienoic acid in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, application no. 15, column 7, and conferring an increase in the amount of the respective fine chemical i.e. heptadecanoic acid and/or hexadecatrienoic acid, preferably delta 7, 10, 13 hexadecatrienoic acid preferably C24:1 delta 15 fatty acid in an organism or a part thereof;
k) nucleic acid molecule encoding the amino acid sequence of a polypeptide encoding a domaine of a polypeptide as indicated in Table II, application no. 15, columns 5 or 7, and conferring an increase in the amount of the respective fine chemical i.e. heptadecanoic acid and/or hexadecatrienoic acid, preferably delta 7, 10 ,13 hexadecatrienoic acid in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 15, columns 5 or 7, or a nucleic acid molecule encoding, preferably at least the mature form of, the polypeptide as indicated in Table II, application no. 15, columns 5 or 7, and conferring an increase in the amount of the respective fine chemical in an organism or a part thereof;
whereby, preferably, the nucleic acid molecule according to (a) to (l) distinguishes over a sequence depicted in as indicated in Table I, application no. 15, columns 5 or 7, by one or more nucleotides. In one embodiment, the nucleic acid molecule of the invention does not consist of a sequence as indicated in Table I, application no. 15, columns 5 or 7, In an other embodiment, the nucleic acid molecule of the present invention is at least 30 % identical and less than 100%, 99,999%, 99,99%, 99,9% or 99% identical to a sequence indicated in Table I, application no. 15, columns 5 or 7, In a further embodiment the nucleic acid molecule does not encode a polypeptide sequence as indicated in Table II, application no. 15, columns 5 or 7, Accordingly, in one embodiment, the nucleic acid molecule of the present invention encodes in one embodiment a polypeptide which differs at least in one or more amino acids from a polypeptide indicated in Table II, application no. 15, columns 5 or 7, In another embodiment, a nucleic acid molecule indicated in Table I, application no. 15, columns 5 or 7, does not encode a protein of a sequence indicated in Table II, application no. 15, columns 5 or 7, Accordingly, in one embodiment, the protein encoded by a sequences of a nucleic acid according to (a) to (I) does not consist of a sequence as indicated in Table II, application no. 15, columns 5 or 7, In a further embodiment, the protein of the present invention is at least 30 % identical to a protein sequence indicated in Table II, application no. 15, columns 5 or 7, and less than 100%, preferably less than 99,999%, 99,99% or 99,9%, more preferably less than 99%, 985, 97%, 96% or 95% identical to a sequence as indicated in Table I, application no. 15, columns 5 or 7.

**[0205.0.0.15] to [0206.0.0.15]: see [0205.0.0.0] to [0206.0.0.0]**

[0207.0.15.15] As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes are genes of the fatty acid metabolism, amino acid metabolism, of glycolysis, of the tricarboxylic acid metabolism, of triacylglycerol or lipid, preferably glycerophospholipids, sphingolipids and/or galactolipids biosynthesis or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.15] to [0226.0.0.15] see [0208.0.0.0] to [0226.0.0.0]**

[0227.0.15.15] The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.

In addition to the sequence mentioned in Table I, application no. 15, columns 5 or 7, or its derivatives, it is advantageous additionally to express and/or mutate further genes in the organisms. Especially advantageously, additionally at least one further gene of the fatty acid biosynthetic pathway such as for palmitate, stearate, palmitoleate, oleate, linoleate and/or linolenate or of the of triacylglycerol or lipid, preferably glycerophospholipids, sphingolipids and/or galactolipids biosynthetic pathway is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine the sequences shown in Table I, application no. 15, columns 5 or 7, with genes which generally support or enhances to growth or yield of the target organism, for example genes which lead to faster growth rate of microorganisms or genes which produces stress-, pathogen, or herbicide resistant plants.

**[0228.0.15.15] to [0229.0.15.15] see [0228.0.7.7] [0229.0.7.7]**

[0229.1.15.15] Further nucleic acid sequences which can be expressed in combination with the sequences used in the process and/or the abovementioned biosynthesis genes are the sequences encoding further genes of the saturated, unsaturated, polyunsaturated and/or hydroxylated fatty acid biosynthesis such as the FA2H gene encoding a fatty acid 2-hydroxylase (Alderson et al., J. Biol. Chem. Vol. 279, No. 47, Issue of November 19, pp. 48562-48568, 2004), ω-3-desaturases encoded by FAD2 and FAD3 (US 20030221217), delta-12 fatty acid desaturase, delta-15 fatty acid desaturase as disclosed by Okuley, et al., Plant Cell 6:147-158 (1994), Lightner et al., WO94/11516, Yadav, N., et al., Plant Physiol., 103:467-476 (1993), WO 93/11245 and Arondel, V. et al., Science, 258:1353-1355 (1992), US 20040083503.

**[0230.0.0.15] see [0230.0.0.0]**

**[0231.0.15.15]** In a further advantageous embodiment of the process of the invention, the organisms used in the process are those in which simultaneously a heptadecanoic acid and/or and/or hexadecatrienoic acid, preferably delta 7, 10 ,13 hexadecatrienoic acid, preferably C24:1 delta 15 fatty acid degrading protein is attenuated, in particular by reducing the rate of expression of the corresponding gene.

**[0232.0.0.15] to [0276.0.0.15] see [0232.0.0.0] to [0276.0.0.0]**

**[0277.0.15.15] to [0282.0.0.15]see [0277.0.7.7] to [0282.0.7.7]**

[0283.0.15.15] Moreover, a native polypeptide conferring the increase of the fine chemical in an organism or part thereof can be isolated from cells (e.g., endothelial cells), for example using the antibody of the present invention as described below, in particular, an antibody against a protein as indicated in Table II, application no. 15, column 3, e.g. an antibody against a polypeptide as indicated in Table II, application no. 15, columns 5 or 7, which can be produced by standard techniques utilizing polypeptides comprising or consisting of above mentioned sequences, e.g. the polypeptide of the present invention or fragment thereof. Preferred are monoclonal antibodies.

**[0284.0.0.15] see [0284.0.0.0]**

**[0285.0.15.15]** In one embodiment, the present invention relates to a polypeptide having a sequence as indicated in Table II, application no. 15, columns 5 or 7, or as coded by a nucleic acid molecule as indicated in Table I, application no. 15, columns 5 or 7, or functional homologues thereof.

**[0286.0.15.15]** In one advantageous embodiment, in the method of the present invention the activity of a polypeptide is increased which comprises or consists of a consensus sequence as indicated in Table IV, application no. 15, column 7, and in one another embodiment, the present invention relates to a polypeptide comprising or consisting of a consensus sequence as indicated in Table IV, application no. 15, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid, or, in an further embodiment, can be replaced and/or absent. In one embodiment, the present invention relates to the method of the present invention comprising a polypeptide or to a polypeptide comprising more than one consensus sequences (of an individual line) as indicated in Table IV, application no. 15, column 7.

**[0287.0.0.15] to [0290.0.0.15] see [0287.0.0.0] to [0290.0.0.0]**

[0291.0.15.15] In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II, application no. 15, columns 5 or 7, by one or more amino acids. In one embodiment, polypeptide distinguishes form a sequence as indicated in Table II, application no. 15, columns 5 or 7, by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 15, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table.

**[0292.0.0.15] see [0292.0.0.0]**

[0293.0.15.15] In one embodiment, the invention relates to polypeptide conferring an increase in the respective fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table II, application no. 15, column 5 and/or 7, by one or more amino acids. In an other embodiment, said polypeptide of the invention does not consist of the sequence as indicated in Table II, application no. 15, columns 5 or 7,. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 15, columns 5 or 7,.

[0294.0.15.15] In one embodiment, the present invention relates to a polypeptide having an activity of a protein as indicated in Table II, application no. 15, column 3, which distinguishes over a sequence as indicated in Table II, application no. 15, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.15] to [0297.0.0.15] see [0295.0.0.0] to [0297.0.0.0]**

**[00297.1.15.15]** Non-polypeptide of the invention-chemicalsare e.g. polypeptides having not the activity of a polypeptide indicated in Table II, application no. 15, columns 5 or 7.

**[0298.0.15.15]** A polypeptide of the invention can participate in the process of the present invention. The polypeptide or a portion thereof comprises preferably an amino acid sequence which is sufficiently homologous to an amino acid sequence as indicated in Table II, application no. 15, columns 5 or 7, such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, application no. 15, columns 5 or 7.

[0299.0.15.15] Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91 %, 92%, 93%, 94% or 95%, and even more preferably at least about 96%, 97%, 98%, 99% or more homologous to one of the amino acid sequences as shown in Table II, application no. 15, columns 5 or 7,. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table I, application no. 15, columns 5 or 7, , or which is homologous thereto, as defined above.

**[0300.0.15.15]** Accordingly the polypeptide of the present invention can vary from the sequences shown in Table II, application no. 15, columns 5 or 7, , in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91 %, 92%, 93%, 94% or 95%, and most preferably at least about 96%, 97%, 98%, 99% or more homologous to an entire amino acid sequence shown in table II, application no. 15, columns 5 or 7,.

**[0301.0.0.15] see [0301.0.0.0]**

[0302.0.15.15] Biologically active portions of an polypeptide of the present invention include peptides comprising amino acid sequences derived from the amino acid sequence of the polypeptide of the present invention or used in the process of the present invention, e.g., the amino acid sequence shown in Table II, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.15] see [0303.0.0.0]**

**[0304.0.15.15]** Manipulation of the nucleic acid molecule of the invention or the nucleic acid molecule used in the method of the invention, may result in the production of a protein having essentially the activity of the polypeptides as indicated in Table, application no. 15, column 3, , but having differences in the sequence from said wild-type protein. These proteins may be improved in efficiency or activity, may be present in greater numbers in the cell than is usual, or may be decreased in efficiency or activity in relation to the wild type protein.

**[0305.0.0.15] to [0308.0.0.15] see [0305.0.0.0] to [0308.0.0.0]**

**[0309.0.15.15]** In one embodiment, an reference to a " protein (= polypeptide)" of the invention or as indicated in Table II, application no. 15, columns 5 or 7, , refers to a polypeptide having an amino acid sequence corresponding to the polypeptide of the invention or used in the process of the invention , whereas a "non-polypeptide of the invention" or "other polypeptide" not being indicated in Table II, application no. 15, columns 5 or 7, refers to a polypeptide having an amino acid sequence corresponding to a protein which is not substantially homologous a polypeptide of the invention, preferably which is not substantially homologous to a as indicated in Table II, application no. 15, columns 5 or 7, e.g., a protein which does not confer the activity described herein or annotated or known for as indicated in Table II, application no. 15, column 3, and which is derived from the same or a different organism. In one embodmeint, a "non-polypeptide of the invention" or "other polypeptide" not being indicated in Table II, application no. 15, columns 5 or 7, does not confer an increase of the respective fine chemical in an organism or part thereof.

**[0310.0.0.15] to [0334.0.0.15] see [0310.0.0.0] to [0334.0.0.0]**

[0335.0.15.15] The dsRNAi method has proved to be particularly effective and advantageous for reducing the expression of the nucleic acid sequences of the table I, application no. 15, columns 5 or 7 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of the nucleic acid sequences of the Table I, application no. 15, columns 5 or 7, and/or homologs thereof. In a double-stranded RNA molecule for reducing the expression of an protein encoded by a nucleic acid sequence of one of the Table I, application no. 15, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.15] to [0342.0.0.15] see [0336.0.0.0] to [0342.0.0.0]**

[0343.0.15.15] As has already been described, 100 % sequence identity between the dsRNA and a gene transcript of a nucleic acid sequence of one of the sequences shown in Table I, application no. 15, columns 5 or 7, , or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of sequences shown in Table I, application no. 15, columns 5 or 7, or homologs thereof of the one organism, may be used to suppress the corresponding expression in another organism.

**[0344.0.0.15] to [0361.0.0.15] see [0344.0.0.0] to [0361.0.0.0]**

[0362.0.15.15] Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 15, columns 5 or 7,. Due to the above mentioned activity the fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. In one embodiment, transgenic for a polypeptide having an activity of a polypeptide as indicated in Table II, application no. 15, columns 5 or 7, means herein that due to modulation or manipulation of the genome, an activity as annotated for a polypeptide as indicated in Table II, application no. 15, column 3, , e.g. having a sequence as indicated in Table II, application no. 15, columns 5 or 7, is increased in a cell or an organism or a part thereof. Examples are described above in context with the process of the invention.

**[0363.0.0.15] see [0363.0.0.0]**

[0364.0.15.15]A naturally occurring expression cassette - for example the naturally occurring combination of a promoter of a polypeptide of the invention as indicated in Table II, application no. 15, column 3 and/or 5, , with the corresponding protein-encoding sequence as indicated in Table I, application no. 15, column 3 and/or 5.

**[0365.0.0.15] to [0378.0.0.15]see [0365.0.7.7] to [0378.0.7.7]**

[0379.0.15.15] In one embodiment, the amino acid, in particular, heptadecanoic acid and/or hexadecatrienoic acid, preferably delta 7, 10 ,13 hexadecatrienoic acid resp., is a mixture comprising of one or more the respective fine chemicals. In one embodiment, the respective fine chemical means here amino acid, in particular heptadecanoic acid and/or hexadecatrienoic acid, preferably delta 7, 10 ,13 hexadecatrienoic acid. In one embodiment, amino acid means here a mixture of the respective fine chemicals.

**[0380.0.0.15] to [0392.0.0.15] see [0380.0.7.7] to [0392.0.7.7]**

[0393.0.15.15] In one embodiment, the present invention relates to a method for the identification of a gene product conferring an increase in the fine chemical production in a cell, comprising the following steps:
(a) contacting-, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence shown in table I, application no. 15, columns 5 or 7, , and, optionally, isolating the full length cDNA clone or complete genomic clone;
(c) introducing the candidate nucleic acid molecules in host cells, preferably in a plant cell or a microorganism, appropriate for producing the fine chemical;
(d) expressing the identified nucleic acid molecules in the host cells;
(e) assaying the fine chemical level in the host cells; and
(f) identifying the nucleic acid molecule and its gene product which expression confers an increase in the fine chemical level in the host cell after expression compared to the wild type.

**[0394.0.0.15] to [0552.0.0.15] see [0394.0.0.7] to [0552.0.0.7]**

[0553.0.15.15] A process for the production of heptadecanoic acid and/or hexadecatrienoic acid, preferably delta 7, 10 ,13 hexadecatrienoic acid which comprises
(a) increasing or generating the activity of a protein as indicated in Table II application no. 15, columns 5 or 7, or a functional equivalent thereof in a non-human organism, or in one or more parts thereof; and
(b) growing the organism under conditions which permit the production of heptadecanoic acid and/or hexadecatrienoic acid, preferably delta 7, 10, 13 hexadecatrienoic acid in said organism.
A process for the production of heptadecanoic acid and/or hexadecatrienoic acid, preferably delta 7, 10 ,13 hexadecatrienoic acid, comprising the increasing or generating in an organism or a part thereof the expression of at least one nucleic acid molecule comprising a nucleic acid molecule selected from the group consisting of:
a) nucleic acid molecule encoding of the polypeptide shown in table II, application no. 15, columns 5 or 7,, or a fragment thereof, which confers an increase in the amount of heptadecanoic acid and/or hexadecatrienoic acid, preferably delta 7, 10 ,13 hexadecatrienoic acid in an organism or a part thereof;
b) nucleic acid molecule comprising of the nucleic acid molecule shown in table I, application no. 15, columns 5 or 7,;
c) nucleic acid molecule whose sequence can be deduced from a polypeptide sequence encoded by a nucleic acid molecule of (a) or (b) as a result of the degeneracy of the genetic code and conferring an increase in the amount of heptadecanoic acid and/or hexadecatrienoic acid, preferably delta 7, 10 ,13 hexadecatrienoic acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of heptadecanoic acid and/or hexadecatrienoic acid, preferably delta 7, 10, 13 hexadecatrienoic acid in an organism or a part thereof;
e) nucleic acid molecule which hybidizes with a nucleic acid molecule of (a) to (c) under under stringent hybridisation conditions and conferring an increase in the amount of heptadecanoic acid and/or hexadecatrienoic acid, preferably delta 7, 10 ,13 hexadecatrienoic acid in an organism or a part thereof;
f) nucleic acid molecule which encompasses a nucleic acid molecule which is obtained by amplifying nucleic acid molecules from a cDNA library or a genomic library using the primers as shown in table III, application no. 15, column 7, and conferring an increase in the amount of the fine chemical in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of heptadecanoic acid and/or hexadecatrienoic acid, preferably delta 7, 10, 13 hexadecatrienoic acid in an organism or a part thereof;
h) nucleic acid molecule encoding a polypeptide comprising the consensus sequence shown in table IV, application no. 15, column 7,., and conferring an increase in the amount of the fine chemical in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
or comprising a sequence which is complementary thereto.
The process as mentioned above, comprising recovering of the free or bound heptadecanoic acid and/or hexadecatrienoic acid, preferably delta 7, 10 ,13 hexadecatrienoic acid.
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound heptadecanoic acid and/or hexadecatrienoic acid, preferably delta 7, 10 ,13 hexadecatrienoic acid produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of the polypeptide shown in table II, application no. 15, columns 5 or 7, or a fragment thereof, which confers an increase in the amount of heptadecanoic acid and/or hexadecatrienoic acid, preferably delta 7, 10 ,13 hexadecatrienoic acid in an organism or a part thereof;
b) nucleic acid molecule comprising of the nucleic acid molecule shown in table I, application no. 15, columns 5 or 7,;
c) nucleic acid molecule whose sequence can be deduced from a polypeptide sequence encoded by a nucleic acid molecule of (a) or (b) as a result of the degeneracy of the genetic code and conferring an increase in the amount of heptadecanoic acid and/or hexadecatrienoic acid, preferably delta 7, 10 ,13 hexadecatrienoic acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of heptadecanoic acid and/or hexadecatrienoic acid, preferably delta 7, 10, 13 hexadecatrienoic acid in an organism or a part thereof;
e) nucleic acid molecule which hybidizes with a nucleic acid molecule of (a) to (c) under stringent hybridisation conditions and conferring an increase in the amount of heptadecanoic acid and/or hexadecatrienoic acid, preferably delta 7, 10 ,13 hexadecatrienoic acid in an organism or a part thereof;
f) nucleic acid molecule which encompasses a nucleic acid molecule which is obtained by amplifying nucleic acid molecules from a cDNA library or a genomic library using the primers as shown in table III, application no. 15, column 7, ,and conferring an increase in the amount of the fine chemical in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of heptadecanoic acid and/or hexadecatrienoic acid, preferably delta 7, 10, 13 hexadecatrienoic acid in an organism or a part thereof;
h) nucleic acid molecule encoding a polypeptide comprising the consensus sequence shown in table IV, application no. 15, column 7, , and conferring an increase in the amount of the fine chemical in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
whereby the nucleic acid molecule distinguishes over the sequence as shown in table I, application no. 15, columns 5 or 7, , by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over the sequence as shown in table II, application no. 15, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of heptadecanoic acid and/or hexadecatrienoic acid, preferably delta 7, 10 ,13 hexadecatrienoic acid in an organism or a part thereof comprising:
(a) contacting cells, tissues , plants or microorganisms which express the a polypeptide encoded by the nucleic acid molecule as mentioned above conferring an increase in the amount of heptadecanoic acid and/or 2-hydroxy palmitic acid and/or hexadecatrienoic acid, preferably delta 7, 10, 13 hexadecatrienoic acid in an organism or a part thereof with a candidate compound or a sample comprising a plurality of compounds under conditions which permit the expression the polypeptide;
(b) assaying the palmitic acid level or the polypeptide expression level in the cell, tissue, plant or microorganism or the media the cell, tissue, plant or microorganisms is cultured or maintained in; and
(c) identifying a agonist or antagonist by comparing the measured palmitic acid level or polypeptide expression level with a standard palmitic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased heptadecanoic acid and/or hexadecatrienoic acid, preferably delta 7, 10 ,13 hexadecatrienoic acid and/or 24:1 fatty acid, preferably C24:1 delta 15 fatty acid production in a plant or microorganism, comprising the steps:
(a) culturing a plant cell or tissue or microorganism or maintaining a plant expressing the polypeptide encoded by the nucleic acid molecule as mentioned above conferring an increase in the amount of heptadecanoic acid and/or hexadecatrienoic acid, preferably delta 7, 10, 13 hexadecatrienoic acid in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of heptadecanoic acid and/or and/or hexadecatrienoic acid, preferably delta 7, 10 ,13 hexadecatrienoic acid in an organism or a part thereof;
(b) identifying if the compound is an effective agonist by detecting the presence or absence or increase of a signal produced by said readout system.
A method for the identification of a gene product conferring an increase in heptadecanoic acid and/or hexadecatrienoic acid, preferably delta 7, 10, ,13 hexadecatrienoic acid production in a cell, comprising the following steps:
(a) contacting the nucleic acid molecules of a sample, which can contain a candidate gene encoding a gene product conferring an increase in heptadecanoic acid hexadecatrienoic acid, preferably delta 7, 10, 13 hexadecatrienoic acid after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing heptadecanoic acid and/or hexadecatrienoic acid, preferably delta 7, 10 ,13 hexadecatrienoic acid;
(d) expressing the identified nucleic acid molecules in the host cells;
(e) assaying the heptadecanoic acid and/or and/or hexadecatrienoic acid, preferably delta 7, 10 ,13 hexadecatrienoic acid level in the host cells; and
(f) identifying nucleic acid molecule and its gene product which expression confers an increase in the heptadecanoic acid and/or hexadecatrienoic acid, preferably delta 7, 10 ,13 hexadecatrienoic acid level in the host cell in the host cell after expression compared to the wild type.
A method for the identification of a gene product conferring an increase in heptadecanoic acid and/or and/or hexadecatrienoic acid, preferably delta 7, 10, 13 hexadecatrienoic acid production in a cell, comprising the following steps:
(a) identifiying in a data bank nucleic acid molecules of an organism; which can contain a candidate gene encoding a gene product conferring an increase in the heptadecanoic acid and/or hexadecatrienoic acid, preferably delta 7, 10, 13 hexadecatrienoic acid amount or level in an organism or a part thereof after expression, and which are at least 20% homolog to the nucleic acid molecule as mentioned above;
(b) introducing the candidate nucleic acid molecules in host cells appropriate for producing heptadecanoic acid acid and/or hexadecatrienoic acid, preferably delta 7, 10 ,13 hexadecatrienoic acid;
(c) expressing the identified nucleic acid molecules in the host cells;
(d) assaying the heptadecanoic acid and/or hexadecatrienoic acid, preferably delta 7, 10 ,13 hexadecatrienoic acid level in the host cells; and
(e) identifying nucleic acid molecule and its gene product which expression confers an increase in the heptadecanoic acid and/or hexadecatrienoic acid, preferably delta 7, 10 ,13 hexadecatrienoic acid level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of heptadecanoic acid and/or 2-hydroxy palmitic acid and/or hexadecatrienoic acid, preferably delta 7, 10 ,13 hexadecatrienoic acid after expression.
Use of the polypeptide as mentioned above or the nucleic acid construct as mentioned above or the gene product identified according to the method as mentioned above for identifying compounds capable of conferring a modulation of heptadecanoic acid and/or hexadecatrienoic acid, preferably delta 7, 10,13 hexadecatrienoic acid levels in an organism.
Food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the protection of a plant against a heptadecanoic acid and/or hexadecatrienoic acid, preferably delta 7, 10 ,13 hexadecatrienoic acid synthesis inhibiting herbicide.

### Process for the production of fine chemicals

### Description

[0000.0.0.16] In a further embodiment, the present invention relates to a further process for the production of fine chemicals as defined below and the corresponding embodiments as described herein as follows.

**[0001.0.0.16] see [0001.0.0.0]**

[0002.0.16.16] The malic acid oxaloacetate shuttle is characteristic for plant cells. It transports redox equivalents intracellularly.
Malic acid is not only a central metabolite in intermediary flow of carbon in organisms. In higher plants, vacuolar malic acid accumulation, and hence, transtonoplast malic acid transport, also plays a paramount role in many physiological functions. These include adjustment of osmotic and turgor potentials in extension growth and movements of stomata and pulvini, pH-regulation, e.g. during nitrate reduction, and others (for review, see Lüttge et al, Plant Physiol,124(2000),1335-1348).
Osawa and Matsumoto, Plant Physiol, 126(2001), 411-420 discuss the involvement malic acid in aluminium resistance in plants.
Malic acid is a common constituent of all plants, and its formation is controlled by an enzyme (protein catalyst) called malic acid dehydrogenase (MDH).
Malic acid occupies a central role in plant metabolism. Its importance in plant mineral nutrition is reflected by the role it plays in symbiotic nitrogen fixation, phosphorus acquisition, and aluminum tolerance.
During phosphorus deficiency, malic acid is frequently secreted from roots to release unavailable forms of phosphorus.
In nitrogen-fixing root nodules, malic acid is the primary substrate for bacteroid respiration, thus fueling nitrogenase.
Trihydroxibutanoic acid, trihydroxybutyric acid lacton can be regarded as hypothetic precusors of pheromone like metabolites in plants.
Due to these interesting physiological roles and agrobiotechnological potential of malic acid, trihydroxybutyric acid or trihydroxybutanoic acid there is a need to identify the genes of enzymes and other proteins involved in malic acid, pyruvic acid, succinic acid, trihydroxybutyric acid or trihydroxybutanoic acid metabolism, and to generate mutants or transgenic plant lines with which to modify the malic acid, or trihydroxybutanoic acid content.

**[0003.0.0.16] to [0007.0.0.16] -/-**

**[0008.0.0.16]** One way to increase the productive capacity of biosynthesis is to apply recombinant DNA technology. Thus, it would be desirable to produce malic acid, or trihydroxybutanoic acid in plants. That type of production permits control over quality, quantity and selection of the most suitable and efficient producer organisms. The latter is especially important for commercial production economics and therefore availability to consumers. In addition it is desirable to produce malic acid, or trihydroxybutanoic acid in plants in order to increase plant productivity and resistance against biotic and abiotic stress as discussed before.

Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Agrobacterium tumefaciens*-mediated transformation of tobacco plants using a vector that contained a ketolase-encoding gene from *H. pluvialis* denominated *crtO* along with the *Pds* gene from tomato as the promoter and to encode a leader sequence. Those results indicated that about 75 percent of the carotenoids found in the flower of the transformed plant contained a keto group.

[0009.0.16.16] Thus, it would be advantageous if an algae, plant or other microorganism were available which produce large amounts malic acid, ortrihydroxybutanoic acid. The invention discussed hereinafter relates in some embodiments to such transformed prokaryotic or eukaryotic microorganisms.

It would also be advantageous if plants were available whose roots, leaves, stem, fruits or flowers produced large amounts of glyceric malic acid, or trihydroxybutanoic acid. The invention discussed hereinafter relates in some embodiments to such transformed plants.

[0010.0.16.16] Therefore improving the quality of foodstuffs and animal feeds is an important task of the food-and-feed industry. This is necessary since, for example malic acid, or trihydroxybutanoic acid, as mentioned above, which occur in plants and some microorganisms are limited with regard to the supply of mammals. Especially advantageous for the quality of foodstuffs and animal feeds is as balanced as possible a specific malic acid, or trihydroxybutanoic acid profile in the diet since an excess of malic acid, or trihydroxybutanoic acid above a specific concentration in the food has a positive effect. A further increase in quality is only possible via addition of further malic acid, and/or trihydroxybutanoic acid, which are limiting.

[0011.0.16.16] To ensure a high quality of foods and animal feeds, it is therefore necessary to add malic acid, and/or trihydroxybutanoic acid in a balanced manner to suit the organism.

**[0012.0.16.16]** Accordingly, there is still a great demand for new and more suitable genes which encode enzymes or other proteins which participate in the biosynthesis of, malic acid, and/or trihydroxybutanoic acid and make it possible to produce them specifically on an industrial scale without unwanted by-products forming. In the selection of genes for biosynthesis two characteristics above all are particularly important. On the one hand, there is as ever a need for improved processes for obtaining the highest possible contents of, malic acid, and/or trihydroxybutanoic acid; on the other hand as less as possible by-products should be produced in the production process.

**[0013.0.0.16] see [0013.0.0.0]**

[0014.0.16.16] Accordingly, in a first embodiment, the invention relates to a process for the production of a fine chemical, whereby the fine chemical is a malic acid, and/or trihydroxybutanoic acid. Accordingly, in the present invention, the term "the fine chemical" as used herein relates to a malic acid, and/or trihydroxybutanoic acid. Further, the term "the fine chemicals" as used herein also relates to fine chemicals comprising, malic acid, and/or trihydroxybutanoic acid.

[0015.0.16.16] In one embodiment, the term "the fine chemical" or "the respective fine chemical" means at least one chemical compound with malic acid, trihydroxybutyric acid and/or trihydroxybutanoic acid activity.

In one embodiment, the term "the fine chemical" or "the respective fine chemical" means malic acid. In one embodiment, the term "the fine chemical" or "the respective fine chemical" means trihydroxybutanoic acid depending on the context in which the term is used. Throughout the specification the term "the fine chemical" or "the respective fine chemical" means malic acid and/or trihydroxybutanoic acid, its salts, ester, thioester or in free form or bound to other compounds such sugars or sugar polymers, like glucoside, e.g. diglucoside.
In particular it is known to the skilled that anionic compounds as acids are present in an equilibrium of the acid and its salts according to the pH present in the respective compartment of the cell or organism and the pK of the acid. Thus, the term "the fine chemical", the term "the respective fine chemical", the term "acid" or the use of a demonination referring to a neutralized anionic compound respectivley relates the anionic form as well as the neutralised status of that compound.
Thus, malic acid also relates to malate, or trihydroxybutanoic acid relates also to trihydroxybutanoic.

In one embodiment, the term "the fine chemical" and the term "the respective fine chemical" mean at least one chemical compound with an activity of the above mentioned fine chemical

**[0016.0.16.16]** Accordingly, the present invention relates to a process for the production of the respective fine chemical comprising
(a) increasing or generating the activity of one or more of a protein as shown in table II, application no. 16, column 3 encoded by the nucleic acid sequences as shown in table I, application no. 16, column 5, in a non-human organism or in one or more parts thereof or
(b) growing the organism under conditions which permit the production of the fine chemical, thus organic acid of the invention or fine chemicals comprising the organic acids of the invention, in said organism or in the culture medium surrounding the organism.

**[0017.0.0.16] to [0018.0.0.16]: see [0017.0.0.0] to [0018.0.0.0]**

[**0019.0.16.16]** Advantageously the process for the production of the respective fine chemical leads to an enhanced production of the fine respective chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% or more percent higher production of the respective fine chemical in comparison to the reference as defined below, e.g. that means in comparison to an organism without the aforementioned modification of the activity of a protein having the activity of a protein indicated in Table II, column 3, application 14 or encoded by nucleic acid molecule indicated in Table I, columns 5 or 7, application 14.

[0020.0.16.16] Surprisingly it was found, that the transgenic expression of the Brassica napus protein as indicated in Table II, application no.16, column 5, line 37 in a plant conferred an increase in Malic Acid content of the transformed plants. Thus, in one embodiment, said protein or its homologs are used for the production of Malic Acid.
Surprisingly it was found, that the transgenic expression of the LInum usitatissimum protein as indicated in Table II, application no.16, column 5, line 38 in thaliana plant conferred an increase in Trihydroxybutanoic acid content of the transformed plants. Thus, in one embodiment, said protein or its homologs are used for the production of Trihydroxybutanoic acid.

**[0021.0.0.16] see [0021.0.0.0]**

**[0022.0.16.16]** The sequence of b1343 from Escherichia coli K12 has been published in Blattner, Science 277(5331), 1453-1474, 1997, and its activity is being defined as a ATP-dependent RNA helicase, stimulated by 23S rRNA. Accordingly, in one embodiment, the process of the present invention comprises the use of a "ATP-dependent RNA helicase, stimulated by 23S rRNA" from E. coli or a plant or its homolog, e.g. as shown herein, for the production of the fine chemical, meaning of trihydroxybutanoic acid and/or compositions containing trihydroxybutanoic acid, in particular for increasing the amount of trihydroxybutanoic acid in free or bound form in an organism or a part thereof, as mentioned. In one embodiment, in the process of the present invention the activity of a protein having an activity in rRNA processing or translation is increased or generated, e.g. from E. coli or a plant or a homolog thereof. Accordingly, in one embodiment, in the process of the present invention the activity of a ATP-dependent RNA helicase, stimulated by 23S rRNA or its homolog is increased for the production of the fine chemical, meaning of trihydroxybutanoic acid, in particular for increasing the amount of trihydroxybutanoic acid in free or bound form in an organism or a part thereof, as mentioned.
The sequence of YFR007W from Saccharomyces cerevisiae has been published in Goffeau, A. et al. Science 274 (5287), 546-547 (1996) and its activity is being defined as an unclassified protein. Accordingly, in one embodiment, the process of the present invention comprises the use of said unclassified protein from Saccharomyces cerevisiae or a plant or its homolog, e.g. as shown herein, for the production of the respective fine chemical, in particular for increasing the amount of fumaric acid preferably in free or bound form in an organism or a part thereof, as mentioned. In one embodiment, in the process of the present invention the activity of the unclassified protein YFR007W is increased.
**[0022.1.0.16] to [0023.0.0.16] see [0022.1.0.16] to [0023.0.0.16]**

**[0023.1.0.16]** Homologs of the polypeptide disclosed in table II, application no. 16, column 5, may have the activity of a protein as disclosed in table I or II, application no. 16, column 3 and may be the polypetides encoded by the nucleic acid molecules indicated in table I , application no. 16, column 7, resp., or may be the polypeptides indicated in table II, application no. 16, column 7, resp.
**[0024.0.0.16] see [0024.0.0.0]**

**[0025.0.16.16]** In accordance with the invention, a protein or polypeptide has the "activity of an protein as shown in table II, application no. 16, column 3 "if its de *novo* activity, or its increased expression directly or indirectly leads to an increased in the level of the fine chemical indicated in the respective line of table II, application no. 16, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 16, column 3, or which has at least 10% of the original enzymatic or biological activity, preferably 20%, particularly preferably 30%, most particularly preferably 40% in comparison to a protein as shown in the respective line of table II, application no. 16, column 3 of a E. coli or Saccharomyces cerevisae, respectively, protein as mentioned in table I to IV, column 3 respectively and as disclosed in paragraph [0022] of the respective invention.

**[0025.1.0.16] and [0025.2.0.16] see [0025.1.0.0] and [0025.2.0.0]**

[0025.3.16.16] In one embodiment, the polypeptide of the invention or used in the method of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0026.0.0.16] to [0033.0.0.16]: see [0026.0.0.0] to [0033.0.0.0]**

[0034.0.16.16] Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 16, column 3 its biochemical or genetical causes and the increased amount of the respective fine chemical.

**[0035.0.0.16] to [0044.0.0.16]: see [0035.0.0.0] to [0044.0.0.0]**

[0045.0.16.16] In one embodiment, the activity of the protein. as indicated in Table II, columns 5 or 7, application no. 16, is increased conferring an increase of the respective fine chemical, indicated in Table II, application no. 16, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more

[0046.0.16.16] In one embodiment, the activity of the protein as indicated in Table II, columns 5 or 7, application no. 16, is increased conferring an increase of the respective fine chemical, indicated in Table II, application no. 16, column 6 of the respective line confers an increase of the respective fine chemical and of further organic acid or their precursors.

**[0047.0.0.16] to [0048.0.0.16]: see [0047.0.0.0] to [0048.0.0.0]**

[0049.0.16.16] A protein having an activity conferring an increase in the amount or level of the fine chemical, has in one embodiment the structure of the polypeptide described herein, in particular of the polypeptides comprising the consensus sequence shown in table IV, application no. 16, column 7 or of the polypeptide as shown in the amino acid sequences as disclosed in table II, application no. 16, columns 5 and 7 or the functional homologues thereof as described herein, or is encoded by the nucleic acid molecule characterized herein or the nucleic acid molecule according to the invention, for example by the nucleic acid molecule as shown in table I, application no. 16, columns 5 and 7 or its herein described functional homologues and has the herein mentioned activity.

[0050.0.16.16] For the purposes of the present invention, the term "the respective fine chemical" also encompass the corresponding salts, such as, for example, the . potassium or sodium salts of malic acid, and/or trihydroxybutanoic acid, resp., or their ester, or glucoside thereof, e.g the diglucoside thereof.

[0051.0.16.16] Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g. compositions comprising malic acid, and/or trihydroxybutanoic acid. Depending on the choice of the organism used for the process according to the present invention, for example a microorganism or a plant, compositions or mixtures of organic acids or there salts, e.g. malic acid, and/or trihydroxybutanoic acid can be produced.

**[0052.0.0.16] see [0052.0.0.0]**

**[0053.0.16.16]In** one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 16, columns 5 and 7 or its homologs activity having herein-mentioned organic acid of the invention increasing activity; and/or
b) stabilizing a mRNA conferring the increased expression of a protein encoded by the nucleic acid molecule of the invention as shown in table I, application no. 16, columns 5 and 7, e.g. a nucleic acid sequence encoding a polypeptide having the activity of a protein as indicated in table II, application no. 16, columns 5 and 7 or its homologs activity or of a mRNA encoding the polypeptide of the present invention having herein-mentioned organic acid of the invention increasing activity; and/or
c) increasing the specific activity of a protein conferring the increased expression of a protein encoded by the nucleic acid molecule of the invention or of the polypeptide of the present invention having herein-mentioned organic acid increasing activity, e.g. of a polypeptide having the activity of a protein as indicated in table II, application no. 16, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned organic acid of the invention increasing activity, e.g. of a polypeptide having the activity of a protein as indicated in table II, application no. 16, columns 5 and 7 or its homologs activity; and/or
e) stimulating activity of a protein conferring the increased expression of a protein encoded by the nucleic acid molecule of the present invention or a polypeptide of the present invention having herein-mentioned organic acid of the invention increasing activity, e.g. of a polypeptide having the activity of a protein as indicated in table II, application no. 16, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned organic acid of the invention increasing activity, e.g. of a polypeptide having the activity of a protein as indicated in table II, application no. 16, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned organic acid of the invention increasing activity, e.g. of a polypeptide having the activity of a protein as indicated in table II, application no. 16, columns 5 and 7 or its homologs activity; and/or
h) increasing the expression of the endogenous gene encoding the polypeptide of the invention, e.g. a polypeptide having the activity of a protein as indicated in table II, application no. 16, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with expecially high activity of the proteins of the invention from natural or from mutagenized resources and breeding them into the target organisms, eg the elite crops.

[0054.0.16.16] Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table II, application no. 16, columns 3 or 5, resp., or its homologs activity, e.g. as indicated in Table II, application no. 16, columns 5 or 7, resp.

**[0055.0.0.16] to [0067.0.0.16]: see [0055.0.0.0] to [0067.0.0.0]**

[0068.0.16.16] The mutation is introduced in such a way that the production of malic acid, and/or trihydroxybutanoic acid is not adversely affected.

**[0069.0.0.16] see [0069.0.0.0]**

[0070.0.16.16] Owing to the introduction of a gene or a plurality of genes conferring the expression of the nucleic acid molecule of the invention or used in the process of the invention or the polypeptide of the invention or used in the process of the invention, for example the nucleic acid construct mentioned below, or encoding a protein of the invention or used in the process of the invention into an organism alone or in combination with other genes, it is possible not only to increase the biosynthetic flux towards the end product, but also to increase, modify or create de *novo* an advantageous, preferably novel metabolite composition in the organism, e.g. an advantageous composition of malic acid, and/or trihydroxybutanoic acid or their biochemical derivatives , e.g. comprising a higher content of (from a viewpoint of nutrional physiology limited) malic acid, and/or trihydroxybutanoic acid or their derivatives.

**[0071.0.0.16] see [0071.0.0.0]**

[0072.0.16.16]

[0073.0.16.16] Accordingly, in one embodiment, the process according to the invention relates to a process, which comprises:
a) providing a non-human organism, preferably a microorganism, a non-human animal, a plant or animal cell, a plant or animal tissue or a plant; and
b) increasing an activity of a polypeptide of the invention or used in the process of the invention or a homolog thereof, e.g. as indicated in Table II, application no. 16, columns 5 or 7, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant, and
c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
d) if desired, recovering, optionally isolating, the free and/or bound the respective fine chemical synthesized by the organism, the microorganism, the non-human animal, the plant or animal cell, the plant or animal tissue or the plant.

[0074.0.16.16] The organism, in particular the microorganism, non-human animal, the plant or animal cell, the plant or animal tissue or the plant is advantageously grown in such a way that it is not only possible to recover, if desired isolate the free or bound respective fine chemical.

### [0075.0.0.16] to [0077.0.0.16]: see [0075.0.0.0] to [0077.0.0.0]

[0078.0.16.16] The organism such as microorganisms or plants or the recovered, and if desired isolated, the respective fine chemical can then be processed further directly into foodstuffs or animal feeds or for other applications. The fermentation broth, fermentation products, plants or plant products can be purified with methods known to the person skilled in the art. Products of these different work-up procedures are malic acid and/or trihydroxybutanoic acid or comprising compositions of malic acid, and/or trihydroxybutanoic acid still comprising fermentation broth, plant particles and cell components in different amounts, advantageously in the range of from 0 to 99% by weight, preferably below 80% by weight, especially preferably below 50% by weight.

**[0079.0.0.16] to [0084.0.0.16]: see [0079.0.0.0] to [0084.0.0.0]**

**[0084.0.16.16] -/-**

**[0085.0.16.16]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, application no. 16, columns 5 or 7, or a derivative thereof, or
b) a genetic regulatory element, for example a promoter, which is functionally linked to the nucleic acid sequence as indicated in Table I, application no. 16, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.16] to [0087.0.0.16]: see [0086.0.0.0] to [0087.0.0.0]**

[0088.0.16.16] In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned fine chemicals or the plants are more resistant to biotic and abiotic stress and the yield is increased.

**[0088.1.0.16] see [0088.1.0.0]**

**[0089.0.0.16] to [0094.0.0.16]: see [0089.0.0.0] to [0094.0.0.0]**

[0095.0.16.16] It may be advantageous to increase the pool of malic acid, and/or trihydroxybutanoic acid in the transgenic organisms by the process according to the invention in order to isolate high amounts of the pure respective fine chemical and/or to obtain increased resistance against biotic and abiotic stresses and to obtain higher yield.

[0096.0.16.16] In another preferred embodiment of the invention a combination of the increased expression of the nucleic acid sequence or the protein of the invention or used in the process of the invention together with the transformation of a protein or polypeptid or a compound, which functions as a sink for the desired fine chemical, for example in the organism, is useful to increase the production of the respective fine chemical.

**[0097.0.16.16] -/-**

**[0098.0.16.16]** In a preferred embodiment, the respective fine chemical is produced in accordance with the invention and, if desired, is isolated.

[0099.0.10.16] In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.16.16]** Transgenic plants which comprise the fine chemicals such as malic acid, and/or trihydroxybutanoic acid synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pre-treated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because malic acid, and/or trihydroxybutanoic acid in microorganisms are localized intracellular, their recovery essentially comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.
[0101.0.10.16] The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.16.16]** Malic acid and/or trihydroxybutanoic acid can for example be detected advantageously via HPLC, LC or GC separation methods. The unambiguous detection for the presence of malic acidand/or trihydroxybutanoic acid containing products can be obtained by analyzing recombinant organisms using analytical standard methods: LC, LC-MS, MS or TLC. The material to be analyzed can be disrupted by sonication, grinding in a glass mill, liquid nitrogen and grinding, cooking, or via other applicable methods. [0103.0.16.16] In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, application no. 16, columns 5 or 7,or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 16, columns 5 or 7,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, application no. 16, columns 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence having a sequences as indicated in Table IV, application no. 16, columns 7, and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, application no. 16, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.18.16]** In one embodiment, the nucleic acid molecule used in the process of the invention distinguishes over the sequence indicated in Table I, application no. 16, columns 5 or 7, by one or more nucleotides. In one embodiment, the nucleic acid molecule used in the process of the invention does not consist of the sequence shown in indicated in Table I, application no. 16, columns 5 or 7: In one embodiment, the nucleic acid molecule used in the process of the invention is less than 100%, 99,999%, 99,99%, 99,9% or 99% identical to a sequence indicated in Table columns 5 or 7,. In another embodiment, the nucleic acid molecule does not encode a polypeptide of a sequence indicated in Table II, application no. 16, columns 5 or 7.

**[0104.0.16.16]** In one embodiment, the nucleic acid molecule used in the process of the present invention or used in the the process of the invention distinguishes over the sequence indicated in Table I, application no. 16, columns 5 or 7, by one or more nucleotides. In one embodiment, the nucleic acid molecule of the invention or used in the process of the invention does not consist of the sequence indicated in Table I, application no. 16, columns 5 or 7. In one embodiment, the nucleic acid molecule of the present invention is less than 100%, 99,999%, 99,99%, 99,9% or 99% identical to the sequence indicated in Table I, application no. 16, columns 5 or 7. In another embodiment, the nucleic acid molecule does not encode a polypeptide of a sequence indicated in Table II, application no. 16, columns 5 or 7.

**[0105.0.0.16] to [0107.0.0.16]: see [0105.0.0.0] to [0107.0.0.0]**

**[0108.0.16.16]** Nucleic acid molecules with the sequence as indicated in Table I, application no. 16, columns 5 or 7, nucleic acid molecules which are derived from an amino acid sequences as indicated in Table II, application no. 16, columns 5 or 7or from polypeptides comprising the consensus sequence as indicated in Table IV, application no. 16, column 7, or their derivatives or homologues encoding polypeptides with the enzymatic or biological activity of an activity of a polypeptide as indicated in Table II, application no. 16, column 3, 5 or 7, e.g. conferring the increase of the respective fine chemical, meaning malic acid, and/or trihydroxybutanoic acid, resp., after increasing its expression or activity, are advantageously increased in the process according to the invention.

[0109.0.16.16] In one embodiment, said sequences are cloned into nucleic acid constructs, either individually or in combination. These nucleic acid constructs enable an optimal synthesis of the respective fine chemicals, in particular malic acid and/or trihydroxybutanoic acid, produced in the process according to the invention.

**[0110.0.0.16] see [0110.0.0.0]**

**[0111.0.0.16] see [0111.0.0.0]**

**[0112.0.16.16]** The nucleic acid molecules used in the process according to the invention take the form of isolated nucleic acid sequences, which encode polypeptides with an activity of a polypeptide as indicated in Table II, application no. 16, column 3, or having the sequence of a polypeptide as indicated in Table II, application no. 16, columns 5 and 7and conferring an increase in the malic acid and/or trihydroxybutanoic acid level.

**[0113.0.0.16] to [0114.0.0.16]: see [0113.0.0.0] to [0114.0.0.0]**

**[0115.0.0.16] see [0115.0.0.0]**

**[0116.0.0.16] to [0120.0.0.16] see [0116.0.0.0] to [0120.0.0.0]**

**[0120.1.0.16]: -/-**

**[0121.0.16.16]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 16, columns 5 or 7, or the functional homologues thereof as described herein, preferably conferring above-mentioned activity, i.e. conferring a malic acid level or conferring a trihydroxybutanoic acid increase after increasing the activity of the polypeptide sequences indicated in Table II, application no. 16, columns 5 or 7,

**[0122.0.0.16] to [0127.0.0.16]: see [0122.0.0.0] to [0127.0.0.0]**

[0128.0.16.16] Synthetic oligonucleotide primers for the amplification, e.g. as the pairs indicated in Table III, application no. 16, columns 7, by means of polymerase chain reaction can be generated on the basis of a sequence shown herein, for example the sequence as indicated in Table I, application no. 16, columns 5 or 7, or the sequences derived from a sequence as indicated in Table II, application no. 16, columns 5 or 7.

[0129.0.16.16] Moreover, it is possible to identify conserved regions from various organisms by carrying out protein sequence alignments with the polypeptide used in the process of the invention, in particular with sequences of the polypeptide of the invention or used in the process of the invention, from which conserved regions, and in turn, degenerate primers can be derived. Conserved region for the polypeptide of the invention or used in the process of the invention are indicated in the alignments shown in the figures. Conserved regions are those, which show a very little variation in the amino acid in one particular position of several homologs from different origin. The consensus sequence indicated in Table IV, application no. 16, columns 7is derived from said alignments.

[0130.0.16.16] Degenerated primers can then be utilized by PCR for the amplification of fragments of novel proteins having above-mentioned activity, e.g. conferring the increase of malic acid, and/or trihydroxybutanoic acid after increasing the expression or activity the protein comprising said fragment.

**[0131.0.0.16] to [0138.0.0.16]: see [0131.0.0.0] to [0138.0.0.0]**

**[0139.0.16.16]** Polypeptides having above-mentioned activity, i.e. conferring the respective fine chemical level increase, derived from other organisms, can be encoded by other DNA sequences which hybridise to a sequence indicated in Table I, application no. 16, columns 5 or 7, for trihydroxybutanoic acid under relaxed hybridization conditions and which code on expression for peptides having the respective fine chemical, i.e. malic acid, and/or trihydroxybutanoic acid, resp., increasing-activity.

**[0140.0.0.16] to [0146.0.0.16]: see [0140.0.0.0] to [0146.0.0.0]**

**[0147.0.16.16]** Further, the nucleic acid molecule of the invention or used in the process of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 16, columns 5 or 7, is one which is sufficiently complementary to one of said nucleotide sequences such that it can hybridise to one of said nucleotide sequences, thereby forming a stable duplex. Preferably, the hybridisation is performed under stringent hybridization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.16.16]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 16, columns 5 or 7, or a portion thereof and preferably has above mentioned activity, in particular having a malic acid, and/or trihydroxybutanoic acid increasing activity after increasing the activity or an activity of a product of a gene encoding said sequences or their homologs.

**[0149.0.16.16]** The nucleic acid molecule of the invention comprises a nucleotide sequence which hybridizes, preferably hybridizes under stringent conditions as defined herein, to one of the nucleotide sequences indicated in Table I, application no. 16, columns 5 or 7or a portion thereof and encodes a protein having above-mentioned activity, e.g. conferring a of glyceric acid, citramalic acid, fumaric acid, malic acid, pyruvic acid, succinic acid, trihydroxybutyric acid and/or trihydroxybutanoic acid increase, resp., and optionally, the activity of protein indicated in Table II, column 5, lines 190 to 226 or lines 564 to 594, preferably in Table II, application no. 16, columns 7.

**[00149.1.0.16]** Optionally, in one embodiment, the nucleotide sequence, which hybridises to one of the nucleotide sequences indicated in Table I, application no. 16, columns 5 or 7, has further one or more of the activities annotated or known for a protein as indicated in Table II, application no. 16, column 3.

**[0150.0.16.16]** Moreover, the nucleic acid molecule of the invention or used in the process of the invention can comprise only a portion of the coding region of one of the sequences indicated in Table I, application no. 16, columns 5 or 7, for example a fragment which can be used as a probe or primer or a fragment encoding a biologically active portion of the polypeptide of the present invention or of a polypeptide used in the process of the present invention, i.e. having above-mentioned activity, e.g. conferring an increase of malic acid, and/or trihydroxybutanoic acid , resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 16, columns 5 or 7, an anti-sense sequence of one of the sequences, e.g., as indicated in Table I, application no. 16, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 16, column 7will result in a fragment of a polynucleotide sequence as indicated in Table I, application no. 16, columns 5 or 7or its gene product. **[0151.0.0.16]: see [0151.0.0.0]**

**[0152.0.16.16]** The nucleic acid molecule of the invention or used in the process of the invention encodes a polypeptide or portion thereof which includes an amino acid sequence which is sufficiently homologous to an amino acid sequence as indicated in Table II, application no. 16, columns 5 or 7, such that the protein or portion thereof maintains the ability to participate in the respective fine chemical production, in particular a malic acid or trihydroxybutanoic acid increasing activity as mentioned above or as described in the examples in plants or microorganisms is comprised.

[0153.0.16.16] As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 16, columns 5 or 7such that the protein or portion thereof is able to participate in the increase of the respective fine chemical production. In one embodiment, a protein or portion thereof as indicated in Table II, application no. 16, columns 5 or 7, has for example an activity of a polypeptide indicated in Table II, application no. 16, column 3.

[0154.0.16.16] In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91 %, 92%, 93% or 94% and most preferably at least about 95%, 97%, 98%, 99% or more homologous to an entire amino acid sequence as indicated in Table II, application no. 16, columns 5 or 7, and has above-mentioned activity, e.g. conferring preferably the increase of the respective fine chemical.

**[0155.0.0.16] to [0156.0.0.16]: see [0155.0.0.0] to [0156.0.0.0]**

[0157.0.16.16] The invention further relates to nucleic acid molecules that differ from one of the nucleotide sequences as indicated in Table I, application no. 16, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the consensus sequence as indicated in Table IV, application no. 16, columns 7, or as polypeptides depicted in Table II, application no. 16, columns 5 or 7, or the functional homologues. Advantageously, the nucleic acid molecule of the invention or used in the process of the invention comprises, or in an other embodiment has, a nucleotide sequence encoding a protein comprising, or in an other embodiment having, an amino acid sequence of a consensus sequences as indicated in Table IV, application no. 16, columns 7, or of the polypeptide as indicated in Table II, application no. 16, columns 5 or 7, resp., or the functional homologues. In a still further embodiment, the nucleic acid molecule of the invention encodes a full length protein which is substantially homologous to an amino acid sequence comprising a consensus sequence as indicated in Table IV, application no. 16, columns 5 or 7, or of a polypeptide as indicated in Table II, application no. 16, columns 5 or 7, or the functional homologues thereof. However, in a preferred embodiment, the nucleic acid molecule of the present invention does not consist of a sequence as indicated in Table I, application no. 16, columns 5 or 7

**[0158.0.0.16]** to **[0160.0.0.16]: see [0158.0.0.0]** to **[0160.0.0.0]**

[0161.0.16.16] Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 16, columns 5 or 7. The nucleic acid molecule is preferably at least 20, 30, 50, 100, 250 or more nucleotides in length.

**[0162.0.0.16] see [0162.0.0.0]**

**[0163.0.16.16]** Preferably, a nucleic acid molecule of the invention or used in the process of the invention that hybridizes under stringent conditions to a sequence as indicated in Table I, application no. 16, columns 5 or 7corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in a organism or a part thereof, e.g. a tissue, a cell, or a compartment of a cell, after increasing the expression or activity thereof or the activity of a protein of the invention or used in the process of the invention.

**[0164.0.0.16] see [0164.0.0.0]**

**[0165.0.16.16]** For example, nucleotide substitutions leading to amino acid substitutions at "non-essential" amino acid residues can be made in a sequence of the nucleic acid molecule of the invention or used in the process of the invention, e.g. as indicated in Table I, application no. 16, columns 5 or 7 resp.

**[0166.0.0.16] to [0167.0.0.16]: see [0166.0.0.0] to [0167.0.0.0]**

[0168.0.16.16] Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table II, application no. 16, columns 5 or 7, preferably in Table II, columns 7, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 16, columns 5 or 7, resp., and is capable of participation in the increase of production of the respective fine chemical after increasing its activity, e.g. its expression. Preferably, the protein encoded by the nucleic acid molecule is at least about 60% identical to a sequence as indicated in Table II, application no. 16, columns 5 or 7, resp., more preferably at least about 70% identical to one of the sequences as indicated in Table II, application no. 16, columns 5 or 7, resp., even more preferably at least about 80%, 90%, 95% homologous to a sequence as indicated in Table II, application no. 16, columns 5 or 7, resp., and most preferably at least about 96%, 97%, 98%, or 99% identical to the sequence as indicated in Table II, application no. 16, columns 5 or 7.

**[0169.0.0.16] to [0172.0.0.16]: see [0169.0.0.0] to [0172.0.0.0]**

**[0173.0.16.16]** For example a sequence, which has 80% homology with sequence SEQ ID NO: 17198 at the nucleic acid level is understood as meaning a sequence which, upon comparison with the sequence SEQ ID NO: 17198 by the above Gap program algorithm with the above parameter set, has a 80% homology.

**[0174.0.0.16] to [0175.0.16.16]see [0174.0.0.0] to [0175.0.0.0]**

**[0176.0.16.16]** Functional equivalents derived from one of the polypeptides as indicated in Table II, application no. 16, columns 5 or 7, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 16, columns 5 or 7, resp., according to the invention and are distinguished by essentially the same properties as a polypeptide as indicated in Table II, application no. 16, columns 5 or 7 resp.

[0177.0.16.16] Functional equivalents derived from a nucleic acid sequence as indicated in Table I, application no. 16, columns 5 or 7, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 16, columns 5 or 7, resp., according to the invention and encode polypeptides having essentially the same properties as a polypeptide as indicated in Table II, application no. 16, columns 5 or 7, , resp.

**[0178.0.0.16] see [0178.0.0.0]**

**[0179.0.16.16]** A nucleic acid molecule encoding a homologous to a protein sequence as indicated in Table II, application no. 16, columns 5 or 7, resp., can be created by introducing one or more nucleotide substitutions, additions or deletions into a nucleotide sequence of the nucleic acid molecule of the present invention, in particular as indicated in Table I, application no. 16, columns 5 or 7, resp., such that one or more amino acid substitutions, additions or deletions are introduced into the encoded protein. Mutations can be introduced into the encoding sequences for example into sequences of nucleic acid molecules as indicated in Table I, application no. 16, columns 5 or 7, resp., by standard techniques, such as site-directed mutagenesis and PCR-mediated mutagenesis.

**[0180.0.0.16] to [0183.0.0.16]: see [0180.0.0.0] to [0183.0.0.0]**

**[0184.0.16.16]** Homologues of the nucleic acid sequences used, with a sequence as indicated in Table I, application no. 16, columns 5 or 7, resp., or of the nucleic acid sequences derived from a sequences as indicated in Table II, application no. 16, columns 5 or 7, resp." comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 16, columns 5 or 7, resp., or from the derived nucleic acid sequences, the intention being, however, that the enzyme activity or the biological activity of the resulting proteins synthesized is advantageously retained or increased.

[0185.0.16.16] In one embodiment of the present invention, the nucleic acid molecule of the invention or used in the process of the invention comprises one or more sequences as indicated in Table I, application no. 16, columns 5 or 7, resp. In one embodiment, it is preferred that the nucleic acid molecule comprises as little as possible other nucleotides not shown in any one of sequences as indicated in Table I, application no. 16, columns 5 or 7, resp. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequence as indicated in Table I, application no. 16, columns 5 or 7, resp.

[0186.0.16.16] Also preferred is that one or more nucleic acid molecule(s) used in the process of the invention encodes a polypeptide comprising a sequence as indicated in Table II, application no. 16, columns 5 or 7, resp. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 16, columns 5 or 7resp.

[0187.0.16.16] In one embodiment, a nucleic acid molecule of the invention or used in the process encodes a polypeptide comprising the sequence as indicated in Table II, application no. 16, columns 5 or 7, resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 16, columns 5 or 7 resp.

[0188.0.16.16] Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 16, columns 5 or 7resp., preferably compared to a sequence as indicated in preferably in Table II, application no. 16, columns 5 or 7, , and is expressed under identical conditions. **[0189.0.16.16]** Homologues of a sequence as indicated in Table I, application no. 16, columns 5 or 7resp., or of a derived sequences as indicated in Table II, application no. 16, columns 5 or 7, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (=ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.16]: see [0190.0.0.0]**

**[0191.0.0.16] see [0191.0.0.0]**

[0191.0.16.16] The organisms or part thereof produce according to the herein mentioned process of the invention an increased level of free and/or bound the respective fine chemical compared to said control or selected organisms or parts thereof.

**[0192.0.0.16] to [0203.0.0.16]: see [0192.0.0.0] to [0203.0.0.0]**

[0204.0.16.16] Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, application no. 16, columns 5 or 7, resp.; or a fragment thereof conferring an increase in the amount of the respective fine chemical, i.e. malic acid or trihydroxybutanoic acid, resp., in an organism or a part thereof
b) nucleic acid molecule comprising, preferably at least the mature form, of a nucleic acid molecule as indicated in Table I, application no. 16, columns 5 or 7, , resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in Table lll, application no. 16, columns 5 or 7, and conferring an increase in the amount of the respective fine chemical, i.e. malic acid or trihydroxybutanoic acid, resp., in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, application no. 16, columns 7, and conferring an increase in the amount of the respective fine chemical, or trihydroxybutanoic acidresp., in an organism or a part thereof;
k) nucleic acid molecule encoding the amino acid sequence of a polypeptide encoding a domain of a polypeptide as indicated in Table II, application no. 16, columns 5 or 7, resp., and conferring an increase in the amount of the respective fine chemical, i.e. malic acid or trihydroxybutanoic acid resp., in an organism or a part thereof; and
I) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 16, columns 5 or 7, resp., or a nucleic acid molecule encoding, preferably at least the mature form of, a polypeptide as indicated in Table II, application no. 16, columns 5 or 7, resp., and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table I, application no. 16, columns 5 or 7, resp., by one or more nucleotides. In one embodiment, the nucleic acid molecule of the invention does not consist of the sequence as indicated in Table I, application no. 16, columns 5 or 7, resp. In an other embodiment, the nucleic acid molecule of the present invention is at least 30 % identical and less than 100%, 99,999%, 99,99%, 99,9% or 99% identical to a sequence as indicated in Table I, application no. 16, columns 5 or 7, resp. In a further embodiment the nucleic acid molecule does not encode a polypeptide sequence as indicated in Table II, application no. 16, columns 5 or 7, resp. Accordingly, in one embodiment, the nucleic acid molecule of the present invention encodes in one embodiment a polypeptide which differs at least in one or more amino acids from a polypeptide indicated in Table II, application no. 16, columns 5 or 7, does not encode a protein of a sequence as indicated in Table II, application no. 16, columns 5 or 7. Accordingly, in one embodiment, the protein encoded by a sequences of a nucleic acid according to (a) to (l) does not consist of a sequence as indicated in Table II, application no. 16, columns 5 or 7. In a further embodiment, the protein of the present invention is at least 30 % identical to a protein sequence indicated in Table II, application no. 16, columns 5 or 7, and less than 100%, preferably less than 99,999%, 99,99% or 99,9%, more preferably less than 99%, 985, 97%, 96% or 95% identical to a sequence as indicated in Table II, application no. 16, columns 5 or 7.

**[0205.0.0.16] to [0206.0.0.16]: see [0205.0.0.0] to [0206.0.0.0]**

[0207.0.16.16] As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.16] to [0226.0.0.16]: see [0208.0.0.0] to [0226.0.0.0]**

[0227.0.16.16] The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.

In addition to a sequence indicated in Table I, application no. 16, columns 5 or 7or its derivatives, it is advantageous to express and/or mutate further genes in the organisms. Especially advantageously, additionally at least one further gene of the glutamic acid or phosphoenolpyruvate metabolic pathway, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table I, application no. 16, columns 5 or 7, resp., with genes which generally support or enhances to growth or yield of the target organism, for example genes which lead to faster growth rate of microorganisms or genes which produces stress-, pathogen, or herbicide resistant plants.

[0228.0.16.16] In a further embodiment of the process of the invention, therefore, organisms are grown, in which there is simultaneous overexpression of at least one nucleic acid or one of the genes which code for proteins directly or indirectly involved in the glutamic acid or phosphoenolpyruvate metabolism.

**[0229.0.0.0] %**

**[0230.0.0.16] see [230.0.0.0]**

[0231.0.16.16] In a further advantageous embodiment of the process of the invention, the organisms used in the process are those in which simultaneously a malic acid, and/or trihydroxybutanoic acid degrading protein is attenuated, in particular by reducing the rate of expression of the corresponding gene.

**[0232.0.0.16] to [0276.0.0.16]: see [0232.0.0.0] to [0276.0.0.0]**

[0277.0.16.16] The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.16] to [0282.0.0.16]: see [0278.0.0.0] to [0282.0.0.0]**

**[0283.0.16.16]** Moreover, native polypeptide conferring the increase of the respective fine chemical in an organism or part thereof can be isolated from cells, for example using the antibody of the present invention as described below, e.g. an antibody against a protein as indicated in Table II, application no. 16, column 3, resp., or an antibody against a polypeptide as indicated in Table II, application no. 16, columns 5 or 7, resp., or an antigenic part thereof which can be produced by standard techniques utilizing polypeptides comprising or consisting of above mentioned sequences, e.g. the polypeptid of the present invention or fragment thereof. Preferred are monoclonal antibodies specifically binding to polypeptide as indicated in Table II, application no. 16, columns 5 or 7.

**[0284.0.0.16] see [0284.0.0.0]**

**[0285.0.16.16]** In one embodiment, the present invention relates to a polypeptide having a sequence as indicated in Table II, application no. 16, columns 5 or 7, resp., or as coded by a nucleic acid molecule as indicated in Table I, application no. 16, columns 5 or 7, resp., or functional homologues thereof.

[0286.0.16.16] In one advantageous embodiment, in the method of the present invention the activity of a polypeptide is increased which comprises or consists of a consensus sequence as indicated in Table IV, application no. 16, columns 7, and in one another embodiment, the present invention relates to a polypeptide comprising or consisting of a consensus sequence as indicated in Table IV, application no. 16, columns 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a poylpeptide or to a polypeptide comprising more than one consensus sequences as indicated in Table IV, application no. 16, column 7.

**[0287.0.0.16]** to **[0289.0.0.16]: see [0287.0.0.0]** to **[0289.0.0.0]**

**[00290.0.16.16]** The alignment was performed with the Software AlignX (sept 25, 2002) a component of Vector NTI Suite 8.0, InforMax™, Invitrogen™ life science software, U.S. Main Office, 7305 Executive Way,Frederick, MD 21704,USA with the following settings: For pair wise alignments: gap opening penalty: 10,0; gap extension penalty 0,1. For multiple alignments: Gap opening penalty: 10,0; Gap extension penalty: 0,1; Gap seperation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids

[0291.0.16.16] In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of plant or microorganism specific consensus sequences.

In one embodiment, said polypeptide of the invention distinguishes over a sequence as indicated in Table II, application no. 16, columns 5 or 7, esp., by one or more amino acids. In one embodiment, polypeptide distinguishes from a sequence as indicated in Table II, application no. 16, columns 5 or 7, resp., by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 16, columns 5 or 7, resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II, application no. 16, columns 5 or 7.

**[0292.0.0.16] see [0292.0.0.0]**

[0293.0.16.16] In one embodiment, the invention relates to a polypeptide conferring an increase in the fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table II, application no. 16, columns 5 or 7, resp., by one or more amino acids. In an other embodiment, said polypeptide of the invention does not consist of the sequence as indicated in Table II, columns 5 or 7 resp. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 16, columns 5 or 7 resp.

**[0294.0.16.16]** In one embodiment, the present invention relates to a polypeptide having an activity of a protein as indicated in Table II, application no. 16, column 3, resp., which distinguishes over a sequence as indicated in Table II, application no. 16, columns 5 or 7, resp., by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.16] to [0296.0.0.16]: see [0295.0.0.0] to [0296.0.0.0]**

**[0297.0.0.16] see [0297.0.0.0]**

**[00297.1.0.16]** Non-polypeptide of the invention-chemicals are e.g. polypeptides having not the activity and/or amino acid sequence of a polypeptide indicated in Table II, application no. 16, columns 3, 5 or 7

**[0298.0.16.16]** A polypeptide of the invention can participate in the process of the present invention. The polypeptide or a portion thereof comprises preferably an amino acid sequence which is sufficiently homologous to an amino acid sequence as indicated in Table II, application no. 16, columns 5 or 7, resp. The portion of the protein is preferably a biologically active portion as described herein. Preferably, the polypeptide used in the process of the invention has an amino acid sequence identical to a sequence as indicated in Table II, application no. 16, columns 5 or 7resp.

[0299.0.16.16] Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91 %, 92%, 93%, 94% or 95%, and even more preferably at least about 96%, 97%, 98%, 99% or more homologous to one of the nucleotide sequence as indicated in Table I, application no. 16, columns 5 or 7, resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 16, columns 5 or 7resp., or which is homologous thereto, as defined above.

**[0300.0.16.16]** Accordingly the polypeptide of the present invention can vary from a sequence as indicated in Table II, application no. 16, columns 5 or 7, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91 %, 92%, 93%, 94% or 95%, and most preferably at least about 96%, 97%, 98%, 99% or more homologous to an entire amino acid sequence of as indicated in Table II, application no. 16, columns 5 or 7 **[0301.0.0.16] see [0301.0.0.0]**

**[0302.0.16.16]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 16, columns 5 or 7, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.16] see [0303.0.0.0]**

[0304.0.16.16] Manipulation of the nucleic acid molecule of the invention or the nucleic acid molecule used in the method of the invention may result in the production of a protein having essentially the activity of the polypeptides as indicated in Table II, application no. 16, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.16] to [0308.0.0.16]: see [0305.0.0.0] to [0308.0.0.0]**

[00306.1.0.0] In one embodiment, the compound is a composition comprising the repective fine chemical, i.e. said organic acids, or a recovered fine chemical, i.e. said organic acid free or in protein-bound form.

**[0309.0.16.16]** In one embodiment, a reference to a protein (= polypeptide) of the invention or as indicated in Table II, application no. 16, columns 5 or 7, resp., refers to a polypeptide having an amino acid sequence corresponding to the polypeptide of the invention or used in the process of the invention, whereas an "other polypeptide" not being indicated in Table II, application no. 16, columns 5 or 7, resp., refers to a polypeptide having an amino acid sequence corresponding to a protein which is not substantially homologous to a polypeptide of the invention, preferably which is not substantially homologous to a polypeptide as indicated in Table II, application no. 16, columns 5 or 7, resp., e.g., a protein which does not confer the activity described herein or annotated or known for as indicated in Table II, application no. 16, column 3resp., and which is derived from the same or a different organism. In one embodiment, an "other polypeptide" not being indicated in Table II, application no. 16, columns 5 or 7, resp., does not confer an increase of the respective fine chemical in an organism or part thereof. In one embodiment, a "non-polypeptide of the invention" or "other polypeptide" not being indicated in Table II, application no. 16, columns 5 or 7resp., does not confer an increase of the respective fine chemical in an organism or part thereof.

**[0310.0.0.16] to [0334.0.0.16] : see [0310.0.0.0] to [0334.0.0.0]**

[0335.0.16.16] The dsRNAi method has proved to be particularly effective and advantageous for reducing the expression of a nucleic acid sequences as indicated in Table I, application no. 16, columns 5 or 7, , resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, application no. 16, columns 5 or 7, resp., and/or homologs thereof. In a double-stranded RNA molecule for reducing the expression of a protein encoded by a nucleic acid sequence as indicated in Table I, application no. 16, columns 5 or 7, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.16] to [0342.0.0.16]: see [0336.0.0.0] to [0342.0.0.0]**

[0343.0.16.16] As has already been described, 100 % sequence identity between the dsRNA and a gene transcript of a nucleic acid sequence as indicated in Table I, application no. 16, columns 5 or 7, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 16, columns 5 or 7, resp., or homologs thereof of the one organism, may be used to suppress the corresponding expression in another organism.

**[0344.0.0.16] to [0361.0.0.16]: see [0344.0.0.0] to [0361.0.0.0]**

**[0362.0.16.16]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 16, columns 5 or 7, resp., e.g. encoding a polypeptide having protein activity, as indicated in Table II, application no. 16, columns 3, resp., Due to the abovementioned activity the respective fine chemical content in a cell or an organism is increased. For example, due to modulation or manipulation, the cellular activity of the polypeptide of the invention or nucleic acid molecule of the invention or the nucleic acid molecule or polypeptide used in the method of the invention is increased, e.g. due to an increased expression or specific activity of the subject matters of the invention in a cell or an organism or a part thereof. Transgenic for a polypeptide having an activity of a polypeptide as indicated in Table II, application no. 16, columns 5 or 7, resp., means herein that due to modulation or manipulation of the genome, an activity as annotated for a polypeptide as indicated in Table II, application no. 16, column 3, e.g. having a sequence as indicated in Table II, application no. 16, columns 5 or 7, resp., is increased in a cell or an organism or a part thereof. Examples are described above in context with the process of the invention.

**[0363.0.0.16] see [0363.0.0.0]**

[0364.0.16.16] A naturally occurring expression cassette - for example the naturally occurring combination of a promoter of a gene encoding a polypeptide of the invention as indicated in Table II, application no. 16, column 3, resp. with the corresponding protein-encoding sequence as indicated in Table I, application no. 16, column 5, resp., becomes a transgenic expression cassette when it is modified by non-natural, synthetic "artificial" methods such as, for example, mutagenization. Such methods have been described (US 5,565,350; WO 00/15815; also see above).

**[0365.0.0.16] to [0373.0.0.16]: see [0365.0.0.0] to [0373.0.0.0]**

[0374.0.16.16] Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. malic acid, and/or trihydroxybutanoic acid, in particular the respective fine chemical, produced in the process according to the invention may, however, also be isolated from the plant in the form of their free malic acid, and/or trihydroxybutanoic acid, in particular the free respective fine chemical, or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.16] to [0376.0.0.16]: see [0375.0.0.0] to [0376.0.0.0]**

[0377.0.16.16] Accordingly, the present invention relates also to a process whereby the produced malic acid, and/or trihydroxybutanoic acid is isolated.

[0378.0.16.16] In this manner, more than 50% by weight, advantageously more than 60% by weight, preferably more than 70% by weight, especially preferably more than 80% by weight, very especially preferably more than 90% by weight, of the malic acid, and/or trihydroxybutanoic acid produced in the process can be isolated. The resulting malic acid, and/or trihydroxybutanoic acid can, if appropriate, subsequently be further purified, if desired mixed with other active ingredients such as vitamins, amino acids, carbohydrates, antibiotics and the like, and, if appropriate, formulated.

[0379.0.16.16] In one embodiment, gamma-aminobutyric acid and shikimate are a mixture of the respective fine chemicals.

**[0380.0.16.16]** The malic acid, and/or trihydroxybutanoic acid obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the malic acid, and/or trihydroxybutanoic acid composition produced or the respective fine chemical produced if desired and formulating the product with a pharmaceutical acceptable carrier or formulating the product in a form acceptable for an application in agriculture. A further embodiment according to the invention is the use of the malic acid and/or trihydroxybutanoic acid produced in the process or of the transgenic organisms in animal feeds, foodstuffs, medicines, food supplements, cosmetics or pharmaceuticals or for the production of malic acid, and/or trihydroxybutanoic acid e.g. after isolation of the respective fine chemical or without, e.g. in situ, e.g. in the organism used for the process for the production of the respective fine chemical.

**[0381.0.0.16] to [0382.0.0.16]: see [0381.0.0.0] to [0382.0.0.0]**

**[0383.0.16.16]** -/-

**[0384.0.0.16] see [0384.0.0.0]**

[0385.0.16.16] The fermentation broths obtained in this way, containing in particular malic acid, and/or trihydroxybutanoic acid in mixtures with other organic acids, amino acids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.

The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.16.16]** Accordingly, it is possible to purify the glyceric acid, citramalic acid, fumaric acid, malic acid, pyruvic acid, succinic acid, trihydroxybutyric acid and/or trihydroxybutanoic acid produced according to the invention further. For this purpose, the product-containing composition is subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.16] to [0392.0.0.16]: see [0387.0.0.0] to [0392.0.0.0]**

[0393.0.16.16] In one embodiment, the present invention relates to a method for the identification of a gene product conferring an increase in the respective fine chemical production in a cell, comprising the following steps:
a) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
b) identifying the nucleic acid molecules, which hybridize under relaxed stringent conditions with the nucleic acid molecule of the present invention in particular to the nucleic acid molecule sequence as indicated in Table I, application no. 16, columns 5 or 7, resp., and, optionally, isolating the full length cDNA clone or complete genomic clone;
c) introducing the candidate nucleic acid molecules in host cells, preferably in a plant cell or a microorganism, appropriate for producing the respective fine chemical;
d) expressing the identified nucleic acid molecules in the host cells;
e) assaying the respective fine chemical level in the host cells; and
f) identifying the nucleic acid molecule and its gene product which expression confers an increase in the respective fine chemical level in the host cell after expression compared to the wild type.

**[0394.0.0.16] to [0552.0.0.16]: see [0394.0.0.0] to [0552.0.0.0]**

**[0553.0.16.16]** A process for the production of malic acid, or trihydroxybutanoic acid, which comprises
a)increasing or generating the activity of a protein as indicated in Table II, application no. 16, columns 5 or 7, or a functional equivalent thereof in a non-human organism or in one or more parts thereof; and
b)growing the organism under conditions which permit the production of malic acid or trihydroxybutanoic acid in said organism.
A process for the production of malic acid, or trihydroxybutanoic acid, comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 16, columns 5 or 7, or a fragment thereof, which confers an increase in the amount of malic acid or trihydroxybutanoic acid in an organism or a part thereof;
b) nucleic acid molecule comprising of a nucleic acid molecule as indicated in Table I, application no. 16, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of malic acid, or trihydroxybutanoic acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of malic acid or trihydroxybutanoic acid in an organism or a part thereof;
e) nucleic acid molecule which hybridizes with a nucleic acid molecule of (a) to (c) under stringent hybridisation conditions and conferring an increase in the amount of malic acid or trihydroxybutanoic acid in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 16, columns 5 or 7and conferring an increase in the amount of malic acid, or trihydroxybutanoic acid in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of malic acid, or trihydroxybutanoic acid in an organism or a part thereof;
h) nucleic acid molecule encoding a polypeptide comprising a consensus as indicated in Table IV, application no. 16, columns 5 or 7, and conferring an increase in the amount of malic acid, or trihydroxybutanoic acid in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of malic acid, or trihydroxybutanoic acid in an organism or a part thereof.
or comprising a sequence which is complementary thereto.
The process as mentioned above, comprising recovering of the free or bound malic acid, or trihydroxybutanoic acid.
The process as mentioned above, comprising the following steps:
a) selecting an organism or a part thereof expressing a polypeptide encoded by the nucleic acid molecule characterized as mentioned above;
b) mutagenizing the selected organism or the part thereof;
c) comparing the activity or the expression level of said polypeptide in the mutagenized organism or the part thereof with the activity or the expression of said polypeptide of the selected organisms or the part thereof;
d) selecting the mutated organisms or parts thereof, which comprise an increased activity or expression level of said polypeptide compared to the selected organism or the part thereof;
e) optionally, growing and cultivating the organisms or the parts thereof; and
f) recovering, and optionally isolating, the free or bound malic acid, trihydroxybutanoic acid produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 16, columns 5 or 7or a fragment thereof, which confers an increase in the amount of glyceric acid. citramalic acid, fumaric acid, malic acid, pyruvic acid, succinic acid, trihydroxybutyric acid or trihydroxybutanoic acid in an organism or a part thereof;
b) nucleic acid molecule comprising of a nucleic acid molecule as indicated in Table I, application no. 16, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of malic acid, or trihydroxybutanoic acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of malic acid, or trihydroxybutanoic acid in an organism or a part thereof;
e) nucleic acid molecule which hybridizes with a nucleic acid molecule of (a) to (c) under stringent hybridisation conditions and conferring an increase in the amount of malic acid, or trihydroxybutanoic acid in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 16, column 7, and conferring an increase in the amount of glyceric acid. citramalic acid, fumaric acid, malic acid, pyruvic acid, succinic acid, trihydroxybutyric acid or trihydroxybutanoic acid in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of malic acid, or trihydroxybutanoic acid in an organism or a part thereof;
h) nucleic acid molecule encoding a polypeptide comprising a consensus as indicated in Table IV, application no. 16, column 7, and conferring an increase in the amount of in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of malic acid, or trihydroxybutanoic acid in an organism or a part thereof.
whereby the nucleic acid molecule distinguishes over the sequence as indicated in Table I, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above. The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 16, columns 5 or 7, by one or more amino acids.
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of malic acid, or trihydroxybutanoic acid in an organism or a part thereof comprising:
(a) contacting cells, tissues, plants or microorganisms which express the a polypeptide encoded by the nucleic acid molecule as mentioned above conferring an increase in the amount of malic acid or trihydroxybutanoic acid in an organism or a part thereof with a candidate compound or a sample comprising a plurality of compounds under conditions which permit the expression the polypeptide;
(b) assaying the malic acid, or trihydroxybutanoic acid level or the polypeptide expression level in the cell, tissue, plant or microorganism or the media the cell, tissue, plant or microorganisms is cultured or maintained in; and
(c) identifying a agonist or antagonist by comparing the malic acid, or trihydroxybutanoic acid level or polypeptide expression level with a standard malic acid, or trihydroxybutanoic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring malic acid, or trihydroxybutanoic acid production in a plant or microorganism, comprising the steps:
(a) culturing a plant cell or tissue or microorganism or maintaining a plant expressing the polypeptide encoded by the nucleic acid molecule as mentioned above conferring an increase in the amount of malic acid, or trihydroxybutanoic acid in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of malic acid or trihydroxybutanoic acid in an organism or a part thereof;
(b) identifying if the compound is an effective agonist by detecting the presence or absence or increase of a signal produced by said readout system.
A method for the identification of a gene product conferring an increase in malic acid, or trihydroxybutanoic acid production in a cell, comprising the following steps:
(a) contacting the nucleic acid molecules of a sample, which can contain a candidate gene encoding a gene product conferring an increase in malic acid or trihydroxybutanoic acid after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing malic acid, or trihydroxybutanoic acid;
(d) expressing the identified nucleic acid molecules in the host cells;
(e) assaying the malic acid or trihydroxybutanoic acid level in the host cells; and
(f) identifying nucleic acid molecule and its gene product which expression confers an increase in the malic acid, or trihydroxybutanoic acid level in the host cell in the host cell after expression compared to the wild type.
A method for the identification of a gene product conferring an increase in malic acid or trihydroxybutanoic acid production in a cell, comprising the following steps:
(a) identifying in a data bank nucleic acid molecules of an organism; which can contain a candidate gene encoding a gene product conferring an increase in the malic acid, or trihydroxybutanoic acid amount or level in an organism or a part thereof after expression, and which are at least 20% homolog to the nucleic acid molecule as mentioned above;
(b) introducing the candidate nucleic acid molecules in host cells appropriate for producing malic acid or trihydroxybutanoic acid;
(c) expressing the identified nucleic acid molecules in the host cells;
(d) assaying the malic acid, or trihydroxybutanoic acid level in the host cells; and
(e) identifying nucleic acid molecule and its gene product which expression confers an increase in the glyceric acid. citramalic acid, fumaric acid, malic acid, pyruvic acid, succinic acid, trihydroxybutyric acid or trihydroxybutanoic acid level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of malic acid or trihydroxybutanoic acid after expression.
Use of the polypeptide as mentioned above or the nucleic acid construct as mentioned above or the gene product identified according to the method as mentioned above for identifying compounds capable of conferring a modulation of malic acid or trihydroxybutanoic acid levels in an organism.
Agrochemical, pharmaceutical, food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
The method as mentioned above, the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above, wherein the fine chemical is malic acid or trihydroxybutanoic acid.

### New genes for a process for the production of fine chemicals

### Description

[0000.0.0.17] In a further embodiment, the present invention relates to a further process for the production of fine chemicals as defined below and the corresponding embodiments as described herein as follows.

**[0001.0.0.17] see [0001.0.0.0]**

[0002.0.17.17] Gamma-aminobutyric acid is used to enhance growth of specified plants, prevent development of powdery mildew on grapes, and suppress certain other plant diseases. Humans and animals normally ingest and metabolize gamma-aminobutyric acid in variable amounts. Gamma-aminobutyric acid was registered (licensed for sale) as growth enhancing pesticidal active ingredient in 1998. Gamma-aminobutyric acid is an important signal which helps to regulate mineral availability in plants. Minerals support the biochemical pathways governing growth and reproduction as well as the pathways that direct plant's response to a variety of biotic and abiotic stresses. Mineral needs are especially high during times of stress and at certain stages of plant growth. Gamma-aminobutyric acid levels in plants naturally increase at these times.
Gamma-Aminobutyric acid (GABA), a nonprotein amino acid, is often accumulated in plants following environmental stimuli that can also cause ethylene production. Exogenous GABA causes up to a 14-fold increase in the ethylene production rate after about 12 h. GABA causes increases in ACC synthase mRNA accumulation, ACC levels, ACC oxidase mRNA levels and in vitro ACC oxidase activity. Possible roles of GABA as a signal transducer are suggested, see Plant Physiol.115(1):129-35(1997)
Gamma-aminobutyric acid (GABA), a four-carbon non-protein amino acid, is a significant component of the free amino acid pool in most prokaryotic and eukaryotic organisms. In plants, stress initiates a signal-transduction pathway, in which increased cytosolic Ca²⁺ activates Ca²⁺/calmodulin-dependent glutamate decarboxylase activity and GABA synthesis. Elevated H⁺ and substrate levels can also stimulate glutamate decarboxylase activity. GABA accumulation probably is mediated primarily by glutamate decarboxylase. Experimental evidence supports the involvement of GABA synthesis in pH regulation, nitrogen storage, plant development and defence, as well as a compatible osmolyte and an alternative pathway for glutamate utilization, see Trends Plant Sci. 4(11):446-452(1999).
Gamma-aminobutyric acid enhances nutrient uptake by roots and leaves so that plant nutrient levels are higher than those achieved by using nutrients alone. When plants are stressed and nutrient uptake is limited, it is believed that gamma-aminobutyric acid facilitates nutrient utilization, thereby enhancing growth during stress.
Rapid GABA accumulation in response to wounding may play a role in plant defense against insects (Ramputh and Brown, Plant Physiol. 111 (1996): 1349-1352). The development of gamma aminobutyrate (GABA) as a potential control agent in plant - invertebrate pest systems has been reviewed in Shelp et al., Canadien Journal of Botany (2003) 81, 11, 1045-1048. The authors describe that available evidence indicates that GABA accumulation in plants in response to biotic and abiotic stresses is mediated via the activation of glutamate decarboxylase. More applied research, based on the fact that GABA acts as an inhibitory neurotransmitter in invertebrate pests, indicates that ingested GABA disrupts nerve functioning and causes damage to oblique-banded leafroller larvae, and that walking or herbivory by tobacco budworm and oblique-banded leafroller larvae stimulate GABA accumulation in soybean and tobacco, respectively. In addition, elevated levels of endogenous GABA in genetically engineered tobacco deter feeding by tobacco budworm larvae and infestation by the northern root-knot nematode. Therefore the author concluded that genetically engineered crop species having high GABA-producing potential may be an alternative strategy to chemical pesticides for the management of invertebrate pests.
During angiosperm reproduction, pollen grains form a tube that navigates through female tissues to the micropyle, delivering sperm to the egg. In vitro, GABA stimulates pollen tube growth. The Arabidopsis POP2 gene encodes a transaminase that degrades GABA and contributes to the formation of a gradient leading up to the micropyle, see Cell. 114(1):47-59(2003).
Due to these interesting physiological roles and agrobiotechnological potential of GABA there is a need to identify the genes of enzymes and other proteins involved in GABA metabolism, and to generate mutants or transgenic plant lines with which to modify the GABA content in plants.

**[0003.0.0.17] %**

**[0004.0.0.17] %**

**[0005.0.0.17] %**

**[0006.0.0.17] %**

**[0007.0.0.17] %**

[0008.0.17.17] One way to increase the productive capacity of biosynthesis is to apply recombinant DNA technology. Thus, it would be desirable to produce gamma-aminobutyric acid in plants. That type of production permits control over quality, quantity and selection of the most suitable and efficient producer organisms. The latter is especially important for commercial production economics and therefore availability to consumers. In addition it is desireable to produce gamma-aminobutyric acid or shikimate in plants in order to increase plant productivity and resistance against biotic and abiotic stress as discussed before.
Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Argobacterium tumifaciens*-mediated transformation of tobacco plants using a vector that contained a ketolase-encoding gene from *H. pluvialis* denominated *crtO* along with the *Pds* gene from tomato as the promoter and to encode a leader sequence. Those results indicated that about 75 percent of the carotenoids found in the flower of the transformed plant contained a keto group.

[0009.0.17.17] Thus, it would be advantageous if an algae, plant or other microorganism were available which produce large amounts gamma-aminobutyric acid. The invention discussed hereinafter relates in some embodiments to such transformed prokaryotic or eukaryotic microorganisms.

It would also be advantageous if plants were available whose roots, leaves, stem, fruits or flowers produced large amounts of gamma-aminobutyric acid. The invention discussed hereinafter relates in some embodiments to such transformed plants.

[0010.0.17.17] Therefore improving the quality of foodstuffs and animal feeds is an important task of the food-and-feed industry. This is necessary since, for example gamma-aminobutyric acid, as mentioned above, which occur in plants and some microorganisms are limited with regard to the supply of mammals. Especially advantageous for the quality of foodstuffs and animal feeds is as balanced as possible a specific gamma-aminobutyric acid profile in the diet since an excess of gamma-aminobutyric acid above a specific concentration in the food has a positive effect. A further increase in quality is only possible via addition of further gamma-aminobutyric acid, which are limiting.

[0011.0.17.17] To ensure a high quality of foods and animal feeds, it is therefore necessary to add gamma-aminobutyric acid in a balanced manner to suit the organism.

[0012.0.17.17] Accordingly, there is still a great demand for new and more suitable genes which encode enzymes or other proteins which participate in the biosynthesis of gamma-aminobutyric acid and make it possible to produce them specifically on an industrial scale without unwanted byproducts forming. In the selection of genes for biosynthesis two characteristics above all are particularly important. On the one hand, there is as ever a need for improved processes for obtaining the highest possible contents of gamma-aminobutyric acid, on the other hand as less as possible byproducts should be produced in the production process.

**[0013.0.0.17] see [0013.0.0.0]**

[0014.0.17.17] Accordingly, in a first embodiment, the invention relates to a process for the production of a fine chemical, whereby the fine chemical is a gamma-aminobutyric acid. Accordingly, in the present invention, the term "the fine chemical" as used herein relates to a gamma-aminobutyric acid. Further, the term "the fine chemicals" as used herein also relates to fine chemicals comprising gamma-aminobutyric acid.

[0015.0.17.17] In one embodiment, the term "the fine chemical" or "the respective fine chemical" means at least one chemical compound with gamma-aminobutyric acid. In one embodiment, the term "the fine chemical" means a gamma-aminobutyric acid. Throughout the specification the term "the fine chemical" means gamma-aminobutyric acid, its salts, ester, thioester or in free form or bound to other compounds such sugars or sugarpolymers, like glucoside, e.g. diglucoside.

**[0016.0.17.17]** Accordingly, the present invention relates to a process for the production of the respective fine chemical comprising
(a) increasing or generating the activity of one or more
   of a protein as shown in table II, application no. 17, column 3 encoded by the nucleic acid sequences as shown in table I, application no. 17, column 5, in a non-human organism or in one or more parts thereof or
(b) growing the organism under conditions which permit the production of the fine chemical, thus GABA of the invention or fine chemicals comprising amino acids of the invention, in said organism or in the culture medium surrounding the organism

**[0017.0.0.17]** to **[0019.0.0.17]:** see **[0017.0.0.0]** to **[0019.0.0.0]**

[0020.0.17.17] Surprisingly it was found, that the transgenic expression of the Helianthus annuus protein as indicated in Table II, application no. 17, column 5, line 39 in a plant conferred an increase in gamma-Aminobutyric acid (GABA) content of the transformed plants. Thus, in one embodiment, said protein or its homologs are used for the production of gamma-Aminobutyric acid (GABA).

**[0021.0.0.17]** see **[0021.0.0.0]**

**[0022.0.17.17]** The sequence of b0651 from Escherichia coli K12 has been published in Blattner et al., Science 277 (5331), 1453-1474, 1997, and its activity is being defined as a pyrimidine specific nucleoside hydrolase. Accordingly, in one embodiment, the process of the present invention comprises the use of a protein with pyrimidine specific nucleoside hydrolase activity from Escherichia coli K12 or a plant or its homolog, e.g. as shown herein, for the production of the respective fine chemical, in particular for increasing the amount of gamma-aminobutyric acid preferably in free or bound form in an organism or a part thereof, as mentioned. In one embodiment, in the process of the present invention said activity, e.g. the activity of a protein with pyrimidine specific nucleoside hydrolase activity, is increased.

**[0022.1.0.17] to [0023.0.0.17] see [0022.1.0.0] [0023.0.0.0]**

**[0023.1.0.17]** Homologs of the polypeptide disclosed in table II, application no. 17, column 5, may have the activity of a protein as disclosed in table I or II, application no. 17, column 3 and may be the polypetides encoded by the nucleic acid molecules indicated in table I , application no. 17, column 7, resp., or may be the polypeptides indicated in table II, application no. 17, column 7, resp.

**[0024.0.0.17]** see **[0024.0.0.0]**

**[0025.0.17.17]ln** accordance with the invention, a protein or polypeptide has the "activity of an protein as shown in table II, application no. 17, column 3 "if its de *novo* activity, or its increased expression directly or indirectly leads to an increased in the level of the fine chemical indicated in the respective line of table II, application no. 17, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 17, column 3, or which has at least 10% of the original enzymatic or biological activity, preferably 20%, particularly preferably 30%, most particularly preferably 40% in comparison to a protein as shown in the respective line of table II, application no. 17, column 3 of a E. coli or Saccharomyces cerevisae, respectively, protein as mentioned in table I to IV, column 3 respectively and as disclosed in paragraph [0022] of the respective invention.

**[0025.1.0.17] see [0025.1.0.0]**

**[0026.0.0.17] to [0033.0.0.17]: see [0026.0.0.0] to [0033.0.0.0]**

**[0034.0.17.17]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 17, column 3 its biochemical or genetical causes and the increased amount of the respective fine chemical.

**[0035.0.0.17] to [0038.0.0.17]: see [0035.0.0.0] to [0038.0.0.0]**

**[0039.0.0.17]: see [0039.0.0.0]**

**[0040.0.0.17] to [0044.0.0.17]: see [0040.0.0.0] to [0044.0.0.0]**

[0045.0.17.17] In one embodiment, the activity of the protein. as indicated in Table II, columns 5 or 7, application no. 17, is increased conferring an increase of the respective fine chemical, indicated in Table II, application no. 17, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more

[0046.0.17.17] In one embodiment, the activity of the protein as indicated in Table II, columns 5 or 7, application no. 17, is increased conferring an increase of the respective fine chemical, indicated in Table II, application no. 17, column 6 of the respective line confers an increase of the respective fine chemical and of further GABA or their precursors.

**[0047.0.0.17] to [0048.0.0.17]: see [0047.0.0.0] to [0048.0.0.0]**

[0049.0.17.17]A protein having an activity conferring an increase in the amount or level of the fine chemical, has in one embodiment the structure of the polypeptide described herein, in particular of the polypeptides comprising the consensus sequence shown in table IV, application no. 17, column 7 or of the polypeptide as shown in the amino acid sequences as disclosed in table II, application no. 17, columns 5 and 7 or the functional homologues thereof as described herein, or is encoded by the nucleic acid molecule characterized herein or the nucleic acid molecule according to the invention, for example by the nucleic acid molecule as shown in table I, application no. 17, columns 5 and 7 or its herein described functional homologues and has the herein mentioned activity.

[0050.0.17.17] For the purposes of the present invention, the term "the respective fine chemical" also encompass the corresponding salts, such as, for example, the potassium or sodium salts of gamma-aminobutyric acid, or their ester, or glucoside thereof, e.g the diglucoside thereof.

[0051.0.17.17] Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising gamma-aminobutyric acid. Depending on the choice of the organism used for the process according to the present invention, for example a microorganism or a plant, compositions or mixtures of gamma-aminobutyric acid can be produced.

**[0052.0.0.17] see [0052.0.0.0]**

**[0053.0.17.17]ln** one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 17, columns 5 and 7 or its homologs activity having herein-mentioned GABA of the invention increasing activity; and/or
b) stabilizing a mRNA conferring the increased expression of a protein encoded by the nucleic acid molecule of the invention as shown in table I, application no. 17, columns 5 and 7, e.g. a nucleic acid sequence encoding a polypeptide having the activity of a protein as indicated in table II, application no. 17, columns 5 and 7 or its homologs activity or of a mRNA encoding the polypeptide of the present invention having herein-mentioned GABAof the invention increasing activity; and/or
c) increasing the specific activity of a protein conferring the increased expression of a protein encoded by the nucleic acid molecule of the invention or of the polypeptide of the present invention having herein-mentioned GABA increasing activity, e.g. of a polypeptide having the activity of a protein as indicated in table II, application no. 17, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned GABA of the invention increasing activity, e.g. of a polypeptide having the activity of a protein as indicated in table II, application no. 17, columns 5 and 7 or its homologs activity; and/or
e) stimulating activity of a protein conferring the increased expression of a protein encoded by the nucleic acid molecule of the present invention or a polypeptide of the present invention having herein-mentioned GABA of the invention increasing activity, e.g. of a polypeptide having the activity of a protein as indicated in table II, application no. 17, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned GABA of the invention increasing activity, e.g. of a polypeptide having the activity of a protein as indicated in table II, application no. 17, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned GABA of the invention increasing activity, e.g. of a polypeptide having the activity of a protein as indicated in table II, application no. 17, columns 5 and 7 or its homologs activity; and/or
h) increasing the expression of the endogenous gene encoding the polypeptide of the invention, e.g. a polypeptide having the activity of a protein as indicated in table II, application no. 17, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with expecially high activity of the proteins of the invention from natural or from mutagenized resources and breeding them into the target organisms, eg the elite crops.

[0054.0.17.17] Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table II, application no. 17, columns 3 or 5, , or its homologs activity, e.g. as indicated in Table II, application no. 17, columns 5 or 7,.

**[0055.0.0.17] to [0067.0.0.17]: see [0055.0.0.0] to [0067.0.0.0]**

[0068.0.17.17] The mutation is introduced in such a way that the production of gamma-aminobutyric acid or shikimate or putrescine is not adversely affected.

**[0069.0.0.17] see [0069.0.0.0]**

[0070.0.17.17] Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create de *novo* an advantageous, preferably novel metabolite composition in the organism, e.g. an advantageous composition of gamma-aminobutyric acid and shikimate or their biochemical derivatives , e.g. comprising a higher content of (from a viewpoint of nutrional physiology limited) gamma-aminobutyric acid and shikimate or putrescine or their derivatives.

**[0071.0.0.17] see [0071.0.0.0]**

[0072.0.17.17] By influencing the metabolism thus, it is possible to produce, in the process according to the invention, further advantageous compounds. Examples of such compounds are, in addition to shikimate their biochemical derivatives like chorismate, prephenate, anthranilate, phenylpyruvate, phenylalanine, 4-hydroxyphenylbutyrate, tyrosine, tryptophan, vanillin, salicylic acid, lawsone or scopletin.

[0073.0.17.17] Accordingly, in one embodiment, the process according to the invention relates to a process, which comprises:
a) providing a non-human organism, preferably a microorganism, a non-human animal, a plant or animal cell, a plant or animal tissue or a plant;
b) increasing an activity of a polypeptide of the invention or a homolog thereof, e.g. as indicated in Table II, application no. 17, columns 5 or 7, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
d) if desired, recovering, optionally isolating, the free and/or bound the respective fine chemical synthesized by the organism, the microorganism, the non-human animal, the plant or animal cell, the plant or animal tissue or the plant.

[0074.0.17.17] The organism, in particular the microorganism, non-human animal, the plant or animal cell, the plant or animal tissue or the plant is advantageously grown in such a way that it is not only possible to recover, if desired isolate the free or bound respective fine chemical.

**[0075.0.0.17] to [0077.0.0.17]: see [0075.0.0.0] to [0077.0.0.0]**

[0078.0.17.17] The organism such as microorganisms or plants or the recovered, and if desired isolated, the respective fine chemical can then be processed further directly into foodstuffs or animal feeds or for other applications. The fermentation broth, fermentation products, plants or plant products can be purified with methods known to the person skilled in the art. Products of these different work-up procedures are gamma-aminobutyric acid or shikimate or putrescine or comprising compositions of gamma-butyric acid or shikimate or putrescine still comprising fermentation broth, plant particles and cell components in different amounts, advantageously in the range of from 0 to 99% by weight, preferably below 80% by weight, especially preferably below 50% by weight.

**[0079.0.0.17] to [0084.0.0.17]: see [0079.0.0.0] to [0084.0.0.0]**

**[0084.0.17.17] %**

[0085.0.17.17] With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, application no. 17, columns 5 or 7, or a derivative thereof, or
b) a genetic regulatory element, for example a promoter, which is functionally linked to the nucleic acid sequence as indicated in Table I, application no. 17, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.17] to [0087.0.0.17]: see [0086.0.0.0] to [0087.0.0.0]**

[0088.0.17.17] In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned fine chemicals or the plants are more resistant to biotic and abiotic stress and the yield is increased.

**[0088.1.0.17] see [0088.1.0.0]**

**[0089.0.0.17] to [0090.0.0.17]: see [0089.0.0.0] to [0090.0.0.0]**

**[0091.0.0.17] see [0091.0.0.0]**

**[0092.0.0.17] to [0094.0.0.17]: see [0092.0.0.0] to [0094.0.0.0]**

[0095.0.17.17] It may be advantageous to increase the pool of gamma-aminobutyric acid or shikimate or putrescine in the transgenic organisms by the process according to the invention in order to isolate high amounts of the pure respective fine chemical and/or to obtain increased resistance against biotic and abiotic stresses and to obtain higher yield.

[0096.0.17.17] In another preferred embodiment of the invention a combination of the increased expression of the nucleic acid sequence or the protein of the invention together with the transformation of a protein or polypeptid or a compound, which functions as a sink for the desired fine chemical, for example in the organism, is useful to increase the production of the respective fine chemical.

**[0097.0.17.17] %**

[0098.0.17.17] In a preferred embodiment, the respective fine chemical is produced in accordance with the invention and, if desired, is isolated.

[0099.0.10.17] In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

[0100.0.17.17] Transgenic plants which comprise the fine chemicals such as gamma-aminobutyric acid or shikimate or putrescine synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because gamma-aminobutyric acid or shikimate or putrescine in microorganisms are localized intracellular, their recovery essentially comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.

[0101.0.10.17] The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

[0102.0.17.17] Gamma-aminobutyric acid or shikimate or putrescine can for example be detected advantageously via HPLC, LC or GC separation methods. The unambiguous detection for the presence of gamma-aminobutyric acid or shikimate containing or putrescine products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.

[0103.0.17.17] In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, application no. 17, columns 5 or 7, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 17, columns 5 or 7,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, application no. 17, columns 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, application no. 17, columns 7, and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, application no. 17, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[0104.0.17.17]** In one embodiment, the nucleic acid molecule used in the process of the present ivention distinguishes over the sequence indicated in Table I, application no. 17, columns 5 or 7, by one or more nucleotides. In one embodiment, the nucleic acid molecule used in the process of the invention does not consist of the sequence indicated in Table I, application no. 17, columns 5 or 7,. In one embodiment, the nucleic acid molecule of the present invention is less than 100%, 99,999%, 99,99%, 99,9% or 99% identical to the sequence indicated in Table I, application no. 17, columns 5 or 7. In another embodiment, the nucleic acid molecule does not encode a polypeptide of a sequence indicated in Table II, application no. 17, columns 5 or 7.

**[0105.0.0.17] to [0107.0.0.17]: see [0105.0.0.0] to [0107.0.0.0]**

[0108.0.17.17] Nucleic acid molecules with the sequence as indicated in Table I, application no. 17, columns 5 or 7, nucleic acid molecules which are derived from an amino acid sequences as indicated in Table II, application no. 17, columns 5 or 7, or from polypeptides comprising the consensus sequence as indicated in Table IV, application no. 17, column 7, or their derivatives or homologues encoding polypeptides with the enzymatic or biological activity of an activity of a polypeptide as indicated in Table II, application no. 17, column 3, 5 or 7, e.g. conferring the increase of the respective fine chemical, meaning gamma-aminobutyric acid or shikimate or putrescine, resp., after increasing its expression or activity, are advantageously increased in the process according to the invention.

[0109.0.17.17] In one embodiment, said sequences are cloned into nucleic acid constructs, either individually or in combination. These nucleic acid constructs enable an optimal synthesis of the respective fine chemicals, in particular gamma-aminobutyric acid or shikimate or putrescine, produced in the process according to the invention.

**[0110.0.0.17] see [0110.0.0.0]**

**[0111.0.0.17] see [0111.0.0.0]**

**[0112.0.17.17]** The nucleic acid molecules used in the process according to the invention take the form of isolated nucleic acid sequences, which encode polypeptides with an activity of a polypeptide as indicated in Table II, application no. 17, column 3, or having the sequence of a polypeptide as indicated in Table II, application no. 17, columns 5 and 7, and conferring an increase in the gamma-aminobutyric acid or shikimate or putrescine level.

**[0113.0.0.17] to [0114.0.0.17]: see [0113.0.0.0] to [0114.0.0.0]**

**[0115.0.0.17] see [0115.0.0.0]**

**[0116.0.0.17] to [0120.0.0.17] see [0116.0.0.0] to [0120.0.0.0]**

**[0120.1.0.17]: %**

[0121.0.17.17] However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 17, columns 5 or 7, or the functional homologues thereof as described herein, preferably conferring above-mentioned activity, i.e. conferring a gamma-aminobutyric acid level increase after increasing the activity of the polypeptide sequences indicated in Table II, columns 5 or 7,

**[0122.0.0.17] to [0127.0.0.17]: see [0122.0.0.0] to [0127.0.0.0]**

**[0128.0.17.17]** Synthetic oligonucleotide primers for the amplification, e.g. as the pairs indicated in Table III, application no. 17, columns 7, by means of polymerase chain reaction can be generated on the basis of a sequence shown herein, for example the sequence as indicated in Table I, application no. 17, columns 5 or 7, or the sequences derived from a sequences as indicated in Table II, application no. 17, columns 5 or 7,.

[0129.0.17.17] Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consensus sequence indicated in Table IV, application no. 17, columns 7, is derived from said alignments.

[0130.0.17.17] Degenerated primers can then be utilized by PCR for the amplification of fragments of novel proteins having above-mentioned activity, e.g. conferring the increase of gamma-aminobutyric acid or shikimate after increasing the expression or activity the protein comprising said fragment.

**[0131.0.0.17] to [0138.0.0.17]: see [0131.0.0.0] to [0138.0.0.0]**

**[0139.0.17.17]** Polypeptides having above-mentioned activity, i.e. conferring the respective fine chemical level increase, derived from other organisms, can be encoded by other DNA sequences which hybridise to a sequence indicated in Table I, application no. 17, columns 5 or 7, under relaxed hybridization conditions and which code on expression for peptides having the respective fine chemical, i.e. gamma-aminobutyric acid increasing-activity.

**[0140.0.0.17] to [0146.0.0.17]: see [0140.0.0.0] to [0146.0.0.0]**

**[0147.0.17.17]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 17, columns 5 or 7, is one which is sufficiently complementary to one of said nucleotide sequences such that it can hybridise to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.17.17]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 17, columns 5 or 7, or a portion thereof and preferably has above mentioned activity, in particular having a gamma-aminobutyric acid or shikimate or putrescine increasing activity after increasing the acitivity or an activity of a product of a gene encoding said sequences or their homologs.

**[0149.0.17.17]** The nucleic acid molecule of the invention comprises a nucleotide sequence which hybridizes, preferably hybridizes under stringent conditions as defined herein, to one of the nucleotide sequences indicated in Table I, application no. 17, columns 5 or 7, or a portion thereof and encodes a protein having above-mentioned activity, e.g. conferring a of gamma-aminobutyric acid or shikimate or putrescine increase, resp., and optionally, the activity of protein indicated in Table II, application no. 17, column 5.

**[00149.1.0.17]** Optionally, in one embodiment, the nucleotide sequence, which hybridises to one of the nucleotide sequences indicated in Table I, application no. 17, columns 5 or 7, has further one or more of the activities annotated or known for a protein as indicated in Table II, application no. 17, column 3.

[0150.0.17.17] Moreover, the nucleic acid molecule of the invention can comprise only a portion of the coding region of one of the sequences indicated in Table I, application no. 17, columns 5 or 7,, for example a fragment which can be used as a probe or primer or a fragment encoding a biologically active portion of the polypeptide of the present invention or of a polypeptide used in the process of the present invention, i.e. having above-mentioned activity, e.g.conferring an increase of gamma-aminobutyric acid if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 17, columns 5 or 7, an anti-sense sequence of one of the sequences, e.g., as indicated in Table I, application no. 17, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 17, column 7, will result in a fragment of a polynucleotide sequence as indicated in Table I, application no. 17, columns 5 or 7, or its gene product.

**[0151.0.0.17]: see [0151.0.0.0]**

**[0152.0.17.17]** The nucleic acid molecule of the invention encodes a polypeptide or portion thereof which includes an amino acid sequence which is sufficiently homologous to an amino acid sequence as indicated in Table II, application no. 17, columns 5 or 7, such that the protein or portion thereof maintains the ability to participate in the respective fine chemical production, in particular a gamma-aminobutyric acid increasing activity as mentioned above or as described in the examples in plants or microorganisms is comprised.

[0153.0.17.17] As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 17, columns 5 or 7such that the protein or portion thereof is able to participate in the increase of the respective fine chemical production. In one embodiment, a protein or portion thereof as indicated in Table II, application no. 17, columns 5 or 7, has for example an activity of a polypeptide indicated in Table II, application no. 17, column 3.

[0154.0.17.17] In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, application no. 17, columns 5 or 7, and has above-mentioned activity, e.g.conferring preferably the increase of the respective fine chemical.

**[0155.0.0.17] to [0156.0.0.17]: see [0155.0.0.0] to [0156.0.0.0]**

[0157.0.17.17] The invention further relates to nucleic acid molecules that differ from one of the nucleotide sequences as indicated in Table I, application no. 17, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, application no. 17, columns 7, or as polypeptides depicted in Table II, application no. 17, columns 5 or 7, or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, application no. 17, columns 7, or of the polypeptide as indicated in Table II, application no. 17, columns 5 or 7, or the functional homologues. In a still further embodiment, the nucleic acid molecule of the invention encodes a full length protein which is substantially homologous to an amino acid sequence comprising a consensus sequence as indicated in Table IV, application no. 17, columns 5 or 7, or of a polypeptide as indicated in Table II, application no. 17, columns 5 or 7, or the functional homologues. However, in a preferred embodiment, the nucleic acid molecule of the present invention does not consist of a sequence as indicated in Table I, application no. 17, columns 5 or 7.

**[0158.0.0.17] to [0160.0.0.17]: see [0158.0.0.0] to [0160.0.0.0]**

[0161.0.17.17] Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 17, columns 5 or 7,. The nucleic acid molecule is preferably at least 20, 30, 50, 100, 250 or more nucleotides in length.

**[0162.0.0.17] see [0162.0.0.0]**

[0163.0.17.17] Preferably, a nucleic acid molecule of the invention that hybridizes under stringent conditions to a sequence as indicated in Table I, application no. 17, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in a organism or a part thereof, e.g. a tissue, a cell, or a compartment of a cell, after increasing the expression or activity thereof or the activity of a protein of the invention or used in the process of the invention.

**[0164.0.0.17] see [0164.0.0.0]**

**[0165.0.17.17]** For example, nucleotide substitutions leading to amino acid substitutions at "non-essential" amino acid residues can be made in a sequence of the nucleic acid molecule of the invention or used in the process of the invention, e.g. as indicated in Table I, application no. 17, columns 5 or 7.

**[0166.0.0.17] to [0167.0.0.17]: see [0166.0.0.0] to [0167.0.0.0]**

[0168.0.17.17] Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table II, application no. 17, columns 5 or 7, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 17, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 17, columns 5 or 7, , more preferably at least about 70% identical to one of the sequences as indicated in Table II, application no. 17, columns 5 or 7, , even more preferably at least about 80%, 90%, 95% homologous to a sequence as indicated in Table II, application no. 17, columns 5 or 7, and most preferably at least about 96%, 97%, 98%, or 99% identical to the sequence as indicated in Table II, application no. 17, columns 5 or 7.

**[0169.0.0.17] to [0172.0.0.17]: see [0169.0.0.0] to [0172.0.0.0]**

**[0173.0.17.17]** For example a sequence, which has 80% homology with sequence SEQ ID NO: 1 at the nucleic acid level is understood as meaning a sequence which, upon comparison with the sequence SEQ ID NO: 1 by the above Gap program algorithm with the above parameter set, has a 80% homology.

**[0174.0.0.17]: see [0174.0.0.0]**

**[0175.0.17.17]** For example a sequence which has a 80% homology with sequence SEQ ID NO: 2 at the protein level is understood as meaning a sequence which, upon comparison with the sequence SEQ ID NO: 2 by the above program algorithm with the above parameter set, has a 80% homology.

**[0176.0.17.17]** Functional equivalents derived from one of the polypeptides as indicated in Table II, application no. 17, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table II, application no. 17, columns 5 or 7, according to the invention and are distinguished by essentially the same properties as a polypeptide as indicated in Table II, application no. 17, columns 5 or 7,.

[0177.0.17.17] Functional equivalents derived from a nucleic acid sequence as indicated in Table I, application no. 17, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table II, application no. 17, columns 5 or 7, according to the invention and encode polypeptides having essentially the same properties as a polypeptide as indicated in Table II, application no. 17, columns 5 or 7,.

**[0178.0.0.17] see [0178.0.0.0]**

**[0179.0.17.17]** A nucleic acid molecule encoding a homologous to a protein sequence as indicated in Table II, application no. 17, columns 5 or 7can be created by introducing one or more nucleotide substitutions, additions or deletions into a nucleotide sequence of the nucleic acid molecule of the present invention, in particular as indicated in Table I, application no. 17, columns 5 or 7, such that one or more amino acid substitutions, additions or deletions are introduced into the encoded protein. Mutations can be introduced into the encoding sequences as indicated in Table I, application no. 17, columns 5 or 7, by standard techniques, such as site-directed mutagenesis and PCR-mediated mutagenesis.

**[0180.0.0.17] to [0183.0.0.17]: see [0180.0.0.0] to [0183.0.0.0]**

**[0184.0.17.17]** Homologues of the nucleic acid sequences used, with a sequence as indicated in Table I, application no. 17, columns 5 or 7, or of the nucleic acid sequences derived from a sequences as indicated in Table II, application no. 17, columns 5 or 7, , comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 17, columns 5 or 7, or from the derived nucleic acid sequences, the intention being, however, that the enzyme activity or the biological activity of the resulting proteins synthesized is advantageously retained or increased.

[0185.0.17.17] In one embodiment of the present invention, the nucleic acid molecule of the invention or used in the process of the invention comprises one or more sequences as indicated in Table I, application no. 17, columns 5 or 7,. In one embodiment, it is preferred that the nucleic acid molecule comprises as little as possible other nucleotides not shown in any one of sequences as indicated in Table I, application no. 17, columns 5 or 7,. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequence as indicated in Table I, columns 5 or 7,.

[0186.0.17.17] Also preferred is that one or more nucleic acid molecule(s) used in the process of the invention encodes a polypeptide comprising a sequence as indicated in Table II, application no. 17, columns 5 or 7,. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 17, columns 5 or 7,.

**[0187.0.17.17]** In one embodiment, a nucleic acid molecule of the invention or used in the process encodes a polypeptide comprising the sequence as indicated in Table II, application no. 17, columns 5 or 7,., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 17, columns 5 or 7.

**[0188.0.17.17]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 17, columns 5 or 7, and is expressed under identical conditions.

[0189.0.17.17] Homologues of a sequences as indicated in Table I, application no. 17, columns 5 or 7, or of a derived sequences as indicated in Table II, application no. 17, columns 5 or 7, also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (=ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.17]: see [0190.0.0.0]**

[0191.0.17.17] The organisms or part thereof produce according to the herein mentioned process of the invention an increased level of free and/or bound the respective fine chemical compared to said control or selected organisms or parts thereof.

**[0192.0.0.17] to [0203.0.0.17]: see [0192.0.0.0] to [0203.0.0.0]**

[0204.0.17.17] Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, application no. 17, columns 5 or 7, or a fragment thereof conferring an increase in the amount of the respective fine chemical, i.e. gamma-aminobutyric in an organism or a part thereof
b) nucleic acid molecule comprising, preferably at least the mature form, of a nucleic acid molecule as indicated in Table I, application no. 17, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in Table III, application no. 17, columns 5 or 7, and conferring an increase in the amount of the respective fine chemical, i.e. gamma-aminobutyric acid in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, application no. 17, columns 5 or 7, and conferring an increase in the amount of the respective fine chemical, i.e. gamma-aminobutyric acid in an organism or a part thereof;
k) nucleic acid molecule encoding the amino acid sequence of a polypeptide encoding a domaine of a polypeptide as indicated in Table II, application no. 17, columns 5 or 7, and conferring an increase in the amount of the respective fine chemical, i.e. gamma-aminobutyric acid in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 17, columns 5 or 7, or a nucleic acid molecule encoding, preferably at least the mature form of, a polypeptide as indicated in Table II, application no. 17, columns 5 or 7, and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table I, application no. 17, columns 5 or 7, by one or more nucleotides. In one embodiment, the nucleic acid molecule of the invention does not consist of the sequence as indicated in Table I, application no. 17, columns 5 or 7. In an other embodiment, the nucleic acid molecule of the present invention is at least 30 % identical and less than 100%, 99,999%, 99,99%, 99,9% or 99% identical to a sequence as indicated in Table I, application no. 17, columns 5 or 7, resp. In a further embodiment the nucleic acid molecule does not encode a polypeptide sequence as indicated in Table II, application no. 17, columns 5 or 7,. Accordingly, in one embodiment, the nucleic acid molecule of the present invention encodes in one embodiment a polypeptide which differs at least in one or more amino acids from a polypeptide indicated in Table II, application no. 17, columns 5 or 7, does not encode a protein of a sequence as indicated in Table II, application no. 17, columns 5 or 7,. Accordingly, in one embodiment, the protein encoded by a sequences of a nucleic acid accoriding to (a) to (I) does not consist of a sequence as indicated in Table II, application no. 17, columns 5 or 7,. In a further embodiment, the protein of the present invention is at least 30 % identical to a protein sequence indicated in Table II, application no. 17, columns 5 or 7, and less than 100%, preferably less than 99,999%, 99,99% or 99,9%, more preferably less than 99%, 98%, 97%, 96% or 95% identical to a sequence as indicated in Table II, application no. 17, columns 5 or 7.

**[0205.0.0.17] to [0206.0.0.17]: see [0205.0.0.0] to [0206.0.0.0]**

[0207.0.17.17] As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.17] to [0226.0.0.17]: see [0208.0.0.0] to [0226.0.0.0]**

[0227.0.17.17] The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table I, application no. 17, columns 5 or 7, or its derivatives, it is advantageous to express and/or mutate further genes in the organisms. Especially advantageously, additionally at least one further gene of the glutamic acid or phosphoenolpyruvate metabolic pathway, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table I, application no. 17, columns 5 or 7, , with genes which generally support or enhances to growth or yield of the target organismen, for example genes which lead to faster growth rate of microorganisms or genes which produces stress-, pathogen, or herbicide resistant plants.

[0228.0.17.17] In a further embodiment of the process of the invention, therefore, organisms are grown, in which there is simultaneous overexpression of at least one nucleic acid or one of the genes which code for proteins directly or indirectly involved in the glutamic acid or phosphoenolpyruvate metabolism.

[0229.0.17.17] Further advantageous nucleic acid sequences which can be expressed in combination with the sequences indicated in Table I, application no. 17, columns 5 or 7, used in the process and/or the abovementioned biosynthesis genes are the sequences encoding further genes of the aromatic amino acid pathway, such as tryptophan, phenylalanine or tyrosine.These genes can lead to an increased synthesis of the essential amino acids tryptophan, phenylalanine or tyrosine.

**[0230.0.0.17] see [230.0.0.0]**

[0231.0.17.17] In a further advantageous embodiment of the process of the invention, the organisms used in the process are those in which simultaneously a gamma-aminobutyric acid degrading protein is attenuated, in particular by reducing the rate of expression of the corresponding gene. A person skilled in the art knows for example, that the inhibition of an enzyme or a gene of the biosynthesis of the essential aromatic amino acids tryptophan, tyrosine or phenylalanine may increase the amount of shikimate accumulating in an organism, in particular in plants. Furthermore the inhibition of tryptophan, phenylalanine or tyrosine degrading enzymes also may result in an increased shikimate accumulation in the organism.

**[0232.0.0.17] to [0276.0.0.17]: see [0232.0.0.0] to [0276.0.0.0]**

[0277.0.17.17] The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.17] to [0282.0.0.17]: see [0278.0.0.0] to [0282.0.0.0]**

**[0283.0.17.17]** Moreover, native polypeptide conferring the increase of the respective fine chemical in an organism or part thereof can be isolated from cells, for example using the antibody of the present invention as described below, e.g. an antibody against a protein as indicated in Table II, application no. 17, column 3, or an antibody against a polypeptide as indicated in Table II, application no. 17, columns 5 or 7, which can be produced by standard techniques utilizing the polypeptid of the present invention or fragment thereof. Preferred are monoclonal antibodies.

**[0284.0.0.17] see [0284.0.0.0]**

**[0285.0.17.17]** In one embodiment, the present invention relates to a polypeptide having a sequence as indicated in Table II, application no. 17, columns 5 or 7, or as coded by a nucleic acid molecule as indicated in Table I, application no. 17, columns 5 or 7, or functional homologues thereof.

[0286.0.17.17] In one advantageous embodiment, in the method of the present invention the activity of a polypeptide is increased which comprises or consists of a consensus sequence as indicated in Table IV, application no. 17, column 7, and in one another embodiment, the present invention relates to a polypeptide comprising or consisting of a consensus sequence as indicated in Table IV, application no. 17, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, application no. 17, column 7.

**[0287.0.0.17]** to **[0289.0.0.17]: see [0287.0.0.0]** to **[0289.0.0.0]**

**[00290.0.17.17]** The alignment was performed with the Software AlignX (sept 25, 2002) a component of Vector NTI Suite 8.0, InforMax™, Invitrogen™ life science software, U.S. Main Office, 7305 Executive Way,Frederick, MD 21704,USA with the following settings: For pairwise alignments: gap opening penality: 10,0; gap extension penality 0,1. For multiple alignments: Gap opening penalty: 10,0; Gap extension penalty: 0,1; Gap separation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids

[0291.0.17.17] In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II, application no. 17, columns 5 or 7, by one or more amino acids. In one embodiment, polypeptide distinguishes from a sequence as indicated in Table II, application no. 17, columns 5 or 7, by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 17, columns 5 or 7, by not more than 80% or 70% of the amino acids, preferably not more than 60% or 50%, more preferred not more than 40% or 30%, even more preferred not more than 20% or 10%. **[0292.0.0.17] see [0292.0.0.0]**

[0293.0.17.17] In one embodiment, the invention relates to a polypeptide conferring an increase in the fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table II, application no. 17, columns 5 or 7, by one or more amino acids. In an other embodiment, said polypeptide of the invention does not consist of the sequence as indicated in Table II, application no. 17, columns 5 or 7. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 17, columns 5 or 7.

[0294.0.17.17] In one embodiment, the present invention relates to a polypeptide having an activity of a protein as indicated in Table II, application no. 17, column 3, which distinguishes over a sequence as indicated in Table II, application no. 17, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.17] to [0296.0.0.17]: see [0295.0.0.0] to [0296.0.0.0]**

**[0297.0.0.17] see [0297.0.0.0]**

**[00297.1.0.17] %**

[0298.0.17.17] A polypeptide of the invention can participate in the process of the present invention. The polypeptide or a portion thereof comprises preferably an amino acid sequence which is sufficiently homologous to an amino acid sequence as indicated in Table II, application no. 17, columns 5 or 7,. The portion of the protein is preferably a biologically active portion as described herein. Preferably, the polypeptide used in the process of the invention has an amino acid sequence identical to a sequence as indicated in Table II, application no. 17, columns 5 or 7.

[0299.0.17.17] Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, application no. 17, columns 5 or 7,. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 17, columns 5 or 7, , or which is homologous thereto, as defined above.

**[0300.0.17.17]** Accordingly the polypeptide of the present invention can vary from a sequence as indicated in Table II, application no. 17, columns 5 or 7 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of as indicated in Table II, application no. 17, columns 5 or 7,.

**[0301.0.0.17] see [0301.0.0.0]**

**[0302.0.17.17]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 17, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.17] see [0303.0.0.0]**

**[0304.0.17.17]** Manipulation of the nucleic acid molecule of the invention may result in the production of a protein having essentially the activity of the polypeptides as indicated in Table II, application no. 17, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.17] to [0308.0.0.17]: see [0305.0.0.0] to [0308.0.0.0]**

**[0309.0.17.17]** In one embodiment, a reference to a protein (= polypeptide) of the invention or as indicated in Table II, application no. 17, columns 5 or 7, refers to a polypeptide having an amino acid sequence corresponding to the polypeptide of the invention or used in the process of the invention, whereas an "other polypeptide" not being indicated in Table II, application no. 17, columns 5 or 7, refers to a polypeptide having an amino acid sequence corresponding to a protein which is not substantially homologous to a polypeptide of the invention, preferably which is not substantially homologous to a polypeptide as indicated in Table II, application no. 17, columns 5 or 7, " e.g., a protein which does not confer the activity described herein or annotated or known for as indicated in Table II, application no. 17, column 3, and which is derived from the same or a different organism. In one embodiment, an "other polypeptide" not being indicated in Table II, application no. 17, columns 5 or 7, does not confer an increase of the respective fine chemical in an organism or part thereof.

**[0310.0.0.17] to [0334.0.0.17]: see [0310.0.0.0] to [0334.0.0.0] [0335.0.17.17]**
The dsRNAi method has proved to be particularly effective and advantageous for reducing the expression of a nucleic acid sequences as indicated in Table IA or IB, columns 5 or 7, lines 227 to 229 or line 230 or lines 595 to 598, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, application no. 17, columns 5 or 7, and/or homologs thereof. In a double-stranded RNA molecule for reducing the expression of aprotein encoded by a nucleic acid sequence as indicated in Table I, application no. 17, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.17] to [0342.0.0.17]: see [0336.0.0.0] to [0342.0.0.0]**

[0343.0.17.17] As has already been described, 100 % sequence identity between the dsRNA and a gene transcript of a nucleic acid sequence as indicated in Table I, application no. 17, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 17, columns 5 or 7, or homologs thereof of the one organism, may be used to suppress the corresponding expression in another organism.

**[0344.0.0.17] to [0350.0.0.17]: see [0344.0.0.0] to [0350.0.0.0]**

**[0351.0.0.17] to [0361.0.0.17]: see [0351.0.0.0] to [0361.0.0.0]**

**[0362.0.17.17]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 17, columns 5 or 7, e.g. encoding a polypeptide having protein activity, as indicated in Table II, application no. 17, columns 3,. Due to the abovementioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, application no. 17, columns 5 or 7, means herein that due to modulation or manipulation of the genome, an activity as annotated for a polypeptide as indicated in Table II, application no. 17, column 3, e.g. having a sequence as indicated in Table II, application no. 17, columns 5 or 7, , is increased in a cell or an organism or a part thereof. Examples are described above in context with the process of the invention.

**[0363.0.0.17] see [0363.0.0.0]**

[0364.0.17.17] A naturally occurring expression cassette - for example the naturally occurring combination of a promoter of a gene encoding a polypeptide of the invention as indicated in Table II, application no. 17, column 3, with the corresponding protein-encoding sequence as indicated in Table I, application no. 17, column 5, , becomes a transgenic expression cassette when it is modified by non-natural, synthetic "artificial" methods such as, for example, mutagenization. Such methods have been described (US 5,565,350; WO 00/15815; also see above).

**[0365.0.0.17] to [0373.0.0.17]: see [0365.0.0.0] to [0373.0.0.0]**

[0374.0.17.17] Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. gamma-aminobutyric acid or shikimate, in particular the respective fine chemical, produced in the process according to the invention may, however, also be isolated from the plant in the form of their free gamma-aminobutyric acid, in particular the free respective fine chemical, or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.17] to [0376.0.0.17]: see [0375.0.0.0] to [0376.0.0.0]**

[0377.0.17.17] Accordingly, the present invention relates also to a process whereby the produced gamma-aminobutyric acid or shikimate is isolated.

[0378.0.17.17] In this manner, more than 50% by weight, advantageously more than 60% by weight, preferably more than 70% by weight, especially preferably more than 80% by weight, very especially preferably more than 90% by weight, of the gamma-aminobutyric acid or shikimate produced in the process can be isolated. The resulting gamma-aminobutyric acid or shikimate can, if appropriate, subsequently be further purified, if desired mixed with other active ingredients such as vitamins, amino acids, carbohydrates, antibiotics and the like, and, if appropriate, formulated.

[0379.0.17.17] In one embodiment, gamma-aminobutyric acid and shikimate are a mixture of the respective fine chemicals.

[0380.0.17.17] The gamma-aminobutyric acid or shikimate obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the gamma-aminobutyric acid or shikimate composition produced or the respective fine chemical produced if desired and formulating the product with a pharmaceutical acceptable carrier or formulating the product in a form acceptable for an application in agriculture. A further embodiment according to the invention is the use of the gamma-aminobutyric acid or shikimate produced in the process or of the transgenic organisms in animal feeds, foodstuffs, medicines, food supplements, cosmetics or pharmaceuticals or for the production of gamma-aminobutyric acid or shikimate e.g. after isolation of the respective fine chemical or without, e.g. in situ, e.g in the organism used for the process for the production of the respective fine chemical.

**[0381.0.0.17] to [0382.0.0.17]: see [0381.0.0.0] to [0382.0.0.0]**

**[0383.0.17.17]**

**[0384.0.0.17] see [0384.0.0.0]**

[0385.0.17.17] The fermentation broths obtained in this way, containing in particular gamma-aminobutyric acid or shikimate in mixtures with other organic acids, aminoacids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

[0386.0.17.17] Accordingly, it is possible to purify the gamma-aminobutyric acid or shikimate produced according to the invention further. For this purpose, the product-containing compositions subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.17] to [0392.0.0.17]: see [0387.0.0.0] to [0392.0.0.0]**

[0393.0.17.17] In one embodiment, the present invention relates to a method for the identification of a gene product conferring an increase in the respective fine chemical production in a cell, comprising the following steps:
a. contacting; e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
b. identifying the nucleic acid molecules, which hybridize under relaxed stringent conditions with the nucleic acid molecule of the present invention in particular to the nucleic acid molecule sequence as indicated in Table I, application no. 17, columns 5 or 7, and, optionally, isolating the full length cDNA clone or complete genomic clone;
c. introducing the candidate nucleic acid molecules in host cells, preferably in a plant cell or a microorganism, appropriate for producing the respective fine chemical;
d. expressing the identified nucleic acid molecules in the host cells;
e. assaying the respective fine chemical level in the host cells; and
f. identifying the nucleic acid molecule and its gene product which expression confers an increase in the respective fine chemical level in the host cell after expression compared to the wild type.

### [0394.0.0.17] to [0398.0.0.17]: see [0394.0.0.0] to [0398.0.0.0]

[0399.0.17.17] Furthermore, in one embodiment, the present invention relates to process for the identification of a compound conferring increase of the respective fine chemical production in a plant or microorganism, comprising the steps:
a) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
b) identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in respective fine chemical production.

[00399.1.0.17] One can think to screen for increased production of the respective fine chemical by for example searching for a resistance to a drug blocking the synthesis of the respective fine chemical and looking whether this effect is dependent on the activity or expression of a polypeptide as indicated in Table II, application no. 17, columns 5 or 7or a homolog thereof, e.g. comparing the phenotype of nearly identical organisms with low and high activity of a protein as indicated in Table II, application no. 17, columns 5 or 7, after incubation with the drug.

**[0400.0.0.17] to [0415.0.0.17]: see [0400.0.0.0] to [0415.0.0.0]**

**[0416.0.0.17] see [0416.0.0.0]**

[0417.0.17.17] The nucleic acid molecule of the invention, the vector of the invention or the nucleic acid construct of the invention may also be useful for the production of organisms resistant to inhibitors of the gamma-aminobutyric acid or shikimate or putrescine biosynthesis pathways. In particular, the overexpression of the polypeptide of the present invention may protect an organism such as a microorganism or a plant against inhibitors, which block the gamma-aminobutyric acid or shikimate or putrescine synthesis.

**[0418.0.0.17] to [0423.0.0.17]: see [0418.0.0.0] to [0423.0.0.0]**

[0424.0.17.17] Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other organic acids. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.17] to [0434.0.0.0]: see [0425.0.0.0] to [0434.0.0.0]**

[0435.0.17.17] Example 3: In-vivo and in-vitro mutagenesis

**[0436.0.17.17]** An *in vivo* mutagenesis of organisms such as Saccharomyces, Mortierella, Escherichia and others mentioned above, which are beneficial for the production of gamma-aminobutyric acid or shikimate or putrescine can be carried out by passing a plasmid DNA (or another vector DNA) containing the desired nucleic acid sequence or nucleic acid sequences, e.g. the nucleic acid molecule of the invention or the vector of the invention, through E.coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (=EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. DpnI site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.17.17]** Example 4: DNA transfer between Escherichia coli, Saccharomyces cerevisiae and Mortierella alpina

[0438.0.17.17] Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which allow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance ( Winnacker, E.L. (1987) "From Genes to Clones - Introduction to Gene Technology", VCH, Weinheim) or for other antibiotic resistance genes such as for G418, gentamycin, neomycin, hygromycin or tetracycline resistance.

[0439.0.17.17] Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255f) or electroporation (Delorme E., Appl. Environ. Microbiol., vol. 55, no. 9, 1989 : 2242 - 2246).

[0440.0.17.17] If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.17] see [0441.0.0.0]**

**[0442.0.0.17] see [0442.0.0.0]**

**[0443.0.0.17] see [0443.0.0.0]**

**[0444.0.17.17]** Example 6: Growth of genetically modified organism: media and culture conditions

[0445.0.17.17] Genetically modified Yeast, Mortierella or Escherichia coli are grown in synthetic or natural growth media known by the skilled worker. A number of different growth media for Yeast, Mortierella or Escherichia coli are well known and widely available. A method for culturing Mortierella is disclosed by Jang et al. [Bot. Bull. Acad. Sin. (2000) 41: 41-48]. Mortierella can be grown at 20°C in a culture medium containing: 10 g/l glucose, 5 g/l yeast extract at pH 6.5. Futhermore Jang et al. teaches a submerged basal medium containing 20 g/l soluble starch, 5 g/l Bacto yeast extract, 10 g/l KNO₃, 1 g/l KH₂PO₄, and 0.5 g/l MgSO₄**·**7H₂O, pH 6.5.

**[0446.0.0.17] to [0450.0.0.17]: see [0446.0.0.0] to [0450.0.0.0]**

**[0451.0.0.17] see [0451.0.5.5]**

**[0452.0.0.17] to [0453.0.0.17]: see [0452.0.0.0] to [0453.0.0.0]**

**[0454.0.17.17]** Analysis of the effect of the nucleic acid molecule on the production of gamma-aminobutyric acid or shikimate or putrescine

[0455.0.17.17] The effect of the genetic modification in plants, fungi, algae, ciliates or on the production of a desired compound (such as a gamma-aminobutyric acid) can be determined by growing the modified microorganisms or the modified plant under suitable conditions (such as those described above) and analyzing the medium and/or the cellular components for the elevated production of desired product (i.e. of gamma-aminobutyric acid or shikimate or putrescine). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).

**[0456.0.0.17]: see [0456.0.0.0]**

[0457.0.17.17] Example 9: Purification of gamma-aminobutyric acid

[0458.0.17.17] Abbreviations; GC-MS, gas liquid chromatography/mass spectrometry; TLC, thin-layer chromatography.
The unambiguous detection for the presence of gamma-aminobutyric acid or shikimate or putrescine can be obtained by analyzing recombinant organisms using analytical standard methods: LC, LC-MSMS or TLC, as described. The total amount produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.
A typical sample pretreatment consists of a total lipid extraction using such polar organic solvents as acetone or alcohols as methanol, or ethers, saponification, partition between phases, seperation of non-polar epiphase from more polar hypophasic derivatives and chromatography.
For analysis, solvent delivery and aliquot removal can be accomplished with a robotic system comprising a single injector valve Gilson 232XL and a 402 2S1V diluter [Gilson, Inc. USA, 3000 W. Beltline Highway, Middleton, WI]. For saponification, 3 ml of 50% potassium hydroxide hydro-ethanolic solution (4 water - 1 ethanol) can be added to each vial, followed by the addition of 3 ml of octanol. The saponification treatment can be conducted at room temperature with vials maintained on an IKA HS 501 horizontal shaker [Labworld-online, Inc., Wilmington, NC] for fifteen hours at 250 movements/minute, followed by a stationary phase of approximately one hour.
Following saponification, the supernatant can be diluted with 0.17 ml of methanol. The addition of methanol can be conducted under pressure to ensure sample homogeneity.
Using a 0.25 ml syringe, a 0.1 ml aliquot can be removed and transferred to HPLC vials for analysis.
For HPLC analysis, a Hewlett Packard 1100 HPLC, complete with a quaternary pump, vacuum degassing system, six-way injection valve, temperature regulated autosampler, column oven and Photodiode Array detector can be used [Agilent Technologies available through Ultra Scientific Inc., 250 Smith Street, North Kingstown, RI]. The column can be a Waters YMC30, 5-micron, 4.6 x 250 mm with a guard column of the same material [Waters, 34 Maple Street, Milford, MA]. The solvents for the mobile phase can be 81 methanol: 4 water: 15 tetrahydrofuran (THF) stabilized with 0.2% BHT (2,6-di-tert-butyl-4-methylphenol). Injections were 20 I. Separation can be isocratic at 30°C with a flow rate of 1.7 ml/minute. The peak responses can be measured by absorbance at 447 nm.

[0459.0.17.17] If required and desired, further chromatography steps with a suitable resin may follow. Advantageously, the gamma-aminobutyric acid can be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.0.17] see [0460.0.0.0]**

**[0461.0.17.17]** Example 10: Cloning SEQ ID NO: 1for the expression in plants

**[0462.0.0.17] see [0462.0.0.0]**

**[0463.0.17.17]** SEQ ID NO: 1 is amplified by PCR as described in the protocol of the Pfu Turbo or DNA Herculase polymerase (Stratagene).

**[0464.0.0.0.17] to [0466.0.0.17]: see [0464.0.0.0] to [0466.0.0.0]**

**[0466.1.0.17]** In case the Herculase enzyme can be used for the amplification, the PCR amplification cycles were as follows: 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.17.17]** The following primer sequences were selected for the gene SEQ ID NO: 1:
i) forward primer SEQ ID NO:3
ii) reverse primer SEQ ID NO: 4
The primer sequences were selected for the genes described in Table III, application no. 17, column 5, and as described in Table III, application no. 17, column 7.

**[0468.0.17.17] to [0470.0.17.17]: see [0468.0.0.0] to [0470.0.0.0]**

[0470.1.17.17] The PCR-products, produced by *Pfu* Turbo DNA polymerase, were phosphorylated using a T4 DNA polymerase using a standard protocol (e.g. MBI Fermentas) and cloned into the processed binary vector.

**[0471.0.17.17] see [0471.0.0.0]**

[0471.1.17.17] The DNA termini of the PCR-products, produced by Herculase DNA polymerase, were blunted in a second synthesis reaction using Pfu Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were phosphorylated using a T4 DNA polymerase using a standard protocol (e.g. MBI Fermentas) and cloned into the processed vector as well.

**[0472.0.17.17] to [0479.0.17.17]: see [0472.0.0.0] to [0479.0.0.0]**

[0480.0.17.17] Example 11: Generation of transgenic plants which express SEQ ID NO: 15720, 15762, 15708,15712, 91094, 91415, 91530 or 91635.

**[0481.0.0.17] to [0513.0.0.17]: see [0481.0.0.0] to [0513.0.0.0]**

[0514.0.17.17] As an alternative, gamma-aminobutyric acid can be detected as described in Haak and Reineke, Antimicrob. Agents Chemother. 19(3): 493(1981)
As an alternative, shikimate can be detected as described in Gould and Erickson, J Antibiot 41 (5), 688-9 (1988).
As an alternative, putrescine can be detected as described in Endo Y., Anal Biochem. 89(1):235-46(1978).

**[0515.0.0.17] to [0552.0.0.17]: see [0515.0.0.0] to [0552.0.0.0]**

[0553.0.17.17] A process for the production of gamma-aminobutyric acid, which comprises
a)increasing or generating the activity of a protein as indicated in Table II, application no. 17, columns 5 or 7, or a functional equivalent thereof in a non-human organism or in one or more parts thereof; and
b)growing the organism under conditions which permit the production of gamma-aminobutyric acid in said organism.
A process for the production of gamma-aminobutyric acid or shikimate or putrescine, comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 17, columns 5 or 7 or a fragment thereof, which confers an increase in the amount of gamma-aminobutyric acid in an organism or a part thereof;
b) nucleic acid molecule comprising of a nucleic acid molecule as indicated in Table I, application no. 17, columns 5 or 7,;
c) nucleic acid molecule whose sequence can be deduced from a polypeptide sequence encoded by a nucleic acid molecule of (a) or (b) as a result of the degeneracy of the genetic code and conferring an increase in the amount of gamma-aminobutyric acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of gamma-aminobutyric acid in an organism or a part thereof;
e) nucleic acid molecule which hybidizes with a nucleic acid molecule of (a) to (c) under under stringent hybridisation conditions and conferring an increase in the amount of gamma-aminobutyric acid in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 17, columns 5 or 7, and conferring an increase in the amount of gamma-aminobutyric acid in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of gamma-aminobutyric acid in an organism or a part thereof;
h) nucleic acid molecule encoding a polypeptide comprising a consensus as indicated in Table IV, application no. 17, column 7, and conferring an increase in the amount of gamma-aminobutyric acid in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of gamma-aminobutyric acid in an organism or a part thereof.
or comprising a sequence which is complementary thereto.
The process as mentioned above, comprising recovering of the free or bound gamma-aminobutyric acid.
The process as mentioned above, comprising the following steps:
a) selecting an organism or a part thereof expressing a polypeptide encoded by the nucleic acid molecule characterized as mentioned above;
b) mutagenizing the selected organism or the part thereof;
c) comparing the activity or the expression level of said polypeptide in the mutagenized organism or the part thereof with the activity or the expression of said polypeptide of the selected organisms or the part thereof;
d) selecting the mutated organisms or parts thereof, which comprise an increased activity or expression level of said polypeptide compared to the selected organism or the part thereof;
e) optionally, growing and cultivating the organisms or the parts thereof; and
f) recovering, and optionally isolating, the free or bound gamma-aminobutyric acid produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 17, columns 5 or 7 or a fragment thereof, which confers an increase in the amount of gamma-aminobutyric acid in an organism or a part thereof;
b) nucleic acid molecule comprising of a nucleic acid molecule as indicated in Table I, application no. 17, columns 5 or 7,;
c) nucleic acid molecule whose sequence can be deduced from a polypeptide sequence encoded by a nucleic acid molecule of (a) or (b) as a result of the degeneracy of the genetic code and conferring an increase in the amount of gamma-aminobutyric acid in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of gamma-aminobutyric acid in an organism or a part thereof;
e) nucleic acid molecule which hybidizes with a nucleic acid molecule of (a) to (c) under stringent hybridisation conditions and conferring an increase in the amount of gamma-aminobutyric acid in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 17, column 7, and conferring an increase in the amount of gamma-aminobutyric acid in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of gamma-aminobutyric acid in an organism or a part thereof;
h) nucleic acid molecule encoding a polypeptide comprising a consensus as indicated in Table IV, application no. 17, column 7, and conferring an increase in the amount of gamma-aminobutyric acid in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of gamma-aminobutyric acid in an organism or a part thereof.
whereby the nucleic acid molecule distinguishes over the sequence as indicated in Table I, application no. 17, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 17, columns 5 or 7, by one or more amino acids.
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of gamma-aminobutyric acid in an organism or a part thereof comprising:
(a) contacting cells, tissues, plants or microorganisms which express the a polypeptide encoded by the nucleic acid molecule as mentioned above conferring an increase in the amount of gamma-aminobutyric acid in an organism or a part thereof with a candidate compound or a sample comprising a plurality of compounds under conditions which permit the expression the polypeptide;
(b) assaying the gamma-aminobutyric acid or shikimate or putrescine level or the polypeptide expression level in the cell, tissue, plant or microorganism or the media the cell, tissue, plant or microorganisms is cultured or maintained in; and
(c) identifying a agonist or antagonist by comparing the measured gamma-aminobutyric acid or shikimate or putrescine level or polypeptide expression level with a standard gamma-aminobutyric acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased gamma - aminobutyric acid or production in a plant or microorganism, comprising the steps:
(a) culturing a plant cell or tissue or microorganism or maintaining a plant expressing the polypeptide encoded by the nucleic acid molecule as mentioned above conferring an increase in the amount of gamma-aminobutyric acid in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of gamma-aminobutyric acid in an organism or a part thereof;
(b) identifying if the compound is an effective agonist by detecting the presence or absence or increase of a signal produced by said readout system.
A method for the identification of a gene product conferring an increase in gamma-aminobutyric acid production in a cell, comprising the following steps:
(a) contacting the nucleic acid molecules of a sample, which can contain a candidate gene encoding a gene product conferring an increase in gamma-aminobutyric acid after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing gamma-aminobutyric acid;
(d) expressing the identified nucleic acid molecules in the host cells;
(e) assaying the gamma-aminobutyric acid level in the host cells; and
(f) identifying nucleic acid molecule and its gene product which expression confers an increase in the gamma-aminobutyric acid level in the host cell in the host cell after expression compared to the wild type.
A method for the identification of a gene product conferring an increase in gamma-aminobutyric acid production in a cell, comprising the following steps:
(a) identifiying in a data bank nucleic acid molecules of an organism; which can contain a candidate gene encoding a gene product conferring an increase in the gamma-aminobutyric acid amount or level in an organism or a part thereof after expression, and which are at least 20% homolog to the nucleic acid molecule as mentioned above;
(b) introducing the candidate nucleic acid molecules in host cells appropriate for producing gamma-aminobutyric acid;
(c) expressing the identified nucleic acid molecules in the host cells;
(d) assaying the gamma-aminobutyric acid level in the host cells; and
(e) identifying nucleic acid molecule and its gene product which expression confers an increase in the gamma-aminobutyric acid level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of gamma-aminobutyric acid after expression.
Use of the polypeptide as mentioned above or the nucleic acid construct as mentioned above or the gene product identified according to the method as mentioned above for identifying compounds capable of conferring a modulation of gamma-aminobutyric acid levels in an organism.
Agrochemical, pharmaceutical, food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
The method as mentioned above, the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above, wherein the fine chemical is gamma-aminobutyric acid.

### New genes for a process for the production of fine chemicals

### Description

[0000.0.0.18] In a further embodiment, the present invention relates to a further process for the production of fine chemicals as defined below and the corresponding embodiments as described herein as follows.

[0001.0.18.18] The present invention relates to a process for the production of the fine chemical in a microorganism, a plant cell, a plant, a plant tissue or in one or more parts thereof. The invention furthermore relates to nucleic acid molecules, polypeptides, nucleic acid constructs, vectors, antisense molecules, antibodies, host cells, plant tissue, propagation material, harvested material, plants, microorganisms as well as agricultural compositions and to their use.

**[0002.0.18.18]** Coenzymes are molecules that cooperate in the catalytic action of an enzyme. Like enzymes, coenzymes are not irreversibly changed during catalysis; they are either unmodified or regenerated. Each kind of coenzyme has a particular chemical function. Coenzymes may either be attached by covalent bonds to a particular enzyme or exist freely in solution, but in either case they participate intimately in the chemical reactions catalyzed by the enzyme.

**[0003.0.18.18]** Coenzyme Q10 (CoQ 10) or ubiquinone is essentially a vitamin or vitamin-like substance. Disagreements on nomenclature notwithstanding, vitamins are defined as organic compounds essential in minute amounts for normal body function acting as coenzymes or precursors to coenzymes. Coenzyme Q10 or CoQ10 belongs to a family of substances called ubiquinones. Ubiquinones, also known as coenzymes Q and mitoquinones, are lipophilic, water-insoluble substances involved in electron transport and energy production in mitochondria. The basic structure of ubiquinones consists of a benzoquinone "head" and a terpinoid "tail." The "head" structure participates in the redox activity of the electron transport chain. The major difference among the various coenzymes Q is in the number of isoprenoid units (5-carbon structures) in the "tail." Coenzymes Q contain one to 12 isoprenoid units in the "tail"; 10 isoprenoid units are common in animals. Coenzymes Q occur in the majority of aerobic organisms, from bacteria to plants and animals. Two numbering systems exist for designation of the number of isoprenoid units in the terpinoid "tail": coenzyme Qn and coenzyme Q(x). N refers to the number of isoprenoid side chains, and x refers to the number of carbons in the terpinoid "tail" and can be any multiple of five. Thus, coenzyme Q10 refers to a coenzyme Q having 10 isoprenoid units in the "tail." Since each isoprenoid unit has five carbons, coenzyme Q10 can also be designated coenzyme Q(50). The structures of coenzymes Q are analogous to those of vitamin K2. Coenzyme Q10 is also known as Coenzyme Q(5O), CoQ10, CoQ(50), ubiquinone (50), ubiquinol- 10 and ubidecarerone.
They are present naturally in foods and sometimes are also synthesized in the body. CoQ10 likewise is found in small amounts in a wide variety of foods and is synthesized in all tissues. The biosynthesis of CoQ10 from the amino acid tyrosine is a multistage process requiring at least eight vitamins and several trace elements. Coenzymes are cofactors upon which the comparatively large and complex enzymes absolutely depend for their function. Coenzyme Q10 is the coenzyme for at least three mitochondrial enzymes (complexes I, II and III) as well as enzymes in other parts of the cell. Mitochondrial enzymes of the oxidative phosphorylation pathway are essential for the production of the high-energy phosphate, adenosine triphosphate (ATP), upon which all cellular functions depend. The electron and proton transfer functions of the quinone ring are of fundamental importance to all life forms; ubiquinone in the mitochondria of animals, plastoquinone in the chloroplast of plants, and menaquinone in bacteria. The term "bioenergetics" has been used to describe the field of biochemistry looking specifically at cellular energy production. In the related field of free radical chemistry, CoQ10 has been studied in its reduced form as a potent antioxidant. The bioenergetics and free radical chemistry of CoQ10 are reviewed in Gian Paolo Littarru's book, Energy and Defense, published in 1994. The precise chemical structure of CoQ10 is 2,3 dimethoxy-5 methyl-6 decaprenyl benzoquinone

**[0004.0.18.18]** Discovered in 1957, CoQ-10 is also called ubiquinone because it belongs to a class of compounds called quinones, and because it's ubiquitous in living organisms, especially in the heart, liver, and kidneys. It plays a crucial role in producing energy in cells. And it acts as a powerful antioxidant, meaning that it helps neutralize cell-damaging molecules called free radicals. Manufactured by all cells in the body, CoQ-10 is also found in small amounts in foods, notably meat and fish. By the mid-1970's, the industrial technology to produce pure CoQ10 in quantities sufficient for larger clinical trials was established. Principally CoQ10 can be isolated from microorganisms or plants or algae; in particular mitochondria are a common source for CoQ10. Alternatively, they are obtained advantageously from animals or fish. **[0005.0.18.18]** Since the actions of supplemental CoQ10 have yet to be clarified, the mechanism of these actions is a matter of speculation. However, much is known about the biochemistry of CoQ10. CoQ10 is an essential cofactor in the mitochondrial electron transport chain, where it accepts electrons from complex I and II, an activity that is vital for the production of ATP. CoQ10 has antioxidant activity in mitochondria and cellular membranes, protecting against peroxidation of lipid membranes. It also inhibits the oxidation of LDL-cholesterol. LDL-cholesterol oxidation is believed to play a significant role in the pathogenesis of atherosclerosis. CoQ10 is biosynthesized in the body and shares a common synthetic pathway with cholesterol.
CoQ10 levels decrease with aging in humans. Why this occurs is not known but may be due to decreased synthesis and/or increased lipid peroxidation which occurs with aging. Significantly decreased levels of CoQ10 have been noted in a wide variety of diseases in both animal and human studies. CoQ10 deficiency may be caused by insufficient dietary CoQ10, impairment in CoQ10 biosynthesis, excessive utilization of CoQ10 by the body, or any combination of the three. Decreased dietary intake is presumed in chronic malnutrition and cachexia.

[0006.0.18.18] The relative contribution of CoQ10 biosynthesis versus dietary CoQ10 is under investigation. Karl Folkers takes the position that the dominant source of CoQ10 in man is biosynthesis. This complex, 17 step process, requiring at least seven vitamins (vitamin B2 - riboflavin, vitamin B3 - niacinamide, vitamin B6, folic acid, vitamin B12, vitamin C, and pantothenic acid) and several trace elements, is, by its nature, highly vulnerable. Karl Folkers argues that suboptimal nutrient intake in man is almost universal and that there is subsequent secondary impairment in CoQ10 biosynthesis. This would mean that average or "normal" levels of CoQ10 are really suboptimal and the very low levels observed in advanced disease states represent only the tip of a deficiency "ice berg".
Supplemental CoQ10 may have cardioprotective, cytoprotective and neuroprotective activities. There are claims that it has positive effects in cancer, muscular dystrophy and immune dysfunction. Similarly, it may inhibit obesity or enhance athletic performance.

**[0007.0.18.18]** HMG-CoA reductase inhibitors used to treat elevated blood cholesterol levels by blocking cholesterol biosynthesis also block CoQ10 biosynthesis. The resulting lowering of blood CoQ10 level is due to the partially shared biosynthetic pathway of CoQ10 and cholesterol. In patients with heart failure this is more than a laboratory observation. It has a significant harmful effect which can be negated by oral CoQ10 supplementation.
Increased body consumption of CoQ10 is the presumed cause of low blood CoQ10 levels seen in excessive exertion, hypermetabolism, and acute shock states. It is likely that all three mechanisms (insufficient dietary CoQ10, impaired CoQ10 biosynthesis, and excessive utilization of CoQ10) are operable to varying degrees in most cases of observed CoQ10 deficiency.

**[0008.0.18.18]** In nature, Coenzymes Q0 to Q9 are found as well. E.g. Coenzyme Q9 is a derivative of CoQ10 found e.g. in the chloroplast of plants. Coenzyme Q9 has a shorter aliphatic group bound to the ring structure. Due to the high structural homology of Coenzymes Q0 to Q9 are expected to provide the same or very similar activities as CoQ10 in cells or organisms. However, Matsura et al., Biochim Biophys Acta, 1992, 1123(3) pp. 309-15 concluded from their study that CoQ9 constantly acts as a potential antioxidant in hepatocytes whereas CoQ10 manly exhibit its antioxidant activity in cells containing CoQ10 as the predominate CoQ homolog. Coenzyme Q10 is actual a very common ingredient in different types of cosmetics, due to its protective role against radicals and its predicted function in skin tautening.

**[0009.0.18.18]** Thus, Coenzymes, in particular CoQ10 or CoQ9 can be used in a lot of different applications, for example in cosmetics, pharmaceuticals and in feed and food.

[0010.0.18.18] Therefore improving the productivity of said Coenzymes and improving the quality of cosmetics, pharmaceuticals, foodstuffs and animal feeds, in particular of nutrition supplements, is an important task of the different industries.

[0011.0.18.18] To ensure a high productivity of said Coenzymes in plants or microorganism, it is necessary to manipulate the natural biosynthesis of said Coenzymes in said organisms.

**[0012.0.18.18]** Accordingly, there is still a great demand for new and more suitable genes which encode enzymes or other regulators which participate in the biosynthesis of said Coenzymes and make it possible to produce certain said Coenzymes specifically on an industrial scale without unwanted byproducts forming. In the selection of genes for biosynthesis two characteristics above all are particularly important. On the one hand, there is as ever a need for improved processes for obtaining the highest possible contents of said Coenzymes on the other hand as less as possible byproducts should be produced in the production process.

[0013.0.0.18] It was now found that this object is achieved by providing the process according to the invention described herein and the embodiments characterized in the claims.

[0014.0.18.18] It was found that the overexpression of the nucleic acid molecules characterized herein confers an increase in the content of Coenzyme Q10 or Coenzyme Q9 in plants. Accordingly, in a first embodiment, the invention relates to a process for the production of Coenyzme Q10 and/or Coenyzme Q9. Accordingly, in a further embodiment, the invention relates to a process for the production of a fine chemical, whereby the fine chemical is Coenzyme Q10 and/or Coenzyme Q9. Accordingly, in the present invention, the term "the fine chemical" as used herein relates to "Coenzyme Q10 and/or Coenzyme Q9". Further, in another embodiment the term "the fine chemicals" as used herein also relates to compositions of the fine chemicals comprising Coenzyme Q10 and/or Coenzyme Q9.

[0015.0.18.18] In one embodiment, the term "the respective fine chemicals" means Coenzyme Q10 and/or Coenzyme Q9, depending on the context. For example, the increase of the gene product of a gene with activity of the Gene/ORF Locus name or its homologs as mentioned confers the increase of the level of coenzymes CoQ9 in plants or parts thereof. For example, the increase of the gene product of a gene with the ORF name b2426 from E. coli or a plant or its homologs confer the increase of the level of coenzymes CoQ9 and CoQ10 in plants or parts thereof.
Accordingly, in one embodiment, the term "the respective fine chemicals" means Coenzyme Q10 and Coenzyme Q9. In one embodiment, the term "the respective fine chemical" means Coenzyme Q10 or Coenzyme Q9. In a further embodiment, the term "the respective fine chemical" means Coenzyme Q10 and/or Coenzyme Q9 and their salts, ester, or thioester or Coenzyme Q10 and/or Coenzyme Q9 in free form or bound to protein(s), e.g. enzyme(s), or peptide(s), e.g. polypeptide(s) or to membranes or parts thereof, e.g. in the form of oils or waxes or in compositions with lipids, oils, fats or lipid mixture, as well as Coenzyme Q10 and/or Coenzyme Q9 in its reduced or oxidized form. In a preferred embodiment, the term "the respective fine chemical" means Coenzyme Q10 or CoQ9, in free form or its salts or bound to peptide(s) or protein(s). Lipids, oils, waxes, fats or lipid mixture shall mean any, lipid, oil, wax and/or fat containing any bound or free Coenzyme Q10 and/or Coenzyme Q9.
In one embodiment, the term "the fine chemical" and the term "the respective fine chemical" mean at least one chemical compound with an activity of the above mentioned fine chemical.

**[0016.0.18.18]**Accordingly, the present invention relates to a process for the production of the respective fine chemical comprising
(a) increasing or generating the activity of one or more
   of a protein as shown in table II, application no. 18, column 3 encoded by the nucleic acid sequences as shown in table I, application no. 18, column 5, in a non-human organism or in one or more parts thereof or
(b) growing the organism under conditions which permit the production of the fine chemical, thus Coenzyme of the invention or fine chemicals comprising coenzyme of the invention, in said organism or in the culture medium surrounding the organism

[0016.1.0.18] In one embodiment, the method of the present invention confers the increase of the content of more than one of the respective fine chemicals, i.e. of Coenzyme Q9 and/or Coenzyme Q10.
Accordingly, the term "the fine chemical" can mean "Coenzyme Q9", and/or "Coenzyme Q10", owing to circumstances and the context. In order to illustrate that the meaning of the term "the fine chemical" means "Coenyzme Q9" and/or "Coenzyme Q10" the term "the respective fine chemical" is also used.

**[0017.0.0.18]** to **[0019.0.0.18]** see **[0017.0.0.0] to [0019.0.0.0]**

**[0019.0.18.18]**

[0020.0.18.18] Surprisingly it was found, that the transgenic expression of the Brassica napus protein as indicated in Table II, application no. 18, column 5, line 40 in a plant conferred an increase in Coenzyme Q9 content of the transformed plants. Thus, in one embodiment, said protein or its homologs are used for the production of Coenzyme Q9.
Surprisingly it was found, that the transgenic expression of the Brassica napus protein as indicated in Table II, application no. 18, column 5, line 41 in a plant conferred an increase in Coenzyme Q9 content of the transformed plants. Thus, in one embodiment, said protein or its homologs are used for the production of Coenzyme Q9.

**[0021.0.0.18]** see **[0021.0.0.0]**

[0022.0.18.18]The sequence of b2426 (Accession number NP 416921) from Escherichia coli K12 has been published in Blattner et al., Science 277 (5331), 1453-1474, 1997, and its activity is being defined as a putative oxidoreductase with NAD(P)-binding domain. Accordingly, in one embodiment, the process of the present invention comprises the use of a gene product with an activity of the "ribitol dehydrogenase, short-chain alcohol dehydrogenase homology" superfamily, preferably being involved in C-compound and carbohydrate utilization, metabolism of vitamins, cofactors and prosthetic groups, biosynthesis of secondary products derived from primary amino acids, aerobic aromate catabolism, anaerobic aromate catabolism, and/or catabolism of secondary metabolites, biosynthesis of the cysteine-aromatic group and/or degradation of amino acids of the cysteine-aromatic group, aromate anabolism, more preferred a protein with the activity of a putative oxidoreductase with NAD(P)-binding domain from E. coli or a plant or its homolog, e.g. as shown herein in Table II, column 5 or 7, line 40 or as encoded by the nucleic acid molecule shown in Table I, column 5 or 7, line 40, for the production of the respective fine chemical, meaning of Coenzyme Q9, in particular for increasing the amount of Coenzyme Q9, preferably Coenzyme Q9 in free or bound form in an organism or a part thereof, as mentioned.
The sequence of YDR513W (ACCESSION NP_010801)from Saccharomyces cerevisiae has been published in Jacq et al., Nature 387 (6632 Suppl), 75-78 (1997) and in Goffeau et al., Science 274 (5287), 546-547, 1996 and its cellular activity has characterized as glutathione reductase. Accordingly, in one embodiment, the process of the present invention comprises the use of glutathione reductase or a protein of the glutaredoxin superfamily, in particular of a protein having a deoxyribonucleotide metabolism, cytoplasm, stress response, detoxification, and/or electron transport and membrane-associated energy conservation activity for the production of Coenzyme Q9. Accordingly, in one embodiment, the process of the present invention comprises the use of protein with tha activity of YDR513W from Saccharomyces cerevisiaeor a plant, e.g. as indicated herein in Table II, line 41, columns 5, or its homologue, e.g. as shown herein in Table II, line 41, column 7, for the production of the respective fine chemical, meaning of Coenzyme Q9, in particular for increasing the amount of reduced and/or oxidized Coenzyme Q9 and/or Coenzyme Q9 in free or bound form in an organism or a part thereof, as mentioned. In one embodiment, in the process of the present invention the activity of a glutathione reductase is increased or generated, e.g. from Saccharomyces cerevisiae or a plant or a homolog thereof.

**[0022.1.0.18] to [0023.0.18.18] see [0022.1.0.0] to [0023.0.0.0]**

**[0023.1.18.18]** Homologs of the polypeptide disclosed in table II, application no. 18, column 5, may have the activity of a protein as disclosed in table I or II, application no. 18, column 3 and may be the polypetides encoded by the nucleic acid molecules indicated in table I , application no. 18, column 7, resp., or may be the polypeptides indicated in table II, application no. 18, column 7, resp.

**[0024.0.0.18]** see **[0024.0.0.0]**

**[0025.0.18.18]** In accordance with the invention, a protein or polypeptide has the "activity of an protein as shown in table II, application no. 18, column 3 "if its de *novo* activity, or its increased expression directly or indirectly leads to an increased in the level of the fine chemical indicated in the respective line of table II, application no. 18, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 18, column 3, or which has at least 10% of the original enzymatic or biological activity, preferably 20%, particularly preferably 30%, most particularly preferably 40% in comparison to a protein as shown in the respective line of table II, application no. 18, column 3 of E. coli or Saccharomyces cerevisae, respectively, protein as mentioned in table I to IV, column 3 respectively and as disclosed in paragraph [0022] of the respective invention.

[0025.3.18.18] In one embodiment, the polypeptide of the invention or used in the method of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0025.2.0.18] see [0025.2.0.0]**

**[0025.1.0.18]** see **[0025.1.0.0]**

**[0026.0.0.18] to [0033.0.0.18] see[0026.0.0.0] to [0033.0.0.0]**

[0034.0.18.18] Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 18, column 3 its biochemical or genetical causes and the increased amount of the respective fine chemical.

**[0035.0.0.18]** to **[0044.0.0.18] see [0035.0.0.0]** to **[0044.0.0.0]**

[0045.0.18.18] In one embodiment, the activity of the protein. as indicated in Table II, columns 5 or 7, application no. 18, is increased conferring an increase of the respective fine chemical, indicated in Table II, application no. 18, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more

**[0046.0.18.18]In** one embodiment, the activity of the protein as indicated in Table II, columns 5 or 7, application no. 18, is increased conferring an increase of the respective fine chemical, indicated in Table II, application no. 18, column 6 of the respective line confers an increase of the respective fine chemical and of further Coenzyme or their precursors.

**[0047.0.0.18] to [0048.0.0.18] see [0047.0.0.0]to [0048.0.0.0]**

[0049.0.18.18]A protein having an activity conferring an increase in the amount or level of the fine chemical, has in one embodiment the structure of the polypeptide described herein, in particular of the polypeptides comprising the consensus sequence shown in table IV, application no. 18, column 7 or of the polypeptide as shown in the amino acid sequences as disclosed in table II, application no. 18, columns 5 and 7 or the functional homologues thereof as described herein, or is encoded by the nucleic acid molecule characterized herein or the nucleic acid molecule according to the invention, for example by the nucleic acid molecule as shown in table I, application no. 18, columns 5 and 7 or its herein described functional homologues and has the herein mentioned activity.

**[0050.0.18.18] ./.**

[0051.0.18.18] Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g. hydrophobic or lipophilic compositions. Depending on the choice of the organism used for the process according to the present invention, for example a microorganism or a plant, compositions or mixtures of the respective fine chemical and various other coenzymes, vitamins and/or antioxidants, e.g. vitamin B6 or vitamin E, can be produced.

**[0052.0.0.18] see [0052.0.0.0]**

[0053.0.18.18] In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 18, columns 5 and 7 or its homologs activity having herein-mentioned Coenzyme of the invention increasing activity; and/or
b) stabilizing a mRNA conferring the increased expression of a protein encoded by the nucleic acid molecule of the invention as shown in table I, application no. 18, columns 5 and 7, e.g. a nucleic acid sequence encoding a polypeptide having the activity of a protein as indicated in table II, application no. 18, columns 5 and 7 or its homologs activity or of a mRNA encoding the polypeptide of the present invention having herein-mentioned Coenzyme of the invention increasing activity; and/or
c) increasing the specific activity of a protein conferring the increased expression of a protein encoded by the nucleic acid molecule of the invention or of the polypeptide of the present invention having herein-mentioned Coenzyme increasing activity, e.g. of a polypeptide having the activity of a protein as indicated in table II, application no. 18, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned Coenzyme of the invention increasing activity, e.g. of a polypeptide having the activity of a protein as indicated in table II, application no. 18, columns 5 and 7 or its homologs activity; and/or
e) stimulating activity of a protein conferring the increased expression of a protein encoded by the nucleic acid molecule of the present invention or a polypeptide of the present invention having herein-mentioned Coenzyme of the invention increasing activity, e.g. of a polypeptide having the activity of a protein as indicated in table II, application no. 18, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned Coenzyme of the invention increasing activity, e.g. of a polypeptide having the activity of a protein as indicated in table II, application no. 18, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned Coenzyme of the invention increasing activity, e.g. of a polypeptide having the activity of a protein as indicated in table II, application no. 18, columns 5 and 7 or its homologs activity; and/or
h) increasing the expression of the endogenous gene encoding the polypeptide of the invention, e.g. a polypeptide having the activity of a protein as indicated in table II, application no. 18, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with expecially high activity of the proteins of the invention from natural or from mutagenized resources and breeding them into the target organisms, eg the elite crops.

**[0054.0.18.18]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of Coenzyme Q10 and/or Coenzyme Q9 after increasing the expression or activity of the encoded polypeptide or having the activity of a polypeptide having an, e.g. of a protein as indicated in Table II, application no. 18, column 5, encoded by a nucleic acid molecule indicated in Table I, application no. 18, column 5, or of their homologs, e.g. as indicated in Table I or II, application no. 18, column 7.

**[0055.0.0.18] to [0067.0.0.18] see [0055.0.0.0] to [0067.0.0.0]**

[0068.0.9.9] The mutation is introduced in such a way that the production of the Coenzyme Q9 and/or Coenzyme Q10 is not adversely affected.

**[0069.0.0.18] see [0069.0.0.0]**

[0070.0.18.18] Owing to the introduction of a gene or a plurality of genes conferring the expression of the nucleic acid molecule of the invention or used in the method of the invention or the polypeptide of the invention or used in the method of the invention, for example the nucleic acid construct mentioned below, or encoding the protein as indicated in Table II, application no. 18, column 5, or being encoded by a nucleic acid molecule indicated in Table I, application no. 18, column 5, or of its homologs, e.g. as indicated in Table II, application no. 18, column 7, into an organism alone or in combination with other genes, it is possible not only to increase the biosynthetic flux towards the end product, but also to increase, modify or create de *novo* an advantageous, preferably novel metabolites composition in the organism, e.g. an advantageous hydrophopic composition comprising a higher content of (from a viewpoint of nutrional physiology limited) coenzymes, vitamins and/or antioxidants, e.g. vitamin B6 and/or vitamin E. In one embodiment, the expression of a protein as indicated in Table II, application no. 18, column 5, or being encoded by a nucleic acid molecule indicated in Table I, application no. 18, column 5, or of its homologs, e.g. as indicated in Table II, application no. 18, column 7, conferring an increase of coenzymes, in particular of Coenzyme Q9 and/or Coenzyme Q10, and preferably of further coenzymes, vitamins, and/or antioxidants.

[0071.0.18.18] Preferably the composition further comprises higher amounts of metabolites positively affecting or lower amounts of metabolites negatively affecting the nutrition or health of animals or humans provided with said compositions or organisms of the invention or parts thereof. Likewise, the number or activity of further genes which are required for the import or export of nutrients or metabolites, including amino acids, fatty acids, vitamins, coenzymes, antioxidants etc. or any one of their precursors, required for the cell's biosynthesis of the respective fine chemical may be increased so that the concentration of necessary or relevant precursors, e.g. of isoprenoids, acetyl CoA, HMG-CoA, mevalonate, Isopentenyl pyrophosphate, Geranyl pyrophosphate, Farnesyl Pyrophosphate, or other cofactors or intermediates within the organelle, e.g. in mitochondria or plastids, resp., within (a) cell(s) or within the corresponding storage compartments is increased. Owing to the increased or novel generated activity of the polypeptide of the invention or used in the method of the invention or owing to the increased number of nucleic acid sequences of the invention or used in the method of the invention and/or to the modulation of further genes which are involved in the biosynthesis of the respective fine chemical, e.g. by increasing the activity of enzymes synthesizing precursors, e.g. Lovastatin, HMG-CoA Reductase, Mevalonate Kinase, or by destroying the activity of one or more genes which are involved in the breakdown of the respective fine chemical, it is possible to increase the yield, production and/or production efficiency of the respective fine chemical in the host organism, such as plants or the microorganisms.

[0072.0.18.18] By influencing the metabolism thus, it is possible to produce, in the process according to the invention, further advantageous compounds. Examples of such compounds are antioxidants, further coenzymes, vitamins, e.g. vitamin B6 or vitamin E, or triglycerides, fatty acids, lipids, oils and/or fats containing Coenzyme Q10 and/or Coenzyme Q9.

[0073.0.18.18] Accordingly, in one embodiment, the process according to the invention relates to a process, which comprises:
a) providing a non-human organism, preferably a microorganism, a non-human animal, a plant or animal cell, an organelle, a plant or animal tissue or a plant;
b) increasing the protein activity or of a polypeptide being encoded by the nucleic acid molecule of the present invention and described below, e.g. of a protein as indicated in Table II, application no. 18, column 5 or being encoded by a nucleic acid molecule indicated in Table I, application no. 18, column 5 or of its homologs, e.g. as indicated in Table II, application no. 18, column 7, and conferring an increase of the respective fine chemical in the organism, preferably in the microorganism, the non-human animal, the plant or animal cell, the plant or animal tissue, the organelle or the plant,
c) growing the organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
d) if desired, recovering, optionally isolating, the free and/or bound the respective fine chemical and, optionally further free and/or bound vitamins, coenzymes or antioxidants synthesized by the organism, the microorganism, the non-human animal, the plant or animal cell, the plant or animal tissue or the plant.

[0074.0.18.18] The organism, in particular the microorganism, the non-human animal, the plant or animal cell, the plant or animal tissue, the organelle or the plant is advantageously grown in such a way that it is not only possible to recover, if desired isolate the free or bound the respective fine chemical or the free and bound the respective fine chemical but as option it is also possible to produce, recover and, if desired isolate, other free or/and bound antioxidants, vitamins or coenzymes and mixtures thereof.

**[0075.0.0.18] to [0077.0.0.18] see [0075.0.0.0] to [0077.0.0.0]**

[0078.0.18.18] The organism such as microorganisms or plants or the recovered, and if desired isolated, fine chemical can then be processed further directly into foodstuffs or animal feeds or for other applications, for example according to the disclosures made in the following US patent publications: 6,380,252: Use of L-acetylcarnitine, L-isovalerylcarnitine, L-propionylcarnitine for increasing the levels of IGF-1, 6,372,198: Dentifrice for the mineralization and remineralization of teeth, 6,368,617: Dietary supplement, 6,350,473: Method for treating hypercholesterolemia, hyperlipidemia, and atherosclerosis , 6,335,361: Method of treating benign forgetfulness, 6,329,432: Mesozeaxanthin formulations for treatment of retinal disorders, 6,328,987: Cosmetic skin care compositions containing alpha interferon, 6,312,703: Compressed lecithin preparations, 6,306,392: Composition comprising a carnitine and glutathione, useful to increase the absorption of glutathione and synergize its effects, 6,303,586: Supportive therapy for diabetes, hyperglycemia and hypoglycemia, 6,297,281: Association of no syntase inhibitors with trappers of oxygen reactive forms, 6,294,697: Discrete-length polyethylene glycols, 6,277,842: Dietary supplemental method for fat and weight reduction, 6,261,250: Method and apparatus for enhancing cardiovascular activity and health through rhythmic limb elevation, 6,258,855: Method of retarding and ameliorating carpal tunnel syndrome, 6,258,848: Methods and compositions for increasing insulin sensitivity, 6,258,847: Use of 2-mercaptoethanolamine (2-MEA) and related aminothiol compounds and copper(II)-3,5 di-isopropyl salicylates and related compounds in the prevention and treatment of various diseases, 6,255,354: Preparation of a pulmonary surfactant for instillation and oral application, 6,254,547: Breath methylated alkane contour: a new marker of oxidative stress and disease, 6,248,552: Enzyme-based assay for determining effects of exogenous and endogenous factors on cellular energy production, 6,248,363: Solid carriers for improved delivery of active ingredients in pharmaceutical compositions, 6,245,800: Method of preventing or treating statin-induced toxic effects using L-carnitine or an alkanoyl L-carnitine, 6,245,378: Nutritional supplement for facilitating skeletal muscle adaptation to strenuous exercise and counteracting defatigation in asthenic individuals, 6,242,491: Use of creatine or creatine compounds for skin preservation, 6,232,346: Composition for improvement of cellular nutrition and mitochondrial energetics, 6,231,836: Folic acid dentifrice, 6,228,891: Use of 2,3-dimethoxy-5-methyl-6-decaprenyl-1,4-benzoquinone, 6,228,402: Xylitol-containing non-human foodstuff and method, 6,228,347: Antioxidant gel for gingival conditions, 6,218,436: Pharmaceutically active carotenoids, 6,203,818: Nutritional supplement for cardiovascular health, 6,200,550: Oral care compositions comprising coenzyme Q10, 6,191,172: Water-soluble compositions of bioactive lipophilic compounds , 6,184,255: Pharmaceutical composition comprising coenzyme Q10, 6,166,077: Use of L-acetylcarnitine, L-isovalerylcarnitine, L-propionylcarnitine for increasing the levels of IGF-1, 6,162,419: Stabilized ascorbyl compositions, 6,159,508: Xylitol-containing non-human foodstuff and method, 6,159,476: Herbal supplement for increased muscle strength and endurance for athletes, 6,153,582: Defined serumfree medical solution for ophthalmology, 6,136,859: Pharmaceutical formulation for treating liver disorders, 6,107,281: Compounds and their combinations for the treatment of influenza infection, 6,106,286: Method and device for administering medicine to the periodontium, 6,099,854: Dry composition containing flavonol useful as a food supplement, 6,086,910: Food supplements, 6,080,788: Composition for improvement of cellular nutrition and mitochondrial energetics, 6,069,167: Use of antioxidant agents to treat cholestatic liver disease, 6,063,820: Medical food for diabetics, 6,054,261: Coenzyme Q.sub.10 compositions for organ protection during perfusion, 6,051,250: Process for the stabilization of vesicles of amphiphilic lipid(s) and composition for topical application containing the said stabilized vesicles,
The fermentation broth, fermentation products, plants or plant products can be purified in the customary manner by hydrolysis with strong bases, extraction and crystallization or via thin layer chromatography and other methods known to the person skilled in the art and described herein below. Products of these different work-up procedures are fatty acids or fatty acid compositions which still comprise fermentation broth, plant particles and cell components in different amounts, advantageously in the range of from 0 to 99% by weight, preferably below 80% by weight, especially preferably between below 50% by weight.

**[0079.0.0.18] to [0084.0.0.18] see [0079.0.0.0] to [0084.0.0.0]**

[0084.2.18.18] Coenzyme Q10 production was reported in Agrobacterium sp., Protaminobacter rubber and Paracoccus denitrificans. Coenzyme Q9 production was reported in Candida tropicalis. Production of ubiquiones with side chain length of 6-10 units, e.g. including Coenzyme Q10 and Coenzyme Q9 was reported for controlled continuous culture of phototrophic bacteria (wild-type strains of Rhodobacter capsulatus, Rhodobacter sphaeroides, Thiocapsa roseopersicina and Ectothiorhodospira shaposhnikovii. Cells mostly contained one main ubiquinone, whereby the content and composition dependent on growth conditions, substrates and other factors. Preferred is a production of more than 0,1, preferably more than 1 to 6mg/g dry cells in one of said organisms or in any other microorganism, even more preferred are more than 10 mg/g dry cells, 20mg/g dry cells, 50mg/g dry cells, 100mg/g dry cells, 200mg/g dry cells, 300mg/g dry cells, 500mg/g dry cells or more.

**[0084.0.0.18] see [0084.0.0.0]**

[0085.0.18.18] With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as shown in table I, application no. 18, columns 5 or 7 or a derivative thereof, or
b) a genetic regulatory element, for example a promoter, which is functionally linked to the nucleic acid sequence as shown table I, application no. 18, columns 5 or 7 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.18] to [0087.0.0.18] see [0086.0.0.0] to [0087.0.0.0]**

[0088.0.18.18] In an advantageous embodiment of the invention, the organism takes the form of a plant whose Coenzyme content, in particular its Coenzyme Q9 and/or Coenzyme Q10 content is modified advantageously owing to the nucleic acid molecule of the present invention expressed. This is important for plant breeders since, for example, the nutritional value of plants for feed or nutrition is dependent on the abovementioned coenzymes, in particular on the essential coenzyme Q10, and the general amount of coenzymes as source in feed or food. After the activityof a protein as indicated in Table II, application no. 18, column 5, or being encoded by a nucleic acid molecule indicated in Table I, application no. 18, column 5, or of its homologs, e.g. as indicated in Table II, application no. 18, column 7has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.18] see [0088.1.0.0]**

**[0089.0.0.18] to [0094.0.0.18] see [0089.0.0.0] to [0094.0.0.0]**

[0095.0.18.18] It may be advantageous to increase the pool of the respective fine chemical in the transgenic organisms by the process according to the invention in order to isolate high amounts of the essentially pure fine chemical.

[0096.0.18.18] In another preferred embodiment of the invention a combination of the increased expression of the nucleic acid sequence or the protein of the invention together with the transformation of a protein or polypeptid for example a coenzyme precursor (e.g. isoprenoids, acetyl CoA, HMG-CoA, mevalonate, isopentyl pyrophosphate, geranyl pyrophosphate, farnesyl pyrophosphate, etc.) transporter protein or a compound, which increases the production of Coenzyme precursors is useful to increase the production of the respective fine chemical (see Bao and Ohlrogge, Plant Physiol. 1999 August; 120 (4): 1057-1062).

[0097.0.18.18] In may also be advantageous to increase the content of the lipid bound respective fine chemical.

[0098.0.18.18] In a preferred embodiment, the respective fine chemical is produced in accordance with the invention and, if desired, is isolated. The production of further antioxidants, vitamins or coenzymes such as coenzymes Q0, Q1, Q2, Q3, Q4, Q5, Q6, Q7, and/or Q8 or Q10 or retinal, vitamin E, vitamin B6, or other fat soluble vitamins or mixtures thereof by the process according to the invention is advantageous, e.g. for the production of compositions used in food and/or feed or cosmetic production.

[0099.0.18.18] In the case of the fermentation of microorganisms, the abovementioned coenzymes may accumulate in membrane fragments and/or in the lipophilic or hydrophobic fraction. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration.. This concentrated fermentation broth advantageously together with compounds for the formulation can subsequently be processed by lyophilization, spray drying, spray granulation or by other methods. Preferably the Coenzymes or the lipophilic or hydrophobic compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

[0100.0.18.18] Transgenic plants which comprise Coenzyme Q9 and Coenzyme Q10 synthesized in the process according to the invention can advantageously be marketed directly without there being any need for the membrane fragments, cells, cell fragments, organelles, plastids, waxes, oils, lipids or fats comprising the respective fine chemical to be isolated. For example, as coenzymes may be isolated from membranes, e.g. mitochondrial or plastid membranes, it can be sufficient to isolate only cell fractions comprising said membrane or fragments of said membranes. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue. However, the respective fine chemical produced in the process according to the invention can also be isolated from the organisms, advantageously plants, in the form of their cells, cell fragments, plastids, organelles, membranes, membrane fragments, waxes oils, fats, lipids and/or fatty acids. Coenzyme Q9 or Coenzyme Q10 produced by this process can be obtained by harvesting the organisms, either from the crop in which they grow, or from the field. This can be done via pressing or extraction of the plant parts, preferably the plant seeds. To increase the efficiency of oil extraction it is beneficial to clean, to temper and if necessary to hull and to flake the plant material especially the seeds. In this context, oils, fats, lipids, waxes and/or fatty acids fractions can be obtained by what is known as cold beating or cold pressing without applying heat. In the case of microorganisms, the latter are, after harvesting, for example extracted directly without further processing steps or else, after disruption, extracted via various methods with which the skilled worker is familiar. Chemically pure the fine chemical comprising compositions are advantageous for applications in the food or feed industry sector, the cosmetic sector and especially the pharmacological industry sector.

**[0101.0.18.18]see [0101.0.0.0]**

**[01 02.0.1 8.18]** Coenzymes can for example be detected advantageously via LC separation methods. The unambiguous detection for the presence of Coenzymes products can be obtained by analyzing recombinant organisms using analytical standard methods like LC-MS, LC-MSMS, or TLC. The material to be analyzed can be disrupted by sonication, grinding in a glass mill, liquid nitrogen and grinding, cooking, or via other applicable methods; see also Biotechnology of Vitamins, Pigments and Growth Factors, Edited by Erik J. Vandamme, London, 1989, p.96 to 103. [0103.0.18.18] In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide shown in table II, application no. 18, columns 5 or 7 or a fragment thereof, which confers an increase in the amount of the respective fine chemical in an organism or a part thereof;
b) nucleic acid molecule comprising, preferably at least the mature form, of the nucleic acid molecule shown in table I, application no. 18, columns 5 or 7;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers shown in table III, application no. 18, column 7 and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence shown in table IV, application no. 18, column 7 and conferring an increase in the amount of the respective fine chemical in an organism or a part thereof ;
k) nucleic acid molecule comprising one or more of the nucleic acid molecule encoding the amino acid sequence of a polypeptide encoding a domain of the polypeptide shown in table II, application no. 18, columns 5 or 7 and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[0103.1.18.18]** In one embodiment, the nucleic acid molecule used in the process of the invention distinguishes over the sequence indicated in Table I, application no. 18, columns 5 or 7, by one or more nucleotides. In one embodiment, the nucleic acid molecule used in the process of the invention does not consist of the sequence indicated in Table I, application no. 18, columns 5 or 7,: In one embodiment, the nucleic acid molecule used in the process of the invention is less than 100%, 99,999%, 99,99%, 99,9% or 99% identical to a sequence indicated in Table I, application no. 18, columns 5 or 7,. In another embodiment, the nucleic acid molecule does not encode a polypeptide of a sequence indicated in Table II, application no. 18, columns 5 or 7.

**[0103.2.18.18] ./.**

**[0104.0.18.18]** In one embodiment, the nucleic acid molecule or the invention or used in the process of the invention distinguishes over the sequence shown in table I, application no. 18, columns 5 or 7 by one or more nucleotides or does not consist of the sequence shown in table I, application no. 18, columns 5 or 7. In one embodiment, the nucleic acid molecule of the present invention is less than 100%, 99,999%, 99,99%, 99,9% or 99% identical to the sequence shown in table I, application no. 18, columns 5 or 7. In another embodiment, the nucleic acid molecule does not encode a polypeptide of the sequence shown in table II, application no. 18, columns 5 or 7.

**[0105.0.0.18] to [0107.0.0.18] see [0105.0.0.0] to [0107.0.0.0]**

[0108.0.18.18] Nucleic acid molecules with the sequence shown in table I, application no. 18, columns 5 or 7, nucleic acid molecules which are derived from the amino acid sequences shown in table II, application no. 18, columns 5 or 7 or from polypeptides comprising the consensus sequence shown in table IV, application no. 18, column 7, or their derivatives or homologues encoding polypeptides with the enzymatic or biological activity of an protein as shown in table II, application no. 18, column 5 or 7,or conferring a Coenzyme Q10 and/or Coenzyme Q9 increase dependent on its expression or its activity, are advantageously increased in the process according to the invention.

**[0109.0.0.18] see [0109.0.0.0]**

[0110.0.18.18] Nucleic acid molecules, which are advantageous for the process according to the invention and which encode polypeptides with an activity of a polypeptide of the invention or the polypeptide used in the method of the invention or used in the process of the invention, e.g. of a protein as indicated in Table II, application no. 18, column 5, or being encoded by a nucleic acid molecule indicated in Table I, application no. 18, column 5, or of its homologs, e.g. as indicated in Table II, application no. 18, column 7, can be determined from generally accessible databases.

**[0111.0.0.18] see [0111.0.0.0]**

**[0112.0.18.18]** The nucleic acid molecules used in the process according to the invention take the form of isolated nucleic acid sequences, which encode polypeptides with protein activity as shown in table I, application no. 18, column 3, and conferring Coenzyme Q10 and/or Coenzyme Q9 increase, e.g. a protein as indicated in Table II, as shown in table II, application no. 18, column 5 or 7,column 5, or being encoded by a nucleic acid molecule indicated in Table I, application no. 18, column 5, or of their homologs, e.g. as indicated in Table I or II, application no. 18, column 7.

**[0113.0.0.18] to [0117.0.0.18] see [0113.0.0.0] to [0117.0.0.0]**

[0118.1.0.18] In one embodiment, the nucleic acid molecule according to the invention or used in the process of the invention originates from a plant with a high Coenzyme Q10 and/or Coenzyme Q9 content. In one embodiment, the nucleic acid molecule according to the invention or used in the process of the invention originates from and/or is transformed into a plant with a high Coenzyme Q10 and/or Coenzyme Q9 content.

**[0118.0.0.18] to [0120.0.0.18] see [0118.0.0.0] to [0120.0.0.0]**

[0121.0.18.18] However, it is also possible to use artificial sequences, which differ in one or more bases from the nucleic acid sequences found in organisms, or in one or more amino acid molecules from polypeptide sequences found in organisms, in particular from the polypeptide sequences shown in table II, application no. 18, columns 5 or 7 or the functional homologues thereof as described herein, preferably conferring above-mentioned activity, i.e. conferring a respective fine chemical increase after increasing its activity, e.g. having the activity of an protein, as indicated in Table II, application no. 18, column 5 or 3, or being encoded by a nucleic acid molecule indicated in Table I, application no. 18, column 5, or of its homologs, e.g. as indicated in Table II, application no. 18, column 7.

**[0122.0.0.18] to [0127.0.0.18] see [0122.0.0.0] to [0127.0.0.0]**

[0128.0.18.18] Synthetic oligonucleotide primers for the amplification, e.g. as the pairs shown in table III, application no. 18, column 7, by means of polymerase chain reaction can be generated on the basis of a sequence shown herein, for example the sequence shown in table I, application no. 18, columns 5 or 7 or the sequences derived from table II, application no. 18, columns 5 or 7.

[0129.0.18.18] Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention or used in the method of the invention are indicated in the alignments shown in the figures. Conserved regions are those, which show a very little variation in the amino acid in one particular position of several homologs from different origin. The consensus sequence shown in table IV, application no. 18, column 7 is derived from said alignments.

[0130.0.18.18] Degenerated primers can then be utilized by PCR for the amplification of fragments of novel proteins having above-mentioned activity, e.g. conferring the increase of the respective fine chemical, in particular of Coenzyme Q10 and/or Coenzyme Q9 after increasing the expression or activity or having an activity as shown in table I, application no. 18, column 3, or further functional homologs of the polypeptide of the invention, e.g. homologs from a protein as indicated in Table II, application no. 18, column 5, or being encoded by a nucleic acid molecule indicated in Table I, application no. 18, column 5, or of its homologs, e.g. as indicated in Table II, application no. 18, column 7, from other organisms.

**[0131.0.0.18] to [0138.0.0.18] see [0131.0.0.0] to [0138.0.0.0]**

**[0139.0.18.18]** Polypeptides having above-mentioned activity, i.e. conferring the respective fine chemical increase, derived from other organisms, can be encoded by other DNA sequences which hybridize to the sequences shown in table I, application no. 18, columns 5 or 7 under relaxed hybridization conditions and which code on expression for peptides having the respective fine chemical-increasing activity.

**[0140.0.0.18] to [0146.0.0.18] see [0140.0.0.0] to [0146.0.0.0]**

[0147.0.18.18] Further, the nucleic acid molecule of the invention or used in the method of the invention comprises a nucleic acid molecule, which is a complement of one of the nucleotide sequences of above mentioned nucleic acid molecules or a portion thereof. A nucleic acid molecule which is complementary to one of the nucleotide sequences shown in table I, application no. 18, columns 5 or 7 is one which is sufficiently complementary to one of the nucleotide sequences shown in table I, application no. 18, columns 5 or 7 such that it can hybridize to one of the nucleotide sequences shown in table I, application no. 18, columns 5 or 7, thereby forming a stable duplex. Preferably, the hybridisation is performed under stringent hybridization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.18.18]** The nucleic acid molecule of the invention or used in the method of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence shown in table Iapplication no. 18 , columns 5 or 7, or a functional portion thereof and preferably has above mentioned activity, in particular having a the respective fine chemical, in particular Coenzyme Q10 and/or Coenzyme Q9-increasing activity after increasing the activity or an activity of an gene product as shown in table I, application no. 18, column 3, e.g. a gene encoding a protein as indicated in Table II, application no. 18, column 5, or comprising or expressing a nucleic acid molecule indicated in Table I, application no. 18, column 5, or of their homologs, e.g. as indicated in Table I or II, application no. 18, column 7.

[0149.0.18.18] The nucleic acid molecule of the invention or used in the method of the invention comprises a nucleotide sequence which hybridizes, preferably hybridizes under stringent conditions as defined herein, to one of the nucleotide sequences shown in table I, application no. 18, columns 5 or 7, or a portion thereof and encodes a protein having above-mentioned activity, e.g. conferring an increase of the fine chemical, and optionally, the activity ofa protein e.g. of a protein as indicated in Table II, application no. 18, column 5, or being encoded by a nucleic acid molecule indicated in Table I, application no. 18, column 5, or of their homologs, e.g. as indicated in Table I or II, application no. 18, column 7.

**[00149.1.18.18]** Optionally, the nucleotide sequence, which hybridises to one of the nucleotide sequences indicated in Table I, application no. 18, columns 5 or 7, has further one or more of the activities annotated or known for the a protein as indicated in Table II, application no. 18, column 3.

**[0150.0.18.18]** Moreover, the nucleic acid molecule of the invention or used in the method of the invention can comprise only a portion of the coding region of one of the sequences shown in table I, application no. 18, for example a fragment which can be used as a probe or primer or a fragment encoding a biologically active portion of the polypeptide of the present invention or of a polypeptide used in the process of the present invention, i.e. having above-mentioned activity, e.g. conferring an increase of

Coenzyme Q10 and/or Coenzyme Q9 if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., in table I, application no. 18, an anti-sense sequence of one of the sequences, e.g., set forth in table I, application no. 18, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primers pairs shown in table III, lines 231 to 234 and/or 235 to 242 and/or 599 to 602 , column 7 will result in a fragment of a gene product, e.g. of a gene encoding of a protein as indicated in Table II, application no. 18, column 5, or expressing a nucleic acid molecule indicated in Table I, application no. 18, column 5, or of its homologs, e.g. as indicated in Table II, application no. 18, column 7.

**[0151.0.0.18] see [0151.0.0.0]**

**[0152.0.18.18]** The nucleic acid molecule of the invention or used in the method of the invention encodes a polypeptide or portion thereof which includes an amino acid sequence which is sufficiently homologous to the amino acid sequence shown in table II, application no. 18, columns 5 or 7 such that the protein or portion thereof maintains the ability to participate in the respective fine chemical production, in particular a Coenzyme Q10 and/or Coenzyme Q9 increasing the activity as mentioned above or as described in the examples in plants or microorganisms is comprised.

[0153.0.18.18] As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in table II, application no. 18, columns 5 or 7 such that the protein or portion thereof is able to participate in the increase of the respective fine chemical production.
In one embodiment, a protein or portion thereof as indicated in Table II, application no. 18, columns 5 or 7, has for example an activity of a polypeptide indicated in Table II, application no. 18, column 3.

[0154.0.18.18] In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91 %, 92%, 93% or 94% and most preferably at least about 95%, 97%, 98%, 99% or more homologous to an entire amino acid sequence of table II, application no. 18, columns 5 or 7 and having above-mentioned activity, e.g. conferring preferably the increase of the respective fine chemical.

**[0155.0.0.18]** to **[0156.0.0.18]see [0155.0.0.0]** to **[0156.0.0.0]**

[0157.0.18.18]The invention further relates to nucleic acid molecules that differ from one of the nucleotide sequences shown in table I, application no. 18, columns 5 or 7 (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptide comprising a consensus sequence as indicated in Table IV, application no. 18, column 7, resp., or of the polypeptides encoded by the sequence shown in table II, application no. 18, columns 5 or 7 or the functional homologues. Advantageously, the nucleic acid molecule of the invention or used in the method of the invention comprises, or in an other embodiment has, a nucleotide sequence encoding a protein comprising, or in an other embodiment having, an amino acid sequence as indicated in Table IV, application no. 18, column 7, resp.,or as shown in table II, application no. 18, columns 5 or 7 or the functional homologues. In a still further embodiment, the nucleic acid molecule of the invention or used in the method of the invention encodes a full length protein which is substantially homologous to an amino acid sequence shown in table II, application no. 18, columns 5 or 7 or the functional homologues. However, in a preferred embodiment, the nucleic acid molecule of the present invention does not consist of the sequence shown in table I, application no. 18,

**[0158.0.0.18] to [0160.0.0.18] see [0158.0.0.0] to [0160.0.0.0]**

**[0161.0.18.18]** Accordingly, in another embodiment, a nucleic acid molecule of the invention or used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence shown in table I, application no. 18, columns 5 or 7. The nucleic acid molecule is preferably at least 20, 30, 50, 100, 250 or more nucleotides in length.

**[0162.0.0.18] see [0162.0.0.0]**

**[0163.0.18.18]** Preferably, a nucleic acid molecule of the invention or used in the method of the invention that hybridizes under stringent conditions to a sequence shown in table I, application no. 18, columns 5 or 7 corresponds to a naturally-occurring nucleic acid molecule of the invention or used in the method of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.18] see [0164.0.0.0]**

**[0165.0.18.18]** For example, nucleotide substitutions leading to amino acid substitutions at "non-essential" amino acid residues can be made in a sequence of the nucleic acid molecule of the invention or used in the process of the invention, e.g. shown in table I, application no. 18,.

**[0166.0.0.18] to [0167.0.0.18] see [0166.0.0.0] to [0167.0.0.0]**

[0168.0.18.18] Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in the sequences shown in table II, application no. 18, columns 5 or 7 yet retain said activity described herein. The nucleic acid molecule can comprise a nucleotide sequence encoding a polypeptide, wherein the polypeptide comprises an amino acid sequence at least about 50% identical to an amino acid sequence shown in table II, application no. 18,. and is capable of participation in the increase of production of the respective fine chemical after increasing its activity, e.g. its expression. Preferably, the protein encoded by the nucleic acid molecule is at least about 60% identical to the sequence shown in table II, application no. 18, columns 5 or 7more preferably at least about 70% identical to one of the sequences shown in table II, application no. 18, columns 5 or 7, even more preferably at least about 80%, 90%, 95% homologous to the sequence shown in table II, application no. 18, columns 5 or 7 and most preferably at least about 96%, 97%, 98%, or 99% identical to the sequence shown in table II, application no. 18, columns 5 or 7.

**[0169.0.0.18] to [0175.0.0.18] see [0169.0.7.7] to [0175.0.7.7]**

[0176.0.18.18] Functional equivalents derived from one of the polypeptides as shown in table II, application no. 18, columns 5 or 7 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in table II, application no. 18, columns 5 or 7 according to the invention and are distinguished by essentially the same properties as a polypeptide as shown in table II, application no. 18, columns 5 or 7.

[0177.0.18.18] Functional equivalents derived from a nucleic acid sequence as shown in table I, application no. 18, columns 5 or 7 according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of a polypeptides as shown in table II, application no. 18, columns 5 or 7 according to the invention and encode polypeptides having essentially the same properties as a polypeptide as shown in table II, application no. 18, columns 5 or 7.

**[0178.0.0.18] see [0178.0.0.0]** [0179.0.18.18] A nucleic acid molecule encoding an homologous to a protein sequence of table II, application no. 18, columns 5 or 7, can be created by introducing one or more nucleotide substitutions, additions or deletions into a nucleotide sequence of the nucleic acid molecule of the present invention, in particular of table I, application no. 18, columns 5 or 7, such that one or more amino acid substitutions, additions or deletions are introduced into the encoded protein. Mutations can be introduced into the encoding sequences of table I, application no. 18, columns 5 or 7, by standard techniques, such as site-directed mutagenesis and PCR-mediated mutagenesis.

**[0180.0.0.18] to [0183.0.0.18] see [0180.0.0.0] to [0183.0.0.0]**

**[0184.0.18.18]** Homologues of the nucleic acid sequences used, with a sequence shown in table I, application no. 18, columns 5 or 7, comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from a sequences shown, preferably from table I, application no. 18, columns 5 or 7 or from the derived nucleic acid sequences, the intention being, however, that the enzyme activity or the biological activity of the resulting proteins synthesized is advantageously retained or increased.

[0185.0.18.18] In one embodiment of the present invention, the nucleic acid molecule of the invention or used in the process of the invention comprises one or more of the sequences shown in any of the table I, application no. 18, columns 5 or 71n one embodiment, it is preferred that the nucleic acid molecule comprises as little as possible other nucleotides not shown in any one of table I, application no. 18, columns 5 or 7,. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, the nucleic acid molecule use in the process of the invention is identical to a sequences shown in table I, application no. 18, columns 5 or 7.

[0186.0.18.18] Also preferred is that one or more nucleic acid molecule used in the process of the invention encodes a polypeptide comprising a sequence shown in table II, application no. 18, columns 5 or 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment used in the inventive process, the encoded polypeptide is identical to a sequence shown in table II, application no. 18, columns 5 or 7.

[0187.0.18.18] In one embodiment, the nucleic acid molecule of the invention or used in the process encodes a polypeptide comprising a sequence shown in table II, application no. 18, columns 5 or 7, and comprises less than 100 further nucleotides. In a further embodiment, said nucleic acid molecule comprises less than 30 further nucleotides. In one embodiment, the nucleic acid molecule used in the process is identical to a coding sequence of a sequence shown in table I, application no. 18, columns 5 or 7,.

[0188.0.18.18] Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide shown in table II, application no. 18, columns 5 or 7, expressed under identical conditions.

[0189.0.18.18] Homologues of nucleic acid sequences shown in table I, application no. 18, columns 5 or 7 or of the derived sequences shown in table II, application no. 18, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.18] to [0191.0.0.18] see [0190.0.0.0] to [0191.0.0.0]**

[0191.1.18.18] In one embodiment, the organisms or a part thereof provides according to the herein mentioned process of the invention an increased level of the fine chemical bound to any wax, triglycerides, lipid, oil and/or fat or any composition thereof containing any bound or free Coenzyme Q10 and/or Coenzyme Q9, for example bound to lipids, lipoproteins, membrane fractions, sphingolipids, phosphoglycerides, lipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride, compared to said control or selected organisms or parts thereof.

**[0192.0.0.18] to [0203.0.0.18] see [0192.0.0.0] to [0203.0.0.0]**

[0204.0.18.18] Accordingly, in one embodiment, the invention relates to a nucleic acid molecule, which comprises a nucleic acid molecule selected from the group consisting of:
a) nucleic acid molecule encoding, preferably at least the mature form, of a polypeptide shown in table II, application no. 18, columns 5 or 7; or a fragment thereof conferring an increase in the amount of the respective fine chemical in an organism or a part thereof
b) nucleic acid molecule comprising, preferably at least the mature form, of a nucleic acid molecule shown in table I, application no. 18, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using the primers or primer pairs indicated in table III, application no. 18, column 7 and conferring an increase in the amount of the respective fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence shown in table IV, application no. 18, column 7 and conferring an increase in the amount of the respective fine chemical in an organism or a part thereof;
k) nucleic acid molecule encoding the amino acid sequence of a polypeptide encoding a domain of the polypeptide shown in table II, application no. 18, columns 5 or 7, and conferring an increase in the amount of the respective fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of the nucleic acid molecule shown in table I, application no. 18, columns 5 or 7 or a nucleic acid molecule encoding, preferably at least the mature form of, a polypeptide shown in table II, application no. 18, columns 5 or 7, and conferring an increase in the amount of the respective fine chemical in an organism or a part thereof; or which encompasses a sequence which is complementary thereto;
whereby, preferably, the nucleic acid molecule according to (a) to (I) distinguishes over a sequence depicted in table I, application no. 18, columns 5 or 7 by one or more nucleotides. In one embodiment, the nucleic acid molecule of the invention or used in the method of the invention does not consist of a sequence shown in table I, columns 5 or 7. In an other embodiment, the nucleic acid molecule of the present invention is at least 30 % identical and less than 100%, 99,999%, 99,99%, 99,9% or 99% identical to a sequence shown in table I, application no. 18, columns 5 or 7. In a further embodiment the nucleic acid molecule does not encode a polypeptide sequence shown in table II, columns 5 or 7. Accordingly, in one embodiment, the nucleic acid molecule of the present invention encodes in one embodiment a polypeptide which differs at least in one or more amino acids from a polypeptide shown in table II, application no. 18, columns 5 or 7 but does not encode a protein of a sequence shown in table II, application no. 18, columns 5 or 7. Accordingly, in one embodiment, the protein encoded by a sequence of a nucleic acid according to (a) to (I) does not consist of a sequence shown in table II, application no. 18, columns 5 or 7. In a further embodiment, the protein of the present invention is at least 30 % identical to protein sequence depicted in table II, application no. 18, columns 5 or 7 and less than 100%, preferably less than 99,999%, 99,99% or 99,9%, more preferably less than 99%, 985, 97%, 96% or 95% identical to a sequence shown in table II, application no. 18, columns 5 or 7.

**[0205.0.0.18] to [0226.0.0.18] see [0205.0.0.0] to [0226.0.0.0]**

[0227.0.18.18] The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence mentioned in table I, application no. 18, column 7, columns 5 or 7 or its derivatives, it is advantageous additionally to express and/or mutate further genes in the organisms. Especially advantageously, additionally at least one further gene of the isoprenoid biosynthetic pathway such as for acetyl CoA, HMG-CoA, Mevalonate, Isopentyl pyrophosphate, Geranyl pyrophosphate, Farnesyl pyrophosphate e.g. HMG-CoA Reductase, Mevalonate, Kinase, etc., is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the isoprenoids, coenzyme precursor or coenzymes, preferably Q9 and/or Q10, as desired since, for example, feedback regulations no longer exist to the same extent or not at all. In addition it might be advantageously to combine one or more sequences shown in table I, application no. 18, columns 5 or 7 with genes which generally support or enhances to growth or yield of the target organism, for example genes which lead to faster growth rate of microorganisms or genes which produces stress-, pathogen, or herbicide resistant plants.

[0227.1.18.18] In addition to the sequence mentioned in table I, application no. 18, columns 5 or 7 or its derivatives, it is advantageous additionally to knock out and/or mutate further genes in the organisms. E.g. it may be advantageous, if one or more genes of the catabolic pathway for isoprenoids or quinones are reduced, deleted or in another way knocked out in the organisms such as plants or microorganisms.

[0228.0.18.18] In a further embodiment of the process of the invention, therefore, organisms are grown, in which there is simultaneous overexpression of at least one nucleic acid or one of the genes which code for proteins involved in the isoprenoid metabolism, in particular in Coenzyme Q9 or Coenzyme Q10 synthesis.

**[0229.0.18.18] ./.**

**[0230.1.0.18] ./.**

**[0230.2.0.18] ./.**

**[0230.0.0.18] see [0230.0.0.0]**

[0231.0.18.18] In a further advantageous embodiment of the process of the invention, the organisms used in the process are those in which simultaneously a Coenzyme Q10 and/or Coenzyme Q9 degrading protein is attenuated, in particular by reducing the rate of expression of the corresponding gene.

**[0232.0.0.18] to [0276.0.0.18] see [0232.0.0.0] to [0276.0.0.0]**

[0277.0.18.18] The coenzymes produced can be isolated from the organism by methods with which the skilled worker is familiar, for example, via extraction, salt precipitation and/or different chromatography methods, e.g. as mentioned above. The process according to the invention can be conducted batchwise, semibatchwise or continuously. The respective fine chemical produced in the process according to the invention can be isolated as mentioned above from the organisms, advantageously plants, in the form of their waxes , membrane fractions, oils, fats, lipids and/or fatty acids. Fractions comprising the coenzymes produced by this process can be obtained by harvesting the organisms, either from the crop in which they grow, or from the field. This can be done via pressing or extraction of the plant parts, preferably the plant seeds.

**[0278.0.0.18] to [0283.0.0.18] see [0278.0.0.0] to [0283.0.0.0]**

[0283.0.18.18] Moreover, a native polypeptide conferring the increase of the respective fine chemical in an organism or part thereof can be isolated from cells (e.g., endothelial cells), for example using the antibody of the present invention as described below, in particular, an anti- protein as shown in table I, application no. 18, column 3-antibody or an antibody against a polypeptide as shown in table II, application no. 18, columns 5 or 7 or a antigenic part thereof, which can be produced by standard techniques utilizing the polypeptid of the present invention or characterized in the process of the present invention or fragment thereof, i.e., the polypeptide of this invention. Preferred are monoclonal antibodies specifically binding to a polypeptide as indicated in Table II, application no. 18,columns 5 or 7.

**[0284.0.0.18] see [0284.0.0.0]**

[0285.0.18.18] In one embodiment, the present invention relates to a polypeptide having a sequence shown in table II, application no. 18, columns 5 or 7 or as coded by a nucleic acid molecule shown in table I, application no. 18, columns 5 or 7 or functional homologues thereof.

[0286.0.18.18] In one advantageous embodiment, in the method of the present invention the activity of a polypeptide is increased comprising or consisting of a consensus sequence shown in table IV, application no. 18, column 7 and in one another embodiment, the present invention relates to a polypeptide comprising or consisting of a consensus sequence shown in table IV, application no. 18, column 7 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, application no. 18,column 7, .

**[0287.0.0.18] to [0289.0.0.18] see [0287.0.0.0] to [0289.0.0.0]**

**[0290.0.0.18] see [0290.0.0.0]**

[0291.0.18.18] In one advantageous embodiment, the method of the present invention comprises the increasing of a polypeptide comprising or consisting of plant or microorganism specific consensus sequences.
In one embodiment, said polypeptide of the invention or used in the method of the invention distinguishes over a sequence shown in table II, columns 5 or 7 by one or more amino acids. In one embodiment, polypeptide distinguishes form a sequence shown in table II, application no. 18, columns 5 or 7 by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence shown in table II, application no. 18, columns 5 or 7 by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence shown in table II, application no. 18, columns 5 or 7.

**[0292.0.0.18] see [0292.0.0.0]**

[0293.0.18.18] In one embodiment, the invention relates to polypeptide conferring an increase in the respective fine chemical in an organism or part being encoded by the nucleic acid molecule of the invention or used in the process of the invention and having a sequence which distinguishes from the sequence as shown in table II, application no. 18, columns 5 or 7 by one or more amino acids. In another embodiment, said polypeptide of the invention does not consist of the sequence shown in table II, application no. 18, columns 5 or 7. In a further embodiment, said polypeptide of the present invention is less than 100%, 99,999%, 99,99%, 99,9% or 99% identical. In one embodiment, said polypeptide does not consist of the sequence encoded by the nucleic acid molecules shown in table I, application no. 18" columns 5 or 7.

[0294.0.18.18] In one embodiment, the present invention relates to a polypeptide protein activity as shown in table I, application no. 18, column 3, which distinguishes over the sequence depicted in table II, application no. 18, columns 5 or 7 by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.18] to [0296.0.0.18] see [0295.0.0.0] to [0296.0.0.0]**

[0297.0.18.18] The language "substantially free of cellular material" includes preparations of the polypeptide of the invention or used in the method of the invention in which the protein is separated from cellular components of the cells in which it is naturally or recombinantly produced. In one embodiment, the language "substantially free of cellular material" includes preparations having less than about 30% (by dry weight) of "contaminating protein", more preferably less than about 20% of "contaminating protein", still more preferably less than about 10% of "contaminating protein", and most preferably less than about 5% "contaminating protein". The term "Contaminating protein" relates to polypeptides, which are not polypeptides of the present invention. When the polypeptide of the present invention or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations in which the polypeptide of the present invention is separated from chemical precursors or other chemicals, which are involved in the synthesis of the protein. The language "substantially free of chemical precursors or other chemicals" includes preparations having less than about 30% (by dry weight) of chemical precursors of a protein as indicated in Table II, application no. 18,column 5, or being encoded by a nucleic acid molecule indicated in Table I, application no. 18,column 5, or of its homologs, e.g. as indicated in Table II, application no. 18,column 7, or chemicals, more preferably less than about 20% chemical precursors of a protein as indicated in Table II, application no. 18, column 5, or being encoded by a nucleic acid molecule indicated in Table I, application no. 18, column 5, or of its homologs, e.g. as indicated in Table II, application no. 18, column 7, or of, still more preferably less than about 10% chemical precursors of a protein as indicated in Table II, application no. 18,column 5, or being encoded by a nucleic acid molecule indicated in Table I, application no. 18, column 5, or of its homologs, e.g. as indicated in Table II, application no. 18,column 7, or of and most preferably less than about 5% chemical precursors of a protein as indicated in Table II, application no. 18,column 5, 1 or being encoded by a nucleic acid molecule indicated in Table I, application no. 18, column 5, or of its homologs, e.g. as indicated in Table II, application no. 18, column 7, or of. In preferred embodiments, isolated proteins or biologically active portions thereof lack contaminating proteins from the same organism from which the polypeptide of the present invention is derived. Typically, such proteins are produced by recombinant techniques.

**[0298.0.18.18]** A polypeptide of the invention or used in the method of the invention can participate in the process of the present invention. The polypeptide or a portion thereof comprises preferably an amino acid sequence which is sufficiently homologous to an amino acid sequence shown in table II, application no. 18, columns 5 or 7.

**[0299.0.18.18]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences as shown in table II, application no. 18, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of table I, application no. 18, columns 5 or 7 or which is homologous thereto, as defined above.

**[0300.0.18.18]** Accordingly the polypeptide of the present invention can vary from the sequences shown in table II, application no. 18, columns 5 or 7 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91 %, 92%, 93%, 94% or 95%, and most preferably at least about 96%, 97%, 98%, 99% or more homologous to an entire amino acid sequence shown in table II, application no. 18, columns 5 or 7.

**[0301.0.0.18] see [0301.0.0.0]**

**[0302.0.18.18]** Biologically active portions of an polypeptide of the present invention include peptides comprising amino acid sequences derived from the amino acid sequence of the polypeptide of the present invention or used in the process of the present invention, e.g., the amino acid sequence shown in table II, application no. 18, columns 5 or 7 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.18] see [0303.0.0.0]**

**[0304.0.18.18]** Manipulation of the nucleic acid molecule of the invention or used in the method of the invention may result in the production of protein indicated in Table II, application no. 18, column 5, or being encoded by a nucleic acid molecule indicated in Table I, application no. 18,column 5, or of its homologs, e.g. as indicated in Table II, application no. 18, column 7, having differences from the wild-type protein. These proteins may be improved in efficiency or activity, may be present in greater numbers in the cell than is usual, or may be decreased in efficiency or activity in relation to the wild type protein.

**[0305.0.18.18]** Any mutagenesis strategies for the polypeptide of the present invention or the polypeptide used in the process of the present invention to result in increasing said activity are not meant to be limiting; variations on these strategies will be readily apparent to one skilled in the art. Using such strategies, and incorporating the mechanisms disclosed herein, the nucleic acid molecule and polypeptide of the invention or used in the method of the invention may be utilized to generate plants or parts thereof, expressing wildtype proteins as indicated in Table II, application no. 18,column 5, or being encoded by a nucleic acid molecule indicated in Table I, application no. 18,column 5 or of its homologs, e.g. as indicated in Table II, application no. 18, column 7, or a mutant of a protein as indicated in Table II, application no. 18,column 5 or 7, or being encoded by a nucleic acid molecule indicated in Table I, application no. 18, column 5, or of its homologs, e.g. as indicated in Table II, application no. 18, column 7, e.g. a mutated , and encoding nucleic acid molecules and polypeptide molecules of the invention or used in the method of the invention such that the yield, production, and/or efficiency of production of a desired compound is improved.

**[0306.0.0.18] to [0308.0.0.18] see [0306.0.0.0] to [0308.0.0.0]**

**[0309.0.18.18]** In one embodiment, an reference to a " protein (= polypeptide) of the invention" or as indicated in Table II, application no. 18, columns 5 or 7, refers to a polypeptide having an amino acid sequence corresponding to the polypeptide of the invention or used in the process of the invention, whereas a "non-polypeptide of the invention" or "other polypeptide" not being indicated in Table II, application no. 18, columns 5 or 7, refers to a polypeptide having an amino acid sequence corresponding to a protein which is not substantially homologous a polypeptide of the invention, preferably which is not substantially homologous to a polypeptide as indicated in Table II, application no. 18, columns 5 or 7, e.g., a protein which does not confer the activity described herein or annotated or known for as indicated in Table II, application no. 18, column 3, and which is derived from the same or a different organism. In one embodiment, a "non-polypeptide of the invention" or "other polypeptide" not being indicated in Table II, application no. 18, columns 5 or 7, does not confer an increase of the respective fine chemical in an organism or part thereof.

**[0310.0.0.18] to [0334.0.0.18] see [0310.0.0.0] to [0334.0.0.0]**

[0335.0.18.18] The dsRNAi method has proved to be particularly effective and advantageous for reducing the expression of the nucleic acid sequences of the table I, application no. 18, columns 5 or 7 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences of the table I, application no. 18, columns 5 or 7 and/or homologs thereof. In a double-stranded RNA molecule for reducing the expression of an protein encoded by a nucleic acid sequence of one of the table I, application no. 18, columns 5 or 7 and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.18] to [0342.0.0.18] see [0336.0.0.0] to [0342.0.0.0]**

[0343.0.18.18] As has already been described, 100 % sequence identity between the dsRNA and a gene transcript of a nucleic acid sequence of one of the sequences shown in table I, application no. 18, columns 5 or 7 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of sequences shown in table I, application no. 18, columns 5 or 7 or homologs thereof of the one organism, may be used to suppress the corresponding expression in another organism.

**[0344.0.0.18] to [0361.0.0.18] see [0344.0.0.0] to [0361.0.0.0]**

[0362.0.18.18] Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or used in the method of the invention, the nucleic acid construct of the invention, the antisense molecule of the invention, the vector of the invention or a nucleic acid molecule encoding the polypeptide of the invention, e.g. encoding a polypeptide having an protein activity a protein as shown in table I, application no.18, column 3 or of a protein as indicated in Table II, application no. 18, column 5, or being encoded by a nucleic acid molecule indicated in Table I, application no. 18, column 5, or of its homologs, e.g. as indicated in Table II, application no. 18, column 7. Due to the above mentioned activity the respective fine chemical content in a cell or an organism is increased. For example, due to modulation or manipulation, the cellular activity is increased, e.g. due to an increased expression or specific activity of the subject matters of the invention in a cell or an organism or a part thereof. Transgenic for a polypeptide having an protein activity a protein e.g. for a protein as indicated in Table II, application no. 18, column 5, or being encoded by a nucleic acid molecule indicated in Table I, application no. 18, column 5, or of its homologs, e.g. as indicated in Table II, application no. 18, column 7, means herein that due to modulation or manipulation of the genome, the activity of a protein as shown in table I, application no.18, column 3 is increased in the cell or organism or part thereof, e.g. of a protein as indicated in Table II, application no. 18, column 5, or being encoded by a nucleic acid molecule indicated in Table I, application no. 18, column 5, or of its homologs, e.g. as indicated in Table II, application no. 18, column 7,. Examples are described above in context with the process of the invention.

**[0363.0.0.18] to [0376.0.0.18] see [0363.0.0.0] to [0376.0.0.0]**

[0377.0.18.18] Accordingly, the present invention relates also to a process according to the present invention whereby the produced coenzymes are isolated to high purity, preferably, to purity of more than 70% w/w or more, more preferred are 80% or more, in particular preferred are 90% w/w or more, even more preferred are 95% w/w or more, e.g. 97% w/w or more, e.g. 98% w/w, 99% w/w, or 99,9% w/w.

[0378.0.18.18] In this manner, more than 50% by weight, advantageously more than 60% by weight, preferably more than 70% by weight, especially preferably more than 80% by weight, very especially preferably more than 90% by weight, of the Coenzyme Q9 or Coenzyme Q10 produced in the process can be recovered, purified or isolated. The resulting coenzymes, e.g. compositions comprising the former, can, if appropriate, subsequently be further purified, if desired mixed with other active ingredients such as vitamins, amino acids, carbohydrates, antibiotics and the like, and, if appropriate, formulated.

[0379.0.18.18] In one embodiment, said coenzyme is the respective fine chemical, preferably reduced Coenzyme Q9 and/or Coenzyme Q10.

[0380.0.18.18] The Coenzyme Q9 or Coenzyme Q10 comprising fraction or the respective fine chemical obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of a pharmaceuticals, food stuff, animal feeds, nutrients or cosmetics comprising the steps of the process according to the invention, including the isolation of Coenzyme Q9 or Coenzyme Q10 composition produced or the respective fine chemical produced if desired and formulating the product with a pharmaceutical acceptable carrier or formulating the product in a form acceptable for an application in agriculture. A further embodiment according to the invention is the use of the respective fine chemical produced in the process or of the transgenic organisms in animal feeds, foodstuffs, medicines, food supplements, cosmetics or pharmaceuticals.

**[0381.0.0.18] to [0382.0.0.18] see [0381.0.0.0] to [0382.0.0.0]**

[0383.0.5.18] For preparing antioxidant, coenzyme and/or vitamin-containing fine chemicals, in particular the respective fine chemical-comprising fine chemicals, it is possible to use as source organic compounds such as, for example, waxes, oils, fats and/or lipids or a membrane comprising fraction, in particular compositions comprising membranes of mitochondria or of plastids.

**[0384.0.0.18] see [0384.0.0.0]**

[0385.0.18.18] The fermentation broths obtained in this way, containing in particular Coenzyme Q10 and/or Coenzyme Q9 in mixtures with other lipids, fats and/or oils and/or with other vitamins or coenzymes or antioxidants normally have a dry matter content of from, 1 % to 50% by weight. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

[0386.0.18.18] However, it is also possible to further purify the fraction or composition comprising the respective fine chemical, e.g. Coenzyme Q9 and/or Coenzyme Q10, as produced. For this purpose, the product-containing composition is subjected for example to a thin layer chromatography on silica gel plates or to a chromatography such as a Florisil column (Bouhours J.F., J. Chromatrogr. 1979, 169, 462), in which case the desired product or the impurities are retained wholly or partly on the chromatography resin. These chromatography steps can be repeated if necessary, using the same or different chromatography resins. The skilled worker is familiar with the choice of suitable chromatography resins and their most effective use. An alternative method to purify the fatty acids is for example crystallization in the presence of urea. These methods can be combined with each other.

**[0387.0.0.18] to [0392.0.0.18] see [0387.0.0.0] to [0392.0.0.0]**

[0393.0.18.18] In one embodiment, the present invention relates to a method for the identification of a gene product conferring an increase in the respective fine chemical production in a cell, comprising the following steps:
(a) contacting-, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence shown in table I, application no. 18, columns 5 or 7 and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the respective fine chemical level in the host cells; and
(f) identifying the nucleic acid molecule and its gene product which expression confers an increase in the respective fine chemical level in the host cell after expression compared to the wild type.

**[0394.0.0.18] to [0522.0.0.18] see [0394.0.7.7] to [0522.0.7.7]**

**[0553.0.18.18]** A process for the production of Coenzyme Q9, which comprises
(a) increasing or generating the activity of a protein as indicated in Table II, application no. 18, columns 5 or 7, or a functional equivalent thereof in a non-human organism, or in one or more parts thereof; and
(b) growing the organism under conditions which permit the production of Coenzyme Q9 in said organism.
A process for the production of Coenzyme Q9, comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 18, columns 5 or 7, or a fragment thereof, which confers an increase in the amount of Coenzyme Q9 in an organism or a part thereof;
b) nucleic acid molecule comprising of a nucleic acid molecule as indicated in Table I, application no. 18, columns 5 or 7,
c) nucleic acid molecule whose sequence can be deduced from a polypeptide sequence encoded by a nucleic acid molecule of (a) or (b) as a result of the degeneracy of the genetic code and conferring an increase in the amount of Coenzyme Q9 in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of Coenzyme Q9in an organism or a part thereof;
e) nucleic acid molecule which hybridizes with a nucleic acid molecule of (a) to (c) under stringent hybridisation conditions and conferring an increase in the amount of Coenzyme Q9 in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 18, column 7, and conferring an increase in the amount of Coenzyme Q9 in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of Coenzyme Q9 in an organism or a part thereof;
h) nucleic acid molecule encoding a polypeptide comprising a consensus as indicated in Table IV, application no. 18, column 7, and conferring an increase in the amount of Coenzyme Q9 in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of Coenzyme Q9 in an organism or a part thereof
or comprising a sequence which is complementary thereto.
The process as mentioned above, comprising recovering of the free or bound Coenzyme Q9.
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound Coenzyme Q9 produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 18, columns 5 or 7, or a fragment thereof, which confers an increase in the amount of Coenzyme Q9 in an organism or a part thereof;
b) nucleic acid molecule comprising of a nucleic acid molecule as indicated in Table I, application no. 18, columns 5 or 7,;
c) nucleic acid molecule whose sequence can be deduced from a polypeptide sequence encoded by a nucleic acid molecule of (a) or (b) as a result of the degeneracy of the genetic code and conferring an increase in the amount of Coenzyme Q9 in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of Coenzyme Q9 in an organism or a part thereof;
e) nucleic acid molecule which hybridizes with a nucleic acid molecule of (a) to (c) under stringent hybridisation conditions and conferring an increase in the amount of Coenzyme Q9 in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 18, column 7, and conferring an increase in the amount of Coenzyme Q9 in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of Coenzyme Q9 in an organism or a part thereof;
h) nucleic acid molecule encoding a polypeptide comprising a consensus as indicated in Table IV, application no. 18, column 7, and conferring an increase in the amount of Coenzyme Q9 in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of Coenzyme Q9 in an organism or a part thereof
whereby the nucleic acid molecule distinguishes over the sequence as indicated in Table I, application no. 18, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 18, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of Coenzyme Q9 in an organism or a part thereof comprising:
(a) contacting cells, tissues , plants or microorganisms which express the a polypeptide encoded by the nucleic acid molecule as mentioned above conferring an increase in the amount of Coenzyme Q9 in an organism or a part thereof with a candidate compound or a sample comprising a plurality of compounds under conditions which permit the expression the polypeptide;
(b) assaying the Coenzyme Q9 level or the polypeptide expression level in the cell, tissue, plant or microorganism or the media the cell, tissue, plant or microorganisms is cultured or maintained in; and
(c) identifying a agonist or antagonist by comparing the measured Coenzyme Q9 level or polypeptide expression level with a standard Coenzyme Q9 or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased Coenzyme Q9 production in a plant or microorganism, comprising the steps:
(a) culturing a plant cell or tissue or microorganism or maintaining a plant expressing the polypeptide encoded by the nucleic acid molecule as mentioned above conferring an increase in the amount of Coenzyme Q9 CoQ 9in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of Coenzyme Q9 in an organism or a part thereof;
(b) identifying if the compound is an effective agonist by detecting the presence or absence or increase of a signal produced by said readout system.
A method for the identification of a gene product conferring an increase in Coenzyme Q9 production in a cell, comprising the following steps:
(a) contacting the nucleic acid molecules of a sample, which can contain a candidate gene encoding a gene product conferring an increase in Coenzyme Q9 after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing Coenzyme Q9;
(d) expressing the identified nucleic acid molecules in the host cells;
(e) assaying the Coenzyme Q9 level in the host cells; and
(f) identifying nucleic acid molecule and its gene product which expression confers an increase in the Coenzyme Q9 level in the host cell in the host cell after expression compared to the wild type.
A method for the identification of a gene product conferring an increase in Coenzyme Q9 production in a cell, comprising the following steps:
(a) identifying in a data bank nucleic acid molecules of an organism; which can contain a candidate gene encoding a gene product conferring an increase in the Coenzyme Q9amount or level in an organism or a part thereof after expression, and which are at least 20% homolog to the nucleic acid molecule as mentioned above;
(b) introducing the candidate nucleic acid molecules in host cells appropriate for producing Coenzyme Q9;
(c) expressing the identified nucleic acid molecules in the host cells;
(d) assaying the Coenzyme Q9 level in the host cells; and
(e) identifying nucleic acid molecule and its gene product which expression confers an increase in the Coenzyme Q9 level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of Coenzyme Q9 after expression.
Use of the polypeptide as mentioned above or the nucleic acid construct as mentioned above or the gene product identified according to the method as mentioned above for identifying compounds capable of conferring a modulation of Coenzyme Q9 levels in an organism.
Cosmetic, pharmaceutical, nutrition composition, food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the protection of vegetable fats, oils, lipids or waxes comprising the respective fine chemical.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the production of oils, lipids, fats or waxes comprising the respective fine chemical derived from microorganisms.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the production of of a pharmaceutical agent for the treatment of congestive heart failure, heart muscle dysfunction, reduced platelet size, limited of platelet activity, idiopathic dilated cardiomyopathy, atigue, chest pain, dyspnea and palpitations, hypertension and other manifestations of cardiovascular disease, lowering levels of Coenzyme Q10 due to treatments with HMG-CoA reductase inhibitors, diseased gingival, gingival inflammation, cancer, AIDS, other immune dysfunctions, Muscular dystrophy associated with cardiac disease, exercise-related muscle exhaustion and damage, obesity, neurodegenerative diseases, male infertility, chronic stable angina, significant reduction of plasma levels of lipid peroxidation, skin photoaging, lipid peroxidation or for reducing the size of tumors, for remission in metastatic breast cancer, for improving sperm motility, for protecting against oxidative stress, for stabilizing cell membranes or protecting seminal fluid or a cosmetic agent or a nutrition supplement for food or feed.

### New genes for a process for the production of fine chemicals

### Description

[0000.0.0.19] In a further embodiment, the present invention relates to a further process for the production of fine chemicals as defined below and the corresponding embodiments as described herein as follows.

[0001.0.0.19] for the disclosure of this paragraph see [0001.0.0.0].

**[0002.0.19.19]** Plants produce several important secondary metabolites from phenylalanine through the phenylpropanoid pathway. Such substances include flavonoids, lignins, tannins, salicylic acid and hydroxycinnamic acid esters. Recent work on the phenylpropanoid pathway has shown that the traditional view of lignin biosynthesis is incorrect. Although the hydroxylation and methylation reactions of the pathway were long thought to occur at the level of the free hydroxycinnamic aicds, it turns now out, that the enzymes catalyzing phenylpropanoid 3-hydroxylation and 3-0-methylation reactions uses shikimate and CoA conjugates as substrates. The recent cloning of a aldehyde dehydrogenase involved in ferulic acid and sinapic acid biosynthesis suggest that both substances are derived at least in part through oxidation of coniferaldehyde and sinapaldehyde (see Nair et al., 2004, Plant Cell, 16, 544-554 and citations therein).

**[0003.0.0.19] %**

**[0004.0.0.19] %**

**[0005.0.0.19] %**

**[0006.0.0.19] %**

**[0007.0.0.19]** Cinnamic acids, which include caffeic and ferulic acids, are also powerful antioxidants. Experiments have found that these compounds can stop the growth of cancer cells.
In addition sinapic acid is an intermediate in syringyl lignin biosynthesis in angiosperms, and in some taxa serves as a precursor for soluble secondary metabolites. The biosynthesis and accumulation of the sinapate esters sinapoylglucose, sinapoylmalate, and sinapoylcholine are developmentally regulated in at least Arabidopsis and other members of the Brassicaceae (Ruegger et al., 1999, 119(1): 101-10, 1999).
Due to these interesting physiological roles and agrobiotechnological potential of sinapic acid there is a need to identify the genes of enzymes and other proteins involved in sinapic acid metabolism, and to generate mutants or transgenic plant lines with which to modify the sinapic acid content in plants.

[0008.0.19.19] One way to increase the productive capacity of biosynthesis is to apply recombinant DNA technology. Thus, it would be desirable to produce sinapic acid in plants. That type of production permits control over quality, quantity and selection of the most suitable and efficient producer organisms. The latter is especially important for commercial production economics and therefore availability to consumers. In addition it is desirable to produce sinapic acid in plants in order to increase plant productivity and resistance against biotic and abiotic stress as discussed before.
Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Argobacterium* tumifaciens-mediated transformation of tobacco plants using a vector that contained a ketolase-encoding gene from *H. pluvialis* denominated *crt*O along with the *Pds* gene from tomato as the promoter and to encode a leader sequence. Those results indicated that about 75 percent of the carotenoids found in the flower of the transformed plant contained a keto group.

[0009.0.19.19] Thus, it would be advantageous if an algae, plant or other microorganism were available which produce large amounts sinapic acid .The invention discussed hereinafter relates in some embodiments to such transformed prokaryotic or eukaryotic microorganisms.
It would also be advantageous if plants were available whose roots, leaves, stem, fruits or flowers produced large amounts of sinapic acid. The invention discussed hereinafter relates in some embodiments to such transformed plants.

[0010.0.19.19] Therefore improving the quality of foodstuffs and animal feeds is an important task of the food-and-feed industry. This is necessary since, for example sinapic acid , as mentioned above, which occur in plants and some microorganisms are limited with regard to the supply of mammals. Especially advantageous for the quality of foodstuffs and animal feeds is as balanced as possible a specific sinapic acid profile in the diet since an excess of sinapic acid above a specific concentration in the food has a positive effect. A further increase in quality is only possible via addition of further sinapic acid , which are limiting.

[0011.0.19.19] To ensure a high quality of foods and animal feeds, it is therefore necessary to add sinapic acid in a balanced manner to suit the organism.

[0012.0.19.19] Accordingly, there is still a great demand for new and more suitable genes which encode enzymes or other proteins which participate in the biosynthesis of sinapic acid and make it possible to produce them specifically on an industrial scale without unwanted byproducts forming. In the selection of genes for biosynthesis two characteristics above all are particularly important. On the one hand, there is as ever a need for improved processes for obtaining the highest possible contents of sinapic acid ; on the other hand as less as possible byproducts should be produced in the production process.

[0013.0.0.19] for the disclosure of this paragraph see [0013.0.0.0].

[0014.0.19.19] Accordingly, in a first embodiment, the invention relates to a process for the production of a fine chemical, whereby the fine chemical is a sinapic acid . Accordingly, in the present invention, the term "the fine chemical" as used herein relates to a sinapic acid . Further, the term "the fine chemicals" as used herein also relates to fine chemicals comprising sinapic acid .

[0015.0.19.19] In one embodiment, the term "the fine chemical" or "the respective fine chemical" means at least one chemical compound with sinapic acid activity.
In one embodiment, the term "the fine chemical" means sinapic acid depending on the context in which the term is used. Throughout the specification the term "the fine chemical" means sinapic acid , its salts, ester, thioester or in free form or bound to other compounds such sugars or sugarpolymers, like glucoside, e.g. diglucoside. [0016.0.19.19]Accordingly, the present invention relates to a process for the production of the respective fine chemical comprising
(a) increasing or generating the activity of one or more
   of a protein as shown in table II, application no. 19, column 3 encoded by the nucleic acid sequences as shown in table I, application no. 19, column 5, in a non-human organism or in one or more parts thereof or
(b) growing the organism under conditions which permit the production of the fine chemical, thus sinapic acid of the invention or fine chemicals comprising sinapic acid of the invention, in said organism or in the culture medium surrounding the organism.

[0016.1.19.19] Accordingly, the term "the fine chemical" means in one embodiment "sinapic acid" in relation to all sequences listed in Tables I to IV, line 42 or homologs thereof.

**[0017.0.0.19] to [0019.0.0.19]** for the disclosure of the paragraphs [0017.0.0.19] to [0019.0.0.19] see paragraphs [0017.0.0.0] and [00198.0.0.0] above.

[0019.0.19.19]

[0020.0.19.19] Surprisingly it was found, that the transgenic expression of the Brassica napus protein as indicated in Table II, column 5, line 42 in a plant conferred an increase in sinapic acid content of the transformed plants. Thus, in one embodiment, said protein or its homologs are used for the production of sinapic acid.

[0021.0.0.19] for the disclosure of this paragraph see [0021.0.0.0] above.

[0022.0.19.19] The sequence of YDR513W from Saccharomyces cerevisiae has been published in Jacq, C. et al., Nature 387 (6632 Suppl), 75-78 (1997) and its activity is being defined as a protein having glutaredoxin (thioltransferase) (glutathione reductase) activity. Accordingly, in one embodiment, the process of the present invention comprises the use of a protein YDR513W having said activity, for the production of the respective fine chemical, in particular for increasing the amount of sinapic acid, preferably in free or bound form in an organism or a part thereof, as mentioned. In one embodiment, in the process of the present invention the activity of the protein YDR513W is increased.

[0022.1.0.19] to [0023.0.0.19] see [0022.1.0.19] to [0023.0.0.19]

[0023.1.0.19] Homologs of the polypeptide disclosed in table II, application no. 19, column 5, may have the activity of a protein as disclosed in table I or II, application no. 19, column 3 and may be the polypetides encoded by the nucleic acid molecules indicated in table I , application no. 19, column 7, resp., or may be the polypeptides indicated in table II, application no. 19, column 7, resp.

[0024.0.0.19] for the disclosure of this paragraph see [0024.0.0.0] above.

**[0025.0.19.19]** In accordance with the invention, a protein or polypeptide has the "activity of an protein as shown in table II, application no. 19, column 3" if its de *novo* activity, or its increased expression directly or indirectly leads to an increased in the level of the fine chemical indicated in the respective line of table II, application no. 19, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 19, column 3, or which has at least 10% of the original enzymatic or biological activity, preferably 20%, particularly preferably 30%, most particularly preferably 40% in comparison to a protein as shown in the respective line of table II, application no. 19, column 3 of a E. coli or Saccharomyces cerevisae, respectively, protein as mentioned in table I to IV, column 3 respectively and as disclosed in paragraph [0022] of the respective invention.

**[0025.1.0.19] and [0025.2.0.19]** for the disclosure of the paragraphs [0025.1.0.19] and [0025.2.0.19] see [0025.1.0.0] and [0025.2.0.0] above.

**[0026.0.0.19] to [0033.0.0.19]** for the disclosure of the paragraphs [0026.0.0.19] to [0033.0.0.19] see [0026.0.0.0] to [0033.0.0.0] above.

**[0034.0.19.19]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 19, column 3 its biochemical or genetical causes and the increased amount of the respective fine chemical.

**[0035.0.0.19] to [0044.0.0.19]** for the disclosure of the paragraphs [0035.0.0.19] to [0044.0.0.19] see paragraphs [0035.0.0.0] to [0044.0.0.0] above.

**[0045.0.19.19]In** one embodiment, the activity of the protein. as indicated in Table II, columns 5 or 7, application no. 19, is increased conferring an increase of the respective fine chemical, indicated in Table II, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more.

**[0046.0.0.19]** In one embodiment, the activity of the protein as indicated in Table II, columns 5 or 7, application no. 19, is increased conferring an increase of the respective fine chemical, indicated in Table II, column 6 of the respective line confers an increase of the respective fine chemical and of further sinapic acid or their precursors.

**[[0047.0.0.19] and [0048.0.0.19]** for the disclosure of the paragraphs [0047.0.0.19] and [0048.0.0.19] see paragraphs [0047.0.0.0] and [0048.0.0.0] above.

**[0049.0.19.19]** A protein having an activity conferring an increase in the amount or level of the fine chemical, has in one embodiment the structure of the polypeptide described herein, in particular of the polypeptides comprising the consensus sequence shown in table IV, application no. 19, column 7 or of the polypeptide as shown in the amino acid sequences as disclosed in table II, application no. 19, columns 5 and 7 or the functional homologues thereof as described herein, or is encoded by the nucleic acid molecule characterized herein or the nucleic acid molecule according to the invention, for example by the nucleic acid molecule as shown in table I, application no. 19, columns 5 and 7 or its herein described functional homologues and has the herein mentioned activity.

[0050.0.19.19] For the purposes of the present invention, the term "the respective fine chemical" also encompass the corresponding salts, such as, for example, the potassium or sodium salts of sinapic acid , resp., or their ester, or glucoside thereof, e.g the diglucoside thereof.

[0051.0.19.19] Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising sinapic acid . Depending on the choice of the organism used for the process according to the present invention, for example a microorganism or a plant, compositions or mixtures of sinapic acid can be produced.

[0052.0.0.19] for the disclosure of this paragraph see paragraph [0052.0.0.0] above.

**[0053.0.19.19]** In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 19, columns 5 and 7 or its homologs activity having herein-mentioned sinapic acid of the invention increasing activity; and/or
b) stabilizing a mRNA conferring the increased expression of a protein encoded by the nucleic acid molecule of the invention as shown in table I, application no. 19, columns 5 and 7, e.g. a nucleic acid sequence encoding a polypeptide having the activity of a protein as indicated in table II, application no. 19, columns 5 and 7 or its homologs activity or of a mRNA encoding the polypeptide of the present invention having herein-mentioned sinapic acid of the invention increasing activity; and/or
c) increasing the specific activity of a protein conferring the increased expression of a protein encoded by the nucleic acid molecule of the invention or of the polypeptide of the present invention having herein-mentioned sinapic acid increasing activity, e.g. of a polypeptide having the activity of a protein as indicated in table II, application no. 19, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned sinapic acid of the invention increasing activity, e.g. of a polypeptide having the activity of a protein as indicated in table II, application no. 19, columns 5 and 7 or its homologs activity; and/or
e) stimulating activity of a protein conferring the increased expression of a protein encoded by the nucleic acid molecule of the present invention or a polypeptide of the present invention having herein-mentioned sinapic acid of the invention increasing activity, e.g. of a polypeptide having the activity of a protein as indicated in table II, application no. 19, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned sinapic acid of the invention increasing activity, e.g. of a polypeptide having the activity of a protein as indicated in table II, application no. 19, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned sinapic acid of the invention increasing activity, e.g. of a polypeptide having the activity of a protein as indicated in table II, application no. 19, columns 5 and 7 or its homologs activity; and/or
h) increasing the expression of the endogenous gene encoding the polypeptide of the invention, e.g. a polypeptide having the activity of a protein as indicated in table II, application no. 19, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with especially high activity of the proteins of the invention from natural or from mutagenized resources and breeding them into the target organisms, eg the elite crops.

**[0054.0.19.19]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 19 in Table I to IV, resp., after increasing the expression or activity of the encoded polypeptide or having the activity of a polypeptide having an activity as the protein as shown in table II, application no. 19, column 3 or its homologs.

**[0055.0.0.19] to [0067.0.0.19]** for the disclosure of the paragraphs [0055.0.0.19] to [0067.0.0.19] see paragraphs [0055.0.0.0] to [0067.0.0.0] above.

[0068.0.19.19] The mutation is introduced in such a way that the production of sinapic acid is not adversely affected.

[0069.0.0.19] for the disclosure of this paragraph see paragraph [0069.0.0.0] above.

[0070.0.19.19] Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create de *novo* an advantageous, preferably novel metabolite composition in the organism, e.g. an advantageous composition of sinapic acid or their biochemical derivatives , e.g. comprising a higher content of (from a viewpoint of nutrional physiology limited) sinapic acid or their derivatives.

[0071.0.0.19] for the disclosure of this paragraph see paragraph [0071.0.0.0] above.

**[0072.0.0.19] %**

[0073.0.19.19] Accordingly, in one embodiment, the process according to the invention relates to a process, which comprises:
(a) providing a non-human organism, preferably a microorganism, a non-human animal, a plant or animal cell, a plant or animal tissue or a plant;
(b) increasing an activity of a polypeptide of the invention or a homolog thereof, e.g. as indicated in Table II, application no. 19, columns 5 or 7, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
(c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
(d) if desired, recovering, optionally isolating, the free and/or bound the respective fine chemical synthesized by the organism, the microorganism, the non-human animal, the plant or animal cell, the plant or animal tissue or the plant.

[0074.0.19.19] The organism, in particular the microorganism, non-human animal, the plant or animal cell, the plant or animal tissue or the plant is advantageously grown in such a way that it is not only possible to recover, if desired isolate the free or bound respective fine chemical.

[0075.0.0.19] to [0077.0.0.19] for the disclosure of the paragraphs [0075.0.0.19] to [0077.0.0.19] see paragraphs [0075.0.0.0] to [0077.0.0.0] above.

[0078.0.19.19] The organism such as microorganisms or plants or the recovered, and if desired isolated, the respective fine chemical can then be processed further directly into foodstuffs or animal feeds or for other applications. The fermentation broth, fermentation products, plants or plant products can be purified with methods known to the person skilled in the art. Products of these different work-up procedures are sinapic acid or comprising compositions of sinapic acid still comprising fermentation broth, plant particles and cell components in different amounts, advantageously in the range of from 0 to 99% by weight, preferably below 80% by weight, especially preferably below 50% by weight.

**[0079.0.0.19] to [0084.0.0.19]** for the disclosure of the paragraphs [0079.0.0.19] to [0084.0.0.19] see paragraphs [0075.0.0.0] to [0084.0.0.0] above.

[0085.0.19.19] With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, application no. 19, columns 5 or 7, or a derivative thereof, or
b) a genetic regulatory element, for example a promoter, which is functionally linked to the nucleic acid sequence as indicated in Table I, application no. 19, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.19] and [0087.0.0.19]** for the disclosure of the paragraphs [0086.0.0.19] and [0087.0.0.19] see paragraphs [0086.0.0.0] and [0087.0.0.0] above.

[0088.0.19.19] In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for animals such as poultry is dependent on the abovementioned fine chemicals or the plants are more resistant to biotic and abiotic stress and the yield is increased.

[0088.1.0.19] for the disclosure of this paragraph see paragraph [0088.1.0.0] above.

**[0089.0.0.19] to [0094.0.0.19]** for the disclosure of the paragraphs [0089.0.0.19] to [0094.0.0.19] see paragraphs [0089.0.0.0] to [0094.0.0.0] above.

[0095.0.19.19] It may be advantageous to increase the pool of sinapic acid in the transgenic organisms by the process according to the invention in order to isolate high amounts of the pure respective fine chemical and/or to obtain increased resistance against biotic and abiotic stresses and to obtain higher yield.

[0096.0.19.19] In another preferred embodiment of the invention a combination of the increased expression of the nucleic acid sequence or the protein of the invention together with the transformation of a protein or polypeptid or a compound, which functions as a sink for the desired fine chemical, for example in the organism, is useful to increase the production of the respective fine chemical.

[0097.0.0.19] for the disclosure of this paragraph see paragraph [0097.0.0.0] above.

[0098.0.19.19] In a preferred embodiment, the respective fine chemical is produced in accordance with the invention and, if desired, is isolated.

[0099.0.19.19] In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

[0100.0.19.19] Transgenic plants which comprise the fine chemicals such as sinapic acid synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because sinapic acid in microorganisms are localized intracellular, their recovery essentially comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.
[0101.0.19.19] The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

[0102.0.19.19] Sinapic acid can for example be detected advantageously via HPLC, LC or GC separation methods. The unambiguous detection for the presence of sinapic acid containing products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.

[0103.0.19.19] In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, application no. 19, columns 5 or 7, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 19, columns 5 or 7,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, application no. 19, columns 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, application no. 19, column 7, and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, application no. 19, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

[00103.1.19.19] In one embodiment, the nucleic acid molecule used in the process of the invention distinguishes over the sequence indicated in Table I, application no. 19, columns 5 or 7 by one or more nucleotides. In one embodiment, the nucleic acid molecule used in the process of the invention does not consist of the sequence shown in indicated in Table I ,application no. 19, columns 5 or 7,. In one embodiment, the nucleic acid molecule used in the process of the invention is less than 100%, 99,999%, 99,99%, 99,9% or 99% identical to a sequence indicated in Table I , application no. 19, columns 5 or 7,. In another embodiment, the nucleic acid molecule does not encode a polypeptide of a sequence indicated in Table II, application no. 19, columns 5 or 7.

[0104.0.19.19] In one embodiment, the nucleic acid molecule used in the process of the present invention distinguishes over the sequence indicated in Table I, application no. 19, columns 5 or 7, by one or more nucleotides. In one embodiment, the nucleic acid molecule used in the process of the invention does not consist of the sequence indicated in Table I, application no. 19, columns 5 or 7. In one embodiment, the nucleic acid molecule of the present invention is less than 100%, 99,999%, 99,99%, 99,9% or 99% identical to the sequence indicated in Table I, application no. 19, columns 5 or 7. In another embodiment, the nucleic acid molecule does not encode a polypeptide of a sequence indicated in Table II, application no. 19, columns 5 or 7.

**[0105.0.0.19] to [0107.0.0.19]** for the disclosure of the paragraphs [0105.0.0.19] to [0107.0.0.19] see paragraphs [0105.0.0.0] and [0107.0.0.0] above.

**[0108.0.19.19]** Nucleic acid molecules with the sequence as indicated in Table I, application no. 19, columns 5 or 7, nucleic acid molecules which are derived from an amino acid sequences as indicated in Table II, application no. 19, columns 5 or 7, or from polypeptides comprising the consensus sequence as indicated in Table IV, application no. 19, column 7, or their derivatives or homologues encoding polypeptides with the enzymatic or biological activity of an activity of a polypeptide as indicated in Table II, application no. 19, column 3, 5 or 7, e.g. conferring the increase of the respective fine chemical, meaning sinapic acid , resp., after increasing its expression or activity, are advantageously increased in the process according to the invention.

[0109.0.19.19] In one embodiment, said sequences are cloned into nucleic acid constructs, either individually or in combination. These nucleic acid constructs enable an optimal synthesis of the respective fine chemicals, in particular sinapic acid , produced in the process according to the invention.

**[0110.0.0.19]** Nucleic acid molecules, which are advantageous for the process according to the invention and which encode polypeptides with an activity of a polypeptide used in the method of the invention or used in the process of the invention, e.g. of a protein as shown in Table II, application no. 19, columns 5 or 7, or being encoded by a nucleic acid molecule indicated in Table I, application no. 19, columns 5 or 7, or of its homologs, e.g. as indicated in Table II, application no. 19, columns 5 or 7, can be determined from generally accessible databases.

[0111.0.0.19] for the disclosure of this paragraph see [0111.0.0.0] above.

**[0112.0.19.19]** The nucleic acid molecules used in the process according to the invention take the form of isolated nucleic acid sequences, which encode polypeptides with an activity of a polypeptide as indicated in Table II, application no. 19, column 3, or having the sequence of a polypeptide as indicated in Table II, application no. 19, columns 5 and 7, and conferring an increase in the sinapic acid level.

**[0113.0.0.19] to [0120.0.0.19]** for the disclosure of the paragraphs [0113.0.0.19] to [0120.0.0.19] see paragraphs [0113.0.0.0] and [0120.0.0.0] above.

[0121.0.19.19] However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 19, columns 5 or 7, or the functional homologues thereof as described herein, preferably conferring above-mentioned activity, i.e. conferring a sinapic acid level increase after increasing the activity of the polypeptide sequences indicated in Table II, application no. 19, columns 5 or 7.

**[0122.0.0.19] to [0127.0.0.19]** for the disclosure of the paragraphs [0122.0.0.19] to [0127.0.0.19] see paragraphs [0122.0.0.0] and [0127.0.0.0] above.

**[0128.0.19.19]** Synthetic oligonucleotide primers for the amplification, e.g. as the pairs indicated in Table III, application no. 19, column 7, by means of polymerase chain reaction can be generated on the basis of a sequence shown herein, for example the sequence as indicated in Table I, application no. 19, columns 5 or 7, or the sequences derived from a sequences as indicated in Table II, application no. 19, columns 5 or 7.

[0129.0.19.19] Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequence shown in Table IV, application no. 19, column 7, is derived from said aligments.

[0130.0.19.19] for the disclosure of this paragraph see [0130.0.0.0].

**[0131.0.0.19] to [0138.0.0.19]** for the disclosure of the paragraphs [0131.0.0.19] to [0138.0.0.19] see paragraphs [0131.0.0.0] to [0138.0.0.0] above.

[0139.0.19.19] Polypeptides having above-mentioned activity, i.e. conferring the respective fine chemical level increase, derived from other organisms, can be encoded by other DNA sequences which hybridise to a sequence indicated in Table I, application no. 19, columns 5 or 7, for sinapic acid under relaxed hybridization conditions and which code on expression for peptides having the respective fine chemical, i.e. sinapic acid , resp., increasing-activity.

**[0140.0.0.19] to [0146.0.0.19]** for the disclosure of the paragraphs [0140.0.0.19] to [0146.0.0.19] see paragraphs [0140.0.0.0] and [0146.0.0.0] above.

**[0147.0.19.19]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 19, columns 5 or 7, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.19.19]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 19, columns 5 or 7 or a portion thereof and preferably has above mentioned activity, in particular, of sinapic acid increasing activity after increasing the acitivity or an activity of a product of a gene encoding said sequences or their homologs.

**[0149.0.19.19]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 19, columns 5 or 7, or a portion thereof and encodes a protein having above-mentioned activity and as indicated in indicated in Table II.

**[00149.1.19.19]** Optionally, in one embodiment, the nucleotide sequence, which hybridises to one of the nucleotide sequences indicated in Table I, columns 5 or 7, lines 243 to 250 and 603has further one or more of the activities annotated or known for a protein as indicated in Table II, column 3, lines 243 to 250 and 603.

**[0150.0.19.19]** Moreover, the nucleic acid molecule of the invention can comprise only a portion of the coding region of one of the sequences indicated in Table I, application no. 19, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of sinapic acid, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 19, columns 5 or 7, , an anti-sense sequence of one of the sequences, e.g., as indicated in Table I, application no. 19, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 19, column 7, will result in a fragment of a polynucleotide sequence as indicated in Table I, application no. 19, columns 5 or 7 or its gene product.

[0151.0.0.19]: for the disclosure of this paragraph see [0151.0.0.0] above.

[0152.0.19.19] The nucleic acid molecule of the invention encodes a polypeptide or portion thereof which includes an amino acid sequence which is sufficiently homologous to an amino acid sequence as indicated in Table II, application no. 19, columns 5 or 7, such that the protein or portion thereof maintains the ability to participate in the respective fine chemical production, in particular a sinapic acid increasing activity as mentioned above or as described in the examples in plants or microorganisms is comprised.

[0153.0.19.19] As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 19, columns 5 or 7, such that the protein or portion thereof is able to participate in the increase of the respective fine chemical production. In one embodiment, a protein or portion thereof as indicated in Table II, application no. 19, columns 5 or 7, has for example an activity of a polypeptide indicated in Table II, application no. 19, column 3.

**[0154.0.19.19]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91 %, 92%, 93% or 94% and most preferably at least about 95%, 97%, 98%, 99% or more homologous to an entire amino acid sequence as indicated in Table II, application no. 19, columns 5 or 7, and has above-mentioned activity, e.g.conferring preferably the increase of the respective fine chemical.

**[0155.0.0.19] and [0156.0.0.19]** for the disclosure of the paragraphs [0155.0.0.19] and [0156.0.0.19] see paragraphs [0155.0.0.0] and [0156.0.0.0] above.

[0157.0.19.19] The invention further relates to nucleic acid molecules that differ from one of the nucleotide sequences as indicated in Table I, application no. 19, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, application no. 19, column 7, or as polypeptides depicted in Table II, application no. 19, columns 5 or 7, or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, application no. 19, column 7, or of the polypeptide as indicated in Table II, application no. 19, columns 5 or 7, or the functional homologues. In a still further embodiment, the nucleic acid molecule of the invention encodes a full length protein which is substantially homologous to an amino acid sequence comprising a consensus sequence as indicated in Table IV, application no. 19, column 7, or of a polypeptide as indicated in Table II, application no. 19, columns 5 or 7, or the functional homologues. However, in a preferred embodiment, the nucleic acid molecule of the present invention does not consist of a sequence as indicated in Table I, application no. 19, columns 5 or 7. Preferably the nucleic acid molecule of the invention is a functional homologue or identical to a nucleic acid molecule indicated in Table I, application no. 19, columns 5 or 7.

**[0158.0.0.19] to [0160.0.0.19]** for the disclosure of the paragraphs [0158.0.0.19] to [0160.0.0.19] see paragraphs [0158.0.0.0] to [0160.0.0.0] above.

**[0161.0.19.19]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 19, columns 5 or 7. The nucleic acid molecule is preferably at least 20, 30, 50, 100, 250 or more nucleotides in length.

[0162.0.0.19] for the disclosure of this paragraph see paragraph [0162.0.0.0] above.

**[0163.0.19.19]** Preferably, a nucleic acid molecule of the invention that hybridizes under stringent conditions to a sequence as indicated in Table I, application no. 19, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in a organism or a part thereof, e.g. a tissue, a cell, or a compartment of a cell, after increasing the expression or activity thereof or the activity of a protein of the invention or used in the process of the invention.

[0164.0.0.19] for the disclosure of this paragraph see paragraph [0164.0.0.0] above.

[0165.0.19.19] For example, nucleotide substitutions leading to amino acid substitutions at "non-essential" amino acid residues can be made in a sequence of the nucleic acid molecule of the invention or used in the process of the invention, e.g. as indicated in Table I, application no. 19, columns 5 or 7.

**[0166.0.0.19] and [0167.0.0.19]** for the disclosure of the paragraphs [0166.0.0.19] and [0167.0.0.19] see paragraphs [0166.0.0.0] and [0167.0.0.0] above.

[0168.0.19.19] Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 19, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 19, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 19, columns 5 or 7, more preferably at least about 70% identical to one of the sequences as indicated in Table II, application no. 19, columns 5 or 7, ,even more preferably at least about 80%, 90%, or 95% homologous to a sequence as indicated in Table II, application no. 19, columns 5 or 7, , and most preferably at least about 96%, 97%, 98%, or 99% identical to the sequence as indicated in Table II, application no. 19, columns 5 or 7.

**[0169.0.0.19] to [0172.0.0.19]** for the disclosure of the paragraphs [0169.0.0.19] to [0172.0.0.19] see paragraphs [0169.0.0.0] to [0172.0.0.0] above.

**[0173.0.19.19]**For example a sequence which has a 80% homology with sequence SEQ ID NO: 227 at the nucleic acid level is understood as meaning a sequence which, upon comparison with the sequence SEQ ID NO: 227 by the above Gap program algorithm with the above parameter set, has a 80% homology.

[0174.0.0.19]: for the disclosure of this paragraph see paragraph [0174.0.0.0] above.

**[0175.0.19.19]**For example a sequence which has a 80% homology with sequence SEQ ID NO: 228 at the protein level is understood as meaning a sequence which, upon comparison with the sequence SEQ ID NO: 228 by the above program algorithm with the above parameter set, has a 80% homology.

[0176.0.19.19] Functional equivalents derived from one of the polypeptides as indicated in Table II, application no. 19, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 19, columns 5 or 7, according to the invention and are distinguished by essentially the same properties as a polypeptide as indicated in Table II, application no. 19, columns 5 or 7.

[0177.0.19.19] Functional equivalents derived from a nucleic acid sequence as indicated in Table I, application no. 19, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptide as indicated in Table II, application no. 19, columns 5 or according to the invention and encode polypeptides having essentially the same properties as a polypeptide as indicated in Table II, application no. 19, columns 5 or 7,.

[0178.0.0.19] for the disclosure of this paragraph see [0178.0.0.0] above.

[0179.0.19.19] A nucleic acid molecule encoding a homologous to a protein sequence as indicated in Table II, application no. 19, columns 5 or 7, can be created by introducing one or more nucleotide substitutions, additions or deletions into a nucleotide sequence of the nucleic acid molecule of the present invention, in particular as indicated in Table I, application no. 19, columns 5 or 7, such that one or more amino acid substitutions, additions or deletions are introduced into the encoded protein. Mutations can be introduced into the encoding sequences as indicated in Table I, application no. 19, columns 5 or 7, by standard techniques, such as site-directed mutagenesis and PCR-mediated mutagenesis.

**[0180.0.0.19] to [0183.0.0.19]** for the disclosure of the paragraphs [0180.0.0.19] to [0183.0.0.19] see paragraphs [0180.0.0.0] to [0183.0.0.0] above.

**[0184.0.19.19]** Homologues of the nucleic acid sequences used, with a sequence as indicated in Table I, application no. 19, columns 5 or 7, or of the nucleic acid sequences derived from a sequences as indicated in Table II, application no. 19, columns 5 or 7, comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 19, columns 5 or 7, or from the derived nucleic acid sequences, the intention being, however, that the enzyme activity or the biological activity of the resulting proteins synthesized is advantageously retained or increased.

[0185.0.19.19] In one embodiment of the present invention, the nucleic acid molecule of the invention or used in the process of the invention comprises one or more sequences as indicated in Table I, application no. 19, columns 5 or 7,. In one embodiment, it is preferred that the nucleic acid molecule comprises as little as possible other nucleotides not shown in any one of sequences as indicated in Table I, application no. 19, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 19, columns 5 or 7.

[0186.0.19.19] Also preferred is that one or more nucleic acid molecule(s) used in the process of the invention encodes a polypeptide comprising a sequence as indicated in Table II, application no. 19, columns 5 or 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 19, columns 5 or 7.

**[0187.0.19.19]** In one embodiment, a nucleic acid molecule of the invention or used in the process encodes a polypeptide comprising the sequence as indicated in Table II, application no. 19, columns 5 or 7, comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 19, columns 5 or 7.

**[0188.0.19.19]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 19, columns 5 or 7 and is expressed under identical conditions.

**[0189.0.19.19]** Homologues of a sequences as indicated in Table I, application no. 19, columns 5 or 7, or of a derived sequences as indicated in Table II, application no. 19, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.19], [0191.0.0.19], [00191.1.0.19] and [0192.0.0.19] to [0203.0.0.19] for** the disclosure of the paragraphs [0190.0.0.19], [0191.0.0.19], [0191.1.0.19] and [0192.0.0.19] to [0203.0.0.19] see paragraphs [0190.0.0.0], [0191.0.0.0], [0191.1.0.0] and [0192.0.0.0] to [0203.0.0.0] above.

[0204.0.19.19] Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 19, columns 5 or 7, or a fragment thereof conferring an increase in the amount of the respective fine chemical, in particular according to Table II, application no. 19, column 6 in an organism or a part thereof
b) nucleic acid molecule comprising, preferably at least the mature form, of a nucleic acid molecule as indicated in Table I, application no. 19, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, application no. 19, column 7, and conferring an increase in the amount of the respective fine chemical, in particular according to Table II, application no. 19, column 6 in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, application no. 19, columns 5 or 7, and conferring an increase in the amount of the respective fine chemical, in particular according to table II, application no. 19, column 6 in an organism or a part thereof;
k) nucleic acid molecule encoding the amino acid sequence of a polypeptide encoding a domaine of a polypeptide as indicated in Table II, application no. 19, columns 5 or 7, and conferring an increase in the amount of the respective fine chemical, in particular accccording to table II, application no. 19, column 6 in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 19, columns 5 or 7, or a nucleic acid molecule encoding, preferably at least the mature form of, a polypeptide as indicated in Table II, application no. 19, columns 5 or 7, and conferring an increase in the amount of the respective fine chemical according to Table II, application no. 19, column 6 in an organism or a part thereof;
   or which encompasses a sequence which is complementary thereto;
whereby, preferably, the nucleic acid molecule according to (a) to (I) distinguishes over the sequence indicated in Table I, application no. 19,columns 5 or 7, by one or more nucleotides. In one embodiment, the nucleic acid molecule does not consist of the sequence shown and indicated in Table I, application no. 19, columns 5 or 7,
In one embodiment, the nucleic acid molecule is less than 100%, 99,999%, 99,99%, 99,9% or 99% identical to a sequence indicated in Table I, application no. 19, columns 5 or 7,.
In another embodiment, the nucleic acid molecule does not encode a polypeptide of a sequence indicated in Table II, application no. 19, columns 5 or 7,.
In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 19, columns 5 or 7,.
In a further embodiment the nucleic acid molecule does not encode a polypeptide sequence as indicated in Table II, application no. 19, columns 5 or 7,.
Accordingly, in one embodiment, the nucleic acid molecule of the differs at least in one or more residues from a nucleic acid molecule indicated in Table I, application no. 19, columns 5 or 7,.
Accordingly, in one embodiment, the nucleic acid molecule of the present invention encodes a polypeptide, which differs at least in one or more amino acids from a polypeptide indicated in Table II, application no. 19, columns 5 or 7,.
In another embodiment, a nucleic acid molecule indicated in Table I, application no. 19, columns 5 or 7,.
Accordingly, in one embodiment, the protein encoded by a sequences of a nucleic acid accoriding to (a) to (I) does not consist of a sequence as indicated in Table II, application no. 19, columns 5 or 7,.
In a further embodiment, the protein of the present invention is at least 30 %, 40 %, 50%, or 60% identical to a protein sequence indicated in Table II, application no. 19, columns 5 or 7, and less than 100%, preferably less than 99,999%, 99,99% or 99,9%, more preferably less than 99%, 985, 97%, 96% or 95% identical to a sequence as indicated in Table I, columns 5 or 7.

**[0205.0.0.19] and [0206.0.0.19]** for the disclosure of the paragraphs [0205.0.0.19] and [0206.0.0.19] see paragraphs [0205.0.0.0] and [0206.0.0.0] above.

[0207.0.19.19] As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.19] to [0226.0.0.19]** for the disclosure of the paragraphs [0208.0.0.19] to [0226.0.0.19] see paragraphs [0208.0.0.0] to [0226.0.0.0] above.

**[0227.0.19.19]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence indicated in Table I, application no. 19, columns 5 or 7, or its derivatives, it is advantageous to express and/or mutate further genes in the organisms. Especially advantageously, additionally at least one further gene of the glutamic acid or phosphoenolpyruvate metabolic pathway, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table I, application no. 19, columns 5 or 7, with genes which generally support or enhances to growth or yield of the target organismen, for example genes which lead to faster growth rate of microorganisms or genes which produces stress-, pathogen, or herbicide resistant plants.

**[0228.0.19.19] %**

[0229.0.19.19] Further advantageous nucleic acid sequences which can be expressed in combination with the sequences indicated in Table I, application no. 19, columns 5 or 7, used in the process and/or the abovementioned biosynthesis genes are the sequences encoding further genes of the aromatic amino acid pathway, such as tryptophan, phenylalanine or tyrosine. These genes can lead to an increased synthesis of the essential amino acids tryptophan, phenylalanine or tyrosine.

[0230.0.0.19] for the disclosure of this paragraph see paragraph [0230.0.0.0] above.

[0231.0.19.19] In a further advantageous embodiment of the process of the invention, the organisms used in the process are those in which simultaneously a sinapic acid degrading protein is attenuated, in particular by reducing the rate of expression of the corresponding gene. A person skilled in the art knows for example, that the inhibition or repression of a sinapic acid degrading enzyme will result in an increased sinapic acidaccumulation in the plant.

**[0232.0.0.19] to [0276.0.0.19]** for the disclosure of the paragraphs [0232.0.0.19] to [0276.0.0.19] see paragraphs [0232.0.0.0] to [0276.0.0.0] above.

[0277.0.19.19] The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.19] to [0282.0.0.19]** for the disclosure of the paragraphs [0278.0.0.19] to [0282.0.0.19] see paragraphs [0278.0.0.0] to [0282.0.0.0] above.

**[0283.0.19.19]** Moreover, native polypeptide conferring the increase of the respective fine chemical in an organism or part thereof can be isolated from cells, for example using the antibody of the present invention as described below, e.g. an antibody against a protein as indicated in Table II, application no. 19, column 3, or an antibody against a polypeptide as indicated in Table II, application no. 19, columns 5 or 7, which can be produced by standard techniques utilizing the polypeptid of the present invention or fragment thereof. Preferred are monoclonal antibodies.

[0284.0.0.19] for the disclosure of this paragraph see [0284.0.0.0] above.

**[0285.0.19.19]** In one embodiment, the present invention relates to a polypeptide having a sequence as indicated in Table II, application no. 19, columns 5 or 7, or as coded by a nucleic acid molecule as indicated in Table I, application no. 19, columns 5 or 7, or functional homologues thereof.

[0286.0.19.19] In one advantageous embodiment, in the method of the present invention the activity of a polypeptide is increased which comprises or consists of a consensus sequence as indicated in Table IV, application no. 19, column 7, and in one another embodiment, the present invention relates to a polypeptide comprising or consisting of a consensus sequence as indicated in Table IV, application no. 19, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid.

**[0287.0.0.19] to [0290.0.0.19]** for the disclosure of the paragraphs [0287.0.0.19] to [0290.0.0.19] see paragraphs [0287.0.0.0] to [0290.0.0.0] above.

[0291.0.19.19] In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II, application no. 19, columns 5 or 7, by one or more amino acids. In one embodiment, polypeptide distinguishes form a sequence as indicated in Table II, application no. 19, columns 5 or 7, by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 19, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II, application no. 19, columns 5 or 7,.

[0292.0.0.19] for the disclosure of this paragraph see [0292.0.0.0] above.

**[0293.0.19.19]** In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II, application no. 19, columns 5 or 7, by one or more amino acids. In an other embodiment, said polypeptide of the invention does not consist of the sequence as indicated in Table II, application no. 19, columns 5 or 7,.
In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 19, columns 5 or 7.

**[0294.0.19.19]** In one embodiment, the present invention relates to a polypeptide having an activity of a protein as indicated in Table II, application no. 19, column 3, which distinguishes over a sequence as indicated in Table II, application no. 19, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

[0295.0.0.19] to [0297.0.0.19] for the disclosure of the paragraphs [0295.0.0.19] to [0297.0.0.19] see paragraphs [0295.0.0.0] to [0297.0.0.0] above.

**[00297.1.0.19]** Non-polypeptide of the invention-chemicals are e.g. polypeptides having not the activity and/or the amino acid sequence of a polypeptide indicated in Table II, application no. 19, columns 3, 5 or 7.

**[0298.0.19.19]** A polypeptide of the invention can participate in the process of the present invention. The polypeptide or a portion thereof comprises preferably an amino acid sequence which is sufficiently homologous to an amino acid sequence as indicated in Table II, application no. 19, columns 5 or 7. The portion of the protein is preferably a biologically active portion as described herein. Preferably, the polypeptide used in the process of the invention has an amino acid sequence identical to a sequence as indicated in Table II, application no. 19, columns 5 or 7.

**[0299.0.19.19]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, application no. 19, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 19, columns 5 or 7, or which is homologous thereto, as defined above.

**[0300.0.19.19]** In one embodiment, the present invention relates to a polypeptide having an activity of a protein as indicated in Table II, application no. 19, column 3, which distinguishes over a sequence as indicated in Table II, application no. 19, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

[0301.0.0.19] for the disclosure of this paragraph see [0301.0.0.0] above.

**[0302.0.19.19]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 19, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

[0303.0.0.19] for the disclosure of this paragraph see [0303.0.0.0] above.

**[0304.0.19.19]** Manipulation of the nucleic acid molecule of the invention may result in the production of a protein having essentially the activity of the polypeptides as indicated in Table II, application no. 19, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.19] to [0308.0.0.19]** for the disclosure of the paragraphs [0305.0.0.19] to [0308.0.0.19] see paragraphs [0305.0.0.0 to [0308.0.0.0] above.

**[0309.0.19.19]** In one embodiment, a reference to a protein (= polypeptide) of the invention or as indicated in Table II, application no. 19, columns 5 or 7, refers to a polypeptide having an amino acid sequence corresponding to the polypeptide of the invention or used in the process of the invention, whereas an "other polypeptide" not being indicated in Table II, application no. 19, columns 5 or 7, refers to a polypeptide having an amino acid sequence corresponding to a protein which is not substantially homologous to a polypeptide of the invention, preferably which is not substantially homologous to a polypeptide as indicated in Table II, application no. 19, columns 5 or 7, e.g., a protein which does not confer the activity described herein or annotated or known for as indicated in Table II, application no. 19, column 3, and which is derived from the same or a different organism. In one embodiment, an "other polypeptide" not being indicated in Table II, application no. 19, columns 5 or 7, does not confer an increase of the respective fine chemical in an organism or part thereof.

**[0310.0.0.19] to [0334.0.0.19]** for the disclosure of the paragraphs [0310.0.0.19] to [0334.0.0.19] see paragraphs [0310.0.0.0] to [0334.0.0.0] above.

[0335.0.19.19] The dsRNAi method has proved to be particularly effective and advantageous for reducing the expression of a nucleic acid sequences as indicated in Table I, application no. 19, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, application no. 19, columns 5 or 7, and/or homologs thereof. In a double-stranded RNA molecule for reducing the expression of aprotein encoded by a nucleic acid sequence as indicated in Table I, application no. 19, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.19] to [0342.0.0.19]** for the disclosure of the paragraphs [0336.0.0.19] to [0342.0.0.19] see paragraphs [0336.0.0.0] to [0342.0.0.0] above.

[0343.0.19.19] As has already been described, 100 % sequence identity between the dsRNA and a gene transcript of a nucleic acid sequence as indicated in Table I, application no. 19, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 19, columns 5 or 7, or homologs thereof of the one organism, may be used to suppress the corresponding expression in another organism.

**[0344.0.0.19] to [0361.0.0.19]** for the disclosure of the paragraphs [0344.0.0.19] to [0361.0.0.19] see paragraphs [0344.0.0.0] to [0361.0.0.0] above.

**[0362.0.19.19]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 19, columns 5 or 7, e.g. encoding a polypeptide having protein activity, as indicated in Table II, application no. 19, columns 3. Due to the abovementioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, application no. 19, columns 5 or 7, means herein that due to modulation or manipulation of the genome, an activity as annotated for a polypeptide as indicated in Table II, application no. 19, column 3, e.g. having a sequence as indicated in Table II, application no. 19, columns 5 or 7, is increased in a cell or an organism or a part thereof. Examples are described above in context with the process of the invention.

[0363.0.0.19] for the disclosure of this paragraph see [0363.0.0.0] above.

[0364.0.19.19] A naturally occurring expression cassette - for example the naturally occurring combination of a promoter of a gene encoding a polypeptide of the invention as indicated in Table II, application no. 19, column 3, with the corresponding protein-encoding sequence as indicated in Table I, application no. 19, column 5, becomes a transgenic expression cassette when it is modified by non-natural, synthetic "artificial" methods such as, for example, mutagenization. Such methods have been described (US 5,565,350; WO 00/15815; also see above).

**[0365.0.0.19] to [0373.0.0.19]** for the disclosure of the paragraphs [0365.0.0.19], to [0373.0.0.19] see paragraphs [0365.0.0.0] to [0373.0.0.0] above.

[0374.0.19.19] Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. sinapic acid , in particular the respective fine chemical, produced in the process according to the invention may, however, also be isolated from the plant in the form of their free sinapic acid , in particular the free respective fine chemical, or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.19] and [0376.0.0.19]** for the disclosure of the paragraphs [0375.0.0.19] and [0376.0.0.19] see paragraphs [0375.0.0.0] and [0376.0.0.0] above.

[0377.0.19.19] Accordingly, the present invention relates also to a process whereby the produced sinapic acid is isolated.

[0378.0.19.19] In this manner, more than 50% by weight, advantageously more than 60% by weight, preferably more than 70% by weight, especially preferably more than 80% by weight, very especially preferably more than 90% by weight, of the sinapic acid produced in the process can be isolated. The resulting sinapic acid can, if appropriate, subsequently be further purified, if desired mixed with other active ingredients such as vitamins, amino acids, carbohydrates, antibiotics and the like, and, if appropriate, formulated.

[0379.0.19.19] In one embodiment, sinapic acid is a mixture of the respective fine chemicals.

[0380.0.19.19] The sinapic acid obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the sinapic acid composition produced or the respective fine chemical produced if desired and formulating the product with a pharmaceutical acceptable carrier or formulating the product in a form acceptable for an application in agriculture. A further embodiment according to the invention is the use of the sinapic acid produced in the process or of the transgenic organisms in animal feeds, foodstuffs, medicines, food supplements, cosmetics or pharmaceuticals or for the production of sinapic acid e.g. after isolation of the respective fine chemical or without, e.g. in situ, e.g in the organism used for the process for the production of the respective fine chemical.

**[0381.0.0.19] to [0384.0.0.19]** for the disclosure of the paragraphs [0381.0.0.19], to [0384.0.0.19] see paragraphs [0381.0.0.0] to [0384.0.0.0] above.

[0385.0.19.19] The fermentation broths obtained in this way, containing in particular sinapic acid in mixtures with other organic acids, aminoacids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

[0386.0.19.19] Accordingly, it is possible to purify the sinapic acid produced according to the invention further. For this purpose, the product-containing composition is subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.19] to [0392.0.0.19]** for the disclosure of the paragraphs [0387.0.0.19] to [0392.0.0.19] see paragraphs [0387.0.0.0] to [0392.0.0.0] above.

[0393.0.19.19] In one embodiment, the present invention relates to a method for the identification of a gene product conferring an increase in the fine chemical production in a cell, comprising the following steps:
a) contacting-, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
b) identifying the nucleic acid molecules, which hybridize under relaxed stringent conditions with the nucleic acid molecule of the present invention in particular to the nucleic acid molecule sequence as indicated in Table I, application no. 19, columns 5 or 7, and, optionally, isolating the full length cDNA clone or complete genomic clone;
c) introducing the candidate nucleic acid molecules in host cells, preferably in a plant cell or a microorganism, appropriate for producing the fine chemical;
d) expressing the identified nucleic acid molecules in the host cells;
e) assaying the the fine chemical level in the host cells; and
f) identifying the nucleic acid molecule and its gene product which expression confers an increase in the the fine chemical level in the host cell after expression compared to the wild type.

**[0394.0.0.19] to [0552.0.0.19]** for the disclosure of the paragraphs [0394.0.0.19] to [0552.0.0.19] see paragraphs [0394.0.0.7] to [0552.0.0.7] above.

**[0553.0.19.19]** A process for the production of sinapic acid resp., which comprises
(a) increasing or generating the activity of a protein as indicated in Table II, application no. 19, columns 5 or 7, or a functional equivalent thereof in a non-human organism, or in one or more parts thereof; and
(b) growing the organism under conditions which permit the production of sinapic acid resp. in said organism.
A process for the production of sinapic acid resp., comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 19, columns 5 or 7, , or a fragment thereof, which confers an increase in the amount of the fine chemical as indicated in table II, column 6, e.g of sinapic acid resp., in an organism or a part thereof;
b) nucleic acid molecule comprising of a nucleic acid molecule as indicated in Table I, application no. 19, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of sinapic acid resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of sinapic acid resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of sinapic acid resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 19, column 7, and conferring an increase in the amount of the fine chemical as indicated in table II, column 6, e.g of sinapic acid resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of sinapic acid resp., in an organism or a part thereof;
h) nucleic acid molecule encoding a polypeptide comprising a consensus sequence as indicated in Table IV, application no. 19, column 7, and conferring an increase in the amount of the fine chemical as indicated in table II, column 6, e.g of sinapic acid resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical as indicated in table II, column 6, e.g of sinapic acid resp., in an organism or a part thereof.
or comprising a sequence which is complementary thereto.
The process as mentioned above, comprising recovering of the free or bound sinapic acid resp..
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound sinapic acid resp., produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 19, columns 5 or 7, or a fragment thereof, which confers an increase in the amount of the fine chemical as indicated in table II, column 6, e.g of sinapic acid resp., in an organism or a part thereof;
b) nucleic acid molecule comprising of a nucleic acid molecule as indicated in Table I, application no. 19, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of sinapic acid resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of sinapic acid resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of sinapic acid resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 19, column 7, and conferring an increase in the amount of the fine chemical as indicated in table II, column 6, e.g of sinapic acid resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of sinapic acid resp., in an organism or a part thereof;
h) nucleic acid molecule encoding a polypeptide comprising a consensus sequence as indicated in Table IV, application no. 19, column 7, and conferring an increase in the amount of the fine chemical as indicated in table II, column 6, e.g of sinapic acid resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemiccal as indicated in table II, column 6, e.g of sinapic acid resp., in an organism or a part thereof.
whereby the nucleic acid molecule distinguishes over the sequence as indicated in Table I, application no. 19, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 19, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of sinapic acid resp., in an organism or a part thereof comprising:
(a) contacting cells, tissues , plants or microorganisms which express the a polypeptide encoded by the nucleic acid molecule as mentioned above conferring an increase in the amount of sinapic acid resp., in an organism or a part thereof with a candidate compound or a sample comprising a plurality of compounds under conditions which permit the expression the polypeptide;
(b) assaying the sinapic acid resp., level or the polypeptide expression level in the cell, tissue, plant or microorganism or the media the cell, tissue, plant or microorganisms is cultured or maintained in; and
(c) identifying a agonist or antagonist by comparing the measured sinapic acid resp., level or polypeptide expression level with a standard sinapic acid resp., or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased sinapic acid resp., production in a plant or microorganism, comprising the steps:
(a) culturing a plant cell or tissue or microorganism or maintaining a plant expressing the polypeptide encoded by the nucleic acid molecule as mentioned above conferring an increase in the amount of sinapic acid resp., in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of sinapic acid rersp., in an organism or a part thereof;
(b) identifying if the compound is an effective agonist by detecting the presence or absence or increase of a signal produced by said readout system.
A method for the identification of a gene product conferring an increase in sinapic acid resp., production in a cell, comprising the following steps:
(a) contacting the nucleic acid molecules of a sample, which can contain a candidate gene encoding a gene product conferring an increase in sinapic acid resp., after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing sinapic acid resp.;
(d) expressing the identified nucleic acid molecules in the host cells;
(e) assaying the sinapic acid rersp., level in the host cells; and
(f) identifying nucleic acid molecule and its gene product which expression confers an increase in the sinapic acid resp., level in the host cell in the host cell after expression compared to the wild type.
A method for the identification of a gene product conferring an increase in sinapic acid resp., production in a cell, comprising the following steps:
(a) identifiying in a data bank nucleic acid molecules of an organism; which can contain a candidate gene encoding a gene product conferring an increase in the sinapic acid resp., amount or level in an organism or a part thereof after expression, and which are at least 20% homolog to the nucleic acid molecule as mentioned above;
(b) introducing the candidate nucleic acid molecules in host cells appropriate for producing sinapic acid resp.,;
(c) expressing the identified nucleic acid molecules in the host cells;
(d) assaying the sinapic acid resp., level in the host cells; and
(e) identifying nucleic acid molecule and its gene product which expression confers an increase in the sinapic acid resp., level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of sinapic acid resp., after expression.
Use of the polypeptide as mentioned above or the nucleic acid construct as mentioned above or the gene product identified according to the method as mentioned above for identifying compounds capable of conferring a modulation of sinapic acid resp., levels in an organism.
Agrochemical, pharmaceutical, food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
The method as mentioned above, the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above, wherein the fine chemical is sinapic acid.

### New genes for a process for the production of fine chemicals

### Description

[0000.0.0.20] In a further embodiment, the present invention relates to a further process for the production of fine chemicals as defined below and the corresponding embodiments as described herein as follows.

**[0001.0.0.20] see [0001.0.0.0]**

**[0002.0.20.20]** Plants produce very long chain fatty acids such as melissic acid (C30:0).
Very long-chain fatty acids (VLCFAs) are synthesized by a membrane-bound fatty acid elongation complex (elongase, FAE) using acyl-CoA substrates. The first reaction of elongation involves condensation of malonyl-CoA with a long chain substrate producing a β-ketoacyl-CoA. Subsequent reactions are reduction of β-hydroxyacyl-CoA, dehydration to an enoyl-CoA, followed by a second reduction to form the elongated acyl-CoA. The β-ketoacyl-CoA synthase (KCS) catalyzing the condensation reaction plays a key role in determining the chain length of fatty acid products found in seed oils and is the rate-limiting enzyme for seed VLCFA production (Lassner et al., Plant Cell, 8(1996), 281-292).
The elongation process can be repeated to yield members that are 20, 22, and 24 carbons long. Although such very long chain fatty acids are minor components of the lipid membranes of the body, they undoubtedly perform valuable functions, apparently helping to stabilize membranes, especially those in peripheral nerve cells.
Melissic acid (C30:0) (triacontanoic acid) is a component of beeswax.
Beeswax (cera alba) is obtained from the product excreted by certain glands of the honeybee from which the honeycomb is made. It is freed of solid impurities by melting and centrifugation (cera flava). Finally, it is bleached completely white (cera alba). Beeswax consists of 10-15 percent paraffin carbohydrates, 35-37 percent esters of C16 to C36 fatty acids and about 15 percent cerotic acid, melissic acid and their homologues. Beeswax is used as a thickener and a humectant in the manufacture of ointments, creams, lipsticks and other cosmetics and skincare products as an emulsifier, emollient, moisturizer and film former.
Beeswax is also used for the production of candles.
Wax is a general term used to refer to the mixture of long-chain apolar lipids forming a protective coating (cutin in the cuticle) on plant leaves and fruits but also in animals (wax of honeybee, cuticular lipids of insects, spermaceti of the sperm whale, skin lipids, uropygial glands of birds, depot fat of planktonic crustacea), algae, fungi and bacteria.
Many of the waxes found in nature have commercial uses in the lubricant, food and cosmetic industry. Jojoba oil has long been suggested as a putative resource of wax, since this desert shrub is unusual in its capacity to produce waxes rather than triacylglycerols (TAG) as seed storage lipids. These waxes are esters of very-long-chain-fatty acids and fatty alcohols (Miwa , 1971, J Am Oil Chem Soc 48, 259-264). As the production cost for jojoba wax, which is primarily used for cosmetic applications, is high, there is a need to engineer crop plants to produce high level of wax esters in its seed oil.
Plant aerial surfaces are covered by epicuticular waxes, complex mixtures of very long (C₂₀-C₃₄) fatty acids, alkanes, aldehydes, ketones and esters. In addition to repelling atmospheric water they prevent dessication and are therefore an important determinant of drought resistance (Riederer and Schreiber, 2001, J. Exp. Bot 52, 2023-2032). Beside abiotic stress resistance the wax layer is part of the plant defense against biotic stressor, especially insects as for example described by Marcell and Beattie, 2002, Mol Plant Microbe Interact. 15(12), 1236-44. Furthermore they provide stability to pollen grains, thus influencing fertility and productivity.
Very-long-chain fatty acids (VLCFAs), consisting of more than 18 carbon atoms like melissic acid, are essential components for the vitality of higher plants. The key enzyme of VLCFA biosynthesis, the extraplastidary fatty acid elongase, is shown for to be the primary target site of chloroacetamide herbicides. With an analysis of the fatty acid composition and the metabolism of 14C-labelled precursors (sterate, malonate, acetate), the reduction of VLCFAs was determined in vivo. The inhibition of the recombinant protein substantiates the first and rate-limiting step of VLCFA biosynthesis, the condensation of acyl-CoA with malonyl-CoA to β-ketoacyl-CoA, to be the primary target site of chloroacetamides (150 = 10-100 nM). The concentration of VLCFAs within the untreated cell is low, the very-long-chain compounds are found mainly in plasma membrane lipids and epicuticular waxes. A shift of fatty acids towards shorter chain length or even the complete depletion of very-long-chain components is the consequence of the inhibition of VLCFA biosynthesis. Especially the loss of plasma membrane VLCFAs is involved in phytotoxic effects of chloroacetamides such as the inhibition of membrane biogenesis and mitosis (Matthes, B., http://www.ub.uni-konstanz.de/kops/volltexte/2001/661/).
Increased wax production in transgenic plants has for example been reported by Broun et al., 2004, Proc Natl. Acad. Sci, 101, 4706-4711. The authors overexpressed the transcriptional activator WIN1 in Arabidopsis, leading to increased wax load on arial organs. As this resulted in a complex change in the wax profile and the transgenic overexpressors had characteristic alterations in growth and development (Broun et al., 2004, Proc Natl. Acad. Sci, 101, 4706-4711) there is still a need for a more controlled increased production of defined VLCFAs.
Very long chain fatty alcohols obtained from plant waxes and beeswax have also been reported to lower plasma cholesterol in humans and existing data support the hypothesis that VLCFA exert regulatory roles in cholesterol metabolism in the peroxisome and also alter LDL uptake and metabolism (discussed in Hargrove et al., 2004, Exp Biol Med (Maywood), 229(3): 215-26).
Due to these interesting physiological roles and the nutritional, cosmetic and agrobiotechnological potential of melissic acid (C30:0) there is a need to identify the genes of enzymes and other proteins involved in melissic acid metabolism, and to generate mutants or transgenic plant lines with which to modify the melissic acid content in plants.

**[0003.0.0.20] %**

**[0004.0.0.20] %**

**[0005.0.0.20] %**

**[0006.0.0.20] %**

**[0007.0.0.20] %**

[0008.0.20.20] One way to increase the productive capacity of biosynthesis is to apply recombinant DNA technology. Thus, it would be desirable to produce melissic acid in plants. That type of production permits control over quality, quantity and selection of the most suitable and efficient producer organisms. The latter is especially important for commercial production economics and therefore availability to consumers. In addition it is desirable to produce melissic acid in plants in order to increase plant productivity and resistance against biotic and abiotic stress as discussed before.
Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Argobacterium tumifaciens*-mediated transformation of tobacco plants using a vector that contained a ketolase-encoding gene from *H. pluvialis* denominated *crtO* along with the Pds gene from tomato as the promoter and to encode a leader sequence. Those results indicated that about 75 percent of the carotenoids found in the flower of the transformed plant contained a keto group.

**[0009.0.20.20]** Thus, it would be advantageous if an algae, plant or other microorganism were available which produce large amounts melissic acid. The invention discussed hereinafter relates in some embodiments to such transformed prokaryotic or eukaryotic microorganisms.
It would also be advantageous if plants were available whose roots, leaves, stem, fruits or flowers produced large amounts of melissic acid. The invention discussed hereinafter relates in some embodiments to such transformed plants.

[0010.0.20.20] Therefore improving the quality of foodstuffs and animal feeds is an important task of the food-and-feed industry. This is necessary since, for example melissic acid, as mentioned above, which occur in plants and some microorganisms are limited with regard to the supply of mammals. Especially advantageous for the quality of foodstuffs and animal feeds is as balanced as possible a specific melissic acid profile in the diet since an excess of melissic acid above a specific concentration in the food has a positive effect. A further increase in quality is only possible via addition of further melissic acid, which are limiting.

[0011.0.20.20] To ensure a high quality of foods and animal feeds, it is therefore necessary to add melissic acid in a balanced manner to suit the organism.

[0012.0.20.20] Accordingly, there is still a great demand for new and more suitable genes which encode enzymes or other proteins which participate in the biosynthesis of melissic acid and make it possible to produce them specifically on an industrial scale without unwanted byproducts forming. In the selection of genes for biosynthesis two characteristics above all are particularly important. On the one hand, there is as ever a need for improved processes for obtaining the highest possible contents of melissic acid; on the other hand as less as possible byproducts should be produced in the production process.

**[0013.0.0.20] see [0013.0.0.0]**

[0014.0.20.20] Accordingly, in a first embodiment, the invention relates to a process for the production of a fine chemical, whereby the fine chemical is a melissic acid. Accordingly, in the present invention, the term "the fine chemical" as used herein relates to a melissic acid. Further, the term "the fine chemicals" as used herein also relates to fine chemicals comprising melissic acid.
In one embodiment, the term "the fine chemical" means melissic acid. Throughout the specification the term "the fine chemical" means melissic acid, its salts, ester, thioester or in free form or bound to other compounds such sugars or sugarpolymers, like glucoside, e.g. diglucoside.

**[0016.0.20.20]**Accordingly, the present invention relates to a process for the production of the respective fine chemical comprising
(a) increasing or generating the activity of one or more
   of a protein as shown in table II, application no. 20, column 3 encoded by the nucleic acid sequences as shown in table I, application no. 20, column 5, in a non-human organism or in one or more parts thereof or
(b) growing the organism under conditions which permit the production of the fine chemical, thus melissic acid of the invention or fine chemicals comprising melissic acid of the invention, in said organism or in the culture medium surrounding the organism.

[0016.1.20.20] Accordingly, the term "the fine chemical" means in one embodiment "melissic acid" in relation to all sequences listed in Tables I to IV, line 43 or homologs thereof.

**[0017.0.0.20] to [0019.0.0.20]: see [0017.0.0.0] to [0019.0.0.0]**

[0020.0.20.20] Surprisingly it was found, that the transgenic expression of the Brassica napus protein as indicated in Table II, column 5, line 43 in a plant conferred an increase in melissic acid content of the transformed plants. Thus, in one embodiment, said protein or its homologs are used for the production of melissic acid.

**[0021.0.0.20]** see **[0021.0.0.0]**

[0022.0.20.20] The sequence of YDR513W from Saccharomyces cerevisiae has been published in Jacq,C. et al., Nature 387 (6632 Suppl), 75-78 (1997) and its activity is being defined as a protein having glutathione reductase activity. Accordingly, in one embodiment, the process of the present invention comprises the use of a protein YDR513W or its homolog, e.g. as shown herein, for the production of the respective fine chemical, in particular for increasing the amount of melissic acid, preferably in free or bound form in an organism or a part thereof, as mentioned. In one embodiment, in the process of the present invention the activity of the protein YDR513W is increased.

**[0022.1.0.20] to [0023.0.20.20] see [0022.1.0.0] to [0023.0.0.0]**

**[0023.1.20.20]** Homologs of the polypeptide disclosed in table II, application no. 20, column 5, may have the activity of a protein as disclosed in table I or II, application no. 20, column 3 and may be the polypetides encoded by the nucleic acid molecules indicated in table I , application no. 20, column 7, resp., or may be the polypeptides indicated in table II, application no. 20, column 7, resp.

**[0024.0.0.20] see [0024.0.0.0]**

**[0025.0.20.20]** In accordance with the invention, a protein or polypeptide has the "activity of an protein as shown in table II, application no. 20, column 3" if its de *novo* activity, or its increased expression directly or indirectly leads to an increased in the level of the fine chemical indicated in the respective line of table II, application no. 20, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 20, column 3, or which has at least 10% of the original enzymatic or biological activity, preferably 20%, particularly preferably 30%, most particularly preferably 40% in comparison to a protein as shown in the respective line of table II, application no. 20, column 3 of a E. coli or Saccharomyces cerevisae, respectively, protein as mentioned in table I to IV, column 3 respectively and as disclosed in paragraph [0022] of the respective invention.

**[0025.1.0.20] see [0025.1.0.0]**

**[0026.0.0.20] to [0033.0.0.20]: see [0026.0.0.0] to [0033.0.0.0]**

[0034.0.20.20]Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 20, column 3 its biochemical or genetical causes and the increased amount of the respective fine chemical.

**[0035.0.0.20] to [0038.0.0.20]: see [0035.0.0.0] to [0038.0.0.0]**

**[0039.0.0.20]: see [0039.0.0.0]**

**[0040.0.0.20] to [0044.0.0.20]: see [0040.0.0.0] to [0044.0.0.0]**

**[0045.0.20.20]** In one embodiment, the activity of the protein. as indicated in Table II, columns 5 or 7, application no. 20, is increased conferring an increase of the respective fine chemical, indicated in Table II, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more.

**[0046.0.0.20]** In one embodiment, the activity of the protein as indicated in Table II, columns 5 or 7, application no. 20, is increased conferring an increase of the respective fine chemical, indicated in Table II, column 6 of the respective line confers an increase of the respective fine chemical and of further melissic acid or their precursors.

**[0047.0.0.20] to [0048.0.0.20]: see [0047.0.0.0] to [0048.0.0.0]**

**[0049.0.20.20]** A protein having an activity conferring an increase in the amount or level of the fine chemical, has in one embodiment the structure of the polypeptide described herein, in particular of the polypeptides comprising the consensus sequence shown in table IV, application no. 20, column 7 or of the polypeptide as shown in the amino acid sequences as disclosed in table II, application no. 20, columns 5 and 7 or the functional homologues thereof as described herein, or is encoded by the nucleic acid molecule characterized herein or the nucleic acid molecule according to the invention, for example by the nucleic acid molecule as shown in table I, application no. 20, columns 5 and 7 or its herein described functional homologues and has the herein mentioned activity.

[0050.0.20.20] For the purposes of the present invention, the term "the respective fine chemical" also encompass the corresponding salts, such as, for example, the potassium or sodium salts of melissic acid, resp., or their ester, or glucoside thereof, e.g the diglucoside thereof.

[0051.0.20.20] Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising melissic acid. Depending on the choice of the organism used for the process according to the present invention, for example a microorganism or a plant, compositions or mixtures of melissic acid can be produced.

**[0052.0.0.20] see [0052.0.0.0]**

**[0053.0.20.20]** In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 20, columns 5 and 7 or its homologs activity having herein-mentioned melissic acid of the invention increasing activity; and/or
b) stabilizing a mRNA conferring the increased expression of a protein encoded by the nucleic acid molecule of the invention as shown in table I, application no. 20, columns 5 and 7, e.g. a nucleic acid sequence encoding a polypeptide having the activity of a protein as indicated in table II, application no. 20, columns 5 and 7 or its homologs activity or of a mRNA encoding the polypeptide of the present invention having herein-mentioned melissic acid of the invention increasing activity; and/or
c) increasing the specific activity of a protein conferring the increased expression of a protein encoded by the nucleic acid molecule of the invention or of the polypeptide of the present invention having herein-mentioned wax component increasing activity, e.g. of a polypeptide having the activity of a protein as indicated in table II, application no. 20, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned melissic acid of the invention increasing activity, e.g. of a polypeptide having the activity of a protein as indicated in table II, application no. 20, columns 5 and 7 or its homologs activity; and/or
e) stimulating activity of a protein conferring the increased expression of a protein encoded by the nucleic acid molecule of the present invention or a polypeptide of the present invention having herein-mentioned melissic acid of the invention increasing activity, e.g. of a polypeptide having the activity of a protein as indicated in table II, application no. 20, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned melissic acid of the invention increasing activity, e.g. of a polypeptide having the activity of a protein as indicated in table II, application no. 20, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned melissic acid of the invention increasing activity, e.g. of a polypeptide having the activity of a protein as indicated in table II, application no. 20, columns 5 and 7 or its homologs activity; and/or
h) increasing the expression of the endogenous gene encoding the polypeptide of the invention, e.g. a polypeptide having the activity of a protein as indicated in table II, application no. 20, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with especially high activity of the proteins of the invention from natural or from mutagenized resources and breeding them into the target organisms, eg the elite crops.

[0054.0.20.20] Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 20 in Table I to IV, resp., after increasing the expression or activity of the encoded polypeptide or having the activity of a polypeptide having an activity as the protein as shown in table II, application no. 20, column 3 or its homologs.

**[0055.0.0.20] to [0067.0.0.20]: see [0055.0.0.0] to [0067.0.0.0]**

[0068.0.20.20] The mutation is introduced in such a way that the production of melissic acid is not adversely affected.

**[0069.0.0.20] see [0069.0.0.0]**

[0070.0.20.20] Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create de *novo* an advantageous, preferably novel metabolite composition in the organism, e.g. an advantageous composition of melissic acid or their biochemical derivatives , e.g. comprising a higher content of (from a viewpoint of nutrional physiology limited) melissic acid or their derivatives.

**[0071.0.0.20] see [0071.0.0.0]**

**[0072.0.0.20] %**

[0073.0.20.20] Accordingly, in one embodiment, the process according to the invention relates to a process, which comprises:
a) providing a non-human organism, preferably a microorganism, a non-human animal, a plant or animal cell, a plant or animal tissue or a plant;
b) increasing an activity of a polypeptide of the invention or a homolog thereof, e.g. as indicated in Table II, application no. 20, columns 5 or 7, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
d) if desired, recovering, optionally isolating, the free and/or bound respective fine chemical synthesized by the organism, the microorganism, the non-human animal, the plant or animal cell, the plant or animal tissue or the plant.

[0074.0.20.20] The organism, in particular the microorganism, non-human animal, the plant or animal cell, the plant or animal tissue or the plant is advantageously grown in such a way that it is not only possible to recover, if desired isolate the free or bound respective fine chemical.

**[0075.0.0.20] to [0077.0.0.20]: see [0075.0.0.0] to [0077.0.0.0]**

[0078.0.20.20] The organism such as microorganisms or plants or the recovered, and if desired isolated, respective fine chemical can then be processed further directly into foodstuffs or animal feeds or for other applications. The fermentation broth, fermentation products, plants or plant products can be purified with methods known to the person skilled in the art. Products of these different work-up procedures are melissic acid or comprising compositions of melissic acid still comprising fermentation broth, plant particles and cell components in different amounts, advantageously in the range of from 0 to 99% by weight, preferably below 80% by weight, especially preferably below 50% by weight.

**[0079.0.0.20] to [0084.0.0.20]: see [0079.0.0.0] to [0084.0.0.0]**

**[0084.0.0.20] %**

[0085.0.20.20] With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, application no. 20, columns 5 or 7, or a derivative thereof, or
b) a genetic regulatory element, for example a promoter, which is functionally linked to the nucleic acid sequence as indicated in Table I, application no. 20, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.20] to [0087.0.0.20]: see [0086.0.0.0] to [0087.0.0.0]**

[0088.0.20.20] In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned fine chemicals or the plants are more resistant to biotic and abiotic stress and the yield is increased.

**[0088.1.0.20] see [0088.1.0.0]**

**[0089.0.0.20] to [0090.0.0.20]: see [0089.0.0.0] to [0090.0.0.0]**

**[0091.0.0.20] see [0091.0.0.0]**

**[0092.0.0.20] to [0094.0.0.20]: see [0092.0.0.0] to [0094.0.0.0]**

[0095.0.20.20] It may be advantageous to increase the pool of melissic acid in the transgenic organisms by the process according to the invention in order to isolate high amounts of the pure respective fine chemical and/or to obtain increased resistance against biotic and abiotic stresses and to obtain higher yield.

[0096.0.20.20] In another preferred embodiment of the invention a combination of the increased expression of the nucleic acid sequence or the protein of the invention together with the transformation of a protein or polypeptid or a compound, which functions as a sink for the desired fine chemical, for example in the organism, is useful to increase the production of the respective fine chemical.

**[0097.0.0.20] %**

[0098.0.20.20] In a preferred embodiment, the respective fine chemical is produced in accordance with the invention and, if desired, is isolated.

[0099.0.20.20] In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.20.20]** Transgenic plants which comprise the fine chemicals such as melissic acid synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because melissic acid in microorganisms are localized intracellular, their recovery essentially comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.

[0101.0.20.20] The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.20.20]** Melissic acid can for example be analyzed advantageously via HPLC, LC or GC separation and MS (masspectrometry)detection methods. The unambiguous detection for the presence of melissic acid containing products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.

[0103.0.20.20] In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, application no. 20, columns 5 or 7, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 20, columns 5 or 7,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as indicated in Table III, application no. 20, columns 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence having sequences as indicated in Table IV, application no.20, column 7, and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, application no. 20, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[0103.1.20.20]** In one embodiment, the nucleic acid molecule used in the process of the invention distinguishes over the sequence indicated in Table I, application no. 20, columns 5 or 7 by one or more nucleotides. In one embodiment, the nucleic acid molecule used in the process of the invention does not consist of the sequence shown in indicated in Table I ,application no. 20, columns 5 or 7,. In one embodiment, the nucleic acid molecule used in the process of the invention is less than 100%, 99,999%, 99,99%, 99,9% or 99% identical to a sequence indicated in Table I, application no. 20, columns 5 or 7,. In another embodiment, the nucleic acid molecule does not encode a polypeptide of a sequence indicated in Table II, application no. 20, columns 5 or 7.

**[0104.0.20.20]** In one embodiment, the nucleic acid molecule used in the process of the present invention distinguishes over the sequence indicated in Table I, application no. 20, columns 5 or 7, by one or more nucleotides. In one embodiment, the nucleic acid molecule used in the process of the invention does not consist of the sequence indicated in Table I, application no. 20, columns 5 or 7. In one embodiment, the nucleic acid molecule of the present invention is less than 100%, 99,999%, 99,99%, 99,9% or 99% identical to the sequence indicated in Table I, application no. 20, columns 5 or 7. In another embodiment, the nucleic acid molecule does not encode a polypeptide of a sequence indicated in Table II, application no. 20, columns 5 or 7.

**[0105.0.0.20] to [0107.0.0.20]: see [0105.0.0.0] to [0107.0.0.0]**

[0108.0.20.20] Nucleic acid molecules with the sequence as indicated in Table I, application no. 20, columns 5 or 7, nucleic acid molecules which are derived from an amino acid sequences as indicated in Table II, application no. 20, columns 5 or 7, or from polypeptides comprising the consensus sequence as indicated in Table IV, application no. 20, column 7, or their derivatives or homologues encoding polypeptides with the enzymatic or biological activity of an activity of a polypeptide as indicated in Table II, application no. 20, column 3, 5 or 7, e.g. conferring the increase of the respective fine chemical, meaning melissic acid, resp., after increasing its expression or activity, are advantageously increased in the process according to the invention.

[0109.0.20.20] In one embodiment, said sequences are cloned into nucleic acid constructs, either individually or in combination. These nucleic acid constructs enable an optimal synthesis of the respective fine chemicals, in particular melissic acid, produced in the process according to the invention.

**[0110.0.0.20] see [0110.0.0.0]**

**[0111.0.0.20] see [0111.0.0.0]**

[0112.0.20.20] The nucleic acid molecules used in the process according to the invention take the form of isolated nucleic acid sequences, which encode polypeptides with an activity of a polypeptide as indicated in Table II, application no. 20, column 3, or having the sequence of a polypeptide as indicated in Table II, application no. 20, columns 5 and 7, and conferring an increase in the melissic acid level.

**[0113.0.0.20] to [0114.0.0.20]: see [0113.0.0.0] to [0114.0.0.0]**

**[0115.0.0.20] see [0115.0.0.0]**

**[0116.0.0.20] to [0120.0.0.20] see [0116.0.0.0] to [0120.0.0.0]**

**[0120.1.0.20]: %**

[0121.0.20.20] However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 20, columns 5 or 7, or the functional homologues thereof as described herein, preferably conferring above-mentioned activity, i.e. conferring a melissic acid level increase after increasing the activity of the polypeptide sequences indicated in Table II, application no. 20, columns 5 or 7.

**[0122.0.0.20] to [0127.0.0.20]: see [0122.0.0.0] to [0127.0.0.0]**

[0128.0.20.20] Synthetic oligonucleotide primers for the amplification, e.g. as the pairs indicated in Table III, application no. 20, columns 7, by means of polymerase chain reaction can be generated on the basis of a sequence shown herein, for example the sequence as indicated in Table I, application no. 20, columns 5 or 7, or the sequences derived from a sequences as indicated in Table II, application no. 20, columns 5 or 7.

[0129.0.20.20] Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved regions are those, which show a very little variation in the amino acid in one particular position of several homologs from different origin. The consensus sequence indicated in Table IV, application no. 20, columns 7, is derived from such alignments.

[0130.0.20.20] Degenerated primers can then be utilized by PCR for the amplification of fragments of novel proteins having above-mentioned activity, e.g. conferring the increase of melissic acid after increasing the expression or activity the protein comprising said fragment.

**[0131.0.0.20] to [0138.0.0.20]: see [0131.0.0.0] to [0138.0.0.0]**

[0139.0.20.20] Polypeptides having above-mentioned activity, i.e. conferring an increase of the respective fine chemical level, derived from other organisms, can be encoded by other DNA sequences which hybridize to a sequences indicated in Table I, application no. 20, columns 5 or 7, under relaxed hybridization conditions and which code on expression for peptides having the respective fine chemical, in particular, of melissic acid resp., increasing activity.

**[0140.0.0.20] to [0146.0.0.20]: see [0140.0.0.0] to [0146.0.0.0]**

[0147.0.20.20] Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 20, columns 5 or 7, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

[0148.0.20.20] The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 20, columns 5 or 7 or a portion thereof and preferably has above mentioned activity, in particular, of melissic acid increasing activity after increasing the acitivity or an activity of a product of a gene encoding said sequences or their homologs.

[0149.0.20.20] The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 20, columns 5 or 7, or a portion thereof and encodes a protein having above-mentioned activity and as indicated in indicated in Table II.

[00149.1.20.20] Optionally, in one embodiment, the nucleotide sequence, which hybridises to one of the nucleotide sequences indicated in Table I, application no. 20, columns 5 or 7, preferably the nucleic acid molecule of the invention is a functional homologue or identical to a nucleic acid molecule indicated in Table I, application no. 20, columns 5 or 7, has further one or more of the activities annotated or known for a protein as indicated in Table II, application no. 20, column 3.

[0150.0.20.20] Moreover, the nucleic acid molecule of the invention can comprise only a portion of the coding region of one of the sequences indicated in Table I, application no. 20, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of melissic acid, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 20, columns 5 or 7, , an anti-sense sequence of one of the sequences, e.g., as indicated in Table I, application no. 20, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 20, column 7, will result in a fragment of a polynucleotide sequence as indicated in Table I, application no. 20, columns 5 or 7 or its gene product.

**[0151.0.0.20]: see [0151.0.0.0]**

[0152.0.20.20] The nucleic acid molecule of the invention encodes a polypeptide or portion thereof which includes an amino acid sequence which is sufficiently homologous to an amino acid sequence as indicated in Table II, application no. 20, columns 5 or 7, such that the protein or portion thereof maintains the ability to participate in the respective fine chemical production, in particular a melissic acid increasing activity as mentioned above or as described in the examples in plants or microorganisms is comprised.

[0153.0.20.20] As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 20, columns 5 or 7, such that the protein or portion thereof is able to participate in the increase of the respective fine chemical production. In one embodiment, a protein or portion thereof as indicated in Table II, application no. 20, columns 5 or 7, has for example an activity of a polypeptide indicated in Table II, application no. 20, column 3.

**[0154.0.20.20]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91 %, 92%, 93% or 94% and most preferably at least about 95%, 97%, 98%, 99% or more homologous to an entire amino acid sequence as indicated in Table II, application no. 20, columns 5 or 7, and has above-mentioned activity, e.g.conferring preferably the increase of the respective fine chemical.

**[0155.0.0.20] to [0156.0.0.20]: see [0155.0.0.0] to [0156.0.0.0]**

[0157.0.20.20] The invention further relates to nucleic acid molecules that differ from one of the nucleotide sequences as indicated in Table I, application no. 20, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, application no. 20, column 7, or as polypeptides depicted in Table II, application no. 20, columns 5 or 7, or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, application no. 20, column 7, or of the polypeptide as indicated in Table II, application no. 20, columns 5 or 7, or the functional homologues. In a still further embodiment, the nucleic acid molecule of the invention encodes a full length protein which is substantially homologous to an amino acid sequence comprising a consensus sequence as indicated in Table IV, application no. 20, column 7, or of a polypeptide as indicated in Table II, application no. 20, columns 5 or 7, or the functional homologues. However, in a preferred embodiment, the nucleic acid molecule of the present invention does not consist of a sequence as indicated in Table I, application no. 20, columns 5 or 7.

**[0158.0.0.20] to [0160.0.0.20]: see [0158.0.0.0] to [0160.0.0.0]**

**[0161.0.20.20]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 20, columns 5 or 7. The nucleic acid molecule is preferably at least 20, 30, 50, 100, 250 or more nucleotides in length.

**[0162.0.0.20] see [0162.0.0.0]**

**[0163.0.20.20]** Preferably, a nucleic acid molecule of the invention that hybridizes under stringent conditions to a sequence as indicated in Table I, application no. 20, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in an organism or a part thereof, e.g. a tissue, a cell, or a compartment of a cell, after increasing the expression or activity thereof or the activity of a protein of the invention or used in the process of the invention.

**[0164.0.0.20] see [0164.0.0.0]**

[0165.0.20.20] For example, nucleotide substitutions leading to amino acid substitutions at "non-essential" amino acid residues can be made in a sequence of the nucleic acid molecule of the invention or used in the process of the invention, e.g. as indicated in Table I, application no. 20, columns 5 or 7.

**[0166.0.0.20] to [0167.0.0.20]: see [0166.0.0.0] to [0167.0.0.0]**

[0168.0.20.20] Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 20, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 20, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 20, columns 5 or 7, more preferably at least about 70% identical to one of the sequences as indicated in Table II, application no. 20, columns 5 or 7, ,even more preferably at least about 80%, 90%, or 95% homologous to a sequence as indicated in Table II, application no. 20, columns 5 or 7, , and most preferably at least about 96%, 97%, 98%, or 99% identical to the sequence as indicated in Table II, application no. 20, columns 5 or 7.

**[0169.0.0.20] to [0172.0.0.20]: see [0169.0.0.0] to [0172.0.0.0]**

**[0173.0.20.20]**For example a sequence which has a 80% homology with sequence SEQ ID NO: 227 at the nucleic acid level is understood as meaning a sequence which, upon comparison with the sequence SEQ ID NO: 227 by the above Gap program algorithm with the above parameter set, has a 80% homology.

**[0174.0.0.20]: see [0174.0.0.0]**

**[0175.0.20.20]** For example a sequence which has a 80% homology with sequence SEQ ID NO: 228 at the protein level is understood as meaning a sequence which, upon comparison with the sequence SEQ ID NO: 228 by the above program algorithm with the above parameter set, has a 80% homology.

**[0176.0.20.20]** Functional equivalents derived from one of the polypeptides as indicated in Table II, application no. 20, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table II, application no. 20, columns 5 or 7, according to the invention and are distinguished by essentially the same properties as a polypeptide as indicated in Table II, application no. 20, columns 5 or 7.

[0177.0.20.20] Functional equivalents derived from a nucleic acid sequence as indicated in Table I, application no. 20, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptide as indicated in Table II, application no. 20, columns 5 or according to the invention and encode polypeptides having essentially the same properties as a polypeptide as indicated in Table II, application no. 20, columns 5 or 7.

**[0178.0.0.20] see [0178.0.0.0]**

**[0179.0.20.20] A** nucleic acid molecule encoding a homologue to a protein sequence as indicated in Table II, application no. 20, columns 5 or 7, can be created by introducing one or more nucleotide substitutions, additions or deletions into a nucleotide sequence of the nucleic acid molecule of the present invention, in particular as indicated in Table I, application no. 20, columns 5 or 7, such that one or more amino acid substitutions, additions or deletions are introduced into the encoded protein. Mutations can be introduced into the encoding sequences as indicated in Table I, application no. 20, columns 5 or 7, by standard techniques, such as site-directed mutagenesis and PCR-mediated mutagenesis.

**[0180.0.0.20] to [0183.0.0.20]: see [0180.0.0.0] to [0183.0.0.0]**

**[0184.0.20.20]** Homologues of the nucleic acid sequences used, with a sequence as indicated in Table I, application no. 20, columns 5 or 7, or of the nucleic acid sequences derived from a sequences as indicated in Table II, application no. 20, columns 5 or 7, comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 20, columns 5 or 7, or from the derived nucleic acid sequences, the intention being, however, that the enzyme activity or the biological activity of the resulting proteins synthesized is advantageously retained or increased.

[0185.0.20.20] In one embodiment of the present invention, the nucleic acid molecule of the invention or used in the process of the invention comprises one or more sequences as indicated in Table I, application no. 20, columns 5 or 7,. In one embodiment, it is preferred that the nucleic acid molecule comprises as little as possible other nucleotides not shown in any one of sequences as indicated in Table I, application no. 20, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 20, columns 5 or 7.

**[0186.0.20.20]** Also preferred is that one or more nucleic acid molecule(s) used in the process of the invention encodes a polypeptide comprising a sequence as indicated in Table II, application no. 20, columns 5 or 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 20, columns 5 or 7.

[0187.0.20.20] In one embodiment, a nucleic acid molecule of the invention or used in the process encodes a polypeptide comprising the sequence as indicated in Table II,, application no. 20, columns 5 or 7, comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 20, columns 5 or 7.

[0188.0.20.20] Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 20, columns 5 or 7 and is expressed under identical conditions.

[0189.0.20.20] Homologues of a sequences as indicated in Table I, application no. 20, columns 5 or 7, or of a derived sequences as indicated in Table II, application no. 20, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.20]: see [0190.0.0.0]**

[0191.0.20.20] The organisms or part thereof produce according to the herein mentioned process of the invention an increased level of free and/or bound the respective fine chemical compared to said control or selected organisms or parts thereof.

**[0192.0.0.20] to [0203.0.0.20]: see [0192.0.0.0] to [0203.0.0.0]**

[0204.0.20.20] Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 20, columns 5 or 7, or a fragment thereof conferring an increase in the amount of the respective fine chemical, in particular according to Table II, application no. 20, column 6 in an organism or a part thereof
b) nucleic acid molecule comprising, preferably at least the mature form, of a nucleic acid molecule as indicated in Table I, application no. 20, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, application no. 20, column 7, and conferring an increase in the amount of the respective fine chemical, in particular according to table II, application no. 20, column 6 in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, application no. 20, columns 5 or 7, and conferring an increase in the amount of the respective fine chemical, in particular according to table II, application no. 20, column 6 in an organism or a part thereof;
k) nucleic acid molecule encoding the amino acid sequence of a polypeptide encoding a domaine of a polypeptide as indicated in Table II, application no. 20, columns 5 or 7, and conferring an increase in the amount of the respective fine chemical, in particular accccording to table II, application no. 20, column 6 in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 20, columns 5 or 7, or a nucleic acid molecule encoding, preferably at least the mature form of, a polypeptide as indicated in Table II, application no. 20, columns 5 or 7, and conferring an increase in the amount of the respective fine chemical according to Table II, application no. 20, column 6 in an organism or a part thereof; or which encompasses a sequence which is complementary thereto;
whereby, preferably, the nucleic acid molecule according to (a) to (I) distinguishes over the sequence indicated in Table I, application no. 20,columns 5 or 7, by one or more nucleotides. In one embodiment, the nucleic acid molecule does not consist of the sequence shown and indicated in Table I, application no. 20, columns 5 or 7,
In one embodiment, the nucleic acid molecule is less than 100%, 99,999%, 99,99%, 99,9% or 99% identical to a sequence indicated in Table I, application no. 20, columns 5 or 7,.
In another embodiment, the nucleic acid molecule does not encode a polypeptide of a sequence indicated in Table II, application no. 20, columns 5 or 7,.
In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 20, columns 5 or 7,.
In a further embodiment the nucleic acid molecule does not encode a polypeptide sequence as indicated in Table II, application no. 20, columns 5 or 7,.
Accordingly, in one embodiment, the nucleic acid molecule of the differs at least in one or more residues from a nucleic acid molecule indicated in Table I, application no. 20, columns 5 or 7,.
Accordingly, in one embodiment, the nucleic acid molecule of the present invention encodes a polypeptide, which differs at least in one or more amino acids from a polypeptide indicated in Table II, application no. 20, columns 5 or 7,.
In another embodiment, a nucleic acid molecule indicated in Table I, application no. 20, columns 5 or 7,.
Accordingly, in one embodiment, the protein encoded by a sequences of a nucleic acid accoriding to (a) to (I) does not consist of a sequence as indicated in Table II, application no. 20, columns 5 or 7,.
In a further embodiment, the protein of the present invention is at least 30 %, 40 %, 50%, or 60% identical to a protein sequence indicated in Table II, application no. 20, columns 5 or 7, and less than 100%, preferably less than 99,999%, 99,99% or 99,9%, more preferably less than 99%, 985, 97%, 96% or 95% identical to a sequence as indicated in Table I, columns 5 or 7.

**[0205.0.0.20] to [0206.0.0.20]: see [0205.0.0.0] to [0206.0.0.0]**

[0207.0.20.20] As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.20] to [0226.0.0.20]: see [0208.0.0.0] to [0226.0.0.0]**

**[0227.0.20.20]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table I, application no. 20, columns 5 or 7, or its derivatives, it is advantageous to express and/or mutate further genes in the organisms. Especially advantageously, additionally at least one further gene of the acetyl-CoA or malonyl-CoA metabolic pathway or a polypeptide having a very long chain fatty acid acyl (VLCFA) CoA synthase activity, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table I, application no. 20, columns 5 or 7, with genes which generally support or enhance to growth or yield of the target organismen, for example genes which lead to faster growth rate of microorganisms or genes which produces stress-, pathogen, or herbicide resistant plants.

**[0228.0.20.20] %**

[0229.0.20.20] Further advantageous nucleic acid sequences which can be expressed in combination with the sequences indicated in Table I, application no. 20, columns 5 or 7, used in the process and/or the abovementioned biosynthesis genes are the sequences encoding further genes of the fatty acid pathway, such as acetyl-CoA or malonyl-CoA or a polypeptide having a very long chain fatty acid acyl (VLCFA) CoA synthase activity. These genes can lead to an increased synthesis of the VLCFAs.

**[0230.0.0.20] see [230.0.0.0]**

[0231.0.20.20] In a further advantageous embodiment of the process of the invention, the organisms used in the process are those in which simultaneously a melissic acid degrading protein is attenuated, in particular by reducing the rate of expression of the corresponding gene. A person skilled in the art knows for example, that the inhibition or repression of a melissic acid degrading enzyme will result in an increased accumulation of melissic acid in plants.

**[0232.0.0.20] to [0276.0.0.20]: see [0232.0.0.0] to [0276.0.0.0]**

[0277.0.20.20] The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.20] to [0282.0.0.20]: see [0278.0.0.0] to [0282.0.0.0]**

[0283.0.20.20] Moreover, native polypeptide conferring the increase of the respective fine chemical in an organism or part thereof can be isolated from cells, for example using the antibody of the present invention as described below, e.g. an antibody against a protein as indicated in Table II, application no. 20, column 3, or an antibody against a polypeptide as indicated in Table II, application no. 20, columns 5 or 7, which can be produced by standard techniques utilizing the polypeptid of the present invention or fragment thereof. Preferred are monoclonal antibodies.

**[0284.0.0.20] see [0284.0.0.0]**

**[0285.0.20.20]** In one embodiment, the present invention relates to a polypeptide having a sequence as indicated in Table II, application no. 20, columns 5 or 7, lines 251 to 261, 627, resp., or as coded by a nucleic acid molecule as indicated in Table I, application no. 20, columns 5 or 7, or functional homologues thereof.

[0286.0.20.20] In one advantageous embodiment, in the method of the present invention the activity of a polypeptide is increased which comprises or consists of a consensus sequence as indicated in Table IV, application no. 20, column 7, and in one another embodiment, the present invention relates to a polypeptide comprising or consisting of a consensus sequence as indicated in Table IV, application no. 20, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, application no. 20, column 7.

**[0287.0.0.20] to [0289.0.0.20]: see [0287.0.0.0] to [0289.0.0.0]**

**[00290.0.20.20]** The alignment was performed with the Software AlignX (sept 25, 2002) a component of Vector NTI Suite 8.0, InforMax™,Invitrogen™ life science software, U.S. Main Office, 7305 Executive Way,Frederick, MD 21704,USA with the following settings: For pairwise alignments: gap opening penality: 10,0; gap extension penality 0,1. For multiple alignments: Gap opening penalty: 10,0; Gap extension penalty: 0,1; Gap separation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids

[0291.0.20.20] In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II, application no. 20, columns 5 or 7, by one or more amino acids. In one embodiment, polypeptide distinguishes form a sequence as indicated in Table II, application no. 20, columns 5 or 7, by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 20, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II, application no. 20, columns 5 or 7.

**[0292.0.0.20] see [0292.0.0.0]**

[0293.0.20.20] In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II, application no. 20, columns 5 or 7, by one or more amino acids. In an other embodiment, said polypeptide of the invention does not consist of the sequence as indicated in Table II, application no. 20, columns 5 or 7,.
In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 20, columns 5 or 7.

[0294.0.20.20] In one embodiment, the present invention relates to a polypeptide having an activity of a protein as indicated in Table II, application no. 20, column 3, which distinguishes over a sequence as indicated in Table II, application no. 20, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.20] to [0296.0.0.20]: see [0295.0.0.0] to [0296.0.0.0]**

**[0297.0.0.20] see [0297.0.0.0]**

**[00297.1.0.20] %**

[0298.0.20.20] A polypeptide of the invention can participate in the process of the present invention. The polypeptide or a portion thereof comprises preferably an amino acid sequence which is sufficiently homologous to an amino acid sequence as indicated in Table II, application no. 20, columns 5 or 7. The portion of the protein is preferably a biologically active portion as described herein. Preferably, the polypeptide used in the process of the invention has an amino acid sequence identical to a sequence as indicated in Table II, application no. 20, columns 5 or 7.

**[0299.0.20.20]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91 %, 92%, 93%, 94% or 95%, and even more preferably at least about 96%, 97%, 98%, 99% or more homologous to one of the nucleotide sequence as indicated in Table I, application no. 20, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 20, columns 5 or 7, or which is homologous thereto, as defined above.

**[0300.0.20.20]** In one embodiment, the present invention relates to a polypeptide having an activity of a protein as indicated in Table II, application no. 20, column 3, which distinguishes over a sequence as indicated in Table II, application no. 20, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0301.0.0.20] see [0301.0.0.0]**

[0302.0.20.20] Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 20, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.20] see [0303.0.0.0]**

**[0304.0.20.20]** Manipulation of the nucleic acid molecule of the invention may result in the production of a protein having essentially the activity of the polypeptides as indicated in Table II, application no. 20, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.20] to [0308.0.0.20]: see [0305.0.0.0] to [0308.0.0.0]**

**[0309.0.20.20]** In one embodiment, a reference to a protein (= polypeptide) of the invention or as indicated in Table II, application no. 20, columns 5 or 7, refers to a polypeptide having an amino acid sequence corresponding to the polypeptide of the invention or used in the process of the invention, whereas an "other polypeptide" not being indicated in Table II, application no. 20, columns 5 or 7, refers to a polypeptide having an amino acid sequence corresponding to a protein which is not substantially homologous to a polypeptide of the invention, preferably which is not substantially homologous to a polypeptide as indicated in Table II, application no. 20, columns 5 or 7, e.g., a protein which does not confer the activity described herein or annotated or known for as indicated in Table II, application no. 20, column 3, and which is derived from the same or a different organism. In one embodiment, an "other polypeptide" not being indicated in Table II, application no. 20, columns 5 or 7, does not confer an increase of the respective fine chemical in an organism or part thereof.

**[0310.0.0.20] to [0334.0.0.20]: see [0310.0.0.0] to [0334.0.0.0]**

[0335.0.20.20] The dsRNAi method has proved to be particularly effective and advantageous for reducing the expression of a nucleic acid sequences as indicated in Table I, application no. 20, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, columns 5 or 7, and/or homologs thereof. In a double-stranded RNA molecule for reducing the expression of a protein encoded by a nucleic acid sequence as indicated in Table I, application no. 20, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.20] to [0342.0.0.20]: see [0336.0.0.0] to [0342.0.0.0]**

[0343.0.20.20] As has already been described, 100 % sequence identity between the dsRNA and a gene transcript of a nucleic acid sequence as indicated in Table I, application no. 20, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 20, columns 5 or 7, or homologs thereof of the one organism, may be used to suppress the corresponding expression in another organism.

**[0344.0.0.20] to [0350.0.0.20]: see [0344.0.0.0] to [0350.0.0.0]**

**[0351.0.0.20] to [0361.0.0.20]: see [0351.0.0.0] to [0361.0.0.0]**

[0362.0.20.20] Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 20, columns 5 or 7, e.g. encoding a polypeptide having protein activity, as indicated in Table II, application no. 20, columns 3. Due to the above-mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, application no. 20, columns 5 or 7, means herein that due to modulation or manipulation of the genome, an activity as annotated for a polypeptide as indicated in Table II, application no. 20, column 3, e.g. having a sequence as indicated in Table II, application no. 20, columns 5 or 7, is increased in a cell or an organism or a part thereof. Examples are described above in context with the process of the invention.

**[0363.0.0.20] see [0363.0.0.0]**

[0364.0.20.20] A naturally occurring expression cassette - for example the naturally occurring combination of a promoter of a gene encoding a polypeptide of the invention as indicated in Table II, application no. 20, column 3, with the corresponding protein-encoding sequence as indicated in Table I, application no. 20, column 5, becomes a transgenic expression cassette when it is modified by non-natural, synthetic "artificial" methods such as, for example, mutagenization. Such methods have been described (US 5,565,350; WO 00/15815; also see above).

**[0365.0.0.20] to [0373.0.0.20]: see [0365.0.0.0] to [0373.0.0.0]**

[0374.0.20.20] Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. melissic acid, in particular the respective fine chemical, produced in the process according to the invention may, however, also be isolated from the plant in the form of their free melissic acid, in particular the free respective fine chemical, or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.20] to [0376.0.0.20]: see [0375.0.0.0] to [0376.0.0.0]**

[0377.0.20.20] Accordingly, the present invention relates also to a process whereby the produced melissic acid is isolated.

[0378.0.20.20] In this manner, more than 50% by weight, advantageously more than 60% by weight, preferably more than 70% by weight, especially preferably more than 80% by weight, very especially preferably more than 90% by weight, of the melissic acid produced in the process can be isolated. The resulting melissic acid can, if appropriate, subsequently be further purified, if desired mixed with other active ingredients such as vitamins, amino acids, carbohydrates, antibiotics and the like, and, if appropriate, formulated.

[0379.0.20.20] In one embodiment the product produced by the present invention is a mixture of the respective fine chemicals melissic acid.

**[0380.0.20.20]** The melissic acid obtained in the process by carrying out the invention is suitable as starting material for the synthesis of further products of value. For example, they can be used in combination with each other or alone for the production of pharmaceuticals, foodstuffs, animal feeds or cosmetics. Accordingly, the present invention relates to a method for the production of pharmaceuticals, food stuff, animal feeds, nutrients or cosmetics comprising the steps of the process according to the invention, including the isolation of the melissic acid composition produced or the respective fine chemical produced if desired and formulating the product with a pharmaceutical acceptable carrier or formulating the product in a form acceptable for an application in agriculture. A further embodiment according to the invention is the use of the melissic acid produced in the process or of the transgenic organism in animal feeds, foodstuffs, medicines, food supplements, cosmetics or pharmaceuticals or for the production of melissic acid e.g. after isolation of the respective fine chemical or without, e.g. in situ, e.g in the organism used for the process for the production of the respective fine chemical.

**[0381.0.0.20] to [0382.0.0.20]: see [0381.0.0.0] to [0382.0.0.0]**

**[0383.0.20.20]**

**[0384.0.0.20] see [0384.0.0.0]**

[0385.0.20.20] The fermentation broths obtained in this way, containing in particular melissic acid in mixtures with other organic acids, amino acids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3 g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.20.20]** Accordingly, it is possible to purify the melissic acid produced according to the invention further. For this purpose, the product-containing composition is subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.20] to [0392.0.0.20]: see [0387.0.0.0] to [0392.0.0.0]**

[0393.0.20.20] In one embodiment, the present invention relates to a method for the identification of a gene product conferring an increase in the fine chemical production in a cell, comprising the following steps:
a) contacting-, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
b) identifying the nucleic acid molecules, which hybridize under relaxed stringent conditions with the nucleic acid molecule of the present invention in particular to the nucleic acid molecule sequence as indicated in Table I, application no. 20, columns 5 or 7, and, optionally, isolating the full length cDNA clone or complete genomic clone;
c) introducing the candidate nucleic acid molecules in host cells, preferably in a plant cell or a microorganism, appropriate for producing the fine chemical;
d) expressing the identified nucleic acid molecules in the host cells;
e) assaying the the fine chemical level in the host cells; and
f) identifying the nucleic acid molecule and its gene product which expression confers an increase in the the fine chemical level in the host cell after expression compared to the wild type.

**[0394.0.0.20] to [0552.0.0.20]: see [0394.0.0.7] to [0552.0.0.7]**

**[0553.0.20.20]** A process for the production of melissic acid resp., which comprises
(a) increasing or generating the activity of a protein as indicated in Table II, application no. 20, columns 5 or 7, or a functional equivalent thereof in a non-human organism, or in one or more parts thereof; and
(b) growing the organism under conditions which permit the production of melissic acid resp. in said organism.
A process for the production of melissic acid resp., comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 20, columns 5 or 7, , or a fragment thereof, which confers an increase in the amount of the fine chemical as indicated in table II, column 6, e.g of melissic acid resp., in an organism or a part thereof;
b) nucleic acid molecule comprising of a nucleic acid molecule as indicated in Table I, application no. 20, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of melissic acid resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of melissic acid resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of melissic acid resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 20, column 7, and conferring an increase in the amount of the fine chemical as indicated in table II, column 6, e.g of melissic acid resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of melissic acid resp., in an organism or a part thereof;
h) nucleic acid molecule encoding a polypeptide comprising a consensus sequence as indicated in Table IV, application no. 20, column 7, and conferring an increase in the amount of the fine chemical as indicated in table II, column 6, e.g of melissic acid resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical as indicated in table II, column 6, e.g of melissic acid resp., in an organism or a part thereof.
or comprising a sequence which is complementary thereto.
The process as mentioned above, comprising recovering of the free or bound melissic acid resp..
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound melissic acid resp., produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 20, columns 5 or 7, or a fragment thereof, which confers an increase in the amount of the fine chemical as indicated in table II, column 6, e.g of melissic acid resp., in an organism or a part thereof;
b) nucleic acid molecule comprising of a nucleic acid molecule as indicated in Table I, application no. 20, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of melissic acid resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of melissic acid resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of melissic acid resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 20, column 7, and conferring an increase in the amount of the fine chemical as indicated in table II, column 6, e.g of melissic acid resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of melissic acid resp., in an organism or a part thereof;
h) nucleic acid molecule encoding a polypeptide comprising a consensus sequence as indicated in Table IV, application no. 20, column 7, and conferring an increase in the amount of the fine chemical as indicated in table II, column 6, e.g of melissic acid resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemiccal as indicated in table II, column 6, e.g of melissic acid resp., in an organism or a part thereof.
whereby the nucleic acid molecule distinguishes over the sequence as indicated in Table I, application no. 20, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 20, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of melissic acid resp., in an organism or a part thereof comprising:
(a) contacting cells, tissues , plants or microorganisms which express the a polypeptide encoded by the nucleic acid molecule as mentioned above conferring an increase in the amount of melissic acid resp., in an organism or a part thereof with a candidate compound or a sample comprising a plurality of compounds under conditions which permit the expression the polypeptide;
(b) assaying the melissic acid resp., level or the polypeptide expression level in the cell, tissue, plant or microorganism or the media the cell, tissue, plant or microorganisms is cultured or maintained in; and
(c) identifying a agonist or antagonist by comparing the measured melissic acid resp., level or polypeptide expression level with a standard melissic acid resp., or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased melissic acid resp., production in a plant or microorganism, comprising the steps:
(a) culturing a plant cell or tissue or microorganism or maintaining a plant expressing the polypeptide encoded by the nucleic acid molecule as mentioned above conferring an increase in the amount of melissic acid resp., in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of melissic acid rersp., in an organism or a part thereof;
(b) identifying if the compound is an effective agonist by detecting the presence or absence or increase of a signal produced by said readout system.
A method for the identification of a gene product conferring an increase in melissic acid resp., production in a cell, comprising the following steps:
(a) contacting the nucleic acid molecules of a sample, which can contain a candidate gene encoding a gene product conferring an increase in melissic acid resp., after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing melissic acid resp.;
(d) expressing the identified nucleic acid molecules in the host cells;
(e) assaying the melissic acid rersp., level in the host cells; and
(f) identifying nucleic acid molecule and its gene product which expression confers an increase in the melissic acid resp., level in the host cell in the host cell after expression compared to the wild type.
A method for the identification of a gene product conferring an increase in melissic acid resp., production in a cell, comprising the following steps:
(a) identifiying in a data bank nucleic acid molecules of an organism; which can contain a candidate gene encoding a gene product conferring an increase in the melissic acid resp., amount or level in an organism or a part thereof after expression, and which are at least 20% homolog to the nucleic acid molecule as mentioned above;
(b) introducing the candidate nucleic acid molecules in host cells appropriate for producing melissic acid resp.,;
(c) expressing the identified nucleic acid molecules in the host cells;
(d) assaying the melissic acid resp., level in the host cells; and
(e) identifying nucleic acid molecule and its gene product which expression confers an increase in the melissic acid resp., level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of melissic acid resp., after expression.
Use of the polypeptide as mentioned above or the nucleic acid construct as mentioned above or the gene product identified according to the method as mentioned above for identifying compounds capable of conferring a modulation of melissic acid resp., levels in an organism.
Agrochemical, pharmaceutical, food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
The method as mentioned above, the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above, wherein the fine chemical is melissic acid.
A host cell or plant as mentioned above which is resistant to a herbicide inhibiting the biosynthesis of melissic acid.

### New genes for a process for the production of fine chemicals

### Description

**[0000.0.0.21]** In a further embodiment, the present invention relates to a further process for the production of fine chemicals as defined below and the corresponding embodiments as described herein as follows.

**[0001.0.0.21]** for the disclosure of this paragraph see [0001.0.0.0].

**[0002.0.21.21]** Plants produce glycerol. and glycerol-3-phosphate. Importantly lipids derived from glycerol are the major components of eukaryotic cells. In terms of dry weight they account for anywhere between 10% and 90% of the total mass of the cell. Triglycerol are the major source of store energy in eucaryotic organisms.
Glycerol-3-phosphate can be synthesized via two different routes in plants. In one route, it is formed from dihydroxyacetone phosphate (DHAP), an intermediate of glycolysis, by the sequential action of triosephosphate isomerase, glyceraldehyd-phosphate phosphatase, glyeraldehyde reductase and glycerol kinase. The last enzyme of this pathway has been suggested as the rate-limiting step of this route for glycerol-3-phosphate synthesis. In an second pathway glycerol-3-phosphate dehydrogenase (NAD(+)-G-3-P oxidoreductase, EC 1.1.1.8) (GPDH) catalyses the reduction of dihydroxyacetone phosphate (DHAP) to form glycerol-3-phosphate (G-3-P). Based on enzymatic studies is has been suggested that this enzyme activity is probably the primary source of glycerol-3-phosphate at least in Brassica campestris seeds (Sharma et al., 2001, Plant Sci 160, 603-610).

**[0003.0.0.21]** In plants, at least two types of GPDH, a cytoplasmatic and a plastidial exist, which also differ in their reducing cosubstrate. The cytsolic GPDH uses NADH as the cosubstrate. The mitochondrial FAD-dependent glycerol-3-phosphate dehydrogenase (FAD-GPDH) of Arabidopsis forms a G-3-P shuttle, as previously established in other eukaryotic organisms, and links cytosolic G-3-P metabolism to carbon source utilization and energy metabolism in plants - also see Shen, W. et al., FEBS Lett. 2003 Feb 11; 536(1-3): 92-6.
Glycerol-insensitive *Arabidopsis* mutants: *gli1* seedlings lack glycerol kinase, accumulate glycerol and are more resistant to abiotic stress, see Eastmond P.J. , HYPERLINK "http://www.ingentaconnect.com/content/bsc/tpj" \o "The Plant Journal"The Plant Journal , 2004, 37(4), 617-625. These data show that glycerol kinase is required for glycerol catabolism in *Arabidopsis* and that the accumulation of glycerol can enhance resistance to a variety of abiotic stresses associated with dehydration.

**[0004.0.0.21]** The major storage lipids (or oils) of seeds occur in the form of triacylglycerols (TAG), or three fatty acids linked to glycerol by ester bonds. Triacylglycerol synthesis involves diverse cellular compartments, including the cytoplasm, the mitochondria, the plastids, and the endoplasmic reticulum (ER). Glycerol-3-phosphate enters the ER for the final step in triacylglycerol synthesis. The newly formed triacylglycerols accumulate between the two layers of the double membrane of the ER, forming an oil body surrounded by a single (or half) unit membrane.

**[0005.0.0.21]** Glycerol-3-phosphate acyltransferase (GPAT) is one of the most important enzymes in TAG biosynthesis, since it initiates TAG synthesis by catalyzing the acylation of the Sn-1 position of Sn-glycerol-3-phosphate, producing Sn-1-acylglycerol-3-phosphate. Lyso-phosphatidic acid (LPA) is then acetylated by LPA acyltransferases to produce phosphatidic acid (PA). Then diacylglycerol (DAG) is released through the dephosphorylation of PA by PA phosphohydrolase. Finally DAG becomes acylated by the activity of the DAG acyltransferase. In a second pathway phosphatidylcholine (PC) is formed and its acyl residues are desaturated further. The choline phosphate residue is then liberated by hydrolysis and the correspondinbg DAG acylated. This second pathway operates frequently in the synthesis of highly unsaturated TAG (Heldt 1997, Plant biochemistry and molecular biology. Oxford University Press, New York).
Additionally at present, many researches have proved that the GPAT is related to plant chilling-resistance, see Liu, Ji-Mei et al., Plant Physiol. 120(1999): 934.
Glycerol-3-phosphate is a primary substrate for triacylglycerol synthesis. Vigeolas and Geigenberger (Planta 219(2004): 827-835) have shown that injection of developing seeds with glycerol leads to increased glycerol-3-phosphate levels. These increased levels of glycerol-3-phosphate were accompanied by an increase in the flux of sucrose into total lipids and triacylglycerol providing evidence that the prevailing levels of glycerol-3-phosphate co-limit triacylglycerol production in developing seeds.
The direct acylation of glycerol by a glycerol: acyl-CoA acyltransferase to form monoacyl-glycerol and, subsequently, diacylglycerol and triacylglycerol has been shown in myoblast and hepatocytes (Lee, D.P. et al. J. Lipid res. 42 (2001): 1979-1986). This direct acylation became more prominent when the glycerol-3-phosphate pathway was attenuated or when glycerol levels become elevated.

**[0006.0.0.21]** Glycerol is used together with water and alcohol (ethyl alcohol) in glycerinated water/alcohol plant extracts and phytoaromatic compounds. These products are used as food supplements, providing concentrates of the minerals, trace elements, active ingredients (alkaloids, polyphenols, pigments, etc.) and aromatic substances to be found in plants. Glycerin acts as a carrier for plant extracts. It is found in the end product (the fresh plant extract) in concentrations of up to 24% or 25%.
Raw glycerol is a by-product of the transesterification process of rape oil to rape methyl ester (RME) and used edible oil to used edible methyl ester (AME), both better known as Biodiesel.
Glycerol world production is estimated to be around 750.000 t/year. Around 90% is manufactured on the basis of natural oils and fats.

**[0007.0.0.21]** The green alga *Dunaliella,* for example, recently has been established in mass culture as a commercial source for glycerol. *Dunaliella* withstands extreme salinities while maintaing a low intracellular salt concentration. Osmotic adjustment is achieved by intracellular accumulation of glycerol to a level counterbalancing the external osmoticum.
The osmoregulatory isoform of dihydroxyacetone phosphate (DHAP) reductase (Osm-DHAPR) is an enzyme unique to Dunaliella tertiolecta and is the osmoregulatory isoform involved in the synthesis of free glycerol for osmoregulation in extreme environments, such as high salinity, see Ghoshal, D., et al., HYPERLINK "http://www.ingentaconnect.com/content/ap/pt;jsessionid=708pg3rt74t81.victoria" \o "Protein Expression and Purification" Protein Expression and Purification, 2002, 24, (3), 404-411.
A unsolved problem in plant biochemistry is the understanding of metabolic regulation of glycerol-3-phosphate synthesis and its use in modifying glyceride metabolism or glycerol production. Practically it will have significance for rationally genetically engineering of plants for increased synthesis of triacylglycerols or for other value added products, and for introducing the glycerol synthesis capability into plants of economic importance for an elevated environmental stress tolerance - see: Durba, G. et al., J. Plant Biochemistry & Biotechnology 10(2001),113-120.

**[0008.0.21.21]** One way to increase the productive capacity of biosynthesis is to apply recombinant DNA technology. Thus, it would be desirable to produce glycerol and/or glycerol-3-phosphate in plants. That type of production permits control over quality, quantity and selection of the most suitable and efficient producer organisms. The latter is especially important for commercial production economics and therefore availability to consumers. In addition it is desirable to produce glycerol and/or glycerol-3-phosphate in plants in order to increase plant productivity and resistance against biotic and abiotic stress as discussed before.
Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Argobacterium tumifaciens*-mediated transformation of tobacco plants using a vector that contained a ketolase-encoding gene from *H. pluvialis* denominated *crtO* along with the Pds gene from tomato as the promoter and to encode a leader sequence. Those results indicated that about 75 percent of the carotenoids found in the flower of the transformed plant contained a keto group.

**[0009.0.21.21]** Thus, it would be advantageous if an algae, plant or other microorganism were available which produce large amounts of glycerol and/or glycerol-3-phosphate. The invention discussed hereinafter relates in some embodiments to such transformed prokaryotic or eukaryotic microorganisms.
It would also be advantageous if plants were available whose roots, leaves, stem, fruits or flowers produced large amounts of glycerol and/or glycerol-3-phosphate. The invention discussed hereinafter relates in some embodiments to such transformed plants.
Furthermore it would be advantageous if plants were available whose seed produced larger amounts of total lipids. The invention discussed hereinafter relates in some embodiments to such transformed plants.

[0010.0.21.21] Therefore improving the quality of foodstuffs and animal feeds is an important task of the food-and-feed industry. This is necessary since, for example glycerol and/or glycerol-3-phosphate, as mentioned above, which occur in plants and some microorganisms are limited with regard to the supply of mammals. Especially advantageous for the quality of foodstuffs and animal feeds is as balanced as possible a specific glycerol and/or glycerol-3-phosphate profile in the diet since an excess of glycerol and/or glycerol-3-phosphate above a specific concentration in the food has a positive effect. A further increase in quality is only possible via addition of further glycerol and/or glycerol-3-phosphate, which are limiting.

[0011.0.21.21] To ensure a high quality of foods and animal feeds, it is therefore necessary to add glycerol and/or glycerol-3-phosphate in a balanced manner to suit the organism.

**[0012.0.21.21]** Accordingly, there is still a great demand for new and more suitable genes which encode enzymes or other proteins which participate in the biosynthesis of glycerol and/or glycerol-3-phosphate and make it possible to produce them specifically on an industrial scale without unwanted byproducts forming. In the selection of genes for biosynthesis two characteristics above all are particularly important. On the one hand, there is as ever a need for improved processes for obtaining the highest possible contents of glycerol and/or glycerol-3-phosphate; on the other hand as less as possible byproducts should be produced in the production process.
Furthermore there is still a great demand for new and more suitable genes, which encode enzymes or other proteins, which participate in the biosynthesis of total lipids and make it possible to produce them specifically on an industrial scale without unwanted byproducts forming. In the selection of genes for biosynthesis two characteristics above all are particularly important. On the one hand, there is as ever a need for improved processes for obtaining the highest possible contents of total lipids; on the other hand as less as possible byproducts should be produced in the production process.
Glycerol or glycerol-3-phosphate is biosynthetic precusor for the biosynthesis of monoacylglycerols, diacylglycerols, triacylglycerols, phosphatidylglycerols and other glycerolipids (e.g. glycosylglycerides, diphosphatidylglycerols, phosphonolipids, phosphatidylcholines, phosphatidylethanolamines, phosphatidylinositols, phytoglycolipids). Therefore the analysis of the glycerol content in cells, tissues or plant parts like seeds and leaves after total lipid extraction and lipid hydrolysis directly correlates with the analysis of the total lipid content. For example if the overexpression of a gene participating in the biosynthesis of triacylglycerols in the seed results in an increase in total lipid content in the seed or leaf this seed will also show an increased glycerol content after total lipid extraction and hydrolysis of the lipids.
Therefore the method as described below which leads to an increase in glycerol in the lipid fraction after cleavage of the ester functions for example with a mixture of methanol and hydrochloric acid clearly represents a method for an increased production of triacylglycerol or total lipids.

**[0013.0.0.21]** for the disclosure of this paragraph see [0013.0.0.0].

**[0014.0.21.21]** Accordingly, in a first embodiment, the invention relates to a process for the production of a fine chemical, whereby the fine chemical is glycerol and/or glycerol-3-phosphate. Accordingly, in the present invention, the term "the fine chemical" as used herein relates to glycerol and/or glycerol-3-phosphate. Further, the term "the fine chemicals" as used herein also relates to fine chemicals comprising glycerol and/or glycerol-3-phosphate.

[0015.0.21.21] In one embodiment, the term "the fine chemical" means glycerol. In one embodiment, the term "the fine chemical" means glycerol-3-phosphate depending on the context in which the term is used. Throughout the specification the term "the fine chemical" means glycerol and/or glycerol-3-phosphate, its salts, ester, thioester or in free form or bound to other compounds such as sugars or sugarpolymers, like glucoside or polyols like myo-inositol.

In one embodiment, the term "the fine chemical" means monoacylglycerols, diacylglycerols, triacylglycerols, phosphatidylglycerols and/or other glycerolipids (e.g. but not limited to glycosylglycerides, diphosphatidylglycerols, phosphonolipids, phosphatidylcholines, phosphatidylethanolamines, phosphatidylinositols or phytoglycolipids) and is hereinafter referred to as "total lipids".

**[0016.0.21.21**] Accordingly, the present invention relates to a process for the production of the respective fine chemical comprising
(a) increasing or generating the activity of one or more
   of a protein as shown in table II, application no. 21, column 3 encoded by the nucleic acid sequences as shown in table I, application no. 21, column 5, in a non-human organism or in one or more parts thereof or
(b) growing the organism under conditions which permit the production of the fine chemical, thus glycerol of the invention or fine chemicals comprising glycerol of the invention, in said organism or in the culture medium surrounding the organism.

[0016.1.21.21] Accordingly, the term "the fine chemical" means in one embodiment "glycerol" in relation to all sequences listed in Tables I to IV, line 44 and/or 45 and/or 46 or homologs thereof.

**[0017.0.0.21] and [0019.0.0.21]** for the disclosure of the paragraphs [0017.0.0.21] and [0019.0.0.21] see paragraphs [0017.0.0.0] and [0019.0.0.0] above.

**[0020.0.21.21]** Surprisingly it was found, that the transgenic expression of the Brassica napus protein as indicated in Table II, column 5, line 44 in a plant conferred an increase in glycerol content of the transformed plants. Thus, in one embodiment, said protein or its homologs are used for the production of glycerol.
Surprisingly it was found, that the transgenic expression of the Zea mays protein as indicated in Table II, column 5, line 45 in a plant conferred an increase in glycerol content of the transformed plants. Thus, in one embodiment, said protein or its homologs are used for the production of glycerol.
Surprisingly it was found, that the transgenic expression of the Glycine max protein as indicated in Table II, column 5, line 46 in a plant conferred an increase in glycerol content of the transformed plants. Thus, in one embodiment, said protein or its homologs are used for the production of glycerol.

**[0021.0.0.21]** for the disclosure of this paragraph see [0021.0.0.0] above.

**[0022.0.21.21]** The sequence of YDR513W from Saccharomyces cerevisiae has been published in Jacq, C. et al., Nature 387 (6632 Suppl), 75-78 (1997) and its activity is being defined as a protein having gluthatione reductase activity. Accordingly, in one embodiment, the process of the present invention comprises the use of a protein YDR513W from Saccharomyces cerevisiae or a plant or its homolog, e.g. as shown herein, for the production of the respective fine chemical, in particular for increasing the amount of glycerol and/or total lipid, preferably in free or bound form in an organism or a part thereof, as mentioned. In one embodiment, in the process of the present invention the YDR513W-protein-activity is increased.
The sequence of YGL237C from Saccharomyces cerevisiae has been published in Tettelin, H. et al. Nature 387 (6632 Suppl), 81-84 (1997) and its activity is being defined as transcriptional activator and global regulator of respiratory gene expression. Accordingly, in one embodiment, the process of the present invention comprises the use of a protein YGL237C from Saccharomyces cerevisiae or a plant or its homolog, e.g. as shown herein, for the production of the respective fine chemical, in particular for increasing the amount of glycerol and/or total lipid, preferably in free or bound form in an organism or a part thereof, as mentioned. In one embodiment, in the process of the present invention the YGL237C-protein-activity a is increased.
The sequence of YMR015C from Saccharomyces cerevisiae has been published Bowman, S. et al., Nature 387 (6632 Suppl), 90-93 (1997) and its activity is being defined as a protein having C-22 sterol desaturase activity. Accordingly, in one embodiment, the process of the present invention comprises the use of a protein YMR015C from Saccharomyces cerevisiae or a plant or its homolog, e.g. as shown herein, for the production of the respective fine chemical, in particular for increasing the amount of glycerol and/or total lipid, preferably in free or bound form in an organism or a part thereof, as mentioned. In one embodiment, in the process of the present invention the YMR015C-protein-activity is increased.

**[0022.1.0.21] and [0023.0.0.21]** for the disclosure of the paragraphs [0022.1.0.21] and [0023.0.0.21] see paragraphs [0022.1.0.0] and [0023.0.0.0] above.

**[0023.1.0.21]** Homologs of the polypeptide disclosed in table II, application no. 21, column 5, may have the activity of a protein as disclosed in table I or II, application no. 21, column 3 and may be the polypetides encoded by the nucleic acid molecules indicated in table I, application no. 21, column 7, resp., or may be the polypeptides indicated in table II, application no. 21, column 7, resp.

**[0024.0.0.21]** for the disclosure of this paragraph see [0024.0.0.0] above.

**[0025.0.21.21]** In accordance with the invention, a protein or polypeptide has the "activity of an protein as shown in table II, application no. 21, column 3" if its de *novo* activity, or its increased expression directly or indirectly leads to an increased in the level of the fine chemical indicated in the respective line of table II, application no. 21, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 21, column 3, or which has at least 10% of the original enzymatic or biological activity, preferably 20%, particularly preferably 30%, most particularly preferably 40% in comparison to a protein as shown in the respective line of table II, application no. 21, column 3 of a E. coli or Saccharomyces cerevisae, respectively, protein as mentioned in table I to IV, column 3 respectively and as disclosed in paragraph [0022] of the respective invention.

**[0025.1.0.21] and [0025.2.0.21]** for the disclosure of the paragraphs [0025.1.0.21] and [0025.2.0.21] see [0025.1.0.0] and [0025.2.0.0] above.

**[0026.0.0.17] to [0033.0.0.17]** for the disclosure of the paragraphs [0026.0.0.17] to [0033.0.0.17] see [0026.0.0.0] to [0033.0.0.0] above.

**[0034.0.21.21]**Preferably**,** the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 21, column 3 its biochemical or genetical causes and the increased amount of the respective fine chemical.

**[0035.0.0.21] to [0044.0.0.21]** for the disclosure of the paragraphs [0035.0.0.21] to [0044.0.0.21] see paragraphs [0035.0.0.0] to [0044.0.0.0] above.

**[0045.0.21.21]** In one embodiment, the activity of the protein. as indicated in Table II, columns 5 or 7, application no. 21, is increased conferring an increase of the respective fine chemical, indicated in Table II, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more.

**[0046.0.0.21]** In one embodiment, the activity of the protein as indicated in Table II, columns 5 or 7, application no. 21, is increased conferring an increase of the respective fine chemical, indicated in Table II, column 6 of the respective line confers an increase of the respective fine chemical and of further glycerol or their precursors.

**[0047.0.0.21] and [0048.0.0.21]** for the disclosure of the paragraphs [0047.0.0.21] and [0048.0.0.21] see paragraphs [0047.0.0.0] and [0048.0.0.0] above.

[0049.0.21.21]A protein having an activity conferring an increase in the amount or level of the fine chemical, has in one embodiment the structure of the polypeptide described herein, in particular of the polypeptides comprising the consensus sequence shown in table IV, application no. 21, column 7 or of the polypeptide as shown in the amino acid sequences as disclosed in table II, application no. 21, columns 5 and 7 or the functional homologues thereof as described herein, or is encoded by the nucleic acid molecule characterized herein or the nucleic acid molecule according to the invention, for example by the nucleic acid molecule as shown in table I, application no. 21, columns 5 and 7 or its herein described functional homologues and has the herein mentioned activity.

**[0050.0.21.21]** For the purposes of the present invention, the term "the respective fine chemical" also encompass the corresponding salts, such as, for example, the potassium or sodium salts of glycerol-3-phosphate, resp., or their esters, e.g. monoacyl or diacyl fatty acids thereof.
%

**[0051.0.21.21]** Owing to the biological activity of the proteins which are used in the process according to the invention and which are encoded by nucleic acid molecules according to the invention, it is possible to produce compositions comprising the respective fine chemical, i.e. an increased amount of the free or bound, e.g compositions comprising glycerol and/or total lipid. Depending on the choice of the organism used for the process according to the present invention, for example a microorganism or a plant, compositions or mixtures of glycerol and/or total lipid can be produced.

**[0052.0.0.21]** for the disclosure of this paragraph see paragraph [0052.0.0.0] above.

**[0053.0.21.21]** In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 21, columns 5 and 7 or its homologs activity having herein-mentioned glycerol of the invention increasing activity; and/or
b) stabilizing a mRNA conferring the increased expression of a protein encoded by the nucleic acid molecule of the invention as shown in table I, application no. 21, columns 5 and 7, e.g. a nucleic acid sequence encoding a polypeptide having the activity of a protein as indicated in table II, application no. 21, columns 5 and 7 or its homologs activity or of a mRNA encoding the polypeptide of the present invention having herein-mentioned glycerol of the invention increasing activity; and/or
c) increasing the specific activity of a protein conferring the increased expression of a protein encoded by the nucleic acid molecule of the invention or of the polypeptide of the present invention having herein-mentioned gylcerol increasing activity, e.g. of a polypeptide having the activity of a protein as indicated in table II, application no. 21, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned glycerol of the invention increasing activity, e.g. of a polypeptide having the activity of a protein as indicated in table II, application no. 21, columns 5 and 7 or its homologs activity; and/or
e) stimulating activity of a protein conferring the increased expression of a protein encoded by the nucleic acid molecule of the present invention or a polypeptide of the present invention having herein-mentioned glycerol of the invention increasing activity, e.g. of a polypeptide having the activity of a protein as indicated in table II, application no. 21, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned glycerol of the invention increasing activity, e.g. of a polypeptide having the activity of a protein as indicated in table II, application no. 21, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned glycerol of the invention increasing activity, e.g. of a polypeptide having the activity of a protein as indicated in table II, application no. 21, columns 5 and 7 or its homologs activity; and/or
h) increasing the expression of the endogenous gene encoding the polypeptide of the invention, e.g. a polypeptide having the activity of a protein as indicated in table II, application no. 21, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with especially high activity of the proteins of the invention from natural or from mutagenized resources and breeding them into the target organisms, eg the elite crops.

**[0054.0.21.21]** Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 21 in Table I to IV, resp., after increasing the expression or activity of the encoded polypeptide or having the activity of a polypeptide having an activity as the protein as shown in table II, application no. 21, column 3 or its homologs.

**[0055.0.0.21] to [0067.0.0.21]** for the disclosure of the paragraphs [0055.0.0.21] to [0067.0.0.21] see paragraphs [0055.0.0.0] to [0067.0.0.0] above.

**[0068.0.21.21]** The mutation is introduced in such a way that the production of the finde chemical, meaning glycerol and/or total lipid is not adversely affected.

**[0069.0.0.21]** for the disclosure of this paragraph see paragraph [0069.0.0.0] above.

[0070.0.21.21] Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create de *novo* an advantageous, preferably novel metabolite composition in the organism, e.g. an advantageous composition of glycerol or their biochemical derivatives , e.g. comprising a higher content of (from a viewpoint of nutrional physiology limited) glycerol or their derivatives including total lipids.

**[0071.0.0.21]** for the disclosure of this paragraph see paragraph [0071.0.0.0] above.

**[0072.0.0.21] %**

**[0073.0.21.21]** Accordingly, in one embodiment, the process according to the invention relates to a process, which comprises:
(a) providing a non-human organism, preferably a microorganism, a non-human animal, a plant or animal cell, a plant or animal tissue or a plant;
(b) increasing an activity of a polypeptide of the invention or a homolog thereof, e.g. as indicated in Table II, application no. 21, columns 5 or 7, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
(c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
(d) if desired, recovering, optionally isolating, the free and/or bound respective fine chemical synthesized by the organism, the microorganism, the non-human animal, the plant or animal cell, the plant or animal tissue or the plant.

**[0074.0.21.21]** for the disclosure of this paragraph see paragraph [0071.0.0.17] above.

**[0075.0.0.21] to [0077.0.0.21]** for the disclosure of the paragraphs [0075.0.0.21] to [0077.0.0.21] see paragraphs [0075.0.0.0] to [0077.0.0.0] above.

**[0078.0.21.21]** The organism such as microorganisms or plants or the recovered, and if desired isolated, respective fine chemical can then be processed further directly into foodstuffs or animal feeds or for other applications. The fermentation broth, fermentation products, plants or plant products can be purified with methods known to the person skilled in the art. Products of these different work-up procedures are glycerol and/or glycerol as a component of lipids or comprising compositions of glycerol and/or glycerol as a component of lipids still comprising fermentation broth, plant particles and cell components in different amounts, advantageously in the range of from 0 to 99% by weight, preferably below 80% by weight, especially preferably below 50% by weight.

**[0079.0.0.21] to [0084.0.0.21]** for the disclosure of the paragraphs [0079.0.0.21] to [0084.0.0.21] see paragraphs [0079.0.0.0] to [0084.0.0.0] above.

**[0085.0.21.21]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, application no. 21, columns 5 or 7, or a derivative thereof, or
b) a genetic regulatory element, for example a promoter, which is functionally linked to the nucleic acid sequence as indicated in Table I, application no. 21, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.21] and [0087.0.0.21]** for the disclosure of the paragraphs [0086.0.0.21] and [0087.0.0.21] see paragraphs [0086.0.0.0] and [0087.0.0.0] above.

**[0088.0.21.21]** In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned fine chemicals or the plants are more resistant to biotic and abiotic stress and the yield is increased.

**[0088.1.0.21]** for the disclosure of this paragraph see paragraph [0088.1.0.0] above.

**[0089.0.0.21] to [0094.0.0.21]** for the disclosure of the paragraphs [0089.0.0.21] to [0094.0.0.21] see paragraphs [0088.0.0.0] to [0094.0.0.0] above.

**[0095.0.21.21]** It may be advantageous to increase the pool of glycerol and/or total lipid in the transgenic organisms by the process according to the invention in order to isolate high amounts of the pure respective fine chemical and/or to obtain increased resistance against biotic and abiotic stresses and to obtain higher yield.

**[0096.0.21.21]** In another preferred embodiment of the invention a combination of the increased expression of the nucleic acid sequence or the protein of the invention together with the transformation of a protein or polypeptid or a compound, which functions as a sink for the desired fine chemical, for example in the organism, is useful to increase the production of the respective fine chemical.

**[0097.0.0.21]** for the disclosure of this paragraph see paragraph [0097.0.0.0] above.

**[0098.0.21.21]** In a preferred embodiment, the respective fine chemical is produced in accordance with the invention and, if desired, is isolated.

**[0099.0.21.21]** In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.21.21]** Transgenic plants which comprise the fine chemicals such as glycerol and/or total lipids synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because glycerol and /or total lipids in microorganisms are localized intracellular, their recovery essentially comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.

**[0101.0.21.21]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.21.21]** Glycerol can for example be analyzed advantageously via HPLC, LC or GC separation and MS (masspectrometry) detection methods. The unambiguous detection for the presence of glycerol containing products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.
Total lipids in the seed of plants can for example be analyzed advantageously by lipid extraction as described by Bligh and Dyer (Can J Biochem Phys 1959, 37, 911-917) followed by the determination of the lipid content by gaschromatography as described by Benning and Sommerville (J Bacteriol 1992, 174, 6479-6487).

**[0103.0.21.21]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, application no. 21, columns 5 or 7, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 21, columns 5 or 7;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as indicated in Table III, application no. 21, column 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence having sequences as indicated in Table IV, application no. 21, column 7, and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, application no. 21, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

[00103.1.21.21] In one embodiment, the nucleic acid molecule used in the process of the invention distinguishes over the sequence indicated in Table I, application no. 21, columns 5 or 7 by one or more nucleotides. In one embodiment, the nucleic acid molecule used in the process of the invention does not consist of the sequence shown in indicated in Table I ,application no. 21, columns 5 or 7,. In one embodiment, the nucleic acid molecule used in the process of the invention is less than 100%, 99,999%, 99,99%, 99,9% or 99% identical to a sequence indicated in Table I , application no. 21, columns 5 or 7,. In another embodiment, the nucleic acid molecule does not encode a polypeptide of a sequence indicated in Table II, application no. 21, columns 5 or 7.

**[0104.0.21.21]** In one embodiment, the nucleic acid molecule used in the process of the present invention distinguishes over the sequence indicated in Table I, application no. 21, columns 5 or 7, by one or more nucleotides. In one embodiment, the nucleic acid molecule used in the process of the invention does not consist of the sequence indicated in Table I, application no. 21, columns 5 or 7. In one embodiment, the nucleic acid molecule of the present invention is less than 100%, 99,999%, 99,99%, 99,9% or 99% identical to the sequence indicated in Table I, application no. 21, columns 5 or 7.. In another embodiment, the nucleic acid molecule does not encode a polypeptide of a sequence indicated in Table II, application no. 21, columns 5 or 7.

**[0105.0.0.21] to [0107.0.0.21]** for the disclosure of the paragraphs [0105.0.0.21] to [0107.0.0.21] see paragraphs [0105.0.0.0] to [0107.0.0.0] above.

**[0108.0.21.21]** Nucleic acid molecules with the sequence as indicated in Table I, application no. 21, columns 5 or 7, nucleic acid molecules which are derived from an amino acid sequences as indicated in Table II, application no. 21, columns 5 or 7, or from polypeptides comprising the consensus sequence as indicated in Table IV, application no. 21, column 7, or their derivatives or homologues encoding polypeptides with the enzymatic or biological activity of an activity of a polypeptide as indicated in Table II, application no. 21, column 3, 5 or 7, e.g. conferring the increase of the respective fine chemical, meaning glycerol and/or total lipid, resp., after increasing its expression or activity, are advantageously increased in the process according to the invention.

**[0109.0.21.21]** In one embodiment, said sequences are cloned into nucleic acid constructs, either individually or in combination. These nucleic acid constructs enable an optimal synthesis of the respective fine chemicals, in particular glycerol and/or total lipids, produced in the process according to the invention.

**[0110.0.0.21]** Nucleic acid molecules, which are advantageous for the process according to the invention and which encode polypeptides with an activity of a polypeptide used in the method of the invention or used in the process of the invention, e.g. of a protein as shown in Table II, application no. 21, columns 5 or 7or being encoded by a nucleic acid molecule indicated in Table I, application no. 21, columns 5 or 7, or of its homologs, e.g. as indicated in Table II, application no. 21, columns 5 or 7, can be determined from generally accessible databases.

**[0111.0.0.21]** for the disclosure of this paragraph see [0111.0.0.0] above. **[0112.0.21.21]** The nucleic acid molecules used in the process according to the invention take the form of isolated nucleic acid sequences, which encode polypeptides with an activity of a polypeptide as indicated in Table II, application no. 21, column 3, lines 173, 262 to 274 and 628 to 632 or having the sequence of a polypeptide as indicated in Table II, application no. 21, columns 5 and 7, and conferring an increase in the glycerol and/or total lipid level.

**[0113.0.0.21] to [0120.0.0.21]** for the disclosure of the paragraphs [0113.0.0.21] to [0120.0.0.21] see paragraphs [0113.0.0.0] and [0120.0.0.0] above.

**[0121.0.21.21]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 21, columns 5 or 7, or the functional homologues thereof as described herein, preferably conferring above-mentioned activity, i.e. conferring a glycerol and/or total lipid level increase after increasing the activity of the polypeptide sequences indicated in Table II, application no. 21, columns 5 or 7.

**[0122.0.0.21] to [0127.0.0.21]** for the disclosure of the paragraphs [0122.0.0.21] to [0127.0.0.21] see paragraphs [0122.0.0.0] and [0127.0.0.0] above.

**[0128.0.21.21]** Synthetic oligonucleotide primers for the amplification, e.g. as the pairs indicated in Table III, application no. 21, column 7, by means of polymerase chain reaction can be generated on the basis of a sequence shown herein, for example the sequence as indicated in Table I, application no. 21, columns 5 or 7, or the sequences derived from a sequences as indicated in Table II, application no. 21, columns 5 or 7.

**[0129.0.21.21]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved regions are those, which show a very little variation in the amino acid in one particular position of several homologs from different origin. The consensus sequence indicated in Table IV, application no. 21, columns 7, is derived from such alignments.

**[0130.0.21.21]** Degenerated primers can then be utilized by PCR for the amplification of fragments of novel proteins having above-mentioned activity, e.g. conferring the increase of glycerol and/or total lipid after increasing the expression or activity the protein comprising said fragment.

**[0131.0.0.21] to [0138.0.0.21]** for the disclosure of the paragraphs [0131.0.0.21] to [0138.0.0.21] see paragraphs [0131.0.0.0] to [0138.0.0.0] above.

**[0139.0.21.21]** Polypeptides having above-mentioned activity, i.e. conferring the respective fine chemical level increase, derived from other organisms, can be encoded by other DNA sequences which hybridise to a sequence indicated in Table I, application no. 21, columns 5 or 7, for glycerol, and/or total lipid under relaxed hybridization conditions and which code on expression for peptides having the respective fine chemical, i.e. glycerol and/or total lipids, resp., increasing-activity.

**[0140.0.0.21] to [0146.0.0.21]** for the disclosure of the paragraphs [0140.0.0.21] to [0146.0.0.21] see paragraphs [0140.0.0.0] to [0146.0.0.0] above.

[0147.0.21.21] Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 21, columns 5 or 7, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

[0148.0.21.21] The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 21, columns 5 or 7 or a portion thereof and preferably has above mentioned activity, in particular, of glycerol increasing activity after increasing the acitivity or an activity of a product of a gene encoding said sequences or their homologs.

**[0149.0.21.21]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 21, columns 5 or 7, or a portion thereof and encodes a protein having above-mentioned activity and as indicated in indicated in Table II.

**[00149.1.21.21]** Optionally, in one embodiment, the nucleotide sequence, which hybridises to one of the nucleotide sequences indicated in Table I, application no. 21, columns 5 or 7, has further one or more of the activities annotated or known for a protein as indicated in Table II, application no. 21, column 3.

**[0150.0.21.21]** Moreover, the nucleic acid molecule of the invention can comprise only a portion of the coding region of one of the sequences indicated in Table I, application no. 21, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of glycerol, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 21, columns 5 or 7, , an anti-sense sequence of one of the sequences, e.g., as indicated in Table I, application no. 21, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 21, column 7, will result in a fragment of a polynucleotide sequence as indicated in Table I, application no. 21, columns 5 or 7 or its gene product.

[0151.0.0.21]: for the disclosure of this paragraph see paragraph [0151.0.0.0] above.

**[0152.0.21.21]** The nucleic acid molecule of the invention encodes a polypeptide or portion thereof which includes an amino acid sequence which is sufficiently homologous to an amino acid sequence as indicated in Table II, application no. 21, columns 5 or 7, such that the protein or portion thereof maintains the ability to participate in the respective fine chemical production, in particular a glycerol and total lipid increasing activity as mentioned above or as described in the examples in plants or microorganisms is comprised.

**[0153.0.21.21]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 21, columns 5 or 7, such that the protein or portion thereof is able to participate in the increase of the respective fine chemical production. In one embodiment, a protein or portion thereof as indicated in Table II, application no. 21, columns 5 or 7, has for example an activity of a polypeptide indicated in Table II, application no. 21, column 3.

**[0154.0.21.21]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, application no. 21, columns 5 or 7, and has above-mentioned activity, e.g.conferring preferably the increase of the respective fine chemical.

**[0155.0.0.21] and [0156.0.0.21]** for the disclosure of the paragraphs [0155.0.0.21] and [0156.0.0.21] see paragraphs [0155.0.0.0] and [0156.0.0.0] above.

**[0157.0.21.21]** The invention further relates to nucleic acid molecules that differ from one of the nucleotide sequences as indicated in Table I, application no. 21, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, application no. 21, column 7, or as polypeptides depicted in Table II, application no. 21, columns 5 or 7, or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, application no. 21, column 7, or of the polypeptide as indicated in Table II, application no. 21, columns 5 or 7, or the functional homologues. In a still further embodiment, the nucleic acid molecule of the invention encodes a full length protein which is substantially homologous to an amino acid sequence comprising a consensus sequence as indicated in Table IV, application no. 21, column 7, or of a polypeptide as indicated in Table II, application no. 21, columns 5 or 7, or the functional homologues. However, in a preferred embodiment, the nucleic acid molecule of the present invention does not consist of a sequence as indicated in Table I, application no. 21, columns 5 or 7.

**[0158.0.0.21] to [0160.0.0.21]** for the disclosure of the paragraphs [0158.0.0.21] to [0160.0.0.21] see paragraphs [0158.0.0.0] to [0160.0.0.0] above.

**[0161.0.21.21]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 21, columns 5 or 7. The nucleic acid molecule is preferably at least 20, 30, 50, 100, 250 or more nucleotides in length.

**[0162.0.0.21]** for the disclosure of this paragraph see paragraph [0162.0.0.0] above.

**[0163.0.21.21]** Preferably, a nucleic acid molecule of the invention that hybridizes under stringent conditions to a sequence as indicated in Table I, application no. 21, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in an organism or a part thereof, e.g. a tissue, a cell, or a compartment of a cell, after increasing the expression or activity thereof or the activity of a protein of the invention or used in the process of the invention.

**[0164.0.0.21]** for the disclosure of this paragraph see paragraph [0164.0.0.0] above.

**[0165.0.21.21]** For example, nucleotide substitutions leading to amino acid substitutions at "non-essential" amino acid residues can be made in a sequence of the nucleic acid molecule of the invention or used in the process of the invention, e.g. as indicated in Table I, application no. 21, columns 5 or 7.

**[0166.0.0.21] and [0167.0.0.21]** for the disclosure of the paragraphs [0166.0.0.21] and [0167.0.0.21] see paragraphs [0166.0.0.0] and [0167.0.0.0] above.

**[0168.0.21.21]** Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 21, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 21, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 21, columns 5 or 7, more preferably at least about 70% identical to one of the sequences as indicated in Table II, application no. 21, columns 5 or 7, ,even more preferably at least about 80%, 90%, or 95% homologous to a sequence as indicated in Table II, application no. 21, columns 5 or 7, , and most preferably at least about 96%, 97%, 98%, or 99% identical to the sequence as indicated in Table II, application no. 21, columns 5 or 7.

**[0169.0.0.21] to [0172.0.0.21]** for the disclosure of the paragraphs [0169.0.0.21] to **[0172.0.0.21]** see paragraphs [0169.0.0.0] to [0172.0.0.0] above.

**[0173.0.21.21]For** example a sequence which has a 80% homology with sequence SEQ ID NO: 227 at the nucleic acid level is understood as meaning a sequence which, upon comparison with the sequence SEQ ID NO: 227 by the above Gap program algorithm with the above parameter set, has a 80% homology.

[0174.0.0.21]: for the disclosure of this paragraph see paragraph [0174.0.0.0] above.

**[0175.0.21.21]For** example a sequence which has a 80% homology with sequence SEQ ID NO: 228 at the protein level is understood as meaning a sequence which, upon comparison with the sequence SEQ ID NO: 228 by the above program algorithm with the above parameter set, has a 80% homology.

**[0176.0.21.21]** Functional equivalents derived from one of the polypeptides as indicated in Table II, application no. 21, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, application no. 21, columns 5 or 7, according to the invention and are distinguished by essentially the same properties as a polypeptide as indicated in Table II, application no. 21, columns 5 or 7.

[0177.0.21.21] Functional equivalents derived from a nucleic acid sequence as indicated in Table I, application no. 21, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of a polypeptide as indicated in Table II, application no. 21, columns 5 or according to the invention and encode polypeptides having essentially the same properties as a polypeptide as indicated in Table II, application no. 21, columns 5 or 7,.

**[0178.0.0.21]** for the disclosure of this paragraph see [0178.0.0.0] above.

**[0179.0.21.21]** A nucleic acid molecule encoding a homologue to a protein sequence as indicated in Table II, application no. 21, columns 5 or 7, can be created by introducing one or more nucleotide substitutions, additions or deletions into a nucleotide sequence of the nucleic acid molecule of the present invention, in particular as indicated in Table I, application no. 21, columns 5 or 7, such that one or more amino acid substitutions, additions or deletions are introduced into the encoded protein. Mutations can be introduced into the encoding sequences as indicated in Table I, application no. 21, columns 5 or 7, by standard techniques, such as site-directed mutagenesis and PCR-mediated mutagenesis.

**[0180.0.0.21] to [0183.0.0.21]** for the disclosure of the paragraphs [0180.0.0.21] to [0183.0.0.21] see paragraphs [0180.0.0.0] to [0183.0.0.0] above.

[0184.0.21.21] Homologues of the nucleic acid sequences used, with a sequence as indicated in Table I, application no. 21, columns 5 or 7, or of the nucleic acid sequences derived from a sequences as indicated in Table II, application no. 21, columns 5 or 7, comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 21, columns 5 or 7, or from the derived nucleic acid sequences, the intention being, however, that the enzyme activity or the biological activity of the resulting proteins synthesized is advantageously retained or increased.

[0185.0.21.21] In one embodiment of the present invention, the nucleic acid molecule of the invention or used in the process of the invention comprises one or more sequences as indicated in Table I, application no. 21, columns 5 or 7,. In one embodiment, it is preferred that the nucleic acid molecule comprises as little as possible other nucleotides not shown in any one of sequences as indicated in Table I, application no. 21, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 21, columns 5 or 7.

**[0186.0.21.21]** Also preferred is that one or more nucleic acid molecule(s) used in the process of the invention encodes a polypeptide comprising a sequence as indicated in Table II, application no. 21, columns 5 or 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 21, columns 5 or 7.

**[0187.0.21.21]** In one embodiment, a nucleic acid molecule of the invention or used in the process encodes a polypeptide comprising the sequence as indicated in Table II, application no. 21, columns 5 or 7, comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 21, columns 5 or 7.

**[0188.0.21.21]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 21, columns 5 or 7 and is expressed under identical conditions.

**[0189.0.21.21]** Homologues of a sequences as indicated in Table I, application no. 21, columns 5 or 7, or of a derived sequences as indicated in Table II, application no. 21, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

[0190.0.0.21]: for the disclosure of this paragraph see [0190.0.0.0] above.

**[0191.0.21.21]** In one embodiment, the organisms or part thereof produce according to the herein mentioned process of the invention an increased level of free and/or bound the respective fine chemical compared to said control or selected organisms or parts thereof.

[0191.1.0.21]: for the disclosure of this paragraph see [0191.1.0.0] above.

**[0192.0.0.21] to [0203.0.0.21]** for the disclosure of the paragraphs [0192.0.0.21] to [0203.0.0.21] see paragraphs [0192.0.0.0] to [0203.0.0.0] above.

[0204.0.21.21] Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 21, columns 5 or 7, or a fragment thereof conferring an increase in the amount of the respective fine chemical, in particular according to table II, application no. 21, column 6 in an organism or a part thereof
b) nucleic acid molecule comprising, preferably at least the mature form, of a nucleic acid molecule as indicated in Table I, application no. 21, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, application no. 21, column 7, and conferring an increase in the amount of the respective fine chemical, in particular according to table II, application no. 21, column 6 in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, application no. 21, columns 5 or 7, and conferring an increase in the amount of the respective fine chemical, in particular according to table II, application no. 21, column 6 in an organism or a part thereof;
k) nucleic acid molecule encoding the amino acid sequence of a polypeptide encoding a domaine of a polypeptide as indicated in Table II, application no. 21, columns 5 or 7, and conferring an increase in the amount of the respective fine chemical, in particular accccording to table II, application no. 21, column 6 in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 21, columns 5 or 7, or a nucleic acid molecule encoding, preferably at least the mature form of, a polypeptide as indicated in Table II, application no. 21, columns 5 or 7, and conferring an increase in the amount of the respective fine chemical according to table II, application no. 21, column 6 in an organism or a part thereof;
   or which encompasses a sequence which is complementary thereto;
whereby, preferably, the nucleic acid molecule according to (a) to (I) distinguishes over the sequence indicated in Table I, application no. 21,columns 5 or 7, by one or more nucleotides. In one embodiment, the nucleic acid molecule does not consist of the sequence shown and indicated in Table I, application no. 21, columns 5 or 7,
In one embodiment, the nucleic acid molecule is less than 100%, 99,999%, 99,99%, 99,9% or 99% identical to a sequence indicated in Table I, application no. 21, columns 5 or 7,.
In another embodiment, the nucleic acid molecule does not encode a polypeptide of a sequence indicated in Table II, application no. 21, columns 5 or 7,.
In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 21, columns 5 or 7,.
In a further embodiment the nucleic acid molecule does not encode a polypeptide sequence as indicated in Table II, application no. 21, columns 5 or 7,.
Accordingly, in one embodiment, the nucleic acid molecule of the differs at least in one or more residues from a nucleic acid molecule indicated in Table I, application no. 21, columns 5 or 7,.
Accordingly, in one embodiment, the nucleic acid molecule of the present invention encodes a polypeptide, which differs at least in one or more amino acids from a polypeptide indicated in Table II, application no. 21, columns 5 or 7,.
In another embodiment, a nucleic acid molecule indicated in Table I, application no. 21, columns 5 or 7,.
Accordingly, in one embodiment, the protein encoded by a sequences of a nucleic acid accoriding to (a) to (I) does not consist of a sequence as indicated in Table II, application no. 21, columns 5 or 7,.
In a further embodiment, the protein of the present invention is at least 30 %, 40 %, 50%, or 60% identical to a protein sequence indicated in Table II, application no. 21, columns 5 or 7, and less than 100%, preferably less than 99,999%, 99,99% or 99,9%, more preferably less than 99%, 985, 97%, 96% or 95% identical to a sequence as indicated in Table I, columns 5 or 7.

**[0205.0.0.21] to [0206.0.0.21] : see [0205.0.0.0] to [0206.0.0.0]**

**[0207.0.21.21]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.21]** to **[0226.0.0.21]** for the disclosure of the paragraphs [0208.0.0.21] to [0226.0.0.21] see paragraphs [0208.0.0.0] to [0226.0.0.0] above.

[0227.0.21.21] The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence indicated in Table I, application no. 21, columns 5 or 7, or its derivatives, it is advantageous to express and/or mutate further genes in the organisms. Especially advantageously, additionally at least one further gene of the acetyl-CoA or malonyl-CoA metabolic pathway or a polypeptide having a very long chain fatty acid acyl (VLCFA) CoA synthase activity, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table I, application no. 21, columns 5 or 7, with genes which generally support or enhance to growth or yield of the target organismen, for example genes which lead to faster growth rate of microorganisms or genes which produces stress-, pathogen, or herbicide resistant plants.

**[0228.0.21.21]** In a further embodiment of the process of the invention, therefore, organisms are grown, in which there is simultaneous overexpression of at least one nucleic acid or one of the genes which code for proteins involved in the glycerol metabolism, in particular in synthesis of glycerol.

**[0229.0.21.21]** Further advantageous nucleic acid sequences which can be expressed in combination with the sequences indicated in Table I, application no. 21, columns 5 or 7, used in the process and/or the abovementioned biosynthesis genes are the sequences encoding further genes of the fatty acid pathway, such as acetyl-CoA or malonyl-CoA or a polypeptide having a very long chain fatty acid acyl (VLCFA) CoA synthase activity. These genes can lead to an increased synthesis of the VLCFAs.

**[0230.0.0.21]** for the disclosure of this paragraph see paragraph [0230.0.0.0] above.

[0231.0.21.21] In a further advantageous embodiment of the process of the invention, the organisms used in the process are those in which simultaneously a glycerol and/or a lipid degrading protein is attenuated, in particular by reducing the rate of expression of the corresponding gene. A person skilled in the art knows for example, that the inhibition or repression of a glycerol or lipid degrading enzyme will result in an increased accumulation of glycerol and/or total lipids in plants.

**[0232.0.0.21] to [0276.0.0.21]** for the disclosure of the paragraphs [0232.0.0.21] to [0276.0.0.21] see paragraphs [0232.0.0.0] to [0276.0.0.0] above.

**[0277.0.21.21]** The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.21] to [0282.0.0.21]** for the disclosure of the paragraphs [0278.0.0.21] to [0282.0.0.21] see paragraphs [0278.0.0.0] to [0282.0.0.0] above.

**[0283.0.21.21]** Moreover, native polypeptide conferring the increase of the respective fine chemical in an organism or part thereof can be isolated from cells, for example using the antibody of the present invention as described below, e.g. an antibody against a protein as indicated in Table II, application no. 21, column 3, or an antibody against a polypeptide as indicated in Table II, application no. 21, columns 5 or 7, which can be produced by standard techniques utilizing the polypeptid of the present invention or fragment thereof. Preferred are monoclonal antibodies specifically binding to polypeptides as indicated in Table II, application no. 21, columns 5 or 7, more preferred specifically binding to polypeptides as indicated in Table II, application no. 21, column 5.

**[0284.0.0.21]** for the disclosure of this paragraph see [0284.0.0.0] above.

**[0285.0.21.21]** In one embodiment, the present invention relates to a polypeptide having a sequence as indicated in Table II, application no. 21, columns 5 or 7, or as coded by a nucleic acid molecule as indicated in Table I, application no. 21, columns 5 or 7, or functional homologues thereof.

**[0286.0.21.21]** In one advantageous embodiment, in the method of the present invention the activity of a polypeptide is increased which comprises or consists of a consensus sequence as indicated in Table IV, application no. 21, column 7, and in one another embodiment, the present invention relates to a polypeptide comprising or consisting of a consensus sequence as indicated in Table IV, application no. 21, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, application no. 21, column 7.

**[0287.0.0.21] to [0290.0.0.21]** for the disclosure of the paragraphs [0287.0.0.21] to [0290.0.0.21] see paragraphs [0287.0.0.0] to [0290.0.0.0] above.

[0291.0.21.21] In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II, application no. 21, columns 5 or 7, by one or more amino acids. In one embodiment, polypeptide distinguishes form a sequence as indicated in Table II, application no. 21, columns 5 or 7, by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 21, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II, application no. 21, columns 5 or 7,.

**[0292.0.0.21]** for the disclosure of this paragraph see [0292.0.0.0] above.

**[0293.0.21.21]** In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II, application no. 21, columns 5 or 7, by one or more amino acids. In an other embodiment, said polypeptide of the invention does not consist of the sequence as indicated in Table II, application no. 21, columns 5 or 7,.
In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 21, columns 5 or 7.

**[0294.0.21.21]** In one embodiment, the present invention relates to a polypeptide having an activity of a protein as indicated in Table II, application no. 21, column 3, which distinguishes over a sequence as indicated in Table II, application no. 21, columns 5 or 7, by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.21] to [0297.0.0.21]** for the disclosure of the paragraphs [0295.0.0.21] to [0297.0.0.21] see paragraphs [0295.0.0.0] to [0297.0.0.0] above.

**[00297.1.21.21]** Non polypeptide of the invention-chemicals are e.g. polypeptides having not the activity of a polypeptide indicated in Table II, application no. 21, columns 3, 5 or 7.

**[0298.0.21.21]** A polypeptide of the invention can participate in the process of the present invention. The polypeptide or a portion thereof comprises preferably an amino acid sequence which is sufficiently homologous to an amino acid sequence as indicated in Table II, application no. 21, columns 5 or 7.The portion of the protein is preferably a biologically active portion as described herein. Preferably, the polypeptide used in the process of the invention has an amino acid sequence identical to a sequence as indicated in Table II, application no. 21, columns 5 or 7.

**[0299.0.21.21]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, application no. 21, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 21, columns 5 or 7, or which is homologous thereto, as defined above.

**[0300.0.21.21]** In one embodiment, the present invention relates to a polypeptide having an activity of a protein as indicated in Table II, application no. 21, column 3, which distinguishes over a sequence as indicated in Table II, application no. 21, columns 5 or 7, by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0301.0.0.21]** for the disclosure of this paragraph see [0301.0.0.0] above.

**[0302.0.21.21]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 21, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.21]** for the disclosure of this paragraph see [0303.0.0.0] above.

**[0304.0.21.21]** Manipulation of the nucleic acid molecule of the invention may result in the production of a protein having essentially the activity of the polypeptides as indicated in Table II, application no. 21, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.21] to [0308.0.0.21]** for the disclosure of the paragraphs [0305.0.0.21] to [0308.0.0.21] see paragraphs [0305.0.0.0] to [0308.0.0.0] above.

**[0306.1.0.21]** %

**[0309.0.21.21]** In one embodiment, a reference to a protein (= polypeptide) of the invention or as indicated in Table II, application no. 21, columns 5 or 7, refers to a polypeptide having an amino acid sequence corresponding to the polypeptide of the invention or used in the process of the invention, whereas an "other polypeptide" not being indicated in Table II, application no. 21, columns 5 or 7, refers to a polypeptide having an amino acid sequence corresponding to a protein which is not substantially homologous to a polypeptide of the invention, preferably which is not substantially homologous to a polypeptide as indicated in Table II, application no. 21, columns 5 or 7, e.g., a protein which does not confer the activity described herein or annotated or known for as indicated in Table II, application no. 21, column 3, and which is derived from the same or a different organism. In one embodiment, an "other polypeptide" not being indicated in Table II, application no. 21, columns 5 or 7, does not confer an increase of the respective fine chemical in an organism or part thereof.

**[0310.0.0.21] to [0334.0.0.21]** for the disclosure of the paragraphs [0310.0.0.21] to [0334.0.0.21] see paragraphs [0310.0.0.0] to [0334.0.0.0] above.

**[0335.0.21.21]** The dsRNAi method has proved to be particularly effective and advantageous for reducing the expression of a nucleic acid sequences as indicated in Table I, application no. 21, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, application no. 21, columns 5 or 7, and/or homologs thereof. In a double-stranded RNA molecule for reducing the expression of a protein encoded by a nucleic acid sequence as indicated in Table I, application no. 21, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.21] to [0342.0.0.21]** for the disclosure of the paragraphs [0336.0.0.21] to [0342.0.0.21] see paragraphs [0336.0.0.0] to [0342.0.0.0] above.

**[0343.0.21.21]** As has already been described, 100 % sequence identity between the dsRNA and a gene transcript of a nucleic acid sequence as indicated in Table I, application no. 21, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 21, columns 5 or 7, or homologs thereof of the one organism, may be used to suppress the corresponding expression in another organism.

**[0344.0.0.21] to [0361.0.0.21]** for the disclosure of the paragraphs [0344.0.0.21] to [0361.0.0.21] see paragraphs [0344.0.0.0] to [0361.0.0.0] above.

**[0362.0.21.21]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 21, columns 5 or 7, e.g. encoding a polypeptide having protein activity, as indicated in Table II, application no. 21, columns 3. Due to the above-mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, application no. 21, columns 5 or 7, means herein that due to modulation or manipulation of the genome, an activity as annotated for a polypeptide as indicated in Table II, application no. 21, column 3, e.g. having a sequence as indicated in Table II, application no. 21, columns 5 or 7, is increased in a cell or an organism or a part thereof. Examples are described above in context with the process of the invention.

**[0363.0.0.21]** for the disclosure of this paragraph see paragraph [0363.0.0.0] above.

**[0364.0.21.21]** A naturally occurring expression cassette - for example the naturally occurring combination of a promoter of a gene encoding a polypeptide of the invention as indicated in Table II, application no. 21, column 3, with the corresponding protein-encoding sequence as indicated in Table I, application no. 21, column 5, becomes a transgenic expression cassette when it is modified by non-natural, synthetic "artificial" methods such as, for example, mutagenization. Such methods have been described (US 5,565,350; WO 00/15815; also see above).

**[0365.0.0.21] to [0373.0.0.21]** for the disclosure of the paragraphs [0365.0.0.21], to [0373.0.0.21] see paragraphs [0365.0.0.0] to [0373.0.0.0] above.

**[0374.0.21.21]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. Glycerol, in particular the respective fine chemical, produced in the process according to the invention may, however, also be isolated from the plant in the form of their free glycerol, in particular the free respective fine chemical, or bound in or to compounds or moieties, as for example but not limited to in mono-, di- or triacylglyceroles, phosphoglycerides, monoacylglycerol phosphate or diacylglycerol phosphate. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.21] and [0376.0.0.21]** for the disclosure of the paragraphs [0375.0.0.21] and [0376.0.0.21] see paragraphs [0375.0.0.0] and [0376.0.0.0] above.

**[0377.0.21.21]** Accordingly, the present invention relates also to a process whereby the produced glycerol and/or total lipid is isolated.

[0378.0.21.21] In this manner, more than 50% by weight, advantageously more than 60% by weight, preferably more than 70% by weight, especially preferably more than 80% by weight, very especially preferably more than 90% by weight, of the glycerol and/or total lipid produced in the process can be isolated. The resulting glycerol and/or total lipid can, if appropriate, subsequently be further purified, if desired mixed with other active ingredients such as vitamins, amino acids, carbohydrates, antibiotics and the like, and, if appropriate, formulated.

**[0379.0.21.21]** In one embodiment the product produced by the present invention is a mixture of the respective fine chemicals glycerol and total lipids.

**[0380.0.21.21]** The glycerol or total lipids obtained in the process by carrying out the invention is suitable as starting material for the synthesis of further products of value. For example, they can be used in combination with each other or alone for the production of pharmaceuticals, foodstuffs, animal feeds or cosmetics. Accordingly, the present invention relates to a method for the production of pharmaceuticals, food stuff, animal feeds, nutrients or cosmetics comprising the steps of the process according to the invention, including the isolation of the glycerol composition produced or the respective fine chemical produced if desired and formulating the product with a pharmaceutical acceptable carrier or formulating the product in a form acceptable for an application in agriculture. A further embodiment according to the invention is the use of the glycerol and/or total lipid produced in the process or of the transgenic organism in animal feeds, foodstuffs, medicines, food supplements, cosmetics or pharmaceuticals or for the production of glycerol e.g. after isolation of the respective fine chemical or without, e.g. in situ, e.g in the organism used for the process for the production of the respective fine chemical.

**[0381.0.0.21] and [0382.0.0.21]** for the disclosure of the paragraphs [0381.0.0.21] and [0382.0.0.21] see paragraphs [0381.0.0.0] and [0382.0.0.0] above.

**[0383.0.21.21] %**

**[0384.0.0.21]** for the disclosure of this paragraph see [0384.0.0.0] above.

[0385.0.21.21] The fermentation broths obtained in this way, containing in particular glycerol and/or total lipids in mixtures with other organic acids, amino acids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3 g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.21.21]** Accordingly, it is possible to purify the glycerol produced according to the invention further. For this purpose, the product-containing composition is subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.21] to [0392.0.0.21]** for the disclosure of the paragraphs [0387.0.0.21] to [0392.0.0.21] see paragraphs [0387.0.0.0] to [0392.0.0.0] above.

[0393.0.21.21] In one embodiment, the present invention relates to a method for the identification of a gene product conferring an increase in the fine chemical production in a cell, comprising the following steps:
a) contacting, - e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
b) identifying the nucleic acid molecules, which hybridize under relaxed stringent conditions with the nucleic acid molecule of the present invention in particular to the nucleic acid molecule sequence as indicated in Table I, application no. 21, columns 5 or 7, and, optionally, isolating the full length cDNA clone or complete genomic clone;
c) introducing the candidate nucleic acid molecules in host cells, preferably in a plant cell or a microorganism, appropriate for producing the fine chemical;
d) expressing the identified nucleic acid molecules in the host cells;
e) assaying the the fine chemical level in the host cells; and
f) identifying the nucleic acid molecule and its gene product which expression confers an increase in the the fine chemical level in the host cell after expression compared to the wild type.

**[0394.0.0.21] to [0552.0.0.21]** for the disclosure of the paragraphs [0394.0.0.21] to [0552.0.0.21] see paragraphs [0394.0.0.7] to [0552.0.0.7] above.

**[0553.0.21.21]** A process for the production of glycerol resp., which comprises
(a) increasing or generating the activity of a protein as indicated in Table II, application no. 21, columns 5 or 7, or a functional equivalent thereof in a non-human organism, or in one or more parts thereof; and
(b) growing the organism under conditions which permit the production of glycerol resp. in said organism.
A process for the production of glycerol resp., comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 21, columns 5 or 7, , or a fragment thereof, which confers an increase in the amount of the fine chemical as indicated in table II, column 6, e.g of glycerol resp., in an organism or a part thereof;
b) nucleic acid molecule comprising of a nucleic acid molecule as indicated in Table I, application no. 21, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of glycerol resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of glycerol resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of glycerol resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 21, column 7, and conferring an increase in the amount of the fine chemical as indicated in table II, column 6, e.g of glycerol resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of glycerol resp., in an organism or a part thereof;
h) nucleic acid molecule encoding a polypeptide comprising a consensus sequence as indicated in Table IV, application no. 21, column 7, and conferring an increase in the amount of the fine chemical as indicated in table II, column 6, e.g of glycerol resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical as indicated in table II, column 6, e.g of glycerol resp., in an organism or a part thereof.
or comprising a sequence which is complementary thereto.
The process as mentioned above, comprising recovering of the free or bound glycerol resp..
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound glycerol resp., produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 21, columns 5 or 7, or a fragment thereof, which confers an increase in the amount of the fine chemical as indicated in table II, column 6, e.g of glycerol resp., in an organism or a part thereof;
b) nucleic acid molecule comprising of a nucleic acid molecule as indicated in Table I, application no. 21, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of glycerol resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of glycerol resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of glycerol resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 21, column 7, and conferring an increase in the amount of the fine chemical as indicated in table II, column 6, e.g of glycerol resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of glycerol resp., in an organism or a part thereof;
h) nucleic acid molecule encoding a polypeptide comprising a consensus sequence as indicated in Table IV, application no. 21, column 7, and conferring an increase in the amount of the fine chemical as indicated in table II, column 6, e.g of glycerol resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemiccal as indicated in table II, column 6, e.g of glycerol resp., in an organism or a part thereof.
whereby the nucleic acid molecule distinguishes over the sequence as indicated in Table I, application no. 21, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 21, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of glycerol resp., in an organism or a part thereof comprising:
(a) contacting cells, tissues , plants or microorganisms which express the a polypeptide encoded by the nucleic acid molecule as mentioned above conferring an increase in the amount of glycerol resp., in an organism or a part thereof with a candidate compound or a sample comprising a plurality of compounds under conditions which permit the expression the polypeptide;
(b) assaying the glycerol resp., level or the polypeptide expression level in the cell, tissue, plant or microorganism or the media the cell, tissue, plant or microorganisms is cultured or maintained in; and
(c) identifying a agonist or antagonist by comparing the measured glycerol resp., level or polypeptide expression level with a standard glycerol resp., or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased glycerol resp., production in a plant or microorganism, comprising the steps:
(a) culturing a plant cell or tissue or microorganism or maintaining a plant expressing the polypeptide encoded by the nucleic acid molecule as mentioned above conferring an increase in the amount of glycerol resp., in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of glycerol rersp., in an organism or a part thereof;
(b) identifying if the compound is an effective agonist by detecting the presence or absence or increase of a signal produced by said readout system.
A method for the identification of a gene product conferring an increase in glycerol resp., production in a cell, comprising the following steps:
(a) contacting the nucleic acid molecules of a sample, which can contain a candidate gene encoding a gene product conferring an increase in glycerol resp., after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing glycerol resp.;
(d) expressing the identified nucleic acid molecules in the host cells;
(e) assaying the glycerol rersp., level in the host cells; and
(f) identifying nucleic acid molecule and its gene product which expression confers an increase in the glycerol resp., level in the host cell in the host cell after expression compared to the wild type.
A method for the identification of a gene product conferring an increase in glycerol resp., production in a cell, comprising the following steps:
(a) identifiying in a data bank nucleic acid molecules of an organism; which can contain a candidate gene encoding a gene product conferring an increase in the glycerol resp., amount or level in an organism or a part thereof after expression, and which are at least 20% homolog to the nucleic acid molecule as mentioned above;
(b) introducing the candidate nucleic acid molecules in host cells appropriate for producing glycerol resp.,;
(c) expressing the identified nucleic acid molecules in the host cells;
(d) assaying the glycerol resp., level in the host cells; and
(e) identifying nucleic acid molecule and its gene product which expression confers an increase in the glycerol resp., level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of glycerol resp., after expression.
Use of the polypeptide as mentioned above or the nucleic acid construct as mentioned above or the gene product identified according to the method as mentioned above for identifying compounds capable of conferring a modulation of glycerol resp., levels in an organism.
Agrochemical, pharmaceutical, food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
The method as mentioned above, the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above, wherein the fine chemical is glycerol.
A host cell or plant as mentioned above which is resistant to a herbicide inhibiting the biosynthesis of glycerol.
A process for the increased production of total lipids, comprising the increasing or generating in an organism or a part thereof the expression of at least one nucleic acid molecule comprising a nucleic acid molecule selected from the group consisting of:
a) nucleic acid molecule encoding a polypeptide as indicated in Table II, application no. 21, columns 5 or 7, or a fragment thereof, which confers an increase in the amount of total lipids in an organism or a part thereof;
b) nucleic acid molecule comprising of a nucleic acid molecule as indicated in Table I, application no. 21, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of total lipids in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of total lipids in an organism or a part thereof;
e) nucleic acid molecule which hybidizes with a nucleic acid molecule of (a) to (c) under stringent hybridisation conditions and conferring an increase in the amount of total lipids in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 21, column 7, and conferring an increase in the amount of total lipids in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of total lipids in an organism or a part thereof;
h) nucleic acid molecule encoding a polypeptide comprising a consensus as indicated in Table IV, application no. 21, column 7, and conferring an increase in the amount of total lipids in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of total lipids in an organism or a part thereof.
or comprising a sequence which is complementary thereto.
The use of the nucleic acid molecule encoding a polypeptide as indicated in Table II, application no. 21, columns 5 or 7, or a fragment thereof, which confers an increase in the amount of total lipids in an organism or a part thereof for the identification of a nucleic acid molecule conferring an increase in the amount of total lipids after expression.

### New genes for a process for the production of fine chemicals

[0000.0.0.24] In a further embodiment, the present invention relates to a further process for the production of fine chemicals as defined below and the corresponding embodiments as described herein as follows.

**[0001.0.0.24]** see **[0001.0.0.0]**

**[0002.0.24.24]** Carotenoids are red, yellow and orange pigments that are widely distributed in nature. Although specific carotenoids have been identified in photosynthetic centers in plants, in bird feathers, in crustaceans and in marigold petals, they are especially abundant in yellow-orange fruits and vegetables and dark green, leafy vegetables. Of the more than 700 naturally occurring carotenoids identified thus far, as many as 50 may be absorbed and metabolized by the human body. To date, only 14 carotenoids have been identified in human serum.
In animals some carotenoids (particularly beta-carotene) serve as dietary precursors to Vitamin A, and many of them may function as fat-soluble antioxidants. In plants carotenes serve for example as antioxidants to protect the highly reactive photosystems and act as accessory photopigments. In vitro experiments have shown that lycopene, alpha-carotene, zeaxanthin, lutein and cryptoxanthin quench singlet oxygen and inhibit lipid peroxidation. The isolation and identification of oxidized metabolites of lutein, zeaxanthin and lycopene provide direct evidence of the antioxidant action of these carotenoids.
Carotenoids are 40-carbon (C₄₀) terpenoids generally comprising eight isoprene (C₅) units joined together. Linking of the units is reversed at the center of the molecule. "Ketocarotenoid" is a general term for carotenoid pigments that contain a keto group in the ionene ring portion of the molecule, whereas "hydroxycarotenoid" refers to carotenoid pigments that contain a hydroxyl group in the ionene ring. Trivial names and abbreviations will be used throughout this disclosure, with IUPAC-recommended semi-systematic names usually being given in parentheses after first mention of a trivial name.
Carotenoids are synthesized from a five carbon atom metabolic precursor, isopentenyl pyrophosphate (IPP). There are at least two known biosynthetic pathways in the formation of IPP, the universal isoprene unit. One pathway begins with mevalonic acid, the first specific precursor of terpenoids, formed from acetyl-CoA via HMG-CoA (3-hydroxy-3-methylglutaryl-CoA), that is itself converted to isopentenyl pyrophosphate (IPP). Later, condensation of two geranylgeranyl pyrophosphate (GGPP) molecules with each other produces colorless phytoene, which is the initial carotenoid. Studies have also shown the existence of an alternative, mevalonate-independent pathway for IPP formation that was characterized initially in several species of eubacteria, a green alga, and in the plastids of higher plants. The first reaction in this alternative pathway is the transketolase-type condensation reaction of pyruvate and D-glyceraldehylde-3-phosphate to yield 1-deoxy-D-xylulose-5-phosphate (DXP) as an intermediate.
Through a series of desaturation reactions, phytoene is converted to phytofluene, - carotene, neurosporene and finally to lycopene. Subsequently, lycopene is converted by a cyclization reaction to β-carotene that contains two β-ionene rings. A keto-group and/or a hydroxyl group are introduced into each ring of β-carotene to thereby synthesize canthaxanthin, zeaxanthin, astaxanthin. A hydroxylase enzyme has been shown to convert canthaxanthin to astaxanthin. Similarly, a ketolase enzyme has been shown to convert zeaxanthin to astaxanthin. The ketolase also converts β-carotene to canthaxanthin and the hydroxylase converts β-carotene to zeaxanthin.
Carotenoids absorb light in the 400-500 nm region of the visible spectrum. This physical property imparts the characteristic red/yellow color of the pigments. A conjugated backbone composed of isoprene units is usually inverted at the center of the molecule, imparting symmetry. Changes in geometrical configuration about the double bonds result in the existence of many *cis-* and *trans-* isomers. Hydroxylated, oxidized, hydrogenated or ring-containing derivatives also exist. Hydrocarbon carotenoids are classified as carotenes while those containing oxygen are known as xanthophylls.
In animals, carotenoids are absorbed from the intestine with the aid of dietary fat and incorporated into chylomicrons for transport in the serum. The different structural features possessed by carotenoids account for selective distribution in organ tissue, biological activity and pro-vitamin A potency, or *in vivo* conversion to vitamin A. Due to the hydrophobic character, carotenoids are associated with lipid portions of human tissues, cells, and membranes. In general, 80-85% of carotenoids are distributed in adipose tissue, with smaller amounts found in the liver, muscle, adrenal glands, and reproductive organs. Approximately 1% circulate in the serum on high and low density lipoproteins. Serum concentrations are fairly constant and slow to change during periods of low intake. The estimated half-life was estimated to be 11-14 days for lycopene, alpha-carotene, beta-carotene, lutein and zeaxanthin. Evidence for the existence of more than one body pool has been published. The major serum carotenoids are alpha-carotene, beta-carotene, lutein, zeaxanthin, lycopene and cryptoxanthin. Smaller amounts of polyenes such as phytoene and phytofluene are also present.
Human serum levels reflect lifestyle choices and dietary habits within and between cultures. Approximately only 15 circulate in the blood, on HDL and LDL. Variations can be attributed to different intakes, unequal abilities to absorb certain carotenoids, and different rates of metabolism and tissue uptake. Decreased serum levels occur with alcohol consumption, the use of oral contraceptives, smoking and prolonged exposure to UV light.
Alpha-Carotene, beta-carotene and beta-cryptoxanthin can be converted to retinol or vitamin A in the intestine and liver by the enzyme 15-15'-b-carotenoid dioxygenase. Such *in vivo* formation of retinol appears to be homeostatically controlled, such that conversion to retinol is limited in persons having adequate vitamin A status.

**[0003.0.24.24]** The established efficacy of beta-carotene in quenching singlet oxygen and intercepting deleterious free radicals and reactive oxygen species makes it part of the diverse antioxidant defense system in humans. Reactive oxygen species have been implicated in the development of many diseases, including ischemic heart disease, various cancers, cataracts and macular degeneration. Because the conjugated polyene portion of beta-carotene confers its antioxidant capability and all carotenoids possess this structural feature, research efforts have been directed at evaluating the efficacy of other carotenoids in the prevention of free radical-mediated diseases. Indeed, *in vitro* experiments have demonstrated that lycopene, alpha-carotene, zeaxanthin, lutein and cryptoxanthin quench singlet oxygen and inhibit lipid peroxidation. The isolation and identification of oxidized metabolites of lutein, zeaxanthin and lycopene may provide direct evidence of the antioxidant action of these carotenoids.
In addition to antioxidant capability, other biological actions of carotenoids include the ability to enhance immunocompetence and in vitro gap junction communication, reduce or inhibited mutagenesis and inhibit cell transformations in vitro.
Many epidemiological studies have established an inverse correlation between dietary intake of yellow-orange fruit and dark green, leafy vegetables and the incidence of various cancers, especially those of the mouth, pharynx, larynx, esophagus, lung, stomach, cervix and bladder. While a number of protective compounds may be responsible for this observation, the co-incidence of carotenoids in these foods has been noted. Because nutritionists and medical professionals currently recognize the occurrence of a large number of distinct carotenoids in food, interest in their functions and biological impact on health is burgeoning.
Lutein exists in the retina. It functions to protect photoreceptor cells from light-generated oxygen radicals, and thus plays a key role in preventing advanced macular degeneration. Lutein possesses chemopreventive activity, induces gap junction communication between cells and inhibits lipid peroxidation in vitro more effectively than beta-carotene, alpha-carotene and lycopene. High levels of lutein in serum have been inversely correlated with lung cancer.
In addition to lutein, zeaxanthin exists in the retina and confers protection against macular degeneration. Zeaxanthin is also prevalent in ovaries and adipocyte tissue. This xanthophyll does not possess provitamin A activity.
Alcohol consumption has been shown to influence lipid peroxidation. Anhydrolutein, an oxidative by-product of lutein and zeaxanthin, was higher in plasma after alcohol ingestion, while concentrations of these xanthophylls were reduced. Lutein and zeaxanthin may therefore have protective effects against LDL oxidation.
The all*-trans* isomer of Lycopene is typically quantified in serum, although signals for 9-, 13- and 15-cis isomers are detectable and account for as much as 50% of the total lycopene. In experiments performed *in vitro,* lycopene quenched singlet oxygen more efficiently than alpha-carotene, beta-carotene, zeaxanthin, lutein and cryptoxanthin. Lycopene induces gap junction communication, inhibits lipid peroxidation and has displays chemopreventive activity. Serum levels of lycopene have been inversely related to the risk of cancer in the pancreas and cervix. This carotenoid has been identified in tissues of the thyroid, kidneys, adrenals, spleen, liver, heart, testes and pancreas. Lycopene is not converted to retinol *in vivo.*
beta-Cryptoxanthin is capable of quenching singlet oxygen. beta-Cryptoxanthin is used to color butter. beta-Cryptoxanthin exhibits provitamin A activity.
The all-trans isomer of this carotenoid is the major source of dietary retinoids, due to its high provitamin A activity. One molecule of trans-beta-carotene can theoretically provide two molecules of trans retinaldehyde in vivo. Signals for 13- and 15-cis isomers of beta-carotene are also observed in the carotenoid profile and account for 10% or less of the total beta-carotene in serum. beta-Carotene quenches singlet oxygen, induces gap junction communication and inhibits lipid peroxidation. High serum levels of beta-carotene are correlated with low incidences of cancer in the mouth, lung, breast, cervix, skin and stomach. beta-Carotene has been identified in tissues of the thyroid, kidney, spleen, liver, heart, pancreas, fat, ovaries and adrenal glands.
alpha-Carotene is similar to beta-carotene in its biological activity, but quenches singlet oxygen more effectively. alpha-Carotene improves gap junction communication, prevents lipid peroxidation and inhibits the formation and uptake of carcinogens in the body. High serum levels have been associated with lower risks of lung cancer. With one half the provitamin A potency of beta-carotene, alpha-carotene also restores normal cell growth and differentiation. Serum levels are usually between 10 and 20% of the values for total beta-carotene.
Alpha-Carotene, beta-carotene and beta-cryptoxanthin can be converted to Vitamin A in the intestine and liver. Vitamin A is essential for the immune response and is also involved in other defenses against infectious agents. Nevertheless, in many individuals, this conversion is slow and ineffectual, particularly for older. Some individuals are known as non or low-responders because they do not convert beta-carotene to Vitamin A at the rate as expected. A number of factors can inhibit this conversion of beta-carotene to Vitamin A. The major reason why so many Americans have a poor vitamin A status is the regular use of excessive alcohol. Intestinal parasites can be a factor. And, any prescription drug that requires liver metabolism will decrease the liver conversion of beta-carotene to retinol in the liver. Diabetics and individuals with hypothyroidism or even borderline hypothyroidism are likely to be low-responders.
[0004.0.24.24] In plants, approximately 80-90% of the carotenoids present in green, leafy vegetables such as broccoli, kale, spinach and brussel sprouts are xanthophylls, whereas 10-20% are carotenes. Conversely, yellow and orange vegetables including carrots, sweet potatoes and squash contain predominantly carotenes. Up to 60% of the xanthophylls and 15% of the carotenes in these foods are destroyed during microwave cooking. Of the xanthophylls, lutein appears to be the most stable.
Lutein occurs in mango, papaya, oranges, kiwi, peaches, squash, peas, lima beans, green beans, broccoli, brussel sprouts, cabbage, kale, lettuce, prunes, pumpkin, sweet potatoes and honeydew melon. Commercial sources are obtained from the extraction of marigold petals. Lutein does not possess provitamin A activity.
Dietary sources of Zeaxanthin include peaches, squash, apricots, oranges, papaya, prunes, pumpkin, mango, kale, kiwi, lettuce, honeydew melon and yellow corn.
The red color of fruits and vegetables such as tomatoes, pink grapefruit, the skin of red grapes, watermelon and red guavas is due to lycopene. Other dietary sources include papaya and apricots.
beta-Cryptoxanthin occurs in oranges, mango, papaya, cantaloupe, peaches, prunes, squash.
Dietary sources of beta-Carotene include mango, cantaloupe, carrots, pumpkin, papaya, peaches, prunes, squash, sweet potato, apricots, cabbage, lima beans, green beans, broccoli, brussel sprouts, kale, kiwi, lettuce, peas, spinach, tomatoes, pink grapefruit, honeydew melon and oranges.
Dietary sources of alpha-Carotene include sweet potatoes, apricots, pumpkin, cantaloupe, green beans, lima beans, broccoli, brussel sprouts, cabbage, kale, kiwi, lettuce, peas, spinach, prunes, peaches, mango, papaya, squash and carrots.

**[0005.0.24.24]**Some carotenoids occur particularly in a wide variety of marine animals including fish such as salmonids and sea bream, and crustaceans such as crab, lobster, and shrimp. Because animals generally cannot biosynthesize carotenoids, they obtain those carotenoids present in microorganisms or plants upon which they feed.
Carotenoids e.g. xanthophylls, e.g. as astaxanthin, supplied from biological sources, such as crustaceans, yeast, and green alga is limited by low yield and costly extraction methods when compared with that obtained by organic synthetic methods. Usual synthetic methods, however, produce by-products that can be considered unacceptable. It is therefore desirable to find a relatively inexpensive source of carotenoids, in particular xantophylls, to be used as a feed supplement in aquaculture and as a valuable chemical for other industrial uses and for diets. Sources of Xanthophylls include crustaceans such as a krill in the Antarctic Ocean, cultured products of the yeast *Phaffia,* cultured products of a green alga *Haematococcus pluvialis,* and products obtained by organic synthetic methods. However, when crustaceans such as a krill or the like are used, a great deal of work and expense are required for the isolation of xanthophylls from contaminants such as lipids and the like during the harvesting and extraction. Moreover, in the case of the cultured product of the yeast *Phaffia,* a great deal of expense is required for the gathering and extraction of astaxanthin because the yeast has rigid cell walls and produces xantophylls only in a low yield. One approach to increase the productivity of some xantophylls' production in a biological system is to use genetic engineering technology.

**[0006.0.24.24]In** many plants, lycopene is a branch point in carotenoid biosynthesis. Thus, some of the plant's lycopene is made into beta-carotene and zeaxanthin, and sometimes zeaxanthin diglucoside, whereas remaining portions of lycopene are formed into alpha-carotene and lutein (3,3'-dihydroxy-α-carotene), another hydroxylated compound. Carotenoids in higher plants; i.e., angiosperms, are found in plastids; i.e., chloroplasts and chromoplasts. Plastids are intracellular storage bodies that differ from vacuoles in being surrounded by a double membrane rather than a single membrane. Plastids such as chloroplasts can also contain their own DNA and ribosomes, can reproduce independently and synthesize some of their own proteins. Plastids thus share several characteristics of mitochondria. In leaves, carotenoids are usually present in the grana of chloroplasts where they provide a photoprotective function. Beta-carotene and lutein are the predominant carotenoids, with the epoxidized carotenoids violaxanthin and neoxanthin being present in smaller amounts. Carotenoids accumulate in developing chromoplasts of flower petals, usually with the disappearance of chlorophyll. As in flower petals, carotenoids appear in fruit chromoplasts as they develop from chloroplasts. Most enzymes that take part in conversion of phytoene to carotenes and xanthophylls are labile, membrane-associated proteins that lose activity upon solubilization. In maize, cartonoids were present in horny endosperm (74% to 86%), floury endosperm (9%-23%) and in the germ and bran of the kernel.

[0007.0.24.24]At the present time only a few plants are widely used for commercial colored carotenoid production. However, the productivity of colored carotenoid synthesis in most of these plants is relatively low and the resulting carotenoids are expensively produced.
Dried marigold petals and marigold petal concentrates obtained from so-called xanthophyll marigolds are used as feed additives in the poultry industry to intensify the yellow color of egg yolks and broiler skin. The pigmenting ability of marigold petal meal resides largely in the carotenoid fraction known as the xanthophylls, primarily lutein esters. The xanthophyll zeaxanthin, also found in marigold petals, has been shown to be effective as a broiler pigmenter, producing a highly acceptable yellow to yellow-orange color. Of the xanthophylls, the pigments lutein and zeaxanthin are the most abundant in commercially available hybrids. Structural formulas for lutein and zeaxanthin are shown below.
Carotenoids have been found in various higher plants in storage organs and in flower petals. For example, marigold flower petals accumulate large quantities of esterified lutein as their predominant xanthophyll carotenoid (about 75 to more than 90 percent), with smaller amounts of esterified zeaxanthin. Besides lutein and zeaxanthin, marigold flower petals also typically exhibit a small accumulation of β-carotene and epoxidized xanthophylls, but do not produce or accumulate canthaxanthin or astaxanthin because a 4-keto-β-ionene ring-forming enzyme is absent in naturally-occurring marigolds or their hybrids.

[0008.0.24.24]One way to increase the productive capacity of biosynthesis is to apply recombinant DNA technology. Thus, it would be desirable to produce colored carotenoids generally and, with the use of recent advances in determining carotenoid biosynthesis from β-carotene to xanthophylls to control the production of carotenoids. That type of production permits control over quality, quantity and selection of the most suitable and efficient producer organisms. The latter is especially important for commercial production economics and therefore availability to consumers.
Methods of recombinant DNA technology have been used for some years to improve the production of Xanthophylls in microorganisms, in particular algae or in plants by amplifying individual xanthophyll biosynthesis genes and investigating the effect on xanthophyll production. It is for example reportet, that the five ketocarotenoids, e.g. the xanthophyll astaxanthin could be produced in the nectaries of transgenic tobacco plants. Those transgenic plants were prepared by *Argobacterium tumifaciens-*mediated transformation of tobacco plants using a vector that contained a ketolase-encoding gene from *H. pluvialis* denominated *crtO* along with the Pds gene from tomato as the promoter and to encode a leader sequence. The Pds gene was said by those workers to direct transcription and expression in chloroplasts and/or chromoplast-containing tissues of plants. Those results indicated that about 75 percent of the carotenoids found in the flower of the transformed plant contained a keto group. Further, in maize the phytonene synthase (Psy), Phytone desaturase (Pds), and the - carotene desaturase were identified and it was shown, that PSY activity is an important control point for the regulation of the flux.
Genes suitable for conversion of microorganisms have also been reported (U.S. Patent No. 6,150,130 WO 99/61652). Two different genes that can convert a carotenoid β-ionene ring compound into astaxanthin have been isolated from the green alga *Haematococcus pluvialis.* Zeaxanthin or -carotene were also found in the marine bacteria *Agrobacterium aurantiacum, Alcaligenes PC-1, Erwinia uredovora.* An *A. aurantiacum crtZ* gene was introduced to an *E. coli* transformant that accumulated all-*trans*-β-carotene. The transformant so formed produced zeaxanthin. A gene cluster encoding the enzymes for a carotenoid biosynthesis pathway has been also cloned from the purple photosynthetic bacterium *Rhodobacter capsulatus.* A similar cluster for carotenoid biosynthesis from ubiquitous precursors such as farnesyl pyrophosphate and geranyl pyrophosphate has been cloned from the non-photosynthetic bacteria *Enwinia herbicola.* Yet another carotenoid biosynthesis gene cluster has been cloned from *Erwinia uredovora.* It is yet unknown and unpredictable as to whether enzymes encoded by other organisms behave similarly to that of *A. aurantiacum in vitro* or *in vivo* after transformation into the cells of a higher plant.

**[0009.0.24.24]** In addition to the above said about the biological importance of carotenoids, e.g. in vision, bone growth, reproduction, immune function, gene expression, emboryonic expression, cell division and cell differation, and respiration, it should be mentioned that in the world, the prevalence of vitamin A deficiency ranges from 100 to 250 million children and an estimated 250.000 to 500.000 children go blind each year from vitamin A deficiency.
Thus, it would be advantageous if an algae or other microorganism were available who produce large amounts of β-carotene, beta-cryptoxanthin, lutein, zeaxanthin, or other carotenoids. It might be advantageous that only small amounts or no lutein is produced so that such organisms could be transformed with e.g. one or more of an appropriate hydroxylase gene and/or an appropriate ketolase gene to produce cryptoxanthin, zeaxanthin or astaxanthin. The invention discussed hereinafter relates in some embodiments to such transformed prokaryotic or eukaryotic microorganisms.
It would also be advantageous if a marigold or other plants were available whose flowers produced large amounts of β-carotene, beta-cryptoxanthin, lutein, zeaxanthin, or other carotenoids. It might be advantageous that only small amounts or no lutein is produced so that such plants could be transformed with one or more of an appropriate hydroxylase gene and an appropriate ketolase gene to produce cryptoxanthin, zeaxanthin or astaxanthin from e.g the flowers of the resulting transformants. The invention discussed hereinafter relates in some embodiments to such transformed plants.

[0010.0.24.24] Therefore improving the quality of foodstuffs and animal feeds is an important task of the food-and-feed industry. This is necessary since, for example, as mentioned above beta-carotene, which occur in plants and some microorganisms are limited with regard to the supply of mammals. Especially advantageous for the quality of foodstuffs and animal feeds is as balanced as possible a carotenoids profile in the diet since a great excess of some carotenoids above a specific concentration in the food has only some positive effect. A further increase in quality is only possible via addition of further carotenoids, which are limiting.

[0011.0.24.24] To ensure a high quality of foods and animal feeds, it is therefore necessary to add one or a plurality of carotenoids in a balanced manner to suit the organism.

**[0012.0.24.24]** Accordingly, there is still a great demand for new and more suitable genes which encode enzymes which participate in the biosynthesis of carotenoids, e.g. beta-carotene or its/their precursor, e.g. isopentyl pyrophosphate (IPP), and make it possible to produce them specifically on an industrial scale without unwanted byproducts forming.

In the selection of genes for biosynthesis two characteristics above all are particularly important. On the one hand, there is as ever a need for improved processes for obtaining the highest possible contents of carotenoids like beta-carotene; on the other hand as less as possible byproducts should be produced in the production process.

**[0013.0.0.24]** see **[0013.0.0.0]**

**[0014.0.24.24]** Accordingly, in a first embodiment, the invention relates to a process for the production of a fine chemical, whereby the fine chemical is a carotene or a precursor thereof. In a preferred embodiment, the fine chemical is beta-carotene, resp. Accordingly, in the present invention, the term "the fine chemical" as used herein relates to a "carotene, in particular beta-carotene. Further, the term "the fine chemicals" as used herein also relates to fine chemicals comprising carotene, in particular beta-carotene.

**[0015.0.24.24]** In one embodiment, the term "carotene, in particular beta-carotene," or "the fine chemical" or "the respective fine chemical" means at least one chemical compound with a carotene-, in particular beta-carotene-like activity.

In one embodiment, the term "the fine chemical" means a "carotene, in particular beta-carotene",. In one embodiment, the term "the fine chemical" means beta-carotene depending on the context in which the term is used. Throughout the specification the term "the fine chemical" includes the free fine chemicals, its salts, ester, thioester or bound to other compounds such sugars or sugarpolymers, like glucoside, e.g. diglucoside. In one embodiment, the term "the fine chemical" means beta-carotene, resp." in free form or their salts or their ester or bound to a glucoside, e.g a diglucoside, resp.

[0016.0.24.24] Accordingly, the present invention relates to a process for the production of the respective fine chemical comprising
(a) increasing or generating the activity of one or more
   of a protein as shown in table II, application no. 24, column 3 encoded by the nucleic acid sequences as shown in table I, application no. 24, column 5, in a non-human organism or in one or more parts thereof or
(b) growing the organism under conditions which permit the production of the fine chemical, thus beta-Carotene of the invention or fine chemicals comprising beta-Carotene of the invention, in said organism or in the culture medium surrounding the organism.

[0016.1.24.24] Accordingly, the term "the fine chemical" means in one embodiment "beta-Carotene" in relation to all sequences listed in Tables I to IV, line 47 or homologs thereof.

**[0017.0.0.24]** to **[0019.0.0.24]:** see **[0017.0.0.0]** to **[0019.0.0.0]**

[0020.0.24.24] Surprisingly it was found, that the transgenic expression of the Brassica napus protein as indicated in Table II, column 5, line 47in a plant conferred an increase in beta-Carotene content of the transformed plants. Thus, in one embodiment, said protein or its homologs are used for the production of beta-Carotene.

**[0021.0.0.24]** see **[0021.0.0.0]**

**[0022.0.24.24]**
The sequence of YDR513W from Saccharomyces cerevisiae has been published in Jacq et al., Nature 387 (6632 Suppl), 75-78, 1997, and Goffeau et al., Science 274 (5287), 546-547, 1996, and its activity is being defined as glutathione reductase. Accordingly, in one embodiment, the process of the present invention comprises the use of a protein of the superfamily of glutaredoxin, in particular being involved in deoxyribonucleotide metabolism, cytoplasm, stress response, detoxification, electron transport and membrane-associated energy conservation, preferably a glutathione reductase, or its homolog, e.g. as shown herein, for the production of the respective fine chemical, meaning of carotene, in particular beta-carotene, in particular for increasing the amount in free or bound form in an organism or a part thereof, as mentioned. In one embodiment, in the process of the present invention said activity, e.g. the activity of a glutathione reductase is increased or generated, e.g. from Saccharomyces cerevisiae or a plant or a homolog thereof.

**[0022.1.0.24] to [0023.0.0.24]** see **[0022.1.0.24]** to **[0023.0.0.24]**

**[0023.1.24.24]** Homologs of the polypeptide disclosed in table II, application no. 24, column 5, may have the activity of a protein as disclosed in table I or II, application no. 24, column 3 and may be the polypetides encoded by the nucleic acid molecules indicated in table I , application no. 24, column 7, resp., or may be the polypeptides indicated in table II, application no. 24, column 7, resp.

**[0024.0.0.24]** see **[0024.0.0.0]**

**[0025.0.24.24]In** accordance with the invention, a protein or polypeptide has the "activity of an protein as shown in table II, application no. 24, column 3" if its de *novo* activity, or its increased expression directly or indirectly leads to an increased in the level of the fine chemical indicated in the respective line of table II, application no. 24, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 24, column 3, or which has at least 10% of the original enzymatic or biological activity, preferably 20%, particularly preferably 30%, most particularly preferably 40% in comparison to a protein as shown in the respective line of table II, application no. 24, column 3 of a E. coli or Saccharomyces cerevisae, respectively, protein as mentioned in table I to IV, column 3 respectively and as disclosed in paragraph [0022] of the respective invention.

**[0025.1.0.24] see [0025.1.0.0]**

**[0026.0.0.24]** to **[0033.0.0.24]: see [0026.0.0.0]** to **[0033.0.0.0]**

[0034.0.24.24]Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 24, column 3 its biochemical or genetical causes and the increased amount of the respective fine chemical.

**[0035.0.0.24]** to **[0038.0.0.24]: see [0035.0.0.0]** to **[0038.0.0.0]**

**[0039.0.0.24]** see **[0039.0.0.0]**

**[0040.0.0.24] to [0044.0.0.24]: see [0040.0.0.0] to [0044.0.0.0]**

**[0045.0.24.24]** In one embodiment, the activity of the protein. as indicated in Table II, columns 5 or 7, application no. 24, is increased conferring an increase of the respective fine chemical, indicated in Table II, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more.

**[0046.0.24.24]ln** one embodiment, the activity of the protein as indicated in Table II, columns 5 or 7, application no. 24, is increased conferring an increase of the respective fine chemical, indicated in Table II, column 6 of the respective line confers an increase of the respective fine chemical and of further beta-carotene or their precursors.

**[0047.0.0.24]** to **[0048.0.0.24]:** see **[0047.0.0.0]** to **[0048.0.0.0]**

[0049.0.24.24]A protein having an activity conferring an increase in the amount or level of the fine chemical, has in one embodiment the structure of the polypeptide described herein, in particular of the polypeptides comprising the consensus sequence shown in table IV, application no. 24, column 7 or of the polypeptide as shown in the amino acid sequences as disclosed in table II, application no. 24, columns 5 and 7 or the functional homologues thereof as described herein, or is encoded by the nucleic acid molecule characterized herein or the nucleic acid molecule according to the invention, for example by the nucleic acid molecule as shown in table I, application no. 24, columns 5 and 7 or its herein described functional homologues and has the herein mentioned activity.

[0050.0.24.24] For the purposes of the present invention, the term "the respective fine chemical" also encompass the corresponding salts, such as, for example, the potassium or sodium salts of beta-carotene, resp., or their ester, or glucoside thereof, e.g the diglucoside thereof.

[0051.0.24.24] Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising beta-carotene. Depending on the choice of the organism used for the process according to the present invention, for example a microorganism or a plant, compositions or mixtures of various beta-carotene can be produced.

**[0052.0.0.24]** see **[0052.0.0.0]**

[0053.0.24.24] In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 24, columns 5 and 7 or its homologs activity having herein-mentioned beta-Carotenes of the invention increasing activity; and/or
b) stabilizing a mRNA conferring the increased expression of a protein encoded by the nucleic acid molecule of the invention as shown in table I, application no. 24, columns 5 and 7, e.g. a nucleic acid sequence encoding a polypeptide having the activity of a protein as indicated in table II, application no. 24, columns 5 and 7 or its homologs activity or of a mRNA encoding the polypeptide of the present invention having herein-mentioned beta-Carotenes of the invention increasing activity; and/or
c) increasing the specific activity of a protein conferring the increased expression of a protein encoded by the nucleic acid molecule of the invention or of the polypeptide of the present invention having herein-mentioned beta-Carotene increasing activity, e.g. of a polypeptide having the activity of a protein as indicated in table II, application no. 24, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned beta-Carotenes of the invention increasing activity, e.g. of a polypeptide having the activity of a protein as indicated in table II, application no. 24, columns 5 and 7 or its homologs activity; and/or
e) stimulating activity of a protein conferring the increased expression of a protein encoded by the nucleic acid molecule of the present invention or a polypeptide of the present invention having herein-mentioned beta-Carotenes of the invention increasing activity, e.g. of a polypeptide having the activity of a protein as indicated in table II, application no. 24, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned beta-Carotenes of the invention increasing activity, e.g. of a polypeptide having the activity of a protein as indicated in table II, application no. 24, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned beta-Carotenes of the invention increasing activity, e.g. of a polypeptide having the activity of a protein as indicated in table II, application no. 24, columns 5 and 7 or its homologs activity; and/or
h) increasing the expression of the endogenous gene encoding the polypeptide of the invention, e.g. a polypeptide having the activity of a protein as indicated in table II, application no. 24, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with especially high activity of the proteins of the invention from natural or from mutagenized resources and breeding them into the target organisms, eg the elite crops.

[0054.0.24.24] Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 24 in Table I to IV, resp., after increasing the expression or activity of the encoded polypeptide or having the activity of a polypeptide having an activity as the protein as shown in table II, application no. 24, column 3 or its homologs.

**[0055.0.0.24] to [0064.0.0.24]: see [0055.0.0.0] to [0064.0.0.0]**

**[0065.0.0.24]:** see **[0065.0.0.0]**

**[0066.0.0.24] to [0067.0.0.24]: see [0066.0.0.0] to [0067.0.0.0]**

[0068.0.24.24] The mutation is introduced in such a way that the production of the respective fine chemical, in particular beta-carotene is not adversely affected.

**[0069.0.0.24] see [0069.0.0.0]**

[0070.0.24.24] Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create de *novo* an advantageous, preferably novel metabolites composition in the organism, e.g. an advantageous composition of carotenoids, in particular carotenes, in particular beta-carotene, e.g. comprising a higher content of (from a viewpoint of nutrional physiology limited) carotenoids, in particular carotene, in particular beta-carotene.

**[0071.0.0.24]** see **[0071.0.0.0]**

[0072.0.24.24] By influencing the metabolism thus, it is possible to produce, in the process according to the invention, further advantageous compounds. Examples of such compounds are further carotenoids, e.g. carotenes or carotene, e.g. ketocarentoids, or hydrocarotenoids, e.g. lutein, lycopene, alpha-carotene, or beta-carentene, or other compounds for which IPP is a precursor well known for the skilled.

[0073.0.24.24] Accordingly, in one embodiment, the process according to the invention relates to a process, which comprises:
(a) providing a non-human organism, preferably a microorganism, a non-human animal, a plant or animal cell, a plant or animal tissue or a plant;
(b) increasing an activity of a polypeptide of the invention or a homolog thereof, e.g. as indicated in Table II, application no. 24, columns 5 or 7, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
(c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
(d) if desired, recovering, optionally isolating, the free and/or bound the respective fine chemical and, optionally further free and/or bound carotenoids, in particular carotene, in particular beta-carotene synthesized by the organism, the microorganism, the non-human animal, the plant or animal cell, the plant or animal tissue or the plant.

[0074.0.24.24] The organism, in particular the microorganism, non-human animal, the plant or animal cell, the plant or animal tissue or the plant is advantageously grown in such a way that it is not only possible to recover, if desired isolate the free or bound the respective fine chemical or the free and bound the respective fine chemical but as option it is also possible to produce, recover and, if desired isolate, other free or/and bound carotenoids, in particular carotene, in particular beta-carotene.

**[0075.0.0.24]** to **[0077.0.0.24]: see [0075.0.0.0]** to **[0077.0.0.0]**

[0078.0.24.24] The organism such as microorganisms or plants or the recovered, and if desired isolated, the respective fine chemical can then be processed further directly into foodstuffs or animal feeds or for other applications, for example according to the disclosures made in 6,399,115: Method and composition for the treatment of benign prostate hypertrophy (BPH) and prevention of prostate cancer
6,399,114: Nutritional system for nervous system disorders
6,399,060: Composition having nematicidal activity
6,399,046: Use of a content of catechins or a content of green tea extract in cosmetic preparations for tanning the skin
6,395,782: Method of increasing longevity and preventing body weight wasting in autoimmune disease by using conjugated linoleic acid
6,395,508: Peptide mixture and products thereof
6,395,315: Fermentation composition, process for preparing the same, and use thereof
6,395,311: Multicomponent biological vehicle
6,394,230: Sterol esters as food additives
6,391,640: Methods and compositions for cellular and metabolic engineering
6,391,332: Therapeutic micronutrient composition for severe trauma, burns and critical illness
6,391,321: Emulsifier-free finely disperse systems of the oil-in-water and water-in-oil type
6,391,319: Cosmetic and dermatological emulsions comprising alkyl glucosides and increased electrolyte concentrations
6,391,289: Use of sunscreen combinations comprising, as essential constituent, 4,4'-diarylbutadienes as photostable UV filters in cosmetic and pharmaceutical preparations
6,387,961: Alkyl 2-acetamido-2-deoxyglucopyranoside and methods of emulsifying
6,387,927: Epothilone derivatives and their use as antitumor agents
6,387,883: Method for the prevention and treatment of cachexia and anorexia
6,387,878: Methods of treating intestinal ischemia using heparin-binding epidermal growth factor
6,387,862: Bleach compositions
6,387,418: Pomegranate extracts and methods of using thereof
6,387,370: Compositions containing extracts of Morinda citrifolia, red wine, prune, blueberry, pomegranate, apple and enzyme mixture
6,387,355: Use of sunscreen combinations comprising, as essential constituent, amino-substituted hydroxybenzophenones as photostable UV filters in cosmetic and pharmaceutical preparations
6,384,090: Preparation of active ingredient dispersions and apparatus therefor
6,384,085: Material separated from Ecklonia cava, method for extracting and purifying the same, and use thereof as antioxidants
6,383,751: Assessing lipid metabolism
6,383,543: Process for the extraction of an organic salt from plants, the salt, and other similar salts
6,383,524: Compositions and methods for enhancing therapeutic effects
6,383,523: Pharmaceutical compositions and methods for managing skin conditions
6,383,503: PREPARATIONS OF THE W/O EMULSION TYPE WITH AN INCREASED WATER CONTENT, ADDITIONALLY COMPRISING ONE OR MORE ALKYLMETHICONE COPOLYOLS AND/OR ALKYLDIMETHICONE COPOLYOLS, AND, IF DESIRED, CATIONIC POLYMERS
6,383,474: Carotenoid preparation
6,383,473: Solid composition for reducing tooth erosion
6,380,442: Process for the isolation of mixed carotenoids from plants
6,380,232: Benzimidazole urea derivatives, and pharmaceutical compositions and unit dosages thereof
6,380,227: Fermentative preparation process for and crystal forms of cytostatics
6,379,697: Stabilization of photosensitive materials
6,379,683: Nanocapsules based on dendritic polymers
6,376,722: Lutein to zeaxanthin isomerization process and product
6,376,717: Preparation of astaxanthin
6,376,544: Nutritional product for a person having renal failure
6,376,498: Pharmaceutical, cosmetic and/or food composition with antioxidant properties
6,376,455: Quaternary ammonium compounds, compositions containing them, and uses thereof
6,376,005: Antimicrobial composition for food and beverage products
6,375,993: Pomegranate extracts and methods of using thereof
6,375,992: Methods of hydrating mammalian skin comprising oral administration of a defined composition
6,375,963: Bioadhesive hot-melt extruded film for topical and mucosal adhesion applications and drug delivery and process for preparation thereof
6,375,956: Strip pack
6,375,873: Process and apparatus for producing stably fine-particle powders
6,372,964: For higher basidiomycetes mushrooms grown as biomass in submerged culture
6,372,946: Preparation of 4,4'-diketo-.beta.-carotene derivatives
The cited literature describe some preferred embodiments. Said applications describe some advantageous embodiments without meant to be limiting.
The fermentation broth, fermentation products, plants or plant products can be purified as described in above mentioned applications and other methods known to the person skilled in the art, e.g. as described in Methods in Enzymology: Carotenoids, Part A: Chemistry, Separation, Quantitation and Antioxidation, by John N Abelson or Part B, Metabolism, Genetics, and Biochemistry, or described herein below. Products of these different work-up procedures are IPP and/or beta-carotenes and optional other carotenoids, comprising compositions which still comprise fermentation broth, plant particles and cell components in different amounts, advantageously in the range of from 0 to 99% by weight, preferably below 80% by weight, especially preferably between below 50% by weight.

**[0079.0.0.24]** to **[0084.0.0.24]: see [0079.0.0.0]** to **[0084.0.0.0]**

**[0084.2.24.24]** The invention also contemplates embodiments in which the β-carotene is present in the flowers of the flowering plant chosen as the host (for example, marigolds). The invention also contemplates embodiments in which a host plant's flowers lack β-carotene. In a plant of the latter type, the inserted DNA includes genes that code for carotenoid precursors (compounds that can be converted biologically into β-carotene) and a ketolase, as well as a hydroxylase, if otherwise absent.
In one embodiment, preferred flowering plants include, but are not limited to: Amaryllidaceae (Allium, Narcissus); Apocynaceae (Catharanthus); Asteraceae, alternatively Compositae (Aster, Calendula, Callistephus, Cichorium, Coreopsis, Dahlia, Dendranthema, Gazania, Gerbera, Helianthus, Helichrysum, Lactuca, Rudbeckia, Tagetes, Zinnia); Balsaminaceae (Impatiens); Begoniaceae (Begonia); Caryophyllaceae (Dianthus); Chenopodiaceae (Beta, Spinacia); Cucurbitaceae (Citrullus, Curcurbita, Cucumis); Cruciferae (Alyssum, Brassica, Erysimum, Matthiola, Raphanus); Gentinaceae (Eustoma); Geraniaceae (Pelargonium); Graminae, alternatively Poaceae, (Avena, Horedum, Oryza, Panicum, Pennisetum, Poa, Saccharum, Secale, Sorghum, Triticum, Zea); Euphorbiaceae (Poinsettia); Labiatae (Salvia); Leguminosae (Glycine, Lathyrus, Medicago, Phaseolus, Pisum); Liliaceae (Lilium); Lobeliaceae (Lobelia); Malvaceae (Abelmoschus, Gossypium, Malva); Plumbaginaceae (Limonium); Polemoniaceae (Phlox); Primulaceae (Cyclamen); Ranunculaceae (Aconitum, Anemone, Aquilegia, Caltha, Delphinium, Ranunculus); Rosaceae (Rosa); Rubiaceae (Pentas); Scrophulariaceae (Angelonia, Antirrhinum, Torenia); Solanaceae (Capsicum, Lycopersicon, Nicotiana, Petunia, Solanum); Umbelliferae (Apium, Daucus, Pastinaca); Verbenaceae (Verbena, Lantana); Violaceae (Viola).

[0085.0.24.24] With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, application no. 24, columns 5 or 7, or a derivative thereof, or
b) a genetic regulatory element, for example a promoter, which is functionally linked to the nucleic acid sequence as indicated in Table I, application no. 24, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.24]** to **[0087.0.0.24]: see [0086.0.0.0]** to **[0087.0.0.0]**

[0088.0.24.24] In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned carotene, in particular beta-carotene and the general amount of xanthohylls as energy source in feed. Further, this is also important for the production of cosmetic compostions since, for example, the antioxidant level of plant extracts is dependent on the amount of th abovementioned carotene, in particular beta-carotene and/or the amount of carotenoids as antioxidants.

**[0088.1.0.24]** see **[0088.1.0.0]**

**[0089.0.0.24]** to **[0090.0.0.24]: see [0089.0.0.0]** to **[0090.0.0.0]**

[0091.0.24.24]Thus, the content of plant components and preferably also further impurities is as low as possible, and the abovementioned carotene, in particular beta-carotene, in particular the fine chemical, is/are obtained in as pure form as possible. In these applications, the content of plant components advantageously amounts to less than 10%, preferably 1%, more preferably 0.1 %, very especially preferably 0.01 % or less.

**[0092.0.0.24] to [0094.0.0.24]: see [0092.0.0.0] to [0094.0.0.0]**

[0095.0.24.24] It may be advantageous to increase the pool of said carotenoids, in particular carotene, in particular beta-carotene in the transgenic organisms by the process according to the invention in order to isolate high amounts of the pure respective fine chemical.

[0096.0.24.24] In another preferred embodiment of the invention a combination of the increased expression of the nucleic acid sequence or the protein of the invention together with the transformation of a protein or polypeptide or a compound, which functions as a sink for the desired fine chemical, for example zeaxanthin or cryptoxanthin in the organism, is useful to increase the production of the respective fine chemical. It has been reported, that the inhibition of Lyopene production increases the amount of other carotene, in particular beta-carotene in the cell. Further it may be, that the inhibition of enzymes using zeaxanthin or cryptoxanthin as substrate increases the amount of said chemicals in a cell. For example, in one embodiment, it can be advantageous to inhibit the production of astaxanthin, if a high amount of cryptoxanthin or zeaxanthin is desired.

**[0097.0.24.24]** Glucosides, in particular, dicglucosides of the zeaxanthin and beta-cryptoxanthin as well as other modification of zeaxanthin and cryptoxanthin are known to a person skilled in the art. In may also be advantageous to increase the content of the bound respective fine chemical, e.g. of modification of zeaxanthin and cryptoxanthin, in particular its glucosides, e.g. diglucosides.

**[0098.0.24.24]** In a preferred embodiment, the respective fine chemical is produced in accordance with the invention and, if desired, is isolated. The production of further carotenoids, e.g. carotenes or carotene, in particular beta-carotene, in particular ketocarentoids or hydrocarotenoids, e.g. lutein, lycopene, alpha-carotene, or beta-carentene, or compounds for which the respective fine chemical is a biosynthesis precursor compounds, e.g. astaxanthin, or mixtures thereof or mixtures of other carotenoids, in particular of carotene, in particular beta-carotene, by the process according to the invention is advantageous.

[0099.0.24.24] In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

[0100.0.24.24] Transgenic plants which comprise the carotenoids such as said carotene, in particular beta-carotene, e.g. cryptoxanthin or zeaxanthin (or astaxanthin as it is synthesized from cryptoxanthin or zeaxanthin) synthesized in the process according to the invention can advantageously be marketed directly without there being any need for the carotenoids synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, (in the form of their oils, fats, lipids, as extracts, e.g. ether, alcohol, or other organic solvents or water containing extract and/or free carotene, in particular beta-carotene. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material..E.g., oils, fats, and/or lipids comprising carotene, in particular beta-carotene can be obtained by what is known as cold beating or cold pressing without applying heat. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because carotenoids in microorganisms are localized intracellular, their recovery essentials comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose. However, it can be advantageous to avoid this treatment as it can result in cells devoid of most carotenoids.

[0101.0.24.24] The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

[0102.0.24.24] Carotene, in particular beta-carotene, in particular beta-cryptoxanthin or zeaxanthin can for example be detected advantageously via HPLC, LC or GC separation methods. The unambiguous detection for the presence of carotene, in particular beta-carotene, in particular beta-cryptoxanthin or zeaxanthin containing products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.

[0103.0.24.24] In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, application no. 24, columns 5 or 7, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 24, columns 5 or 7,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, application no. 24, columns 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, application no. 24, columns 7, and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, application no. 24, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof; or which comprises a sequence which is complementary thereto.

[0103.1.24.24] In one embodiment, the nucleic acid molecule used in the process of the invention distinguishes over the sequence indicated in Table I, application no. 24, columns 5 or 7 by one or more nucleotides. In one embodiment, the nucleic acid molecule used in the process of the invention does not consist of the sequence shown in indicated in Table I ,application no. 24, columns 5 or 7,. In one embodiment, the nucleic acid molecule used in the process of the invention is less than 100%, 99,999%, 99,99%, 99,9% or 99% identical to a sequence indicated in Table I , application no. 24, columns 5 or 7,. In another embodiment, the nucleic acid molecule does not encode a polypeptide of a sequence indicated in Table II, application no. 24, columns 5 or 7.

[0104.0.24.24] In one embodiment, the nucleic acid molecule used in the process distinguishes over the sequence indicated in Table I, application no. 24, columns 5 or 7, by one or more nucleotides. In one embodiment, the nucleic acid molecule used in the process of the invention does not consist of the sequence indicated in Table I, application no. 24, columns 5 or 7. In one embodiment, the nucleic acid molecule of the present invention is less than 100%, 99,999%, 99,99%, 99,9% or 99% identical to the sequence indicated in Table I, application no. 24, columns 5 or 7. In another embodiment, the nucleic acid molecule does not encode a polypeptide of a sequence indicated in Table II, application no. 24, columns 5 or 7.

**[0105.0.0.24]** to **[0107.0.0.24]: see [0105.0.0.0]** to **[0107.0.0.0]**

**[0108.0.24.24]** Nucleic acid molecules with the sequence as indicated in Table I, application no. 24, columns 5 or 7, nucleic acid molecules which are derived from an amino acid sequences as indicated in Table II, application no. 24, columns 5 or 7, or from polypeptides comprising the consensus sequence as indicated in Table IV, application no. 24, columns 7, or their derivatives or homologues encoding polypeptides with the enzymatic or biological activity of an activity of a polypeptide as indicated in Table II, application no. 24, column 3, 5 or 7, e.g. conferring the increase of the respective fine chemical, meaning carotenoids, in particular carotene, in particular beta-carotene, resp., after increasing its expression or activity, are advantageously increased in the process according to the invention.

[0109.0.24.24] In one embodiment, said sequences are cloned into nucleic acid constructs, either individually or in combination. These nucleic acid constructs enable an optimal synthesis of the respective fine chemicals, in particular beta-carotene, produced in the process according to the invention.

**[0110.0.0.24] see. [0110.0.0.0]**

**[0111.0.0.24] see [0111.0.0.0]**

[0112.0.24.24] The nucleic acid molecules used in the process according to the invention take the form of isolated nucleic acid sequences, which encode polypeptides with an activity of a polypeptide as indicated in Table II, application no. 24, column 3, or having the sequence of a polypeptide as indicated in Table II, application no. 24, columns 5 and 7, and conferring an increase in the beta-carotene level, respectively.

**[0113.0.0.24]** to **[0114.0.0.24]:** see **[0113.0.0.0]** to **[0114.0.0.0]**

**[0115.0.0.24]** see **[0115.0.0.0]**

**[0116.0.0.24]** to **[0120.0.0.24]** see **[0116.0.0.0]** to **[0120.0.0.0]**

[0120.1.0.24] Production strains which are also advantageously selected in the process according to the invention are microorganisms selected from the group green algae , like Spongioccoccum exentricum, Chlorella sorokiniana (pyrenoidosa, 7-11-05), or form the group of fungi like fungi belonging to the Daccrymycetaceae family, or non-photosynthetic bacteria, like methylotrophs, flavobacteria, actinomycetes, like streptomyces chrestomyceticus, Mycobacteria like Mycobacterim phlei, or Rhodobacter capsulatus.

[0121.0.24.24] However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 24, columns 5 or 7, or the functional homologues thereof as described herein, preferably conferring above-mentioned activity I.e. they confer a beta-carotene increase after increasing the activity of the polypeptide sequences indicated in Table II, application no. 24, columns 5 or 7.

**[0122.0.0.24]** to **[0127.0.0.24]:** see **[0122.0.0.0]** to **[0127.0.0.0]**

[0128.0.24.24] Synthetic oligonucleotide primers for the amplification, e.g. as the pairs indicated in Table III, application no. 24, columns 7, by means of polymerase chain reaction can be generated on the basis of a sequence shown herein, for example the sequence as indicated in Table I, application no. 24, columns 5 or 7, or the sequences derived from a sequences as indicated in Table II, application no. 24, columns 5 or 7.

[0129.0.24.24] Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consensus sequence indicated in Table IV, application no. 24, columns 7, is derived from said alignments.

[0130.0.24.24] Degenerated primers can then be utilized by PCR for the amplification of fragments of novel proteins having above-mentioned activity, e.g. conferring the increase of the respective fine chemical after increasing the expression or activity the protein comprising said fragment.

**[0131.0.0.24] to [0138.0.0.24]: see [0131.0.0.0] to [0138.0.0.0]**

[0139.0.24.24] Polypeptides having above-mentioned activity, i.e. conferring the respective fine chemical level increase, derived from other organisms, can be encoded by other DNA sequences which hybridise to a sequence indicated in Table I, application no. 24, columns 5 or 7, for beta-carotene under relaxed hybridization conditions and which code on expression for peptides having the respective fine chemical, i.e. beta-carotene, resp.,-increasing activity.

**[0140.0.0.24] to [0146.0.0.24]: see [0140.0.0.0] to [0146.0.0.0]**

[0147.0.24.24] Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 24, columns 5 or 7, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.24.24]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 24, columns 5 or 7 or a portion thereof and preferably has above mentioned activity, in particular, of beta-Carotene increasing activity after increasing the acitivity or an activity of a product of a gene encoding said sequences or their homologs.

[0149.0.24.24] The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 24, columns 5 or 7, or a portion thereof and encodes a protein having above-mentioned activity and as indicated in indicated in Table II.

**[00149.1.24.24]** Optionally, in one embodiment, the nucleotide sequence, which hybridises to one of the nucleotide sequences indicated in Table I, application no. 24, columns 5 or 7, has further one or more of the activities annotated or known for the a protein as indicated in Table II, application no. 24, column 3.

**[0150.0.24.24]** Moreover, the nucleic acid molecule of the invention can comprise only a portion of the coding region of one of the sequences indicated in Table I, application no. 24, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of beta-Carotene, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 24, columns 5 or 7, , an anti-sense sequence of one of the sequences, e.g., as indicated in Table I, application no. 24, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 24, column 7, will result in a fragment of a polynucleotide sequence as indicated in Table I, application no. 24, columns 5 or 7 or its gene product.

**[0151.0.0.24]:** see **[0151.0.0.0]**

**[0152.0.24.24]** The nucleic acid molecule of the invention encodes a polypeptide or portion thereof which includes an amino acid sequence which is sufficiently homologous to an amino acid sequence as indicated in Table II, application no. 24, columns 5 or 7, such that the protein or portion thereof maintains the ability to participate in the respective fine chemical production, in particular a beta-carotene increasing activity as mentioned above or as described in the examples in plants or microorganisms is comprised.

[0153.0.24.24] As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 24, columns 5 or 7, such that the protein or portion thereof is able to participate in the increase of the respective fine chemical production. In one embodiment, a protein or portion thereof as indicated in Table II, application no. 24, columns 5 or 7, has for example an activity of a polypeptide indicated in Table II, application no. 24, column 3.

[0154.0.24.24] In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91 %, 92%, 93% or 94% and most preferably at least about 95%, 97%, 98%, 99% or more homologous to an entire amino acid sequence as indicated in Table II, application no. 24, columns 5 or 7, and has above-mentioned activity, e.g. conferring preferably the increase of the respective fine chemical.

**[0155.0.0.24]** to **[0156.0.0.24]: see [0155.0.0.0]** to **[0156.0.0.0]**

[0157.0.24.24] The invention further relates to nucleic acid molecules that differ from one of the nucleotide sequences as indicated in Table I, application no. 24, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, application no. 24, columns 7, or as polypeptides depicted in Table II, application no. 24, columns 5 or 7, or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, application no. 24, columns 7, or of the polypeptide as indicated in Table II, application no. 24, columns 5 or 7, or the functional homologues. In a still further embodiment, the nucleic acid molecule of the invention encodes a full length protein which is substantially homologous to an amino acid sequence comprising a consensus sequence as indicated in Table IV, application no. 24, columns 5 or 7, or of a polypeptide as indicated in Table II, application no. 24, columns 5 or 7, or the functional homologues. However, in a preferred embodiment, the nucleic acid molecule of the present invention does not consist of a sequence as indicated in Table I, application no. 24, columns 5 or 7.

**[0158.0.0.24]** to **[0160.0.0.24]: see [0158.0.0.0]** to **[0160.0.0.0]**

[0161.0.24.24] Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 24, columns 5 or 7. The nucleic acid molecule is preferably at least 20, 30, 50, 100, 250 or more nucleotides in length.

**[0162.0.0.24] see [0162.0.0.0]**

[0163.0.24.24] Preferably, nucleic acid molecule of the invention that hybridizes under stringent conditions to a sequence as indicated in Table I, application no. 24, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in a organism or a part thereof, e.g. a tissue, a cell, or a compartment of a cell, after increasing the expression or activity thereof or the activity of a protein of the invention or used in the process of the invention.

**[0164.0.0.24] see [0164.0.0.0]**

[0165.0.24.24] For example, nucleotide substitutions leading to amino acid substitutions at "non-essential" amino acid residues can be made in a sequence of the nucleic acid molecule of the invention or used in the process of the invention, e.g. as indicated in Table I, application no. 24, columns 5 or 7.

**[0166.0.0.24] to [0167.0.0.24]: see [0166.0.0.0] to [0167.0.0.0]**

[0168.0.24.24] Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 24, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 24, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 24, columns 5 or 7, more preferably at least about 70% identical to one of the sequences as indicated in Table II, application no. 24, columns 5 or 7, ,even more preferably at least about 80%, 90%, or 95% homologous to a sequence as indicated in Table II, application no. 24, columns 5 or 7, , and most preferably at least about 96%, 97%, 98%, or 99% identical to the sequence as indicated in Table II, application no. 24, columns 5 or 7.

**[0169.0.0.24]** to **[0172.0.0.24]: see [0169.0.0.0]** to **[0172.0.0.0]**

**[0173.0.24.24]For** example a sequence which has a 80% homology with sequence SEQ ID NO: 227 at the nucleic acid level is understood as meaning a sequence which, upon comparison with the sequence SEQ ID NO: 227 by the above Gap program algorithm with the above parameter set, has a 80% homology.

**[0174.0.0.24]: see [0174.0.0.0]**

**[0175.0.24.24]**For example a sequence which has a 80% homology with sequence SEQ ID NO: 228 at the protein level is understood as meaning a sequence which, upon comparison with the sequence SEQ ID NO: 228 by the above program algorithm with the above parameter set, has a 80% homology.

**[0176.0.24.24]** Functional equivalents derived from one of the polypeptides as indicated in Table II, application no. 24, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table II, application no. 24, columns 5 or 7, according to the invention and are distinguished by essentially the same properties as a polypeptide as indicated in Table II, application no. 24, columns 5 or 7.

[0177.0.24.24] Functional equivalents derived from a nucleic acid sequence as indicated in Table I, application no. 24, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptide as indicated in Table II, application no. 24, columns 5 or according to the invention and encode polypeptides having essentially the same properties as a polypeptide as indicated in Table II, application no. 24, columns 5 or 7.

**[0178.0.0.24] see [0178.0.0.0]**

[0179.0.24.24] A nucleic acid molecule encoding an homologous to a protein sequence as indicated in Table II, application no. 24, columns 5 or 7, can be created by introducing one or more nucleotide substitutions, additions or deletions into a nucleotide sequence of the nucleic acid molecule of the present invention, in particular as indicated in Table I, application no. 24, columns 5 or 7, such that one or more amino acid substitutions, additions or deletions are introduced into the encoded protein. Mutations can be introduced into the encoding sequences of a sequences as indicated in Table I, application no. 24, columns 5 or 7, by standard techniques, such as site-directed mutagenesis and PCR-mediated mutagenesis.

**[0180.0.0.24] to [0183.0.0.24]: see [0180.0.0.0] to [0183.0.0.0]**

[0184.0.24.24] Homologues of the nucleic acid sequences used, with a sequence as indicated in Table I, application no. 24, columns 5 or 7, or of the nucleic acid sequences derived from a sequences as indicated in Table II, application no. 24, columns 5 or 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 24, columns 5 or 7, or from the derived nucleic acid sequences, the intention being, however, that the enzyme activity or the biological activity of the resulting proteins synthesized is advantageously retained or increased.

[0185.0.24.24] In one embodiment of the present invention, the nucleic acid molecule of the invention or used in the process of the invention comprises one or more sequences as indicated in Table I, application no. 24, columns 5 or 7,. In one embodiment, it is preferred that the nucleic acid molecule comprises as little as possible other nucleotides not shown in any one of sequences as indicated in Table I, application no. 24, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 24, columns 5 or 7.

[0186.0.24.24] Also preferred is that one or more nucleic acid molecule(s) used in the process of the invention encodes a polypeptide comprising a sequence as indicated in Table II, application no. 24, columns 5 or 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 24, columns 5 or 7.

[0187.0.24.24] In one embodiment, a nucleic acid molecule of the invention or used in the process encodes a polypeptide comprising the sequence as indicated in Table II, application no. 24, columns 5 or 7, comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, application no. 24, columns 5 or 7.

[0188.0.24.24] Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 24, columns 5 or 7 and is expressed under identical conditions.

[0189.0.24.24] Homologues of a sequences as indicated in Table I, application no. 24, columns 5 or 7, or of a derived sequences as indicated in Table II, application no. 24, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.24]: see [0190.0.0.0]**

[0191.0.0.24] The organisms or part thereof produce according to the herein mentioned process of the invention an increased level of free and/or bound the respective fine chemical compared to said control or selected organisms or parts thereof.

**[0192.0.0.24] to [0203.0.0.24]: see [0192.0.0.0] to [0203.0.0.0]**

[0204.0.24.24] Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 24, columns 5 or 7, or a fragment thereof conferring an increase in the amount of the respective fine chemical, in particular according to tableTable II, application no. 24, column 6 in an organism or a part thereof
b) nucleic acid molecule comprising, preferably at least the mature form, of a nucleic acid molecule as indicated in Table I, application no. 24, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, application no. 24, column 7, and conferring an increase in the amount of the respective fine chemical, in particular according to table Table II, application no. 24, column 6 in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, application no. 24, columns 5 or 7, and conferring an increase in the amount of the respective fine chemical, in particular according to table II, application no. 24, column 6 in an organism or a part thereof;
k) nucleic acid molecule encoding the amino acid sequence of a polypeptide encoding a domaine of a polypeptide as indicated in Table II, application no. 24, columns 5 or 7, and conferring an increase in the amount of the respective fine chemical, in particular accccording to table II, application no. 24, column 6 in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 24, columns 5 or 7, or a nucleic acid molecule encoding, preferably at least the mature form of, a polypeptide as indicated in Table II, application no. 24, columns 5 or 7, and conferring an increase in the amount of the respective fine chemical according to table Table II, application no. 24, column 6 in an organism or a part thereof;
   or which encompasses a sequence which is complementary thereto;
whereby, preferably, the nucleic acid molecule according to (a) to (I) distinguishes over the sequence indicated in Table I, application no. 24,columns 5 or 7, by one or more nucleotides. In one embodiment, the nucleic acid molecule does not consist of the sequence shown and indicated in Table I, application no. 24, columns 5 or 7,
In one embodiment, the nucleic acid molecule is less than 100%, 99,999%, 99,99%, 99,9% or 99% identical to a sequence indicated in Table I, application no. 24, columns 5 or 7,.
In another embodiment, the nucleic acid molecule does not encode a polypeptide of a sequence indicated in Table II, application no. 24, columns 5 or 7,.
In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 24, columns 5 or 7,.
In a further embodiment the nucleic acid molecule does not encode a polypeptide sequence as indicated in Table II, application no. 24, columns 5 or 7,.
Accordingly, in one embodiment, the nucleic acid molecule of the differs at least in one or more residues from a nucleic acid molecule indicated in Table I, application no. 24, columns 5 or 7,.
Accordingly, in one embodiment, the nucleic acid molecule of the present invention encodes a polypeptide, which differs at least in one or more amino acids from a polypeptide indicated in Table II, application no. 24, columns 5 or 7,.
In another embodiment, a nucleic acid molecule indicated in Table I, application no. 24, columns 5 or 7,.
Accordingly, in one embodiment, the protein encoded by a sequences of a nucleic acid accoriding to (a) to (I) does not consist of a sequence as indicated in Table II, application no. 24, columns 5 or 7,.
In a further embodiment, the protein of the present invention is at least 30 %, 40 %, 50%, or 60% identical to a protein sequence indicated in Table II, application no. 24, columns 5 or 7, and less than 100%, preferably less than 99,999%, 99,99% or 99,9%, more preferably less than 99%, 985, 97%, 96% or 95% identical to a sequence as indicated in Table I, columns 5 or 7.

**[0205.0.0.24]** to **[0206.0.0.24]: see [0205.0.0.0]** to **[0206.0.0.0]**

[0207.0.24.24] As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the carotenoid metabolism, the carotene, in particular beta-carotene metabolism, the astaxanthin metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.24] to [0226.0.0.24]: see [0208.0.0.0] to [0226.0.0.0]**

[0227.0.24.24] The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table I, application no. 24, columns 5 or 7, or its derivatives, it is advantageous to express and/or mutate further genes in the organisms. Especially advantageously, additionally at least one further gene of the carotenoid, in particular carotene biosynthetic pathway, e.g. one of the above mentioned genes of this pathway, or e.g. for the synthesis of astaxanthin or for another provitamin A or for another carotenoids or carotene, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine one or more of the sequences indicated in Table I, application no. 24, columns 5 or 7, with genes which generally support or enhances to growth or yield of the target organismen, for example genes which lead to faster growth rate of microorganisms or genes which produces stress-, pathogen, or herbicide resistant plants.

[0228.0.24.24] In a further embodiment of the process of the invention, therefore, organisms are grown, in which there is simultaneous overexpression of at least one nucleic acid or one of the genes which code for proteins involved in the carotenoid, in particular carotenes metabolism, in particular in synthesis of beta-cryptoxanthin, zeaxanthin, astaxanthin or lutein.

[0229.0.24.24] Further advantageous nucleic acid sequences which can be expressed in combination with the sequences used in the process and/or the abovementioned biosynthesis genes are the sequences encoding further genes of the carotenoids biosynthetic pathway, such as phytoene synthase (Psy), which is an important control point for the regulation of the flux (Fraser et al., 2002), phytoene desaturase (Pds), z-carotene desaturase, above mentioned enzymes (s. introduction of the application), e.g. hydroxylases such as beta-carotene hydroxylase (US 6,214,575), ketolases, or cyclases such as the beta-cyclase (US 6,232,530) or oxygenases such as the beta-C4-oxygenase described in US 6,218,599 or homologs thereof, astaxanthin synthase (US 6,365,386), or other genes as described in US 6,150,130. These genes can lead to an increased synthesis of the essential carotenoids, in particular carotene, in particular beta-carotenes.

**[0230.0.0.24] see [230.0.0.0].**

[0231.0.24.24] In a further advantageous embodiment of the process of the invention, the organisms used in the process are those in which simultaneously a beta-carotene degrading protein is attenuated, in particular by reducing the rate of expression of the corresponding gene.

**[0232.0.0.24] to [0276.0.0.24]: see [0232.0.0.0] to [0276.0.0.0]**

[0277.0.24.24] The respective fine chemical produced can be isolated from the organism by methods with which the skilled worker are familiar, for example via extraction, salt precipitation, and/or different chromatography methods. The process according to the invention can be conducted batchwise, semibatchwise or continuously. The fine respective chemical, i.e. e.g. beta-carotene, and other carotenoids, in particular carotenes produced by this process can be obtained by harvesting the organisms, either from the crop in which they grow, or from the field. This can be done via pressing or extraction of the plant parts

**[0278.0.0.24]** to **[0282.0.0.24]: see [0278.0.0.0]** to **[0282.0.0.0]**

**[0283.0.24.24]** Moreover, native polypeptide conferring the increase of the respective fine chemical in an organism or part thereof can be isolated from cells (e.g., endothelial cells), for example using the antibody of the present invention as described below, e.g. an antibody against a protein as indicated in Table II, application no. 24, column 3, or an antibody against a polypeptide as indicated in Table II, application no. 24, columns 5 or 7, which can be produced by standard techniques utilizing the polypeptid of the present invention or fragment thereof, i.e., the polypeptide of this invention. Preferred are monoclonal antibodies.

**[0284.0.0.24] see [0284.0.0.0]**

**[0285.0.24.24]** In one embodiment, the present invention relates to a polypeptide having a sequence as indicated in Table II, application no. 24, columns 5 or 7, or as coded by a nucleic acid molecule as indicated in Table I, application no. 24, columns 5 or 7, or functional homologues thereof.

[0286.0.24.24] In one advantageous embodiment, in the method of the present invention the activity of a polypeptide is increased which comprises or consists of a consensus sequence as indicated in Table IV, application no. 24, columns 5 or 7, and in one another embodiment, the present invention relates to a polypeptide comprising or consisting of a consensus sequence as indicated in Table IV, application no. 24, columns 5 or 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, application no. 24, column 7.

**[0287.0.0.24]** to **[0289.0.0.24]: see [0287.0.0.0]** to **[0289.0.0.0]**

**[00290.0.24.24]** The alignment was performed with the Software AlignX (sept 25, 2002) a component of Vector NTI Suite 8.0 , InforMax™,Invitrogen™ life science software, U.S. Main Office, 7305 Executive Way,Frederick, MD 21704,USA with the following settings: For pairwise alignments: gap opening penality: 10,0; gap extension penality 0,1. For multiple alignments: Gap opening penalty: 10,0; Gap extension penalty: 0,1; Gap separation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids

[0291.0.24.24] In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II, application no. 24, columns 5 or 7, by one or more amino acids. In one embodiment, polypeptide distinguishes form a sequence as indicated in Table II, application no. 24, columns 5 or 7, by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 24, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in TableTable II, application no. 24, columns 5 or 7,.

**[0292.0.0.24] see [0292.0.0.0]**

[0293.0.24.24] In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II, application no. 24, columns 5 or 7, by one or more amino acids. In an other embodiment, said polypeptide of the invention does not consist of the sequence as indicated in Table II, application no. 24, columns 5 or 7,.
In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 24, columns 5 or 7.

[0294.0.24.24] In one embodiment, the present invention relates to a polypeptide having an activity of a protein as indicated in Table II, application no. 24, column 3, which distinguishes over a sequence as indicated in Table II, application no. 24, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.24]** to **[0296.0.0.24]: see [0295.0.0.0]** to **[0296.0.0.0]**

**[0297.0.0.24] see [0297.0.0.0]**

[00297.1.24.24] Non-polypeptide of the invention-chemicals are e.g. polypeptides having not the activity of a polypeptide indicated in Table II, application no. 24, columns 3, 5 or 7.

**[0298.0.24.24]** A polypeptide of the invention can participate in the process of the present invention. The polypeptide or a portion thereof comprises preferably an amino acid sequence which is sufficiently homologous to an amino acid sequence as indicated in Table II, application no. 24, columns 5 or 7. The portion of the protein is preferably a biologically active portion as described herein. Preferably, the polypeptide used in the process of the invention has an amino acid sequence identical to a sequence as indicated in Table II, application no. 24, columns 5 or 7.

**[0299.0.24.24]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91 %, 92%, 93%, 94% or 95%, and even more preferably at least about 96%, 97%, 98%, 99% or more homologous to one of the nucleotide sequence as indicated in Table I, application no. 24, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 24, columns 5 or 7, or which is homologous thereto, as defined above.

**[0300.0.24.24]** In one embodiment, the present invention relates to a polypeptide having an activity of a protein as indicated in Table II, application no. 24, column 3, which distinguishes over a sequence as indicated in Table II, application no. 24, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0301.0.0.24] see [0301.0.0.0]**

[0302.0.24.24] Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 24, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.24] see [0303.0.0.0]**

**[0304.0.24.24]** Manipulation of the nucleic acid molecule of the invention may result in the production of a protein having essentially the activity of the polypeptides as indicated in Table II, application no. 24, column 3, but having differences in the sequence from said wild-type protein. These proteins may be improved in efficiency or activity, may be present in greater numbers in the cell than is usual, or may be decreased in efficiency or activity in relation to the wild type protein.

**[0305.0.0.24]** to **[0308.0.0.24]: see [0305.0.0.0]** to **[0308.0.0.0]**

**[0309.0.0.24]** In one embodiment, a reference to a protein (= polypeptide) of the invention or as indicated in Table II, application no. 24, columns 5 or 7, refers to a polypeptide having an amino acid sequence corresponding to the polypeptide of the invention or used in the process of the invention , whereas a " non-polypeptide of the invention" or "other polypeptide" not being indicated in Table II, application no. 24, columns 5 or 7, refers to a polypeptide having an amino acid sequence corresponding to a protein which is not substantially homologous a polypeptide of the invention, preferably which is not substantially homologous to a polypeptide as indicated in Table II, application no. 24, columns 5 or 7, e.g., a protein which does not confer the activity described herein or annotated or known for as indicated in Table II, application no. 24, column 3, and which is derived from the same or a different organism. In one embodiment, a "non-polypeptide of the invention" or "other polypeptide" not being indicated in Table II, application no. 24, columns 5 or 7, does not confer an increase of the respective fine chemical in an organism or part thereof.

**[0310.0.0.24] to [0334.0.0.24]: see [0310.0.0.0] to [0334.0.0.0]**

[0335.0.24.24] The dsRNAi method has proved to be particularly effective and advantageous for reducing the expression of a nucleic acid sequences as indicated in Table I, application no. 24, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, application no. 24, columns 5 or 7, and/or homologs thereof. In a double-stranded RNA molecule for reducing the expression of an protein encoded by a nucleic acid sequence sequences as indicated in Table I, application no. 24, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.24] to [0342.0.0.24]: see [0336.0.0.0] to [0342.0.0.0]**

[0343.0.24.24] As has already been described, 100 % sequence identity between the dsRNA and a gene transcript of a nucleic acid sequence as indicated in Table I, application no. 24, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 24, columns 5 or 7, or homologs thereof of the one organism, may be used to suppress the corresponding expression in another organism.

**[0344.0.0.24] to [0350.0.0.24]: see [0344.0.0.0] to [0350.0.0.0]**

**[0351.0.0.24] t**o **[0361.0.0.24]: see [0351.0.0.0]** to **[0361.0.0.0]**

[0362.0.24.24] Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 24, columns 5 or 7, e.g. encoding a polypeptide having protein activity, as indicated in Table II, application no. 24, columns 3. Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, application no. 24, columns 5 or 7, means herein that due to modulation or manipulation of the genome, an activity as annotated for a polypeptide as indicated in Table II, application no. 24, column 3, e.g. having a sequence as indicated in Table II, application no. 24, columns 5 or 7, is increased in a cell or an organism or a part thereof. Examples are described above in context with the process of the invention.

**[0363.0.0.24] see [0363.0.0.0]**

[0364.0.24.24] A naturally occurring expression cassette - for example the naturally occurring combination of a promoter of a gene encoding a polypeptide of the invention as indicated in Table II, application no. 24, column 3, 5 or 7, with the corresponding protein-encoding sequence as indicated in Table I, application no. 24, column 5 or 7 , - becomes a transgenic expression cassette when it is modified by non-natural, synthetic "artificial" methods such as, for example, mutagenization. Such methods have been described (US 5,565,350; WO 00/15815; also see above).

**[0365.0.0.24] to [0373.0.0.24]: see [0365.0.0.0] to [0373.0.0.0]**

[0374.0.24.24] Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. The respective fine chemical produced in the process according to the invention may, however, also be isolated from the plant in the form of their free beta-carotene or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.24] to [0376.0.0.24]: see [0375.0.0.0] to [0376.0.0.0]**

[0377.0.24.24] Accordingly, the present invention relates also to a process according to the present invention whereby the produced carotenoids, e.g the fine chemical(s) comprising composition is isolated. In one embodiment, the produced respective fine chemical is isolated.

[0378.0.24.24] In this manner, more than 50% by weight, advantageously more than 60% by weight, preferably more than 70% by weight, especially preferably more than 80% by weight, very especially preferably more than 90% by weight, of the respective fine chemical produced in the process can be isolated. The resulting respective fine chemical can, if appropriate, subsequently be further purified, if desired mixed with other active ingredients such as vitamins, amino acids, carbohydrates, antibiotics and the like, and, if appropriate, formulated.

[0379.0.24.24] In one embodiment, the product of the process is a mixture of the fine chemicals.ln one embodiment, the product is a mixture of the respective fine chemicals with other carotenoids.

[0380.0.24.24] The respective fine chemicals obtained in the process are suitable as starting material for the synthesis of further products of value. For example, they can be used in combination with each other or alone for the production of pharmaceuticals, foodstuffs, animal feeds or cosmetics. Accordingly, the present invention relates to a method for the production of pharmaceuticals, food stuff, animal feeds, nutrients or cosmetics comprising the steps of the process according to the invention, including the isolation of the respective fine chemical containing composition produced by the process of the invention or the respective fine chemical produced by the process of the invention - if desired - and formulating the product with a pharmaceutical or cosmetic acceptable carrier or formulating the product in a form acceptable for an application in agriculture. A further embodiment according to the invention is the use of the the respective fine chemical produced in the process or of the transgenic organisms in animal feeds, foodstuffs, medicines, food supplements, cosmetics or pharmaceuticals or for the production of other carotenoids, e.g. in after isolation of the respective fine chemical or without, e.g. in situ, e.g within the organism used for the process for the production of the respective fine chemical.

**[0381.0.0.24]** to **[0382.0.0.24]: see [0381.0.0.0]** to **[0382.0.0.0]**

**[0383.0.24.24]./.**

**[0384.0.0.24] see [0384.0.0.0]**

[0385.0.24.24] The fermentation broths obtained in this way, containing in particular Beta-carotene in mixtures with other carotenoids, in particular with other carotenoids, in particular carotenes, or containing microorganisms or parts of microorganisms, like plastids, containing the respective fine chemical or the carotenoids produced in mixtures with other carotenoids, in particular with other carotene, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3 g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.
As carotenoids are often localized in membranes or plastids, in one embodiment it is advantageous to avoid a leaching of the cells when the biomass is isolated entirely or partly by separation methods, such as, for example, centrifugation, filtration, decantation, coagulation/flocculation or a combination of these methods, from the fermentation broth. The dry biomass can directly be added to animal feed, provided the carotenoids concentration is sufficiently high and no toxic compounds are present. In view of the instability of carentoids, conditions for drying, e.g. spray or flash-drying, can be mild and can be avoiding oxidation and *cis*/*trans* isomerization. For example antioxidants, e.g. BHT, ethoxyquin or other, can be added. In case the carotenoids concentration in the biomass is to dilute, solvent extraction can be used for their isolation, e.g. with alcohols, ether or other organic solvents, e.g. with methanol, ethanol, aceton, alcoholic potassium hydroxide, glycerol-phenol, liquefied phenol or for example with acids or bases, like trichloroacetatic acid or potassium hydroxide. A wide range of advantageous Methods and techniques for the isolation of carotenoids, in particular of the respective fine chemical, in particular of beta-carotene can be found in the state of the art. In case phenol is used it can for example be removed with ether and water extraction and the dry eluate comprises a mixture of the carotenoids of the biomass.

[0386.0.24.24] Accordingly, it is possible to further purify the respective fine chemicals or other carotenoids, in particular the carotenes produced according to the invention. For this purpose, the product-containing composition, e.g. a total or partial lipid extraction fraction using organic solvents, e.g. as described above, is subjected for example (without meaning to be limited:) to a saponification to remove triglycerides, partition between e.g. hexane/methanol (separation of non-polar epiphase from more polar hypophasic derivates) and separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.24]** to **[0392.0.0.24] : see [0387.0.0.0]** to **[0392.0.0.0]**

[0393.0.24.24] In one embodiment, the present invention relates to a method for the identification of a gene product conferring an increase in the fine chemical production in a cell, comprising the following steps:
a) contacting, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
b) identifying the nucleic acid molecules, which hybridize under relaxed stringent conditions with the nucleic acid molecule of the present invention in particular to the nucleic acid molecule sequence as indicated in Table I, application no. 24, columns 5 or 7, and, optionally, isolating the full length cDNA clone or complete genomic clone;
c) introducing the candidate nucleic acid molecules in host cells, preferably in a plant cell or a microorganism, appropriate for producing the fine chemical;
d) expressing the identified nucleic acid molecules in the host cells;
e) assaying the the fine chemical level in the host cells; and
f) identifying the nucleic acid molecule and its gene product which expression confers an increase in the the fine chemical level in the host cell after expression compared to the wild type.

**[0394.0.0.24] to [0552.0.0.24]: see [0394.0.0.7] to [0552.0.0.7]**

**[0553.0.24.24]** A process for the production of beta-Carotene resp., which comprises
(a) increasing or generating the activity of a protein as indicated in Table II, application no. 24, columns 5 or 7, or a functional equivalent thereof in a non-human organism, or in one or more parts thereof; and
(b) growing the organism under conditions which permit the production of beta-Carotene resp. in said organism.
A process for the production of beta-Carotene resp., comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 24, columns 5 or 7, , or a fragment thereof, which confers an increase in the amount of the fine chemical as indicated in table II, column 6, e.g of beta-Carotene resp., in an organism or a part thereof;
b) nucleic acid molecule comprising of a nucleic acid molecule as indicated in Table I, application no. 24, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of beta-Carotene resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of beta-Carotene resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of beta-Carotene resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 24, column 7, and conferring an increase in the amount of the fine chemical as indicated in table II, column 6, e.g of beta-Carotene resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of beta-Carotene resp., in an organism or a part thereof;
h) nucleic acid molecule encoding a polypeptide comprising a consensus sequence as indicated in Table IV, application no. 24, column 7, and conferring an increase in the amount of the fine chemical as indicated in table II, column 6, e.g of beta-Carotene resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical as indicated in table II, column 6, e.g of beta-Carotene resp., in an organism or a part thereof. or comprising a sequence which is complementary thereto.
The process as mentioned above, comprising recovering of the free or bound beta-Carotene resp..
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound beta-Carotene resp., produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 24, columns 5 or 7, or a fragment thereof, which confers an increase in the amount of the fine chemical as indicated in table II, column 6, e.g of beta-Carotene resp., in an organism or a part thereof;
b) nucleic acid molecule comprising of a nucleic acid molecule as indicated in Table I, application no. 24, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of beta-Carotene resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of beta-Carotene resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of beta-Carotene resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 24, column 7, and conferring an increase in the amount of the fine chemical as indicated in table II, column 6, e.g of beta-Carotene resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of beta-Carotene resp., in an organism or a part thereof;
h) nucleic acid molecule encoding a polypeptide comprising a consensus sequence as indicated in Table IV, application no. 24, column 7, and conferring an increase in the amount of the fine chemical as indicated in table II, column 6, e.g of beta-Carotene resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemiccal as indicated in table II, column 6, e.g of beta-Carotene resp., in an organism or a part thereof.
whereby the nucleic acid molecule distinguishes over the sequence as indicated in Table I, application no. 24, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 24, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of beta-Carotene resp., in an organism or a part thereof comprising:
(a) contacting cells, tissues , plants or microorganisms which express the a polypeptide encoded by the nucleic acid molecule as mentioned above conferring an increase in the amount of beta-Carotene resp., in an organism or a part thereof with a candidate compound or a sample comprising a plurality of compounds under conditions which permit the expression the polypeptide;
(b) assaying the beta-Carotene resp., level or the polypeptide expression level in the cell, tissue, plant or microorganism or the media the cell, tissue, plant or microorganisms is cultured or maintained in; and
(c) identifying a agonist or antagonist by comparing the measured beta-Carotene resp., level or polypeptide expression level with a standard beta-Carotene resp., or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased beta-Carotene resp., production in a plant or microorganism, comprising the steps:
(a) culturing a plant cell or tissue or microorganism or maintaining a plant expressing the polypeptide encoded by the nucleic acid molecule as mentioned above conferring an increase in the amount of beta-Carotene resp., in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of beta-Carotene rersp., in an organism or a part thereof;
(b) identifying if the compound is an effective agonist by detecting the presence or absence or increase of a signal produced by said readout system.
A method for the identification of a gene product conferring an increase in beta-Carotene resp., production in a cell, comprising the following steps:
(a) contacting the nucleic acid molecules of a sample, which can contain a candidate gene encoding a gene product conferring an increase in beta-Carotene resp., after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing beta-Carotene resp.;
(d) expressing the identified nucleic acid molecules in the host cells;
(e) assaying the beta-Carotene rersp., level in the host cells; and
(f) identifying nucleic acid molecule and its gene product which expression confers an increase in the beta-Carotene resp., level in the host cell in the host cell after expression compared to the wild type.
A method for the identification of a gene product conferring an increase in beta-Carotene resp., production in a cell, comprising the following steps:
(a) identifiying in a data bank nucleic acid molecules of an organism; which can contain a candidate gene encoding a gene product conferring an increase in the beta-Carotene resp., amount or level in an organism or a part thereof after expression, and which are at least 20% homolog to the nucleic acid molecule as mentioned above;
(b) introducing the candidate nucleic acid molecules in host cells appropriate for producing beta-Carotene resp.,;
(c) expressing the identified nucleic acid molecules in the host cells;
(d) assaying the beta-Carotene resp., level in the host cells; and
(e) identifying nucleic acid molecule and its gene product which expression confers an increase in the beta-Carotene resp., level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of beta-Carotene resp., after expression.
Use of the polypeptide as mentioned above or the nucleic acid construct as mentioned above or the gene product identified according to the method as mentioned above for identifying compounds capable of conferring a modulation of beta-Carotene resp., levels in an organism.
Cosmetic, pharmaceutical, food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
The method as mentioned above, the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above, wherein the carotene is beta-carotene
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the protection of a plant against a oxidative stress.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the protection of a plant against a oxidative stress causing or a carotenoid synthesis inhibiting herbicide.
Use of the agonist identified as mentioned above, the plant or plant tissue as mentioned above, the harvested material as mentioned above, or the host cell as mentioned above for the production of a cosmetic composition.

### New genes for a process for the production of fine chemicals

[0000.0.0.25] In a further embodiment, the present invention relates to a further process for the production of fine chemicals as defined below and the corresponding embodiments as described herein as follows.

**[0001.0.0.25]** see **[0001.0.0.0]**

[0002.0.25.25] Carbohydrates are aldehyde or ketone compounds with multiple hydroxyl groups. Many carbohydrates have the empirical formula (CH2O)n; some also contain nitrogen, phosphorus, or sulfur.
Carbohydrates are classsfied in monosaccharides, oligosaccharides, and polysaccharides.
Monosaccharides, or simple sugars, consist of a single polyhydroxy aldehyde or ketone unit. Monosaccharides of more than four carbons tend to have cyclic structures.
Oligosaccharides consist of short chains of monosaccharide units, or residues, usually 2 to 19 units, joined by glycosidic bonds.
The polysaccharides are sugar polymers containing more than 20 or so monosaccharide units, and some have hundreds or thousands of units. Some polysaccharides are linear chains; others are branched.
Carbohydrates are called saccharides or, if they are relatively small, sugars.
In the present invention, saccharides means all of the aforementioned carbohydrate, e.g. monosaccharides, preferably fructose, glucose, inositol, galactose, arabinose xylose or other pentoses or hexoses; oligosaccharides, preferably disaccharides like sucrose, lactose or trisaccharides like raffinose; or polysaccharides like starch or cellulose.

[0003.0.25.25] Carbohydrates are the most abundant class of organic compounds found in living organisms.
They are a major source of metabolic energy, both for plants and for animals. Aside from the sugars and starches that meet this vital nutritional role, carbohydrates function in energy storage (for example starch or glycogen), in signaling (by glycoproteins and glycolipids, e.g. blood group determinants), fuel the nervous system, muscle and virtually all cells, are parts of nucleic acids (in genes, mRNA, tRNA, ribosomes), and as cell surface markers as recognition sites on cell surfaces and signaling in glycolipids and glycoproteins and also serve as a structural material for example as cell wall components (cellulose).

[0004.0.25.25] Glucose, also called dextrose, is the most widely distributed sugar in the plant and animal kingdoms and it is the sugar present in blood as "blood sugar". It occupies a central position in the metabolism of plants, animals, and many microorganisms. Glucose is rich in potential energy, and thus a good fuel; in the body is catabolised to produce ATP. It is stored as a high molecular weight polymer such as starch or glycogen or is converted to fatty acids. It is also a remarkably versatile precursor, capable of supplying a huge array of metabolic intermediates for biosynthetic reactions.
Based on its manifold features, glucose is used in nutrition and medicine.
Fructose, also called levulose or "fruit sugar", is the most important ketose sugar. Fructose is a hexose and is a reducing sugar. Fructose is used a a sweetener by diabetics because it does not rise the blood sugar level, even in large amounts. Fructose and glucose are the main carbohydrate constituents of honey. Those hexoses are further the main components of many oligo- and polysaccharides, like sucrose, raffinose, stachyose, trehalose, starch, cellulose or dextran.
The most frequent disaccharide is sucrose (saccharose, b-D-fructofuranosyl-a-D-glucopyranosid, cane sugar, beet sugar, sugar in a narrow sense of a name for commercially available sucrose meaning sucrose is the sugar that is commonly called "sugar") which consists of the six-carbon sugars D-glucose and D-fructose. It is formed by plants but not by animals. Sucrose is a major intermediate product of photosynthesis; in many plants it is the principal form in which sugar is transported from the leaves to other parts of the plant body. In mammalians sucrose is an obligatory component of blood and its content in blood is kept at the stable level. It is strongly necessary for brain cells as well as for normal functioning of the central nervous system. Sugar is widely-known as a source of glycogen - a substance, feeding liver, heart and muscles. It is one of the most widely-used food products and is the major disaccharide in most diets. It is present in honey, maple sugar, fruits, berries, and vegetables. It may be added to food products as liquid or crystalline sucrose or as invert sugar. It is commercially prepared from sugar cane or sugar beets. Sucrose can provide a number of desirable functional qualities to food products including sweetness, mouth-feel, and the ability to transform between amorphous and crystalline states. High-concentrated sucrose is a natural preserving agent, it determines gel-formation processes, gives necessary viscosity to the products. Sucrose is a raw material for caramel, colour etc.
Sucrose is further an excellent fermentation feedstock, which is of specific interest for fermentation industry (including a number of non-food industries -pharmaceutical industries). The presence of eight hydroxyl groups in the sucrose molecule provides a theoretical possibility of a very large number of sucrose derivatives. Sucrose derivatives are used by industries in production of detergents, emulsifiers (sucrose + fatty acids) and adhesives (sucrose octa acetate).
Sucrose is a precursor to a group of carbohydrates in plants known as the raffinose family of oligosaccharides found in many plant seeds especially legumes. This family contains the trisaccharide raffinose, the tetrasaccharide stachyose and the pentasaccharide verbascose. Oligosaccharides of the raffinose-series are major components in many food legumes (Shallenberger et al., J.Agric. Food Chem., 9,1372; 1961). Raffinose (beta-D-fructofuranosyl-6-0-alpha-D-galactopyranosyl- alpha- D-glucopyranosid, melitriose, gossypose, melitose), which consists of sucrose with a-galactose attached through its C-4 atom to the 1 position on the fructose residue and is thought to be second only to sucrose among the nonstructural carbohydrates with respect to abundance in the plant kingdom. It may be ubiquitous, at least among higher plants. Raffinose accumulate in significant quantities in the edible portion of many economically significant crop species. Examples include soybean, sugar beet, cotton, canola and all of the major edible leguminous crops including beans, peas, lentil and lupine.
An important key intermediates in the formation of raffinose and stachyose is myo-inositol (cyclohexan-1,2,3,4,5,6-hexaole), the most common cyclitol. Myo-inositol is fundamental to many different aspects of plant growth and development. In addition to its role as the precursor for phytic acid biosynthesis, myo-inositol is also used for uronide and pentose biosynthesis, it is also present in phosphoinositides of plant cell membranes, as well as other complex plant lipids including glycophosphoceramides. Furthermore, it is also a precursor of other naturally occurring inositol isomers, and many of these as well as myo-inositol are distributed as methyl ethers in a species specific pattern throughout the plant kingdom. Myo-inositol is an important growth factor.
The most carbohydrates found in nature occur as polysaccharides which are polymers of medium to high molecular weight. Polysaccharides, also called glycans, differ from each other in the identity of their recurring monosaccharide units, in the length of their chains, in the types of bonds linking the units, and in the degree of branching.
Starch is the most valuable polysaccharide. Normal native starches consist of a mixture of 15-30 % amylose and 70-85 % amylopectin. Amylose structurally is a linear polymer of anhydroglucose units, of molecular weight approximately between 40 000 and 340 000, the chains containing 250 to 2000 anhydroglucose units. Amylopectin is considered to be composed of anhydroglucose chains with many branch points; the molecular weight may reach as high as 80 000 000.
Starch is the most important, abundant, digestible food polysaccharide. It occurs as the reserve polysaccharide in the leaf, stem, root, seed, fruit and pollen of many higher plants. It occurs as discrete, partially-crystalline granules whose size, shape, and gelatinization temperature depend on the botanical source of the starch. Common food starches are derived from seed (wheat, maize, rice, barley) and root (potato, cassava/tapioca) sources. Starches have been modified to improve desired functional characteristics and are added in relatively small amounts to foods as food additives. Another important polysaccharide is cellulose. Cellulose is the most commonly seen polysaccharide and scientist estimate that over one trillion tons of cellulose are synthesized by plants each year. Cellulose forms the cell wall of plants. It is yet a third polymer of the monosaccharide glucose. Cellulose differs from starch and glycogen because the glucose units form a two-dimensional structure, with hydrogen bonds holding together nearby polymers, thus giving the molecule added stability. A single "cellulose fiber" can consist of up to 10000 individual anhydroglucose units. In cellulose, the individual fiber molecules are arranged in bundles and thus form so called micro fibrils which ultimately result in a "densely woven" net like structure of cellulose molecules. The strong cohesion between the individual cellulose fibers is due to the huge number of strong hydrogen bonds.
Cellulose is the major polysaccharide of grass, leaves and trees and is said to include around 50% of all biological carbon found on our planet. It is the basic material of natural substances such as wood, flax or cotton and consists of long, unbranched fiber molecules. Cellulose, as plant fiber, cannot be digested by human beings therefore cellulose passes through the digestive tract without being absorbed into the body. Some animals, such as cows and termites, contain bacteria in their digestive tract that help them to digest cellulose. Nevertheless, cellulose is of importance in human nutrition in that fiber is an essential part of the diet, giving bulk to food and promoting intestinal motility.

**[0005.0.25.25]** The polysaccharides starch and cellulose are the most important raw material in the industrial and comercial production of glucose. In the common procedure starch or cellulose are acidly or enzymatically hydrolysed to glucose. Fructose is usually also produced from starch by enzymatically transforming it into glucose syrup and subsequently treating with isomerase, leading to a conversion of glucose to fructose.
Succrose is obtained comercially from the expressed juice of sugar cane or of sugar beet.
Myo-inositol exists in nature either in its free form (found, for example, in sugarcane, beet molasses, and almond hulls) or as a hexaphosphate called phytin (found, for example, in corn steep liquor). Industrial purification of phytin from corn steep liquor involves precipitation with calcium, followed by hydrolysis with a strong acid. Separation of free form inositols from plant extracts involves treatment with acid and separation of myo-inositol by column (US 5,482,631) or the use of ion-exchange (US 4,482,761).
Cellulose is a very important industrial product. As disclosed above, it serves as row material for monosaccharides. It is further used in the manufacture of paper, textiles, plastics, explosives, packaging material (cellophane®), feed, food and fermentation products. Cellulose is obtained primarily by acid or alkaline hydrolize.
Starch occurs intracellularly as large clusters or granules. These granular starch consists of microscopic granules, which differ in size and shape, depending on the plant source. The granules are insoluble in water at room temperature. There is a quite number of methods known for the extraction of starch. For example a slurry of grinded starch containing plant material is heated, whereby the granules swell and eventually burst, dispersing the starch molecules into the solution. During the liquefaction step, the long-chained starch is further degraded into smaller branched and linear units (maltodextrins) by an alpha-amylase. A large number of processes have been described for converting starch to starch hydrolysates, such as maltose, glucose or specialty syrups, either for use as sweeteners or as precursors for other saccharides such as fructose. A process for enzymatic hydrolysis of granular starch into a soluble starch hydrolysate is disclosed in US 20050042737.

**[0006.0.25.25]** Carbohydrates play a major role in human and animal diets, comprising some 40-75% of energy intake. Their most important nutritional property is digestibility. Some of them are hydrolyzed by enzymes of the human gastrointestinal system to monosaccharides that are absorbed in the small intestine and enter the pathways of carbohydrate metabolism. Others can be digested by certain animals. Carbohydrates, fat and protein are the energy yielding nutrients in animal feed. In the average diet for farm animals, carbohydrates are included at levels of 70-80%. For example pig diets are mainly based on cereals which contain the main part of the energy providing nutrients that are essential for pigs.
With view to the increasing global demand for food because of the growing world population and at the same time the shrinking availability of arable land, it is important to increase the food and feed quality, particulary the availability of certain essential nutrients, preferably carbohydrates, preferably polysaccharides like starch or cellulose and/or monosaccharides like myo-inositol.. Nutritional improvements in foods and feeds could help to meet these demands for improved quality. Modern agricultural biotechnology, which involves the application of cellular and molecular techniques to transfer DNA that encodes a desired trait to food and feed crops, is a powerful complement to traditional methods to meet global food and feed requirements.

[0007.0.25.25] Furthermore the physicochemical properties such as viscosity and the capacity to bind water and ions, vary between different cereals. Consequently, different cereal properties affect digestion and fermentation as well as microbial populations in the gastro-intestinal tract in various ways. Gastro-intestinal disturbances comprise a major problem for health of humans and animals.
There is a need for suitable dietary composition and food or feed ingredients, preferably cereals, legumes or fruits which promotes a beneficial gut environment and thereby preventing gastro-intestinal disorders.
Therefore improving the quality of foodstuffs and animal feeds is an important task of the food-and-feed industry. This is necessary since, for example, carbohydrates, which occur in plants and some microorganisms are limited with regard to the supply of mammals. Especially advantageous for the quality of foodstuffs and animal feeds is as balanced as possible a carbohydrate profile in the diet since a great excess of some sugars above a specific concentration in the food has only some or little or no positive effect.

[0008.0.25.25] Genetically modified plants having improved nutritional profiles are known in the state of art. US 20030070192 discloses a DNA expression cassette which alters the sugar alcohol of tranformed plants.
US 5,908,975 concerns methods for synthesis and accumulation of fructose polymers in transgenic plants by selective expression of bacterial fructosyltransferase genes using tissue specific promoters and a vacuole targeting sequence.
WO89/12386 describes a method for the production of glucose and fructose polymers in transgenic tomato plants.
A stress tolerance sequences including proteins like galactinol synthase (GOLS) and raffinose synthase (RAFS), which are up regulated in response to stress and lead to the production of raffinose is disclosed in US 20050055748.
US 6,887,708 provides nucleotide sequences encoding polypeptides having the function of GIGANTEA gene of Arabidopsis thaliana which allows the manipulation of the starch accumulation process in plants.
Grain having an embryo with a genotype heterozygous for two or more wild type genes (for example, Aa/Bb) and an endosperm having a genotype heterozygous for such genes and leading to plants with altered the normal starch synthesis pathway is disclosed in US 20050091716.

[0009.0.25.25] Nevertheles, there is a constant need for providing novel enzyme activities or direct or indirect regulators and thus alternative methods with advantageous properties for producing carbohydrates, preferably polysaccharides like starch or cellulose and/or monosaccharides like myo-inositol or its precursor in organisms, e.g. in transgenic organisms.

[0010.0.25.25] Another problem is the seasonal change in carbohydrate composition of plants and optimum harvest periods for are complicated by issues of timing.

[0011.0.25.25] To ensure constantly a high quality of foods and animal feeds, it is necessary to add one or a plurality of carbohydrates, preferably polysaccharides like starch or cellulose and/or monosaccharides like myo-inositol in a balanced manner to suit the organism.

[0012.0.25.25] Accordingly, there is still a great demand for new and more suitable genes which encode enzymes which participate in the biosynthesis of carbohydrates, preferably polysaccharides like starch or cellulose and/or monosaccharides like myo-inositol and make it possible to produce them specifically on an industrial scale without unwanted byproducts forming. In the selection of genes for or regulators of biosynthesis two characteristics above all are particularly important. On the one hand, there is as ever a need for improved processes for obtaining the highest possible contents of carbohydrates, preferably polysaccharides like starch or cellulose and/or monosaccharides like myo-inositol; on the other hand as less as possible byproducts should be produced in the production process.
The added carbohydrates further beneficially affects the microflora by selectively stimulating the growth and/or activity of beneficial bacteria.
Another aspect is the significant reduction of cost of production and manufacturing not only to the nutrition, in particular sweetener industry, but also agriculture and cosmetic and health industry.

**[0013.0.0.25]** see **[0013.0.0.0]**

[0014.0.25.25] Accordingly, in a first embodiment, the invention relates to a process for the production of a fine chemical, whereby the fine chemical is carbohydrates, preferably polysaccharides, more preferably starch and/or cellulose and/or monosaccharides, more preferably myo-inositoi.,. ,. Accordingly, in the present invention, the term "the fine chemical" as used herein relates to (a) "carbohydrates, preferably polysaccharides, more preferably starch and/or cellulose and/or monosaccharides, more preferably myo-inositol. Further, the term "the fine chemicals" as used herein also relates to compositions of fine chemicals comprising carbohydrates, preferably polysaccharides, more preferably starch and/or cellulose and/or monosaccharides, more preferably myo-inositol

**[0015.0.25.25]** In one embodiment, the term " carbohydrate" or "the fine chemical" or "the respective fine chemical" means at least one chemical compound with carbohydrate activity selected from the group of preferably polysaccharides, more preferably starch and/or cellulose and/or monosaccharides, more preferably myo-inositol. In an preferred embodiment, the term "the fine chemical" or the term " carbohydrate" or the term "the respective fine chemical" means at least one chemical compound with carbohydrate activity selected from the group "carbohydrates, preferably polysaccharides, more preferably starch and/or cellulose and/or monosaccharides, more preferably myo-inositol. An increased carbohydrate content normally means an increased total carbohydrate content. However, an increased carbohydrate content also means, in particular, a modified content of the above-described compounds with carbohydrate activity, without the need for an inevitable increase in the total carbohydrate content.. In a preferred embodiment, the term "the fine chemical" means carbohydrates, preferably polysaccharides, more preferably starch and/or cellulose and/or monosaccharides, more preferably myo-inositol in free form or its salts or its ester or its ether or bound to acylglycerol.

[0015.1.22.22] A measure for the content of the polysaccharides, preferably starch and cellulose, of the invention can be the content of anhydroglucose. This compound is the analyte which indicates the presence of the polysaccharides, preferably starch and cellulose, of the invention if the samples are prepared and measured as described in the examples.

[0016.0.25.25] Accordingly, the present invention relates to a process for the production of the respective fine chemical comprising
(a) increasing or generating the activity of one or more
   of a protein as shown in table II, application no. 25, column 3 encoded by the nucleic acid sequences as shown in table I, application no. 25, column 5, in a non-human organism or in one or more parts thereof or
(b) growing the organism under conditions which permit the production of the fine chemical, thus myo-inositol and/or anhydroglucose of the invention or fine chemicals comprising myo-inositol and/or anhydroglucose of the invention, in said organism or in the culture medium surrounding the organism.

[0016.1.25.25] Accordingly, the term "the fine chemical" means in one embodiment "myo-inositol" in relation to all sequences listed in Tables I to IV, line 48 or homologs thereof and
means in one embodiment "anhydroglucose", preferably "starch" and/or "cellulose"in relation to all sequences listed in Table I to IV, line 49 and/or 50 or homologs thereof.

**[0017.0.25.25]** to **[0019.0.0.25]: see [0017.0.0.0]** to **[0019.0.0.0]**

[0020.0.25.25] Surprisingly it was found, that the transgenic expression of the Brassica napus protein as indicated in Table II, column 5, line 48 in a plant conferred an increase in myo-inositol content of the transformed plants. Thus, in one embodiment, said protein or its homologs are used for the production of myo-inositol. Surprisingly it was found, that the transgenic expression of the Brassica napus protein as indicated in Table II, column 5, line 49 in a plant conferred an increase in anhydroglucose content of the transformed plants. Thus, in one embodiment, said protein or its homologs are used for the production of anhydroglucose.
Surprisingly it was found, that the transgenic expression of the Glycine max protein as indicated in Table II, column 5, line 50 in a plant conferred an increase in anhydroglucose-inositol content of the transformed plants. Thus, in one embodiment, said protein or its homologs are used for the production of anhydroglucose.

**[0021.0.25.25]** see **[0021.0.0.0]**

**[0022.0.25.25]** The sequence of b3430 from Escherichia coli K12 has been published in Blattner et al., Science 277(5331), 1453-1474, 1997, and its activity is being defined as a glucose-1-phosphate adenylyltransferase. Accordingly, in one embodiment, the process of the present invention comprises the use of a gene product with an activity of the glucose-1-phosphate adenylyltransferase superfamily, preferably a protein with a glucose-1-phosphate adenylyltransferase activity or its homolog, e.g. as shown herein, from Escherichia coli K12 or a plant or its homolog, e.g. as shown herein, for the production of the fine chemical, meaning of carbohydrate, preferably myo-inositol in free or bound form in an organism or a part thereof, as mentioned.
The sequence of b1539 (Accession number NP_416057) from Escherichia coli K12 has been published in Blattner et al., Science 277 (5331), 1453-1474, 1997, and its activity is being defined as a NADP-dependent L-serine/L-allo-threonine dehydrogenase. Accordingly, in one embodiment, the process of the present invention comprises the use of a gene product with an activity of ribitol dehydrogenase, short-chain alcohol dehydrogenase homology superfamily, preferably a protein with the activity of a NADP-dependent L-serine/L-allo-threonine dehydrogenase from E. coli or a plant or its homolog, e.g. as shown herein, for the production of the fine chemical, meaning of starch and/or cellulose, particular for increasing the amount of starch and/or cellulose, preferably starch and/or cellulose in free or bound form in an organism or a part thereof, as mentioned.
The sequence of b4232 from Escherichia coli K12 (ACCESSION No. NP_416986) has been published in Blattner, Science 277(5331), 1453-1474, 1997, and its activity is beeing defined as a fructose-1,6-bisphosphatase. Accordingly, in one embodiment, the process of the present invention comprises the use of a "fructose-1,6-bisphosphatase" from E. coli or a plant or its homolog, e.g. as shown herein, for the production of the fine chemical, meaning of methionin, in particular for increasing the amount of methionine in free or bound form in an organism or a part thereof, as mentioned. In one embodiment, in the process of the present invention the activity of a protein of the superfamily "fructose-bisphosphatase", preferably having an activity in C-compound and carbohydrate metabolism, C-compound and carbohydrate utilization, energy, glycolysis and gluconeogenesis, plastid, photosynthesis, more preferred having an "fructose-1,6-bisphosphatase"-activity, is increased or generated, e.g. from E. coli or a plant or a homolog thereof. Accordingly, in one embodiment, in the process of the present invention the activity of a "fructose-1,6-bisphosphatase" or its homolog is increased for the production of the fine chemical, meaning of starch and/or cellulose, in particular for increasing the amount of starch and/or cellulose in free or bound form in an organism or a part thereof, as mentioned.

**[0022.1.0.25]** to **[00239.0.0.25]: see [0022.1.0.0]** to **[0023.0.0.0]**

**[0023.1.25.25]Homologs** of the polypeptide disclosed in table II, application no. 25, column 5, may have the activity of a protein as disclosed in table I or II, application no. 25, column 3 and may be the polypetides encoded by the nucleic acid molecules indicated in table I, application no. 25, column 7, resp., or may be the polypeptides indicated in table II, application no. 25, column 7, resp.

**[0024.0.0.9]** see **[0024.0.0.0]**

**[0025.0.25.25]** In accordance with the invention, a protein or polypeptide has the "activity of an protein as shown in table II, application no. 25, column 3" if its de *novo* activity, or its increased expression directly or indirectly leads to an increased in the level of the fine chemical indicated in the respective line of table II, application no. 25, column 6 "metabolite" in the organism or a part thereof, preferably in a cell of said organism.
Throughout the specification the activity or preferably the biological activity of such a protein or polypeptide or an nucleic acid molecule or sequence encoding such protein or polypeptide is identical or similar if it still has the biological or enzymatic activity of a protein as shown in table II, application no. 25, column 3, or which has at least 10% of the original enzymatic or biological activity, preferably 20%, particularly preferably 30%, most particularly preferably 40% in comparison to a protein as shown in the respective line of table II, application no. 25, column 3 of a E. coli or Saccharomyces cerevisae, respectively, protein as mentioned in table I to IV, column 3 respectively and as disclosed in paragraph [0022] of the respective invention.

**[0025.1.0.25]: see [0025.1.0.0]**

**[0026.0.0.25]** to **[0033.0.0.25]: see [0026.0.0.0]** to **[0033.0.0.0]**

**[0034.0.25.25]** Preferably, the reference, control or wild type differs form the subject of the present invention only in the cellular activity, preferably of the acitvity of the polypeptide of the invention, e.g. as result of an increase in the level of the nucleic acid molecule of the present invention or an increase of the specific activity of the polypeptide of the invention, e.g. by or in the expression level or activity of an protein having the activity of a respective protein as shown in table II, application no. 25, column 3 its biochemical or genetical causes and the increased amount of the respective fine chemical.

**[0035.0.0.25]** to **[0044.0.0.25]: see [0035.0.0.0]** to **[0044.0.0.0]**

**[0045.0.25.25]** In one embodiment, the activity of the protein. as indicated in Table II, columns 5 or 7, application no. 25, is increased conferring an increase of the respective fine chemical, indicated in Table II, column 6 of the respective line, between 10% and 50%, 10% and 100%, 10% and 200%, 10% and 300%, 10% and 400%, 10% and 500%, 20% and 50%, 20% and 250%, 20% and 500%, 50% and 100%, 50% and 250%, 50% and 500%, 500% and 1000% or more.

**[0046.0.25.25]** In one embodiment, the activity of the protein as indicated in Table II, columns 5 or 7, application no. 25, is increased conferring an increase of the respective fine chemical, indicated in Table II, column 6 of the respective line confers an increase of the respective fine chemical and of further myo-inositol and/or anhydroglucose or their precursors.

**[0047.0.0.25]** to **[0048.0.0.25]: see [0047.0.0.0]** to **[0048.0.0.0]**

[0049.0.25.25]A protein having an activity conferring an increase in the amount or level of the fine chemical, has in one embodiment the structure of the polypeptide described herein, in particular of the polypeptides comprising the consensus sequence shown in table IV, application no. 25, column 7 or of the polypeptide as shown in the amino acid sequences as disclosed in table II, application no. 25, columns 5 and 7 or the functional homologues thereof as described herein, or is encoded by the nucleic acid molecule characterized herein or the nucleic acid molecule according to the invention, for example by the nucleic acid molecule as shown in table I, application no. 25, columns 5 and 7 or its herein described functional homologues and has the herein mentioned activity.

[0050.0.25.25] For the purposes of the present invention, the term "the respective fine chemical" also encompass the corresponding salts, ester and ethers, preferably etherificated with other mono-, di- oligo- or polysaccharides, with alkyl, alkenyl or alkinyl alcohols.

[0051.0.25.25] Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising carbohydrates, preferably polysaccharides, more preferably starch and/or cellulose and/or monosaccharides, more preferably myo-inositol resp.,. Depending on the choice of the organism used for the process according to the present invention, for example a microorganism or a plant, compositions or mixtures of various respective fine chemicals can be produced.

**[0052.0.0.25]** see **[0052.0.0.0]**

**[0053.0.25.25]In** one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having the activity of a protein as indicated in table II, application no. 25, columns 5 and 7 or its homologs activity having herein-mentioned myo-inositol and/or anhydroglucose of the invention increasing activity; and/or
b) stabilizing a mRNA conferring the increased expression of a protein encoded by the nucleic acid molecule of the invention as shown in table I, application no. 25, columns 5 and 7, e.g. a nucleic acid sequence encoding a polypeptide having the activity of a protein as indicated in table II, application no. 25, columns 5 and 7 or its homologs activity or of a mRNA encoding the polypeptide of the present invention having herein-mentioned myo-inositol and/or anhydroglucose of the invention increasing activity; and/or
c) increasing the specific activity of a protein conferring the increased expression of a protein encoded by the nucleic acid molecule of the invention or of the polypeptide of the present invention having herein-mentioned myo-inositol and/or anhydroglucose increasing activity, e.g. of a polypeptide having the activity of a protein as indicated in table II, application no. 25, columns 5 and 7 or its homologs activity, or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned myo-inositol and/or anhydroglucose of the invention increasing activity, e.g. of a polypeptide having the activity of a protein as indicated in table II, application no. 25, columns 5 and 7 or its homologs activity; and/or
e) stimulating activity of a protein conferring the increased expression of a protein encoded by the nucleic acid molecule of the present invention or a polypeptide of the present invention having herein-mentioned myo-inositol and/or anhydroglucose of the invention increasing activity, e.g. of a polypeptide having the activity of a protein as indicated in table II, application no. 25, columns 5 and 7 or its homologs activity, by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention, having herein-mentioned myo-inositol and/or anhydroglucose of the invention increasing activity, e.g. of a polypeptide having the activity of a protein as indicated in table II, application no. 25, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned myo-inositol and/or anhydroglucose of the invention increasing activity, e.g. of a polypeptide having the activity of a protein as indicated in table II, application no. 25, columns 5 and 7 or its homologs activity; and/or
h) increasing the expression of the endogenous gene encoding the polypeptide of the invention, e.g. a polypeptide having the activity of a protein as indicated in table II, application no. 25, columns 5 and 7 or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with especially high activity of the proteins of the invention from natural or from mutagenized resources and breeding them into the target organisms, eg the elite crops.

[0054.0.25.25] Preferably, said mRNA is the nucleic acid molecule of the present invention and/or the protein conferring the increased expression of a protein encoded by the nucleic acid molecule of the present invention or the polypeptide having the herein mentioned activity, e.g. conferring the increase of the respective fine chemical as indicated in column 6 of application no. 25 in Table I to IV, resp., after increasing the expression or activity of the encoded polypeptide or having the activity of a polypeptide having an activity as the protein as shown in table II, application no. 25, column 3 or its homologs.

**[0055.0.0.25] to [0064.0.0.25]: see [0055.0.0.0] to [0064.0.0.0]**

**[0065.0.0.25]: see [0065.0.0.0]**

**[0066.0.0.25] to [0067.0.0.25]: see [0066.0.0.0] to [0067.0.0.0]**

[0068.0.25.25] The mutation is introduced in such a way that the production of the carbohydrates, preferably polysaccharides, more preferably starch and/or cellulose and/or monosaccharides, more preferably myo-inositolis not adversely affected.

**[0069.0.0.25] see [0069.0.0.0]**

[0070.0.25.25] Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create de *novo* an advantageous, preferably novel metabolites composition in the organism, e.g. an advantageous composition carbohydrates comprising a higher content of (from a viewpoint of nutrional physiology limited) preferably polysaccharides, more preferably starch and/or cellulose and/or monosaccharides, more preferably myo-inositol.

**[0071.0.25.25]** Preferably the composition further comprises higher amounts of metabolites positively affecting or lower amounts of metabolites negatively affecting the nutrition or health of animals or humans provided with said compositions or organisms of the invention or parts thereof. Likewise, the number or activity of further genes which are required for the import or export of nutrients or metabolites, including carbohydrates or its precursors, required for the cell's biosynthesis of carbohydratesmay be increased so that the concentration of necessary or relevant precursors, cofactors or intermediates within the cell(s) or within the corresponding storage compartments is increased. Owing to the increased or novel generated activity of the polypeptide of the invention or the polypeptide used in the method of the invention or owing to the increased number of nucleic acid sequences of the invention and/or to the modulation of further genes which are involved in the biosynthesis of the carbohydrates, e.g. by increasing the acitivty of enzymes synthesizing precursors or by destroying the activity of one or more genes which are involved in the breakdown of the carbohydrates, it is possible to increase the yield, production and/or production efficiency of carbohydratesin the host organism, such as the plants or the microorganims.

[0072.0.25.25] By influencing the metabolism thus, it is possible to produce, in the process according to the invention, further advantageous compounds. Examples of such compounds are, in addition to polysaccharides, more preferably starch and/or cellulose and/or monosaccharides, more preferably myo-inositol, further carbohydrates, preferably saccharides.

[0073.0.25.25] Accordingly, in one embodiment, the process according to the invention relates to a process, which comprises:
a) providing a non-human organism, preferably a microorganism, a non-human animal, a plant or animal cell, a plant or animal tissue or a plant;
b) increasing an activity of a polypeptide of the invention or a homolog thereof, e.g. as indicated in Table II, application no. 25, columns 5 or 7, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
d) if desired, revovering, optionally isolating, the free and/or bound the respective fine chemical and, optionally further free and/or bound carbohydrate(s) synthetized by the organism, the microorganism, the non-human animal, the plant or animal cell, the plant or animal tissue or the plant.

[0074.0.25.25] The organism, in particular the microorganism, non-human animal, the plant or animal cell, the plant or animal tissue or the plant is advantageously grown in such a way that it is not only possible to recover, if desired isolate the free or bound the respective fine chemical or the free and bound the respective fine chemical but as option it is also possible to produce, recover and, if desired isolate, other free or/and bound carbohydrate(s).

**[0075.0.0.25]** to **[0077.0.0.25]: see [0075.0.0.0]** to **[0077.0.0.0]**

[0078.0.25.25] The organism such as microorganisms or plants or the recovered, and if desired isolated, respective fine chemical can then be processed further directly into foodstuffs or animal feeds or for other applications in nutrition or medicine or cosmetics, for example according to the disclosures made in US 6,669,962: Starch microcapsules for delivery of active agents; US 20050042737 : Starch process; US 20050054071: Enzymes for starch processing; US 20050091716 : Novel plants and processes for obtaining them; U.S. Pat. No. 5,096,594 and 5,482,631 discloses a method of purifying cyclitols; U.S. Pat. No. 4,997,489 discloses soaking almond hulls in water to obtain a syrup containing fructose, glucose, inositol, and sorbitol; U.S. Pat. No. 5,296,364 discloses a microbial method for producing inositol; US. 4,734,402; US. 4,788,065; US 6,465,037 and US 6,355,295 : relates to soy food ingredient based on carbohydrates, US 6,653,451; US 20040128713: pertains to soybean plants having in their seeds significantly lower contents of raffinose, stachyose and phytic acid and significantly higher contents of sucrose and inorganic phosphate; US 20050008713 discloses compositions of plant carbohydrates for dietary supplements and nutritional support; which are expressly incorporated herein by reference. The fermentation broth, fermentation products, plants or plant products can be treated and processed as described in above mentioned applications or by other methods known to the person skilled in the art and described herein below.
In the method for producing carbohydrates, preferably polysaccharides, more preferably starch and/or cellulose and/or monosaccharides, more preferably myo-inositol according to the invention, the cultivation step of the genetically modified organisms, also referred to as transgenic organisms hereinbelow, is preferably followed by harvesting said organisms and isolating the respective carbohydrate(s) from said organisms.
The organisms are harvested in a manner known per se and appropriate for the particular organism. Microorganisms such as bacteria, mosses, yeasts and fungi or plant cells which are cultured in liquid media by fermentation may be removed, for example, by centrifugation, decanting or filtration. Plants are grown on solid media in a manner known per se and harvested accordingly.
Carbohydrates, preferably polysaccharides, more preferably starch and/or cellulose and/or monosaccharides, more preferably myo-inositol are isolated from the harvested biomass in a manner known per se, for example by extraction and, where appropriate, further chemical or physical purification processes such as, for example, chemical and/or enzymatical degradation, precipitation methods, crystallography, thermal separation methods such as rectification methods or physical separation methods such as, for example, chromatography.
Products of these different work-up procedures are carbohydrates, preferably polysaccharides, more preferably starch and/or cellulose and/or monosaccharides, more preferably myo-inositol comprising compositions, e.g. compostions comprising carbohydrates, preferably polysaccharides, more preferably starch and/or cellulose and/or monosaccharides, more preferably myo-inositol which still comprise fermentation broth, plant particles and/or cell components in different amounts, advantageously in the range of from 0 to 99% by weight, preferably below 80% by weight, especially preferably between 50%, 40%, 30%, 20%, 20%, 10%, 5%, 3%, 2%, 1%, 05%, 0,1 %, 0,01 % and 0% by weight resp..
In one embodiment, preferred plants include, but are not limited to: sugar beet, sugar cane, soybeans and/or potato (Solanum tuberosum).

**[0079.0.0.25]** to **[0084.0.0.25]: see [0079.0.0.0]** to **[0084.0.0.0]**

[0085.0.25.25] With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, application no. 25, columns 5 or 7, or a derivative thereof, or
b) a genetic regulatory element, for example a promoter, which is functionally linked to the nucleic acid sequence as indicated in Table I, application no. 25, columns 5 or 7, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.25]** to **[0087.0.0.25]: see [0086.0.0.0]** to **[0087.0.0.0]**

[0088.0.25.25] In an advantageous embodiment of the invention, the organism takes the form of a plant whose content the respective fine chemical is modified advantageously owing to the nucleic acid molecule of the present invention expressed. This is important for humans and animals since, for example, the nutritional value of plants for nutrition is dependent on the abovementioned carbohydrates and the general amount ofsaccharides as energy source in feed.

**[0088.1.0.25]** to **[0095.0.0.25]:** see **[0088.1.0.0]** to **[0095.0.0.0]**

[0096.0..25.25] In another preferred embodiment of the invention a combination of the increased expression of the nucleic acid sequence or the protein of the invention together with the transformation of a protein or polypeptide or a compound, which functions as a sink for the desired fine chemical, for example carbohydrates, preferably polysaccharides, more preferably starch and/or cellulose and/or monosaccharides, more preferably myo-inositol in the organism, is useful to increase the production of the respective fine chemical.

**[0097.0.0.25] see [0097.0.0.0]**

[0098.0.25.25] In a preferred embodiment, the respective fine chemical (carbohydrates, preferably polysaccharides, more preferably starch and/or cellulose and/or monosaccharides, more preferably myo-inositol) is produced in accordance with the invention and, if desired, is isolated. The production of carbohydrates, or mixtures thereof or mixtures with other compounds by the process according to the invention is advantageous.

[0099.0.25.25] In the case of the fermentation of microorganisms, the abovementioned carbohydrates, preferably polysaccharides, more preferably starch and/or cellulose and/or monosaccharides, more preferably myo-inositol may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical or the carbohydrates, preferably polysaccharides, more preferably starch and/or cellulose and/or monosaccharides, more preferably myo-inositol compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

[0100.0.25.25] Transgenic plants which comprise the carbohydrates, preferably polysaccharides, more preferably starch and/or cellulose and/or monosaccharides, more preferably myo-inositol synthesized in the process according to the invention can advantageously be marketed directly without there being any need for the oils, lipids or fatty acids synthesized to be isolated. Plants for the process according to the invention are listed as meaning intact plants and all plant parts, plant organs or plant parts such as leaf, stem, seeds, root, tubers, anthers, fibers, fruits, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, in the form of their extracts, e.g. water containing extract, or as fibre in case of starch and/or cellulose, as degradation products (chemical ar enzymatical degradation) or crystallisation product or as their ethers and/or esters and/or free carbohydrates, preferably polysaccharides, more preferably starch and/or cellulose and/or monosaccharides, more preferably myo-inositol. The respective fine chemical produced by this process can be obtained by harvesting the organisms, either from the crop in which they grow, or from the field. This can be done via pressing or extraction of the plant parts, e.g. in the plant stem, seeds, root, tubers, anthers, fibers, fruits. To increase the efficiency of extraction it is beneficial to clean, to temper and if necessary to hull and to flake the plant material expecially the stem, seeds, root, tubers, anthers, fibers, fruits. extracts and/ carbohydrates, preferably polysaccharides, more preferably starch and/or cellulose and/or monosaccharides, more preferably myo-inositol can be obtained by what is known as cold beating or cold pressing without applying heat. To allow for greater ease of disruption of the plant parts, specifically the stem, seeds, root, tubers, anthers, fibers, fruits, they can previously be comminuted, steamed or roasted. Plant parts, which have been pretreated in this manner can subsequently be pressed or extracted with solvents or water. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degradiated, crystallized and/or refined.

**[0101.0.25.25] see [0101.0.0.0]**

[0102.0..25.25] Carbohydrates, preferably polysaccharides, more preferably starch and/or cellulose and/or monosaccharides, more preferably myo-inositol can for example be analyzed advantageously via HPLC or GC separation methods and detected by MS oder MSMS methods. The unambiguous detection for the presence of carbohydrates, preferably polysaccharides, more preferably starch and/or cellulose and/or monosaccharides, more preferably myo-inositolcontaining products can be obtained by analyzing recombinant organisms using analytical standard methods: GC, GC-MS, LC, LC-MSMS or TLC, as described on several occasions.. The carbohydrates can be analized further in plant extracts by anion-exchange chromatography with pulsed amperometric detection (Cataldi et al., Anal Chem.;72(16):3902-7, 2000), by enzymatic "BioAnalysis" usind test kits from R-Biopharm and Roche or from Megazyme, Ireland.

[0103.0.25.25] In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, application no. 25, columns 5 or 7, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, application no. 25, columns 5 or 7,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybidizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, application no. 25, column 7, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, application no. 25, column 7, and conferring an increase in the amount of the respective fine chemical in an organism or a part thereof ;
k) nucleic acid molecule comprising one or more of the nucleic acid molecule encoding the amino acid sequence of a polypeptide encoding a domain of the polypeptide indicated in Table II, application no. 25, columns 5 or 7, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.0.4.]** In one embodiment, the nucleic acid molecule used in the process of the invention distinguishes over the sequence indicated in Table I, application no. 25, columns 5 or 7 by one or more nucleotides. In one embodiment, the nucleic acid molecule used in the process of the invention does not consist of the sequence shown in indicated in Table I ,application no. 25, columns 5 or 7,. In one embodiment, the nucleic acid molecule used in the process of the invention is less than 100%, 99,999%, 99,99%, 99,9% or 99% identical to a sequence indicated in Table I , application no. 25, columns 5 or 7,. In another embodiment, the nucleic acid molecule does not encode a polypeptide of a sequence indicated in Table II, application no. 25, columns 5 or 7.

**[0104.0.25.25]** In one embodiment, the nucleic acid molecule used in the process distinguishes over the sequence indicated in Table I, application no. 25, columns 5 or 7, by one or more nucleotides. In one embodiment, the nucleic acid molecule used in the process of the invention does not consist of the sequence indicated in Table I, application no. 25, columns 5 or 7. In one embodiment, the nucleic acid molecule of the present invention is less than 100%, 99,999%, 99,99%, 99,9% or 99% identical to the sequence indicated in Table I, application no. 25, columns 5 or 7. In another embodiment, the nucleic acid molecule does not encode a polypeptide of a sequence indicated in Table II, application no. 25, columns 5 or 7.

**[0105.0.0.25]** to **[0107.0.0.25]: see [0105.0.0.0]** to **[0107.0.0.0]**

**[0108.0.25.25]** Nucleic acid molecules with the sequence as indicated in Table I, application no. 25, columns 5 or 7, nucleic acid molecules which are derived from an amino acid sequences as indicated in Table II, application no. 25, columns 5 or 7, or from polypeptides comprising the consensus sequence as indicated in Table IV, application no. 25, column 7, or their derivatives or homologues encoding polypeptides with the enzymatic or biological activity of an activity of a polypeptide as indicated in Table II, application no. 25, column 3, 5 or 7, or conferring an increase of the respective fine chemical, meaning carbohydrates, preferably polysaccharides, more preferably starch and/or cellulose and/or monosaccharides, more preferably myo-inositol after increasing its expression or activity are advantageously increased in the process according to the invention.

**[0109.0.0.25] see [0109.0.0.0]**

**[0110.0.25.25]** Nucleic acid molecules, which are advantageous for the process according to the invention and which encode polypeptides with an activity of a polypeptide of the invention or the polypeptide used in the method of the invention or used in the process of the invention , e.g. of a protein as indicated in Table II, application no. 25, column 5, or being encoded by a nucleic acid molecule indicated in Table I, application no. 25, column 5, or of its homologs, e.g. as indicated in Table II, application no. 25, column 7, can be determined from generally accessible databases.

**[0111.0.0.25] see [0111.0.0.0]**

[0112.0.25.25] The nucleic acid molecules used in the process according to the invention take the form of isolated nucleic acid sequences, which encode polypeptides with an activity of a polypeptide as indicated in Table II, application no. 25, column 3, or having the sequence of a polypeptide as indicated in Table II, application no. 25, columns 5 and 7, and conferring an increase in the level of carbohydrates, preferably polysaccharides, more preferably starch and/or cellulose and/or monosaccharides, more preferably myo-inositol.

**[0113.0.0.25] to [0120.0.0.25]: see [0113.0.0.0] to [0120.0.0.0]**

[0121.0.25.25] However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, application no. 25, columns 5 or 7, or the functional homologues thereof as described herein, preferably conferring above-mentioned activity, i.e. conferring an increase in the level of the fine chemical of the invention after increasing the activity of the polypeptide sequences indicated in Table II, application no. 25, columns 5 or 7.

**[0122.0.0.25] to [0127.0.0.259]: see [0122.0.0.0] to [0127.0.0.0]**

[0128.0.9.9] Synthetic oligonucleotide primers for the amplification, e.g. as the pairs indicated in Table III, application no. 25, columns 7, by means of polymerase chain reaction can be generated on the basis of a sequence shown herein, for example the sequence as indicated in Table I, application no. 25, columns 5 or 7, or the sequences derived from a sequence as indicated in Table II, application no. 25, columns 5 or 7.

[0129.0.25.25] Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived.. Conserved regions are those, which show a very little variation in the amino acid in one particular position of several homologs from different origin. The consenus sequences indicated in Table IV, application no. 25, column 7, are derived from said aligments.

[0130.0.25.25] Degenerated primers can then be utilized by PCR for the amplification of fragments of novel proteins having above-mentioned activity, e.g. conferring the increase of carbohydrates, preferably polysaccharides, more preferably starch and/or cellulose and/or monosaccharides, more preferably myo-inositol after increasing the expression or activity of the protein comprising said fragment.

**[0131.0.0.25] to [0138.0.0.25]: see [0131.0.0.0] to [0138.0.0.0]**

**[0139.0.25.25]** Polypeptides having above-mentioned activity, i.e. conferring the increase of the respective fine chemical level, derived from other organisms, can be encoded by other DNA sequences which hybridize to a sequences indicated in Table I, application no. 25, columns 5 or 7, under relaxed hybridization conditions and which code on expression for peptides having the respective fine chemical, in particular, of starch and/or cellulose and/or myo-inositol, resp., increasing activity.

**[0140.0.0.25] to [0146.0.0.25]: see [0140.0.0.0] to [0146.0.0.0]**

**[0147.0.25.25]:** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, application no. 25, columns 5 or 7, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.25.25]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, application no. 25, columns 5 or 7 or a portion thereof and preferably has above mentioned activity, in particular, of myo-inositol and/or anhydroglucose increasing activity after increasing the acitivity or an activity of a product of a gene encoding said sequences or their homologs.

**[0149.0.25.25]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, application no. 25, columns 5 or 7, or a portion thereof and encodes a protein having above-mentioned activity and as indicated in indicated in Table II.

**[00149.1.25.25]** Optionally, the nucleotide sequence, which hybridises to one of the nucleotide sequences indicated in Table I, application no. 25, columns 5 or 7, has further one or more of the activities annotated or known for the a protein as indicated in Table II, application no. 25, column 3.

**[0150.0.25.25]** Moreover, the nucleic acid molecule of the invention can comprise only a portion of the coding region of one of the sequences indicated in Table I, application no. 25, columns 5 or 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of myo-inositol and/or anhydroglucose, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, application no. 25, columns 5 or 7, , an anti-sense sequence of one of the sequences, e.g., as indicated in Table I, application no. 25, columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, application no. 25, column 7, will result in a fragment of a polynucleotide sequence as indicated in Table I, application no. 25, columns 5 or 7 or its gene product.

**[0151.0.0.25]:** see **[0151.0.0.0]**

[0152.0.25.25] The nucleic acid molecule of the invention encodes a polypeptide or portion thereof which includes an amino acid sequence which is sufficiently homologous to an amino acid sequence as indicated in Table II, application no. 25, columns 5 or 7, such that the protein or portion thereof maintains the ability to participate in the respective fine chemical production, in particular an activity increasing the level of carbohydrates, preferably polysaccharides, more preferably starch and/or cellulose and/or monosaccharides, more preferably myo-inositol resp., as mentioned above or as described in the examples in plants or microorganisms is comprised.

[0153.0.25.25] As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, application no. 25, columns 5 or 7, such that the protein or portion thereof is able to participate in the increase of the respective fine chemical production. In one embodiment, a protein or portion thereof as indicated in Table II, application no. 25, columns 5 or 7, has for example an activity of a polypeptide as indicaded in Table II, application no. 25, column 3.

[0154.0.25.25] In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, application no. 25, columns 5 or 7, and has above-mentioned activity, e.g.conferring preferably the increase of the respective fine chemical.

**[0155.0.0.25]** to **[0156.0.0.25]: see [0155.0.0.0]** to **[0156.0.0.0]**

[0157.0.25]The invention further relates to nucleic acid molecules that differ from one of the nucleotide sequences indicated in Table I, application no. 25, columns 5 or 7, (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising a consensus sequence as indicated in Table IV, application no. 25, column 7, or of the polypeptide as indicated in Table II, application no. 25, columns 5 or 7, or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, application no. 25, column 7, or of the polypeptide as indicated in Table II, application no. 25, column 7, or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, application no. 25, column 7, or of a polypeptide as indicated in Table II, application no. 25, columns 5 or 7, or the functional homologues thereof. However, in a preferred embodiment, the nucleic acid molecule of the present invention does not consist of a sequence as indicated in Table I, application no. 25, columns 5 or 7.

**[0158.0.0.25]** to **[0160.0.0.25]: see [0158.0.0.0]** to **[0160.0.0.0]**

**[0161.0.25.25]:** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, application no. 25, columns 5 or 7. The nucleic acid molecule is preferably at least 20, 30, 50, 100, 250 or more nucleotides in length.

**[0162.0.0.25]:** see **[0162.0.0.0]**

**[0163.0.25.25]:** Preferably, nucleic acid molecule of the invention that hybridizes under stringent conditions to a sequence as indicated in Table I, application no. 25, columns 5 or 7, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.25]: see [0164.0.0.0]**

[0165.0.25.25] For example, nucleotide substitutions leading to amino acid substitutions at "non-essential" amino acid residues can be made in a sequence of the nucleic acid molecule of the invention or used in the process of the invention, e.g. as indicated in Table I, application no. 25, columns 5 or 7.

**[0166.0.0.25] to [0167.0.0.25]: see [0166.0.0.0] to [0167.0.0.0]**

[0168.0.25.25]: Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, application no. 25, columns 5 or 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, application no. 25, columns 5 or 7, and is capable of participation in the increase of production of the respective fine chemical after increasing its activity, e.g. its expression. Preferably, the protein encoded by the nucleic acid molecule is at least about 60% identical to a sequence as indicated in Table II, application no. 25, columns 5 or 7, more preferably at least about 70% identical to one of the sequences as indicated in Table II, application no. 25, columns 5 or 7, even more preferably at least about 80%, 90%, or 95% homologous to a sequence as indicated in Table II, application no. 25, columns 5 or 7, , and most preferably at least about 96%, 97%, 98%, or 99% identical to the sequence as indicated in Table II, application no. 25, columns 5 or 7.

**[0169.0.0.25]** to **[0172.0.0.25]: see [0169.0.0.0]** to **[0172.0.0.0]**

[0173.0.25.25]: For example a sequence which has a 80% homology with sequence SEQ lD NO: 159 at the nucleic acid level is understood as meaning a sequence which, upon comparison with the sequence SEQ ID NO: 159 by the above Gap program algorithm with the above parameter set, has a 80% homology.

**[0174.0.0.25]: see [0174.0.0.0]**

[0175.0.25.25]: For example a sequence which has a 80% homology with sequence SEQ ID NO: 160 at the protein level is understood as meaning a sequence which, upon comparison with the sequence SEQ ID NO: 160 by the above program algorithm with the above parameter set, has a 80% homology.

**[0176.0.25.25]:** Functional equivalents derived from one of the polypeptides as indicated in Table II, application no. 25, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table II, application no. 25, columns 5 or 7, according to the invention and are distinguished by essentially the same properties as a polypeptide as indicated in Table II, application no. 25, columns 5 or 7.

[0177.0.25.25]: Functional equivalents derived from a nucleic acid sequence as indicated in Table I, application no. 25, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of a polypeptide as indicated in Table II, application no. 25, columns 5 or according to the invention and encode polypeptides having essentially the same properties as a polypeptide as indicated in Table II, application no. 25, columns 5 or 7.

**[0178.0.0.25]: see [0178.0.0.0]**

[0179.0.25.25]: A nucleic acid molecule encoding an homologous to a protein sequence as indicated in Table II, application no. 25, columns 5 or 7, can be created by introducing one or more nucleotide substitutions, additions or deletions into a nucleotide sequence of the nucleic acid molecule of the present invention, in particular as indicated in Table I, application no. 25, columns 5 or 7, such that one or more amino acid substitutions, additions or deletions are introduced into the encoded protein. Mutations can be introduced into the encoding sequences of a sequences as indicated in Table I, application no. 25, columns 5 or 7, by standard techniques, such as site-directed mutagenesis and PCR-mediated mutagenesis.

**[0180.0.0.25] to [0183.0.0.25]: see [0180.0.0.0] to [0183.0.0.0]**

**[0184.0.25.25]** Homologues of the nucleic acid sequences used, with a sequence as indicated in Table I, columns 5 or 7, application no. 25, column 7, or of the nucleic acid sequences derived from a sequences as indicated in Table II, application no. 25, columns 5 or 7, comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, application no. 25, columns 5 or 7, or from the derived nucleic acid sequences, the intention being, however, that the enzyme activity or the biological activity of the resulting proteins synthesized is advantageously retained or increased.

[0185.0.25.25] In one embodiment of the present invention, the nucleic acid molecule of the invention or used in the process of the invention comprises one or more sequences as indicated in Table I, application no. 25, columns 5 or 7,. In one embodiment, it is preferred that the nucleic acid molecule comprises as little as possible other nucleotides not shown in any one of sequences as indicated in Table I, application no. 25, columns 5 or 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, application no. 25, columns 5 or 7.

**[0186.0.25.25]** Also preferred is that one or more nucleic acid molecule(s) used in the process of the invention encodes a polypeptide comprising a sequence as indicated in Table II, application no. 25, columns 5 or 7.. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, application no. 25, columns 5 or 7.

**[0187.0.25.25]** In one embodiment, a nucleic acid molecule of the invention or used in the process encodes a polypeptide comprising the sequence as indicated in Table II, application no. 25, columns 5 or 7, and comprises less than 100 further nucleotides. In a further embodiment, said nucleic acid molecule comprises less than 30 further nucleotides. In one embodiment, the nucleic acid molecule used in the process is identical to a coding sequence encoding a polypeptide as indicated in Table II, application no. 25, columns 5 or 7.

[0188.0.25.25] Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, application no. 25, columns 5 or 7 and is expressed under identical conditions.

[0189.0.25.25] Homologues of a sequences as indicated in Table I, application no. 25, columns 5 or 7, or of a derived sequences as indicated in Table II, application no. 25, columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.25] see [0190.0.0.0]**

**[0191.0.0.25] see [0191.0.0.0]:**

**[0192.0.0.25] to [0203.0.0.25] see [0192.0.0.0] to [0203.0.0.0]**

[0204.0.25.25] Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, application no. 25, columns 5 or 7, or a fragment thereof conferring an increase in the amount of the respective fine chemical, in particular according to table II, application no. 25, column 6 in an organism or a part thereof
b) nucleic acid molecule comprising, preferably at least the mature form, of a nucleic acid molecule as indicated in Table I, application no. 25, columns 5 or 7, or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, application no. 25, column 7, and conferring an increase in the amount of the respective fine chemical, in particular according to table II, application no. 25, column 6 in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, application no. 25, columns 5 or 7, and conferring an increase in the amount of the respective fine chemical, in particular according to table II, application no. 25, column 6 in an organism or a part thereof;
k) nucleic acid molecule encoding the amino acid sequence of a polypeptide encoding a domaine of a polypeptide as indicated in Table II, application no. 25, columns 5 or 7, and conferring an increase in the amount of the respective fine chemical, in particular accccording to table II, application no. 25, column 6 in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, application no. 25, columns 5 or 7, or a nucleic acid molecule encoding, preferably at least the mature form of, a polypeptide as indicated in Table II, application no. 25, columns 5 or 7, and conferring an increase in the amount of the respective fine chemical according to table II, application no. 25, column 6 in an organism or a part thereof;
   or which encompasses a sequence which is complementary thereto;
whereby, preferably, the nucleic acid molecule according to (a) to (I) distinguishes over the sequence indicated in Table I, application no. 25,columns 5 or 7, by one or more nucleotides. In one embodiment, the nucleic acid molecule does not consist of the sequence shown and indicated in Table I, application no. 25, columns 5 or 7,
In one embodiment, the nucleic acid molecule is less than 100%, 99,999%, 99,99%, 99,9% or 99% identical to a sequence indicated in Table I, application no. 25, columns 5 or 7,.
In another embodiment, the nucleic acid molecule does not encode a polypeptide of a sequence indicated in Table II, application no. 25, columns 5 or 7,.
In an other embodiment, the nucleic acid molecule of the present invention is at least 30 %, 40 %, 50%, or 60% identical and less than 100%, 99,999%, 99,99%, 99,9% or 99% identical to a sequence indicated in Table I, application no. 25, columns 5 or 7,.
In a further embodiment the nucleic acid molecule does not encode a polypeptide sequence as indicated in Table II, application no. 25, columns 5 or 7,.
Accordingly, in one embodiment, the nucleic acid molecule of the differs at least in one or more residues from a nucleic acid molecule indicated in Table I, application no. 25, columns 5 or 7,.
Accordingly, in one embodiment, the nucleic acid molecule of the present invention encodes a polypeptide, which differs at least in one or more amino acids from a polypeptide indicated in Table II, application no. 25, columns 5 or 7,.
In another embodiment, a nucleic acid molecule indicated in Table I, application no. 25, columns 5 or 7,.
Accordingly, in one embodiment, the protein encoded by a sequences of a nucleic acid accoriding to (a) to (l) does not consist of a sequence as indicated in Table II, application no. 25, columns 5 or 7,.
In a further embodiment, the protein of the present invention is at least 30 %, 40 %, 50%, or 60% identical to a protein sequence indicated in Table II, application no. 25, columns 5 or 7, and less than 100%, preferably less than 99,999%, 99,99% or 99,9%, more preferably less than 99%, 985, 97%, 96% or 95% identical to a sequence as indicated in Table I, columns 5 or 7.

**[0205.0.0.25]** to **[0206.0.0.25] see [0205.0.0.0]** to **[0206.0.0.0]**

[0207.0.25.25] As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the carbohydrate or starch metabolism, the glucose metabolism, the saccharide metabolism, the metabolism of glycolysis, or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.25] to [0226.0.0.25]: see [0208.0.0.0] to [0226.0.0.0]**

[0227.0.25.25]: The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table I, application no. 25, columns 5 or 7, lines 290 to 294 and/or 604 to 607; or its derivatives, it is advantageous additionally to express and/or mutate further genes in the organisms. Especially advantageously, additionally at least one further gene of the carbohydrate biosynthetic pathway is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the carbohydrate desired since, for example, feedback regulations no longer exist to the same extent or not at all. In addition it might be advantageously to combine one or more of the sequences indicated in Table I, application no. 25, columns 5 or 7, with genes which generally support or enhances to growth or yield of the target organismen, for example genes which lead to faster growth rate of microorganisms or genes which produces stress-, pathogen, or herbicide resistant plants.

[0228.0.25.25: In a further embodiment of the process of the invention, therefore, organisms are grown, in which there is simultaneous overexpression of at least one nucleic acid or one of the genes which code for proteins involved in the carbohydrate metabolism, in particular in synthesis of polysaccharides, more preferably starch and/or cellulose and/or monosaccharides, more preferably myo-inositol.

[0229.0.25.25]: Further advantageous nucleic acid sequences which can be expressed in combination with the sequences used in the process and/or the abovementioned biosynthesis genes are the sequences encoding further genes of the carbohydrate biosynthetic pathway. These genes can lead to an increased synthesis of the carbohydrates, preferably polysaccharides, more preferably starch and/or cellulose and/or monosaccharides, more preferably myo-inositol resp.

**[0230.0.0.25] see [0230.0.0.0]**

[0231.0.25.25] In a further advantageous embodiment of the process of the invention, the organisms used in the process are those in which simultaneously a carbohydrate degrading protein is attenuated, in particular by reducing the rate of expression of the corresponding gene.

**[0232.0.0.25] to [0276.0.0.25]: see [0232.0.0.0] to [0276.0.0.0]**

[0277.0..25.25] The respective fine chemical produced can be isolated from the organism by methods with which the skilled worker is familiar. For example, via extraction, chemical or enzymatical degradiation, crystallization, salt precipitation, and/or different chromatography methods. The process according to the invention can be conducted batchwise, semibatchwise or continuously. The respective fine chemcical produced by this process can be obtained by harvesting the organisms, either from the crop in which they grow, or from the field. This can be done via pressing or extraction of the plant parts

**[0278.0.0.25]** to **[0282.0.0.25]: see [0278.0.0.0]** to **[0282.0.0.0]**

**[0283.0.25.25]:** Moreover, a native polypeptide conferring the increase of the respective fine chemical in an organism or part thereof can be isolated from cells (e.g., endothelial cells), for example using the antibody of the present invention as described below, e.g. an antibody against a protein as indicated in Table II, application no. 25, column 3, or an antibody against a polypeptide as indicated in Table II, application no. 25, columns 5 or 7, which can be produced by standard techniques utilizing the polypeptid of the present invention or fragment thereof, i.e., the polypeptide of this invention. Preferred are monoclonal antibodies.

**[0284.0.0.25]: see [0284.0.0.0]**

**[0285.0.25.25]:** In one embodiment, the present invention relates to a polypeptide having a sequence as indicated in Table II, application no. 25, columns 5 or 7, or as encoded by a nucleic acid molecule as indicated in Table I, application no. 25, columns 5 or 7, or functional homologues thereof.

[0286.0.25.25]: In one advantageous embodiment, in the method of the present invention the activity of a polypeptide is increased which comprises or consists of a consensus sequence as indicated in Table IV, application no. 25, column 7. In another embodiment, the present invention relates to a polypeptide comprising or consisting of a consensus sequence as indicated in Table IV, application no. 25, column 7, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid and/or be absent.
In one embodiment, the present invention relates to the method of the present invention comprising a polypeptide or to a polypeptide comprising more than one consensus sequences (of an individual line) as indicated in Table IV, application no. 25, column 7.

**[0287.0.0.25]** to **[0290.0.0.25]: see [0287.0.0.0]** to **[0290.0.0.0]**

[0291.0.25.25]: In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II, application no. 25, columns 5 or 7, by one or more amino acids. In one embodiment, polypeptide distinguishes form a sequence as indicated in Table II, application no. 25, columns 5 or 7, by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II, application no. 25, columns 5 or 7, by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II, application no. 25, columns 5 or 7.

**[0292.0.0.25]: see [0292.0.0.0]**

[0293.0.25.25]: In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II, application no. 25, columns 5 or 7, by one or more amino acids. In an other embodiment, said polypeptide of the invention does not consist of the sequence as indicated in Table II, application no. 25, columns 5 or 7,.
In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I, application no. 25, columns 5 or 7.

[0294.0.25.25] In one embodiment, the present invention relates to a polypeptide having an activity of a protein as indicated in Table II, application no. 25, column 3, which distinguishes over a sequence as indicated in Table II, application no. 25, columns 5 or 7, by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.25]** to **[0297.0.0.25]: see [0295.0.0.0]** to **[0297.0.0.0]**

[0297.1.25.25] Non polypeptide of the invention-chemicals are e.g. polypeptides having not the activity of a polypeptide indicated in Table II, application no. 25, columns 3, 5 or 7.

**[0298.0.25.25]:** A polypeptide of the invention can participate in the process of the present invention. The polypeptide or a portion thereof comprises preferably an amino acid sequence which is sufficiently homologous to an amino acid sequence as indicated in Table II, application no. 25, columns 5 or 7. The portion of the protein is preferably a biologically active portion as described herein. Preferably, the polypeptide used in the process of the invention has an amino acid sequence identical to a sequence as indicated in Table II, application no. 25, columns 5 or 7.

**[0299.0.25.25]:** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, application no. 25, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, application no. 25, columns 5 or 7, or which is homologous thereto, as defined above.

**[0300.0.25.25]:** In one embodiment, the present invention relates to a polypeptide having an activity of a protein as indicated in Table II, application no. 25, column 3, which distinguishes over a sequence as indicated in Table II, application no. 25, columns 5 or 7, by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0301.0.0.25] see [0301.0.0.0]**

[0302.0.25.25] Biologically active portions of an polypeptide of the present invention include peptides comprising amino acid sequences derived from the amino acid sequence of the polypeptide of the present invention or used in the process of the present invention, e.g., an amino acid sequence as indicated in Table II, application no. 25, columns 5 or 7, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.25]: see [0303.0.0.0]**

**[0304.0.25.25]:** Manipulation of the nucleic acid molecule of the invention may result in the production of a protein having essentially the activity of the polypeptides as indicated in Table II, application no. 25, column 3, but having differences in the sequence from said wild-type protein. These proteins may be improved in efficiency or activity, may be present in greater numbers in the cell than is usual, or may be decreased in efficiency or activity in relation to the wild type protein. **[0305.0.0.25]** to **[0308.0.0.25]: see [0305.0.0.0]** to **[0308.0.0.0]**

[0309.0.25.25]: In one embodiment, a reference to a protein (= polypeptide) of the invention or as indicated in Table II, application no. 25, columns 5 or 7, refers to a polypeptide having an amino acid sequence corresponding to the polypeptide of the invention or used in the process of the invention , whereas a " non-polypeptide of the invention" or "other polypeptide" not being indicated in Table II, application no. 25, columns 5 or 7, refers to a polypeptide having an amino acid sequence corresponding to a protein which is not substantially homologous a polypeptide of the invention, preferably which is not substantially homologous to a polypeptide as indicated in Table II, application no. 25, columns 5 or 7, e.g., a protein which does not confer the activity described herein or annotated or known for as indicated in Table II, application no. 25, column 3, and which is derived from the same or a different organism. In one embodmeint, a "non-polypeptide of the invention" or "other polypeptide" not being indicated in Table II, application no. 25, columns 5 or 7, does not confer an increase of the respective fine chemical in an organism or part therof.

**[0310.0.0.25] to [0334.0.0.25]: see [0310.0.0.0] to [0334.0.0.0]**

[0335.0.25.25]: The dsRNAi method has proved to be particularly effective and advantageous for reducing the expression of a nucleic acid sequences as indicated in Table I, application no. 25, columns 5 or 7, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, application no. 25, columns 5 or 7, and/or homologs thereof. In a double-stranded RNA molecule for reducing the expression of an protein encoded by a nucleic acid sequence sequences as indicated in Table I, application no. 25, columns 5 or 7, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.25] to [0342.0.0.25]: see [0336.0.0.0] to [0342.0.0.0]**

[0343.0.25.25]: As has already been described, 100 % sequence identity between the dsRNA and a gene transcript of a nucleic acid sequence as indicated in Table I, application no. 25, columns 5 or 7, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, application no. 25, columns 5 or 7, or homologs thereof of the one organism, may be used to suppress the corresponding expression in another organism.

**[0344.0.0.25] to [0361.0.0.25]: see [0344.0.0.0]** to **[0361.0.0.0]**

**[0362.0.25.25]:** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, application no. 25, e.g. encoding a polypeptide having protein activity, as indicated in Table II, application no. 25, columns 3. Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, application no. 25, columns 5 or 7, means herein that due to modulation or manipulation of the genome, an activity as annotated for a polypeptide as indicated in Table II, application no. 25, column 3, e.g. having a sequence as indicated in Table II, application no. 25, columns 5 or 7, is increased in a cell or an organism or a part thereof. Examples are described above in context with the process of the invention

**[0363.0.0.25]: see [0363.0.0.0]**

**[0364.0.0.25]: see [0364.0.0.0]**

**[0365.0.0.25] to [0373.0.0.25]: see [0365.0.0.0] to [0373.0.0.0]**

[0374.0.25.25]: Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. Carbohydrates, preferably polysaccharides, more preferably starch and/or cellulose and/or monosaccharides, more preferably myo-inositol resp., produced in the process according to the invention may, however, also be isolated from the plant in the form of their free form or bound in or to compounds or moieties. Carbohydrates, preferably polysaccharides, more preferably starch and/or cellulose and/or monosaccharides, more preferably myo-inositol resp.,produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.25] to [0376.0.0.25]: see [0375.0.0.0] to [0376.0.0.0]**

[0377.0.25.25]: Accordingly, the present invention relates also to a process according to the present invention whereby the produced carbohydrates, preferably polysaccharides, more preferably starch and/or cellulose and/or monosaccharides, more preferably myo-inositol comprising composition or the produced the respective fine chemical is isolated.

[0378.0.25.25]: In this manner, more than 50% by weight, advantageously more than 60% by weight, preferably more than 70% by weight, especially preferably more than 80% by weight, very especially preferably more than 90% by weight, of the carbohydrates, preferably polysaccharides, more preferably starch and/or cellulose and/or monosaccharides, more preferably myo-inositol resp., produced in the process can be isolated. The resulting carbohydrates, preferably polysaccharides, more preferably starch and/or cellulose and/or monosaccharides, more preferably myo-inositol resp.,can, if appropriate, subsequently be further purified, if desired mixed with other active ingredients such as vitamins, amino acids, carbohydrates, antibiotics and the like, and, if appropriate, formulated.

**[0379.0.25.25]:** In one embodiment, the carbohydrates, preferably polysaccharides, more preferably starch and/or cellulose and/or monosaccharides, more preferably myo-inositol resp., is a mixture comprising of one or more the respective fine chemicals. In one embodiment, the respective fine chemical means here carbohydrates, preferably polysaccharides, more preferably starch and/or cellulose and/or monosaccharides, more preferably myo-inositol. In one embodiment, carbohydrate means here a mixture of the respective fine chemicals.

**[0380.0.25.25]:** The carbohydrates, preferably polysaccharides, more preferably starch and/or cellulose and/or monosaccharides, more preferably myo-inositol resp., obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the carbohydrates, preferably polysaccharides, more preferably starch and/or cellulose and/or monosaccharides, more preferably myo-inositol comprising composition produced or the respective fine chemical produced if desired and formulating the product with a pharmaceutical acceptable carrier or formulating the product in a form acceptable for an application in agriculture. A further embodiment according to the invention is the use of the carbohydrates, preferably polysaccharides, more preferably starch and/or cellulose and/or monosaccharides, more preferably myo-inositol resp., produced in the process or of the transgenic organisms in animal feeds, foodstuffs, medicines, food supplements, cosmetics or pharmaceuticals.

**[0381.0.0.25]** to **[0382.0.0.25]: see [0381.0.0.0]** to **[0382.0.0.0]**

**[0383.0.9.25]: ./.**

**[0384.0.0.25]: see [0384.0.0.0]**

[0385.0.25.25]: The fermentation broths obtained in this way, containing in particular carbohydrates, preferably polysaccharides, more preferably starch and/or cellulose and/or monosaccharides, more preferably myo-inositol, resp., in mixtures with other compounds, in particular with other carbohydrates , or fatty acids building lipids or containing microorganisms or parts of microorganisms, like plastids, containing carbohydrates, preferably polysaccharides, more preferably starch and/or cellulose and/or monosaccharides, more preferably myo-inositol resp., in mixtures with other compounds, e.g. carbohydrates, normally have a dry matter content of from 7.5 to 25% by weight.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0..0.25] -/-**

**[0387.0.0.25]** to **[0392.0.0.25]: see [0387.0.0.0]** to **[0392.0.0.0]**

[0393.0.25.25]: In one embodiment, the present invention relates to a method for the identification of a gene product conferring an increase in the fine chemical production in a cell, comprising the following steps:
a) contacting-, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
b) identifying the nucleic acid molecules, which hybridize under relaxed stringent conditions with the nucleic acid molecule of the present invention in particular to the nucleic acid molecule sequence as indicated in Table I, application no. 25, columns 5 or 7, and, optionally, isolating the full length cDNA clone or complete genomic clone;
c) introducing the candidate nucleic acid molecules in host cells, preferably in a plant cell or a microorganism, appropriate for producing the fine chemical;
d) expressing the identified nucleic acid molecules in the host cells;
e) assaying the the fine chemical level in the host cells; and
f) identifying the nucleic acid molecule and its gene product which expression confers an increase in the the fine chemical level in the host cell after expression compared to the wild type.

**[0394.0.0.25] to [0399.0.0.25]: see [0394.0.0.0] to [0399.0.0.0]**

[00399.1.9.9]: One can think to screen for increased production of the respective fine chemical by for example searching for a resistance to a drug blocking the synthesis of the respective fine chemical and looking whether this effect is dependent on the activity or expression of a polypeptide as indicated in Table II, application no. 25, columns 5 or 7, or a homolog thereof, e.g. comparing the phenotyp of nearly identical organisms with low and high acitivity of a protein as indicated in Table II, application no. 25, columns 5 or 7, after incubation with the drug.

**[0400.0.0.25]** to **[0416.0.0.25]: see [0400.0.0.0]** to **[0416.0.0.0]**

[0417.0.9.9]: The nucleic acid molecule of the invention, the vector of the invention or the nucleic acid construct of the invention may also be useful for the production of organisms resistant to inhibitors of the carbohydrates, preferably polysaccharides, more preferably starch and/or cellulose and/or monosaccharides, more preferably myo-inositol, resp.,production biosynthesis pathways. In particular, the overexpression of the polypeptide of the present invention may protect an organism such as a microorganism or a plant against inhibitors, which block the carbohydrate, in particular the respective fine chemical synthesis in said organism. Examples of inhibitors or herbicides blocking the synthesis in organism such as microorganism or plants are for example the cellulose synthesis inhibitors 2,6-dichlorobenzonitrile (DCB), N2-(1-ethyl-3-phenylpropyl)-6-(1-fluoro-1-methylethyl)-1,3,5-triazine-2,4-diamine, called AE F150944 or isoxaben.

**[0418.0.0.25]** to **[0423.0.0.25]: see [0418.0.0.0]** to **[0423.0.0.0]**

[0424.0.25.25]: Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other carbohydrate.
Accordingly, the nucleic acid of the invention, or the nucleic acid molecule identified with the method of the present invention or the complement sequences thereof, the polypeptide of the invention, the nucleic acid construct of the invention, the organisms, the host cell, the microorgansims, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.25] to [0454.0.0.25]: see [0425.0.0.0] to [0453.0.0.0]**

[0454.0.25.25] Example 8: Analysis of the effect of the nucleic acid molecule on the production of the fine chemical resp.

**[0455.0.25.25]** The effect of the genetic modification in C. glutamicum or other microbial especially bacterial strains for carbohydrate production on the production of an carbohydrate can be determined by growing the modified microorganisms under suitable conditions (such as those described above) and analyzing the medium and/or the cellular components for the increased production of the amino acid. Such analytical techniques are well known to the skilled worker and encompass spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, pp. 89-90 and pp. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", pp. 469-714, VCH: Weinheim; Belter, P.A. et al. (1988) Bioseparations: downstream processing for Biotechnology, John Wiley and Sons; Kennedy, J.F. and Cabral, J.M.S. (1992) Recovery processes for biological Materials, John Wiley and Sons; Shaeiwitz, J.A. and Henry, J.D. (1988) Biochemical Separations, in Ullmann's Encyclopedia of Industrial Chemistry, Vol. B3; chapter 11, pp. 1-27, VCH: Weinheim; and Dechow, F.J. (1989) Separation and purification techniques in biotechnology, Noyes Publications).

**[0456.0.0.25]: see [0456.0.0.0]**

[0457.0.25.25]: Example 9: Purification of the carbohydrates, preferably polysaccharides, more preferably starch and/or cellulose and/or monosaccharides, more preferably myo-inositol

[0458.0.25.25]: Abbreviations:; GC-MS, gas liquid chromatography/mass spectrometry; TLC, thin-layer chromatography.
The unambiguous detection for the presence of carbohydrates, preferably polysaccharides, more preferably starch and/or cellulose and/or monosaccharides, more preferably myo-inositolcan be obtained by analyzing recombinant organisms using analytical standard methods: GC, GC-MS or TLC, as described (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353).
The total carbohydrate produced in the organism for example in yeasts used in the inventive process can be analysed for example according to the following procedure: The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.

**[0459.0.25.25]: -/-**

**[0460.0.0.25] see [0460.0.0.0]**

**[0461.0.25.25]** Example 10: Cloning SEQ ID NO: 159 for the expression in plants

**[0462.0.0.25]: see [0462.0.0.0]**

**[0463.025.25]** SEQ ID NO: 159 is amplified by PCR as described in the protocol of the Pfu Turbo or DNA Herculase polymerase (Stratagene).

**[0464.0.0.25]** to **[0466.0.0.25]: see [0464.0.0.0]** to **[0466.0.0.0]**

**[0467.025.25]** The following primer sequences were selected for the gene SEQ ID NO: 159:
i) forward primer: SEQ ID NO: 165
ii) reverse primer: SEQ ID NO: 166

**[0468.0.0.25] to [0479.0.0.25]: see [0468.0.0.0] to [0479.0.0.0]**

**[0480.025.25]:** Example 11: Generation of transgenic plants which express SEQ ID NO: 159

**[0481.0.0.25] to [0513.0.0.25]: see [0481.0.0.0] to [0513.0.0.0]**

[0514.0.25.25]: As an alternative, the carbohydrates, preferably polysaccharides, more preferably starch and/or cellulose and/or monosaccharides, more preferably myo-inositol, can be detected advantageously as for example described by Sonnebald et al., (Nat Biotechnol. 1997 Aug;15(8):794-7), or Panikulangara et al., Plant Physiol. 2004 Oct;136(2):3148-58.

**[0515.0.0.25]** to **[0552.0.0.25]: see [0515.0.0.0]** to **[0552.0.0.0]**

**[0553.0.25.25]** A process for the production of myo-inositol and/or anhydroglucose resp., which comprises
(a) increasing or generating the activity of a protein as indicated in Table II, application no. 25, columns 5 or 7, or a functional equivalent thereof in a non-human organism, or in one or more parts thereof; and
(b) growing the organism under conditions which permit the production of myo-inositol and/or anhydroglucose resp. in said organism.
A process for the production of myo-inositol and/or anhydroglucose resp., comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 25, columns 5 or 7, , or a fragment thereof, which confers an increase in the amount of the fine chemical as indicated in table II, column 6, e.g of myo-inositol and/or anhydroglucose resp., in an organism or a part thereof;
b) nucleic acid molecule comprising of a nucleic acid molecule as indicated in Table I, application no. 25, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of myo-inositol and/or anhydroglucose resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of myo-inositol and/or anhydroglucose resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of myo-inositol and/or anhydroglucose resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 25, column 7, and conferring an increase in the amount of the fine chemical as indicated in table II, column 6, e.g of myo-inositol and/or anhydroglucose resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of myo-inositol and/or anhydroglucose resp., in an organism or a part thereof;
h) nucleic acid molecule encoding a polypeptide comprising a consensus sequence as indicated in Table IV, application no. 25, column 7, and conferring an increase in the amount of the fine chemical as indicated in table II, column 6, e.g of myo-inositol and/or anhydroglucose resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical as indicated in table II, column 6, e.g of myo-inositol and/or anhydroglucose resp., in an organism or a part thereof.
or comprising a sequence which is complementary thereto.
The process as mentioned above, comprising recovering of the free or bound myo-inositol and/or anhydroglucose resp..
The process as mentioned above, comprising the following steps:
(a) selecting an organism or a part thereof expressing a polypeptide encoded by the nucleic acid molecule characterized as mentioned above;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound myo-inositol and/or anhydroglucose resp., produced by the selected mutated organisms or parts thereof.
The process as mentioned above, wherein the activity of said protein or the expression of said nucleic acid molecule is increased or generated transiently or stably.
An isolated nucleic acid molecule comprising a nucleic acid molecule selected from the group consisting of:
a) nucleic acid molecule encoding of a polypeptide as indicated in Table II, application no. 25, columns 5 or 7, or a fragment thereof, which confers an increase in the amount of the fine chemical as indicated in table II, column 6, e.g of myo-inositol and/or anhydroglucose resp., in an organism or a part thereof;
b) nucleic acid molecule comprising of a nucleic acid molecule as indicated in Table I, application no. 25, columns 5 or 7, ,
c) nucleic acid molecule whose sequence can be deduced from a polypeptide sequence encoded by a nucleic acid molecule of (a) or (b) as a result of the degeneracy of the genetic code and conferring an increase in the amount of the fine chemical as indicated in table II, column 6, e.g of myo-inositol and/or anhydroglucose resp., in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of the fine chemical as indicated in table II, column 6, e.g of myo-inositol and/or anhydroglucose resp., in an organism or a part thereof;
e) nucleic acid molecule which hybidizes with a nucleic acid molecule of (a) to (c) under under stringent hybridisation conditions and conferring an increase in the amount of the fine chemical as indicated in table II, column 6, e.g of myo-inositol and/or anhydroglucose resp., in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 25, column 7, and conferring an increase in the amount of the fine chemical as indicated in table II, column 6, e.g of myo-inositol and/or anhydroglucose resp., in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of the fine chemical as indicated in table II, column 6, e.g of myo-inositol and/or anhydroglucose resp., in an organism or a part thereof;
h) nucleic acid molecule encoding a polypeptide comprising a consensus sequence as indicated in Table IV, application no. 25, column 7, and conferring an increase in the amount of the fine chemical as indicated in table II, column 6, e.g of myo-inositol and/or anhydroglucose resp., in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemiccal as indicated in table II, column 6, e.g of myo-inositol and/or anhydroglucose resp., in an organism or a part thereof.
whereby the nucleic acid molecule distinguishes over the sequence as indicated in Table I, application no. 25, columns 5 or 7, by one or more nucleotides.
A nucleic acid construct which confers the expression of the nucleic acid molecule as mentioned above, comprising one or more regulatory elements.
A vector comprising the nucleic acid molecule as mentioned above or the nucleic acid construct as mentioned above.
The vector as mentioned above, wherein the nucleic acid molecule is in operable linkage with regulatory sequences for the expression in a prokaryotic or eukaryotic, or in a prokaryotic and eukaryotic, host.
A host cell, which has been transformed stably or transiently with the vector as mentioned above or the nucleic acid molecule as mentioned above or the nucleic acid construct as mentioned above or produced as described above.
The host cell as mentioned above, which is a transgenic host cell.
The host cell as mentioned above, which is a plant cell, an animal cell, a microorganism, or a yeast cell, a fungus cell, a prokaryotic cell, an eukaryotic cell or an archaebacterium.
A process for producing a polypeptide, wherein the polypeptide is expressed in a host cell as mentioned above.
A polypeptide produced by the process as mentioned above or encoded by the nucleic acid molecule as mentioned above whereby the polypeptide distinguishes over a sequence as indicated in Table II, application no. 25, columns 5 or 7, by one or more amino acids
An antibody, which binds specifically to the polypeptide as mentioned above.
A plant tissue, propagation material, harvested material or a plant comprising the host cell as mentioned above which is plant cell or an Agrobacterium.
A method for screening for agonists and antagonists of the activity of a polypeptide encoded by the nucleic acid molecule as mentioned above conferring an increase in the amount of myo-inositol and/or anhydroglucose resp., in an organism or a part thereof comprising:
(a) contacting cells, tissues , plants or microorganisms which express the a polypeptide encoded by the nucleic acid molecule as mentioned above conferring an increase in the amount of myo-inositol and/or anhydroglucose resp., in an organism or a part thereof with a candidate compound or a sample comprising a plurality of compounds under conditions which permit the expression the polypeptide;
(b) assaying the myo-inositol and/or anhydroglucose resp., level or the polypeptide expression level in the cell, tissue, plant or microorganism or the media the cell, tissue, plant or microorganisms is cultured or maintained in; and
(c) identifying a agonist or antagonist by comparing the measured myo-inositol and/or anhydroglucose resp., level or polypeptide expression level with a standard myo-inositol and/or anhydroglucose resp., or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
A process for the identification of a compound conferring increased myo-inositol, and/or anhydroglucose resp., production in a plant or microorganism, comprising the steps:
(a) culturing a plant cell or tissue or microorganism or maintaining a plant expressing the polypeptide encoded by the nucleic acid molecule as mentioned above conferring an increase in the amount of myo-inositol and/or anhydroglucose resp., in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule as mentioned above conferring an increase in the amount of myo-inositol and/or anhydroglucose rersp., in an organism or a part thereof;
(b) identifying if the compound is an effective agonist by detecting the presence or absence or increase of a signal produced by said readout system.
A method for the identification of a gene product conferring an increase in myo-inositol and/or anhydroglucose resp., production in a cell, comprising the following steps:
(a) contacting the nucleic acid molecules of a sample, which can contain a candidate gene encoding a gene product conferring an increase in myo-inositol and/or anhydroglucose resp., after expression with the nucleic acid molecule as mentioned above;
(b) identifying the nucleic acid molecules, which hybridise under relaxed stringent conditions with the nucleic acid molecule as mentioned above;
(c) introducing the candidate nucleic acid molecules in host cells appropriate for producing myo-inositol and/or anhydroglucose resp.;
(d) expressing the identified nucleic acid molecules in the host cells;
(e) assaying the myo-inositol and/or anhydroglucose rersp., level in the host cells; and
(f) identifying nucleic acid molecule and its gene product which expression confers an increase in the myo-inositol and/or anhydroglucose resp., level in the host cell in the host cell after expression compared to the wild type.
A method for the identification of a gene product conferring an increase in myo-inositol and/or anhydroglucose resp., production in a cell, comprising the following steps:
(a) identifiying in a data bank nucleic acid molecules of an organism; which can contain a candidate gene encoding a gene product conferring an increase in the myo-inositol and/or anhydroglucose resp., amount or level in an organism or a part thereof after expression, and which are at least 20% homolog to the nucleic acid molecule as mentioned above;
(b) introducing the candidate nucleic acid molecules in host cells appropriate for producing myo-inositol and/or anhydroglucose resp.,;
(c) expressing the identified nucleic acid molecules in the host cells;
(d) assaying the myo-inositol and/or anhydroglucose resp., level in the host cells; and
(e) identifying nucleic acid molecule and its gene product which expression confers an increase in the myo-inositol and/or anhydroglucose resp., level in the host cell after expression compared to the wild type.
A method for the production of an agricultural composition comprising the steps of the method as mentioned above and formulating the compound identified as mentioned above in a form acceptable for an application in agriculture.
A composition comprising the nucleic acid molecule as mentioned above, the polypeptide as mentioned above, the nucleic acid construct as mentioned above, the vector as mentioned above, an antagonist or agonist identified as mentioned above, the compound as mentioned above, the gene product as mentioned above, the antibody as mentioned above, and optionally an agricultural acceptable carrier.
Use of the nucleic acid molecule as mentioned above for the identification of a nucleic acid molecule conferring an increase of myo-inositol and/or anhydroglucose resp., after expression.
Use of the polypeptide as mentioned above or the nucleic acid construct as mentioned above or the gene product identified according to the method as mentioned above for identifying compounds capable of conferring a modulation of myo-inositol and/or anhydroglucose resp., levels in an organism.
Cosmetic, pharmaceutical, food or feed composition comprising the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above.
Use of the nucleic acid molecule as mentioned above, the polypeptide as mentioned above, the nucleic acid construct as mentioned above, the vector as mentioned above, the antagonist or agonist identified as mentioned above, the antibody as mentioned above, the plant or plant tissue as mentioned above, the harvested material as mentioned above, the host cell as mentioned above or the gene product identified according to the method as mentioned above for the protection of a plant against a myo-inositol and/or anhydroglucose synthesis inhibiting herbicide.

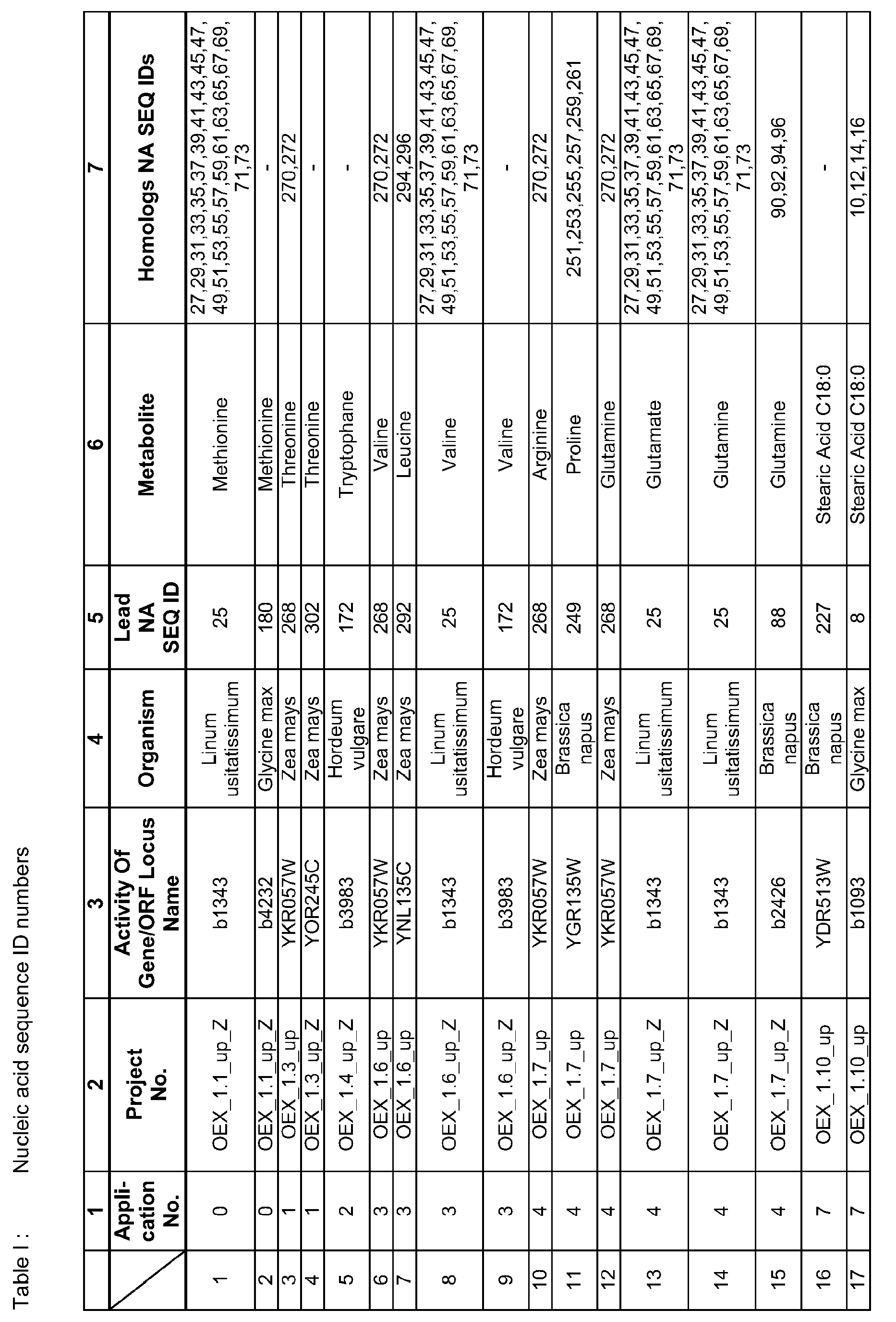

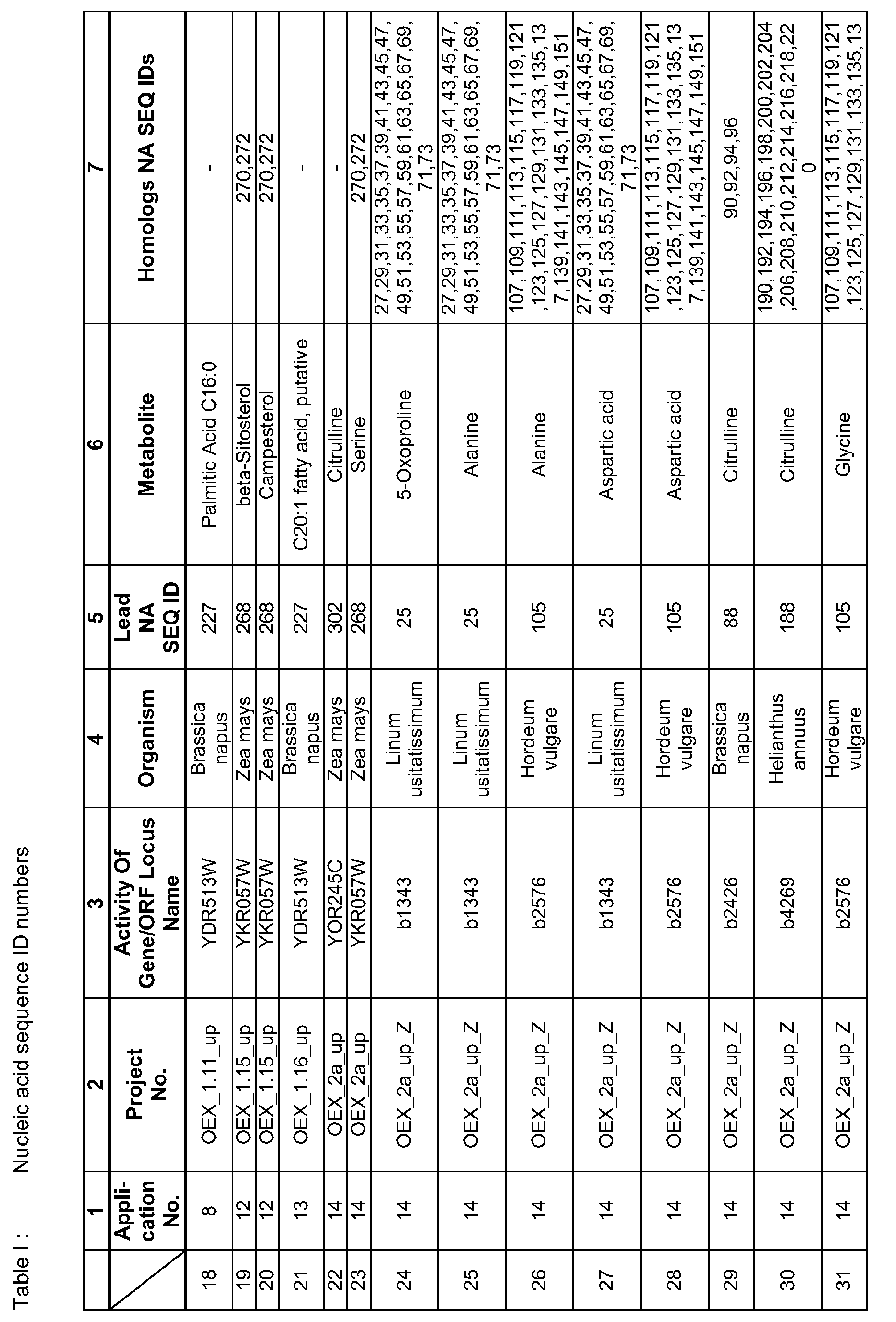

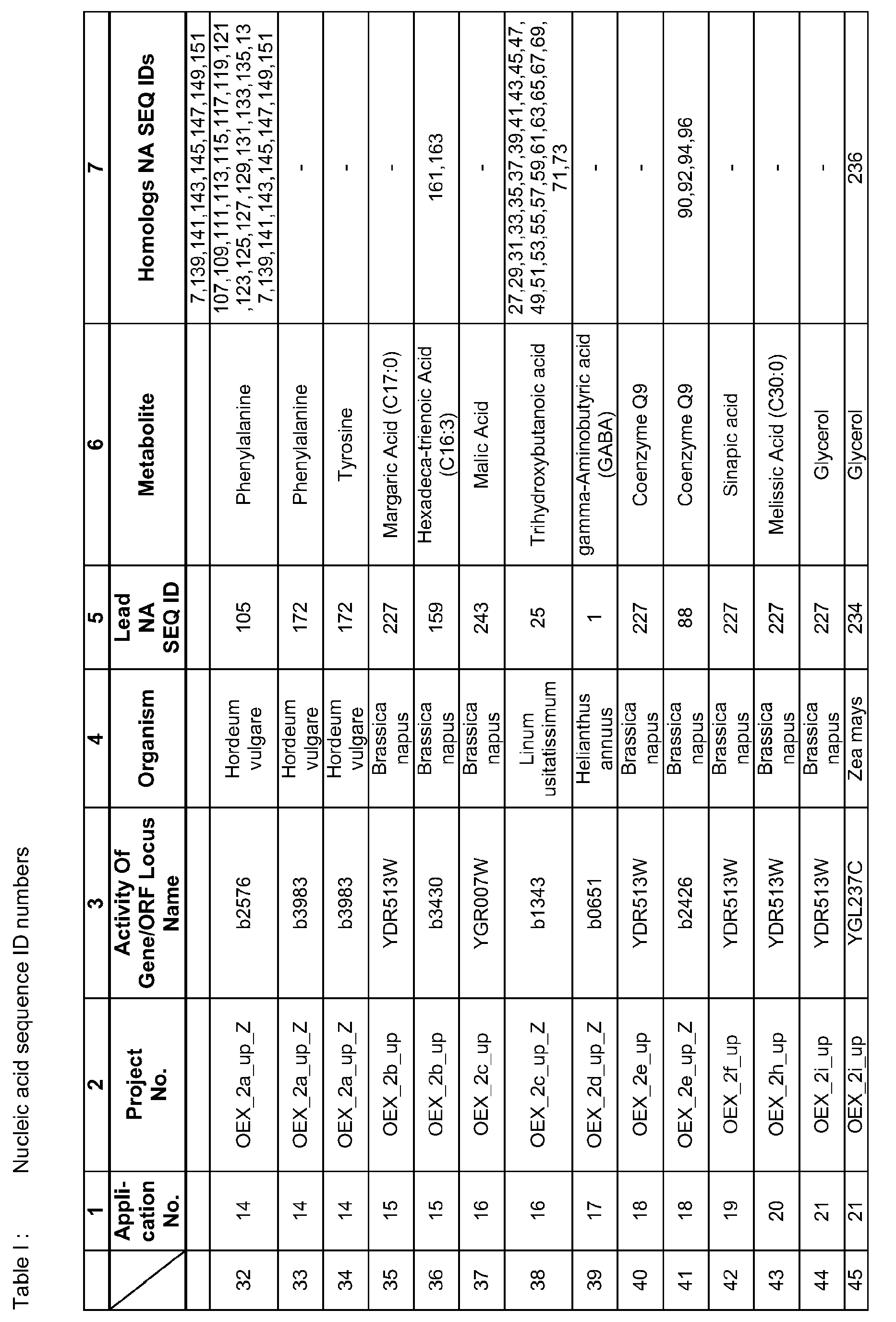

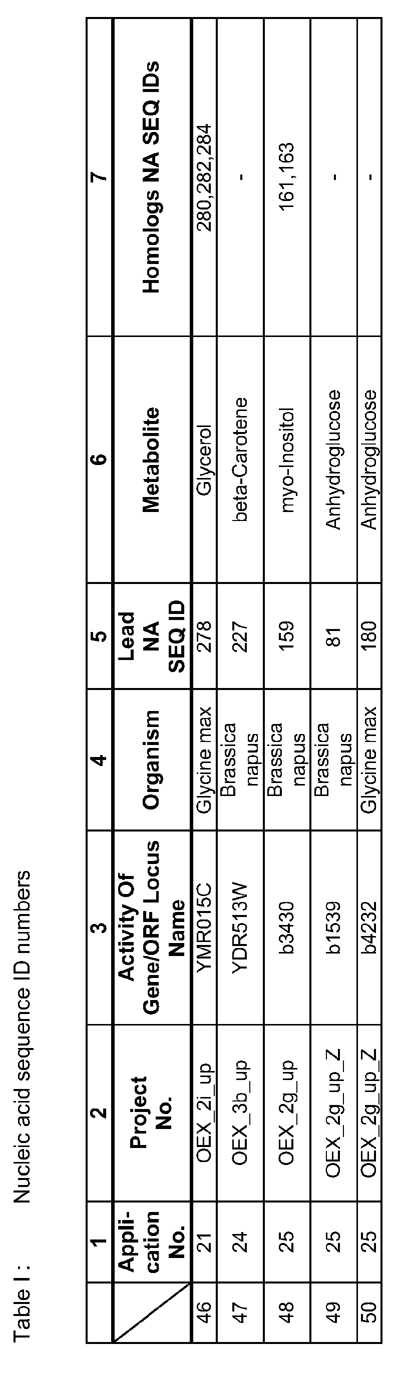

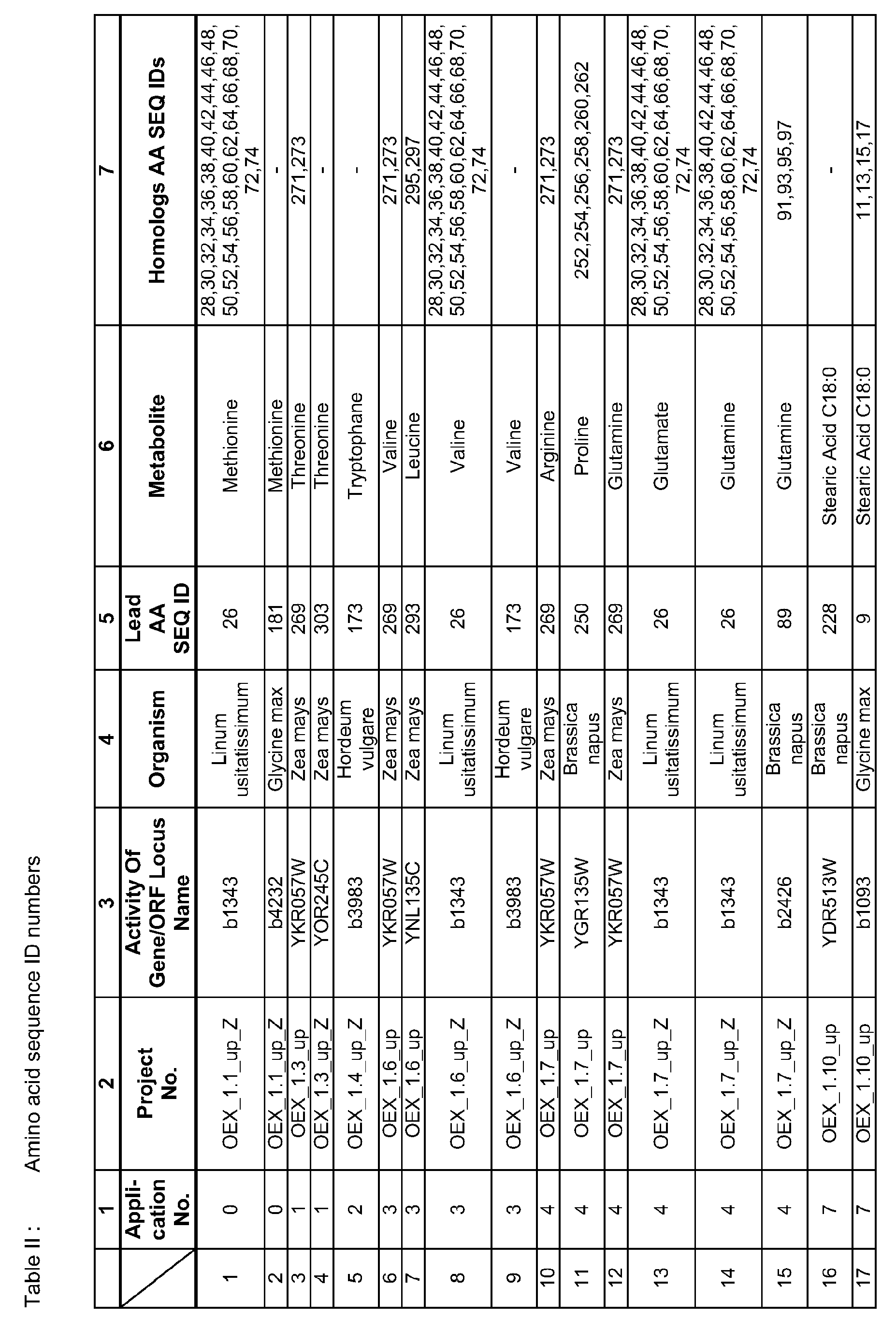

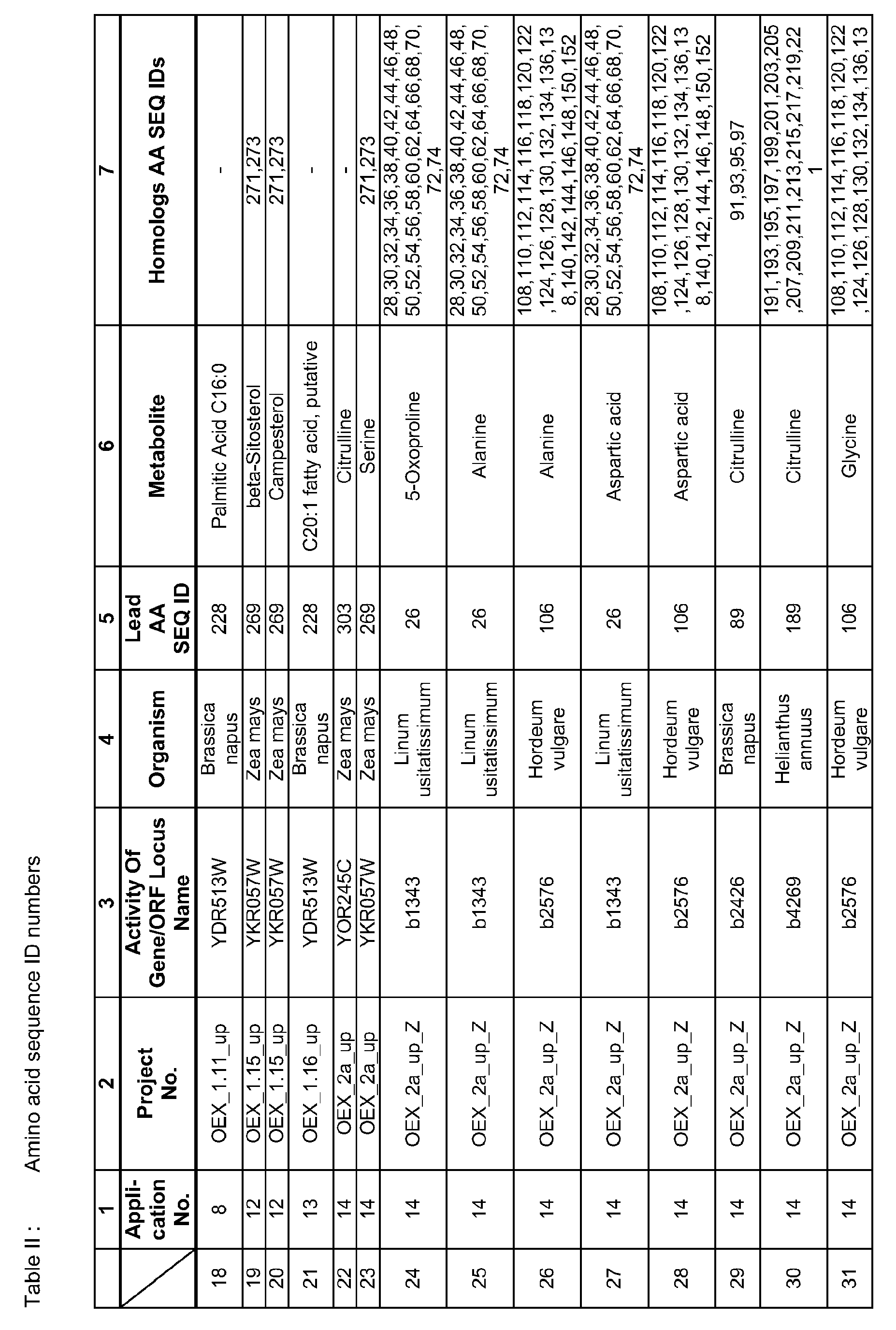

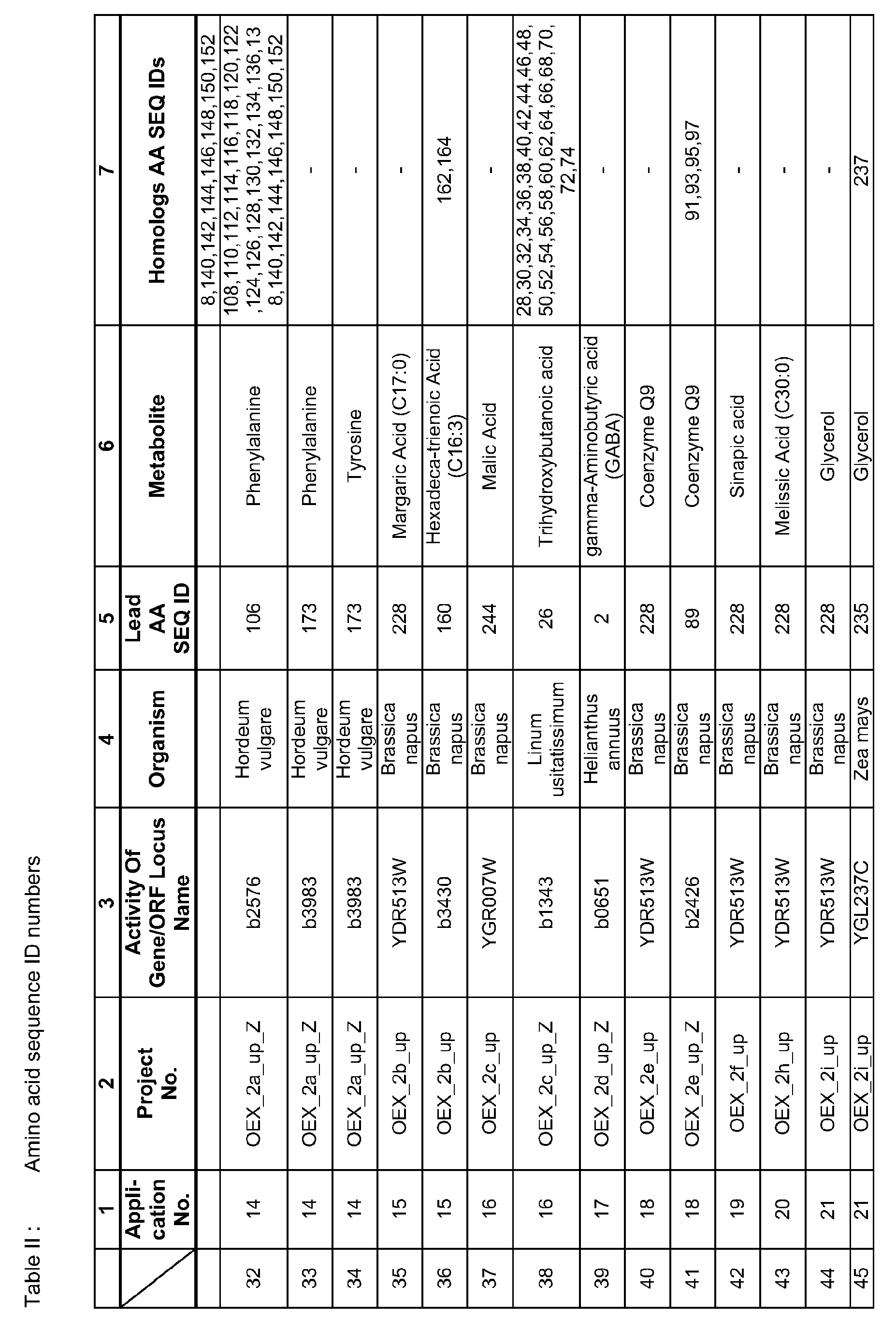

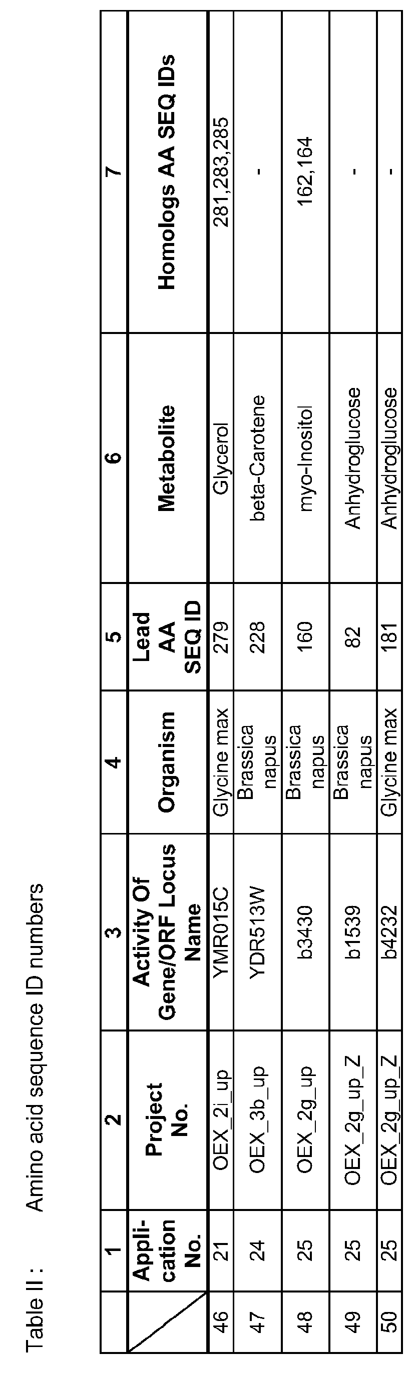

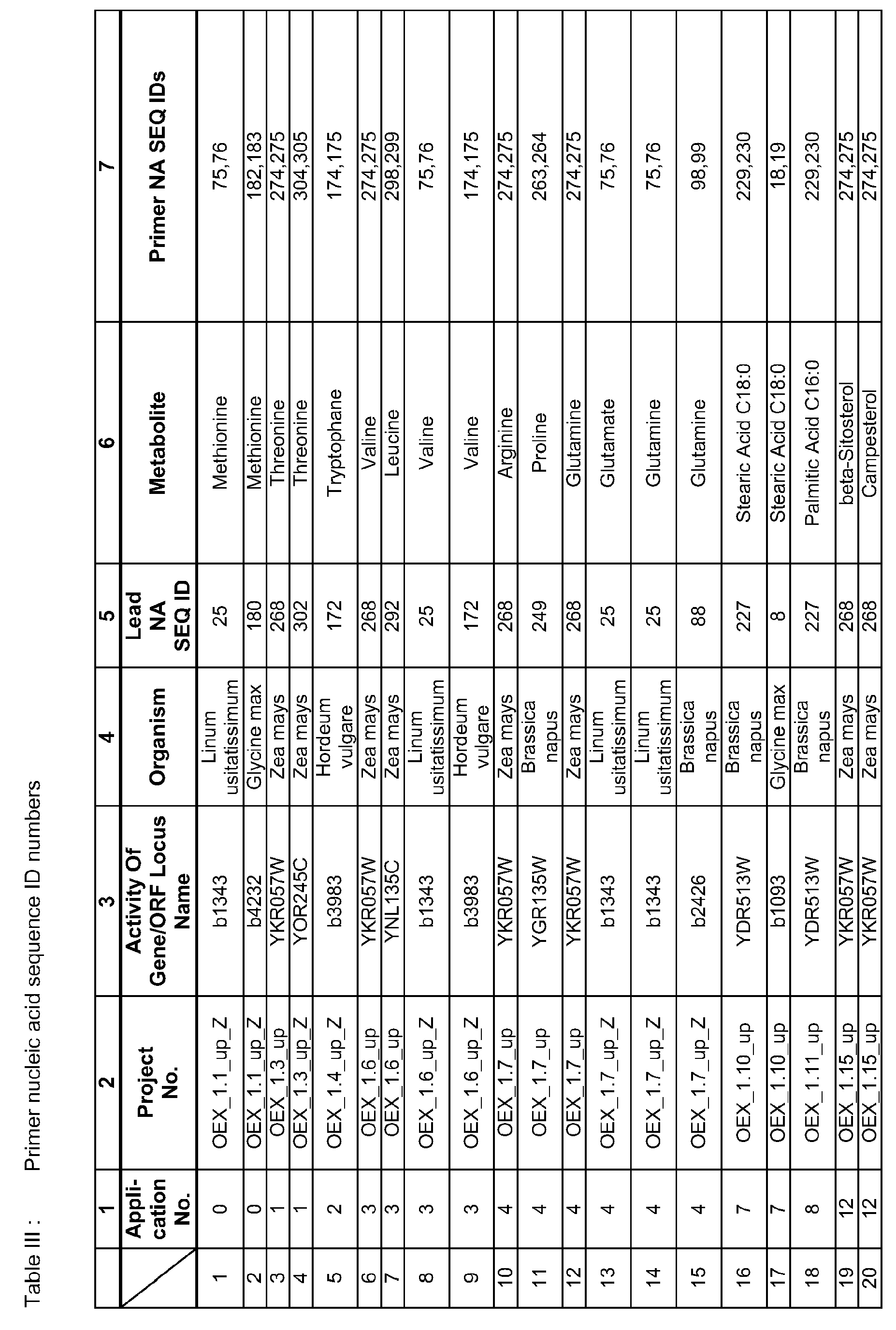

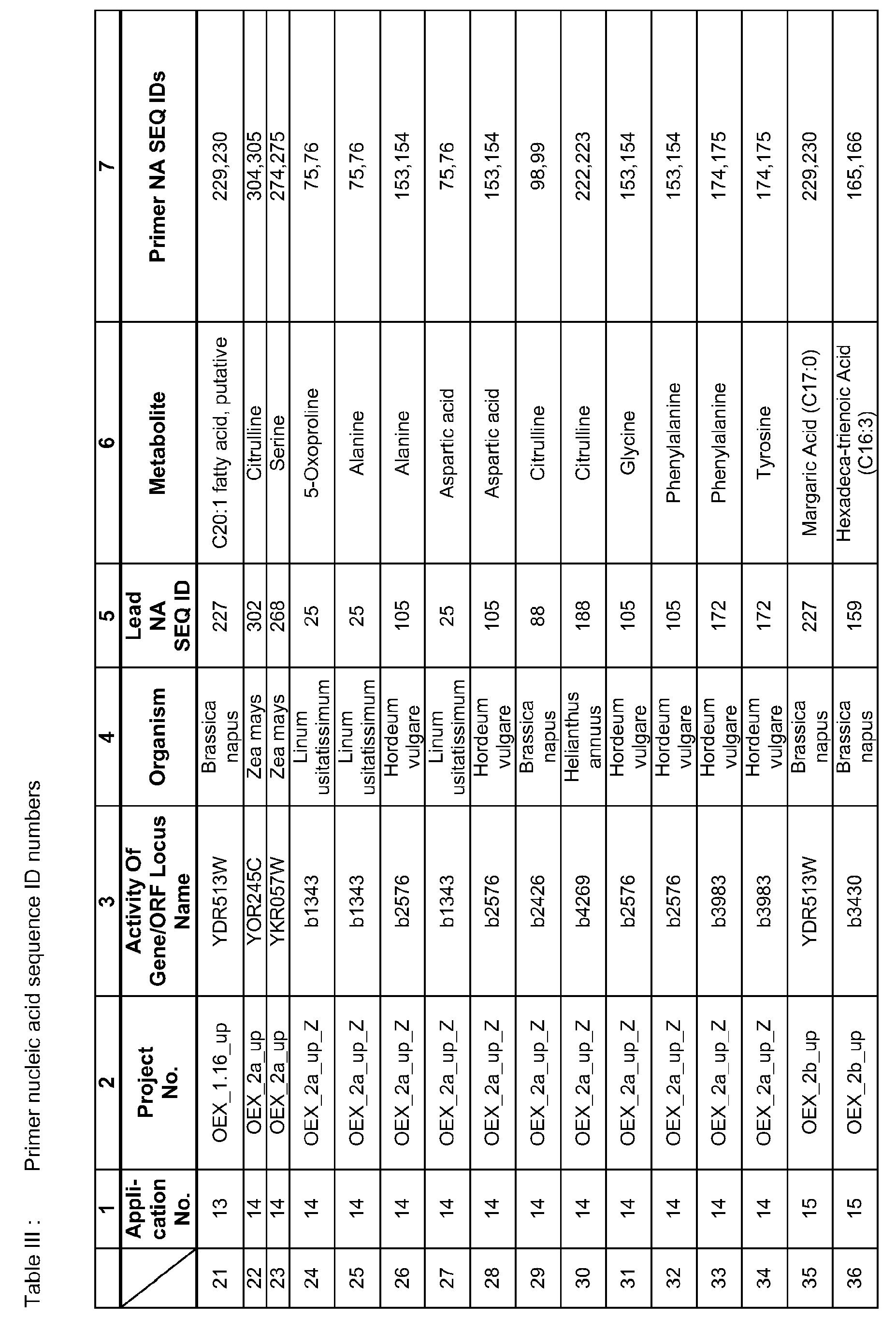

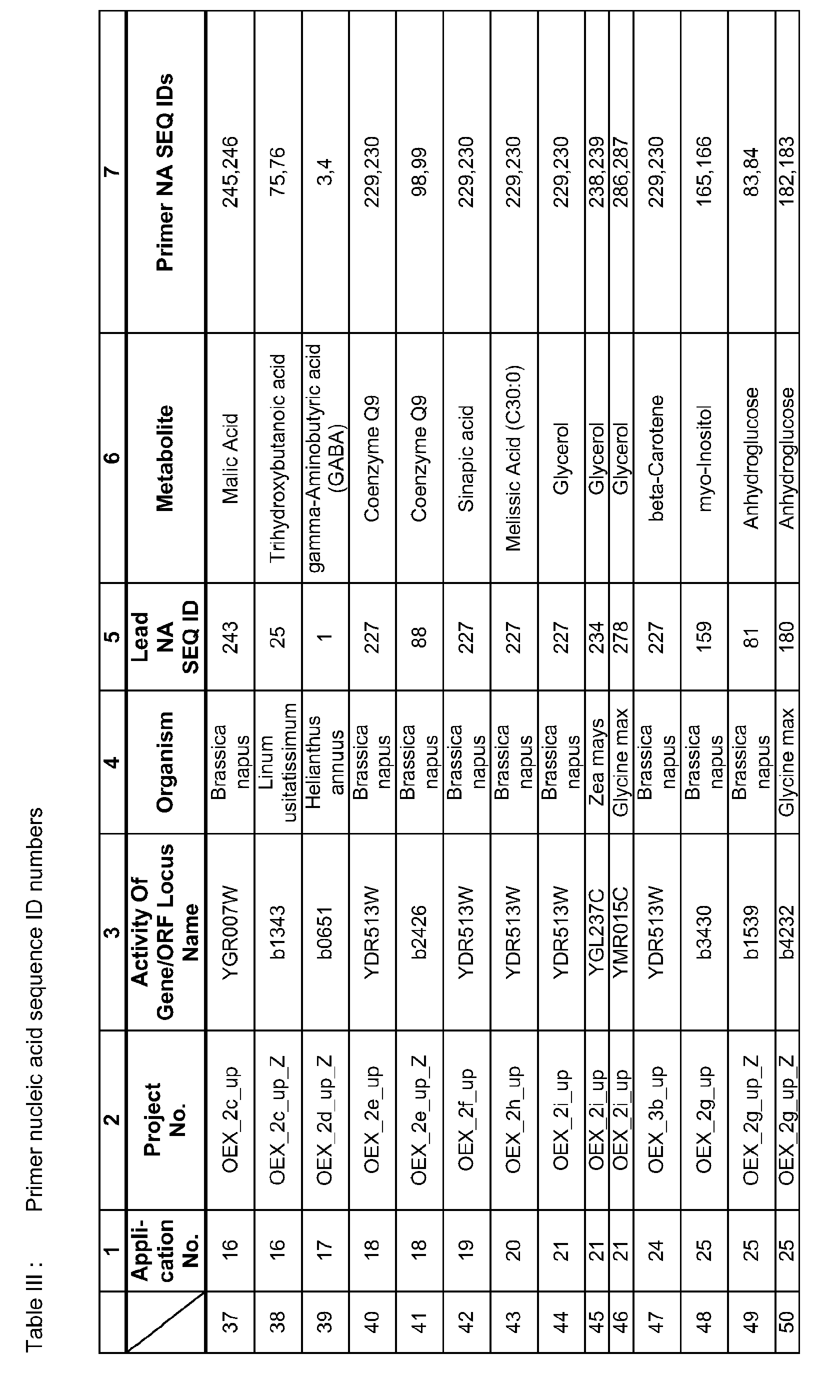

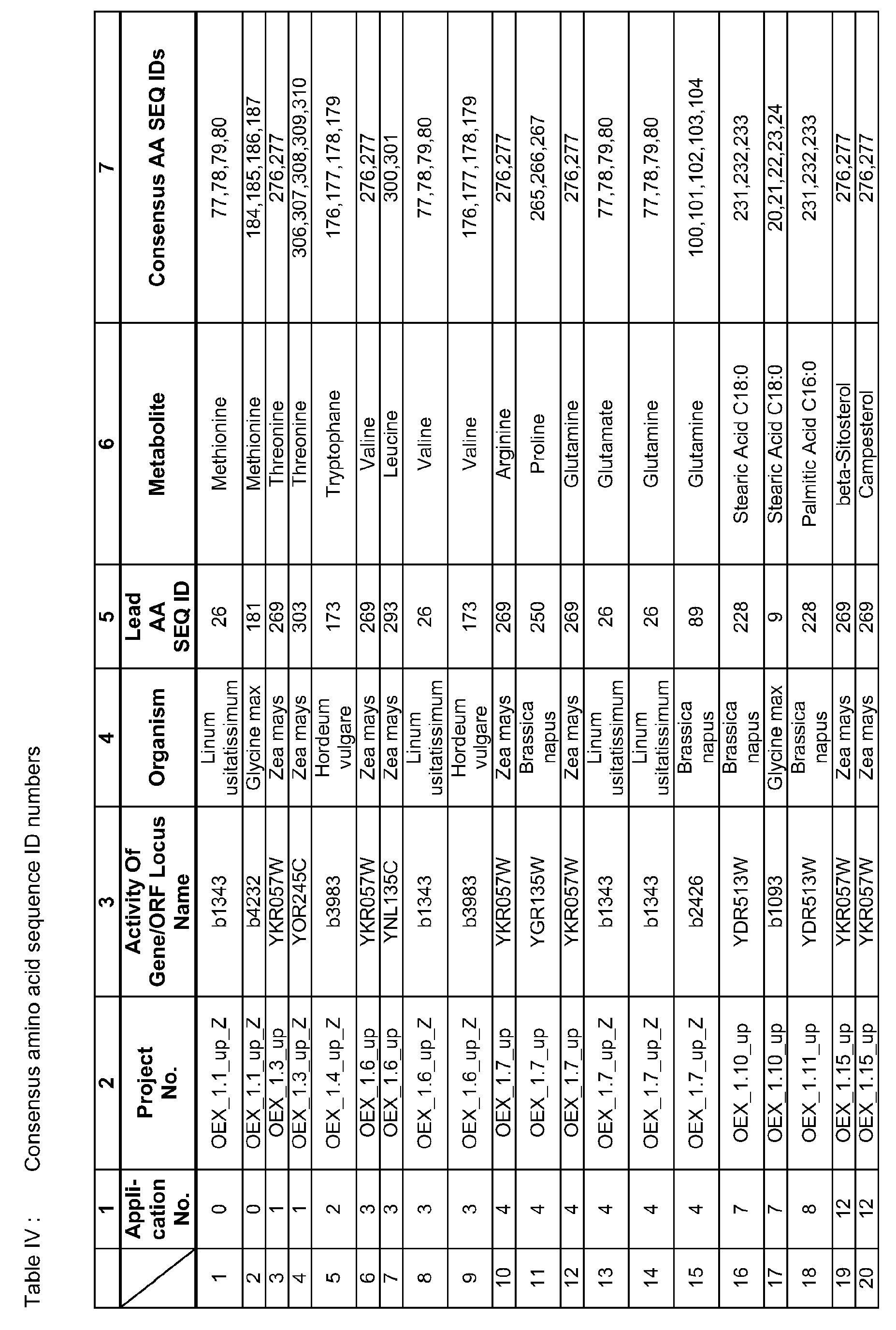

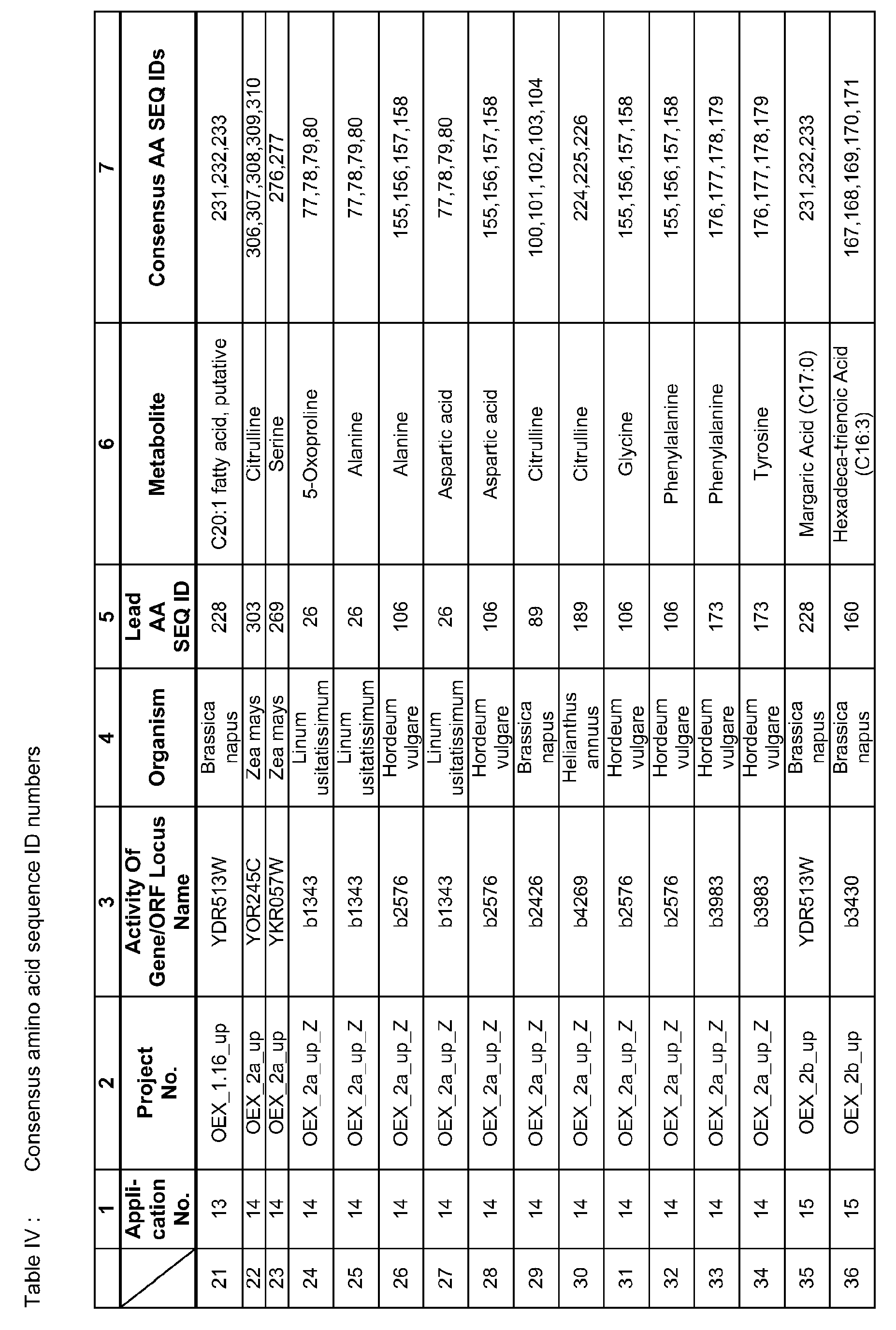

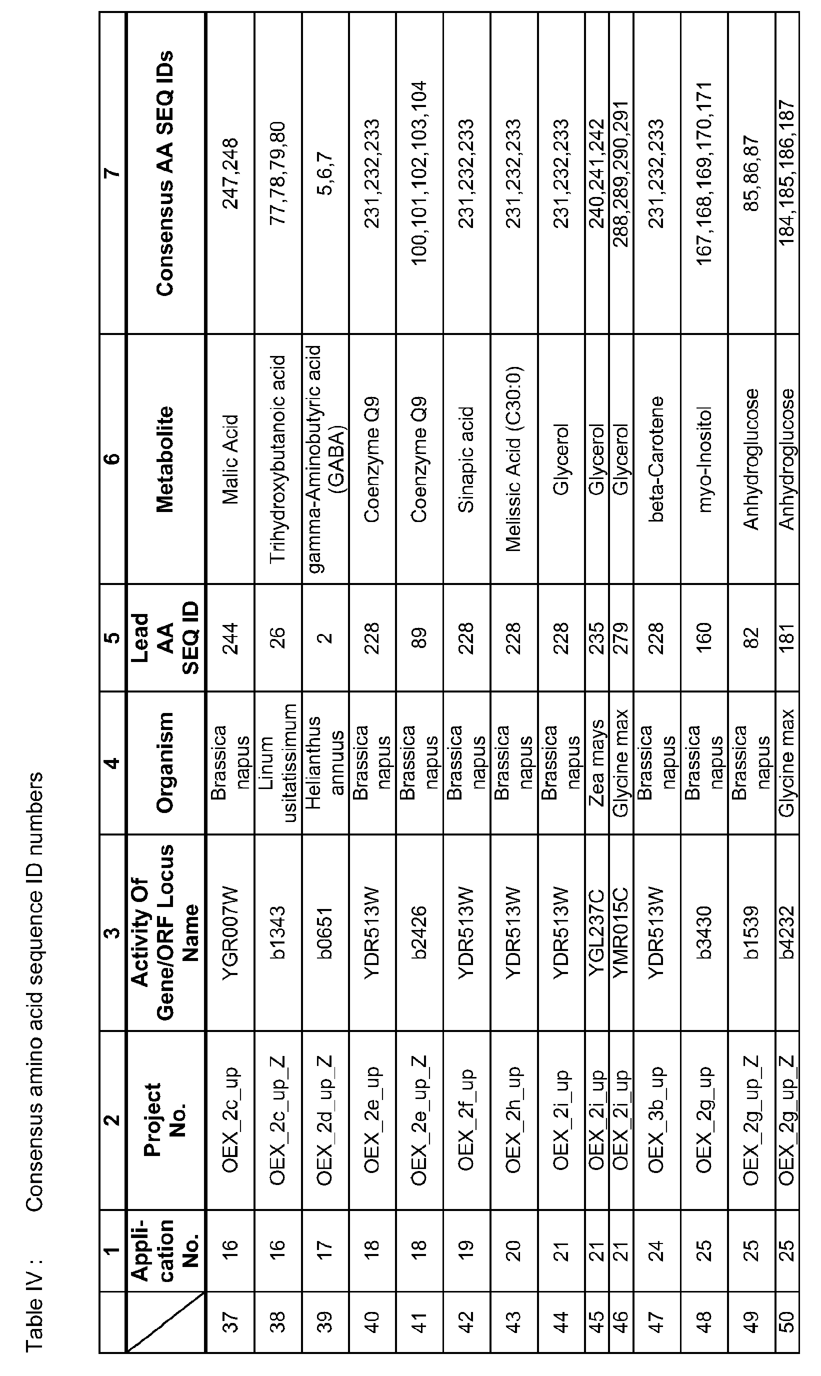

## Claims

1. A process for the production of methionine, which comprises
(a) increasing or generating the activity of a protein as indicated in Table II, application no. 0, columns 5 or 7, or a functional equivalent thereof in a non-human organism, or in one or more parts thereof; and
(b) growing the organism under conditions which permit the production of methionine in said organism.

2. A process for the production of methionine, comprising the increasing or generating in an organism or a part thereof the expression of at least one nucleic acid molecule comprising a nucleic acid molecule selected from the group consisting of:
a) nucleic acid molecule encoding of a polypeptide as indicated in Table II, application no. 0, columns 5 or 7, or a fragment thereof, which confers an increase in the amount of methionine in an organism or a part thereof;
b) nucleic acid molecule comprising of a nucleic acid molecule as indicated in Table I, application no. 0, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of methionine in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of methionine in an organism or a part thereof;
e) nucleic acid molecule which hybridizes with a nucleic acid molecule of (a) to (c) under stringent hybridisation conditions and conferring an increase in the amount of methionine in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 0, columns 7, and conferring an increase in the amount of the respective fine chemical in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of methionine in an organism or a part thereof;
h) nucleic acid molecule encoding a polypeptide comprising a consensus sequence as indicated in Table IV, application no. 0, columns 7, and conferring an increase in the amount of the respective fine chemical in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule **characterized in** (a) to (k) and conferring an increase in the amount of the respective fine chemical in an organism or a part thereof.
or comprising a sequence which is complementary thereto.

3. The process of claim 1 or 2, comprising recovering of the free or bound methionine.

4. The process of any one of claim 1 to 3, comprising the following steps:
(a) selecting an organism or a part thereof expressing a polypeptide encoded by the nucleic acid molecule **characterized in** claim 2;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound methionine produced by the selected mutated organisms or parts thereof.

5. The process of any one of claims 1 to 4, wherein the activity of said protein or the expression of said nucleic acid molecule is increased or generated transiently or stably.

6. An isolated nucleic acid molecule comprising a nucleic acid molecule selected from the group consisting of:
a) nucleic acid molecule encoding of a polypeptide as indicated in Table II, application no. 0, columns 5 or 7, or a fragment thereof, which confers an increase in the amount of methionine in an organism or a part thereof;
b) nucleic acid molecule comprising of a nucleic acid molecule as indicated in Table I, application no. 0, columns 5 or 7;
c) nucleic acid molecule whose sequence can be deduced from a polypeptide sequence encoded by a nucleic acid molecule of (a) or (b) as a result of the degeneracy of the genetic code and conferring an increase in the amount of methionine in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of methionine in an organism or a part thereof;
e) nucleic acid molecule which hybridizes with a nucleic acid molecule of (a) to (c) under stringent hybridisation conditions and conferring an increase in the amount of methionine in an organism or a part thereof;
f) nucleic acid molecule which encompasses a nucleic acid molecule which is obtained by amplifying nucleic acid molecules from a cDNA library or a genomic library using the primers or primer pairs as indicated in Table III, application no. 0, columns 7, and conferring an increase in the amount of the respective fine chemical in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of methionine in an organism or a part thereof;
h) nucleic acid molecule encoding a polypeptide comprising a consensus sequence as indicated in Table IV, application no. 0, columns 7, and conferring an increase in the amount of the respective fine chemical in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule **characterized in** (a) to (k) and conferring an increase in the amount of the respective fine chemical in an organism or a part thereof.
whereby the nucleic acid molecule distinguishes over the sequence as indicated in Table I, application no. 0, columns 5 or 7, by one or more nucleotides.

7. A nucleic acid construct which confers the expression of the nucleic acid molecule of claim 6, comprising one or more regulatory elements.

8. A vector comprising the nucleic acid molecule as claimed in claim 6 or the nucleic acid construct of claim 7.

9. The vector as claimed in claim 8, wherein the nucleic acid molecule is in operable linkage with regulatory sequences for the expression in a prokaryotic or eukaryotic, or in a prokaryotic and eukaryotic, host.

10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.

11. The host cell of claim 10, which is a transgenic host cell.

12. The host cell of claim 10 or 11, which is a plant cell, an animal cell, a microorganism, or a yeast cell, a fungus cell, a prokaryotic cell, an eukaryotic cell or an archaebacterium.

13. A process for producing a polypeptide, wherein the polypeptide is expressed in a host cell as claimed in any one of claims 10 to 12.

14. A polypeptide produced by the process as claimed in claim 13 or encoded by the nucleic acid molecule as claimed in claim 6 whereby the polypeptide distinguishes over a sequence as indicated in Table II, application no. 0, columns 5 or 7, by one or more amino acids.

15. An antibody, which binds specifically to the polypeptide as claimed in claim 14.

16. A plant tissue, propagation material, harvested material or a plant comprising the host cell as claimed in claim 12 which is plant cell or an Agrobacterium.

17. A method for screening for agonists and antagonists of the activity of a polypeptide encoded by the nucleic acid molecule of claim 6 conferring an increase in the amount of methionine in an organism or a part thereof comprising:
(a) contacting cells, tissues , plants or microorganisms which express the a polypeptide encoded by the nucleic acid molecule of claim 5 conferring an increase in the amount of methionine in an organism or a part thereof with a candidate compound or a sample comprising a plurality of compounds under conditions which permit the expression the polypeptide;
(b) assaying the methionine level or the polypeptide expression level in the cell, tissue, plant or microorganism or the media the cell, tissue, plant or microorganisms is cultured or maintained in; and
(c) identifying a agonist or antagonist by comparing the measured methionine level or polypeptide expression level with a standard methionine or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.

18. A process for the identification of a compound conferring increased methionine production in a plant or microorganism, comprising the steps:
(a) culturing a plant cell or tissue or microorganism or maintaining a plant expressing the polypeptide encoded by the nucleic acid molecule of claim 6 conferring an increase in the amount of methionine in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of methionine in an organism or a part thereof;
(b) identifying if the compound is an effective agonist by detecting the presence or absence or increase of a signal produced by said readout system.

19. A method for the identification of a gene product conferring an increase in methionine production in a cell, comprising the following steps:
(a) contacting the nucleic acid molecules of a sample, which can contain a candidate gene encoding a gene product conferring an increase in methionine after expression with the nucleic acid molecule of claim 6;
(b) identifying the nucleic acid molecules, which hybridise under relaxed stringent conditions with the nucleic acid molecule of claim 6;
(c) introducing the candidate nucleic acid molecules in host cells appropriate for producing methionine;
(d) expressing the identified nucleic acid molecules in the host cells;
(e) assaying the methionine level in the host cells; and
(f) identifying nucleic acid molecule and its gene product which expression confers an increase in the methionine level in the host cell in the host cell after expression compared to the wild type.

20. A method for the identification of a gene product conferring an increase in methionine production in a cell, comprising the following steps:
(a) identifying in a data bank nucleic acid molecules of an organism; which can contain a candidate gene encoding a gene product conferring an increase in the methionine amount or level in an organism or a part thereof after expression, and which are at least 20% homolog to the nucleic acid molecule of claim 6;
(b) introducing the candidate nucleic acid molecules in host cells appropriate for producing methionine;
(c) expressing the identified nucleic acid molecules in the host cells;
(d) assaying the methionine level in the host cells; and
(e) identifying nucleic acid molecule and its gene product which expression confers an increase in the methionine level in the host cell after expression compared to the wild type.

21. A method for the production of an agricultural composition comprising the steps of the method of any one of claims 17 to 20 and formulating the compound identified in any one of claims 17 to 20 in a form acceptable for an application in agriculture.

22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.

23. Use of the nucleic acid molecule as claimed in claim 6 for the identification of a nucleic acid molecule conferring an increase of methionine after expression.

24. Use of the polypeptide of claim 14 or the nucleic acid construct claim 7 or the gene product identified according to the method of claim 19 or 20 for identifying compounds capable of conferring a modulation of methionine levels in an organism.

25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
